# EUROPEAN PATENT APPLICATION

(11) **EP 1 403 282 A1**
(43) Date of publication of application: **31.03.2004**
(21) Application number: 02021809.5
(22) Date of filing: 26.09.2002
(51) Int. Cl.: C07K 14/705, C07K 14/715, C07K 16/28, C07K 17/00, A61K 38/17, G01N 33/50

(54) **Protein complexes of the Tumor necrosis factor-alpha (TNF-alpha) signalling pathway**

(71) Applicant: CELLZOME AG, 69117 Heidelberg (DE)
(72) Inventor: Bouwmeester, Tewis, 69120 Heidelberg (DE); Huhse, Bettina, 69126 Heidelberg (DE); Bauch, Angela, 69117 Heidelberg (DE); Ruffner, Heinz, 69245 Bammertal (DE); Bauer, Andreas, 69123 Heidelberg (DE); Kruse, Ulrich, 69221 Dossenheim (DE); Küster, Bernhard, 69254 Malsch (DE); Superti-Furga, Guilio, 69118 Heidelberg (DE)
(74) Representative: Grünecker, Kinkeldey, Stockmair & Schwanhäusser Anwaltssozietät

(57) **Abstract**

The present invention relates to protein complexes of the Tumor necrosis factor-α-signaling pathway, component proteins of said complexes, fragments and derivatives of the component proteins and antibodies specific to the complexes. The present invention also relates to methods for use of the complexes of the TNF-α-signaling pathway and their interaction in screening, diagnosis and therapy as well to methods of preparing the complexes.

## Description

### 1. FIELD OF THE INVENTION

The present invention relates to the protein complexes of the Tumor necrosis factor-α-(TNF-α)-signaling pathway, component proteins of the said complexes, fragments and derivatives of the component proteins, and antibodies specific to the complexes. The present invention also relates to methods for use of the complexes of the TNF-α-signaling pathway and their interaction in, inter alia, screening, diagnosis, and therapy, as well as to methods of preparing the complexes.

### 2. BACKGROUND OF THE INVENTION

Tumor necrosis factor-α (TNF-α) is an important cytokine involved in the signaling of a number of cellular responses including inflammatory, immunoregulatory, proliferative, cytotoxic, and anti-viral activities [Beutler B and Cerami A, Ann. Rev. Biochem, 57:505-518 (1988)]. The induction of the various cellular responses mediated by TNF-α is initiated by its interaction with two distinct cell surface receptors, TNFR1 (55 kDa) and TNFR2 (75 kDa), both members of the TNF receptor superfamily [Locksley RM Cell 104:487-501 (2001)]. Both receptors mediate the activation of the evolutionary conserved transcription factor NF-κB [Lenardo M and Baltimore D Cell 58(2):227-9 (1989)]. The NF-κB system is composed of homo- and heterodimers of members of the Rel family of related transcription factors. The activity of NF-κB transcription factors is regulated by their subcellular localization [Verma IM Genes Dev 9(22):2723-35 (1995)]. In resting cells NF-κB is sequestered in the cytoplasm in an inactive form, complexed to members of a family of inhibitory proteins referred to as IκB [Verma IM Genes Dev 9(22):2723-35 (1995); Finco TS and Baldwin AS Immunity 3(3):263-72 (1995)]. IκBs mask the nuclear localization signal of hetero- and homodimeric NF-κB-complexes and thereby prevent nuclear translocation of NF-κB. In response to TNF-α IκBs are phosphorylated and subsequently degraded via an ubiquitin-dependent mechanism, allowing NF-κB translocation into the nucleus and concomitant activation of genes involved in immune and inflammatory responses.

In recent years, a number of components of the TNF-α pathway has been identified and insights in their regulation and regulatory potential have been obtained. An overview of those findings is given below.

### TNF-α RECEPTORS:

Tumor necrosis factor-α (TNF-α) is a pleiotropic cytokine that plays an important role in immunological and inflammatory responses. It exerts its biological effects via two distinct membrane receptors of apparent molecular weight of 55 kDa (p55TNFR; TNFR1) and 75 kDa (p75TNFR; TNFR2), respectively. In contrast to the extracellular domains, the cytoplasmic domains of TNFR1 and TNFR2 are unrelated.

### TNFR1:

TNFR1, is the predominant receptor through which TNF-α elicits its unusually wide range of biological responses, including inflammation, tumor necrosis, cell proliferation, differentiation, and apoptosis. Homotrimeric TNF-α binding to the extracellular domain of TNFR1 leads to homotrimerisation and activation of the receptor. Ligation of TNFR1 by TNFα recruits the death domain-containing adaptor TRADD (TNFR associated death domain) to the receptor's cytosolic death domain. TRADD recruits FADD which in turn recruits procaspase-8, thereby transducing the apoptotic signal. TRADD also serves as an adaptor to recruit the serine/threonine kinase RIP and the adaptor TRAF2, which are implicated in activation of the NF-κB and the JNK/AP-1 pathways.

### TNFR2:

TNFR2 is believed to transduce only anti-apoptotic and inflammatory responses through the recruitment of TRAF1 and TRAF2. TNF-α stimulation of TNFR2 results in the recruitment of TRAF1 and TRAF2 and the 'inhibitors of apoptosis proteins', c-lAP1 and c-IAP2, which both have RING domain-dependent ubiquitin protein ligase (E3) activity. TNF-α binding to TNFR2 induces ubiquitination and proteasomal degradation of TRAF2 due to the E3 ligase activity of c-IAP1. These findings suggest a mechanism by which TNFR2-regulated ubiquitin protein ligase activity can potentiate TNF-α-induced apoptosis.

### ADAPTORS:

### I-TRAF:

TRAF proteins associate with and transduce signals from members of the TNF receptor superfamily. TRAF proteins share a conserved C-terminal TRAF domain that is involved in oligomerization and receptor association. TANK (i-TRAF) is a ubiquitously expressed 425-amino acid protein. I-TRAF binds to the TRAF-C domains of TRAF1, 2, and 3. Overexpression of I-TRAF inhibits TRAF2-mediated NF-κB activation by CD40, TNFR1 and TNFR2 suggesting that I-TRAF is an inhibitor of TRAF function by sequestering TRAFs in a latent state in the cytoplasm.

### TRADD:

TNFR1-associated death domain protein (TRADD) is a 34 kDa cytosolic protein that binds to the 80 amino acid intracellular, death domain of TNFR1. Overexpression of TRADD induces two major TNF-α-mediated responses, namely an apoptotic response and the activation of NF-κB, a pro-inflammatory response. The predicted 312-amino acid TRADD protein contains a carboxy-terminal, 111-amino acid death domain with sequence similarity to that of TNFR1. This region of TRADD is capable of mediating TRADD oligomerization, TNFR1 interaction, stimulation of apoptosis, and NF-κB activation. The TRADD death domain can either bind to FADD, which propagates the TNFR1-mediated apoptotic response or RIP, which activates the pro-inflammatory, NF-κB pathway. The amino-terminal domain of TRADD binds to TRAF2, which contributes to the pro-inflammatory cascade. TRADD recruitment to TNFR1 is TNF-α-dependent and is believed to displace SODD ('silencer of death domains') in the first step of receptor activation.

### FADD:

FADD (FAS-associating protein with death domain) was isolated by a yeast 2-hybrid screen with the cytoplasmic domain of FAS as bait. The in vivo interaction of FADD with FAS is due to the association of the respective death domains. Overexpression of FADD in mammalian cells induces apoptosis, which like FAS-induced apoptosis, was blocked by CrmA, a poxvirus gene product. Based on this it has been proposed that FADD may play an important role in the proximal signal transduction of FAS and TNF-α. The interaction of FADD and FAS through their C-terminal death domains unmasks the N-terminal effector domain of FADD, allowing it to recruit caspase-8 (CASP8) to the FAS signalling complex and thereby activating a cysteine protease cascade, leading to cell death.

### INHIBITORS:

### IκB

NF-κB complexes are normally inhibited by IκB proteins, which retain NF-κB in the cytoplasm. Phosphorylation of serine residues on the IκB proteins by IκB kinases, in particular the IKK complex marks them for destruction via the ubiquitination pathway, thereby allowing nuclear translocation of the NF-κB complex. NF-κB is rapidly activated by a variety of stimuli, including pro-inflammatory cytokines such as TNF-α and IL-1. There are seven known IκB family members. The IκBs are characterized by the presence of multiple ankyrin repeats, which are protein-protein interaction domains that interact with NF-κB.

### IKK-signalosome:

The IκB kinase (IKK) complex is an essential mediator in the activation of NF-κB in response to a variety of pro-inflammatory stimuli, such as TNF-α and IL-1. The IKK complex consists of two catalytic kinase subunits, IKKα and IKKβ, and a scaffolding, regulatory subunit, IKKγ (NEMO). Both kinase subunits are associated with a dimeric, kinase-specific chaperone complex, consisiting of CDC37 and HSP90. The IKK signalosome activates nuclear translocation of NF-κB complexes via phosphorylation of the inhibitor IκB proteins. This phosphorylation earmarks IκBs for ubiquitination and subsequent proteasome-mediated proteolytic degradation. The IKK complex might also phosphorylate RelA, enhancing its transcriptional activity. Because the IKK complex is a converging point for integrating a variety of NF-κB-activating stimuli, the essential kinase component, IKKβ is a hot therapeutic target for pro-inflammatory conditions. However, the precise mechanism by which the IKK complex is activated and inactivated is still elusive.

### KINASES:

### RIP:

The death domain of TNFR1 triggers distinct signalling pathways leading to apoptosis and NF-κB activation through its interaction with the death domain protein TRADD and disctinct secondary adaptor proteins. TRADD recruits receptor-interacting protein (RIP), a serine/threonine kinase to the TNFR1 complex in a TNF-α-dependent process. RIP is a 671-amino acid protein with an N-terminal protein kinase domain, a C-terminal death domain, and a unique internal region, called the intermediate domain. RIP can interact with TRADD, TRAF1, TRAF2, and TRAF3. RIP and TRADD interact via their respective death domains; RIP interacts with TRAF2 via either the kinase or the intermediate domain. Overexpression of RIP induces both NF-κB activation and apoptosis, but overexpression of the RIP death domain only blocks TNF-α-mediated NF-κB activation. Although RIP has serine/threonine kinase activity, this kinase activity is not required to activate NF-κB. RIP activity is essential for TNF-α-mediated activation of NF-κB. The precise mechanism by which RIP activates distinct intracellular cascades remains to be determined.

### RIP2:

RIP2 (RICK) is a 540-amino acid protein with a N-terminal serine/threonine kinase domain and a C-terminal caspase activation and recruitment domain (CARD). CARDs mediate homophilic interactions allowing for the recruitment of caspases to receptor complexes, and have been identified in a number of molecules involved in apoptotic signalling, including caspase-2 and c-IAPs. RIP2 physically interacts with CLARP, a caspase-like molecule known to bind to FADD and caspase-8. Expression of RIP2 promoted the activation of caspase-8 and potentiated apoptosis induced by FAS, FADD, CLARP, and caspase-8. RIP2 is required for multiple pathways regulating immune and inflammatory responses.

### RIP3:

RIP3 is a 518-amino acid protein with a N-terminal kinase domain similar to those of RIP and RIP2 but a distinct C-terminus that lacks homology to the death domain of RIP or the caspase-recruiting domain (CARD) of RIP2. Overexpression of RIP3, like RIP can activate NF-κB and induce apoptosis. The kinase domain is dispensable for both NF-κB activation or apoptosis induction. Coimmunoprecipitation analysis suggest that the kinase and C-terminal regions of RIP3 interact with RIP. RIP3 was also found to interact with TNFR1 and TRAF2. Overexpression of a dominant-negative mutant of RIP3 only inhibited caspase activation, suggesting that RIP3 acts as an intermediary in TNF-α-induced apoptosis.

### MODULATORS:

### ABIN-2:

ABIN-2 is an evolutionarily non-conserved, novel, cytosolic protein that binds to the zinc finger protein A20. Overexpression of ABIN-2, akin to ectopic expression of A20 blocks NF-κB activation by pro-inflammatory stimuli, like TNF-α and IL-1. Since A20 is a TNF-α inducible target gene, ABIN-2 is believed to play a role in an A20-dependent negative regulatory feedback loop. ABIN-2 has no obvious motifs, but does share a short region of homology with ABIN, an otherwise unrelated A20 binding protein and IKKγ, the regulatory subunit of the IKK signalosome.

### c-IAP1:

The "inhibitor of apoptosis" (IAP) gene family encodes a group of structurally related proteins that have the ability to suppress apoptotic cell death. The prototype IAP was described in baculovirus genomes by means of a genetic screen to identify regulators of host-cell viability during virus infection. The discovery led to the identification of cellular orthologues in several species such as yeast, nematodes, flies and humans. Two domains were identified in baculovirus IAPs: the baculovirus IAP repeat (BIR) and the RING domain. c-IAP1 and c-IAP2 are structural components of the TNFR2-complex. c-IAP1 was found to direct the ubiquitination of TRAF2 that required an intact and E3-competent RING domain.

### MAP-KINASES:

Mitogen-activated protein kinases (MAPK) have crucial roles in cellular responses to various extracellular signals. The prototypical module of MAP kinase activation is a cascade of three kinases, consisting of MAP3K, MAPKK and MAPK.

### MEKK1:

MEKK1 is a 196-kDa protein that generates anti-apoptotic signalling as a full-length protein but induces apoptosis when cleaved by caspases. Caspase cleavage of MEKK1 is a dynamic regulatory mechanism that alters the subcellular distribution of MEKK1, changing its function to pro-apoptotic signalling. MEKK1 acts also as an E3 ubiquitin ligase through its PHD domain mediating ubiquitination and degradation of ERK1/2. This novel MEKK1 signalling role implicates a negative feedback mechanism for the regulation of survival pathways during persistant stress stimuli.

### MEKK3:

MEKK3 is a ubiquitously expressed MAP3K that was described as a regulator of JNK and ERK pathways by activating SEK and MEK1/2, but not p38. Later it was shown that MEKK3 directly activates MKK6 and MKK7, specific activators of the p38 and JNK kinases. It also regulates MEK5 activity as part of the big mitogen-activated protein kinase 1 (BMK1) pathway. The ability of MEKK3 to simultaneously activate the SAPK and ERK pathways is remarkable, given that they have divergent roles in cellular homeostasis. MEKK3 (and MEKK2) enhanced transcription from a NF-KB dependent reporter as was previously shown only for NIK and MEKK1. This is consistent with a role in the JNK pathway. In MEKK3-deficient fibroblasts, it was shown that MEKK3 is required for IKK activation and functions downstream of RIP and TRAF2. MEKK3 interacts with RIP and phosphorylates IKK. The kinase activity of MEKK3 is pivotal to its function. It is suggested that MEKK3 links RIP and IKK in TNF-α-induced NF-KB activation.

### TAK1:

TAK1 is a MAP kinase kinase kinase (MAP3K) involved primarily in the IL-1 signalling pathway. Following IL-1 stimulation, TAK1 is recruited to the TRAF6 complex and activated via TRAF6-mediated ubiquitination. Activated TAK1 then in turn stimulates a mitogen-activated protein (MAP)-kinase cascade, in particular JNK leading to NF-κB activation. TAK1 binds to TAB1 and TAB2, the latter of which facilitates the interaction between TAK1 and TRAF6.

### p38:

p38 is a member of the stress-activated protein kinase (SAPK) class of MAPKs, which are activated by most environmental stresses. p38 is also strongly activated by pro-inflammatory stimuli such as IL-1 and TNF-α, which play an important role in the regulation of the inflammatory response. p38 MAPK is activated by MAPKKs MKK3 and MKK6 as well as by a TAB1-dependent autophosphorylation of p38. The latter implies an alternative p38 activation mechanism mediated by protein interactions that are independent of phosphorylation mediated by MAPKK.

### NF-κBs:

### p105/p50 (NF-κB1):

NF-κB represents a group of structurally related and evolutionarily conserved proteins, with five members in mammals: Rel (c-Rel), RelA (p65), RelB, NF-κB1 (p50 and its precursor p105) and NF-κB2 (p52 and its precursor p100). All NF-κB proteins share a highly conserved 300 amino acid Rel homology domain (RHD) responsible for DNA binding, dimerization and association with IκB proteins. Proteolytic processing of p105, the precursor of NF-κB1 p50 is a constitutive event and liberates NF-κB1 p50-containing complexes into the nucleus. Processing requires phosphorylation and ubiquitination of the carboxy-terminal ankyrin repeats.

### ReIA (p65):

ReIA can bind DNA and has strong transactivation potential. The most widely studied and most abundant form of NF-κB is a heterodimer of p50/p65 which is a potent activator of transcription requiring the transcriptional activity of ReIA (p65), which is regulated by inducible phosphorylation. ReIA can interact with a variety of coactivator and corepressor proteins.

Thus, new approaches to modulating NF-κB activation, regulating IκB turnover and more generally the discovery of novel regulatory proteins in the TNF-α signalling pathway at all signalling levels and the elucidation of their roles in inflammatory gene activation gives the opportunity to the identification of the next generation of important anti-inflammatory drugs.

However, up to date, the development of novel potent drugs for the large spectrum of diseases in which the TNF-α-pathway is involved in is still very much hampered by an insufficient knowledge of the molecular interactions and networks underlying the regulation and function of that pathway.

Thus, in order to achieve these aims, there is an as yet unfulfilled need in the art to chararterize the molecular context in which the known components of the TNF-α-pathway function.

### 3. SUMMARY OF THE INVENTION

An object of the present invention was to identify protein complexes of the Tumor necrosis factor-α (TNF-α) signalling pathway, component proteins of the said complexes, fragments and derivatives of the component proteins, and antibodies specific to the complexes. The present invention also relates to methods for use of the complexes of the TNF-α signalling pathway and their interaction in, inter alia, screening, diagnosis, and therapy, as well as to methods of preparing the complexes.

By applying the process according to the invention said complexes were identified. The components are listed in table 1.
Furthermore, the interactions between the protein complexes of the TNF-α-signalling pathway and the differential assembly of the complexes in different cellular conditions provide new insights into the mechanisms of the physiological and pathological processes associated with this biological pathway.
Said object is further achieved by the characterisation of component proteins. These proteins are listed in table 2

Thus, the invention relates to the following embodiments
1. A protein complex selected from complex (I) and comprising
   (a) at least one first protein, which first protein is selected from the group of proteins in table 1, seventh column of a given complex, or a functionally active derivative thereof, or a functionally active fragment thereof, or a homolog thereof, or a variant of said protein encoded by a nucleic acid that hybridizes to the nucleic acid encoding said protein under low stringency conditions; and
   (b) at least one second protein, which second protein is selected from the group of proteins in table 1, eighth column of said complex, or a functionally active derivative thereof, or a functionally active fragment thereof, or a homolog thereof, or a variant of said second protein encoded by a nucleic acid that hybridizes to the nucleic acid encoding said protein under low stringency conditions;
   and a complex (II) comprising at least two of said second proteins,
   wherein said low stringency conditions comprise hybridization in a buffer comprising 35% formamide, 5X SSC, 50mM Tris-HCl (pH 7.5), 5 mM EDTA, 0.02% PVP, 0.02% BSA, 100 microg/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1-5 hours at 55°C, and washing in a buffer consisting of 2X SSC, 26 mM Tris-HCl (pH 7.4) 5 mM EDTA, and 0.1% SDS for 1.5 hours at 60°C.
2. A protein complex comprising a first protein selected from the proteins listed in table 1, second column of a given complex or a homolog or variant thereof, or a functionally active fragment or functionally active derivative of said first protein, the variant being encoded by a nucleic acid that hybridizes to the nucleic acid of said first protein under low stringency conditions, and at least one second protein selected from the group of proteins in table 1, eighth column of a given complex, or a variant or homolog thereof, or a functionally active fragment or a functionally active derivative of said second protein, the variant of said second protein being encoded by a nucleic acid that hybridizes to the nucleic acid of said second protein under low-stringency conditions, and wherein said low stringency conditions comprise hybridization in a buffer comprising 35% formamide, 5X SSC, 50mM Tris-HCl (pH 7.5), 5 mM EDTA, 0.02% PVP, 0.02% BSA, 100 microg/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1-5 hours at 55°C, and washing in a buffer consisting of 2X SSC, 26 mM Tris-HCl (pH 7.4) 5 mM EDTA, and 0.1% SDS for 1.5 hours at 60°C.
3. A protein complex selected from complex (I) and comprising the proteins selected from the proteins in table 1, third column or a homolog thereof, or a variant thereof or functionally active fragments or functionally active derivatives of said proteins, said variant being encoded by a nucleic acid that hybridizes to the nucleic acid of said protein under low stringency conditions;
   and a protein complex selected from complex (II) and comprising the proteins selected from the proteins in table 1, fourth column or a homolog thereof, or a variant thereof or functionally active fragments or functionally active derivatives of said proteins, the variant being encoded by a nucleic acid that hybridizes to the nucleic acid of said protein under low stringency conditions, to the nucleic acid of said protein under low stringency conditions;
   and a protein complex selected from complex (III) and comprising the proteins selected from the proteins in table 1, fifth column or a homolog thereof, or a variant thereof or functionally active fragments or functionally active derivatives of said proteins, the variant being encoded by a nucleic acid that hybridizes to the nucleic acid of said protein under low stringency conditions;
   and a protein complex selected from complex (IV) and comprising the proteins selected from the proteins in table 1, sixth column or a homolog thereof, or a variant thereof or functionally active fragments or functionally active derivatives of said proteins, the variant being encoded by a nucleic acid that hybridizes to the nucleic acid of said protein under low stringency conditions;
   wherein said low stringency conditions comprise hybridization in a buffer comprising 35% formamide, 5X SSC, 50mM Tris-HCl (pH 7.5), 5mM EDTA, 0.02% PVP, 0.02% Ficoll, 0.2% BSA, 100 microg/ml denatured salmon sperm DNA, and 10% (wt/vol) dextran sulfate for 18-20 hours at 40°C, washing in a buffer consisting of 2X SSC, 25mM Tris-HCl (pH 7.4), 5mM EDTA, and 0.1% SDS for 1-5 hours at 55°C, and washing in a buffer consisting of 2X SSC, 25mM Tris-HCl (pH 7.4), 5mM EDTA, and 0.1 % SDS for 1.5 hours at 60°C.
4. A protein complex that comprises all proteins as listed in table 1, sixth column for a given complex or a homolog or a variant thereof, or a functionally active fragment or a functionally active derivative thereof, the variant being encoded by a nucleic acid that hybridizises to the nucleic acid of any of said proteins under low stringency conditions, but 1 to the number of proteins listed in table 1, eighth column of said complex, or a homolog or a variant thereof, or a functionally active fragment or functionally active derivative thereof, the variant being encoded by a nucleic acid that hybridizes to the nucleic acid of any of said proteins of said eigth column under low stringency conditions, wherein said low stringency conditions comprise hybridization in a buffer comprising 35% formamide, 5X SSC, 50mM Tris-HCl (pH 7.5), 5mM EDTA, 0.02% PVP, 0.02% Ficoll, 0.2% BSA, 100 microg/ml denatured salmon sperm DNA, and 10% (wt/vol) dextran sulfate for 18-20 hours at 40°C, washing in a buffer consisting of 2X SSC, 25mM Tris-HCl (pH 7.4), 5mM EDTA, and 0.1% SDS for 1-5 hours at 55°C, and washing in a buffer consisting of 2X SSC, 25mM Tris-HCl (pH 7.4), 5mM EDTA, and 0.1 % SDS for 1.5 hours at 60°C..
5. The complex of any of No.1 to 4 comprising at least one functionally active derivative of said first protein and/or a functionally active derivative of said second protein, wherein the functionally active derivative is a fusion protein comprising said first protein or said second protein fused to an amino acid sequence different from the first protein or second protein.
6. The complex of No. 5 wherein the functionally active derivative is a fusion protein comprising said first protein or said second protein fused to an affinity tag or label.
7. The complex of any of No.1 to 4 comprising a fragment of said first protein and/or a fragment of said second protein, which fragment binds to another protein component of said complex.
8. The complex of any of No.1 to 7 that is involved in the biochemical activity as stated in table 3.
9. A process for preparing a complex of any of No.1-8 and optionally the components thereof comprising the following steps:
   Expressing a protein of the complex, preferably a tagged protein, in a target cell, isolating the protein complex which is attached to the protein, preferably a tagged protein, and optionally disassociating the protein complex and isolating the individual complex members.
10. The process according to No.9 wherein the tagged protein comprises two different tags which allow two separate affinity purification steps.
11. The process according to any of No.9-10 wherein the two tags are separated by a cleavage site for a protease.
12. Component of a protein complex obtainable by a process according to any of No.9-11.
13. Protein selected from the group of proteins in table 1, ninth column of a given complex a homolog thereof, and a variant of thereof, or a functionally active fragment or a functionally active derivative of said protein, the variant being encoded by a nucleic acid that hybridizes to the nucleic acid encoding said protein under low stringency conditions, wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 microg/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1.5 hours at 55°C, and washing in a buffer consisting of 2X SSC, 25 mM Tris-HCl (pH 7.4), 5 mM EDTA, and 0.1 % SDS for 1.5 hours at 60°C.
14. Nucleic acid encoding a protein according to No.13.
15. Construct, preferably a vector construct, comprising
   (a) a nucleic acid according to No.14 and at least one further nucleic acid which is normally not associated with said nucleic acid, or
   (b) at least two separate nucleic acid sequences each encoding a different protein, or a functionally active fragment or a functionally active derivative thereof, or a homolog or a variant thereof, at least one of said proteins being selected from the first group of proteins according to No.1 (a) and at least one of said proteins, being selected from the second group of proteins according to No.1 (b).
16. Host cell, containing a vector comprising at least one of the nucleic acid of No. 14 and /or a construct of No.15 or containing several vectors each comprising at least the nucleic acid encoding at least one protein selected from the first group of proteins according to No.1(a) and the nucleic acid encoding at least one protein selected from the second group of proteins according to No. 1(b).
17. An antibody or a fragment of said antibody containing the binding domain thereof, which binds the complex of any of No.1 to 8 and which does not bind any of the proteins of said complex when uncomplexed and/or an antibody of a fragment of said antibody containing the binding domain thereof which binds to any of the group of proteins according to No.13.
18. A kit comprising in one or more containers the complex of any of No.1-8 and/or the proteins of No.13, optionally together with an antibody according to No.17 and/or further components such as reagents and working instructions.
19. The kit according to No.18 for processing a substrate of a complex of any one of No.1-8.
20. The kit according to No.18 for the diagnosis or prognosis of a disease or a disease risk, preferentially for a disease or disorder such as inflammation, such as chronic inflammatory diseases, rheumatoid arthritis, inflammatory bowel disease; infectious diseases such as septic shock and bacterial infections; neurological diseases such as stroke-induced inflammation in neurons; neurodegenerative diseases; cancer such as prostate cancer, breast cancer and skin cancer.
21. Array in which at least a complex according to any of No.1 to 8 and/or at least one protein according to No. 13 and/or at least one antibody according to No. 17 is attached to a solid carrier.
22. A process for processing a substrate of a complex of any one of No.1-8 comprising the step of bringing into contact a complex to any of No.1-8 with said substrate, such that said substrate is processed.
23. A pharmaceutical composition comprising the protein complex of any of No.1-8 and/or any of the proteins according to No. 13.
24. A pharmaceutical composition according to No.23 for the treatment of diseases and disorders, preferentially for diseases or disorders such as inflammation, such as chronic inflammatory diseases, rheumatoid arthritis, inflammatory bowel disease; infectious diseases such as septic shock and bacterial infections; neurological diseases such as stroke-induced inflammation in neurons; neurodegenerative diseases; cancer such as prostate cancer, breast cancer and skin cancer.
25. A method for screening for a molecule that binds to the complex of any one of No.1-8 and/or any of the proteins of No.13 , comprising the following steps:
   (a) exposing said complex or protein, or a cell or organism containing said complex or said protein, to one or more candidate molecules; and
   (b) determining whether said candidate molecule is bound to the complex or protein.
26. A method for screening for a molecule that modulates directly or indirectly the function, activity, composition or formation of the complex of any one of No. 1-8 comprising the steps of:
   (a) exposing said complex, or a cell or organism containing said complex to one or more candidate molecules; and
   (b) determining the amount of, activity of, protein components of, and/or intracellular localization of, said complex and/or the transcription level of a gene dependent on the complex and/or the abundance and/or activity of a protein or protein complex dependent upon the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependent on the complex and/or the abundance and/or activity of a protein or protein complex dependent on the function of the complex and/or product of a gene dependent on the complex in the absence of said candidate molecules indicates that the molecule modulates function, activity, or composition of said complex.
27. The method of No.26, wherein the amount of said complex is determined.
28. The method of No.26, wherein the activity of said complex is determined.
29. The method of No.28, wherein said determining step comprises isolating from the cell or organism said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
30. The method of No.26, wherein the amount of the individual protein components of said complex are determined.
31. The method of No.30, wherein said determining step comprises determining whether any of the proteins listed in table 1, sixth column of said complex, or a functionally active fragment or a functionally active derivative thereof, or a variant or a homolog thereof, the variant being encoded by a nucleic acid that hybridizes to the nucleic acid of said protein under low-stringency conditions, is present in the complex.
32.The method of any of No.26 to 31, wherein said method is a method of screening for a drug for treatment or prevention of a disease or disorder, preferentially of a disease or disorder selected from the diseases or disorders such as inflammation, such as chronic inflammatory diseases, rheumatoid arthritis, inflammatory bowel disease; infectious diseases such as septic shock and bacterial infections; neurological diseases such as stroke-induced inflammation in neurons; neurodegenerative diseases; cancer such as prostate cancer, breast cancer and skin cancer.
33. Use of a molecule that modulates the amount of, activity of, or the protein components of the complex of any one of No. 1-8 for the manufacture of a medicament for the treatment or prevention of a disease or disorder, preferentially of a disease or disorder such as inflammation, such as chronic inflammatory diseases, rheumatoid arthritis, inflammatory bowel disease; infectious diseases such as septic shock and bacterial infections; neurological diseases such as stroke-induced inflammation in neurons; neurodegenerative diseases; cancer such as prostate cancer, breast cancer and skin cancer.
34. A method for the production of a pharmaceutical composition comprising carrying out the method of No.26-31 to identify a molecule that modulates the function, activity, composition or formation of said complex, and further comprising mixing the identified molecule with a pharmaceutically acceptable carrier.
35. A method for diagnosing or screening for the presence of a disease or disorder or a predisposition for developing a disease or disorder in a subject, which disease or disorder is characterized by an aberrant amount of, component disposition of, or intracellular localization of the complex of any one of the No.1-8, comprising determining the amount of, activity of, protein components of, and/or intracellular localization of, said complex and/or the transcription level of a gene dependent on the complex and/or the abundance and/or activity of a protein or protein complex dependent on the function of the complex and/or product of a gene dependent on the complex in a comparative sample derived from a subject, wherein a difference in said amount, activity, or protein components of, said complex in an analogous sample from a subject not having the disease or disorder or predisposition indicated the presence in the subject of the disease or disorder or predisposition in the subject.
36. The method of No.35, wherein the amount of said complex is determined.
37. The method of No.35, wherein the activity of said complex is determined.
38. The method of No.37, wherein said determining step comprises isolating from the cell or organism said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
39. The method of No. 35, wherein the amount of the individual protein components of said complex are determined.
40. The method of No.39, wherein said determining step comprises determining whether any of the proteins according to No. 13 is present in the complex.
41. The complex of any one of No.1-8, or proteins of No.13 or the antibody of fragment of No. 17, for use in a method of diagnosing a disease or disorder, preferentially of a disease or disorder such as inflammation, such as chronic inflammatory diseases, rheumatoid arthritis, inflammatory bowel disease; infectious diseases such as septic shock and bacterial infections; neurological diseases such as stroke-induced inflammation in neurons; neurodegenerative diseases; cancer such as prostate cancer, breast cancer and skin cancer.
42. A method for treating or preventing a disease or disorder characterized by an aberrant amount of, activity of, component composition of or intracellular localization of, the complex of any one of No.1-8, comprising administering to a subject in need of such treatment or prevention a therapeutically effective amount of one or more molecules that modulate the amount of, activity or, or protein components of, said complex.
43. The method according to No.42, wherein said disease or disorder involves decreased levels of the amount or activity of said complex.
44. The method according to No.42, wherein said disease or disorder involves increased levels of the amount or activity of said complex.
45. Complex of No.1-8 and/or any of the proteins listed in table 1, eighth column of said complex as a target for an active agent of a pharmaceutical, preferably a drug target in the treatment or prevention of a disease or disorder, preferentially of a disease or disorder such as inflammation, such as chronic inflammatory diseases, rheumatoid arthritis, inflammatory bowel disease; infectious diseases such as septic shock and bacterial infections; neurological diseases such as stroke-induced inflammation in neurons; neurodegenerative diseases; cancer such as prostate cancer, breast cancer and skin cancer.

### 3.1 DEFINITIONS

The term "activity" as used herein, refers to the function of a molecule in its broadest sense. It generally includes, but is not limited to, biological, biochemical, physical or chemical functions of the molecule. It includes for example the enzymatic activity, the ability to interact with other molecules and ability to activate, facilitate, inhibit, suppress or destabilize the function of other molecules, stability, ability to localize to certain subcellular locations. Where applicable, said term also relate to the function of a protein complex in its broadest sense.

The term "agonist" as used herein, means a molecule which modulates the formation of a protein complex or which, when bound to a complex or protein of the inventionor a molecule in the protein complex, increases the amount of, or prolongs the duration of, the activity of the complex. The stimulation may be direct or indirect, including effects on the expression of a gene encoding a member of the protein complex, or by a competitive or non-competitive mechanism. Agonists may include proteins, nucleic acids, carbohydrates or any other organic or anorganic molecule or metals. Agonists also include a functional peptide or peptide fragment derived from a protein member of the complexes of the invention or a protein member itself of the complexes of the invention. Preferred activators are those which,, when added to the complex and/or the protein of the invention under physiological conditions and/or in vitro assays, including diagnostic or prognostic assays, result in a change of the level of any of the activities of the protein complex and/or the proteins of the invention as exemplary illustrated above by at least 10%, at least 25%, at least 50%, at least 100%, at least, 200%, at least 500% or at least 1000% at a concentration of the activator 1µg ml⁻¹, 10µg ml⁻¹, 100µg ml⁻¹, 500µg ml⁻¹, 1mg ml⁻¹, 10mg ml⁻¹ or 100mg ml⁻¹. Any combination of the above mentioned degrees of percentages and concentration may be used to define anagonist of the invention, with greater effect at lower concentrations being preferred.

The term "amount" as used herein and as applicable to the embodiment described relates to the amount of the particular protein or protein complex described, including the value of null, i.e. where no protein or protein complex described in that particular embodiment is present under the or any of the conditions which might be specified in that particular embodiment.

The term "animal" as used herein includes, but is not limited to mammals, preferably mammals such as cows, pigs, horses, mice, rats, cats, dogs, sheeps, goats and most preferably humans. Other animals used in agriculture, such as chickens, ducks etc are also included in the definition as used herein.
The term "animal" as used herein does not include humans if being used in the context of genetic alterations to the germline

The term "antagonist" as used herein, means a molecule which modulates the formation of a protein complex or which, when bound to a complex or protein of the inventionor a molecule in the protein complex, decreases the amount of, or the duration or level of activity of the complex. The effect may be direct or indirect, including effects on the expression of a gene encoding a member of the protein complex, or by a competitive or non-competitive mechanism. Antagonists may include proteins, including antibodies, nucleic acids, carbohydrates or any other organic or anorganic molecule or metals. Antagonists also include a functional peptide or peptide fragment derived from a protein member of the complexes of the invention or a protein member itself of the complexes of the invention. Preferred antagonists are those which, when added to the complex and/or the protein of the invention under physiological conditions and/or in vitro assays, including diagnostic or prognostic assays, result in a change of the level of any of the activities of the protein complex and/or the proteins of the invention as exemplary illustrated above by at least 10%, at least 20%, at least 30%, at least 40% at least 50%, at least 60%, at least 70%, at least 80%, at least 90%, at least 95% or at least 99% at a concentration of the inhibitor of 1µg ml⁻¹, 10µg ml⁻¹, 100µg ml⁻¹, 500µg ml⁻¹, 1mg ml⁻¹, 10mg ml⁻¹ or 100mg ml⁻¹.
Any combination of the above mentioned degrees of percentages and concentration may be used to define antaagonist of the invention, with greater effect at lower concentrations being preferred.

The term "antibodies" as used herein, include include, but are not limited to, polyclonal, monoclonal, chimeric, single chain, Fab fragments, and an Fab expression library.

The term "binding" as used herein means a stable or transient association between two molecules, including electrostatic, hydrophobic, ionic and/or hydrogen-bond interaction under physiological conditions and/or conditions being used in diagnostic or prognostic method or process or procedure.

The term "carrier" as used herein refers to a diluent, adjuvant, excipient, or vehicle with which the therapeutic is administered. Such pharmaceutical carriers can be sterile liquids, such as water and oils, including those of petroleum, animal, vegetable or synthetic origin, including but not limited to peanut oil, soybean oil, mineral oil, sesame oil and the like. Water is a preferred carrier when the pharmaceutical composition is administered orally. Saline and aqueous dextrose are preferred carriers when the pharmaceutical composition is administered intravenously. Saline solutions and aqueous dextrose and glycerol solutions are preferably employed as liquid carriers for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsions, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the Therapeutic, preferably in purified form, together with a suitable amount of carrier so as to provide the form for proper administration to the patient. The formulation should suit the mode of administration.

If not stated otherwise, the terms "complex" and "protein complex" are used interchangeably herein and refer to a complex of proteins that is able to perform one or more functions of the wild type protein complex. The protein complex may or may not include and/or be associated with other molecules such as nucleic acid, such as RNA or DNA, or lipids or further cofactors or moieties selected from a metal ions,hormones, second messengers, phosphate, sugars.
A "complex" of the invention may also be part of or a unit of a larger physiological protein assembly.

The term "component of the TNF-α-signalling pathway" as used herein refers to a protein and/or protein complex which is involved in mediating TNFα signalling in a cell. Components of the TNF-α-signalling pathway include the following protein complexes as provided herein and components thereof:

ABIN-2 protein complex, TNRF1 protein complex, TNFR2 protein complex, RelA (p65) protein complex, p50 (NF-κB1) protein complex, IKK-α protein complex, IKK-γ protein complex, p38 protein complex, p105 (NF-κB1) protein complex, IκBα protein complex, IκBβ protein complex, IκBε protein complex, TRAF6 protein complex, I-TRAF protein complex, MEKK3 protein complex, TAK1 protein complex, MEKK1 protein complex, RIP protein complex, RIP2 protein complex, RIP3 protein complex, TRADD protein complex, FADD protein complex, c-IAP1 protein complex.

If not stated otherwise, the term "compound" as used herein are include but are not limited to peptides, nucleic acids, carbohydrates, natural product extract librariesorganic molecules, preferentially small organic molecules, anorganic molecules, including but not limited to chemicals, metals and organometallic molecules

The terms "derivatives" or "analogs of component proteins or "variants" as used herein include, but are not limited, to molecules comprising regions that are substantially homologous to the component proteins, in various embodiments, by at least 30%, 40%, 50%, 60%, 70%, 80%, 90%, 95% or 99% identity over an amino acid sequence of identical size or when compared to an aligned sequence in which the alignment is done by a computer homology program known in the art, or whose encoding nucleic acid is capable of hybridizing to a sequence encoding the component protein under stringent, moderately stringent, or nonstringent conditions. It means a protein which is the outcome of a modification of the naturally occurring protein, by amino acid substitutions, deletions and additios, respectively, which derivatives still exhibit the biological function of the naturally occurring protein although not necessarily to the same degree. The biological function of such proteins can e.g. be examined by suitable available in vitro assays as provided in the invention..

The term "functionally active" as used herein refers to a polypeptide, namely a fragment or derivative, having structural, regulatory, or biochemical functions of the protein according to the embodiment of which this polypeptide, namely fragment or derivative is related to.

The term "fragment" as used herein refers to a polypeptide of at least 10, 20, 30, 40 or 50 amino acids of the component protein according to the embodiment. IN specific embodiments, such fragments are not larger than 35, 100 or 200 amino acids.

The term "gene" as used herein refers to a nucleic acid comprising an open reading frame encoding a polypeptide of, if not stated otherwise, the present invention, including both exon and optionally intron sequences.

The term " homolog" or "homologous gene products" as used herein means a protein in another species, preferably mammals, which performs the same biological function as the a protein component of the complex further described herein. Such homologs are also termed "orthologue gene products". The algorithm for the detection of orthologue gene pairs from humanst and mammalians or other species uses the whole genome of these organisms. First, pairwise best hits are retrieved, using a full Smith-Waterman alignment of predicted proteins. To further improve reliability, these pairs are clustered with pairwise best hits involving Drosophila melanogaster and C. elegans proteins. Such analysis is given, e.g., in Nature, 2001, 409:860-921. The homologs of the proteins according to the invention can either be isolated based on the sequence homology of the genes encoding the proteins provided herein to the genes of other species by cloning the respective gene applying conventional technology and expressing the protein from such gene, or by isolating proteins of the other species by isolating the analogous complex according to the methods provided herein or to other suitable methods commonly known in the art.

The term "host cells" or, were applicable, "cells" or "hosts" as used herein is intended to be understood in a broadest sense and include, but are not limited to mammalian cell systems infected with virus (e.g., vaccinia virus, adenovirus, etc.); insect cell systems infected with virus (e.g., baculovirus); microorganisms such as yeast containing yeast vectors; or bacteria transformed with bacteriophage, DNA, plasmid DNA, or cosmid DNA. The expression elements of vectors vary in their strengths and specificities. Depending on the host-vector system utilized, any one of a number of suitable transcription and translation elements may be used.
It is understood that this term not only refers to the particular subject cell but to the progeny or potetial progeny of such a cell. Because certain modifications may occur in succeeding generations due to either mutation of environmental inlcuences, such progeny may not, in fact, be identical to the parent cell, but are still included within the scope of the term as used herein.

The term "nuleic acid" as used herein refers to polynucleotides such as deoxyribonucleic acid (DNA), and, where appropriate, ribonucleic acid (RNA). They may also be polynucleotides which include within them synthetic or modified nucleotides. A number of different types of modification to polynucleotides are known in the art. These include methylphosphonate and phosphorothioate backbones, addition of acridine or polylysine chains at the 3' and/or 5' ends of the molecule. For the purposes of the present invention, it is to be understood that the polynucleotides described herein may be modified by any method available in the art. Such modifications may be carried out in order to enhance the in vivo activity or lifespan of polynucleotides of the invention. Polynucleotides according to the invention may be produced recombinantly, synthetically, or by any means available to those of skill in the art. They may also be cloned by standard techniques. The polynucleotides are typically provided in isolated and/or purified form. As applicable to the embodiment being described, they include both single stranded and double-stranded polynucleotides.

The term "percent identity", as used herein, means the number of identical residues as defined by an optimal alignment using the Smith-Waterman algorithm divided by the length of the overlap multiplied by 100. The alignment is performed by the search program (W.R. Pearson, 1991, Genomics 11:635-650) with the constraint to align the maximum of both sequences.

The terms "polypeptides" and "proteins" are, where applicable, used interchangeably herein. They may be chemically modified, e.g. post-translationally modified. For example, they may be glycosylated or comprise modified amino acid residues. They may also be modified by the addition of a signal sequence to promote their secretion from a cell where the polypeptide does not naturally contain such a sequence. They maybe tagged with a tag. They may be tagged with different labels which may assists in identification of the proteins in a protein complex. Polypeptides/proteins for use in the invention may be in a substantially isolated form. It will be understood that the polypeptid/protine may be mixed with carriers or diluents which will not interfere with the intended purpose of the polypeptide and still be regarded as substantially isolated. A polypeptide/protein for use in the invention may also be in a substantially purified form, in which case it will generally comprise the polypeptide in a preparation in which more than 50%, e.g. more than 80%, 90%, 95% or 99%, by weight of the polypeptide in the preparation is a polypeptide of the invention.

"Target for therapeutic drug" means that the respective protein (target) can bind the active ingredient of a pharmaceutical composition and thereby changes its biological activity in response to the drug binding.

The term "tag" as used herein is meant to be understood in its broadest sense and to include, but is not limited to any suitable enzymatic, fluorescent, or radioactive labels and suitable epitopes, incuding but not limited to HA-tag, Myc-tag, T7, His-tag, FLAG-tag, Calmodulin binding proteins, glutatione-S-transferase, strep-tag, KT3-epitope, EEF-epitpopes, green-fluorescent protein and variants thereof.

The term "Therapeutics" as used herein, includes, but are not limited to, a protein complex of the present invention, the individual component proteins, and analogs and derivatives (including fragments); antibodies thereto; nucleic acids encoding the component protein, and analogs or derivatives, thereof); component protein antisense nucleic acids, and agents that modulate complex formation and/or activity (i.e., agonists and antagonists).

The term "vector" as used herein means a nucleic acid molecule capable of transporting another nucleic acid sequence to which it has been linked. Preferred vectors are those capable of autonomous replication and/or expression of nueclic acids to which they linked. The terms "plasmid" and "vector" are used interchangeably herein when applicable to the embodiment. However, vectors other than plasmids are also included herein. The expression elements of vectors vary in their strengths and specificities. Depending on the host-vector system utilized, any one of a number of suitable transcription and translation elements may be used.

### 4. DETAILED DESCRIPTION OF THE INVENTION

### Overview:

An object of the present invention was to identify protein complexes of the Tumor necrosis factor-α-(TNF-α) -signalling pathway, component proteins of the said complexes, fragments and derivatives of the component proteins, and antibodies specific to the complexes. The present invention also relates to methods for use of the complexes/components of the TNF-α-signalling pathway and their interaction in, inter alia, screening, diagnosis, and therapy, as well as to methods of preparing the complexes.

By applying the process according to the invention said complexes were identified. The components are listed in table 1.
Furthermore, the interactions between the protein complexes of the TNF-α-signalling pathway and the differential assembly of the complexes in different cellular conditions provide new insights into the mechanisms of the physiological and pathological processes associated with this biological pathway.
Said object is further achieved by the characterisation of component proteins. These proteins are listed in table 2

The invention thus relates to the following embodiments:
1. A protein complex selected from complex (I) and comprising
   (a) at least one first protein, which first protein is selected from the group of proteins in table 1, seventh column of a given complex, or a functionally active derivative thereof, or a functionally active fragment thereof, or a homolog thereof, or a variant of said protein encoded by a nucleic acid that hybridizes to the nucleic acid encoding said protein under low stringency conditions; and
   (b) at least one second protein, which second protein is selected from the group of proteins in table 1, eighth column of said complex, or a functionally active derivative thereof, or a functionally active fragment thereof, or a homolog thereof, or a variant of said second protein encoded by a nucleic acid that hybridizes to the nucleic acid encoding said protein under low stringency conditions;
   and a complex (II) comprising at least two of said second proteins,
   wherein said low stringency conditions comprise hybridization in a buffer comprising 35% formamide, 5X SSC, 50mM Tris-HCl (pH 7.5), 5 mM EDTA, 0.02% PVP, 0.02% BSA, 100 microg/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1-5 hours at 55°C, and washing in a buffer consisting of 2X SSC, 26 mM Tris-HCl (pH 7.4) 5 mM EDTA, and 0.1 % SDS for 1.5 hours at 60°C.
2. A protein complex comprising a first protein selected from the proteins listed in table 1, second column of a given complex or a homolog or variant thereof, or a functionally active fragment or functionally active derivative of said first protein, the variant being encoded by a nucleic acid that hybridizes to the nucleic acid of said first protein under low stringency conditions, and at least one second protein selected from the group of proteins in table 1, eighth column of a given complex, or a variant or homolog thereof, or a functionally active fragment or a functionally active derivative of said second protein, the variant of said second protein being encoded by a nucleic acid that hybridizes to the nucleic acid of said second protein under low-stringency conditions, and wherein said low stringency conditions comprise hybridization in a buffer comprising 35% formamide, 5X SSC, 50mM Tris-HCl (pH 7.5), 5 mM EDTA, 0.02% PVP, 0.02% BSA, 100 microg/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1-5 hours at 55°C, and washing in a buffer consisting of 2X SSC, 26 mM Tris-HCl (pH 7.4) 5 mM EDTA, and 0.1% SDS for 1.5 hours at 60°C.
3. A protein complex selected from complex (I) and comprising the proteins selected from the proteins in table 1, third column or a homolog thereof, or a variant thereof or functionally active fragments or functionally active derivatives of said proteins, said variant being encoded by a nucleic acid that hybridizes to the nucleic acid of said protein under low stringency conditions;
   and a protein complex selected from complex (II) and comprising the proteins selected from the proteins in table 1, fourth column or a homolog thereof, or a variant thereof or functionally active fragments or functionally active derivatives of said proteins, the variant being encoded by a nucleic acid that hybridizes to the nucleic acid of said protein under low stringency conditions, to the nucleic acid of said protein under low stringency conditions;
   and a protein complex selected from complex (III) and comprising the proteins selected from the proteins in table 1, fifth column or a homolog thereof, or a variant thereof or functionally active fragments or functionally active derivatives of said proteins, the variant being encoded by a nucleic acid that hybridizes to the nucleic acid of said protein under low stringency conditions;
   and a protein complex selected from complex (IV) and comprising the proteins selected from the proteins in table 1, sixth column or a homolog thereof, or a variant thereof or functionally active fragments or functionally active derivatives of said proteins, the variant being encoded by a nucleic acid that hybridizes to the nucleic acid of said protein under low stringency conditions;
   wherein said low stringency conditions comprise hybridization in a buffer comprising 35% formamide, 5X SSC, 50mM Tris-HCl (pH 7.5), 5mM EDTA, 0.02% PVP, 0.02% Ficoll, 0.2% BSA, 100 microg/ml denatured salmon sperm DNA, and 10% (wt/vol) dextran sulfate for 18-20 hours at 40°C, washing in a buffer consisting of 2X SSC, 25mM Tris-HCl (pH 7.4), 5mM EDTA, and 0.1% SDS for 1-5 hours at 55°C, and washing in a buffer consisting of 2X SSC, 25mM Tris-HCl (pH 7.4), 5mM EDTA, and 0.1% SDS for 1.5 hours at 60°C.
4. A protein complex that comprises all proteins as listed in table 1, sixth column for a given complex or a homolog or a variant thereof, or a functionally active fragment or a functionally active derivative thereof, the variant being encoded by a nucleic acid that hybridizises to the nucleic acid of any of said proteins under low stringency conditions, but 1 to the number of proteins listed in table 1, eighth column of said complex, or a homolog or a variant thereof, or a functionally active fragment or functionally active derivative thereof, the variant being encoded by a nucleic acid that hybridizes to the nucleic acid of any of said proteins of said eigth column under low stringency conditions, wherein said low stringency conditions comprise hybridization in a buffer comprising 35% formamide, 5X SSC, 50mM Tris-HCl (pH 7.5), 5mM EDTA, 0.02% PVP, 0.02% Ficoll, 0.2% BSA, 100 microg/ml denatured salmon sperm DNA, and 10% (wt/vol) dextran sulfate for 18-20 hours at 40°C, washing in a buffer consisting of 2X SSC, 25mM Tris-HCl (pH 7.4), 5mM EDTA, and 0.1% SDS for 1-5 hours at 55°C, and washing in a buffer consisting of 2X SSC, 25mM Tris-HCl (pH 7.4), 5mM EDTA, and 0.1% SDS for 1.5 hours at 60°C..
5. The complex of any of No.1 to 4 comprising at least one functionally active derivative of said first protein and/or a functionally active derivative of said second protein, wherein the functionally active derivative is a fusion protein comprising said first protein or said second protein fused to an amino acid sequence different from the first protein or second protein.
6. The complex of No.5 wherein the functionally active derivative is a fusion protein comprising said first protein or said second protein fused to an affinity tag or label.
7. The complex of any of No.1 to 4 comprising a fragment of said first protein and/or a fragment of said second protein, which fragment binds to another protein component of said complex.
8. The complex of any of No.1 to 7 that is involved in the biochemical activity as stated in table 3.
9. A process for preparing a complex of any of No.1-8 and optionally the components thereof comprising the following steps:
   Expressing a protein of the complex, preferably a tagged protein, in a target cell, isolating the protein complex which is attached to the protein, preferably a tagged protein, and optionally disassociating the protein complex and isolating the individual complex members.
10. The process according to No.9 wherein the tagged protein comprises two different tags which allow two separate affinity purification steps.
11. The process according to any of No. 9-10 wherein the two tags are separated by a cleavage site for a protease.
12. Component of a protein complex obtainable by a process according to any of No.9-11.
13. Protein selected from the group of proteins in table 1, ninth column of a given complex a homolog thereof, and a variant of thereof, or a functionally active fragment or a functionally active derivative of said protein, the variant being encoded by a nucleic acid that hybridizes to the nucleic acid encoding said protein under low stringency conditions, wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 microg/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
14. Nucleic acid encoding a protein according to No.13.
15. Construct, preferably a vector construct, comprising
   (a) a nucleic acid according to No.14 and at least one further nucleic acid which is normally not associated with said nucleic acid, or
   (b) at least two separate nucleic acid sequences each encoding a different protein, or a functionally active fragment or a functionally active derivative thereof, or a homolog or a variant thereof, at least one of said proteins being selected from the first group of proteins according to No.1 (a) and at least one of said proteins, being selected from the second group of proteins according to No.1(b).
16. Host cell, containing a vector comprising at least one of the nucleic acid of No. 14 and /or a construct of No. 15 or containing several vectors each comprising at least the nucleic acid encoding at least one protein selected from the first group of proteins according to No.1 (a) and the nucleic acid encoding at least one protein selected from the second group of proteins according to No.1(b).
17. An antibody or a fragment of said antibody containing the binding domain thereof, which binds the complex of any of No.1 to 8 and which does not bind any of the proteins of said complex when uncomplexed and/or an antibody of a fragment of said antibody containing the binding domain thereof which binds to any of the group of proteins according to No.13.
18. A kit comprising in one or more containers the complex of any of No.1-8 and/or the proteins of No. 13, optionally together with an antibody according to No.17 and/or further components such as reagents and working instructions.
19. The kit according to No.18 for processing a substrate of a complex of any one of No.1-8.
20. The kit according to No.18 for the diagnosis or prognosis of a disease or a disease risk, preferentially for a disease or disorder such as inflammation, such as chronic inflammatory diseases, rheumatoid arthritis, inflammatory bowel disease; infectious diseases such as septic shock and bacterial infections; neurological diseases such as stroke-induced inflammation in neurons; neurodegenerative diseases; cancer such as prostate cancer, breast cancer and skin cancer.
21. Array in which at least a complex according to any of No.1 to 8 and/or at least one protein according to No.13 and/or at least one antibody according to No. 17 is attached to a solid carrier.
22. A process for processing a substrate of a complex of any one of No.1-8 comprising the step of bringing into contact a complex to any of No.1-8 with said substrate, such that said substrate is processed.
23. A pharmaceutical composition comprising the protein complex of any of No.1-8 and/or any of the proteins according to No. 13.
24. A pharmaceutical composition according to No.23 for the treatment of diseases and disorders, preferentially for diseases or disorders such as inflammation, such as chronic inflammatory diseases, rheumatoid arthritis, inflammatory bowel disease; infectious diseases such as septic shock and bacterial infections; neurological diseases such as stroke-induced inflammation in neurons; neurodegenerative diseases; cancer such as prostate cancer, breast cancer and skin cancer.
25. A method for screening for a molecule that binds to the complex of any one of No.1-8 and/or any of the proteins of No.13 , comprising the following steps:
   (a) exposing said complex or protein, or a cell or organism containing said complex or said protein, to one or more candidate molecules; and
   (b) determining whether said candidate molecule is bound to the complex or protein.
26. A method for screening for a molecule that modulates directly or indirectly the function, activity, composition or formation of the complex of any one of No.1-8 comprising the steps of:
   (a) exposing said complex, or a cell or organism containing said complex to one or more candidate molecules; and
   (b) determining the amount of, activity of, protein components of, and/or intracellular localization of, said complex and/or the transcription level of a gene dependent on the complex and/or the abundance and/or activity of a protein or protein complex dependent upon the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependent on the complex and/or the abundance and/or activity of a protein or protein complex dependent on the function of the complex and/or product of a gene dependent on the complex in the absence of said candidate molecules indicates that the molecule modulates function, activity, or composition of said complex.
27. The method of No.26, wherein the amount of said complex is determined.
28. The method of No.26, wherein the activity of said complex is determined.
29. The method of No.28, wherein said determining step comprises isolating from the cell or organism said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
30. The method of No.26, wherein the amount of the individual protein components of said complex are determined.
31. The method of No.30, wherein said determining step comprises determining whether any of the proteins listed in table 1, sixth column of said complex, or a functionally active fragment or a functionally active derivative thereof, or a variant or a homolog thereof, the variant being encoded by a nucleic acid that hybridizes to the nucleic acid of said protein under low-stringency conditions, is present in the complex.
32.The method of any of No.26 to 31, wherein said method is a method of screening for a drug for treatment or prevention of a disease or disorder, preferentially of a disease or disorder selected from the diseases or disorders such as inflammation, such as chronic inflammatory diseases, rheumatoid arthritis, inflammatory bowel disease; infectious diseases such as septic shock and bacterial infections; neurological diseases such as stroke-induced inflammation in neurons; neurodegenerative diseases; cancer such as prostate cancer, breast cancer and skin cancer.
33. Use of a molecule that modulates the amount of, activity of, or the protein components of the complex of any one of No.1-8 for the manufacture of a medicament for the treatment or prevention of a disease or disorder, preferentially of a disease or disorder such as inflammation, such as chronic inflammatory diseases, rheumatoid arthritis, inflammatory bowel disease; infectious diseases such as septic shock and bacterial infections; neurological diseases such as stroke-induced inflammation in neurons; neurodegenerative diseases; cancer such as prostate cancer, breast cancer and skin cancer.
34. A method for the production of a pharmaceutical composition comprising carrying out the method of No.26-31 to identify a molecule that modulates the function, activity, composition or formation of said complex, and further comprising mixing the identified molecule with a pharmaceutically acceptable carrier.
35. A method for diagnosing or screening for the presence of a disease or disorder or a predisposition for developing a disease or disorder in a subject, which disease or disorder is characterized by an aberrant amount of, component disposition of, or intracellular localization of the complex of any one of the No.1-8, comprising determining the amount of, activity of, protein components of, and/or intracellular localization of, said complex and/or the transcription level of a gene dependent on the complex and/or the abundance and/or activity of a protein or protein complex dependent on the function of the complex and/or product of a gene dependent on the complex in a comparative sample derived from a subject, wherein a difference in said amount, activity, or protein components of, said complex in an analogous sample from a subject not having the disease or disorder or predisposition indicated the presence in the subject of the disease or disorder or predisposition in the subject.
36. The method of No.35, wherein the amount of said complex is determined.
37. The method of No.35, wherein the activity of said complex is determined.
38. The method of No.37, wherein said determining step comprises isolating from the cell or organism said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
39. The method of No.35, wherein the amount of the individual protein components of said complex are determined.
40. The method of No.39, wherein said determining step comprises determining whether any of the proteins according to No. 13 is present in the complex.
41. The complex of any one of No.1-8, or proteins of No. 13 or the antibody of fragment of No.17, for use in a method of diagnosing a disease or disorder, preferentially of a disease or disorder such as inflammation, such as chronic inflammatory diseases, rheumatoid arthritis, inflammatory bowel disease; infectious diseases such as septic shock and bacterial infections; neurological diseases such as stroke-induced inflammation in neurons; neurodegenerative diseases; cancer such as prostate cancer, breast cancer and skin cancer.
42. A method for treating or preventing a disease or disorder characterized by an aberrant amount of, activity of, component composition of or intracellular localization of, the complex of any one of No.1-8, comprising administering to a subject in need of such treatment or prevention a therapeutically effective amount of one or more molecules that modulate the amount of, activity or, or protein components of, said complex.
43. The method according to No.42, wherein said disease or disorder involves decreased levels of the amount or activity of said complex.
44. The method according to No.42, wherein said disease or disorder involves increased levels of the amount or activity of said complex.
45. Complex of No.1-8 and/or any of the proteins listed in table 1, eighth column of said complex as a target for an active agent of a pharmaceutical, preferably a drug target in the treatment or prevention of a disease or disorder, preferentially of a disease or disorder such as inflammation, such as chronic inflammatory diseases, rheumatoid arthritis, inflammatory bowel disease; infectious diseases such as septic shock and bacterial infections; neurological diseases such as stroke-induced inflammation in neurons; neurodegenerative diseases; cancer such as prostate cancer, breast cancer and skin cancer.

### Animal models are also provided herein

Preferably, the protein components of the complexes described herein are all mammalian proteins. The complexes can also consist only of the respective homologs from other mammals such as mouse, rat, pig, cow, dog, monkey, sheep or horse or other species such as Drosophila, C.elegans or chicken. In another preferred embodiment, the complexes are a mixture of proteins from two or more species.

### TABLES:

Table 1: Composition of Complexes
   First column ('Name of complex'): Lists the name of the protein complex as used herein. Second column ('Entry point'): Lists the bait proteins that have been chosen for the isolation of the given complex.
   Third column ('Treatment independent complex'): Lists the interactors of the given complex which have been identified as interactors of the complex both in treated and untreated cells (see section 5, particulary section 5.4).
   Fourth column ('Complex of TNF-α-treated cells'): Lists the interactors of the given complex which have been identified as interactors of the complex in treated cells (see section 5, particularly section 5.4).
   Fifth column ('Complex of untreated cells): Lists the interactors of the given complex which have been identified as interactors of the complex in untreated cells (see section 5, particularly section 5.4).
   Sixth column ('All interactors'): Lists all novel interactors which have been identified as members of the complex and all interactors which have been known to be associated with the bait so far.
   Seventh column ('Known interactors'): Lists all interactors which have been known to be associated with the bait so far.
   Eigth column ('Novel interactors of the complex'): Lists all novel interactors of the complex which have been identified in the experiments provided herein.
   Ninth column: Separately lists the members of the newly identified complex which previously have not been annotated.
Table 2: Individual Proteins of the Complexes
   First column ('Protein'): Lists in alphabetical order all proteins which have been identified as interactors of the complexes presented herein.
   Second column ('SEQ ID'): Lists the SEQ ID (Sequence Identifications) of the proteins herein as used herein.
   Third column ('IPI-Numbers'): Lists the IPI-Numbers of the proteins herein. The IPI-Numbers refer to the International Protein Index created by the European Bioinformatics Institute (EMBL-EBI), Hinxton, UK. For KIAA1040 also the NCBI-number is listed.
   Fourth column ('Molecular Weight'): Lists the Molecular Weight of the proteins in Dalton.
Table 3: Biochemical Activities of the Complexes of the invention.
   First column ('Name of complex'): Lists the name of any the protein complex as used herein.
   Second column ('Biochemical Activity'): Lists biochemical activities of the complexes. Assays in order to test these activities are also provided herein (infra).

### 4.1. PROTEIN COMPLEXES/PROTEINS OF THE INVENTION

The protein complexes of the present invention and their component proteins are described in the Tables 1-3. The protein complexes and component proteins can be obtained by methods well known in the art for protein purification and recombinant protein expression. For example, the protein complexes of the present invention can be isolated using the TAP method described in Section 5, infra, and in WO 00/09716 and Rigaut et al., 1999, Nature Biotechnology 17:1030-1032, which are each incorporated by reference in their entirety. Additionally, the protein complexes can be isolated by immunoprecipitation of the component proteins and combining the immunoprecipitated proteins. The protein complexes can also be produced by recombinantly expressing the component proteins and combining the expressed proteins.

The nucleic and amino acid sequences of the component proteins of the protein complexes of the present invention are provided herein (SEQ ID NO:1-268), and can be obtained by any method known in the art, e.g., by PCR amplification using synthetic primers hybridizable to the 3' and 5' ends of each sequence, and/or by cloning from a cDNA or genomic library using an oligonucleotide specific for each nucleotide sequence.

Homologs (e.g., nucleic acids encoding component proteins from other species) or other related sequences (e.g., variants, paralogs) which are members of a native cellular protein complex can be obtained by low, moderate or high stringency hybridization with all or a portion of the particular nucleic acid sequence as a probe, using methods well known in the art for nucleic acid hybridization and cloning.

Exemplary moderately stringent hybridization conditions are as follows: prehybridization of filters containing DNA is carried out for 8 hours to overnight at 65□C in buffer composed of 6X SSC, 50 mM Tris-HCl (pH 7.5), 1 mM EDTA, 0.02% PVP, 0.02% Ficoll, 0.02% BSA, and 500 µg/ml denatured salmon sperm DNA. Filters are hybridized for 48 hours at 65 °C in prehybridization mixture containing 100 µg/ml denatured salmon sperm DNA and 5-20 X 10⁶ cpm of ³²P-labeled probe. Washing of filters is done at 37 °C for 1 hour in a solution containing 2X SSC, 0.01% PVP, 0.01% Ficoll, and 0.01% BSA. This is followed by a wash in 0.1X SSC at 50 °C for 45 min before autoradiography. Alternatively, exemplary conditions of high stringency are as follows: e.g., hybridization to filter-bound DNA in 0.5 M NaHPO₄, 7% sodium dodecyl sulfate (SDS), 1 mM EDTA at 65 °C, and washing in 0.1xSSC/0.1% SDS at 68 °C (Ausubel F.M. et al., eds., 1989, Current Protocols in Molecular Biology, Vol. I, Green Publishing Associates, Inc., and John Wiley & sons, Inc., New York, at p. 2.10.3). Other conditions of high stringency which may be used are well known in the art. Exemplary low stringency hybridization conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 µg/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1.5 hours at 55°C, and washing in a buffer consisting of 2X SSC, 25 mM Tris-HCl (pH 7.4), 5 mM EDTA, and 0.1% SDS for 1.5 hours at 60°C.

For recombinant expression of one or more of the proteins, the nucleic acid containing all or a portion of the nucleotide sequence encoding the protein can be inserted into an appropriate expression vector, i.e., a vector that contains the necessary elements for the transcription and translation of the inserted protein coding sequence. The necessary transcriptional and translational signals can also be supplied by the native promoter of the component protein gene, and/or flanking regions.

A variety of host-vector systems may be utilized to express the protein coding sequence. These include but are not limited to mammalian cell systems infected with virus (e.g., vaccinia virus, adenovirus, etc.); insect cell systems infected with virus (e.g., baculovirus); microorganisms such as yeast containing yeast vectors; or bacteria transformed with bacteriophage, DNA, plasmid DNA, or cosmid DNA. The expression elements of vectors vary in their strengths and specificities. Depending on the host-vector system utilized, any one of a number of suitable transcription and translation elements may be used.

In a preferred embodiment, a complex of the present invention is obtained by expressing the entire coding sequences of the component proteins in the same cell, either under the control of the same promoter or separate promoters. In yet another embodiment, a derivative, fragment or homolog of a component protein is recombinantly expressed. Preferably the derivative, fragment or homolog of the protein forms a complex with the other components of the complex, and more preferably forms a complex that binds to an anti-complex antibody. Such an antibody is further described infra.

Any method available in the art can be used for the insertion of DNA fragments into a vector to construct expression vectors containing a chimeric gene consisting of appropriate transcriptional/translational control signals and protein coding sequences. These methods may include in vitro recombinant DNA and synthetic techniques and in vivo recombinant techniques (genetic recombination). Expression of nucleic acid sequences encoding a component protein, or a derivative, fragment or homolog thereof, may be regulated by a second nucleic acid sequence so that the gene or fragment thereof is expressed in a host transformed with the recombinant DNA molecule(s). For example, expression of the proteins may be controlled by any promoter/enhancer known in the art. In a specific embodiment, the promoter is not native to the gene for the component protein. Promoters that may be used can be selected from among the many known in the art, and are chosen so as to be operative in the selected host cell.

In a specific embodiment, a vector is used that comprises a promoter operably linked to nucleic acid sequences encoding a component protein, or a fragment, derivative or homolog thereof, one or more origins of replication, and optionally, one or more selectable markers (e.g., an antibiotic resistance gene).

In another specific embodiment, an expression vector containing the coding sequence, or a portion thereof, of a component protein, either together or separately, is made by subcloning the gene sequences into the EcoRI restriction site of each of the three pGEX vectors (glutathione S-transferase expression vectors; Smith and Johnson, 1988, Gene 7:31-40). This allows for the expression of products in the correct reading frame.

Expression vectors containing the sequences of interest can be identified by three general approaches: (a) nucleic acid hybridization, (b) presence or absence of "marker" gene function, and (c) expression of the inserted sequences. In the first approach, coding sequences can be detected by nucleic acid hybridization to probes comprising sequences homologous and complementary to the inserted sequences. In the second approach, the recombinant vector/host system can be identified and selected based upon the presence or absence of certain "marker" functions (e.g., resistance to antibiotics, occlusion body formation in baculovirus, etc.) caused by insertion of the sequences of interest in the vector. For example, if a component protein gene, or portion thereof, is inserted within the marker gene sequence of the vector, recombinants containing the encoded protein or portion will be identified by the absence of the marker gene function (e.g., loss of β-galactosidase activity). In the third approach, recombinant expression vectors can be identified by assaying for the component protein expressed by the recombinant vector. Such assays can be based, for example, on the physical or functional properties of the interacting species in in vitro assay systems, e.g., formation of a complex comprising the protein or binding to an anti-complex antibody.

Once recombinant component protein molecules are identified and the complexes or individual proteins isolated, several methods known in the art can be used to propagate them. Using a suitable host system and growth conditions, recombinant expression vectors can be propagated and amplified in quantity. As previously described, the expression vectors or derivatives which can be used include, but are not limited to, human or animal viruses such as vaccinia virus or adenovirus; insect viruses such as baculovirus, yeast vectors; bacteriophage vectors such as lambda phage; and plasmid and cosmid vectors.

In addition, a host cell strain may be chosen that modulates the expression of the inserted sequences, or modifies or processes the expressed proteins in the specific fashion desired. Expression from certain promoters can be elevated in the presence of certain inducers; thus expression of the genetically-engineered component proteins may be controlled. Furthermore, different host cells have characteristic and specific mechanisms for the translational and post-translational processing and modification (e.g., glycosylation, phosphorylation, etc.) of proteins. Appropriate cell lines or host systems can be chosen to ensure that the desired modification and processing of the foreign protein is achieved. For example, expression in a bacterial system can be used to produce an unglycosylated core protein, while expression in mammalian cells ensures "native" glycosylation of a heterologous protein. Furthermore, different vector/host expression systems may effect processing reactions to different extents.

In other specific embodiments, a component protein or a fragment, homolog or derivative thereof, may be expressed as fusion or chimeric protein product comprising the protein, fragment, homolog, or derivative joined via a peptide bond to a heterologous protein sequence of a different protein. Such chimeric products can be made by ligating the appropriate nucleic acid sequences encoding the desired amino acids to each other by methods known in the art, in the proper coding frame, and expressing the chimeric products in a suitable host by methods commonly known in the art. Alternatively, such a chimeric product can be made by protein synthetic techniques, e.g., by use of a peptide synthesizer. Chimeric genes comprising a portion of a component protein fused to any heterologous protein-encoding sequences may be constructed.

In particular, protein component derivatives can be made by altering their sequences by substitutions, additions or deletions that provide for functionally equivalent molecules. Due to the degeneracy of nucleotide coding sequences, other DNA sequences that encode substantially the same amino acid sequence as a component gene or cDNA can be used in the practice of the present invention. These include but are not limited to nucleotide sequences comprising all or portions of the component protein gene that are altered by the substitution of different codons that encode a functionally equivalent amino acid residue within the sequence, thus producing a silent change. Likewise, the derivatives of the invention include, but are not limited to, those containing, as a primary amino acid sequence, all or part of the amino acid sequence of a component protein, including altered sequences in which functionally equivalent amino acid residues are substituted for residues within the sequence resulting in a silent change. For example, one or more amino acid residues within the sequence can be substituted by another amino acid of a similar polarity that acts as a functional equivalent, resulting in a silent alteration. Substitutes for an amino acid within the sequence may be selected from other members of the class to which the amino acid belongs. For example, the nonpolar (hydrophobic) amino acids include alanine, leucine, isoleucine, valine, proline, phenylalanine, tryptophan and methionine. The polar neutral amino acids include glycine, serine, threonine, cysteine, tyrosine, asparagine, and glutamine. The positively charged (basic) amino acids include arginine, lysine and histidine. The negatively charged (acidic) amino acids include aspartic acid and glutamic acid.

In a specific embodiment, up to 1%, 2%, 5%, 10%, 15% or 20% of the total number of amino acids in the wild type protein are substituted or deleted; or 1, 2, 3, 4, 5, or 6 or up to 10 or up to 20 amino acids are inserted, substituted or deleted relative to the wild type protein.

In a specific embodiment of the invention, the nucleic acids encoding a protein component and protein components consisting of or comprising a fragment of or consisting of at least 6 (continuous) amino acids of the protein are provided. In other embodiments, the fragment consists of at least 10, 20, 30, 40, or 50 amino acids of the component protein. In specific embodiments, such fragments are not larger than 35, 100 or 200 amino acids. Derivatives or analogs of component proteins include, but are not limited, to molecules comprising regions that are substantially homologous to the component proteins, in various embodiments, by at least 30%, 40%, 50%, 60%, 70%, 80%, 90%, 95% or 99% identity over an amino acid sequence of identical size or when compared to an aligned sequence in which the alignment is done by a computer homology program known in the art, or whose encoding nucleic acid is capable of hybridizing to a sequence encoding the component protein under stringent, moderately stringent, or nonstringent conditions.

In a specific embodiment, proteins are provided herein, which share an identical region of 20, 30, 40, 50 or 60 contiguos amino acids of the proteins listed in table 2

The protein component derivatives and analogs of the invention can be produced by various methods known in the art. The manipulations which result in their production can occur at the gene or protein level. For example, the cloned gene sequences can be modified by any of numerous strategies known in the art (Sambrook et al., 1989, Molecular Cloning, A Laboratory Manual, 2d Ed., Cold Spring Harbor Laboratory Press, Cold Spring Harbor, New York). The sequences can be cleaved at appropriate sites with restriction endonuclease(s), followed by further enzymatic modification if desired, isolated, and ligated in vitro. In the production of the gene encoding a derivative, homolog or analog of a component protein, care should be taken to ensure that the modified gene retains the original translational reading frame, uninterrupted by translational stop signals, in the gene region where the desired activity is encoded.

Additionally, the encoding nucleic acid sequence can be mutated in vitro or in vivo, to create and/or destroy translation, initiation, and/or termination sequences, or to create variations in coding regions and/or form new restriction endonuclease sites or destroy pre-existing ones, to facilitate further in vitro modification. Any technique for mutagenesis known in the art can be used, including but not limited to, chemical mutagenesis and in vitro site-directed mutagenesis (Hutchinson et al., 1978, J. Biol. Chem 253:6551-6558), amplification with PCR primers containing a mutation, etc.

Once a recombinant cell expressing a component protein, or fragment or derivative thereof, is identified, the individual gene product or complex can be isolated and analyzed. This is achieved by assays based on the physical and/or functional properties of the protein or complex, including, but not limited to, radioactive labeling of the product followed by analysis by gel electrophoresis, immunoassay, cross-linking to marker-labeled product, etc.

The component proteins and complexes may be isolated and purified by standard methods known in the art (either from natural sources or recombinant host cells expressing the complexes or proteins), including but not restricted to column chromatography (e.g., ion exchange, affinity, gel exclusion, reversed-phase high pressure, fast protein liquid, etc.), differential centrifugation, differential solubility, or by any other standard technique used for the purification of proteins. Functional properties may be evaluated using any suitable assay known in the art.

Alternatively, once a component protein or its derivative, is identified, the amino acid sequence of the protein can be deduced from the nucleic acid sequence of the chimeric gene from which it was encoded. As a result, the protein or its derivative can be synthesized by standard chemical methods known in the art (e.g., Hunkapiller et al., 1984, Nature 310: 105-111).

Manipulations of component protein sequences may be made at the protein level. Included within the scope of the invention is a complex in which the component proteins or derivatives and analogs that are differentially modified during or after translation, e.g., by glycosylation, acetylation, phosphorylation, amidation, derivatization by known protecting/blocking groups, proteolytic cleavage, linkage to an antibody molecule or other cellular ligand, etc. Any of numerous chemical modifications may be carried out by known techniques, including but not limited to specific chemical cleavage by cyanogen bromide, trypsin, chymotrypsin, papain, V8 protease, NaBH₄, acetylation, formylation, oxidation, reduction, metabolic synthesis in the presence of tunicamycin, etc.

In specific embodiments, the amino acid sequences are modified to include a fluorescent label. In another specific embodiment, the protein sequences are modified to have a heterofunctional reagent; such heterofunctional reagents can be used to crosslink the members of the complex.

In addition, complexes of analogs and derivatives of component proteins can be chemically synthesized. For example, a peptide corresponding to a portion of a component protein, which comprises the desired domain or mediates the desired activity in vitro (e.g., complex formation) can be synthesized by use of a peptide synthesizer. Furthermore, if desired, non-classical amino acids or chemical amino acid analogs can be introduced as a substitution or addition into the protein sequence.

In cases where natural products are suspected of being mutant or are isolated from new species, the amino acid sequence of a component protein isolated from the natural source, as well as those expressed in vitro, or from synthesized expression vectors in vivo or in vitro, can be determined from analysis of the DNA sequence, or alternatively, by direct sequencing of the isolated protein. Such analysis can be performed by manual sequencing or through use of an automated amino acid sequenator.

The complexes can also be analyzed by hydrophilicity analysis (Hopp and Woods, 1981, Proc. Natl. Acad. Sci. USA 78:3824-3828). A hydrophilicity profile can be used to identify the hydrophobic and hydrophilic regions of the proteins, and help predict their orientation in designing substrates for experimental manipulation, such as in binding experiments, antibody synthesis, etc. Secondary structural analysis can also be done to identify regions of the component proteins, or their derivatives, that assume specific structures (Chou and Fasman, 1974, Biochemistry 13:222-23). Manipulation, translation, secondary structure prediction, hydrophilicity and hydrophobicity profile predictions, open reading frame prediction and plotting, and determination of sequence homologies, etc., can be accomplished using computer software programs available in the art.

Other methods of structural analysis including but not limited to X-ray crystallography (Engstrom, 1974 Biochem. Exp. Biol. 11:7-13), mass spectroscopy and gas chromatography (Methods in Protein Science, J. Wiley and Sons, New York, 1997), and computer modeling (Fletterick and Zoller, eds., 1986, Computer Graphics and Molecular Modeling, In: Current Communications in Molecular Biology, Cold Spring Harbor Laboratory, Cold Spring Harbor Press, New York) can also be employed.

### 4.2. ANTIBODIES TO PROTEIN COMPLEXES/PROTEINS OF THE INVENTION

According to the present invention, a protein complex of the present invention comprising a first protein, or a functionally active fragment or functionally active derivative thereof, selected from the group consisting of proteins listed in third column of table 1; and a second protein, or a functionally active fragment or functionally active derivative thereof, selected from the group consisting of proteins listed in forth column of table 1, or a functionally active fragment or functionally active derivative thereof, can be used as an immunogen to generate antibodies which immunospecifically bind such immunogen. According to the present invention, also a protein complex of the present invention can be used as an immunogen to generate antibodies which immunospecifically bind to such immunogen comprising all proteins listed in fifth column of table 1

Such antibodies include, but are not limited to, polyclonal, monoclonal, chimeric, single chain, Fab fragments, and an Fab expression library. In a specific embodiment, antibodies to a complex comprising human protein components are produced. In another embodiment, a complex formed from a fragment of said first protein and a fragment of said second protein, which fragments contain the protein domain that interacts with the other member of the complex, are used as an immunogen for antibody production. In a preferred embodiment, the antibody specific for the complex in that the antibody does not bind the individual protein components of the complex.

Polyclonal antibodies can be prepared as described above by immunizing a suitable subject with a polypeptide of the invention as an immunogen. Preferred polyclonal antibody compositions are ones that have been selected for antibodies directed against a polypeptide or polypeptides of the invention. Particularly preferred polyclonal antibody preparations are ones that contain only antibodies directed against a polypeptide or polypeptides of the invention. Particularly preferred immunogen compositions are those that contain no other human proteins such as, for example, immunogen compositions made using a non-human host cell for recombinant expression of a polypeptide of the invention. In such a manner, the only human epitope or epitopes recognized by the resulting antibody compositions raised against this immunogen will be present as part of a polypeptide or polypeptides of the invention.

The antibody titer in the immunized subject can be monitored over time by standard techniques, such as with an enzyme linked immunosorbent assay (ELISA) using immobilized polypeptide. If desired, the antibody molecules can be isolated from the mammal (e.g., from the blood) and further purified by well-known techniques, such as protein A chromatography to obtain the IgG fraction. Alternatively, antibodies specific for a protein or polypeptide of the invention can be selected for (e.g., partially purified) or purified by, e.g., affinity chromatography. For example, a recombinantly expressed and purified (or partially purified) protein of the invention is produced as described herein, and covalently or non-covalently coupled to a solid support such as, for example, a chromatography column. The column can then be used to affinity purify antibodies specific for the proteins of the invention from a sample containing antibodies directed against a large number of different epitopes, thereby generating a substantially purified antibody composition, i.e., one that is substantially free of contaminating antibodies. By a substantially purified antibody composition is meant, in this context, that the antibody sample contains at most only 30% (by dry weight) of contaminating antibodies directed against epitopes other than those on the desired protein or polypeptide of the invention, and preferably at most 20%, yet more preferably at most 10%, and most preferably at most 5% (by dry weight) of the sample is contaminating antibodies. A purified antibody composition means that at least 99% of the antibodies in the composition are directed against the desired protein or polypeptide of the invention.

At an appropriate time after immunization, e.g., when the specific antibody titers are highest, antibody-producing cells can be obtained from the subject and used to prepare monoclonal antibodies by standard techniques, such as the hybridoma technique originally described by Kohler and Milstein, 1975, Nature 256:495-497, the human B cell hybridoma technique (Kozbor et al., 1983, Immunol. Today 4:72), the EBV-hybridoma technique (Cole et al., 1985, Monoclonal Antibodies and Cancer Therapy, Alan R. Liss, Inc., pp. 77-96) or trioma techniques. The technology for producing hybridomas is well known (see generally Current Protocols in Immunology (1994) Coligan et al. (eds.) John Wiley & Sons, Inc., New York, NY). Hybridoma cells producing a monoclonal antibody of the invention are detected by screening the hybridoma culture supernatants for antibodies that bind the polypeptide of interest, e.g., using a standard ELISA assay.

Alternative to preparing monoclonal antibody-secreting hybridomas, a monoclonal antibody directed against a polypeptide of the invention can be identified and isolated by screening a recombinant combinatorial immunoglobulin library (e.g., an antibody phage display library) with the polypeptide of interest. Kits for generating and screening phage display libraries are commercially available (e.g., the Pharmacia Recombinant Phage Antibody System, Catalog No. 27-9400-01; and the Stratagene SurfZAP Phage Display Kit, Catalog No. 240612). Additionally, examples of methods and reagents particularly amenable for use in generating and screening antibody display library can be found in, for example, U.S. Patent No. 5,223,409; PCT Publication No. WO 92/18619; PCT Publication No. WO 91/17271; PCT Publication No. WO 92/20791; PCT Publication No. WO 92/15679; PCT Publication No. WO 93/01288; PCT Publication No. WO 92/01047; PCT Publication No. WO 92/09690; PCT Publication No. WO 90/02809; Fuchs et al., 1991, Bio/Technology 9:1370-1372; Hay et al., 1992, Hum. Antibod. Hybridomas 3:81-85; Huse et al., 1989, Science 246:1275-1281; Griffiths et al., 1993, EMBO J. 12:725-734.

Additionally, recombinant antibodies, such as chimeric and humanized monoclonal antibodies, comprising both human and non-human portions, which can be made using standard recombinant DNA techniques, are within the scope of the invention. A chimeric antibody is a molecule in which different portions are derived from different animal species, such as those having a variable region derived from a murine mAb and a human immunoglobulin constant region. (See, e.g., Cabilly et al., U.S. Patent No. 4,816,567; and Boss et al., U.S. Patent No. 4,816,397, which are incorporated herein by reference in their entirety.) Humanized antibodies are antibody molecules from non-human species having one or more complementarily determining regions (CDRs) from the non-human species and a framework region from a human immunoglobulin molecule. (See, e.g., Queen, U.S. Patent No. 5,585,089, which is incorporated herein by reference in its entirety.) Such chimeric and humanized monoclonal antibodies can be produced by recombinant DNA techniques known in the art, for example using methods described in PCT Publication No. WO 87/02671; European Patent Application 184,187; European Patent Application 171,496; European Patent Application 173,494; PCT Publication No. WO 86/01533; U.S. Patent No. 4,816,567; European Patent Application 125,023; Better et al., 1988, Science 240:1041-1043; Liu et al., 1987, Proc. Natl. Acad. Sci. USA 84:3439-3443; Liu et al., 1987, J. Immunol. 139:3521-3526; Sun et al., 1987, Proc. Natl. Acad. Sci. USA 84:214-218; Nishimura et al., 1987, Canc. Res. 47:999-1005; Wood et al., 1985, Nature 314:446-449; and Shaw et al., 1988, J. Natl. Cancer Inst. 80:1553-1559); Morrison, 1985, Science 229:1202-1207; Oi et al., 1986, Bio/Techniques 4:214; U.S. Patent 5,225,539; Jones et al., 1986, Nature 321:552-525; Verhoeyan et al., 1988, Science 239:1534; and Beidler et al., 1988, J. Immunol. 141:4053-4060.

Completely human antibodies are particularly desirable for therapeutic treatment of human patients. Such antibodies can be produced, for example, using transgenic mice which are incapable of expressing endogenous immunoglobulin heavy and light chains genes, but which can express human heavy and light chain genes. The transgenic mice are immunized in the normal fashion with a selected antigen, e.g., all or a portion of a polypeptide of the invention. Monoclonal antibodies directed against the antigen can be obtained using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA and IgE antibodies. For an overview of this technology for producing human antibodies, see Lonberg and Huszar, 1995, Int. Rev. Immunol. 13:65-93). For a detailed discussion of this technology for producing human antibodies and human monoclonal antibodies and protocols for producing such antibodies, see, e.g., U.S. Patent 5,625,126; U.S. Patent 5,633,425; U.S. Patent 5,569,825; U.S. Patent 5,661,016; and U.S. Patent 5,545,806. In addition, companies such as Abgenix, Inc. (Freemont, CA), can be engaged to provide human antibodies directed against a selected antigen using technology similar to that described above.

Completely human antibodies which recognize a selected epitope can be generated using a technique referred to as "guided selection." In this approach a selected non-human monoclonal antibody, e.g., a murine antibody, is used to guide the selection of a completely human antibody recognizing the same epitope. (Jespers et al., 1994, Bio/technology 12:899-903).

Antibody fragments that contain the idiotypes of the complex can be generated by techniques known in the art. For example, such fragments include, but are not limited to, the F(ab')2 fragment which can be produced by pepsin digestion of the antibody molecule; the Fab' fragment that can be generated by reducing the disulfide bridges of the F(ab')2 fragment; the Fab fragment that can be generated by treating the antibody molecular with papain and a reducing agent; and Fv fragments.

In the production of antibodies, screening for the desired antibody can be accomplished by techniques known in the art, e.g., ELISA (enzyme-linked immunosorbent assay). To select antibodies specific to a particular domain of the complex, or a derivative thereof, one may assay generated hybridomas for a product that binds to the fragment of the complex, or a derivative thereof, that contains such a domain. For selection of an antibody that specifically binds a complex of the present, or a derivative, or homolog thereof, but which does not specifically bind to the individual proteins of the complex, or a derivative, or homolog thereof, one can select on the basis of positive binding to the complex and a lack of binding to the individual protein components.

Antibodies specific to a domain of the complex, or a derivative, or homolog thereof, are also provided.

The foregoing antibodies can be used in methods known in the art relating to the localization and/or quantification of the complexes of the invention, e.g., for imaging these proteins, measuring levels thereof in appropriate physiological samples (by immunoassay), in diagnostic methods, etc. This hold true also for a derivative, or homolog thereof of a complex.

In another embodiment of the invention (see infra), an antibody to a complex or a fragment of such antibodies containing the antibody binding domain, is a Therapeutic.

### 4.3. DIAGNOSTIC, PROGNOSTIC, AND SCREENING USES OF THE PROTEIN COMPLEXES/PROTEINS OF THE INVENTION

The particular protein complexes and proteins of the present invention may be markers of normal physiological processes, and thus have diagnostic utility. Further, definition of particular groups of patients with elevations or deficiencies of a protein complex of the present invention, or wherein the protein complex has a change in protein component composition, can lead to new nosological classifications of diseases, furthering diagnostic ability.
Examples for diseases or disorders are inflammation such as chronic inflammatory diseases, rheumatoid arthritis, inflammatory bowel disease; infectious diseases such as septic shock and bacterial infection; neurological diseases such as stroke-induced inflammation in neurons; neurodegenerative diseases; cancer such as prostate cancer, breast cancer and skin cancer.

Detecting levels of protein complexes, or individual component proteins that form the complexes, or detecting levels of the mRNAs encoding the components of the complex, may be used in diagnosis, prognosis, and/or staging to follow the course of a disease state, to follow a therapeutic response, etc.

A protein complex of the present invention and the individual components of the complex and a derivative, analog or subsequence thereof, encoding nucleic acids (and sequences complementary thereto), and anti-complex antibodies and antibodies directed against individual components that can form the complex, are useful in diagnostics. The foregoing molecules can be used in assays, such as immunoassays, to detect, prognose, diagnose, or monitor various conditions, diseases, and disorders characterized by aberrant levels of a complex or aberrant component composition of a complex, or monitor the treatment of such various conditions, diseases, and disorders.

In particular, such an immunoassay is carried out by a method comprising contacting a sample derived from a patient with an anti-complex antibody under conditions such that immunospecific binding can occur, and detecting or measuring the amount of any immunospecific binding by the antibody. In a specific aspect, such binding of antibody, in tissue sections, can be used to detect aberrant complex localization, or aberrant (e.g., high, low or absent) levels of a protein complex or complexes. In a specific embodiment, an antibody to the complex can be used to assay a patient tissue or serum sample for the presence of the complex, where an aberrant level of the complex is an indication of a diseased condition. By "aberrant levels" is meant increased or decreased levels relative to that present, or a standard level representing that present, in an analogous sample from a portion or fluid of the body, or from a subject not having the disorder.

The immunoassays which can be used include but are not limited to competitive and non-competitive assay systems using techniques such as Western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, to name but a few known in the art.

Nucleic acids encoding the components of the protein complex and related nucleic acid sequences and subsequences, including complementary sequences, can be used in hybridization assays. The nucleic acid sequences, or subsequences thereof, comprising about at least 8 nucleotides, can be used as hybridization probes. Hybridization assays can be used to detect, prognose, diagnose, or monitor conditions, disorders, or disease states associated with aberrant levels of the mRNAs encoding the components of a complex as described, supra. In particular, such a hybridization assay is carried out by a method comprising contacting a sample containing nucleic acid with a nucleic acid probe capable of hybridizing to component protein coding DNA or RNA, under conditions such that hybridization can occur, and detecting or measuring any resulting hybridization.

In specific embodiments, diseases and disorders involving or characterized by aberrant levels of a protein complex or aberrant complex composition can be diagnosed, or its suspected presence can be screened for, or a predisposition to develop such disorders can be detected, by determining the component protein composition of the complex, or detecting aberrant levels of a member of the complex or un-complexed component proteins or encoding nucleic acids, or functional activity including, but not restricted to, binding to an interacting partner, or by detecting mutations in component protein RNA, DNA or protein (e.g., mutations such as translocations, truncations, changes in nucleotide or amino acid sequence relative to wild-type that cause increased or decreased expression or activity of a complex, and/or component protein.
Such diseases and disorders include, but are not limited to inflammation such as chronic inflammatory diseases, rheumatoid arthritis, inflammatory bowel disease; infectious diseases such as septic shock and bacterial infection; neurological diseases such as stroke-induced inflammation in neurons; neurodegenerative diseases; cancer such as prostate cancer, breast cancer and skin cancer.

By way of example, levels of a protein complex and the individual components of a complex can be detected by immunoassay, levels of component protein RNA or DNA can be detected by hybridization assays (e.g., Northern blots, dot blots, RNase protection assays), and binding of component proteins to each other (e.g., complex formation) can be measured by binding assays commonly known in the art. Translocations and point mutations in component protein genes can be detected by Southern blotting, RFLP analysis, PCR using primers that preferably generate a fragment spanning at least most of the gene by sequencing of genomic DNA or cDNA obtained from the patient, etc.

Assays well known in the art (e.g., assays described above such as immunoassays, nucleic acid hybridization assays, activity assays, etc.) can be used to determine whether one or more particular protein complexes are present at either increased or decreased levels, or are absent, in samples from patients suffering from a particular disease or disorder, or having a predisposition to develop such a disease or disorder, as compared to the levels in samples from subjects not having such a disease or disorder, or having a predisposition to develop such a disease or disorder. Additionally, these assays can be used to determine whether the ratio of the complex to the un-complexed components of the complex, is increased or decreased in samples from patients suffering from a particular disease or disorder, or having a predisposition to develop such a disease or disorder, as compared to the ratio in samples from subjects not having such a disease or disorder.

In the event that levels of one or more particular protein complexes (i.e., complexes formed from component protein derivatives, homologs, fragments, or analogs) are determined to be increased in patients suffering from a particular disease or disorder, or having a predisposition to develop such a disease or disorder, then the particular disease or disorder, or predisposition for a disease or disorder, can be diagnosed, have prognosis defined for, be screened for, or be monitored by detecting increased levels of the one or more protein complexes, increased levels of the mRNA that encodes one or more members of the one or more particular protein complexes, or by detecting increased complex functional activity.

Accordingly, in a specific embodiment of the present invention, diseases and disorders involving increased levels of one or more protein complexes can be diagnosed, or their suspected presence can be screened for, or a predisposition to develop such disorders can be detected, by detecting increased levels of the one or more protein complexes, the mRNA encoding both members of the complex, or complex functional activity, or by detecting mutations in the component proteins that stabilize or enhance complex formation, e.g., mutations such as translocations in nucleic acids, truncations in the gene or protein, changes in nucleotide or amino acid sequence relative to wild-type, that stabilize or enhance complex formation.

In the event that levels of one or more particular protein complexes are determined to be decreased in patients suffering from a particular disease or disorder, or having a predisposition to develop such a disease or disorder, then the particular disease or disorder or predisposition for a disease or disorder can be diagnosed, have its prognosis determined, be screened for, or be monitored by detecting decreased levels of the one or more protein complexes, the mRNA that encodes one or more members of the particular one or more protein complexes, or by detecting decreased protein complex functional activity.

Accordingly, in a specific embodiment of the invention, diseases and disorders involving decreased levels of one or more protein complexes can be diagnosed, or their suspected presence can be screened for, or a predisposition to develop such disorders can be detected, by detecting decreased levels of the one or more protein complexes, the mRNA encoding one or more members of the one or more complexes, or complex functional activity, or by detecting mutations in the component proteins that decrease complex formation, e.g., mutations such as translocations in nucleic acids, truncations in the gene or protein, changes in nucleotide or amino acid sequence relative to wild-type, that decrease complex formation.

Accordingly, in a specific embodiment of the invention, diseases and disorders involving aberrant compositions of the complexes can be diagnosed, or their suspected presence can be screened for, or a predisposition to develop such disorders can be detected, by detecting the component proteins of one or more complexes, or the mRNA encoding the members of the one or more complexes.

The use of detection techniques, especially those involving antibodies against a protein complex, provides a method of detecting specific cells that express the complex or component proteins. Using such assays, specific cell types can be defined in which one or more particular protein complexes are expressed, and the presence of the complex or component proteins can be correlated with cell viability, state, health, etc.

Also embodied are methods to detect a protein complex of the present invention in cell culture models that express particular protein complexes or derivatives thereof, for the purpose of characterizing or preparing the complexes for harvest. This embodiment includes cell sorting of prokaryotes such as but not restricted to bacteria (Davey and Kell, 1996, Microbiol. Rev. 60:641-696), primary cultures and tissue specimens from eukaryotes, including mammalian species such as human (Steele et al., 1996, Clin. Obstet. Gynecol 39:801-813), and continuous cell cultures (Orfao and Ruiz-Arguelles, 1996, Clin. Biochem. 29:5-9). Such isolations can be used as methods of diagnosis, described, supra.

In a further specific embodiment, a modulation of the formation process of a complex can be determined.

Such a modulation can either be a change in the typical time course of its formation or a change in the typical steps leading to the formation of the complete complex.
Such changes can for example be detected by analysing and comparing the process of complex formation in untreated wild type cells of a particular type and/or cells showing or having the predisposition to develop a certain disease phenotype and/or cells which have been treated with particular conditions and/or particular agents in a particular situation. Methods to study such changes in time course are well known in the art and include for example Western-blot analysis of the proteins in the complex isolated at different steps of its formation.

Furthermore an aberrant intracellular localization of the protein complex and/or an abberant transcription level of a gene dependent on the complex and/or the abundance and/or activity of a protein or protein complex dependent on the function of the complex and/or a gene dependent on the complex can serve as a marker for a disease and thus have diagnostic utility for any disease which is caused by an aberrant activity, function, composition or formation of the complex of the invention.
Methods to study the intracellular localization are well known in the art and include, but are not limited to immunofluorescence analysis using antibodies specific for components of the protein. Preferentially, double-stainings including staining of other cellular structures are being used to facilitate the detection of the intracellular localization. Methods to analyse the transcription levels of a gene dependent on the complex are also well known in the art and include Northern blot analysis, quantitative PCR etc. The abundance of proteins dependent on the protein can be analyzed as described supra. Methods to study changes in the activity of proteins dependent on complex depend on the protein. The choice of such methods will be apparent to any person skilled in the art.

### 4.4. THERAPEUTIC USES OF PROTEIN COMPLEXES/PROTEINS OF THE INVENTION

The present invention is directed to a method for treatment or prevention of various diseases and disorders by administration of a therapeutic compound (termed herein "Therapeutic"). Such "Therapeutics" include, but are not limited to, a protein complex of the present invention, the individual component proteins, and analogs and derivatives (including fragments) of the foregoing (e.g., as described hereinabove); antibodies thereto (as described hereinabove); nucleic acids encoding the component protein, and analogs or derivatives, thereof (e.g., as described hereinabove); component protein antisense nucleic acids, and agents that modulate complex formation and/or activity (i.e., agonists and antagonists).

The protein complexes as identified herein can be implicated in processes which are implicated in or associated with pathological conditions.
Diseases and disorders which can be treated and/or prevented and/or diagnosed by Therapeutics interacting with any of the complexes provided herein are for example cancer such as solid tumours such as breast cancer, colorectal cancer, melanoma, prostate cancer and lung cancer;cancer such as hematological cancers such as lymphoma and myeloma.

These disorders are treated or prevented by administration of a Therapeutic that modulates (i.e. inhibits or promotes) protein complex activity or formation or modulates its function or composition. Diseases or disorders associated with aberrant levels of complex activity or formation, or aberrant levels or activity of the component proteins, or aberrant complex composition or a change in the function, may be treated by administration of a Therapeutic that modulates complex formation or activity or by the administration of a protein complex.

Therapeutic may also be administered to modulate complex formation or activity or level thereof in a microbial organism such as yeast, fungi such as candida albicans causing an infectious disease in animals or humans.

Diseases and disorders characterized by increased (relative to a subject not suffering from the disease or disorder) complex levels or activity can be treated with Therapeutics that antagonize (i.e., reduce or inhibit) complex formation or activity. Therapeutics that can be used include, but are not limited to, the component proteins or an analog, derivative or fragment of the component protein; anti-complex antibodies (e.g., antibodies specific for the protein complex, or a fragment or derivative of the antibody containing the binding region thereof; nucleic acids encoding the component proteins; antisense nucleic acids complementary to nucleic acids encoding the component proteins; and nucleic acids encoding the component protein that are dysfunctional due to, e.g., a heterologous insertion within the protein coding sequence, that are used to "knockout" endogenous protein function by homologous recombination, see, e.g., Capecchi, 1989, Science 244:1288-1292. In one embodiment, a Therapeutic is 1, 2 or more antisense nucleic acids which are complementary to 1, 2, or more nucleic acids, respectfully, that encode component proteins of a complex.

In a specific embodiment of the present invention, a nucleic acid containing a portion of a component protein gene in which gene sequences flank (are both 5' and 3' to) a different gene sequence, is used as a component protein antagonist, or to promote component protein inactivation by homologous recombination (see also, Koller and Smithies, 1989, Proc. Natl. Acad. Sci. USA 86:8932-8935; Zijlstra et al., 1989, Nature 342: 435-438). Additionally, mutants or derivatives of a component protein that has greater affinity for another component protein or the complex than wild type may be administered to compete with wild type protein for binding, thereby reducing the levels of complexes containing the wild type protein. Other Therapeutics that inhibit complex function can be identified by use of known convenient in vitro assays, e.g., based on their ability to inhibit complex formation, or as described in Section 4.5, infra.

In specific embodiments, Therapeutics that antagonize complex formation or activity are administered therapeutically, including prophylactically, (1) in diseases or disorders involving an increased (relative to normal or desired) level of a complex, for example, in patients where complexes are overactive or overexpressed; or (2) in diseases or disorders where an in vitro (or in vivo) assay (see infra) indicates the utility of antagonist administration. Increased levels of a complex can be readily detected, e.g., by quantifying protein and/or RNA, by obtaining a patient tissue sample (e.g., from biopsy tissue) and assaying it in vitro for RNA or protein levels, or structure and/or activity of the expressed complex (or the encoding mRNA). Many methods standard in the art can be thus employed including, but not limited to, immunoassays to detect complexes and/or visualize complexes (e.g., Western blot analysis, immunoprecipitation followed by sodium dodecyl sulfate polyacrylamide gel electrophoresis [SDS-PAGE], immunocytochemistry, etc.), and/or hybridization assays to detect concurrent expression of component protein mRNA (e.g., Northern assays, dot blot analysis, in situ hybridization, etc.).

A more specific embodiment of the present invention is directed to a method of reducing complex expression (i.e., expression of the protein components of the complex and/or formation of the complex) by targeting mRNAs that express the protein moieties. RNA therapeutics currently fall within three classes, antisense species, ribozymes, or RNA aptamers (Good et al., 1997, Gene Therapy 4:45-54).

Antisense oligonucleotides have been the most widely used. By way of example, but not limitation, antisense oligonucleotide methodology to reduce complex formation is presented below, infra. Ribozyme therapy involves the administration, induced expression, etc. of small RNA molecules with enzymatic ability to cleave, bind, or otherwise inactivate specific RNAs, to reduce or eliminate expression of particular proteins (Grassi and Marini, 1996, Annals of Medicine 28:499-510; Gibson, 1996, Cancer and Metastasis Reviews 15:287-299). RNA aptamers are specific RNA ligand proteins, such as for Tat and Rev RNA (Good et al., 1997, Gene Therapy 4:45-54) that can specifically inhibit their translation. Aptamers specific for component proteins can be identified by many methods well known in the art, for example, by affecting the formation of a complex in the protein-protein interaction assay described, infra.

In another embodiment, the activity or levels of a component protein are reduced by administration of another component protein, or the encoding nucleic acid, or an antibody that immunospecifically binds to the component protein, or a fragment or a derivative of the antibody containing the binding domain thereof.

In another aspect of the invention, diseases or disorders associated with increased levels of an component protein of the complex may be treated or prevented by administration of a Therapeutic that increases complex formation if the complex formation acts to reduce or inactivate the component protein through complex formation. Such diseases or disorders can be treated or prevented by administration of one component member of the complex, administration of antibodies or other molecules that stabilize the complex, etc.

Diseases and disorders associated with underexpression of a complex, or a component protein, are treated or prevented by administration of a Therapeutic that promotes (i.e., increases or supplies) complex levels and/or function, or individual component protein function. Examples of such a Therapeutic include but are not limited to a complex or a derivative, analog or fragment of the complex that are functionally active (e.g., able to form a complex), un-complexed component proteins and derivatives, analogs, and fragments of un-complexed component proteins, and nucleic acids encoding the members of a complex or functionally active derivatives or fragments of the members of the complex, e.g., for use in gene therapy. In a specific embodiment, a Therapeutic includes derivatives, homologs or fragments of a component protein that increase and/or stabilize complex formation. Examples of other agonists can be identified using in vitro assays or animal models, examples of which are described, infra.

In yet other specific embodiments of the present invention, Therapeutics that promote complex function are administered therapeutically, including prophylactically, (1) in diseases or disorders involving an absence or decreased (relative to normal or desired) level of a complex, for example, in patients where a complex, or the individual components necessary to form the complex, is lacking, genetically defective, biologically inactive or underactive, or under-expressed; or (2) in diseases or disorders wherein an in vitro or in vivo assay (see, infra) indicates the utility of complex agonist administration. The absence or decreased level of a complex, component protein or function can be readily detected, e.g., by obtaining a patient tissue sample (e.g., from biopsy tissue) and assaying it in vitro for RNA or protein levels, structure and/or activity of the expressed complex and/or the concurrent expression of mRNA encoding the two components of the complex. Many methods standard in the art can be thus employed, including but not limited to immunoassays to detect and/or visualize a complex, or the individual components of a complex (e.g., Western blot analysis, immunoprecipitation followed by sodium dodecyl sulfate polyacrylamide gel electrophoresis [SDS-PAGE], immunocytochemistry, etc.) and/or hybridization assays to detect expression of mRNAs encoding the individual protein components of a complex by detecting and/or visualizing component mRNA concurrently or separately using, e.g., Northern assays, dot blot analysis, in situ hybridization, etc.

In specific embodiments, the activity or levels of a component protein are increased by administration of another component protein of the same complex, or a derivative, homolog or analog thereof, a nucleic acid encoding the other component, or an agent that stabilizes or enhances the other component, or a fragment or derivative of such an agent.

Generally, administration of products of species origin or species reactivity (in the case of antibodies) that is the same species as that of the patient is preferred. Thus, in a preferred embodiment, a human complex, or derivative, homolog or analog thereof; nucleic acids encoding the members of the human complex or a derivative, homolog or analog thereof; an antibody to a human complex, or a derivative thereof; or other human agents that affect component proteins or the complex, are therapeutically or prophylactically administered to a human patient.

Preferably, suitable in vitro or in vivo assays are utilized to determine the effect of a specific Therapeutic and whether its administration is indicated for treatment of the affected tissue or individual.

In various specific embodiments, in vitro assays can be carried out with representative cells of cell types involved in a patient's disorder, to determine if a Therapeutic has a desired effect upon such cell types.

Compounds for use in therapy can be tested in suitable animal model systems prior to testing in humans, including, but not limited to, rats, mice, chicken, cows, monkeys, rabbits, etc. For in vivo testing, prior to administration to humans, any animal model system known in the art may be used. Additional descriptions and sources of Therapeutics that can be used according to the invention are found in Sections 4.1 to 4.3 and 4.7 herein.

### 4.4.1. GENE THERAPY

In a specific embodiment of the present invention, nucleic acids comprising a sequence encoding the component proteins, or a functional derivative thereof, are administered to modulate complex activity or formation by way of gene therapy. Gene therapy refers to therapy performed by the administration of a nucleic acid to a subject. In this embodiment of the present invention, the nucleic acid expresses its encoded protein(s) that mediates a therapeutic effect by modulating complex activity or formation. Any of the methods for gene therapy available in the art can be used according to the present invention. Exemplary methods are described below.

For general reviews of the methods of gene therapy, see Goldspiel et al., 1993, Clinical Pharmacy 12:488-505; Wu and Wu, 1991, Biotherapy 3:87-95; Tolstoshev, 1993, Ann. Rev. Pharmacol. Toxicol. 32:573-596; Mulligan, 1993, Science 260:926-932; Morgan and Anderson, 1993, Ann. Rev. Biochem. 62:191-217; and May, 1993, TIBTECH 11:155-215. Methods commonly known in the art of recombinant DNA technology which can be used are described in Ausubel et al., eds., 1993, Current Protocols in Molecular Biology, John Wiley & Sons, NY; and Kriegler, 1990, Gene Transfer and Expression, A Laboratory Manual, Stockton Press, NY.

In a preferred aspect, the Therapeutic comprises a nucleic acid that is part of an expression vector that expresses one or more of the component proteins, or fragments or chimeric proteins thereof, in a suitable host. In particular, such a nucleic acid has a promoter operably linked to the protein coding region(s) (or, less preferably separate promoters linked to the separate coding regions separately), said promoter being inducible or constitutive, and optionally, tissue-specific. In another particular embodiment, a nucleic acid molecule is used in which the coding sequences, and any other desired sequences, are flanked by regions that promote homologous recombination at a desired site in the genome, thus providing for intra-chromosomal expression of the component protein nucleic acids (Koller and Smithies, 1989, Proc. Natl. Acad. Sci. USA 86:8932-8935; Zijlstra et al., 1989, Nature 342:435-438).

Delivery of the nucleic acid into a patient may be either direct, in which case the patient is directly exposed to the nucleic acid or nucleic acid-carrying vector, or indirect, in which case, cells are first transformed with the nucleic acid in vitro, then transplanted into the patient. These two approaches are known, respectively, as in vivo or ex vivo gene therapy.

In a specific embodiment, the nucleic acid is directly administered in vivo, where it is expressed to produce the encoded product. This can be accomplished by any of numerous methods known in the art, e.g., by constructing it as part of an appropriate nucleic acid expression vector and administering it so that it becomes intracellular, e.g., by infection using a defective or attenuated retroviral or other viral vector (U.S. Patent No. 4,980,286), or by direct injection of naked DNA, or by use of microparticle bombardment (e.g., a gene gun; Biolistic, Dupont), or coating with lipids or cell-surface receptors, or through use of transfecting agents, by encapsulation in liposomes, microparticles, or microcapsules, or by administering it in linkage to a peptide that is known to enter the nucleus, or by administering it in linkage to a ligand subject to receptor-mediated endocytosis that can be used to target cell types specifically expressing the receptors (e.g., Wu and Wu, 1987, J. Biol. Chem. 262:4429-4432), etc. In another embodiment, a nucleic acid-ligand complex can be formed in which the ligand comprises a fusogenic viral peptide that disrupts endosomes, allowing the nucleic acid to avoid lysosomal degradation. In yet another embodiment, the nucleic acid can be targeted in vivo for cell specific uptake and expression, by targeting a specific receptor (see, e.g., International Patent Publications WO 92/06180; WO 92/22635; WO 92/20316; WO 93/14188; and WO 93/20221. Alternatively, the nucleic acid can be introduced intracellularly and incorporated within host cell DNA for expression, by homologous recombination (Koller and Smithies, 1989, Proc. Natl. Acad. Sci. USA 86:8932-8935; Zijlstra et al., 1989, Nature 342:435-438).

In a specific embodiment, a viral vector that contains the component protein encoding nucleic acids is used. For example, a retroviral vector can be used (Miller et al., 1993, Meth. Enzymol. 217:581-599). These retroviral vectors have been modified to delete retroviral sequences that are not necessary for packaging of the viral genome and integration into host cell DNA. The encoding nucleic acids to be used in gene therapy is/are cloned into the vector, which facilitates delivery of the gene into a patient. More detail about retroviral vectors can be found in Boesen et al., 1994, Biotherapy 6:291-302, which describes the use of a retroviral vector to deliver the mdr1 gene to hematopoetic stem cells in order to make the stem cells more resistant to chemotherapy. Other references illustrating the use of retroviral vectors in gene therapy are Clowes et al., 1994, J. Clin. Invest. 93:644-651; Kiem et al., 1994, Blood 83:1467-1473; Salmons and Gunzberg, 1993, Human Gene Therapy 4:129-141; and Grossman and Wilson, 1993, Curr. Opin. in Genetics and Devel. 3:110-114.

Adenoviruses are other viral vectors that can be used in gene therapy. Adenoviruses are especially attractive vehicles for delivering genes to respiratory epithelia. Adenoviruses naturally infect respiratory epithelia where they cause a mild disease. Other targets for adenovirus-based delivery systems are the liver, the central nervous system, endothelial cells and muscle. Adenoviruses have the advantage of being capable of infecting non-dividing cells. Kozarsky and Wilson, 1993, Current Opinion in Genetics and Development 3:499-503, discuss adenovirus-based gene therapy. The use of adenovirus vectors to transfer genes to the respiratory epithelia of rhesus monkeys has been demonstrated by Bout et al., 1994, Human Gene Therapy 5:3-10. Other instances of the use of adenoviruses in gene therapy can be found in Rosenfeld et al., 1991, Science 252:431-434; Rosenfeld et al., 1992, Cell 68:143-155; and Mastrangeli et al., 1993, J. Clin. Invest. 91:225-234.

Adeno-associated virus (AAV) has also been proposed for use in gene therapy (Walsh et al., 1993, Proc. Soc. Exp. Biol. Med. 204:289-300.

Another approach to gene therapy involves transferring a gene into cells in tissue culture by methods such as electroporation, lipofection; calcium phosphate-mediated transfection, or viral infection. Usually, the method of transfer includes the transfer of a selectable marker to the cells. The cells are then placed under selection to isolate those cells that have taken up and are expressing the transferred gene from these that have not. Those cells are then delivered to a patient.

In this embodiment, the nucleic acid is introduced into a cell prior to administration in vivo of the resulting recombinant cell. Such introduction can be carried out by any method known in the art including, but not limited to, transfection by electroporation, microinjection, infection with a viral or bacteriophage vector containing the nucleic acid sequences, cell fusion, chromosome-mediated gene transfer, microcell-mediated gene transfer, spheroplast fusion, etc. Numerous techniques are known in the art for the introduction of foreign genes into cells (see, e.g., Loeffler and Behr, 1993, Meth. Enzymol. 217:599-618; Cohen et al., 1993, Meth. Enzymol. 217:618-644; Cline, 1985, Pharmac. Ther. 29:69-92) and may be used in accordance with the present invention, provided that the necessary developmental and physiological functions of the recipient cells are not disrupted. The technique should provide for the stable transfer of the nucleic acid to the cell, so that the nucleic acid is expressible by the cell and preferably, is heritable and expressible by its cell progeny.

The resulting recombinant cells can be delivered to a patient by various methods known in the art. In a preferred embodiment, epithelial cells are injected, e.g., subcutaneously. In another embodiment, recombinant skin cells may be applied as a skin graft onto the patient. Recombinant blood cells (e.g., hematopoetic stem or progenitor cells) are preferably administered intravenously. The amount of cells envisioned for use depends on the desired effect, patient state, etc., and can be determined by one skilled in the art.

Cells into which a nucleic acid can be introduced for purposes of gene therapy encompass any desired, available cell type, and include but are not limited to epithelial cells, endothelial cells, keratinocytes, fibroblasts, muscle cells, hepatocytes, blood cells such as T lymphocytes, B lymphocytes, monocytes, macrophages, neutrophils, eosinophils, megakaryocytes, and granulocytes, various stem or progenitor cells, in particular hematopoetic stem or progenitor cells, e.g., as obtained from bone marrow, umbilical cord blood, peripheral blood, fetal liver, etc.

In a preferred embodiment, the cell used for gene therapy is autologous to the patient.

In an embodiment in which recombinant cells are used in gene therapy, a component protein encoding nucleic acid is/are introduced into the cells such that the gene or genes are expressible by the cells or their progeny, and the recombinant cells are then administered in vivo for therapeutic effect. In a specific embodiment, stem or progenitor cells are used. Any stem and/or progenitor cells which can be isolated and maintained in vitro can potentially be used in accordance with this embodiment of the present invention. Such stem cells include but are not limited to hematopoetic stem cells (HSCs), stem cells of epithelial tissues such as the skin and the lining of the gut, embryonic heart muscle cells, liver stem cells (International Patent Publication WO 94/08598), and neural stem cells (Stemple and Anderson, 1992, Cell 71:973-985).

Epithelial stem cells (ESCs), or keratinocytes, can be obtained from tissues such as the skin and the lining of the gut by known procedures (Rheinwald, 1980, Meth. Cell Biol. 2A:229). In stratified epithelial tissue such as the skin, renewal occurs by mitosis of stem cells within the germinal layer, the layer closest to the basal lamina. Similarly, stem cells within the lining of the gut provide for a rapid renewal rate of this tissue. ESCs or keratinocytes obtained from the skin or lining of the gut of a patient or donor can be grown in tissue culture (Rheinwald, 1980, Meth. Cell Bio. 2A:229; Pittelkow and Scott, 1986, Mayo Clinic Proc. 61:771). If the ESCs are provided by a donor, a method for suppression of host versus graft reactivity (e.g., irradiation, or drug or antibody administration to promote moderate immunosuppression) can also be used.

With respect to hematopoetic stem cells (HSCs), any technique that provides for the isolation, propagation, and maintenance in vitro of HSCs can be used in this embodiment of the invention. Techniques by which this may be accomplished include (a) the isolation and establishment of HSC cultures from bone marrow cells isolated from the future host, or a donor, or (b) the use of previously established long-term HSC cultures, which may be allogeneic or xenogeneic. Non-autologous HSCs are used preferably in conjunction with a method of suppressing transplantation immune reactions between the future host and patient. In a particular embodiment of the present invention, human bone marrow cells can be obtained from the posterior iliac crest by needle aspiration (see, e.g., Kodo et al., 1984, J. Clin. Invest. 73: 1377-1384). In a preferred embodiment of the present invention, the HSCs can be made highly enriched or in substantially pure form. This enrichment can be accomplished before, during, or after long-term culturing, and can be done by any technique known in the art. Long-term cultures of bone marrow cells can be established and maintained by using, for example, modified Dexter cell culture techniques (Dexter et al., 1977, J. Cell Physiol. 91:335) or Witlock-Witte culture techniques (Witlock and Witte, 1982, Proc. Natl. Acad. Sci. USA 79:3608-3612).

In a specific embodiment, the nucleic acid to be introduced for purposes of gene therapy comprises an inducible promoter operably linked to the coding region, such that expression of the nucleic acid is controllable by controlling the presence or absence of the appropriate inducer of transcription.

Additional methods can be adapted for use to deliver a nucleic acid encoding the component proteins, or functional derivatives thereof, e.g., as described in Section 4.1, supra.

### 4.4.2. USE OF ANTISENSE OLIGONUCLEOTIDES FOR SUPPRESSION OF PROTEIN COMPLEX FORMATION OR PROTEIN COMPLEX/PROTEIN ACTIVITY

In a specific embodiment of the present invention, protein complex activity and formation and protein activity is inhibited by use of antisense nucleic acids for the component proteins of the complex, that inhibit transcription and/or translation of their complementary sequence. The present invention provides the therapeutic or prophylactic use of nucleic acids of at least six nucleotides that are antisense to a gene or cDNA encoding a component protein, or a portion thereof. An "antisense" nucleic acid as used herein refers to a nucleic acid capable of hybridizing to a sequence-specific portion of a component protein RNA (preferably mRNA) by virtue of some sequence complementarity. The antisense nucleic acid may be complementary to a coding and/or noncoding region of a component protein mRNA. Such antisense nucleic acids that inhibit complex formation or activity have utility as Therapeutics, and can be used in the treatment or prevention of disorders as described supra.

The antisense nucleic acids of the invention can be oligonucleotides that are double-stranded or single-stranded, RNA or DNA, or a modification or derivative thereof, which can be directly administered to a cell, or which can be produced intracellularly by transcription of exogenous, introduced sequences.

In another embodiment, the present invention is directed to a method for inhibiting the expression of component protein nucleic acid sequences, in a prokaryotic or eukaryotic cell, comprising providing the cell with an effective amount of a composition comprising an antisense nucleic acid of the component protein, or a derivative thereof, of the invention.

The antisense nucleic acids are of at least six nucleotides and are preferably oligonucleotides, ranging from 6 to about 200 nucleotides. In specific aspects, the oligonucleotide is at least 10 nucleotides, at least 15 nucleotides, at least 100 nucleotides, or at least 200 nucleotides. The oligonucleotides can be DNA or RNA or chimeric mixtures, or derivatives or modified versions thereof, and either single-stranded or double-stranded. The oligonucleotide can be modified at the base moiety, sugar moiety, or phosphate backbone. The oligonucleotide may include other appending groups such as peptides, agents facilitating transport across the cell membrane (see, e.g., Letsinger et al., 1989, Proc. Natl. Acad. Sci. U.S.A. 86:6553-6556; Lemaitre et al., 1987, Proc. Natl. Acad. Sci. 84:648-652; International Patent Publication No. WO 88/09810) or blood-brain barrier (see, e.g., International Patent Publication No. WO 89/10134), hybridization-triggered cleavage agents (see, e.g., Krol et al., 1988, BioTechniques 6:958-976), or intercalating agents (see, e.g., Zon, 1988, Pharm. Res. 5:539-549).

In a preferred aspect of the invention, an antisense oligonucleotide is provided, preferably as single-stranded DNA. The oligonucleotide may be modified at any position in its structure with constituents generally known in the art.

The antisense oligonucleotides may comprise at least one modified base moiety which is selected from the group including but not limited to 5-fluorouracil, 5-bromouracil, 5-chlorouracil, 5-iodouracil, hypoxanthine, xanthine, 4-acetylcytosine, 5-(carboxyhydroxylmethyl)uracil, 5-carboxymethylaminomethyl-2-thio-uridine, 5-carboxymethylaminomethyluracil, dihydrouracil, β-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, β-D-mannosylqueosine, 5N-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

In another embodiment, the oligonucleotide comprises at least one modified sugar moiety selected from the group including, but not limited to, arabinose, 2-fluoroarabinose, xylulose, and hexose.

In yet another embodiment, the oligonucleotide comprises at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal, or an analog of the foregoing.

In yet another embodiment, the oligonucleotide is a 2-a-anomeric oligonucleotide. An a-anomeric oligonucleotide forms specific double-stranded hybrids with complementary RNA in which, contrary to the usual β-units, the strands run parallel to each other (Gautier et al., 1987, Nucl. Acids Res. 15:6625-6641).

The oligonucleotide may be conjugated to another molecule, e.g., a peptide, hybridization-triggered cross-linking agent, transport agent, hybridization-triggered cleavage agent, etc.

Oligonucleotides of the invention may be synthesized by standard methods known in the art, e.g., by use of an automated DNA synthesizer (such as are commercially avail-able from Biosearch, Applied Biosystems, etc.). As examples, phosphorothioate oligo-nucleotides may be synthesized by the method of Stein et al. (1988, Nucl. Acids Res. 16:3209), methylphosphonate oligonucleotides can be prepared by use of controlled pore gass polymer supports (Sarin et al., 1988, Proc. Natl. Acad. Sci. U.S.A. 85:7448-7451), etc.

In a specific embodiment, the antisense oligonucleotides comprise catalytic RNAs, or ribozymes (see, e.g., International Patent Publication No. WO 90/11364; Sarver et al., 1990, Science 247:1222-1225). In another embodiment, the oligonucleotide is a 2'-0-methylribonucleotide (Inoue et at., 1987, Nucl. Acids Res. 15:6131-6148), or a chimeric RNA-DNA analog (Inoue et al., 1987, FEBS Lett. 215:327-330).

In an alternative embodiment, the antisense nucleic acids of the invention are produced intracellularly by transcription from an exogenous sequence. For example, a vector can be introduced in vivo such that it is taken up by a cell, within which cell the vector or a portion thereof is transcribed, producing an antisense nucleic acid (RNA) of the invention. Such a vector would contain a sequence encoding the component protein. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art to be capable of replication and expression in mammalian cells. Expression of the sequences encoding the antisense RNAs can be by any promoter known in the art to act in mammalian, preferably human, cells. Such promoters can be inducible or constitutive. Such promoters include, but are not limited to, the SV40 early promoter region (Bernoist and Chambon, 1981, Nature 290:304-310), the promoter contained in the 3' long terminal repeat of Rous sarcoma virus (Yamamoto et al., 1980, Cell 22:787-797), the herpes thymidine kinase promoter (Wagner et al., 1981, Proc. Natl. Acad. Sci. U.S.A. 78:1441-1445), the regulatory sequences of the metallothionein gene (Brinster et al., 1982, Nature 296:39-42), etc.

The antisense nucleic acids of the invention comprise a sequence complementary to at least a portion of an RNA transcript of a component protein gene, preferably a human gene. However, absolute complementarity, although preferred, is not required. A sequence "complementary to at least a portion of an RNA," as referred to herein, means a sequence having sufficient complementarity to be able to hybridize with the RNA, forming a stable duplex; in the case of double-stranded antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with a component protein RNA it may contain and still form a stable duplex (or triplex, as the case may be). One skilled in the art can ascertain a tolerable degree of mismatch by use of standard procedures to determine the melting point of the hybridized complex.

The component protein antisense nucleic acids can be used to treat (or prevent) disorders of a cell type that expresses, or preferably overexpresses, a protein complex.

Cell types that express or overexpress component protein RNA can be identified by various methods known in the art. Such methods include, but are not limited to, hybridization with component protein-specific nucleic acids (e.g., by Northern blot hybridization, dot blot hybridization, or in situ hybridization), or by observing the ability of RNA from the cell type to be translated in vitro into the component protein by immunohistochemistry, Western blot analysis, ELISA, etc. In a preferred aspect, primary tissue from a patient can be assayed for protein expression prior to treatment, e.g., by immunocytochemistry, in situ hybridization, or any number of methods to detect protein or mRNA expression.

Pharmaceutical compositions of the invention (see Section 4.7, infra), comprising an effective amount of a protein component antisense nucleic acid in a pharmaceutically acceptable carrier can be administered to a patient having a disease or disorder that is of a type that expresses or overexpresses a protein complex of the present invention.

The amount of antisense nucleic acid that will be effective in the treatment of a particular disorder or condition will depend on the nature of the disorder or condition, and can be determined by standard clinical techniques. Where possible, it is desirable to determine the antisense cytotoxicity in vitro, and then in useful animal model systems, prior to testing and use in humans.

In a specific embodiment, pharmaceutical compositions comprising antisense nucleic acids are administered via liposomes, microparticles, or microcapsules. In various embodiments of the invention, it may be useful to use such compositions to achieve sustained release of the antisense nucleic acids. In a specific embodiment, it may be desirable to utilize liposomes targeted via antibodies to specific identifiable central nervous system cell types (Leonetti et al., 1990, Proc. Natl. Acad. Sci. U.S.A. 87:2448-2451; Renneisen et al., 1990, J. Biol. Chem. 265:16337-16342).

### 4.5. ASSAYS OF PROTEIN COMPLEXES/PROTEINS OF THE INVENTION AND DERIVATIVES AND ANALOGS THEREOF

The functional activity of a protein complex of the present invention, or a derivative, fragment or analog thereof or protein component thereof, can be assayed by various methods. Potential modulators (e.g., agonists and antagonists) of complex activity or formation, e.g., anti- complex antibodies and antisense nucleic acids, can be assayed for the ability to modulate complex activity or formation.

In one embodiment of the present invention, where one is assaying for the ability to bind or compete with a wild-type complex for binding to an anti-complex antibody, various immunoassays known in the art can be used, including but not limited to competitive and non-competitive assay systems using techniques such as radioimmunoassay, ELISA (enzyme linked immunosorbent assay), "sandwich" immunoassays, immunoradiometric assays, gel diffusion precipitin reactions, immunodiffusion assays, in situ immunoassays (using colloidal gold, enzyme or radioisotope labels), western blot analysis, precipitation reactions, agglutination assays (e.g., gel agglutination assays, hemagglutination assays), complement fixation assays, immunofluorescence assays, protein A assays, immunoelectrophoresis assays, etc. In one embodiment, antibody binding is detected by detecting a label on the primary antibody. In another embodiment, the primary antibody is detected by detecting binding of a secondary antibody or reagent to the primary antibody. In a further embodiment, the secondary antibody is labeled. Many means are known in the art for detecting binding in an immunoassay and are within the scope of the present invention.

The expression of the component protein genes (both endogenous and those expressed from cloned DNA containing the genes) can be detected using techniques known in the art, including but not limited to Southern hybridization (Southern, 1975, J. Mol. Biol. 98:503-517), northern hybridization (see, e.g., Freeman et al., 1983, Proc. Natl. Acad. Sci. USA 80:4094-4098), restriction endonuclease mapping (Sambrook et al., 1989, Molecular Cloning, A Laboratory Manual, 2^{nd} Ed. Cold Spring Harbor Laboratory Press, New York), RNase protection assays (Current Protocols in Molecular Biology, John Wiley and Sons, New York, 1997), DNA sequence analysis, and polymerase chain reaction amplification (PCR; U.S. Patent Nos. 4,683,202, 4,683,195, and 4,889,818; Gyllenstein et al., 1988, Proc. Natl. Acad. Sci. USA 85:7652-7657; Ochman et al., 1988, Genetics 120:621-623; Loh et al., 1989, Science 243:217-220) followed by Southern hybridization with probes specific for the component protein genes, in various cell types. Methods of amplification other than PCR commonly known in the art can be employed. In one embodiment, Southern hybridization can be used to detect genetic linkage of component protein gene mutations to physiological or pathological states. Various cell types, at various stages of development, can be characterized for their expression of component proteins at the same time and in the same cells. The stringency of the hybridization conditions for northern or Southern blot analysis can be manipulated to ensure detection of nucleic acids with the desired degree of relatedness to the specific probes used. Modifications to these methods and other methods commonly known in the art can be used.

Derivatives (e.g., fragments), homologs and analogs of one component protein can be assayed for binding to another component protein in the same complex by any method known in the art, for example the modified yeast matrix mating test described in Section 4.6.1 infra, immunoprecipitation with an antibody that binds to the component protein complexed with other component proteins in the same complex, followed by size fractionation of the immunoprecipitated proteins (e.g., by denaturing or nondenaturing polyacrylamide gel electrophoresis), Western blot analysis, etc.

One embodiment of the invention provides a method for screening a derivative, homolog or analog of a component protein for biological activity comprising contacting said derivative, homolog or analog of the component protein with the other component proteins in the same complex; and detecting the formation of a complex between said derivative, homolog or analog of the component protein and the other component proteins; wherein detecting formation of said complex indicates that said derivative, homolog or analog of has biological (e.g., binding) activity.

The invention also provides methods of modulating the activity of a component protein that can participate in a protein complex by administration of a binding partner of that protein or derivative, homolog or analog thereof.

In a specific embodiment of the present invention, a protein complex of the present invention is administered to treat or prevent a disease or disorder, since the complex and/or component proteins have been implicated in the disease and disorder. Accordingly, a protein complex or a derivative, homolog, analog or fragment thereof, nucleic acids encoding the component proteins, anti-complex antibodies, and other modulators of protein complex activity, can be tested for activity in treating or preventing a disease or disorder in in vitro and in vivo assays.

In one embodiment, a Therapeutic of the invention can be assayed for activity in treating or preventing a disease by contacting cultured cells that exhibit an indicator of the disease in vitro, with the Therapeutic, and comparing the level of said indicator in the cells contacted with the Therapeutic, with said level of said indicator in cells not so contacted, wherein a lower level in said contacted cells indicates that the Therapeutic has activity in treating or preventing the disease.

In another embodiment of the invention, a Therapeutic of the invention can be assayed for activity in treating or preventing a disease by administering the Therapeutic to a test animal that is predisposed to develop symptoms of a disease, and measuring the change in said symptoms of the disease after administration of said Therapeutic, wherein a reduction in the severity of the symptoms of the disease or prevention of the symptoms of the disease indicates that the Therapeutic has activity in treating or preventing the disease. Such a test animal can be any one of a number of animal models known in the art for disease. These animal models are well known in the art. These animal models include, but are not limited to those which are listed in the section 4.6 (supra) as exemplary animald models to study any of the complexes provided in the invention.

### 4.6 SCREENING FOR MODULATORS OF THE PROTEIN COMPLEXES/PROTEINS OF THE INVENTION

A complex of the present invention, the component proteins of the complex and nucleic acids encoding the component proteins, as well as derivatives and fragments of the amino and nucleic acids, can be used to screen for compounds that bind to, or modulate the amount of, activity of, or protein component composition of, said complex, and thus, have potential use as modulators, i.e., agonists or antagonists, of complex activity, and/or complex formation, i.e., the amount of complex formed, and/or protein component composition of the complex.

Thus, the present invention is also directed to methods for screening for molecules that bind to, or modulate the function of, amount of, activity of, formation of or protein component composition of, a complex of the present invention. In one embodiment of the invention, the method for screening for a molecule that modulates directly or indirectly the function, activity or formation of a complex of the present invention comprises exposing said complex, or a cell or organism containing the complex machinery, to one or more candidate molecules under conditions conducive to modulation; and determining the amount of, the biochemical activity of, protein components of, and/or intracellular localization of, said complex and/or the transcription level of a gene dependend on the complex and/or the abundance and/or activity of a protein or protein complex dependend on the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependend on the complex and/or the abundance and/or activity of a protein or protein complex dependent on the function of the complex and/or product of a gene dependent on the complex in the absence of said candidate molecules indicates that the molecule modulates function, activity or composition of said complex.

In a further specific embodiment, a modulation of the formation process of a complex can be determined.

Such a modulation can either be a change in the typical time course of its formation or a change in the typical steps leading to the formation of the complete complex.
Such changes can for example be detected by analysing and comparing the process of complex formation in untreated wild type cells of a particular type and/or cells showing or having the predisposition to develop a certain disease phenotype and/or cells which have been treated with particular conditions and/or particular agents in a particular situation. Methods to study such changes in time course are well known in the art and include for example Western-blot analysis of the proteins in the complex isolated at different steps of its formation.

Furthermore an aberrant intracellular localization of the protein complex and/or an abberant transcription level of a gene dependent on the complex and/or the abundance and/or activity of a protein or protein complex dependent on the function of the complex and/or a gene dependent on the complex can serve as a marker for a disease and thus have diagnostic utility for any disease which is caused by an aberrant activity, function, composition or formation of the complex of the invention.
Methods to study the intracellular localization are well known in the art and include, but are not limited to immunofluorescence analysis using antibodies specific for components of the protein. Preferentially, double-stainings including staining of other cellular structures are being used to facilitate the detection of the intracellular localization. Methods to analyse the transcription levels of a gene dependent on the complex are also well known in the art and include Northern blot analysis, quantitative PCR etc. The abundance of proteins dependent on the protein can be analyzed as described supra. Methods to study changes in the activity of proteins dependent on complex depend on the protein. The choice of such methods will be apparent to any person skilled in the art.

In another embodiment, the present invention further relates to a process for the identification and/or preparation of an effector of the complex comprising the step of bringing into contact a product of any of claims 1 to 7 with a compound, a mixture or a library of compounds and determining whether the compound or a certain compound of the mixture or library binds to the product and/or effects the products biological activity and optionally further purifying the compound positively tested as effector.

In another embodiment, the present invention is directed to a method for screening for a molecule that binds a protein complex of the present invention comprising exposing said complex, or a cell or organism containing the complex machinery, to one or more candidate molecules; and determining whether said complex is bound by any of said candidate molecules. Such screening assays can be carried out using cell-free and cell-based methods that are commonly known in the art in vitro, in vivo or ex vivo. For example, an isolated complex can be employed, or a cell can be contacted with the candidate molecule and the complex can be isolated from such contacted cells and the isolated complex can be assayed for activity or component composition. In another example, a cell containing the complex can be contacted with the candidate molecule and the levels of the complex in the contacted cell can be measured. Additionally, such assays can be carried out in cells recombinantly expressing a component protein from the third column of table 1, or a functionally active fragment or functionally active derivative thereof, and a component protein from forth column of table 1, or a functionally active fragment or functionally active derivative thereof. Additionally, such assays can also be carried out in cells recombinantly expressing all component proteins from the group of proteins in the fifth column of table 1.

For example, assays can be carried out using recombinant cells expressing the protein components of a complex, to screen for molecules that bind to, or interfere with, or promote complex activity or formation. In preferred embodiments, polypeptide derivatives that have superior stabilities but retain the ability to form a complex (e.g., one or more component proteins modified to be resistant to proteolytic degradation in the binding assay buffers, or to be resistant to oxidative degradation), are used to screen for modulators of complex activity or formation. Such resistant molecules can be generated, e.g., by substitution of amino acids at proteolytic cleavage sites, the use of chemically derivatized amino acids at proteolytic susceptible sites, and the replacement of amino acid residues subject to oxidation, i.e. methionine and cysteine.

A particular aspect of the present invention relates to identifying molecules that inhibit or promote formation or degradation of a complex of the present invention, e.g., using the method described for isolating the complex and identifying members of the complex using the TAP assay described in Section 4, infra, and in WO 00/09716 and Rigaut et al., 1999, Nature Biotechnology 17:1030-1032, which are each incorporated by reference in their entirety. TNRF1

In another embodiment of the invention, a modulator is identified by administering a candidate molecule to a transgenic non-human animal expressing the complex component proteins from promoters that are not the native promoters of the respective proteins, more preferably where the candidate molecule is also recombinantly expressed in the transgenic non-human animal. Alternatively, the method for identifying such a modulator can be carried out in vitro, preferably with a purified complex, and a purified candidate molecule.

Agents/molecules (candidate molecules) to be screened can be provided as mixtures of a limited number of specified compounds, or as compound libraries, peptide libraries and the like. Agents/molecules to be screened may also include all forms of antisera, antisense nucleic acids, etc., that can modulate complex activity or formation. Exemplary candidate molecules and libraries for screening are set forth in Section 4.6.1, infra.

Screening the libraries can be accomplished by any of a variety of commonly known methods. See, e.g., the following references, which disclose screening of peptide libraries: Parmley and Smith, 1989, Adv. Exp. Med. Biol. 251:215-218; Scott and Smith, 1990, Science 249:386-390; Fowlkes et al., 1992, BioTechniques 13:422-427; Oldenburg et al., 1992, Proc. Natl. Acad. Sci. USA 89:5393-5397; Yu et al., 1994, Cell 76:933-945; Staudt et al., 1988, Science 241:577-580; Bock et al., 1992, Nature 355:564-566; Tuerk et al., 1992, Proc. Natl. Acad. Sci. USA 89:6988-6992; Ellington et al., 1992, Nature 355:850-852; U.S. Patent No. 5,096,815, U.S. Patent No. 5,223,409, and U.S. Patent No. 5,198,346, all to Ladner et al.; Rebar and Pabo, 1993, Science 263:671-673; and International Patent Publication No. WO 94/18318.

In a specific embodiment, screening can be carried out by contacting the library members with a complex immobilized on a solid phase, and harvesting those library members that bind to the protein (or encoding nucleic acid or derivative). Examples of such screening methods, termed "panning" techniques, are described by way of example in Parmley and Smith, 1988, Gene 73:305-318; Fowlkes et al., 1992, BioTechniques 13:422-427; International Patent Publication No. WO 94/18318; and in references cited hereinabove.

In a specific embodiment, fragments and/or analogs of protein components of a complex, especially peptidomimetics, are screened for activity as competitive or non-competitive inhibitors of complex formation (amount of complex or composition of complex) or activity in the cell, which thereby inhibit complex activity or formation in the cell.

In one embodiment, agents that modulate (i.e., antagonize or agonize) complex activity or formation can be screened for using a binding inhibition assay, wherein agents are screened for their ability to modulate formation of a complex under aqueous, or physiological, binding conditions in which complex formation occurs in the absence of the agent to be tested. Agents that interfere with the formation of complexes of the invention are identified as antagonists of complex formation. Agents that promote the formation of complexes are identified as agonists of complex formation. Agents that completely block the formation of complexes are identified as inhibitors of complex formation.

Methods for screening may involve labeling the component proteins of the complex with radioligands (e.g., ¹²⁵| or ³H), magnetic ligands (e.g., paramagnetic beads covalently attached to photobiotin acetate), fluorescent ligands (e.g., fluorescein or rhodamine), or enzyme ligands (e.g., luciferase or β-galactosidase). The reactants that bind in solution can then be isolated by one of many techniques known in the art, including but not restricted to, co-immunoprecipitation of the labeled complex moiety using antisera against the unlabeled binding partner (or labeled binding partner with a distinguishable marker from that used on the second labeled complex moiety), immunoaffinity chromatography, size exclusion chromatography, and gradient density centrifugation. In a preferred embodiment, the labeled binding partner is a small fragment or peptidomimetic that is not retained by a commercially available filter. Upon binding, the labeled species is then unable to pass through the filter, providing for a simple assay of complex formation.

Methods commonly known in the art are used to label at least one of the component members of the complex. Suitable labeling methods include, but are not limited to, radiolabeling by incorporation of radiolabeled amino acids, e.g., ³H-leucine or ³⁵ S-methionine, radiolabeling by post-translational iodination with ¹²⁵I or ¹³¹I using the chloramine T method, Bolton-Hunter reagents, etc., or labeling with ³²P using phosphorylase and inorganic radiolabeled phosphorous, biotin labeling with photobiotin-acetate and sunlamp exposure, etc. In cases where one of the members of the complex is immobilized, e.g., as described infra, the free species is labeled. Where neither of the interacting species is immobilized, each can be labeled with a distinguishable marker such that isolation of both moieties can be followed to provide for more accurate quantification, and to distinguish the formation of homomeric from heteromeric complexes. Methods that utilize accessory proteins that bind to one of the modified interactants to improve the sensitivity of detection, increase the stability of the complex, etc., are provided.

Typical binding conditions are, for example, but not by way of limitation, in an aqueous salt solution of 10-250 mM NaCl, 5-50 mM Tris-HCl, pH 5-8, and 0.5% Triton X-100 or other detergent that improves specificity of interaction. Metal chelators and/or divalent cations may be added to improve binding and/or reduce proteolysis. Reaction temperatures may include 4, 10, 15, 22, 25, 35, or 42 degrees Celsius, and time of incubation is typically at least 15 seconds, but longer times are preferred to allow binding equilibrium to occur. Particular complexes can be assayed using routine protein binding assays to determine optimal binding conditions for reproducible binding.

The physical parameters of complex formation can be analyzed by quantification of complex formation using assay methods specific for the label used, e.g., liquid scintillation counting for radioactivity detection, enzyme activity for enzyme-labeled moieties, etc. The reaction results are then analyzed utilizing Scatchard analysis, Hill analysis, and other methods commonly known in the arts (see, e.g., Proteins, Structures, and Molecular Principles, 2^{nd} Edition (1993) Creighton, Ed., W.H. Freeman and Company, New York).

In a second common approach to binding assays, one of the binding species is immobilized on a filter, in a microtiter plate well, in a test tube, to a chromatography matrix, etc., either covalently or non-covalently. Proteins can be covalently immobilized using any method well known in the art, for example, but not limited to the method of Kadonaga and Tjian, 1986, Proc. Natl. Acad. Sci. USA 83:5889-5893, i.e., linkage to a cyanogen-bromide derivatized substrate such as CNBr-Sepharose 4B (Pharmacia). Where needed, the use of spacers can reduce steric hindrance by the substrate. Noncovalent attachment of proteins to a substrate include, but are not limited to, attachment of a protein to a charged surface, binding with specific antibodies, binding to a third unrelated interacting protein, etc.

Assays of agents (including cell extracts or a library pool) for competition for binding of one member of a complex (or derivatives thereof) with another member of the complex labeled by any means (e.g., those means described above) are provided to screen for competitors or enhancers of complex formation.

In specific embodiments, blocking agents to inhibit non-specific binding of reagents to other protein components, or absorptive losses of reagents to plastics, immobilization matrices, etc., are included in the assay mixture. Blocking agents include, but are not restricted to bovine serum albumin, β-casein, nonfat dried milk, Denhardt's reagent, Ficoll, polyvinylpyrolidine, nonionic detergents (NP40, Triton X-100, Tween 20, Tween 80, etc.), ionic detergents (e.g., SDS, LDS, etc.), polyethylene glycol, etc. Appropriate blocking agent concentrations allow complex formation.

After binding is performed, unbound, labeled protein is removed in the supernatant, and the immobilized protein retaining any bound, labeled protein is washed extensively. The amount of bound label is then quantified using standard methods in the art to detect the label as described, supra.

In another specific embodiments screening for modulators of the protein complexes/protein as provided herein can be carried out by attaching those and/or the antibodies as provided herein to a solid carrier. In a further specific embodiment, the invention relates to an array of said molecules.

The preparation of such an array containing different types of proteins, inducting antibodies) is well known in the art and is apparent to a person skilled in the art (see e.g. Ekins RP et. al. (1989) Journal of Pharmaceutical and Biomedical Analysis 7: 155-168; Mitchell P et. al. (2002) Nature Biotechnology 20: 225-229; Petricoin EF et. al. (2002) Lancet 359: 572-577; Templin MF et. al. (2001) Trends in Biotechnology 20: 160-166; Wilson DS and Nock S (2001) Curr Opin Chem Biol 6: 81-85; Lee KB et. al. (2002) Science 295: 1702-1705; MacBeath G and Schreiber S.L (2000) Science 289, 1760; Blawas AS and Reichert WM (1998) Biomaterials 19, 595; Kane RS et. al. (1999) Biomaterials 20: 2363; Chen CS et al. (1997) Science 276, 1425; Vaugham TJ et. al (1996) Nature Biotechnol 14: 309-314; Mahler SM et al. (1997) Immunotechnology 3, 31-43; Roberts et al. (1999) Curr Opin Chem Biol 3: 268-273; Nord K et al. (1997) Nature Biotechlol 15: 772-777; Nord K et al. (2001) Eur J Biochem 268: 4269-4277; Brody E and Gold L (2000) Rev Mol Biotechlol 74: 5-13; Karlstroem A and Nygren PA (2001) Anal Biochem 295, 22-30; Nelson RW et. al. (2000) Electrophoresis 21: 1155-1163. Honore B et al. (2001) Expert Rev Mol Diagn 3: 265-274, Albala JS (2001) Expert Rev Mol Diagn 2: 145-152, Figeys D and Pinto D (2001) Electrophoresis 2: 208-16 and references in the publications listed here.).

Complexes can be attached to an array by different means as will be apparent to a person skilled in the art. Complexes can for example be added to the array via a TAP-tag (as described in WO/0009716 and in Rigaut, G et. al. (1999), Nature Biotechnology. Vol 17 (10): 1030-1032 ) after the purification step or by another suitable purification scheme as will be apparent to a person skilled in the art.

Optionally, the proteins of the complex can be cross-linked to enhance the stability of the complex. Different methods to cross-link proteins are well known in the art. Reactive end-groups of cross-linking agents include but are not limited to-COOH, -SH, -NH2 or N-oxy-succinamate.
The spacer of the cross-linking agent should be chosen with respect to the size of the complex to be cross-linked. For small protein complexes, comprising only a few proteins, relatively short spacers are preferable in order to reduce the likelihood of cross-linking separate complexes in the reaction mixture. For larger protein complexes, additional use of larger spacers is preferable in order to facilitate cross-linking between proteins within the complex.

It is preferable to check the success-rate of cross-linking before linking the complex to the carrier.

As will be apparent to a person skilled in the art, the optimal rate of cross-linking need to be determined on a case by case basis. This can be achieved by methods well known in the art, some of which are exemplary described below.

A sufficient rate of cross-linking can be checked f.e. by analysing the cross-linked complex vs. a non-cross-linked complex on a denaturating Protein-Gel.
If cross-linking has been performed successfully, the proteins of the complex are expected to be found in the same lane, whereas the proteins of the non-cross-linked complex are expected to be separated according to their individual characteristics. Optionally the presence of all proteins of the complex can be further checked by peptide-sequencing of proteins in the respective bands using methods well known in the art such as Mass-spectrometry and/or Edman degradation.

In addition, a rate of crosslinking which is too high should also be avoided. If cross-linking has been carried out too extensively, there will be an increasing amount of cross-linking of the individual protein complex, which potentially interferes with a screening for potential binding partners and/or modulators etc. using the arrays.
The presence of such structures can be determined by methods well known in the art and include f.e. gel-filtration experiments comparing the gel filtration profile solutions containing cross-linked complexes vs. uncross-linked complexes.

Optionally, functional assays as will be apparent to a person skilled in the art, some of which are exemplarily provided herein, can be performed to check the integrity of the complex.
Alternatively, members of the protein can be expressed as a single fusion protein and coupled to the matrix as will be apparent to a person skilled in the art.

Optionally, the attachment of the complex or proteins or antibody as outlined above can be further monitored by various methods apparent to a person skilled in the art. Those include, but are not limited to surface plasmon resonance (see f.e. McDonnel JM (2001) Curr Opin Chem Biol 5: 572-7; Lee KH (2001) Trends Biotechnol 19: 217-22; Weinberger SR, Morris TS, Pawlak M (2000) 1 (395-416; Pearson JE, Gill A, Vadagma P (2000) Ann Clin Biochem 37: 119-45; Vely F, Trautmann A, Vivier E (2000) Methods Mol Biol 121: 313-21; Slepak VZ (2000) J. Mol Recognti 13: 20-6)

Exemplary assays useful for measuring the ABIN-2 binding activity of the ABIN-2 protein-complex include but are not limited to those described in Van Huffel S et al., 2001, J Biol Chem, 276:30216-23.

Exemplary assays useful for measuring the receptor activity of the TNFR1-protein-complex include but are not limited to those described in Chan F K et al., 2000, Science, 288:2351-4.

Exemplary assays useful for measuring the in vitro ubiquitination activity of the c-IAP 1 protein-complex include but are not limited to those described in Li Xiaoming et al., 2002, Nature, 416:345-7.

Exemplary assays useful for measuring the induction of cell death activity of the c-IAP 1 protein-complex include but are not limited to those described in Li Xiaoming et al., 2002, Nature, 416:345-7.

Exemplary assays useful for measuring the apoptosis activity of the TRADD protein-complex include but are not limited to those described in Hsu H et al., 1996, Cell, 84:299-308.

Exemplary assays useful for measuring the apoptosis activity of the FADD protein-complex include but are not limited to those described in Hsu H et al., 1996, Cell, 84:299-308.

Exemplary assays useful for measuring the in vitro kinase activity of the RIP3 protein-complex include but are not limited to those described in McCarthy J V et al., 1998, J Biol Chem, 273:16968-75.

Exemplary assays useful for measuring the apoptosis activity of the RIP3 protein-complex include but are not limited to those described in Sun X et al., 1999, J Biol Chem, 274:16871-5.

Exemplary assays useful for measuring the apoptosis activity of the RIP2 protein-complex include but are not limited to those described in McCarthy J V et al., 1998, J Biol Chem, 273:16968-75.

Exemplary assays useful for measuring the in vitro kinase activity of the RIP2 protein-complex include but are not limited to those described in McCarthy J V et al., 1998, J Biol Chem, 273:16968-75.

Exemplary assays useful for measuring the in vitro kinase activity of the RIP protein-complex include but are not limited to those described in Hsu H et al., 1996, Immunity, 4:387-96.

Exemplary assays useful for measuring the apoptosis activity of the RIP protein-complex include but are not limited to those described in Hsu H et al., 1996, Immunity, 4:387-96.

Exemplary assays useful for measuring the MEKK1 activity of the MEKK1 protein-complex include but are not limited to those described in Cardone M H et al., 1997, Cell, 90:315-23.

Exemplary assays useful for measuring the ubiquitination activity of the MEKK1 protein-complex include but are not limited to those described in Lu Zhimin et al., 2002, Mol Cell, 9:945-56.

Exemplary assays useful for measuring the apoptosis activity of the MEKK1 protein-complex include but are not limited to those described in Hsu H et al., 1996, Cell, 84:299-308.

Exemplary assays useful for measuring the kinase activity of the MEKK3 protein-complex include but are not limited to those described in Yang J et al., 2001, Nat Immunol 2:620-4.

Exemplary assays useful for measuring the TAK1 kinase activity of the TAK1 protein-complex include but are not limited to those described in Wang C et al., 2001, Nature, 412:346-51.

Exemplary assays useful for measuring the I-TRAF binding activity of the I-TRAF protein-complex include but are not limited to those described in Pomerantz J L and ., 1999, EMBO J, 18:6694-704.

Exemplary assays useful for measuring the ubiquitination activity of the TRAF6 protein-complex include but are not limited to those described in Deng L et al., 2000, Cell, 103:351-61.

Exemplary assays useful for measuring the IKK kinase activity of the TRAF6 protein-complex include but are not limited to those described in Regnier C H et al., 1997, Cell, 90:373-83.

Exemplary assays useful for measuring the deubiquitination activity of the IκB ε protein-complex include but are not limited to those described in Strayhorn W David et al., 2002, Arch Biochem Biophys, 400:76-84.

Exemplary assays useful for measuring the deubiquitination activity of the IκB β protein-complex include but are not limited to those described in Strayhorn W David et al., 2002, Arch Biochem Biophys, 400:76-84.

Exemplary assays useful for measuring the nuclear import activity of the IκB α protein-complex include but are not limited to those described in Turpin P et al., 1999, J Biol Chem, 274:6804-12.

Exemplary assays useful for measuring the deubiquitination activity of the IκB α protein-complex include but are not limited to those described in Strayhorn W David et al., 2002, Arch Biochem Biophys, 400:76-84.

Exemplary assays useful for measuring the processing activity of the p105 protein-complex include but are not limited to those described in Salmeron A et al., 2001, J Biol Chem, 276:22215-22.

Exemplary assays useful for measuring the degradation activity of the p105 protein-complex include but are not limited to those described in Salmeron A et al., 2001, J Biol Chem, 276:22215-22.

Exemplary assays useful for measuring the in vitro kinase activity of the p38 protein-complex include but are not limited to those described in Ge Baoxue et al., 2002, Science, 295:1291-4.

Exemplary assays useful for measuring the IKK kinase activity of the IKK γ protein-complex include but are not limited to those described in Regnier C H et al., 1997, Cell, 90:373-83.

Exemplary assays useful for measuring the IKK recruitment to TNFR1 of the IKK γ protein-complex include but are not limited to those described in Zhang S Q et al., 2000, Immunity, 12:301-11.

Exemplary assays useful for measuring the IKK kinase activity of the IKK α protein-complex include but are not limited to those described in Regnier C H et al., 1997, Cell, 90:373-83.

Exemplary assays useful for measuring the IKK recruitment to TNFR1 of the IKK α protein-complex include but are not limited to those described in Zhang S Q et al., 2000, Immunity, 12:301-11.

Exemplary assays useful for measuring the DNA binding activity of the p50 protein-complex include but are not limited to those described in Yeh W C et al., 1997, Immunity, 7:715-25.

Exemplary assays useful for measuring the DNA binding activity of the p65 NF-κB protein-complex include but are not limited to those described in Yeh W C et al., 1997, Immunity, 7:715-25.

Exemplary assays useful for measuring the receptor activity of the TNFR2 protein-complex include but are not limited to those described in Chan F K et al., 2000, Science, 288:2351-4

### 4.6.1. CANDIDATE MOLECULES

Any molecule known in the art can be tested for its ability to modulate (increase or decrease) the amount of, activity of, or protein component composition of a complex of the present invention as detected by a change in the amount of, activity of, or protein component composition of, said complex. By way of example, a change in the amount of the complex can be detected by detecting a change in the amount of the complex that can be isolated from a cell expressing the complex machinery. For identifying a molecule that modulates complex activity, candidate molecules can be directly provided to a cell expressing the complex machinery, or, in the case of candidate proteins, can be provided by providing their encoding nucleic acids under conditions in which the nucleic acids are recombinantly expressed to produce the candidate proteins within the cell expressing the complex machinery, the complex is then isolated from the cell and the isolated complex is assayed for activity using methods well known in the art, not limited to those described, supra.

This embodiment of the invention is well suited to screen chemical libraries for molecules which modulate, e.g., inhibit, antagonize, or agonize, the amount of, activity of, or protein component composition of the complex. The chemical libraries can be peptide libraries, peptidomimetic libraries, chemically synthesized libraries, recombinant, e.g., phage display libraries, and in vitro translation-based libraries, other non-peptide synthetic organic libraries, etc.

Exemplary libraries are commercially available from several sources (ArQule, Tripos/PanLabs, ChemDesign, Pharmacopoeia). In some cases, these chemical libraries are generated using combinatorial strategies that encode the identity of each member of the library on a substrate to which the member compound is attached, thus allowing direct and immediate identification of a molecule that is an effective modulator. Thus, in many combinatorial approaches, the position on a plate of a compound specifies that compound's composition. Also, in one example, a single plate position may have from 1-20 chemicals that can be screened by administration to a well containing the interactions of interest. Thus, if modulation is detected, smaller and smaller pools of interacting pairs can be assayed for the modulation activity. By such methods, many candidate molecules can be screened.

Many diversity libraries suitable for use are known in the art and can be used to provide compounds to be tested according to the present invention. Alternatively, libraries can be constructed using standard methods. Chemical (synthetic) libraries, recombinant expression libraries, or polysome-based libraries are exemplary types of libraries that can be used.

The libraries can be constrained or semirigid (having some degree of structural rigidity), or linear or nonconstrained. The library can be a cDNA or genomic expression library, random peptide expression library or a chemically synthesized random peptide library, or non-peptide library. Expression libraries are introduced into the cells in which the assay occurs, where the nucleic acids of the library are expressed to produce their encoded proteins.

In one embodiment, peptide libraries that can be used in the present invention may be libraries that are chemically synthesized in vitro. Examples of such libraries are given in Houghten et al., 1991, Nature 354:84-86, which describes mixtures of free hexapeptides in which the first and second residues in each peptide were individually and specifically defined; Lam et al., 1991, Nature 354:82-84, which describes a "one bead, one peptide" approach in which a solid phase split synthesis scheme produced a library of peptides in which each bead in the collection had immobilized thereon a single, random sequence of amino acid residues; Medynski, 1994, Bio/Technology 12:709-710, which describes split synthesis and T-bag synthesis methods; and Gallop et al., 1994, J. Medicinal Chemistry 37(9):1233-1251. Simply by way of other examples, a combinatorial library may be prepared for use, according to the methods of Ohlmeyer et al., 1993, Proc. Natl. Acad. Sci. USA 90:10922-10926; Erb et al., 1994, Proc. Natl. Acad. Sci. USA 91:11422-11426; Houghten et al., 1992, Biotechniques 13:412; Jayawickreme et al., 1994, Proc. Natl. Acad. Sci. USA 91:1614-1618; or Salmon et al., 1993, Proc. Natl. Acad. Sci. USA 90:11708-11712. PCT Publication No. WO 93/20242 and Brenner and Lerner, 1992, Proc. Natl. Acad. Sci. USA 89:5381-5383 describe "encoded combinatorial chemical libraries," that contain oligonucleotide identifiers for each chemical polymer library member.

In a preferred embodiment, the library screened is a biological expression library that is a random peptide phage display library, where the random peptides are constrained (e.g., by virtue of having disulfide bonding).

Further, more general, structurally constrained, organic diversity (e.g., nonpeptide) libraries, can also be used. By way of example, a benzodiazepine library (see e.g., Bunin et al., 1994, Proc. Natl. Acad. Sci. USA 91:4708-4712) may be used.

Conformationally constrained libraries that can be used include but are not limited to those containing invariant cysteine residues which, in an oxidizing environment, cross-link by disulfide bonds to form cystines, modified peptides (e.g., incorporating fluorine, metals, isotopic labels, are phosphorylated, etc.), peptides containing one or more non-naturally occurring amino acids, non-peptide structures, and peptides containing a significant fraction of -carboxyglutamic acid.

Libraries of non-peptides, e.g., peptide derivatives (for example, that contain one or more non-naturally occurring amino acids) can also be used. One example of these are peptoid libraries (Simon et al., 1992, Proc. Natl. Acad. Sci. USA 89:9367-9371). Peptoids are polymers of non-natural amino acids that have naturally occurring side chains attached not to the α carbon but to the backbone amino nitrogen. Since peptoids are not easily degraded by human digestive enzymes, they are advantageously more easily adaptable to drug use. Another example of a library that can be used, in which the amide functionalities in peptides have been permethylated to generate a chemically transformed combinatorial library, is described by Ostresh et al., 1994, Proc. Natl. Acad. Sci. USA 91:11138-11142).

The members of the peptide libraries that can be screened according to the invention are not limited to containing the 20 naturally occurring amino acids. In particular, chemically synthesized libraries and polysome based libraries allow the use of amino acids in addition to the 20 naturally occurring amino acids (by their inclusion in the precursor pool of amino acids used in library production). In specific embodiments, the library members contain one or more non-natural or non-classical amino acids or cyclic peptides. Non-classical amino acids include but are not limited to the D-isomers of the common amino acids, -amino isobutyric acid, 4-aminobutyric acid, Abu, 2-amino butyric acid; -Abu,.-Ahx, 6-amino hexanoic acid; Aib, 2-amino isobutyric acid; 3-amino propionic acid; ornithine; norleucine; norvaline, hydroxyproline, sarcosine, citrulline, cysteic acid, t-butylglycine, t-butylalanine, phenylglycine, cyclohexylalanine, β-alanine, designer amino acids such as β-methyl amino acids, C-methyl amino acids, N-methyl amino acids, fluoro-amino acids and amino acid analogs in general. Furthermore, the amino acid can be D (dextrorotary) or L (levorotary).

In a specific embodiment, fragments and/or analogs of complexes of the invention, or protein components thereof, especially peptidomimetics, are screened for activity as competitive or non-competitive inhibitors of complex activity or formation.

In another embodiment of the present invention, combinatorial chemistry can be used to identify modulators of a the complexes. Combinatorial chemistry is capable of creating libraries containing hundreds of thousands of compounds, many of which may be structurally similar. While high throughput screening programs are capable of screening these vast libraries for affinity for known targets, new approaches have been developed that achieve libraries of smaller dimension but which provide maximum chemical diversity. (See e.g., Matter, 1997, Journal of Medicinal Chemistry 40:1219-1229).

One method of combinatorial chemistry, affinity fingerprinting, has previously been used to test a discrete library of small molecules for binding affinities for a defined panel of proteins. The fingerprints obtained by the screen are used to predict the affinity of the individual library members for other proteins or receptors of interest (in the instant invention, the protein complexes of the present invention and protein components thereof.) The fingerprints are compared with fingerprints obtained from other compounds known to react with the protein of interest to predict whether the library compound might similarly react. For example, rather than testing every ligand in a large library for interaction with a complex or protein component, only those ligands having a fingerprint similar to other compounds known to have that activity could be tested. (See, e.g., Kauvar et al., 1995, Chemistry and Biology 2:107-118; Kauvar, 1995, Affinity fingerprinting, Pharmaceutical Manufacturing International. 8:25-28; and Kauvar, Toxic-Chemical Detection by Pattern Recognition in New Frontiers in Agrochemical Immunoassay, D. Kurtz. L. Stanker and J.H. Skerritt. Editors, 1995, AOAC: Washington, D.C., 305-312).

Kay et al., 1993, Gene 128:59-65 (Kay) discloses a method of constructing peptide libraries that encode peptides of totally random sequence that are longer than those of any prior conventional libraries. The libraries disclosed in Kay encode totally synthetic random peptides of greater than about 20 amino acids in length. Such libraries can be advantageously screened to identify complex modulators. (See also U.S. Patent No. 5,498,538 dated March 12, 1996; and PCT Publication No. WO 94/18318 dated August 18, 1994).

A comprehensive review of various types of peptide libraries can be found in Gallop et al., 1994, J. Med. Chem. 37:1233-1251.

### 4.7. PHARMACEUTICAL COMPOSITIONS AND THERAPEUTIC/PROPHYLACTIC ADMINISTRATION

The invention provides methods of treatment (and prophylaxis) by administration to a subject of an effective amount of a Therapeutic of the invention. In a preferred aspect, the Therapeutic is substantially purified. The subject is preferably an animal including, but not limited to animals such as cows, pigs, horses, chickens, cats, dogs, etc., and is preferably a mammal, and most preferably human. In a specific embodiment, a non-human mammal is the subject.

Various delivery systems are known and can be used to administer a Therapeutic of the invention, e.g., encapsulation in liposomes, microparticles, and microcapsules: use of recombinant cells capable of expressing the Therapeutic, use of receptor-mediated endocytosis (e.g., Wu and Wu, 1987, J. Biol. Chem. 262:4429-4432); construction of a Therapeutic nucleic acid as part of a retroviral or other vector, etc. Methods of introduction include but are not limited to intradermal, intramuscular, intraperitoneal, intravenous, subcutaneous, intranasal, epidural, and oral routes. The compounds may be administered by any convenient route, for example by infusion, by bolus injection, by absorption through epithelial or mucocutaneous linings (e.g., oral, rectal and intestinal mucosa, etc.), and may be administered together with other biologically active agents. Administration can be systemic or local. In addition, it may be desirable to introduce the pharmaceutical compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

In a specific embodiment, it may be desirable to administer the pharmaceutical compositions of the invention locally to the area in need of treatment. This may be achieved by, for example, and not by way of limitation, local infusion during surgery, topical application, e.g., in conjunction with a wound dressing after surgery, by injection, by means of a catheter, by means of a suppository, or by means of an implant, said implant being of a porous, non-porous, or gelatinous material, including membranes, such as sialastic membranes, or fibers. In one embodiment, administration can be by direct injection at the site (or former site) of a malignant tumor or neoplastic or pre-neoplastic tissue.

In another embodiment, the Therapeutic can be delivered in a vesicle, in particular a liposome (Langer, 1990, Science 249:1527-1533; Treat et al., 1989, In: Liposomes in the Therapy of Infectious Disease and Cancer, Lopez-Berestein and Fidler, eds., Liss, New York, pp. 353-365; Lopez-Berestein, ibid., pp. 317-327; see generally ibid.)

In yet another embodiment, the Therapeutic can be delivered via a controlled release system. In one embodiment, a pump may be used (Langer, supra; Sefton, 1987, CRC Crit. Ref. Biomed. Eng. 14:201-240; Buchwald et al., 1980, Surgery 88:507-516; Saudek et al., 1989, N. Engl. J. Med. 321:574-579). In another embodiment, polymeric materials can be used (Medical Applications of Controlled Release, Langer and Wise, eds., CRC Press, Boca Raton, Florida, 1974; Controlled Drug Bioavailability, Drug Product Design and Performance, Smolen and Ball, eds., Wiley, New York, 1984; Ranger and Peppas, 1983, Macromol. Sci. Rev. Macromol. Chem. 23:61; Levy et al., 1985, Science 228:190-192; During et al., 1989, Ann. Neurol. 25:351-356; Howard et al., 1989, J. Neurosurg. 71:858-863). In yet another embodiment, a controlled release system can be placed in proximity of the therapeutic target, i.e., the brain, thus requiring only a fraction of the systemic dose (e.g., Goodson, 1984, In: Medical Applications of Controlled Release, supra, Vol. 2, pp. 115-138). Other controlled release systems are discussed in the review by Langer (1990, Science 249:1527-1533).

In a specific embodiment where the Therapeutic is a nucleic acid encoding a protein Therapeutic, the nucleic acid can be administered in vivo to promote expression of its encoded protein, by constructing it as part of an appropriate nucleic acid expression vector and administering it so that it becomes intracellular, e.g., by use of a retroviral vector (U.S. Patent No. 4,980,286), or by direct injection, or by use of microparticle bombardment (e.g., a gene gun; Biolistic, Dupont), or by coating it with lipids, cell-surface receptors or transfecting agents, or by administering it in linkage to a homeobox-like peptide which is known to enter the nucleus (e.g., Joliot et al., 1991, Proc. Natl. Acad. Sci. USA 88:1864-1868), etc. Alternatively, a nucleic acid Therapeutic can be introduced intracellularly and incorporated by homologous recombination within host cell DNA for expression.

The present invention also provides pharmaceutical compositions. Such compositions comprise a therapeutically effective amount of a Therapeutic, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly, in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, including but not limited to peanut oil, soybean oil, mineral oil, sesame oil and the like. Water is a preferred carrier when the pharmaceutical composition is administered orally. Saline and aqueous dextrose are preferred carriers when the pharmaceutical composition is administered intravenously. Saline solutions and aqueous dextrose and glycerol solutions are preferably employed as liquid carriers for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsions, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the Therapeutic, preferably in purified form, together with a suitable amount of carrier so as to provide the form for proper administration to the patient. The formulation should suit the mode of administration.

In a preferred embodiment, the composition is formulated, in accordance with routine procedures, as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water-free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water or saline for injection can be provided so that the ingredients may be mixed prior to administration.

The Therapeutics of the invention can be formulated as neutral or salt forms. Pharmaceutically acceptable salts include those formed with free carboxyl groups such as those derived from hydrochloric, phosphoric, acetic, oxalic, tartaric acids, etc., those formed with free amine groups such as those derived from isopropylamine, triethylamine, 2-ethylamino ethanol, histidine, procaine, etc., and those derived from sodium, potassium, ammonium, calcium, and ferric hydroxides, etc.

The amount of the Therapeutic of the invention which will be effective in the treatment of a particular disorder or condition will depend on the nature of the disorder or condition, and can be determined by standard clinical techniques. In addition, in vitro assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. However, suitable dosage ranges for intravenous administration are generally about 20-500 micrograms of active compound per kilogram body weight. Suitable dosage ranges for intranasal administration are generally about 0.01 pg/kg body weight to 1 mg/kg body weight. Effective doses may be extrapolated from dose-response curves derived from in vitro or animal model test systems.

Suppositories generally contain active ingredient in the range of 0.5% to 10% by weight; oral formulations preferably contain 10% to 95% active ingredient.

The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

The invention also provides a pharmaceutical pack or kit comprising one or more containers filled with one or more of the ingredients of the pharmaceutical compositions of the invention. For example, the kit can comprise in one or more containers a first protein, or a functionally active fragment or functionally active derivative thereof, which first protein is selected from the group consisting of proteins listed in the third column of table 1; and a second protein, or a functionally active fragment or functionally active derivative thereof, which second protein is selected from the group consisting of proteins listed in the forth column of table 1.
Alternatively, the kit can comprise in one or more containers, all proteins, functionally active fragments or functionally active derivatives thereof of from the group of proteins in the fifth column of table 1.

The kits of the present invention can also contain expression vectors encoding the essential components of the complex machinery, which components after being expressed can be reconstituted in order to form a biologically active complex. Such a kit preferably also contains the required buffers and reagents. Optionally associated with such container(s) can be instructions for use of the kit and/or a notice in the form prescribed by a governmental agency regulating the manufacture, use or sale of pharmaceuticals or biological products, which notice reflects approval by the agency of manufacture, use or sale for human administration.

### 4.8 ANIMAL MODELS

The present invention also provides animal models. In one embodiment, animal models for diseases and disorders involving the protein complexes of the present invention are provided. These animal models are well known in the art. These animal models include, but are not limited to those which are listed in the section 4.6 (supra) as exemplary animald models to study any of the complexes provided in the invention. Such animals can be initially produced by promoting homologous recombination or insertional mutagenesis between genes encoding the protein components of the complexes in the chromosome, and exogenous genes encoding the protein components of the complexes that have been rendered biologically inactive or deleted (preferably by insertion of a heterologous sequence, e.g., an antibiotic resistance gene). In a preferred aspect, homologous recombination is carried out by transforming embryo-derived stem (ES) cells with one or more vectors containing one or more insertionally inactivated genes, such that homologous recombination occurs, followed by injecting the transformed ES cells into a blastocyst, and implanting the blastocyst into a foster mother, followed by the birth of the chimeric animal ("knockout animal") in which a gene encoding a component protein from the third column of table 1, or a functionally active fragment or functionally active derivative thereof, and a gene encoding a component protein from the forth column of table 1, or a functionally active fragment or functionally active derivative thereof, has been inactivated or deleted (Capecchi, 1989, Science 244:1288-1292)..
In another preferred aspect, homologous recombination is carried out by transforming embryo-derived stem (ES) cells with one or more vectors containing one or more insertionally inactivated genes, such that homologous recombination occurs, followed by injecting the transformed ES cells into a blastocyst, and implanting the blastocyst into a foster mother, followed by the birth of the chimeric animal ("knockout animal") in which the genes of all component proteins from the group of proteins listed in the third column of table 1 or of all proteins from the group of proteins listed in the forth column of table 1 have been inactivated or deleted.

The chimeric animal can be bred to produce additional knockout animals. Such animals can be mice, hamsters, sheep, pigs, cattle, etc., and are preferably non-human mammals. In a specific embodiment, a knockout mouse is produced.

Such knockout animals are expected to develop, or be predisposed to developing, diseases or disorders associated with mutations involving the protein complexes of the present invention, and thus, can have use as animal models of such diseases and disorders, e.g., to screen for or test molecules (e.g., potential Therapeutics) for such diseases and disorders.

In a different embodiment of the invention, transgenic animals that have incorporated and express (or over-express or mis-express) a functional gene encoding a protein component of the complex, e.g. by introducing the a gene encoding one or more of the components of the complex under the control of a heterologous promoter (i.e., a promoter that is not the native promoter of the gene) that either over-expresses the protein or proteins, or expresses them in tissues not normally expressing the complexes or proteins, can have use as animal models of diseases and disorders characterized by elevated levels of the protein complexes. Such animals can be used to screen or test molecules for the ability to treat or prevent the diseases and disorders cited supra.

In one embodiment, the present invention provides a recombinant non-human animal in which an endogenous gene encoding a first protein, or a functionally active fragment or functionally active derivative thereof, which first protein is selected from the group of proteins listed in the third column of table 1, and and endogenous gene encoding a second protein, or a functionally active fragment or functionally active derivative thereof, which second protein is selected from the group consisting of proteins listed in the forth column of table 1 has been deleted or inactivated by homologous recombination or insertional mutagenesis of said animal or an ancestor thereof. In addition, the present invention provides a recombinant non-human animal in which the endogenous genes of all proteins, or functionally active fragments or functionally active derivatives thereof of one of the group of proteins listed in the fifth column have been deleted or inactivated by homologous recombination or insertional mutagenesis of said animal or an ancestor thereof:

In another embodiment, the present invention provides a recombinant non-human animal in which an endogenous gene encoding a first protein, or a functionally active fragment or functionally active derivative thereof, which first protein is selected from the group consisting of proteins of the third column of table 1, and endogenous gene encoding a second protein, or a functionally active fragment or functionally active derivative thereof, which second protein is selected from the group consisting of proteins of the fourth column, of table 1 are recombinantly expressed in said animal or an ancestor thereof.

The following series of examples are presented by way of illustration and not by way of limitation on the scope of the invention.

### EXAMPLES

An object of the present invention was to identify protein complexes of the Tumor necrosis factor-α-(TNF-α) signalling pathway, component proteins of the said complexes, fragments and derivatives of the component proteins, and antibodies specific to the complexes. The present invention also relates to methods for use of the complexes of the TNF-α signalling pathway and their interaction in, inter alia, screening, diagnosis, and therapy, as well as to methods of preparing the complexes.

By applying the process according to the invention said complexes were identified. The components are listed in table 1.
Those complexes are, as called herein, the following complexes:
ABIN-2 protein complex, TNRF1 protein complex, TNFR2 protein complex, ReIA (p65) protein complex, p50 (NF-κB1 ) protein complex, IKK-α protein complex, IKK-γ protein complex, p38 protein complex, p105 (NF-κB1) protein complex, IκBα protein complex, IκBβ protein complex, IκBε protein complex, TRAF6 protein complex, I-TRAF protein complex, MEKK3 protein complex, TAK1 protein complex, MEKK1 protein complex, RIP protein complex, RIP2 protein complex, RIP3 protein complex, TRADD protein complex, FADD protein complex, c-IAP1 protein complex.

Furthermore, the interactions between the protein complexes of the TNF-α-signalling pathway and the differential assembly of the complexes in different cellular conditions provide new insights into the mechanisms of the physiological and pathological processes associated with this biological pathway.

Said object is further achieved by the characterisation of component proteins. These proteins are listed in table 2
Thus, the invention relates to the following embodiments:
Thus the invention relates to the ABIN-2-protein complex
1. A protein complex selected from complex (I) and comprising
   (a) at least one first protein selected from the group consisting of:
      (i) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions, and
   (b) at least one second protein, which second protein is selected from the group consisting of:
      (i) "TIAF1" (SEQ ID No:2) or a functionally active derivative thereof, or a functionally active fragment thereof, or a homolog thereof, or a variant of "TIAF1" encoded by a nucleic acid that hybridizes to the "TIAF1" nucleic acid under low stringency conditions,
      (ii) "LCK" (SEQ ID No:3) or a functionally active derivative thereof, or a functionally active fragment thereof, or a homolog thereof, or a variant of "LCK" encoded by a nucleic acid that hybridizes to the "LCK" nucleic acid under low stringency conditions,
      (iii) "KIAA0564 PROTEIN" (SEQ ID No:4) or a functionally active derivative thereof, or a functionally active fragment thereof, or a homolog thereof, or a variant of "KIAA0564 PROTEIN" encoded by a nucleic acid that hybridizes to the "KIAA0564 PROTEIN" nucleic acid under low stringency conditions,
      (iv) "UBIQUITIN-PROTEIN LIGASE E3A" (SEQ ID No:5) or a functionally active derivative thereof, or a functionally active fragment thereof, or a homolog thereof, or a variant of "UBIQUITIN-PROTEIN LIGASE E3A" encoded by a nucleic acid that hybridizes to the "UBIQUITIN-PROTEIN LIGASE E3A" nucleic acid under low stringency conditions,
      (v) "TYROSINE-PROTEIN KINASE CSK" (SEQ ID No:6) or a functionally active derivative thereof, or a functionally active fragment thereof, or a homolog thereof, or a variant of "TYROSINE-PROTEIN KINASE CSK" encoded by a nucleic acid that hybridizes to the "TYROSINE-PROTEIN KINASE CSK" nucleic acid under low stringency conditions,
      (vi) "INOSINE-5'-MONOPHOSPHATE DEHYDROGENASE 2" (SEQ ID No:7) or a functionally active derivative thereof, or a functionally active fragment thereof, or a homolog thereof, or a variant of "INOSINE-5'-MONOPHOSPHATE DEHYDROGENASE 2" encoded by a nucleic acid that hybridizes to the "INOSINE-5'-MONOPHOSPHATE DEHYDROGENASE 2" nucleic acid under low stringency conditions,
      (vii) "BAG-FAMILY MOLECULAR CHAPERONE REGULATOR-4" (SEQ ID No:8) or a functionally active derivative thereof, or a functionally active fragment thereof, or a homolog thereof, or a variant of "BAG-FAMILY MOLECULAR CHAPERONE REGULATOR-4" encoded by a nucleic acid that hybridizes to the "BAG-FAMILY MOLECULAR CHAPERONE REGULATOR-4" nucleic acid under low stringency conditions, and
      (viii) "HSP 90-B 1"(SEQ ID No:9) or a functionally active derivative thereof, or a functionally active fragment thereof, or a homolog thereof, or a variant of "HSP 90-B 1 "encoded by a nucleic acid that hybridizes to the "HSP 90-B 1 "nucleic acid under low stringency conditions;
   and a complex (II) comprising at least two of said second proteins,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
2. The protein complex according to claim 1 wherein the first protein is the protein "ABIN-2" (xSEQ ID NO. 1), or a functionally active derivative thereof, or a functionally active fragment thereof, or a homolog thereof, or a variant of "ABIN-2" encoded by a nucleic acid that hybridizes to the "ABIN-2" nucleic acid under low stringency conditions.
3. The protein complex according to No. 1 selected from complex (I) and comprising the following proteins:
   (i) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions,
   (ii) "LCK" (SEQ ID No:3) or a functionally active derivative thereof, or a functionally active fragment thereof, or a homolog thereof, or a variant of "LCK" encoded by a nucleic acid that hybridizes to the "LCK" nucleic acid under low stringency conditions,
   (iii) "KIAA0564 PROTEIN" (SEQ ID No:4) or a functionally active derivative thereof, or a functionally active fragment thereof, or a homolog thereof, or a variant of "KIAA0564 PROTEIN" encoded by a nucleic acid that hybridizes to the "KIAA0564 PROTEIN" nucleic acid under low stringency conditions,
   (iv) "HSP90 β-1" (SEQ ID No:9) or a functionally active derivative thereof, or a functionally active fragment thereof, or a homolog thereof, or a variant of "HSP90 β-1" encoded by a nucleic acid that hybridizes to the "HSP90 β-1" nucleic acid under low stringency conditions;
   and a protein complex selected from complex (II) and comprising the following proteins:
   (i) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions,
   (ii) "LCK" (SEQ ID No:3) or a functionally active derivative thereof, or a functionally active fragment thereof, or a homolog thereof, or a variant of "LCK" encoded by a nucleic acid that hybridizes to the "LCK" nucleic acid under low stringency conditions,
   (iii) "KIAA0564 PROTEIN" (SEQ ID No:4) or a functionally active derivative thereof, or a functionally active fragment thereof, or a homolog thereof, or a variant of "KIAA0564 PROTEIN" encoded by a nucleic acid that hybridizes to the "KIAA0564 PROTEIN" nucleic acid under low stringency conditions,
   (iv) "INOSINE-5'-MONOPHOSPHATE DEHYDROGENASE 2" (SEQ ID No:7) or a functionally active derivative thereof, or a functionally active fragment thereof, or a homolog thereof, or a variant of "INOSINE-5'-MONOPHOSPHATE DEHYDROGENASE 2" encoded by a nucleic acid that hybridizes to the "INOSINE-5'-MONOPHOSPHATE DEHYDROGENASE 2" nucleic acid under low stringency conditions,
   (v) "BAG-FAMILY MOLECULAR CHAPERONE REGULATOR-4" (SEQ ID No:8) or a functionally active derivative thereof, or a functionally active fragment thereof, or a homolog thereof, or a variant of "BAG-FAMILY MOLECULAR CHAPERONE REGULATOR-4" encoded by a nucleic acid that hybridizes to the "BAG-FAMILY MOLECULAR CHAPERONE REGULATOR-4" nucleic acid under low stringency conditions,
   (vi) "HSP90 β-1" (SEQ ID No:9) or a functionally active derivative thereof, or a functionally active fragment thereof, or a homolog thereof, or a variant of "HSP90 B-1" encoded by a nucleic acid that hybridizes to the "HSP90 β-1" nucleic acid under low stringency conditions;
   and a protein complex selected from complex (III) and comprising the following proteins:
   (i) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions,
   (ii) "TIAF1" (SEQ ID No:2) or a functionally active derivative thereof, or a functionally active fragment thereof, or a homolog thereof, or a variant of "TIAF1" encoded by a nucleic acid that hybridizes to the "TIAF1" nucleic acid under low stringency conditions,
   (iii) "LCK" (SEQ ID No:3) or a functionally active derivative thereof, or a functionally active fragment thereof, or a homolog thereof, or a variant of "LCK" encoded by a nucleic acid that hybridizes to the "LCK" nucleic acid under low stringency conditions,
   (iv) "KIAA0564 PROTEIN" (SEQ ID No:4) or a functionally active derivative thereof, or a functionally active fragment thereof, or a homolog thereof, or a variant of "KIAA0564 PROTEIN" encoded by a nucleic acid that hybridizes to the "KIAA0564 PROTEIN" nucleic acid under low stringency conditions,
   (v) "UBIQUITIN-PROTEIN LIGASE E3A" (SEQ ID No:5) or a functionally active derivative thereof, or a functionally active fragment thereof, or a homolog thereof, or a variant of "UBIQUITIN-PROTEIN LIGASE E3A" encoded by a nucleic acid that hybridizes to the "UBIQUITIN-PROTEIN LIGASE E3A" nucleic acid under low stringency conditions,
   (vi) "TYROSINE-PROTEIN KINASE CSK" (SEQ ID No:6) or a functionally active derivative thereof, or a functionally active fragment thereof, or a homolog thereof, or a variant of "TYROSINE-PROTEIN KINASE CSK" encoded by a nucleic acid that hybridizes to the "TYROSINE-PROTEIN KINASE CSK" nucleic acid under low stringency conditions, and
   (vii) "HSP90 β-1" (SEQ to No:9) or a functionally active derivative thereof, or a functionary active fragment thereof, or a homolog thereof, or a variant of "HSP90 β-1" encoded by a nucleic acid that hybridizes to the "HSP90 β-1" nucleic acid under low stringency conditions,
   and protein complex selected from complex (IV) and comprising the following proteins:
   (i) "ABIN-2" (SEQ ID No:1 ) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions,
   (ii) "TIAF1" (SEQ ID No:2) or a functionally active derivative thereof, or a functionally active fragment thereof, or a homolog thereof, or a variant of "TIAF1" encoded by a nucleic acid that hybridizes to the "TIAF1" nucleic acid under low stringency conditions,
   (iii) "LCK" (SEQ ID No:3) or a functionally active derivative thereof, or a functionally active fragment thereof, or a homolog thereof, or a variant of "LCK" encoded by a nucleic acid that hybridizes to the "LCK" nucleic acid under low stringency conditions,
   (iv) "KIAA0564 PROTEIN" (SEQ ID No:4) or a functionally active derivative thereof, or a functionally active fragment thereof, or a homolog thereof, or a variant of "KIAA0564 PROTEIN" encoded by a nucleic acid that hybridizes to the "KIAA0564 PROTEIN" nucleic acid under low stringency conditions,
   (v) "UBIQUITIN-PROTEIN LIGASE E3A" (SEQ ID No:5) or a functionally active derivative thereof, or a functionally active fragment thereof, or a homolog thereof, or a variant of "UBIQUITIN-PROTEIN LIGASE E3A" encoded by a nucleic acid that hybridizes to the "UBIQUITIN-PROTEIN LIGASE E3A" nucleic acid under low stringency conditions,
   (vi) "TYROSINE-PROTEIN KINASE CSK" (SEQ ID No:6) or a functionally active derivative thereof, or a functionally active fragment thereof, or a homolog thereof, or a variant of "TYROSINE-PROTEIN KINASE CSK" encoded by a nucleic acid that hybridizes to the "TYROSINE-PROTEIN KINASE CSK" nucleic acid under low stringency conditions,
   (vii) "INOSINE-5'-MONOPHOSPHATE DEHYDROGENASE 2" (SEQ ID No:7) or a functionally active derivative thereof, or a functionally active fragment thereof, or a homolog thereof, or a variant of "INOSINE-5'-MONOPHOSPHATE DEHYDROGENASE 2" encoded by a nucleic acid that hybridizes to the "INOSINE-5'-MONOPHOSPHATE DEHYDROGENASE 2" nucleic acid under low stringency conditions,
   (viii) "BAG-FAMILY MOLECULAR CHAPERONE REGULATOR-4" (SEQ ID No:8) or a functionally active derivative thereof, or a functionally active fragment thereof, or a homolog thereof, or a variant of "BAG-FAMILY MOLECULAR CHAPERONE REGULATOR-4" encoded by a nucleic acid that hybridizes to the "BAG-FAMILY MOLECULAR CHAPERONE REGULATOR-4" nucleic acid under low stringency conditions, and
   (ix) "HSP90 β-1" (SEQ ID No:9) or a functionally active derivative thereof, or a functionally active fragment thereof, or a homolog thereof, or a variant of "HSP90 β-1" encoded by a nucleic acid that hybridizes to the "HSP90 B-1" nucleic acid under low stringency conditions.
4. The protein complex according to No. 1 comprising all but 1 - 7 of the following proteins:
   (i) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions,
   (ii) "TIAF1" (SEQ ID No:2) or a functionally active derivative thereof, or a functionally active fragment thereof, or a homolog thereof, or a variant of "TIAF1" encoded by a nucleic acid that hybridizes to the "TIAF1" nucleic acid under low stringency conditions,
   (iii) "LCK" (SEQ ID No:3) or a functionally active derivative thereof, or a functionally active fragment thereof, or a homolog thereof, or a variant of "LCK" encoded by a nucleic acid that hybridizes to the "LCK" nucleic acid under low stringency conditions,
   (iv) "KIAA0564 PROTEIN" (SEQ ID No:4) or a functionally active derivative thereof, or a functionally active fragment thereof, or a homolog thereof, or a variant of "KIAA0564 PROTEIN" encoded by a nucleic acid that hybridizes to the "KIAA0564 PROTEIN" nucleic acid under low stringency conditions,
   (v) "UBIQUITIN-PROTEIN LIGASE E3A" (SEQ ID No:5) or a functionally active derivative thereof, or a functionally active fragment thereof, or a homolog thereof, or a variant of "UBIQUITIN-PROTEIN LIGASE E3A" encoded by a nucleic acid that hybridizes to the "UBIQUITIN-PROTEIN LIGASE E3A" nucleic acid under low stringency conditions,
   (vi) "TYROSINE-PROTEIN KINASE CSK" (SEQ ID No:6) or a functionally active derivative thereof, or a functionally active fragment thereof, or a homolog thereof, or a variant of "TYROSINE-PROTEIN KINASE CSK" encoded by a nucleic acid that hybridizes to the "TYROSINE-PROTEIN KINASE CSK" nucleic acid under low stringency conditions,
   (vii) "INOSINE-5'-MONOPHOSPHATE DEHYDROGENASE 2" (SEQ ID No:7) or a functionally active derivative thereof, or a functionally active fragment thereof, or a homolog thereof, or a variant of "INOSINE-5'-MONOPHOSPHATE DEHYDROGENASE 2" encoded by a nucleic acid that hybridizes to the "INOSINE-5'-MONOPHOSPHATE DEHYDROGENASE 2" nucleic acid under low stringency conditions,
   (viii) "BAG-FAMILY MOLECULAR CHAPERONE REGULATOR-4" (SEQ ID No:8) or a functionally active derivative thereof,or a functionally active fragement thereof, or a homolog thereof, or a variant of "BAG-FAMILY MOLECULAR CHAPERONE REGULATOR-4" that hybridizes to the "BAG-FAMILY MOLECULAR CHAPERONE REGULATOR-4" nucleic acid under low stringency conditions, and
   (ix) "HSP 90-β 1" (SEQ ID No:9) or a functionally active derivative thereof, or a functionally active fragment thereof, or a homolog thereof, or a variant of "HSP 90-β 1" encoded by a nucleic acid that hybridizes to the "HSP 90-β1" nucleic acid under low stringency conditions.
5. The complex of any of No. 1-4 comprising a functionally active derivative of said first protein and/or a functionally active derivative of said second protein, wherein the functionally active derivative is a fusion protein comprising said first protein or said second protein fused to an amino acid sequence different from the first protein or second protein, respectively.
6. The complex of No. 5 wherein the functionally active derivative is a fusion protein comprising said first protein or said second protein fused to an affinity tag or label.
7. The complex of any of No. 1 - 4 comprising a fragment of said first protein and/or a fragment of said second protein, which fragment binds to another protein component of said complex.
8. The complex of any of No. 1 -7 that is involved in the ABIN-2 binding activity.
9. A process for preparing complex of any of No. 1 - 8 and optionally the components thereof comprising the following steps:
   the expressing a protein (bait) of the complex, preferably a tagged protein, in a target cell, isolating the protein complex which is attached to the bait protein, and optionally disassociating the protein complex and isolating the individual complex members.
10. The process according to No. 9 wherein the tagged protein comprises two different tags which allow two separate affinity purification steps.
11. The process according to any of No. 9-10 wherein the two tags are separated by a cleavage site for a protease.
12. Component of the ABIN-2 complex obtainable by a process according to any of No. 9 to 11.
13. Protein of the ABIN-2 complex selected from
   (i) "KIAA0564 PROTEIN" (SEQ ID No:4) or a functionally active derivative thereof, or a functionally active fragment thereof, or a homolog thereof, or a variant of "KIAA0564 PROTEIN" encoded by a nucleic acid that hybridizes to the "KIAA0564 PROTEIN" nucleic acid under low stringency conditions,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
14. Nucleic acid encoding a protein according to No. 13.
15. Construct, preferably a vector construct, comprising (a) a nucleic acid according to No. 14 and at least one further nucleic acid which is normally not associated with said nucleic acid,or(b) at least two separate nucleic acid sequences each encoding a different protein, or a functionally active fragment or a functionally active derivative thereof at least one of said proteins, or functionally active fragments or functionally active derivative thereof being selected from the first group of proteins according to No. 1(a) and at least one of said proteins, or functionally active fragments or functionally active derivative thereof being selected from the second group of proteins according to No. 1(b).
16. Host cell, containing a vector comprising at least one of the nucleic acid of No. 14 and/or a construct of No. 15 or containing several vectors each comprising at least the nucleic acid sequence encoding at least one of the proteins, or functionally active fragments or functionally active derivatives thereof selected from the first group of proteins according to No. 1(a) and the proteins, or functionally active fragments or functionally active derivatives thereof selected from the second group of proteins according to No. 1(b).
17. An antibody or a fragment of said antibody containing the binding domain thereof, selected from an antibody or fragment thereof, which binds the complex of any of No. 1-8 and which does not bind any of the proteins of said complex when uncomplexed and an antibody or a fragment of said antibody which binds to any of the proteins according to No. 13.
18. A kit comprising in one or more container the complex of any of No. 1 - 8 and/or the proteins of No. 13optionally together with an antibody according to No. 17 and/or further components such as reagents and working instructions.
19. The kit according to No.18 for processing a substrate of said complex.
20. The kit according to No. 18 for the diagnosis or prognosis of a disease or a disease risk, preferentially for a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
21. Array, in which at least a complex according to any of No. 1 - 8 and/or at least one protein according to No. 14 and/or at least one antibody according to No. 17 is attached to a solid carrier.
22. A process for processing a physiological substrate of the complex comprising the step of bringing into contact a complex to any of No. 1 - 8 with said substrate, such that said substrate is processed.
23. A pharmaceutical composition comprising the protein complex of any of No. 1 to 8 and/or any of the following the proteins:
   (i) "KIAA0564 PROTEIN" (SEQ ID No:4) or a functionally active derivative thereof, or a functionally active fragment thereof, or a homolog thereof, or a variant of "KIAA0564 PROTEIN" encoded by a nucleic acid that hybridizes to the "KIAA0564 PROTEIN" nucleic acid under low stringency conditions, and a pharmaceutical acceptable carrier.
24. The pharmaceutical composition according to No. 23 for the treatment of diseases and disorders such as inflammation such as chronic inflammatory diseases, rheumatoid arthritis, inflammatory bowel disease; infectious diseases such as septic shock and bacterial infection; neurological diseases such as stroke-induced inflammation in neurons;neurodegenerative diseases;cancer such as prostate cancer, breast cancer and skin cancer.
25. A method for screening for a molecule that binds to the complex of anyone of No.1 - 8 and/or any of the following proteins:
   (i) "KIAA0564 PROTEIN" (SEQ ID No:4) or a functionally active derivative thereof, or a functionally active fragment thereof, or a homolog thereof, or a variant of "KIAA0564 PROTEIN" encoded by a nucleic acid that hybridizes to the "KIAA0564 PROTEIN" nucleic acid under low stringency conditions, comprising the steps of
      (a) exposing said complex, or a cell or organism containing same to one or more candidate molecules; and
      (b) determinig whether said candidate molecule is bound to the complex or protein.
26. A method for screening for a molecule that modulates directly or indirectly the function, activity, composition or formation of the complex of any one of No. 1 - 8 comprising the steps of (a) exposing said complex, or a cell or organism containing ABIN-2 complex to one or more candidate molecules; and
   (b) determining the amount of activity of protein components of, and/or intracellular localization of, said complex and/or the transcription level of a gene dependent on the complex and/or the abundance and/or activity of a protein or protein complex dependent on the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependent on the complex and/or the abundance and/or activity of a protein or protein complex dependent on the function of the complex and/or product of a gene dependent on the complex in the absence of said candidate molecules indicates that the molecule modulates function, activity or composition of said complex.
27. The method of No. 26, wherein the amount of said complex is determined.
28. The method of No. 26, wherein the activity of said complex is determined.
29. The method of No. 28, wherein said determining step comprises isolating from the cell or organism said complex to produce said isolated complex and contacting said isolated complex in the presence or absence of a candidate molecule with a substrate of said complex and determining the processing of said substrate is modified in the presence of said candidate molecule.
30. The method of No. 26, wherein the amount of the individual protein components of said complex are determined.
31. The method of No. 30, determining step comprises determining whether
   (i) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions, and/or
   (ii) "TIAF1" (SEQ ID No:2) or a functionally active derivative thereof, or a functionally active fragment thereof, or a homolog thereof, or a variant of "TIAF1" encoded by a nucleic acid that hybridizes to the "TIAF1" nucleic acid under low stringency conditions, and/or
   (iii) "LCK" (SEQ ID No:3) or a functionally active derivative thereof, or a functionally active fragment thereof, or a homolog thereof, or a variant of "LCK" encoded by a nucleic acid that hybridizes to the "LCK" nucleic acid under low stringency conditions, and/or
   (iv) "KIAA0564 PROTEIN" (SEQ ID No:4) or a functionally active derivative thereof, or a functionally active fragment thereof, or a homolog thereof, or a variant of "KIAA0564 PROTEIN" encoded by a nucleic acid that hybridizes to the "KIAA0564 PROTEIN" nucleic acid under low stringency conditions, and/or
   (v) "UBIQUITIN-PROTEIN LIGASE E3A" (SEQ ID No:5) or a functionally active derivative thereof, or a functionally active fragment thereof, or a homolog thereof, or a variant of "UBIQUITIN-PROTEIN LIGASE E3A" encoded by a nucleic acid that hybridizes to the "UBIQUITIN-PROTEIN LIGASE E3A" nucleic acid under low stringency conditions, and/or
   (vi) "TYROSINE-PROTEIN KINASE CSK" (SEQ ID No:6) or a functionally active derivative thereof, or a functionally active fragment thereof, or a homolog thereof, or a variant of "TYROSINE-PROTEIN KINASE CSK" encoded by a nucleic acid that hybridizes to the "TYROSINE-PROTEIN KINASE CSK" nucleic acid under low stringency conditions, and/or
   (vii) "INOSINE-5'-MONOPHOSPHATE DEHYDROGENASE 2" (SEQ ID No:7) or a functionally active derivative thereof, or a functionally active fragment thereof, or a homolog thereof, or a variant of "INOSINE-5'-MONOPHOSPHATE DEHYDROGENASE 2" encoded by a nucleic acid that hybridizes to the "INOSINE-5'-MONOPHOSPHATE DEHYDROGENASE 2" nucleic acid under low stringency conditions, and/or
   (viii) "BAG-FAMILY MOLECULAR CHAPERONE REGULATOR-4" (SEQ ID No:8) or a functionally active derivative thereof, or a functionally active fragment thereof, or a homolog thereof, or a variant of "BAG-FAMILY MOLECULAR CHAPERONE REGULATOR-4" encoded by a nucleic acid that hybridizes to the "BAG-FAMILY MOLECULAR CHAPERONE REGULATOR-4" nucleic acid under low stringency conditions, and/or
   (ix) "HSP 90-β 1" (SEQ ID No:9) or a functionally active derivative thereof, or a functionally active fragment thereof, or a homolog thereof, or a variant of "HSP 90-β1" encoded by a nucleic acid that hybridizes to the "HSP 90-β1" nucleic acid under low stringency conditions, is present in the complex.
32. The method of any of No. 26 to 31, wherein said method is a method of screening for a drug for treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
33. Use of a molecule that modulates the amount of, activity of, or the protein components of the complex of any one of No. 1 - 8 for the manufacture of a medicament for the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
34. A method for the production of a pharmaceutical composition comprising carrying out the method of any of No. 1 - 8 to identify a molecule that modulates the function, activity, composition or formation of said complex, and further comprising mixing the identified molecule with a pharmaceutically acceptable carrier.
35. A method for diagnosing or screening for the presence of a disease or disorder or a predisposition for developing a disease or disorder in a subject, which disease or disorder is characterized by an aberrant amount of, activity of, or component composition of, or intracellular localization of the complex of any one of the No. 1-8, comprising determining the amount of, activity of, protein components of, and/or intracellular localization of, said complex and/or the transcription level of a gene dependent on the complex and/or the abundance and/or activity of a protein or protein complex dependent on the function of the complex and/or product of a gene dependent on the complex in a comparative sample derived from a subject, wherein a difference in said amount, activity, or protein components of, said complex in an analogous sample from a subject not having the disease or disorder or predisposition indicates the presence in the subject of the disease or disorder or predisposition in the subject.
36. The method of No. 35, wherein the amount of said complex is determined.
37. The method of No. 35, wherein the activity of said complex is determined.
38. The method of No. 37, wherein said determining step comprises isolating from the subject said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
39. The method of No. 35, wherein the amount of the individual protein components of said complex are determined.
40. The method of No. 39, wherein said determining step comprises determining whether
   (i) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions, and/or
   (ii) "TIAF1" (SEQ ID No:2) or a functionally active derivative thereof, or a functionally active fragment thereof, or a homolog thereof, or a variant of "TIAF1" encoded by a nucleic acid that hybridizes to the "TIAF1" nucleic acid under low stringency conditions, and/or
   (iii) "LCK" (SEQ ID No:3) or a functionally active derivative thereof, or a functionally active fragment thereof, or a homolog thereof, or a variant of "LCK" encoded by a nucleic acid that hybridizes to the "LCK" nucleic acid under low stringency conditions, and/or
   (iv) "KIAA0564 PROTEIN" (SEQ ID No:4) or a functionally active derivative thereof, or a functionally active fragment thereof, or a homolog thereof, or a variant of "KIAA0564 PROTEIN" encoded by a nucleic acid that hybridizes to the "KIAA0564 PROTEIN" nucleic acid under low stringency conditions, and/or
   (v) "UBIQUITIN-PROTEIN LIGASE E3A" (SEQ ID No:5) or a functionally active derivative thereof, or a functionally active fragment thereof, or a homolog thereof, or a variant of "UBIQUITIN-PROTEIN LIGASE E3A" encoded by a nucleic acid that hybridizes to the "UBIQUITIN-PROTEIN LIGASE E3A" nucleic acid under low stringency conditions, and/or
   (vi) "TYROSINE-PROTEIN KINASE CSK" (SEQ ID No:6) or a functionally active derivative thereof, or a functionally active fragment thereof, or a homolog thereof, or a variant of "TYROSINE-PROTEIN KINASE CSK" encoded by a nucleic acid that hybridizes to the "TYROSINE-PROTEIN KINASE CSK" nucleic acid under low stringency conditions, and/or
   (vii) "INOSINE-5'-MONOPHOSPHATE DEHYDROGENASE 2" (SEQ ID No:7) or a functionally active derivative thereof, or a functionally active fragment thereof, or a homolog thereof, or a variant of "INOSINE-5'-MONOPHOSPHATE DEHYDROGENASE 2" encoded by a nucleic acid that hybridizes to the "INOSINE-5'-MONOPHOSPHATE DEHYDROGENASE 2" nucleic acid under low stringency conditions, and/or
   (viii) "BAG-FAMILY MOLECULAR CHAPERONE REGULATOR-4" (SEQ ID No:8) or a functionally active derivative thereof, or a functionally active fragment thereof, or a homolog thereof, or a variant of "BAG-FAMILY MOLECULAR CHAPERONE REGULATOR-4" encoded by a nucleic acid that hybridizes to the "BAG-FAMILY MOLECULAR CHAPERONE REGULATOR-4" nucleic acid under low stringency conditions, and/or
   (ix) "HSP 90-β 1"(SEQ ID No:9) or a functionally active derivative thereof, or a functionally active fragment thereof, or a homolog thereof, or a variant of "HSP 90-β 1" encoded by a nucleic acid that hybridizes to the "HSP 90-β 1" nucleic acid under low stringency conditions, is present in the complex.
41. The complex of any one of No. 1 - 8, or proteins of No. 13 or the antibody or fragment of No. 17, for use in a method of diagnosing a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
42. A method for treating or preventing a disease or disorder characterized by an aberrant amount of, activity or component composition of or intracellular localization of, the complex of anyone of No. 1 - 8, comprising administering to a subject in need of such treatment or prevention a therapeutically effective amount of one or more molecules that modulate the amount of, ABIN-2 binding activity, or protein components of, said complex.
43. The method according to No. 42, wherein said disease or disorder involves decreased levels of the amount or activity of said complex.
44. The method according to No. 42 , wherein said disease or disorder involves increased levels of the amount or activity of said complex.
45. Complex of any of No. 1-8 and/or protein selected from the following proteins
   (i) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions,
   (ii) "TIAF1" (SEQ ID No:2) or a functionally active derivative thereof, or a functionally active fragment thereof, or a homolog thereof, or a variant of "TIAF1" encoded by a nucleic acid that hybridizes to the "TIAF1" nucleic acid under low stringency conditions,
   (iii) "LCK" (SEQ ID No:3) or a functionally active derivative thereof, or a functionally active fragment thereof, or a homolog thereof, or a variant of "LCK" encoded by a nucleic acid that hybridizes to the "LCK" nucleic acid under low stringency conditions,
   (iv) "KIAA0564 PROTEIN" (SEQ ID No:4) or a functionally active derivative thereof, or a functionally active fragment thereof, or a homolog thereof, or a variant of "KIAA0564 PROTEIN" encoded by a nucleic acid that hybridizes to the "KIAA0564 PROTEIN" nucleic acid under low stringency conditions,
   (v) "UBIQUITIN-PROTEIN LIGASE E3A" (SEQ ID No:5) or a functionally active derivative thereof, or a functionally active fragment thereof, or a homolog thereof, or a variant of "UBIQUITIN-PROTEIN LIGASE E3A" encoded by a nucleic acid that hybridizes to the "UBIQUITIN-PROTEIN LIGASE E3A" nucleic acid under low stringency conditions,
   (vi) "TYROSINE-PROTEIN KINASE CSK" (SEQ ID No:6) or a functionally active derivative thereof, or a functionally active fragment thereof, or a homolog thereof, or a variant of "TYROSINE-PROTEIN KINASE CSK" encoded by a nucleic acid that hybridizes to the "TYROSINE-PROTEIN KINASE CSK" nucleic acid under low stringency conditions,
   (vii) "INOSINE-5'-MONOPHOSPHATE DEHYDROGENASE 2" (SEQ ID No:7) or a functionally active derivative thereof, or a functionally active fragment thereof, or a homolog thereof, or a variant of "INOSINE-5'-MONOPHOSPHATE DEHYDROGENASE 2" encoded by a nucleic acid that hybridizes to the "INOSINE-5'-MONOPHOSPHATE DEHYDROGENASE 2" nucleic acid under low stringency conditions,
   (viii) "BAG-FAMILY MOLECULAR CHAPERONE REGULATOR-4" (SEQ ID No:8) or a functionally active derivative thereof,or a functionally active fragement thereof, or a homolog thereof, or a variant of "BAG-FAMILY MOLECULAR CHAPERONE REGULATOR-4" that hybridizes to the "BAG-FAMILY MOLECULAR CHAPERONE REGULATOR-4" nucleic acid under low stringency conditions, and
   (ix) "HSP 90-β 1"(SEQ ID No:9) or a functionally active derivative thereof, or a functionally active fragment thereof, or a homolog thereof, or a variant of "HSP 90-β 1" encoded by a nucleic acid that hybridizes to the "HSP 90-β 1" nucleic acid under low stringency conditions,
as a target for an active agent of a pharmaceutical, preferably a drug target in the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.

The present invention further relates to the TNFR1-protein complex
1. A protein complex selected from complex (I) and comprising
   (a) at least one first protein selected from the group consisting of:
      (i) "TRAF1" (SEQ ID No:14) or a functionally active derivative thereof, or a functionally active fragment thereof, or a homolog thereof, or a variant of "TRAF1" encoded by a nucleic acid that hybridizes to the "TRAF1" nucleic acid under low stringency conditions,
      (ii) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions,
      (iii) "RIP"(SEQ ID No:16) or a functionally active derivative thereof, or a functionally active fragment thereof, or a homolog thereof, or a variant of "RIP"encoded by a nucleic acid that hybridizes to the "RIP"nucleic acid under low stringency conditions,
      (iv) "TRADD" (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions,
      (v) "TNFR1" (SEQ ID No:21) or a functionally active derivative thereof, or a functionally active fragment thereof, or a homolog thereof, or a variant of "TNFR1" encoded by a nucleic acid that hybridizes to the "TNFR1" nucleic acid under low stringency conditions,
      (vi) "FADD" (SEQ ID No:22) or a functionally active derivative thereof, or a functionally active fragment thereof, or a homolog thereof, or a variant of "FADD" encoded by a nucleic acid that hybridizes to the "FADD" nucleic acid under low stringency conditions,
      (vii) IKK γ (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
      (viii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
      (ix) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
      (x) "c-IAP2" (SEQ ID No:26) or a functionally active derivative thereof, or a functionally active fragment thereof, or a homolog thereof, or a variant of "c-IAP2" encoded by a nucleic acid that hybridizes to the "c-IAP2" nucleic acid under low stringency conditions, and
      (xi) "c-IAP1" (SEQ ID No:35) or a functionally active derivative thereof, or a functionally active fragment thereof, or a homolog thereof, or a variant of "c-IAP1" encoded by a nucleic acid that hybridizes to the "c-IAP1" nucleic acid under low stringency conditions, and
   (b) at least one second protein, which second protein is selected from the group consisting of:
      (i) "CDNA FLJ20733 FIS, CLONE HEP08550" (SEQ ID No:10) or a functionally active derivative thereof, or a functionally active fragment thereof, or a homolog thereof, or a variant of "CDNA FLJ20733 FIS, CLONE HEP08550" encoded by a nucleic acid that hybridizes to the "CDNA FLJ20733 FIS, CLONE HEP08550" nucleic acid under low stringency conditions,
      (ii) "ANNEXIN I" (SEQ ID No:11) or a functionally active derivative thereof, or a functionally active fragment thereof, or a homolog thereof, or a variant of "ANNEXIN I" encoded by a nucleic acid that hybridizes to the "ANNEXIN I" nucleic acid under low stringency conditions,
      (iii) "THIOREDOXIN" (SEQ ID No:12) or a functionally active derivative thereof, or a functionally active fragment thereof, or a homolog thereof, or a variant of "THIOREDOXIN" encoded by a nucleic acid that hybridizes to the "THIOREDOXIN" nucleic acid under low stringency conditions,
      (iv) "PEROXIREDOXIN 1" (SEQ ID No:13) or a functionally active derivative thereof, or a functionally active fragment thereof, or a homolog thereof, or a variant of "PEROXIREDOXIN 1" encoded by a nucleic acid that hybridizes to the "PEROXIREDOXIN 1" nucleic acid under low stringency conditions,
      (v) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,
      (vi) "FLJ10719 (KIAA1794)" (SEQ ID No:19) or a functionally active derivative thereof, or a functionally active fragment thereof, or a homolog thereof, or a variant of "FLJ10179 (KIAA1794)" encoded by a nucleic acid that hybridizes to the "FLJ10179 (KIAA1794)" nucleic acid under low stringency conditions,
      (vii) "HYPOTHETICAL PROTEIN PRO1855" (SEQ ID No:20) or a functionally active derivative thereof, or a functionally active fragment thereof, or a homolog thereof, or a variant of "HYPOTHETICAL PROTEIN PRO1855 encoded by a nucleic acid that hybridizes to the "HYPOTHETICAL PROTEIN PRO1855" nucleic acid under low stringency conditions,
      (viii) "PROTEIN-L-ISOASPARTATE(D-ASPARTATE) O-METHYLTRANSFERASE" (SEQ ID No:27) or a functionally active derivative thereof, or a functionally active fragment thereof, or a homolog thereof, or a variant of "PROTEIN-L-ISOASPARTATE(D-ASPARTATE) O-METHYLTRANSFERASE" encoded by a nucleic acid that hybridizes to the "PROTEIN-L-ISOASPARTATE(D-ASPARTATE) O-METHYLTRANSFERASE" nucleic acid under low stringency conditions,
      (ix) "UDP-GLUCOSE:GLYCOPROTEIN GLUCOSYLTRANSFERASE 1 PRECURSOR" (SEQ ID No:28) or a functionally active derivative thereof, or a functionally active fragment thereof, or a homolog thereof, or a variant of "UDP-GLUCOSE:GLYCOPROTEIN GLUCOSYLTRANSFERASE 1 PRECURSOR" encoded by a nucleic acid that hybridizes to the "UDP-GLUCOSE:GLYCOPROTEIN GLUCOSYLTRANSFERASE 1 PRECURSOR" nucleic acid under low stringency conditions,
      (x) "CDNA FLJ14515 FIS, CLONE NT2RM1000800, WEAKLY SIMILAR TO MUS MUSCULUS PARTIAL B-IND1 PROTEIN" (SEQ ID No:29) or a functionally active derivative thereof, or a functionally active fragment thereof, or a homolog thereof, or a variant of "CDNA FLJ14515 FIS, CLONE NT2RM1000800, WEAKLY SIMILAR TO MUS MUSCULUS PARTIAL B-IND1 PROTEIN" encoded by a nucleic acid that hybridizes to the "CDNA FLJ14515 FIS, CLONE NT2RM1000800, WEAKLY SIMILAR TO MUS MUSCULUS PARTIAL B-IND1 PROTEIN" nucleic acid under low stringency conditions,
      (xi) "KIAA1040 PROTEIN" (SEQ ID No:30) or a functionally active derivative thereof, or a functionally active fragment thereof, or a homolog thereof, or a variant of "KIAA1040 PROTEIN" encoded by a nucleic acid that hybridizes to the "KIAA1040 PROTEIN" nucleic acid under low stringency conditions,
      (xii) "ADENOSYLHOMOCYSTEINASE" (SEQ ID No:31) or a functionally active derivative thereof, or a functionally active fragment thereof, or a homolog thereof, or a variant of "ADENOSYLHOMOCYSTEINASE" encoded by a nucleic acid that hybridizes to the "ADENOSYLHOMOCYSTEINASE" nucleic acid under low stringency conditions,
      (xiii) "ATAXIA-TELANGIECTASIA GROUP D-ASSOCIATED PROTEIN" (SEQ ID No:32) or a functionally active derivative thereof, or a functionally active fragment thereof, or a homolog thereof, or a variant of "ATAXIA-TELANGIECTASIA GROUP D-ASSOCIATED PROTEIN" encoded by a nucleic acid that hybridizes to the "ATAXIA-TELANGIECTASIA GROUP D-ASSOCIATED PROTEIN" nucleic acid under low stringency conditions,
      (xiv) "MACROPHAGE MIGRATION INHIBITORY FACTOR" (SEQ ID No:33) or a functionally active derivative thereof, or a functionally active fragment thereof, or a homolog thereof, or a variant of "MACROPHAGE MIGRATION INHIBITORY FACTOR" encoded by a nucleic acid that hybridizes to the "MACROPHAGE MIGRATION INHIBITORY FACTOR" nucleic acid under low stringency conditions,
      (xv) "MONOCARBOXYLATE TRANSPORTER 1" (SEQ ID No:34) or a functionally active derivative thereof, or a functionally active fragment thereof, or a homolog thereof, or a variant of "MONOCARBOXYLATE TRANSPORTER 1" encoded by a nucleic acid that hybridizes to the "MONOCARBOXYLATE TRANSPORTER 1" nucleic acid under low stringency conditions,
      (xvi) "ANNEXIN II" (SEQ ID No:36) or a functionally active derivative thereof, or a functionally active fragment thereof, or a homolog thereof, or a variant of "ANNEXIN II" encoded by a nucleic acid that hybridizes to the "ANNEXIN II" nucleic acid under low stringency conditions,
      (xvii) "NAD(P)-DEPENDENT STEROID DEHYDROGENASE" (SEQ ID No:37) or a functionally active derivative thereof, or a functionally active fragment thereof, or a homolog thereof, or a variant of "NAD(P)-DEPENDENT STEROID DEHYDROGENASE" encoded by a nucleic acid that hybridizes to the "NAD(P)-DEPENDENT STEROID DEHYDROGENASE" nucleic acid under low stringency conditions,
      (xviii) "PEPTIDYL-PROLYL CIS-TRANS ISOMERASE B PRECURSOR" (SEQ ID No:38) or a functionally active derivative thereof, or a functionally active fragment thereof, or a homolog thereof, or a variant of "PEPTIDYL-PROLYL CIS-TRANS ISOMERASE B PRECURSOR" encoded by a nucleic acid that hybridizes to the "PEPTIDYL-PROLYL CIS-TRANS ISOMERASE B PRECURSOR" nucleic acid under low stringency conditions,
      (xix) "CARBAMOYL-PHOSPHATE SYNTHASE [AMMONIA], MITOCHONDRIAL PRECURSOR" (SEQ ID No:39) or a functionally active derivative thereof, or a functionally active fragment thereof, or a homolog thereof, or a variant of "CARBAMOYL-PHOSPHATE SYNTHASE [AMMONIA], MITOCHONDRIAL PRECURSOR" encoded by a nucleic acid that hybridizes to the "CARBAMOYL-PHOSPHATE SYNTHASE [AMMONIA], MITOCHONDRIAL PRECURSOR" nucleic acid under low stringency conditions,
      (xx) "ZINC-FINGER PROTEIN RFP" (SEQ ID No:40) or a functionally active derivative thereof, or a functionally active fragment thereof, or a homolog thereof, or a variant of "ZINC-FINGER PROTEIN RFP" encoded by a nucleic acid that hybridizes to the "ZINC-FINGER PROTEIN RFP" nucleic acid under low stringency conditions,
      (xxi) "52 KDA PROTEIN" (SEQ ID No:41) or a functionally active derivative thereof, or a functionally active fragment thereof, or a homolog thereof, or a variant of "52 KDA PROTEIN" encoded by a nucleic acid that hybridizes to the "52 KDA PROTEIN" nucleic acid under low stringency conditions,
      (xxii) "SIMILAR TO SUBTILISIN-LIKE SERINE PROTEINASE" (SEQ ID No:42) or a functionally active derivative thereof, or a functionally active fragment thereof, or a homolog thereof, or a variant of "SIMILAR TO SUBTILISIN-LIKE SERINE PROTEINASE" encoded by a nucleic acid that hybridizes to the "SIMILAR TO SUBTILISIN-LIKE SERINE PROTEINASE" nucleic acid under low stringency conditions,
      (xxiii) "ANTILEUKOPROTEINASE 1 PRECURSOR" (SEQ ID No:43) or a functionally active derivative thereof, or a functionally active fragment thereof, or a homolog thereof, or a variant of "ANTILEUKOPROTEINASE 1 PRECURSOR" encoded by a nucleic acid that hybridizes to the "ANTILEUKOPROTEINASE 1 PRECURSOR" nucleic acid under low stringency conditions,
      (xxiv) "7-DEHYDROCHOLESTEROL REDUCTASE" (SEQ ID No:44) or a functionally active derivative thereof, or a functionally active fragment thereof, or a homolog thereof, or a variant of "7-DEHYDROCHOLESTEROL REDUCTASE" encoded by a nucleic acid that hybridizes to the "7-DEHYDROCHOLESTEROL REDUCTASE" nucleic acid under low stringency conditions,
      (xxv) "HYPOTHETICAL 82.0 KDA PROTEIN" (SEQ ID No:45) or a functionally active derivative thereof, or a functionally active fragment thereof, or a homolog thereof, or a variant of "HYPOTHETICAL 82.0 KDA PROTEIN" encoded by a nucleic acid that hybridizes to the "HYPOTHETICAL 82.0 KDA PROTEIN" nucleic acid under low stringency conditions,
      (xxvi) "CDNA FLJ14601 FIS, CLONE NT2RP1000124" (SEQ ID No:46) or a functionally active derivative thereof, or a functionally active fragment thereof, or a homolog thereof, or a variant of "CDNA FLJ14601 FIS, CLONE NT2RP1000124" encoded by a nucleic acid that hybridizes to the "CDNA FLJ14601 FIS, CLONE NT2RP1000124" nucleic acid under low stringency conditions,
      (xxvii) "TRANSCRIPTION FACTOR AP-2 Γ" (SEQ ID No:47) or a functionally active derivative thereof, or a functionally active fragment thereof, or a homolog thereof, or a variant of "TRANSCRIPTION FACTOR AP-2 Γ" encoded by a nucleic acid that hybridizes to the "TRANSCRIPTION FACTOR AP-2 Γ" nucleic acid under low stringency conditions,
      (xxviii) "SERINE/THREONINE PROTEIN PHOSPHATASE 2A, CATALYTIC SUBUNIT, B ISOFORM" (SEQ ID No:48) or a functionally active derivative thereof, or a functionally active fragment thereof, or a homolog thereof, or a variant of "SERINE/THREONINE PROTEIN PHOSPHATASE 2A, CATALYTIC SUBUNIT, B ISOFORM" encoded by a nucleic acid that hybridizes to the "SERINE/THREONINE PROTEIN PHOSPHATASE 2A, CATALYTIC SUBUNIT, B ISOFORM" nucleic acid under low stringency conditions,
      (xxix) "SEVENTRANSMEMBRANE-DOMAIN PROTEIN" (SEQ ID No:49) or a functionally active derivative thereof, or a functionally active fragment thereof, or a homolog thereof, or a variant of "SEVENTRANSMEMBRANE-DOMAIN PROTEIN" encoded by a nucleic acid that hybridizes to the "SEVENTRANSMEMBRANE-DOMAIN PROTEIN" nucleic acid under low stringency conditions,
      (xxx) "SERPIN B11" (SEQ ID No:50) or a functionally active derivative thereof, or a functionally active fragment thereof, or a homolog thereof, or a variant of "SERPIN B11" encoded by a nucleic acid that hybridizes to the "SERPIN B11" nucleic acid under low stringency conditions, and
      (xxxi) "KIAA1228 PROTEIN" (SEQ ID No:51) or a functionally active derivative thereof, or a functionally active fragment thereof, or a homolog thereof, or a variant of "KIAA1228 PROTEIN" encoded by a nucleic acid that hybridizes to the "KIAA1228 PROTEIN" nucleic acid under low stringency conditions;
   and a complex (II) comprising at least two of said second proteins,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
2. The protein complex according to claim 1 wherein the first protein is the protein "TNFR1"(xSEQ ID NO. 21), or a functionally active derivative thereof, or a functionally active fragment thereof, or a homolog thereof, or a variant of "TNFR1" encoded by a nucleic acid that hybridizes to the "TNFR1" nucleic acid under low stringency conditions.
3. The protein complex according to No. 1 selected from complex (I) and comprising the following proteins:
   (i) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,
   (ii) "FLJ10179 (KIAA1794)" (SEQ ID No:19) or a functionally active derivative thereof, or a functionally active fragment thereof, or a homolog thereof, or a variant of "FLJ10179 (KIAA1794)" encoded by a nucleic acid that hybridizes to the "FLJ10179 (KIAA1794)" nucleic acid under low stringency conditions,
   (iii) "HYPOTHETICAL PROTEIN PRO1855" (SEQ ID No:20) or a functionally active derivative thereof, or a functionally active fragment thereof, or a homolog thereof, or a variant of "HYPOTHETICAL PROTEIN PRO1855" encoded by a nucleic acid that hybridizes to the "HYPOTHETICAL PROTEIN PRO1855" nucleic acid under low stringency conditions,
   (iv) "TNFR1" (SEQ ID No:21) or a functionally active derivative thereof, or a functionally active fragment thereof, or a homolog thereof, or a variant of "TNRF1" encoded by a nucleic acid that hybridizes to the "TNRF1" nucleic acid under low stringency conditions,
   (v) "PROTEIN-L-ISOASPARTATE(D-ASPARTATE) O-METHYLTRANSFERASE" (SEQ ID No:27) or a functionally active derivative thereof, or a functionally active fragment thereof, or a homolog thereof, or a variant of "PROTEIN-L-ISOASPARTATE(D-ASPARTATE) O-METHYLTRANSFERASE" encoded by a nucleic acid that hybridizes to the "PROTEIN-L-ISOASPARTATE(D-ASPARTATE) O-METHYLTRANSFERASE" nucleic acid under low stringency conditions,
   (vi) "KIAA1040 PROTEIN" (SEQ ID No:30) or a functionally active derivative thereof, or a functionally active fragment thereof, or a homolog thereof, or a variant of "KIAA1040 PROTEIN" encoded by a nucleic acid that hybridizes to the "KIAA1040 PROTEIN" nucleic acid under low stringency conditions,
   (vii) "MACROPHAGE MIGRATION INHIBITORY FACTOR" (SEQ ID No:33) or a functionally active derivative thereof, or a functionally active fragment thereof, or a homolog thereof, or a variant of "MACROPHAGE MIGRATION INHIBITORY FACTOR" encoded by a nucleic acid that hybridizes to the "MACROPHAGE MIGRATION INHIBITORY FACTOR" nucleic acid under low stringency conditions,
   (viii) "ANNEXIN II" (SEQ ID No:36) or a functionally active derivative thereof, or a functionally active fragment thereof, or a homolog thereof, or a variant of "ANNEXIN II" encoded by a nucleic acid that hybridizes to the "ANNEXIN II" nucleic acid under low stringency conditions,
   (ix) "CARBAMOYL-PHOSPHATE SYNTHASE [AMMONIA], MITOCHONDRIAL PRECURSOR" (SEQ ID No:39) or a functionally active derivative thereof, or a functionally active fragment thereof, or a homolog thereof, or a variant of "CARBAMOYL-PHOSPHATE SYNTHASE [AMMONIA], MITOCHONDRIAL PRECURSOR" encoded by a nucleic acid that hybridizes to the "CARBAMOYL-PHOSPHATE SYNTHASE [AMMONIA], MITOCHONDRIAL PRECURSOR" nucleic acid under low stringency conditions,
   (x) "ZINC-FINGER PROTEIN RFP" (SEQ ID No:40) or a functionally active derivative thereof, or a functionally active fragment thereof, or a homolog thereof, or a variant of "ZINC-FINGER PROTEIN RFP" encoded by a nucleic acid that hybridizes to the "ZINC-FINGER PROTEIN RFP" nucleic acid under low stringency conditions,
   (xi) "52 KDA PROTEIN" (SEQ ID No:41) or a functionally active derivative thereof, or a functionally active fragment thereof, or a homolog thereof, or a variant of "52 KDA PROTEIN" encoded by a nucleic acid that hybridizes to the "52 KDA PROTEIN" nucleic acid under low stringency conditions, and
   (xii) "SIMILAR TO SUBTILISIN-LIKE SERINE PROTEINASE" (SEQ ID No:42) or a functionally active derivative thereof, or a functionally active fragment thereof, or a homolog thereof, or a variant of "SIMILAR TO SUBTILISIN-LIKE SERINE PROTEINASE" encoded by a nucleic acid that hybridizes to the "SIMILAR TO SUBTILISIN-LIKE SERINE PROTEINASE" nucleic acid under low stringency conditions; and a protein complex selected from complex (II) and comprising all but 1 - 30 of the following proteins:

   (i) "ANNEXIN I" (SEQ ID No:11) or a functionally active derivative thereof, or a functionally active fragment thereof, or a homolog thereof, or a variant of "ANEXIN I" encoded by a nucleic acid that hybridizes to the "ANNEXIN I" nucleic acid under low stringency conditions,
   (ii) "THIOREDOXIN" (SEQ ID No:12) or a functionally active derivative thereof, or a functionally active fragment thereof, or a homolog thereof, or a variant of "THIOREDOXIN" encoded by a nucleic acid that hybridizes to the "THIOREDOXIN" nucleic acid under low stringency conditions,
   (iii) "PEROXIREDOXIN 1" (SEQ ID No:13) or a functionally active derivative thereof, or a functionally active fragment thereof, or a homolog thereof, or a variant of "PEROXiREDOXIN 1" encoded by a nucleic acid that hybridizes to the "PEROXIREDOXIN" nucleic acid under low stringency conditions,
   (iv) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions,
   (v) "TRADD" (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions,
   (vi) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,
   (vii) "FLJ10179 (KIAA1794)" (SEQ ID No:19) or a functionally active derivative thereof, or a functionally active fragment thereof, or a homolog thereof, or a variant of "FLJ10179 (KIAA1794)" encoded by a nucleic acid that hybridizes to the "FLJ10179 (KIAA1794)" nucleic acid under low stringency conditions,
   (viii) "HYPOTHETICAL PROTEIN PRO1855" (SEQ ID No:20) or a functionally active derivative thereof, or a functionally active fragment thereof, or a homolog thereof, or a variant of "HYPOTHETICAL PROTEIN PRO1855" encoded by a nucleic acid that hybridizes to the "HYPOTHETICAL PROTEIN PRO1855" nucleic acid under low stringency conditions,
   (ix) (iv) "TNFR1" (SEQ ID No:21) or a functionally active derivative thereof, or a functionally active fragment thereof, or a homolog thereof, or a variant of "TNRF1" encoded by a nucleic acid that hybridizes to the "TNRF1" nucleic acid under low stringency conditions,
   (x) "PROTEIN-L-ISOASPARTATE(D-ASPARTATE) O-METHYLTRANSFERASE" (SEQ ID No:27) or a functionally active derivative thereof, or a functionally active fragment thereof, or a homolog thereof, or a variant of "PROTEIN-L-ISOASPARTATE(D-ASPARTATE) O-METHYLTRANSFERASE" encoded by a nucleic acid that hybridizes to the "PROTEIN-L-ISOASPARTATE(D-ASPARTATE) O-METHYLTRANSFERASE" nucleic acid under low stringency conditions,
   (xi) "UDP-GLUCOSE:GLYCOPROTEIN GLUCOSYLTRANSFERASE 1 PRECURSOR" (SEQ ID No:28) or a functionally active derivative thereof, or a functionally active fragment thereof, or a homolog thereof, or a variant of "UDP-GLUCOSE:GLYCOPROTEIN GLUCOSYLTRANSFERASE 1 PRECURSOR" encoded by a nucleic acid that hybridizes to the "UDP-GLUCOSE:GLYCOPROTEIN GLUCOSYLTRANSFERASE 1 PRECURSOR" nucleic acid under low stringency conditions,
   (xii) "CDNA FLJ14515 FIS, CLONE NT2RM1000800, WEAKLY SIMILAR TO MUS MUSCULUS PARTIAL B-IND1 PROTEIN" (SEQ ID No:29) or a functionally active derivative thereof, or a functionally active fragment thereof, or a homolog thereof, or a variant of "CDNA FLJ14515 FIS, CLONE NT2RM1000800, WEAKLY SIMILAR TO MUS MUSCULUS PARTIAL B-IND1 PROTEIN" encoded by a nucleic acid that hybridizes to the "CDNA FLJ14515 FIS, CLONE NT2RM1000800, WEAKLY SIMILAR TO MUS MUSCULUS PARTIAL B-IND1 PROTEIN" nucleic acid under low stringency conditions,
   (xiii) "KIAA1040 PROTEIN" (SEQ ID No:30) or a functionally active derivative thereof, or a functionally active fragment thereof, or a homolog thereof, or a variant of "KIAA1040 PROTEIN" encoded by a nucleic acid that hybridizes to the "KIAA1040 PROTEIN" nucleic acid under low stringency conditions,
   (xiv) "ADENOSYLHOMOCYSTEINASE" (SEQ ID No:31) or a functionally active derivative thereof, or a functionally active fragment thereof, or a homolog thereof, or a variant of "ADENOSYLHOMOCYSTEINASE" encoded by a nucleic acid that hybridizes to the "ADENOSYLHOMOCYSTEINASE" nucleic acid under low stringency conditions,
   (xv) "ATAXIA-TELANGIECTASIA GROUP D-ASSOCIATED PROTEIN" (SEQ ID No:32) or a functionally active derivative thereof, or a functionally active fragment thereof, or a homolog thereof, or a variant of "ATAXIA-TELANGIECTASIA GROUP D-ASSOCIATED PROTEIN" encoded by a nucleic acid that hybridizes to the "ATAXIA-TELANGIECTASIA GROUP D-ASSOCIATED PROTEIN" nucleic acid under low stringency conditions,
   (xvi) "MACROPHAGE MIGRATION INHIBITORY FACTOR" (SEQ ID No:33) or a functionally active derivative thereof, or a functionally active fragment thereof, or a homolog thereof, or a variant of "MACROPHAGE MIGRATION INHIBITORY FACTOR" encoded by a nucleic acid that hybridizes to the "MACROPHAGE MIGRATION INHIBITORY FACTOR" nucleic acid under low stringency conditions,
   (xvii) "MONOCARBOXYLATE TRANSPORTER 1" (SEQ ID No:34) or a functionally active derivative thereof, or a functionally active fragment thereof, or a homolog thereof, or a variant of "MONOCARBOXYLATE TRANSPORTER 1" encoded by a nucleic acid that hybridizes to the "MONOCARBOXYLASE TRANSPORTER 1" nucleic acid under low stringency conditions,
   (xviii) "ANNEXIN II" (SEQ ID No:36) or a functionally active derivative thereof, or a functionally active fragment thereof, or a homolog thereof, or a variant of "ANNEXIN II" encoded by a nucleic acid that hybridizes to the "ANNEXIN II" nucleic acid under low stringency conditions,
   (xix) "NAD(P)-DEPENDENT STEROID DEHYDROGENASE" (SEQ ID No:37) or a functionally active derivative thereof, or a functionally active fragment thereof, or a homolog thereof, or a variant of "NAD(P)-DEPENDENT STEROID DEHYDROGENASE" encoded by a nucleic acid that hybridizes to the "NAD(P)-DEPENDENT STEROID DEHYDROGENASE" nucleic acid under low stringency conditions,
   (xx) "PEPTIDYL-PROLYL CIS-TRANS ISOMERASE B PRECURSOR" (SEQ ID No:38) or a functionally active derivative thereof, or a functionally active fragment thereof, or a homolog thereof, or a variant of "PEPTIDYL-PROLYL CIS-TRANS ISOMERASE B PRECURSOR" encoded by a nucleic acid that hybridizes to the "PEPTIDYL-PROLYL CIS-TRANS ISOMERASE B PRECURSOR" nucleic acid under low stringency conditions,
   (xxi) "CARBAMOYL-PHOSPHATE SYNTHASE [AMMONIA], MITOCHONDRIAL PRECURSOR" (SEQ ID No:39) or a functionally active derivative thereof, or a functionally active fragment thereof, or a homolog thereof, or a variant of "CARBAMOYL-PHOSPHATE SYNTHASE [AMMONIA], MITOCHONDRIAL PRECURSOR" encoded by a nucleic acid that hybridizes to the "CARBAMOYL-PHOSPHATE SYNTHASE [AMMONIA], MITOCHONDRIAL PRECURSOR" nucleic acid under low stringency conditions,
   (xxii) "ZINC-FINGER PROTEIN RFP" (SEQ ID No:40) or a functionally active derivative thereof, or a functionally active fragment thereof, or a homolog thereof, or a variant of "ZINC-FINGER PROTEIN RFP" encoded by a nucleic acid that hybridizes to the "ZINC-FINGER PROTEIN RFP" nucleic acid under low stringency conditions,
   (xxiii) "52 KDA PROTEIN" (SEQ ID No:41) or a functionally active derivative thereof, or a functionally active fragment thereof, or a homolog thereof, or a variant of "52 KDA PROTEIN" encoded by a nucleic acid that hybridizes to the "52 KDA PROTEIN" nucleic acid under low stringency conditions,
   (xviv) "SIMILAR TO SUBTILISIN-LIKE SERINE PROTEINASE" (SEQ ID No:42) or a functionally active derivative thereof, or a functionally active fragment thereof, or a homolog thereof, or a variant of "SIMILAR TO SUBTILISIN-LIKE SERINE PROTEINASE" encoded by a nucleic acid that hybridizes to the "SIMILAR TO SUBTILISIN-LIKE SERINE PROTEINASE" nucleic acid under low stringency conditions,
   (xxv) "HYPOTHETICAL 82.0 KDA PROTEIN" (SEQ ID No:45) or a functionally active derivative thereof, or a functionally active fragment thereof, or a homolog thereof, or a variant of "HYPOTHETICAL 82.0 KDA PROTEIN" encoded by a nucleic acid that hybridizes to the "HYPOTHETICAL 82.0 KDA PROTEIN" nucleic acid under low stringency conditions,
   (xxvi) "CDNA FLJ14601 FIS, CLONE NT2RP1000124" (SEQ ID No:46) or a functionally active derivative thereof, or a functionally active fragment thereof, or a homolog thereof, or a variant of "CDNA FLJ14601 FIS, CLONE NT2RP1000124" encoded by a nucleic acid that hybridizes to the "CDNA FLJ14601 FIS, CLONE NT2RP1000124" nucleic acid under low stringency conditions,
   (xxvii) "TRANSCRIPTION FACTOR AP-2 γ" (SEQ ID No:47) or a functionally active derivative thereof, or a functionally active fragment thereof, or a homolog thereof, or a variant of "TRANSCRIPTION FACTOR AP-2 γ" encoded by a nucleic acid that hybridizes to the "TRANSCRIPTION FACTOR AP-2 γ" nucleic acid under low stringency conditions,
   (xxviii) "SERINE/THREONINE PROTEIN PHOSPHATASE 2A, CATALYTIC SUBUNIT, β ISOFORM" (SEQ ID No:48) or a functionary active derivative thereof, or a functionally active fragment thereof, or a homolog thereof, or a variant of "SERINE/THREONINE PROTEIN PHOSPHATASE 2A, CATALYTIC SUBUNIT, β ISOFORM" encoded by a nucleic acid that hybridizes to the "SERINE/THREONINE PROTEIN PHOSPHATASE 2A, CATALYTIC SUBUNIT, β ISOFORM" nucleic acid under low stringency conditions, and
   (xxix) "SEVENTRANSMEMBRANE-DOMAIN PROTEIN" (SEQ ID No:49) or a functionally active derivative thereof, or a functionally active fragment thereof, or a homolog thereof, or a variant of "SEVENTRANSMEMBRANE-DOMAIN PROTEIN" encoded by a nucleic acid that hybridizes to the "SEVENTRANSMEMBRANE-DOMAIN PROTEIN" nucleic acid under low stringency conditions;
   and a protein complex selected from complex (III) and comprising the following proteins:
   (i) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,
   (ii) "FLJ10179 (KIAA1794)" (SEQ ID No:19) or a functionally active derivative thereof, or a functionally active fragment thereof, or a homolog thereof, or a variant of "FLJ10179 (KIAA1794)" encoded by a nucleic acid that hybridizes to the "FLJ10179 (KIAA1794)" nucleic acid under low stringency conditions,
   (iii) "HYPOTHETICAL PROTEIN PRO1855" (SEQ ID No:20) or a functionally active derivative thereof, or a functionally active fragment thereof, or a homolog thereof, or a variant of "HYPOTHETICAL PROTEIN PRO1855" encoded by a nucleic acid that hybridizes to the "HYPOTHETICAL PROTEIN PRO1855" nucleic acid under low stringency conditions,
   (iv) "TNFR1" (SEQ ID No:21) or a functionally active derivative thereof, or a functionally active fragment thereof, or a homolog thereof, or a variant of "TNRF1" encoded by a nucleic acid that hybridizes to the "TNRF1" nucleic acid under low stringency conditions,
   (v) "PROTEIN-L-ISOASPARTATE(D-ASPARTATE) O-METHYLTRANSFERASE" (SEQ ID No:27) or a functionally active derivative thereof, or a functionally active fragment thereof, or a homolog thereof, or a variant of "PROTEIN-L-ISOASPARTATE(D-ASPARTATE) O-METHYLTRANSFERASE" encoded by a nucleic acid that hybridizes to the "PROTEIN-L-ISOASPARTATE(D-ASPARTATE) O-METHYLTRANSFERASE" nucleic acid under low stringency conditions,
   (vi) "KIAA1040 PROTEIN" (SEQ ID No:30) or a functionally active derivative thereof, or a functionally active fragment thereof, or a homolog thereof, or a variant of "KIAA1040 PROTEIN" encoded by a nucleic acid that hybridizes to the "KIAA1040 PROTEIN" nucleic acid under low stringency conditions,
   (vii) "MACROPHAGE MIGRATION INHIBITORY FACTOR" (SEQ ID No:33) or a functionally active derivative thereof, or a functionally active fragment thereof, or a homolog thereof, or a variant of "MACROPHAGE MIGRATION INHIBITORY FACTOR" encoded by a nucleic acid that hybridizes to the "MACROPHAGE MIGRATION INHIBITORY FACTOR" nucleic acid under low stringency conditions,
   (viii) "ANNEXIN II" (SEQ ID No:36) or a functionally active derivative thereof, or a functionally active fragment thereof, or a homolog thereof, or a variant of "ANNEXIN II" encoded by a nucleic acid that hybridizes to the "ANNEXIN II" nucleic acid under low stringency conditions,
   (ix) "CARBAMOYL-PHOSPHATE SYNTHASE [AMMONIA], MITOCHONDRIAL PRECURSOR" (SEQ ID No:39) or a functionally active derivative thereof, or a functionally active fragment thereof, or a homolog thereof, or a variant of "CARBAMOYL-PHOSPHATE SYNTHASE [AMMONIA], MITOCHONDRIAL PRECURSOR" encoded by a nucleic acid that hybridizes to the "CARBAMOYL-PHOSPHATE SYNTHASE [AMMONIA], MITOCHONDRIAL PRECURSOR" nucleic acid under low stringency conditions,
   (x) "ZINC-FINGER PROTEIN RFP" (SEQ ID No:40) or a functionally active derivative thereof, or a functionally active fragment thereof, or a homolog thereof, or a variant of "ZINC-FINGER PROTEIN RFP" encoded by a nucleic acid that hybridizes to the "ZINC-FINGER PROTEIN RFP" nucleic acid under low stringency conditions,
   (xi) "52 KDA PROTEIN" (SEQ ID No:41) or a functionally active derivative thereof, or a functionally active fragment thereof, or a homolog thereof, or a variant of "52 KDA PROTEIN" encoded by a nucleic acid that hybridizes to the "52 KDA PROTEIN" nucleic acid under low stringency conditions,
   (xii) "SIMILAR TO SUBTILISIN-LIKE SERINE PROTEINASE" (SEQ ID No:42) or a functionally active derivative thereof, or a functionally active fragment thereof, or a homolog thereof, or a variant of "SIMILAR TO SUBTILISIN-LIKE SERINE PROTEINASE" encoded by a nucleic acid that hybridizes to the "SIMILAR TO SUBTILISIN-LIKE SERINE PROTEINASE" nucleic acid under low stringency conditions,
   (xiii) "ANTILEUKOPROTEINASE 1 PRECURSOR" (SEQ ID No:43) or a functionally active derivative thereof, or a functionally active fragment thereof, or a homolog thereof, or a variant of "ANTILEUKOPROTEINASE 1 PRECURSOR" encoded by a nucleic acid that hybridizes to the "ANTILEUKOPROTEINASE 1 PRECURSOR" nucleic acid under low stringency conditions,
   (xiv) "7-DEHYDROCHOLESTEROL REDUCTASE" (SEQ ID No:44) or a functionally active derivative thereof, or a functionally active fragment thereof, or a homolog thereof, or a variant of "7-DEHYDROCHOLESTEROL REDUCTASE" encoded by a nucleic acid that hybridizes to the "7-DEHYDROCHOLESTEROL REDUCTASE" nucleic acid under low stringency conditions,
   (xv) "HYPOTHETICAL 82.0 KDA PROTEIN" (SEQ ID No:45) or a functionally active derivative thereof, or a functionally active fragment thereof, or a homolog thereof, or a variant of "HYPOTHETICAL 82.0 KDA PROTEIN" encoded by a nucleic acid that hybridizes to the "HYPOTHETICAL 82.0 KDA PROTEIN" nucleic acid under low stringency conditions,
   (xvi) "SERPIN B11" (SEQ ID No:50) or a functionally active derivative thereof,or a functionally active fragement thereof, or a homolog thereof, or a variant of "SERPIN B11" that hybridizes to the "SERPIN B11" nucleic acid under low stringency conditions, and
   (xvii) "KIAA1228 PROTEIN" (SEQ ID No:51) or a functionally active derivative thereof, or a functionally active fragment thereof, or a homolog thereof, or a variant of "KIAA1228 PROTEIN" encoded by a nucleic acid that hybridizes to the "KIAA1228 PROTEIN" nucleic acid under low stringency conditions;
   and a protein complex selected from complex (IV) and comprising the following proteins:
   (i) "CDNA FLJ20733 FIS, CLONE HEP08550" (SEQ ID No:10) or a functionally active derivative thereof, or a functionally active fragment thereof, or a homolog thereof, or a variant of "CDNA FLJ20733 FIS, CLONE HEP08550" encoded by a nucleic acid that hybridizes to the "CDNA FLJ20733 FIS, CLONE HEP08550" nucleic acid under low stringency conditions,
   (ii) "ANNEXIN I" (SEQ ID No:11) or a functionally active derivative thereof, or a functionally active fragment thereof, or a homolog thereof, or a variant of "ANEXIN I" encoded by a nucleic acid that hybridizes to the "ANNEXIN I" nucleic acid under low stringency conditions,
   (iii) "THIOREDOXIN" (SEQ ID No:12) or a functionally active derivative thereof, or a functionally active fragment thereof, or a homolog thereof, or a variant of "THIOREDOXIN" encoded by a nucleic acid that hybridizes to the "THIOREDOXIN" nucleic acid under low stringency conditions,
   (iv) "PEROXIREDOXIN 1" (SEQ ID No:13) or a functionally active derivative thereof, or a functionally active fragment thereof, or a homolog thereof, or a variant of "PEROXiREDOXIN 1" encoded by a nucleic acid that hybridizes to the "PEROXIREDOXIN" nucleic acid under low stringency conditions,
   (v) "TRAF1" (SEQ ID No: 14) or a functionally active derivative thereof, or a functionally active fragment thereof, or a homolog thereof, or a variant of "TRAF1" encoded by a nucleic acid that hybridizes to the "TRAF1" nucleic acid under low stringency conditions,
   (vi) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions,
   (vii) "RIP" (SEQ ID No:16) or a functionally active derivative thereof, or a functionally active fragment thereof, or a homolog thereof, or a variant of "RIP" encoded by a nucleic acid that hybridizes to the "RIP" nucleic acid under low stringency conditions,
   (viii) "TRADD" (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions,
   (ix) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,
   (x) "FLJ10179 (KIAA1794)" (SEQ ID No:19) or a functionally active derivative thereof, or a functionally active fragment thereof, or a homolog thereof, or a variant of "FLJ10179 (KIAA1794)" encoded by a nucleic acid that hybridizes to the "FLJ10179 (KIAA1794)" nucleic acid under low stringency conditions,
   (xi) "HYPOTHETICAL PROTEIN PRO1855" (SEQ ID No:20) or a functionally active derivative thereof, or a functionally active fragment thereof, or a homolog thereof, or a variant of "HYPOTHETICAL PROTEIN PRO1855" encoded by a nucleic acid that hybridizes to the "HYPOTHETICAL PROTEIN PRO1855" nucleic acid under low stringency conditions,
   (xii) (iv) "TNFR1" (SEQ ID No:21) or a functionally active derivative thereof, or a functionally active fragment thereof, or a homolog thereof, or a variant of "TNRF1" encoded by a nucleic acid that hybridizes to the "TNRF1" nucleic acid under low stringency conditions,
   (xiii) "FADD" (SEQ ID No:22) or a functionally active derivative thereof, or a functionally active fragment thereof, or a homolog thereof, or a variant of "FADD" encoded by a nucleic acid that hybridizes to the "FADD" nucleic acid under low stringency conditions,
   (xiv) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (xv) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (xvi) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (xvii) "c-IAP2" (SEQ ID No:26) or a functionally active derivative thereof, or a functionally active fragment thereof, or a homolog thereof, or a variant of "c-IAP2" encoded by a nucleic acid that hybridizes to the "c-IAP2" nucleic acid under low stringency conditions,
   (xviii) "PROTEIN-L-ISOASPARTATE(D-ASPARTATE) O-METHYLTRANSFERASE" (SEQ ID No:27) or a functionally active derivative thereof, or a functionally active fragment thereof, or a homolog thereof, or a variant of "PROTEIN-L-ISOASPARTATE(D-ASPARTATE) O-METHYLTRANSFERASE" encoded by a nucleic acid that hybridizes to the "PROTEIN-L-ISOASPARTATE(D-ASPARTATE) O-METHYLTRANSFERASE" nucleic acid under low stringency conditions,
   (xix) "UDP-GLUCOSE:GLYCOPROTEIN GLUCOSYLTRANSFERASE 1 PRECURSOR" (SEQ ID No:28) or a functionally active derivative thereof, or a functionally active fragment thereof, or a homolog thereof, or a variant of "UDP-GLUCOSE:GLYCOPROTEIN GLUCOSYLTRANSFERASE 1 PRECURSOR" encoded by a nucleic acid that hybridizes to the "UDP-GLUCOSE:GLYCOPROTEIN GLUCOSYLTRANSFERASE 1 PRECURSOR" nucleic acid under low stringency conditions,
   (xx) "CDNA FLJ14515 FIS, CLONE NT2RM1000800, WEAKLY SIMILAR TO MUS MUSCULUS PARTIAL B-IND1 PROTEIN" (SEQ ID No:29) or a functionally active derivative thereof, or a functionally active fragment thereof, or a homolog thereof, or a variant of "CDNA FLJ14515 FIS, CLONE NT2RM1000800, WEAKLY SIMILAR TO MUS MUSCULUS PARTIAL B-IND1 PROTEIN" encoded by a nucleic acid that hybridizes to the "CDNA FLJ14515 FIS, CLONE NT2RM1000800, WEAKLY SIMILAR TO MUS MUSCULUS PARTIAL B-IND1 PROTEIN" nucleic acid under low stringency conditions,
   (xxi) "KIAA1040 PROTEIN" (SEQ ID No:30) or a functionally active derivative thereof, or a functionally active fragment thereof, or a homolog thereof, or a variant of "KIAA1040 PROTEIN" encoded by a nucleic acid that hybridizes to the "KIAA1040 PROTEIN" nucleic acid under low stringency conditions,
   (xxii) "ADENOSYLHOMOCYSTEINASE" (SEQ ID No:31) or a functionally active derivative thereof, or a functionally active fragment thereof, or a homolog thereof, or a variant of "ADENOSYLHOMOCYSTEINASE" encoded by a nucleic acid that hybridizes to the "ADENOSYLHOMOCYSTEINASE" nucleic acid under low stringency conditions,
   (xxiii) "ATAXIA-TELANGIECTASIA GROUP D-ASSOCIATED PROTEIN" (SEQ ID No:32) or a functionally active derivative thereof, or a functionally active fragment thereof, or a homolog thereof, or a variant of "ATAXIA-TELANGIECTASIA GROUP D-ASSOCIATED PROTEIN" encoded by a nucleic acid that hybridizes to the "ATAXIA-TELANGIECTASIA GROUP D-ASSOCIATED PROTEIN" nucleic acid under low stringency conditions,
   (xxiv) "MACROPHAGE MIGRATION INHIBITORY FACTOR" (SEQ ID No:33) or a functionally active derivative thereof, or a functionally active fragment thereof, or a homolog thereof, or a variant of "MACROPHAGE MIGRATION INHIBITORY FACTOR" encoded by a nucleic acid that hybridizes to the "MACROPHAGE MIGRATION INHIBITORY FACTOR" nucleic acid under low stringency conditions,
   (xxv) "MONOCARBOXYLATE TRANSPORTER 1" (SEQ ID No:34) or a functionally active derivative thereof, or a functionally active fragment thereof, or a homolog thereof, or a variant of "MONOCARBOXYLATE TRANSPORTER 1" encoded by a nucleic acid that hybridizes to the "MONOCARBOXYLASE TRANSPORTER 1" nucleic acid under low stringency conditions,
   (xxvi) "c-IAP1" (SEQ ID No:35) or a functionally active derivative thereof, or a functionally active fragment thereof, or a homolog thereof, or a variant of "c-IAP1" encoded by a nucleic acid that hybridizes to the "c-IAP1" nucleic acid under low stringency conditions,
   (xxvii) "ANNEXIN II" (SEQ ID No:36) or a functionally active derivative thereof, or a functionally active fragment thereof, or a homolog thereof, or a variant of "ANNEXIN II" encoded by a nucleic acid that hybridizes to the "ANNEXIN II" nucleic acid under low stringency conditions,
   (xxviii) "NAD(P)-DEPENDENT STEROID DEHYDROGENASE" (SEQ ID No:37) or a functionally active derivative thereof, or a functionally active fragment thereof, or a homolog thereof, or a variant of "NAD(P)-DEPENDENT STEROID DEHYDROGENASE" encoded by a nucleic acid that hybridizes to the "NAD(P)-DEPENDENT STEROID DEHYDROGENASE" nucleic acid under low stringency conditions,
   (xxix) "PEPTIDYL-PROLYL CIS-TRANS ISOMERASE B PRECURSOR" (SEQ ID No:38) or a functionally active derivative thereof, or a functionally active fragment thereof, or a homolog thereof, or a variant of "PEPTIDYL-PROLYL CIS-TRANS ISOMERASE B PRECURSOR" encoded by a nucleic acid that hybridizes to the "PEPTIDYL-PROLYL CIS-TRANS ISOMERASE B PRECURSOR" nucleic acid under low stringency conditions,
   (xxx) "CARBAMOYL-PHOSPHATE SYNTHASE [AMMONIA], MITOCHONDRIAL PRECURSOR" (SEQ ID No:39) or a functionally active derivative thereof, or a functionally active fragment thereof, or a homolog thereof, or a variant of "CARBAMOYL-PHOSPHATE SYNTHASE [AMMONIA], MITOCHONDRIAL PRECURSOR" encoded by a nucleic acid that hybridizes to the "CARBAMOYL-PHOSPHATE SYNTHASE [AMMONIA], MITOCHONDRIAL PRECURSOR" nucleic acid under low stringency conditions,
   (xxxi) "ZINC-FINGER PROTEIN RFP" (SEQ ID No:40) or a functionally active derivative thereof, or a functionally active fragment thereof, or a homolog thereof, or a variant of "ZINC-FINGER PROTEIN RFP" encoded by a nucleic acid that hybridizes to the "ZINC-FINGER PROTEIN RFP" nucleic acid under low stringency conditions,
   (xxxii) "52 KDA PROTEIN" (SEQ ID No:41) or a functionally active derivative thereof, or a functionally active fragment thereof, or a homolog thereof, or a variant of "52 KDA PROTEIN" encoded by a nucleic acid that hybridizes to the "52 KDA PROTEIN" nucleic acid under low stringency conditions,
   (xxxiii) "SIMILAR TO SUBTILISIN-LIKE SERINE PROTEINASE" (SEQ ID No:42) or a functionally active derivative thereof, or a functionally active fragment thereof, or a homolog thereof, or a variant of "SIMILAR TO SUBTILISIN-LIKE SERINE PROTEINASE" encoded by a nucleic acid that hybridizes to the "SIMILAR TO SUBTILISIN-LIKE SERINE PROTEINASE" nucleic acid under low stringency conditions,
   (xxxiv) "ANTILEUKOPROTEINASE 1 PRECURSOR" (SEQ ID No:43) or a functionally active derivative thereof, or a functionally active fragment thereof, or a homolog thereof, or a variant of "ANTILEUKOPROTEINASE 1 PRECURSOR" encoded by a nucleic acid that hybridizes to the "ANTILEUKOPROTEINASE 1 PRECURSOR" nucleic acid under low stringency conditions,
   (xxxv) "7-DEHYDROCHOLESTEROL REDUCTASE" (SEQ ID No:44) or a functionally active derivative thereof, or a functionally active fragment thereof, or a homolog thereof, or a variant of "7-DEHYDROCHOLESTEROL REDUCTASE" encoded by a nucleic acid that hybridizes to the "7-DEHYDROCHOLESTEROL REDUCTASE" nucleic acid under low stringency conditions,
   (xxxvi) "HYPOTHETICAL 82.0 KDA PROTEIN" (SEQ ID No:45) or a functionally active derivative thereof, or a functionally active fragment thereof, or a homolog thereof, or a variant of "HYPOTHETICAL 82.0 KDA PROTEIN" encoded by a nucleic acid that hybridizes to the "HYPOTHETICAL 82.0 KDA PROTEIN" nucleic acid under low stringency conditions,
   (xxxvii) "CDNA FLJ14601 FIS, CLONE NT2RP1000124" (SEQ ID No:46) or a functionally active derivative thereof, or a functionally active fragment thereof, or a homolog thereof, or a variant of "CDNA FLJ14601 FIS, CLONE NT2RP1000124" encoded by a nucleic acid that hybridizes to the "CDNA FLJ14601 FIS, CLONE NT2RP1000124" nucleic acid under low stringency conditions,
   (xxxviii) "TRANSCRIPTION FACTOR AP-2 γ" (SEQ ID No:47) or a functionally active derivative thereof, or a functionally active fragment thereof, or a homolog thereof, or a variant of "TRANSCRIPTION FACTOR AP-2 γ" encoded by a nucleic acid that hybridizes to the "TRANSCRIPTION FACTOR AP-2 γ" nucleic acid under low stringency conditions,
   (xxxix) "SERINE/THREONINE PROTEIN PHOSPHATASE 2A, CATALYTIC SUBUNIT, β ISOFORM" (SEQ ID No:48) or a functionally active derivative thereof, or a functionally active fragment thereof, or a homolog thereof, or a variant of "SERINE/THREONINE PROTEIN PHOSPHATASE 2A, CATALYTIC SUBUNIT, β ISOFORM" encoded by a nucleic acid that hybridizes to the "SERINE/THREONINE PROTEIN PHOSPHATASE 2A, CATALYTIC SUBUNIT, β ISOFORM" nucleic acid under low stringency conditions,
   (xl) "SEVENTRANSMEMBRANE-DOMAIN PROTEIN" (SEQ ID No:49) or a functionally active derivative thereof, or a functionally active fragment thereof, or a homolog thereof, or a variant of "SEVENTRANSMEMBRANE-DOMAIN PROTEIN" encoded by a nucleic acid that hybridizes to the "SEVENTRANSMEMBRANE-DOMAIN PROTEIN" nucleic acid under low stringency conditions,
   (xli) "SERPIN B11" (SEQ ID No:50) or a functionally active derivative thereof,or a functionally active fragement thereof, or a homolog thereof, or a variant of "SERPIN B11" that hybridizes to the "SERPIN B11" nucleic acid under low stringency conditions, and
   (xlii) "KIAA1228 PROTEIN" (SEQ ID No:51) or a functionally active derivative thereof, or a functionally active fragment thereof, or a homolog thereof, or a variant of "KIAA1228 PROTEIN" encoded by a nucleic acid that hybridizes to the "KIAA1228 PROTEIN" nucleic acid under low stringency conditions.
4. The protein complex according to No. 1 selected from complex (I) comprising all but 1 - 30 of the following proteins:
   (i) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,
   (ii) "FLJ10179 (KIAA1794)" (SEQ ID No:19) or a functionally active derivative thereof, or a functionally active fragment thereof, or a homolog thereof, or a variant of "FLJ10179 (KIAA1794)" encoded by a nucleic acid that hybridizes to the "FLJ10179 (KIAA1794)" nucleic acid under low stringency conditions,
   (iii) "HYPOTHETICAL PROTEIN PRO1855" (SEQ ID No:20) or a functionally active derivative thereof, or a functionally active fragment thereof, or a homolog thereof, or a variant of "HYPOTHETICAL PROTEIN PRO1855" encoded by a nucleic acid that hybridizes to the "HYPOTHETICAL PROTEIN PRO1855" nucleic acid under low stringency conditions,
   (iv) (iv) "TNFR1" (SEQ ID No:21) or a functionally active derivative thereof, or a functionally active fragment thereof, or a homolog thereof, or a variant of "TNRF1" encoded by a nucleic acid that hybridizes to the "TNRF1" nucleic acid under low stringency conditions,
   (v) "PROTEIN-L-ISOASPARTATE(D-ASPARTATE) O-METHYLTRANSFERASE" (SEQ ID No:27) or a functionally active derivative thereof, or a functionally active fragment thereof, or a homolog thereof, or a variant of "PROTEIN-L-ISOASPARTATE(D-ASPARTATE) O-METHYLTRANSFERASE" encoded by a nucleic acid that hybridizes to the "PROTEIN-L-ISOASPARTATE(D-ASPARTATE) O-METHYLTRANSFERASE" nucleic acid under low stringency conditions,
   (vi) "KIAA1040 PROTEIN" (SEQ ID No:30) or a functionally active derivative thereof, or a functionally active fragment thereof, or a homolog thereof, or a variant of "KIAA1040 PROTEIN" encoded by a nucleic acid that hybridizes to the "KIAA1040 PROTEIN" nucleic acid under low stringency conditions,
   (vii) "MACROPHAGE MIGRATION INHIBITORY FACTOR" (SEQ ID No:33) or a functionally active derivative thereof, or a functionally active fragment thereof, or a homolog thereof, or a variant of "MACROPHAGE MIGRATION INHIBITORY FACTOR" encoded by a nucleic acid that hybridizes to the "MACROPHAGE MIGRATION INHIBITORY FACTOR" nucleic acid under low stringency conditions,
   (viii) "ANNEXIN II" (SEQ ID No:36) or a functionally active derivative thereof, or a functionally active fragment thereof, or a homolog thereof, or a variant of "ANNEXIN II" encoded by a nucleic acid that hybridizes to the "ANNEXIN II" nucleic acid under low stringency conditions,
   (ix) "CARBAMOYL-PHOSPHATE SYNTHASE [AMMONIA], MITOCHONDRIAL PRECURSOR" (SEQ ID No:39) or a functionally active derivative thereof, or a functionally active fragment thereof, or a homolog thereof, or a variant of "CARBAMOYL-PHOSPHATE SYNTHASE [AMMONIA], MITOCHONDRIAL PRECURSOR" encoded by a nucleic acid that hybridizes to the "CARBAMOYL-PHOSPHATE SYNTHASE [AMMONIA], MITOCHONDRIAL PRECURSOR" nucleic acid under low stringency conditions,
   (x) "ZINC-FINGER PROTEIN RFP" (SEQ ID No:40) or a functionally active derivative thereof, or a functionally active fragment thereof, or a homolog thereof, or a variant of "ZINC-FINGER PROTEIN RFP" encoded by a nucleic acid that hybridizes to the "ZINC-FINGER PROTEIN RFP" nucleic acid under low stringency conditions,
   (xi) "52 KDA PROTEIN" (SEQ ID No:41) or a functionally active derivative thereof, or a functionally active fragment thereof, or a homolog thereof, or a variant of "52 KDA PROTEIN" encoded by a nucleic acid that hybridizes to the "52 KDA PROTEIN" nucleic acid under low stringency conditions,
   (xii) "SIMILAR TO SUBTILISIN-LIKE SERINE PROTEINASE" (SEQ ID No:42) or a functionally active derivative thereof, or a functionally active fragment thereof, or a homolog thereof, or a variant of "SIMILAR TO SUBTILISIN-LIKE SERINE PROTEINASE" encoded by a nucleic acid that hybridizes to the "SIMILAR TO SUBTILISIN-LIKE SERINE PROTEINASE" nucleic acid under low stringency conditions,
   (xiii) "ANTILEUKOPROTEINASE 1 PRECURSOR" (SEQ ID No:43) or a functionally active derivative thereof, or a functionally active fragment thereof, or a homolog thereof, or a variant of "ANTILEUKOPROTEINASE 1 PRECURSOR" encoded by a nucleic acid that hybridizes to the "ANTILEUKOPROTEINASE 1 PRECURSOR" nucleic acid under low stringency conditions,
   (xiv) "7-DEHYDROCHOLESTEROL REDUCTASE" (SEQ ID No:44) or a functionally active derivative thereof, or a functionally active fragment thereof, or a homolog thereof, or a variant of "7-DEHYDROCHOLESTEROL REDUCTASE" encoded by a nucleic acid that hybridizes to the "7-DEHYDROCHOLESTEROL REDUCTASE" nucleic acid under low stringency conditions,
   (xv) "HYPOTHETICAL 82.0 KDA PROTEIN" (SEQ ID No:45) or a functionally active derivative thereof, or a functionally active fragment thereof, or a homolog thereof, or a variant of "HYPOTHETICAL 82.0 KDA PROTEIN" encoded by a nucleic acid that hybridizes to the "HYPOTHETICAL 82.0 KDA PROTEIN" nucleic acid under low stringency conditions,
   (xvi) "SERPIN B11" (SEQ ID No:50) or a functionally active derivative thereof,or a functionally active fragement thereof, or a homolog thereof, or a variant of "SERPIN B11" that hybridizes to the "SERPIN B11" nucleic acid under low stringency conditions, and
   (xvii) "KIAA1228 PROTEIN" (SEQ ID No:51) or a functionally active derivative thereof, or a functionally active fragment thereof, or a homolog thereof, or a variant of "KIAA1228 PROTEIN" encoded by a nucleic acid that hybridizes to the "KIAA1228 PROTEIN" nucleic acid under low stringency conditions;
   and a protein complex selected from complex (IV) and comprising the following proteins:
   (i) "CDNA FLJ20733 FIS, CLONE HEP08550" (SEQ ID No:10) or a functionally active derivative thereof, or a functionally active fragment thereof, or a homolog thereof, or a variant of "CDNA FLJ20733 FIS, CLONE HEP08550" encoded by a nucleic acid that hybridizes to the "CDNA FLJ20733 FIS, CLONE HEP08550" nucleic acid under low stringency conditions,
   (ii) "ANNEXIN I" (SEQ ID No: 11) or a functionally active derivative thereof, or a functionally active fragment thereof, or a homolog thereof, or a variant of "ANEXIN I" encoded by a nucleic acid that hybridizes to the "ANNEXIN I" nucleic acid under low stringency conditions,
   (iii) "THIOREDOXIN" (SEQ ID No:12) or a functionally active derivative thereof, or a functionally active fragment thereof, or a homolog thereof, or a variant of "THIOREDOXIN" encoded by a nucleic acid that hybridizes to the "THIOREDOXIN" nucleic acid under low stringency conditions,
   (iv) "PEROXIREDOXIN 1" (SEQ ID No:13) or a functionally active derivative thereof, or a functionally active fragment thereof, or a homolog thereof, or a variant of "PEROXiREDOXIN 1" encoded by a nucleic acid that hybridizes to the "PEROXIREDOXIN" nucleic acid under low stringency conditions,
   (v) "TRAF1" (SEQ ID No:14) or a functionally active derivative thereof, or a functionally active fragment thereof, or a homolog thereof, or a variant of "TRAF1" encoded by a nucleic acid that hybridizes to the "TRAF1" nucleic acid under low stringency conditions,
   (vi) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions,
   (vii) "RIP" (SEQ ID No:16) or a functionally active derivative thereof, or a functionally active fragment thereof, or a homolog thereof, or a variant of "RIP" encoded by a nucleic acid that hybridizes to the "RIP" nucleic acid under low stringency conditions,
   (viii) "TRADD" (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions,
   (ix) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,
   (x) "FLJ10179 (KIAA1794)" (SEQ ID No:19) or a functionally active derivative thereof, or a functionally active fragment thereof, or a homolog thereof, or a variant of "FLJ10179 (KIAA1794)" encoded by a nucleic acid that hybridizes to the "FLJ10179 (KIAA1794)" nucleic acid under low stringency conditions,
   (xi) "HYPOTHETICAL PROTEIN PRO1855" (SEQ ID No:20) or a functionally active derivative thereof, or a functionally active fragment thereof, or a homolog thereof, or a variant of "HYPOTHETICAL PROTEIN PRO1855" encoded by a nucleic acid that hybridizes to the "HYPOTHETICAL PROTEIN PRO1855" nucleic acid under low stringency conditions,
   (xii) "TNFR1" (SEQ ID No:21) or a functionally active derivative thereof, or a functionally active fragment thereof, or a homolog thereof, or a variant of "TNRF1" encoded by a nucleic acid that hybridizes to the "TNRF1" nucleic acid under low stringency conditions,
   (xiii) "FADD" (SEQ ID No:22) or a functionally active derivative thereof, or a functionally active fragment thereof, or a homolog thereof, or a variant of "FADD" encoded by a nucleic acid that hybridizes to the "FADD" nucleic acid under low stringency conditions,
   (xiv) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (xv) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (xvi) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (xvii) "c-IAP2" (SEQ ID No:26) or a functionally active derivative thereof, or a functionally active fragment thereof, or a homolog thereof, or a variant of "c-IAP2" encoded by a nucleic acid that hybridizes to the "c-IAP2" nucleic acid under low stringency conditions,
   (xviii) "PROTEIN-L-ISOASPARTATE(D-ASPARTATE) O-METHYLTRANSFERASE" (SEQ ID No:27) or a functionally active derivative thereof, or a functionally active fragment thereof, or a homolog thereof, or a variant of "PROTEIN-L-ISOASPARTATE(D-ASPARTATE) O-METHYLTRANSFERASE" encoded by a nucleic acid that hybridizes to the "PROTEIN-L-ISOASPARTATE(D-ASPARTATE) O-METHYLTRANSFERASE" nucleic acid under low stringency conditions,
   (xix) "UDP-GLUCOSE:GLYCOPROTEIN GLUCOSYLTRANSFERASE 1 PRECURSOR" (SEQ ID No:28) or a functionally active derivative thereof, or a functionally active fragment thereof, or a homolog thereof, or a variant of "UDP-GLUCOSE:GLYCOPROTEIN GLUCOSYLTRANSFERASE 1 PRECURSOR" encoded by a nucleic acid that hybridizes to the "UDP-GLUCOSE:GLYCOPROTEIN GLUCOSYLTRANSFERASE 1 PRECURSOR" nucleic acid under low stringency conditions,
   (xx) "CDNA FLJ14515 FIS, CLONE NT2RM1000800, WEAKLY SIMILAR TO MUS MUSCULUS PARTIAL B-IND1 PROTEIN" (SEQ ID No:29) or a functionally active derivative thereof, or a functionally active fragment thereof, or a homolog thereof, or a variant of "CDNA FLJ14515 FIS, CLONE NT2RM1000800, WEAKLY SIMILAR TO MUS MUSCULUS PARTIAL B-IND1 PROTEIN" encoded by a nucleic acid that hybridizes to the "CDNA FLJ14515 FIS, CLONE NT2RM1000800, WEAKLY SIMILAR TO MUS MUSCULUS PARTIAL B-IND1 PROTEIN" nucleic acid under low stringency conditions,
   (xxi) "KIAA1040 PROTEIN" (SEQ ID No:30) or a functionally active derivative thereof, or a functionally active fragment thereof, or a homolog thereof, or a variant of "KIAA1040 PROTEIN" encoded by a nucleic acid that hybridizes to the "KIAA1040 PROTEIN" nucleic acid under low stringency conditions,
   (xxii) "ADENOSYLHOMOCYSTEINASE" (SEQ ID No:31) or a functionally active derivative thereof, or a functionally active fragment thereof, or a homolog thereof, or a variant of "ADENOSYLHOMOCYSTEINASE" encoded by a nucleic acid that hybridizes to the "ADENOSYLHOMOCYSTEINASE" nucleic acid under low stringency conditions,
   (xxiii) "ATAXIA-TELANGIECTASIA GROUP D-ASSOCIATED PROTEIN" (SEQ ID No:32) or a functionally active derivative thereof, or a functionally active fragment thereof, or a homolog thereof, or a variant of "ATAXIA-TELANGIECTASIA GROUP D-ASSOCIATED PROTEIN" encoded by a nucleic acid that hybridizes to the "ATAXIA-TELANGIECTASIA GROUP D-ASSOCIATED PROTEIN" nucleic acid under low stringency conditions,
   (xxiv) "MACROPHAGE MIGRATION INHIBITORY FACTOR" (SEQ ID No:33) or a functionally active derivative thereof, or a functionally active fragment thereof, or a homolog thereof, or a variant of "MACROPHAGE MIGRATION INHIBITORY FACTOR" encoded by a nucleic acid that hybridizes to the "MACROPHAGE MIGRATION INHIBITORY FACTOR" nucleic acid under low stringency conditions,
   (xxv) "MONOCARBOXYLATE TRANSPORTER 1" (SEQ ID No:34) or a functionally active derivative thereof, or a functionally active fragment thereof, or a homolog thereof, or a variant of "MONOCARBOXYLATE TRANSPORTER 1" encoded by a nucleic acid that hybridizes to the "MONOCARBOXYLASE TRANSPORTER 1" nucleic acid under low stringency conditions,
   (xxvi) "c-IAP1" (SEQ ID No:35) or a functionally active derivative thereof, or a functionally active fragment thereof, or a homolog thereof, or a variant of "c-IAP1" encoded by a nucleic acid that hybridizes to the "c-IAP1" nucleic acid under low stringency conditions,
   (xxvii) "ANNEXIN II" (SEQ ID No:36) or a functionally active derivative thereof, or a functionally active fragment thereof, or a homolog thereof, or a variant of "ANNEXIN II" encoded by a nucleic acid that hybridizes to the "ANNEXIN II" nucleic acid under low stringency conditions,
   (xxviii) "NAD(P)-DEPENDENT STEROID DEHYDROGENASE" (SEQ ID No:37) or a functionally active derivative thereof, or a functionally active fragment thereof, or a homolog thereof, or a variant of "NAD(P)-DEPENDENT STEROID DEHYDROGENASE" encoded by a nucleic acid that hybridizes to the "NAD(P)-DEPENDENT STEROID DEHYDROGENASE" nucleic acid under low stringency conditions,
   (xxix) "PEPTIDYL-PROLYL CIS-TRANS ISOMERASE B PRECURSOR" (SEQ ID No:38) or a functionally active derivative thereof, or a functionally active fragment thereof, or a homolog thereof, or a variant of "PEPTIDYL-PROLYL CIS-TRANS ISOMERASE B PRECURSOR" encoded by a nucleic acid that hybridizes to the "PEPTIDYL-PROLYL CIS-TRANS ISOMERASE B PRECURSOR" nucleic acid under low stringency conditions,
   (xxx) "CARBAMOYL-PHOSPHATE SYNTHASE [AMMONIA], MITOCHONDRIAL PRECURSOR" (SEQ ID No:39) or a functionally active derivative thereof, or a functionally active fragment thereof, or a homolog thereof, or a variant of "CARBAMOYL-PHOSPHATE SYNTHASE [AMMONIA], MITOCHONDRIAL PRECURSOR" encoded by a nucleic acid that hybridizes to the "CARBAMOYL-PHOSPHATE SYNTHASE [AMMONIA], MITOCHONDRIAL PRECURSOR" nucleic acid under low stringency conditions,
   (xxxi) "ZINC-FINGER PROTEIN RFP" (SEQ ID No:40) or a functionally active derivative thereof, or a functionally active fragment thereof, or a homolog thereof, or a variant of "ZINC-FINGER PROTEIN RFP" encoded by a nucleic acid that hybridizes to the "ZINC-FINGER PROTEIN RFP" nucleic acid under low stringency conditions,
   (xxxii) "52 KDA PROTEIN" (SEQ ID No:41) or a functionally active derivative thereof, or a functionally active fragment thereof, or a homolog thereof, or a variant of "52 KDA PROTEIN" encoded by a nucleic acid that hybridizes to the "52 KDA PROTEIN" nucleic acid under low stringency conditions,
   (xxxiii) "SIMILAR TO SUBTILISIN-LIKE SERINE PROTEINASE" (SEQ ID No:42) or a functionally active derivative thereof, or a functionally active fragment thereof, or a homolog thereof, or a variant of "SIMILAR TO SUBTILISIN-LIKE SERINE PROTEINASE" encoded by a nucleic acid that hybridizes to the "SIMILAR TO SUBTILISIN-LIKE SERINE PROTEINASE" nucleic acid under low stringency conditions,
   (xxxiv) "ANTILEUKOPROTEINASE 1 PRECURSOR" (SEQ ID No:43) or a functionally active derivative thereof, or a functionally active fragment thereof, or a homolog thereof, or a variant of "ANTILEUKOPROTEINASE 1 PRECURSOR" encoded by a nucleic acid that hybridizes to the "ANTILEUKOPROTEINASE 1 PRECURSOR" nucleic acid under low stringency conditions,
   (xxxv) "7-DEHYDROCHOLESTEROL REDUCTASE" (SEQ ID No:44) or a functionally active derivative thereof, or a functionally active fragment thereof, or a homolog thereof, or a variant of "7-DEHYDROCHOLESTEROL REDUCTASE" encoded by a nucleic acid that hybridizes to the "7-DEHYDROCHOLESTEROL REDUCTASE" nucleic acid under low stringency conditions,
   (xxxvi) "HYPOTHETICAL 82.0 KDA PROTEIN" (SEQ ID No:45) or a functionally active derivative thereof, or a functionally active fragment thereof, or a homolog thereof, or a variant of "HYPOTHETICAL 82.0 KDA PROTEIN" encoded by a nucleic acid that hybridizes to the "HYPOTHETICAL 82.0 KDA PROTEIN" nucleic acid under low stringency conditions,
   (xxxvii) "CDNA FLJ14601 FIS, CLONE NT2RP1000124" (SEQ ID No:46) or a functionally active derivative thereof, or a functionally active fragment thereof, or a homolog thereof, or a variant of "CDNA FLJ14601 FIS, CLONE NT2RP1000124" encoded by a nucleic acid that hybridizes to the "CDNA FLJ14601 FIS, CLONE NT2RP1000124" nucleic acid under low stringency conditions,
   (xxxviii) "TRANSCRIPTION FACTOR AP-2 γ" (SEQ ID No:47) or a functionally active derivative thereof, or a functionally active fragment thereof, or a homolog thereof, or a variant of "TRANSCRIPTION FACTOR AP-2 γ" encoded by a nucleic acid that hybridizes to the "TRANSCRIPTION FACTOR AP-2 γ" nucleic acid under low stringency conditions,
   (xxxix) "SERINE/THREONINE PROTEIN PHOSPHATASE 2A, CATALYTIC SUBUNIT, β ISOFORM" (SEQ ID No:48) or a functionally active derivative thereof, or a functionally active fragment thereof, or a homolog thereof, or a variant of "SERINE/THREONINE PROTEIN PHOSPHATASE 2A, CATALYTIC SUBUNIT, β ISOFORM" encoded by a nucleic acid that hybridizes to the "SERINE/THREONINE PROTEIN PHOSPHATASE 2A, CATALYTIC SUBUNIT, β ISOFORM" nucleic acid under low stringency conditions,
   (xl) "SEVENTRANSMEMBRANE-DOMAIN PROTEIN" (SEQ ID No:49) or a functionally active derivative thereof, or a functionally active fragment thereof, or a homolog thereof, or a variant of "SEVENTRANSMEMBRANE-DOMAIN PROTEIN" encoded by a nucleic acid that hybridizes to the "SEVENTRANSMEMBRANE-DOMAIN PROTEIN" nucleic acid under low stringency conditions,
   (xli) "SERPIN B11" (SEQ ID No:50) or a functionally active derivative thereof,or a functionally active fragement thereof, or a homolog thereof, or a variant of "SERPIN B11" that hybridizes to the "SERPIN B11" nucleic acid under low stringency conditions, and
   (xlii) "KIAA1228 PROTEIN" (SEQ ID No:51) or a functionally active derivative thereof, or a functionally active fragment thereof, or a homolog thereof, or a variant of "KIAA1228 PROTEIN" encoded by a nucleic acid that hybridizes to the "KIAA1228 PROTEIN" nucleic acid under low stringency conditions.
5. The complex of any of No. 1 - 4 comprising a functionally active derivative of said first protein and/or a functionally active derivative of said second protein, wherein the functionally active derivative is a fusion protein comprising said first protein or said second protein fused to an amino acid sequence different from the first protein or second protein, respectively.
6. The complex of No. 5 wherein the functionally active derivative is a fusion protein comprising said first protein or said second protein fused to an affinity tag or label.
7. The complex of any of No. 1 - 4 comprising a fragment of said first protein and/or a fragment of said second protein, which fragment binds to another protein component of said complex.
8. The complex of any of No. 1 - 7 that is involved in the Receptor activity.
9. A process for preparing complex of any of No. 1 - 8 and optionally the components thereof comprising the following steps:
   the expressing a protein (bait) of the complex, preferably a tagged protein, in a target
   cell, isolating the protein complex which is attached to the bait protein, and optionally disassociating the protein complex and isolating the individual complex members.
10. The process according to No. 9 wherein the tagged protein comprises two different tags which allow two separate affinity purification steps.
11. The process according to any of No. 9 - 10 wherein the two tags are separated by a cleavage site for a protease.
12. Component of the TNFR1 complex obtainable by a process according to any of No. 9 to 11.
13. Protein of the TNFR1 complex selected from
   (i) "FLJ10179 (KIAA1794)" (SEQ ID No:19) or a functionally active derivative thereof, or a functionally active fragment thereof, or a homolog thereof, or a variant of "FLJ10179 (KIAA1794)" encoded by a nucleic acid that hybridizes to the "FLJ10179 (KIAA1794)" nucleic acid under low stringency conditions,
   (ii) "HYPOTHETICAL PROTEIN PRO1855" (SEQ ID No:20) or a functionally active derivative thereof, or a functionally active fragment thereof, or a homolog thereof, or a variant of "HYPOTHETICAL PROTEIN PRO1855" encoded by a nucleic acid that hybridizes to the "HYPOTHETICAL PROTEIN PRO1855" nucleic acid under low stringency conditions,
   (iii) "KIAA1040 PROTEIN" (SEQ ID No:30) or a functionally active derivative thereof, or a functionally active fragment thereof, or a homolog thereof, or a variant of "KIAA1040 PROTEIN" encoded by a nucleic acid that hybridizes to the "KIAA1040 PROTEIN" nucleic acid under low stringency conditions, and
   (iv) "KIAA1228 PROTEIN" (SEQ ID No:51) or a functionally active derivative thereof, or a functionally active fragment thereof, or a homolog thereof, or a variant of "KIAA1228 PROTEIN" encoded by a nucleic acid that hybridizes to the "KIAA1228 PROTEIN" nucleic acid under low stringency conditions,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1.5 hours at 55°C, and washing in a buffer consisting of 2X SSC, 25 mM Tris-HCl (pH 7.4), 5 mM EDTA, and 0.1 % SDS for 1.5 hours at 60°C.
14. Nucleic acid encoding a protein according to No. 13.
15. Construct, preferably a vector construct, comprising (a) a nucleic acid according to No. 14 and at least one further nucleic acid which is normally not associated with said nucleic acid,or(b) at least two separate nucleic acid sequences each encoding a different protein, or a functionally active fragment or a functionally active derivative thereof at least one of said proteins, or functionally active fragments or functionally active derivative thereof being selected from the first group of proteins according to No. 1(a) and at least one of said proteins, or functionally active fragments or functionally active derivative thereof being selected from the second group of proteins according to No. 1(b).
16. Host cell, containing a vector comprising at least one of the nucleic acid of No. 14 and/or a construct of No. 15 or containing several vectors each comprising at least the nucleic acid sequence encoding at least one of the proteins, or functionally active fragments or functionally active derivatives thereof selected from the first group of proteins according to No. 1(a) and the proteins, or functionally active fragments or functionally active derivatives thereof selected from the second group of proteins according to No. 1(b).
17. An antibody or a fragment of said antibody containing the binding domain thereof, selected from an antibody or fragment thereof, which binds the complex of any of No. 1-8 and which does not bind any of the proteins of said complex when uncomplexed and an antibody or a fragment of said antibody which binds to any of the proteins according to No. 13.
18. A kit comprising in one or more container the complex of any of No. 1 - 8 and/or the proteins of No. 13optionally together with an antibody according to No. 17 and/or further components such as reagents and working instructions.
19. The kit according to No.18 for processing a substrate of said complex.
20. The kit according to No. 18 for the diagnosis or prognosis of a disease or a disease risk, preferentially for a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
21. Array, in which at least a complex according to any of No. 1 - 8 and/or at least one protein according to No. 14 and/or at least one antibody according to No. 17 is attached to a solid carrier.
22. A process for processing a physiological substrate of the complex comprising the step of bringing into contact a complex to any of No. 1 - 8 with said substrate, such that said substrate is processed.
23. A pharmaceutical composition comprising the protein complex of any of No. 1 to 8 and/or any of the following the proteins:
   (i) "FLJ10179 (KIAA1794)" (SEQ ID No:19) or a functionally active derivative thereof, or a functionally active fragment thereof, or a homolog thereof, or a variant of "FLJ10179 (KIAA1794)" encoded by a nucleic acid that hybridizes to the "FLJ10179 (KIAA1794)" nucleic acid under low stringency conditions,
   (ii) "HYPOTHETICAL PROTEIN PRO1855" (SEQ ID No:20) or a functionally active derivative thereof, or a functionally active fragment thereof, or a homolog thereof, or a variant of "HYPOTHETICAL PROTEIN PRO1855" encoded by a nucleic acid that hybridizes to the "HYPOTHETICAL PROTEIN PRO1855" nucleic acid under low stringency conditions,
   (iii) "KIAA1040 PROTEIN" (SEQ ID No:30) or a functionally active derivative thereof,or a functionally active fragement thereof, or a homolog thereof, or a variant of "KIAA1040 PROTEIN" that hybridizes to the "KIAA1040 PROTEIN" nucleic acid under low stringency conditions, and
   (iv) "KIAA1228 PROTEIN" (SEQ ID No:51) or a functionally active derivative thereof, or a functionally active fragment thereof, or a homolog thereof, or a variant of "KIAA1228 PROTEIN" encoded by a nucleic acid that hybridizes to the "KIAA1228 PROTEIN" nucleic acid under low stringency conditions,
   and a pharmaceutical acceptable carrier.
24. The pharmaceutical composition according to No. 23 for the treatment of diseases and disorders such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
25. A method for screening for a molecule that binds to the complex of anyone of No. 1 - 8 and/or any of the following proteins:
   (i) "FLJ10179 (KIAA1794)" (SEQ ID No:19) or a functionally active derivative thereof, or a functionally active fragment thereof, or a homolog thereof, or a variant of "FLJ10179 (KIAA1794)" encoded by a nucleic acid that hybridizes to the "FLJ10179 (KIAA1794)" nucleic acid under low stringency conditions,
   (ii) "HYPOTHETICAL PROTEIN PRO1855 (SEQ ID No:20) or a functionally active derivative thereof, or a functionally active fragment thereof, or a homolog thereof, or a variant of "HYPOTHETICAL PROTEIN PRO1855" encoded by a nucleic acid that hybridizes to the "HYPOTHETICAL PROTEIN PRO1855" nucleic acid under low stringency conditions,
   (iii) "KIAA1040 PROTEIN" (SEQ ID No:30) or a functionally active derivative thereof,or a functionally active fragement thereof, or a homolog thereof, or a variant of "KIAA1040 PROTEIN" that hybridizes to the "KIAA1040 PROTEIN" nucleic acid under low stringency conditions, and
   (iv) "KIAA1228 PROTEIN" (SEQ ID No:51) or a functionally active derivative thereof, or a functionally active fragment thereof, or a homolog thereof, or a variant of "KIAA1228 PROTEIN" encoded by a nucleic acid that hybridizes to the "KIAA1228 PROTEIN" nucleic acid under low stringency conditions, comprising the steps of
      (a) exposing said complex, or a cell or organism containing same to one or more candidate molecules; and
      (b) determinig whether said candidate molecule is bound to the complex or protein.
26. A method for screening for a molecule that modulates directly or indirectly the function, activity, composition or formation of the complex of any one of No. 1 - 8 comprising the steps of (a) exposing said complex, or a cell or organism containing TNFR1 complex to one or more candidate molecules; and
   (b) determining the amount of activity of protein components of, and/or intracellular localization of, said complex and/or the transcription level of a gene dependent on the complex and/or the abundance and/or activity of a protein or protein complex dependent on the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependent on the complex and/or the abundance and/or activity of a protein or protein complex dependent on the function of the complex and/or product of a gene dependent on the complex in the absence of said candidate molecules indicates that the molecule modulates function, activity or composition of said complex.
27. The method of No. 26, wherein the amount of said complex is determined.
28. The method of No. 26, wherein the activity of said complex is determined.
29. The method of No. 28, wherein said determining step comprises isolating from the cell or organism said complex to produce said isolated complex and contacting said isolated complex in the presence or absence of a candidate molecule with a substrate of said complex and determining the processing of said substrate is modified in the presence of said candidate molecule.
30. The method of No. 26, wherein the amount of the individual protein components of said complex are determined.
31. The method of No. 30, determining step comprises determining whether
   (i) "CDNA FLJ20733 FIS, CLONE HEP08550" (SEQ ID No:10) or a functionally active derivative thereof, or a functionally active fragment thereof, or a homolog thereof, or a variant of "CDNA FLJ20733 FIS, CLONE HEP08550" encoded by a nucleic acid that hybridizes to the "CDNA FLJ20733 FIS, CLONE HEP08550" nucleic acid under low stringency conditions, and/or
   (ii) "ANNEXIN I" (SEQ ID No:11) or a functionally active derivative thereof, or a functionally active fragment thereof, or a homolog thereof, or a variant of "ANEXIN I" encoded by a nucleic acid that hybridizes to the "ANNEXIN I" nucleic acid under low stringency conditions, and/or
   (iii) "THIOREDOXIN" (SEQ ID No:12) or a functionally active derivative thereof, or a functionally active fragment thereof, or a homolog thereof, or a variant of "THIOREDOXIN" encoded by a nucleic acid that hybridizes to the "THIOREDOXIN" nucleic acid under low stringency conditions, and/or
   (iv) "PEROXIREDOXIN 1" (SEQ ID No:13) or a functionally active derivative thereof, or a functionally active fragment thereof, or a homolog thereof, or a variant of "PEROXiREDOXIN 1" encoded by a nucleic acid that hybridizes to the "PEROXIREDOXIN" nucleic acid under low stringency conditions, and/or
   (v) "TRAF1" (SEQ ID No:14) or a functionally active derivative thereof, or a functionally active fragment thereof, or a homolog thereof, or a variant of "TRAF1" encoded by a nucleic acid that hybridizes to the "TRAF1" nucleic acid under low stringency conditions, and/or
   (vi) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions, and/or
   (vii) "RIP" (SEQ ID No:16) or a functionally active derivative thereof, or a functionally active fragment thereof, or a homolog thereof, or a variant of "RIP" encoded by a nucleic acid that hybridizes to the "RIP" nucleic acid under low stringency conditions, and/or
   (viii) "TRADD" (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions, and/or
   (ix) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions, and/or
   (x) "FLJ10179 (KIAA1794)" (SEQ ID No:19) or a functionally active derivative thereof, or a functionally active fragment thereof, or a homolog thereof, or a variant of "FLJ10179 (KIAA1794)" encoded by a nucleic acid that hybridizes to the "FLJ10179 (KIAA1794)" nucleic acid under low stringency conditions, and/or
   (xi) "HYPOTHETICAL PROTEIN PRO1855" (SEQ ID No:20) or a functionally active derivative thereof, or a functionally active fragment thereof, or a homolog thereof, or a variant of "HYPOTHETICAL PROTEIN PRO1855 encoded by a nucleic acid that hybridizes to the "HYPOTHETICAL PROTEIN PRO1855 nucleic acid under low stringency conditions, and/or
   (xii) "TNFR1" (SEQ ID No:21) or a functionally active derivative thereof, or a functionally active fragment thereof, or a homolog thereof, or a variant of "TNRF1" encoded by a nucleic acid that hybridizes to the "TNRF1" nucleic acid under low stringency conditions, and/or
   (xiii) "FADD" (SEQ ID No:22) or a functionally active derivative thereof, or a functionally active fragment thereof, or a homolog thereof, or a variant of "FADD" encoded by a nucleic acid that hybridizes to the "FADD" nucleic acid under low stringency conditions, and/or
   (xiv) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions, and/or
   (xv) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions, and/or
   (xvi) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions, and/or
   (xvii) "c-IAP2" (SEQ ID No:26) or a functionally active derivative thereof, or a functionally active fragment thereof, or a homolog thereof, or a variant of "c-IAP2" encoded by a nucleic acid that hybridizes to the "c-IAP2" nucleic acid under low stringency conditions, and/or
   (xviii) "PROTEIN-L-ISOASPARTATE(D-ASPARTATE) O-METHYLTRANSFERASE" (SEQ ID No:27) or a functionally active derivative thereof, or a functionally active fragment thereof, or a homolog thereof, or a variant of "PROTEIN-L-ISOASPARTATE(D-ASPARTATE) O-METHYLTRANSFERASE" encoded by a nucleic acid that hybridizes to the "PROTEIN-L-ISOASPARTATE(D-ASPARTATE) O-METHYLTRANSFERASE" nucleic acid under low stringency conditions, and/or
   (xix) "UDP-GLUCOSE:GLYCOPROTEIN GLUCOSYLTRANSFERASE 1 PRECURSOR" (SEQ ID No:28) or a functionally active derivative thereof, or a functionally active fragment thereof, or a homolog thereof, or a variant of "UDP-GLUCOSE:GLYCOPROTEIN GLUCOSYLTRANSFERASE 1 PRECURSOR" encoded by a nucleic acid that hybridizes to the "UDP-GLUCOSE:GLYCOPROTEIN GLUCOSYLTRANSFERASE 1 PRECURSOR" nucleic acid under low stringency conditions, and/or
   (xx) "CDNA FLJ14515 FIS, CLONE NT2RM1000800, WEAKLY SIMILAR TO MUS MUSCULUS PARTIAL B-IND1 PROTEIN" (SEQ ID No:29) or a functionally active derivative thereof, or a functionally active fragment thereof, or a homolog thereof, or a variant of "CDNA FLJ14515 FIS, CLONE NT2RM1000800, WEAKLY SIMILAR TO MUS MUSCULUS PARTIAL B-IND1 PROTEIN" encoded by a nucleic acid that hybridizes to the "CDNA FLJ14515 FIS, CLONE NT2RM1000800, WEAKLY SIMILAR TO MUS MUSCULUS PARTIAL B-IND1 PROTEIN" nucleic acid under low stringency conditions, and/or
   (xxi) "KIAA1040 PROTEIN" (SEQ ID No:30) or a functionally active derivative thereof, or a functionally active fragment thereof, or a homolog thereof, or a variant of "KIAA1040 PROTEIN" encoded by a nucleic acid that hybridizes to the "KIAA1040 PROTEIN" nucleic acid under low stringency conditions, and/or
   (xxii) "ADENOSYLHOMOCYSTElNASE" (SEQ ID No:31) or a functionally active derivative thereof, or a functionally active fragment thereof, or a homolog thereof, or a variant of "ADENOSYLHOMOCYSTEINASE" encoded by a nucleic acid that hybridizes to the "ADENOSYLHOMOCYSTEINASE" nucleic acid under low stringency conditions, and/or
   (xxiii) "ATAXIA-TELANGIECTASIA GROUP D-ASSOCIATED PROTEIN" (SEQ ID No:32) or a functionally active derivative thereof, or a functionally active fragment thereof, or a homolog thereof, or a variant of "ATAXIA-TELANGIECTASIA GROUP D-ASSOCIATED PROTEIN" encoded by a nucleic acid that hybridizes to the "ATAXIA-TELANGIECTASIA GROUP D-ASSOCIATED PROTEIN" nucleic acid under low stringency conditions, and/or
   (xxiv) "MACROPHAGE MIGRATION INHIBITORY FACTOR" (SEQ ID No:33) or a functionally active derivative thereof, or a functionally active fragment thereof, or a homolog thereof, or a variant of "MACROPHAGE MIGRATION INHIBITORY FACTOR" encoded by a nucleic acid that hybridizes to the "MACROPHAGE MIGRATION INHIBITORY FACTOR" nucleic acid under low stringency conditions, and/or
   (xxv) "MONOCARBOXYLATE TRANSPORTER 1" (SEQ ID No:34) or a functionally active derivative thereof, or a functionally active fragment thereof, or a homolog thereof, or a variant of "MONOCARBOXYLATE TRANSPORTER 1" encoded by a nucleic acid that hybridizes to the "MONOCARBOXYLASE TRANSPORTER 1" nucleic acid under low stringency conditions, and/or
   (xxvi) "c-IAP1" (SEQ ID No:35) or a functionally active derivative thereof, or a functionally active fragment thereof, or a homolog thereof, or a variant of "c-IAP1" encoded by a nucleic acid that hybridizes to the "c-IAP1" nucleic acid under low stringency conditions, and/or
   (xxvii) "ANNEXIN II" (SEQ ID No:36) or a functionally active derivative thereof, or a functionally active fragment thereof, or a homolog thereof, or a variant of "ANNEXIN II" encoded by a nucleic acid that hybridizes to the "ANNEXIN II" nucleic acid under low stringency conditions, and/or
   (xxviii) "NAD(P)-DEPENDENT STEROID DEHYDROGENASE" (SEQ ID No:37) or a functionally active derivative thereof, or a functionally active fragment thereof, or a homolog thereof, or a variant of "NAD(P)-DEPENDENT STEROID DEHYDROGENASE" encoded by a nucleic acid that hybridizes to the "NAD(P)-DEPENDENT STEROID DEHYDROGENASE" nucleic acid under low stringency conditions, and/or
   (xxix) "PEPTIDYL-PROLYL CIS-TRANS ISOMERASE B PRECURSOR" (SEQ ID No:38) or a functionally active derivative thereof, or a functionally active fragment thereof, or a homolog thereof, or a variant of "PEPTIDYL-PROLYL CIS-TRANS ISOMERASE B PRECURSOR" encoded by a nucleic acid that hybridizes to the "PEPTIDYL-PROLYL CIS-TRANS ISOMERASE B PRECURSOR" nucleic acid under low stringency conditions, and/or
   (xxx) "CARBAMOYL-PHOSPHATE SYNTHASE [AMMONIA], MITOCHONDRIAL PRECURSOR" (SEQ ID No:39) or a functionally active derivative thereof, or a functionally active fragment thereof, or a homolog thereof, or a variant of "CARBAMOYL-PHOSPHATE SYNTHASE [AMMONIA], MITOCHONDRIAL PRECURSOR" encoded by a nucleic acid that hybridizes to the "CARBAMOYL-PHOSPHATE SYNTHASE [AMMONIA], MITOCHONDRIAL PRECURSOR" nucleic acid under low stringency conditions, and/or
   (xxxi) "ZINC-FINGER PROTEIN RFP" (SEQ ID No:40) or a functionally active derivative thereof, or a functionally active fragment thereof, or a homolog thereof, or a variant of "ZINC-FINGER PROTEIN RFP" encoded by a nucleic acid that hybridizes to the "ZINC-FINGER PROTEIN RFP" nucleic acid under low stringency conditions, and/or
   (xxxii) "52 KDA PROTEIN" (SEQ ID No:41) or a functionally active derivative thereof, or a functionally active fragment thereof, or a homolog thereof, or a variant of "52 KDA PROTEIN" encoded by a nucleic acid that hybridizes to the "52 KDA PROTEIN" nucleic acid under low stringency conditions, and/or
   (xxxiii) "SIMILAR TO SUBTILISIN-LIKE SERINE PROTEINASE" (SEQ ID No:42) or a functionally active derivative thereof, or a functionally active fragment thereof, or a homolog thereof, or a variant of "SIMILAR TO SUBTILISIN-LIKE SERINE PROTEINASE" encoded by a nucleic acid that hybridizes to the "SIMILAR TO SUBTILISIN-LIKE SERINE PROTEINASE" nucleic acid under low stringency conditions, and/or
   (xxxiv) "ANTILEUKOPROTEINASE 1 PRECURSOR" (SEQ ID No:43) or a functionally active derivative thereof, or a functionally active fragment thereof, or a homolog thereof, or a variant of "ANTILEUKOPROTEINASE 1 PRECURSOR" encoded by a nucleic acid that hybridizes to the "ANTILEUKOPROTEINASE 1 PRECURSOR" nucleic acid under low stringency conditions, and/or
   (xxxv) "7-DEHYDROCHOLESTEROL REDUCTASE" (SEQ ID No:44) or a functionally active derivative thereof, or a functionally active fragment thereof, or a homolog thereof, or a variant of "7-DEHYDROCHOLESTEROL REDUCTASE" encoded by a nucleic acid that hybridizes to the "7-DEHYDROCHOLESTEROL REDUCTASE" nucleic acid under low stringency conditions, and/or
   (xxxvi) "HYPOTHETICAL 82.0 KDA PROTEIN" (SEQ ID No:45) or a functionally active derivative thereof, or a functionally active fragment thereof, or a homolog thereof, or a variant of "HYPOTHETICAL 82.0 KDA PROTEIN" encoded by a nucleic acid that hybridizes to the "HYPOTHETICAL 82.0 KDA PROTEIN" nucleic acid under low stringency conditions, and/or
   (xxxvii) "CDNA FLJ14601 FIS, CLONE NT2RP1000124" (SEQ ID No:46) or a functionally active derivative thereof, or a functionally active fragment thereof, or a homolog thereof, or a variant of "CDNA FLJ14601 FIS, CLONE NT2RP1000124" encoded by a nucleic acid that hybridizes to the "CDNA FLJ14601 FIS, CLONE NT2RP1000124" nucleic acid under low stringency conditions, and/or
   (xxxviii) "TRANSCRIPTION FACTOR AP-2 γ" (SEQ ID No:47) or a functionally active derivative thereof, or a functionally active fragment thereof, or a homolog thereof, or a variant of "TRANSCRIPTION FACTOR AP-2 γ" encoded by a nucleic acid that hybridizes to the "TRANSCRIPTION FACTOR AP-2 γ" nucleic acid under low stringency conditions, and/or
   (xxxix) "SERINE/THREONINE PROTEIN PHOSPHATASE 2A, CATALYTIC SUBUNIT, β ISOFORM" (SEQ ID No:48) or a functionally active derivative thereof, or a functionally active fragment thereof, or a homolog thereof, or a variant of "SERINE/THREONINE PROTEIN PHOSPHATASE 2A, CATALYTIC SUBUNIT, β ISOFORM" encoded by a nucleic acid that hybridizes to the "SERINE/THREONINE PROTEIN PHOSPHATASE 2A, CATALYTIC SUBUNIT, β ISOFORM" nucleic acid under low stringency conditions, and/or
   (xl) "SEVENTRANSMEMBRANE-DOMAIN PROTEIN" (SEQ ID No:49) or a functionally active derivative thereof, or a functionally active fragment thereof, or a homolog thereof, or a variant of "SEVENTRANSMEMBRANE-DOMAIN PROTEIN" encoded by a nucleic acid that hybridizes to the "SEVENTRANSMEMBRANE-DOMAIN PROTEIN" nucleic acid under low stringency conditions, and/or
   (xli) "SERPIN B11" (SEQ ID No:50) or a functionally active derivative thereof, or a functionally active fragment thereof, or a homolog thereof, or a variant of "SERPIN B11" encoded by a nucleic acid that hybridizes to the "SERPIN B11" nucleic acid under low stringency conditions, and/or
   (xlii) "KIAA1228 PROTEIN" (SEQ ID No:51) or a functionally active derivative thereof, or a functionally active fragment thereof, or a homolog thereof, or a variant of "KIAA1228 PROTEIN" encoded by a nucleic acid that hybridizes to the "KIAA1228 PROTEIN" nucleic acid under low stringency conditions,
   is present in the complex.
32. The method of any of No. 26 to 31, wherein said method is a method of screening for a drug for treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
33. Use of a molecule that modulates the amount of, activity of, or the protein components of the complex of any one of No. 1 - 8 for the manufacture of a medicament for the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
34. A method for the production of a pharmaceutical composition comprising carrying out the method of any of No. 1 - 8 to identify a molecule that modulates the function, activity, composition or formation of said complex, and further comprising mixing the identified molecule with a pharmaceutically acceptable carrier.
35. A method for diagnosing or screening for the presence of a disease or disorder or a predisposition for developing a disease or disorder in a subject, which disease or disorder is characterized by an aberrant amount of, activity of, or component composition of, or intracellular localization of the complex of any one of the No. 1 - 8, comprising determining the amount of, activity of, protein components of, and/or intracellular localization of, said complex and/or the transcription level of a gene dependent on the complex and/or the abundance and/or activity of a protein or protein complex dependent on the function of the complex and/or product of a gene dependent on the complex in a comparative sample derived from a subject, wherein a difference in said amount, activity, or protein components of, said complex in an analogous sample from a subject not having the disease or disorder or predisposition indicates the presence in the subject of the disease or disorder or predisposition in the subject.
36. The method of No. 35, wherein the amount of said complex is determined.
37. The method of No. 35, wherein the activity of said complex is determined.
38. The method of No. 37, wherein said determining step comprises isolating from the subject said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
39. The method of No. 35, wherein the amount of the individual protein components of said complex are determined.
40. The method of No. 39, wherein said determining step comprises determining whether
   (i) "CDNA FLJ20733 FIS, CLONE HEP08550" (SEQ ID No:10) or a functionally active derivative thereof, or a functionally active fragment thereof, or a homolog thereof, or a variant of "CDNA FLJ20733 FIS, CLONE HEP08550" encoded by a nucleic acid that hybridizes to the "CDNA FLJ20733 FIS, CLONE HEP08550" nucleic acid under low stringency conditions, and/or
   (ii) "ANNEXIN I" (SEQ ID No:11) or a functionally active derivative thereof, or a functionally active fragment thereof, or a homolog thereof, or a variant of "ANEXIN I" encoded by a nucleic acid that hybridizes to the "ANNEXIN I" nucleic acid under low stringency conditions, and/or
   (iii) "THIOREDOXIN" (SEQ ID No:12) or a functionally active derivative thereof, or a functionally active fragment thereof, or a homolog thereof, or a variant of "THIOREDOXIN" encoded by a nucleic acid that hybridizes to the "THIOREDOXIN" nucleic acid under low stringency conditions, and/or
   (iv) "PEROXIREDOXIN 1" (SEQ ID No:13) or a functionally active derivative thereof, or a functionally active fragment thereof, or a homolog thereof, or a variant of "PEROXiREDOXIN 1" encoded by a nucleic acid that hybridizes to the "PEROXIREDOXIN" nucleic acid under low stringency conditions, and/or
   (v) "TRAF1" (SEQ ID No: 14) or a functionally active derivative thereof, or a functionally active fragment thereof, or a homolog thereof, or a variant of "TRAF1" encoded by a nucleic acid that hybridizes to the "TRAF1" nucleic acid under low stringency conditions, and/or
   (vi) "TRAF2" (SEQ ID No: 15) or a functionally active derivative thereof, or a functionally active fragment thereof, or a homolog thereof, or a variant of "TRAF2" encoded by a nucleic acid that hybridizes to the "TRAF2" nucleic acid under low stringency conditions, and/or
   (vii) "RIP" (SEQ ID No:16) or a functionally active derivative thereof, or a functionally active fragment thereof, or a homolog thereof, or a variant of "RIP" encoded by a nucleic acid that hybridizes to the "RIP" nucleic acid under low stringency conditions, and/or
   (viii) "TRADD" (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions, and/or
   (ix) " FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions, and/or
   (x) "FLJ10179 (KIAA1794)" (SEQ ID No:19) or a functionally active derivative thereof, or a functionally active fragment thereof, or a homolog thereof, or a variant of "FLJ10179 (KIAA1794)" encoded by a nucleic acid that hybridizes to the "FLJ10179 (KIAA1794)" nucleic acid under low stringency conditions, and/or
   (xi) "HYPOTHETICAL PROTEIN PRO1855" (SEQ ID No:20) or a functionally active derivative thereof, or a functionally active fragment thereof, or a homolog thereof, or a variant of "HYPOTHETICAL PROTEIN PRO1855" encoded by a nucleic acid that hybridizes to the "HYPOTHETICAL PROTEIN PRO1855" nucleic acid under low stringency conditions, and/or
   (xii) "TNFR1" (SEQ ID No:21) or a functionally active derivative thereof, or a functionally active fragment thereof, or a homolog thereof, or a variant of "TNRF1" encoded by a nucleic acid that hybridizes to the "TNRF1" nucleic acid under low stringency conditions, and/or
   (xiii) "FADD" (SEQ ID No:22) or a functionally active derivative thereof, or a functionally active fragment thereof, or a homolog thereof, or a variant of "FADD" encoded by a nucleic acid that hybridizes to the "FADD" nucleic acid under low stringency conditions, and/or
   (xiv) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions, and/or
   (xv) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions, and/or
   (xvi) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions, and/or
   (xvii) "c-IAP2" (SEQ ID No:26) or a functionally active derivative thereof, or a functionally active fragment thereof, or a homolog thereof, or a variant of "c-IAP2" encoded by a nucleic acid that hybridizes to the "c-IAP2" nucleic acid under low stringency conditions, and/or
   (xviii) "PROTEIN-L-ISOASPARTATE(D-ASPARTATE) O-METHYLTRANSFERASE" (SEQ ID No:27) or a functionally active derivative thereof, or a functionally active fragment thereof, or a homolog thereof, or a variant of "PROTEIN-L-ISOASPARTATE(D-ASPARTATE) O-METHYLTRANSFERASE" encoded by a nucleic acid that hybridizes to the "PROTEIN-L-ISOASPARTATE(D-ASPARTATE) O-METHYLTRANSFERASE" nucleic acid under low stringency conditions, and/or
   (xix) "UDP-GLUCOSE:GLYCOPROTEIN GLUCOSYLTRANSFERASE 1 PRECURSOR" (SEQ ID No:28) or a functionally active derivative thereof, or a functionally active fragment thereof, or a homolog thereof, or a variant of "UDP-GLUCOSE:GLYCOPROTEIN GLUCOSYLTRANSFERASE 1 PRECURSOR" encoded by a nucleic acid that hybridizes to the "UDP-GLUCOSE:GLYCOPROTEIN GLUCOSYLTRANSFERASE 1 PRECURSOR" nucleic acid under low stringency conditions, and/or
   (xx) " CDNA FLJ14515 FIS, CLONE NT2RM1000800, WEAKLY SIMILAR TO MUS MUSCULUS PARTIAL B-IND1 PROTEIN " (SEQ ID No:29) or a functionally active derivative thereof, or a functionally active fragment thereof, or a homolog thereof, or a variant of "CDNA FLJ14515 FIS, CLONE NT2RM1000800, WEAKLY SIMILAR TO MUS MUSCULUS PARTIAL B-IND1 PROTEIN" encoded by a nucleic acid that hybridizes to the "CDNA FLJ14515 FIS, CLONE NT2RM1000800, WEAKLY SIMILAR TO MUS MUSCULUS PARTIAL B-IND1 PROTEIN" nucleic acid under low stringency conditions, and/or
   (xxi) "KIAA1040 PROTEIN" (SEQ ID No:30) or a functionally active derivative thereof, or a functionally active fragment thereof, or a homolog thereof, or a variant of "KIAA1040 PROTEIN" encoded by a nucleic acid that hybridizes to the "KIAA1040 PROTEIN" nucleic acid under low stringency conditions, and/or
   (xxii) "ADENOSYLHOMOCYSTEINASE" (SEQ ID No:31) or a functionally active derivative thereof, or a functionally active fragment thereof, or a homolog thereof, or a variant of "ADENOSYLHOMOCYSTEINASE" encoded by a nucleic acid that hybridizes to the "ADENOSYLHOMOCYSTEINASE" nucleic acid under low stringency conditions, and/or
   (xxiii) "ATAXIA-TELANGIECTASIA GROUP D-ASSOCIATED PROTEIN" (SEQ ID No:32) or a functionally active derivative thereof, or a functionally active fragment thereof, or a homolog thereof, or a variant of "ATAXIA-TELANGIECTASIA GROUP D-ASSOCIATED PROTEIN" encoded by a nucleic acid that hybridizes to the "ATAXIA-TELANGIECTASIA GROUP D-ASSOCIATED PROTEIN" nucleic acid under low stringency conditions, and/or
   (xxiv) "MACROPHAGE MIGRATION INHIBITORY FACTOR" (SEQ ID No:33) or a functionally active derivative thereof, or a functionally active fragment thereof, or a homolog thereof, or a variant of "MACROPHAGE MIGRATION INHIBITORY FACTOR" encoded by a nucleic acid that hybridizes to the "MACROPHAGE MIGRATION INHIBITORY FACTOR" nucleic acid under low stringency conditions, and/or
   (xxv) "MONOCARBOXYLATE TRANSPORTER 1" (SEQ ID No:34) or a functionally active derivative thereof, or a functionally active fragment thereof, or a homolog thereof, or a variant of "MONOCARBOXYLATE TRANSPORTER 1" encoded by a nucleic acid that hybridizes to the "MONOCARBOXYLASE TRANSPORTER 1" nucleic acid under low stringency conditions, and/or
   (xxvi) "c-IAP1" (SEQ ID No:35) or a functionally active derivative thereof, or a functionally active fragment thereof, or a homolog thereof, or a variant of "c-IAP1" encoded by a nucleic acid that hybridizes to the "c-IAP1" nucleic acid under low stringency conditions, and/or
   (xxvii) "ANNEXIN II" (SEQ ID No:36) or a functionally active derivative thereof, or a functionally active fragment thereof, or a homolog thereof, or a variant of "ANNEXIN II" encoded by a nucleic acid that hybridizes to the "ANNEXIN II" nucleic acid under low stringency conditions, and/or
   (xxviii) "NAD(P)-DEPENDENT STEROID DEHYDROGENASE" (SEQ ID No:37) or a functionally active derivative thereof, or a functionally active fragment thereof, or a homolog thereof, or a variant of "NAD(P)-DEPENDENT STEROID DEHYDROGENASE" encoded by a nucleic acid that hybridizes to the "NAD(P)-DEPENDENT STEROID DEHYDROGENASE" nucleic acid under low stringency conditions, and/or
   (xxix) "PEPTIDYL-PROLYL CIS-TRANS ISOMERASE B PRECURSOR" (SEQ ID No:38) or a functionally active derivative thereof, or a functionally active fragment thereof, or a homolog thereof, or a variant of "PEPTIDYL-PROLYL CIS-TRANS ISOMERASE B PRECURSOR" encoded by a nucleic acid that hybridizes to the "PEPTIDYL-PROLYL CIS-TRANS ISOMERASE B PRECURSOR" nucleic acid under low stringency conditions, and/or
   (xxx) "CARBAMOYL-PHOSPHATE SYNTHASE [AMMONIA], MITOCHONDRIAL PRECURSOR" (SEQ ID No:39) or a functionally active derivative thereof, or a functionally active fragment thereof, or a homolog thereof, or a variant of "CARBAMOYL-PHOSPHATE SYNTHASE [AMMONIA], MITOCHONDRIAL PRECURSOR" encoded by a nucleic acid that hybridizes to the "CARBAMOYL-PHOSPHATE SYNTHASE [AMMONIA], MITOCHONDRIAL PRECURSOR" nucleic acid under low stringency conditions, and/or
   (xxxi) "ZINC-FINGER PROTEIN RFP" (SEQ ID No:40) or a functionally active derivative thereof, or a functionally active fragment thereof, or a homolog thereof, or a variant of "ZINC-FINGER PROTEIN RFP" encoded by a nucleic acid that hybridizes to the "ZINC-FINGER PROTEIN RFP" nucleic acid under low stringency conditions, and/or
   (xxxii) "52 KDA PROTEIN" (SEQ ID No:41) or a functionally active derivative thereof, or a functionally active fragment thereof, or a homolog thereof, or a variant of "52 KDA PROTEIN" encoded by a nucleic acid that hybridizes to the "52 KDA PROTEIN" nucleic acid under low stringency conditions, and/or
   (xxxiii) "SIMILAR TO SUBTILISIN-LIKE SERINE PROTEINASE" (SEQ ID No:42) or a functionally active derivative thereof, or a functionally active fragment thereof, or a homolog thereof, or a variant of "SIMILAR TO SUBTILISIN-LIKE SERINE PROTEINASE" encoded by a nucleic acid that hybridizes to the "SIMILAR TO SUBTILISIN-LIKE SERINE PROTEINASE" nucleic acid under low stringency conditions, and/or
   (xxxiv) "ANTILEUKOPROTEINASE 1 PRECURSOR" (SEQ ID No:43) or a functionally active derivative thereof, or a functionally active fragment thereof, or a homolog thereof, or a variant of "ANTILEUKOPROTEINASE 1 PRECURSOR" encoded by a nucleic acid that hybridizes to the "ANTILEUKOPROTEINASE 1 PRECURSOR" nucleic acid under low stringency conditions, and/or
   (xxxv) "7-DEHYDROCHOLESTEROL REDUCTASE" (SEQ ID No:44) or a functionally active derivative thereof, or a functionally active fragment thereof, or a homolog thereof, or a variant of "7-DEHYDROCHOLESTEROL REDUCTASE" encoded by a nucleic acid that hybridizes to the "7-DEHYDROCHOLESTEROL REDUCTASE" nucleic acid under low stringency conditions, and/or
   (xxxvi) "HYPOTHETICAL 82.0 KDA PROTEIN" (SEQ ID No:45) or a functionally active derivative thereof, or a functionally active fragment thereof, or a homolog thereof, or a variant of "HYPOTHETICAL 82.0 KDA PROTEIN" encoded by a nucleic acid that hybridizes to the "HYPOTHETICAL 82.0 KDA PROTEIN" nucleic acid under low stringency conditions, and/or
   (xxxvii) "CDNA FLJ14601 FIS, CLONE NT2RP1000124" (SEQ ID No:46) or a functionally active derivative thereof, or a functionally active fragment thereof, or a homolog thereof, or a variant of "CDNA FLJ14601 FIS, CLONE NT2RP1000124" encoded by a nucleic acid that hybridizes to the "CDNA FLJ14601 FIS, CLONE NT2RP1000124" nucleic acid under low stringency conditions, and/or
   (xxxviii) "TRANSCRIPTION FACTOR AP-2 γ" (SEQ ID No:47) or a functionally active derivative thereof, or a functionally active fragment thereof, or a homolog thereof, or a variant of "TRANSCRIPTION FACTOR AP-2 γ" encoded by a nucleic acid that hybridizes to the "TRANSCRIPTION FACTOR AP-2 γ" nucleic acid under low stringency conditions, and/or
   (xxxix) "SERINE/THREONINE PROTEIN PHOSPHATASE 2A, CATALYTIC SUBUNIT, β ISOFORM" (SEQ ID No:48) or a functionally active derivative thereof, or a functionally active fragment thereof, or a homolog thereof, or a variant of "SERINE/THREONINE PROTEIN PHOSPHATASE 2A, CATALYTIC SUBUNIT, β ISOFORM" encoded by a nucleic acid that hybridizes to the "SERINE/THREONINE PROTEIN PHOSPHATASE 2A, CATALYTIC SUBUNIT, β ISOFORM" nucleic acid under low stringency conditions, and/or
   (xl) "SEVENTRANSMEMBRANE-DOMAIN PROTEIN" (SEQ ID No:49) or a functionally active derivative thereof, or a functionally active fragment thereof, or a homolog thereof, or a variant of "SEVENTRANSMEMBRANE-DOMAIN PROTEIN" encoded by a nucleic acid that hybridizes to the "SEVENTRANSMEMBRANE-DOMAIN PROTEIN" nucleic acid under low stringency conditions, and/or
   (xli) "SERPIN B11" (SEQ ID No:50) or a functionally active derivative thereof, or a functionally active fragment thereof, or a homolog thereof, or a variant of "SERPIN B11" encoded by a nucleic acid that hybridizes to the "SERPIN B11" nucleic acid under low stringency conditions, and/or
   (xlii) "KIAA1228 PROTEIN" (SEQ ID No:51) or a functionally active derivative thereof, or a functionally active fragment thereof, or a homolog thereof, or a variant of "KIAA1228 PROTEIN" encoded by a nucleic acid that hybridizes to the "KIAA1228 PROTEIN" nucleic acid under low stringency conditions,
   is present in the complex.
41. The complex of any one of No. 1 - 8, or proteins of No. 13 or the antibody or fragment of No. 17, for use in a method of diagnosing a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
42. A method for treating or preventing a disease or disorder characterized by an aberrant amount of, activity or component composition of or intracellular localization of, the complex of anyone of No. 1 - 8, comprising administering to a subject in need of such treatment or prevention a therapeutically effective amount of one or more molecules that modulate the amount of, Receptor activity, or protein components of, said complex.
43. The method according to No. 42, wherein said disease or disorder involves decreased levels of the amount or activity of said complex.
44. The method according to No. 42 , wherein said disease or disorder involves increased levels of the amount or activity of said complex.
45. Complex of any of No. 1 - 8 and/or protein selected from the following proteins
   (i) "CDNA FLJ20733 FIS, CLONE HEP08550" (SEQ ID No:10) or a functionally active derivative thereof, or a functionally active fragment thereof, or a homolog thereof, or a variant of "CDNA FLJ20733 FIS, CLONE HEP08550" encoded by a nucleic acid that hybridizes to the "CDNA FLJ20733 FIS, CLONE HEP08550" nucleic acid under low stringency conditions,
   (ii) "ANNEXIN I" (SEQ ID No:11) or a functionally active derivative thereof, or a functionally active fragment thereof, or a homolog thereof, or a variant of "ANEXIN I" encoded by a nucleic acid that hybridizes to the "ANNEXIN I" nucleic acid under low stringency conditions,
   (iii) "THIOREDOXIN" (SEQ ID No:12) or a functionally active derivative thereof, or a functionally active fragment thereof, or a homolog thereof, or a variant of "THIOREDOXIN" encoded by a nucleic acid that hybridizes to the "THIOREDOXIN" nucleic acid under low stringency conditions,
   (iv) "PEROXIREDOXIN 1" (SEQ ID No:13) or a functionally active derivative thereof, or a functionally active fragment thereof, or a homolog thereof, or a variant of "PEROXiREDOXIN 1" encoded by a nucleic acid that hybridizes to the "PEROXIREDOXIN" nucleic acid under low stringency conditions,
   (v) "TRAF1" (SEQ ID No:14) or a functionally active derivative thereof, or a functionally active fragment thereof, or a homolog thereof, or a variant of "TRAF1" encoded by a nucleic acid that hybridizes to the "TRAF1" nucleic acid under low stringency conditions,
   (vi) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions,
   (vii) "RIP" (SEQ ID No:16) or a functionally active derivative thereof, or a functionally active fragment thereof, or a homolog thereof, or a variant of "RIP" encoded by a nucleic acid that hybridizes to the "RIP" nucleic acid under low stringency conditions,
   (viii) "TRADD" (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions,
   (ix) " FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,
   (x) "FLJ10179 (KIAA1794)" (SEQ ID No:19) or a functionally active derivative thereof, or a functionally active fragment thereof, or a homolog thereof, or a variant of "FLJ10179 (KIAA1794)" encoded by a nucleic acid that hybridizes to the "FLJ10179 (KIAA1794)" nucleic acid under low stringency conditions,
   (xi) "HYPOTHETICAL PROTEIN PRO1855" (SEQ ID No:20) or a functionally active derivative thereof, or a functionally active fragment thereof, or a homolog thereof, or a variant of "HYPOTHETICAL PROTEIN PRO1855 encoded by a nucleic acid that hybridizes to the "HYPOTHETICAL PROTEIN PRO1855 nucleic acid under low stringency conditions,
   (xii) (iv) "TNFR1" (SEQ ID No:21) or a functionally active derivative thereof, or a functionally active fragment thereof, or a homolog thereof, or a variant of "TNRF1" encoded by a nucleic acid that hybridizes to the "TNRF1" nucleic acid under low stringency conditions,
   (xiii) "FADD" (SEQ ID No:22) or a functionally active derivative thereof, or a functionally active fragment thereof, or a homolog thereof, or a variant of "FADD" encoded by a nucleic acid that hybridizes to the "FADD" nucleic acid under low stringency conditions,
   (xiv) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (xv) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (xvi) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (xvii) "c-IAP2" (SEQ ID No:26) or a functionally active derivative thereof, or a functionally active fragment thereof, or a homolog thereof, or a variant of "c-IAP2" encoded by a nucleic acid that hybridizes to the "c-IAP2" nucleic acid under low stringency conditions,
   (xviii) "PROTEIN-L-ISOASPARTATE(D-ASPARTATE) O-METHYLTRANSFERASE" (SEQ ID No:27) or a functionally active derivative thereof, or a functionally active fragment thereof, or a homolog thereof, or a variant of "PROTEIN-L-ISOASPARTATE(D-ASPARTATE) O-METHYLTRANSFERASE" encoded by a nucleic acid that hybridizes to the "PROTEIN-L-ISOASPARTATE(D-ASPARTATE) O-METHYLTRANSFERASE" nucleic acid under low stringency conditions,
   (xix) "UDP-GLUCOSE:GLYCOPROTEIN GLUCOSYLTRANSFERASE 1 PRECURSOR" (SEQ ID No:28) or a functionally active derivative thereof, or a functionally active fragment thereof, or a homolog thereof, or a variant of "UDP-GLUCOSE:GLYCOPROTEIN GLUCOSYLTRANSFERASE 1 PRECURSOR" encoded by a nucleic acid that hybridizes to the "UDP-GLUCOSE:GLYCOPROTEIN GLUCOSYLTRANSFERASE 1 PRECURSOR" nucleic acid under low stringency conditions,
   (xx) "CDNA FLJ14515 FIS, CLONE NT2RM1000800, WEAKLY SIMILAR TO MUS MUSCULUS PARTIAL B-IND1 PROTEIN" (SEQ ID No:29) or a functionally active derivative thereof, or a functionally active fragment thereof, or a homolog thereof, or a variant of "CDNA FLJ14515 FIS, CLONE NT2RM1000800, WEAKLY SIMILAR TO MUS MUSCULUS PARTIAL B-IND1 PROTEIN" encoded by a nucleic acid that hybridizes to the "CDNA FLJ14515 FIS, CLONE NT2RM1000800, WEAKLY SIMILAR TO MUS MUSCULUS PARTIAL B-IND1 PROTEIN" nucleic acid under low stringency conditions,
   (xxi) "KIAA1040 PROTEIN" (SEQ ID No:30) or a functionally active derivative thereof, or a functionally active fragment thereof, or a homolog thereof, or a variant of "KIAA1040 PROTEIN" encoded by a nucleic acid that hybridizes to the "KIAA1040 PROTEIN" nucleic acid under low stringency conditions,
   (xxii) "ADENOSYLHOMOCYSTEINASE" (SEQ ID No:31) or a functionally active derivative thereof, or a functionally active fragment thereof, or a homolog thereof, or a variant of "ADENOSYLHOMOCYSTEINASE" encoded by a nucleic acid that hybridizes to the "ADENOSYLHOMOCYSTEINASE" nucleic acid under low stringency conditions,
   (xxiii) "ATAXIA-TELANGIECTASIA GROUP D-ASSOCIATED PROTEIN" (SEQ ID No:32) or a functionally active derivative thereof, or a functionally active fragment thereof, or a homolog thereof, or a variant of "ATAXIA-TELANGIECTASIA GROUP D-ASSOCIATED PROTEIN" encoded by a nucleic acid that hybridizes to the "ATAXIA-TELANGIECTASIA GROUP D-ASSOCIATED PROTEIN" nucleic acid under low stringency conditions,
   (xxiv) "MACROPHAGE MIGRATION INHIBITORY FACTOR" (SEQ ID No:33) or a functionally active derivative thereof, or a functionally active fragment thereof, or a homolog thereof, or a variant of "MACROPHAGE MIGRATION INHIBITORY FACTOR" encoded by a nucleic acid that hybridizes to the "MACROPHAGE MIGRATION INHIBITORY FACTOR" nucleic acid under low stringency conditions,
   (xxv) "MONOCARBOXYLATE TRANSPORTER 1" (SEQ ID No:34) or a functionally active derivative thereof, or a functionally active fragment thereof, or a homolog thereof, or a variant of "MONOCARBOXYLATE TRANSPORTER 1" encoded by a nucleic acid that hybridizes to the "MONOCARBOXYLASE TRANSPORTER 1" nucleic acid under low stringency conditions,
   (xxvi) "c-IAP1" (SEQ ID No:35) or a functionally active derivative thereof, or a functionally active fragment thereof, or a homolog thereof, or a variant of "c-IAP1" encoded by a nucleic acid that hybridizes to the "c-IAP1" nucleic acid under low stringency conditions,
   (xxvii) "ANNEXIN II" (SEQ ID No:36) or a functionally active derivative thereof, or a functionally active fragment thereof, or a homolog thereof, or a variant of "ANNEXIN II" encoded by a nucleic acid that hybridizes to the "ANNEXIN II" nucleic acid under low stringency conditions,
   (xxviii) "NAD(P)-DEPENDENT STEROID DEHYDROGENASE" (SEQ ID No:37) or a functionally active derivative thereof, or a functionally active fragment thereof, or a homolog thereof, or a variant of "NAD(P)-DEPENDENT STEROID DEHYDROGENASE" encoded by a nucleic acid that hybridizes to the "NAD(P)-DEPENDENT STEROID DEHYDROGENASE" nucleic acid under low stringency conditions,
   (xxix) "PEPTIDYL-PROLYL CIS-TRANS ISOMERASE B PRECURSOR" (SEQ ID No:38) or a functionally active derivative thereof, or a functionally active fragment thereof, or a homolog thereof, or a variant of "PEPTIDYL-PROLYL CIS-TRANS ISOMERASE B PRECURSOR" encoded by a nucleic acid that hybridizes to the "PEPTIDYL-PROLYL CIS-TRANS ISOMERASE B PRECURSOR" nucleic acid under low stringency conditions,
   (xxx) "CARBAMOYL-PHOSPHATE SYNTHASE [AMMONIA], MITOCHONDRIAL PRECURSOR" (SEQ ID No:39) or a functionally active derivative thereof, or a functionally active fragment thereof, or a homolog thereof, or a variant of "CARBAMOYL-PHOSPHATE SYNTHASE [AMMONIA], MITOCHONDRIAL PRECURSOR" encoded by a nucleic acid that hybridizes to the "CARBAMOYL-PHOSPHATE SYNTHASE [AMMONIA], MITOCHONDRIAL PRECURSOR" nucleic acid under low stringency conditions,
   (xxxi) "ZINC-FINGER PROTEIN RFP" (SEQ ID No:40) or a functionally active derivative thereof, or a functionally active fragment thereof, or a homolog thereof, or a variant of "ZINC-FINGER PROTEIN RFP" encoded by a nucleic acid that hybridizes to the "ZINC-FINGER PROTEIN RFP" nucleic acid under low stringency conditions,
   (xxxii) "52 KDA PROTEIN" (SEQ ID No:41) or a functionally active derivative thereof, or a functionally active fragment thereof, or a homolog thereof, or a variant of "52 KDA PROTEIN" encoded by a nucleic acid that hybridizes to the "52 KDA PROTEIN" nucleic acid under low stringency conditions,
   (xxxiii) "SIMILAR TO SUBTILISIN-LIKE SERINE PROTEINASE" (SEQ ID No:42) or a functionally active derivative thereof, or a functionally active fragment thereof, or a homolog thereof, or a variant of "SIMILAR TO SUBTILISIN-LIKE SERINE PROTEINASE" encoded by a nucleic acid that hybridizes to the "SIMILAR TO SUBTILISIN-LIKE SERINE PROTEINASE" nucleic acid under low stringency conditions,
   (xxxiv) "ANTILEUKOPROTEINASE 1 PRECURSOR" (SEQ ID No:43) or a functionally active derivative thereof, or a functionally active fragment thereof, or a homolog thereof, or a variant of "ANTILEUKOPROTEINASE 1 PRECURSOR" encoded by a nucleic acid that hybridizes to the "ANTILEUKOPROTEINASE 1 PRECURSOR" nucleic acid under low stringency conditions,
   (xxxv) "7-DEHYDROCHOLESTEROL REDUCTASE" (SEQ ID No:44) or a functionally active derivative thereof, or a functionally active fragment thereof, or a homolog thereof, or a variant of "7-DEHYDROCHOLESTEROL REDUCTASE" encoded by a nucleic acid that hybridizes to the "7-DEHYDROCHOLESTEROL REDUCTASE" nucleic acid under low stringency conditions,
   (xxxvi) "HYPOTHETICAL 82.0 KDA PROTEIN" (SEQ ID No:45) or a functionally active derivative thereof, or a functionally active fragment thereof, or a homolog thereof, or a variant of "HYPOTHETICAL 82.0 KDA PROTEIN" encoded by a nucleic acid that hybridizes to the "HYPOTHETICAL 82.0 KDA PROTEIN" nucleic acid under low stringency conditions,
   (xxxvii) "CDNA FLJ14601 FIS, CLONE NT2RP1000124" (SEQ ID No:46) or a functionally active derivative thereof, or a functionally active fragment thereof, or a homolog thereof, or a variant of "CDNA FLJ14601 FIS, CLONE NT2RP1000124" encoded by a nucleic acid that hybridizes to the "CDNA FLJ14601 FIS, CLONE NT2RP1000124" nucleic acid under low stringency conditions,
   (xxxviii) "TRANSCRIPTION FACTOR AP-2 γ" (SEQ ID No:47) or a functionally active derivative thereof, or a functionally active fragment thereof, or a homolog thereof, or a variant of "TRANSCRIPTION FACTOR AP-2 γ" encoded by a nucleic acid that hybridizes to the "TRANSCRIPTION FACTOR AP-2 γ" nucleic acid under low stringency conditions,
   (xxxix) "SERINE/THREONINE PROTEIN PHOSPHATASE 2A, CATALYTIC SUBUNIT, β ISOFORM" (SEQ ID No:48) or a functionary active derivative thereof, or a functionally active fragment thereof, or a homolog thereof, or a variant of "SERINE/THREONINE PROTEIN PHOSPHATASE 2A, CATALYTIC SUBUNIT, β ISOFORM" encoded by a nucleic acid that hybridizes to the "SERINE/THREONINE PROTEIN PHOSPHATASE 2A, CATALYTIC SUBUNIT, β ISOFORM" nucleic acid under low stringency conditions,
   (xl) "SEVENTRANSMEMBRANE-DOMAIN PROTEIN" (SEQ ID No:49) or a functionally active derivative thereof, or a functionally active fragment thereof, or a homolog thereof, or a variant of "SEVENTRANSMEMBRANE-DOMAIN PROTEIN" encoded by a nucleic acid that hybridizes to the "SEVENTRANSMEMBRANE-DOMAIN PROTEIN" nucleic acid under low stringency conditions,
   (xli) "SERPIN B11" (SEQ ID No:50) or a functionally active derivative thereof,or a functionally active fragement thereof, or a homolog thereof, or a variant of "SERPIN B11" that hybridizes to the "SERPIN B11" nucleic acid under low stringency conditions, and
   (xlii) "KIAA1228 PROTEIN" (SEQ ID No:51) or a functionally active derivative thereof, or a functionally active fragment thereof, or a homolog thereof, or a variant of "KIAA1228 PROTEIN" encoded by a nucleic acid that hybridizes to the "KIAA1228 PROTEIN" nucleic acid under low stringency conditions,
as a target for an active agent of a pharmaceutical, preferably a drug target in the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.

The present invention further relates to the TNFR2-protein complex
1. A protein complex selected from complex (I) and comprising
   (a) at least one first protein selected from the group consisting of:
      (i) "TRAF2" (SEQ ID No: 15) or a functionally active derivative thereof, or a functionally active fragment thereof, or a homolog thereof, or a variant of "TRAF2" encoded by a nucleic acid that hybridizes to the "TRAF2" nucleic acid under low stringency conditions,
      (ii) "TRAF1" (SEQ ID No: 14) or a functionally active derivative thereof, or a functionally active fragment thereof, or a homolog thereof, or a variant of "TRAF1" encoded by a nucleic acid that hybridizes to the "TRAF1" nucleic acid under low stringency conditions, and
      (iii) "TNFR2" (SEQ ID No:52) or a functionally active derivative thereof, or a functionally active fragment thereof, or a homolog thereof, or a variant of "TNFR2" encoded by a nucleic acid that hybridizes to the "TNFR2" nucleic acid under low stringency conditions, and
   (b) at least one second protein, which second protein is selected from the group consisting of:
      (i) "TRICARBOXYLATE TRANSPORT PROTEIN, MITOCHONDRIAL PRECURSOR" (SEQ ID No:53) or a functionally active derivative thereof, or a functionally active fragment thereof, or a homolog thereof, or a variant of "TRICARBOXYLATE TRANSPORT PROTEIN, MITOCHONDRIAL PRECURSOR" encoded by a nucleic acid that hybridizes to the "TRICARBOXYLATE TRANSPORT PROTEIN, MITOCHONDRIAL PRECURSOR" nucleic acid under low stringency conditions,
      (ii) "KIAA1040 PROTEIN" (SEQ ID No:30) or a functionally active derivative thereof, or a functionally active fragment thereof, or a homolog thereof, or a variant of "KIAA1040 PROTEIN" encoded by a nucleic acid that hybridizes to the "KIAA1040 PROTEIN" nucleic acid under low stringency conditions,
      (iii) "ENDOOLIGOPEPTIDASE A" (SEQ ID No:54) or a functionally active derivative thereof, or a functionally active fragment thereof, or a homolog thereof, or a variant of "ENDOOLIGOPEPTIDASE A" encoded by a nucleic acid that hybridizes to the "ENDOOLIGOPEPTIDASE A" nucleic acid under low stringency conditions,
      (iv) "OXYSTEROL BINDING PROTEIN-RELATED PROTEIN 8" (SEQ ID No:55) or a functionally active derivative thereof, or a functionally active fragment thereof, or a homolog thereof, or a variant of "OXYSTEROL BINDING PROTEIN-RELATED PROTEIN 8" encoded by a nucleic acid that hybridizes to the "OXYSTEROL BINDING PROTEIN-RELATED PROTEIN 8" nucleic acid under low stringency conditions,
      (v) "LECTIN, GALACTOSIDE-BINDING, SOLUBLE, 8 (GALECTIN 8)" (SEQ ID No:56) or a functionally active derivative thereof, or a functionally active fragment thereof, or a homolog thereof, or a variant of "LECTIN, GALACTOSIDE-BINDING, SOLUBLE, 8 (GALECTIN 8)" encoded by a nucleic acid that hybridizes to the "LECTIN, GALACTOSIDE-BINDING, SOLUBLE, 8 (GALECTIN 8)" nucleic acid under low stringency conditions,
      (vi) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,
      (vii) "β-1,4 MANNOSYLTRANSFERASE" (SEQ ID No:57) or a functionally active derivative thereof, or a functionally active fragment thereof, or a homolog thereof, or a variant of "β-1,4 MANNOSYLTRANSFERASE" encoded by a nucleic acid that hybridizes to the "β-1,4 MANNOSYLTRANSFERASE" nucleic acid under low stringency conditions,
      (viii) "β-FILAMIN" (SEQ ID No:58) or a functionally active derivative thereof, or a functionally active fragment thereof, or a homolog thereof, or a variant of "β-FILAMIN" encoded by a nucleic acid that hybridizes to the "β-FILAMIN" nucleic acid under low stringency conditions,
      (ix) "HYPOTHETICAL 57.0 KDA PROTEIN" (SEQ ID No:59) or a functionally active derivative thereof, or a functionally active fragment thereof, or a homolog thereof, or a variant of "HYPOTHETICAL 57.0 KDA PROTEIN" encoded by a nucleic acid that hybridizes to the "HYPOTHETICAL 57.0 KDA PROTEIN" nucleic acid under low stringency conditions,
      (x) "FILAMIN A" (SEQ ID No:60) or a functionally active derivative thereof, or a functionally active fragment thereof, or a homolog thereof, or a variant of "FILAMIN A" encoded by a nucleic acid that hybridizes to the "FILAMIN A" nucleic acid under low stringency conditions,
      (xi) "HS1 -ASSOCIATED PROTEIN X-1" (SEQ ID No:61) or a functionally active derivative thereof, or a functionally active fragment thereof, or a homolog thereof, or a variant of "HS1-ASSOCIATED PROTEIN X-1" encoded by a nucleic acid that hybridizes to the "HS1-ASSOCIATED PROTEIN X-1" nucleic acid under low stringency conditions,
      (xii) "LIPOAMIDE ACYLTRANSFERASE COMPONENT OF BRANCHED-CHAIN α-KETO ACID DEHYDROGENASE COMPLEX, MITOCHONDRIAL PRECURSOR" (SEQ ID No:62) or a functionally active derivative thereof, or a functionally active fragment thereof, or a homolog thereof, or a variant of "LIPOAMIDE ACYLTRANSFERASE COMPONENT OF BRANCHED-CHAIN α-KETO ACID DEHYDROGENASE COMPLEX, MITOCHONDRIAL PRECURSOR" encoded by a nucleic acid that hybridizes to the "LIPOAMIDE ACYLTRANSFERASE COMPONENT OF BRANCHED-CHAIN α-KETO ACID DEHYDROGENASE COMPLEX, MITOCHONDRIAL PRECURSOR" nucleic acid under low stringency conditions,
      (xiii) "FLJ10719 (KIAA1794)" (SEQ ID No:19) or a functionally active derivative thereof, or a functionally active fragment thereof, or a homolog thereof, or a variant of "FLJ10719 (KIAA1794)" encoded by a nucleic acid that hybridizes to the "FLJ10719 (KIAA1794)" nucleic acid under low stringency conditions,
      (xiv) "SYNAPTIC GLYCOPROTEIN SC2" (SEQ ID No:63) or a functionally active derivative thereof, or a functionally active fragment thereof, or a homolog thereof, or a variant of "SYNAPTIC GLYCOPROTEIN SC2" encoded by a nucleic acid that hybridizes to the "SYNAPTIC GLYCOPROTEIN SC2" nucleic acid under low stringency conditions,
      (xv) "TYROSINE-PROTEIN KINASE CSK" (SEQ ID No:6) or a functionally active derivative thereof, or a functionally active fragment thereof, or a homolog thereof, or a variant of "TYROSINE-PROTEIN KINASE CSK" encoded by a nucleic acid that hybridizes to the "TYROSINE-PROTEIN KINASE CSK" nucleic acid under low stringency conditions,
      (xvi) "RAN-GTP BINDING PROTEIN" (SEQ ID No:64) or a functionally active derivative thereof, or a functionally active fragment thereof, or a homolog thereof, or a variant of "RAN-GTP BINDING PROTEIN" encoded by a nucleic acid that hybridizes to the "RAN-GTP BINDING PROTEIN" nucleic acid under low stringency conditions, and
      (xvii) "2-OXOGLUTARATE DEHYDROGENASE E1 COMPONENT, MITOCHONDRIAL PRECURSOR" (SEQ ID No:65) or a functionally active derivative thereof, or a functionally active fragment thereof, or a homolog thereof, or a variant of "2-OXOGLUTARATE DEHYDROGENASE E1 COMPONENT, MITOCHONDRIAL PRECURSOR" encoded by a nucleic acid that hybridizes to the "2-OXOGLUTARATE DEHYDROGENASE E1 COMPONENT, MITOCHONDRIAL PRECURSOR" nucleic acid under low stringency conditions;
   and a complex (II) comprising at least two of said second proteins,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
2. The protein complex according to claim 1 wherein the first protein is the protein "TNFR2" (SEQ ID No:52), or a functionally active derivative thereof, or a functionally active fragment thereof, or a homolog thereof, or a variant of "TNFR2" encoded by a nucleic acid that hybridizes to the "TNFR2" nucleic acid under low stringency conditions.
3. The protein complex according to No. 1 selected from complex (I) and comprising the following proteins:
   (i) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions,
   (ii) "TNFR2" (SEQ ID No:52) or a functionally active derivative thereof, or a functionally active fragment thereof, or a homolog thereof, or a variant of "TNFR2" encoded by a nucleic acid that hybridizes to the "TNFR2" nucleic acid under low stringency conditions,
   (iii) "TRICARBOXYLATE TRANSPORT PROTEIN, MITOCHONDRIAL PRECURSOR" (SEQ ID No:53) or a functionally active derivative thereof, or a functionally active fragment thereof, or a homolog thereof, or a variant of "TRICARBOXYLATE TRANSPORT PROTEIN, MITOCHONDRIAL PRECURSOR" encoded by a nucleic acid that hybridizes to the "TRICARBOXYLATE TRANSPORT PROTEIN, MITOCHONDRIAL PRECURSOR" nucleic acid under low stringency conditions,
   (iv) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,
   (v) "FILAMIN A" (SEQ ID No:60) or a functionally active derivative thereof, or a functionally active fragment thereof, or a homolog thereof, or a variant of "FILAMIN A" encoded by a nucleic acid that hybridizes to the "FILAMIN A" nucleic acid under low stringency conditions, and
   (vi) "RAN-GTP BINDING PROTEIN" (SEQ ID No:64) or a functionally active derivative thereof, or a functionally active fragment thereof, or a homolog thereof, or a variant of "RAN-GTP BINDING PROTEIN" encoded by a nucleic acid that hybridizes to the "RAN-GTP BINDING PROTEIN" nucleic acid under ow stringency conditions;
   and a protein complex selected from complex (II) and comprising the following proteins:
   (i) "TRAF2" (SEQ ID No: 15) or a functionally active derivative thereof, or a functionally active fragment thereof, or a homolog thereof, or a variant of "TRAF2" encoded by a nucleic acid that hybridizes to the "TRAF2" nucleic acid under low stringency conditions,
   (ii) "TNFR2" (SEQ ID No:52) or a functionally active derivative thereof, or a functionally active fragment thereof, or a homolog thereof, or a variant of "TNFR2" encoded by a nucleic acid that hybridizes to the "TNFR2" nucleic acid under low stringency conditions,
   (iii) "TRICARBOXYLATE TRANSPORT PROTEIN, MITOCHONDRIAL PRECURSOR" (SEQ ID No:53) or a functionally active derivative thereof, or a functionally active fragment thereof, or a homolog thereof, or a variant of "TRICARBOXYLATE TRANSPORT PROTEIN, MITOCHONDRIAL PRECURSOR" encoded by a nucleic acid that hybridizes to the "TRICARBOXYLATE TRANSPORT PROTEIN, MITOCHONDRIAL PRECURSOR" nucleic acid under low stringency conditions,
   (iv) "KIAA1040 PROTEIN" (SEQ ID No:30) or a functionally active derivative thereof, or a functionally active fragment thereof, or a homolog thereof, or a variant of "KIAA1040 PROTEIN" encoded by a nucleic acid that hybridizes to the "KIAA1040 PROTEIN" nucleic acid under low stringency conditions,
   (v) "OXYSTEROL BINDING PROTEIN-RELATED PROTEIN 8" (SEQ ID No:55) or a functionally active derivative thereof, or a functionally active fragment thereof, or a homolog thereof, or a variant of "OXYSTEROL BINDING PROTEIN-RELATED PROTEIN 8" encoded by a nucleic acid that hybridizes to the "OXYSTEROL BINDING PROTEIN-RELATED PROTEIN 8" nucleic acid under low stringency conditions,
   (vi) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,
   (vii) "β-1,4 MANNOSYLTRANSFERASE" (SEQ ID No:57) or a functionally active derivative thereof, or a functionally active fragment thereof, or a homolog thereof, or a variant of "β-1,4 MANNOSYLTRANSFERASE" encoded by a nucleic acid that hybridizes to the "β-1,4 MANNOSYLTRANSFERASE" nucleic acid under low stringency conditions,
   (viii) "HYPOTHETICAL 57.0 KDA PROTEIN" (SEQ ID No:59) or a functionally active derivative thereof, or a functionally active fragment thereof, or a homolog thereof, or a variant of "HYPOTHETICAL 57.0 KDA PROTEIN" encoded by a nucleic acid that hybridizes to the "HYPOTHETICAL 57.0 KDA PROTEIN" nucleic acid under low stringency conditions,
   (ix) "FILAMIN A" (SEQ ID No:60) or a functionally active derivative thereof, or a functionally active fragment thereof, or a homolog thereof, or a variant of "FILAMIN A" encoded by a nucleic acid that hybridizes to the "FILAMIN A" nucleic acid under low stringency conditions,
   (x) "HS1-ASSOCIATED PROTEIN X-1" (SEQ ID No:61) or a functionally active derivative thereof, or a functionally active fragment thereof, or a homolog thereof, or a variant of "HS1-ASSOCIATED PROTEIN X-1" encoded by a nucleic acid that hybridizes to the "HS1-ASSOCIATED PROTEIN X-1" nucleic acid under low stringency conditions,
   (xi) "FLJ10719 (KIAA1794)" (SEQ ID No:19) or a functionally active derivative thereof, or a functionally active fragment thereof, or a homolog thereof, or a variant of "FLJ10719 (KIAA1794)" encoded by a nucleic acid that hybridizes to the "FLJ10719 (KIAA1794)" nucleic acid under low stringency conditions,
   (xii) "SYNAPTIC GLYCOPROTEIN SC2" (SEQ ID No:63) or a functionally active derivative thereof, or a functionally active fragment thereof, or a homolog thereof, or a variant of "SYNAPTIC GLYCOPROTEIN SC2" encoded by a nucleic acid that hybridizes to the "SYNAPTIC GLYCOPROTEIN SC2" nucleic acid under low stringency conditions, and
   (xiii) "RAN-GTP BINDING PROTEIN" (SEQ ID No:64) or a functionally active derivative thereof, or a functionally active fragment thereof, or a homolog thereof, or a variant of "RAN-GTP BINDING PROTEIN" encoded by a nucleic acid that hybridizes to the "RAN-GTP BINDING PROTEIN" nucleic acid under ow stringency conditions;
   and a protein complex selected from complex (III) and comprising the following proteins:
   (i) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions,
   (ii) "TNFR2" (SEQ ID No:52) or a functionally active derivative thereof, or a functionally active fragment thereof, or a homolog thereof, or a variant of "TNFR2" encoded by a nucleic acid that hybridizes to the "TNFR2" nucleic acid under low stringency conditions,
   (iii) "TRICARBOXYLATE TRANSPORT PROTEIN, MITOCHONDRIAL PRECURSOR" (SEQ ID No:53) or a functionally active derivative thereof, or a functionally active fragment thereof, or a homolog thereof, or a variant of "TRICARBOXYLATE TRANSPORT PROTEIN, MITOCHONDRIAL PRECURSOR" encoded by a nucleic acid that hybridizes to the "TRICARBOXYLATE TRANSPORT PROTEIN, MITOCHONDRIAL PRECURSOR" nucleic acid under low stringency conditions,
   (iv) "ENDOOLIGOPEPTIDASE A" (SEQ ID No:54) or a functionally active derivative thereof, or a functionally active fragment thereof, or a homolog thereof, or a variant of "ENDOOLIGOPEPTIDASE A" encoded by a nucleic acid that hybridizes to the "ENDOOLIGOPEPTIDASE A" nucleic acid under low stringency conditions,
   (v) "LECTIN, GALACTOSIDE-BINDING, SOLUBLE, 8 (GALECTIN 8)" (SEQ ID No:56) or a functionally active derivative thereof, or a functionally active fragment thereof, or a homolog thereof, or a variant of "LECTIN, GALACTOSIDE-BINDING, SOLUBLE, 8 (GALECTIN 8)" encoded by a nucleic acid that hybridizes to the "LECTIN, GALACTOSIDE-BINDING, SOLUBLE, 8 (GALECTIN 8)" nucleic acid under low stringency conditions,
   (vi) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,

   (i) "β-FILAMIN" (SEQ ID No:58) or a functionally active derivative thereof, or a functionally active fragment thereof, or a homolog thereof, or a variant of "β-FILAMIN" encoded by a nucleic acid that hybridizes to the "β-FILAMIN" nucleic acid under low stringency conditions,
   (viii) "FILAMIN A" (SEQ ID No:60) or a functionally active derivative thereof, or a functionally active fragment thereof, or a homolog thereof, or a variant of "FILAMIN A" encoded by a nucleic acid that hybridizes to the "FILAMIN A" nucleic acid under low stringency conditions,
   (i) "LIPOAMIDE ACYLTRANSFERASE COMPONENT OF BRANCHED-CHAIN α-KETO ACID DEHYDROGENASE COMPLEX, MITOCHONDRIAL PRECURSOR" (SEQ ID No:62) or a functionally active derivative thereof, or a functionally active fragment thereof, or a homolog thereof, or a variant of "LIPOAMIDE ACYLTRANSFERASE COMPONENT OF BRANCHED-CHAIN α-KETO ACID DEHYDROGENASE COMPLEX, MITOCHONDRIAL PRECURSOR" encoded by a nucleic acid that hybridizes to the "LIPOAMIDE ACYLTRANSFERASE COMPONENT OF BRANCHED-CHAIN α-KETO ACID DEHYDROGENASE COMPLEX, MITOCHONDRIAL PRECURSOR" nucleic acid under low stringency conditions,
   (x) "TYROSINE-PROTEIN KINASE CSK" (SEQ ID No:6) or a functionally active derivative thereof, or a functionally active fragment thereof, or a homolog thereof, or a variant of "TYROSINE-PROTEIN KINASE CSK" encoded by a nucleic acid that hybridizes to the "TYROSINE-PROTEIN KINASE CSK" nucleic acid under low stringency conditions,
   (xi) "RAN-GTP BINDING PROTEIN" (SEQ ID No:64) or a functionally active derivative thereof, or a functionally active fragment thereof, or a homolog thereof, or a variant of "RAN-GTP BINDING PROTEIN" encoded by a nucleic acid that hybridizes to the "RAN-GTP BINDING PROTEIN" nucleic acid under low stringency conditions, and
   (xii) "2-OXOGLUTARATE DEHYDROGENASE E1 COMPONENT,
   MITOCHONDRIAL PRECURSOR" (SEQ ID No:65) or a functionally active derivative thereof, or a functionally active fragment thereof, or a homolog thereof, or a variant of "2-OXOGLUTARATE DEHYDROGENASE E1 COMPONENT, MITOCHONDRIAL PRECURSOR" encoded by a nucleic acid that hybridizes to the "2-OXOGLUTARATE DEHYDROGENASE E1 COMPONENT, MITOCHONDRIAL PRECURSOR" nucleic acid under ow stringency conditions;
   and a protein complex selected from complex (IV) and comprising the following proteins:
   (i) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions,
   (ii) "TRAF1" (SEQ ID No:14) or a functionally active derivative thereof, or a functionally active fragment thereof, or a homolog thereof, or a variant of "TRAF1" encoded by a nucleic acid that hybridizes to the "TRAF1" nucleic acid under low stringency conditions,
   (iii) "TNFR2" (SEQ ID No:52) or a functionally active derivative thereof, or a functionally active fragment thereof, or a homolog thereof, or a variant of "TNFR2" encoded by a nucleic acid that hybridizes to the "TNFR2" nucleic acid under low stringency conditions,
   (iv) "TRICARBOXYLATE TRANSPORT PROTEIN, MITOCHONDRIAL PRECURSOR" (SEQ ID No:53) or a functionally active derivative thereof, or a functionally active fragment thereof, or a homolog thereof, or a variant of "TRICARBOXYLATE TRANSPORT PROTEIN, MITOCHONDRIAL PRECURSOR" encoded by a nucleic acid that hybridizes to the "TRICARBOXYLATE TRANSPORT PROTEIN, MITOCHONDRIAL PRECURSOR" nucleic acid under low stringency conditions,
   (v) "KIAA1040 PROTEIN" (SEQ ID No:30) or a functionally active derivative thereof, or a functionally active fragment thereof, or a homolog thereof, or a variant of "KIAA1040 PROTEIN" encoded by a nucleic acid that hybridizes to the "KIAA1040 PROTEIN" nucleic acid under low stringency conditions,
   (vi) "ENDOOLIGOPEPTIDASE A" (SEQ ID No:54) or a functionally active derivative thereof, or a functionally active fragment thereof, or a homolog thereof, or a variant of "ENDOOLIGOPEPTIDASE A" encoded by a nucleic acid that hybridizes to the "ENDOOLIGOPEPTIDASE A" nucleic acid under low stringency conditions,
   (vii) "OXYSTEROL BINDING PROTEIN-RELATED PROTEIN 8" (SEQ ID No:55) or a functionally active derivative thereof, or a functionally active fragment thereof, or a homolog thereof, or a variant of "OXYSTEROL BINDING PROTEIN-RELATED PROTEIN 8" encoded by a nucleic acid that hybridizes to the "OXYSTEROL BINDING PROTEIN-RELATED PROTEIN 8" nucleic acid under low stringency conditions,
   (viii) "LECTIN, GALACTOSIDE-BINDING, SOLUBLE, 8 (GALECTIN 8)" (SEQ ID No:56) or a functionally active derivative thereof, or a functionally active fragment thereof, or a homolog thereof, or a variant of "LECTIN, GALACTOSIDE-BINDING, SOLUBLE, 8 (GALECTIN 8)" encoded by a nucleic acid that hybridizes to the "LECTIN, GALACTOSIDE-BINDING, SOLUBLE, 8 (GALECTIN 8)" nucleic acid under low stringency conditions,
   (ix) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,
   (x) "β-1,4 MANNOSYLTRANSFERASE" (SEQ ID No:57) or a functionally active derivative thereof, or a functionally active fragment thereof, or a homolog thereof, or a variant of "β-1,4 MANNOSYLTRANSFERASE" encoded by a nucleic acid that hybridizes to the "β-1,4 MANNOSYLTRANSFERASE" nucleic acid under low stringency conditions,
   (xi) "β-FILAMIN" (SEQ ID No:58) or a functionally active derivative thereof, or a functionally active fragment thereof, or a homolog thereof, or a variant of "β-FILAMIN" encoded by a nucleic acid that hybridizes to the "β-FILAMIN" nucleic acid under low stringency conditions,
   (xii) "HYPOTHETICAL 57.0 KDA PROTEIN" (SEQ ID No:59) or a functionally active derivative thereof, or a functionally active fragment thereof, or a homolog thereof, or a variant of "HYPOTHETICAL 57.0 KDA PROTEIN" encoded by a nucleic acid that hybridizes to the "HYPOTHETICAL 57.0 KDA PROTEIN" nucleic acid under low stringency conditions,
   (xiii) "FILAMIN A" (SEQ ID No:60) or a functionally active derivative thereof, or a functionally active fragment thereof, or a homolog thereof, or a variant of "FILAMIN A" encoded by a nucleic acid that hybridizes to the "FILAMIN A" nucleic acid under low stringency conditions,
   (xiv) "HS1-ASSOCIATED PROTEIN X-1" (SEQ ID No:61) or a functionally active derivative thereof, or a functionally active fragment thereof, or a homolog thereof, or a variant of "HS1-ASSOCIATED PROTEIN X-1" encoded by a nucleic acid that hybridizes to the "HS1-ASSOCIATED PROTEIN X-1" nucleic acid under low stringency conditions,
   (xv) "LIPOAMIDE ACYLTRANSFERASE COMPONENT OF BRANCHED-CHAIN α-KETO ACID DEHYDROGENASE COMPLEX, MITOCHONDRIAL PRECURSOR" (SEQ ID No:62) or a functionally active derivative thereof, or a functionally active fragment thereof, or a homolog thereof, or a variant of "LIPOAMIDE ACYLTRANSFERASE COMPONENT OF BRANCHED-CHAIN α-KETO ACID DEHYDROGENASE COMPLEX, MITOCHONDRIAL PRECURSOR" encoded by a nucleic acid that hybridizes to the "LIPOAMIDE ACYLTRANSFERASE COMPONENT OF BRANCHED-CHAIN α-KETO ACID DEHYDROGENASE COMPLEX, MITOCHONDRIAL PRECURSOR" nucleic acid under low stringency conditions,
   (xvi) "FLJ10719 (KIAA1794)" (SEQ ID No:19) or a functionally active derivative thereof, or a functionally active fragment thereof, or a homolog thereof, or a variant of "FLJ10719 (KIAA1794)" encoded by a nucleic acid that hybridizes to the "FLJ10719 (KIAA1794)" nucleic acid under low stringency conditions,
   (xvii) "SYNAPTIC GLYCOPROTEIN SC2" (SEQ ID No:63) or a functionally active derivative thereof, or a functionally active fragment thereof, or a homolog thereof, or a variant of "SYNAPTIC GLYCOPROTEIN SC2" encoded by a nucleic acid that hybridizes to the "SYNAPTIC GLYCOPROTEIN SC2" nucleic acid under low stringency conditions,
   (xviii) "TYROSINE-PROTEIN KINASE CSK" (SEQ ID No:6) or a functionally active derivative thereof, or a functionally active fragment thereof, or a homolog thereof, or a variant of "TYROSINE-PROTEIN KINASE CSK" encoded by a nucleic acid that hybridizes to the "TYROSINE-PROTEIN KINASE CSK" nucleic acid under low stringency conditions,
   (xix) "RAN-GTP BINDING PROTEIN" (SEQ ID No:64) or a functionally active derivative thereof, or a functionally active fragment thereof, or a homolog thereof, or a variant of "RAN-GTP BINDING PROTEIN" encoded by a nucleic acid that hybridizes to the "RAN-GTP BINDING PROTEIN" nucleic acid under low stringency conditions, and
   (xx) "2-OXOGLUTARATE DEHYDROGENASE E1 COMPONENT, MITOCHONDRIAL PRECURSOR" (SEQ ID No:65) or a functionally active derivative thereof, or a functionally active fragment thereof, or a homolog thereof, or a variant of "2-OXOGLUTARATE DEHYDROGENASE E1 COMPONENT, MITOCHONDRIAL PRECURSOR" encoded by a nucleic acid that hybridizes to the "2-OXOGLUTARATE DEHYDROGENASE E1 COMPONENT, MITOCHONDRIAL PRECURSOR" nucleic acid under low stringency conditions.
4. The protein complex according to No. 1 selected from complex (I) comprising all but 1 - 16 of the following proteins:
   (i) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions,
   (ii) "TRAF1" (SEQ ID No:14) or a functionally active derivative thereof, or a functionally active fragment thereof, or a homolog thereof, or a variant of "TRAF1" encoded by a nucleic acid that hybridizes to the "TRAF1" nucleic acid under low stringency conditions,
   (iii) "TNFR2" (SEQ ID No:52) or a functionally active derivative thereof, or a functionally active fragment thereof, or a homolog thereof, or a variant of "TNFR2" encoded by a nucleic acid that hybridizes to the "TNFR2" nucleic acid under low stringency conditions,
   (iv) "TRICARBOXYLATE TRANSPORT PROTEIN, MITOCHONDRIAL PRECURSOR" (SEQ ID No:53) or a functionally active derivative thereof, or a functionally active fragment thereof, or a homolog thereof, or a variant of "TRICARBOXYLATE TRANSPORT PROTEIN, MITOCHONDRIAL PRECURSOR" encoded by a nucleic acid that hybridizes to the "TRICARBOXYLATE TRANSPORT PROTEIN, MITOCHONDRIAL PRECURSOR" nucleic acid under low stringency conditions,
   (v) "KIAA1040 PROTEIN" (SEQ ID No:30) or a functionally active derivative thereof, or a functionally active fragment thereof, or a homolog thereof, or a variant of "KIAA1040 PROTEIN" encoded by a nucleic acid that hybridizes to the "KIAA1040 PROTEIN" nucleic acid under low stringency conditions,
   (vi) "ENDOOLIGOPEPTIDASE A" (SEQ ID No:54) or a functionally active derivative thereof, or a functionally active fragment thereof, or a homolog thereof, or a variant of "ENDOOLIGOPEPTIDASE A" encoded by a nucleic acid that hybridizes to the "ENDOOLIGOPEPTIDASE A" nucleic acid under low stringency conditions,
   (vii) "OXYSTEROL BINDING PROTEIN-RELATED PROTEIN 8" (SEQ ID No:55) or a functionally active derivative thereof, or a functionally active fragment thereof, or a homolog thereof, or a variant of "OXYSTEROL BINDING PROTEIN-RELATED PROTEIN 8" encoded by a nucleic acid that hybridizes to the "OXYSTEROL BINDING PROTEIN-RELATED PROTEIN 8" nucleic acid under low stringency conditions,
   (viii) "LECTIN, GALACTOSIDE-BINDING, SOLUBLE, 8 (GALECTIN 8)" (SEQ ID No:56) or a functionally active derivative thereof, or a functionally active fragment thereof, or a homolog thereof, or a variant of "LECTIN, GALACTOSIDE-BINDING, SOLUBLE, 8 (GALECTIN 8)" encoded by a nucleic acid that hybridizes to the "LECTIN, GALACTOSIDE-BINDING, SOLUBLE, 8 (GALECTIN 8)" nucleic acid under low stringency conditions,
   (ix) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,
   (x) "β-1,4 MANNOSYLTRANSFERASE" (SEQ ID No:57) or a functionally active derivative thereof, or a functionally active fragment thereof, or a homolog thereof, or a variant of "β-1,4 MANNOSYLTRANSFERASE" encoded by a nucleic acid that hybridizes to the "β-1,4 MANNOSYLTRANSFERASE" nucleic acid under low stringency conditions,
   (xi) "β-FILAMIN" (SEQ ID No:58) or a functionally active derivative thereof, or a functionally active fragment thereof, or a homolog thereof, or a variant of "β-FILAMIN" encoded by a nucleic acid that hybridizes to the "β-FILAMIN" nucleic acid under low stringency conditions,
   (xii) "HYPOTHETICAL 57.0 KDA PROTEIN" (SEQ ID No:59) or a functionally active derivative thereof, or a functionally active fragment thereof, or a homolog thereof, or a variant of "HYPOTHETICAL 57.0 KDA PROTEIN" encoded by a nucleic acid that hybridizes to the "HYPOTHETICAL 57.0 KDA PROTEIN" nucleic acid under low stringency conditions,
   (xiii) "FILAMIN A" (SEQ ID No:60) or a functionally active derivative thereof, or a functionally active fragment thereof, or a homolog thereof, or a variant of "FILAMIN A" encoded by a nucleic acid that hybridizes to the "FILAMIN A" nucleic acid under low stringency conditions,
   (xiv) "HS1-ASSOCIATED PROTEIN X-1" (SEQ ID No:61) or a functionally active derivative thereof, or a functionally active fragment thereof, or a homolog thereof, or a variant of "HS1-ASSOCIATED PROTEIN X-1" encoded by a nucleic acid that hybridizes to the "HS1-ASSOCIATED PROTEIN X-1" nucleic acid under low stringency conditions,
   (xv) "LIPOAMIDE ACYLTRANSFERASE COMPONENT OF BRANCHED-CHAIN α-KETO ACID DEHYDROGENASE COMPLEX, MITOCHONDRIAL PRECURSOR" (SEQ ID No:62) or a functionally active derivative thereof, or a functionally active fragment thereof, or a homolog thereof, or a variant of "LIPOAMIDE ACYLTRANSFERASE COMPONENT OF BRANCHED-CHAIN α-KETO ACID DEHYDROGENASE COMPLEX, MITOCHONDRIAL PRECURSOR" encoded by a nucleic acid that hybridizes to the "LIPOAMIDE ACYLTRANSFERASE COMPONENT OF BRANCHED-CHAIN α-KETO ACID DEHYDROGENASE COMPLEX, MITOCHONDRIAL PRECURSOR" nucleic acid under low stringency conditions,
   (xvi) "FLJ10719 (KIAA1794)" (SEQ ID No:19) or a functionally active derivative thereof, or a functionally active fragment thereof, or a homolog thereof, or a variant of "FLJ10719 (KIAA1794)" encoded by a nucleic acid that hybridizes to the "FLJ10719 (KIAA1794)" nucleic acid under low stringency conditions,
   (xvii) "SYNAPTIC GLYCOPROTEIN SC2" (SEQ ID No:63) or a functionally active derivative thereof, or a functionally active fragment thereof, or a homolog thereof, or a variant of "SYNAPTIC GLYCOPROTEIN SC2" encoded by a nucleic acid that hybridizes to the "SYNAPTIC GLYCOPROTEIN SC2" nucleic acid under low stringency conditions,
   (xviii) "TYROSINE-PROTEIN KINASE CSK" (SEQ ID No:6) or a functionally active derivative thereof, or a functionally active fragment thereof, or a homolog thereof, or a variant of "TYROSINE-PROTEIN KINASE CSK" encoded by a nucleic acid that hybridizes to the "TYROSINE-PROTEIN KINASE CSK" nucleic acid under low stringency conditions,
   (xix) "RAN-GTP BINDING PROTEIN" (SEQ ID No:64) or a functionally active derivative thereof,or a functionally active fragement thereof, or a homolog thereof, or a variant of "RAN-GTP BINDING PROTEIN" that hybridizes to the "RAN-GTP BINDING PROTEIN" nucleic acid under low stringency conditions, and
   (xx) "2-OXOGLUTARATE DEHYDROGENASE E1 COMPONENT, MITOCHONDRIAL PRECURSOR" (SEQ ID No:65) or a functionally active derivative thereof, or a functionally active fragment thereof, or a homolog thereof, or a variant of "2-OXOGLUTARATE DEHYDROGENASE E1 COMPONENT, MITOCHONDRIAL PRECURSOR" encoded by a nucleic acid that hybridizes to the "2-OXOGLUTARATE DEHYDROGENASE E1 COMPONENT, MITOCHONDRIAL PRECURSOR" nucleic acid under low stringency conditions;
   and a protein complex selected from complex (II) that comprises all but 1 - 16 of the following proteins:
   (i) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions,
   (ii) "TNFR2" (SEQ ID No:52) or a functionally active derivative thereof, or a functionally active fragment thereof, or a homolog thereof, or a variant of "TNFR2" encoded by a nucleic acid that hybridizes to the "TNFR2" nucleic acid under low stringency conditions,
   (iii) "TRICARBOXYLATE TRANSPORT PROTEIN, MITOCHONDRIAL PRECURSOR" (SEQ ID No:53) or a functionally active derivative thereof, or a functionally active fragment thereof, or a homolog thereof, or a variant of "TRICARBOXYLATE TRANSPORT PROTEIN, MITOCHONDRIAL PRECURSOR" encoded by a nucleic acid that hybridizes to the "TRICARBOXYLATE TRANSPORT PROTEIN, MITOCHONDRIAL PRECURSOR" nucleic acid under low stringency conditions,
   (iv) "KIAA1040 PROTEIN" (SEQ ID No:30) or a functionally active derivative thereof, or a functionally active fragment thereof, or a homolog thereof, or a variant of "KIAA1040 PROTEIN" encoded by a nucleic acid that hybridizes to the "KIAA1040 PROTEIN" nucleic acid under low stringency conditions,
   (v) "ENDOOLIGOPEPTIDASE A" (SEQ ID No:54) or a functionary active derivative thereof, or a functionally active fragment thereof, or a homolog thereof, or a variant of "ENDOOLIGOPEPTIDASE A" encoded by a nucleic acid that hybridizes to the "ENDOOLIGOPEPTIDASE A" nucleic acid under low stringency conditions,
   (vi) "OXYSTEROL BINDING PROTEIN-RELATED PROTEIN 8" (SEQ ID No:55) or a functionally active derivative thereof, or a functionally active fragment thereof, or a homolog thereof, or a variant of "OXYSTEROL BINDING PROTEIN-RELATED PROTEIN 8" encoded by a nucleic acid that hybridizes to the "OXYSTEROL BINDING PROTEIN-RELATED PROTEIN 8" nucleic acid under low stringency conditions,
   (vii) "LECTIN, GALACTOSIDE-BINDING, SOLUBLE, 8 (GALECTIN 8)" (SEQ ID No:56) or a functionally active derivative thereof, or a functionally active fragment thereof, or a homolog thereof, or a variant of "LECTIN, GALACTOSIDE-BINDING, SOLUBLE, 8 (GALECTIN 8)" encoded by a nucleic acid that hybridizes to the "LECTIN, GALACTOSIDE-BINDING, SOLUBLE, 8 (GALECTIN 8)" nucleic acid under low stringency conditions,
   (viii) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,
   (ix) "β-1,4 MANNOSYLTRANSFERASE" (SEQ ID No:57) or a functionally active derivative thereof, or a functionally active fragment thereof, or a homolog thereof, or a variant of "β-1,4 MANNOSYLTRANSFERASE" encoded by a nucleic acid that hybridizes to the "β-1,4 MANNOSYLTRANSFERASE" nucleic acid under low stringency conditions,
   (x) "β-FILAMIN" (SEQ ID No:58) or a functionally active derivative thereof, or a functionally active fragment thereof, or a homolog thereof, or a variant of "β-FILAMIN" encoded by a nucleic acid that hybridizes to the "β-FILAMIN" nucleic acid under low stringency conditions,
   (xi) "HYPOTHETICAL 57.0 KDA PROTEIN" (SEQ ID No:59) or a functionally active derivative thereof, or a functionally active fragment thereof, or a homolog thereof, or a variant of "HYPOTHETICAL 57.0 KDA PROTEIN" encoded by a nucleic acid that hybridizes to the "HYPOTHETICAL 57.0 KDA PROTEIN" nucleic acid under low stringency conditions,
   (xii) "FILAMIN A" (SEQ ID No:60) or a functionally active derivative thereof, or a functionally active fragment thereof, or a homolog thereof, or a variant of "FILAMIN A" encoded by a nucleic acid that hybridizes to the "FILAMIN A" nucleic acid under low stringency conditions,
   (xiii) "HS1-ASSOCIATED PROTEIN X-1" (SEQ ID No:61) or a functionally active derivative thereof, or a functionally active fragment thereof, or a homolog thereof, or a variant of "HS1-ASSOCIATED PROTEIN X-1" encoded by a nucleic acid that hybridizes to the "HS1-ASSOCIATED PROTEIN X-1" nucleic acid under low stringency conditions,
   (xiv) "LIPOAMIDE ACYLTRANSFERASE COMPONENT OF BRANCHED-CHAIN α-KETO ACID DEHYDROGENASE COMPLEX, MITOCHONDRIAL PRECURSOR" (SEQ ID No:62) or a functionally active derivative thereof, or a functionally active fragment thereof, or a homolog thereof, or a variant of "LIPOAMIDE ACYLTRANSFERASE COMPONENT OF BRANCHED-CHAIN α-KETO ACID DEHYDROGENASE COMPLEX, MITOCHONDRIAL PRECURSOR" encoded by a nucleic acid that hybridizes to the "LIPOAMIDE ACYLTRANSFERASE COMPONENT OF BRANCHED-CHAIN α-KETO ACID DEHYDROGENASE COMPLEX, MITOCHONDRIAL PRECURSOR" nucleic acid under low stringency conditions,
   (xv) "FLJ10719 (KIAA1794)" (SEQ ID No:19) or a functionally active derivative thereof, or a functionally active fragment thereof, or a homolog thereof, or a variant of "FLJ10719 (KIAA1794)" encoded by a nucleic acid that hybridizes to the "FLJ10719 (KIAA1794)" nucleic acid under low stringency conditions,
   (xvi) "SYNAPTIC GLYCOPROTEIN SC2" (SEQ ID No:63) or a functionally active derivative thereof, or a functionally active fragment thereof, or a homolog thereof, or a variant of "SYNAPTIC GLYCOPROTEIN SC2" encoded by a nucleic acid that hybridizes to the "SYNAPTIC GLYCOPROTEIN SC2" nucleic acid under low stringency conditions,
   (xvii) "TYROSINE-PROTEIN KINASE CSK" (SEQ ID No:6) or a functionally active derivative thereof, or a functionally active fragment thereof, or a homolog thereof, or a variant of "TYROSINE-PROTEIN KINASE CSK" encoded by a nucleic acid that hybridizes to the "TYROSINE-PROTEIN KINASE CSK" nucleic acid under low stringency conditions,
   (xviii) "RAN-GTP BINDING PROTEIN" (SEQ ID No:64) or a functionally active derivative thereof,or a functionally active fragement thereof, or a homolog thereof, or a variant of "RAN-GTP BINDING PROTEIN" that hybridizes to the "RAN-GTP BINDING PROTEIN" nucleic acid under low stringency conditions, and
   (xix) "2-OXOGLUTARATE DEHYDROGENASE E1 COMPONENT, MITOCHONDRIAL PRECURSOR" (SEQ ID No:65) or a functionally active derivative thereof, or a functionally active fragment thereof, or a homolog thereof, or a variant of "2-OXOGLUTARATE DEHYDROGENASE E1 COMPONENT, MITOCHONDRIAL PRECURSOR" encoded by a nucleic acid that hybridizes to the "2-OXOGLUTARATE DEHYDROGENASE E1 COMPONENT, MITOCHONDRIAL PRECURSOR" nucleic acid under low stringency conditions.
5. The complex of any of No. 1 - 4 comprising a functionally active derivative of said first protein and/or a functionally active derivative of said second protein, wherein the functionally active derivative is a fusion protein comprising said first protein or said second protein fused to an amino acid sequence different from the first protein or second protein, respectively.
6. The complex of No. 5 wherein the functionally active derivative is a fusion protein comprising said first protein or said second protein fused to an affinity tag or label.
7. The complex of any of No. 1 - 4 comprising a fragment of said first protein and/or a fragment of said second protein, which fragment binds to another protein component of said complex.
8. The complex of any of No. 1 -7 that is involved in the Receptor activity.
9. A process for preparing complex of any of No. 1 - 8 and optionally the components thereof comprising the following steps:
   the expressing a protein (bait) of the complex, preferably a tagged protein, in a target cell, isolating the protein complex which is attached to the bait protein, and optionally disassociating the protein complex and isolating the individual complex members.
10. The process according to No. 9 wherein the tagged protein comprises two different tags which allow two separate affinity purification steps.
11. The process according to any of No. 9 - 10 wherein the two tags are separated by a cleavage site for a protease.
12. Component of the TNFR2 complex obtainable by a process according to any of No. 9 to 11.
13. Protein of the TNFR2 complex selected from
   (i) "KIAA1040 PROTEIN" (SEQ ID No:30) or a functionally active derivative thereof, or a functionally active fragment thereof, or a homolog thereof, or a variant of "KIAA1040 PROTEIN" encoded by a nucleic acid that hybridizes to the "KIAA1040 PROTEIN" nucleic acid under low stringency conditions, and
   (ii) "FLJ10719 (KIAA1794)" (SEQ ID No:19) or a functionally active derivative thereof, or a functionally active fragment thereof, or a homolog thereof, or a variant of "FLJ10719 (KIAA1794)" encoded by a nucleic acid that hybridizes to the "FLJ10719 (KIAA1794)" nucleic acid under low stringency conditions,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
14. Nucleic acid encoding a protein according to No. 13.
15. Construct, preferably a vector construct, comprising (a) a nucleic acid according to No. 14 and at least one further nucleic acid which is normally not associated with said nucleic acid,or(b) at least two separate nucleic acid sequences each encoding a different protein, or a functionally active fragment or a functionally active derivative thereof at least one of said proteins, or functionally active fragments or functionally active derivative thereof being selected from the first group of proteins according to No. 1(a) and at least one of said proteins, or functionally active fragments or functionally active derivative thereof being selected from the second group of proteins according to No. 1 (b).
16. Host cell, containing a vector comprising at least one of the nucleic acid of No. 14 and/or a construct of No. 15 or containing several vectors each comprising at least the nucleic acid sequence encoding at least one of the proteins, or functionally active fragments or functionally active derivatives thereof selected from the first group of proteins according to No. 1(a) and the proteins, or functionally active fragments or functionally active derivatives thereof selected from the second group of proteins according to No. 1(b).
17. An antibody or a fragment of said antibody containing the binding domain thereof, selected from an antibody or fragment thereof, which binds the complex of any of No. 1-8 and which does not bind any of the proteins of said complex when uncomplexed and an antibody or a fragment of said antibody which binds to any of the proteins according to No. 13.
18. A kit comprising in one or more container the complex of any of No. 1 - 8 and/or the proteins of No. 13 optionally together with an antibody according to No. 17 and/or further components such as reagents and working instructions.
19. The kit according to No. 18 for processing a substrate of said complex.
20. The kit according to No. 18 for the diagnosis or prognosis of a disease or a disease risk, preferentially for a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
21. Array, in which at least a complex according to any of No. 1-8 and/or at least one protein according to No. 14 and/or at least one antibody according to No. 17 is attached to a solid carrier.
22. A process for processing a physiological substrate of the complex comprising the step of bringing into contact a complex to any of No. 1 - 8 with said substrate, such that said substrate is processed.
23. A pharmaceutical composition comprising the protein complex of any of No. 1 to 8 and/or any of the following the proteins:
   (i) "KIAA1040 PROTEIN" (SEQ ID No:30) or a functionally active derivative thereof,or a functionally active fragement thereof, or a homolog thereof, or a variant of "KIAA1040 PROTEIN" that hybridizes to the "KIAA1040 PROTEIN" nucleic acid under low stringency conditions, and
   (ii) "FLJ10719 (KIAA1794)" (SEQ ID No:19) or a functionally active derivative thereof, or a functionally active fragment thereof, or a homolog thereof, or a variant of "FLJ10719 (KIAA1794)" encoded by a nucleic acid that hybridizes to the "FLJ10719 (KIAA1794)" nucleic acid under low stringency conditions,
   and a pharmaceutical acceptable carrier.
24. The pharmaceutical composition according to No. 23 for the treatment of diseases and disorders such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
25. A method for screening for a molecule that binds to the complex of anyone of No. 1 - 8 and/or any of the following the proteins:
   (i) "KIAA1040 PROTEIN" (SEQ ID No:30) or a functionally active derivative thereof,or a functionally active fragement thereof, or a homolog thereof, or a variant of "KIAA1040 PROTEIN" that hybridizes to the "KIAA1040 PROTEIN" nucleic acid under low stringency conditions, and
   (ii) "FLJ10719 (KIAA1794)" (SEQ ID No:19) or a functionally active derivative thereof, or a functionally active fragment thereof, or a homolog thereof, or a variant of "FLJ10719 (KIAA1794)" encoded by a nucleic acid that hybridizes to the "FLJ10719 (KIAA1794)" nucleic acid under low stringency conditions, comprising the steps of
      (a) exposing said complex, or a cell or organism containing same to one or more candidate molecules; and
      (b) determinig whether said candidate molecule is bound to the complex or protein.
26. A method for screening for a molecule that modulates directly or indirectly the function, activity, composition or formation of the complex of any one of No. 1 - 8 comprising the steps of (a) exposing said complex, or a cell or organism containing TNFR2 complex to one or more candidate molecules; and
   (b) determining the amount of activity of protein components of, and/or intracellular localization of, said complex and/or the transcription level of a gene dependent on the complex and/or the abundance and/or activity of a protein or protein complex dependent on the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependent on the complex and/or the abundance and/or activity of a protein or protein complex dependent on the function of the complex and/or product of a gene dependent on the complex in the absence of said candidate molecules indicates that the molecule modulates function, activity or composition of said complex.
27. The method of No. 26, wherein the amount of said complex is determined.
28. The method of No. 26, wherein the activity of said complex is determined.
29. The method of No. 28, wherein said determining step comprises isolating from the cell or organism said complex to produce said isolated complex and contacting said isolated complex in the presence or absence of a candidate molecule with a substrate of said complex and determining the processing of said substrate is modified in the presence of said candidate molecule.
30. The method of No. 26, wherein the amount of the individual protein components of said complex are determined.
31. The method of No. 30, determining step comprises determining whether
   (i) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions, and/or
   (ii) "TRAF1" (SEQ ID No:14) or a functionally active derivative thereof, or a functionally active fragment thereof, or a homolog thereof, or a variant of "TRAF1" encoded by a nucleic acid that hybridizes to the "TRAF1" nucleic acid under low stringency conditions, and/or
   (iii) "TNFR2" (SEQ ID No:52) or a functionally active derivative thereof, or a functionally active fragment thereof, or a homolog thereof, or a variant of "TNFR2" encoded by a nucleic acid that hybridizes to the "TNFR2" nucleic acid under low stringency conditions, and/or
   (iv) "TRICARBOXYLATE TRANSPORT PROTEIN, MITOCHONDRIAL PRECURSOR" (SEQ ID No:53) or a,functionally active derivative thereof, or a functionally active fragment thereof, or a homolog thereof, or a variant of "TRICARBOXYLATE TRANSPORT PROTEIN, MITOCHONDRIAL PRECURSOR" encoded by a nucleic acid that hybridizes to the "TRICARBOXYLATE TRANSPORT PROTEIN, MITOCHONDRIAL PRECURSOR" nucleic acid under low stringency conditions, and/or
   (v) "KIAA1040 PROTEIN" (SEQ ID No:30) or a functionally active derivative thereof, or a functionally active fragment thereof, or a homolog thereof, or a variant of "KIAA1040 PROTEIN" encoded by a nucleic acid that hybridizes to the "KIAA1040 PROTEIN" nucleic acid under low stringency conditions, and/or
   (vi) "ENDOOLIGOPEPTIDASE A" (SEQ ID No:54) or a functionally active derivative thereof, or a functionally active fragment thereof, or a homolog thereof, or a variant of "ENDOOLIGOPEPTIDASE A" encoded by a nucleic acid that hybridizes to the "ENDOOLIGOPEPTIDASE A" nucleic acid under low stringency conditions, and/or
   (vii) "OXYSTEROL BINDING PROTEIN-RELATED PROTEIN 8" (SEQ ID No:55) or a functionally active derivative thereof, or a functionally active fragment thereof, or a homolog thereof, or a variant of "OXYSTEROL BINDING PROTEIN-RELATED PROTEIN 8" encoded by a nucleic acid that hybridizes to the "OXYSTEROL BINDING PROTEIN-RELATED PROTEIN 8" nucleic acid under low stringency conditions, and/or
   (viii) "LECTIN, GALACTOSIDE-BINDING, SOLUBLE, 8 (GALECTIN 8)" (SEQ ID No:56) or a functionally active derivative thereof, or a functionally active fragment thereof, or a homolog thereof, or a variant of "LECTIN, GALACTOSIDE-BINDING, SOLUBLE, 8 (GALECTIN 8)" encoded by a nucleic acid that hybridizes to the "LECTIN, GALACTOSIDE-BINDING, SOLUBLE, 8 (GALECTIN 8)" nucleic acid under low stringency conditions, and/or
   (ix) "FANCONI ANEMIA COMPLEMENTATION GROUP D2 PROTEIN, ISOFORM 1" (SEQ ID No: 18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions, and/or
   (x) "β-1,4 MANNOSYLTRANSFERASE" (SEQ ID No:57) or a functionally active derivative thereof, or a functionally active fragment thereof, or a homolog thereof, or a variant of "β-1,4 MANNOSYLTRANSFERASE" encoded by a nucleic acid that hybridizes to the "β-1,4 MANNOSYLTRANSFERASE" nucleic acid under low stringency conditions, and/or
   (xi) "β-FILAMIN" (SEQ ID No:58) or a functionally active derivative thereof, or a functionally active fragment thereof, or a homolog thereof, or a variant of "β-FILAMIN" encoded by a nucleic acid that hybridizes to the "β-FILAMIN" nucleic acid under low stringency conditions, and/or
   (xii) "HYPOTHETICAL 57.0 KDA PROTEIN" (SEQ ID No:59) or a functionally active derivative thereof, or a functionally active fragment thereof, or a homolog thereof, or a variant of "HYPOTHETICAL 57.0 KDA PROTEIN" encoded by a nucleic acid that hybridizes to the "HYPOTHETICAL 57.0 KDA PROTEIN" nucleic acid under low stringency conditions, and/or
   (xiii) "FILAMIN A" (SEQ ID No:60) or a functionally active derivative thereof, or a functionally active fragment thereof, or a homolog thereof, or a variant of "FILAMIN A" encoded by a nucleic acid that hybridizes to the "FILAMIN A" nucleic acid under low stringency conditions, and/or
   (xiv) "HS1-ASSOCIATED PROTEIN X-1" (SEQ ID No:61) or a functionally active derivative thereof, or a functionally active fragment thereof, or a homolog thereof, or a variant of "HS1-ASSOCIATED PROTEIN X-1" encoded by a nucleic acid that hybridizes to the "HS1-ASSOClATED PROTEIN X-1" nucleic acid under low stringency conditions, and/or
   (xv) "LIPOAMIDE ACYLTRANSFERASE COMPONENT OF BRANCHED-CHAIN α-KETO ACID DEHYDROGENASE COMPLEX, MITOCHONDRIAL PRECURSOR" (SEQ ID No:62) or a functionally active derivative thereof, or a functionally active fragment thereof, or a homolog thereof, or a variant of "LIPOAMIDE ACYLTRANSFERASE COMPONENT OF BRANCHED-CHAIN α-KETO ACID DEHYDROGENASE COMPLEX, MITOCHONDRIAL PRECURSOR" encoded by a nucleic acid that hybridizes to the "LIPOAMIDE ACYLTRANSFERASE COMPONENT OF BRANCHED-CHAIN α-KETO ACID DEHYDROGENASE COMPLEX, MITOCHONDRIAL PRECURSOR" nucleic acid under low stringency conditions, and/or
   (xvi) "FLJ10719 (KIAA1794)" (SEQ ID No: 19) or a functionally active derivative thereof, or a functionally active fragment thereof, or a homolog thereof, or a variant of "FLJ10719 (KIAA1794)" encoded by a nucleic acid that hybridizes to the "FLJ10719 (KIAA1794)" nucleic acid under low stringency conditions, and/or
   (xvii) "SYNAPTIC GLYCOPROTEIN SC2" (SEQ ID No:63) or a functionally active derivative thereof, or a functionally active fragment thereof, or a homolog thereof, or a variant of "SYNAPTIC GLYCOPROTEIN SC2" encoded by a nucleic acid that hybridizes to the "SYNAPTIC GLYCOPROTEIN SC2" nucleic acid under low stringency conditions, and/or
   (xviii) "TYROSINE-PROTEIN KINASE CSK" (SEQ ID No:6) or a functionally active derivative thereof, or a functionally active fragment thereof, or a homolog thereof, or a variant of "TYROSINE-PROTEIN KINASE CSK" encoded by a nucleic acid that hybridizes to the "TYROSINE-PROTEIN KINASE CSK" nucleic acid under low stringency conditions, and/or
   (xix) "RAN-GTP BINDING PROTEIN" (SEQ ID No:64) or a functionally active derivative thereof, or a functionally active fragment thereof, or a homolog thereof, or a variant of "RAN-GTP BINDING PROTEIN" encoded by a nucleic acid that hybridizes to the "RAN-GTP BINDING PROTEIN" nucleic acid under low stringency conditions, and/or
   (xx) "2-OXOGLUTARATE DEHYDROGENASE E1 COMPONENT, MITOCHONDRIAL PRECURSOR" (SEQ ID No:65) or a functionally active derivative thereof, or a functionally active fragment thereof, or a homolog thereof, or a variant of "2-OXOGLUTARATE DEHYDROGENASE E1 COMPONENT, MITOCHONDRIAL PRECURSOR" encoded by a nucleic acid that hybridizes to the "2-OXOGLUTARATE DEHYDROGENASE E1 COMPONENT, MITOCHONDRIAL PRECURSOR" nucleic acid under low stringency conditions,
   is present in the complex.
32. The method of any of No. 26 to 31, wherein said method is a method of screening for a drug for treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
33. Use of a molecule that modulates the amount of, activity of, or the protein components of the complex of any one of No. 1 - 8 for the manufacture of a medicament for the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
34. A method for the production of a pharmaceutical composition comprising carrying out the method of any of No. 1 - 8 to identify a molecule that modulates the function, activity, composition or formation of said complex, and further comprising mixing the identified molecule with a pharmaceutically acceptable carrier.
35. A method for diagnosing or screening for the presence of a disease or disorder or a predisposition for developing a disease or disorder in a subject, which disease or disorder is characterized by an aberrant amount of, activity of, or component composition of, or intracellular localization of the complex of any one of the No. 1 - 8, comprising determining the amount of, activity of, protein components of, and/or intracellular localization of, said complex and/or the transcription level of a gene dependent on the complex and/or the abundance and/or activity of a protein or protein complex dependent on the function of the complex and/or product of a gene dependent on the complex in a comparative sample derived from a subject, wherein a difference in said amount, activity, or protein components of, said complex in an analogous sample from a subject not having the disease or disorder or predisposition indicates the presence in the subject of the disease or disorder or predisposition in the subject.
36. The method of No. 35, wherein the amount of said complex is determined.
37. The method of No. 35, wherein the activity of said complex is determined.
38. The method of No. 37, wherein said determining step comprises isolating from the subject said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
39. The method of No. 35, wherein the amount of the individual protein components of said complex are determined.
40. The method of No. 39, wherein said determining step comprises determining whether
   (i) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions, and/or
   (ii) "TRAF1" (SEQ ID No: 14) or a functionally active derivative thereof, or a functionally active fragment thereof, or a homolog thereof, or a variant of "TRAF1" encoded by a nucleic acid that hybridizes to the "TRAF1" nucleic acid under low stringency conditions, and/or
   (iii) "TNFR2" (SEQ ID No:52) or a functionally active derivative thereof, or a functionally active fragment thereof, or a homolog thereof, or a variant of "TNFR2" encoded by a nucleic acid that hybridizes to the "TNFR2" nucleic acid under low stringency conditions, and/or
   (iv) "TRICARBOXYLATE TRANSPORT PROTEIN, MITOCHONDRIAL PRECURSOR" (SEQ ID No:53) or a functionally active derivative thereof, or a functionally active fragment thereof, or a homolog thereof, or a variant of "TRICARBOXYLATE TRANSPORT PROTEIN, MITOCHONDRIAL PRECURSOR" encoded by a nucleic acid that hybridizes to the "TRICARBOXYLATE TRANSPORT PROTEIN, MITOCHONDRIAL PRECURSOR" nucleic acid under low stringency conditions, and/or
   (v) "KIAA1040 PROTEIN" (SEQ ID No:30) or a functionally active derivative thereof, or a functionally active fragment thereof, or a homolog thereof, or a variant of "KIAA1040 PROTEIN" encoded by a nucleic acid that hybridizes to the "KIAA1040 PROTEIN" nucleic acid under low stringency conditions, and/or
   (vi) "ENDOOLIGOPEPTIDASE A" (SEQ ID No:54) or a functionally active derivative thereof, or a functionally active fragment thereof, or a homolog thereof, or a variant of "ENDOOLIGOPEPTIDASE A" encoded by a nucleic acid that hybridizes to the "ENDOOLIGOPEPTIDASE A" nucleic acid under low stringency conditions, and/or
   (vii) "OXYSTEROL BINDING PROTEIN-RELATED PROTEIN 8" (SEQ ID No:55) or a functionally active derivative thereof, or a functionally active fragment thereof, or a homolog thereof, or a variant of "OXYSTEROL BINDING PROTEIN-RELATED PROTEIN 8" encoded by a nucleic acid that hybridizes to the "OXYSTEROL BINDING PROTEIN-RELATED PROTEIN 8" nucleic acid under low stringency conditions, and/or
   (viii) "LECTIN, GALACTOSIDE-BINDING, SOLUBLE, 8 (GALECTIN 8)" (SEQ ID No:56) or a functionally active derivative thereof, or a functionally active fragment thereof, or a homolog thereof, or a variant of "LECTIN, GALACTOSIDE-BINDING, SOLUBLE, 8 (GALECTIN 8)" encoded by a nucleic acid that hybridizes to the "LECTIN, GALACTOSIDE-BINDING, SOLUBLE, 8 (GALECTIN 8)" nucleic acid under low stringency conditions, and/or
   (ix) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions, and/or
   (x) "β-1,4 MANNOSYLTRANSFERASE" (SEQ ID No:57) or a functionally active derivative thereof, or a functionally active fragment thereof, or a homolog thereof, or a variant of "β-1,4 MANNOSYLTRANSFERASE" encoded by a nucleic acid that hybridizes to the "β-1,4 MANNOSYLTRANSFERASE" nucleic acid under low stringency conditions, and/or
   (xi) "β-FILAMIN" (SEQ ID No:58) or a functionally active derivative thereof, or a functionally active fragment thereof, or a homolog thereof, or a variant of "β-FILAMIN" encoded by a nucleic acid that hybridizes to the "β-FILAMIN" nucleic acid under low stringency conditions, and/or
   (xii) "HYPOTHETICAL 57.0 KDA PROTEIN" (SEQ ID No:59) or a functionally active derivative thereof, or a functionally active fragment thereof, or a homolog thereof, or a variant of "HYPOTHETICAL 57.0 KDA PROTEIN" encoded by a nucleic acid that hybridizes to the "HYPOTHETICAL 57.0 KDA PROTEIN" nucleic acid under low stringency conditions, and/or
   (xiii) "FILAMIN A" (SEQ ID No:60) or a functionally active derivative thereof, or a functionally active fragment thereof, or a homolog thereof, or a variant of "FILAMIN A" encoded by a nucleic acid that hybridizes to the "FILAMIN A" nucleic acid under low stringency conditions, and/or
   (xiv) "HS1-ASSOCIATED PROTEIN X-1" (SEQ ID No:61) or a functionally active derivative thereof, or a functionally active fragment thereof, or a homolog thereof, or a variant of "HS1-ASSOCIATED PROTEIN X-1" encoded by a nucleic acid that hybridizes to the "HS1-ASSOCIATED PROTEIN X-1" nucleic acid under low stringency conditions, and/or
   (xv) "LIPOAMIDE ACYLTRANSFERASE COMPONENT OF BRANCHED-CHAIN α-KETO ACID DEHYDROGENASE COMPLEX, MITOCHONDRIAL PRECURSOR" (SEQ ID No:62) or a functionally active derivative thereof, or a functionally active fragment thereof, or a homolog thereof, or a variant of "LIPOAMIDE ACYLTRANSFERASE COMPONENT OF BRANCHED-CHAIN α-KETO ACID DEHYDROGENASE COMPLEX, MITOCHONDRIAL PRECURSOR" encoded by a nucleic acid that hybridizes to the "LIPOAMIDE ACYLTRANSFERASE COMPONENT OF BRANCHED-CHAIN α-KETO ACID DEHYDROGENASE COMPLEX, MITOCHONDRIAL PRECURSOR" nucleic acid under low stringency conditions, and/or
   (xvi) "FLJ10719 (KIAA1794)" (SEQ ID No:19) or a functionally active derivative thereof, or a functionally active fragment thereof, or a homolog thereof, or a variant of "FLJ10719 (KIAA1794)" encoded by a nucleic acid that hybridizes to the "FLJ10719 (KIAA1794)" nucleic acid under low stringency conditions, and/or
   (xvii) "SYNAPTIC GLYCOPROTEIN SC2" (SEQ ID No:63) or a functionally active derivative thereof, or a functionally active fragment thereof, or a homolog thereof, or a variant of "SYNAPTIC GLYCOPROTEIN SC2" encoded by a nucleic acid that hybridizes to the "SYNAPTIC GLYCOPROTEIN SC2" nucleic acid under low stringency conditions, and/or
   (xviii) "TYROSINE-PROTEIN KINASE CSK" (SEQ ID No:6) or a functionally active derivative thereof, or a functionally active fragment thereof, or a homolog thereof, or a variant of "TYROSINE-PROTEIN KINASE CSK" encoded by a nucleic acid that hybridizes to the "TYROSINE-PROTEIN KINASE CSK" nucleic acid under low stringency conditions, and/or
   (xix) "RAN-GTP BINDING PROTEIN" (SEQ ID No:64) or a functionally active derivative thereof, or a functionally active fragment thereof, or a homolog thereof, or a variant of "RAN-GTP BINDING PROTEIN" encoded by a nucleic acid that hybridizes to the "RAN-GTP BINDING PROTEIN" nucleic acid under low stringency conditions, and/or
   (xx) "2-OXOGLUTARATE DEHYDROGENASE E1 COMPONENT, MITOCHONDRIAL PRECURSOR" (SEQ ID No:65) or a functionally active derivative thereof, or a functionally active fragment thereof, or a homolog thereof, or a variant of "2-OXOGLUTARATE DEHYDROGENASE E1 COMPONENT, MITOCHONDRIAL PRECURSOR" encoded by a nucleic acid that hybridizes to the "2-OXOGLUTARATE DEHYDROGENASE E1 COMPONENT, MITOCHONDRIAL PRECURSOR" nucleic acid under low stringency conditions,
   is present in the complex.
41. The complex of any one of No. 1 - 8, or proteins of No. 13 or the antibody or fragment of No. 17, for use in a method of diagnosing a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
42. A method for treating or preventing a disease or disorder characterized by an aberrant amount of, activity or component composition of or intracellular localization of, the complex of anyone of No. 1 - 8, comprising administering to a subject in need of such treatment or prevention a therapeutically effective amount of one or more molecules that modulate the amount of, Receptor activity, or protein components of, said complex.
43. The method according to No. 42, wherein said disease or disorder involves decreased levels of the amount or activity of said complex.
44. The method according to No. 42 , wherein said disease or disorder involves increased levels of the amount or activity of said complex.
45. Complex of any of No. 1-8 and/or protein selected from the following proteins
   (i) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions,
   (ii) "TRAF1" (SEQ ID No:14) or a functionally active derivative thereof, or a functionally active fragment thereof, or a homolog thereof, or a variant of "TRAF1" encoded by a nucleic acid that hybridizes to the "TRAF1" nucleic acid under low stringency conditions,
   (iii) "TNFR2" (SEQ ID No:52) or a functionally active derivative thereof, or a functionally active fragment thereof, or a homolog thereof, or a variant of "TNFR2" encoded by a nucleic acid that hybridizes to the "TNFR2" nucleic acid under low stringency conditions,
   (iv) "TRICARBOXYLATE TRANSPORT PROTEIN, MITOCHONDRIAL PRECURSOR" (SEQ ID No:53) or a functionally active derivative thereof, or a functionally active fragment thereof, or a homolog thereof, or a variant of "TRICARBOXYLATE TRANSPORT PROTEIN, MITOCHONDRIAL PRECURSOR" encoded by a nucleic acid that hybridizes to the "TRICARBOXYLATE TRANSPORT PROTEIN, MITOCHONDRIAL PRECURSOR" nucleic acid under low stringency conditions,
   (v) "KIAA1040 PROTEIN" (SEQ ID No:30) or a functionally active derivative thereof, or a functionally active fragment thereof, or a homolog thereof, or a variant of "KIAA1040 PROTEIN" encoded by a nucleic acid that hybridizes to the "KIAA1040 PROTEIN" nucleic acid under low stringency conditions,
   (vi) "ENDOOLIGOPEPTIDASE A" (SEQ ID No:54) or a functionally active derivative thereof, or a functionally active fragment thereof, or a homolog thereof, or a variant of "ENDOOLIGOPEPTIDASE A" encoded by a nucleic acid that hybridizes to the "ENDOOLIGOPEPTIDASE A" nucleic acid under low stringency conditions,
   (vii) "OXYSTEROL BINDING PROTEIN-RELATED PROTEIN 8" (SEQ ID No:55) or a functionally active derivative thereof, or a functionally active fragment thereof, or a homolog thereof, or a variant of "OXYSTEROL BINDING PROTEIN-RELATED PROTEIN 8" encoded by a nucleic acid that hybridizes to the "OXYSTEROL BINDING PROTEIN-RELATED PROTEIN 8" nucleic acid under low stringency conditions,
   (viii) "LECTIN, GALACTOSIDE-BINDING, SOLUBLE, 8 (GALECTIN 8)" (SEQ ID No:56) or a functionally active derivative thereof, or a functionally active fragment thereof, or a homolog thereof, or a variant of "LECTIN, GALACTOSIDE-BINDING, SOLUBLE, 8 (GALECTIN 8)" encoded by a nucleic acid that hybridizes to the "LECTIN, GALACTOSIDE-BINDING, SOLUBLE, 8 (GALECTIN 8)" nucleic acid under low stringency conditions,
   (ix) "FANCONI ANEMIA COMPLEMENTATION GROUP D2 PROTEIN, ISOFORM 1" (SEQ ID No: 18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,
   (x) "β-1,4 MANNOSYLTRANSFERASE" (SEQ ID No:57) or a functionally active derivative thereof, or a functionally active fragment thereof, or a homolog thereof, or a variant of "β-1,4 MANNOSYLTRANSFERASE" encoded by a nucleic acid that hybridizes to the "β-1,4 MANNOSYLTRANSFERASE" nucleic acid under low stringency conditions,
   (xi) "β-FILAMIN" (SEQ ID No:58) or a functionally active derivative thereof, or a functionally active fragment thereof, or a homolog thereof, or a variant of "β-FILAMIN" encoded by a nucleic acid that hybridizes to the "β-FILAMIN" nucleic-acid under low stringency conditions,
   (xii) "HYPOTHETICAL 57.0 KDA PROTEIN" (SEQ ID No:59) or a functionally active derivative thereof, or a functionally active fragment thereof, or a homolog thereof, or a variant of "HYPOTHETICAL 57.0 KDA PROTEIN" encoded by a nucleic acid that hybridizes to the "HYPOTHETICAL 57.0 KDA PROTEIN" nucleic acid under low stringency conditions,
   (xiii) "FILAMIN A" (SEQ ID No:60) or a functionally active derivative thereof, or a functionally active fragment thereof, or a homolog thereof, or a variant of "FILAMIN A" encoded by a nucleic acid that hybridizes to the "FILAMIN A" nucleic acid under low stringency conditions,
   (xiv) "HS1-ASSOCIATED PROTEIN X-1" (SEQ ID No:61) or a functionally active derivative thereof, or a functionally active fragment thereof, or a homolog thereof, or a variant of "HS1-ASSOCIATED PROTEIN X-1" encoded by a nucleic acid that hybridizes to the "HS1-ASSOCIATED PROTEIN X-1" nucleic acid under low stringency conditions,
   (xv) "LIPOAMIDE ACYLTRANSFERASE COMPONENT OF BRANCHED-CHAIN α-KETO ACID DEHYDROGENASE COMPLEX, MITOCHONDRIAL PRECURSOR" (SEQ ID No:62) or a functionally active derivative thereof, or a functionally active fragment thereof, or a homolog thereof, or a variant of "LIPOAMIDE ACYLTRANSFERASE COMPONENT OF BRANCHED-CHAIN α-KETO ACID DEHYDROGENASE COMPLEX, MITOCHONDRIAL PRECURSOR" encoded by a nucleic acid that hybridizes to the "LIPOAMIDE ACYLTRANSFERASE COMPONENT OF BRANCHED-CHAIN α-KETO ACID DEHYDROGENASE COMPLEX, MITOCHONDRIAL PRECURSOR" nucleic acid under low stringency conditions,
   (xvi) "FLJ10719 (KIAA1794)" (SEQ ID No:19) or a functionally active derivative thereof, or a functionally active fragment thereof, or a homolog thereof, or a variant of "FLJ10719 (KIAA1794)" encoded by a nucleic acid that hybridizes to the "FLJ10719 (KIAA1794)" nucleic acid under low stringency conditions,
   (xvii) "SYNAPTIC GLYCOPROTEIN SC2" (SEQ ID No:63) or a functionally active derivative thereof, or a functionally active fragment thereof, or a homolog thereof, or a variant of "SYNAPTIC GLYCOPROTEIN SC2" encoded by a nucleic acid that hybridizes to the "SYNAPTIC GLYCOPROTEIN SC2" nucleic acid under low stringency conditions,
   (xviii) "TYROSINE-PROTEIN KINASE CSK" (SEQ ID No:6) or a functionally active derivative thereof, or a functionally active fragment thereof, or a homolog thereof, or a variant of "TYROSINE-PROTEIN KINASE CSK" encoded by a nucleic acid that hybridizes to the "TYROSINE-PROTEIN KINASE CSK" nucleic acid under low stringency conditions,
   (xix) "RAN-GTP BINDING PROTEIN" (SEQ ID No:64) or a functionally active derivative thereof,or a functionally active fragement thereof, or a homolog thereof, or a variant of "RAN-GTP BINDING PROTEIN" that hybridizes to the "RAN-GTP BINDING PROTEIN" nucleic acid under low stringency conditions, and
   (xx) "2-OXOGLUTARATE DEHYDROGENASE E1 COMPONENT, MITOCHONDRIAL PRECURSOR" (SEQ ID No:65) or a functionally active derivative thereof, or a functionally active fragment thereof, or a homolog thereof, or a variant of "2-OXOGLUTARATE DEHYDROGENASE E1 COMPONENT, MITOCHONDRIAL PRECURSOR" encoded by a nucleic acid that hybridizes to the "2-OXOGLUTARATE DEHYDROGENASE E1 COMPONENT, MITOCHONDRIAL PRECURSOR" nucleic acid under low stringency conditions,
as a target for an active agent of a pharmaceutical, preferably a drug target in the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.

The present invention further relates to the RelA (p65)-protein complex
1. A protein complex selected from complex (I) and comprising
   (a) at least one first protein selected from the group consisting of:
      (i) "Menin" (SEQ ID No:66) or a functionally active derivative thereof, or a functionally active fragment thereof, or a homolog thereof, or a variant of "Menin" encoded by a nucleic acid that hybridizes to the "Menin" nucleic acid under low stringency conditions,
      (ii) "SMRT" (SEQ ID No:67) or a functionally active derivative thereof, or a functionally active fragment thereof, or a homolog thereof, or a variant of "SMRT " encoded by a nucleic acid that hybridizes to the "SMRT " nucleic acid under low stringency conditions,
      (iii) "HDAC3" (SEQ ID No:68) or a functionally active derivative thereof, or a functionally active fragment thereof, or a homolog thereof, or a variant of "HDAC3" encoded by a nucleic acid that hybridizes to the "HDAC3" nucleic acid under low stringency conditions,
      (iv) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
      (v) "HDAC2" (SEQ ID No:69) or a functionally active derivative thereof, or a functionally active fragment thereof, or a homolog thereof, or a variant of "HDAC2" encoded by a nucleic acid that hybridizes to the "HDAC2" nucleic acid under low stringency conditions,
      (vi) "Cot/Tpl-2" (SEQ ID No:70) or a functionally active derivative thereof, or a functionally active fragment thereof, or a homolog thereof, or a variant of "Cot/Tpl-2" encoded by a nucleic acid that hybridizes to the "Cot/Tpl-2" nucleic acid under low stringency conditions,

      (i) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
      (viii) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
      (i) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
      (x) "p50 (NF-κB1)" (SEQ ID No:73) or a functionally active derivative thereof, or a functionally active fragment thereof, or a homolog thereof, or a variant of "p50 (NF-κB1)" encoded by a nucleic acid that hybridizes to the "p50 (NF-κB1)" nucleic acid under low stringency conditions,
      (xi) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
      (xii) "RelA (p65)" (SEQ ID No:75) or a functionally active derivative thereof, or a functionally active fragment thereof, or a homolog thereof, or a variant of "RelA (p65)" encoded by a nucleic acid that hybridizes to the "RelA (p65)" nucleic acid under low stringency conditions,
      (xiii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
      (xiv) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
      (xv) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
      (xvi) "IKK ε" (SEQ ID No:79) or a functionally active derivative thereof, or a functionally active fragment thereof, or a homolog thereof, or a variant of "IKK ε" encoded by a nucleic acid that hybridizes to the "IKK ε" nucleic acid under low stringency conditions,
      (xvii) "HDAC1" (SEQ ID No:80) or a functionally active derivative thereof, or a functionally active fragment thereof, or a homolog thereof, or a variant of "HDAC1" encoded by a nucleic acid that hybridizes to the "HDAC1" nucleic acid under low stringency conditions,
      (xviii) "IKK β" (SEQ ID No:24) or a functionally active derivative thereof, or a functionally active fragment thereof, or a homolog thereof, or a variant of "I KK β" encoded by a nucleic acid that hybridizes to the "IKK β" nucleic acid under low stringency conditions,
      (xix) "ReIB" (SEQ ID No:81) or a functionally active derivative thereof, or a functionally active fragment thereof, or a homolog thereof, or a variant of "ReIB" encoded by a nucleic acid that hybridizes to the "ReIB" nucleic acid under low stringency conditions,
      (xx) "DNA-DIRECTED RNA POLYMERASE II 33 KDA POLYPEPTIDE" (SEQ ID No:95) or a functionally active derivative thereof, or a functionally active fragment thereof, or a homolog thereof, or a variant of "DNA-DIRECTED RNA POLYMERASE II 33 KDA POLYPEPTIDE encoded by a nucleic acid that hybridizes to the "DNA-DIRECTED RNA POLYMERASE II 33 KDA POLYPEPTIDE" nucleic acid under low stringency conditions, and
      (xxi) "p52 (NF-kappaB2)" (SEQ ID No:231) or a functionally active derivative thereof, or a functionally active fragment thereof, or a homolog thereof, or a variant of "p52 (NF-kappaB2)" encoded by a nucleic acid that hybridises to the "p52 (NF-kappaB2)" nucleic acid under low stringency conditions,
      and
   (b) at least one second protein, which second protein is selected from the group consisting of:
      (i) "NAKAP 95" (SEQ ID No:82) or a functionally active derivative thereof, or a functionally active fragment thereof, or a homolog thereof, or a variant of "NAKAP 95" encoded by a nucleic acid that hybridizes to the "NAKAP 95" nucleic acid under low stringency conditions,
      (ii) "IMPORTIN α-3 SUBUNIT" (SEQ ID No:83) or a functionally active derivative thereof, or a functionally active fragment thereof, or a homolog thereof, or a variant of "IMPORTIN α-3 SUBUNIT" encoded by a nucleic acid that hybridizes to the "IMPORTIN α-3 SUBUNIT" nucleic acid under low stringency conditions,
      (iii) "UBIQUITIN-PROTEIN LIGASE EDD" (SEQ ID No:84) or a functionally active derivative thereof, or a functionally active fragment thereof, or a homolog thereof, or a variant of "UBIQUITIN--PROTEIN LIGASE EDD" encoded by a nucleic acid that hybridizes to the "UBIQUITIN--PROTEIN LIGASE EDD" nucleic acid under low stringency conditions,
      (iv) "HYPOTH 35.7 KDA PROTEIN" (SEQ ID No:85) or a functionally active derivative thereof, or a functionally active fragment thereof, or a homolog thereof, or a variant of "HYPOTH 35.7 KDA PROTEIN" encoded by a nucleic acid that hybridizes to the "HYPOTH 35.7 KDA PROTEIN" nucleic acid under low stringency conditions,
      (v) "MATRIN 3" (SEQ ID No:86) or a functionally active derivative thereof, or a functionally active fragment thereof, or a homolog thereof, or a variant of "MATRIN 3" encoded by a nucleic acid that hybridizes to the "MATRIN 3" nucleic acid under low stringency conditions,
      (vi) "NF45 PROTEIN" (SEQ ID No:87) or a functionally active derivative thereof, or a functionally active fragment thereof, or a homolog thereof, or a variant of "NF45 PROTEIN" encoded by a nucleic acid that hybridizes to the "NF45 PROTEIN" nucleic acid under low stringency conditions,
      (vii) "PCNA" (SEQ ID No:88) or a functionally active derivative thereof, or a functionally active fragment thereof, or a homolog thereof, or a variant of "PCNA" encoded by a nucleic acid that hybridizes to the "PCNA" nucleic acid under low stringency conditions,
      (viii) "54 KDA NUCLEAR RNA- AND DNA-BINDING PROTEIN" (SEQ ID No:89) or a functionally active derivative thereof, or a functionally active fragment thereof, or a homolog thereof, or a variant of "54 KDA NUCLEAR RNA- AND DNA-BINDING PROTEIN" encoded by a nucleic acid that hybridizes to the "54 KDA NUCLEAR RNA-AND DNA-BINDING PROTEIN" nucleic acid under low stringency conditions,
      (ix) "UBIQUITIN CARBOXYL-TERMINAL HYDROLASE 11" (SEQ ID No:90) or a functionally active derivative thereof, or a functionally active fragment thereof, or a homolog thereof, or a variant of "UBIQUITIN CARBOXYL-TERMINAL HYDROLASE 11" encoded by a nucleic acid that hybridizes to the "UBIQUITIN CARBOXYL-TERMINAL HYDROLASE 11" nucleic acid under low stringency conditions,
      (x) "CELL DIFFERENTIATION PROTEIN RCD1" (SEQ ID No:91) or a functionally active derivative thereof, or a functionally active fragment thereof, or a homolog thereof, or a variant of "CELL DIFFERENTIATION PROTEIN RCD1" encoded by a nucleic acid that hybridizes to the "CELL DIFFERENTIATION PROTEIN RCD1" nucleic acid under low stringency conditions,
      (xi) "CHROMATIN ASSEMBLY FACTOR 1 SUBUNIT C" (SEQ ID No:92) or a functionally active derivative thereof, or a functionally active fragment thereof, or a homolog thereof, or a variant of "CHROMATIN ASSEMBLY FACTOR 1 SUBUNIT C" encoded by a nucleic acid that hybridizes to the "CHROMATIN ASSEMBLY FACTOR 1 SUBUNIT C" nucleic acid under low stringency conditions,
      (xii) "HISTONE ACETYLTRANSFERASE TYPE B SUBUNIT 2" (SEQ ID No:93) or a functionally active derivative thereof, or a functionally active fragment thereof, or a homolog thereof, or a variant of "HISTONE ACETYLTRANSFERASE TYPE B SUBUNIT 2" encoded by a nucleic acid that hybridizes to the "HISTONE ACETYLTRANSFERASE TYPE B SUBUNIT 2" nucleic acid under low stringency conditions,
      (xiii) "HYPOTHETICAL PROTEIN KIAA0310" (SEQ ID No:94) or a functionally active derivative thereof, or a functionally active fragment thereof, or a homolog thereof, or a variant of "HYPOTHETICAL PROTEIN KIAA0310" encoded by a nucleic acid that hybridizes to the "HYPOTHETICAL PROTEIN KIAA0310" nucleic acid under low stringency conditions,
      (xiv) "CALCIUM-DEPENDENT PROTEASE, SMALL SUBUNIT. CAPNS1" (SEQ ID No:96) or a functionally active derivative thereof, or a functionally active fragment thereof, or a homolog thereof, or a variant of "CALCIUM-DEPENDENT PROTEASE, SMALL SUBUNIT. CAPNS1" encoded by a nucleic acid that hybridizes to the "CALCIUM-DEPENDENT PROTEASE, SMALL SUBUNIT. CAPNS1" nucleic acid under low stringency conditions,
      (xv) "PCAF-ASSOCIATED FACTOR 400" (SEQ ID No:97) or a functionally active derivative thereof, or a functionally active fragment thereof, or a homolog thereof, or a variant of "PCAF-ASSOCIATED FACTOR 400" encoded by a nucleic acid that hybridizes to the "PCAF-ASSOCIATED FACTOR 400" nucleic acid under low stringency conditions,
      (xvi) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions,
      (xvii) "COMPLEMENT C1R-LIKE PROTEINASE" (SEQ ID No:98) or a functionally active derivative thereof, or a functionally active fragment thereof, or a homolog thereof, or a variant of "COMPLEMENT C1R-LIKE PROTEINASE" encoded by a nucleic acid that hybridizes to the "COMPLEMENT C1R-LIKE PROTEINASE" nucleic acid under low stringency conditions,
      (xviii) "MMS19 PROTEIN" (SEQ ID No:99) or a functionally active derivative thereof, or a functionally active fragment thereof, or a homolog thereof, or a variant of "MMS19 PROTEIN" encoded by a nucleic acid that hybridizes to the "MMS19 PROTEIN" nucleic acid under low stringency conditions, and
      (xix) "TRANSCRIPTION INTERMEDIARY FACTOR 1-β" (SEQ ID No:100) or a functionally active derivative thereof, or a functionally active fragment thereof, or a homolog thereof, or a variant of "TRANSCRIPTION INTERMEDIARY FACTOR 1-β" encoded by a nucleic acid that hybridizes to the "TRANSCRIPTION INTERMEDIARY FACTOR 1-β" nucleic acid under low stringency conditions;
   and a complex (II) comprising at least two of said second proteins,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
2. The protein complex according to claim 1 wherein the first protein is the protein "RelA (p65)" (SEQ ID No:75), or a functionally active derivative thereof, or a functionally active fragment thereof, or a homolog thereof, or a variant of "RelA (p65)" encoded by a nucleic acid that hybridizes to the "RelA (p65)" nucleic acid under low stringency conditions.
3. The protein complex according to No. 1 selected from complex (I) and comprising the following proteins:
   (i) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
   (i) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (iii) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (iv) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
   (v) "IMPORTIN α-3 SUBUNIT" (SEQ ID No:83) or a functionally active derivative thereof, or a functionally active fragment thereof, or a homolog thereof, or a variant of "IMPORTIN α-3 SUBUNIT" encoded by a nucleic acid that hybridizes to the "IMPORTIN α-3 SUBUNIT" nucleic acid under low stringency conditions,
   (vi) "NF45 PROTEIN" (SEQ ID No:87) or a functionally active derivative thereof, or a functionally active fragment thereof, or a homolog thereof, or a variant of "NF45 PROTEIN" encoded by a nucleic acid that hybridizes to the "NF45 PROTEIN" nucleic acid under low stringency conditions,
   (vii) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (viii) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (ix) "p50 (NF-κB1)" (SEQ ID No:73) or a functionally active derivative thereof, or a functionally active fragment thereof, or a homolog thereof, or a variant of "p50 (NF-κB1)" encoded by a nucleic acid that hybridizes to the "p50 (NF-κB1)" nucleic acid under low stringency conditions, and
   (x) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions;
   and a protein complex selected from complex (II) and comprising the following proteins:
   (i) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (ii) "HDAC2" (SEQ ID No:69) or a functionally active derivative thereof, or a functionally active fragment thereof, or a homolog thereof, or a variant of "HDAC2" encoded by a nucleic acid that hybridizes to the "HDAC2" nucleic acid under low stringency conditions,
   (iii) "Cot/Tpl-2" (SEQ ID No:70) or a functionally active derivative thereof, or a functionally active fragment thereof, or a homolog thereof, or a variant of "Cot/Tpl-2" encoded by a nucleic acid that hybridizes to the "Cot/Tpl-2" nucleic acid under low stringency conditions,
   (iv) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (v) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
   (i) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (vii) "p50 (NF-κB1)" (SEQ ID No:73) or a functionally active derivative thereof, or a functionally active fragment thereof, or a homolog thereof, or a variant of "p50 (NF-κB1)" encoded by a nucleic acid that hybridizes to the "p50 (NF-κB1)" nucleic acid under low stringency conditions,
   (viii) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (ix) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions,
   (x) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (xi) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (xii) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
   (xiii) ""IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (xiv) "ReIB" (SEQ ID No:81) or a functionally active derivative thereof, or a functionally active fragment thereof, or a homolog thereof, or a variant of "ReIB" encoded by a nucleic acid that hybridizes to the "ReIB" nucleic acid under low stringency conditions,
   (xv) "NAKAP 95" (SEQ ID No:82) or a functionally active derivative thereof, or a functionally active fragment thereof, or a homolog thereof, or a variant of "NAKAP 95" encoded by a nucleic acid that hybridizes to the "NAKAP 95" nucleic acid under low stringency conditions,

   (i) "IMPORTIN A-3 SUBUNIT" (SEQ ID No:83) or a functionally active derivative thereof, or a functionally active fragment thereof, or a homolog thereof, or a variant of "IMPORTIN α-3 SUBUNIT" encoded by a nucleic acid that hybridizes to the "IMPORTIN α-3 SUBUNIT" nucleic acid under low stringency conditions,
   (xvii) "UBIQUITIN-PROTEIN LIGASE EDD" (SEQ ID No:84) or a functionally active derivative thereof, or a functionally active fragment thereof, or a homolog thereof, or a variant of "UBIQUITIN--PROTEIN LIGASE EDD" encoded by a nucleic acid that hybridizes to the "UBIQUITIN--PROTEIN LIGASE EDD" nucleic acid under low stringency conditions,
   (xviii) "HYPOTH 35.7 KDA PROTEIN" (SEQ ID No:85) or a functionally active derivative thereof, or a functionally active fragment thereof, or a homolog thereof, or a variant of "HYPOTH 35.7 KDA PROTEIN" encoded by a nucleic acid that hybridizes to the "HYPOTH 35.7 KDA PROTEIN" nucleic acid under low stringency conditions,
   (xix) "MATRIN 3" (SEQ ID No:86) or a functionally active derivative thereof, or a functionally active fragment thereof, or a homolog thereof, or a variant of "MATRIN 3" encoded by a nucleic acid that hybridizes to the "MATRIN 3" nucleic acid under low stringency conditions,
   (xx) "NF45 PROTEIN" (SEQ ID No:87) or a functionally active derivative thereof, or a functionally active fragment thereof, or a homolog thereof, or a variant of "NF45 PROTEIN" encoded by a nucleic acid that hybridizes to the "NF45 PROTEIN" nucleic acid under low stringency conditions,
   (xxi) "PCNA" (SEQ ID No:88) or a functionally active derivative thereof, or a functionally active fragment thereof, or a homolog thereof, or a variant of "PCNA" encoded by a nucleic acid that hybridizes to the "PCNA" nucleic acid under low stringency conditions,
   (xxii) "54 KDA NUCLEAR RNA- AND DNA-BINDING PROTEIN" (SEQ ID No:89) or a functionally active derivative thereof, or a functionally active fragment thereof, or a homolog thereof, or a variant of "54 KDA NUCLEAR RNA- AND DNA-BINDING PROTEIN" encoded by a nucleic acid that hybridizes to the "54 KDA NUCLEAR RNA-AND DNA-BINDING PROTEIN" nucleic acid under low stringency conditions,
   (xxiii) "UBIQUITIN CARBOXYL-TERMINAL HYDROLASE 11" (SEQ ID No:90) or a functionally active derivative thereof, or a functionally active fragment thereof, or a homolog thereof, or a variant of "UBIQUITIN CARBOXYL-TERMINAL HYDROLASE 11" encoded by a nucleic acid that hybridizes to the "UBIQUITIN CARBOXYL-TERMINAL HYDROLASE 11" nucleic acid under low stringency conditions,
   (xxiv) "CELL DIFFERENTIATION PROTEIN RCD1" (SEQ ID No:91) or a functionally active derivative thereof, or a functionally active fragment thereof, or a homolog thereof, or a variant of "CELL DIFFERENTIATION PROTEIN RCD1" encoded by a nucleic acid that hybridizes to the "CELL DIFFERENTIATION PROTEIN RCD1" nucleic acid under low stringency conditions,
   (xxv) "CHROMATIN ASSEMBLY FACTOR 1 SUBUNIT C" (SEQ ID No:92) or a functionally active derivative thereof, or a functionally active fragment thereof, or a homolog thereof, or a variant of "CHROMATIN ASSEMBLY FACTOR 1 SUBUNIT C" encoded by a nucleic acid that hybridizes to the "CHROMATIN ASSEMBLY FACTOR 1 SUBUNIT C" nucleic acid under low stringency conditions,
   (xxvi) "HISTONE ACETYLTRANSFERASE TYPE B SUBUNIT 2" (SEQ ID No:93) or a functionally active derivative thereof, or a functionally active fragment thereof, or a homolog thereof, or a variant of "HISTONE ACETYLTRANSFERASE TYPE B SUBUNIT 2" encoded by a nucleic acid that hybridizes to the "HISTONE ACETYLTRANSFERASE TYPE B SUBUNIT 2" nucleic acid under low stringency conditions,
   (xxvii) "HYPOTHETICAL PROTEIN KIAA0310" (SEQ ID No:94) or a functionally active derivative thereof, or a functionally active fragment thereof, or a homolog thereof, or a variant of "HYPOTHETICAL PROTEIN KIAA0310" encoded by a nucleic acid that hybridizes to the "HYPOTHETICAL PROTEIN KIAA0310" nucleic acid under low stringency conditions,
   (xxviii) "DNA-DIRECTED RNA POLYMERASE II 33 KDA POLYPEPTIDE" (SEQ ID No:95) or a functionally active derivative thereof, or a functionally active fragment thereof, or a homolog thereof, or a variant of "DNA-DIRECTED RNA POLYMERASE II 33 KDA POLYPEPTIDE" encoded by a nucleic acid that hybridizes to the "DNA-DIRECTED RNA POLYMERASE II 33 KDA POLYPEPTIDE" nucleic acid under low stringency conditions,
   (xxix) "CALCIUM-DEPENDENT PROTEASE, SMALL SUBUNIT. CAPNS1" (SEQ ID No:96) or a functionally active derivative thereof, or a functionally active fragment thereof, or a homolog thereof, or a variant of "CALCIUM-DEPENDENT PROTEASE, SMALL SUBUNIT. CAPNS1" encoded by a nucleic acid that hybridizes to the "CALCIUM-DEPENDENT PROTEASE, SMALL SUBUNIT. CAPNS1" nucleic acid under low stringency conditions,
   (xxx) "PCAF-ASSOCIATED FACTOR 400" (SEQ ID No:97) or a functionally active derivative thereof, or a functionally active fragment thereof, or a homolog thereof, or a variant of "PCAF-ASSOCIATED FACTOR 400" encoded by a nucleic acid that hybridizes to the "PCAF-ASSOCIATED FACTOR 400" nucleic acid under low stringency conditions,
   (xxxi) "ABIN-2" (SEQ ID No: 1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions,
   (xxxii) "COMPLEMENT C1R-LIKE PROTEINASE" (SEQ ID No:98) or a functionally active derivative thereof, or a functionally active fragment thereof, or a homolog thereof, or a variant of "COMPLEMENT C1R-LIKE PROTEINASE" encoded by a nucleic acid that hybridizes to the "COMPLEMENT C1R-LIKE PROTEINASE" nucleic acid under low stringency conditions,
   (xxxiii) "MMS19 PROTEIN" (SEQ ID No:99) or a functionally active derivative thereof,or a functionally active fragement thereof, or a homolog thereof, or a variant of "MMS19 PROTEIN" that hybridizes to the "MMS19 PROTEIN" nucleic acid under low stringency conditions, and
   (xxxiv) "TRANSCRIPTION INTERMEDIARY FACTOR 1-β" (SEQ ID No:100) or a functionally active derivative thereof, or a functionally active fragment thereof, or a homolog thereof, or a variant of "TRANSCRIPTION INTERMEDIARY FACTOR 1-β" encoded by a nucleic acid that hybridizes to the "TRANSCRIPTION INTERMEDIARY FACTOR 1-β" nucleic acid under low stringency conditions;
   and a protein complex selected from complex (III) and comprising the following proteins:
   (i) "Menin" (SEQ ID No:66) or a functionally active derivative thereof, or a functionally active fragment thereof, or a homolog thereof, or a variant of "Menin" encoded by a nucleic acid that hybridizes to the "Menin" nucleic acid under low stringency conditions,
   (ii) "SMRT" (SEQ ID No:67) or a functionally active derivative thereof, or a functionally active fragment thereof, or a homolog thereof, or a variant of "SMRT" encoded by a nucleic acid that hybridizes to the "SMRT" nucleic acid under low stringency conditions,
   (iii) "HDAC3" (SEQ ID No:68) or a functionally active derivative thereof, or a functionally active fragment thereof, or a homolog thereof, or a variant of "HDAC3" encoded by a nucleic acid that hybridizes to the "HDAC3" nucleic acid under low stringency conditions,
   (iv) "IKK γ" (SEQ ID No:23) or a functionally active derivative thereof, or a functionary active fragment thereof, or a homolog thereof, or a variant of "IKK γ" encoded by a nucleic acid that hybridizes to the "IKK γ" nucleic acid under low stringency conditions,
   (v) "HDAC2" (SEQ ID No:69) or a functionally active derivative thereof, or a functionally active fragment thereof, or a homolog thereof, or a variant of "HDAC2" encoded by a nucleic acid that hybridizes to the "HDAC2" nucleic acid under low stringency conditions,
   (vi) "Cot/Tpl-2" (SEQ ID No:70) or a functionally active derivative thereof, or a functionally active fragment thereof, or a homolog thereof, or a variant of "Cot/Tpl-2" encoded by a nucleic acid that hybridizes to the "Cot/Tpl-2" nucleic acid under low stringency conditions,

   (i) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (viii) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,

   (i) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (x) "p50 (NF-κB1)" (SEQ ID No:73) or a functionally active derivative thereof, or a functionally active fragment thereof, or a homolog thereof, or a variant of "p50 (NF-κB1)" encoded by a nucleic acid that hybridizes to the "p50 (NF-κB1)" nucleic acid under low stringency conditions,
   (xi) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (xii) "RelA (p65)" (SEQ ID No:75) or a functionally active derivative thereof, or a functionally active fragment thereof, or a homolog thereof, or a variant of "RelA (p65)" encoded by a nucleic acid that hybridizes to the "RelA (p65)" nucleic acid under low stringency conditions,
   (xiii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (xiv) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (xv) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
   (xvi) "IKK ε" (SEQ ID No:79) or a functionally active derivative thereof, or a functionally active fragment thereof, or a homolog thereof, or a variant of "IKK ε" encoded by a nucleic acid that hybridizes to the "IKK ε" nucleic acid under low stringency conditions,
   (xvii) "HDAC1" (SEQ ID No:80) or a functionally active derivative thereof, or a functionally active fragment thereof, or a homolog thereof, or a variant of "HDAC1" encoded by a nucleic acid that hybridizes to the "HDAC1" nucleic acid under low stringency conditions,
   (xviii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (xix) "ReIB" (SEQ ID No:81) or a functionally active derivative thereof, or a functionally active fragment thereof, or a homolog thereof, or a variant of "ReIB" encoded by a nucleic acid that hybridizes to the "ReIB" nucleic acid under low stringency conditions,
   (xx) "NAKAP 95" (SEQ ID No:82) or a functionally active derivative thereof, or a functionally active fragment thereof, or a homolog thereof, or a variant of "NAKAP 95" encoded by a nucleic acid that hybridizes to the "NAKAP 95" nucleic acid under low stringency conditions,

   (i) "IMPORTIN α-3 SUBUNIT" (SEQ ID No:83) or a functionally active derivative thereof, or a functionally active fragment thereof, or a homolog thereof, or a variant of "IMPORTIN α-3 SUBUNIT" encoded by a nucleic acid that hybridizes to the "IMPORTIN α-3 SUBUNIT" nucleic acid under low stringency conditions,
   (xxii) "UBIQUITIN--PROTEIN LIGASE EDD" (SEQ ID No:84) or a functionally active derivative thereof, or a functionally active fragment thereof, or a homolog thereof, or a variant of "UBIQUITIN--PROTEIN LIGASE EDD" encoded by a nucleic acid that hybridizes to the "UBIQUITIN--PROTEIN LIGASE EDD" nucleic acid under low stringency conditions,
   (xxiii) "HYPOTH 35.7 KDA PROTEIN" (SEQ ID No:85) or a functionally active derivative thereof, or a functionally active fragment thereof, or a homolog thereof, or a variant of "HYPOTH 35.7 KDA PROTEIN" encoded by a nucleic acid that hybridizes to the "HYPOTH 35.7 KDA PROTEIN" nucleic acid under low stringency conditions,
   (xxiv) "MATRIN 3" (SEQ ID No:86) or a functionally active derivative thereof, or a functionally active fragment thereof, or a homolog thereof, or a variant of "MATRIN 3" encoded by a nucleic acid that hybridizes to the "MATRIN 3" nucleic acid under low stringency conditions,
   (xxv) "NF45 PROTEIN" (SEQ ID No:87) or a functionally active derivative thereof, or a functionally active fragment thereof, or a homolog thereof, or a variant of "NF45 PROTEIN" encoded by a nucleic acid that hybridizes to the "NF45 PROTEIN" nucleic acid under low stringency conditions,
   (xxvi) "PCNA" (SEQ ID No:88) or a functionally active derivative thereof, or a functionally active fragment thereof, or a homolog thereof, or a variant of "PCNA" encoded by a nucleic acid that hybridizes to the "PCNA" nucleic acid under low stringency conditions,
   (xxvii) "54 KDA NUCLEAR RNA- AND DNA-BINDING PROTEIN" (SEQ ID No:89) or a functionally active derivative thereof, or a functionally active fragment thereof, or a homolog thereof, or a variant of "54 KDA NUCLEAR RNA- AND DNA-BINDING PROTEIN" encoded by a nucleic acid that hybridizes to the "54 KDA NUCLEAR RNA-AND DNA-BINDING PROTEIN" nucleic acid under low stringency conditions,
   (xxviii) "UBIQUITIN CARBOXYL-TERMINAL HYDROLASE 11" (SEQ ID No:90) or a functionally active derivative thereof, or a functionally active fragment thereof, or a homolog thereof, or a variant of "UBIQUITIN CARBOXYL-TERMINAL HYDROLASE 11" encoded by a nucleic acid that hybridizes to the "UBIQUITIN CARBOXYL-TERMINAL HYDROLASE 11" nucleic acid under low stringency conditions,
   (xxix) "CELL DIFFERENTIATION PROTEIN RCD1" (SEQ ID No:91) or a functionally active derivative thereof, or a functionally active fragment thereof, or a homolog thereof, or a variant of "CELL DIFFERENTIATION PROTEIN RCD1" encoded by a nucleic acid that hybridizes to the "CELL DIFFERENTIATION PROTEIN RCD1" nucleic acid under low stringency conditions,
   (xxx) "CHROMATIN ASSEMBLY FACTOR 1 SUBUNIT C" (SEQ ID No:92) or a functionally active derivative thereof, or a functionally active fragment thereof, or a homolog thereof, or a variant of "CHROMATIN ASSEMBLY FACTOR 1 SUBUNIT C" encoded by a nucleic acid that hybridizes to the "CHROMATIN ASSEMBLY FACTOR 1 SUBUNIT C" nucleic acid under low stringency conditions,
   (xxxi) "HISTONE ACETYLTRANSFERASE TYPE B SUBUNIT 2" (SEQ ID No:93) or a functionally active derivative thereof, or a functionally active fragment thereof, or a homolog thereof, or a variant of "HISTONE ACETYLTRANSFERASE TYPE B SUBUNIT 2" encoded by a nucleic acid that hybridizes to the "HISTONE ACETYLTRANSFERASE TYPE B SUBUNIT 2" nucleic acid under low stringency conditions,
   (xxxii) "HYPOTHETICAL PROTEIN KIAA0310" (SEQ ID No:94) or a functionally active derivative thereof, or a functionally active fragment thereof, or a homolog thereof, or a variant of "HYPOTHETICAL PROTEIN KIAA0310" encoded by a nucleic acid that hybridizes to the "HYPOTHETICAL PROTEIN KIAA0310" nucleic acid under low stringency conditions,
   (xxxiii) "DNA-DIRECTED RNA POLYMERASE II 33 KDA POLYPEPTIDE" (SEQ ID No:95) or a functionally active derivative thereof, or a functionally active fragment thereof, or a homolog thereof, or a variant of "DNA-DIRECTED RNA POLYMERASE II 33 KDA POLYPEPTIDE" encoded by a nucleic acid that hybridizes to the "DNA-DIRECTED RNA POLYMERASE II 33 KDA POLYPEPTIDE" nucleic acid under low stringency conditions,
   (xxxiv) "CALCIUM-DEPENDENT PROTEASE, SMALL SUBUNIT. CAPNS1" (SEQ ID No:96) or a functionally active derivative thereof, or a functionally active fragment thereof, or a homolog thereof, or a variant of "CALCIUM-DEPENDENT PROTEASE, SMALL SUBUNIT. CAPNS1" encoded by a nucleic acid that hybridizes to the "CALCIUM-DEPENDENT PROTEASE, SMALL SUBUNIT. CAPNS1" nucleic acid under low stringency conditions,
   (xxxv) "PCAF-ASSOCIATED FACTOR 400" (SEQ ID No:97) or a functionally active derivative thereof, or a functionally active fragment thereof, or a homolog thereof, or a variant of "PCAF-ASSOCIATED FACTOR 400" encoded by a nucleic acid that hybridizes to the "PCAF-ASSOCIATED FACTOR 400" nucleic acid under low stringency conditions,
   (xxxvi) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions,
   (xxxvii) "COMPLEMENT C1R-LIKE PROTEINASE" (SEQ ID No:98) or a functionally active derivative thereof, or a functionally active fragment thereof, or a homolog thereof, or a variant of "COMPLEMENT C1R-LIKE PROTEINASE" encoded by a nucleic acid that hybridizes to the "COMPLEMENT C1R-LIKE PROTEINASE" nucleic acid under low stringency conditions,
   (xxxviii) "MMS19 PROTEIN" (SEQ ID No:99) or a functionally active derivative thereof, or a homolog thereof, or a variant of "MMS19 PROTEIN" encoded by a nucleic acid that hybridizes to the "MMS19 PROTEIN" nucleic acid under low stringency conditions,
   (xxxix) "TRANSCRIPTION INTERMEDIARY FACTOR 1-β" (SEQ ID No:100) or a functionally active derivative thereof, or a homolog thereof, or a variant of "TRANSCRIPTION INTERMEDIARY FACTOR 1-β" encoded by a nucleic acid that hybridizes to the "TRANSCRIPTION INTERMEDIARY FACTOR 1-β" nucleic acid under low stringency conditions, and
   (xl) P52" (SEQ ID No:231) or a functionally active derivative thereof, or a functionally active fragment thereof, or a homolog thereof, or a variant of "p52 (NF-kappaB2)" encoded by a nucleic acid that hybridises to the "p52 (NF-kappaB2)" nucleic acid under low stringency conditions.
4. The protein complex according to No. 1 selected from complex (I) comprising all but 1 - 18 of the following proteins:
   (i) "Menin" (SEQ ID No:66) or a functionally active derivative thereof, or a functionally active fragment thereof, or a homolog thereof, or a variant of "Menin" encoded by a nucleic acid that hybridizes to the "Menin" nucleic acid under low stringency conditions,
   (ii) "SMRT" (SEQ ID No:67) or a functionally active derivative thereof, or a functionally active fragment thereof, or a homolog thereof, or a variant of "SMRT" encoded by a nucleic acid that hybridizes to the "SMRT" nucleic acid under low stringency conditions,
   (iii) "HDAC3" (SEQ ID No:68) or a functionally active derivative thereof, or a functionally active fragment thereof, or a homolog thereof, or a variant of "HDAC3" encoded by a nucleic acid that hybridizes to the "HDAC3" nucleic acid under low stringency conditions,
   (iv) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (v) "HDAC2" (SEQ ID No:69) or a functionally active derivative thereof, or a functionally active fragment thereof, or a homolog thereof, or a variant of "HDAC2" encoded by a nucleic acid that hybridizes to the "HDAC2" nucleic acid under low stringency conditions,
   (vi) "Cot/Tpl-2" (SEQ ID No:70) or a functionally active derivative thereof, or a functionally active fragment thereof, or a homolog thereof, or a variant of "Cot/Tpl-2" encoded by a nucleic acid that hybridizes to the "Cot/Tpl-2" nucleic acid under low stringency conditions,

   (i) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (viii) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,

   (i) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (x) "p50 (NF-κB1)" (SEQ ID No:73) or a functionally active derivative thereof, or a functionally active fragment thereof, or a homolog thereof, or a variant of "p50 (NF-κB1)" encoded by a nucleic acid that hybridizes to the "p50 (NF-κB1)" nucleic acid under low stringency conditions,
   (xi) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (xii) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions,
   (xiii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (xiv) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (xv) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
   (xvi) "IKK ε" (SEQ ID No:79) or a functionally active derivative thereof, or a functionally active fragment thereof, or a homolog thereof, or a variant of "IKK ε" encoded by a nucleic acid that hybridizes to the "IKK ε" nucleic acid under low stringency conditions,
   (xvii) "HDAC1" (SEQ ID No:80) or a functionally active derivative thereof, or a functionally active fragment thereof, or a homolog thereof, or a variant of "HDAC1" encoded by a nucleic acid that hybridizes to the "HDAC1" nucleic acid under low stringency conditions,
   (xviii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (xix) "ReIB" (SEQ ID No:81) or a functionally active derivative thereof, or a functionally active fragment thereof, or a homolog thereof, or a variant of "ReIB" encoded by a nucleic acid that hybridizes to the "ReIB" nucleic acid under low stringency conditions,
   (xx) "NAKAP 95" (SEQ ID No:82) or a functionally active derivative thereof, or a functionally active fragment thereof, or a homolog thereof, or a variant of "NAKAP 95" encoded by a nucleic acid that hybridizes to the "NAKAP 95" nucleic acid under low stringency conditions,

   (i) "IMPORTIN α-3 SUBUNIT" (SEQ ID No:83) or a functionally active derivative thereof, or a functionally active fragment thereof, or a homolog thereof, or a variant of "IMPORTIN α-3 SUBUNIT" encoded by a nucleic acid that hybridizes to the "IMPORTIN α-3 SUBUNIT" nucleic acid under low stringency conditions,
   (xxii) "UBIQUITIN--PROTEIN LIGASE EDD" (SEQ ID No:84) or a functionally active derivative thereof, or a functionally active fragment thereof, or a homolog thereof, or a variant of "UBIQUITIN--PROTEIN LIGASE EDD" encoded by a nucleic acid that hybridizes to the "UBIQUITIN--PROTEIN LIGASE EDD" nucleic acid under low stringency conditions,
   (xxiii) "HYPOTH 35.7 KDA PROTEIN" (SEQ ID No:85) or a functionally active derivative thereof, or a functionally active fragment thereof, or a homolog thereof, or a variant of "HYPOTH 35.7 KDA PROTEIN" encoded by a nucleic acid that hybridizes to the "HYPOTH 35.7 KDA PROTEIN" nucleic acid under low stringency conditions,
   (xxiv) "MATRIN 3" (SEQ ID No:86) or a functionally active derivative thereof, or a functionally active fragment thereof, or a homolog thereof, or a variant of "MATRIN 3" encoded by a nucleic acid that hybridizes to the "MATRIN 3" nucleic acid under low stringency conditions,
   (xxv) "NF45 PROTEIN" (SEQ ID No:87) or a functionally active derivative thereof, or a functionally active fragment thereof, or a homolog thereof, or a variant of "NF45 PROTEIN" encoded by a nucleic acid that hybridizes to the "NF45 PROTEIN" nucleic acid under low stringency conditions,
   (xxvi) "PCNA" (SEQ ID No:88) or a functionally active derivative thereof, or a functionally active fragment thereof, or a homolog thereof, or a variant of "PCNA" encoded by a nucleic acid that hybridizes to the "PCNA" nucleic acid under low stringency conditions,
   (xxvii) "54 KDA NUCLEAR RNA- AND DNA-BINDING PROTEIN" (SEQ ID No:89) or a functionally active derivative thereof, or a functionally active fragment thereof, or a homolog thereof, or a variant of "54 KDA NUCLEAR RNA- AND DNA-BINDING PROTEIN" encoded by a nucleic acid that hybridizes to the "54 KDA NUCLEAR RNA-AND DNA-BINDING PROTEIN" nucleic acid under low stringency conditions,
   (xxviii) "UBIQUITIN CARBOXYL-TERMINAL HYDROLASE 11" (SEQ ID No:90) or a functionally active derivative thereof, or a functionally active fragment thereof, or a homolog thereof, or a variant of "UBIQUITIN CARBOXYL-TERMINAL HYDROLASE 11" encoded by a nucleic acid that hybridizes to the "UBIQUITIN CARBOXYL-TERMINAL HYDROLASE 11" nucleic acid under low stringency conditions,
   (xxix) "CELL DIFFERENTIATION PROTEIN RCD1" (SEQ ID No:91) or a functionally active derivative thereof, or a functionally active fragment thereof, or a homolog thereof, or a variant of "CELL DIFFERENTIATION PROTEIN RCD1" encoded by a nucleic acid that hybridizes to the "CELL DIFFERENTIATION PROTEIN RCD1" nucleic acid under low stringency conditions,
   (xxx) "CHROMATIN ASSEMBLY FACTOR 1 SUBUNIT C" (SEQ ID No:92) or a functionally active derivative thereof, or a functionally active fragment thereof, or a homolog thereof, or a variant of "CHROMATIN ASSEMBLY FACTOR 1 SUBUNIT C" encoded by a nucleic acid that hybridizes to the "CHROMATIN ASSEMBLY FACTOR 1 SUBUNIT C" nucleic acid under low stringency conditions,
   (xxxi) "HISTONE ACETYLTRANSFERASE TYPE B SUBUNIT 2" (SEQ ID No:93) or a functionally active derivative thereof, or a functionally active fragment thereof, or a homolog thereof, or a variant of "HISTONE ACETYLTRANSFERASE TYPE B SUBUNIT 2" encoded by a nucleic acid that hybridizes to the "HISTONE ACETYLTRANSFERASE TYPE B SUBUNIT 2" nucleic acid under low stringency conditions,
   (xxxii) "HYPOTHETICAL PROTEIN KIAA0310" (SEQ ID No:94) or a functionally active derivative thereof, or a functionally active fragment thereof, or a homolog thereof, or a variant of "HYPOTHETICAL PROTEIN KIAA0310" encoded by a nucleic acid that hybridizes to the "HYPOTHETICAL PROTEIN KIAA0310" nucleic acid under low stringency conditions,
   (xxxiii) "DNA-DIRECTED RNA POLYMERASE II 33 KDA POLYPEPTIDE" (SEQ ID No:95) or a functionally active derivative thereof, or a functionally active fragment thereof, or a homolog thereof, or a variant of "DNA-DIRECTED RNA POLYMERASE II 33 KDA POLYPEPTIDE" encoded by a nucleic acid that hybridizes to the "DNA-DIRECTED RNA POLYMERASE II 33 KDA POLYPEPTIDE" nucleic acid under low stringency conditions,
   (xxxiv) "CALCIUM-DEPENDENT PROTEASE, SMALL SUBUNIT. CAPNS1" (SEQ ID No:96) or a functionally active derivative thereof, or a functionally active fragment thereof, or a homolog thereof, or a variant of "CALCIUM-DEPENDENT PROTEASE, SMALL SUBUNIT. CAPNS1" encoded by a nucleic acid that hybridizes to the "CALCIUM-DEPENDENT PROTEASE, SMALL SUBUNIT. CAPNS1" nucleic acid under low stringency conditions,
   (xxxv) "PCAF-ASSOCIATED FACTOR 400" (SEQ ID No:97) or a functionally active derivative thereof, or a functionally active fragment thereof, or a homolog thereof, or a variant of "PCAF-ASSOCIATED FACTOR 400" encoded by a nucleic acid that hybridizes to the "PCAF-ASSOCIATED FACTOR 400" nucleic acid under low stringency conditions,
   (xxxvi) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions,
   (xxxvii) "COMPLEMENT C1R-LiKE PROTEINASE" (SEQ ID No:98) or a functionally active derivative thereof, or a functionally active fragment thereof, or a homolog thereof, or a variant of "COMPLEMENT C1R-LlKE PROTEINASE" encoded by a nucleic acid that hybridizes to the "COMPLEMENT C1R-LIKE PROTEINASE" nucleic acid under low stringency conditions,
   (xxxviii) "MMS19 PROTEIN" (SEQ ID No:99) or a functionally active derivative thereof, or a functionally active fragment thereof, or a homolog thereof, or a variant of "MMS19 PROTEIN" encoded by a nucleic acid that hybridizes to the "MMS19 PROTEIN" nucleic acid under low stringency conditions,
   (xxxix) "TRANSCRIPTION INTERMEDIARY FACTOR 1-β" (SEQ ID No:100) or a functionally active derivative thereof, or a functionally active fragment thereof, or a homolog thereof, or a variant of "TRANSCRIPTION INTERMEDIARY FACTOR 1-β" encoded by a nucleic acid that hybridizes to the "TRANSCRIPTION INTERMEDIARY FACTOR 1-β" nucleic acid under low stringency conditions, and
   (xl) P52" (SEQ ID No:231) or a functionally active derivative thereof, or a functionally active fragment thereof, or a homolog thereof, or a variant of "p52 (NF-kappaB2)" encoded by a nucleic acid that hybridises to the "p52 (NF-kappaB2)" nucleic acid under low stringency conditions;
   and a protein complex selected from complex (I) comprising all but 1 - 18 of the following proteins:
   (i) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (ii) "HDAC2" (SEQ ID No:69) or a functionally active derivative thereof, or a functionally active fragment thereof, or a homolog thereof, or a variant of "HDAC2" encoded by a nucleic acid that hybridizes to the "HDAC2" nucleic acid under low stringency conditions,
   (iii) "Cot/Tpl-2" (SEQ ID No:70) or a functionally active derivative thereof, or a functionally active fragment thereof, or a homolog thereof, or a variant of "Cot/Tpl-2" encoded by a nucleic acid that hybridizes to the "Cot/Tpl-2" nucleic acid under low stringency conditions,
   (iv) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (v) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,

   (i) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (vii) "p50 (NF-κB1)" (SEQ ID No:73) or a functionally active derivative thereof, or a functionally active fragment thereof, or a homolog thereof, or a variant of "p50 (NF-κB1)" encoded by a nucleic acid that hybridizes to the "p50 (NF-κB1)" nucleic acid under low stringency conditions,
   (viii) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (ix) "RelA (p65)" (SEQ ID No:75) or a functionally active derivative thereof, or a functionally active fragment thereof, or a homolog thereof, or a variant of "RelA (p65)" encoded by a nucleic acid that hybridizes to the "RelA (p65)" nucleic acid under low stringency conditions,
   (x) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (xi) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (xii) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
   (xiii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (xiv) "ReIB" (SEQ ID No:81) or a functionally active derivative thereof, or a functionally active fragment thereof, or a homolog thereof, or a variant of "ReIB" encoded by a nucleic acid that hybridizes to the "ReIB" nucleic acid under low stringency conditions,
   (xv) "NAKAP 95" (SEQ ID No:82) or a functionally active derivative thereof, or a functionally active fragment thereof, or a homolog thereof, or a variant of "NAKAP 95" encoded by a nucleic acid that hybridizes to the "NAKAP 95" nucleic acid under low stringency conditions,

   (i) "IMPORTIN α-3 SUBUNIT' (SEQ ID No:83) or a functionally active derivative thereof, or a functionally active fragment thereof, or a homolog thereof, or a variant of "IMPORTIN α-3 SUBUNIT" encoded by a nucleic acid that hybridizes to the "IMPORTIN α-3 SUBUNIT" nucleic acid under low stringency conditions,
   (xvii) "UBIQUITIN--PROTEIN LIGASE EDD" (SEQ ID No:84) or a functionally active derivative thereof, or a functionally active fragment thereof, or a homolog thereof, or a variant of "UBIQUITIN--PROTEIN LIGASE EDD" encoded by a nucleic acid that hybridizes to the "UBIQUITIN--PROTEIN LIGASE EDD" nucleic acid under low stringency conditions,
   (xviii) "HYPOTH 35.7 KDA PROTEIN" (SEQ ID No:85) or a functionally active derivative thereof, or a functionally active fragment thereof, or a homolog thereof, or a variant of "HYPOTH 35.7 KDA PROTEIN" encoded by a nucleic acid that hybridizes to the "HYPOTH 35.7 KDA PROTEIN" nucleic acid under low stringency conditions,
   (xix) "MATRIN 3" (SEQ ID No:86) or a functionally active derivative thereof, or a functionally active fragment thereof, or a homolog thereof, or a variant of "MATRIN 3" encoded by a nucleic acid that hybridizes to the "MATRIN 3" nucleic acid under low stringency conditions,
   (xx) "NF45 PROTEIN" (SEQ ID No:87) or a functionally active derivative thereof, or a functionally active fragment thereof, or a homolog thereof, or a variant of "NF45 PROTEIN" encoded by a nucleic acid that hybridizes to the "NF45 PROTEIN" nucleic acid under low stringency conditions,
   (xxi) "PCNA" (SEQ ID No:88) or a functionally active derivative thereof, or a functionally active fragment thereof, or a homolog thereof, or a variant of "PCNA" encoded by a nucleic acid that hybridizes to the "PCNA" nucleic acid under low stringency conditions,
   (xxii) "54 KDA NUCLEAR RNA- AND DNA-BINDING PROTEIN" (SEQ ID No:89) or a functionally active derivative thereof, or a functionally active fragment thereof, or a homolog thereof, or a variant of "54 KDA NUCLEAR RNA- AND DNA-BINDING PROTEIN" encoded by a nucleic acid that hybridizes to the "54 KDA NUCLEAR RNA-AND DNA-BINDING PROTEIN" nucleic acid under low stringency conditions,
   (xxiii) "UBIQUITIN CARBOXYL-TERMINAL HYDROLASE 11" (SEQ ID No:90) or a functionally active derivative thereof, or a functionally active fragment thereof, or a homolog thereof, or a variant of "UBIQUITIN CARBOXYL-TERMINAL HYDROLASE 11" encoded by a nucleic acid that hybridizes to the "UBIQUITIN CARBOXYL-TERMINAL HYDROLASE 11" nucleic acid under low stringency conditions,
   (xxiv) "CELL DIFFERENTIATION PROTEIN RCD1" (SEQ ID No:91) or a functionally active derivative thereof, or a functionally active fragment thereof, or a homolog thereof, or a variant of "CELL DIFFERENTIATION PROTEIN RCD1" encoded by a nucleic acid that hybridizes to the "CELL DIFFERENTIATION PROTEIN RCD1" nucleic acid under low stringency conditions,
   (xxv) "CHROMATIN ASSEMBLY FACTOR 1 SUBUNIT C" (SEQ ID No:92) or a functionally active derivative thereof, or a functionally active fragment thereof, or a homolog thereof, or a variant of "CHROMATIN ASSEMBLY FACTOR 1 SUBUNIT C" encoded by a nucleic acid that hybridizes to the "CHROMATIN ASSEMBLY FACTOR 1 SUBUNIT C" nucleic acid under low stringency conditions,
   (xxvi) "HISTONE ACETYLTRANSFERASE TYPE B SUBUNIT 2" (SEQ ID No:93) or a functionally active derivative thereof, or a functionally active fragment thereof, or a homolog thereof, or a variant of "HISTONE ACETYLTRANSFERASE TYPE B SUBUNIT 2" encoded by a nucleic acid that hybridizes to the "HISTONE ACETYLTRANSFERASE TYPE B SUBUNIT 2" nucleic acid under low stringency conditions,
   (xxvii) "HYPOTHETICAL PROTEIN KIAA0310" (SEQ ID No:94) or a functionally active derivative thereof, or a functionally active fragment thereof, or a homolog thereof, or a variant of "HYPOTHETICAL PROTEIN KIAA0310" encoded by a nucleic acid that hybridizes to the "HYPOTHETICAL PROTEIN KIAA0310" nucleic acid under low stringency conditions,
   (xxviii) "DNA-DIRECTED RNA POLYMERASE II 33 KDA POLYPEPTIDE" (SEQ ID No:95) or a functionally active derivative thereof, or a functionally active fragment thereof, or a homolog thereof, or a variant of "DNA-DIRECTED RNA POLYMERASE II 33 KDA POLYPEPTIDE" encoded by a nucleic acid that hybridizes to the "DNA-DIRECTED RNA POLYMERASE II 33 KDA POLYPEPTIDE" nucleic acid under low stringency conditions,
   (xxix) "CALCIUM-DEPENDENT PROTEASE, SMALL SUBUNIT. CAPNS1" (SEQ ID No:96) or a functionally active derivative thereof, or a functionally active fragment thereof, or a homolog thereof, or a variant of "CALCIUM-DEPENDENT PROTEASE, SMALL SUBUNIT. CAPNS1" encoded by a nucleic acid that hybridizes to the "CALCIUM-DEPENDENT PROTEASE, SMALL SUBUNIT. CAPNS1" nucleic acid under low stringency conditions,
   (xxx) "PCAF-ASSOCIATED FACTOR 400" (SEQ ID No:97) or a functionally active derivative thereof, or a functionally active fragment thereof, or a homolog thereof, or a variant of "PCAF-ASSOCIATED FACTOR 400" encoded by a nucleic acid that hybridizes to the "PCAF-ASSOCIATED FACTOR 400" nucleic acid under low stringency conditions,
   (xxxi) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions,
   (xxxii) "COMPLEMENT C1R-LIKE PROTEINASE" (SEQ ID No:98) or a functionally active derivative thereof, or a functionally active fragment thereof, or a homolog thereof, or a variant of "COMPLEMENT C1R-LIKE PROTEINASE" encoded by a nucleic acid that hybridizes to the "COMPLEMENT C1R-LIKE PROTEINASE" nucleic acid under low stringency conditions,
   (xxxiii) "MMS19 PROTEIN" (SEQ ID No:99) or a functionally active derivative thereof, or a functionally active fragment thereof, or a homolog thereof, or a variant of "MMS19 PROTEIN" encoded by a nucleic acid that hybridizes to the "MMS19 PROTEIN" nucleic acid under low stringency conditions, and
   (xxxiv)"TRANSCRIPTION INTERMEDIARY FACTOR 1-β" (SEQ ID No:100) or a functionally active derivative thereof, or a functionally active fragment thereof, or a homolog thereof, or a variant of ''TRANSCRIPTION INTERMEDIARY FACTOR 1-β" encoded by a nucleic acid that hybridizes to the "TRANSCRIPTION INTERMEDIARY FACTOR 1-β" nucleic acid under low stringency conditions.
5. The complex of any of No. 1 - 4 comprising a functionally active derivative of said first protein and/or a functionally active derivative of said second protein, wherein the functionally active derivative is a fusion protein comprising said first protein or said second protein fused to an amino acid sequence different from the first protein or second protein, respectively.
6. The complex of No. 5 wherein the functionally active derivative is a fusion protein comprising said first protein or said second protein fused to an affinity tag or label.
7. The complex of any of No. 1 - 4 comprising a fragment of said first protein and/or a fragment of said second protein, which fragment binds to another protein component of said complex.
8. The complex of any of No. 1 -7 that is involved in the DNA binding activity.
9. A process for preparing complex of any of No. 1 - 8 and optionally the components thereof comprising the following steps:
   the expressing a protein (bait) of the complex, preferably a tagged protein, in a target cell, isolating the protein complex which is attached to the bait protein, and optionally disassociating the protein complex and isolating the individual complex members.
10. The process according to No. 9 wherein the tagged protein comprises two different tags which allow two separate affinity purification steps.
11. The process according to any of No.9 - 10 wherein the two tags are separated by a cleavage site for a protease.
12. Component of the NF-κB protein complexobtainable by a process according to any of No. 9 to 11.
13. Protein of the NF-κB protein complexselected from
   (i) "HYPOTH 35.7 KDA PROTEIN" (SEQ ID No:85) or a functionally active derivative thereof, or a functionally active fragment thereof, or a homolog thereof, or a variant of "HYPOTH 35.7 KDA PROTEIN" encoded by a nucleic acid that hybridizes to the "HYPOTH 35.7 KDA PROTEIN" nucleic acid under low stringency conditions, and
   (ii) "HYPOTHETICAL PROTEIN KIAA0310" (SEQ ID No:94) or a functionally active derivative thereof, or a functionally active fragment thereof, or a homolog thereof, or a variant of "HYPOTHETICAL PROTEIN KIAA0310" encoded by a nucleic acid that hybridizes to the "HYPOTHETICAL PROTEIN KIAA0310" nucleic acid under low stringency conditions,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
14. Nucleic acid encoding a protein according to No. 13.
15. Construct, preferably a vector construct, comprising (a) a nucleic acid according to No. 14 and at least one further nucleic acid which is normally not associated with said nucleic acid,or(b) at least two separate nucleic acid sequences each encoding a different protein, or a functionally active fragment or a functionally active derivative thereof at least one of said proteins, or functionally active fragments or functionally active derivative thereof being selected from the first group of proteins according to No. 1(a) and at least one of said proteins, or functionally active fragments or functionally active derivative thereof being selected from the second group of proteins according to No. 1(b).
16. Host cell, containing a vector comprising at least one of the nucleic acid of No. 14 and/or a construct of No. 15 or containing several vectors each comprising at least the nucleic acid sequence encoding at least one of the proteins, or functionally active fragments or functionally active derivatives thereof selected from the first group of proteins according to No. 1(a) and the proteins, or functionally active fragments or functionally active derivatives thereof selected from the second group of proteins according to No. 1(b).
17. An antibody or a fragment of said antibody containing the binding domain thereof, selected from an antibody or fragment thereof, which binds the complex of any of No. 1-8 and which does not bind any of the proteins of said complex when uncomplexed and an antibody or a fragment of said antibody which binds to any of the proteins according to No. 13.
18. A kit comprising in one or more container the complex of any of No. 1-8 and/or the proteins of No. 13 optionally together with an antibody according to No. 17 and/or further components such as reagents and working instructions.
19. The kit according to No.18 for processing a substrate of said complex.
20. The kit according to No. 18 for the diagnosis or prognosis of a disease or a disease risk, preferentially for a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
21. Array, in which at least a complex according to any of No. 1 - 8 and/or at least one protein according to No. 14 and/or at least one antibody according to No. 17 is attached to a solid carrier.
22. A process for processing a physiological substrate of the complex comprising the step of bringing into contact a complex to any of No. 1 - 8 with said substrate, such that said substrate is processed.
23. A pharmaceutical composition comprising the protein complex of any of No. 1 to 8 and/or any of the following the proteins:
   (i) "HYPOTH 35.7 KDA PROTEIN" (SEQ ID No:85) or a functionally active derivative thereof,or a functionally active fragement thereof, or a homolog thereof, or a variant of "HYPOTH 35.7 KDA PROTEIN" that hybridizes to the "HYPOTH 35.7 KDA PROTEIN" nucleic acid under low stringency conditions, and
   (ii) "HYPOTHETICAL PROTEIN KIAA0310" (SEQ ID No:94) or a functionally active derivative thereof, or a functionally active fragment thereof, or a homolog thereof, or a variant of "HYPOTHETICAL PROTEIN KIAA0310" encoded by a nucleic acid that hybridizes to the "HYPOTHETICAL PROTEIN KIAA0310" nucleic acid under low stringency conditions,
   and a pharmaceutical acceptable carrier.
24. The pharmaceutical composition according to No. 23 for the treatment of diseases and disorders such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
25. A method for screening for a molecule that binds to the complex of anyone of No. 1 - 8 and/or any of the following proteins:
   (i) "HYPOTH 35.7 KDA PROTEIN" (SEQ ID No:85) or a functionally active derivative thereof,or a functionally active fragement thereof, or a homolog thereof, or a variant of "HYPOTH 35.7 KDA PROTEIN" that hybridizes to the "HYPOTH 35.7 KDA PROTEIN" nucleic acid under low stringency conditions, and
   (ii) "HYPOTHETICAL PROTEIN KIAA0310" (SEQ ID No:94) or a functionally active derivative thereof, or a functionally active fragment thereof, or a homolog thereof, or a variant of "HYPOTHETICAL PROTEIN KIAA0310" encoded by a nucleic acid that hybridizes to the "HYPOTHETICAL PROTEIN KIAA0310" nucleic acid under low stringency conditions, comprising the steps of
      (a) exposing said complex, or a cell or organism containing same to one or more candidate molecules; and
      (b) determinig whether said candidate molecule is bound to the complex or protein.
26. A method for screening for a molecule that modulates directly or indirectly the function, activity, composition or formation of the complex of any one of No. 1 - 8 comprising the steps of (a) exposing said complex, or a cell or organism containing NF-κB protein complexto one or more candidate molecules; and
   (b) determining the amount of activity of protein components of, and/or intracellular localization of, said complex and/or the transcription level of a gene dependent on the complex and/or the abundance and/or activity of a protein or protein complex dependent on the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependent on the complex and/or the abundance and/or activity of a protein or protein complex dependent on the function of the complex and/or product of a gene dependent on the complex in the absence of said candidate molecules indicates that the molecule modulates function, activity or composition of said complex.
27. The method of No. 26, wherein the amount of said complex is determined.
28. The method of No. 26, wherein the activity of said complex is determined.
29. The method of No. 28, wherein said determining step comprises isolating from the cell or organism said complex to produce said isolated complex and contacting said isolated complex in the presence or absence of a candidate molecule with a substrate of said complex and determining the processing of said substrate is modified in the presence of said candidate molecule.
30. The method of No. 26, wherein the amount of the individual protein components of said complex are determined.
31. The method of No. 30, determining step comprises determining whether
   (i) "Menin" (SEQ ID No:66) or a functionally active derivative thereof, or a functionally active fragment thereof, or a homolog thereof, or a variant of "Menin" encoded by a nucleic acid that hybridizes to the "Menin" nucleic acid under low stringency conditions, and/or
   (ii) "SMRT" (SEQ ID No:67) or a functionally active derivative thereof, or a functionally active fragment thereof, or a homolog thereof, or a variant of "SMRT" encoded by a nucleic acid that hybridizes to the "SMRT" nucleic acid under low stringency conditions, and/or
   (iii) "HDAC3" (SEQ ID No:68) or a functionally active derivative thereof, or a functionally active fragment thereof, or a homolog thereof, or a variant of "HDAC3" encoded by a nucleic acid that hybridizes to the "HDAC3" nucleic acid under low stringency conditions, and/or

   (i) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions, and/or
   (v) "HDAC2" (SEQ ID No:69) or a functionally active derivative thereof, or a functionally active fragment thereof, or a homolog thereof, or a variant of "HDAC2" encoded by a nucleic acid that hybridizes to the "HDAC2" nucleic acid under low stringency conditions, and/or
   (vi) "Cot/Tpl-2" (SEQ ID No:70) or a functionally active derivative thereof, or a functionally active fragment thereof, or a homolog thereof, or a variant of "Cot/Tpl-2" encoded by a nucleic acid that hybridizes to the "Cot/Tpl-2" nucleic acid under low stringency conditions, and/or
   (vii) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions, and/or
   (viii) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions, and/or

   (i) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions, and/or
   (x) "p50 (NF-κB1)" (SEQ ID No:73) or a functionally active derivative thereof, or a functionally active fragment thereof, or a homolog thereof, or a variant of "p50 (NF-κB1)" encoded by a nucleic acid that hybridizes to the "p50 (NF-κB1)" nucleic acid under low stringency conditions, and/or
   (xi) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions, and/or
   (xii) "ReIA (p65)" (SEQ ID No:75) or a functionally active derivative thereof, or a functionally active fragment thereof, or a homolog thereof, or a variant of ''RelA (p65)" encoded by a nucleic acid that hybridizes to the "ReIA (p65)" nucleic acid under low stringency conditions, and/or
   (xiii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions, and/or
   (xiv) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions, and/or
   (xv) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions, and/or
   (xvi) "IKK ε" (SEQ ID No:79) or a functionally active derivative thereof, or a functionally active fragment thereof, or a homolog thereof, or a variant of "IKK ε" encoded by a nucleic acid that hybridizes to the "IKK ε" nucleic acid under low stringency conditions, and/or
   (xvii) "HDAC1" (SEQ ID No:80) or a functionally active derivative thereof, or a functionally active fragment thereof, or a homolog thereof, or a variant of "HDAC1" encoded by a nucleic acid that hybridizes to the "HDAC1" nucleic acid under low stringency conditions, and/or
   (xviii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions, and/or
   (xix) "ReIB" (SEQ ID No:81) or a functionally active derivative thereof, or a functionally active fragment thereof, or a homolog thereof, or a variant of "ReIB" encoded by a nucleic acid that hybridizes to the "ReIB" nucleic acid under low stringency conditions, and/or
   (xx) "NAKAP 95" (SEQ ID No:82) or a functionally active derivative thereof, or a functionally active fragment thereof, or a homolog thereof, or a variant of "NAKAP 95" encoded by a nucleic acid that hybridizes to the "NAKAP 95" nucleic acid under low stringency conditions, and/or

   (i) "IMPORTIN α-3 SUBUNIT" (SEQ ID No:83) or a functionally active derivative thereof, or a functionally active fragment thereof, or a homolog thereof, or a variant of "IMPORTIN α-3 SUBUNIT" encoded by a nucleic acid that hybridizes to the "IMPORTIN α-3 SUBUNIT" nucleic acid under low stringency conditions, and/or
   (xxii) "UBIQUITIN-PROTEIN LIGASE EDD" (SEQ ID No:84) or a functionally active derivative thereof, or a functionally active fragment thereof, or a homolog thereof, or a variant of "UBIQUITIN--PROTEIN LIGASE EDD" encoded by a nucleic acid that hybridizes to the "UBIQUITIN--PROTEIN LIGASE EDD" nucleic acid under low stringency conditions, and/or
   (xxiii) "HYPOTH 35.7 KDA PROTEIN" (SEQ ID No:85) or a functionally active derivative thereof, or a functionally active fragment thereof, or a homolog thereof, or a variant of "HYPOTH 35.7 KDA PROTEIN" encoded by a nucleic acid that hybridizes to the "HYPOTH 35.7 KDA PROTEIN" nucleic acid under low stringency conditions, and/or
   (xxiv) "MATRIN 3" (SEQ ID No:86) or a functionally active derivative thereof, or a functionally active fragment thereof, or a homolog thereof, or a variant of "MATRIN 3" encoded by a nucleic acid that hybridizes to the "MATRIN 3" nucleic acid under low stringency conditions, and/or
   (xxv) "NF45 PROTEIN" (SEQ ID No:87) or a functionally active derivative thereof, or a functionally active fragment thereof, or a homolog thereof, or a variant of "NF45 PROTEIN" encoded by a nucleic acid that hybridizes to the "NF45 PROTEIN" nucleic acid under low stringency conditions, and/or
   (xxvi) "PCNA" (SEQ ID No:88) or a functionally active derivative thereof, or a functionally active fragment thereof, or a homolog thereof, or a variant of "PCNA" encoded by a nucleic acid that hybridizes to the "PCNA" nucleic acid under low stringency conditions, and/or
   (xxvii) "54 KDA NUCLEAR RNA- AND DNA-BINDING PROTEIN" (SEQ ID No:89) or a functionally active derivative thereof, or a functionally active fragment thereof, or a homolog thereof, or a variant of "54 KDA NUCLEAR RNA- AND DNA-BINDING PROTEIN" encoded by a nucleic acid that hybridizes to the "54 KDA NUCLEAR RNA-AND DNA-BINDING PROTEIN" nucleic acid under low stringency conditions, and/or
   (xxviii) "UBIQUITIN CARBOXYL-TERMINAL HYDROLASE 11" (SEQ ID No:90) or a functionally active derivative thereof, or a functionally active fragment thereof, or a homolog thereof, or a variant of "UBIQUITIN CARBOXYL-TERMINAL HYDROLASE 11" encoded by a nucleic acid that hybridizes to the "UBIQUITIN CARBOXYL-TERMINAL HYDROLASE 11" nucleic acid under low stringency conditions, and/or
   (xxix) "CELL DIFFERENTIATION PROTEIN RCD1" (SEQ ID No:91) or a functionally active derivative thereof, or a functionally active fragment thereof, or a homolog thereof, or a variant of "CELL DIFFERENTIATION PROTEIN RCD1" encoded by a nucleic acid that hybridizes to the "CELL DIFFERENTIATION PROTEIN RCD1" nucleic acid under low stringency conditions, and/or
   (xxx) "CHROMATIN ASSEMBLY FACTOR 1 SUBUNIT C" (SEQ ID No:92) or a functionally active derivative thereof, or a functionally active fragment thereof, or a homolog thereof, or a variant of "CHROMATIN ASSEMBLY FACTOR 1 SUBUNIT C" encoded by a nucleic acid that hybridizes to the "CHROMATIN ASSEMBLY FACTOR 1 SUBUNIT C" nucleic acid under low stringency conditions, and/or
   (xxxi) "HISTONE ACETYLTRANSFERASE TYPE B SUBUNIT 2" (SEQ ID No:93) or a functionally active derivative thereof, or a functionally active fragment thereof, or a homolog thereof, or a variant of "HISTONE ACETYLTRANSFERASE TYPE B SUBUNIT 2" encoded by a nucleic acid that hybridizes to the "HISTONE ACETYLTRANSFERASE TYPE B SUBUNIT 2" nucleic acid under low stringency conditions, and/or
   (xxxii) "HYPOTHETICAL PROTEIN KIAA0310" (SEQ ID No:94) or a functionally active derivative thereof, or a functionally active fragment thereof, or a homolog thereof, or a variant of "HYPOTHETICAL PROTEIN KIAA0310" encoded by a nucleic acid that hybridizes to the "HYPOTHETICAL PROTEIN KIAA0310" nucleic acid under low stringency conditions, and/or
   (xxxiii) "DNA-DIRECTED RNA POLYMERASE II 33 KDA POLYPEPTIDE" (SEQ ID No:95) or a functionally active derivative thereof, or a functionally active fragment thereof, or a homolog thereof, or a variant of "DNA-DIRECTED RNA POLYMERASE II 33 KDA POLYPEPTIDE" encoded by a nucleic acid that hybridizes to the "DNA-DIRECTED RNA POLYMERASE II 33 KDA POLYPEPTIDE" nucleic acid under low stringency conditions, and/or
   (xxxiv) "CALCIUM-DEPENDENT PROTEASE, SMALL SUBUNIT. CAPNS1" (SEQ ID No:96) or a functionally active derivative thereof, or a functionally active fragment thereof, or a homolog thereof, or a variant of "CALCIUM-DEPENDENT PROTEASE, SMALL SUBUNIT. CAPNS1" encoded by a nucleic acid that hybridizes to the "CALCIUM-DEPENDENT PROTEASE, SMALL SUBUNIT. CAPNS1" nucleic acid under low stringency conditions, and/or
   (xxxv) "PCAF-ASSOCIATED FACTOR 400" (SEQ ID No:97) or a functionally active derivative thereof, or a functionally active fragment thereof, or a homolog thereof, or a variant of "PCAF-ASSOCIATED FACTOR 400" encoded by a nucleic acid that hybridizes to the "PCAF-ASSOCIATED FACTOR 400 nucleic acid under low stringency conditions, and/or
   (xxxvi) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions, and/or
   (xxxvii) "COMPLEMENT C1R-LIKE PROTEINASE" (SEQ ID No:98) or a functionally active derivative thereof, or a functionally active fragment thereof, or a homolog thereof, or a variant of "COMPLEMENT C1R-LIKE PROTEINASE" encoded by a nucleic acid that hybridizes to the "COMPLEMENT C1R-LIKE PROTEINASE" nucleic acid under low stringency conditions, and/or
   (xxxviii) "MMS19 PROTEIN" (SEQ ID No:99) or a functionally active derivative thereof, or a functionally active fragment thereof, or a homolog thereof, or a variant of "MMS19 PROTEIN" encoded by a nucleic acid that hybridizes to the "MMS19 PROTEIN" nucleic acid under low stringency conditions, and/or
   (xxxix) "TRANSCRIPTION INTERMEDIARY FACTOR 1-β" (SEQ ID No:100) or a functionally active derivative thereof, or a functionally active fragment thereof, or a homolog thereof, or a variant of "TRANSCRIPTION INTERMEDIARY FACTOR 1-β" encoded by a nucleic acid that hybridizes to the "TRANSCRIPTION INTERMEDIARY FACTOR 1-β" nucleic acid under low stringency conditions, and/or
   (xl) P52" (SEQ ID No:231) or a functionally active derivative thereof, or a functionally active fragment thereof, or a homolog thereof, or a variant of "p52 (NF-kappaB2)" encoded by a nucleic acid that hybridises to the "p52 (NF-kappaB2)" nucleic acid under low stringency conditions. is present in the complex.
32. The method of any of No. 26 to 31, wherein said method is a method of screening for a drug for treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
33. Use of a molecule that modulates the amount of, activity of, or the protein components of the complex of any one of No. 1 - 8 for the manufacture of a medicament for the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
34. A method for the production of a pharmaceutical composition comprising carrying out the method of any of No. 1 - 8 to identify a molecule that modulates the function, activity, composition or formation of said complex, and further comprising mixing the identified molecule with a pharmaceutically acceptable carrier.
35. A method for diagnosing or screening for the presence of a disease or disorder or a predisposition for developing a disease or disorder in a subject, which disease or disorder is characterized by an aberrant amount of, activity of, or component composition of, or intracellular localization of the complex of any one of the No. 1 - 8, comprising determining the amount of, activity of, protein components of, and/or intracellular localization of, said complex and/or the transcription level of a gene dependent on the complex and/or the abundance and/or activity of a protein or protein complex dependent on the function of the complex and/or product of a gene dependent on the complex in a comparative sample derived from a subject, wherein a difference in said amount, activity, or protein components of, said complex in an analogous sample from a subject not having the disease or disorder or predisposition indicates the presence in the subject of the disease or disorder or predisposition in the subject.
36. The method of No. 35, wherein the amount of said complex is determined.
37. The method of No. 35, wherein the activity of said complex is determined.
38. The method of No. 37, wherein said determining step comprises isolating from the subject said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
39. The method of No. 35, wherein the amount of the individual protein components of said complex are determined.
40. The method of No. 39, wherein said determining step comprises determining whether
   (i) "Menin" (SEQ ID No:66) or a functionally active derivative thereof, or a functionally active fragment thereof, or a homolog thereof, or a variant of "Menin" encoded by a nucleic acid that hybridizes to the "Menin" nucleic acid under low stringency conditions, and/or
   (ii) "SMRT" (SEQ ID No:67) or a functionally active derivative thereof, or a functionally active fragment thereof, or a homolog thereof, or a variant of "SMRT" encoded by a nucleic acid that hybridizes to the "SMRT" nucleic acid under low stringency conditions, and/or
   (iii) "HDAC3" (SEQ ID No:68) or a functionally active derivative thereof, or a functionally active fragment thereof, or a homolog thereof, or a variant of "HDAC3" encoded by a nucleic acid that hybridizes to the "HDAC3" nucleic acid under low stringency conditions, and/or
   (iv) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions, and/or
   (v) "HDAC2" (SEQ ID No:69) or a functionally active derivative thereof, or a functionally active fragment thereof, or a homolog thereof, or a variant of "HDAC2" encoded by a nucleic acid that hybridizes to the "HDAC2" nucleic acid under low stringency conditions, and/or
   (vi) "Cot/Tpl-2" (SEQ ID No:70) or a functionally active derivative thereof, or a functionally active fragment thereof, or a homolog thereof, or a variant of "Cot/Tpl-2" encoded by a nucleic acid that hybridizes to the "Cot/Tpl-2" nucleic acid under low stringency conditions, and/or

   (i) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions, and/or
   (viii) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions, and/or

   (i) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions, and/or
   (x) "p50 (NF-κB1)" (SEQ ID No:73) or a functionally active derivative thereof, or a functionally active fragment thereof, or a homolog thereof, or a variant of "p50 (NF-κB1)" encoded by a nucleic acid that hybridizes to the "p50 (NF-κB1)" nucleic acid under low stringency conditions, and/or
   (xi) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions, and/or
   (xii) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions, and/or
   (xiii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions, and/or
   (xiv) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions, and/or
   (xv) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions, and/or
   (xvi) "IKK ε" (SEQ ID No:79) or a functionally active derivative thereof, or a functionally active fragment thereof, or a homolog thereof, or a variant of "IKK ε" encoded by a nucleic acid that hybridizes to the "IKK ε" nucleic acid under low stringency conditions, and/or
   (xvii) "HDAC1" (SEQ ID No:80) or a functionally active derivative thereof, or a functionally active fragment thereof, or a homolog thereof, or a variant of "HDAC1" encoded by a nucleic acid that hybridizes to the "HDAC1" nucleic acid under low stringency conditions, and/or
   (xviii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions, and/or
   (xix) "RelB" (SEQ ID No:81) or a functionally active derivative thereof, or a functionally active fragment thereof, or a homolog thereof, or a variant of "RelB" encoded by a nucleic acid that hybridizes to the "RelB" nucleic acid under low stringency conditions, and/or
   (xx) "NAKAP 95" (SEQ ID No:82) or a functionally active derivative thereof, or a functionally active fragment thereof, or a homolog thereof, or a variant of "NAKAP 95" encoded by a nucleic acid that hybridizes to the "NAKAP 95" nucleic acid under low stringency conditions, and/or

   (i) "IMPORTIN α-3 SUBUNIT" (SEQ ID No:83) or a functionally active derivative thereof, or a functionally active fragment thereof, or a homolog thereof, or a variant of "IMPORTIN α-3 SUBUNIT" encoded by a nucleic acid that hybridizes to the "IMPORTIN α-3 SUBUNIT" nucleic acid under low stringency conditions, and/or
   (xxii) "UBIQUITIN--PROTEIN LIGASE EDD" (SEQ ID No:84) or a functionally active derivative thereof, or a functionally active fragment thereof, or a homolog thereof, or a variant of "UBIQUITIN--PROTEIN LIGASE EDD" encoded by a nucleic acid that hybridizes to the "UBIQUITIN--PROTEIN LIGASE EDD" nucleic acid under low stringency conditions, and/or
   (xxiii) "HYPOTH 35.7 KDA PROTEIN" (SEQ ID No:85) or a functionally active derivative thereof, or a functionally active fragment thereof, or a homolog thereof, or a variant of "HYPOTH 35.7 KDA PROTEIN" encoded by a nucleic acid that hybridizes to the "HYPOTH 35.7 KDA PROTEIN" nucleic acid under low stringency conditions, and/or
   (xxiv) "MATRIN 3" (SEQ ID No:86) or a functionally active derivative thereof, or a functionally active fragment thereof, or a homolog thereof, or a variant of "MATRIN 3" encoded by a nucleic acid that hybridizes to the "MATRIN 3" nucleic acid under low stringency conditions, and/or
   (xxv) "NF45 PROTEIN" (SEQ ID No:87) or a functionally active derivative thereof, or a functionally active fragment thereof, or a homolog thereof, or a variant of "NF45 PROTEIN" encoded by a nucleic acid that hybridizes to the "NF45 PROTEIN" nucleic acid under low stringency conditions, and/or
   (xxvi) "PCNA" (SEQ ID No:88) or a functionally active derivative thereof, or a functionally active fragment thereof, or a homolog thereof, or a variant of "PCNA" encoded by a nucleic acid that hybridizes to the "PCNA" nucleic acid under low stringency conditions, and/or
   (xxvii) "54 KDA NUCLEAR RNA- AND DNA-BINDING PROTEIN" (SEQ ID No:89) or a functionally active derivative thereof, or a functionally active fragment thereof, or a homolog thereof, or a variant of "54 KDA NUCLEAR RNA- AND DNA-BINDING PROTEIN" encoded by a nucleic acid that hybridizes to the "54 KDA NUCLEAR RNA-AND DNA-BINDING PROTEIN" nucleic acid under low stringency conditions, and/or
   (xxviii) "UBIQUITIN CARBOXYL-TERMINAL HYDROLASE 11" (SEQ ID No:90) or a functionally active derivative thereof, or a functionally active fragment thereof, or a homolog thereof, or a variant of "UBIQUITIN CARBOXYL-TERMINAL HYDROLASE 11" encoded by a nucleic acid that hybridizes to the "UBIQUITIN CARBOXYL-TERMINAL HYDROLASE 11" nucleic acid under low stringency conditions, and/or
   (xxix) "CELL DIFFERENTIATION PROTEIN RCD1" (SEQ ID No:91) or a functionally active derivative thereof, or a functionally active fragment thereof, or a homolog thereof, or a variant of "CELL DIFFERENTIATION PROTEIN RCD1" encoded by a nucleic acid that hybridizes to the "CELL DIFFERENTIATION PROTEIN RCD1" nucleic acid under low stringency conditions, and/or
   (xxx) "CHROMATIN ASSEMBLY FACTOR 1 SUBUNIT C" (SEQ ID No:92) or a functionally active derivative thereof, or a functionally active fragment thereof, or a homolog thereof, or a variant of "CHROMATIN ASSEMBLY FACTOR 1 SUBUNIT C" encoded by a nucleic acid that hybridizes to the "CHROMATIN ASSEMBLY FACTOR 1 SUBUNIT C" nucleic acid under low stringency conditions, and/or
   (xxxi) "HISTONE ACETYLTRANSFERASE TYPE B SUBUNIT 2" (SEQ ID No:93) or a functionally active derivative thereof, or a functionally active fragment thereof, or a homolog thereof, or a variant of "HISTONE ACETYLTRANSFERASE TYPE B SUBUNIT 2" encoded by a nucleic acid that hybridizes to the "HISTONE ACETYLTRANSFERASE TYPE B SUBUNIT 2" nucleic acid under low stringency conditions, and/or
   (xxxii) "HYPOTHETICAL PROTEIN KIAA0310" (SEQ ID No:94) or a functionally active derivative thereof, or a functionally active fragment thereof, or a homolog thereof, or a variant of "HYPOTHETICAL PROTEIN KIAA0310" encoded by a nucleic acid that hybridizes to the "HYPOTHETICAL PROTEIN KIAA0310" nucleic acid under low stringency conditions, and/or
   (xxxiii) "DNA-DIRECTED RNA POLYMERASE II 33 KDA POLYPEPTIDE" (SEQ ID No:95) or a functionally active derivative thereof, or a functionally active fragment thereof, or a homolog thereof, or a variant of "DNA-DIRECTED RNA POLYMERASE II 33 KDA POLYPEPTIDE" encoded by a nucleic acid that hybridizes to the "DNA-DIRECTED RNA POLYMERASE II 33 KDA POLYPEPTIDE" nucleic acid under low stringency conditions, and/or
   (xxxiv) "CALCIUM-DEPENDENT PROTEASE, SMALL SUBUNIT. CAPNS1" (SEQ ID No:96) or a functionally active derivative thereof, or a functionally active fragment thereof, or a homolog thereof, or a variant of "CALClUM-DEPENDENT PROTEASE, SMALL SUBUNIT. CAPNS1" encoded by a nucleic acid that hybridizes to the "CALCIUM-DEPENDENT PROTEASE, SMALL SUBUNIT. CAPNS1" nucleic acid under low stringency conditions, and/or
   (xxxv) "PCAF-ASSOCIATED FACTOR 400" (SEQ ID No:97) or a functionally active derivative thereof, or a functionally active fragment thereof, or a homolog thereof, or a variant of "PCAF-ASSOCIATED FACTOR 400" encoded by a nucleic acid that hybridizes to the "PCAF-ASSOCIATED FACTOR 400" nucleic acid under low stringency conditions, and/or
   (xxxvi) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions, and/or
   (xxxvii) "COMPLEMENT C1R-LIKE PROTEINASE" (SEQ ID No:98) or a functionally active derivative thereof, or a functionally active fragment thereof, or a homolog thereof, or a variant of "COMPLEMENT C1R-LIKE PROTEINASE" encoded by a nucleic acid that hybridizes to the "COMPLEMENT C1R-LIKE PROTEINASE" nucleic acid under low stringency conditions, and/or
   (xxxviii) "MMS19 PROTEIN" (SEQ ID No:99) or a functionally active derivative thereof, or a functionally active fragment thereof, or a homolog thereof, or a variant of "MMS19 PROTEIN" encoded by a nucleic acid that hybridizes to the "MMS19 PROTEIN" nucleic acid under low stringency conditions, and/or
   (xxxix) "TRANSCRIPTION INTERMEDIARY FACTOR 1-β" (SEQ ID No:100) or a functionally active derivative thereof, or a functionally active fragment thereof, or a homolog thereof, or a variant of "TRANSCRIPTION INTERMEDIARY FACTOR 1-β" encoded by a nucleic acid that hybridizes to the "TRANSCRIPTION INTERMEDIARY FACTOR 1-β" nucleic acid under low stringency conditions, and
   (xl) P52" (SEQ ID No:231) or a functionally active derivative thereof, or a functionally active fragment thereof, or a homolog thereof, or a variant of "p52 (NF-kappaB2)" encoded by a nucleic acid that hybridises to the "p52 (NF-kappaB2)" nucleic acid under low stringency conditions.is present in the complex.
41. The complex of any one of No. 1 - 8, or proteins of No. 13 or the antibody or fragment of No. 17, for use in a method of diagnosing a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
42. A method for treating or preventing a disease or disorder characterized by an aberrant amount of, activity or component composition of or intracellular localization of, the complex of anyone of No. 1-8, comprising administering to a subject in need of such treatment or prevention a therapeutically effective amount of one or more molecules that modulate the amount of, DNA binding activity, or protein components of, said complex.
43. The method according to No. 42, wherein said disease or disorder involves decreased levels of the amount or activity of said complex.
44. The method according to No. 42 , wherein said disease or disorder involves increased levels of the amount or activity of said complex.
45. Complex of any of No. 1-8 and/or protein selected from the following proteins
   (i) "Menin" (SEQ ID No:66) or a functionally active derivative thereof, or a functionally active fragment thereof, or a homolog thereof, or a variant of "Menin" encoded by a nucleic acid that hybridizes to the "Menin" nucleic acid under low stringency conditions,
   (ii) "SMRT" (SEQ ID No:67) or a functionally active derivative thereof, or a functionally active fragment thereof, or a homolog thereof, or a variant of "SMRT" encoded by a nucleic acid that hybridizes to the "SMRT" nucleic acid under low stringency conditions,
   (iii) "HDAC3" (SEQ ID No:68) or a functionally active derivative thereof, or a functionally active fragment thereof, or a homolog thereof, or a variant of "HDAC3" encoded by a nucleic acid that hybridizes to the "HDAC3" nucleic acid under low stringency conditions,

   (i) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (v) "HDAC2" (SEQ ID No:69) or a functionally active derivative thereof, or a functionally active fragment thereof, or a homolog thereof, or a variant of "HDAC2" encoded by a nucleic acid that hybridizes to the "HDAC2" nucleic acid under low stringency conditions,
   (vi) "Cot/Tpl-2" (SEQ ID No:70) or a functionally active derivative thereof, or a functionally active fragment thereof, or a homolog thereof, or a variant of "Cot/Tpl-2" encoded by a nucleic acid that hybridizes to the "Cot/Tpl-2" nucleic acid under low stringency conditions,
   (vii) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (viii) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,

   (i) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (x) "p50 (NF-κB1)" (SEQ ID No:73) or a functionally active derivative thereof, or a functionally active fragment thereof, or a homolog thereof, or a variant of "p50 (NF-κB1)" encoded by a nucleic acid that hybridizes to the "p50 (NF-κB1 )" nucleic acid under low stringency conditions,
   (xi) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (xii) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "RelA (p65)" nucleic acid under low stringency conditions,
   (xiii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (xiv) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (xv) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
   (xvi) "IKK ε" (SEQ ID No:79) or a functionally active derivative thereof, or a functionally active fragment thereof, or a homolog thereof, or a variant of "IKK ε" encoded by a nucleic acid that hybridizes to the "IKK ε" nucleic acid under low stringency conditions,
   (xvii) "HDAC1" (SEQ ID No:80) or a functionally active derivative thereof, or a functionally active fragment thereof, or a homolog thereof, or a variant of "HDAC1" encoded by a nucleic acid that hybridizes to the "HDAC1" nucleic acid under low stringency conditions,
   (xviii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (xix) "ReIB" (SEQ ID No:81) or a functionally active derivative thereof, or a functionally active fragment thereof, or a homolog thereof, or a variant of "ReIB" encoded by a nucleic acid that hybridizes to the "ReIB" nucleic acid under low stringency conditions,
   (xx) "NAKAP 95" (SEQ ID No:82) or a functionally active derivative thereof, or a functionally active fragment thereof, or a homolog thereof, or a variant of "NAKAP 95" encoded by a nucleic acid that hybridizes to the "NAKAP 95" nucleic acid under low stringency conditions,

   (i) "IMPORTIN A-3 SUBUNIT" (SEQ ID No:83) or a functionally active derivative thereof, or a functionally active fragment thereof, or a homolog thereof, or a variant of "IMPORTIN α-3 SUBUNIT" encoded by a nucleic acid that hybridizes to the "IMPORTIN α-3 SUBUNIT" nucleic acid under low stringency conditions,
   (xxii) "UBIQUITIN--PROTEIN LIGASE EDD" (SEQ ID No:84) or a functionally active derivative thereof, or a functionally active fragment thereof, or a homolog thereof, or a variant of "UBIQUITIN--PROTEIN LIGASE EDD" encoded by a nucleic acid that hybridizes to the "UBIQUITIN--PROTEIN LIGASE EDD" nucleic acid under low stringency conditions,
   (xxiii) "HYPOTH 35.7 KDA PROTEIN" (SEQ ID No:85) or a functionally active derivative thereof, or a functionally active fragment thereof, or a homolog thereof, or a variant of "HYPOTH 35.7 KDA PROTEIN" encoded by a nucleic acid that hybridizes to the "HYPOTH 35.7 KDA PROTEIN" nucleic acid under low stringency conditions,
   (xxiv) "MATRIN 3" (SEQ ID No:86) or a functionally active derivative thereof, or a functionally active fragment thereof, or a homolog thereof, or a variant of "MATRIN 3" encoded by a nucleic acid that hybridizes to the "MATRIN 3" nucleic acid under low stringency conditions,
   (xxv) "NF45 PROTEIN" (SEQ ID No:87) or a functionally active derivative thereof, or a functionally active fragment thereof, or a homolog thereof, or a variant of "NF45 PROTEIN" encoded by a nucleic acid that hybridizes to the "NF45 PROTEIN" nucleic acid under low stringency conditions,
   (xxvi) "PCNA" (SEQ ID No:88) or a functionally active derivative thereof, or a functionally active fragment thereof, or a homolog thereof, or a variant of "PCNA" encoded by a nucleic acid that hybridizes to the "PCNA" nucleic acid under low stringency conditions,
   (xxvii) "54 KDA NUCLEAR RNA- AND DNA-BINDING PROTEIN" (SEQ ID No:89) or a functionally active derivative thereof, or a functionally active fragment thereof, or a homolog thereof, or a variant of "54 KDA NUCLEAR RNA- AND DNA-BINDING PROTEIN" encoded by a nucleic acid that hybridizes to the "54 KDA NUCLEAR RNA-AND DNA-BINDING PROTEIN" nucleic acid under low stringency conditions,
   (xxviii) "UBIQUITIN CARBOXYL-TERMINAL HYDROLASE 11" (SEQ ID No:90) or a functionally active derivative thereof, or a functionally active fragment thereof, or a homolog thereof, or a variant of "UBIQUITIN CARBOXYL-TERMINAL HYDROLASE 11" encoded by a nucleic acid that hybridizes to the "UBIQUITIN CARBOXYL-TERMINAL HYDROLASE 11" nucleic acid under low stringency conditions,
   (xxix) "CELL DIFFERENTIATION PROTEIN RCD1" (SEQ ID No:91) or a functionally active derivative thereof, or a functionally active fragment thereof, or a homolog thereof, or a variant of "CELL DIFFERENTIATION PROTEIN RCD1" encoded by a nucleic acid that hybridizes to the "CELL DIFFERENTIATION PROTEIN RCD1" nucleic acid under low stringency conditions,
   (xxx) "CHROMATIN ASSEMBLY FACTOR 1 SUBUNIT C" (SEQ ID No:92) or a functionally active derivative thereof, or a functionally active fragment thereof, or a homolog thereof, or a variant of "CHROMATIN ASSEMBLY FACTOR 1 SUBUNIT C" encoded by a nucleic acid that hybridizes to the "CHROMATIN ASSEMBLY FACTOR 1 SUBUNIT C" nucleic acid under low stringency conditions,
   (xxxi) "HISTONE ACETYLTRANSFERASE TYPE B SUBUNIT 2" (SEQ ID No:93) or a functionally active derivative thereof, or a functionally active fragment thereof, or a homolog thereof, or a variant of "HISTONE ACETYLTRANSFERASE TYPE B SUBUNIT 2" encoded by a nucleic acid that hybridizes to the "HISTONE ACETYLTRANSFERASE TYPE B SUBUNIT 2" nucleic acid under low stringency conditions,
   (xxxii) "HYPOTHETICAL PROTEIN KIAA0310" (SEQ ID No:94) or a functionally active derivative thereof, or a functionally active fragment thereof, or a homolog thereof, or a variant of "HYPOTHETICAL PROTEIN KIAA0310" encoded by a nucleic acid that hybridizes to the "HYPOTHETICAL PROTEIN KIAA0310" nucleic acid under low stringency conditions,
   (xxxiii) "DNA-DIRECTED RNA POLYMERASE II 33 KDA POLYPEPTIDE" (SEQ ID No:95) or a functionally active derivative thereof, or a functionally active fragment thereof, or a homolog thereof, or a variant of "DNA-DIRECTED RNA POLYMERASE II 33 KDA POLYPEPTIDE" encoded by a nucleic acid that hybridizes to the "DNA-DIRECTED RNA POLYMERASE II 33 KDA POLYPEPTIDE" nucleic acid under low stringency conditions,
   (xxxiv) "CALCIUM-DEPENDENT PROTEASE, SMALL SUBUNIT. CAPNS1" (SEQ ID No:96) or a functionally active derivative thereof, or a functionally active fragment thereof, or a homolog thereof, or a variant of "CALCIUM-DEPENDENT PROTEASE, SMALL SUBUNIT. CAPNS1" encoded by a nucleic acid that hybridizes to the "CALCIUM-DEPENDENT PROTEASE, SMALL SUBUNIT. CAPNS1" nucleic acid under low stringency conditions,
   (xxxv) "PCAF-ASSOCIATED FACTOR 400" (SEQ ID No:97) or a functionally active derivative thereof, or a functionally active fragment thereof, or a homolog thereof, or a variant of "PCAF-ASSOCIATED FACTOR 400" encoded by a nucleic acid that hybridizes to the "PCAF-ASSOCIATED FACTOR 400" nucleic acid under low stringency conditions,
   (xxxvi) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions,
   (xxxvii) "COMPLEMENT C1R-LIKE PROTEINASE" (SEQ ID No:98) or a functionally active derivative thereof, or a functionally active fragment thereof, or a homolog thereof, or a variant of "COMPLEMENT C1R-LIKE PROTEINASE" encoded by a nucleic acid that hybridizes to the "COMPLEMENT C1R-LIKE PROTEINASE" nucleic acid under low stringency conditions,
   (xxxviii) "MMS19 PROTEIN" (SEQ ID No:99) or a functionally active derivative thereof,or a functionally active fragement thereof, or a homolog thereof, or a variant of "MMS19 PROTEIN" that hybridizes to the "MMS19 PROTEIN" nucleic acid under low stringency conditions, and
   (xxxix) "TRANSCRIPTION INTERMEDIARY FACTOR 1-β" (SEQ ID No:100) or a functionally active derivative thereof, or a functionally active fragment thereof, or a homolog thereof, or a variant of "TRANSCRIPTION INTERMEDIARY FACTOR 1-β" encoded by a nucleic acid that hybridizes to the "TRANSCRIPTION INTERMEDIARY FACTOR 1-β" nucleic acid under low stringency conditions, and
   (xl) P52" (SEQ ID No:231) or a functionally active derivative thereof, or a functionally active fragment thereof, or a homolog thereof, or a variant of "p52 (NF-kappaB2)" encoded by a nucleic acid that hybridises to the "p52 (NF-kappaB2)" nucleic acid under low stringency conditions,
as a target for an active agent of a pharmaceutical, preferably a drug target in the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.

The present invention further relates to the p50-(NF-kappaB1) protein complex
1. A protein complex selected from complex (I) and comprising
   (a) at least one first protein selected from the group consisting of:
      (i) "Bcl-3" (SEQ ID No:101) or a functionally active derivative thereof, or a functionally active fragment thereof, or a homolog thereof, or a variant of "Bcl-3" encoded by a nucleic acid that hybridizes to the "Bcl-3" nucleic acid under low stringency conditions,
      (ii) "Menin" (SEQ ID No:66) or a functionally active derivative thereof, or a functionally active fragment thereof, or a homolog thereof, or a variant of "Menin" encoded by a nucleic acid that hybridizes to the "Menin" nucleic acid under low stringency conditions,
      (iii) "Cot/Tpl-2" (SEQ ID No:70) or a functionally active derivative thereof, or a functionally active fragment thereof, or a homolog thereof, or a variant of "Cot/Tpl-2" encoded by a nucleic acid that hybridizes to the "Cot/Tpl-2" nucleic acid under low stringency conditions,
      (iv) "glucocorticoid receptor (GR)" (SEQ ID No:102) or a functionally active derivative thereof, or a functionally active fragment thereof, or a homolog thereof, or a variant of "glucocorticoid receptor (GR)" encoded by a nucleic acid that hybridizes to the "glucocorticoid receptor (GR)" nucleic acid under low stringency conditions,
      (v) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
      (vi) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
      (vii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
      (viii) "Iκ-B β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
      (ix) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
      (x) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
      (xi) "p52 (NF-kappaB2)" (SEQ ID No:231) or a functionally active derivative thereof, or a functionally active fragment thereof, or a homolog thereof, or a variant of "p52 (NF-kappaB2)" encoded by a nucleic acid that hybridises to the "p52 (NF-kappaB2)" nucleic acid under low stringency conditions,
      (xii) "RelB" (SEQ ID No:81) or a functionally active derivative thereof, or a functionally active fragment thereof, or a homolog thereof, or a variant of "RelB" encoded by a nucleic acid that hybridizes to the "RelB" nucleic acid under low stringency conditions,
      (xiii) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
      (xiv) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
      (xv) "RelA (p65)" (SEQ ID No:75) or a functionally active derivative thereof, or a functionally active fragment thereof, or a homolog thereof, or a variant of "RelA (p65)" encoded by a nucleic acid that hybridizes to the "RelA (p65)" nucleic acid under low stringency conditions,
      (xvi) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions, and
      (xvii) "p50 (NF-kappaB1)" (SEQ ID No:73) or a functionally active derivative thereof, or a functionally active fragment thereof, or a homolog thereof, or a variant of "p50 (NF-kappaB1)" encoded by a nucleic acid that hybridizes to the "p50 (NF-kappaB1)" nucleic acid under low stringency conditions,
      and
      (b) at least one second protein, which second protein is selected from the group consisting of:
      (i) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions,
      (ii) "IMPORTIN α-3 SUBUNIT" (SEQ ID No:83) or a functionally active derivative thereof, or a functionally active fragment thereof, or a homolog thereof, or a variant of "IMPORTIN α-3 SUBUNIT" encoded by a nucleic acid that hybridizes to the "IMPORTIN α-3 SUBUNIT" nucleic acid under low stringency conditions,
      (iii) "karyopherin α 1" (SEQ ID No:103) or a functionally active derivative thereof, or a functionally active fragment thereof, or a homolog thereof, or a variant of "karyopherin α 1" encoded by a nucleic acid that hybridizes to the "karyopherin α 1" nucleic acid under low stringency conditions, and
      (iv) "COMPLEMENT C1R-LIKE PROTEINASE" (SEQ ID No:98) or a functionally active derivative thereof, or a functionally active fragment thereof, or a homolog thereof, or a variant of "COMPLEMENT C1R-LIKE PROTEINASE" encoded by a nucleic acid that hybridizes to the "COMPLEMENT C1R-LIKE PROTEINASE" nucleic acid under low stringency conditions;
   and a complex (II) comprising at least two of said second proteins,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
2. The protein complex according to claim 1 wherein the first protein is the protein "p50 (NF-κB1)" (SEQ ID No:73), or a functionally active derivative thereof, or a functionally active fragment thereof, or a homolog thereof, or a variant of "p50 (NF-κB1)" encoded by a nucleic acid that hybridizes to the "p50 (NF-κB1)" nucleic acid under low stringency conditions.
3. The protein complex according to No. 1 selected from complex (I) and comprising the following proteins:
   (i) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
   (ii) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (iii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (iv) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (v) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (vi) "p105 (NF-κB1 )" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (vii) "p50 (NF-κB1)" (SEQ ID No:73) or a functionally active derivative thereof, or a functionally active fragment thereof, or a homolog thereof, or a variant of "p50 (NF-κB1)" encoded by a nucleic acid that hybridizes to the "p50 (NF-κB1)" nucleic acid under low stringency conditions,
   (viii) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions,
   (ix) "ReIB" (SEQ ID No:81) or a functionally active derivative thereof, or a functionally active fragment thereof, or a homolog thereof, or a variant of "ReIB" encoded by a nucleic acid that hybridizes to the "ReIB" nucleic acid under low stringency conditions,
   (x) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions, and
   (xi) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions; and a protein complex selected from complex (II) and comprising the following proteins:

   (i) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
   (ii) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (iii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (iv) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (v) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (vi) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (vii) "p50 (NF-κB1)" (SEQ ID No:73) or a functionally active derivative thereof, or a functionally active fragment thereof, or a homolog thereof, or a variant of "p50 (NF-κB1)" encoded by a nucleic acid that hybridizes to the "p50 (NF-κB1)" nucleic acid under low stringency conditions,
   (viii) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions,
   (ix) "ReIB" (SEQ ID No:81) or a functionally active derivative thereof, or a functionally active fragment thereof, or a homolog thereof, or a variant of "ReIB" encoded by a nucleic acid that hybridizes to the "ReIB" nucleic acid under low stringency conditions,
   (x) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions,
   (xi) "c-Rel" (SEQ I D No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions, ( ) "IMPORTIN α-3 SUBUNIT" (SEQ ID No:83) or a functionally active derivative thereof, or a functionally active fragment thereof, or a homolog thereof, or a variant of "IMPORTIN α-3 SUBUNIT" encoded by a nucleic acid that hybridizes to the "IMPORTIN α-3 SUBUNIT" nucleic acid under low stringency conditions, and
   (xiii) "karyopherin α 1" (SEQ ID No:103) or a functionally active derivative thereof,or a functionally active fragement thereof, or a homolog thereof, or a variant of "karyopherin α 1" that hybridizes to the "karyopherin α 1" nucleic acid under low stringency conditions; and a protein complex selected from complex (III) and comprising the following proteins:

   (i) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
   (ii) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (iii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (iv) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (v) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (vi) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (vii) "p50 (NF-κB1)" (SEQ ID No:73) or a functionally active derivative thereof, or a functionally active fragment thereof, or a homolog thereof, or a variant of "p50 (NF-κB1)" encoded by a nucleic acid that hybridizes to the "p50 (NF-κB1)" nucleic acid under low stringency conditions,
   (viii) "RelA (p65)" (SEQ ID No:75) or a functionally active derivative thereof, or a functionally active fragment thereof, or a homolog thereof, or a variant of "RelA (p65)" encoded by a nucleic acid that hybridizes to the "RelA (p65)" nucleic acid under low stringency conditions,
   (ix) "RelB" (SEQ ID No:81) or a functionally active derivative thereof, or a functionally active fragment thereof, or a homolog thereof, or a variant of "RelB" encoded by a nucleic acid that hybridizes to the "RelB" nucleic acid under low stringency conditions,
   (x) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions,
   (xi) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
   (xii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions, and
   (xiii) "COMPLEMENT C1R-LIKE PROTEINASE" (SEQ ID No:98) or a functionally active derivative thereof, or a functionally active fragment thereof, or a homolog thereof, or a variant of "COMPLEMENT C1R-LIKE PROTEINASE" encoded by a nucleic acid that hybridizes to the "COMPLEMENT C1R-LIKE PROTEINASE" nucleic acid under low stringency conditions;
   and a protein complex selected from complex (IV) and comprising the following proteins:
   (i) "Bcl-3" (SEQ ID No:101) or a functionally active derivative thereof, or a functionally active fragment thereof, or a homolog thereof, or a variant of "Bcl-3" encoded by a nucleic acid that hybridizes to the "Bcl-3" nucleic acid under low stringency conditions,
   (ii) "Menin" (SEQ ID No:66) or a functionally active derivative thereof, or a functionally active fragment thereof, or a homolog thereof, or a variant of "Menin" encoded by a nucleic acid that hybridizes to the "Menin" nucleic acid under low stringency conditions,
   (iii) "Cot /Tpl-2" (SEQ ID No:70) or a functionally active derivative thereof, or a functionally active fragment thereof, or a homolog thereof, or a variant of "Cot /Tpl-2" encoded by a nucleic acid that hybridizes to the "Cot/Tpl-2" nucleic acid under low stringency conditions,
   (iv) "glucocorticoid receptor (GR)" (SEQ ID No:102) or a functionally active derivative thereof, or a functionally active fragment thereof, or a homolog thereof, or a variant of "glucocorticoid rececptor (GR)" encoded by a nucleic acid that hybridizes to the "glucocorticoid receptor (GR)" nucleic acid under low stringency conditions,
   (v) "ABIN-2" (SEQ ID No: 1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions,

   (i) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (vii) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (viii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (ix) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (x) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,

   (i) "IKK γ" (SEQ ID No:23) or a functionary active derivative thereof, or a functionally active fragment thereof, or a homolog thereof, or a variant of "IKK γ" encoded by a nucleic acid that hybridizes to the "IKK γ" nucleic acid under low stringency conditions,
   (xii) "p50 (NF-κB1)" (SEQ ID No:73) or a functionally active derivative thereof, or a functionally active fragment thereof, or a homolog thereof, or a variant of "p50 (NF-κB1)" encoded by a nucleic acid that hybridizes to the "p50 (NF-κB1)" nucleic acid under low stringency conditions,
   (xiii) "RelB" (SEQ ID No:81) or a functionally active derivative thereof, or a functionally active fragment thereof, or a homolog thereof, or a variant of "RelB" encoded by a nucleic acid that hybridizes to the "RelB" nucleic acid under low stringency conditions,

   (i) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
   (xv) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (xvi) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions,

   (i) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (xviii) "IMPORTIN α-3 SUBUNIT" (SEQ ID No:83) or a functionally active derivative thereof, or a functionally active fragment thereof, or a homolog thereof, or a variant of "IMPORTIN α-3 SUBUNIT" encoded by a nucleic acid that hybridizes to the "IMPORTIN α-3 SUBUNIT" nucleic acid under low stringency conditions,
   (xix) "karyopherin α 1" (SEQ ID No:103) or a functionally active derivative thereof,or a functionally active fragement thereof, or a homolog thereof, or a variant of "karyopherin α 1" that hybridizes to the "karyopherin α 1" nucleic acid under low stringency conditions,
   (xx) "COMPLEMENT C1R-LIKE PROTEINASE" (SEQ ID No:98) or a functionally active derivative thereof, or a functionally active fragment thereof, or a homolog thereof, or a variant of "COMPLEMENT C1R-LIKE PROTEINASE" encoded by a nucleic acid that hybridizes to the "COMPLEMENT C1 R-LIKE PROTEINASE" nucleic acid under low stringency conditions, and
   (xxi) P52" (SEQ ID No:231) or a functionally active derivative thereof, or a functionally active fragment thereof, or a homolog thereof, or a variant of "p52 (NF-kappaB2)" encoded by a nucleic acid that hybridises to the "p52 (NF-kappaB2)" nucleic acid under low stringency conditions.
4. The protein complex according to No. 1 selected from complex (I) comprising all but 1 - 3 of the following proteins:
   (i) "Bcl-3" (SEQ ID No:101 ) or a functionally active derivative thereof, or a functionally active fragment thereof, or a homolog thereof, or a variant of "Bcl-3" encoded by a nucleic acid that hybridizes to the "Bcl-3" nucleic acid under low stringency conditions,
   (ii) "Menin" (SEQ ID No:66) or a functionally active derivative thereof, or a functionally active fragment thereof, or a homolog thereof, or a variant of "Menin" encoded by a nucleic acid that hybridizes to the "Menin" nucleic acid under low stringency conditions,
   (iii) "Cot/TpI-2" (SEQ ID No:70) or a functionally active derivative thereof, or a functionally active fragment thereof, or a homolog thereof, or a variant of "Cot /TpI-2" encoded by a nucleic acid that hybridizes to the "Cot /TpI-2" nucleic acid under low stringency conditions,
   (iv) "glucocorticoid receptor (GR)" (SEQ ID No:102) or a functionally active derivative thereof, or a functionally active fragment thereof, or a homolog thereof, or a variant of "glucocorticoid rececptor (GR)" encoded by a nucleic acid that hybridizes to the "glucocorticoid receptor (GR)" nucleic acid under low stringency conditions,
   (v) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions, ( ) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (vii) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (viii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (ix) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (x) "c-Rel" (SEQ ID No:71) or a functionally active derivative thereof, or a functionally active fragment thereof, or a homolog thereof, or a variant of "c-ReI" encoded by a nucleic acid that hybridizes to the "c-ReI" nucleic acid under low stringency conditions,
   ( ) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (xii) "p50 (NF-κB1)" (SEQ ID No:73) or a functionally active derivative thereof, or a functionally active fragment thereof, or a homolog thereof, or a variant of "p50 (NF-κB1)" encoded by a nucleic acid that hybridizes to the "p50 (NF-κB1)" nucleic acid under low stringency conditions,
   (xiii) "RelB" (SEQ ID No:81) or a functionally active derivative thereof, or a functionally active fragment thereof, or a homolog thereof, or a variant of "RelB" encoded by a nucleic acid that hybridizes to the "RelB" nucleic acid under low stringency conditions,
   ( ) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
   (xv) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (xvi) "RelA (p65)" (SEQ ID No:75) or a functionally active derivative thereof, or a functionally active fragment thereof, or a homolog thereof, or a variant of "RelA (p65)" encoded by a nucleic acid that hybridizes to the "ReIA (p65)" nucleic acid under low stringency conditions,
   ( ) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (xviii) "IMPORTIN α-3 SUBUNIT" (SEQ ID No:83) or a functionally active derivative thereof, or a functionally active fragment thereof, or a homolog thereof, or a variant of "IMPORTIN α-3 SUBUNIT" encoded by a nucleic acid that hybridizes to the "IMPORTIN α-3 SUBUNIT" nucleic acid under low stringency conditions,
   (xix) "karyopherin α1" (SEQ ID No:103) or a functionally active derivative thereof,or a functionally active fragement thereof, or a homolog thereof, or a variant of "karyopherin α 1" that hybridizes to the "karyopherin α 1" nucleic acid under low stringency conditions,
   (xx) "COMPLEMENT C1R-LIKE PROTEINASE" (SEQ ID No:98) or a functionally active derivative thereof, or a functionally active fragment thereof, or a homolog thereof, or a variant of "COMPLEMENT C1R-LIKE PROTEINASE" encoded by a nucleic acid that hybridizes to the "COMPLEMENT C1R-LIKE PROTEINASE" nucleic acid under low stringency conditions, and
   (xxi) P52" (SEQ ID No:231) or a functionally active derivative thereof, or a functionally active fragment thereof, or a homolog thereof, or a variant of "p52 (NF-kappaB2)" encoded by a nucleic acid that hybridises to the "p52 (NF-kappaB2)" nucleic acid under low stringency conditions;
   and a protein complex selected from complex (II) and comprising all but 1-3 of the following proteins:
   (i) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions,

   (i) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (iii) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (iv) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (v) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (vi) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,

   (i) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (viii) "p50 (NF-κB1)" (SEQ ID No:73) or a functionally active derivative thereof, or a functionally active fragment thereof, or a homolog thereof, or a variant of "p50 (NF-κB1)" encoded by a nucleic acid that hybridizes to the "p50 (NF-κB1)" nucleic acid under low stringency conditions,
   (ix) "ReIB" (SEQ ID No:81) or a functionally active derivative thereof, or a functionally active fragment thereof, or a homolog thereof, or a variant of "ReIB" encoded by a nucleic acid that hybridizes to the "ReIB" nucleic acid under low stringency conditions,

   (i) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
   (xi) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (xii) "RelA (p65)" (SEQ ID No:75) or a functionally active derivative thereof, or a functionally active fragment thereof, or a homolog thereof, or a variant of "RelA (p65)" encoded by a nucleic acid that hybridizes to the "RelA (p65)" nucleic acid under low stringency conditions,

   (i) ""IMPORTIN A-3 SUBUNIT" (SEQ ID No:83) or a functionally active derivative thereof, or a functionally active fragment thereof, or a homolog thereof, or a variant of "IMPORTIN α-3 SUBUNIT" encoded by a nucleic acid that hybridizes to the "IMPORTIN α-3 SUBUNIT" nucleic acid under low stringency conditions,
   (xiv) "karyopherin α 1" (SEQ ID No:103) or a functionally active derivative thereof,or a functionally active fragement thereof, or a homolog thereof, or a variant of "karyopherin α 1" that hybridizes to the "karyopherin α 1" nucleic acid under low stringency conditions, and
   (xv) "COMPLEMENT C1R-LIKE PROTEINASE" (SEQ ID No:98) or a functionally active derivative thereof, or a functionally active fragment thereof, or a homolog thereof, or a variant of "COMPLEMENT C1 R-LIKE PROTEINASE" encoded by a nucleic acid that hybridizes to the "COMPLEMENT C1R-LIKE PROTEINASE" nucleic acid under low stringency conditions.
5. The complex of any of No. 1 - 4 comprising a functionally active derivative of said first protein and/or a functionally active derivative of said second protein, wherein the functionally active derivative is a fusion protein comprising said first protein or said second protein fused to an amino acid sequence different from the first protein or second protein, respectively.
6. The complex of No. 5 wherein the functionally active derivative is a fusion protein comprising said first protein or said second protein fused to an affinity tag or label.
7. The complex of any of No. 1 - 4 comprising a fragment of said first protein and/or a fragment of said second protein, which fragment binds to another protein component of said complex.
8. The complex of any of No. 1 - 7 that is involved in the DNA binding activity.
9. A process for preparing complex of any of No. 1 - 8 and optionally the components thereof comprising the following steps:
   the expressing a protein (bait) of the complex, preferably a tagged protein, in a target cell, isolating the protein complex which is attached to the bait protein, and optionally disassociating the protein complex and isolating the individual complex members.
10. The process according to No. 9 wherein the tagged protein comprises two different tags which allow two separate affinity purification steps.
11. The process according to any of No. 9 - 10 wherein the two tags are separated by a cleavage site for a protease.
12. Component of the P50-protein-complexobtainable by a process according to any of No. 9 to 11.
13. Construct, preferably a vector construct, comprising at least two separate nucleic acid sequences each encoding a different protein, or a functionally active fragment or a functionally active derivative therof at least one of said proteins, or functionally active fragments or functionally active derivative therof being selected from the first group of proteins according to No. 1(a) and at least one of said proteines, or functionally active fragments of functionally active derivative thereof being selected from the second group of proteins according to No. 1(b)
14. Host cell containting a construct of No. 13 or containing several vectors each comprising at least the nucleic acid sequence encoding at least one of the proteins, or functionally active fragments or functionally active derivatives thereof selected from the first group of proteins according to No. 1(a) and the proteins, or functionally active fragments or functionally active derivatives thereof selected from the second group of proteins according to No. 1 (b).
15. An antibody or a fragment of said antibody containing the binding domain thereof, selected from an antibody or fragment thereof, which binds the complex of any of No. 1-8 and which does not bind any of the proteins of said complex when uncomplexed and an antibody or a fragment of said antibody which binds to any of the proteins according to No..
16. A kit comprising in one or more container the complex of any of No. 1 - 8 optionally together with an antibody according to No. 15 and/or further components such as reagents and working instructions.
17. The kit according to No.16 for processing a substrate of said complex.
18. A kit according to No. 16 for the diagnosis or prognosis of a disease or a disease risk, preferentially for a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
19. Array, in which at least a complex according to any of No. 1-8 and/or at least one antibody according to No. 15 is attached to a solid carrier.
20. A process for processing a physiological substrate of the complex comprising the step of bringing into contact a complex to any of No. 1 - 8 with said substrate, such that said substrate is processed.
21. A pharmaceutical composition comprising the protein complex of any of No. 1 to 8..
22. The pharmaceutical composition according to No. 21 for the treatment of diseases and disorders such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
23. A method for screening for a molecule that binds to the complex of anyone of No. 1 - 8. comprising the steps of
   (a) exposing said complex, or a cell or organism containing same to one or more candidate molecules; and
   (b) determinig whether said candidate molecule is bound to the complex or protein.
24. A method for screening for a molecule that modulates directly or indirectly the function, activity, composition or formation of the complex of any one of No. 1 - 8 comprising the steps of (a) exposing said complex, or a cell or organism containing P50-protein-complexto one or more candidate molecules; and
   (b) determining the amount of activity of protein components of, and/or intracellular localization of, said complex and/or the transcription level of a gene dependent on the complex and/or the abundance and/or activity of a protein or protein complex dependent on the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependent on the complex and/or the abundance and/or activity of a protein or protein complex dependent on the function of the complex and/or product of a gene dependent on the complex in the absence of said candidate molecules indicates that the molecule modulates function, activity or composition of said complex.
25. The method of No. 24, wherein the amount of said complex is determined.
26. The method of No. 24, wherein the activity of said complex is determined.
27. The method of No. 26, wherein said determining step comprises isolating from the cell or organism said complex to produce said isolated complex and contacting said isolated complex in the presence or absence of a candidate molecule with a substrate of said complex and determining the processing of said substrate is modified in the presence of said candidate molecule.
28. The method of No. 24, wherein the amount of the individual protein components of said complex are determined.
29. The method of No. 28, determining step comprises determining whether
   (i) "Bcl-3" (SEQ ID No:101) or a functionally active derivative thereof, or a functionally active fragment thereof, or a homolog thereof, or a variant of "BcI-3" encoded by a nucleic acid that hybridizes to the "BcI-3" nucleic acid under low stringency conditions, and/or
   (ii) "Menin" (SEQ ID No:66) or a functionally active derivative thereof, or a functionally active fragment thereof, or a homolog thereof, or a variant of "Menin" encoded by a nucleic acid that hybridizes to the "Menin" nucleic acid under low stringency conditions, and/or
   (iii) "Cot/TpI-2" (SEQ ID No:70) or a functionally active derivative thereof, or a functionally active fragment thereof, or a homolog thereof, or a variant of "Cot /Tpl-2" encoded by a nucleic acid that hybridizes to the "Cot /TpI-2" nucleic acid under low stringency conditions, and/or
   (iv) "glucocorticoid receptor (GR)" (SEQ ID No:102) or a functionally active derivative thereof, or a functionally active fragment thereof, or a homolog thereof, or a variant of "glucocorticoid rececptor (GR)" encoded by a nucleic acid that hybridizes to the "glucocorticoid receptor (GR)" nucleic acid under low stringency conditions, and/or
   (v) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions, and/or
   (vi) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions, and/or
   (vii) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions, and/or
   (viii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions, and/or
   (ix) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions, and/or
   (x) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions, and/or
   (xi) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions, and/or
   (xii) "p50 (NF-κB1)" (SEQ ID No:73) or a functionally active derivative thereof, or a functionally active fragment thereof, or a homolog thereof, or a variant of "p50 (NF-κB1)" encoded by a nucleic acid that hybridizes to the "p50 (NF-κB1)" nucleic acid under low stringency conditions, and/or
   (xiii) "ReIB" (SEQ ID No:81) or a functionally active derivative thereof, or a functionally active fragment thereof, or a homolog thereof, or a variant of "ReIB" encoded by a nucleic acid that hybridizes to the "ReIB" nucleic acid under low stringency conditions, and/or
   (xiv) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions, and/or
   (xv) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions, and/or
   (xvi) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions, and/or
   (xvii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions, and/or
   (xviii) "IMPORTIN α-3 SUBUNIT" (SEQ ID No:83) or a functionally active derivative thereof, or a functionally active fragment thereof, or a homolog thereof, or a variant of "IMPORTIN α-3 SUBUNIT" encoded by a nucleic acid that hybridizes to the "IMPORTIN α-3 SUBUNIT" nucleic acid under low stringency conditions, and/or
   (xix) "karyopherin α1" (SEQ ID No:103) or a functionally active derivative thereof, or a functionally active fragment thereof, or a homolog thereof, or a variant of "karyopherin α1" encoded by a nucleic acid that hybridizes to the "karyopherin α1" nucleic acid under low stringency conditions, and/or
   (xx) "COMPLEMENT C1R-LIKE PROTEINASE" (SEQ ID No:98) or a functionally active derivative thereof, or a functionally active fragment thereof, or a homolog thereof, or a variant of "COMPLEMENT C1R-LIKE PROTEINASE" encoded by a nucleic acid that hybridizes to the "COMPLEMENT C1R-LIKE PROTEINASE" nucleic acid under low stringency conditions, and/or
   (xxi) p52" (SEQ ID No:231) or a functionally active derivative thereof, or a functionally active fragment thereof, or a homolog thereof, or a variant of "p52 (NF-kappaB2)" encoded by a nucleic acid that hybridises to the "p52 (NF-kappaB2)" nucleic acid under low stringency conditions,
   Is present in the complex.
30. The method of any of No. 24 to 29, wherein said method is a method of screening for a drug for treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
31. Use of a molecule that modulates the amount of, activity of, or the protein components of the complex of any one of No. 1 - 8 for the manufacture of a medicament for the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
32. A method for the production of a pharmaceutical composition comprising carrying out the method of any of No. 1 - 8 to identify a molecule that modulates the function, activity, composition or formation of said complex, and further comprising mixing the identified molecule with a pharmaceutically acceptable carrier.
33. A method for diagnosing or screening for the presence of a disease or disorder or a predisposition for developing a disease or disorder in a subject, which disease or disorder is characterized by an aberrant amount of, activity of, or component composition of, or intracellular localization of the complex of any one of the No. 1 - 8, comprising determining the amount of, activity of, protein components of, and/or intracellular localization of, said complex and/or the transcription level of a gene dependent on the complex and/or the abundance and/or activity of a protein or protein complex dependent on the function of the complex and/or product of a gene dependent on the complex in a comparative sample derived from a subject, wherein a difference in said amount, activity, or protein components of, said complex in an analogous sample from a subject not having the disease or disorder or predisposition indicates the presence in the subject of the disease or disorder or predisposition in the subject.
34. The method of No. 33, wherein the amount of said complex is determined.
35. The method of No. 33, wherein the activity of said complex is determined.
36. The method of No. 35, wherein said determining step comprises isolating from the subject said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
37. The method of No. 33, wherein the amount of the individual protein components of said complex are determined.
38. The method of No. 37, wherein said determining step comprises determining whether
   (i) "Bcl-3" (SEQ ID No:101) or a functionally active derivative thereof, or a functionally active fragment thereof, or a homolog thereof, or a variant of "Bcl-3" encoded by a nucleic acid that hybridizes to the "Bcl-3" nucleic acid under low stringency conditions, and/or
   (ii) "Menin" (SEQ ID No:66) or a functionally active derivative thereof, or a functionally active fragment thereof, or a homolog thereof, or a variant of "Menin" encoded by a nucleic acid that hybridizes to the "Menin" nucleic acid under low stringency conditions, and/or
   (iii) "Cot/Tpl-2" (SEQ ID No:70) or a functionally active derivative thereof, or a functionally active fragment thereof, or a homolog thereof, or a variant of "Cot/Tpl-2" encoded by a nucleic acid that hybridizes to the "Cot/Tpl-2" nucleic acid under low stringency conditions, and/or
   (iv) "glucocorticoid receptor (GR)" (SEQ ID No:102) or a functionally active derivative thereof, or a functionally active fragment thereof, or a homolog thereof, or a variant of "glucocorticoid rececptor (GR)" encoded by a nucleic acid that hybridizes to the "glucocorticoid receptor (GR)" nucleic acid under low stringency conditions, and/or
   (v) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions, and/or

   (i) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions, and/or
   (vii) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions, and/or
   (viii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions, and/or
   (ix) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions, and/or
   (x) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions, and/or
   (xi) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions, and/or
   (xii) "p50 (NF-κB1)" (SEQ ID No:73) or a functionally active derivative thereof, or a functionally active fragment thereof, or a homolog thereof, or a variant of "p50 (NF-κB1)" encoded by a nucleic acid that hybridizes to the "p50 (NF-κB1)" nucleic acid under low stringency conditions, and/or
   (xiii) "ReIB" (SEQ ID No:81) or a functionally active derivative thereof, or a functionally active fragment thereof, or a homolog thereof, or a variant of "ReIB" encoded by a nucleic acid that hybridizes to the "ReIB" nucleic acid under low stringency conditions, and/or
   (xiv) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions, and/or
   (xv) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions, and/or
   (xvi) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions, and/or
   (xvii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions, and/or
   (xviii) "IMPORTIN α-3 SUBUNIT" (SEQ ID No:83) or a functionally active derivative thereof, or a functionally active fragment thereof, or a homolog thereof, or a variant of "IMPORTIN α-3 SUBUNIT" encoded by a nucleic acid that hybridizes to the "IMPORTIN α-3 SUBUNIT" nucleic acid under low stringency conditions, and/or
   (xix) "karyopherin α1" (SEQ ID No:103) or a functionally active derivative thereof, or a functionally active fragment thereof, or a homolog thereof, or a variant of "karyopherin α1" encoded by a nucleic acid that hybridizes to the "karyopherin α1" nucleic acid under low stringency conditions, and/or
   (xx) "COMPLEMENT C1R-LIKE PROTEINASE" (SEQ ID No:98) or a functionally active derivative thereof, or a functionally active fragment thereof, or a homolog thereof, or a variant of "COMPLEMENT C1R-LIKE PROTEINASE" encoded by a nucleic acid that hybridizes to the "COMPLEMENT C1R-LIKE PROTEINASE" nucleic acid under low stringency conditions, and/or
   (xxi) P52" (SEQ ID No:231) or a functionally active derivative thereof, or a functionally active fragment thereof, or a homolog thereof, or a variant of "p52 (NF-kappaB2)" encoded by a nucleic acid that hybridises to the "p52 (NF-kappaB2)" nucleic acid under low stringency conditions,
   is present in the complex.
39. The complex of any one of No. 1 - 8 or the antibody or fragment of No. 15, for use in a method of diagnosing a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
40. A method for treating or preventing a disease or disorder characterized by an aberrant amount of, activity or component composition of or intracellular localization of, the complex of anyone of No. 1 - 8, comprising administering to a subject in need of such treatment or prevention a therapeutically effective amount of one or more molecules that modulate the amount of, DNA binding activity, or protein components of, said complex.
41. The method according to No. 40, wherein said disease or disorder involves decreased levels of the amount or activity of said complex.
42. The method according to No. 40 , wherein said disease or disorder involves increased levels of the amount or activity of said complex.
43. Complex of any of No. 1 - 8 and/or protein selected from the following proteins
   (xxi) "Bcl-3" (SEQ ID No:101) or a functionally active derivative thereof, or a functionally active fragment thereof, or a homolog thereof, or a variant of "Bcl-3" encoded by a nucleic acid that hybridizes to the "Bcl-3" nucleic acid under low stringency conditions,
   (xxii) "Menin" (SEQ ID No:66) or a functionally active derivative thereof, or a functionally active fragment thereof, or a homolog thereof, or a variant of "Menin" encoded by a nucleic acid that hybridizes to the "Menin" nucleic acid under low stringency conditions,
   (xxiii) "Cot /Tpl-2" (SEQ ID No:70) or a functionally active derivative thereof, or a functionally active fragment thereof, or a homolog thereof, or a variant of "Cot /Tpl-2" encoded by a nucleic acid that hybridizes to the "Cot/Tpl-2" nucleic acid under low stringency conditions,
   (xxiv) "glucocorticoid receptor (GR)" (SEQ ID No:102) or a functionally active derivative thereof, or a functionally active fragment thereof, or a homolog thereof, or a variant of "glucocorticoid rececptor (GR)" encoded by a nucleic acid that hybridizes to the "glucocorticoid receptor (GR)" nucleic acid under low stringency conditions,
   (xxv) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions,
   (xxvi) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (xxvii) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (xxviii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (xxix) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (xxx) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
   (xxxi) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (xxxii) "p50 (NF-κB1)" (SEQ ID No:73) or a functionally active derivative thereof, or a functionally active fragment thereof, or a homolog thereof, or a variant of "p50 (NF-κB1)" encoded by a nucleic acid that hybridizes to the "p50 (NF-κB1)" nucleic acid under low stringency conditions,
   (xxxiii) "ReIB" (SEQ ID No:81) or a functionally active derivative thereof, or a functionally active fragment thereof, or a homolog thereof, or a variant of "ReIB" encoded by a nucleic acid that hybridizes to the "ReIB" nucleic acid under low stringency conditions,
   (xxxiv) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
   (xxxv) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (xxxvi) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions,
   (xxxvii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (xxxviii) "IMPORTIN α-3 SUBUNIT" (SEQ ID No:83) or a functionally active derivative thereof, or a functionally active fragment thereof, or a homolog thereof, or a variant of "IMPORTIN α-3 SUBUNIT" encoded by a nucleic acid that hybridizes to the "IMPORTIN α-3 SUBUNIT" nucleic acid under low stringency conditions,
   (xxxix) "karyopherin α1" (SEQ ID No:103) or a functionally active derivative thereof,or a functionally active fragement thereof, or a homolog thereof, or a variant of "karyopherin α 1" that hybridizes to the "karyopherin α1" nucleic acid under low stringency conditions, and
   (xl) "COMPLEMENT C1R-LIKE PROTEINASE" (SEQ ID No:98) or a functionally active derivative thereof, or a functionally active fragment thereof, or a homolog thereof, or a variant of "COMPLEMENT C1R-LIKE PROTEINASE" encoded by a nucleic acid that hybridizes to the "COMPLEMENT C1R-LIKE PROTEINASE" nucleic acid under low stringency conditions,
   (xli) p52" (SEQ ID No:231) or a functionally active derivative thereof, or a functionally active fragment thereof, or a homolog thereof, or a variant of "p52 (NF-kappaB2)" encoded by a nucleic acid that hybridises to the "p52 (NF-kappaB2)" nucleic acid under low stringency conditions,
as a target for an active agent of a pharmaceutical, preferably a drug target in the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.

The present invention further relates to the IKK-α protein complex
1. A protein complex selected from complex (I) and comprising
   (a) at least one first protein selected from the group consisting of:
      (i) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
      (ii) "Hsp90 α" (SEQ ID No:105) or a functionally active derivative thereof, or a functionally active fragment thereof, or a homolog thereof, or a variant of "Hsp90 α" encoded by a nucleic acid that hybridizes to the "Hsp90 α" nucleic acid under low stringency conditions,
      (iii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
      (iv) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
      (v) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
      (vi) "HSP90 β 2" (SEQ ID No:107) or a functionally active derivative thereof, or a functionally active fragment thereof, or a homolog thereof, or a variant of "HSP90 β2" encoded by a nucleic acid that hybridizes to the "HSP90 β2" nucleic acid under low stringency conditions,
      (vii) "MITOGEN-ACTIVATED PROTEIN KINASE KINASE KINASE 14" (SEQ ID No:108) or a functionally active derivative thereof, or a functionally active fragment thereof, or a homolog thereof, or a variant of "MITOGEN-ACTIVATED PROTEIN KINASE KINASE KINASE 14" encoded by a nucleic acid that hybridizes to the "MITOGEN-ACTIVATED PROTEIN KINASE KINASE KINASE 14" nucleic acid under low stringency conditions, and
      (viii) "TRAF2 " (SEQ ID No:15) or a functionally active derivative thereof, or a functionally active fragment thereof, or a homolog thereof, or a variant of "TRAF2 " encoded by a nucleic acid that hybridizes to the "TRAF2 " nucleic acid under low stringency conditions,
      and
   (b) at least one second protein, which second protein is selected from the group consisting of:
      (i) "51 KDA FK506-BINDING PROTEIN" (SEQ ID No:106) or a functionally active derivative thereof, or a functionally active fragment thereof, or a homolog thereof, or a variant of "51 KDA FK506-BINDING PROTEIN" encoded by a nucleic acid that hybridizes to the "51 KDA FK506-BINDING PROTEIN" nucleic acid under low stringency conditions;
   and a complex (II) comprising at least two of said second proteins,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
2. The protein complex according to claim 1 wherein the first protein is the protein "IKK α" (SEQ ID No:25), or a functionally active derivative thereof, or a functionally active fragment thereof, or a homolog thereof, or a variant of "IKK α" encoded by a nucleic acid that hybridizes to the "IKK α" nucleic acid under low stringency conditions.
3. The protein complex according to No. 1 selected from complex (I) and comprising the following proteins:
   (i) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,

   (i) "HSP90 α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions,
   (iii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (iv) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (v) "51 KDA FK506-BINDING PROTEIN" (SEQ ID No:106) or a functionally active derivative thereof, or a functionally active fragment thereof, or a homolog thereof, or a variant of "51 KDA FK506-BINDING PROTEIN" encoded by a nucleic acid that hybridizes to the "51 KDA FK506-BINDING PROTEIN" nucleic acid under low stringency conditions,

   (i) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (vii) "HSP90 β-2" (SEQ ID No:107) or a functionally active derivative thereof, or a functionally active fragment thereof, or a homolog thereof, or a variant of "HSP90 β-2" encoded by a nucleic acid that hybridizes to the "HSP90 β-2" nucleic acid under low stringency conditions;
   and a protein complex selected from complex (II) and comprising the following proteins:
   (i) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (ii) "HSP90 α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions,
   (iii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (iv) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (v) "51 KDA FK506-BINDING PROTEIN" (SEQ ID No:106) or a functionally active derivative thereof, or a functionally active fragment thereof, or a homolog thereof, or a variant of "51 KDA FK506-BINDING PROTEIN" encoded by a nucleic acid that hybridizes to the "51 KDA FK506-BINDING PROTEIN" nucleic acid under low stringency conditions,
   (vi) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (vii) "HSP90 β-2" (SEQ ID No:107) or a functionally active derivative thereof, or a functionally active fragment thereof, or a homolog thereof, or a variant of "HSP90 β-2" encoded by a nucleic acid that hybridizes to the "HSP90 β-2" nucleic acid under low stringency conditions,
   (viii) "MITOGEN-ACTIVATED PROTEIN KINASE KINASE KINASE 14" (SEQ ID No:108) or a functionally active derivative thereof, or a functionally active fragment thereof, or a homolog thereof, or a variant of "MITOGEN-ACTIVATED PROTEIN KINASE KINASE KINASE 14" encoded by a nucleic acid that hybridizes to the "MITOGEN-ACTIVATED PROTEIN KINASE KINASE KINASE 14" nucleic acid under low stringency conditions, and
   (ix) "TRAF2 " (SEQ ID No: 15) or a functionally active derivative thereof, or a functionally active fragment thereof, or a homolog thereof, or a variant of "TRAF2" encoded by a nucleic acid that hybridizes to the "TRAF2" nucleic acid under low stringency conditions.
4. The protein complex according to No. 1 selected from complex (I) comprising all but 1 of the following proteins:
   (i) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (ii) "HSP90 α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions,
   (iii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (iv) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (v) "51 KDA FK506-BINDING PROTEIN" (SEQ ID No:106) or a functionally active derivative thereof, or a functionally active fragment thereof, or a homolog thereof, or a variant of "51 KDA FK506-BINDING PROTEIN" encoded by a nucleic acid that hybridizes to the "51 KDA FK506-BINDING PROTEIN" nucleic acid under low stringency conditions,
   (vi) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (vii) "HSP90 β-2" (SEQ ID No:107) or a functionally active derivative thereof, or a functionally active fragment thereof, or a homolog thereof, or a variant of "HSP90 β-2" encoded by a nucleic acid that hybridizes to the "HSP90 β-2" nucleic acid under low stringency conditions,
   (viii) "MITOGEN-ACTIVATED PROTEIN KINASE KINASE KINASE 14" (SEQ ID No:108) or a functionally active derivative thereof,or a functionally active fragement thereof, or a homolog thereof, or a variant of "MITOGEN-ACTIVATED PROTEIN KINASE KINASE KINASE 14" that hybridizes to the "MITOGEN-ACTIVATED PROTEIN KINASE KINASE KINASE 14" nucleic acid under low stringency conditions, and
   (ix) "TRAF2 " (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions; and a protein complex selected from complex (II) that comprises all but 1 of the following proteins:

   (i) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (ii) "HSP90 α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions,
   (iii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (iv) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (v) "51 KDA FK506-BINDING PROTEIN" (SEQ ID No:106) or a functionally active derivative thereof, or a functionally active fragment thereof, or a homolog thereof, or a variant of "51 KDA FK506-BINDING PROTEIN" encoded by a nucleic acid that hybridizes to the "51 KDA FK506-BINDING PROTEIN" nucleic acid under low stringency conditions,
   (vi) "IKK α" (SEQ ID No:25) or a functionally active derivative thereof,or a functionally active fragement thereof, or a homolog thereof, or a variant of "IKK α" that hybridizes to the "IKK α" nucleic acid under low stringency conditions, and
   (vii) "HSP90 β-2" (SEQ ID No:107) or a functionally active derivative thereof,or a functionally active fragement thereof, or a homolog thereof, or a variant of "HSP90 β-2" that hybridizes to the "HSP90 β-2" nucleic acid under low stringency conditions.
5. The complex of any of No. 1 - 4 comprising a functionally active derivative of said first protein and/or a functionally active derivative of said second protein, wherein the functionally active derivative is a fusion protein comprising said first protein or said second protein fused to an amino acid sequence different from the first protein or second protein, respectively.
6. The complex of No. 5 wherein the functionally active derivative is a fusion protein comprising said first protein or said second protein fused to an affinity tag or label.
7. The complex of any of No. 1 - 4 comprising a fragment of said first protein and/or a fragment of said second protein, which fragment binds to another protein component of said complex.
8. The complex of any of No. 1 -7 that is involved in the IKK kinase activity or IKK recruitment to TNFR1.
9. A process for preparing complex of any of No. 1 - 8 and optionally the components thereof comprising the following steps:
   the expressing a protein (bait) of the complex, preferably a tagged protein, in a target cell, isolating the protein complex which is attached to the bait protein, and optionally disassociating the protein complex and isolating the individual complex members.
10. The process according to No. 9 wherein the tagged protein comprises two different tags which allow two separate affinity purification steps.
11. The process according to any of No. 9 - 10 wherein the two tags are separated by a cleavage site for a protease.
12. Component of the IKK-α protein complex obtainable by a process according to any of No. 9 to 11.
13. Construct, preferably a vector construct, comprising at least two separate nucleic acid sequences each encoding a different protein, or a functionally active fragment or a functionally active derivative therof at least one of said proteins, or functionally active fragments or functionally active derivative therof being selected from the first group of proteins according to No. 1(a) and at least one of said proteines, or functionally active fragments of functionally active derivative thereof being selected from the second group of proteins according to No. 1(b)
14. Host cell containting a construct of No. 13 or containing several vectors each comprising at least the nucleic acid sequence encoding at least one of the proteins, or functionally active fragments or functionally active derivatives thereof selected from the first group of proteins according to No. 1(a) and the proteins, or functionally active fragments or functionally active derivatives thereof selected from the second group of proteins according to No. 1(b).
15. An antibody or a fragment of said antibody containing the binding domain thereof, selected from an antibody or fragment thereof, which binds the complex of any of No. 1-8 and which does not bind any of the proteins of said complex when uncomplexed and an antibody or a fragment of said antibody which binds to any of the proteins according to No..
16. A kit comprising in one or more container the complex of any of No. 1 - 8optionally together with an antibody according to No. 15 and/or further components such as reagents and working instructions.
17. The kit according to No.16 for processing a substrate of said complex.
18. A kit according to No. 16 for the diagnosis or prognosis of a disease or a disease risk, preferentially for a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
19. Array, in which at least a complex according to any of No. 1-8 and/or at least one antibody according to No. 15 is attached to a solid carrier.
20. A process for processing a physiological substrate of the complex comprising the step of bringing into contact a complex to any of No. 1 - 8 with said substrate, such that said substrate is processed.
21. A pharmaceutical composition comprising the protein complex of any of No. 1 to 8..
22. The pharmaceutical composition according to No. 21 for the treatment of diseases and disorders such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
23. A method for screening for a molecule that binds to the complex of anyone of No. 1 - 8. comprising the steps of
   (a) exposing said complex, or a cell or organism containing same to one or more candidate molecules; and
   (b) determinig whether said candidate molecule is bound to the complex or protein.
24. A method for screening for a molecule that modulates directly or indirectly the function, activity, composition or formation of the complex of any one of No. 1 - 8 comprising the steps of (a) exposing said complex, or a cell or organism containing IKKα protein complex to one or more candidate molecules; and
   (b) determining the amount of activity of protein components of, and/or intracellular localization of, said complex and/or the transcription level of a gene dependent on the complex and/or the abundance and/or activity of a protein or protein complex dependent on the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependent on the complex and/or the abundance and/or activity of a protein or protein complex dependent on the function of the complex and/or product of a gene dependent on the complex in the absence of said candidate molecules indicates that the molecule modulates function, activity or composition of said complex.
25. The method of No. 24, wherein the amount of said complex is determined.
26. The method of No. 24, wherein the activity of said complex is determined.
27. The method of No. 26, wherein said determining step comprises isolating from the cell or organism said complex to produce said isolated complex and contacting said isolated complex in the presence or absence of a candidate molecule with a substrate of said complex and determining the processing of said substrate is modified in the presence of said candidate molecule.
28. The method of No. 24, wherein the amount of the individual protein components of said complex are determined.
29. The method of No. 28, determining step comprises determining whether
   (i) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions, and/or
   (ii) "HSP90 α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions, and/or
   (iii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions, and/or
   (iv) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions, and/or
   (v) "51 KDA FK506-BINDING PROTEIN" (SEQ ID No:106) or a functionally active derivative thereof, or a functionally active fragment thereof, or a homolog thereof, or a variant of "51 KDA FK506-BINDING PROTEIN" encoded by a nucleic acid that hybridizes to the "51 KDA FK506-BINDING PROTEIN" nucleic acid under low stringency conditions, and/or
   (vi) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions, and/or
   (vii) "HSP90 β 2" (SEQ ID No:107) or a functionally active derivative thereof, or a functionally active fragment thereof, or a homolog thereof, or a variant of "HSP90 β 2" encoded by a nucleic acid that hybridizes to the "HSP90 β 2" nucleic acid under low stringency conditions, and/or
   (viii) "MITOGEN-ACTIVATED PROTEIN KINASE KINASE KINASE 14" (SEQ ID No:108) or a functionally active derivative thereof, or a functionally active fragment thereof, or a homolog thereof, or a variant of "MITOGEN-ACTIVATED PROTEIN KINASE KINASE KINASE 14" encoded by a nucleic acid that hybridizes to the "MITOGEN-ACTIVATED PROTEIN KINASE KINASE KINASE 14" nucleic acid under low stringency conditions, and/or
   (ix) "TRAF2 "(SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions,
   is present in the complex.
30. The method of any of No. 24 to 29, wherein said method is a method of screening for a drug for treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
31. Use of a molecule that modulates the amount of, activity of, or the protein components of the complex of any one of No. 1-8 for the manufacture of a medicament for the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
32. A method for the production of a pharmaceutical composition comprising carrying out the method of any of No. 1 - 8 to identify a molecule that modulates the function, activity, composition or formation of said complex, and further comprising mixing the identified molecule with a pharmaceutically acceptable carrier.
33. A method for diagnosing or screening for the presence of a disease or disorder or a predisposition for developing a disease or disorder in a subject, which disease or disorder is characterized by an aberrant amount of, activity of, or component composition of, or intracellular localization of the complex of any one of the No. 1-8, comprising determining the amount of, activity of, protein components of, and/or intracellular localization of, said complex and/or the transcription level of a gene dependent on the complex and/or the abundance and/or activity of a protein or protein complex dependent on the function of the complex and/or product of a gene dependent on the complex in a comparative sample derived from a subject, wherein a difference in said amount, activity, or protein components of, said complex in an analogous sample from a subject not having the disease or disorder or predisposition indicates the presence in the subject of the disease or disorder or predisposition in the subject.
34. The method of No. 33, wherein the amount of said complex is determined.
35. The method of No. 33, wherein the activity of said complex is determined.
36. The method of No. 35, wherein said determining step comprises isolating from the subject said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
37. The method of No. 33, wherein the amount of the individual protein components of said complex are determined.
38. The method of No. 37, wherein said determining step comprises determining whether
   (i) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions, and/or
   (ii) "HSP90 α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions, and/or
   (iii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions, and/or
   (iv) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions, and/or
   (v) "51 KDA FK506-BINDING PROTEIN" (SEQ ID No:106) or a functionally active derivative thereof, or a functionally active fragment thereof, or a homolog thereof, or a variant of "51 KDA FK506-BINDING PROTEIN" encoded by a nucleic acid that hybridizes to the "51 KDA FK506-BINDING PROTEIN" nucleic acid under low stringency conditions, and/or
   (vi) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions, and/or
   (vii) "HSP90 β2" (SEQ ID No:107) or a functionally active derivative thereof, or a functionally active fragment thereof, or a homolog thereof, or a variant of "HSP90 β2" encoded by a nucleic acid that hybridizes to the "HSP90 β2" nucleic acid under low stringency conditions, and/or
   (viii) "MITOGEN-ACTIVATED PROTEIN KINASE KINASE KINASE 14" (SEQ ID No:108) or a functionally active derivative thereof, or a functionally active fragment thereof, or a homolog thereof, or a variant of "MITOGEN-ACTIVATED PROTEIN KINASE KINASE KINASE 14" encoded by a nucleic acid that hybridizes to the "MITOGEN-ACTIVATED PROTEIN KINASE KINASE KINASE 14" nucleic acid under low stringency conditions, and/or
   (ix) "TRAF2 " (SEQ ID No: 15) or a functionally active derivative thereof, or a functionally active fragment thereof, or a homolog thereof, or a variant of "TRAF2" encoded by a nucleic acid that hybridizes to the "TRAF2" nucleic acid under low stringency conditions,
   is present in the complex.
39. The complex of any one of No. 1 - 8 or the antibody or fragment of No. 15, for use in a method of diagnosing a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
40. A method for treating or preventing a disease or disorder characterized by an aberrant amount of, activity or component composition of or intracellular localization of, the complex of anyone of No. 1-8, comprising administering to a subject in need of such treatment or prevention a therapeutically effective amount of one or more molecules that modulate the amount of, IKK kinase activity or IKK recruitment to TNFR1, or protein components of, said complex.
41. The method according to No. 40, wherein said disease or disorder involves decreased levels of the amount or activity of said complex.
42. The method according to No. 40 , wherein said disease or disorder involves increased levels of the amount or activity of said complex.
43. Complex of any of No. 1 - 8 and/or protein selected from the following proteins
   (i) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (ii) "HSP90 α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions,
   (iii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (iv) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (v) "51 KDA FK506-BINDING PROTEIN" (SEQ ID No:106) or a functionally active derivative thereof, or a functionally active fragment thereof, or a homolog thereof, or a variant of "51 KDA FK506-BINDING PROTEIN" encoded by a nucleic acid that hybridizes to the "51 KDA FK506-BINDING PROTEIN" nucleic acid under low stringency conditions,
   (vi) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (vii) "HSP90 β 2" (SEQ ID No:107) or a functionally active derivative thereof, or a functionally active fragment thereof, or a homolog thereof, or a variant of "HSP90 β 2" encoded by a nucleic acid that hybridizes to the "HSP90 β 2" nucleic acid under low stringency conditions,
   (viii) "MITOGEN-ACTIVATED PROTEIN KINASE KINASE KINASE 14" (SEQ ID No:108) or a functionally active derivative thereof,or a functionally active fragement thereof, or a homolog thereof, or a variant of "MITOGEN-ACTIVATED PROTEIN KINASE KINASE KINASE 14" that hybridizes to the "MITOGEN-ACTIVATED PROTEIN KINASE KINASE KINASE 14" nucleic acid under low stringency conditions, and
   (ix) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions, as a target for an active agent of a pharmaceutical, preferably a drug target in the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.

The present invention further relates to the IKKγ-protein complex
1. A protein complex selected from complex (I) and comprising
   (a) at least one first protein selected from the group consisting of:
      (i) "HSP90 α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions,
      (ii) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
      (iii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
      (iv) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
      (v) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions, and
      (vi) "HSP90 B 2" (SEQ ID No:107) or a functionally active derivative thereof, or a functionally active fragment thereof, or a homolog thereof, or a variant of "HSP90 β 2" encoded by a nucleic acid that hybridizes to the "HSP90 β 2" nucleic acid under low stringency conditions,
      and
   (b) at least one second protein, which second protein is selected from the group consisting of:
      (i) "PP1-γ CATALYTIC SUBUNIT" (SEQ ID No:109) or a functionally active derivative thereof, or a functionally active fragment thereof, or a homolog thereof, or a variant of "PP1-γ CATALYTIC SUBUNIT" encoded by a nucleic acid that hybridizes to the "PP1-γ CATALYTIC SUBUNIT" nucleic acid under low stringency conditions,
      (ii) "WUGSC:H_DJ0687K01.2 PROTEIN" (SEQ ID No:110) or a functionally active derivative thereof, or a functionally active fragment thereof, or a homolog thereof, or a variant of "WUGSC:H_DJ0687K01.2 PROTEIN" encoded by a nucleic acid that hybridizes to the "WUGSC:H_DJ0687K01.2 PROTEIN" nucleic acid under low stringency conditions,
      (iii) "PROTEIN ARGININE N-METHYLTRANSFERASE 1" (SEQ ID No:111 ) or a functionally active derivative thereof, or a functionally active fragment thereof, or a homolog thereof, or a variant of "PROTEIN ARGININE N-METHYLTRANSFERASE 1" encoded by a nucleic acid that hybridizes to the "PROTEIN ARGININE N-METHYLTRANSFERASE 1" nucleic acid under low stringency conditions,
      (iv) "GUANINE NUCLEOTIDE-BINDING PROTEIN G(I)/G(S)/G(O) γ-12 SUBUNIT" (SEQ ID No:112) or a functionally active derivative thereof, or a functionally active fragment thereof, or a homolog thereof, or a variant of "GUANINE NUCLEOTIDE-BINDING PROTEIN G(I)/G(S)/G(O) γ-12 SUBUNIT" encoded by a nucleic acid that hybridizes to the "GUANINE NUCLEOTIDE-BINDING PROTEIN G(I)/G(S)/G(O) γ-12 SUBUNIT" nucleic acid under low stringency conditions,
      (v) "CamKII δ" (SEQ ID No:113) or a functionally active derivative thereof, or a functionally active fragment thereof, or a homolog thereof, or a variant of "CamKII δ" encoded by a nucleic acid that hybridizes to the "CamKII δ" nucleic acid under low stringency conditions,
      (vi) "PP1-β CATAL SUBUNIT" (SEQ ID No:114) or a functionally active derivative thereof, or a functionally active fragment thereof, or a homolog thereof, or a variant of "PP1-β CATAL SUBUNIT" encoded by a nucleic acid that hybridizes to the "PP1-β CATAL SUBUNIT" nucleic acid under low stringency conditions,
      (vii) "TANK BINDING KINASE TBK1" (SEQ ID No:115) or a functionally active derivative thereof, or a functionally active fragment thereof, or a homolog thereof, or a variant of "TANK BINDING KINASE TBK1" encoded by a nucleic acid that hybridizes to the "TANK BINDING KINASE TBK1" nucleic acid under low stringency conditions, and
      (viii) "CSD-SAP" (SEQ ID No:116) or a functionally active derivative thereof, or a functionally active fragment thereof, or a homolog thereof, or a variant of "CSD-SAP" encoded by a nucleic acid that hybridizes to the "CSD-SAP" nucleic acid under low stringency conditions;
   and a complex (II) comprising at least two of said second proteins,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
2. The protein complex according to claim 1 wherein the first protein is the protein "IKK γ" (SEQ ID No:23), or a functionally active derivative thereof, or a functionally active fragment thereof, or a homolog thereof, or a variant of "IKK γ" encoded by a nucleic acid that hybridizes to the "IKK γ" nucleic acid under low stringency conditions.
3. The protein complex according to No. 1 selected from complex (I) and comprising the following proteins:
   (i) "HSP90 α" (SEQ ID No: 105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions,
   (ii) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (iii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (iv) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (v) "PP1-γ CATALYTIC SUBUNIT" (SEQ ID No:109) or a functionally active derivative thereof, or a functionally active fragment thereof, or a homolog thereof, or a variant of "PP1-γ CATALYTIC SUBUNIT" encoded by a nucleic acid that hybridizes to the "PP1-γ CATALYTIC SUBUNIT" nucleic acid under low stringency conditions,
   (vi) "WUGSC:H_DJ0687K01.2 PROTEIN" (SEQ ID No:110) or a functionally active derivative thereof, or a functionally active fragment thereof, or a homolog thereof, or a variant of "WUGSC:H_DJ0687K01.2 PROTEIN" encoded by a nucleic acid that hybridizes to the "WUGSC:H_DJ0687K01.2 PROTEIN" nucleic acid under low stringency conditions,
   (vii) "GUANINE NUCLEOTIDE-BINDING PROTEIN G(I)/G(S)/G(O) γ-12 SUBUNIT" (SEQ ID No:112) or a functionally active derivative thereof, or a functionally active fragment thereof, or a homolog thereof, or a variant of "GUANINE NUCLEOTIDE-BINDING PROTEIN G(I)/G(S)/G(O) γ-12 SUBUNIT" encoded by a nucleic acid that hybridizes to the "GUANINE NUCLEOTIDE-BINDING PROTEIN G(I)/G(S)/G(O)γ-12 SUBUNIT" nucleic acid under low stringency conditions,
   (viii) "TANK BINDING KINASE TBK1" (SEQ ID No:115) or a functionally active derivative thereof, or a functionally active fragment thereof, or a homolog thereof, or a variant of "TANK BINDING KINASE TBK1" encoded by a nucleic acid that hybridizes to the "TANK BINDING KINASE TBK1" nucleic acid under low stringency conditions,
   (ix) "CSD-SAP" (SEQ ID No:116) or a functionally active derivative thereof, or a functionally active fragment thereof, or a homolog thereof, or a variant of "CSD-SAP" encoded by a nucleic acid that hybridizes to the "CSD-SAP" nucleic acid under low stringency conditions,

   (i) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions, and
   (xi) "Hsp90 β-2" (SEQ ID No:107) or a functionally active derivative thereof, or a functionally active fragment thereof, or a homolog thereof, or a variant of "Hsp90 β-2" encoded by a nucleic acid that hybridizes to the "Hsp90 β-2" nucleic acid under low stringency conditions;
   and a protein complex selected from complex (II) and comprising the following proteins
   (i) "HSP90 α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions,
   (ii) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (iii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (iv) "IKK α" (SEQ ID No:25) or a functionally active derivative thereof, or a functionally active fragment thereof, or a homolog thereof, or a variant of "IKK α" encoded by a nucleic acid that hybridizes to the "I KK α" nucleic acid under low stringency conditions,
   (v) "PP1-γ CATALYTIC SUBUNIT" (SEQ ID No:109) or a functionally active derivative thereof, or a functionally active fragment thereof, or a homolog thereof, or a variant of "PP1-γ CATALYTIC SUBUNIT" encoded by a nucleic acid that hybridizes to the "PP1-γ CATALYTIC SUBUNIT" nucleic acid under low stringency conditions,
   (vi) "WUGSC:H_DJ0687K01.2 PROTEIN" (SEQ ID No:110) or a functionally active derivative thereof, or a functionally active fragment thereof, or a homolog thereof, or a variant of "WUGSC:H_DJ0687K01.2 PROTEIN" encoded by a nucleic acid that hybridizes to the "WUGSC:H_DJ0687K01.2 PROTEIN" nucleic acid under low stringency conditions,
   (vii) "PROTEIN ARGININE N-METHYLTRANSFERASE 1" (SEQ ID No:111) or a functionally active derivative thereof, or a functionally active fragment thereof, or a homolog thereof, or a variant of "PROTEIN ARGININE N-METHYLTRANSFERASE 1" encoded by a nucleic acid that hybridizes to the "PROTEIN ARGININE N-METHYLTRANSFERASE 1" nucleic acid under low stringency conditions,
   (viii) "GUANINE NUCLEOTIDE-BINDING PROTEIN G(I)/G(S)/G(O) γ-12 SUBUNIT" (SEQ ID No: 112) or a functionally active derivative thereof, or a functionally active fragment thereof, or a homolog thereof, or a variant of "GUANINE NUCLEOTIDE-BINDING PROTEIN G(I)/G(S)/G(O) γ-12 SUBUNIT" encoded by a nucleic acid that hybridizes to the "GUANINE NUCLEOTIDE-BINDING PROTEIN G(I)/G(S)/G(O) γ-12 SUBUNIT" nucleic acid under low stringency conditions,
   (ix) "TANK BINDING KINASE TBK1" (SEQ ID No:115) or a functionally active derivative thereof, or a functionally active fragment thereof, or a homolog thereof, or a variant of "TANK BINDING KINASE TBK1" encoded by a nucleic acid that hybridizes to the "TANK BINDING KINASE TBK1" nucleic acid under low stringency conditions,
   (x) "CSD-SAP" (SEQ ID No:116) or a functionally active derivative thereof, or a functionally active fragment thereof, or a homolog thereof, or a variant of "CSD-SAP" encoded by a nucleic acid that hybridizes to the "CSD-SAP" nucleic acid under low stringency conditions,
   (xi) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions, and
   (xii) "Hsp90 β-2" (SEQ ID No:107) or a functionally active derivative thereof, and a protein complex selected from complex (III) and comprising the following proteins

   (i) "HSP90 α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions,
   (ii) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,

   (i) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (iv) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (v) "PP1-γ CATALYTIC SUBUNIT" (SEQ ID No:109) or a functionally active derivative thereof, or a functionally active fragment thereof, or a homolog thereof, or a variant of "PP1-γ CATALYTIC SUBUNIT" encoded by a nucleic acid that hybridizes to the "PP1-γ CATALYTIC SUBUNIT" nucleic acid under low stringency conditions,
   (vi) "WUGSC:H_DJ0687K01.2 PROTEIN" (SEQ ID No:110) or a functionally active derivative thereof, or a functionally active fragment thereof, or a homolog thereof, or a variant of "WUGSC:H_DJ0687K01.2 PROTEIN" encoded by a nucleic acid that hybridizes to the "WUGSC:H_DJ0687K01.2 PROTEIN" nucleic acid under low stringency conditions,

   (i) "GUANINE NUCLEOTIDE-BINDING PROTEIN G(I)/G(S)/G(O) γ-12 SUBUNIT" (SEQ ID No:112) or a functionally active derivative thereof, or a functionally active fragment thereof, or a homolog thereof, or a variant of "GUANINE NUCLEOTIDE-BINDING PROTEIN G(I)/G(S)/G(O) y-12 SUBUNIT" encoded by a nucleic acid that hybridizes to the "GUANINE NUCLEOTIDE-BINDING PROTEIN G(I)/G(S)/G(O) γ-12 SUBUNIT" nucleic acid under low stringency conditions,
   (viii) "CamKII δ" (SEQ ID No:113) or a functionally active derivative thereof, or a functionally active fragment thereof, or a homolog thereof, or a variant of "CamKII δ" encoded by a nucleic acid that hybridizes to the "CamKII δ" nucleic acid under low stringency conditions,
   (ix) "PP1-β CATAL SUBUNIT" (SEQ ID No:114) or a functionally active derivative thereof, or a functionally active fragment thereof, or a homolog thereof, or a variant of "PP1-β CATAL SUBUNIT" encoded by a nucleic acid that hybridizes to the "PP1-B CATAL SUBUNIT" nucleic acid under low stringency conditions,
   (x) "TANK BINDING KINASE TBK1" (SEQ ID No:115) or a functionally active derivative thereof, or a functionally active fragment thereof, or a homolog thereof, or a variant of "TANK BINDING KINASE TBK1" encoded by a nucleic acid that hybridizes to the "TANK BINDING KINASE TBK1" nucleic acid under low stringency conditions,
   (xi) "CSD-SAP" (SEQ ID No:116) or a functionally active derivative thereof, or a functionally active fragment thereof, or a homolog thereof, or a variant of "CSD-SAP" encoded by a nucleic acid that hybridizes to the "CSD-SAP" nucleic acid under low stringency conditions,
   (xii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions, and
   (xiii) "Hsp90 β-2" (SEQ ID No:107) or a functionally active derivative thereof, or a functionally active fragment thereof, or a homolog thereof, or a variant of "Hsp90 β-2" encoded by a nucleic acid that hybridizes to the "Hsp90 β-2" nucleic acid under low stringency conditions;
   and a protein complex selected from complex (IV) and comprising the following proteins
   (i) "HSP90 α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions,
   (ii) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (iii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (iv) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (v) "PP1-γ CATALYTIC SUBUNIT" (SEQ ID No: 109) or a functionally active derivative thereof, or a functionally active fragment thereof, or a homolog thereof, or a variant of "PP1-γ CATALYTIC SUBUNIT" encoded by a nucleic acid that hybridizes to the "PP1-γ CATALYTIC SUBUNIT" nucleic acid under low stringency conditions,
   (vi) "WUGSC:H_DJ0687K01.2 PROTEIN" (SEQ ID No:110) or a functionally active derivative thereof, or a functionally active fragment thereof, or a homolog thereof, or a variant of "WUGSC:H_DJ0687K01.2 PROTEIN" encoded by a nucleic acid that hybridizes to the "WUGSC:H_DJ0687K01.2 PROTEIN" nucleic acid under low stringency conditions,
   (vii) "PROTEIN ARGININE N-METHYLTRANSFERASE 1" (SEQ ID No:111) or a functionally active derivative thereof, or a functionally active fragment thereof, or a homolog thereof, or a variant of "PROTEIN ARGININE N-METHYLTRANSFERASE 1" encoded by a nucleic acid that hybridizes to the "PROTEIN ARGININE N-METHYLTRANSFERASE 1" nucleic acid under low stringency conditions,
   (viii) "GUANINE NUCLEOTIDE-BINDING PROTEIN G(I)/G(S)/G(O) γ-12 SUBUNIT" (SEQ ID No:112) or a functionally active derivative thereof, or a functionally active fragment thereof, or a homolog thereof, or a variant of "GUANINE NUCLEOTIDE-BINDING PROTEIN G(I)/G(S)/G(O) y-12 SUBUNIT" encoded by a nucleic acid that hybridizes to the "GUANINE NUCLEOTIDE-BINDING PROTEIN G(I)/G(S)/G(O) y-12 SUBUNIT" nucleic acid under low stringency conditions,
   (ix) "CamKII δ" (SEQ ID No:113) or a functionally active derivative thereof, or a functionally active fragment thereof, or a homolog thereof, or a variant of "CamKII δ" encoded by a nucleic acid that hybridizes to the "CamKII δ" nucleic acid under low stringency conditions,
   (x) "PP1-β CATAL SUBUNIT" (SEQ ID No:114) or a functionally active derivative thereof, or a functionally active fragment thereof, or a homolog thereof, or a variant of "PP1-β CATAL SUBUNIT" encoded by a nucleic acid that hybridizes to the "PP1-β CATAL SUBUNIT" nucleic acid under low stringency conditions,
   (xi) "TANK BINDING KINASE TBK1" (SEQ ID No:115) or a functionally active derivative thereof, or a functionally active fragment thereof, or a homolog thereof, or a variant of "TANK BINDING KINASE TBK1" encoded by a nucleic acid that hybridizes to the "TANK BINDING KINASE TBK1" nucleic acid under low stringency conditions,
   (xii) "CSD-SAP" (SEQ ID No:116) or a functionally active derivative thereof, or a functionally active fragment thereof, or a homolog thereof, or a variant of "CSD-SAP" encoded by a nucleic acid that hybridizes to the "CSD-SAP" nucleic acid under low stringency conditions,
   (xiii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions, and
   (xiv) "Hsp90 β-2" (SEQ ID No:107) or a functionally active derivative thereof, or a functionally active fragment thereof, or a homolog thereof, or a variant of "Hsp90 β-2" encoded by a nucleic acid that hybridizes to the "Hsp90 β-2" nucleic acid under low stringency conditions.
4. The protein complex according to No. 1 selected from complex (I) comprising all but 1 - 7 of the following proteins:
   (i) "HSP90 α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions,
   (ii) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (iii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (iv) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (v) "PP1-γ CATALYTIC SUBUNIT" (SEQ ID No:109) or a functionally active derivative thereof, or a functionally active fragment thereof, or a homolog thereof, or a variant of "PP1-γ CATALYTIC SUBUNIT" encoded by a nucleic acid that hybridizes to the "PP1-γ CATALYTIC SUBUNIT" nucleic acid under low stringency conditions,
   (vi) "WUGSC:H_DJ0687K01.2 PROTEIN" (SEQ ID No:110) or a functionally active derivative thereof, or a functionally active fragment thereof, or a homolog thereof, or a variant of "WUGSC:H_DJ0687K01.2 PROTEIN" encoded by a nucleic acid that hybridizes to the "WUGSC:H_DJ0687K01.2 PROTEIN" nucleic acid under low stringency conditions,
   (vii) "PROTEIN ARGININE N-METHYLTRANSFERASE 1" (SEQ ID No:111) or a functionally active derivative thereof, or a functionally active fragment thereof, or a homolog thereof, or a variant of "PROTEIN ARGININE N-METHYLTRANSFERASE 1" encoded by a nucleic acid that hybridizes to the "PROTEIN ARGININE N-METHYLTRANSFERASE 1" nucleic acid under low stringency conditions,
   (viii) "GUANINE NUCLEOTIDE-BINDING PROTEIN G(I)/G(S)/G(O) γ-12 SUBUNIT" (SEQ ID No:112) or a functionally active derivative thereof, or a functionally active fragment thereof, or a homolog thereof, or a variant of "GUANINE NUCLEOTIDE-BINDING PROTEIN G(I)/G(S)/G(O) γ-12 SUBUNIT" encoded by a nucleic acid that hybridizes to the "GUANINE NUCLEOTIDE-BINDING PROTEIN G(I)/G(S)lG(O) γ-12 SUBUNIT" nucleic acid under low stringency conditions,
   (ix) "CamKII δ" (SEQ ID No:113) or a functionally active derivative thereof, or a functionally active fragment thereof, or a homolog thereof, or a variant of "CamKII δ" encoded by a nucleic acid that hybridizes to the "CamKII δ" nucleic acid under low stringency conditions,
   (x) "PP1-P CATAL SUBUNIT" (SEQ ID No:114) or a functionally active derivative thereof, or a functionally active fragment thereof, or a homolog thereof, or a variant of "PP1-β CATAL SUBUNIT" encoded by a nucleic acid that hybridizes to the "PP1-β CATAL SUBUNIT" nucleic acid under low stringency conditions,
   (xi) "TANK BINDING KINASE TBK1" (SEQ ID No:115) or a functionally active derivative thereof, or a functionally active fragment thereof, or a homolog thereof, or a variant of "TANK BINDING KINASE TBK1" encoded by a nucleic acid that hybridizes to the "TANK BINDING KINASE TBK1" nucleic acid under low stringency conditions,
   (xii) "CSD-SAP" (SEQ ID No:116) or a functionally active derivative thereof, or a functionally active fragment thereof, or a homolog thereof, or a variant of "CSD-SAP" encoded by a nucleic acid that hybridizes to the "CSD-SAP" nucleic acid under low stringency conditions,
   (xiii) "IKK γ" (SEQ ID No:23) or a functionally active derivative thereof,or a functionally active fragement thereof, or a homolog thereof, or a variant of "IKK γ" that hybridizes to the "IKK γ" nucleic acid under low stringency conditions, and
   (xiv) "HSP90 β 2" (SEQ ID No:107) or a functionally active derivative thereof, or a functionally active fragment thereof, or a homolog thereof, or a variant of "Hsp90 β-2" encoded by a nucleic acid that hybridizes to the "Hsp90 β-2" nucleic acid under low stringency conditions.
5. The complex of any of No. 1 - 4 comprising a functionally active derivative of said first protein and/or a functionally active derivative of said second protein, wherein the functionally active derivative is a fusion protein comprising said first protein or said second protein fused to an amino acid sequence different from the first protein or second protein, respectively.
6. The complex of No. 5 wherein the functionally active derivative is a fusion protein comprising said first protein or said second protein fused to an affinity tag or label.
7. The complex of any of No. 1 - 4 comprising a fragment of said first protein and/or a fragment of said second protein, which fragment binds to another protein component of said complex.
8. The complex of any of No. 1 -7 that is involved in the IKK kinase activity or IKK recruitment to TNFR1.
9. A process for preparing complex of any of No. 1 - 8 and optionally the components thereof comprising the following steps:
   the expressing a protein (bait) of the complex, preferably a tagged protein, in a target cell, isolating the protein complex which is attached to the bait protein, and optionally disassociating the protein complex and isolating the individual complex members.
10. The process according to No. 9 wherein the tagged protein comprises two different tags which allow two separate affinity purification steps.
11. The process according to any of No. 9 - 10 wherein the two tags are separated by a cleavage site for a protease.
12. Component of the IKK γ protein complex obtainable by a process according to any of No. 9 to 11.
13. Protein of the IKK γ protein complex selected from
   (i) "CSD-SAP" (SEQ ID No:116) or a functionally active derivative thereof, or a functionally active fragment thereof, or a homolog thereof, or a variant of "CSD-SAP" encoded by a nucleic acid that hybridizes to the "CSD-SAP" nucleic acid under low stringency conditions,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
14. Nucleic acid encoding a protein according to No. 13.
15. Construct, preferably a vector construct, comprising (a) a nucleic acid according to No. 14 and at least one further nucleic acid which is normally not associated with said nucleic acid,or(b) at least two separate nucleic acid sequences each encoding a different protein, or a functionally active fragment or a functionally active derivative thereof at least one of said proteins, or functionally active fragments or functionally active derivative thereof being selected from the first group of proteins according to No. 1(a) and at least one of said proteins, or functionally active fragments or functionally active derivative thereof being selected from the second group of proteins according to No. 1(b).
16. Host cell, containing a vector comprising at least one of the nucleic acid of No. 14 and/or a construct of No. 15 or containing several vectors each comprising at least the nucleic acid sequence encoding at least one of the proteins, or functionally active fragments or functionally active derivatives thereof selected from the first group of proteins according to No. 1(a) and the proteins, or functionally active fragments or functionally active derivatives thereof selected from the second group of proteins according to No. 1(b).
17. An antibody or a fragment of said antibody containing the binding domain thereof, selected from an antibody or fragment thereof, which binds the complex of any of No. 1-8 and which does not bind any of the proteins of said complex when uncomplexed and an antibody or a fragment of said antibody which binds to any of the proteins according to No. 13.
18. A kit comprising in one or more container the complex of any of No. 1 - 8 and/or the proteins of No. 13 optionally together with an antibody according to No. 17 and/or further components such as reagents and working instructions.
19. The kit according to No. 18 for processing a substrate of said complex.
20. The kit according to No. 18 for the diagnosis or prognosis of a disease or a disease risk, preferentially for a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
21. Array, in which at least a complex according to any of No. 1 - 8 and/or at least one protein according to No. 14 and/or at least one antibody according to No. 17 is attached to a solid carrier.
22. A process for processing a physiological substrate of the complex comprising the step of bringing into contact a complex to any of No. 1-8 with said substrate, such that said substrate is processed.
23. A pharmaceutical composition comprising the protein complex of any of No. 1 to 8 and/or any of the following the proteins:
   (i) "CSD-SAP" (SEQ ID No:116) or a functionally active derivative thereof, or a functionally active fragment thereof, or a homolog thereof, or a variant of "CSD-SAP" encoded by a nucleic acid that hybridizes to the "CSD-SAP" nucleic acid under low stringency conditions,
   and a pharmaceutical acceptable carrier.
24. The pharmaceutical composition according to No. 23 for the treatment of diseases and disorders such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
25. A method for screening for a molecule that binds to the complex of anyone of No. 1 - 8 and/or any of the following proteins:
   (i) "CSD-SAP" (SEQ ID No:116) or a functionally active derivative thereof, or a functionally active fragment thereof, or a homolog thereof, or a variant of "CSD-SAP" encoded by a nucleic acid that hybridizes to the "CSD-SAP" nucleic acid under low stringency conditions, comprising the steps of
      (a) exposing said complex, or a cell or organism containing same to one or more candidate molecules; and
      (b) determinig whether said candidate molecule is bound to the complex or protein.
26. A method for screening for a molecule that modulates directly or indirectly the function, activity, composition or formation of the complex of any one of No. 1 - 8 comprising the steps of (a) exposing said complex, or a cell or organism containing IKK γ protein complex to one or more candidate molecules; and
   (b) determining the amount of activity of protein components of, and/or intracellular localization of, said complex and/or the transcription level of a gene dependent on the complex and/or the abundance and/or activity of a protein or protein complex dependent on the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependent on the complex and/or the abundance and/or activity of a protein or protein complex dependent on the function of the complex and/or product of a gene dependent on the complex in the absence of said candidate molecules indicates that the molecule modulates function, activity or composition of said complex.
27. The method of No. 26, wherein the amount of said complex is determined.
28. The method of No. 26, wherein the activity of said complex is determined.
29. The method of No. 28, wherein said determining step comprises isolating from the cell or organism said complex to produce said isolated complex and contacting said isolated complex in the presence or absence of a candidate molecule with a substrate of said complex and determining the processing of said substrate is modified in the presence of said candidate molecule.
30. The method of No. 26, wherein the amount of the individual protein components of said complex are determined.
31. The method of No. 30, determining step comprises determining whether
   (i) "HSP90 α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions, and/or
   (ii) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions, and/or
   (iii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions, and/or
   (iv) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions, and/or
   (v) "PP1-γ CATALYTIC SUBUNIT" (SEQ ID No:109) or a functionally active derivative thereof, or a functionally active fragment thereof, or a homolog thereof, or a variant of "PP1-γ CATALYTIC SUBUNIT" encoded by a nucleic acid that hybridizes to the "PP1-γ CATALYTIC SUBUNIT" nucleic acid under low stringency conditions, and/or
   (vi) "WUGSC:H_DJ0687K01.2 PROTEIN" (SEQ ID No:110) or a functionally active derivative thereof, or a functionally active fragment thereof, or a homolog thereof, or a variant of "WUGSC:H_DJ0687K01.2 PROTEIN" encoded by a nucleic acid that hybridizes to the "WUGSC:H_DJ0687K01.2 PROTEIN" nucleic acid under low stringency conditions, and/or
   (vii) "PROTEIN ARGININE N-METHYLTRANSFERASE 1" (SEQ ID No:111) or a functionally active derivative thereof, or a functionally active fragment thereof, or a homolog thereof, or a variant of "PROTEIN ARGININE N-METHYLTRANSFERASE 1" encoded by a nucleic acid that hybridizes to the "PROTEIN ARGININE N-METHYLTRANSFERASE 1" nucleic acid under low stringency conditions, and/or
   (viii) "GUANINE NUCLEOTIDE-BINDING PROTEIN G(I)/G(S)/G(O) γ-12 SUBUNIT" (SEQ ID No:112) or a functionally active derivative thereof, or a functionally active fragment thereof, or a homolog thereof, or a variant of "GUANINE NUCLEOTIDE-BINDING PROTEIN G(I)/G(S)/G(O) γ-12 SUBUNIT" encoded by a nucleic acid that hybridizes to the "GUANINE NUCLEOTIDE-BINDING PROTEIN G(I)/G(S)/G(O) γ-12 SUBUNIT" nucleic acid under low stringency conditions, and/or
   (ix) "CamKII δ" (SEQ ID No:113) or a functionally active derivative thereof, or a functionally active fragment thereof, or a homolog thereof, or a variant of "CamKII δ" encoded by a nucleic acid that hybridizes to the "CamKII δ" nucleic acid under low stringency conditions, and/or
   (x) "PP1-β CATAL SUBUNIT" (SEQ ID No:114) or a functionally active derivative thereof, or a functionally active fragment thereof, or a homolog thereof, or a variant of "PP1-β CATAL SUBUNIT" encoded by a nucleic acid that hybridizes to the "PP1-β CATAL SUBUNIT" nucleic acid under low stringency conditions, and/or
   (xi) "TANK BINDING KINASE TBK1" (SEQ ID No:115) or a functionally active derivative thereof, or a functionally active fragment thereof, or a homolog thereof, or a variant of "TANK BINDING KINASE TBK1" encoded by a nucleic acid that hybridizes to the "TANK BINDING KINASE TBK1" nucleic acid under low stringency conditions, and/or
   (xii) "CSD-SAP" (SEQ ID No:116) or a functionally active derivative thereof, or a functionally active fragment thereof, or a homolog thereof, or a variant of "CSD-SAP" encoded by a nucleic acid that hybridizes to the "CSD-SAP" nucleic acid under low stringency conditions, and/or
   (xiii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions, and/or
   (xiv) "HSP90 β 2" (SEQ ID No:107) or a functionally active derivative thereof, or a functionally active fragment thereof, or a homolog thereof, or a variant of "HSP90 β 2" encoded by a nucleic acid that hybridizes to the "HSP90 β 2" nucleic acid under low stringency conditions,
   is present in the complex.
32. The method of any of No. 26 to 31, wherein said method is a method of screening for a drug for treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
33. Use of a molecule that modulates the amount of, activity of, or the protein components of the complex of any one of No. 1 - 8 for the manufacture of a medicament for the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
34. A method for the production of a pharmaceutical composition comprising carrying out the method of any of No. 1 - 8 to identify a molecule that modulates the function, activity, composition or formation of said complex, and further comprising mixing the identified molecule with a pharmaceutically acceptable carrier.
35. A method for diagnosing or screening for the presence of a disease or disorder or a predisposition for developing a disease or disorder in a subject, which disease or disorder is characterized by an aberrant amount of, activity of, or component composition of, or intracellular localization of the complex of any one of the No. 1-8, comprising determining the amount of, activity of, protein components of, and/or intracellular localization of, said complex and/or the transcription level of a gene dependent on the complex and/or the abundance and/or activity of a protein or protein complex dependent on the function of the complex and/or product of a gene dependent on the complex in a comparative sample derived from a subject, wherein a difference in said amount, activity, or protein components of, said complex in an analogous sample from a subject not having the disease or disorder or predisposition indicates the presence in the subject of the disease or disorder or predisposition in the subject.
36. The method of No. 35, wherein the amount of said complex is determined.
37. The method of No. 35, wherein the activity of said complex is determined.
38. The method of No. 37, wherein said determining step comprises isolating from the subject said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
39. The method of No. 35, wherein the amount of the individual protein components of said complex are determined.
40. The method of No. 39, wherein said determining step comprises determining whether
   (i) "HSP90 α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions, and/or
   (ii) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions, and/or
   (iii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions, and/or
   (iv) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions, and/or
   (v) "PP1-γ CATALYTIC SUBUNIT" (SEQ ID No:109) or a functionally active derivative thereof, or a functionally active fragment thereof, or a homolog thereof, or a variant of "PP1-γ CATALYTIC SUBUNIT" encoded by a nucleic acid that hybridizes to the "PP1-γ CATALYTIC SUBUNIT" nucleic acid under low stringency conditions, and/or
   (vi) "WUGSC:H_DJ0687K01.2 PROTEIN" (SEQ ID No:110) or a functionally active derivative thereof, or a functionally active fragment thereof, or a homolog thereof, or a variant of "WUGSC:H_DJ0687K01.2 PROTEIN" encoded by a nucleic acid that hybridizes to the "WUGSC:H_DJ0687K01.2 PROTEIN" nucleic acid under low stringency conditions, and/or
   (vii) "PROTEIN ARGININE N-METHYLTRANSFERASE 1" (SEQ ID No: 111) or a functionally active derivative thereof, or a functionally active fragment thereof, or a homolog thereof, or a variant of "PROTEIN ARGININE N-METHYLTRANSFERASE 1" encoded by a nucleic acid that hybridizes to the "PROTEIN ARGININE N-METHYLTRANSFERASE 1" nucleic acid under low stringency conditions, and/or
   (viii) "GUANINE NUCLEOTIDE-BINDING PROTEIN G(I)/G(S)/G(O) γ-12 SUBUNIT" (SEQ ID No:112) or a functionally active derivative thereof, or a functionally active fragment thereof, or a homolog thereof, or a variant of "GUANINE NUCLEOTIDE-BINDING PROTEIN G(I)/G(S)/G(O) γ-12 SUBUNIT" encoded by a nucleic acid that hybridizes to the "GUANINE NUCLEOTIDE-BINDING PROTEIN G(I)/G(S)/G(O) γ-12 SUBUNIT" nucleic acid under low stringency conditions, and/or
   (ix) "CamKII δ" (SEQ ID No:113) or a functionally active derivative thereof, or a functionally active fragment thereof, or a homolog thereof, or a variant of "CamKII δ" encoded by a nucleic acid that hybridizes to the "CamKII δ" nucleic acid under low stringency conditions, and/or
   (x) "PP1-β CATAL SUBUNIT" (SEQ ID No:114) or a functionally active derivative thereof, or a functionally active fragment thereof, or a homolog thereof, or a variant of "PP1-β CATAL SUBUNIT" encoded by a nucleic acid that hybridizes to the "PP1-β CATAL SUBUNIT" nucleic acid under low stringency conditions, and/or
   (xi) "TANK BINDING KINASE TBK1" (SEQ ID No:115) or a functionally active derivative thereof, or a functionally active fragment thereof, or a homolog thereof, or a variant of "TANK BINDING KINASE TBK1" encoded by a nucleic acid that hybridizes to the "TANK BINDING KINASE TBK1" nucleic acid under low stringency conditions, and/or
   (xii) "CSD-SAP" (SEQ ID No:116) or a functionally active derivative thereof, or a functionally active fragment thereof, or a homolog thereof, or a variant of "CSD-SAP" encoded by a nucleic acid that hybridizes to the "CSD-SAP" nucleic acid under low stringency conditions, and/or
   (xiii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions, and/or
   (xiv) "HSP90 β 2" (SEQ ID No:107) or a functionally active derivative thereof, or a functionally active fragment thereof, or a homolog thereof, or a variant of "HSP90 β 2" encoded by a nucleic acid that hybridizes to the "HSP90 β 2" nucleic acid under low stringency conditions,
   is present in the complex.
41. The complex of any one of No. 1 - 8, or proteins of No. 13 or the antibody or fragment of No. 17, for use in a method of diagnosing a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
42. A method for treating or preventing a disease or disorder characterized by an aberrant amount of, activity or component composition of or intracellular localization of, the complex of anyone of No. 1 - 8, comprising administering to a subject in need of such treatment or prevention a therapeutically effective amount of one or more molecules that modulate the amount of, IKK kinase activity or IKK recruitment to TNFR1, or protein components of, said complex.
43. The method according to No. 42, wherein said disease or disorder involves decreased levels of the amount or activity of said complex.
44. The method according to No. 42 , wherein said disease or disorder involves increased levels of the amount or activity of said complex.
45. Complex of any of No. 1 - 8 and/or protein selected from the following proteins
   (i) "HSP90 α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions,
   (ii) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (iii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (iv) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (v) "PP1-γ CATALYTIC SUBUNIT" (SEQ ID No:109) or a functionally active derivative thereof, or a functionally active fragment thereof, or a homolog thereof, or a variant of "PP1-γ CATALYTIC SUBUNIT" encoded by a nucleic acid that hybridizes to the "PP1-γ CATALYTIC SUBUNIT" nucleic acid under low stringency conditions,
   (vi) "WUGSC:H_DJ0687K01.2 PROTEIN" (SEQ ID No:110) or a functionally active derivative thereof, or a functionally active fragment thereof, or a homolog thereof, or a variant of "WUGSC:H_DJ0687K01.2 PROTEIN" encoded by a nucleic acid that hybridizes to the "WUGSC:H_DJ0687K01.2 PROTEIN" nucleic acid under low stringency conditions,
   (vii) "PROTEIN ARGININE N-METHYLTRANSFERASE 1" (SEQ ID No:111) or a functionally active derivative thereof, or a functionally active fragment thereof, or a homolog thereof, or a variant of "PROTEIN ARGININE N-METHYLTRANSFERASE 1" encoded by a nucleic acid that hybridizes to the "PROTEIN ARGININE N-METHYLTRANSFERASE 1" nucleic acid under low stringency conditions,
   (viii) "GUANINE NUCLEOTIDE-BINDING PROTEIN G(I)/G(S)/G(O)γ-12 SUBUNIT" (SEQ ID No:112) or a functionally active derivative thereof, or a functionally active fragment thereof, or a homolog thereof, or a variant of "GUANINE NUCLEOTIDE-BINDING PROTEIN G(I)/G(S)/G(O) γ-12 SUBUNIT" encoded by a nucleic acid that hybridizes to the "GUANINE NUCLEOTIDE-BINDING PROTEIN G(I)/G(S)/G(O) γ-12 SUBUNIT" nucleic acid under low stringency conditions,
   (ix) "CamKII δ" (SEQ ID No:113) or a functionally active derivative thereof, or a functionally active fragment thereof, or a homolog thereof, or a variant of "CamKII δ" encoded by a nucleic acid that hybridizes to the "CamKII δ" nucleic acid under low stringency conditions,
   (x) "PP1-β CATAL SUBUNIT" (SEQ ID No:114) or a functionally active derivative thereof, or a functionally active fragment thereof, or a homolog thereof, or a variant of "PP1-β CATAL SUBUNIT" encoded by a nucleic acid that hybridizes to the "PP1-β CATAL SUBUNIT" nucleic acid under low stringency conditions,
   (xi) "TANK BINDING KINASE TBK1" (SEQ ID No:115) or a functionally active derivative thereof, or a functionally active fragment thereof, or a homolog thereof, or a variant of "TANK BINDING KINASE TBK1" encoded by a nucleic acid that hybridizes to the "TANK BINDING KINASE TBK1" nucleic acid under low stringency conditions,
   (xii) "CSD-SAP" (SEQ ID No:116) or a functionally active derivative thereof, or a functionally active fragment thereof, or a homolog thereof, or a variant of "CSD-SAP" encoded by a nucleic acid that hybridizes to the "CSD-SAP" nucleic acid under low stringency conditions,
   (xiii) "IKK γ" (SEQ ID No:23) or a functionally active derivative thereof,or a functionary active fragement thereof, or a homolog thereof, or a variant of "IKK γ" that hybridizes to the "IKK γ" nucleic acid under low stringency conditions, and
   (xiv) "HSP90 β2" (SEQ ID No:107) or a functionally active derivative thereof, or a functionally active fragment thereof, or a homolog thereof, or a variant of "HSP90 β2" encoded by a nucleic acid that hybridizes to the "HSP90 β2" nucleic acid under low stringency conditions,
   as a target for an active agent of a pharmaceutical, preferably a drug target in the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.

The present invention further relates to the p38 protein complex
1. A protein complex selected from complex (I) and comprising
   (a) at least one first protein selected from the group consisting of:
      (i) "Cdc25B/C" (SEQ ID No:117) or a functionally active derivative thereof, or a functionally active fragment thereof, or a homolog thereof, or a variant of "Cdc25B/C" encoded by a nucleic acid that hybridizes to the "Cdc25B/C" nucleic acid under low stringency conditions, and
      (ii) "p38" (SEQ ID No:118) or a functionally active derivative thereof, or a functionally active fragment thereof, or a homolog thereof, or a variant of "p38" encoded by a nucleic acid that hybridizes to the "p38" nucleic acid under low stringency conditions,
      and
   (b) at least one second protein, which second protein is selected from the group consisting of:
      (i) "ANKHZN" (SEQ ID No:119) or a functionally active derivative thereof, or a functionally active fragment thereof, or a homolog thereof, or a variant of "ANKHZN" encoded by a nucleic acid that hybridizes to the "ANKHZN" nucleic acid under low stringency conditions,
      (ii) "SIM TO FLJ22604" (SEQ ID No:120) or a functionally active derivative thereof, or a functionally active fragment thereof, or a homolog thereof, or a variant of "SIM TO FLJ22604" encoded by a nucleic acid that hybridizes to the "SIM TO FLJ22604" nucleic acid under low stringency conditions,
      (iii) "PITUITARY-SPECIFIC POSITIVE TRANSCRIPTION FACTOR 1" (SEQ ID No:121) or a functionally active derivative thereof, or a functionally active fragment thereof, or a homolog thereof, or a variant of "PITUITARY-SPECIFIC POSITIVE TRANSCRIPTION FACTOR 1" encoded by a nucleic acid that hybridizes to the "PITUITARY-SPECIFIC POSITIVE TRANSCRIPTION FACTOR 1" nucleic acid under low stringency conditions,
      (iv) "LYSOSOMAL ACID PHOSPHATASE PRECURSOR" (SEQ ID No:122) or a functionally active derivative thereof, or a functionally active fragment thereof, or a homolog thereof, or a variant of "LYSOSOMAL ACID PHOSPHATASE PRECURSOR" encoded by a nucleic acid that hybridizes to the "LYSOSOMAL ACID PHOSPHATASE PRECURSOR" nucleic acid under low stringency conditions,
      (v) "BAT4" (SEQ ID No:123) or a functionally active derivative thereof, or a functionally active fragment thereof, or a homolog thereof, or a variant of "BAT4" encoded by a nucleic acid that hybridizes to the "BAT4" nucleic acid under low stringency conditions,
      (vi) "HES2" (SEQ ID No:124) or a functionally active derivative thereof, or a functionally active fragment thereof, or a homolog thereof, or a variant of "HES2" encoded by a nucleic acid that hybridizes to the "HES2" nucleic acid under low stringency conditions, and
      (vii) "KIAA1147 PROTEIN" (SEQ ID No:125) or a functionally active derivative thereof, or a functionally active fragment thereof, or a homolog thereof, or a variant of "KIAA1147 PROTEIN" encoded by a nucleic acid that hybridizes to the "KIAA1147 PROTEIN" nucleic acid under low stringency conditions;
   and a complex (II) comprising at least two of said second proteins,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
2. The protein complex according to claim 1 wherein the first protein is the protein "p38" (SEQ ID No:118) , or a functionally active derivative thereof, or a functionally active fragment thereof, or a homolog thereof, or a variant of "p38" encoded by a nucleic acid that hybridizes to the "p38" nucleic acid under low stringency conditions.
3. The protein complex according to No. 1 selected from complex (I) and comprising the following proteins:
   (i) "p38" (SEQ ID No:118) or a functionally active derivative thereof, or a functionally active fragment thereof, or a homolog thereof, or a variant of "p38" encoded by a nucleic acid that hybridizes to the "p38" nucleic acid under low stringency conditions,
   (ii) "ANKHZN" (SEQ ID No:119) or a functionally active derivative thereof, or a functionally active fragment thereof, or a homolog thereof, or a variant of "ANKHZN" encoded by a nucleic acid that hybridizes to the "ANKHZN" nucleic acid under low stringency conditions,
   (iii) "SIM TO FLJ22604" (SEQ ID No:120) or a functionally active derivative thereof, or a functionally active fragment thereof, or a homolog thereof, or a variant of "SIM TO FLJ22604" encoded by a nucleic acid that hybridizes to the "SIM TO FLJ22604" nucleic acid under low stringency conditions,
   (iv) "PITUITARY-SPECIFIC POSITIVE TRANSCRIPTION FACTOR 1" (SEQ ID No: 121) or a functionally active derivative thereof, or a functionally active fragment thereof, or a homolog thereof, or a variant of "PITUITARY-SPECIFIC POSITIVE TRANSCRIPTION FACTOR 1" encoded by a nucleic acid that hybridizes to the "PITUITARY-SPECIFIC POSITIVE TRANSCRIPTION FACTOR 1" nucleic acid under low stringency conditions,
   (v) "BAT4" (SEQ ID No:123) or a functionally active derivative thereof, or a functionally active fragment thereof, or a homolog thereof, or a variant of "BAT4" encoded by a nucleic acid that hybridizes to the "BAT4" nucleic acid under low stringency conditions, and
   (vi) "KIAA1147 PROTEIN" (SEQ ID No:125) or a functionally active derivative thereof, or a functionally active fragment thereof, or a homolog thereof, or a variant of "KIAA1147 PROTEIN" encoded by a nucleic acid that hybridizes to the "KIAA1147 PROTEIN" nucleic acid under low stringency conditions,
   and a protein complex selected from complex (II) and comprising the following proteins:
   (i) "p38" (SEQ ID No:118) or a functionally active derivative thereof, or a functionally active fragment thereof, or a homolog thereof, or a variant of "p38" encoded by a nucleic acid that hybridizes to the "p38" nucleic acid under low stringency conditions,
   (ii) "LYSOSOMAL ACID PHOSPHATASE PRECURSOR" (SEQ ID No:122) or a functionally active derivative thereof, or a functionally active fragment thereof, or a homolog thereof, or a variant of "LYSOSOMAL ACID PHOSPHATASE PRECURSOR" encoded by a nucleic acid that hybridizes to the "LYSOSOMAL ACID PHOSPHATASE PRECURSOR" nucleic acid under low stringency conditions, and
   (iii) "HES2" (SEQ ID No:124) or a functionally active derivative thereof, or a functionally active fragment thereof, or a homolog thereof, or a variant of "HES2" encoded by a nucleic acid that hybridizes to the "HES2" nucleic acid under low stringency conditions;
   and a protein complex selected from complex (III) and comprising the following proteins:
   (i) "Cdc25B/C" (SEQ ID No:117) or a functionally active derivative thereof, or a functionally active fragment thereof, or a homolog thereof, or a variant of "Cdc25B/C" encoded by a nucleic acid that hybridizes to the "Cdc25B/C" nucleic acid under low stringency conditions,
   (ii) "p38" (SEQ ID No: 118) or a functionally active derivative thereof, or a functionally active fragment thereof, or a homolog thereof, or a variant of "p38" encoded by a nucleic acid that hybridizes to the "p38" nucleic acid under low stringency conditions,
   (iii) "ANKHZN" (SEQ ID No: 119) or a functionally active derivative thereof, or a functionally active fragment thereof, or a homolog thereof, or a variant of "ANKHZN" encoded by a nucleic acid that hybridizes to the "ANKHZN" nucleic acid under low stringency conditions,
   (iv) "SIM TO FLJ22604" (SEQ ID No:120) or a functionally active derivative thereof, or a functionally active fragment thereof, or a homolog thereof, or a variant of "SIM TO FLJ22604" encoded by a nucleic acid that hybridizes to the "SIM TO FLJ22604" nucleic acid under low stringency conditions,
   (v) "PITUITARY-SPECIFIC POSITIVE TRANSCRIPTION FACTOR 1" (SEQ ID No:121) or a functionally active derivative thereof, or a functionally active fragment thereof, or a homolog thereof, or a variant of "PITUITARY-SPECIFIC POSITIVE TRANSCRIPTION FACTOR 1" encoded by a nucleic acid that hybridizes to the "PITUITARY-SPECIFIC POSITIVE TRANSCRIPTION FACTOR 1" nucleic acid under low stringency conditions,
   (vi) "LYSOSOMAL ACID PHOSPHATASE PRECURSOR" (SEQ ID No:122) or a functionally active derivative thereof, or a functionally active fragment thereof, or a homolog thereof, or a variant of "LYSOSOMAL ACID PHOSPHATASE PRECURSOR" encoded by a nucleic acid that hybridizes to the "LYSOSOMAL ACID PHOSPHATASE PRECURSOR" nucleic acid under low stringency conditions,
   (vii) "BAT4" (SEQ ID No:123) or a functionally active derivative thereof, or a functionally active fragment thereof, or a homolog thereof, or a variant of "BAT4" encoded by a nucleic acid that hybridizes to the "BAT4" nucleic acid under low stringency conditions,
   (viii) "HES2" (SEQ ID No:124) or a functionally active derivative thereof, or a functionally active fragment thereof, or a homolog thereof, or a variant of "HES2" encoded by a nucleic acid that hybridizes to the "HES2" nucleic acid under low stringency conditions, and
   (ix)"KIAA1147 PROTEIN" (SEQ ID No:125) or a functionally active derivative thereof, or a functionally active fragment thereof, or a homolog thereof, or a variant of "KIAA1147 PROTEIN" encoded by a nucleic acid that hybridizes to the "KIAA1147 PROTEIN" nucleic acid under low stringency conditions.
4. The protein complex according to No. 1 selected from complex (I) comprising all but 1 - 6 of the following proteins:
   (i) "Cdc25B/C" (SEQ ID No:117) or a functionally active derivative thereof, or a functionally active fragment thereof, or a homolog thereof, or a variant of "Cdc25B/C" encoded by a nucleic acid that hybridizes to the "Cdc25B/C" nucleic acid under low stringency conditions,
   (ii) "p38" (SEQ ID No:118) or a functionally active derivative thereof, or a functionally active fragment thereof, or a homolog thereof, or a variant of "p38" encoded by a nucleic acid that hybridizes to the "p38" nucleic acid under low stringency conditions,
   (iii) "ANKHZN" (SEQ ID No:119) or a functionally active derivative thereof, or a functionally active fragment thereof, or a homolog thereof, or a variant of "ANKHZN" encoded by a nucleic acid that hybridizes to the "ANKHZN" nucleic acid under low stringency conditions,
   (iv) "SIM TO FLJ22604" (SEQ ID No:120) or a functionally active derivative thereof, or a functionally active fragment thereof, or a homolog thereof, or a variant of "SIM TO FLJ22604" encoded by a nucleic acid that hybridizes to the "SIM TO FLJ22604" nucleic acid under low stringency conditions,
   (v) "PITUITARY-SPECIFIC POSITIVE TRANSCRIPTION FACTOR 1" (SEQ ID No:121) or a functionally active derivative thereof, or a functionally active fragment thereof, or a homolog thereof, or a variant of "PITUITARY-SPECIFIC POSITIVE TRANSCRIPTION FACTOR 1" encoded by a nucleic acid that hybridizes to the "PITUITARY-SPECIFIC POSITIVE TRANSCRIPTION FACTOR 1" nucleic acid under low stringency conditions,
   (vi) "LYSOSOMAL ACID PHOSPHATASE PRECURSOR" (SEQ ID No:122) or a functionally active derivative thereof, or a functionally active fragment thereof, or a homolog thereof, or a variant of "LYSOSOMAL ACID PHOSPHATASE PRECURSOR" encoded by a nucleic acid that hybridizes to the "LYSOSOMAL ACID PHOSPHATASE PRECURSOR" nucleic acid under low stringency conditions,
   (vii) "BAT4" (SEQ ID No:123) or a functionally active derivative thereof, or a functionally active fragment thereof, or a homolog thereof, or a variant of "BAT4" encoded by a nucleic acid that hybridizes to the "BAT4" nucleic acid under low stringency conditions,
   (viii) "HES2" (SEQ ID No:124) or a functionally active derivative thereof,or a functionally active fragement thereof, or a homolog thereof, or a variant of "HES2" that hybridizes to the "HES2" nucleic acid under low stringency conditions, and
   (ix) "KIAA1147 PROTEIN" (SEQ ID No:125) or a functionally active derivative thereof, or a functionally active fragment thereof, or a homolog thereof, or a variant of "KIAA1147 PROTEIN" encoded by a nucleic acid that hybridizes to the "KIAA1147 PROTEIN" nucleic acid under low stringency conditions;
   and a protein complex selected from complex (II) that comprises all but 1 - 6 of the following proteins:
   (i) "p38" (SEQ ID No:118) or a functionally active derivative thereof, or a functionally active fragment thereof, or a homolog thereof, or a variant of "p38" encoded by a nucleic acid that hybridizes to the "p38" nucleic acid under low stringency conditions,
   (ii) "ANKHZN" (SEQ ID No:119) or a functionally active derivative thereof, or a functionally active fragment thereof, or a homolog thereof, or a variant of "ANKHZN" encoded by a nucleic acid that hybridizes to the "ANKHZN" nucleic acid under low stringency conditions,
   (iii) "SIM TO FLJ22604" (SEQ ID No:120) or a functionally active derivative thereof, or a functionally active fragment thereof, or a homolog thereof, or a variant of "SIM TO FLJ22604" encoded by a nucleic acid that hybridizes to the "SIM TO FLJ22604" nucleic acid under low stringency conditions,
   (iv) "PITUITARY-SPECIFIC POSITIVE TRANSCRIPTION FACTOR 1" (SEQ ID No:121) or a functionally active derivative thereof, or a functionally active fragment thereof, or a homolog thereof, or a variant of "PITUITARY-SPECIFIC POSITIVE TRANSCRIPTION FACTOR 1" encoded by a nucleic acid that hybridizes to the "PITUITARY-SPECIFIC POSITIVE TRANSCRIPTION FACTOR 1" nucleic acid under low stringency conditions,
   (v) "LYSOSOMAL ACID PHOSPHATASE PRECURSOR" (SEQ ID No:122) or a functionally active derivative thereof, or a functionally active fragment thereof, or a homolog thereof, or a variant of "LYSOSOMAL ACID PHOSPHATASE PRECURSOR" encoded by a nucleic acid that hybridizes to the "LYSOSOMAL ACID PHOSPHATASE PRECURSOR" nucleic acid under low stringency conditions,
   (vi) "BAT4" (SEQ ID No:123) or a functionally active derivative thereof, or a functionally active fragment thereof, or a homolog thereof, or a variant of "BAT4" encoded by a nucleic acid that hybridizes to the "BAT4" nucleic acid under low stringency conditions,
   (vii) "HES2" (SEQ ID No:124) or a functionally active derivative thereof,or a functionally active fragement thereof, or a homolog thereof, or a variant of "HES2" that hybridizes to the "HES2" nucleic acid under low stringency conditions, and
   (viii) "KIAA1147 PROTEIN" (SEQ ID No:125) or a functionally active derivative thereof,or a functionally active fragement thereof, or a homolog thereof, or a variant of "KIAA1147 PROTEIN" that hybridizes to the "KIAA1147 PROTEIN" nucleic acid under low stringency conditions.
5. The complex of any of No. 1 - 4 comprising a functionally active derivative of said first protein and/or a functionally active derivative of said second protein, wherein the functionally active derivative is a fusion protein comprising said first protein or said second protein fused to an amino acid sequence different from the first protein or second protein, respectively.
6. The complex of No. 5 wherein the functionally active derivative is a fusion protein comprising said first protein or said second protein fused to an affinity tag or label.
7. The complex of any of No. 1 - 4 comprising a fragment of said first protein and/or a fragment of said second protein, which fragment binds to another protein component of said complex.
8. The complex of any of No. 1 -7 that is involved in the in vitro kinase activity.
9. A process for preparing complex of any of No. 1 - 8 and optionally the components thereof comprising the following steps:
   the expressing a protein (bait) of the complex, preferably a tagged protein, in a target cell, isolating the protein complex which is attached to the bait protein, and optionally disassociating the protein complex and isolating the individual complex members.
10. The process according to No. 9 wherein the tagged protein comprises two different tags which allow two separate affinity purification steps.
11. The process according to any of No. 9 - 10 wherein the two tags are separated by a cleavage site for a protease.
12. Component of the p38 complex obtainable by a process according to any of No. 9 to 11.
13. Protein of the p38 complex selected from
   (i) "ANKHZN" (SEQ ID No:119) or a functionally active derivative thereof, or a functionally active fragment thereof, or a homolog thereof, or a variant of "ANKHZN" encoded by a nucleic acid that hybridizes to the "ANKHZN" nucleic acid under low stringency conditions,
   (ii) "SIM TO FLJ22604" (SEQ ID No: 120) or a functionally active derivative thereof, or a functionally active fragment thereof, or a homolog thereof, or a variant of "SIM TO FLJ22604" encoded by a nucleic acid that hybridizes to the "SIM TO FLJ22604" nucleic acid under low stringency conditions, and
   (iii) "KIAA1147 PROTEIN" (SEQ ID No:125) or a functionally active derivative thereof, or a functionally active fragment thereof, or a homolog thereof, or a variant of "KIAA1147 PROTEIN" encoded by a nucleic acid that hybridizes to the "KIAA1147 PROTEIN" nucleic acid under low stringency conditions,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1 % SDS for 1.5 hours at 60°C.
14. Nucleic acid encoding a protein according to No. 13.
15. Construct, preferably a vector construct, comprising (a) a nucleic acid according to No. 14 and at least one further nucleic acid which is normally not associated with said nucleic acid,or(b) at least two separate nucleic acid sequences each encoding a different protein, or a functionally active fragment or a functionally active derivative thereof at least one of said proteins, or functionally active fragments or functionally active derivative thereof being selected from the first group of proteins according to No. 1(a) and at least one of said proteins, or functionally active fragments or functionally active derivative thereof being selected from the second group of proteins according to No. 1(b).
16. Host cell, containing a vector comprising at least one of the nucleic acid of No. 14 and/or a construct of No. 15 or containing several vectors each comprising at least the nucleic acid sequence encoding at least one of the proteins, or functionally active fragments or functionally active derivatives thereof selected from the first group of proteins according to No. 1(a) and the proteins, or functionally active fragments or functionally active derivatives thereof selected from the second group of proteins according to No. 1(b).
17. An antibody or a fragment of said antibody containing the binding domain thereof, selected from an antibody or fragment thereof, which binds the complex of any of No. 1-8 and which does not bind any of the proteins of said complex when uncomplexed and an antibody or a fragment of said antibody which binds to any of the proteins according to No. 13.
18. A kit comprising in one or more container the complex of any of No. 1 - 8 and/or the proteins of No. 13optionally together with an antibody according to No. 17 and/or further components such as reagents and working instructions.
19. The kit according to No.18 for processing a substrate of said complex.
20. The kit according to No. 18 for the diagnosis or prognosis of a disease or a disease risk, preferentially for a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
21. Array, in which at least a complex according to any of No. 1-8 and/or at least one protein according to No. 14 and/or at least one antibody according to No. 17 is attached to a solid carrier.
22. A process for processing a physiological substrate of the complex comprising the step of bringing into contact a complex to any of No. 1 - 8 with said substrate, such that said substrate is processed.
23. A pharmaceutical composition comprising the protein complex of any of No. 1 to 8 and/or any of the following the proteins:
   (i) "ANKHZN" (SEQ ID No:119) or a functionally active derivative thereof, or a functionally active fragment thereof, or a homolog thereof, or a variant of "ANKHZN" encoded by a nucleic acid that hybridizes to the "ANKHZN" nucleic acid under low stringency conditions,
   (ii) "SIM TO FLJ22604" (SEQ ID No:120) or a functionally active derivative thereof,or a functionally active fragement thereof, or a homolog thereof, or a variant of "SIM TO FLJ22604" that hybridizes to the "SIM TO FLJ22604" nucleic acid under low stringency conditions, and
   (iii) "KIAA1147 PROTEIN" (SEQ ID No:125) or a functionally active derivative thereof, or a functionally active fragment thereof, or a homolog thereof, or a variant of "KIAA1147 PROTEIN" encoded by a nucleic acid that hybridizes to the "KIAA1147 PROTEIN" nucleic acid under low stringency conditions,
   and a pharmaceutical acceptable carrier.
24. The pharmaceutical composition according to No. 23 for the treatment of diseases and disorders such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
25. A method for screening for a molecule that binds to the complex of anyone of No. 1 - 8 and/or any of the following the proteins:
   (i) "ANKHZN" (SEQ ID No:119) or a functionally active derivative thereof, or a functionally active fragment thereof, or a homolog thereof, or a variant of "ANKHZN" encoded by a nucleic acid that hybridizes to the "ANKHZN" nucleic acid under low stringency conditions,
   (ii) "SIM TO FLJ22604" (SEQ ID No:120) or a functionally active derivative thereof,or a functionally active fragement thereof, or a homolog thereof, or a variant of "SIM TO FLJ22604" that hybridizes to the "SIM TO FLJ22604" nucleic acid under low stringency conditions, and
   (iii) "KIAA1147 PROTEIN" (SEQ ID No:125) or a functionally active derivative thereof, or a functionally active fragment thereof, or a homolog thereof, or a variant of "KIAA1147 PROTEIN" encoded by a nucleic acid that hybridizes to the "KIAA1147 PROTEIN" nucleic acid under low stringency conditions, comprising the steps of
      (a) exposing said complex, or a cell or organism containing same to one or more candidate molecules; and
      (b) determinig whether said candidate molecule is bound to the complex or protein.
26. A method for screening for a molecule that modulates directly or indirectly the function, activity, composition or formation of the complex of any one of No. 1-8 comprising the steps of (a) exposing said complex, or a cell or organism containing p38 complex to one or more candidate molecules; and
   (b) determining the amount of activity of protein components of, and/or intracellular localization of, said complex and/or the transcription level of a gene dependent on the complex and/or the abundance and/or activity of a protein or protein complex dependent on the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependent on the complex and/or the abundance and/or activity of a protein or protein complex dependent on the function of the complex and/or product of a gene dependent on the complex in the absence of said candidate molecules indicates that the molecule modulates function, activity or composition of said complex.
27. The method of No. 26, wherein the amount of said complex is determined.
28. The method of No. 26, wherein the activity of said complex is determined.
29. The method of No. 28, wherein said determining step comprises isolating from the cell or organism said complex to produce said isolated complex and contacting said isolated complex in the presence or absence of a candidate molecule with a substrate of said complex and determining the processing of said substrate is modified in the presence of said candidate molecule.
30. The method of No. 26, wherein the amount of the individual protein components of said complex are determined.
31. The method of No. 30, determining step comprises determining whether
   (i) "Cdc25B/C" (SEQ ID No:117) or a functionally active derivative thereof, or a functionally active fragment thereof, or a homolog thereof, or a variant of "Cdc25B/C" encoded by a nucleic acid that hybridizes to the "Cdc25B/C" nucleic acid under low stringency conditions, and/or
   (ii) "p38" (SEQ ID No: 118) or a functionally active derivative thereof, or a functionally active fragment thereof, or a homolog thereof, or a variant of "p38" encoded by a nucleic acid that hybridizes to the "p38" nucleic acid under low stringency conditions, and/or
   (iii) "ANKHZN" (SEQ ID No:119) or a functionally active derivative thereof, or a functionally active fragment thereof, or a homolog thereof, or a variant of "ANKHZN" encoded by a nucleic acid that hybridizes to the "ANKHZN" nucleic acid under low stringency conditions, and/or
   (iv) "SIM TO FLJ22604" (SEQ ID No:120) or a functionally active derivative thereof, or a functionally active fragment thereof, or a homolog thereof, or a variant of "SIM TO FLJ22604" encoded by a nucleic acid that hybridizes to the "SIM TO FLJ22604" nucleic acid under low stringency conditions, and/or
   (v) "PITUITARY-SPECIFIC POSITIVE TRANSCRIPTION FACTOR 1" (SEQ ID No:121) or a functionally active derivative thereof, or a functionally active fragment thereof, or a homolog thereof, or a variant of "PITUITARY-SPECIFIC POSITIVE TRANSCRIPTION FACTOR 1" encoded by a nucleic acid that hybridizes to the "PITUITARY-SPECIFIC POSITIVE TRANSCRIPTION FACTOR 1" nucleic acid under low stringency conditions, and/or
   (vi) "LYSOSOMAL ACID PHOSPHATASE PRECURSOR" (SEQ ID No:122) or a functionally active derivative thereof, or a functionally active fragment thereof, or a homolog thereof, or a variant of "LYSOSOMAL ACID PHOSPHATASE PRECURSOR" encoded by a nucleic acid that hybridizes to the "LYSOSOMAL ACID PHOSPHATASE PRECURSOR" nucleic acid under low stringency conditions, and/or
   (vii) "BAT4" (SEQ ID No:123) or a functionally active derivative thereof, or a functionally active fragment thereof, or a homolog thereof, or a variant of "BAT4" encoded by a nucleic acid that hybridizes to the "BAT4" nucleic acid under low stringency conditions, and/or
   (viii) "HES2" (SEQ ID No:124) or a functionally active derivative thereof, or a functionally active fragment thereof, or a homolog thereof, or a variant of "HES2" encoded by a nucleic acid that hybridizes to the "HES2" nucleic acid under low stringency conditions, and/or
   (ix) "KIAA1147 PROTEIN" (SEQ ID No:125) or a functionally active derivative thereof, or a functionally active fragment thereof, or a homolog thereof, or a variant of "KIAA1147 PROTEIN" encoded by a nucleic acid that hybridizes to the "KIAA1147 PROTEIN" nucleic acid under low stringency conditions,
   is present in the complex.
32. The method of any of No. 26 to 31, wherein said method is a method of screening for a drug for treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
33. Use of a molecule that modulates the amount of, activity of, or the protein components of the complex of any one of No. 1 - 8 for the manufacture of a medicament for the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
34. A method for the production of a pharmaceutical composition comprising carrying out the method of any of No. 1 - 8 to identify a molecule that modulates the function, activity, composition or formation of said complex, and further comprising mixing the identified molecule with a pharmaceutically acceptable carrier.
35. A method for diagnosing or screening for the presence of a disease or disorder or a predisposition for developing a disease or disorder in a subject, which disease or disorder is characterized by an aberrant amount of, activity of, or component composition of, or intracellular localization of the complex of any one of the No. 1 - 8, comprising determining the amount of, activity of, protein components of, and/or intracellular localization of, said complex and/or the transcription level of a gene dependent on the complex and/or the abundance and/or activity of a protein or protein complex dependent on the function of the complex and/or product of a gene dependent on the complex in a comparative sample derived from a subject, wherein a difference in said amount, activity, or protein components of, said complex in an analogous sample from a subject not having the disease or disorder or predisposition indicates the presence in the subject of the disease or disorder or predisposition in the subject.
36. The method of No. 35, wherein the amount of said complex is determined.
37. The method of No. 35, wherein the activity of said complex is determined.
38. The method of No. 37, wherein said determining step comprises isolating from the subject said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
39. The method of No. 35, wherein the amount of the individual protein components of said complex are determined.
40. The method of No. 39, wherein said determining step comprises determining whether
   (i) "Cdc25B/C" (SEQ ID No:117) or a functionally active derivative thereof, or a functionally active fragment thereof, or a homolog thereof, or a variant of "Cdc25B/C" encoded by a nucleic acid that hybridizes to the "Cdc25B/C" nucleic acid under low stringency conditions, and/or
   (ii) "p38" (SEQ ID No:118) or a functionally active derivative thereof, or a functionally active fragment thereof, or a homolog thereof, or a variant of "p38" encoded by a nucleic acid that hybridizes to the "p38" nucleic acid under low stringency conditions, and/or
   (iii) "ANKHZN" (SEQ ID No:119) or a functionally active derivative thereof, or a functionally active fragment thereof, or a homolog thereof, or a variant of "ANKHZN" encoded by a nucleic acid that hybridizes to the "ANKHZN" nucleic acid under low stringency conditions, and/or
   (iv) "SIM TO FLJ22604" (SEQ ID No:120) or a functionally active derivative thereof, or a functionally active fragment thereof, or a homolog thereof, or a variant of "SIM TO FLJ22604" encoded by a nucleic acid that hybridizes to the "SIM TO FLJ22604" nucleic acid under low stringency conditions, and/or
   (v) "PITUITARY-SPECIFIC POSITIVE TRANSCRIPTION FACTOR 1" (SEQ ID No:121) or a functionally active derivative thereof, or a functionally active fragment thereof, or a homolog thereof, or a variant of "PITUITARY-SPECIFIC POSITIVE TRANSCRIPTION FACTOR 1" encoded by a nucleic acid that hybridizes to the "PITUITARY-SPECIFIC POSITIVE TRANSCRIPTION FACTOR 1" nucleic acid under low stringency conditions, and/or
   (vi) "LYSOSOMAL ACID PHOSPHATASE PRECURSOR" (SEQ ID No: 122) or a functionally active derivative thereof, or a functionally active fragment thereof, or a homolog thereof, or a variant of "LYSOSOMAL ACID PHOSPHATASE PRECURSOR" encoded by a nucleic acid that hybridizes to the "LYSOSOMAL ACID PHOSPHATASE PRECURSOR" nucleic acid under low stringency conditions, and/or
   (vii) "BAT4" (SEQ ID No:123) or a functionally active derivative thereof, or a functionally active fragment thereof, or a homolog thereof, or a variant of "BAT4" encoded by a nucleic acid that hybridizes to the "BAT4" nucleic acid under low stringency conditions, and/or
   (viii) "HES2" (SEQ ID No:124) or a functionally active derivative thereof, or a functionally active fragment thereof, or a homolog thereof, or a variant of "HES2" encoded by a nucleic acid that hybridizes to the "HES2" nucleic acid under low stringency conditions, and/or
   (ix) "KIAA1147 PROTEIN" (SEQ ID No:125) or a functionally active derivative thereof, or a functionally active fragment thereof, or a homolog thereof, or a variant of "KIAA1147 PROTEIN" encoded by a nucleic acid that hybridizes to the "KIAA1147 PROTEIN" nucleic acid under low stringency conditions,
   is present in the complex.
41. The complex of any one of No. 1 - 8, or proteins of No. 13 or the antibody or fragment of No. 17, for use in a method of diagnosing a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
42. A method for treating or preventing a disease or disorder characterized by an aberrant amount of, activity or component composition of or intracellular localization of, the complex of anyone of No. 1 - 8, comprising administering to a subject in need of such treatment or prevention a therapeutically effective amount of one or more molecules that modulate the amount of, in vitro kinase activity, or protein components of, said complex.
43. The method according to No. 42, wherein said disease or disorder involves decreased levels of the amount or activity of said complex.
44. The method according to No. 42 , wherein said disease or disorder involves increased levels of the amount or activity of said complex.
45. Complex of any of No. 1 - 8 and/or protein selected from the following proteins
   (i) "Cdc25B/C" (SEQ ID No:117) or a functionally active derivative thereof, or a functionally active fragment thereof, or a homolog thereof, or a variant of "Cdc25B/C" encoded by a nucleic acid that hybridizes to the "Cdc25B/C" nucleic acid under low stringency conditions,
   (ii) "p38" (SEQ ID No:118) or a functionally active derivative thereof, or a functionally active fragment thereof, or a homolog thereof, or a variant of "p38" encoded by a nucleic acid that hybridizes to the "p38" nucleic acid under low stringency conditions,
   (iii) "ANKHZN" (SEQ ID No:119) or a functionally active derivative thereof, or a functionally active fragment thereof, or a homolog thereof, or a variant of "ANKHZN" encoded by a nucleic acid that hybridizes to the "ANKHZN" nucleic acid under low stringency conditions,
   (iv) "SIM TO FLJ22604" (SEQ ID No:120) or a functionally active derivative thereof, or a functionally active fragment thereof, or a homolog thereof, or a variant of "SIM TO FLJ22604" encoded by a nucleic acid that hybridizes to the "SIM TO FLJ22604" nucleic acid under low stringency conditions,
   (v) "PITUITARY-SPECIFIC POSITIVE TRANSCRIPTION FACTOR 1" (SEQ ID No:121) or a functionally active derivative thereof, or a functionally active fragment thereof, or a homolog thereof, or a variant of "PITUITARY-SPECIFIC POSITIVE TRANSCRIPTION FACTOR 1" encoded by a nucleic acid that hybridizes to the "PITUITARY-SPECIFIC POSITIVE TRANSCRIPTION FACTOR 1" nucleic acid under low stringency conditions,
   (vi) "LYSOSOMAL ACID PHOSPHATASE PRECURSOR" (SEQ ID No:122) or a functionally active derivative thereof, or a functionally active fragment thereof, or a homolog thereof, or a variant of "LYSOSOMAL ACID PHOSPHATASE PRECURSOR" encoded by a nucleic acid that hybridizes to the "LYSOSOMAL ACID PHOSPHATASE PRECURSOR" nucleic acid under low stringency conditions,
   (vii) "BAT4" (SEQ ID No:123) or a functionally active derivative thereof, or a functionally active fragment thereof, or a homolog thereof, or a variant of "BAT4" encoded by a nucleic acid that hybridizes to the "BAT4" nucleic acid under low stringency conditions,
   (viii) "HES2" (SEQ ID No:124) or a functionally active derivative thereof,or a functionally active fragement thereof, or a homolog thereof, or a variant of "HES2" that hybridizes to the "HES2" nucleic acid under low stringency conditions, and
   (ix) "KIAA1147 PROTEIN" (SEQ ID No:125) or a functionally active derivative thereof, or a functionally active fragment thereof, or a homolog thereof, or a variant of "KIAA1147 PROTEIN" encoded by a nucleic acid that hybridizes to the "KIAA1147 PROTEIN" nucleic acid under low stringency conditions,
as a target for an active agent of a pharmaceutical, preferably a drug target in the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.

The present invention further relates to the p105 (NF-κB1) protein complex
1. A protein complex selected from complex (I) and comprising
   (a) at least one first protein selected from the group consisting of:
      (i) "Cot/Tpl2" (SEQ ID No:70) or a functionally active derivative thereof, or a functionally active fragment thereof, or a homolog thereof, or a variant of "Cot/Tpl2" encoded by a nucleic acid that hybridizes to the "Cot/TpI2" nucleic acid under low stringency conditions,
      (ii) "β TrCP2 - 2" (SEQ ID No:128) or a functionally active derivative thereof, or a functionally active fragment thereof, or a homolog thereof, or a variant of "β TrCP2 - 2" encoded by a nucleic acid that hybridizes to the "β TrCP2 - 2" nucleic acid under low stringency conditions,
      (iii) "βTrCP1 " (SEQ ID No:129) or a functionally active derivative thereof, or a functionally active fragment thereof, or a homolog thereof, or a variant of "βTrCP1" encoded by a nucleic acid that hybridizes to the "βTrCP1" nucleic acid under low stringency conditions,
      (iv) "p50 (NF-kappaB1)" (SEQ ID No:73) or a functionally active derivative thereof, or a functionally active fragment thereof, or a homolog thereof, or a variant of "p50 (NF-kappaB1)" encoded by a nucleic acid that hybridizes to the "p50 (NF-kappaB1)" nucleic acid under low stringency conditions,
      (v) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
      (vi) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
      (vii) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions,
      (viii) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
      (ix) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
      (x) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
      (xi) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
      (xii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
      (xiii) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
      (xiv) "Rel B" (SEQ ID No:81) or a functionally active derivative thereof, or a functionally active fragment thereof, or a homolog thereof, or a variant of "Rel B" encoded by a nucleic acid that hybridizes to the "Rel B" nucleic acid under low stringency conditions, and
      (xv) "c-ReI" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions, and
   (b) at least one second protein, which second protein is selected from the group consisting of:
      (i) "NEF-ASSOCIATED FACTOR 1" (SEQ ID No:126) or a functionally active derivative thereof, or a functionally active fragment thereof, or a homolog thereof, or a variant of "NEF-ASSOCIATED FACTOR 1" encoded by a nucleic acid that hybridizes to the "NEF-ASSOCIATED FACTOR 1" nucleic acid under low stringency conditions,
      (ii) "COATOMER B' SUBUNIT" (SEQ ID No:127) or a functionally active derivative thereof, or a functionally active fragment thereof, or a homolog thereof, or a variant of "COATOMER B' SUBUNIT" encoded by a nucleic acid that hybridizes to the "COATOMER β' SUBUNIT" nucleic acid under low stringency conditions,
      (iii) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions, and
      (iv) "SPERM SPECIFIC PROTEIN" (SEQ ID No:130) or a functionally active derivative thereof, or a functionally active fragment thereof, or a homolog thereof, or a variant of "SPERM SPECIFIC PROTEIN" encoded by a nucleic acid that hybridizes to the "SPERM SPECIFIC PROTEIN" nucleic acid under low stringency conditions;
   and a complex (II) comprising at least two of said second proteins,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
2. The protein complex according to claim 1 wherein the first protein is the protein "p105 (NF-κB1)" (SEQ ID No:74), or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions.
3. The protein complex according to No. 1 selected from complex (I) and comprising the following proteins:
   (i) "NEF-ASSOCIATED FACTOR 1" (SEQ ID No:126) or a functionally active derivative thereof, or a functionally active fragment thereof, or a homolog thereof, or a variant of "NEF-ASSOCIATED FACTOR 1" encoded by a nucleic acid that hybridizes to the "NEF-ASSOCIATED FACTOR 1" nucleic acid under low stringency conditions,

   (i) "COATOMER β' SUBUNIT" (SEQ ID No:127) or a functionally active derivative thereof, or a functionally active fragment thereof, or a homolog thereof, or a variant of "COATOMER β' SUBUNIT" encoded by a nucleic acid that hybridizes to the "COATOMER β' SUBUNIT" nucleic acid under low stringency conditions,
   (iii) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions,

   (i) "p50 (NF-kappaB1)" (SEQ ID No:73) or a functionally active derivative thereof, or a functionally active fragment thereof, or a homolog thereof, or a variant of "p50 (NF-κB1)" encoded by a nucleic acid that hybridizes to the "p50 (NF-κB1)" nucleic acid under low stringency conditions,
   (v) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (vi) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions,
   (vii) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (viii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (ix) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (x) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (xi) "SPERM SPECIFIC PROTEIN" (SEQ ID No:130) or a functionally active derivative thereof, or a functionally active fragment thereof, or a homolog thereof, or a variant of "SPERM SPECIFIC PROTEIN" encoded by a nucleic acid that hybridizes to the "SPERM SPECIFIC PROTEIN" nucleic acid under low stringency conditions,

   (i) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
   (xiii) "Rel B" (SEQ ID No:81) or a functionally active derivative thereof, or a functionally active fragment thereof, or a homolog thereof, or a variant of "Rel B" encoded by a nucleic acid that hybridizes to the "Rel B" nucleic acid under low stringency conditions,
   (xiv) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions, and
   (xv) "IκBε (xSEQ ID: 76) or a functionally active derivative thereof, or a functionally active fragment thereof, or a homolog thereof, or a variant of "IκBε" encoded by a nucleic acid that hybridizes to the "IκBε" nucleic acid under low stringency conditions.
   and a protein complex selected from complex (II) and comprising the following proteins:
   (i) "NEF-ASSOCIATED FACTOR 1" (SEQ ID No:126) or a functionally active derivative thereof, or a functionally active fragment thereof, or a homolog thereof, or a variant of "NEF-ASSOCIATED FACTOR 1" encoded by a nucleic acid that hybridizes to the "NEF-ASSOCIATED FACTOR 1" nucleic acid under low stringency conditions,

   (i) "COATOMER β' SUBUNIT" (SEQ ID No: 127) or a functionally active derivative thereof, or a functionally active fragment thereof, or a homolog thereof, or a variant of "COATOMER β' SUBUNIT" encoded by a nucleic acid that hybridizes to the "COATOMER β' SUBUNIT" nucleic acid under low stringency conditions,
   (iii) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions,

   (i) "p50 (NF-kappaB1)" (SEQ ID No:73) or a functionally active derivative thereof, or a functionally active fragment thereof, or a homolog thereof, or a variant of "p50 (NF-κB1)" encoded by a nucleic acid that hybridizes to the "p50 (NF-κB1)" nucleic acid under low stringency conditions,
   (v) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (vi) "RelA (p65)" (SEQ ID No:75) or a functionally active derivative thereof, or a functionally active fragment thereof, or a homolog thereof, or a variant of "RelA (p65)" encoded by a nucleic acid that hybridizes to the "RelA (p65)" nucleic acid under low stringency conditions,

   (i) "p105" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (viii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (ix) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (x) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (xi) "SPERM SPECIFIC PROTEIN" (SEQ ID No:130) or a functionally active derivative thereof, or a functionally active fragment thereof, or a homolog thereof, or a variant of "SPERM SPECIFIC PROTEIN" encoded by a nucleic acid that hybridizes to the "SPERM SPECIFIC PROTEIN" nucleic acid under low stringency conditions,
   (xii) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
   (xiii) "ReI B" (SEQ ID No:81) or a functionally active derivative thereof, or a functionally active fragment thereof, or a homolog thereof, or a variant of "ReI B" encoded by a nucleic acid that hybridizes to the "ReI B" nucleic acid under low stringency conditions,
   (xiv) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions, and

   (i) "IκBε (xSEQ ID: 76) or a functionally active derivative thereof, or a functionally active fragment thereof, or a homolog thereof, or a variant of "IκBε" encoded by a nucleic acid that hybridizes to the "IκBε," nucleic acid under low stringency conditions,
   (xvi) IKKβ (xSEQ ID: 24) or a functionally active derivative thereof;
   and a protein complex selected from complex (III) and comprising the following proteins:
   (i) "NEF-ASSOCIATED FACTOR 1" (SEQ ID No:126) or a functionally active derivative thereof, or a functionally active fragment thereof, or a homolog thereof, or a variant of "NEF-ASSOCIATED FACTOR 1" encoded by a nucleic acid that hybridizes to the "NEF-ASSOCIATED FACTOR 1" nucleic acid under low stringency conditions,
   (ii) "COATOMER β' SUBUNIT" (SEQ ID No:127) or a functionally active derivative thereof, or a functionally active fragment thereof, or a homolog thereof, or a variant of "COATOMER β' SUBUNIT" encoded by a nucleic acid that hybridizes to the "COATOMER β' SUBUNIT" nucleic acid under low stringency conditions,
   (iii) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions,

   (i) "p50 (NF-kappaB1)" (SEQ ID No:73) or a functionally active derivative thereof, or a functionally active fragment thereof, or a homolog thereof, or a variant of "p50 (NF-κB1)" encoded by a nucleic acid that hybridizes to the "p50 (NF-κB1)" nucleic acid under low stringency conditions,
   (v) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (vi) "RelA (p65)" (SEQ ID No:75) or a functionally active derivative thereof, or a functionally active fragment thereof, or a homolog thereof, or a variant of "RelA (p65)" encoded by a nucleic acid that hybridizes to the "RelA (p65)" nucleic acid under low stringency conditions,

   (i) "p105" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (viii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (ix) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (x) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (xi) "SPERM SPECIFIC PROTEIN" (SEQ ID No:130) or a functionally active derivative thereof, or a functionally active fragment thereof, or a homolog thereof, or a variant of "SPERM SPECIFIC PROTEIN" encoded by a nucleic acid that hybridizes to the "SPERM SPECIFIC PROTEIN" nucleic acid under low stringency conditions,
   (xii) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
   (xiii) "ReI B" (SEQ ID No:81) or a functionally active derivative thereof, or a functionally active fragment thereof, or a homolog thereof, or a variant of "ReI B" encoded by a nucleic acid that hybridizes to the "ReI B" nucleic acid under low stringency conditions,
   (xiv) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,

   (i) "IκBε (xSEQ ID: 76) or a functionally active derivative thereof, or a functionally active fragment thereof, or a homolog thereof, or a variant of "IκBε" encoded by a nucleic acid that hybridizes to the "IκBε" nucleic acid under low stringency conditions,
   (xvi) "IKKβ" (xSEQ ID: 24) or a functionally active derivative thereof, or a functionally active fragment thereof, or a homolog thereof, or a variant of "IKKβ" encoded by a nucleic acid that hybridizes to the "IKKβ" nucleic acid under low stringency conditions,
   (xvii) "Cot/Tpl2" (SEQ ID No:70) or a functionally active derivative thereof, or a functionally active fragment thereof, or a homolog thereof, or a variant of "Cot/Tpl-2" encoded by a nucleic acid that hybridizes to the "Cot/Tpl-2" nucleic acid under low stringency conditions, and

   (i) "β TrCP2 - 2" (SEQ ID No:128) or a functionally active derivative thereof, or a functionally active fragment thereof, or a homolog thereof, or a variant of "β TrCP2 - 2" encoded by a nucleic acid that hybridizes to the "β TrCP2 -2" nucleic acid under low stringency conditions,
   (xix) "βTrCP1 " (SEQ ID No:129) or a functionally active derivative thereof, or a functionally active fragment thereof, or a homolog thereof, or a variant of "β TrCP1" encoded by a nucleic acid that hybridizes to the "β TrCP1" nucleic acid under low stringency conditions.
4. The protein complex according to No. 1 selected from complex (I) comprising all but 1 - 3 of the following proteins:
   (i) "NEF-ASSOCIATED FACTOR 1" (SEQ ID No:126) or a functionally active derivative thereof, or a functionally active fragment thereof, or a homolog thereof, or a variant of "NEF-ASSOCIATED FACTOR 1" encoded by a nucleic acid that hybridizes to the "NEF-ASSOCIATED FACTOR 1" nucleic acid under low stringency conditions,

   (i) "COATOMER β' SUBUNIT" (SEQ ID No:127) or a functionally active derivative thereof, or a functionally active fragment thereof, or a homolog thereof, or a variant of "COATOMER β' SUBUNIT" encoded by a nucleic acid that hybridizes to the "COATOMER β' SUBUNIT" nucleic acid under low stringency conditions,
   (iii) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions,

   (i) "p50 (NF-kappaB1)" (SEQ ID No:73) or a functionally active derivative thereof, or a functionally active fragment thereof, or a homolog thereof, or a variant of "p50 (NF-κB1)" encoded by a nucleic acid that hybridizes to the "p50 (NF-κB1)" nucleic acid under low stringency conditions,
   (v) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (vi) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions,

   (i) "p105" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (viii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (ix) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (x) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (xi) "SPERM SPECIFIC PROTEIN" (SEQ ID No:130) or a functionally active derivative thereof, or a functionally active fragment thereof, or a homolog thereof, or a variant of "SPERM SPECIFIC PROTEIN" encoded by a nucleic acid that hybridizes to the "SPERM SPECIFIC PROTEIN" nucleic acid under low stringency conditions,
   (xii) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
   (xiii) "ReI B" (SEQ ID No:81) or a functionally active derivative thereof, or a functionally active fragment thereof, or a homolog thereof, or a variant of "ReI B" encoded by a nucleic acid that hybridizes to the "Rel B" nucleic acid under low stringency conditions,
   (xiv) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,

   (i) "IκBε (xSEQ ID: 76) or a functionally active derivative thereof, or a functionally active fragment thereof, or a homolog thereof, or a variant of "IκBε" encoded by a nucleic acid that hybridizes to the "IκBε" nucleic acid under low stringency conditions,
   (xvi) "IKKβ" (xSEQ ID: 24) or a functionally active derivative thereof, or a functionally active fragment thereof, or a homolog thereof, or a variant of "iKK β" encoded by a nucleic acid that hybridizes to the "IKK β" nucleic acid under low stringency conditions,
   (xvii) "Cot/Tpl2" (SEQ ID No:70) or a functionally active derivative thereof, or a functionally active fragment thereof, or a homolog thereof, or a variant of "Cot/Tpl-2" encoded by a nucleic acid that hybridizes to the "Cot/Tpl-2" nucleic acid under low stringency conditions, and

   (i) "β TrCP2 - 2" (SEQ ID No:128) or a functionally active derivative thereof, or a functionally active fragment thereof, or a homolog thereof, or a variant of "β TrCP2 - 2" encoded by a nucleic acid that hybridizes to the "β TrCP2 -2" nucleic acid under low stringency conditions,
   (xix) "βTrCP1" (SEQ ID No:129) or a functionally active derivative thereof, or a functionally active fragment thereof, or a homolog thereof, or a variant of "β TrCP1" encoded by a nucleic acid that hybridizes to the "β TrCP1" nucleic acid under low stringency conditions;
   and a protein complex selected from complex (II) comprising all but 1 - 3 o the following proteins:
   (i) "NEF-ASSOCIATED FACTOR 1" (SEQ ID No:126) or a functionally active derivative thereof, or a functionally active fragment thereof, or a homolog thereof, or a variant of "NEF-ASSOCIATED FACTOR 1" encoded by a nucleic acid that hybridizes to the "NEF-ASSOCIATED FACTOR 1" nucleic acid under low stringency conditions,

   (i) "COATOMER β' SUBUNIT" (SEQ ID No:127) or a functionally active derivative thereof, or a functionally active fragment thereof, or a homolog thereof, or a variant of "COATOMER β' SUBUNIT" encoded by a nucleic acid that hybridizes to the "COATOMER β' SUBUNIT" nucleic acid under low stringency conditions,
   (iii) "ABIN-2" (SEQ ID No: 1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions,

   (i) "p50 (NF-kappaB1)" (SEQ ID No:73) or a functionally active derivative thereof, or a functionally active fragment thereof, or a homolog thereof, or a variant of "p50 (NF-κB1)" encoded by a nucleic acid that hybridizes to the "p50 (NF-κB1)" nucleic acid under low stringency conditions,
   (v) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (vi) "RelA (p65)" (SEQ ID No:75) or a functionally active derivative thereof, or a functionally active fragment thereof, or a homolog thereof, or a variant of "RelA (p65)" encoded by a nucleic acid that hybridizes to the "RelA (p65)" nucleic acid under low stringency conditions,

   (i) "p105" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (viii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (ix) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (x) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (xi) "SPERM SPECIFIC PROTEIN" (SEQ ID No:130) or a functionally active derivative thereof, or a functionally active fragment thereof, or a homolog thereof, or a variant of "SPERM SPECIFIC PROTEIN" encoded by a nucleic acid that hybridizes to the "SPERM SPECIFIC PROTEIN" nucleic acid under low stringency conditions,
   (xii) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
   (xiii) "Rel B" (SEQ ID No:81) or a functionally active derivative thereof, or a functionally active fragment thereof, or a homolog thereof, or a variant of "ReI B" encoded by a nucleic acid that hybridizes to the "ReI B" nucleic acid under low stringency conditions,
   (xiv) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,

   (i) "IκBε (xSEQ ID: 76) or a functionally active derivative thereof, or a functionally active fragment thereof, or a homolog thereof, or a variant of "IκBε" encoded by a nucleic acid that hybridizes to the "IκBε" nucleic acid under low stringency conditions, and
   (xvi) "IKKβ" (xSEQ ID: 24) or a functionally active derivative thereof, or a functionally active fragment thereof, or a homolog thereof, or a variant of "iKK β" encoded by a nucleic acid that hybridizes to the "IKK β" nucleic acid under low stringency conditions.
5. The complex of any of No. 1-4 comprising a functionally active derivative of said first protein and/or a functionally active derivative of said second protein, wherein the functionally active derivative is a fusion protein comprising said first protein or said second protein fused to an amino acid sequence different from the first protein or second protein, respectively.
6. The complex of No. 5 wherein the functionally active derivative is a fusion protein comprising said first protein or said second protein fused to an affinity tag or label.
7. The complex of any of No. 1 - 4 comprising a fragment of said first protein and/or a fragment of said second protein, which fragment binds to another protein component of said complex.
8. The complex of any of No. 1 -7 that is involved in the Processing activity or Degradation activity.
9. A process for preparing complex of any of No. 1 - 8 and optionally the components thereof comprising the following steps:
   the expressing a protein (bait) of the complex, preferably a tagged protein, in a target cell, isolating the protein complex which is attached to the bait protein, and optionally disassociating the protein complex and isolating the individual complex members.
10. The process according to No. 9 wherein the tagged protein comprises two different tags which allow two separate affinity purification steps.
11. The process according to any of No. 9-10 wherein the two tags are separated by a cleavage site for a protease.
12. Component of the p105 (NF-κB1) protein complex obtainable by a process according to any of No. 9 to 11.
13. Protein of the p105 (NF-κB1) protein complex selected from
   (i) "SPERM SPECIFIC PROTEIN" (SEQ ID No:130) or a functionally active derivative thereof, or a functionally active fragment thereof, or a homolog thereof, or a variant of "SPERM SPECIFIC PROTEIN" encoded by a nucleic acid that hybridizes to the "SPERM SPECIFIC PROTEIN" nucleic acid under low stringency conditions,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
14. Nucleic acid encoding a protein according to No. 13.
15. Construct, preferably a vector construct, comprising (a) a nucleic acid according to No. 14 and at least one further nucleic acid which is normally not associated with said nucleic acid,or(b) at least two separate nucleic acid sequences each encoding a different protein, or a functionally active fragment or a functionally active derivative thereof at least one of said proteins, or functionally active fragments or functionally active derivative thereof being selected from the first group of proteins according to No. 1(a) and at least one of said proteins, or functionally active fragments or functionally active derivative thereof being selected from the second group of proteins according to No. 1(b).
16. Host cell, containing a vector comprising at least one of the nucleic acid of No. 14 and/or a construct of No. 15 or containing several vectors each comprising at least the nucleic acid sequence encoding at least one of the proteins, or functionally active fragments or functionally active derivatives thereof selected from the first group of proteins according to No. 1(a) and the proteins, or functionally active fragments or functionally active derivatives thereof selected from the second group of proteins according to No. 1(b).
17. An antibody or a fragment of said antibody containing the binding domain thereof, selected from an antibody or fragment thereof, which binds the complex of any of No. 1-8 and which does not bind any of the proteins of said complex when uncomplexed and an antibody or a fragment of said antibody which binds to any of the proteins according to No. 13.
18. A kit comprising in one or more container the complex of any of No. 1-8 and/or the proteins of No. 13 optionally together with an antibody according to No. 17 and/or further components such as reagents and working instructions.
19. The kit according to No. 18 for processing a substrate of said complex.
20. The kit according to No. 18 for the diagnosis or prognosis of a disease or a disease risk, preferentially for a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
21. Array, in which at least a complex according to any of No. 1 - 8 and/or at least one protein according to No. 14 and/or at least one antibody according to No. 17 is attached to a solid carrier.
22. A process for processing a physiological substrate of the complex comprising the step of bringing into contact a complex to any of No. 1 - 8 with said substrate, such that said substrate is processed.
23. A pharmaceutical composition comprising the protein complex of any of No. 1 to 8 and/or any of the following the proteins:
   (i) "SPERM SPECIFIC PROTEIN" (SEQ ID No:130) or a functionally active derivative thereof, or a functionally active fragment thereof, or a homolog thereof, or a variant of "SPERM SPECIFIC PROTEIN" encoded by a nucleic acid that hybridizes to the "SPERM SPECIFIC PROTEIN" nucleic acid under low stringency conditions,
   and a pharmaceutical acceptable carrier.
24. The pharmaceutical composition according to No. 23 for the treatment of diseases and disorders such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
25. A method for screening for a molecule that binds to the complex of anyone of No. 1 - 8 and/or any of the following the proteins:
   (i) "SPERM SPECIFIC PROTEIN" (SEQ ID No:130) or a functionally active derivative thereof, or a functionally active fragment thereof, or a homolog thereof, or a variant of "SPERM SPECIFIC PROTEIN" encoded by a nucleic acid that hybridizes to the "SPERM SPECIFIC PROTEIN" nucleic acid under low stringency conditions, comprising the steps of
      (a) exposing said complex, or a cell or organism containing same to one or more candidate molecules; and
      (b) determinig whether said candidate molecule is bound to the complex or protein.
26. A method for screening for a molecule that modulates directly or indirectly the function, activity, composition or formation of the complex of any one of No. 1 - 8 comprising the steps of (a) exposing said complex, or a cell or organism containing p105/p50 (NF-κB1) protein complex to one or more candidate molecules; and
   (b) determining the amount of activity of protein components of, and/or intracellular localization of, said complex and/or the transcription level of a gene dependent on the complex and/or the abundance and/or activity of a protein or protein complex dependent on the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependent on the complex and/or the abundance and/or activity of a protein or protein complex dependent on the function of the complex and/or product of a gene dependent on the complex in the absence of said candidate molecules indicates that the molecule modulates function, activity or composition of said complex.
27. The method of No. 26, wherein the amount of said complex is determined.
28. The method of No. 26, wherein the activity of said complex is determined.
29. The method of No. 28, wherein said determining step comprises isolating from the cell or organism said complex to produce said isolated complex and contacting said isolated complex in the presence or absence of a candidate molecule with a substrate of said complex and determining the processing of said substrate is modified in the presence of said candidate molecule.
30. The method of No. 26, wherein the amount of the individual protein components of said complex are determined.
31. The method of No. 30, determining step comprises determining whether
   (i) "NEF-ASSOCIATED FACTOR 1" (SEQ ID No:126) or a functionally active derivative thereof, or a functionally active fragment thereof, or a homolog thereof, or a variant of "NEF-ASSOCIATED FACTOR 1" encoded by a nucleic acid that hybridizes to the "NEF-ASSOCIATED FACTOR 1" nucleic acid under low stringency conditions, and/or
   (ii) "COATOMER β' SUBUNIT" (SEQ ID No:127) or a functionally active derivative thereof, or a functionally active fragment thereof, or a homolog thereof, or a variant of "COATOMER β' SUBUNIT" encoded by a nucleic acid that hybridizes to the "COATOMER β' SUBUNIT" nucleic acid under low stringency conditions, and/or
   (iii) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions, and/or
   (iv) "Cot/Tpl2" (SEQ ID No:70) or a functionally active derivative thereof, or a functionally active fragment thereof, or a homolog thereof, or a variant of "Cot/Tpl-2" encoded by a nucleic acid that hybridizes to the "Cot/Tpl-2" nucleic acid under low stringency conditions, and/or
   (v) "β TrCP2 - 2" (SEQ ID No:128) or a functionally active derivative thereof, or a functionally active fragment thereof, or a homolog thereof, or a variant of "β TrCP2 - 2" encoded by a nucleic acid that hybridizes to the "β TrCP2 -2" nucleic acid under low stringency conditions, and/or
   (vi) "βTrCP1 " (SEQ ID No:129) or a functionally active derivative thereof, or a functionally active fragment thereof, or a homolog thereof, or a variant of "β TrCP1" encoded by a nucleic acid that hybridizes to the "β TrCP1" nucleic acid under low stringency conditions, and/or
   (vii) "p50 (NF-kappaB1)" (SEQ ID No:73) or a functionally active derivative thereof, or a functionally active fragment thereof, or a homolog thereof, or a variant of "p50 (NF-κB1)" encoded by a nucleic acid that hybridizes to the "p50 (NF-κB1)" nucleic acid under low stringency conditions, and/or
   (viii) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions, and/or
   (ix) "IKK β " (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions, and/or
   (x) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions, and/or
   (xi) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions, and/or
   (xii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions, and/or
   (xiii) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions, and/or
   (xiv) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions, and/or
   (xv) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions, and/or
   (xvi) "SPERM SPECIFIC PROTEIN" (SEQ ID No:130) or a functionally active derivative thereof, or a functionally active fragment thereof, or a homolog thereof, or a variant of "SPERM SPECIFIC PROTEIN" encoded by a nucleic acid that hybridizes to the "SPERM SPECIFIC PROTEIN" nucleic acid under low stringency conditions, and/or
   (xvii) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions, and/or
   (xviii) "ReI B" (SEQ ID No:81) or a functionally active derivative thereof, or a functionally active fragment thereof, or a homolog thereof, or a variant of "ReI B" encoded by a nucleic acid that hybridizes to the "ReI B" nucleic acid under low stringency conditions, and/or
   (xix) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions, is present in the complex.
32. The method of any of No. 26 to 31, wherein said method is a method of screening for a drug for treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
33. Use of a molecule that modulates the amount of, activity of, or the protein components of the complex of any one of No. 1 - 8 for the manufacture of a medicament for the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
34. A method for the production of a pharmaceutical composition comprising carrying out the method of any of No. 1 - 8 to identify a molecule that modulates the function, activity, composition or formation of said complex, and further comprising mixing the identified molecule with a pharmaceutically acceptable carrier.
35. A method for diagnosing or screening for the presence of a disease or disorder or a predisposition for developing a disease or disorder in a subject, which disease or disorder is characterized by an aberrant amount of, activity of, or component composition of, or intracellular localization of the complex of any one of the No. 1 - 8, comprising determining the amount of, activity of, protein components of, and/or intracellular localization of, said complex and/or the transcription level of a gene dependent on the complex and/or the abundance and/or activity of a protein or protein complex dependent on the function of the complex and/or product of a gene dependent on the complex in a comparative sample derived from a subject, wherein a difference in said amount, activity, or protein components of, said complex in an analogous sample from a subject not having the disease or disorder or predisposition indicates the presence in the subject of the disease or disorder or predisposition in the subject.
36. The method of No. 35, wherein the amount of said complex is determined.
37. The method of No. 35, wherein the activity of said complex is determined.
38. The method of No. 37, wherein said determining step comprises isolating from the subject said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
39. The method of No. 35, wherein the amount of the individual protein components of said complex are determined.
40. The method of No. 39, wherein said determining step comprises determining whether
   (i) "NEF-ASSOCIATED FACTOR 1" (SEQ ID No:126) or a functionally active derivative thereof, or a functionally active fragment thereof, or a homolog thereof, or a variant of "NEF-ASSOCIATED FACTOR 1" encoded by a nucleic acid that hybridizes to the "NEF-ASSOCIATED FACTOR 1" nucleic acid under low stringency conditions, and/or
   (ii) "COATOMER β' SUBUNIT" (SEQ ID No:127) or a functionally active derivative thereof, or a functionally active fragment thereof, or a homolog thereof, or a variant of "COATOMER β' SUBUNIT" encoded by a nucleic acid that hybridizes to the "COATOMER β' SUBUNIT" nucleic acid under low stringency conditions, and/or
   (iii) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions, and/or
   (iv) "Cot/Tpl2" (SEQ ID No:70) or a functionally active derivative thereof, or a functionally active fragment thereof, or a homolog thereof, or a variant of "Cot/Tpl-2" encoded by a nucleic acid that hybridizes to the "Cot/Tpl-2" nucleic acid under low stringency conditions, and/or
   (v) "β TrCP2 - 2" (SEQ ID No:128) or a functionally active derivative thereof, or a functionally active fragment thereof, or a homolog thereof, or a variant of "β TrCP2 - 2" encoded by a nucleic acid that hybridizes to the "β TrCP2 -2" nucleic acid under low stringency conditions, and/or
   (vi) "βTrCP1 " (SEQ ID No:129) or a functionally active derivative thereof, or a functionally active fragment thereof, or a homolog thereof, or a variant of "β TrCP1" encoded by a nucleic acid that hybridizes to the "β TrCP1" nucleic acid under low stringency conditions, and/or
   (vii) "p50 (NF-kappaB1)" (SEQ ID No:73) or a functionally active derivative thereof, or a functionally active fragment thereof, or a homolog thereof, or a variant of "p50 (NF-κB1)" encoded by a nucleic acid that hybridizes to the "p50 (NF-κB1 )" nucleic acid under low stringency conditions, and/or
   (viii) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions, and/or
   (ix) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions, and/or
   (x) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "RelA (p65)" nucleic acid under low stringency conditions, and/or
   (xi) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions, and/or
   (xii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions, and/or
   (xiii) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions, and/or
   (xiv) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions, and/or
   (xv) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions, and/or
   (xvi) "SPERM SPECIFIC PROTEIN" (SEQ ID No:130) or a functionally active derivative thereof, or a functionally active fragment thereof, or a homolog thereof, or a variant of "SPERM SPECIFIC PROTEIN" encoded by a nucleic acid that hybridizes to the "SPERM SPECIFIC PROTEIN" nucleic acid under low stringency conditions, and/or
   (xvii) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions, and/or
   (xviii) "Rel B" (SEQ ID No:81) or a functionally active derivative thereof, or a functionally active fragment thereof, or a homolog thereof, or a variant of "Rel B" encoded by a nucleic acid that hybridizes to the "Rel B" nucleic acid under low stringency conditions, and/or
   (xix) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions, is present in the complex.
41. The complex of any one of No. 1 - 8, or proteins of No. 13 or the antibody or fragment of No. 17, for use in a method of diagnosing a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
42. A method for treating or preventing a disease or disorder characterized by an aberrant amount of, activity or component composition of or intracellular localization of, the complex of anyone of No. 1-8, comprising administering to a subject in need of such treatment or prevention a therapeutically effective amount of one or more molecules that modulate the amount of, Processing activity or Degradation activity, or protein components of, said complex.
43. The method according to No. 42, wherein said disease or disorder involves decreased levels of the amount or activity of said complex.
44. The method according to No. 42 , wherein said disease or disorder involves increased levels of the amount or activity of said complex.
45. Complex of any of No. 1 - 8 and/or protein selected from the following proteins
   (i) "NEF-ASSOCIATED FACTOR 1" (SEQ ID No:126) or a functionally active derivative thereof, or a functionally active fragment thereof, or a homolog thereof, or a variant of "NEF-ASSOCIATED FACTOR 1" encoded by a nucleic acid that hybridizes to the "NEF-ASSOCIATED FACTOR 1" nucleic acid under low stringency conditions,
   (ii) "COATOMER β' SUBUNIT" (SEQ ID No:127) or a functionally active derivative thereof, or a functionally active fragment thereof, or a homolog thereof, or a variant of "COATOMER β' SUBUNIT" encoded by a nucleic acid that hybridizes to the "COATOMER β' SUBUNIT" nucleic acid under low stringency conditions,
   (iii) "ABIN-2" (SEQ ID No:1) or a functionally active derivative thereof, or a functionally active fragment thereof, or a homolog thereof, or a variant of "ABIN-2" encoded by a nucleic acid that hybridizes to the "ABIN-2" nucleic acid under low stringency conditions,
   (iv) "Cot/Tpl2" (SEQ ID No:70) or a functionally active derivative thereof, or a functionally active fragment thereof, or a homolog thereof, or a variant of "Cot/Tpl-2" encoded by a nucleic acid that hybridizes to the "Cot/Tpl-2" nucleic acid under low stringency conditions,
   (v) "β TrCP2 - 2" (SEQ ID No:128) or a functionally active derivative thereof, or a functionally active fragment thereof, or a homolog thereof, or a variant of "β TrCP2 - 2" encoded by a nucleic acid that hybridizes to the "β TrCP2 -2" nucleic acid under low stringency conditions,
   (vi) "βTrCP1 " (SEQ ID No:129) or a functionally active derivative thereof, or a functionally active fragment thereof, or a homolog thereof, or a variant of "β TrCP1" encoded by a nucleic acid that hybridizes to the "β TrCP1" nucleic acid under low stringency conditions,
   (vii) "p50 (NF-kappaB1)" (SEQ ID No:73) or a functionally active derivative thereof, or a functionally active fragment thereof, or a homolog thereof, or a variant of "p50 (NF-κB1)" encoded by a nucleic acid that hybridizes to the "p50 (NF-κB1)" nucleic acid under low stringency conditions,
   (viii) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (ix) "IKK β " (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (x) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions,
   (xi) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (xii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (xiii) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (xiv) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (xv) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (xvi) "SPERM SPECIFIC PROTEIN" (SEQ ID No:130) or a functionally active derivative thereof, or a functionally active fragment thereof, or a homolog thereof, or a variant of "SPERM SPECIFIC PROTEIN" encoded by a nucleic acid that hybridizes to the "SPERM SPECIFIC PROTEIN" nucleic acid under low stringency conditions,
   (xvii) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
   (xviii) "Rel B" (SEQ ID No:81) or a functionally active derivative thereof,or a functionally active fragement thereof, or a homolog thereof, or a variant of "Rel B" that hybridizes to the "Rel B" nucleic acid under low stringency conditions, and
   (xix) "c-Rel" (SEQ ID No:71) or a functionally active derivative thereof, or a functionally active fragment thereof, or a homolog thereof, or a variant of "c-Rel" encoded by a nucleic acid that hybridizes to the "c-ReI" nucleic acid under low stringency conditions, as a target for an active agent of a pharmaceutical, preferably a drug target in the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.

The present invention further relates to the IκBα protein complex
1. A protein complex selected from complex (I) and comprising
   (a) at least one first protein selected from the group consisting of:
      (i) "κB - ras2" (SEQ ID No:131 ) or a functionally active derivative thereof, or a functionally active fragment thereof, or a homolog thereof, or a variant of "κB - ras2" encoded by a nucleic acid that hybridizes to the "κB - ras2" nucleic acid under low stringency conditions,
      (ii) "κB - ras1" (SEQ ID No:132) or a functionally active derivative thereof, or a functionally active fragment thereof, or a homolog thereof, or a variant of "κB - ras1" encoded by a nucleic acid that hybridizes to the "κB - ras1" nucleic acid under low stringency conditions,
      (iii) "β TrCP1" (SEQ ID No:129) or a functionally active derivative thereof, or a functionally active fragment thereof, or a homolog thereof, or a variant of "β TrCP1" encoded by a nucleic acid that hybridizes to the "β TrCP1" nucleic acid under low stringency conditions,
      (iv) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions, (v) "c-Rel" (SEQ ID No:71) or a functionally active derivative thereof, or a functionally active fragment thereof, or a homolog thereof, or a variant of "c-ReI" encoded by a nucleic acid that hybridizes to the "c-Rel" nucleic acid under low stringency conditions,
      (vi) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
      (vii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
      (viii) "hnRNPA1" (SEQ ID No:134) or a functionally active derivative thereof, or a functionally active fragment thereof, or a homolog thereof, or a variant of "hnRNPA1" encoded by a nucleic acid that hybridizes to the "hnRNPA1" nucleic acid under low stringency conditions,
      (ix) "SKP1 (SCF complex complonent)" (SEQ ID No:135) or a functionally active derivative thereof, or a functionally active fragment thereof, or a homolog thereof, or a variant of "SKP1 (SCF complex complonent)" encoded by a nucleic acid that hybridizes to the "SKP1 (SCF complex complonent)" nucleic acid under low stringency conditions,
      (x) "Cullin1" (SEQ ID No:136) or a functionally active derivative thereof, or a functionally active fragment thereof, or a homolog thereof, or a variant of "Cullin1" encoded by a nucleic acid that hybridizes to the "Cullin1" nucleic acid under low stringency conditions,
      (xi) "Hos1" (SEQ ID No:137) or a functionally active derivative thereof, or a functionally active fragment thereof, or a homolog thereof, or a variant of "Hos1" encoded by a nucleic acid that hybridizes to the "Hos1" nucleic acid under low stringency conditions,
      (xii) "ReIB" (SEQ ID No:81) or a functionally active derivative thereof, or a functionally active fragment thereof, or a homolog thereof, or a variant of "ReIB" encoded by a nucleic acid that hybridizes to the "ReIB" nucleic acid under low stringency conditions,
      (xiii) "karyopherin α2" (SEQ ID No: 138) or a functionally active derivative thereof, or a functionally active fragment thereof, or a homolog thereof, or a variant of "karyopherin α2" encoded by a nucleic acid that hybridizes to the "karyopherin α2" nucleic acid under low stringency conditions,
      (xiv) "karyoherin α1" (SEQ ID No:103) or a functionally active derivative thereof, or a functionally active fragment thereof, or a homolog thereof, or a variant of "karyoherin α1" encoded by a nucleic acid that hybridizes to the "karyoherin α1" nucleic acid under low stringency conditions,
      (xv) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
      (xvi) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
      (xvii) "IκBα" (SEQ ID No:78) or a functionally active derivative thereof, or a functionally active fragment thereof, or a homolog thereof, or a variant of "IκBα" encoded by a nucleic acid that hybridizes to the "IκBα" nucleic acid under low stringency conditions,
      (xviii) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions,
      (xix) "PTP-BAS " (SEQ ID No:147) or a functionally active derivative thereof, or a functionally active fragment thereof, or a homolog thereof, or a variant of "PTP-BAS " encoded by a nucleic acid that hybridizes to the "PTP-BAS" nucleic acid under low stringency conditions,
      (xx) "β TrCP2-1" (SEQ ID No: 148) or a functionally active derivative thereof, or a functionally active fragment thereof, or a homolog thereof, or a variant of "β TrCP2-1" encoded by a nucleic acid that hybridizes to the "β TrCP2-1" nucleic acid under low stringency conditions, and
      (xxi) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
      (xxii) "IκBε" (SEQ ID No:76) or a functionally active derivative thereof, or a functionally active fragment thereof, or a homolog thereof, or a variant of "IκBε" encoded by a nucleic acid that hybridizes to the "IκBε" nucleic acid under low stringency conditions,
      (xxiii) "Roc1/Rbx1" (SEQ ID No:146) or a functionally active derivative thereof, or a functionally active fragment thereof, or a homolog thereof, or a variant of "Roc1/Rbx1" encoded by a nucleic acid that hybridizes to the "Roc1/Rbx1" nucleic acid under low stringency conditions, and
      (xxiv) "SKP2 (SCF complex component)" (SEQ ID No:133) or a functionally active derivative thereof, or a functionally active fragment thereof, or a homolog thereof, or a variant of "SKP2 (SCF complex component)" encoded by a nucleic acid that hybridizes to the "SKP2 (SCF complex component)" nucleic acid under low stringency conditions, and
   (b) at least one second protein, which second protein is selected from the group consisting of:
      (i) "CDNA FLJ10422 FIS, CLONE NT2RP1000243" (SEQ ID No:139) or a functionally active derivative thereof, or a functionally active fragment thereof, or a homolog thereof, or a variant of "CDNA FLJ10422 FIS, CLONE NT2RP1000243" encoded by a nucleic acid that hybridizes to the "CDNA FLJ10422 FIS, CLONE NT2RP1000243" nucleic acid under low stringency conditions,
      (ii) "DNA REPLICATION LICENSING FACTOR MCM5" (SEQ ID No: 140) or a functionally active derivative thereof, or a functionally active fragment thereof, or a homolog thereof, or a variant of "DNA REPLICATION LICENSING FACTOR MCM5" encoded by a nucleic acid that hybridizes to the "DNA REPLICATION LICENSING FACTOR MCM5" nucleic acid under low stringency conditions,
      (iii) "DNA REPLICATION LICENSING FACTOR MCM3" (SEQ ID No:141) or a functionally active derivative thereof, or a functionally active fragment thereof, or a homolog thereof, or a variant of "DNA REPLICATION LICENSING FACTOR MCM3" encoded by a nucleic acid that hybridizes to the "DNA REPLICATION LICENSING FACTOR MCM3" nucleic acid under low stringency conditions,
      (iv) "DNA REPLICATION LICENSING FACTOR MCM7" (SEQ ID No:142) or a functionally active derivative thereof, or a functionally active fragment thereof, or a homolog thereof, or a variant of "DNA REPLICATION LICENSING FACTOR MCM7" encoded by a nucleic acid that hybridizes to the "DNA REPLICATION LICENSING FACTOR MCM7" nucleic acid under low stringency conditions,
      (v) "SIMILAR TO RIKEN CDNA 2610005H11 GENE" (SEQ ID No:143) or a functionally active derivative thereof, or a functionally active fragment thereof, or a homolog thereof, or a variant of "SIMILAR TO RIKEN CDNA 2610005H11 GENE" encoded by a nucleic acid that hybridizes to the "SIMILAR TO RIKEN CDNA 2610005H11 GENE" nucleic acid under low stringency conditions,
      (vi) "HYDROXYACYL-COENZYME A DEHYDROGENASE/3-KETOACYL-COENZYME A THIOLASE/ENOYL-COENZYME A HYDRATASE (TRIFUNCTIONAL PROTEIN), α SUBUNIT" (SEQ ID No:144) or a functionally active derivative thereof, or a functionally active fragment thereof, or a homolog thereof, or a variant of "HYDROXYACYL-COENZYME A DEHYDROGENASE/3-KETOACYL-COENZYME A THIOLASE/ENOYL-COENZYME A HYDRATASE (TRIFUNCTIONAL PROTEIN), α SUBUNIT" encoded by a nucleic acid that hybridizes to the "HYDROXYACYL-COENZYME A DEHYDROGENASE/3-KETOACYL-COENZYME A THIOLASE/ENOYL-COENZYME A HYDRATASE (TRIFUNCTIONAL PROTEIN), α SUBUNIT" nucleic acid under low stringency conditions,
      (vii) "MEVALONATE KINASE" (SEQ ID No:145) or a functionally active derivative thereof, or a functionally active fragment thereof, or a homolog thereof, or a variant of "MEVALONATE KINASE" encoded by a nucleic acid that hybridizes to the "MEVALONATE KINASE" nucleic acid under low stringency conditions,
      (viii) "ASB-3 PROTEIN" (SEQ ID No:149) or a functionally active derivative thereof, or a functionally active fragment thereof, or a homolog thereof, or a variant of "ASB-3 PROTEIN" encoded by a nucleic acid that hybridizes to the "ASB-3 PROTEIN" nucleic acid under low stringency conditions,
      (ix) "CDNA FLJ14103 FIS, CLONE MAMMA1001073" (SEQ ID No:150) or a functionally active derivative thereof, or a functionally active fragment thereof, or a homolog thereof, or a variant of "CDNA FLJ14103 FIS, CLONE MAMMA1001073" encoded by a nucleic acid that hybridizes to the "CDNA FLJ14103 FIS, CLONE MAMMA1001073" nucleic acid under low stringency conditions, and
      (x) "SERINE HYDROXYMETHYLTRANSFERASE, MITOCHONDRIAL PRECURSOR" (SEQ ID No:151) or a functionally active derivative thereof, or a functionally active fragment thereof, or a homolog thereof, or a variant of "SERINE HYDROXYMETHYLTRANSFERASE, MITOCHONDRIAL PRECURSOR" encoded by a nucleic acid that hybridizes to the "SERINE HYDROXYMETHYLTRANSFERASE, MITOCHONDRIAL PRECURSOR" nucleic acid under low stringency conditions;
   and a complex (II) comprising at least two of said second proteins,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
2. The protein complex according to claim 1 wherein the first protein is the protein "IκBα" (SEQ ID No:78), or a functionally active derivative thereof, or a functionally active fragment thereof, or a homolog thereof, or a variant of "IκBα" encoded by a nucleic acid that hybridizes to the "IκBα" nucleic acid under low stringency conditions.
3. The protein complex according to No. 1 selected from complex (I) and comprising the following proteins:
   (i) "β TrCP2 - 1" (SEQ ID No:148) or a functionally active derivative thereof, or a functionally active fragment thereof, or a homolog thereof, or a variant of "β TrCP2-1" encoded by a nucleic acid that hybridizes to the "β TrCP2-1" nucleic acid under low stringency conditions,
   (ii) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
   (iii) "DNA REPLICATION LICENSING FACTOR MCM3" (SEQ ID No:141) or a functionally active derivative thereof, or a functionally active fragment thereof, or a homolog thereof, or a variant of "DNA REPLICATION LICENSING FACTOR MCM3" encoded by a nucleic acid that hybridizes to the "DNA REPLICATION LICENSING FACTOR MCM3" nucleic acid under low stringency conditions,
   (iv) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (v) "IκBα" (SEQ ID No:78) or a functionally active derivative thereof, or a functionally active fragment thereof, or a homolog thereof, or a variant of "IκBα" encoded by a nucleic acid that hybridizes to the "IκBα" nucleic acid under low stringency conditions,
   (vi) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (vii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (viii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (ix) "MEVALONATE KINASE" (SEQ ID No:145) or a functionally active derivative thereof, or a functionally active fragment thereof, or a homolog thereof, or a variant of "MEVALONATE KINASE" encoded by a nucleic acid that hybridizes to the "MEVALONATE KINASE" nucleic acid under low stringency conditions,
   (x) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions,
   (xi) "p100 (NF-KB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions, and
   (xii) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions;
   and a protein complex selected from complex (II) and comprising the following proteins:
   (i) "β TrCP2-1" (SEQ ID No:148) or a functionally active derivative thereof, or a functionally active fragment thereof, or a homolog thereof, or a variant of "β TrCP2-1" encoded by a nucleic acid that hybridizes to the "β TrCP2-1" nucleic acid under low stringency conditions,
   (ii) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
   (iii) "DNA REPLICATION LICENSING FACTOR MCM3" (SEQ ID No:141) or a functionally active derivative thereof, or a functionally active fragment thereof, or a homolog thereof, or a variant of "DNA REPLICATION LICENSING FACTOR MCM3" encoded by a nucleic acid that hybridizes to the "DNA REPLICATION LICENSING FACTOR MCM3" nucleic acid under low stringency conditions,
   (iv) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (v) "IκBα" (SEQ ID No:78) or a functionally active derivative thereof, or a functionally active fragment thereof, or a homolog thereof, or a variant of "IκBα" encoded by a nucleic acid that hybridizes to the "IκBα" nucleic acid under low stringency conditions,
   (vi) "IKK α" (SEQ ID No:25) or a functionally active derivative thereof, or a functionally active fragment thereof, or a homolog thereof, or a variant of "I KK α" encoded by a nucleic acid that hybridizes to the "IKK α" nucleic acid under low stringency conditions,
   (vii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (viii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (ix) "MEVALONATE KINASE" (SEQ ID No:145) or a functionally active derivative thereof, or a functionally active fragment thereof, or a homolog thereof, or a variant of "MEVALONATE KINASE" encoded by a nucleic acid that hybridizes to the "MEVALONATE KINASE" nucleic acid under low stringency conditions,
   (x) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions,
   (xi) "p100 (NF-KB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (xii) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (xiii) "β TrCP1" (SEQ ID No:129) or a functionally active derivative thereof, or a functionally active fragment thereof, or a homolog thereof, or a variant of "β TrCP1" encoded by a nucleic acid that hybridizes to the "β TrCP1" nucleic acid under low stringency conditions,
   (xiv) "Cullin1" (SEQ ID No:136) or a functionally active derivative thereof, or a functionally active fragment thereof, or a homolog thereof, or a variant of "Cullin 1" encoded by a nucleic acid that hybridizes to the "Cullin 1" nucleic acid under low stringency conditions, and
   (xv) "SKP1 (SCF complex complonent)" (SEQ ID No:135) or a functionally active derivative thereof, or a functionally active fragment thereof, or a homolog thereof, or a variant of "SKP1 (SCF COMPLEX COMPONTENT" encoded by a nucleic acid that hybridizes to the "SKP1 (SCF COMPLEX COMPONENT" nucleic acid under low stringency conditions;
   and a protein complex selected from complex (III) and comprising the following proteins:
   (i) "β TrCP2 - 1" (SEQ ID No:148) or a functionally active derivative thereof, or a functionally active fragment thereof, or a homolog thereof, or a variant of "β TrCP2 - 1" encoded by a nucleic acid that hybridizes to the "β TrCP2 - 1" nucleic acid under low stringency conditions,
   (ii) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
   (iii) "DNA REPLICATION LICENSING FACTOR MCM3" (SEQ ID No:141) or a functionally active derivative thereof, or a functionally active fragment thereof, or a homolog thereof, or a variant of "DNA REPLICATION LICENSING FACTOR MCM3" encoded by a nucleic acid that hybridizes to the "DNA REPLICATION LICENSING FACTOR MCM3" nucleic acid under low stringency conditions,
   (iv) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (v) "IκBα" (SEQ ID No:78) or a functionally active derivative thereof, or a functionally active fragment thereof, or a homolog thereof, or a variant of "IκBα" encoded by a nucleic acid that hybridizes to the "IκBα" nucleic acid under low stringency conditions,
   (vi) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (vii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (viii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (ix) "MEVALONATE KINASE" (SEQ ID No:145) or a functionally active derivative thereof, or a functionally active fragment thereof, or a homolog thereof, or a variant of "MEVALONATE KINASE" encoded by a nucleic acid that hybridizes to the "MEVALONATE KINASE" nucleic acid under low stringency conditions,
   (x) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions,
   (xi) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (xii) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (xiii) "ASB-3 PROTEIN" (SEQ ID No:149) or a functionally active derivative thereof, or a functionally active fragment thereof, or a homolog thereof, or a variant of "ASB-3 PROTEIN" encoded by a nucleic acid that hybridizes to the "ASB-3 PROTEIN" nucleic acid under low stringency conditions,
   (xiv) "CDNA FLJ10422 FIS, CLONE NT2RP1000243" (SEQ ID No:139) or a functionally active derivative thereof, or a functionally active fragment thereof, or a homolog thereof, or a variant of "CDNA FLJ10422 FIS, CLONE NT2RP1000243" encoded by a nucleic acid that hybridizes to the "CDNA FLJ10422 FIS, CLONE NT2RP1000243" nucleic acid under low stringency conditions,
   (xv) "DNA REPLICATION LICENSING FACTOR MCM5" (SEQ ID No:140) or a functionally active derivative thereof, or a functionally active fragment thereof, or a homolog thereof, or a variant of "DNA REPLICATION LICENSING FACTOR MCM5" encoded by a nucleic acid that hybridizes to the "DNA REPLICATION LICENSING FACTOR MCM5" nucleic acid under low stringency conditions,
   (xvi) "DNA REPLICATION LICENSING FACTOR MCM7" (SEQ ID No:142) or a functionally active derivative thereof, or a functionally active fragment thereof, or a homolog thereof, or a variant of "DNA REPLICATION LICENSING FACTOR MCM7" encoded by a nucleic acid that hybridizes to the "DNA REPLICATION LICENSING FACTOR MCM7" nucleic acid under low stringency conditions,
   (xvii) "HYDROXYACYL-COENZYME A DEHYDROGENASE/3-KETOACYL-COENZYME A THIOLASE/ENOYL-COENZYME A HYDRATASE (TRIFUNCTIONAL PROTEIN), α SUBUNIT" (SEQ ID No:144) or a functionally active derivative thereof, or a functionally active fragment thereof, or a homolog thereof, or a variant of "HYDROXYACYL-COENZYME A DEHYDROGENASE/3-KETOACYL-COENZYME A THIOLASE/ENOYL-COENZYME A HYDRATASE (TRIFUNCTIONAL PROTEIN), α SUBUNIT" encoded by a nucleic acid that hybridizes to the "HYDROXYACYL-COENZYME A DEHYDROGENASE/3-KETOACYL-COENZYME A THIOLASE/ENOYL-COENZYME A HYDRATASE (TRIFUNCTIONAL PROTEIN), α SUBUNIT" nucleic acid under low stringency conditions,
   (xviii) "PTP-BAS" (SEQ ID No:147) or a functionally active derivative thereof, or a functionally active fragment thereof, or a homolog thereof, or a variant of "PTP-BAS" encoded by a nucleic acid that hybridizes to the "PTP-BAS" nucleic acid under low stringency conditions,
   (xxix) "SERINE HYDROXYMETHYLTRANSFERASE, MITOCHONDRIAL PRECURSOR" (SEQ ID No:151) or a functionally active derivative thereof, or a functionally active fragment thereof, or a homolog thereof, or a variant of "SERINE HYDROXYMETHYLTRANSFERASE, MITOCHONDRIAL PRECURSOR" encoded by a nucleic acid that hybridizes to the "SERINE HYDROXYMETHYL TRANSFERASE, MITOCHONDRIAL PRECURSOR" nucleic acid under low stringency conditions,
   (xx) "SIMILAR TO RIKEN CDNA 2610005H11 GENE" (SEQ ID No:143) or a functionally active derivative thereof, or a functionally active fragment thereof, or a homolog thereof, or a variant of "SIMILAR TO RIKEN CDNA 2610005H11 GENE" encoded by a nucleic acid that hybridizes to the "SIMILAR TO RIKEN CDNA 2610005H11 GENE" nucleic acid under low stringency conditions, and
   (xxxi) "CDNA FLJ14103 FIS, CLONE MAMMA1001073" (SEQ ID No:150) or a functionally active derivative thereof, or a functionally active fragment thereof, or a homolog thereof, or a variant of "CDNA FLJ14103 FIS, CLONE MAMMA1001073" encoded by a nucleic acid that hybridizes to the "CDNA FLJ14103 FIS, CLONE MAMMA1001073" nucleic acid under low stringency conditions;
   and a protein complex selected from complex (IV) and comprising the following proteins:
   (i) "κB - ras2" (SEQ ID No:131) or a functionally active derivative thereof, or a functionally active fragment thereof, or a homolog thereof, or a variant of "κB - ras2" encoded by a nucleic acid that hybridizes to the "κB - ras2" nucleic acid under low stringency conditions,
   (ii) "κB - ras1" (SEQ ID No:132) or a functionally active derivative thereof, or a functionally active fragment thereof, or a homolog thereof, or a variant of "κB - ras1" encoded by a nucleic acid that hybridizes to the "κB - ras1" nucleic acid under low stringency conditions,
   (iii) "β TrCP1" (SEQ ID No:129) or a functionally active derivative thereof, or a functionally active fragment thereof, or a homolog thereof, or a variant of "β TrCP1" encoded by a nucleic acid that hybridizes to the "β TrCP1" nucleic acid under low stringency conditions,
   (iv) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (v) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
   (vi) "p100 (NF-KB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (vii) "SKP2 (SCF complex component)" (SEQ ID No:133) or a functionally active derivative thereof, or a functionally active fragment thereof, or a homolog thereof, or a variant of "SKP2 (SCF COMPLEX COMPONTENT" encoded by a nucleic acid that hybridizes to the "SKP2 (SCF COMPLEX COMPONENT" nucleic acid under low stringency conditions,
   (viii) "IKKγ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (ix) "hnRNPA1" (SEQ ID No:134) or a functionally active derivative thereof, or a functionally active fragment thereof, or a homolog thereof, or a variant of "hnRNPA1" encoded by a nucleic acid that hybridizes to the "hnRNPA1" nucleic acid under low stringency conditions,
   (x) "SKP1 (SCF complex complonent)" (SEQ ID No:135) or a functionally active derivative thereof, or a functionally active fragment thereof, or a homolog thereof, or a variant of "SKP1 (SCF COMPLEX COMPONTENT" encoded by a nucleic acid that hybridizes to the "SKP1 (SCF COMPLEX COMPONENT" nucleic acid under low stringency conditions,
   (xi) "Cullin1" (SEQ ID No: 136) or a functionally active derivative thereof, or a functionally active fragment thereof, or a homolog thereof, or a variant of "Cullin 1" encoded by a nucleic acid that hybridizes to the "Cullin 1" nucleic acid under low stringency conditions,
   (xii) "Hos1" (SEQ ID No:137) or a functionally active derivative thereof, or a functionally active fragment thereof, or a homolog thereof, or a variant of "Hos1" encoded by a nucleic acid that hybridizes to the "Hos1" nucleic acid under low stringency conditions,
   (xiii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (xiv) "ReIB" (SEQ ID No:81) or a functionally active derivative thereof, or a functionally active fragment thereof, or a homolog thereof, or a variant of "ReIB" encoded by a nucleic acid that hybridizes to the "ReIB" nucleic acid under low stringency conditions,
   (xv) "karyopherin α2" (SEQ ID No: 138) or a functionally active derivative thereof, or a functionally active fragment thereof, or a homolog thereof, or a variant of "karyopherin α2" encoded by a nucleic acid that hybridizes to the "karyopherin α2" nucleic acid under low stringency conditions,
   (xvi) "karyoherin α1" (SEQ ID No:103) or a functionally active derivative thereof, or a functionally active fragment thereof, or a homolog thereof, or a variant of "karyopherin α1" encoded by a nucleic acid that hybridizes to the "karyopherin α1" nucleic acid under low stringency conditions,
   (xvii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (xviii) "CDNA FLJ10422 FIS, CLONE NT2RP1000243" (SEQ ID No:139) or a functionally active derivative thereof, or a functionally active fragment thereof, or a homolog thereof, or a variant of "CDNA FLJ10422 FIS, CLONE NT2RP1000243" encoded by a nucleic acid that hybridizes to the "CDNA FLJ10422 FIS, CLONE NT2RP1000243" nucleic acid under low stringency conditions,
   (xix) "DNA REPLICATION LICENSING FACTOR MCM5" (SEQ ID No:140) or a functionally active derivative thereof, or a functionally active fragment thereof, or a homolog thereof, or a variant of "DNA REPLICATION LICENSING FACTOR MCM5" encoded by a nucleic acid that hybridizes to the "DNA REPLICATION LICENSING FACTOR MCM5" nucleic acid under low stringency conditions,
   (xx) "DNA REPLICATION LICENSING FACTOR MCM3" (SEQ ID No:141) or a functionally active derivative thereof, or a functionally active fragment thereof, or a homolog thereof, or a variant of "DNA REPLICATION LICENSING FACTOR MCM3" encoded by a nucleic acid that hybridizes to the "DNA REPLICATION LICENSING FACTOR MCM3" nucleic acid under low stringency conditions,
   (xxi) "DNA REPLICATION LICENSING FACTOR MCM7" (SEQ ID No: 142) or a functionally active derivative thereof, or a functionally active fragment thereof, or a homolog thereof, or a variant of "DNA REPLICATION LICENSING FACTOR MCM7" encoded by a nucleic acid that hybridizes to the "DNA REPLICATION LICENSING FACTOR MCM7" nucleic acid under low stringency conditions,
   (xxii) "SIMILAR TO RIKEN CDNA 2610005H11 GENE" (SEQ ID No: 143) or a functionally active derivative thereof, or a functionally active fragment thereof, or a homolog thereof, or a variant of "SIMILAR TO RIKEN CDNA 2610005H11 GENE" encoded by a nucleic acid that hybridizes to the "SIMILAR TO RIKEN CDNA 2610005H11 GENE" nucleic acid under low stringency conditions,
   (xxiii) "HYDROXYACYL-COENZYME A DEHYDROGENASE/3-KETOACYL-COENZYME A THIOLASE/ENOYL-COENZYME A HYDRATASE (TRIFUNCTIONAL PROTEIN), A SUBUNIT" (SEQ ID No:144) or a functionally active derivative thereof, or a functionally active fragment thereof, or a homolog thereof, or a variant of "HYDROXYACYL-COENZYME A DEHYDROGENASE/3-KETOACYL-COENZYME A THIOLASE/ENOYL-COENZYME A HYDRATASE (TRIFUNCTIONAL PROTEIN), α SUBUNIT" encoded by a nucleic acid that hybridizes to the "HYDROXYACYL-COENZYME A DEHYDROGENASE/3-KETOACYL-COENZYME A THIOLASE/ENOYL-COENZYME A HYDRATASE (TRIFUNCTIONAL PROTEIN), α SUBUNIT" nucleic acid under low stringency conditions,
   (xxiv) " MEVALONATE KINASE " (SEQ ID No:145) or a functionally active derivative thereof, or a functionally active fragment thereof, or a homolog thereof, or a variant of "MEVALONATE KINASE" encoded by a nucleic acid that hybridizes to the "MEVALONATE KINASE" nucleic acid under low stringency conditions,
   (xxv) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (xxvi) "Roc1 / RBX1" (SEQ ID No:146) or a functionally active derivative thereof, or a functionally active fragment thereof, or a homolog thereof, or a variant of "Roc/RBX1" encoded by a nucleic acid that hybridizes to the "Roc1/RBX1" nucleic acid under low stringency conditions,
   (xxvii) "IκBα" (SEQ ID No:78) or a functionally active derivative thereof, or a functionally active fragment thereof, or a homolog thereof, or a variant of "IκBα" encoded by a nucleic acid that hybridizes to the "IκBα" nucleic acid under low stringency conditions,
   (xxviii) "RelA (p65)" (SEQ ID No:75) or a functionally active derivative thereof, or a functionally active fragment thereof, or a homolog thereof, or a variant of "RelA (p65)" encoded by a nucleic acid that hybridizes to the "RelA (p65)" nucleic acid under low stringency conditions,
   (xxix) "PTP-BAS" (SEQ ID No:147) or a functionally active derivative thereof, or a functionally active fragment thereof, or a homolog thereof, or a variant of "PTP-BAS" encoded by a nucleic acid that hybridizes to the "PTP-BAS" nucleic acid under low stringency conditions,
   (xxx) "β TrCP2 - 1" (SEQ ID No:148) or a functionally active derivative thereof, or a functionally active fragment thereof, or a homolog thereof, or a variant of "β TrCP2 - 1" encoded by a nucleic acid that hybridizes to the "β TrCP2 - 1" nucleic acid under low stringency conditions,
   (xxxi) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (xxxii) "ASB-3 PROTEIN" (SEQ ID No:149) or a functionally active derivative thereof, or a functionally active fragment thereof, or a homolog thereof, or a variant of "ASB-3 PROTEIN" encoded by a nucleic acid that hybridizes to the "ASB-3 PROTEIN" nucleic acid under low stringency conditions,
   (xxxiii) "CDNA FLJ14103 FIS, CLONE MAMMA1001073" (SEQ ID No:150) or a functionally active derivative thereof,or a functionally active fragement thereof, or a homolog thereof, or a variant of "CDNA FLJ14103 FIS, CLONE MAMMA1001073" that hybridizes to the "CDNA FLJ14103 FIS, CLONE MAMMA1001073" nucleic acid under low stringency conditions, and
   (xxxiv) "SERINE HYDROXYMETHYLTRANSFERASE, MITOCHONDRIAL PRECURSOR" (SEQ ID No:151) or a functionally active derivative thereof, or a functionally active fragment thereof, or a homolog thereof, or a variant of "SERINE HYDROXYMETHYLTRANSFERASE, MITOCHONDRIAL PRECURSOR" encoded by a nucleic acid that hybridizes to the "SERINE HYDROXYMETHYLTRANSFERASE, MITOCHONDRIAL PRECURSOR" nucleic acid under low stringency conditions.
4. The protein complex according to No. 1 selected from complex (I) comprising all but 1 - 9 of the following proteins:
   (i) "κB - ras2" (SEQ ID No:131) or a functionally active derivative thereof, or a functionally active fragment thereof, or a homolog thereof, or a variant of "κB - ras2" encoded by a nucleic acid that hybridizes to the "κB - ras2" nucleic acid under low stringency conditions,
   (ii) "κB - ras1" (SEQ ID No:132) or a functionally active derivative thereof, or a functionally active fragment thereof, or a homolog thereof, or a variant of "κB - ras1" encoded by a nucleic acid that hybridizes to the "κB - ras1" nucleic acid under low stringency conditions,
   (iii) "β TrCP1" (SEQ ID No:129) or a functionally active derivative thereof, or a functionally active fragment thereof, or a homolog thereof, or a variant of "β TrCP1" encoded by a nucleic acid that hybridizes to the "β TrCP1" nucleic acid under low stringency conditions,
   (iv) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (v) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
   (vi) "p100 (NF-KB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (vii) "SKP2 (SCF complex component)" (SEQ ID No:133) or a functionally active derivative thereof, or a functionally active fragment thereof, or a homolog thereof, or a variant of "SKP2 (SCF COMPLEX COMPONTENT" encoded by a nucleic acid that hybridizes to the "SKP2 (SCF COMPLEX COMPONENT" nucleic acid under low stringency conditions,
   (viii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (ix) "hnRNPA1" (SEQ ID No:134) or a functionally active derivative thereof, or a functionally active fragment thereof, or a homolog thereof, or a variant of "hnRNPA1" encoded by a nucleic acid that hybridizes to the "hnRNPA1" nucleic acid under low stringency conditions,
   (x) "SKP1 (SCF complex complonent)" (SEQ ID No:135) or a functionally active derivative thereof, or a functionally active fragment thereof, or a homolog thereof, or a variant of "SKP1 (SCF COMPLEX COMPONTENT" encoded by a nucleic acid that hybridizes to the "SKP1 (SCF COMPLEX COMPONENT" nucleic acid under low stringency conditions,
   (xi) "Cullin 1" (SEQ ID No: 136) or a functionally active derivative thereof, or a functionally active fragment thereof, or a homolog thereof, or a variant of "Cullin 1" encoded by a nucleic acid that hybridizes to the "Cullin 1" nucleic acid under low stringency conditions,
   (xii) "Hos1" (SEQ ID No: 137) or a functionally active derivative thereof, or a functionally active fragment thereof, or a homolog thereof, or a variant of "Hos1" encoded by a nucleic acid that hybridizes to the "Hos1" nucleic acid under low stringency conditions,
   (xiii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (xiv) "ReIB" (SEQ ID No:81) or a functionally active derivative thereof, or a functionally active fragment thereof, or a homolog thereof, or a variant of "ReIB" encoded by a nucleic acid that hybridizes to the "ReIB" nucleic acid under low stringency conditions,
   (xv) "karyopherin α2" (SEQ ID No:138) or a functionally active derivative thereof, or a functionally active fragment thereof, or a homolog thereof, or a variant of "karyopherin α2" encoded by a nucleic acid that hybridizes to the "karyopherin α2" nucleic acid under low stringency conditions,
   (xvi) "karyoherin α1" (SEQ ID No:103) or a functionally active derivative thereof, or a functionally active fragment thereof, or a homolog thereof, or a variant of "karyopherin α1" encoded by a nucleic acid that hybridizes to the "karyopherin α1" nucleic acid under low stringency conditions,
   (xvii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (xviii) "CDNA FLJ10422 FIS, CLONE NT2RP1000243" (SEQ ID No:139) or a functionally active derivative thereof, or a functionally active fragment thereof, or a homolog thereof, or a variant of "CDNA FLJ10422 FIS, CLONE NT2RP1000243" encoded by a nucleic acid that hybridizes to the "CDNA FLJ10422 FIS, CLONE NT2RP1000243" nucleic acid under low stringency conditions,
   (xix) "DNA REPLICATION LICENSING FACTOR MCM5" (SEQ ID No:140) or a functionally active derivative thereof, or a functionally active fragment thereof, or a homolog thereof, or a variant of "DNA REPLICATION LICENSING FACTOR MCM5" encoded by a nucleic acid that hybridizes to the "DNA REPLICATION LICENSING FACTOR MCM5" nucleic acid under low stringency conditions,
   (xx) "DNA REPLICATION LICENSING FACTOR MCM3" (SEQ ID No:141) or a functionally active derivative thereof, or a functionally active fragment thereof, or a homolog thereof, or a variant of "DNA REPLICATION LICENSING FACTOR MCM3" encoded by a nucleic acid that hybridizes to the "DNA REPLICATION LICENSING FACTOR MCM3" nucleic acid under low stringency conditions,
   (xxi) "DNA REPLICATION LICENSING FACTOR MCM7" (SEQ ID No:142) or a functionally active derivative thereof, or a functionally active fragment thereof, or a homolog thereof, or a variant of "DNA REPLICATION LICENSING FACTOR MCM7" encoded by a nucleic acid that hybridizes to the "DNA REPLICATION LICENSING FACTOR MCM7" nucleic acid under low stringency conditions,
   (xxii) "SIMILAR TO RIKEN CDNA 2610005H11 GENE" (SEQ ID No:143) or a functionally active derivative thereof, or a functionally active fragment thereof, or a homolog thereof, or a variant of "SIMILAR TO RIKEN CDNA 2610005H11 GENE" encoded by a nucleic acid that hybridizes to the "SIMILAR TO RIKEN CDNA 2610005H11 GENE" nucleic acid under low stringency conditions,
   (xxiii) "HYDROXYACYL-COENZYME A DEHYDROGENASE/3-KETOACYL-COENZYME A THIOLASE/ENOYL-COENZYME A HYDRATASE (TRIFUNCTIONAL PROTEIN), κ SUBUNIT" (SEQ ID No:144) or a functionally active derivative thereof, or a functionally active fragment thereof, or a homolog thereof, or a variant of "HYDROXYACYL-COENZYME A DEHYDROGENASE/3-KETOACYL-COENZYME A THIOLASE/ENOYL-COENZYME A HYDRATASE (TRIFUNCTIONAL PROTEIN), κ SUBUNIT" encoded by a nucleic acid that hybridizes to the "HYDROXYACYL-COENZYME A DEHYDROGENASE/3-KETOACYL-COENZYME A THIOLASE/ENOYL-COENZYME A HYDRATASE (TRIFUNCTIONAL PROTEIN), κ SUBUNIT" nucleic acid under low stringency conditions,
   (xxiv) "MEVALONATE KINASE" (SEQ ID No:145) or a functionally active derivative thereof, or a functionally active fragment thereof, or a homolog thereof, or a variant of "MEVALONATE KINASE" encoded by a nucleic acid that hybridizes to the "MEVALONATE KINASE" nucleic acid under low stringency conditions,
   (xxv) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (xxvi) "Roc1 / RBX1" (SEQ ID No:146) or a functionally active derivative thereof, or a functionally active fragment thereof, or a homolog thereof, or a variant of "Roc/RBX1" encoded by a nucleic acid that hybridizes to the "Roc1/RBX1" nucleic acid under low stringency conditions,
   (xxvii) "IκBα" (SEQ ID No:78) or a functionally active derivative thereof, or a functionally active fragment thereof, or a homolog thereof, or a variant of "IκBα" encoded by a nucleic acid that hybridizes to the "IκBα" nucleic acid under low stringency conditions,
   (xxviii) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions,
   (xxix) "PTP-BAS" (SEQ ID No:147) or a functionally active derivative thereof, or a functionally active fragment thereof, or a homolog thereof, or a variant of "PTP-BAS" encoded by a nucleic acid that hybridizes to the "PTP-BAS" nucleic acid under low stringency conditions,
   (xxx) "β TrCP2 - 1" (SEQ ID No:148) or a functionally active derivative thereof, or a functionally active fragment thereof, or a homolog thereof, or a variant of "β TrCP2 - 1" encoded by a nucleic acid that hybridizes to the "β TrCP2 - 1" nucleic acid under low stringency conditions,
   (xxxi) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (xxxii) "ASB-3 PROTEIN" (SEQ ID No:149) or a functionally active derivative thereof, or a functionally active fragment thereof, or a homolog thereof, or a variant of "ASB-3 PROTEIN" encoded by a nucleic acid that hybridizes to the "ASB-3 PROTEIN" nucleic acid under low stringency conditions,
   (xxxiii) "CDNA FLJ14103 FIS, CLONE MAMMA1001073" (SEQ ID No:150) or a functionally active derivative thereof,or a functionally active fragement thereof, or a homolog thereof, or a variant of "CDNA FLJ14103 FIS, CLONE MAMMA1001073" that hybridizes to the "CDNA FLJ14103 FIS, CLONE MAMMA1001073" nucleic acid under low stringency conditions, and

   (i) (xxxiv) "SERINE HYDROXYMETHYLTRANSFERASE, MITOCHONDRIAL PRECURSOR" (SEQ ID No:151 ) or a functionally active derivative thereof, or a functionally active fragment thereof, or a homolog thereof, or a variant of "SERINE HYDROXYMETHYLTRANSFERASE, MITOCHONDRIAL PRECURSOR" encoded by a nucleic acid that hybridizes to the "SERINE HYDROXYMETHYLTRANSFERASE, MITOCHONDRIAL PRECURSOR" nucleic acid under low stringency conditions;
   and a protein complex selected from complex (II) comprising all but 1 - 9 of the following proteins:
   (ii) "β TrCP1" (SEQ ID No:129) or a functionally active derivative thereof, or a functionally active fragment thereof, or a homolog thereof, or a variant of "β TrCP1" encoded by a nucleic acid that hybridizes to the "β TrCP1" nucleic acid under low stringency conditions,
   (iii) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (iv) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,

   (i) "p100 (NF-KB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (vi) "SKP2 (SCF complex component)" (SEQ ID No:133) or a functionally active derivative thereof, or a functionally active fragment thereof, or a homolog thereof, or a variant of "SKP2 (SCF COMPLEX COMPONTENT" encoded by a nucleic acid that hybridizes to the "SKP2 (SCF COMPLEX COMPONENT" nucleic acid under low stringency conditions,

   (i) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions, (viii) "SKP1 (SCF complex complonent)" (SEQ ID No:135) or a functionally active derivative thereof, or a functionally active fragment thereof, or a homolog thereof, or a variant of "SKP1 (SCF COMPLEX COMPONTENT" encoded by a nucleic acid that hybridizes to the "SKP1 (SCF COMPLEX COMPONENT" nucleic acid under low stringency conditions,
   (ix) "Cullin 1" (SEQ ID No:136) or a functionally active derivative thereof, or a functionally active fragment thereof, or a homolog thereof, or a variant of "Cullin 1" encoded by a nucleic acid that hybridizes to the "Cullin 1" nucleic acid under low stringency conditions,
   (x) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (xi) (xvii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (xii) (xviii) "CDNA FLJ10422 FIS, CLONE NT2RP1000243" (SEQ ID No:139) or a functionally active derivative thereof, or a functionally active fragment thereof, or a homolog thereof, or a variant of "CDNA FLJ10422 FIS, CLONE NT2RP1000243" encoded by a nucleic acid that hybridizes to the "CDNA FLJ10422 FIS, CLONE NT2RP1000243" nucleic acid under low stringency conditions,
   (xiii) (xix) "DNA REPLICATION LICENSING FACTOR MCM5" (SEQ ID No:140) or a functionally active derivative thereof, or a functionally active fragment thereof, or a homolog thereof, or a variant of "DNA REPLICATION LICENSING FACTOR MCM5" encoded by a nucleic acid that hybridizes to the "DNA REPLICATION LICENSING FACTOR MCM5" nucleic acid under low stringency conditions,
   (xiv) "DNA REPLICATION LICENSING FACTOR MCM3" (SEQ ID No:141) or a functionally active derivative thereof, or a functionally active fragment thereof, or a homolog thereof, or a variant of "DNA REPLICATION LICENSING FACTOR MCM3" encoded by a nucleic acid that hybridizes to the "DNA REPLICATION LICENSING FACTOR MCM3" nucleic acid under low stringency conditions,
   (xv) "DNA REPLICATION LICENSING FACTOR MCM7" (SEQ ID No:142) or a functionally active derivative thereof, or a functionally active fragment thereof, or a homolog thereof, or a variant of "DNA REPLICATION LICENSING FACTOR MCM7" encoded by a nucleic acid that hybridizes to the "DNA REPLICATION LICENSING FACTOR MCM7" nucleic acid under low stringency conditions,
   (xvi) "SIMILAR TO RIKEN CDNA 2610005H11 GENE" (SEQ ID No:143) or a functionally active derivative thereof, or a functionally active fragment thereof, or a homolog thereof, or a variant of "SIMILAR TO RIKEN CDNA 2610005H11 GENE" encoded by a nucleic acid that hybridizes to the "SIMILAR TO RIKEN CDNA 2610005H11 GENE" nucleic acid under low stringency conditions,

   (i) "HYDROXYACYL-COENZYME A DEHYDROGENASE/3-KETOACYL-COENZYME A THIOLASE/ENOYL-COENZYME A HYDRATASE (TRIFUNCTIONAL PROTEIN), α SUBUNIT" (SEQ ID No:144) or a functionally active derivative thereof, or a functionally active fragment thereof, or a homolog thereof, or a variant of "HYDROXYACYL-COENZYME A DEHYDROGENASE/3-KETOACYL-COENZYME A THIOLASE/ENOYL-COENZYME A HYDRATASE (TRIFUNCTIONAL PROTEIN), α SUBUNIT" encoded by a nucleic acid that hybridizes to the "HYDROXYACYL-COENZYME A DEHYDROGENASE/3-KETOACYL-COENZYME A THIOLASE/ENOYL-COENZYME A HYDRATASE (TRIFUNCTIONAL PROTEIN), α SUBUNIT" nucleic acid under low stringency conditions,
   (xviii) "MEVALONATE KINASE" (SEQ ID No:145) or a functionally active derivative thereof, or a functionally active fragment thereof, or a homolog thereof, or a variant of "MEVALONATE KINASE" encoded by a nucleic acid that hybridizes to the "MEVALONATE KINASE" nucleic acid under low stringency conditions,

   (i) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (xx) "Roc1 / RBX1" (SEQ ID No:146) or a functionally active derivative thereof, or a functionally active fragment thereof, or a homolog thereof, or a variant of "Roc/RBX1" encoded by a nucleic acid that hybridizes to the "Roc1/RBX1" nucleic acid under low stringency conditions,

   (i) "IκBα" (SEQ ID No:78) or a functionally active derivative thereof, or a functionally active fragment thereof, or a homolog thereof, or a variant of "IκBα" encoded by a nucleic acid that hybridizes to the "IκBα" nucleic acid under low stringency conditions,
   (xxii) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions,
   (xxiii) "PTP-BAS" (SEQ ID No:147) or a functionally active derivative thereof, or a functionally active fragment thereof, or a homolog thereof, or a variant of "PTP-BAS" encoded by a nucleic acid that hybridizes to the "PTP-BAS" nucleic acid under low stringency conditions,
   (xxiv) "β TrCP2 - 1" (SEQ ID No:148) or a functionally active derivative thereof, or a functionally active fragment thereof, or a homolog thereof, or a variant of "β TrCP2 - 1" encoded by a nucleic acid that hybridizes to the "β TrCP2 - 1" nucleic acid under low stringency conditions,
   (xxv) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (xxvi) "ASB-3 PROTEIN" (SEQ ID No:149) or a functionally active derivative thereof, or a functionally active fragment thereof, or a homolog thereof, or a variant of "ASB-3 PROTEIN" encoded by a nucleic acid that hybridizes to the "ASB-3 PROTEIN" nucleic acid under low stringency conditions,
   (xxvii) "CDNA FLJ14103 FIS, CLONE MAMMA1001073" (SEQ ID No:150) or a functionally active derivative thereof,or a functionally active fragement thereof, or a homolog thereof, or a variant of "CDNA FLJ14103 FIS, CLONE MAMMA1001073" that hybridizes to the "CDNA FLJ14103 FIS, CLONE MAMMA1001073" nucleic acid under low stringency conditions, and
   (xxviii) "SERINE HYDROXYMETHYL TRANSFERASE, MITOCHONDRIAL PRECURSOR" (SEQ ID No:151 ) or a functionally active derivative thereof, or a functionally active fragment, or a homolog thereof, or a variant of SERINE HYDROXYMETHYLTRANSFERASE, MITOCHONDRIAL PRECURSOR that hybridises to the SERINE HYDROXYMETHYLTRANSFERASE, MITOCHONDRIAL PRECURSOR nucleic acid under low stringency conditions.
5. The complex of any of No. 1 - 4 comprising a functionally active derivative of said first protein and/or a functionally active derivative of said second protein, wherein the functionally active derivative is a fusion protein comprising said first protein or said second protein fused to an amino acid sequence different from the first protein or second protein, respectively.
6. The complex of No. 5 wherein the functionally active derivative is a fusion protein comprising said first protein or said second protein fused to an affinity tag or label.
7. The complex of any of No. 1-4 comprising a fragment of said first protein and/or a fragment of said second protein, which fragment binds to another protein component of said complex.
8. The complex of any of No. 1 -7 that is involved in the nuclear import activity or deubiquitination activity.
9. A process for preparing complex of any of No. 1 - 8 and optionally the components thereof comprising the following steps:
   the expressing a protein (bait) of the complex, preferably a tagged protein, in a target cell, isolating the protein complex which is attached to the bait protein, and optionally disassociating the protein complex and isolating the individual complex members.
10. The process according to No. 9 wherein the tagged protein comprises two different tags which allow two separate affinity purification steps.
11. The process according to any of No. 9-10 wherein the two tags are separated by a cleavage site for a protease.
12. Component of the IκBα complex obtainable by a process according to any of No. 9 to 11.
13. Protein of the IκBα complex selected from
   (i) "CDNA FLJ10422 FIS, CLONE NT2RP1000243" (SEQ ID No:139) or a functionally active derivative thereof, or a functionally active fragment thereof, or a homolog thereof, or a variant of "CDNA FLJ10422 FIS, CLONE NT2RP1000243" encoded by a nucleic acid that hybridizes to the "CDNA FLJ10422 FIS, CLONE NT2RP1000243" nucleic acid under low stringency conditions,
   (ii) "SIMILAR TO RIKEN CDNA 2610005H11 GENE" (SEQ ID No:143) or a functionally active derivative thereof, or a functionally active fragment thereof, or a homolog thereof, or a variant of "SIMILAR TO RIKEN CDNA 2610005H11 GENE" encoded by a nucleic acid that hybridizes to the "SIMILAR TO RIKEN CDNA 2610005H11 GENE" nucleic acid under low stringency conditions, and
   (iii) "CDNA FLJ14103 FIS, CLONE MAMMA1001073" (SEQ ID No:150) or a functionally active derivative thereof, or a functionally active fragment thereof, or a homolog thereof, or a variant of "CDNA FLJ14103 FIS, CLONE MAMMA1001073" encoded by a nucleic acid that hybridizes to the "CDNA FLJ14103 FIS, CLONE MAMMA1001073" nucleic acid under low stringency conditions,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
14. Nucleic acid encoding a protein according to No. 13.
15. Construct, preferably a vector construct, comprising (a) a nucleic acid according to No. 14 and at least one further nucleic acid which is normally not associated with said nucleic acid,or(b) at least two separate nucleic acid sequences each encoding a different protein, or a functionally active fragment or a functionally active derivative thereof at least one of said proteins, or functionally active fragments or functionally active derivative thereof being selected from the first group of proteins according to No. 1(a) and at least one of said proteins, or functionally active fragments or functionally active derivative thereof being selected from the second group of proteins according to No. 1 (b).
16. Host cell, containing a vector comprising at least one of the nucleic acid of No. 14 and/or a construct of No. 15 or containing several vectors each comprising at least the nucleic acid sequence encoding at least one of the proteins, or functionally active fragments or functionally active derivatives thereof selected from the first group of proteins according to No. 1(a) and the proteins, or functionally active fragments or functionally active derivatives thereof selected from the second group of proteins according to No. 1(b).
17. An antibody or a fragment of said antibody containing the binding domain thereof, selected from an antibody or fragment thereof, which binds the complex of any of No. 1-8 and which does not bind any of the proteins of said complex when uncomplexed and an antibody or a fragment of said antibody which binds to any of the proteins according to No. 13.
18. A kit comprising in one or more container the complex of any of No. 1-8 and/or the proteins of No. 13 optionally together with an antibody according to No. 17 and/or further components such as reagents and working instructions.
19. The kit according to No.18 for processing a substrate of said complex.
20. The kit according to No. 18 for the diagnosis or prognosis of a disease or a disease risk, preferentially for a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
21. Array, in which at least a complex according to any of No. 1 - 8 and/or at least one protein according to No. 14 and/or at least one antibody according to No. 17 is attached to a solid carrier.
22. A process for processing a physiological substrate of the complex comprising the step of bringing into contact a complex to any of No. 1 - 8 with said substrate, such that said substrate is processed.
23. A pharmaceutical composition comprising the protein complex of any of No. 1 to 8 and/or any of the following the proteins:
   (i) "CDNA FLJ10422 FIS, CLONE NT2RP1000243" (SEQ ID No:139) or a functionally active derivative thereof, or a functionally active fragment thereof, or a homolog thereof, or a variant of "CDNA FLJ10422 FIS, CLONE NT2RP1000243" encoded by a nucleic acid that hybridizes to the "CDNA FLJ10422 FIS, CLONE NT2RP1000243" nucleic acid under low stringency conditions,
   (ii) "SIMILAR TO RIKEN CDNA 2610005H11 GENE" (SEQ ID No:143) or a functionally active derivative thereof,or a functionally active fragement thereof, or a homolog thereof, or a variant of "SIMILAR TO RIKEN CDNA 2610005H11 GENE" that hybridizes to the "SIMILAR TO RIKEN CDNA 2610005H11 GENE" nucleic acid under low stringency conditions, and
   (iii) "CDNA FLJ14103 FIS, CLONE MAMMA1001073" (SEQ ID No:150) or a functionally active derivative thereof, or a functionally active fragment thereof, or a homolog thereof, or a variant of "CDNA FLJ14103 FIS, CLONE MAMMA1001073" encoded by a nucleic acid that hybridizes to the "CDNA FLJ14103 FIS, CLONE MAMMA1001073" nucleic acid under low stringency conditions,
   and a pharmaceutical acceptable carrier.
24. The pharmaceutical composition according to No. 23 for the treatment of diseases and disorders such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
25. A method for screening for a molecule that binds to the complex of anyone of No.1 - 8 and/or any of the following proteins:
   (i) "CDNA FLJ10422 FIS, CLONE NT2RP1000243" (SEQ ID No:139) or a functionally active derivative thereof, or a functionally active fragment thereof, or a homolog thereof, or a variant of "CDNA FLJ10422 FIS, CLONE NT2RP1000243" encoded by a nucleic acid that hybridizes to the "CDNA FLJ10422 FIS, CLONE NT2RP1000243" nucleic acid under low stringency conditions,
   (ii) "SIMILAR TO RIKEN CDNA 2610005H11 GENE" (SEQ ID No:143) or a functionally active derivative thereof,or a functionally active fragement thereof, or a homolog thereof, or a variant of "SIMILAR TO RIKEN CDNA 2610005H11 GENE" that hybridizes to the "SIMILAR TO RIKEN CDNA 2610005H11 GENE" nucleic acid under low stringency conditions, and
   (iii) "CDNA FLJ14103 FIS, CLONE MAMMA1001073" (SEQ ID No:150) or a functionally active derivative thereof, or a functionally active fragment thereof, or a homolog thereof, or a variant of "CDNA FLJ14103 FIS, CLONE MAMMA1001073" encoded by a nucleic acid that hybridizes to the "CDNA FLJ14103 FIS, CLONE MAMMA1001073" nucleic acid under low stringency conditions, comprising the steps of
      (a) exposing said complex, or a cell or organism containing same to one or more candidate molecules; and
      (b) determinig whether said candidate molecule is bound to the complex or protein.
26. A method for screening for a molecule that modulates directly or indirectly the function, activity, composition or formation of the complex of any one of No. 1 - 8 comprising the steps of (a) exposing said complex, or a cell or organism containing IκBα complex to one or more candidate molecules; and
   (b) determining the amount of activity of protein components of, and/or intracellular localization of, said complex and/or the transcription level of a gene dependent on the complex and/or the abundance and/or activity of a protein or protein complex dependent on the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependent on the complex and/or the abundance and/or activity of a protein or protein complex dependent on the function of the complex and/or product of a gene dependent on the complex in the absence of said candidate molecules indicates that the molecule modulates function, activity or composition of said complex.
27. The method of No. 26, wherein the amount of said complex is determined.
28. The method of No. 26, wherein the activity of said complex is determined.
29. The method of No. 28, wherein said determining step comprises isolating from the cell or organism said complex to produce said isolated complex and contacting said isolated complex in the presence or absence of a candidate molecule with a substrate of said complex and determining the processing of said substrate is modified in the presence of said candidate molecule.
30. The method of No. 26, wherein the amount of the individual protein components of said complex are determined.
31. The method of No. 30, determining step comprises determining whether
   (i) "κB - ras2" (SEQ ID No:131) or a functionally active derivative thereof, or a functionally active fragment thereof, or a homolog thereof, or a variant of "κB - ras2" encoded by a nucleic acid that hybridizes to the "κB - ras2" nucleic acid under low stringency conditions, and/or
   (ii) "κB - ras1" (SEQ ID No:132) or a functionally active derivative thereof, or a functionally active fragment thereof, or a homolog thereof, or a variant of "κB - ras1" encoded by a nucleic acid that hybridizes to the "κB - ras1" nucleic acid under low stringency conditions, and/or
   (iii) "β TrCP1" (SEQ ID No:129) or a functionally active derivative thereof, or a functionally active fragment thereof, or a homolog thereof, or a variant of "β TrCP1" encoded by a nucleic acid that hybridizes to the "β TrCP1" nucleic acid under low stringency conditions, and/or
   (iv) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions, and/or
   (v) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions, and/or
   (vi) "p100 (NF-KB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions, and/or
   (vii) "SKP2 (SCF complex component)" (SEQ ID No:133) or a functionally active derivative thereof, or a functionally active fragment thereof, or a homolog thereof, or a variant of "SKP2 (SCF COMPLEX COMPONTENT" encoded by a nucleic acid that hybridizes to the "SKP2 (SCF COMPLEX COMPONENT" nucleic acid under low stringency conditions, and/or
   (viii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions, and/or
   (ix) "hnRNPA1" (SEQ ID No:134) or a functionally active derivative thereof, or a functionally active fragment thereof, or a homolog thereof, or a variant of "hnRNPA1" encoded by a nucleic acid that hybridizes to the "hnRNPA1" nucleic acid under low stringency conditions, and/or
   (x) "SKP1 (SCF complex complonent)" (SEQ ID No:135) or a functionally active derivative thereof, or a functionally active fragment thereof, or a homolog thereof, or a variant of "SKP1 (SCF COMPLEX COMPONTENT" encoded by a nucleic acid that hybridizes to the "SKP1 (SCF COMPLEX COMPONENT" nucleic acid under low stringency conditions, and/or
   (xi) "Cullin1" (SEQ ID No: 136) or a functionally active derivative thereof, or a functionally active fragment thereof, or a homolog thereof, or a variant of "Cullin1" encoded by a nucleic acid that hybridizes to the "Cullin1" nucleic acid under low stringency conditions, and/or
   (xii) "Hos1" (SEQ ID No:137) or a functionally active derivative thereof, or a functionally active fragment thereof, or a homolog thereof, or a variant of "Hos1" encoded by a nucleic acid that hybridizes to the "Hos1" nucleic acid under low stringency conditions, and/or
   (xiii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions, and/or
   (xiv) "ReIB" (SEQ ID No:81) or a functionally active derivative thereof, or a functionally active fragment thereof, or a homolog thereof, or a variant of "ReIB" encoded by a nucleic acid that hybridizes to the "ReIB" nucleic acid under low stringency conditions, and/or
   (xv) "karyopherin α2" (SEQ ID No:138) or a functionally active derivative thereof, or a functionally active fragment thereof, or a homolog thereof, or a variant of "karyopherin α2" encoded by a nucleic acid that hybridizes to the "karyopherin α2" nucleic acid under low stringency conditions, and/or
   (xvi) "karyoherin α1" (SEQ ID No:103) or a functionally active derivative thereof, or a functionally active fragment thereof, or a homolog thereof, or a variant of "karyopherin α1" encoded by a nucleic acid that hybridizes to the "karyopherin α1" nucleic acid under low stringency conditions, and/or
   (xvii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions, and/or
   (xviii) "CDNA FLJ10422 FIS, CLONE NT2RP1000243" (SEQ ID No:139) or a functionally active derivative thereof, or a functionally active fragment thereof, or a homolog thereof, or a variant of "CDNA FLJ10422 FIS, CLONE NT2RP1000243" encoded by a nucleic acid that hybridizes to the "CDNA FLJ10422 FIS, CLONE NT2RP1000243" nucleic acid under low stringency conditions, and/or
   (xix) "DNA REPLICATION LICENSING FACTOR MCM5" (SEQ ID No:140) or a functionally active derivative thereof, or a functionally active fragment thereof, or a homolog thereof, or a variant of "DNA REPLICATION LICENSING FACTOR MCM5" encoded by a nucleic acid that hybridizes to the "DNA REPLICATION LICENSING FACTOR MCM5" nucleic acid under low stringency conditions, and/or
   (xx) "DNA REPLICATION LICENSING FACTOR MCM3" (SEQ ID No:141) or a functionally active derivative thereof, or a functionally active fragment thereof, or a homolog thereof, or a variant of "DNA REPLICATION LICENSING FACTOR MCM3" encoded by a nucleic acid that hybridizes to the "DNA REPLICATION LICENSING FACTOR MCM3" nucleic acid under low stringency conditions, and/or
   (xxi) "DNA REPLICATION LICENSING FACTOR MCM7" (SEQ ID No:142) or a functionally active derivative thereof, or a functionally active fragment thereof, or a homolog thereof, or a variant of "DNA REPLICATION LICENSING FACTOR MCM7" encoded by a nucleic acid that hybridizes to the "DNA REPLICATION LICENSING FACTOR MCM7" nucleic acid under low stringency conditions, and/or
   (xxii) "SIMILAR TO RIKEN CDNA 2610005H11 GENE" (SEQ ID No:143) or a functionally active derivative thereof, or a functionally active fragment thereof, or a homolog thereof, or a variant of "SIMILAR TO RIKEN CDNA 2610005H11 GENE" encoded by a nucleic acid that hybridizes to the "SIMILAR TO RIKEN CDNA 2610005H11 GENE" nucleic acid under low stringency conditions, and/or
   (xxiii) "HYDROXYACYL-COENZYME A DEHYDROGENASE/3-KETOACYL-COENZYME A THIOLASE/ENOYL-COENZYME A HYDRATASE (TRIFUNCTIONAL PROTEIN), A SUBUNIT" (SEQ ID No:144) or a functionally active derivative thereof, or a functionally active fragment thereof, or a homolog thereof, or a variant of "HYDROXYACYL-COENZYME A DEHYDROGENASE/3-KETOACYL-COENZYME A THIOLASE/ENOYL-COENZYME A HYDRATASE (TRIFUNCTIONAL PROTEIN), A SUBUNIT" encoded by a nucleic acid that hybridizes to the "HYDROXYACYL-COENZYME A DEHYDROGENASE/3-KETOACYL-COENZYME A THIOLASE/ENOYL-COENZYME A HYDRATASE (TRIFUNCTIONAL PROTEIN), α SUBUNIT" nucleic acid under low stringency conditions, and/or
   (xxiv) "MEVALONATE KINASE" (SEQ ID No:145) or a functionally active derivative thereof, or a functionally active fragment thereof, or a homolog thereof, or a variant of "MEVALONATE KINASE" encoded by a nucleic acid that hybridizes to the "MEVALONATE KINASE" nucleic acid under low stringency conditions, and/or
   (xxv) "IKK α" (SEQ ID No:25) or a functionally active derivative thereof, or a functionally active fragment thereof, or a homolog thereof, or a variant of "IKK α" encoded by a nucleic acid that hybridizes to the "I KK α" nucleic acid under low stringency conditions, and/or
   (xxvi) "Roc1 / RBX1" (SEQ ID No:146) or a functionally active derivative thereof, or a functionally active fragment thereof, or a homolog thereof, or a variant of "Roc/RBX1" encoded by a nucleic acid that hybridizes to the "Roc1/RBX1" nucleic acid under low stringency conditions, and/or
   (xxvii) "IκBα" (SEQ ID No:78) or a functionally active derivative thereof, or a functionally active fragment thereof, or a homolog thereof, or a variant of "IκBα" encoded by a nucleic acid that hybridizes to the "IκBα" nucleic acid under low stringency conditions, and/or
   (xxviii) "RelA (p65)" (SEQ ID No:75) or a functionally active derivative thereof, or a functionally active fragment thereof, or a homolog thereof, or a variant of "RelA (p65)" encoded by a nucleic acid that hybridizes to the "RelA (p65)" nucleic acid under low stringency conditions, and/or
   (xxix) "PTP-BAS" (SEQ ID No:147) or a functionally active derivative thereof, or a functionally active fragment thereof, or a homolog thereof, or a variant of "PTP-BAS" encoded by a nucleic acid that hybridizes to the "PTP-BAS" nucleic acid under low stringency conditions, and/or
   (xxx) "β TrCP2 - 1" (SEQ ID No:148) or a functionally active derivative thereof, or a functionally active fragment thereof, or a homolog thereof, or a variant of "β TrCP2 - 1" encoded by a nucleic acid that hybridizes to the "β TrCP2 - 1" nucleic acid under low stringency conditions, and/or
   (xxxi) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions, and/or
   (xxxii) "ASB-3 PROTEIN" (SEQ ID No:149) or a functionally active derivative thereof, or a functionally active fragment thereof, or a homolog thereof, or a variant of "ASB-3 PROTEIN" encoded by a nucleic acid that hybridizes to the "ASB-3 PROTEIN" nucleic acid under low stringency conditions, and/or
   (xxxiii) "CDNA FLJ14103 FIS, CLONE MAMMA1001073" (SEQ ID No:150) or a functionally active derivative thereof, or a functionally active fragment thereof, or a homolog thereof, or a variant of "CDNA FLJ14103 FIS, CLONE MAMMA1001073" encoded by a nucleic acid that hybridizes to the "CDNA FLJ14103 FIS, CLONE MAMMA1001073" nucleic acid under low stringency conditions, and/or
   (xxxiv) "SERINE HYDROXYMETHYLTRANSFERASE, MITOCHONDRIAL PRECURSOR" (SEQ ID No: 151) or a functionally active derivative thereof, or a functionally active fragment thereof, or a homolog thereof, or a variant of "SERINE HYDROXYMETHYLTRANSFERASE, MITOCHONDRIAL PRECURSOR" encoded by a nucleic acid that hybridizes to the "SERINE HYDROXYMETHYLTRANSFERASE, MITOCHONDRIAL PRECURSOR" nucleic acid under low stringency conditions, is present in the complex.
32. The method of any of No. 26 to 31, wherein said method is a method of screening for a drug for treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
33. Use of a molecule that modulates the amount of, activity of, or the protein components of the complex of any one of No. 1 - 8 for the manufacture of a medicament for the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
34. A method for the production of a pharmaceutical composition comprising carrying out the method of any of No. 1 - 8 to identify a molecule that modulates the function, activity, composition or formation of said complex, and further comprising mixing the identified molecule with a pharmaceutically acceptable carrier.
35. A method for diagnosing or screening for the presence of a disease or disorder or a predisposition for developing a disease or disorder in a subject, which disease or disorder is characterized by an aberrant amount of, activity of, or component composition of, or intracellular localization of the complex of any one of the No. 1 - 8, comprising determining the amount of, activity of, protein components of, and/or intracellular localization of, said complex and/or the transcription level of a gene dependent on the complex and/or the abundance and/or activity of a protein or protein complex dependent on the function of the complex and/or product of a gene dependent on the complex in a comparative sample derived from a subject, wherein a difference in said amount, activity, or protein components of, said complex in an analogous sample from a subject not having the disease or disorder or predisposition indicates the presence in the subject of the disease or disorder or predisposition in the subject.
36. The method of No. 35, wherein the amount of said complex is determined.
37. The method of No. 35, wherein the activity of said complex is determined.
38. The method of No. 37, wherein said determining step comprises isolating from the subject said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
39. The method of No. 35, wherein the amount of the individual protein components of said complex are determined.
40. The method of No. 39, wherein said determining step comprises determining whether
   (i) "κB - ras2" (SEQ ID No:131) or a functionally active derivative thereof, or a functionally active fragment thereof, or a homolog thereof, or a variant of "κB - ras2" encoded by a nucleic acid that hybridizes to the "κB - ras2" nucleic acid under low stringency conditions, and/or
   (ii) "κB - ras1" (SEQ ID No:132) or a functionally active derivative thereof, or a functionally active fragment thereof, or a homolog thereof, or a variant of "κB - ras1" encoded by a nucleic acid that hybridizes to the "κB - ras1" nucleic acid under low stringency conditions, and/or
   (iii) "β TrCP1" (SEQ ID No:129) or a functionally active derivative thereof, or a functionally active fragment thereof, or a homolog thereof, or a variant of "β TrCP1" encoded by a nucleic acid that hybridizes to the "β TrCP1" nucleic acid under low stringency conditions, and/or
   (iv) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions, and/or
   (v) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions, and/or
   (vi) "p100 (NF-KB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions, and/or
   (vii) "SKP2 (SCF complex component)" (SEQ ID No:133) or a functionally active derivative thereof, or a functionally active fragment thereof, or a homolog thereof, or a variant of "SKP2 (SCF COMPLEX COMPONTENT" encoded by a nucleic acid that hybridizes to the "SKP2 (SCF COMPLEX COMPONENT" nucleic acid under low stringency conditions, and/or
   (viii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions, and/or
   (ix) "hnRNPA1" (SEQ ID No:134) or a functionally active derivative thereof, or a functionally active fragment thereof, or a homolog thereof, or a variant of "hnRNPA1" encoded by a nucleic acid that hybridizes to the "hnRNPA1" nucleic acid under low stringency conditions, and/or
   (x) "SKP1 (SCF complex complonent)" (SEQ ID No: 135) or a functionally active derivative thereof, or a functionally active fragment thereof, or a homolog thereof, or a variant of "SKP1 (SCF COMPLEX COMPONTENT" encoded by a nucleic acid that hybridizes to the "SKP1 (SCF COMPLEX COMPONENT" nucleic acid under low stringency conditions, and/or
   (xi) "Cullin1" (SEQ ID No:136) or a functionally active derivative thereof, or a functionally active fragment thereof, or a homolog thereof, or a variant of "Cullin 1" encoded by a nucleic acid that hybridizes to the "Cullin 1" nucleic acid under low stringency conditions, and/or
   (xii) "Hos1" (SEQ ID No: 137) or a functionally active derivative thereof, or a functionally active fragment thereof, or a homolog thereof, or a variant of "Hos1" encoded by a nucleic acid that hybridizes to the "Hos1" nucleic acid under low stringency conditions, and/or
   (xiii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions, and/or
   (xiv) "ReIB" (SEQ ID No:81) or a functionally active derivative thereof, or a functionally active fragment thereof, or a homolog thereof, or a variant of "ReIB" encoded by a nucleic acid that hybridizes to the "ReIB" nucleic acid under low stringency conditions, and/or
   (xv) "karyopherin α2" (SEQ ID No:138) or a functionally active derivative thereof, or a functionally active fragment thereof, or a homolog thereof, or a variant of "karyopherin α2" encoded by a nucleic acid that hybridizes to the "karyopherin α2" nucleic acid under low stringency conditions, and/or
   (xvi) "karyoherin α1" (SEQ ID No:103) or a functionally active derivative thereof, or a functionally active fragment thereof, or a homolog thereof, or a variant of "karyopherin α1" encoded by a nucleic acid that hybridizes to the "karyopherin α1" nucleic acid under low stringency conditions, and/or
   (xvii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions, and/or
   (xviii) "CDNA FLJ10422 FIS, CLONE NT2RP1000243" (SEQ ID No:139) or a functionally active derivative thereof, or a functionally active fragment thereof, or a homolog thereof, or a variant of "CDNA FLJ10422 FIS, CLONE NT2RP1000243" encoded by a nucleic acid that hybridizes to the "CDNA FLJ10422 FIS, CLONE NT2RP1000243" nucleic acid under low stringency conditions, and/or
   (xix) "DNA REPLICATION LICENSING FACTOR MCM5" (SEQ ID No: 140) or a functionally active derivative thereof, or a functionally active fragment thereof, or a homolog thereof, or a variant of "DNA REPLICATION LICENSING FACTOR MCM5" encoded by a nucleic acid that hybridizes to the "DNA REPLICATION LICENSING FACTOR MCM5" nucleic acid under low stringency conditions, and/or
   (xx) "DNA REPLICATION LICENSING FACTOR MCM3" (SEQ ID No:141) or a functionally active derivative thereof, or a functionally active fragment thereof, or a homolog thereof, or a variant of "DNA REPLICATION LICENSING FACTOR MCM3" encoded by a nucleic acid that hybridizes to the "DNA REPLICATION LICENSING FACTOR MCM3" nucleic acid under low stringency conditions, and/or
   (xxi) "DNA REPLICATION LICENSING FACTOR MCM7" (SEQ ID No:142) or a functionally active derivative thereof, or a functionally active fragment thereof, or a homolog thereof, or a variant of "DNA REPLICATION LICENSING FACTOR MCM7" encoded by a nucleic acid that hybridizes to the "DNA REPLICATION LICENSING FACTOR MCM7" nucleic acid under low stringency conditions, and/or
   (xxii) "SIMILAR TO RIKEN CDNA 2610005H11 GENE" (SEQ ID No:143) or a functionally active derivative thereof, or a functionally active fragment thereof, or a homolog thereof, or a variant of "SIMILAR TO RIKEN CDNA 2610005H11 GENE" encoded by a nucleic acid that hybridizes to the "SIMILAR TO RIKEN CDNA 2610005H11 GENE" nucleic acid under low stringency conditions, and/or
   (xxiii) "HYDROXYACYL-COENZYME A DEHYDROGENASE/3-KETOACYL-COENZYME A THIOLASE/ENOYL-COENZYME A HYDRATASE (TRIFUNCTIONAL PROTEIN), α SUBUNIT" (SEQ ID No:144) or a functionally active derivative thereof, or a functionally active fragment thereof, or a homolog thereof, or a variant of "HYDROXYACYL-COENZYME A DEHYDROGENASE/3-KETOACYL-COENZYME A THIOLASE/ENOYL-COENZYME A HYDRATASE (TRIFUNCTIONAL PROTEIN), α SUBUNIT" encoded by a nucleic acid that hybridizes to the "HYDROXYACYL-COENZYME A DEHYDROGENASE/3-KETOACYL-COENZYME A THIOLASE/ENOYL-COENZYME A HYDRATASE (TRIFUNCTIONAL PROTEIN), α SUBUNIT" nucleic acid under low stringency conditions, and/or
   (xxiv) "MEVALONATE KINASE" (SEQ ID No:145) or a functionally active derivative thereof, or a functionally active fragment thereof, or a homolog thereof, or a variant of "MEVALONATE KINASE" encoded by a nucleic acid that hybridizes to the "MEVALONATE KINASE" nucleic acid under low stringency conditions, and/or
   (xxv) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions, and/or
   (xxvi) "Roc1 / RBX1" (SEQ ID No:146) or a functionally active derivative thereof, or a functionally active fragment thereof, or a homolog thereof, or a variant of "Roc/RBX1" encoded by a nucleic acid that hybridizes to the "Roc1/RBX1" nucleic acid under low stringency conditions, and/or
   (xxvii) "IκBα" (SEQ ID No:78) or a functionally active derivative thereof, or a functionally active fragment thereof, or a homolog thereof, or a variant of "IκBα" encoded by a nucleic acid that hybridizes to the "IκBα" nucleic acid under low stringency conditions, and/or
   (xxviii) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions, and/or
   (xxix) "PTP-BAS" (SEQ ID No:147) or a functionally active derivative thereof, or a functionally active fragment thereof, or a homolog thereof, or a variant of "PTP-BAS" encoded by a nucleic acid that hybridizes to the "PTP-BAS" nucleic acid under low stringency conditions, and/or
   (xxx) "β TrCP2 - 1" (SEQ ID No:148) or a functionally active derivative thereof, or a functionally active fragment thereof, or a homolog thereof, or a variant of "β TrCP2 - 1" encoded by a nucleic acid that hybridizes to the "β TrCP2 - 1" nucleic acid under low stringency conditions, and/or
   (xxxi) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions, and/or
   (xxxii) "ASB-3 PROTEIN" (SEQ ID No:149) or a functionally active derivative thereof, or a functionally active fragment thereof, or a homolog thereof, or a variant of "ASB-3 PROTEIN" encoded by a nucleic acid that hybridizes to the "ASB-3 PROTEIN" nucleic acid under low stringency conditions, and/or
   (xxxiii) "CDNA FLJ14103 FIS, CLONE MAMMA1001073" (SEQ ID No:150) or a functionally active derivative thereof, or a functionally active fragment thereof, or a homolog thereof, or a variant of "CDNA FLJ14103 FIS, CLONE MAMMA1001073" encoded by a nucleic acid that hybridizes to the "CDNA FLJ14103 FIS, CLONE MAMMA1001073" nucleic acid under low stringency conditions, and/or
   (xxxiv) "SERINE HYDROXYMETHYLTRANSFERASE, MITOCHONDRIAL PRECURSOR" (SEQ ID No:151) or a functionally active derivative thereof, or a functionally active fragment thereof, or a homolog thereof, or a variant of "SERINE HYDROXYMETHYLTRANSFERASE, MITOCHONDRIAL PRECURSOR" encoded by a nucleic acid that hybridizes to the "SERINE HYDROXYMETHYLTRANSFERASE, MITOCHONDRIAL PRECURSOR" nucleic acid under low stringency conditions, is present in the complex.
41. The complex of any one of No. 1 - 8, or proteins of No. 13 or the antibody or fragment of No. 17, for use in a method of diagnosing a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
42. A method for treating or preventing a disease or disorder characterized by an aberrant amount of, activity or component composition of or intracellular localization of, the complex of anyone of No. 1-8, comprising administering to a subject in need of such treatment or prevention a therapeutically effective amount of one or more molecules that modulate the amount of, nuclear import activity or deubiquitination activity, or protein components of, said complex.
43. The method according to No. 42, wherein said disease or disorder involves decreased levels of the amount or activity of said complex.
44. The method according to No. 42 , wherein said disease or disorder involves increased levels of the amount or activity of said complex.
45. Complex of any of No. 1 - 8 and/or protein selected from the following proteins
   (i) "κB - ras2" (SEQ ID No:131) or a functionally active derivative thereof, or a functionally active fragment thereof, or a homolog thereof, or a variant of "κB - ras2" encoded by a nucleic acid that hybridizes to the "κB - ras2" nucleic acid under low stringency conditions,
   (ii) "κB - ras1" (SEQ ID No:132) or a functionally active derivative thereof, or a functionally active fragment thereof, or a homolog thereof, or a variant of "κB - ras1" encoded by a nucleic acid that hybridizes to the "κB - ras1" nucleic acid under low stringency conditions,
   (iii) "β TrCP1" (SEQ ID No:129) or a functionally active derivative thereof, or a functionally active fragment thereof, or a homolog thereof, or a variant of "β TrCP1" encoded by a nucleic acid that hybridizes to the "β TrCP1" nucleic acid under low stringency conditions,
   (iv) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (v) "c-ReI" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
   (vi) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (vii) "SKP2 (SCF complex component)" (SEQ ID No:133) or a functionally active derivative thereof, or a functionally active fragment thereof, or a homolog thereof, or a variant of "SKP2 (SCF COMPLEX COMPONTENT" encoded by a nucleic acid that hybridizes to the "SKP2 (SCF COMPLEX COMPONENT" nucleic acid under low stringency conditions,
   (viii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (ix) "hnRNPA1" (SEQ ID No:134) or a functionally active derivative thereof, or a functionally active fragment thereof, or a homolog thereof, or a variant of "hnRNPA1" encoded by a nucleic acid that hybridizes to the "hnRNPA1" nucleic acid under low stringency conditions,
   (x) "SKP1 (SCF complex complonent)" (SEQ ID No:135) or a functionally active derivative thereof, or a functionally active fragment thereof, or a homolog thereof, or a variant of "SKP1 (SCF COMPLEX COMPONTENT" encoded by a nucleic acid that hybridizes to the "SKP1 (SCF COMPLEX COMPONENT" nucleic acid under low stringency conditions,
   (xi) "Cullin1" (SEQ ID No:136) or a functionally active derivative thereof, or a functionally active fragment thereof, or a homolog thereof, or a variant of "Cullin 1" encoded by a nucleic acid that hybridizes to the "Cullin 1" nucleic acid under low stringency conditions,
   (xii) "Hos1" (SEQ ID No:137) or a functionally active derivative thereof, or a functionally active fragment thereof, or a homolog thereof, or a variant of "Hos1" encoded by a nucleic acid that hybridizes to the "Hos1" nucleic acid under low stringency conditions,
   (xiii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (xiv) "ReIB" (SEQ ID No:81) or a functionally active derivative thereof, or a functionally active fragment thereof, or a homolog thereof, or a variant of "ReIB" encoded by a nucleic acid that hybridizes to the "ReIB" nucleic acid under low stringency conditions,
   (xv) "karyopherin α2" (SEQ ID No:138) or a functionally active derivative thereof, or a functionally active fragment thereof, or a homolog thereof, or a variant of "karyopherin α2" encoded by a nucleic acid that hybridizes to the "karyopherin α2" nucleic acid under low stringency conditions,
   (xvi) "karyoherin α1" (SEQ ID No:103) or a functionally active derivative thereof, or a functionally active fragment thereof, or a homolog thereof, or a variant of "karyopherin α1" encoded by a nucleic acid that hybridizes to the "karyopherin α1" nucleic acid under low stringency conditions,
   (xvii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (xviii) "CDNA FLJ10422 FIS, CLONE NT2RP1000243" (SEQ ID No:139) or a functionally active derivative thereof, or a functionally active fragment thereof, or a homolog thereof, or a variant of "CDNA FLJ10422 FIS, CLONE NT2RP1000243" encoded by a nucleic acid that hybridizes to the "CDNA FLJ10422 FIS, CLONE NT2RP1000243" nucleic acid under low stringency conditions,
   (xix) "DNA REPLICATION LICENSING FACTOR MCM5" (SEQ ID No:140) or a functionally active derivative thereof, or a functionally active fragment thereof, or a homolog thereof, or a variant of "DNA REPLICATION LICENSING FACTOR MCM5" encoded by a nucleic acid that hybridizes to the "DNA REPLICATION LICENSING FACTOR MCM5" nucleic acid under low stringency conditions,
   (xx) "DNA REPLICATION LICENSING FACTOR MCM3" (SEQ ID No:141) or a functionally active derivative thereof, or a functionally active fragment thereof, or a homolog thereof, or a variant of "DNA REPLICATION LICENSING FACTOR MCM3" encoded by a nucleic acid that hybridizes to the "DNA REPLICATION LICENSING FACTOR MCM3" nucleic acid under low stringency conditions,
   (xxi) "DNA REPLICATION LICENSING FACTOR MCM7" (SEQ ID No:142) or a functionally active derivative thereof, or a functionally active fragment thereof, or a homolog thereof, or a variant of "DNA REPLICATION LICENSING FACTOR MCM7" encoded by a nucleic acid that hybridizes to the "DNA REPLICATION LICENSING FACTOR MCM7" nucleic acid under low stringency conditions,
   (xxii) "SIMILAR TO RIKEN CDNA 2610005H11 GENE" (SEQ ID No:143) or a functionally active derivative thereof, or a functionally active fragment thereof, or a homolog thereof, or a variant of "SIMILAR TO RIKEN CDNA 2610005H11 GENE" encoded by a nucleic acid that hybridizes to the "SIMILAR TO RIKEN CDNA 2610005H11 GENE" nucleic acid under low stringency conditions,
   (xxiii) "HYDROXYACYL-COENZYME A DEHYDROGENASE/3-KETOACYL-COENZYME A THIOLASE/ENOYL-COENZYME A HYDRATASE (TRIFUNCTIONAL PROTEIN), α SUBUNIT" (SEQ ID No: 144) or a functionally active derivative thereof, or a functionally active fragment thereof, or a homolog thereof, or a variant of "HYDROXYACYL-COENZYME A DEHYDROGENASE/3-KETOACYL-COENZYME A THIOLASE/ENOYL-COENZYME A HYDRATASE (TRIFUNCTIONAL PROTEIN), α SUBUNIT" encoded by a nucleic acid that hybridizes to the "HYDROXYACYL-COENZYME A DEHYDROGENASE/3-KETOACYL-COENZYME A THIOLASE/ENOYL-COENZYME A HYDRATASE (TRIFUNCTIONAL PROTEIN), α SUBUNIT" nucleic acid under low stringency conditions,
   (xxiv) "MEVALONATE KINASE" (SEQ ID No:145) or a functionally active derivative thereof, or a functionally active fragment thereof, or a homolog thereof, or a variant of "MEVALONATE KINASE" encoded by a nucleic acid that hybridizes to the "MEVALONATE KINASE" nucleic acid under low stringency conditions,
   (xxv) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (xxvi) "Roc1 / RBX1" (SEQ ID No:146) or a functionally active derivative thereof, or a functionally active fragment thereof, or a homolog thereof, or a variant of "Roc/RBX1" encoded by a nucleic acid that hybridizes to the "Roc1/RBX1" nucleic acid under low stringency conditions,
   (xxvii) "IκBα" (SEQ ID No:78) or a functionally active derivative thereof, or a functionally active fragment thereof, or a homolog thereof, or a variant of "IκBα" encoded by a nucleic acid that hybridizes to the "IκBα" nucleic acid under low stringency conditions,
   (xxviii) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions,
   (xxix) "PTP-BAS" (SEQ ID No:147) or a functionally active derivative thereof, or a functionally active fragment thereof, or a homolog thereof, or a variant of "PTP-BAS" encoded by a nucleic acid that hybridizes to the "PTP-BAS" nucleic acid under low stringency conditions,
   (xxx) "β TrCP2 - 1" (SEQ ID No:148) or a functionally active derivative thereof, or a functionally active fragment thereof, or a homolog thereof, or a variant of "β TrCP2 - 1" encoded by a nucleic acid that hybridizes to the "β TrCP2 - 1" nucleic acid under low stringency conditions,
   (xxxi) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (xxxii) "ASB-3 PROTEIN" (SEQ ID No:149) or a functionally active derivative thereof, or a functionally active fragment thereof, or a homolog thereof, or a variant of "ASB-3 PROTEIN" encoded by a nucleic acid that hybridizes to the "ASB-3 PROTEIN" nucleic acid under low stringency conditions,
   (xxxiii) "CDNA FLJ14103 FIS, CLONE MAMMA1001073" (SEQ ID No:150) or a functionally active derivative thereof,or a functionally active fragement thereof, or a homolog thereof, or a variant of "CDNA FLJ14103 FIS, CLONE MAMMA1001073" that hybridizes to the "CDNA FLJ14103 FIS, CLONE MAMMA1001073" nucleic acid under low stringency conditions, and
   (xxxiv) "SERINE HYDROXYMETHYLTRANSFERASE, MITOCHONDRIAL PRECURSOR" (SEQ ID No:151) or a functionally active derivative thereof, or a functionally active fragment thereof, or a homolog thereof, or a variant of "SERINE HYDROXYMETHYLTRANSFERASE, MITOCHONDRIAL PRECURSOR" encoded by a nucleic acid that hybridizes to the "SERINE HYDROXYMETHYLTRANSFERASE, MITOCHONDRIAL PRECURSOR" nucleic acid under low stringency conditions, as a target for an active agent of a pharmaceutical, preferably a drug target in the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.

The present invention further relates to the IκBβ protein complex
1. A protein complex selected from complex (I) and comprising
   (a) at least one first protein selected from the group consisting of:
      (i) "β-TrCP1" (SEQ ID No:129) or a functionally active derivative thereof, or a functionally active fragment thereof, or a homolog thereof, or a variant of "β- TrCP1" encoded by a nucleic acid that hybridizes to the "β-TrCP1" nucleic acid under low stringency conditions,
      (ii) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
      (iii) "β TrCP2 - 1" (SEQ ID No:148) or a functionally active derivative thereof, or a functionally active fragment thereof, or a homolog thereof, or a variant of "β TrCP2 - 1" encoded by a nucleic acid that hybridizes to the "β TrCP2 - 1" nucleic acid under low stringency conditions,
      (iv) "karyopherin α2" (SEQ ID No:138) or a functionally active derivative thereof, or a functionally active fragment thereof, or a homolog thereof, or a variant of "karyopherin α2" encoded by a nucleic acid that hybridizes to the "karyopherin α2" nucleic acid under low stringency conditions,
      (v) "karyopherin α1" (SEQ ID No:103) or a functionally active derivative thereof, or a functionally active fragment thereof, or a homolog thereof, or a variant of "karyopherin α1" encoded by a nucleic acid that hybridizes to the "karyopherin α1" nucleic acid under low stringency conditions,
      (vi) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
      (vii) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
      (viii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
      (ix) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
      (x) "Hos1" (SEQ ID No:137) or a functionally active derivative thereof, or a functionally active fragment thereof, or a homolog thereof, or a variant of "Hos1" encoded by a nucleic acid that hybridizes to the "Hos1" nucleic acid under low stringency conditions,
      (xi) "SKP1 (SCF complex component)" (SEQ ID No:135) or a functionally active derivative thereof, or a functionally active fragment thereof, or a homolog thereof, or a variant of "SKP1 (SCF COMPLEX COMPONENT)" encoded by a nucleic acid that hybridizes to the "SKP1 (SCF COMPLEX COMPONENT)" nucleic acid under low stringency conditions,
      (xii) "ReIB" (SEQ ID No:81) or a functionally active derivative thereof, or a functionally active fragment thereof, or a homolog thereof, or a variant of "ReIB" encoded by a nucleic acid that hybridizes to the "ReIB" nucleic acid under low stringency conditions,
      (xiii) "c-ReI" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
      (xiv) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
      (xv) "p100 (NF-κB1)" (SEQ ID No:74) or a functionally active derivative thereof, or a functionally active fragment thereof, or a homolog thereof, or a variant of "p100 (NF-κB1)" encoded by a nucleic acid that hybridizes to the "p100 (NF-κB1)" nucleic acid under low stringency conditions,
      (xvi) "Cullin 1" (SEQ ID No:136) or a functionally active derivative thereof, or a functionally active fragment thereof, or a homolog thereof, or a variant of "Cullin 1" encoded by a nucleic acid that hybridizes to the "Cullin 1" nucleic acid under low stringency conditions,
      (xvii) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions, and
      (xviii) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions,
      (xix) "Roc1/RBX1" (SEQ ID No:146) or a functionally active derivative thereof, or a functionally active fragment thereof, or a homolog thereof, or a variant of "Roc/RBX1" encoded by a nucleic acid that hybridizes to the "Roc1/RBX1" nucleic acid under low stringency conditions,
      (xx) "SKP2 (SCF complex component)" (SEQ ID No:133) or a functionally active derivative thereof, or a functionally active fragment thereof, or a homolog thereof, or a variant of "SKP2 (SCF COMPLEX COMPONTENT" encoded by a nucleic acid that hybridizes to the "SKP2 (SCF COMPLEX COMPONENT" nucleic acid under low stringency conditions,
      (xxi) "κB-Ras1 " (SEQ ID No:132) or a functionally active derivative thereof, or a functionally active fragment thereof, or a homolog thereof, or a variant of "κB - ras1" encoded by a nucleic acid that hybridizes to the "κB - ras1" nucleic acid under low stringency conditions,
      (xxii) "κB-Ras2" (SEQ ID No:131) or a functionally active derivative thereof, or a functionally active fragment thereof, or a homolog thereof, or a variant of "κB - ras2" encoded by a nucleic acid that hybridizes to the "κB - ras2" nucleic acid under low stringency conditions,
      and
   (b) at least one second protein, which second protein is selected from the group consisting of:
      (i) "DNA-DIRECTED RNA POLYMERASE II 14.4 KDA POLYPEPTIDE" (SEQ ID No:152) or a functionally active derivative thereof, or a functionally active fragment thereof, or a homolog thereof, or a variant of "DNA-DIRECTED RNA POLYMERASE II 14.4 KDA POLYPEPTIDE" encoded by a nucleic acid that hybridizes to the "DNA-DIRECTED RNA POLYMERASE II 14.4 KDA POLYPEPTIDE" nucleic acid under low stringency conditions,
      (ii) "G PROTEIN PATHWAY SUPPRESSOR 1" (SEQ ID No:153) or a functionally active derivative thereof, or a functionally active fragment thereof, or a homolog thereof, or a variant of "G PROTEIN PATHWAY SUPPRESSOR 1" encoded by a nucleic acid that hybridizes to the "G PROTEIN PATHWAY SUPPRESSOR 1" nucleic acid under low stringency conditions,

      (i) "KARYOPHERIN α6 (IMPORTIN α7)" (SEQ ID No:154) or a functionally active derivative thereof, or a functionally active fragment thereof, or a homolog thereof, or a variant of "KARYOPHERIN α6 (IMPORTIN α7)" encoded by a nucleic acid that hybridizes to the "KARYOPHERIN α6 (IMPORTIN α7)" nucleic acid under low stringency conditions,
      (iv) "CDNA: FLJ22055 FIS, CLONE HEP09645" (SEQ ID No: 155) or a functionally active derivative thereof, or a functionally active fragment thereof, or a homolog thereof, or a variant of "CDNA: FLJ22055 FIS, CLONE HEP09645" encoded by a nucleic acid that hybridizes to the "CDNA: FLJ22055 FIS, CLONE HEP09645" nucleic acid under low stringency conditions,
      (v) "HYPOTHETICAL 14.5 KDA PROTEIN" (SEQ ID No:156) or a functionally active derivative thereof, or a functionally active fragment thereof, or a homolog thereof, or a variant of "HYPOTHETICAL 14.5 KDA PROTEIN" encoded by a nucleic acid that hybridizes to the "HYPOTHETICAL 14.5 KDA PROTEIN" nucleic acid under low stringency conditions,
      (vi) "RNA-BINDING PROTEIN FUS" (SEQ ID No:157) or a functionally active derivative thereof, or a functionally active fragment thereof, or a homolog thereof, or a variant of "RNA-BINDING PROTEIN FUS" encoded by a nucleic acid that hybridizes to the "RNA-BINDING PROTEIN FUS" nucleic acid under low stringency conditions,
      (vii) "CD3E-ASSOCIATED PROTEIN" (SEQ ID No:158) or a functionally active derivative thereof, or a functionally active fragment thereof, or a homolog thereof, or a variant of "CD3E-ASSOCIATED PROTEIN" encoded by a nucleic acid that hybridizes to the "CD3E-ASSOCIATED PROTEIN" nucleic acid under low stringency conditions,
      (viii) "COLORECTAL MUTANT CANCER PROTEIN" (SEQ ID No:159) or a functionally active derivative thereof, or a functionally active fragment thereof, or a homolog thereof, or a variant of "COLORECTAL MUTANT CANCER PROTEIN" encoded by a nucleic acid that hybridizes to the "COLORECTAL MUTANT CANCER PROTEIN" nucleic acid under low stringency conditions,
      (ix) "DNA-DIRECTED RNA POLYMERASE I 16 KDA POLYPEPTIDE" (SEQ ID No: 160) or a functionally active derivative thereof, or a functionally active fragment thereof, or a homolog thereof, or a variant of "DNA-DIRECTED RNA POLYMERASE 116 KDA POLYPEPTIDE" encoded by a nucleic acid that hybridizes to the "DNA-DIRECTED RNA POLYMERASE I 16 KDA POLYPEPTIDE" nucleic acid under low stringency conditions,
      (x) "DNA-DIRECTED RNA POLYMERASE I 40 KDA POLYPEPTIDE" (SEQ ID No:161) or a functionally active derivative thereof, or a functionally active fragment thereof, or a homolog thereof, or a variant of "DNA-DIRECTED RNA POLYMERASE 140 KDA POLYPEPTIDE" encoded by a nucleic acid that hybridizes to the "DNA-DIRECTED RNA POLYMERASE I 40 KDA POLYPEPTIDE" nucleic acid under low stringency conditions,
      (xi) "DNA-DIRECTED RNA POLYMERASE I LARGEST SUBUNIT" (SEQ ID No:162) or a functionally active derivative thereof, or a functionally active fragment thereof, or a homolog thereof, or a variant of "DNA-DIRECTED RNA POLYMERASE I LARGEST SUBUNIT" encoded by a nucleic acid that hybridizes to the "DNA-DIRECTED RNA POLYMERASE I LARGEST SUBUNIT" nucleic acid under low stringency conditions,
      (xii) "DNA-DIRECTED RNA POLYMERASE II 7.6 KDA POLYPEPTIDE" (SEQ ID No:163) or a functionally active derivative thereof, or a functionally active fragment thereof, or a homolog thereof, or a variant of "DNA-DIRECTED RNA POLYMERASE II 7.6 KDA POLYPEPTIDE" encoded by a nucleic acid that hybridizes to the "DNA-DIRECTED RNA POLYMERASE II 7.6 KDA POLYPEPTIDE" nucleic acid under low stringency conditions,
      (xiii) "HYPOTHETICAL 47.2 KDA PROTEIN" (SEQ ID No:164) or a functionally active derivative thereof, or a functionally active fragment thereof, or a homolog thereof, or a variant of "HYPOTHETICAL 47.2 KDA PROTEIN" encoded by a nucleic acid that hybridizes to the "HYPOTHETICAL 47.2 KDA PROTEIN" nucleic acid under low stringency conditions,
      (xiv) "TRANSCRIPTION INITIATION FACTOR TFIID" (SEQ ID No:165) or a functionally active derivative thereof, or a functionally active fragment thereof, or a homolog thereof, or a variant of "TRANSCRIPTION INITIATION FACTOR TFIID" encoded by a nucleic acid that hybridizes to the "TRANSCRIPTION INITIATION FACTOR TFIID" nucleic acid under low stringency conditions,
      (xv) "TRANSCRIPTION-ASSOCIATED ZINC RIBBON PROTEIN" (SEQ ID No:166) or a functionally active derivative thereof, or a functionally active fragment thereof, or a homolog thereof, or a variant of "TRANSCRIPTION-ASSOCIATED ZINC RIBBON PROTEIN" encoded by a nucleic acid that hybridizes to the "TRANSCRIPTION-ASSOCIATED ZINC RIBBON PROTEIN" nucleic acid under low stringency conditions,
      (xvi) "PROGRAMED CELL DEATH PROTEIN 2" (SEQ ID No:167) or a functionally active derivative thereof, or a functionally active fragment thereof, or a homolog thereof, or a variant of "PROGRAMED CELL DEATH PROTEIN 2" encoded by a nucleic acid that hybridizes to the "PROGRAMED CELL DEATH PROTEIN 2" nucleic acid under low stringency conditions,
      (xvii) "DNA-DIRECTED RNA POLYMERASE II 23 KDA POLYPEPTIDE" (SEQ ID No: 168) or a functionally active derivative thereof, or a functionally active fragment thereof, or a homolog thereof, or a variant of "DNA-DIRECTED RNA POLYMERASE II 23 KDA POLYPEPTIDE" encoded by a nucleic acid that hybridizes to the "DNA-DIRECTED RNA POLYMERASE II 23 KDA POLYPEPTIDE" nucleic acid under low stringency conditions,
      (xviii) "RAS GTPASE-ACTIVATING-LIKE PROTEIN IQGAP2" (SEQ ID No:169) or a functionally active derivative thereof, or a functionally active fragment thereof, or a homolog thereof, or a variant of "RAS GTPASE-ACTIVATING-LIKE PROTEIN IQGAP2" encoded by a nucleic acid that hybridizes to the "RAS GTPASE-ACTIVATING-LIKE PROTEIN IQGAP2" nucleic acid under low stringency conditions,
      (xix) "CDNA FLJ14782 FIS, CLONE NT2RP4000524, HIGHLY SIMILAR TO MUS MUSCULUS SEC8 MRNA" (SEQ ID No:170) or a functionally active derivative thereof, or a functionally active fragment thereof, or a homolog thereof, or a variant of "CDNA FLJ14782 FIS, CLONE NT2RP4000524, HIGHLY SIMILAR TO MUS MUSCULUS SEC8 MRNA" encoded by a nucleic acid that hybridizes to the "CDNA FLJ14782 FIS, CLONE NT2RP4000524, HIGHLY SIMILAR TO MUS MUSCULUS SEC8 MRNA" nucleic acid under low stringency conditions,
      (xx) "DNA-DIRECTED RNA POLYMERASE I 135 KDA POLYPEPTIDE" (SEQ ID No: 171) or a functionally active derivative thereof, or a functionally active fragment thereof, or a homolog thereof, or a variant of "DNA-DIRECTED RNA POLYMERASE I 135 KDA POLYPEPTIDE" encoded by a nucleic acid that hybridizes to the "DNA-DIRECTED RNA POLYMERASE I 135 KDA POLYPEPTIDE" nucleic acid under low stringency conditions,
      (xxi) "DNA-DIRECTED RNA POLYMERASES I, II, AND III 17.1 KDA POLYPEPTIDE" (SEQ ID No:172) or a functionally active derivative thereof, or a functionally active fragment thereof, or a homolog thereof, or a variant of "DNA-DIRECTED RNA POLYMERASES I, II, AND III 17.1 KDA POLYPEPTIDE" encoded by a nucleic acid that hybridizes to the "DNA-DIRECTED RNA POLYMERASES I, II, AND III 17.1 KDA POLYPEPTIDE" nucleic acid under low stringency conditions,
      (xxii) "HYPOTHETICAL PROTEIN XP_108399" (SEQ ID No:173) or a functionally active derivative thereof, or a functionally active fragment thereof, or a homolog thereof, or a variant of "HYPOTHETICAL PROTEIN XP_108399" encoded by a nucleic acid that hybridizes to the "HYPOTHETICAL PROTEIN XP_108399" nucleic acid under low stringency conditions,
      (xxiii) "SIMILAR TO CG5341 GENE PRODUCT" (SEQ ID No:174) or a functionally active derivative thereof, or a functionally active fragment thereof, or a homolog thereof, or a variant of "SIMILAR TO CG5341 GENE PRODUCT" encoded by a nucleic acid that hybridizes to the "SIMILAR TO CG5341 GENE PRODUCT" nucleic acid under low stringency conditions,
      (xxiv) "HYPOTHETICAL PROTEIN XP_108582" (SEQ ID No:175) or a functionally active derivative thereof, or a functionally active fragment thereof, or a homolog thereof, or a variant of "HYPOTHETICAL PROTEIN XP_108582" encoded by a nucleic acid that hybridizes to the "HYPOTHETICAL PROTEIN XP_108582" nucleic acid under low stringency conditions, and
      (xxv) "SEC5 PROTEIN" (SEQ ID No:176) or a functionally active derivative thereof, or a functionally active fragment thereof, or a homolog thereof, or a variant of "SEC5 PROTEIN" encoded by a nucleic acid that hybridizes to the "SEC5 PROTEIN" nucleic acid under low stringency conditions;
   and a complex (II) comprising at least two of said second proteins,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
2. The protein complex according to claim 1 wherein the first protein is the protein "IκB β" (SEQ ID No:77), or a functionally active derivative thereof, or a functionally active fragment thereof, or a homolog thereof, or a variant of "IκB β" encoded by a nucleic acid that hybridizes to the "IκB β" nucleic acid under low stringency conditions.
3. The protein complex according to No. 1 selected from complex (I) and comprising the following proteins:
   (i) "CD3E-ASSOCIATED PROTEIN" (SEQ ID No:158) or a functionally active derivative thereof, or a functionally active fragment thereof, or a homolog thereof, or a variant of "CD3E-ASSOCIATED PROTEIN" encoded by a nucleic acid that hybridizes to the "CD3E-ASSOCIATED PROTEIN" nucleic acid under low stringency conditions,
   (ii) "CDNA FLJ14782 FIS, CLONE NT2RP4000524, HIGHLY SIMILAR TO MUS MUSCULUS SEC8 MRNA" (SEQ ID No:170) or a functionally active derivative thereof, or a functionally active fragment thereof, or a homolog thereof, or a variant of "CDNA FLJ14782 FIS, CLONE NT2RP4000524, HIGHLY SIMILAR TO MUS MUSCULUS SEC8 MRNA" encoded by a nucleic acid that hybridizes to the "CDNA FLJ14782 FIS, CLONE NT2RP4000524, HIGHLY SIMILAR TO MUS MUSCULUS SEC8 MRNA" nucleic acid under low stringency conditions,
   (iii) "COLORECTAL MUTANT CANCER PROTEIN" (SEQ ID No:159) or a functionally active derivative thereof, or a functionally active fragment thereof, or a homolog thereof, or a variant of "COLORECTAL MUTANT CANCER PROTEIN" encoded by a nucleic acid that hybridizes to the "COLORECTAL MUTANT CANCER PROTEIN" nucleic acid under low stringency conditions,
   (iv) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
   (v) "DNA-DIRECTED RNA POLYMERASE I 135 KDA POLYPEPTIDE" (SEQ ID No:171) or a functionally active derivative thereof, or a functionally active fragment thereof, or a homolog thereof, or a variant of "DNA-DIRECTED RNA POLYMERASE I 135 KDA POLYPEPTIDE" encoded by a nucleic acid that hybridizes to the "DNA-DIRECTED RNA POLYMERASE I 135 KDA POLYPEPTIDE" nucleic acid under low stringency conditions,
   (vi) "DNA-DIRECTED RNA POLYMERASE I 16 KDA POLYPEPTIDE" (SEQ ID No:160) or a functionally active derivative thereof, or a functionally active fragment thereof, or a homolog thereof, or a variant of "DNA-DIRECTED RNA POLYMERASE I 16 KDA POLYPEPTIDE" encoded by a nucleic acid that hybridizes to the "DNA-DIRECTED RNA POLYMERASE I 16 KDA POLYPEPTIDE" nucleic acid under low stringency conditions,
   (vii) "DNA-DIRECTED RNA POLYMERASE I 40 KDA POLYPEPTIDE" (SEQ ID No:161) or a functionally active derivative thereof, or a functionally active fragment thereof, or a homolog thereof, or a variant of "DNA-DIRECTED RNA POLYMERASE I 40 KDA POLYPEPTIDE" encoded by a nucleic acid that hybridizes to the "DNA-DIRECTED RNA POLYMERASE I 40 KDA POLYPEPTIDE" nucleic acid under low stringency conditions,
   (viii) "DNA-DIRECTED RNA POLYMERASE II 23 KDA POLYPEPTIDE" (SEQ ID No:168) or a functionally active derivative thereof, or a functionally active fragment thereof, or a homolog thereof, or a variant of "DNA-DIRECTED RNA POLYMERASE II 23 KDA POLYPEPTIDE" encoded by a nucleic acid that hybridizes to the "DNA-DIRECTED RNA POLYMERASE II 23 KDA POLYPEPTIDE" nucleic acid under low stringency conditions,
   (ix) "DNA-DIRECTED RNA POLYMERASES I, II, AND III 17.1 KDA POLYPEPTIDE" (SEQ ID No:172) or a functionally active derivative thereof, or a functionally active fragment thereof, or a homolog thereof, or a variant of "DNA-DIRECTED RNA POLYMERASES I, II, AND III 17.1 KDA POLYPEPTIDE" encoded by a nucleic acid that hybridizes to the "DNA-DIRECTED RNA POLYMERASES I, II, AND III 17.1 KDA POLYPEPTIDE" nucleic acid under low stringency conditions,
   (x) "HYPOTHETICAL 47.2 KDA PROTEIN" (SEQ ID No:164) or a functionally active derivative thereof, or a functionally active fragment thereof, or a homolog thereof, or a variant of "HYPOTHETICAL 47.2 KDA PROTEIN" encoded by a nucleic acid that hybridizes to the "HYPOTHETICAL 47.2 KDA PROTEIN" nucleic acid under low stringency conditions,

   (i) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (xii) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (xiii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (xiv) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (xv) "karyopherin α2" (SEQ ID No:138) or a functionally active derivative thereof, or a functionally active fragment thereof, or a homolog thereof, or a variant of "karyopherin α2" encoded by a nucleic acid that hybridizes to the "karyopherin α2" nucleic acid under low stringency conditions,
   (xvi) "p100 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (xvii) "RelA (p65)" (SEQ ID No:75) or a functionally active derivative thereof, or a functionally active fragment thereof, or a homolog thereof, or a variant of "RelA (p65)" encoded by a nucleic acid that hybridizes to the "RelA (p65)" nucleic acid under low stringency conditions,
   (xviii) "RAS GTPASE-ACTIVATING-LIKE PROTEIN IQGAP2" (SEQ ID No:169) or a functionally active derivative thereof, or a functionally active fragment thereof, or a homolog thereof, or a variant of "RAS GTPASE-ACTIVATING-LIKE PROTEIN IQGAP2" encoded by a nucleic acid that hybridizes to the "RAS GTPASE-ACTIVATING-LIKE PROTEIN IQGAP2" nucleic acid under low stringency conditions,
   (xix) "TRANSCRIPTION-ASSOCIATED ZINC RIBBON PROTEIN" (SEQ ID No:166) or a functionally active derivative thereof, or a functionally active fragment thereof, or a homolog thereof, or a variant of "TRANSCRIPTION-ASSOCIATED ZINC RIBBON PROTEIN" encoded by a nucleic acid that hybridizes to the "TRANSCRIPTION-ASSOCIATED ZINC RIBBON PROTEIN" nucleic acid under low stringency conditions, and
   (xx) "DNA-DIRECTED RNA POLYMERASE I LARGEST SUBUNIT" (SEQ ID No:162) or a functionally active derivative thereof, or a functionally active fragment thereof, or a homolog thereof, or a variant of "DNA-DIRECTED RNA POLYMERASE I LARGEST SUBUNIT" encoded by a nucleic acid that hybridizes to the "DNA-DIRECTED RNA POLYMERASE I LARGEST SUBUNIT" nucleic acid under low stringency conditions;
   and a protein complex selected from complex (II) and comprising the following proteins:
   (i) "CD3E-ASSOCIATED PROTEIN" (SEQ ID No:158) or a functionally active derivative thereof, or a functionally active fragment thereof, or a homolog thereof, or a variant of "CD3E-ASSOCIATED PROTEIN" encoded by a nucleic acid that hybridizes to the "CD3E-ASSOCIATED PROTEIN" nucleic acid under low stringency conditions,
   (ii) "CDNA FLJ14782 FIS, CLONE NT2RP4000524, HIGHLY SIMILAR TO MUS MUSCULUS SEC8 MRNA" (SEQ ID No:170) or a functionally active derivative thereof, or a functionally active fragment thereof, or a homolog thereof, or a variant of "CDNA FLJ14782 FIS, CLONE NT2RP4000524, HIGHLY SIMILAR TO MUS MUSCULUS SEC8 MRNA" encoded by a nucleic acid that hybridizes to the "CDNA FLJ14782 FIS, CLONE NT2RP4000524, HIGHLY SIMILAR TO MUS MUSCULUS SEC8 MRNA" nucleic acid under low stringency conditions,
   (iii) "COLORECTAL MUTANT CANCER PROTEIN" (SEQ ID No:159) or a functionally active derivative thereof, or a functionally active fragment thereof, or a homolog thereof, or a variant of "COLORECTAL MUTANT CANCER PROTEIN" encoded by a nucleic acid that hybridizes to the "COLORECTAL MUTANT CANCER PROTEIN" nucleic acid under low stringency conditions,
   (iv) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
   (v) "DNA-DIRECTED RNA POLYMERASE I 135 KDA POLYPEPTIDE" (SEQ ID No:171) or a functionally active derivative thereof, or a functionally active fragment thereof, or a homolog thereof, or a variant of "DNA-DIRECTED RNA POLYMERASE I 135 KDA POLYPEPTIDE" encoded by a nucleic acid that hybridizes to the "DNA-DIRECTED RNA POLYMERASE I 135 KDA POLYPEPTIDE" nucleic acid under low stringency conditions,
   (vi) "DNA-DIRECTED RNA POLYMERASE I 16 KDA POLYPEPTIDE" (SEQ ID No: 160) or a functionally active derivative thereof, or a functionally active fragment thereof, or a homolog thereof, or a variant of "DNA-DIRECTED RNA POLYMERASE I 16 KDA POLYPEPTIDE" encoded by a nucleic acid that hybridizes to the "DNA-DIRECTED RNA POLYMERASE I 16 KDA POLYPEPTIDE" nucleic acid under low stringency conditions,
   (vii) "DNA-DIRECTED RNA POLYMERASE I 40 KDA POLYPEPTIDE" (SEQ ID No:161) or a functionally active derivative thereof, or a functionally active fragment thereof, or a homolog thereof, or a variant of "DNA-DIRECTED RNA POLYMERASE I 40 KDA POLYPEPTIDE" encoded by a nucleic acid that hybridizes to the "DNA-DIRECTED RNA POLYMERASE I 40 KDA POLYPEPTIDE" nucleic acid under low stringency conditions,
   (viii) "DNA-DIRECTED RNA POLYMERASE II 23 KDA POLYPEPTIDE" (SEQ ID No: 168) or a functionally active derivative thereof, or a functionally active fragment thereof, or a homolog thereof, or a variant of "DNA-DIRECTED RNA POLYMERASE II 23 KDA POLYPEPTIDE" encoded by a nucleic acid that hybridizes to the "DNA-DIRECTED RNA POLYMERASE II 23 KDA POLYPEPTIDE" nucleic acid under low stringency conditions,
   (ix) "DNA-DIRECTED RNA POLYMERASES I, 11, AND III 17.1 KDA POLYPEPTIDE" (SEQ ID No: 172) or a functionally active derivative thereof, or a functionally active fragment thereof, or a homolog thereof, or a variant of "DNA-DIRECTED RNA POLYMERASES I, II, AND III 17.1 KDA POLYPEPTIDE" encoded by a nucleic acid that hybridizes to the "DNA-DIRECTED RNA POLYMERASES I, II, AND III 17.1 KDA POLYPEPTIDE" nucleic acid under low stringency conditions,
   (x) "HYPOTHETICAL 47.2 KDA PROTEIN" (SEQ ID No:164) or a functionally active derivative thereof, or a functionally active fragment thereof, or a homolog thereof, or a variant of "HYPOTHETICAL 47.2 KDA PROTEIN" encoded by a nucleic acid that hybridizes to the "HYPOTHETICAL 47.2 KDA PROTEIN" nucleic acid under low stringency conditions,

   (i) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (xii) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (xiii) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (xiv) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (xv) "karyopherin α2" (SEQ ID No:138) or a functionally active derivative thereof, or a functionally active fragment thereof, or a homolog thereof, or a variant of "karyopherin α2" encoded by a nucleic acid that hybridizes to the "karyopherin α2" nucleic acid under low stringency conditions,
   (xvi) "p100 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (xvii) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the ''ReIA (p65)" nucleic acid under low stringency conditions,
   (xviii) "RAS GTPASE-ACTIVATING-LIKE PROTEIN IQGAP2" (SEQ ID No:169) or a functionally active derivative thereof, or a functionally active fragment thereof, or a homolog thereof, or a variant of "RAS GTPASE-ACTIVATING-LIKE PROTEIN IQGAP2" encoded by a nucleic acid that hybridizes to the "RAS GTPASE-ACTIVATING-LIKE PROTEIN IQGAP2" nucleic acid under low stringency conditions,
   (xix) "TRANSCRIPTION-ASSOCIATED ZINC RIBBON PROTEIN" (SEQ ID No:166) or a functionally active derivative thereof, or a functionally active fragment thereof, or a homolog thereof, or a variant of "TRANSCRIPTION-ASSOCIATED ZINC RIBBON PROTEIN" encoded by a nucleic acid that hybridizes to the "TRANSCRIPTION-ASSOCIATED ZINC RIBBON PROTEIN" nucleic acid under low stringency conditions,
   (xx) "DNA-DIRECTED RNA POLYMERASE I LARGEST SUBUNIT" (SEQ ID No:162) or a functionally active derivative thereof, or a functionally active fragment thereof, or a homolog thereof, or a variant of "DNA-DIRECTED RNA POLYMERASE I LARGEST SUBUNIT" encoded by a nucleic acid that hybridizes to the "DNA-DIRECTED RNA POLYMERASE I LARGEST SUBUNIT" nucleic acid under low stringency conditions,
   (xxi) "HYPOTHETICAL PROTEIN XP_108399" (SEQ ID No:173) or a functionally active derivative thereof, or a functionally active fragment thereof, or a homolog thereof, or a variant of "HYPOTHETICAL PROTEIN XP_108399" encoded by a nucleic acid that hybridizes to the "HYPOTHETICAL PROTEIN XP_108399" nucleic acid under low stringency conditions, and
   (xxii) "HYPOTHETICAL PROTEIN XP_108582" (SEQ ID No:175) or a functionally active derivative thereof,or a functionally active fragement thereof, or a homolog thereof, or a variant of "HYPOTHETICAL PROTEIN XP_108582" that hybridizes to the "HYPOTHETICAL PROTEIN XP_108582" nucleic acid under low stringency conditions; and a protein complex selected from complex (III) and comprising the following proteins:

   (i) "karyopherin α2" (SEQ ID No:138) or a functionally active derivative thereof, or a functionally active fragment thereof, or a homolog thereof, or a variant of "karyopherin α2" encoded by a nucleic acid that hybridizes to the "karyopherin α2" nucleic acid under low stringency conditions,
   (ii) "DNA-DIRECTED RNA POLYMERASE II 14.4 KDA POLYPEPTIDE" (SEQ ID No:152) or a functionally active derivative thereof, or a functionally active fragment thereof, or a homolog thereof, or a variant of "DNA-DIRECTED RNA POLYMERASE II 14.4 KDA POLYPEPTIDE" encoded by a nucleic acid that hybridizes to the "DNA-DIRECTED RNA POLYMERASE II 14.4 KDA POLYPEPTIDE" nucleic acid under low stringency conditions,
   (iii) "G PROTEIN PATHWAY SUPPRESSOR 1" (SEQ ID No:153) or a functionally active derivative thereof, or a functionally active fragment thereof, or a homolog thereof, or a variant of "G PROTEIN PATHWAY SUPPRESSOR 1" encoded by a nucleic acid that hybridizes to the "G PROTEIN PATHWAY SUPPRESSOR 1" nucleic acid under low stringency conditions,

   (i) "KARYOPHERIN α 6 (IMPORTIN α 7)" (SEQ ID No:154) or a functionally active derivative thereof, or a functionally active fragment thereof, or a homolog thereof, or a variant of "KARYOPHERIN α6 (IMPORTIN α7)" encoded by a nucleic acid that hybridizes to the "KARYOPHERIN α6 (IMPORTIN α7)" nucleic acid under low stringency conditions,
   (v) "karyopherin α1" (SEQ ID No:103) or a functionally active derivative thereof, or a functionally active fragment thereof, or a homolog thereof, or a variant of "karyopherin α1" encoded by a nucleic acid that hybridizes to the "karyopherin α1" nucleic acid under low stringency conditions,
   (vi) " CDNA: FLJ22055 FIS, CLONE HEP09645" (SEQ ID No:155) or a functionally active derivative thereof, or a functionally active fragment thereof, or a homolog thereof, or a variant of "CDNA: FLJ22055 FIS, CLONE HEP09645" encoded by a nucleic acid that hybridizes to the "CDNA: FLJ22055 FIS, CLONE HEP09645" nucleic acid under low stringency conditions,
   (vii) "HYPOTHETICAL 14.5 KDA PROTEIN" (SEQ ID No:156) or a functionally active derivative thereof, or a functionally active fragment thereof, or a homolog thereof, or a variant of "HYPOTHETICAL 14.5 KDA PROTEIN" encoded by a nucleic acid that hybridizes to the "HYPOTHETICAL 14.5 KDA PROTEIN" nucleic acid under low stringency conditions,
   (viii) "RNA-BINDING PROTEIN FUS" (SEQ ID No:157) or a functionally active derivative thereof, or a functionally active fragment thereof, or a homolog thereof, or a variant of "RNA-BINDING PROTEIN FUS" encoded by a nucleic acid that hybridizes to the "RNA-BINDING PROTEIN FUS" nucleic acid under low stringency conditions,

   (i) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (x) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (xi) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (xii) "c-Rel" (SEQ ID No:71) or a functionally active derivative thereof, or a functionally active fragment thereof, or a homolog thereof, or a variant of "c-ReI" encoded by a nucleic acid that hybridizes to the "c-Rel" nucleic acid under low stringency conditions,

   (i) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (xiv) "p100 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (xv) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (xvi) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions,
   (xvii) "CD3E-ASSOCIATED PROTEIN" (SEQ ID No:158) or a functionally active derivative thereof, or a functionally active fragment thereof, or a homolog thereof, or a variant of "CD3E-ASSOCIATED PROTEIN" encoded by a nucleic acid that hybridizes to the "CD3E-ASSOCIATED PROTEIN" nucleic acid under low stringency conditions,
   (xviii) "COLORECTAL MUTANT CANCER PROTEIN" (SEQ ID No:159) or a functionally active derivative thereof, or a functionally active fragment thereof, or a homolog thereof, or a variant of "COLORECTAL MUTANT CANCER PROTEIN" encoded by a nucleic acid that hybridizes to the "COLORECTAL MUTANT CANCER PROTEIN" nucleic acid under low stringency conditions,
   (xix) "DNA-DIRECTED RNA POLYMERASE 116 KDA POLYPEPTIDE" (SEQ ID No:160) or a functionally active derivative thereof, or a functionally active fragment thereof, or a homolog thereof, or a variant of "DNA-DIRECTED RNA POLYMERASE I 16 KDA POLYPEPTIDE" encoded by a nucleic acid that hybridizes to the "DNA-DIRECTED RNA POLYMERASE I 16 KDA POLYPEPTIDE" nucleic acid under low stringency conditions,
   (xx) "DNA-DIRECTED RNA POLYMERASE I 40 KDA POLYPEPTIDE" (SEQ ID No:161) or a functionally active derivative thereof, or a functionally active fragment thereof, or a homolog thereof, or a variant of "DNA-DIRECTED RNA POLYMERASE I 40 KDA POLYPEPTIDE" encoded by a nucleic acid that hybridizes to the "DNA-DIRECTED RNA POLYMERASE I 40 KDA POLYPEPTIDE" nucleic acid under low stringency conditions,
   (xxi) "DNA-DIRECTED RNA POLYMERASE I LARGEST SUBUNIT" (SEQ ID No:162) or a functionally active derivative thereof, or a functionally active fragment thereof, or a homolog thereof, or a variant of "DNA-DIRECTED RNA POLYMERASE LARGEST SUBUNIT" encoded by a nucleic acid that hybridizes to the "DNA-DIRECTED RNA POLYMERASE LARGEST SUBUNIT" nucleic acid under low stringency conditions,
   (xxii) "DNA-DIRECTED RNA POLYMERASE II 7.6 KDA POLYPEPTIDE" (SEQ ID No:163) or a functionally active derivative thereof, or a functionally active fragment thereof, or a homolog thereof, or a variant of "DNA-DIRECTED RNA POLYMERASE II 7.6 KDA POLYPEPTIDE" encoded by a nucleic acid that hybridizes to the "DNA-DIRECTED RNA POLYMERASE II 7.6 KDA POLYPEPTIDE" nucleic acid under low stringency conditions,
   (xxiii) "HYPOTHETICAL 47.2 KDA PROTEIN" (SEQ ID No:164) or a functionally active derivative thereof, or a functionally active fragment thereof, or a homolog thereof, or a variant of "HYPOTHETICAL 47.2 KDA PROTEIN" encoded by a nucleic acid that hybridizes to the "HYPOTHETICAL 47.2 KDA PROTEIN" nucleic acid under low stringency conditions,
   (xxiv) "TRANSCRIPTION INITIATION FACTOR TFIID" (SEQ ID No:165) or a functionally active derivative thereof, or a functionally active fragment thereof, or a homolog thereof, or a variant of "TRANSCRIPTION INITIATION FACTOR TFIID" encoded by a nucleic acid that hybridizes to the "TRANSCRIPTION INITIATION FACTOR TFIID" nucleic acid under low stringency conditions,
   (xxv) "TRANSCRIPTION-ASSOCIATED ZINC RIBBON PROTEIN" (SEQ ID No:166) or a functionally active derivative thereof, or a functionally active fragment thereof, or a homolog thereof, or a variant of "TRANSCRIPTION-ASSOCIATED ZINC RIBBON PROTEIN" encoded by a nucleic acid that hybridizes to the "TRANSCRIPTION-ASSOCIATED ZINC RIBBON PROTEIN" nucleic acid under low stringency conditions,
   (xxvi) "PROGRAMED CELL DEATH PROTEIN 2" (SEQ ID No:167) or a functionally active derivative thereof, or a functionally active fragment thereof, or a homolog thereof, or a variant of "PROGRAMED CELL DEATH PROTEIN 2" encoded by a nucleic acid that hybridizes to the "PROGRAMED CELL DEATH PROTEIN 2" nucleic acid under low stringency conditions,
   (xxvii) "DNA-DIRECTED RNA POLYMERASE II 23 KDA POLYPEPTIDE" (SEQ ID No:168) or a functionally active derivative thereof, or a functionally active fragment thereof, or a homolog thereof, or a variant of "DNA-DIRECTED RNA POLYMERASE II 23 KDA POLYPEPTIDE" encoded by a nucleic acid that hybridizes to the "DNA-DIRECTED RNA POLYMERASE II 23 KDA POLYPEPTIDE" nucleic acid under low stringency conditions,
   (xxviii) "RAS GTPASE-ACTIVATING-LIKE PROTEIN IQGAP2" (SEQ ID No:169) or a functionally active derivative thereof, or a functionally active fragment thereof, or a homolog thereof, or a variant of "RAS GTPASE-ACTIVATING-LIKE PROTEIN IQGAP2" encoded by a nucleic acid that hybridizes to the "RAS GTPASE-ACTIVATING-LIKE PROTEIN IQGAP2" nucleic acid under low stringency conditions,
   (xxix) "CDNA FLJ14782 FIS, CLONE NT2RP4000524, HIGHLY SIMILAR TO MUS MUSCULUS SEC8 MRNA" (SEQ ID No:170) or a functionally active derivative thereof, or a functionally active fragment thereof, or a homolog thereof, or a variant of "CDNA FLJ14782 FIS, CLONE NT2RP4000524, HIGHLY SIMILAR TO MUS MUSCULUS SEC8 MRNA" encoded by a nucleic acid that hybridizes to the "CDNA FLJ14782 FIS, CLONE NT2RP4000524, HIGHLY SIMILAR TO MUS MUSCULUS SEC8 MRNA" nucleic acid under low stringency conditions,
   (xxx) "DNA-DIRECTED RNA POLYMERASE I 135 KDA POLYPEPTIDE" (SEQ ID No: 171) or a functionally active derivative thereof, or a functionally active fragment thereof, or a homolog thereof, or a variant of "DNA-DIRECTED RNA POLYMERASE I 135 KDA POLYPEPTIDE" encoded by a nucleic acid that hybridizes to the "DNA-DIRECTED RNA POLYMERASE I 135 KDA POLYPEPTIDE" nucleic acid under low stringency conditions,
   (xxxi) "DNA-DIRECTED RNA POLYMERASES I, II, AND III 17.1 KDA POLYPEPTIDE" (SEQ ID No: 172) or a functionally active derivative thereof, or a functionally active fragment thereof, or a homolog thereof, or a variant of "DNA-DIRECTED RNA POLYMERASES I, II, AND III 17.1 KDA POLYPEPTIDE" encoded by a nucleic acid that hybridizes to the "DNA-DIRECTED RNA POLYMERASES I, II, AND III 17.1 KDA POLYPEPTIDE" nucleic acid under low stringency conditions,
   (xxxii) "SIMILAR TO CG5341 GENE PRODUCT" (SEQ ID No:174) or a functionally active derivative thereof,or a functionally active fragement thereof, or a homolog thereof, or a variant of "SIMILAR TO CG5341 GENE PRODUCT" that hybridizes to the "SIMILAR TO CG5341 GENE PRODUCT" nucleic acid under low stringency conditions, and
   (xxxiii) "SEC5 PROTEIN" (SEQ ID No:176) or a functionally active derivative thereof, or a homolog thereof, or a variant of "SEC5 PROTEIN" that hybridizes to the "SEC5 PROTEIN" nucleic acid under low stringency conditions;
   and a protein complex selected from complex (IV) and comprising the following proteins:
   (i) "karyopherin α2" (SEQ ID No:138) or a functionally active derivative thereof, or a functionally active fragment thereof, or a homolog thereof, or a variant of "karyopherin α2" encoded by a nucleic acid that hybridizes to the "karyopherin α2" nucleic acid under low stringency conditions,
   (ii) "DNA-DIRECTED RNA POLYMERASE II 14.4 KDA POLYPEPTIDE" (SEQ ID No: 152) or a functionally active derivative thereof, or a functionally active fragment thereof, or a homolog thereof, or a variant of "DNA-DIRECTED RNA POLYMERASE II 14.4 KDA POLYPEPTIDE" encoded by a nucleic acid that hybridizes to the "DNA-DIRECTED RNA POLYMERASE II 14.4 KDA POLYPEPTIDE" nucleic acid under low stringency conditions,
   (iii) "G PROTEIN PATHWAY SUPPRESSOR 1" (SEQ ID No:153) or a functionally active derivative thereof, or a functionally active fragment thereof, or a homolog thereof, or a variant of "G PROTEIN PATHWAY SUPPRESSOR 1" encoded by a nucleic acid that hybridizes to the "G PROTEIN PATHWAY SUPPRESSOR 1" nucleic acid under low stringency conditions,
   (iv) "KARYOPHERIN α 6 (IMPORTIN α 7)" (SEQ ID No:154) or a functionally active derivative thereof, or a functionally active fragment thereof, or a homolog thereof, or a variant of "KARYOPHERIN α 6 (IMPORTIN α 7)" encoded by a nucleic acid that hybridizes to the "KARYOPHERIN α 6 (IMPORTIN α 7)" nucleic acid under low stringency conditions,
   (v) "karyopherin α1" (SEQ ID No:103) or a functionally active derivative thereof, or a functionally active fragment thereof, or a homolog thereof, or a variant of "karyopherin α1" encoded by a nucleic acid that hybridizes to the "karyopherin α1" nucleic acid under low stringency conditions,
   (vi) "CDNA: FLJ22055 FIS, CLONE HEP09645" (SEQ ID No:155) or a functionally active derivative thereof, or a functionally active fragment thereof, or a homolog thereof, or a variant of "CDNA: FLJ22055 FIS, CLONE HEP09645" encoded by a nucleic acid that hybridizes to the "CDNA: FLJ22055 FIS, CLONE HEP09645" nucleic acid under low stringency conditions,
   (vii) "HYPOTHETICAL 14.5 KDA PROTEIN" (SEQ ID No:156) or a functionally active derivative thereof, or a functionally active fragment thereof, or a homolog thereof, or a variant of "HYPOTHETICAL 14.5 KDA PROTEIN" encoded by a nucleic acid that hybridizes to the "HYPOTHETICAL 14.5 KDA PROTEIN" nucleic acid under low stringency conditions,
   (viii) "RNA-BINDING PROTEIN FUS" (SEQ ID No:157) or a functionally active derivative thereof, or a functionally active fragment thereof, or a homolog thereof, or a variant of "RNA-BINDING PROTEIN FUS" encoded by a nucleic acid that hybridizes to the "RNA-BINDING PROTEIN FUS" nucleic acid under low stringency conditions,
   (ix) "β-TrCP1" (SEQ ID No:129) or a functionally active derivative thereof, or a functionally active fragment thereof, or a homolog thereof, or a variant of "β TrCP1" encoded by a nucleic acid that hybridizes to the "β TrCP1" nucleic acid under low stringency conditions,
   (x) "κB-Ras1" (SEQ ID No:132) or a functionally active derivative thereof, or a functionally active fragment thereof, or a homolog thereof, or a variant of "κB - ras1" encoded by a nucleic acid that hybridizes to the "κB - ras1" nucleic acid under low stringency conditions,
   (xi) "κB-Ras2" (SEQ ID No:131) or a functionally active derivative thereof, or a functionally active fragment thereof, or a homolog thereof, or a variant of "κB - ras2" encoded by a nucleic acid that hybridizes to the "κB - ras2" nucleic acid under low stringency conditions,
   (xii) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
   (xiii) "β TrCP2 - 1" (SEQ ID No:148) or a functionally active derivative thereof, or a functionally active fragment thereof, or a homolog thereof, or a variant of "β TrCP2 - 1" encoded by a nucleic acid that hybridizes to the "β TrCP2-1" nucleic acid under low stringency conditions,
   (xiv) "IKK β" (SEQ 10 No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (xv) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (xvi) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (xvii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (xviii) "Hos1" (SEQ ID No:137) or a functionally active derivative thereof, or a functionally active fragment thereof, or a homolog thereof, or a variant of "Hos1" encoded by a nucleic acid that hybridizes to the "Hos1" nucleic acid under low stringency conditions,
   (xix) "SKP2 (SCF complex component)" (SEQ ID No: 133) or a functionally active derivative thereof, or a functionally active fragment thereof, or a homolog thereof, or a variant of "SKP2 (SCF COMPLEX COMPONTENT" encoded by a nucleic acid that hybridizes to the "SKP2 (SCF COMPLEX COMPONENT" nucleic acid under low stringency conditions,
   (xx) "SKP1 (SCF COMPLEX COMPONENT)" (SEQ ID No:135) or a functionally active derivative thereof, or a functionally active fragment thereof, or a homolog thereof, or a variant of "SKP1 (SCF COMPLEX COMPONTENT" encoded by a nucleic acid that hybridizes to the "SKP1 (SCF COMPLEX COMPONENT" nucleic acid under low stringency conditions,
   (xxi) "ReIB" (SEQ ID No:81) or a functionally active derivative thereof, or a functionally active fragment thereof, or a homolog thereof, or a variant of "ReIB" encoded by a nucleic acid that hybridizes to the "ReIB" nucleic acid under low stringency conditions,
   (xxii) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
   (xxiii) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (xxiv) "p100 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (xxv) "Cullin 1" (SEQ ID No:136) or a functionally active derivative thereof, or a functionally active fragment thereof, or a homolog thereof, or a variant of "Cullin 1" encoded by a nucleic acid that hybridizes to the "Cullin 1" nucleic acid under low stringency conditions,
   (xxvi) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (xxvii) "Roc1/RBX1" (SEQ ID No:146) or a functionally active derivative thereof, or a functionally active fragment thereof, or a homolog thereof, or a variant of "Roc/RBX1" encoded by a nucleic acid that hybridizes to the "Roc1/RBX1" nucleic acid under low stringency conditions,
   (xxviii) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions,
   (xxix) "CD3E-ASSOCIATED PROTEIN" (SEQ ID No:158) or a functionally active derivative thereof, or a functionally active fragment thereof, or a homolog thereof, or a variant of "CD3E-ASSOCIATED PROTEIN" encoded by a nucleic acid that hybridizes to the "CD3E-ASSOCIATED PROTEIN" nucleic acid under low stringency conditions,
   (xxx) "COLORECTAL MUTANT CANCER PROTEIN" (SEQ ID No:159) or a functionally active derivative thereof, or a functionally active fragment thereof, or a homolog thereof, or a variant of "COLORECTAL MUTANT CANCER PROTEIN" encoded by a nucleic acid that hybridizes to the "COLORECTAL MUTANT CANCER PROTEIN" nucleic acid under low stringency conditions,
   (xxxi) "DNA-DIRECTED RNA POLYMERASE I 16 KDA POLYPEPTIDE" (SEQ ID No:160) or a functionally active derivative thereof, or a functionally active fragment thereof, or a homolog thereof, or a variant of "DNA-DIRECTED RNA POLYMERASE I 16 KDA POLYPEPTIDE" encoded by a nucleic acid that hybridizes to the "DNA-DIRECTED RNA POLYMERASE I 16 KDA POLYPEPTIDE" nucleic acid under low stringency conditions,
   (xxxii) "DNA-DIRECTED RNA POLYMERASE I 40 KDA POLYPEPTIDE" (SEQ ID No:161) or a functionally active derivative thereof, or a functionally active fragment thereof, or a homolog thereof, or a variant of "DNA-DIRECTED RNA POLYMERASE I 40 KDA POLYPEPTIDE" encoded by a nucleic acid that hybridizes to the "DNA-DIRECTED RNA POLYMERASE I 40 KDA POLYPEPTIDE" nucleic acid under low stringency conditions,
   (xxxiii) "DNA-DIRECTED RNA POLYMERASE I LARGEST SUBUNIT" (SEQ ID No:162) or a functionally active derivative thereof, or a functionally active fragment thereof, or a homolog thereof, or a variant of "DNA-DIRECTED RNA POLYMERASE I LARGEST SUBUNIT" encoded by a nucleic acid that hybridizes to the "DNA-DIRECTED RNA POLYMERASE I LARGEST SUBUNIT" nucleic acid under low stringency conditions,
   (xxxiv) "DNA-DIRECTED RNA POLYMERASE II 7.6 KDA POLYPEPTIDE" (SEQ ID No:163) or a functionally active derivative thereof, or a functionally active fragment thereof, or a homolog thereof, or a variant of "DNA-DIRECTED RNA POLYMERASE II 7.6 KDA POLYPEPTIDE" encoded by a nucleic acid that hybridizes to the "DNA-DIRECTED RNA POLYMERASE II 7.6 KDA POLYPEPTIDE" nucleic acid under low stringency conditions,
   (xxxv) "HYPOTHETICAL 47.2 KDA PROTEIN" (SEQ ID No:164) or a functionally active derivative thereof, or a functionally active fragment thereof, or a homolog thereof, or a variant of "HYPOTHETICAL 47.2 KDA PROTEIN" encoded by a nucleic acid that hybridizes to the "HYPOTHETICAL 47.2 KDA PROTEIN" nucleic acid under low stringency conditions,
   (xxxvi) "TRANSCRIPTION INITIATION FACTOR TFIID" (SEQ ID No:165) or a functionally active derivative thereof, or a functionally active fragment thereof, or a homolog thereof, or a variant of "TRANSCRIPTION INITIATION FACTOR TFIID" encoded by a nucleic acid that hybridizes to the "TRANSCRIPTION INITIATION FACTOR TFIID" nucleic acid under low stringency conditions,
   (xxxvii) "TRANSCRIPTION-ASSOCIATED ZINC RIBBON PROTEIN" (SEQ ID No:166) or a functionally active derivative thereof, or a functionally active fragment thereof, or a homolog thereof, or a variant of "TRANSCRIPTION-ASSOCIATED ZINC RIBBON PROTEIN" encoded by a nucleic acid that hybridizes to the "TRANSCRIPTION-ASSOCIATED ZINC RIBBON PROTEIN" nucleic acid under low stringency conditions,
   (xxxviii) "PROGRAMED CELL DEATH PROTEIN 2" (SEQ ID No:167) or a functionally active derivative thereof, or a functionally active fragment thereof, or a homolog thereof, or a variant of "PROGRAMED CELL DEATH PROTEIN 2" encoded by a nucleic acid that hybridizes to the "PROGRAMED CELL DEATH PROTEIN 2" nucleic acid under low stringency conditions,
   (xxxix) "DNA-DIRECTED RNA POLYMERASE II 23 KDA POLYPEPTIDE" (SEQ ID No:168) or a functionally active derivative thereof, or a functionally active fragment thereof, or a homolog thereof, or a variant of "DNA-DIRECTED RNA POLYMERASE II 23 KDA POLYPEPTIDE" encoded by a nucleic acid that hybridizes to the "DNA-DIRECTED RNA POLYMERASE II 23 KDA POLYPEPTIDE" nucleic acid under low stringency conditions,
   (xl) "RAS GTPASE-ACTIVATING-LIKE PROTEIN IQGAP2" (SEQ ID No:169) or a functionally active derivative thereof, or a functionally active fragment thereof, or a homolog thereof, or a variant of "RAS GTPASE-ACTIVATING-LIKE PROTEIN IQGAP2" encoded by a nucleic acid that hybridizes to the "RAS GTPASE-ACTIVATING-LIKE PROTEIN IQGAP2" nucleic acid under low stringency conditions,
   (xli) "CDNA FLJ14782 FIS, CLONE NT2RP4000524, HIGHLY SIMILAR TO MUS MUSCULUS SEC8 MRNA" (SEQ ID No:170) or a functionally active derivative thereof, or a functionally active fragment thereof, or a homolog thereof, or a variant of "CDNA FLJ14782 FIS, CLONE NT2RP4000524, HIGHLY SIMILAR TO MUS MUSCULUS SEC8 MRNA" encoded by a nucleic acid that hybridizes to the "CDNA FLJ14782 FIS, CLONE NT2RP4000524, HIGHLY SIMILAR TO MUS MUSCULUS SEC8 MRNA" nucleic acid under low stringency conditions,
   (xlii) "DNA-DIRECTED RNA POLYMERASE I 135 KDA POLYPEPTIDE" (SEQ ID No:171) or a functionally active derivative thereof, or a functionally active fragment thereof, or a homolog thereof, or a variant of "DNA-DIRECTED RNA POLYMERASE I 135 KDA POLYPEPTIDE" encoded by a nucleic acid that hybridizes to the "DNA-DIRECTED RNA POLYMERASE I 135 KDA POLYPEPTIDE" nucleic acid under low stringency conditions,
   (xliii) "DNA-DIRECTED RNA POLYMERASES I, II, AND III 17.1 KDA POLYPEPTIDE" (SEQ ID No:172) or a functionally active derivative thereof, or a functionally active fragment thereof, or a homolog thereof, or a variant of "DNA-DIRECTED RNA POLYMERASES I, II, AND III 17.1 KDA POLYPEPTIDE" encoded by a nucleic acid that hybridizes to the "DNA-DIRECTED RNA POLYMERASES I, II, AND III 17.1 KDA POLYPEPTIDE" nucleic acid under low stringency conditions,
   (xliv) "HYPOTHETICAL PROTEIN XP_108399" (SEQ ID No:173) or a functionally active derivative thereof, or a functionally active fragment thereof, or a homolog thereof, or a variant of "HYPOTHETICAL PROTEIN XP_108399" encoded by a nucleic acid that hybridizes to the "HYPOTHETICAL PROTEIN XP_108399" nucleic acid under low stringency conditions,
   (xlv) "SIMILAR TO CG5341 GENE PRODUCT" (SEQ ID No:174) or a functionally active derivative thereof, or a functionally active fragment thereof, or a homolog thereof, or a variant of "SIMILAR TO CG5341 GENE PRODUCT" encoded by a nucleic acid that hybridizes to the "SIMILAR TO CG5341 GENE PRODUCT" nucleic acid under low stringency conditions,
   (xlvi) "HYPOTHETICAL PROTEIN XP_108582" (SEQ ID No:175) or a functionally active derivative thereof,or a functionally active fragement thereof, or a homolog thereof, or a variant of "HYPOTHETICAL PROTEIN XP_108582" that hybridizes to the "HYPOTHETICAL PROTEIN XP_108582" nucleic acid under low stringency conditions, and
   (xlvii) "SEC5 PROTEIN" (SEQ ID No:176) or a functionally active derivative thereof, or a functionally active fragment thereof, or a homolog thereof, or a variant of "SEC5 PROTEIN" encoded by a nucleic acid that hybridizes to the "SEC5 PROTEIN" nucleic acid under low stringency conditions.
4. The protein complex according to No. 1 selected from complex (I) comprising all but 1 - 24 of the following proteins:
   (i) "karyopherin α2" (SEQ ID No:138) or a functionally active derivative thereof, or a functionally active fragment thereof, or a homolog thereof, or a variant of "karyopherin α2" encoded by a nucleic acid that hybridizes to the "karyopherin α2" nucleic acid under low stringency conditions,
   (ii) "DNA-DIRECTED RNA POLYMERASE II 14.4 KDA POLYPEPTIDE" (SEQ ID No: 152) or a functionally active derivative thereof, or a functionally active fragment thereof, or a homolog thereof, or a variant of "DNA-DIRECTED RNA POLYMERASE II 14.4 KDA POLYPEPTIDE" encoded by a nucleic acid that hybridizes to the "DNA-DIRECTED RNA POLYMERASE II 14.4 KDA POLYPEPTIDE" nucleic acid under low stringency conditions,
   (iii) "G PROTEIN PATHWAY SUPPRESSOR 1" (SEQ ID No:153) or a functionally active derivative thereof, or a functionally active fragment thereof, or a homolog thereof, or a variant of "G PROTEIN PATHWAY SUPPRESSOR 1" encoded by a nucleic acid that hybridizes to the "G PROTEIN PATHWAY SUPPRESSOR 1" nucleic acid under low stringency conditions,
   (iv) "KARYOPHERIN α 6 (IMPORTIN α 7)" (SEQ ID No:154) or a functionally active derivative thereof, or a functionally active fragment thereof, or a homolog thereof, or a variant of "KARYOPHERIN α6 (IMPORTIN α7)" encoded by a nucleic acid that hybridizes to the "KARYOPHERIN α6 (IMPORTIN α7)" nucleic acid under low stringency conditions,
   (v) "karyopherin α1" (SEQ ID No:103) or a functionally active derivative thereof, or a functionally active fragment thereof, or a homolog thereof, or a variant of "karyopherin α1" encoded by a nucleic acid that hybridizes to the "karyopherin α1" nucleic acid under low stringency conditions,
   (vi) "CDNA: FLJ22055 FIS, CLONE HEP09645" (SEQ ID No:155) or a functionally active derivative thereof, or a functionally active fragment thereof, or a homolog thereof, or a variant of "CDNA: FLJ22055 FIS, CLONE HEP09645" encoded by a nucleic acid that hybridizes to the "CDNA: FLJ22055 FIS, CLONE HEP09645" nucleic acid under low stringency conditions,
   (vii) "HYPOTHETICAL 14.5 KDA PROTEIN" (SEQ ID No:156) or a functionally active derivative thereof, or a functionally active fragment thereof, or a homolog thereof, or a variant of "HYPOTHETICAL 14.5 KDA PROTEIN" encoded by a nucleic acid that hybridizes to the "HYPOTHETICAL 14.5 KDA PROTEIN" nucleic acid under low stringency conditions,
   (viii) "RNA-BINDING PROTEIN FUS" (SEQ ID No:157) or a functionally active derivative thereof, or a functionally active fragment thereof, or a homolog thereof, or a variant of "RNA-BINDING PROTEIN FUS" encoded by a nucleic acid that hybridizes to the "RNA-BINDING PROTEIN FUS" nucleic acid under low stringency conditions,
   (ix) "β-TrCP1" (SEQ ID No:129) or a functionally active derivative thereof, or a functionally active fragment thereof, or a homolog thereof, or a variant of "β TrCP1" encoded by a nucleic acid that hybridizes to the "β TrCP1" nucleic acid under low stringency conditions,
   (x) "κB-Ras1 " (SEQ ID No:132) or a functionally active derivative thereof, or a functionally active fragment thereof, or a homolog thereof, or a variant of "κB - ras1" encoded by a nucleic acid that hybridizes to the "κB - ras1" nucleic acid under low stringency conditions,
   (xi) "κB-Ras2" (SEQ ID No: 131) or a functionally active derivative thereof, or a functionally active fragment thereof, or a homolog thereof, or a variant of "κB - ras2" encoded by a nucleic acid that hybridizes to the "κB - ras2" nucleic acid under low stringency conditions,
   (xii) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
   (xiii) "β TrCP2-1" (SEQ ID No:148) or a functionally active derivative thereof, or a functionally active fragment thereof, or a homolog thereof, or a variant of "β TrCP2-1" encoded by a nucleic acid that hybridizes to the "β TrCP2 - 1" nucleic acid under low stringency conditions,
   (xiv) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (xv) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (xvi) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (xvii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (xviii) "Hos1" (SEQ ID No:137) or a functionally active derivative thereof, or a functionally active fragment thereof, or a homolog thereof, or a variant of "Hos1" encoded by a nucleic acid that hybridizes to the "Hos1" nucleic acid under low stringency conditions,
   (xix) "SKP2 (SCF complex component)" (SEQ ID No:133) or a functionally active derivative thereof, or a functionally active fragment thereof, or a homolog thereof, or a variant of "SKP2 (SCF COMPLEX COMPONTENT" encoded by a nucleic acid that hybridizes to the "SKP2 (SCF COMPLEX COMPONENT" nucleic acid under low stringency conditions,
   (xx) "SKP1 (SCF COMPLEX COMPONENT)" (SEQ ID No:135) or a functionally active derivative thereof, or a functionally active fragment thereof, or a homolog thereof, or a variant of "SKP1 (SCF COMPLEX COMPONTENT" encoded by a nucleic acid that hybridizes to the "SKP1 (SCF COMPLEX COMPONENT" nucleic acid under low stringency conditions,
   (xxi) "ReIB" (SEQ ID No:81) or a functionally active derivative thereof, or a functionally active fragment thereof, or a homolog thereof, or a variant of "ReIB" encoded by a nucleic acid that hybridizes to the "ReIB" nucleic acid under low stringency conditions,
   (xxii) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
   (xxiii) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (xxiv) "p100 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (xxv) "Cullin 1" (SEQ ID No:136) or a functionally active derivative thereof, or a functionally active fragment thereof, or a homolog thereof, or a variant of "Cullin 1" encoded by a nucleic acid that hybridizes to the "Cullin 1" nucleic acid under low stringency conditions,
   (xxvi) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (xxvii) "Roc1 / RBX1" (SEQ ID No:146) or a functionally active derivative thereof, or a functionally active fragment thereof, or a homolog thereof, or a variant of "Roc/RBX1" encoded by a nucleic acid that hybridizes to the "Roc1/RBX1" nucleic acid under low stringency conditions,
   (xxviii) "RelA (p65)" (SEQ ID No:75) or a functionally active derivative thereof, or a functionally active fragment thereof, or a homolog thereof, or a variant of "RelA (p65)" encoded by a nucleic acid that hybridizes to the "RelA (p65)" nucleic acid under low stringency conditions,
   (xxix) "CD3E-ASSOCIATED PROTEIN" (SEQ ID No:158) or a functionally active derivative thereof, or a functionally active fragment thereof, or a homolog thereof, or a variant of "CD3E-ASSOCIATED PROTEIN" encoded by a nucleic acid that hybridizes to the "CD3E-ASSOCIATED PROTEIN" nucleic acid under low stringency conditions,
   (xxx) "COLORECTAL MUTANT CANCER PROTEIN" (SEQ ID No:159) or a functionally active derivative thereof, or a functionally active fragment thereof, or a homolog thereof, or a variant of "COLORECTAL MUTANT CANCER PROTEIN" encoded by a nucleic acid that hybridizes to the "COLORECTAL MUTANT CANCER PROTEIN" nucleic acid under low stringency conditions,
   (xxxi) "DNA-DIRECTED RNA POLYMERASE I 16 KDA POLYPEPTIDE" (SEQ ID No:160) or a functionally active derivative thereof, or a functionally active fragment thereof, or a homolog thereof, or a variant of "DNA-DIRECTED RNA POLYMERASE I 16 KDA POLYPEPTIDE" encoded by a nucleic acid that hybridizes to the "DNA-DIRECTED RNA POLYMERASE I 16 KDA POLYPEPTIDE" nucleic acid under low stringency conditions,
   (xxxii) "DNA-DIRECTED RNA POLYMERASE I 40 KDA POLYPEPTIDE" (SEQ ID No: 161) or a functionally active derivative thereof, or a functionally active fragment thereof, or a homolog thereof, or a variant of "DNA-DIRECTED RNA POLYMERASE I 40 KDA POLYPEPTIDE" encoded by a nucleic acid that hybridizes to the "DNA-DIRECTED RNA POLYMERASE I 40 KDA POLYPEPTIDE" nucleic acid under low stringency conditions,
   (xxxiii) "DNA-DIRECTED RNA POLYMERASE I LARGEST SUBUNIT" (SEQ ID No:162) or a functionally active derivative thereof, or a functionally active fragment thereof, or a homolog thereof, or a variant of "DNA-DIRECTED RNA POLYMERASE I LARGEST SUBUNIT" encoded by a nucleic acid that hybridizes to the "DNA-DIRECTED RNA POLYMERASE I LARGEST SUBUNIT" nucleic acid under low stringency conditions,
   (xxxiv) "DNA-DIRECTED RNA POLYMERASE II 7.6 KDA POLYPEPTIDE" (SEQ ID No:163) or a functionally active derivative thereof, or a functionally active fragment thereof, or a homolog thereof, or a variant of "DNA-DIRECTED RNA POLYMERASE II 7.6 KDA POLYPEPTIDE" encoded by a nucleic acid that hybridizes to the "DNA-DIRECTED RNA POLYMERASE II 7.6 KDA POLYPEPTIDE" nucleic acid under low stringency conditions,
   (xxxv) "HYPOTHETICAL 47.2 KDA PROTEIN" (SEQ ID No:164) or a functionally active derivative thereof, or a functionally active fragment thereof, or a homolog thereof, or a variant of "HYPOTHETICAL 47.2 KDA PROTEIN" encoded by a nucleic acid that hybridizes to the "HYPOTHETICAL 47.2 KDA PROTEIN" nucleic acid under low stringency conditions,
   (xxxvi) "TRANSCRIPTION INITIATION FACTOR TFIID" (SEQ ID No:165) or a functionally active derivative thereof, or a functionally active fragment thereof, or a homolog thereof, or a variant of "TRANSCRIPTION INITIATION FACTOR TFIID" encoded by a nucleic acid that hybridizes to the 'TRANSCRIPTION INITIATION FACTOR TFIID" nucleic acid under low stringency conditions,
   (xxxvii) 'TRANSCRIPTION-ASSOCIATED ZINC RIBBON PROTEIN" (SEQ ID No:166) or a functionally active derivative thereof, or a functionally active fragment thereof, or a homolog thereof, or a variant of "TRANSCRIPTION-ASSOCIATED ZINC RIBBON PROTEIN" encoded by a nucleic acid that hybridizes to the "TRANSCRIPTION-ASSOCIATED ZINC RIBBON PROTEIN" nucleic acid under low stringency conditions,
   (xxxviii) "PROGRAMED CELL DEATH PROTEIN 2" (SEQ ID No:167) or a functionally active derivative thereof, or a functionally active fragment thereof, or a homolog thereof, or a variant of "PROGRAMED CELL DEATH PROTEIN 2" encoded by a nucleic acid that hybridizes to the "PROGRAMED CELL DEATH PROTEIN 2" nucleic acid under low stringency conditions,
   (xxxix) "DNA-DIRECTED RNA POLYMERASE II 23 KDA POLYPEPTIDE" (SEQ ID No:168) or a functionally active derivative thereof, or a functionally active fragment thereof, or a homolog thereof, or a variant of "DNA-DIRECTED RNA POLYMERASE II 23 KDA POLYPEPTIDE" encoded by a nucleic acid that hybridizes to the "DNA-DIRECTED RNA POLYMERASE II 23 KDA POLYPEPTIDE" nucleic acid under low stringency conditions,
   (xl) "RAS GTPASE-ACTIVATING-LIKE PROTEIN IQGAP2" (SEQ ID No:169) or a functionally active derivative thereof, or a functionally active fragment thereof, or a homolog thereof, or a variant of "RAS GTPASE-ACTIVATING-LIKE PROTEIN IQGAP2" encoded by a nucleic acid that hybridizes to the "RAS GTPASE-ACTIVATING-LIKE PROTEIN IQGAP2" nucleic acid under low stringency conditions,
   (xli) "CDNA FLJ14782 FIS, CLONE NT2RP4000524, HIGHLY SIMILAR TO MUS MUSCULUS SEC8 MRNA" (SEQ ID No: 170) or a functionally active derivative thereof, or a functionally active fragment thereof, or a homolog thereof, or a variant of "CDNA FLJ14782 FIS, CLONE NT2RP4000524, HIGHLY SIMILAR TO MUS MUSCULUS SEC8 MRNA" encoded by a nucleic acid that hybridizes to the "CDNA FLJ14782 FIS, CLONE NT2RP4000524, HIGHLY SIMILAR TO MUS MUSCULUS SEC8 MRNA" nucleic acid under low stringency conditions,
   (xlii) "DNA-DIRECTED RNA POLYMERASE I 135 KDA POLYPEPTIDE" (SEQ ID No: 171) or a functionally active derivative thereof, or a functionally active fragment thereof, or a homolog thereof, or a variant of "DNA-DIRECTED RNA POLYMERASE I 135 KDA POLYPEPTIDE" encoded by a nucleic acid that hybridizes to the "DNA-DIRECTED RNA POLYMERASE I 135 KDA POLYPEPTIDE" nucleic acid under low stringency conditions,
   (xliii) "DNA-DIRECTED RNA POLYMERASES I, II, AND III 17.1 KDA POLYPEPTIDE" (SEQ ID No:172) or a functionally active derivative thereof, or a functionally active fragment thereof, or a homolog thereof, or a variant of "DNA-DIRECTED RNA POLYMERASES I, II, AND III 17.1 KDA POLYPEPTIDE" encoded by a nucleic acid that hybridizes to the "DNA-DIRECTED RNA POLYMERASES I, II, AND III 17.1 KDA POLYPEPTIDE" nucleic acid under low stringency conditions,
   (xliv) "HYPOTHETICAL PROTEIN XP_108399" (SEQ ID No:173) or a functionally active derivative thereof, or a functionally active fragment thereof, or a homolog thereof, or a variant of "HYPOTHETICAL PROTEIN XP_108399" encoded by a nucleic acid that hybridizes to the "HYPOTHETICAL PROTEIN XP_108399" nucleic acid under low stringency conditions,
   (xlv) "SIMILAR TO CG5341 GENE PRODUCT" (SEQ ID No:174) or a functionally active derivative thereof, or a functionally active fragment thereof, or a homolog thereof, or a variant of "SIMILAR TO CG5341 GENE PRODUCT" encoded by a nucleic acid that hybridizes to the "SIMILAR TO CG5341 GENE PRODUCT" nucleic acid under low stringency conditions,
   (xlvi) "HYPOTHETICAL PROTEIN XP_108582" (SEQ ID No:175) or a functionally active derivative thereof,or a functionally active fragement thereof, or a homolog thereof, or a variant of "HYPOTHETICAL PROTEIN XP_108582" that hybridizes to the "HYPOTHETICAL PROTEIN XP_108582" nucleic acid under low stringency conditions, and
   (xlvii) "SEC5 PROTEIN" (SEQ ID No:176) or a functionally active derivative thereof, or a functionally active fragment thereof, or a homolog thereof, or a variant of "SEC5 PROTEIN" encoded by a nucleic acid that hybridizes to the "SEC5 PROTEIN" nucleic acid under low stringency conditions;
   and a protein complex selected from complex (I) comprising all but 1 - 24 of the following proteins:
   (i) "karyopherin α2" (SEQ ID No:138) or a functionally active derivative thereof, or a functionally active fragment thereof, or a homolog thereof, or a variant of "karyopherin α2" encoded by a nucleic acid that hybridizes to the "karyopherin α2" nucleic acid under low stringency conditions,
   (ii) "DNA-DIRECTED RNA POLYMERASE II 14.4 KDA POLYPEPTIDE" (SEQ ID No: 152) or a functionally active derivative thereof, or a functionally active fragment thereof, or a homolog thereof, or a variant of "DNA-DIRECTED RNA POLYMERASE II 14.4 KDA POLYPEPTIDE" encoded by a nucleic acid that hybridizes to the "DNA-DIRECTED RNA POLYMERASE II 14.4 KDA POLYPEPTIDE" nucleic acid under low stringency conditions,
   (iii) "G PROTEIN PATHWAY SUPPRESSOR 1" (SEQ ID No:153) or a functionally active derivative thereof, or a functionally active fragment thereof, or a homolog thereof, or a variant of "G PROTEIN PATHWAY SUPPRESSOR 1" encoded by a nucleic acid that hybridizes to the "G PROTEIN PATHWAY SUPPRESSOR 1" nucleic acid under low stringency conditions,

   (i) "KARYOPHERIN α 6 (IMPORTIN α7)" (SEQ ID No:154) or a functionally active derivative thereof, or a functionally active fragment thereof, or a homolog thereof, or a variant of "KARYOPHERIN α6 (IMPORTIN α7)" encoded by a nucleic acid that hybridizes to the "KARYOPHERIN α6 (IMPORTIN α7)" nucleic acid under low stringency conditions,
   (v) "karyopherin α1" (SEQ ID No:103) or a functionally active derivative thereof, or a functionally active fragment thereof, or a homolog thereof, or a variant of "karyopherin α1" encoded by a nucleic acid that hybridizes to the "karyopherin α1" nucleic acid under low stringency conditions,
   (vi) "CDNA: FLJ22055 FIS, CLONE HEP09645" (SEQ ID No:155) or a functionally active derivative thereof, or a functionally active fragment thereof, or a homolog thereof, or a variant of "CDNA: FLJ22055 FIS, CLONE HEP09645" encoded by a nucleic acid that hybridizes to the "CDNA: FLJ22055 FIS, CLONE HEP09645" nucleic acid under low stringency conditions,
   (vii) "HYPOTHETICAL 14.5 KDA PROTEIN" (SEQ ID No:156) or a functionally active derivative thereof, or a functionally active fragment thereof, or a homolog thereof, or a variant of "HYPOTHETICAL 14.5 KDA PROTEIN" encoded by a nucleic acid that hybridizes to the "HYPOTHETICAL 14.5 KDA PROTEIN" nucleic acid under low stringency conditions,
   (viii) "RNA-BINDING PROTEIN FUS" (SEQ ID No:157) or a functionally active derivative thereof, or a functionally active fragment thereof, or a homolog thereof, or a variant of "RNA-BINDING PROTEIN FUS" encoded by a nucleic acid that hybridizes to the "RNA-BINDING PROTEIN FUS" nucleic acid under low stringency conditions,

   (i) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (x) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (xi) "IKK γ' (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (xii) "Hos1" (SEQ ID No:137) or a functionally active derivative thereof, or a functionally active fragment thereof, or a homolog thereof, or a variant of "Hos1" encoded by a nucleic acid that hybridizes to the "Hos1" nucleic acid under low stringency conditions,
   (xiii) "c-ReI" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
   (xiv) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (xv) "p100 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (xvi) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (xvii) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the ''ReIA (p65)" nucleic acid under low stringency conditions,
   (xviii) "CD3E-ASSOCIATED PROTEIN" (SEQ ID No:158) or a functionally active derivative thereof, or a functionally active fragment thereof, or a homolog thereof, or a variant of "CD3E-ASSOCIATED PROTEIN" encoded by a nucleic acid that hybridizes to the "CD3E-ASSOCIATED PROTEIN" nucleic acid under low stringency conditions,
   (xix) "COLORECTAL MUTANT CANCER PROTEIN" (SEQ ID No:159) or a functionally active derivative thereof, or a functionally active fragment thereof, or a homolog thereof, or a variant of "COLORECTAL MUTANT CANCER PROTEIN" encoded by a nucleic acid that hybridizes to the "COLORECTAL MUTANT CANCER PROTEIN" nucleic acid under low stringency conditions,
   (xx) "DNA-DIRECTED RNA POLYMERASE I 16 KDA POLYPEPTIDE" (SEQ ID No:160) or a functionally active derivative thereof, or a functionally active fragment thereof, or a homolog thereof, or a variant of "DNA-DIRECTED RNA POLYMERASE I 16 KDA POLYPEPTIDE" encoded by a nucleic acid that hybridizes to the "DNA-DIRECTED RNA POLYMERASE I 16 KDA POLYPEPTIDE" nucleic acid under low stringency conditions,
   (xxi) "DNA-DIRECTED RNA POLYMERASE I 40 KDA POLYPEPTIDE" (SEQ ID No: 161) or a functionally active derivative thereof, or a functionally active fragment thereof, or a homolog thereof, or a variant of "DNA-DIRECTED RNA POLYMERASE I 40 KDA POLYPEPTIDE" encoded by a nucleic acid that hybridizes to the "DNA-DIRECTED RNA POLYMERASE I 40 KDA POLYPEPTIDE" nucleic acid under low stringency conditions,
   (xxii) "DNA-DIRECTED RNA POLYMERASE I LARGEST SUBUNIT" (SEQ ID No:162) or a functionally active derivative thereof, or a functionally active fragment thereof, or a homolog thereof, or a variant of "DNA-DIRECTED RNA POLYMERASE I LARGEST SUBUNIT" encoded by a nucleic acid that hybridizes to the "DNA-DIRECTED RNA POLYMERASE I LARGEST SUBUNIT" nucleic acid under low stringency conditions,
   (xxiii) "DNA-DIRECTED RNA POLYMERASE II 7.6 KDA POLYPEPTIDE" (SEQ ID No: 163) or a functionally active derivative thereof, or a functionally active fragment thereof, or a homolog thereof, or a variant of "DNA-DIRECTED RNA POLYMERASE II 7.6 KDA POLYPEPTIDE" encoded by a nucleic acid that hybridizes to the "DNA-DIRECTED RNA POLYMERASE II 7.6 KDA POLYPEPTIDE" nucleic acid under low stringency conditions,
   (xxiv) "HYPOTHETICAL 47.2 KDA PROTEIN" (SEQ ID No:164) or a functionally active derivative thereof, or a functionally active fragment thereof, or a homolog thereof, or a variant of "HYPOTHETICAL 47.2 KDA PROTEIN" encoded by a nucleic acid that hybridizes to the "HYPOTHETICAL 47.2 KDA PROTEIN" nucleic acid under low stringency conditions,
   (xxv) "TRANSCRIPTION INITIATION FACTOR TFIID" (SEQ ID No:165) or a functionally active derivative thereof, or a functionally active fragment thereof, or a homolog thereof, or a variant of "TRANSCRIPTION INITIATION FACTOR TFIID" encoded by a nucleic acid that hybridizes to the "TRANSCRIPTION INITIATION FACTOR TFIID" nucleic acid under low stringency conditions,
   (xxvi) "TRANSCRIPTION-ASSOCIATED ZINC RIBBON PROTEIN" (SEQ ID No:166) or a functionally active derivative thereof, or a functionally active fragment thereof, or a homolog thereof, or a variant of "TRANSCRIPTION-ASSOCIATED ZINC RIBBON PROTEIN" encoded by a nucleic acid that hybridizes to the "TRANSCRIPTION-ASSOCIATED ZINC RIBBON PROTEIN" nucleic acid under low stringency conditions,
   (xxvii) "PROGRAMED CELL DEATH PROTEIN 2" (SEQ ID No:167) or a functionally active derivative thereof, or a functionally active fragment thereof, or a homolog thereof, or a variant of "PROGRAMED CELL DEATH PROTEIN 2" encoded by a nucleic acid that hybridizes to the "PROGRAMED CELL DEATH PROTEIN 2" nucleic acid under low stringency conditions,
   (xxviii) "DNA-DIRECTED RNA POLYMERASE II 23 KDA POLYPEPTIDE" (SEQ ID No:168) or a functionally active derivative thereof, or a functionally active fragment thereof, or a homolog thereof, or a variant of "DNA-DIRECTED RNA POLYMERASE II 23 KDA POLYPEPTIDE" encoded by a nucleic acid that hybridizes to the "DNA-DIRECTED RNA POLYMERASE II 23 KDA POLYPEPTIDE" nucleic acid under low stringency conditions,
   (xxix) "RAS GTPASE-ACTIVATING-LIKE PROTEIN IQGAP2" (SEQ ID No:169) or a functionally active derivative thereof, or a functionally active fragment thereof, or a homolog thereof, or a variant of "RAS GTPASE-ACTIVATING-LIKE PROTEIN IQGAP2" encoded by a nucleic acid that hybridizes to the "RAS GTPASE-ACTIVATING-LIKE PROTEIN IQGAP2" nucleic acid under low stringency conditions,
   (xxx) "CDNA FLJ14782 FIS, CLONE NT2RP4000524, HIGHLY SIMILAR TO MUS MUSCULUS SEC8 MRNA" (SEQ ID No:170) or a functionally active derivative thereof, or a functionally active fragment thereof, or a homolog thereof, or a variant of "CDNA FLJ14782 FIS, CLONE NT2RP4000524, HIGHLY SIMILAR TO MUS MUSCULUS SEC8 MRNA" encoded by a nucleic acid that hybridizes to the "CDNA FLJ14782 FIS, CLONE NT2RP4000524, HIGHLY SIMILAR TO MUS MUSCULUS SEC8 MRNA" nucleic acid under low stringency conditions,
   (xxxi) "DNA-DIRECTED RNA POLYMERASE I 135 KDA POLYPEPTIDE" (SEQ ID No: 171) or a functionally active derivative thereof, or a functionally active fragment thereof, or a homolog thereof, or a variant of "DNA-DIRECTED RNA POLYMERASE I 135 KDA POLYPEPTIDE" encoded by a nucleic acid that hybridizes to the "DNA-DIRECTED RNA POLYMERASE I 135 KDA POLYPEPTIDE" nucleic acid under low stringency conditions,
   (xxxii) "DNA-DIRECTED RNA POLYMERASES I, II, AND III 17.1 KDA POLYPEPTIDE" (SEQ ID No:172) or a functionally active derivative thereof, or a functionally active fragment thereof, or a homolog thereof, or a variant of "DNA-DIRECTED RNA POLYMERASES I, II, AND III 17.1 KDA POLYPEPTIDE" encoded by a nucleic acid that hybridizes to the "DNA-DIRECTED RNA POLYMERASES I, II, AND III 17.1 KDA POLYPEPTIDE" nucleic acid under low stringency conditions,
   (xxxiii) "HYPOTHETICAL PROTEIN XP_108399" (SEQ ID No:173) or a functionally active derivative thereof, or a functionally active fragment thereof, or a homolog thereof, or a variant of "HYPOTHETICAL PROTEIN XP_108399" encoded by a nucleic acid that hybridizes to the "HYPOTHETICAL PROTEIN XP_108399" nucleic acid under low stringency conditions,
   (xxxiv) "SIMILAR TO CG5341 GENE PRODUCT" (SEQ ID No:174) or a functionally active derivative thereof, or a functionally active fragment thereof, or a homolog thereof, or a variant of "SIMILAR TO CG5341 GENE PRODUCT" encoded by a nucleic acid that hybridizes to the "SIMILAR TO CG5341 GENE PRODUCT" nucleic acid under low stringency conditions, and
   (xxxv) "HYPOTHETICAL PROTEIN XP_108582" (SEQ ID No:175) or a functionally active derivative thereof, or a functionally active fragment thereof, or a homolog thereof, or a variant of "HYPOTHETICAL PROTEIN XP_108582" encoded by a nucleic acid that hybridizes to the "HYPOTHETICAL PROTEIN XP_108582" nucleic acid under low stringency conditions.
5. The complex of any of No. 1 - 4 comprising a functionally active derivative of said first protein and/or a functionally active derivative of said second protein, wherein the functionally active derivative is a fusion protein comprising said first protein or said second protein fused to an amino acid sequence different from the first protein or second protein, respectively.
6. The complex of No. 5 wherein the functionally active derivative is a fusion protein comprising said first protein or said second protein fused to an affinity tag or label.
7. The complex of any of No. 1-4 comprising a fragment of said first protein and/or a fragment of said second protein, which fragment binds to another protein component of said complex.
8. The complex of any of No. 1 - 7 that is involved in the deubiquitination activity.
9. A process for preparing complex of any of No. 1-8 and optionally the components thereof comprising the following steps:
   the expressing a protein (bait) of the complex, preferably a tagged protein, in a target cell, isolating the protein complex which is attached to the bait protein, and optionally disassociating the protein complex and isolating the individual complex members.
10. The process according to No. 9 wherein the tagged protein comprises two different tags which allow two separate affinity purification steps.
11. The process according to any of No. 9 - 10 wherein the two tags are separated by a cleavage site for a protease.
12. Component of the IκBβ complex obtainable by a process according to any of No. 9 to 11.
13. Protein of the IκBβ complex selected from
   (i) "CDNA: FLJ22055 FIS, CLONE HEP09645" (SEQ ID No:155) or a functionally active derivative thereof, or a functionally active fragment thereof, or a homolog thereof, or a variant of "CDNA: FLJ22055 FIS, CLONE HEP09645" encoded by a nucleic acid that hybridizes to the "CDNA: FLJ22055 FIS, CLONE HEP09645" nucleic acid under low stringency conditions,
   (ii) "HYPOTHETICAL 14.5 KDA PROTEIN" (SEQ ID No:156) or a functionally active derivative thereof, or a functionally active fragment thereof, or a homolog thereof, or a variant of "HYPOTHETICAL 14.5 KDA PROTEIN" encoded by a nucleic acid that hybridizes to the "HYPOTHETICAL 14.5 KDA PROTEIN" nucleic acid under low stringency conditions,
   (iii) "RAS GTPASE-ACTIVATING-LIKE PROTEIN IQGAP2" (SEQ ID No:169) or a functionally active derivative thereof, or a functionally active fragment thereof, or a homolog thereof, or a variant of "RAS GTPASE-ACTIVATING-LIKE PROTEIN IQGAP2" encoded by a nucleic acid that hybridizes to the "RAS GTPASE-ACTIVATING-LIKE PROTEIN IQGAP2" nucleic acid under low stringency conditions,
   (iv) "HYPOTHETICAL PROTEIN XP_108399" (SEQ ID No:173) or a functionally active derivative thereof, or a functionally active fragment thereof, or a homolog thereof, or a variant of "HYPOTHETICAL PROTEIN XP_108399" encoded by a nucleic acid that hybridizes to the "HYPOTHETICAL PROTEIN XP_108399" nucleic acid under low stringency conditions, and
   (v) "HYPOTHETICAL PROTEIN XP_108582" (SEQ ID No: 175) or a functionally active derivative thereof, or a functionally active fragment thereof, or a homolog thereof, or a variant of "HYPOTHETICAL PROTEIN XP_108582" encoded by a nucleic acid that hybridizes to the "HYPOTHETICAL PROTEIN XP_108582" nucleic acid under low stringency conditions,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
14. Nucleic acid encoding a protein according to No. 13.
15. Construct, preferably a vector construct, comprising (a) a nucleic acid according to No. 14 and at least one further nucleic acid which is normally not associated with said nucleic acid,or(b) at least two separate nucleic acid sequences each encoding a different protein, or a functionally active fragment or a functionally active derivative thereof at least one of said proteins, or functionally active fragments or functionally active derivative thereof being selected from the first group of proteins according to No. 1(a) and at least one of said proteins, or functionally active fragments or functionally active derivative thereof being selected from the second group of proteins according to No. 1(b).
16. Host cell, containing a vector comprising at least one of the nucleic acid of No. 14 and/or a construct of No. 15 or containing several vectors each comprising at least the nucleic acid sequence encoding at least one of the proteins, or functionally active fragments or functionally active derivatives thereof selected from the first group of proteins according to No. 1(a) and the proteins, or functionally active fragments or functionally active derivatives thereof selected from the second group of proteins according to No. 1(b).
17. An antibody or a fragment of said antibody containing the binding domain thereof, selected from an antibody or fragment thereof, which binds the complex of any of No. 1-8 and which does not bind any of the proteins of said complex when uncomplexed and an antibody or a fragment of said antibody which binds to any of the proteins according to No. 13.
18. A kit comprising in one or more container the complex of any of No. 1 - 8 and/or the proteins of No. 13 optionally together with an antibody according to No. 17 and/or further components such as reagents and working instructions.
19. The kit according to No. 18 for processing a substrate of said complex.
20. The kit according to No. 18 for the diagnosis or prognosis of a disease or a disease risk, preferentially for a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
21. Array, in which at least a complex according to any of No. 1 - 8 and/or at least one protein according to No. 14 and/or at least one antibody according to No. 17 is attached to a solid carrier.
22. A process for processing a physiological substrate of the complex comprising the step of bringing into contact a complex to any of No. 1 - 8 with said substrate, such that said substrate is processed.
23. A pharmaceutical composition comprising the protein complex of any of No. 1 to 8 and/or any of the following the proteins:
   (i) "CDNA: FLJ22055 FIS, CLONE HEP09645" (SEQ ID No:155) or a functionally active derivative thereof, or a functionally active fragment thereof, or a homolog thereof, or a variant of "CDNA: FLJ22055 FIS, CLONE HEP09645" encoded by a nucleic acid that hybridizes to the "CDNA: FLJ22055 FIS, CLONE HEP09645" nucleic acid under low stringency conditions,
   (ii) "HYPOTHETICAL 14.5 KDA PROTEIN" (SEQ ID No:156) or a functionally active derivative thereof, or a functionally active fragment thereof, or a homolog thereof, or a variant of "HYPOTHETICAL 14.5 KDA PROTEIN" encoded by a nucleic acid that hybridizes to the "HYPOTHETICAL 14.5 KDA PROTEIN" nucleic acid under low stringency conditions,
   (iii) "RAS GTPASE-ACTIVATING-LIKE PROTEIN IQGAP2" (SEQ ID No:169) or a functionally active derivative thereof, or a functionally active fragment thereof, or a homolog thereof, or a variant of "RAS GTPASE-ACTIVATING-LIKE PROTEIN IQGAP2" encoded by a nucleic acid that hybridizes to the "RAS GTPASE-ACTIVATING-LIKE PROTEIN IQGAP2" nucleic acid under low stringency conditions,
   (iv) "HYPOTHETICAL PROTEIN XP_108399" (SEQ ID No:173) or a functionally active derivative thereof,or a functionally active fragement thereof, or a homolog thereof, or a variant of "HYPOTHETICAL PROTEIN XP_108399" that hybridizes to the "HYPOTHETICAL PROTEIN XP_108399" nucleic acid under low stringency conditions, and
   (v) "HYPOTHETICAL PROTEIN XP_108582" (SEQ ID No:175) or a functionally active derivative thereof, or a functionally active fragment thereof, or a homolog thereof, or a variant of "HYPOTHETICAL PROTEIN XP_108582" encoded by a nucleic acid that hybridizes to the "HYPOTHETICAL PROTEIN XP_108582" nucleic acid under low stringency conditions,
   and a pharmaceutical acceptable carrier.
24. The pharmaceutical composition according to No. 23 for the treatment of diseases and disorders such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
25. A method for screening for a molecule that binds to the complex of anyone of No. 1 - 8 and/or any of the following proteins:
   (i) "CDNA: FLJ22055 FIS, CLONE HEP09645" (SEQ ID No:155) or a functionally active derivative thereof, or a functionally active fragment thereof, or a homolog thereof, or a variant of "CDNA: FLJ22055 FIS, CLONE HEP09645" encoded by a nucleic acid that hybridizes to the "CDNA: FLJ22055 FIS, CLONE HEP09645" nucleic acid under low stringency conditions,
   (ii) "HYPOTHETICAL 14.5 KDA PROTEIN" (SEQ ID No: 156) or a functionally active derivative thereof, or a functionally active fragment thereof, or a homolog thereof, or a variant of "HYPOTHETICAL 14.5 KDA PROTEIN" encoded by a nucleic acid that hybridizes to the "HYPOTHETICAL 14.5 KDA PROTEIN" nucleic acid under low stringency conditions,
   (iii) "RAS GTPASE-ACTIVATING-LIKE PROTEIN IQGAP2" (SEQ ID No:169) or a functionally active derivative thereof, or a functionally active fragment thereof, or a homolog thereof, or a variant of "RAS GTPASE-ACTIVATING-LIKE PROTEIN IQGAP2" encoded by a nucleic acid that hybridizes to the "RAS GTPASE-ACTIVATING-LIKE PROTEIN IQGAP2" nucleic acid under low stringency conditions,
   (iv) "HYPOTHETICAL PROTEIN XP_108399" (SEQ ID No: 173) or a functionally active derivative thereof,or a functionally active fragement thereof, or a homolog thereof, or a variant of "HYPOTHETICAL PROTEIN XP_108399" that hybridizes to the "HYPOTHETICAL PROTEIN XP_108399" nucleic acid under low stringency conditions, and
   (v) "HYPOTHETICAL PROTEIN XP_108582" (SEQ ID No:175) or a functionally active derivative thereof, or a functionally active fragment thereof, or a homolog thereof, or a variant of "HYPOTHETICAL PROTEIN XP_108582" encoded by a nucleic acid that hybridizes to the "HYPOTHETICAL PROTEIN XP_108582" nucleic acid under low stringency conditions, comprising the steps of
      (a) exposing said complex, or a cell or organism containing same to one or more candidate molecules; and
      (b) determinig whether said candidate molecule is bound to the complex or protein.
26. A method for screening for a molecule that modulates directly or indirectly the function, activity, composition or formation of the complex of any one of No. 1 - 8 comprising the steps of (a) exposing said complex, or a cell or organism containing IκBβ complex to one or more candidate molecules; and
   (b) determining the amount of activity of protein components of, and/or intracellular localization of, said complex and/or the transcription level of a gene dependent on the complex and/or the abundance and/or activity of a protein or protein complex dependent on the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependent on the complex and/or the abundance and/or activity of a protein or protein complex dependent on the function of the complex and/or product of a gene dependent on the complex in the absence of said candidate molecules indicates that the molecule modulates function, activity or composition of said complex.
27. The method of No. 26, wherein the amount of said complex is determined.
28. The method of No. 26, wherein the activity of said complex is determined.
29. The method of No. 28, wherein said determining step comprises isolating from the cell or organism said complex to produce said isolated complex and contacting said isolated complex in the presence or absence of a candidate molecule with a substrate of said complex and determining the processing of said substrate is modified in the presence of said candidate molecule.
30. The method of No. 26, wherein the amount of the individual protein components of said complex are determined.
31. The method of No. 30, determining step comprises determining whether
   (i) "karyopherin α2" (SEQ ID No:138) or a functionally active derivative thereof, or a functionally active fragment thereof, or a homolog thereof, or a variant of "karyopherin α2" encoded by a nucleic acid that hybridizes to the "karyopherin α2" nucleic acid under low stringency conditions, and/or
   (ii) "DNA-DIRECTED RNA POLYMERASE II 14.4 KDA POLYPEPTIDE" (SEQ ID No:152) or a functionally active derivative thereof, or a functionally active fragment thereof, or a homolog thereof, or a variant of "DNA-DIRECTED RNA POLYMERASE II 14.4 KDA POLYPEPTIDE" encoded by a nucleic acid that hybridizes to the "DNA-DIRECTED RNA POLYMERASE II 14.4 KDA POLYPEPTIDE" nucleic acid under low stringency conditions, and/or
   (iii) "G PROTEIN PATHWAY SUPPRESSOR 1" (SEQ ID No:153) or a functionally active derivative thereof, or a functionally active fragment thereof, or a homolog thereof, or a variant of "G PROTEIN PATHWAY SUPPRESSOR 1" encoded by a nucleic acid that hybridizes to the "G PROTEIN PATHWAY SUPPRESSOR 1" nucleic acid under low stringency conditions, and/or
   (iv) "KARYOPHERIN α 6 (IMPORTIN α 7)" (SEQ ID No:154) or a functionally active derivative thereof, or a functionally active fragment thereof, or a homolog thereof, or a variant of "KARYOPHERIN α 6 (IMPORTIN α 7)" encoded by a nucleic acid that hybridizes to the "KARYOPHERIN α 6 (IMPORTIN α 7)" nucleic acid under low stringency conditions, and/or
   (v) "karyopherin α 1" (SEQ ID No:103) or a functionally active derivative thereof, or a functionally active fragment thereof, or a homolog thereof, or a variant of "karyopherin α1" encoded by a nucleic acid that hybridizes to the "karyopherin α1" nucleic acid under low stringency conditions,
   (vi) "CDNA: FLJ22055 FIS, CLONE HEP09645" (SEQ ID No:155) or a functionally active derivative thereof, or a functionally active fragment thereof, or a homolog thereof, or a variant of "CDNA: FLJ22055 FIS, CLONE HEP09645" encoded by a nucleic acid that hybridizes to the "CDNA: FLJ22055 FIS, CLONE HEP09645" nucleic acid under low stringency conditions, and/or
   (vii) "HYPOTHETICAL 14.5 KDA PROTEIN" (SEQ ID No:156) or a functionally active derivative thereof, or a functionally active fragment thereof, or a homolog thereof, or a variant of "HYPOTHETICAL 14.5 KDA PROTEIN" encoded by a nucleic acid that hybridizes to the "HYPOTHETICAL 14.5 KDA PROTEIN" nucleic acid under low stringency conditions, and/or
   (viii) "RNA-BINDING PROTEIN FUS" (SEQ ID No:157) or a functionally active derivative thereof, or a functionally active fragment thereof, or a homolog thereof, or a variant of "RNA-BINDING PROTEIN FUS" encoded by a nucleic acid that hybridizes to the "RNA-BINDING PROTEIN FUS" nucleic acid under low stringency conditions, and/or
   (ix) "β-TrCP1" (SEQ ID No:129) or a functionally active derivative thereof, or a functionally active fragment thereof, or a homolog thereof, or a variant of "β TrCP1" encoded by a nucleic acid that hybridizes to the "β TrCP1" nucleic acid under low stringency conditions, and/or
   (x) "κB-Ras1" (SEQ I D No:132) or a functionally active derivative thereof, or a functionally active fragment thereof, or a homolog thereof, or a variant of "κB - ras1" encoded by a nucleic acid that hybridizes to the "κB - ras1" nucleic acid under low stringency conditions, and/or
   (xi) "κB-Ras2" (SEQ ID No:131) or a functionally active derivative thereof, or a functionally active fragment thereof, or a homolog thereof, or a variant of "κB - ras2" encoded by a nucleic acid that hybridizes to the "κB - ras2" nucleic acid under low stringency conditions, and/or
   (xii) "IκB α" (SEQ ID No:78) or a functionally active derivative thereof, or a functionally active fragment thereof, or a homolog thereof, or a variant of "IκB α" encoded by a nucleic acid that hybridizes to the "IκBα" nucleic acid under low stringency conditions, and/or
   (xiii) "β TrCP2 - 1" (SEQ ID No:148) or a functionally active derivative thereof, or a functionally active fragment thereof, or a homolog thereof, or a variant of "β TrCP2-1" encoded by a nucleic acid that hybridizes to the "β TrCP2 - 1" nucleic acid under low stringency conditions, and/or
   (xiv) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions, and/or
   (xv) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions, and/or
   (xvi) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions, and/or
   (xvii) "IκBε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions, and/or
   (xviii) "Hos1" (SEQ ID No:137) or a functionally active derivative thereof, or a functionally active fragment thereof, or a homolog thereof, or a variant of "Hos1" encoded by a nucleic acid that hybridizes to the "Hos1" nucleic acid under low stringency conditions, and/or
   (xix) "SKP2 (SCF complex component)" (SEQ ID No:133) or a functionally active derivative thereof, or a functionally active fragment thereof, or a homolog thereof, or a variant of "SKP2 (SCF COMPLEX COMPONTENT" encoded by a nucleic acid that hybridizes to the "SKP2 (SCF COMPLEX COMPONENT" nucleic acid under low stringency conditions, and/or
   (xx) "SKP1 (SCF COMPLEX COMPONENT)" (SEQ ID No: 135) or a functionally active derivative thereof, or a functionally active fragment thereof, or a homolog thereof, or a variant of "SKP1 (SCF COMPLEX COMPONTENT" encoded by a nucleic acid that hybridizes to the "SKP1 (SCF COMPLEX COMPONENT" nucleic acid under low stringency conditions, and/or
   (xxi) "ReIB" (SEQ ID No:81) or a functionally active derivative thereof, or a functionally active fragment thereof, or a homolog thereof, or a variant of "ReIB" encoded by a nucleic acid that hybridizes to the "RelB" nucleic acid under low stringency conditions, and/or
   (xxii) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions, and/or
   (xxiii) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions, and/or
   (xxiv) "p100 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions, and/or
   (xxv) "Cullin 1" (SEQ ID No:136) or a functionally active derivative thereof, or a functionally active fragment thereof, or a homolog thereof, or a variant of "Cullin 1" encoded by a nucleic acid that hybridizes to the "Cullin 1" nucleic acid under low stringency conditions, and/or
   (xxvi) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions, and/or
   (xxvii) "Roc1 / RBX1" (SEQ ID No:146) or a functionally active derivative thereof, or a functionally active fragment thereof, or a homolog thereof, or a variant of "Roc/RBX1" encoded by a nucleic acid that hybridizes to the "Roc1/RBX1" nucleic acid under low stringency conditions, and/or
   (xxviii) "RelA (p65)" (SEQ ID No:75) or a functionally active derivative thereof, or a functionally active fragment thereof, or a homolog thereof, or a variant of "RelA (p65)" encoded by a nucleic acid that hybridizes to the "RelA (p65)" nucleic acid under low stringency conditions, and/or
   (xxix) "CD3E-ASSOCIATED PROTEIN" (SEQ ID No:158) or a functionally active derivative thereof, or a functionally active fragment thereof, or a homolog thereof, or a variant of "CD3E-ASSOCIATED PROTEIN" encoded by a nucleic acid that hybridizes to the "CD3E-ASSOCIATED PROTEIN" nucleic acid under low stringency conditions, and/or
   (xxx) "COLORECTAL MUTANT CANCER PROTEIN" (SEQ ID No:159) or a functionally active derivative thereof, or a functionally active fragment thereof, or a homolog thereof, or a variant of "COLORECTAL MUTANT CANCER PROTEIN" encoded by a nucleic acid that hybridizes to the "COLORECTAL MUTANT CANCER PROTEIN" nucleic acid under low stringency conditions, and/or
   (xxxi) "DNA-DIRECTED RNA POLYMERASE I 16 KDA POLYPEPTIDE" (SEQ ID No:160) or a functionally active derivative thereof, or a functionally active fragment thereof, or a homolog thereof, or a variant of "DNA-DIRECTED RNA POLYMERASE I 16 KDA POLYPEPTIDE" encoded by a nucleic acid that hybridizes to the "DNA-DIRECTED RNA POLYMERASE I 16 KDA POLYPEPTIDE" nucleic acid under low stringency conditions, and/or
   (xxxii) "DNA-DIRECTED RNA POLYMERASE 140 KDA POLYPEPTIDE" (SEQ ID No:161) or a functionally active derivative thereof, or a functionally active fragment thereof, or a homolog thereof, or a variant of "DNA-DIRECTED RNA POLYMERASE 140 KDA POLYPEPTIDE" encoded by a nucleic acid that hybridizes to the "DNA-DIRECTED RNA POLYMERASE I 40 KDA POLYPEPTIDE" nucleic acid under low stringency conditions, and/or
   (xxxiii) "DNA-DIRECTED RNA POLYMERASE I LARGEST SUBUNIT" (SEQ ID No:162) or a functionally active derivative thereof, or a functionally active fragment thereof, or a homolog thereof, or a variant of "DNA-DIRECTED RNA POLYMERASE I LARGEST SUBUNIT" encoded by a nucleic acid that hybridizes to the "DNA-DIRECTED RNA POLYMERASE LARGEST SUBUNIT" nucleic acid under low stringency conditions, and/or
   (xxxiv) "DNA-DIRECTED RNA POLYMERASE II 7.6 KDA POLYPEPTIDE" (SEQ ID No:163) or a functionally active derivative thereof, or a functionally active fragment thereof, or a homolog thereof, or a variant of "DNA-DIRECTED RNA POLYMERASE II 7.6 KDA POLYPEPTIDE" encoded by a nucleic acid that hybridizes to the "DNA-DIRECTED RNA POLYMERASE II 7.6 KDA POLYPEPTIDE" nucleic acid under low stringency conditions, and/or
   (xxxv) "HYPOTHETICAL 47.2 KDA PROTEIN" (SEQ ID No:164) or a functionally active derivative thereof, or a functionally active fragment thereof, or a homolog thereof, or a variant of "HYPOTHETICAL 47.2 KDA PROTEIN" encoded by a nucleic acid that hybridizes to the "HYPOTHETICAL 47.2 KDA PROTEIN" nucleic acid under low stringency conditions, and/or
   (xxxvi) "TRANSCRIPTION INITIATION FACTOR TFIID" (SEQ ID No:165) or a functionally active derivative thereof, or a functionally active fragment thereof, or a homolog thereof, or a variant of "TRANSCRIPTION INITIATION FACTOR TFIID" encoded by a nucleic acid that hybridizes to the "TRANSCRIPTION INITIATION FACTOR TFIID" nucleic acid under low stringency conditions, and/or
   (xxxvii) "TRANSCRIPTION-ASSOCIATED ZINC RIBBON PROTEIN" (SEQ ID No:166) or a functionally active derivative thereof, or a functionally active fragment thereof, or a homolog thereof, or a variant of "TRANSCRIPTION-ASSOCIATED ZINC RIBBON PROTEIN" encoded by a nucleic acid that hybridizes to the "TRANSCRIPTION-ASSOCIATED ZINC RIBBON PROTEIN" nucleic acid under low stringency conditions, and/or
   (xxxviii) "PROGRAMED CELL DEATH PROTEIN 2" (SEQ ID No:167) or a functionally active derivative thereof, or a functionally active fragment thereof, or a homolog thereof, or a variant of "PROGRAMED CELL DEATH PROTEIN 2" encoded by a nucleic acid that hybridizes to the "PROGRAMED CELL DEATH PROTEIN 2" nucleic acid under low stringency conditions, and/or
   (xxxix) "DNA-DIRECTED RNA POLYMERASE II 23 KDA POLYPEPTIDE" (SEQ ID No:168) or a functionally active derivative thereof, or a functionally active fragment thereof, or a homolog thereof, or a variant of "DNA-DIRECTED RNA POLYMERASE II 23 KDA POLYPEPTIDE" encoded by a nucleic acid that hybridizes to the "DNA-DIRECTED RNA POLYMERASE II 23 KDA POLYPEPTIDE" nucleic acid under low stringency conditions, and/or
   (xl) "RAS GTPASE-ACTIVATING-LIKE PROTEIN IQGAP2" (SEQ ID No:169) or a functionally active derivative thereof, or a functionally active fragment thereof, or a homolog thereof, or a variant of "RAS GTPASE-ACTIVATING-LIKE PROTEIN IQGAP2" encoded by a nucleic acid that hybridizes to the "RAS GTPASE-ACTIVATING-LIKE PROTEIN IQGAP2" nucleic acid under low stringency conditions, and/or
   (xli) "CDNA FLJ14782 FIS, CLONE NT2RP4000524, HIGHLY SIMILAR TO MUS MUSCULUS SEC8 MRNA" (SEQ ID No:170) or a functionally active derivative thereof, or a functionally active fragment thereof, or a homolog thereof, or a variant of "CDNA FLJ14782 FIS, CLONE NT2RP4000524, HIGHLY SIMILAR TO MUS MUSCULUS SEC8 MRNA" encoded by a nucleic acid that hybridizes to the "CDNA FLJ14782 FIS, CLONE NT2RP4000524, HIGHLY SIMILAR TO MUS MUSCULUS SEC8 MRNA" nucleic acid under low stringency conditions, and/or
   (xlii) "DNA-DIRECTED RNA POLYMERASE I 135 KDA POLYPEPTIDE" (SEQ ID No: 171) or a functionally active derivative thereof, or a functionally active fragment thereof, or a homolog thereof, or a variant of "DNA-DIRECTED RNA POLYMERASE I 135 KDA POLYPEPTIDE" encoded by a nucleic acid that hybridizes to the "DNA-DIRECTED RNA POLYMERASE I 135 KDA POLYPEPTIDE" nucleic acid under low stringency conditions, and/or
   (xliii) "DNA-DIRECTED RNA POLYMERASES I, II, AND III 17.1 KDA POLYPEPTIDE" (SEQ ID No:172) or a functionally active derivative thereof, or a functionally active fragment thereof, or a homolog thereof, or a variant of "DNA-DIRECTED RNA POLYMERASES I, II, AND III 17.1 KDA POLYPEPTIDE" encoded by a nucleic acid that hybridizes to the "DNA-DIRECTED RNA POLYMERASES I, II, AND I11 17.1 KDA POLYPEPTIDE" nucleic acid under low stringency conditions, and/or
   (xliv) "HYPOTHETICAL PROTEIN XP_108399" (SEQ ID No:173) or a functionally active derivative thereof, or a functionally active fragment thereof, or a homolog thereof, or a variant of "HYPOTHETICAL PROTEIN XP_108399" encoded by a nucleic acid that hybridizes to the "HYPOTHETICAL PROTEIN XP_108399" nucleic acid under low stringency conditions, and/or
   (xlv) "SIMILAR TO CG5341 GENE PRODUCT" (SEQ ID No:174) or a functionally active derivative thereof, or a functionally active fragment thereof, or a homolog thereof, or a variant of "SIMILAR TO CG5341 GENE PRODUCT" encoded by a nucleic acid that hybridizes to the "SIMILAR TO CG5341 GENE PRODUCT" nucleic acid under low stringency conditions, and/or
   (xlvi) "HYPOTHETICAL PROTEIN XP_108582" (SEQ ID No:175) or a functionally active derivative thereof, or a functionally active fragment thereof, or a homolog thereof, or a variant of "HYPOTHETICAL PROTEIN XP_108582" encoded by a nucleic acid that hybridizes to the "HYPOTHETICAL PROTEIN XP_108582" nucleic acid under low stringency conditions, and/or
   (xlvii) "SEC5 PROTEIN" (SEQ ID No:176) or a functionally active derivative thereof, or a functionally active fragment thereof, or a homolog thereof, or a variant of "SEC5 PROTEIN" encoded by a nucleic acid that hybridizes to the "SEC5 PROTEIN" nucleic acid under low stringency conditions,
   is present in the complex.
32. The method of any of No. 26 to 31, wherein said method is a method of screening for a drug for treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
33. Use of a molecule that modulates the amount of, activity of, or the protein components of the complex of any one of No. 1 - 8 for the manufacture of a medicament for the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
34. A method for the production of a pharmaceutical composition comprising carrying out the method of any of No. 1 - 8 to identify a molecule that modulates the function, activity, composition or formation of said complex, and further comprising mixing the identified molecule with a pharmaceutically acceptable carrier.
35. A method for diagnosing or screening for the presence of a disease or disorder or a predisposition for developing a disease or disorder in a subject, which disease or disorder is characterized by an aberrant amount of, activity of, or component composition of, or intracellular localization of the complex of any one of the No. 1 - 8, comprising determining the amount of, activity of, protein components of, and/or intracellular localization of, said complex and/or the transcription level of a gene dependent on the complex and/or the abundance and/or activity of a protein or protein complex dependent on the function of the complex and/or product of a gene dependent on the complex in a comparative sample derived from a subject, wherein a difference in said amount, activity, or protein components of, said complex in an analogous sample from a subject not having the disease or disorder or predisposition indicates the presence in the subject of the disease or disorder or predisposition in the subject.
36. The method of No. 35, wherein the amount of said complex is determined.
37. The method of No. 35, wherein the activity of said complex is determined.
38. The method of No. 37, wherein said determining step comprises isolating from the subject said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
39. The method of No. 35, wherein the amount of the individual protein components of said complex are determined.
40. The method of No. 39, wherein said determining step comprises determining whether
   (i) "karyopherin α2" (SEQ ID No:138) or a functionally active derivative thereof, or a functionally active fragment thereof, or a homolog thereof, or a variant of "karyopherin α2" encoded by a nucleic acid that hybridizes to the "karyopherin α2" nucleic acid under low stringency conditions, and/or
   (ii) "DNA-DIRECTED RNA POLYMERASE II 14.4 KDA POLYPEPTIDE" (SEQ ID No: 152) or a functionally active derivative thereof, or a functionally active fragment thereof, or a homolog thereof, or a variant of "DNA-DIRECTED RNA POLYMERASE II 14.4 KDA POLYPEPTIDE" encoded by a nucleic acid that hybridizes to the "DNA-DIRECTED RNA POLYMERASE II 14.4 KDA POLYPEPTIDE" nucleic acid under low stringency conditions, and/or
   (iii) "G PROTEIN PATHWAY SUPPRESSOR 1" (SEQ ID No:153) or a functionally active derivative thereof, or a functionally active fragment thereof, or a homolog thereof, or a variant of "G PROTEIN PATHWAY SUPPRESSOR 1" encoded by a nucleic acid that hybridizes to the "G PROTEIN PATHWAY SUPPRESSOR 1" nucleic acid under low stringency conditions, and/or
   (iv) "KARYOPHERIN α6 (IMPORTIN α7)" (SEQ ID No:154) or a functionally active derivative thereof, or a functionally active fragment thereof, or a homolog thereof, or a variant of "KARYOPHERIN α6 (IMPORTIN α7)" encoded by a nucleic acid that hybridizes to the "KARYOPHERIN α6 (IMPORTIN α7)" nucleic acid under low stringency conditions, and/or
   (v) "karyopherin α1" (SEQ ID No:103) or a functionally active derivative thereof, or a functionally active fragment thereof, or a homolog thereof, or a variant of "karyopherin α1" encoded by a nucleic acid that hybridizes to the "karyopherin α1" nucleic acid under low stringency conditions,
   (vi) "CDNA: FLJ22055 FIS, CLONE HEP09645" (SEQ ID No:155) or a functionally active derivative thereof, or a functionally active fragment thereof, or a homolog thereof, or a variant of "CDNA: FLJ22055 FIS, CLONE HEP09645" encoded by a nucleic acid that hybridizes to the "CDNA: FLJ22055 FIS, CLONE HEP09645" nucleic acid under low stringency conditions, and/or
   (vii) "HYPOTHETICAL 14.5 KDA PROTEIN" (SEQ ID No:156) or a functionally active derivative thereof, or a functionally active fragment thereof, or a homolog thereof, or a variant of "HYPOTHETICAL 14.5 KDA PROTEIN" encoded by a nucleic acid that hybridizes to the "HYPOTHETICAL 14.5 KDA PROTEIN" nucleic acid under low stringency conditions, and/or
   (viii) "RNA-BINDING PROTEIN FUS" (SEQ ID No:157) or a functionally active derivative thereof, or a functionally active fragment thereof, or a homolog thereof, or a variant of "RNA-BINDING PROTEIN FUS" encoded by a nucleic acid that hybridizes to the "RNA-BINDING PROTEIN FUS" nucleic acid under low stringency conditions, and/or
   (ix) "β-TrCP1" (SEQ ID No:129) or a functionally active derivative thereof, or a functionally active fragment thereof, or a homolog thereof, or a variant of "β TrCP1" encoded by a nucleic acid that hybridizes to the "β TrCP1" nucleic acid under low stringency conditions, and/or
   (x) "κB-Ras1" (SEQ ID No:132) or a functionally active derivative thereof, or a functionally active fragment thereof, or a homolog thereof, or a variant of "κB - ras1" encoded by a nucleic acid that hybridizes to the "κB - ras1" nucleic acid under low stringency conditions, and/or
   (xi) "κB-Ras2" (SEQ ID No:131) or a functionally active derivative thereof, or a functionally active fragment thereof, or a homolog thereof, or a variant of "κB - ras2" encoded by a nucleic acid that hybridizes to the "κB - ras2" nucleic acid under low stringency conditions, and/or
   (xii) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions, and/or
   (xiii) "β TrCP2 - 1" (SEQ ID No: 148) or a functionally active derivative thereof, or a functionally active fragment thereof, or a homolog thereof, or a variant of "β TrCP2 - 1" encoded by a nucleic acid that hybridizes to the "β TrCP2 - 1" nucleic acid under low stringency conditions, and/or
   (xiv) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions, and/or
   (xv) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions, and/or
   (xvi) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions, and/or
   (xvii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions, and/or
   (xviii) "Hos1" (SEQ ID No:137) or a functionally active derivative thereof, or a functionally active fragment thereof, or a homolog thereof, or a variant of "Hos1" encoded by a nucleic acid that hybridizes to the "Hos1" nucleic acid under low stringency conditions, and/or
   (xix) "SKP2 (SCF complex component)" (SEQ ID No:133) or a functionally active derivative thereof, or a functionally active fragment thereof, or a homolog thereof, or a variant of "SKP2 (SCF COMPLEX COMPONTENT" encoded by a nucleic acid that hybridizes to the "SKP2 (SCF COMPLEX COMPONENT" nucleic acid under low stringency conditions, and/or
   (xx) "SKP1 (SCF COMPLEX COMPONENT)" (SEQ ID No:135) or a functionally active derivative thereof, or a functionally active fragment thereof, or a homolog thereof, or a variant of "SKP1 (SCF COMPLEX COMPONTENT" encoded by a nucleic acid that hybridizes to the "SKP1 (SCF COMPLEX COMPONENT" nucleic acid under low stringency conditions, and/or
   (xxi) "ReIB" (SEQ ID No:81) or a functionally active derivative thereof, or a functionally active fragment thereof, or a homolog thereof, or a variant of "ReIB" encoded by a nucleic acid that hybridizes to the "ReIB" nucleic acid under low stringency conditions, and/or
   (xxii) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions, and/or
   (xxiii) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions, and/or
   (xxiv) "p100 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions, and/or
   (xxv) "Cullin 1" (SEQ ID No:136) or a functionally active derivative thereof, or a functionally active fragment thereof, or a homolog thereof, or a variant of "Cullin 1" encoded by a nucleic acid that hybridizes to the "Cullin 1" nucleic acid under low stringency conditions, and/or
   (xxvi) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions, and/or
   (xxvii) "Roc1 / RBX1" (SEQ ID No:146) or a functionally active derivative thereof, or a functionally active fragment thereof, or a homolog thereof, or a variant of "Roc/RBX1" encoded by a nucleic acid that hybridizes to the "Roc1/RBX1" nucleic acid under low stringency conditions, and/or
   (xxviii) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions, and/or
   (xxix) "CD3E-ASSOCIATED PROTEIN" (SEQ ID No:158) or a functionally active derivative thereof, or a functionally active fragment thereof, or a homolog thereof, or a variant of "CD3E-ASSOCIATED PROTEIN" encoded by a nucleic acid that hybridizes to the "CD3E-ASSOCIATED PROTEIN" nucleic acid under low stringency conditions, and/or
   (xxx) "COLORECTAL MUTANT CANCER PROTEIN" (SEQ ID No:159) or a functionally active derivative thereof, or a functionally active fragment thereof, or a homolog thereof, or a variant of "COLORECTAL MUTANT CANCER PROTEIN" encoded by a nucleic acid that hybridizes to the "COLORECTAL MUTANT CANCER PROTEIN" nucleic acid under low stringency conditions, and/or
   (xxxi) "DNA-DIRECTED RNA POLYMERASE I 16 KDA POLYPEPTIDE" (SEQ ID No:160) or a functionally active derivative thereof, or a functionally active fragment thereof, or a homolog thereof, or a variant of "DNA-DIRECTED RNA POLYMERASE I 16 KDA POLYPEPTIDE" encoded by a nucleic acid that hybridizes to the "DNA-DIRECTED RNA POLYMERASE I 16 KDA POLYPEPTIDE" nucleic acid under low stringency conditions, and/or
   (xxxii) "DNA-DIRECTED RNA POLYMERASE I 40 KDA POLYPEPTIDE" (SEQ ID No:161) or a functionally active derivative thereof, or a functionally active fragment thereof, or a homolog thereof, or a variant of "DNA-DIRECTED RNA POLYMERASE I 40 KDA POLYPEPTIDE" encoded by a nucleic acid that hybridizes to the "DNA-DIRECTED RNA POLYMERASE I 40 KDA POLYPEPTIDE" nucleic acid under low stringency conditions, and/or
   (xxxiii) "DNA-DIRECTED RNA POLYMERASE LARGEST SUBUNIT" (SEQ ID No: 162) or a functionally active derivative thereof, or a functionally active fragment thereof, or a homolog thereof, or a variant of "DNA-DIRECTED RNA POLYMERASE I LARGEST SUBUNIT" encoded by a nucleic acid that hybridizes to the "DNA-DIRECTED RNA POLYMERASE I LARGEST SUBUNIT" nucleic acid under low stringency conditions, and/or
   (xxxiv) "DNA-DIRECTED RNA POLYMERASE II 7.6 KDA POLYPEPTIDE" (SEQ ID No: 163) or a functionally active derivative thereof, or a functionally active fragment thereof, or a homolog thereof, or a variant of "DNA-DIRECTED RNA POLYMERASE II 7.6 KDA POLYPEPTIDE" encoded by a nucleic acid that hybridizes to the "DNA-DIRECTED RNA POLYMERASE II 7.6 KDA POLYPEPTIDE" nucleic acid under low stringency conditions, and/or
   (xxxv) "HYPOTHETICAL 47.2 KDA PROTEIN" (SEQ ID No:164) or a functionally active derivative thereof, or a functionally active fragment thereof, or a homolog thereof, or a variant of "HYPOTHETICAL 47.2 KDA PROTEIN" encoded by a nucleic acid that hybridizes to the "HYPOTHETICAL 47.2 KDA PROTEIN" nucleic acid under low stringency conditions, and/or
   (xxxvi) "TRANSCRIPTION INITIATION FACTOR TFIID" (SEQ ID No:165) or a functionally active derivative thereof, or a functionally active fragment thereof, or a homolog thereof, or a variant of "TRANSCRIPTION INITIATION FACTOR TFIID" encoded by a nucleic acid that hybridizes to the "TRANSCRIPTION INITIATION FACTOR TFIID" nucleic acid under low stringency conditions, and/or
   (xxxvii) "TRANSCRIPTION-ASSOCIATED ZINC RIBBON PROTEIN" (SEQ ID No:166) or a functionally active derivative thereof, or a functionally active fragment thereof, or a homolog thereof, or a variant of "TRANSCRIPTION-ASSOCIATED ZINC RIBBON PROTEIN" encoded by a nucleic acid that hybridizes to the "TRANSCRIPTION-ASSOCIATED ZINC RIBBON PROTEIN" nucleic acid under low stringency conditions, and/or
   (xxxviii) "PROGRAMED CELL DEATH PROTEIN 2" (SEQ ID No:167) or a functionally active derivative thereof, or a functionally active fragment thereof, or a homolog thereof, or a variant of "PROGRAMED CELL DEATH PROTEIN 2" encoded by a nucleic acid that hybridizes to the "PROGRAMED CELL DEATH PROTEIN 2" nucleic acid under low stringency conditions, and/or
   (xxxix) "DNA-DIRECTED RNA POLYMERASE II 23 KDA POLYPEPTIDE" (SEQ ID No: 168) or a functionally active derivative thereof, or a functionally active fragment thereof, or a homolog thereof, or a variant of "DNA-DIRECTED RNA POLYMERASE II 23 KDA POLYPEPTIDE" encoded by a nucleic acid that hybridizes to the "DNA-DIRECTED RNA POLYMERASE II 23 KDA POLYPEPTIDE" nucleic acid under low stringency conditions, and/or
   (xl) "RAS GTPASE-ACTIVATING-LIKE PROTEIN IQGAP2" (SEQ ID No:169) or a functionally active derivative thereof, or a functionally active fragment thereof, or a homolog thereof, or a variant of "RAS GTPASE-ACTIVATING-LIKE PROTEIN IQGAP2" encoded by a nucleic acid that hybridizes to the "RAS GTPASE-ACTIVATING-LIKE PROTEIN IQGAP2" nucleic acid under low stringency conditions, and/or
   (xli) "CDNA FLJ14782 FIS, CLONE NT2RP4000524, HIGHLY SIMILAR TO MUS MUSCULUS SEC8 MRNA" (SEQ ID No:170) or a functionally active derivative thereof, or a functionally active fragment thereof, or a homolog thereof, or a variant of "CDNA FLJ14782 FIS, CLONE NT2RP4000524, HIGHLY SIMILAR TO MUS MUSCULUS SEC8 MRNA" encoded by a nucleic acid that hybridizes to the "CDNA FLJ14782 FIS, CLONE NT2RP4000524, HIGHLY SIMILAR TO MUS MUSCULUS SEC8 MRNA" nucleic acid under low stringency conditions, and/or
   (xlii) "DNA-DIRECTED RNA POLYMERASE I 135 KDA POLYPEPTIDE" (SEQ ID No:171) or a functionally active derivative thereof, or a functionally active fragment thereof, or a homolog thereof, or a variant of "DNA-DIRECTED RNA POLYMERASE I 135 KDA POLYPEPTIDE" encoded by a nucleic acid that hybridizes to the "DNA-DIRECTED RNA POLYMERASE I 135 KDA POLYPEPTIDE" nucleic acid under low stringency conditions, and/or
   (xliii) "DNA-DIRECTED RNA POLYMERASES I, II, AND III 17.1 KDA POLYPEPTIDE" (SEQ ID No:172) or a functionally active derivative thereof, or a functionally active fragment thereof, or a homolog thereof, or a variant of "DNA-DIRECTED RNA POLYMERASES I, II, AND III 17.1 KDA POLYPEPTIDE" encoded by a nucleic acid that hybridizes to the "DNA-DIRECTED RNA POLYMERASES I, II, AND III 17.1 KDA POLYPEPTIDE" nucleic acid under low stringency conditions, and/or
   (xliv) "HYPOTHETICAL PROTEIN XP_108399" (SEQ ID No:173) or a functionally active derivative thereof, or a functionally active fragment thereof, or a homolog thereof, or a variant of "HYPOTHETICAL PROTEIN XP_108399" encoded by a nucleic acid that hybridizes to the "HYPOTHETICAL PROTEIN XP_108399" nucleic acid under low stringency conditions, and/or
   (xlv) "SIMILAR TO CG5341 GENE PRODUCT" (SEQ ID No:174) or a functionally active derivative thereof, or a functionally active fragment thereof, or a homolog thereof, or a variant of "SIMILAR TO CG5341 GENE PRODUCT" encoded by a nucleic acid that hybridizes to the "SIMILAR TO CG5341 GENE PRODUCT" nucleic acid under low stringency conditions, and/or
   (xlvi) "HYPOTHETICAL PROTEIN XP_108582" (SEQ ID No:175) or a functionally active derivative thereof, or a functionally active fragment thereof, or a homolog thereof, or a variant of "HYPOTHETICAL PROTEIN XP_108582" encoded by a nucleic acid that hybridizes to the "HYPOTHETICAL PROTEIN XP_108582" nucleic acid under low stringency conditions, and/or
   (xlvii) "SEC5 PROTEIN" (SEQ ID No:176) or a functionally active derivative thereof, or a functionally active fragment thereof, or a homolog thereof, or a variant of "SEC5 PROTEIN" encoded by a nucleic acid that hybridizes to the "SEC5 PROTEIN" nucleic acid under low stringency conditions,
   is present in the complex.
41. The complex of any one of No. 1 - 8, or proteins of No. 13 or the antibody or fragment of No. 17, for use in a method of diagnosing a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
42. A method for treating or preventing a disease or disorder characterized by an aberrant amount of, activity or component composition of or intracellular localization of, the complex of anyone of No. 1 - 8, comprising administering to a subject in need of such treatment or prevention a therapeutically effective amount of one or more molecules that modulate the amount of, deubiquitination activity, or protein components of, said complex.
43. The method according to No. 42, wherein said disease or disorder involves decreased levels of the amount or activity of said complex.
44. The method according to No. 42 , wherein said disease or disorder involves increased levels of the amount or activity of said complex.
45. Complex of any of No. 1 - 8 and/or protein selected from the following proteins
   (i) "karyopherin α2" (SEQ ID No:138) or a functionally active derivative thereof, or a functionally active fragment thereof, or a homolog thereof, or a variant of "karyopherin α2" encoded by a nucleic acid that hybridizes to the "karyopherin α2" nucleic acid under low stringency conditions,
   (ii) "DNA-DIRECTED RNA POLYMERASE II 14.4 KDA POLYPEPTIDE" (SEQ ID No: 152) or a functionally active derivative thereof, or a functionally active fragment thereof, or a homolog thereof, or a variant of "DNA-DIRECTED RNA POLYMERASE II 14.4 KDA POLYPEPTIDE" encoded by a nucleic acid that hybridizes to the "DNA-DIRECTED RNA POLYMERASE II 14.4 KDA POLYPEPTIDE" nucleic acid under low stringency conditions,
   (iii) "G PROTEIN PATHWAY SUPPRESSOR 1" (SEQ ID No:153) or a functionally active derivative thereof, or a functionally active fragment thereof, or a homolog thereof, or a variant of "G PROTEIN PATHWAY SUPPRESSOR 1" encoded by a nucleic acid that hybridizes to the "G PROTEIN PATHWAY SUPPRESSOR 1" nucleic acid under low stringency conditions,
   (iv) "KARYOPHERIN α6 (IMPORTIN α7)" (SEQ ID No:154) or a functionally active derivative thereof, or a functionally active fragment thereof, or a homolog thereof, or a variant of "KARYOPHERIN α6 (IMPORTIN α7)" encoded by a nucleic acid that hybridizes to the "KARYOPHERIN α6 (IMPORTIN α7)" nucleic acid under low stringency conditions,
   (v) "karyopherin α1" (SEQ ID No:103) or a functionally active derivative thereof, or a functionally active fragment thereof, or a homolog thereof, or a variant of "karyopherin α1" encoded by a nucleic acid that hybridizes to the "karyopherin α1" nucleic acid under low stringency conditions,
   (vi) "CDNA: FLJ22055 FIS, CLONE HEP09645" (SEQ ID No:155) or a functionally active derivative thereof, or a functionally active fragment thereof, or a homolog thereof, or a variant of "CDNA: FLJ22055 FIS, CLONE HEP09645" encoded by a nucleic acid that hybridizes to the "CDNA: FLJ22055 FIS, CLONE HEP09645" nucleic acid under low stringency conditions,
   (vii) "HYPOTHETICAL 14.5 KDA PROTEIN" (SEQ ID No:156) or a functionally active derivative thereof, or a functionally active fragment thereof, or a homolog thereof, or a variant of "HYPOTHETICAL 14.5 KDA PROTEIN" encoded by a nucleic acid that hybridizes to the "HYPOTHETICAL 14.5 KDA PROTEIN" nucleic acid under low stringency conditions,
   (viii) "RNA-BINDING PROTEIN FUS" (SEQ ID No:157) or a functionally active derivative thereof, or a functionally active fragment thereof, or a homolog thereof, or a variant of "RNA-BINDING PROTEIN FUS" encoded by a nucleic acid that hybridizes to the "RNA-BINDING PROTEIN FUS" nucleic acid under low stringency conditions,
   (ix) "β-TrCP1" (SEQ ID No: 129) or a functionally active derivative thereof, or a functionally active fragment thereof, or a homolog thereof, or a variant of "β TrCP1" encoded by a nucleic acid that hybridizes to the "β TrCP1" nucleic acid under low stringency conditions,
   (x) "κB-Ras1 " (SEQ ID No:132) or a functionally active derivative thereof, or a functionally active fragment thereof, or a homolog thereof, or a variant of "κB - ras1" encoded by a nucleic acid that hybridizes to the "κB - ras1" nucleic acid under low stringency conditions,
   (xi) "κB-Ras2" (SEQ ID No:131) or a functionally active derivative thereof, or a functionally active fragment thereof, or a homolog thereof, or a variant of "κB - ras2" encoded by a nucleic acid that hybridizes to the "κB - ras2" nucleic acid under low stringency conditions,
   (xii) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
   (xiii) "β TrCP2 - 1" (SEQ ID No:148) or a functionally active derivative thereof, or a functionally active fragment thereof, or a homolog thereof, or a variant of "β TrCP2-1" encoded by a nucleic acid that hybridizes to the "β TrCP2 -1" nucleic acid under low stringency conditions,
   (xiv) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (xv) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (xvi) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (xvii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (xviii) "Hos1" (SEQ ID No:137) or a functionally active derivative thereof, or a functionally active fragment thereof, or a homolog thereof, or a variant of "Hos1" encoded by a nucleic acid that hybridizes to the "Hos1" nucleic acid under low stringency conditions,
   (xix) "SKP2 (SCF complex component)" (SEQ ID No: 133) or a functionally active derivative thereof, or a functionally active fragment thereof, or a homolog thereof, or a variant of "SKP2 (SCF COMPLEX COMPONTENT" encoded by a nucleic acid that hybridizes to the "SKP2 (SCF COMPLEX COMPONENT" nucleic acid under low stringency conditions,
   (xx) "SKP1 (SCF COMPLEX COMPONENT)" (SEQ ID No: 135) or a functionally active derivative thereof, or a functionally active fragment thereof, or a homolog thereof, or a variant of "SKP1 (SCF COMPLEX COMPONTENT" encoded by a nucleic acid that hybridizes to the "SKP1 (SCF COMPLEX COMPONENT" nucleic acid under low stringency conditions,
   (xxi) "ReIB" (SEQ ID No:81) or a functionally active derivative thereof, or a functionally active fragment thereof, or a homolog thereof, or a variant of "ReIB" encoded by a nucleic acid that hybridizes to the "ReIB" nucleic acid under low stringency conditions,
   (xxii) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
   (xxiii) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (xxiv) "p105 (NF-κB1 )" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (xxv) "Cullin 1" (SEQ ID No:136) or a functionally active derivative thereof, or a functionally active fragment thereof, or a homolog thereof, or a variant of "Cullin 1" encoded by a nucleic acid that hybridizes to the "Cullin 1" nucleic acid under low stringency conditions,
   (xxvi) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (xxvii) "Roc1/RBX1" (SEQ ID No:146) or a functionally active derivative thereof, or a functionally active fragment thereof, or a homolog thereof, or a variant of "Roc/RBX1" encoded by a nucleic acid that hybridizes to the "Roc1/RBX1" nucleic acid under low stringency conditions,
   (xxviii) "RelA (p65)" (SEQ ID No:75) or a functionally active derivative thereof, or a functionally active fragment thereof, or a homolog thereof, or a variant of "RelA (p65)" encoded by a nucleic acid that hybridizes to the "RelA (p65)" nucleic acid under low stringency conditions,
   (xxix) "CD3E-ASSOCIATED PROTEIN" (SEQ ID No:158) or a functionally active derivative thereof, or a functionally active fragment thereof, or a homolog thereof, or a variant of "CD3E-ASSOCIATED PROTEIN" encoded by a nucleic acid that hybridizes to the "CD3E-ASSOCIATED PROTEIN" nucleic acid under low stringency conditions,
   (xxx) "COLORECTAL MUTANT CANCER PROTEIN" (SEQ ID No:159) or a functionally active derivative thereof, or a functionally active fragment thereof, or a homolog thereof, or a variant of "COLORECTAL MUTANT CANCER PROTEIN" encoded by a nucleic acid that hybridizes to the "COLORECTAL MUTANT CANCER PROTEIN" nucleic acid under low stringency conditions,
   (xxxi) "DNA-DIRECTED RNA POLYMERASE I 16 KDA POLYPEPTIDE" (SEQ ID No: 160) or a functionally active derivative thereof, or a functionally active fragment thereof, or a homolog thereof, or a variant of "DNA-DIRECTED RNA POLYMERASE I 16 KDA POLYPEPTIDE" encoded by a nucleic acid that hybridizes to the "DNA-DIRECTED RNA POLYMERASE I 16 KDA POLYPEPTIDE" nucleic acid under low stringency conditions,
   (xxxii) "DNA-DIRECTED RNA POLYMERASE I 40 KDA POLYPEPTIDE" (SEQ ID No:161) or a functionally active derivative thereof, or a functionally active fragment thereof, or a homolog thereof, or a variant of "DNA-DIRECTED RNA POLYMERASE I 40 KDA POLYPEPTIDE" encoded by a nucleic acid that hybridizes to the "DNA-DIRECTED RNA POLYMERASE I 40 KDA POLYPEPTIDE" nucleic acid under low stringency conditions,
   (xxxiii) "DNA-DIRECTED RNA POLYMERASE I LARGEST SUBUNIT" (SEQ ID No: 162) or a functionally active derivative thereof, or a functionally active fragment thereof, or a homolog thereof, or a variant of "DNA-DIRECTED RNA POLYMERASE I LARGEST SUBUNIT" encoded by a nucleic acid that hybridizes to the "DNA-DIRECTED RNA POLYMERASE I LARGEST SUBUNIT" nucleic acid under low stringency conditions,
   (xxxiv) "DNA-DIRECTED RNA POLYMERASE II 7.6 KDA POLYPEPTIDE" (SEQ ID No:163) or a functionally active derivative thereof, or a functionally active fragment thereof, or a homolog thereof, or a variant of "DNA-DIRECTED RNA POLYMERASE II 7.6 KDA POLYPEPTIDE" encoded by a nucleic acid that hybridizes to the "DNA-DIRECTED RNA POLYMERASE II 7.6 KDA POLYPEPTIDE" nucleic acid under low stringency conditions,
   (xxxv) "HYPOTHETICAL 47.2 KDA PROTEIN" (SEQ ID No:164) or a functionally active derivative thereof, or a functionally active fragment thereof, or a homolog thereof, or a variant of "HYPOTHETICAL 47.2 KDA PROTEIN" encoded by a nucleic acid that hybridizes to the "HYPOTHETICAL 47.2 KDA PROTEIN" nucleic acid under low stringency conditions,
   (xxxvi) "TRANSCRIPTION INITIATION FACTOR TFIID" (SEQ ID No:165) or a functionally active derivative thereof, or a functionally active fragment thereof, or a homolog thereof, or a variant of "TRANSCRIPTION INITIATION FACTOR TFIID" encoded by a nucleic acid that hybridizes to the "TRANSCRIPTION INITIATION FACTOR TFIID" nucleic acid under low stringency conditions,
   (xxxvii) "TRANSCRIPTION-ASSOCIATED ZINC RIBBON PROTEIN" (SEQ ID No:166) or a functionally active derivative thereof, or a functionally active fragment thereof, or a homolog thereof, or a variant of "TRANSCRIPTION-ASSOCIATED ZINC RIBBON PROTEIN" encoded by a nucleic acid that hybridizes to the "TRANSCRIPTION-ASSOCIATED ZINC RIBBON PROTEIN" nucleic acid under low stringency conditions,
   (xxxviii) "PROGRAMED CELL DEATH PROTEIN 2" (SEQ ID No:167) or a functionally active derivative thereof, or a functionally active fragment thereof, or a homolog thereof, or a variant of "PROGRAMED CELL DEATH PROTEIN 2" encoded by a nucleic acid that hybridizes to the "PROGRAMED CELL DEATH PROTEIN 2" nucleic acid under low stringency conditions,
   (xxxix) "DNA-DIRECTED RNA POLYMERASE II 23 KDA POLYPEPTIDE" (SEQ ID No: 168) or a functionally active derivative thereof, or a functionally active fragment thereof, or a homolog thereof, or a variant of "DNA-DIRECTED RNA POLYMERASE II 23 KDA POLYPEPTIDE" encoded by a nucleic acid that hybridizes to the "DNA-DIRECTED RNA POLYMERASE II 23 KDA POLYPEPTIDE" nucleic acid under low stringency conditions,
   (xl) "RAS GTPASE-ACTIVATING-LIKE PROTEIN IQGAP2" (SEQ ID No:169) or a functionally active derivative thereof, or a functionally active fragment thereof, or a homolog thereof, or a variant of "RAS GTPASE-ACTIVATING-LIKE PROTEIN IQGAP2" encoded by a nucleic acid that hybridizes to the "RAS GTPASE-ACTIVATING-LIKE PROTEIN IQGAP2" nucleic acid under low stringency conditions,
   (xli) "CDNA FLJ14782 FIS, CLONE NT2RP4000524, HIGHLY SIMILAR TO MUS MUSCULUS SEC8 MRNA" (SEQ ID No:170) or a functionally active derivative thereof, or a functionally active fragment thereof, or a homolog thereof, or a variant of "CDNA FLJ14782 FIS, CLONE NT2RP4000524, HIGHLY SIMILAR TO MUS MUSCULUS SEC8 MRNA" encoded by a nucleic acid that hybridizes to the "CDNA FLJ14782 FIS, CLONE NT2RP4000524, HIGHLY SIMILAR TO MUS MUSCULUS SEC8 MRNA" nucleic acid under low stringency conditions,
   (xlii) "DNA-DIRECTED RNA POLYMERASE I 135 KDA POLYPEPTIDE" (SEQ ID No:171) or a functionally active derivative thereof, or a functionally active fragment thereof, or a homolog thereof, or a variant of "DNA-DIRECTED RNA POLYMERASE I 135 KDA POLYPEPTIDE" encoded by a nucleic acid that hybridizes to the "DNA-DIRECTED RNA POLYMERASE I 135 KDA POLYPEPTIDE" nucleic acid under low stringency conditions,
   (xliii) "DNA-DIRECTED RNA POLYMERASES I, II, AND III 17.1 KDA POLYPEPTIDE" (SEQ ID No:172) or a functionally active derivative thereof, or a functionally active fragment thereof, or a homolog thereof, or a variant of "DNA-DIRECTED RNA POLYMERASES I, II, AND III 17.1 KDA POLYPEPTIDE" encoded by a nucleic acid that hybridizes to the "DNA-DIRECTED RNA POLYMERASES I, II, AND II( 17.1 KDA POLYPEPTIDE" nucleic acid under low stringency conditions,
   (xliv) "HYPOTHETICAL PROTEIN XP_108399" (SEQ ID No:173) or a functionally active derivative thereof, or a functionally active fragment thereof, or a homolog thereof, or a variant of "HYPOTHETICAL PROTEIN XP_108399" encoded by a nucleic acid that hybridizes to the "HYPOTHETICAL PROTEIN XP_108399" nucleic acid under low stringency conditions,
   (xlv) "SIMILAR TO CG5341 GENE PRODUCT" (SEQ ID No:174) or a functionally active derivative thereof, or a functionally active fragment thereof, or a homolog thereof, or a variant of "SIMILAR TO CG5341 GENE PRODUCT" encoded by a nucleic acid that hybridizes to the "SIMILAR TO CG5341 GENE PRODUCT" nucleic acid under low stringency conditions,
   (xlvi) "HYPOTHETICAL PROTEIN XP_108582" (SEQ ID No: 175) or a functionally active derivative thereof,or a functionally active fragement thereof, or a homolog thereof, or a variant of "HYPOTHETICAL PROTEIN XP_108582" that hybridizes to the "HYPOTHETICAL PROTEIN XP_108582" nucleic acid under low stringency conditions, and
   (xlvii) "SEC5 PROTEIN" (SEQ ID No:176) or a functionally active derivative thereof, or
   a functionally active fragment thereof, or a homolog thereof, or a variant of "SEC5 PROTEIN" encoded by a nucleic acid that hybridizes to the "SEC5 PROTEIN" nucleic acid under low stringency conditions,
as a target for an active agent of a pharmaceutical, preferably a drug target in the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer .

The present invention further relates to the IκBε protein complex
1. A protein complex selected from complex (I) and comprising
   (a) at least one first protein selected from the group consisting of:
      (i) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
      (ii) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
      (iii) "Cullin1" (SEQ ID No:136) or a functionally active derivative thereof, or a functionally active fragment thereof, or a homolog thereof, or a variant of "Cullin1" encoded by a nucleic acid that hybridizes to the "Cullin1" nucleic acid under low stringency conditions,
      (iv) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
      (v) "RelB" (SEQ ID No:81) or a functionally active derivative thereof, or a functionally active fragment thereof, or a homolog thereof, or a variant of "RelB" encoded by a nucleic acid that hybridizes to the "RelB" nucleic acid under low stringency conditions,
      (vi) "p105 (NF-KB1)" (SEQ ID No:74) or a functionally active derivative thereof, or a functionally active fragment thereof, or a homolog thereof, or a variant of "p105 (NF-KB1)" encoded by a nucleic acid that hybridizes to the "p105 (NF-KB1)" nucleic acid under low stringency conditions,
      (vii) "RelA (p65)" (SEQ ID No:75) or a functionally active derivative thereof, or a functionally active fragment thereof, or a homolog thereof, or a variant of "RelA (p65)" encoded by a nucleic acid that hybridizes to the "RelA (p65)" nucleic acid under low stringency conditions,
      (viii) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
      (ix) "β TrCP2 -1" (SEQ ID No:148) or a functionally active derivative thereof, or a functionally active fragment thereof, or a homolog thereof, or a variant of "β TrCP2 - 1" encoded by a nucleic acid that hybridizes to the "β TrCP2 - 1" nucleic acid under low stringency conditions,
      (x) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
      (xi) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
      (xii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
      (xiii) "UVEAL AUTOANTIGEN" (SEQ ID No:179) or a functionally active derivative thereof, or a functionally active fragment thereof, or a homolog thereof, or a variant of "UVEAL AUTOANTIGEN" encoded by a nucleic acid that hybridizes to the "UVEAL AUTOANTIGEN" nucleic acid under low stringency conditions,
      (xiv) "Hos1" (SEQ ID No:137) or a functionally active derivative thereof, or a functionally active fragment thereof, or a homolog thereof, or a variant of "Hos1" encoded by a nucleic acid that hybridizes to the "Hos1" nucleic acid under low stringency conditions,
      (xv) "SKP1 (SCF COMPLEX COMPONENT)" (SEQ ID No: 135) or a functionally active derivative thereof, or a functionally active fragment thereof, or a homolog thereof, or a variant of "SKP1 (SCF COMPLEX COMPONENT)" encoded by a nucleic acid that hybridizes to the "SKP1 (SCF COMPLEX COMPONENT)" nucleic acid under low stringency conditions,
      (xvi) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions, and
      (xvii) "β-TrCP1" (SEQ ID No:129) or a functionally active derivative thereof, or a functionally active fragment thereof, or a homolog thereof, or a variant of "β-TrCP1" encoded by a nucleic acid that hybridizes to the "β- TrCP1" nucleic acid under low stringency conditions,
      (xviii) "Roc1 /RBX1" (SEQ ID No:146) or a functionally active derivative thereof, or a functionally active fragment thereof, or a homolog thereof, or a variant of "Roc/RBX1" encoded by a nucleic acid that hybridizes to the "Roc1/RBX1" nucleic acid under low stringency conditions, and
      (xix) "SKP2 (SCF complex component)" (SEQ ID No:133) or a functionally active derivative thereof, or a functionally active fragment thereof, or a homolog thereof, or a variant of "SKP2 (SCF COMPLEX COMPONTENT" encoded by a nucleic acid that hybridizes to the "SKP2 (SCF COMPLEX COMPONENT" nucleic acid under low stringency conditions,
      and
   (b) at least one second protein, which second protein is selected from the group consisting of:
      (i) "KIAA0685 PROTEIN" (SEQ ID No:177) or a functionally active derivative thereof, or a functionally active fragment thereof, or a homolog thereof, or a variant of "KIAA0685 PROTEIN" encoded by a nucleic acid that hybridizes to the "KIAA0685 PROTEIN" nucleic acid under low stringency conditions,
      (ii) "SERINE/THREONINE PROTEIN PHOSPHATASE 6" (SEQ ID No:178) or a functionally active derivative thereof, or a functionally active fragment thereof, or a homolog thereof, or a variant of "SERINE/THREONINE PROTEIN PHOSPHATASE 6" encoded by a nucleic acid that hybridizes to the "SERINE/THREONINE PROTEIN PHOSPHATASE 6" nucleic acid under low stringency conditions,
      (iii) "ASB-3 PROTEIN" (SEQ ID No:149) or a functionally active derivative thereof, or a functionally active fragment thereof, or a homolog thereof, or a variant of "ASB-3 PROTEIN" encoded by a nucleic acid that hybridizes to the "ASB-3 PROTEIN" nucleic acid under low stringency conditions,
      (iv) "SIMILAR TO PYRROLINE 5-CARBOXYLATE REDUCTASE ISOFORM" (SEQ ID No:180) or a functionally active derivative thereof, or a functionally active fragment thereof, or a homolog thereof, or a variant of "SIMILAR TO PYRROLINE 5-CARBOXYLATE REDUCTASE ISOFORM" encoded by a nucleic acid that hybridizes to the "SIMILAR TO PYRROLINE 5-CARBOXYLATE REDUCTASE ISOFORM" nucleic acid under low stringency conditions,
      (v) "RAD50 HOMOLOG" (SEQ ID No:181) or a functionally active derivative thereof, or a functionally active fragment thereof, or a homolog thereof, or a variant of "RAD50 HOMOLOG " encoded by a nucleic acid that hybridizes to the "RAD50 HOMOLOG " nucleic acid under low stringency conditions,
      (vi) "SEROLOGICALLY DEFINED BREAST CANCER ANTIGEN NY-BR-16" (SEQ ID No:182) or a functionally active derivative thereof, or a functionally active fragment thereof, or a homolog thereof, or a variant of "SEROLOGICALLY DEFINED BREAST CANCER ANTIGEN NY-BR-16" encoded by a nucleic acid that hybridizes to the "SEROLOGICALLY DEFINED BREAST CANCER ANTIGEN NY-BR-16" nucleic acid under low stringency conditions,
      (vii) "KIAA1115 PROTEIN" (SEQ ID No:183) or a functionally active derivative thereof, or a functionally active fragment thereof, or a homolog thereof, or a variant of "KIAA1115 PROTEIN" encoded by a nucleic acid that hybridizes to the "KIAA1115 PROTEIN" nucleic acid under low stringency conditions,
      (viii) "FLJ00246 (FRAGMENT)" (SEQ ID No:184) or a functionally active derivative thereof, or a functionally active fragment thereof, or a homolog thereof, or a variant of "FLJ00246 (FRAGMENT)" encoded by a nucleic acid that hybridizes to the "FLJ00246 (FRAGMENT)" nucleic acid under low stringency conditions,
      (ix) "KIAA0379 (FRAGMENT)" (SEQ ID No:185) or a functionally active derivative thereof, or a functionally active fragment thereof, or a homolog thereof, or a variant of "KIAA0379 (FRAGMENT)" encoded by a nucleic acid that hybridizes to the "KIAA0379 (FRAGMENT)" nucleic acid under low stringency conditions, and
      (x) "COP9 SIGNALOSOME COMPLEX SUBUNIT 1" (SEQ ID No:186) or a functionally active derivative thereof, or a functionally active fragment thereof, or a homolog thereof, or a variant of "COP9 SIGNALOSOME COMPLEX SUBUNIT 1" encoded by a nucleic acid that hybridizes to the "COP9 SIGNALOSOME COMPLEX SUBUNIT 1" nucleic acid under low stringency conditions;
   and a complex (II) comprising at least two of said second proteins,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1 % SDS for 1.5 hours at 60°C.
2. The protein complex according to claim 1 wherein the first protein is the protein "IκB ε" (SEQ ID No:76) , or a functionally active derivative thereof, or a functionally active fragment thereof, or a homolog thereof, or a variant of "IκB ε" encoded by a nucleic acid that hybridizes to the "IκB ε" nucleic acid under low stringency conditions.
3. The protein complex according to No. 1 selected from complex (I) and comprising the following proteins:
   (i) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
   (ii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (iii) "p105 (NF-KB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (iv) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions,
   (v) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (vi) "SERINE/THREONINE PROTEIN PHOSPHATASE 6" (SEQ ID No:178) or a functionally active derivative thereof,or a functionally active fragement thereof, or a homolog thereof, or a variant of "SERINE/THREONINE PROTEIN PHOSPHATASE 6" that hybridizes to the "SERINE/THREONINE PROTEIN PHOSPHATASE 6" nucleic acid under low stringency conditions, and
   (vii) "KIAA0379 (FRAGMENT)" (SEQ ID No:185) or a functionally active derivative thereof, or a functionally active fragment thereof, or a homolog thereof, or a variant of "KIAA0379 (FRAGMENT)" encoded by a nucleic acid that hybridizes to the "KIAA0379 (FRAGMENT)" nucleic acid under low stringency conditions;
   and a protein complex selected from complex (II) and comprising the following proteins:
   (i) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
   (ii) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (iii) "p105 (NF-KB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (iv) "RelA (p65)" (SEQ ID No:75) or a functionally active derivative thereof, or a functionally active fragment thereof, or a homolog thereof, or a variant of "RelA (p65)" encoded by a nucleic acid that hybridizes to the "ReIA (p65)" nucleic acid under low stringency conditions,
   (v) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (vi) "SERINE/THREONINE PROTEIN PHOSPHATASE 6" (SEQ ID No:178) or a functionally active derivative thereof, or a functionally active fragment thereof, or a homolog thereof, or a variant of "SERINE/THREONINE PROTEIN PHOSPHATASE 6" encoded by a nucleic acid that hybridizes to the "SERINE/THREONINE PROTEIN PHOSPHATASE 6" nucleic acid under low stringency conditions,
   (vii) "KIAA0379 (FRAGMENT)" (SEQ ID No:185) or a functionally active derivative thereof, or a functionally active fragment thereof, or a homolog thereof, or a variant of "KIAA0379 (FRAGMENT)" encoded by a nucleic acid that hybridizes to the "KIAA0379 (FRAGMENT)" nucleic acid under low stringency conditions,
   (viii) "β TrCP2 - 1" (SEQ ID No:148) or a functionally active derivative thereof, or a functionally active fragment thereof, or a homolog thereof, or a variant of "β TrCP2 - 1" encoded by a nucleic acid that hybridizes to the "β TrCP2 - 1" nucleic acid under low stringency conditions,
   (ix) "COP9 SIGNALOSOME COMPLEX SUBUNIT 1" (SEQ ID No:186) or a functionally active derivative thereof,or a functionally active fragement thereof, or a homolog thereof, or a variant of "COP9 SIGNALOSOME COMPLEX SUBUNIT 1" that hybridizes to the "COP9 SIGNALOSOME COMPLEX SUBUNIT 1" nucleic acid under low stringency conditions, and
   (x) "Cullin1" (SEQ ID No:136) or a functionally active derivative thereof, or a functionally active fragment thereof, or a homolog thereof, or a variant of "Cullin 1" encoded by a nucleic acid that hybridizes to the "Cullin 1" nucleic acid under low stringency conditions; and a protein complex selected from complex (III) and comprising the following proteins:

   (i) "ASB-3 PROTEIN" (SEQ ID No:149) or a functionally active derivative thereof, or a functionally active fragment thereof, or a homolog thereof, or a variant of "ASB-3 PROTEIN" encoded by a nucleic acid that hybridizes to the "ASB-3 PROTEIN" nucleic acid under low stringency conditions,
   (ii) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
   (iii) "FLJ00246 (FRAGMENT)" (SEQ ID No:184) or a functionally active derivative thereof, or a functionally active fragment thereof, or a homolog thereof, or a variant of "FLJ00246 (FRAGMENT)" encoded by a nucleic acid that hybridizes to the "FLJ00246 (FRAGMENT)" nucleic acid under low stringency conditions,

   (i) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (v) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (vi) "KIAA0379 (FRAGMENT)" (SEQ ID No:185) or a functionally active derivative thereof, or a functionally active fragment thereof, or a homolog thereof, or a variant of "KIAA0379 (FRAGMENT)" encoded by a nucleic acid that hybridizes to the "KIAA0379 (FRAGMENT)" nucleic acid under low stringency conditions,
   (vii) "KIAA0685 PROTEIN" (SEQ ID No:177) or a functionally active derivative thereof, or a functionally active fragment thereof, or a homolog thereof, or a variant of "KlAA0685 PROTEIN" encoded by a nucleic acid that hybridizes to the "KIAA0685 PROTEIN" nucleic acid under low stringency conditions,
   (viii) "KIAA1115 PROTEIN" (SEQ ID No:183) or a functionally active derivative thereof, or a functionally active fragment thereof, or a homolog thereof, or a variant of "KIAA1115 PROTEIN" encoded by a nucleic acid that hybridizes to the "KIAA1115 PROTEIN" nucleic acid under low stringency conditions,

   (i) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (x) "p105 (NF-KB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (xi) "RAD50 HOMOLOG" (SEQ ID No:181) or a functionally active derivative thereof, or a functionally active fragment thereof, or a homolog thereof, or a variant of "RAD50 HOMOLOG" encoded by a nucleic acid that hybridizes to the "RAD50 HOMOLOG" nucleic acid under low stringency conditions,
   (xii) "RelB" (SEQ ID No:81) or a functionally active derivative thereof, or a functionally active fragment thereof, or a homolog thereof, or a variant of "RelB" encoded by a nucleic acid that hybridizes to the "RelB" nucleic acid under low stringency conditions,
   (xiii) "SERINE/THREONINE PROTEIN PHOSPHATASE 6" (SEQ ID No:178) or a functionally active derivative thereof, or a functionally active fragment thereof, or a homolog thereof, or a variant of "SERINE/THREONINE PROTEIN PHOSPHATASE 6" encoded by a nucleic acid that hybridizes to the "SERINE/THREONINE PROTEIN PHOSPHATASE 6" nucleic acid under low stringency conditions,
   (xiv) "SEROLOGICALLY DEFINED BREAST CANCER ANTIGEN NY-BR-16" (SEQ ID No:182) or a functionally active derivative thereof,or a functionally active fragement thereof, or a homolog thereof, or a variant of "SEROLOGICALLY DEFINED BREAST CANCER ANTIGEN NY-BR-16" that hybridizes to the "SEROLOGICALLY DEFINED BREAST CANCER ANTIGEN NY-BR-16" nucleic acid under low stringency conditions, and
   (xv) (xviii) "SIMILAR TO PYRROLINE 5-CARBOXYLATE REDUCTASE ISOFORM" (SEQ ID No:180) or a functionally active derivative thereof, or a functionally active fragment thereof, or a homolog thereof, or a variant of "SIMILAR TO PYRROLINE 5-CARBOXYLATE REDUCTASE ISOFORM" encoded by a nucleic acid that hybridizes to the "SIMILAR TO PYRROLINE 5-CARBOXYLATE REDUCTASE ISOFORM" nucleic acid under low stringency conditions;
   and a protein complex selected from complex (IV) and comprising the following proteins:
   (i) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (ii) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
   (iii) "Cullin1" (SEQ ID No:136) or a functionally active derivative thereof, or a functionally active fragment thereof, or a homolog thereof, or a variant of "Cullin 1" encoded by a nucleic acid that hybridizes to the "Cullin 1" nucleic acid under low stringency conditions,
   (iv) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (v) "RelB" (SEQ ID No:81) or a functionally active derivative thereof, or a functionally active fragment thereof, or a homolog thereof, or a variant of "RelB" encoded by a nucleic acid that hybridizes to the "RelB" nucleic acid under low stringency conditions,
   (vi) "p105 (NF-KB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (vii) "RelA (p65)" (SEQ ID No:75) or a functionally active derivative thereof, or a functionally active fragment thereof, or a homolog thereof, or a variant of "RelA (p65)" encoded by a nucleic acid that hybridizes to the "RelA (p65)" nucleic acid under low stringency conditions,
   (viii) "Roc1 / RBX1" (SEQ ID No:146) or a functionally active derivative thereof, or a functionally active fragment thereof, or a homolog thereof, or a variant of "Roc/RBX1" encoded by a nucleic acid that hybridizes to the "Roc1/RBX1" nucleic acid under low stringency conditions,
   (ix) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (x) "β TrCP2 - 1" (SEQ ID No:148) or a functionally active derivative thereof, or a functionally active fragment thereof, or a homolog thereof, or a variant of "β TrCP2 - 1" encoded by a nucleic acid that hybridizes to the "β TrCP2 - 1" nucleic acid under low stringency conditions,
   (xi) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (xii) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (xiii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (xiv) "KIAA0685 PROTEIN" (SEQ ID No:177) or a functionally active derivative thereof, or a functionally active fragment thereof, or a homolog thereof, or a variant of "KIAA0685 PROTEIN" encoded by a nucleic acid that hybridizes to the "KIAA0685 PROTEIN" nucleic acid under low stringency conditions,
   (xv) "SERINE/THREONINE PROTEIN PHOSPHATASE 6" (SEQ ID No:178) or a functionally active derivative thereof, or a functionally active fragment thereof, or a homolog thereof, or a variant of "SERINE/THREONINE PROTEIN PHOSPHATASE 6" encoded by a nucleic acid that hybridizes to the "SERINE/THREONINE PROTEIN PHOSPHATASE 6" nucleic acid under low stringency conditions,
   (xvi) "UVEAL AUTOANTIGEN" (SEQ ID No:179) or a functionally active derivative thereof, or a functionally active fragment thereof, or a homolog thereof, or a variant of "UVEAL AUTOANTIGEN" encoded by a nucleic acid that hybridizes to the "UVEAL AUTOANTIGEN" nucleic acid under low stringency conditions,
   (xvii) "ASB-3 PROTEIN" (SEQ ID No: 149) or a functionally active derivative thereof, or a functionally active fragment thereof, or a homolog thereof, or a variant of "ASB-3 PROTEIN" encoded by a nucleic acid that hybridizes to the "ASB-3 PROTEIN" nucleic. acid under low stringency conditions,
   (xviii) "SIMILAR TO PYRROLINE 5-CARBOXYLATE REDUCTASE ISOFORM" (SEQ ID No:180) or a functionally active derivative thereof, or a functionally active fragment thereof, or a homolog thereof, or a variant of "SIMILAR TO PYRROLINE 5-CARBOXYLATE REDUCTASE ISOFORM" encoded by a nucleic acid that hybridizes to the "SIMILAR TO PYRROLINE 5-CARBOXYLATE REDUCTASE ISOFORM" nucleic acid under low stringency conditions,
   (xix) "RAD50 HOMOLOG" (SEQ ID No:181) or a functionally active derivative thereof, or a functionally active fragment thereof, or a homolog thereof, or a variant of "RAD50 HOMOLOG" encoded by a nucleic acid that hybridizes to the "RAD50 HOMOLOG" nucleic acid under low stringency conditions,
   (xx) "SEROLOGICALLY DEFINED BREAST CANCER ANTIGEN NY-BR-16" (SEQ ID No:182) or a functionally active derivative thereof, or a functionally active fragment thereof, or a homolog thereof, or a variant of "SEROLOGICALLY DEFINED BREAST CANCER ANTIGEN NY-BR-16" encoded by a nucleic acid that hybridizes to the "SEROLOGICALLY DEFINED BREAST CANCER ANTIGEN NY-BR-16" nucleic acid under low stringency conditions,
   (xxi) "KIAA1115 PROTEIN" (SEQ ID No:183) or a functionally active derivative thereof, or a functionally active fragment thereof, or a homolog thereof, or a variant of "KIAA1115 PROTEIN" encoded by a nucleic acid that hybridizes to the "KIAA1115 PROTEIN" nucleic acid under low stringency conditions,
   (xxii) "Hos1" (SEQ ID No:137) or a functionally active derivative thereof, or a functionally active fragment thereof, or a homolog thereof, or a variant of "Hos1" encoded by a nucleic acid that hybridizes to the "Hos1" nucleic acid under low stringency conditions,
   (xxiii) "SKP2 (SCF complex component)" (SEQ ID No:133) or a functionally active derivative thereof, or a functionally active fragment thereof, or a homolog thereof, or a variant of "SKP2 (SCF COMPLEX COMPONTENT" encoded by a nucleic acid that hybridizes to the "SKP2 (SCF COMPLEX COMPONENT" nucleic acid under low stringency conditions,
   (xxiv) "SKP1 (SCF COMPLEX COMPONENT)" (SEQ ID No:135) or a functionally active derivative thereof, or a functionally active fragment thereof, or a homolog thereof, or a variant of "SKP1 (SCF COMPLEX COMPONTENT" encoded by a nucleic acid that hybridizes to the "SKP1 (SCF COMPLEX COMPONENT" nucleic acid under low stringency conditions,
   (xxv) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
   (xxvi) "β-TrCP1" (SEQ ID No:129) or a functionally active derivative thereof, or a functionally active fragment thereof, or a homolog thereof, or a variant of "β TrCP1" encoded by a nucleic acid that hybridizes to the "β TrCP1" nucleic acid under low stringency conditions,
   (xxvii) "FLJ00246 (FRAGMENT)" (SEQ ID No:184) or a functionally active derivative thereof, or a functionally active fragment thereof, or a homolog thereof, or a variant of "FLJ00246 (FRAGMENT)" encoded by a nucleic acid that hybridizes to the "FLJ00246 (FRAGMENT)" nucleic acid under low stringency conditions,
   (xxviii) "KIAA0379 (FRAGMENT)" (SEQ ID No:185) or a functionally active derivative thereof,or a functionally active fragement thereof, or a homolog thereof, or a variant of "KIAA0379 (FRAGMENT)" that hybridizes to the "KIAA0379 (FRAGMENT)" nucleic acid under low stringency conditions, and
   (xxix) "COP9 SIGNALOSOME COMPLEX SUBUNIT 1" (SEQ ID No:186) or a functionally active derivative thereof, or a functionally active fragment thereof, or a homolog thereof, or a variant of "COP9 SIGNALOSOME COMPLEX SUBUNIT 1" encoded by a nucleic acid that hybridizes to the "COP9 SIGNALOSOME COMPLEX SUBUNIT 1" nucleic acid under low stringency conditions.
4. The protein complex according to No. 1 comprising all but 1 - 9 of the following proteins:
   (i) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (ii) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
   (iii) "Cullin1" (SEQ ID No:136) or a functionally active derivative thereof, or a functionally active fragment thereof, or a homolog thereof, or a variant of "Cullin 1" encoded by a nucleic acid that hybridizes to the "Cullin 1" nucleic acid under low stringency conditions,
   (iv) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (v) "RelB" (SEQ ID No:81) or a functionally active derivative thereof, or a functionally active fragment thereof, or a homolog thereof, or a variant of "RelB" encoded by a nucleic acid that hybridizes to the "RelB" nucleic acid under low stringency conditions,
   (vi) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (vii) "RelA (p65)" (SEQ ID No:75) or a functionally active derivative thereof, or a functionally active fragment thereof, or a homolog thereof, or a variant of "RelA (p65)" encoded by a nucleic acid that hybridizes to the "RelA (p65)" nucleic acid under low stringency conditions,
   (viii) "Roc1 / RBX1" (SEQ ID No: 146) or a functionally active derivative thereof, or a functionally active fragment thereof, or a homolog thereof, or a variant of "Roc/RBX1" encoded by a nucleic acid that hybridizes to the "Roc1/RBX1" nucleic acid under low stringency conditions,
   (ix) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (x) "β TrCP2 - 1" (SEQ ID No:148) or a functionally active derivative thereof, or a functionally active fragment thereof, or a homolog thereof, or a variant of "β TrCP2 - 1" encoded by a nucleic acid that hybridizes to the "β TrCP2 - 1" nucleic acid under low stringency conditions,
   (xi) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (xii) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (xiii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (xiv) "KIAA0685 PROTEIN" (SEQ ID No:177) or a functionally active derivative thereof, or a functionally active fragment thereof, or a homolog thereof, or a variant of "KIAA0685 PROTEIN" encoded by a nucleic acid that hybridizes to the "KIAA0685 PROTEIN" nucleic acid under low stringency conditions,
   (xv) "SERINE/THREONINE PROTEIN PHOSPHATASE 6" (SEQ ID No:178) or a functionally active derivative thereof, or a functionally active fragment thereof, or a homolog thereof, or a variant of "SERINE/THREONINE PROTEIN PHOSPHATASE 6" encoded by a nucleic acid that hybridizes to the "SERINE/THREONINE PROTEIN PHOSPHATASE 6" nucleic acid under low stringency conditions,
   (xvi) "UVEAL AUTOANTIGEN" (SEQ ID No:179) or a functionally active derivative thereof, or a functionally active fragment thereof, or a homolog thereof, or a variant of "UVEAL AUTOANTIGEN" encoded by a nucleic acid that hybridizes to the "UVEAL AUTOANTIGEN" nucleic acid under low stringency conditions,
   (xvii) "ASB-3 PROTEIN" (SEQ ID No:149) or a functionally active derivative thereof, or a functionally active fragment thereof, or a homolog thereof, or a variant of "ASB-3 PROTEIN" encoded by a nucleic acid that hybridizes to the "ASB-3 PROTEIN" nucleic acid under low stringency conditions,
   (xviii) "SIMILAR TO PYRROLINE 5-CARBOXYLATE REDUCTASE ISOFORM" (SEQ ID No:180) or a functionally active derivative thereof, or a functionally active fragment thereof, or a homolog thereof, or a variant of "SIMILAR TO PYRROLINE 5-CARBOXYLATE REDUCTASE ISOFORM" encoded by a nucleic acid that hybridizes to the "SIMILAR TO PYRROLINE 5-CARBOXYLATE REDUCTASE ISOFORM" nucleic acid under low stringency conditions,
   (xix) "RAD50 HOMOLOG " (SEQ ID No:181) or a functionally active derivative thereof, or a functionally active fragment thereof, or a homolog thereof, or a variant of "RAD50 HOMOLOG" encoded by a nucleic acid that hybridizes to the "RAD50 HOMOLOG" nucleic acid under low stringency conditions,
   (xx) "SEROLOGICALLY DEFINED BREAST CANCER ANTIGEN NY-BR-16" (SEQ ID No: 182) or a functionally active derivative thereof, or a functionally active fragment thereof, or a homolog thereof, or a variant of "SEROLOGICALLY DEFINED BREAST CANCER ANTIGEN NY-BR-16" encoded by a nucleic acid that hybridizes to the "SEROLOGICALLY DEFINED BREAST CANCER ANTIGEN NY-BR-16" nucleic acid under low stringency conditions,
   (xxi) "KIAA1115 PROTEIN" (SEQ ID No:183) or a functionally active derivative thereof, or a functionally active fragment thereof, or a homolog thereof, or a variant of "KIAA1115 PROTEIN" encoded by a nucleic acid that hybridizes to the "KIAA1115 PROTEIN" nucleic acid under low stringency conditions,
   (xxii) "Hos1" (SEQ ID No:137) or a functionally active derivative thereof, or a functionally active fragment thereof, or a homolog thereof, or a variant of "Hos1" encoded by a nucleic acid that hybridizes to the "Hos1" nucleic acid under low stringency conditions,
   (xxiii) "SKP2 (SCF complex component)" (SEQ ID No:133) or a functionally active derivative thereof, or a functionally active fragment thereof, or a homolog thereof, or a variant of "SKP2 (SCF COMPLEX COMPONTENT" encoded by a nucleic acid that hybridizes to the "SKP2 (SCF COMPLEX COMPONENT" nucleic acid under low stringency conditions,
   (xxiv) "SKP1 (SCF COMPLEX COMPONENT)" (SEQ ID No:135) or a functionally active derivative thereof, or a functionally active fragment thereof, or a homolog thereof, or a variant of "SKP1 (SCF COMPLEX COMPONTENT" encoded by a nucleic acid that hybridizes to the "SKP1 (SCF COMPLEX COMPONENT" nucleic acid under low stringency conditions,
   (xxv) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
   (xxvi) "β-TrCP1" (SEQ ID No:129) or a functionally active derivative thereof, or a functionally active fragment thereof, or a homolog thereof, or a variant of "β TrCP1" encoded by a nucleic acid that hybridizes to the "β TrCP1" nucleic acid under low stringency conditions,
   (xxvii) "FLJ00246 (FRAGMENT)" (SEQ ID No:184) or a functionally active derivative thereof, or a functionally active fragment thereof, or a homolog thereof, or a variant of "FLJ00246 (FRAGMENT)" encoded by a nucleic acid that hybridizes to the "FLJ00246 (FRAGMENT)" nucleic acid under low stringency conditions,
   (xxviii) "KIAA0379 (FRAGMENT)" (SEQ ID No:185) or a functionally active derivative thereof,or a functionally active fragement thereof, or a homolog thereof, or a variant of "KIAA0379 (FRAGMENT)" that hybridizes to the "KIAA0379 (FRAGMENT)" nucleic acid under low stringency conditions, and
   (xxix) "COP9 SIGNALOSOME COMPLEX SUBUNIT 1" (SEQ ID No:186) or a functionally active derivative thereof, or a functionally active fragment thereof, or a homolog thereof, or a variant of "COP9 SIGNALOSOME COMPLEX SUBUNIT 1" encoded by a nucleic acid that hybridizes to the "COP9 SIGNALOSOME COMPLEX SUBUNIT 1" nucleic acid under low stringency conditions;
   and a protein complex selected from complex (II) comprising all but 1 - 9 of the following proteins:
   (i) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (ii) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
   (iii) "Cullin1" " (SEQ ID No:136) or a functionally active derivative thereof, or a functionally active fragment thereof, or a homolog thereof, or a variant of "Cullin 1" encoded by a nucleic acid that hybridizes to the "Cullin 1" nucleic acid under low stringency conditions,

   (i) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (v) "RelB" (SEQ ID No:81) or a functionally active derivative thereof, or a functionally active fragment thereof, or a homolog thereof, or a variant of "RelB" encoded by a nucleic acid that hybridizes to the "RelB" nucleic acid under low stringency conditions,

   (i) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (vii) "RelA (p65)" (SEQ ID No:75) or a functionally active derivative thereof, or a functionally active fragment thereof, or a homolog thereof, or a variant of "RelA (p65)" encoded by a nucleic acid that hybridizes to the "RelA (p65)" nucleic acid under low stringency conditions,

   (i) "β TrCP2 - 1" (SEQ ID No:148) or a functionally active derivative thereof, or a functionally active fragment thereof, or a homolog thereof, or a variant of "β TrCP2 - 1" encoded by a nucleic acid that hybridizes to the "β TrCP2 - 1" nucleic acid under low stringency conditions,
   (ix) "KIAA0685 PROTEIN" (SEQ ID No:177) or a functionally active derivative thereof, or a functionally active fragment thereof, or a homolog thereof, or a variant of "KIAA0685 PROTEIN" encoded by a nucleic acid that hybridizes to the "KIAA0685 PROTEIN" nucleic acid under low stringency conditions,
   (x) "SERINE/THREONINE PROTEIN PHOSPHATASE 6" (SEQ ID No:178) or a functionally active derivative thereof, or a functionally active fragment thereof, or a homolog thereof, or a variant of "SERINE/THREONINE PROTEIN PHOSPHATASE 6" encoded by a nucleic acid that hybridizes to the "SERINE/THREONINE PROTEIN PHOSPHATASE 6" nucleic acid under low stringency conditions,
   (xi) "ASB-3 PROTEIN" (SEQ ID No:149) or a functionally active derivative thereof, or a functionally active fragment thereof, or a homolog thereof, or a variant of "ASB-3 PROTEIN" encoded by a nucleic acid that hybridizes to the "ASB-3 PROTEIN" nucleic acid under low stringency conditions,
   (xii) "SIMILAR TO PYRROLINE 5-CARBOXYLATE REDUCTASE ISOFORM" (SEQ ID No:180) or a functionally active derivative thereof, or a functionally active fragment thereof, or a homolog thereof, or a variant of "SIMILAR TO PYRROLINE 5-CARBOXYLATE REDUCTASE ISOFORM" encoded by a nucleic acid that hybridizes to the "SIMILAR TO PYRROLINE 5-CARBOXYLATE REDUCTASE ISOFORM" nucleic acid under low stringency conditions,
   (xiii) "RAD50 HOMOLOG " (SEQ ID No:181) or a functionally active derivative thereof, or a functionally active fragment thereof, or a homolog thereof, or a variant of "RAD50 HOMOLOG" encoded by a nucleic acid that hybridizes to the "RAD50 HOMOLOG" nucleic acid under low stringency conditions,
   (xiv) "SEROLOGICALLY DEFINED BREAST CANCER ANTIGEN NY-BR-16" (SEQ ID No: 182) or a functionally active derivative thereof, or a functionally active fragment thereof, or a homolog thereof, or a variant of "SEROLOGICALLY DEFINED BREAST CANCER ANTIGEN NY-BR-16" encoded by a nucleic acid that hybridizes to the "SEROLOGICALLY DEFINED BREAST CANCER ANTIGEN NY-BR-16" nucleic acid under low stringency conditions,
   (xv) "KIAA1115 PROTEIN" (SEQ ID No:183) or a functionally active derivative thereof, or a functionally active fragment thereof, or a homolog thereof, or a variant of "KIAA1115 PROTEIN" encoded by a nucleic acid that hybridizes to the "KIAA1115 PROTEIN" nucleic acid under low stringency conditions,
   (xvi) "FLJ00246 (FRAGMENT)" (SEQ ID No:184) or a functionally active derivative thereof, or a functionally active fragment thereof, or a homolog thereof, or a variant of "FLJ00246 (FRAGMENT)" encoded by a nucleic acid that hybridizes to the "FLJ00246 (FRAGMENT)" nucleic acid under low stringency conditions,
   (xvii) "KIAA0379 (FRAGMENT)" (SEQ ID No:185) or a functionally active derivative thereof,or a functionally active fragement thereof, or a homolog thereof, or a variant of "KIAA0379 (FRAGMENT)" that hybridizes to the "KIAA0379 (FRAGMENT)" nucleic acid under low stringency conditions, and
   (xviii) "COP9 SIGNALOSOME COMPLEX SUBUNIT 1" (SEQ ID No:186) or a functionally active derivative thereof, or a functionally active fragment thereof, or a homolog thereof, or a variant of "COP9 SIGNALOSOME COMPLEX SUBUNIT 1" encoded by a nucleic acid that hybridizes to the "COP9 SIGNALOSOME COMPLEX SUBUNIT 1" nucleic acid under low stringency conditions.
5. The complex of any of No. 1 - 4 comprising a functionally active derivative of said first protein and/or a functionally active derivative of said second protein, wherein the functionally active derivative is a fusion protein comprising said first protein or said second protein fused to an amino acid sequence different from the first protein or second protein, respectively.
6. The complex of No. 5 wherein the functionally active derivative is a fusion protein comprising said first protein or said second protein fused to an affinity tag or label.
7. The complex of any of No. 1-4 comprising a fragment of said first protein and/or a fragment of said second protein, which fragment binds to another protein component of said complex.
8. The complex of any of No. 1 -7 that is involved in the deubiquitination activity.
9. A process for preparing complex of any of No. 1-8 and optionally the components thereof comprising the following steps:
   the expressing a protein (bait) of the complex, preferably a tagged protein, in a target cell, isolating the protein complex which is attached to the bait protein, and optionally disassociating the protein complex and isolating the individual complex members.
10. The process according to No. 9 wherein the tagged protein comprises two different tags which allow two separate affinity purification steps.
11. The process according to any of No. 9-10 wherein the two tags are separated by a cleavage site for a protease.
12. Component of the IκBε complex obtainable by a process according to any of No. 9 to 11.
13. Protein of the IκBε complex selected from
   (i) "KIAA0685 PROTEIN" (SEQ ID No:177) or a functionally active derivative thereof, or a functionally active fragment thereof, or a homolog thereof, or a variant of "KIAA0685 PROTEIN" encoded by a nucleic acid that hybridizes to the "KIAA0685 PROTEIN" nucleic acid under low stringency conditions,
   (ii) "SEROLOGICALLY DEFINED BREAST CANCER ANTIGEN NY-BR-16" (SEQ ID No: 182) or a functionally active derivative thereof, or a functionally active fragment thereof, or a homolog thereof, or a variant of "SEROLOGICALLY DEFINED BREAST CANCER ANTIGEN NY-BR-16" encoded by a nucleic acid that hybridizes to the "SEROLOGICALLY DEFINED BREAST CANCER ANTIGEN NY-BR-16" nucleic acid under low stringency conditions,
   (iii) "KIAA1115 PROTEIN" (SEQ ID No:183) or a functionally active derivative thereof, or a functionally active fragment thereof, or a homolog thereof, or a variant of "KIAA1115 PROTEIN" encoded by a nucleic acid that hybridizes to the "KIAA1115 PROTEIN" nucleic acid under low stringency conditions,
   (iv) "FLJ00246 (FRAGMENT)" (SEQ ID No:184) or a functionally active derivative thereof, or a functionally active fragment thereof, or a homolog thereof, or a variant of "FLJ00246 (FRAGMENT)" encoded by a nucleic acid that hybridizes to the "FLJ00246 (FRAGMENT)" nucleic acid under low stringency conditions, and
   (v) "KIAA0379 (FRAGMENT)" (SEQ ID No:185) or a functionally active derivative thereof, or a functionally active fragment thereof, or a homolog thereof, or a variant of "KIAA0379 (FRAGMENT)" encoded by a nucleic acid that hybridizes to the "KIAA0379 (FRAGMENT)" nucleic acid under low stringency conditions,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
14. Nucleic acid encoding a protein according to No. 13.
15. Construct, preferably a vector construct, comprising (a) a nucleic acid according to No. 14 and at least one further nucleic acid which is normally not associated with said nucleic acid,or(b) at least two separate nucleic acid sequences each encoding a different protein, or a functionally active fragment or a functionally active derivative thereof at least one of said proteins, or functionally active fragments or functionally active derivative thereof being selected from the first group of proteins according to No. 1(a) and at least one of said proteins, or functionally active fragments or functionally active derivative thereof being selected from the second group of proteins according to No. 1 (b).
16. Host cell, containing a vector comprising at least one of the nucleic acid of No. 14 and/or a construct of No. 15 or containing several vectors each comprising at least the nucleic acid sequence encoding at least one of the proteins, or functionally active fragments or functionally active derivatives thereof selected from the first group of proteins according to No. 1(a) and the proteins, or functionally active fragments or functionally active derivatives thereof selected from the second group of proteins according to No. 1 (b).
17. An antibody or a fragment of said antibody containing the binding domain thereof, selected from an antibody or fragment thereof, which binds the complex of any of No. 1-8 and which does not bind any of the proteins of said complex when uncomplexed and an antibody or a fragment of said antibody which binds to any of the proteins according to No. 13.
18. A kit comprising in one or more container the complex of any of No. 1 - 8 and/or the proteins of No. 13optionally together with an antibody according to No. 17 and/or further components such as reagents and working instructions.
19. The kit according to No.18 for processing a substrate of said complex.
20. The kit according to No. 18 for the diagnosis or prognosis of a disease or a disease risk, preferentially for a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
21. Array, in which at least a complex according to any of No. 1- 8 and/or at least one protein according to No. 14 and/or at least one antibody according to No. 17 is attached to a solid carrier.
22. A process for processing a physiological substrate of the complex comprising the step of bringing into contact a complex to any of No. 1-8 with said substrate, such that said substrate is processed.
23. A pharmaceutical composition comprising the protein complex of any of No. 1 to 8 and/or any of the following the proteins:
   (i) "KIAA0685 PROTEIN" (SEQ ID No:177) or a functionally active derivative thereof, or a functionally active fragment thereof, or a homolog thereof, or a variant of "KIAA0685 PROTEIN" encoded by a nucleic acid that hybridizes to the "KIAA0685 PROTEIN" nucleic acid under low stringency conditions,
   (ii) "SEROLOGICALLY DEFINED BREAST CANCER ANTIGEN NY-BR-16" (SEQ ID No: 182) or a functionally active derivative thereof, or a functionally active fragment thereof, or a homolog thereof, or a variant of "SEROLOGICALLY DEFINED BREAST CANCER ANTIGEN NY-BR-16" encoded by a nucleic acid that hybridizes to the "SEROLOGICALLY DEFINED BREAST CANCER ANTIGEN NY-BR-16" nucleic acid under low stringency conditions,
   (iii) "KIAA1115 PROTEIN" (SEQ ID No:183) or a functionally active derivative thereof, or a functionally active fragment thereof, or a homolog thereof, or a variant of "KIAA1115 PROTEIN" encoded by a nucleic acid that hybridizes to the "KlAA1115 PROTEIN" nucleic acid under low stringency conditions,
   (iv) "FLJ00246 (FRAGMENT)" (SEQ ID No:184) or a functionally active derivative thereof,or a functionally active fragement thereof, or a homolog thereof, or a variant of "FLJ00246 (FRAGMENT)" that hybridizes to the "FLJ00246 (FRAGMENT)" nucleic acid under low stringency conditions, and
   (v) "KIAA0379 (FRAGMENT)" (SEQ ID No:185) or a functionally active derivative thereof, or a functionally active fragment thereof, or a homolog thereof, or a variant of "KIAA0379 (FRAGMENT)" encoded by a nucleic acid that hybridizes to the "KIAA0379 (FRAGMENT)" nucleic acid under low stringency conditions,
   and a pharmaceutical acceptable carrier.
24. The pharmaceutical composition according to No. 23 for the treatment of diseases and disorders such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
25. A method for screening for a molecule that binds to the complex of anyone of No. 1 - 8 and/or any of the following the proteins:
   (i) "KIAA0685 PROTEIN" (SEQ ID No:177) or a functionally active derivative thereof, or a functionally active fragment thereof, or a homolog thereof, or a variant of "KIAA0685 PROTEIN" encoded by a nucleic acid that hybridizes to the "KIAA0685 PROTEIN" nucleic acid under low stringency conditions,
   (ii) "SEROLOGICALLY DEFINED BREAST CANCER ANTIGEN NY-BR-16" (SEQ ID No:182) or a functionally active derivative thereof, or a functionally active fragment thereof, or a homolog thereof, or a variant of "SEROLOGICALLY DEFINED BREAST CANCER ANTIGEN NY-BR-16" encoded by a nucleic acid that hybridizes to the "SEROLOGICALLY DEFINED BREAST CANCER ANTIGEN NY-BR-16" nucleic acid under low stringency conditions,
   (iii) "KIAA1115 PROTEIN" (SEQ ID No:183) or a functionally active derivative thereof, or a functionally active fragment thereof, or a homolog thereof, or a variant of "KIAA1115 PROTEIN" encoded by a nucleic acid that hybridizes to the "KIAA1115 PROTEIN" nucleic acid under low stringency conditions,
   (iv) "FLJ00246 (FRAGMENT)" (SEQ ID No:184) or a functionally active derivative thereof,or a functionally active fragement thereof, or a homolog thereof, or a variant of "FLJ00246 (FRAGMENT)" that hybridizes to the "FLJ00246 (FRAGMENT)" nucleic acid under low stringency conditions, and
   (v) "KIAA0379 (FRAGMENT)" (SEQ ID No:185) or a functionally active derivative thereof, or a functionally active fragment thereof, or a homolog thereof, or a variant of "KIAA0379 (FRAGMENT)" encoded by a nucleic acid that hybridizes to the "KIAA0379 (FRAGMENT)" nucleic acid under low stringency conditions, comprising the steps of
      (a) exposing said complex, or a cell or organism containing same to one or more candidate molecules; and
      (b) determinig whether said candidate molecule is bound to the complex or protein.
26. A method for screening for a molecule that modulates directly or indirectly the function, activity, composition or formation of the complex of any one of No. 1 - 8 comprising the steps of (a) exposing said complex, or a cell or organism containing IκBε complex to one or more candidate molecules; and
   (b) determining the amount of activity of protein components of, and/or intracellular localization of, said complex and/or the transcription level of a gene dependent on the complex and/or the abundance and/or activity of a protein or protein complex dependent on the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependent on the complex and/or the abundance and/or activity of a protein or protein complex dependent on the function of the complex and/or product of a gene dependent on the complex in the absence of said candidate molecules indicates that the molecule modulates function, activity or composition of said complex.
27. The method of No. 26, wherein the amount of said complex is determined.
28. The method of No. 26, wherein the activity of said complex is determined.
29. The method of No. 28, wherein said determining step comprises isolating from the cell or organism said complex to produce said isolated complex and contacting said isolated complex in the presence or absence of a candidate molecule with a substrate of said complex and determining the processing of said substrate is modified in the presence of said candidate molecule.
30. The method of No. 26, wherein the amount of the individual protein components of said complex are determined.
31. The method of No. 30, determining step comprises determining whether
   (i) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions, and/or
   (ii) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions, and/or
   (iii) "Cullin 1" (SEQ ID No:136) or a functionally active derivative thereof, or a functionally active fragment thereof, or a homolog thereof, or a variant of "Cullin 1" encoded by a nucleic acid that hybridizes to the "Cullin 1" nucleic acid under low stringency conditions, and/or
   (iv) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions, and/or
   (v) "ReIB" (SEQ ID No:81) or a functionally active derivative thereof, or a functionally active fragment thereof, or a homolog thereof, or a variant of "ReIB" encoded by a nucleic acid that hybridizes to the "ReIB" nucleic acid under low stringency conditions, and/or
   (vi) "p105 (NF-kB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions, and/or
   (vii) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions, and/or
   (viii) "Roc1 /RBX1" (SEQ ID No: 146) or a functionally active derivative thereof, or a functionally active fragment thereof, or a homolog thereof, or a variant of "Roc/RBX1" encoded by a nucleic acid that hybridizes to the "Roc1/RBX1" nucleic acid under low stringency conditions, and/or
   (ix) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions, and/or
   (x) "β TrCP2 -1" (SEQ ID No:148) or a functionally active derivative thereof, or a functionally active fragment thereof, or a homolog thereof, or a variant of "β TrCP2 -1" encoded by a nucleic acid that hybridizes to the "β TrCP2 - 1" nucleic acid under low stringency conditions, and/or
   (xi) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions, and/or
   (xii) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions, and/or
   (xiii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions, and/or
   (xiv) "KIAA0685 PROTEIN" (SEQ ID No:177) or a functionally active derivative thereof, or a functionally active fragment thereof, or a homolog thereof, or a variant of "KIAA0685 PROTEIN" encoded by a nucleic acid that hybridizes to the "KIAA0685 PROTEIN" nucleic acid under low stringency conditions, and/or
   (xv) "SERINE/THREONINE PROTEIN PHOSPHATASE 6" (SEQ ID No:178) or a functionally active derivative thereof, or a functionally active fragment thereof, or a homolog thereof, or a variant of "SERINE/THREONINE PROTEIN PHOSPHATASE 6" encoded by a nucleic acid that hybridizes to the "SERINE/THREONINE PROTEIN PHOSPHATASE 6" nucleic acid under low stringency conditions, and/or
   (xvi) "UVEAL AUTOANTIGEN" (SEQ ID No: 179) or a functionally active derivative thereof, or a functionally active fragment thereof, or a homolog thereof, or a variant of "UVEAL AUTOANTIGEN" encoded by a nucleic acid that hybridizes to the "UVEAL AUTOANTIGEN" nucleic acid under low stringency conditions, and/or
   (xvii) "ASB-3 PROTEIN" (SEQ ID No: 149) or a functionally active derivative thereof, or a functionally active fragment thereof, or a homolog thereof, or a variant of "ASB-3 PROTEIN" encoded by a nucleic acid that hybridizes to the "ASB-3 PROTEIN" nucleic acid under low stringency conditions, and/or
   (xviii) "SIMILAR TO PYRROLINE 5-CARBOXYLATE REDUCTASE ISOFORM" (SEQ ID No:180) or a functionally active derivative thereof, or a functionally active fragment thereof, or a homolog thereof, or a variant of "SIMILAR TO PYRROLINE 5-CARBOXYLATE REDUCTASE ISOFORM" encoded by a nucleic acid that hybridizes to the "SIMILAR TO PYRROLINE 5-CARBOXYLATE REDUCTASE ISOFORM" nucleic acid under low stringency conditions, and/or
   (xix) "RAD50 HOMOLOG " (SEQ ID No:181) or a functionally active derivative thereof, or a functionally active fragment thereof, or a homolog thereof, or a variant of "RAD50 HOMOLOG" encoded by a nucleic acid that hybridizes to the "RAD50 HOMOLOG" nucleic acid under low stringency conditions, and/or
   (xx) "SEROLOGICALLY DEFINED BREAST CANCER ANTIGEN NY-BR-16" (SEQ ID No:182) or a functionally active derivative thereof, or a functionally active fragment thereof, or a homolog thereof, or a variant of "SEROLOGICALLY DEFINED BREAST CANCER ANTIGEN NY-BR-16" encoded by a nucleic acid that hybridizes to the "SEROLOGICALLY DEFINED BREAST CANCER ANTIGEN NY-BR-16" nucleic acid under low stringency conditions, and/or
   (xxi) "KIAA1115 PROTEIN" (SEQ ID No:183) or a functionally active derivative thereof, or a functionally active fragment thereof, or a homolog thereof, or a variant of "KIAA1115 PROTEIN" encoded by a nucleic acid that hybridizes to the "KIAA1115 PROTEIN" nucleic acid under low stringency conditions, and/or
   (xxii) "Hos1" (SEQ ID No:137) or a functionally active derivative thereof, or a functionally active fragment thereof, or a homolog thereof, or a variant of "Hos1" encoded by a nucleic acid that hybridizes to the "Hos1" nucleic acid under low stringency conditions, and/or
   (xxiii) "SKP2 (SCF complex component)" (SEQ ID No:133) or a functionally active derivative thereof, or a functionally active fragment thereof, or a homolog thereof, or a variant of "SKP2 (SCF COMPLEX COMPONTENT" encoded by a nucleic acid that hybridizes to the "SKP2 (SCF COMPLEX COMPONENT" nucleic acid under low stringency conditions, and/or
   (xxiv) "SKP1 (SCF COMPLEX COMPONENT)" (SEQ ID No:135) or a functionally active derivative thereof, or a functionally active fragment thereof, or a homolog thereof, or a variant of "SKP1 (SCF COMPLEX COMPONTENT" encoded by a nucleic acid that hybridizes to the "SKP1 (SCF COMPLEX COMPONENT" nucleic acid under low stringency conditions, and/or
   (xxv) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions, and/or
   (xxvi) "β-TrCP1" (SEQ ID No:129) or a functionally active derivative thereof, or a functionally active fragment thereof, or a homolog thereof, or a variant of "β TrCP1" encoded by a nucleic acid that hybridizes to the "β TrCP1" nucleic acid under low stringency conditions, and/or
   (xxvii) "FLJ00246 (FRAGMENT)" (SEQ ID No:184) or a functionally active derivative thereof, or a functionally active fragment thereof, or a homolog thereof, or a variant of "FLJ00246 (FRAGMENT)" encoded by a nucleic acid that hybridizes to the "FLJ00246 (FRAGMENT)" nucleic acid under low stringency conditions, and/or
   (xxviii) "KIAA0379 (FRAGMENT)" (SEQ ID No:185) or a functionally active derivative thereof, or a functionally active fragment thereof, or a homolog thereof, or a variant of "KIAA0379 (FRAGMENT)" encoded by a nucleic acid that hybridizes to the "KIAA0379 (FRAGMENT)" nucleic acid under low stringency conditions, and/or
   (xxix) "COP9 SIGNALOSOME COMPLEX SUBUNIT 1" (SEQ ID No:186) or a functionally active derivative thereof, or a functionally active fragment thereof, or a homolog thereof, or a variant of "COP9 SIGNALOSOME COMPLEX SUBUNIT 1" encoded by a nucleic acid that hybridizes to the "COP9 SIGNALOSOME COMPLEX SUBUNIT 1" nucleic acid under low stringency conditions,
   is present in the complex.
32. The method of any of No. 26 to 31, wherein said method is a method of screening for a drug for treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
33. Use of a molecule that modulates the amount of, activity of, or the protein components of the complex of any one of No. 1 - 8 for the manufacture of a medicament for the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
34. A method for the production of a pharmaceutical composition comprising carrying out the method of any of No. 1 - 8 to identify a molecule that modulates the function, activity, composition or formation of said complex, and further comprising mixing the identified molecule with a pharmaceutically acceptable carrier.
35. A method for diagnosing or screening for the presence of a disease or disorder or a predisposition for developing a disease or disorder in a subject, which disease or disorder is characterized by an aberrant amount of, activity of, or component composition of, or intracellular localization of the complex of any one of the No. 1-8, comprising determining the amount of, activity of, protein components of, and/or intracellular localization of, said complex and/or the transcription level of a gene dependent on the complex and/or the abundance and/or activity of a protein or protein complex dependent on the function of the complex and/or product of a gene dependent on the complex in a comparative sample derived from a subject, wherein a difference in said amount, activity, or protein components of, said complex in an analogous sample from a subject not having the disease or disorder or predisposition indicates the presence in the subject of the disease or disorder or predisposition in the subject.
36. The method of No. 35, wherein the amount of said complex is determined.
37. The method of No. 35, wherein the activity of said complex is determined.
38. The method of No. 37, wherein said determining step comprises isolating from the subject said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
39. The method of No. 35, wherein the amount of the individual protein components of said complex are determined.
40. The method of No. 39, wherein said determining step comprises determining whether
   (i) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions, and/or
   (ii) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions, and/or
   (iii) "Cullin1" (SEQ ID No:136) or a functionally active derivative thereof, or a functionally active fragment thereof, or a homolog thereof, or a variant of "Cullin 1" encoded by a nucleic acid that hybridizes to the "Cullin 1" nucleic acid under low stringency conditions, and/or
   (iv) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions, and/or
   (v) "RelB" (SEQ ID No:81) or a functionally active derivative thereof, or a functionally active fragment thereof, or a homolog thereof, or a variant of "RelB" encoded by a nucleic acid that hybridizes to the "RelB" nucleic acid under low stringency conditions, and/or
   (vi) "p105 (NF-KB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions, and/or
   (vii) "ReIA (p65)" (SEQ ID No:75) or a functionally active derivative thereof, or a functionally active fragment thereof, or a homolog thereof, or a variant of "ReIA (p65)" encoded by a nucleic acid that hybridizes to the "ReIA (p65)" nucleic acid under low stringency conditions, and/or
   (viii) "Roc1 / RBX1" (SEQ ID No:146) or a functionally active derivative thereof, or a functionally active fragment thereof, or a homolog thereof, or a variant of "Roc/RBX1" encoded by a nucleic acid that hybridizes to the "Roc1/RBX1" nucleic acid under low stringency conditions, and/or
   (ix) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions, and/or
   (x) "β TrCP2-1" (SEQ ID No:148) or a functionally active derivative thereof, or a functionally active fragment thereof, or a homolog thereof, or a variant of "β TrCP2-1" encoded by a nucleic acid that hybridizes to the "β TrCP2 - 1" nucleic acid under low stringency conditions, and/or
   (xi) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions, and/or
   (xii) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions, and/or
   (xiii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions, and/or
   (xiv) "KIAA0685 PROTEIN" (SEQ ID No:177) or a functionally active derivative thereof, or a functionally active fragment thereof, or a homolog thereof, or a variant of "KIAA0685 PROTEIN" encoded by a nucleic acid that hybridizes to the "KIAA0685 PROTEIN" nucleic acid under low stringency conditions, and/or
   (xv) "SERINE/THREONINE PROTEIN PHOSPHATASE 6" (SEQ ID No:178) or a functionally active derivative thereof, or a functionally active fragment thereof, or a homolog thereof, or a variant of "SERINE/THREONINE PROTEIN PHOSPHATASE 6" encoded by a nucleic acid that hybridizes to the "SERINE/THREONINE PROTEIN PHOSPHATASE 6" nucleic acid under low stringency conditions, and/or
   (xvi) "UVEAL AUTOANTIGEN" (SEQ ID No:179) or a functionally active derivative thereof, or a functionally active fragment thereof, or a homolog thereof, or a variant of "UVEAL AUTOANTIGEN" encoded by a nucleic acid that hybridizes to the "UVEAL AUTOANTIGEN" nucleic acid under low stringency conditions, and/or
   (xvii) "ASB-3 PROTEIN" (SEQ ID No:149) or a functionally active derivative thereof, or a functionally active fragment thereof, or a homolog thereof, or a variant of "ASB-3 PROTEIN" encoded by a nucleic acid that hybridizes to the "ASB-3 PROTEIN" nucleic acid under low stringency conditions, and/or
   (xviii) "SIMILAR TO PYRROLINE 5-CARBOXYLATE REDUCTASE ISOFORM" (SEQ ID No:180) or a functionally active derivative thereof, or a functionally active fragment thereof, or a homolog thereof, or a variant of "SIMILAR TO PYRROLINE 5-CARBOXYLATE REDUCTASE ISOFORM" encoded by a nucleic acid that hybridizes to the "SIMILAR TO PYRROLINE 5-CARBOXYLATE REDUCTASE ISOFORM" nucleic acid under low stringency conditions, and/or
   (xix) "RAD50 HOMOLOG" (SEQ ID No:181) or a functionally active derivative thereof, or a functionally active fragment thereof, or a homolog thereof, or a variant of "RAD50 HOMOLOG" encoded by a nucleic acid that hybridizes to the "RAD50 HOMOLOG" nucleic acid under low stringency conditions, and/or
   (xx) "SEROLOGICALLY DEFINED BREAST CANCER ANTIGEN NY-BR-16" (SEQ ID No: 182) or a functionally active derivative thereof, or a functionally active fragment thereof, or a homolog thereof, or a variant of "SEROLOGICALLY DEFINED BREAST CANCER ANTIGEN NY-BR-16" encoded by a nucleic acid that hybridizes to the "SEROLOGICALLY DEFINED BREAST CANCER ANTIGEN NY-BR-16" nucleic acid under low stringency conditions, and/or
   (xxi) "KIAA1115 PROTEIN" (SEQ ID No:183) or a functionally active derivative thereof, or a functionally active fragment thereof, or a homolog thereof, or a variant of "KIAA1115 PROTEIN" encoded by a nucleic acid that hybridizes to the "KIAA1115 PROTEIN" nucleic acid under low stringency conditions, and/or
   (xxii) "Hos1" (SEQ ID No:137) or a functionally active derivative thereof, or a functionally active fragment thereof, or a homolog thereof, or a variant of "Hos1" encoded by a nucleic acid that hybridizes to the "Hos1" nucleic acid under low stringency conditions, and/or
   (xxiii) "SKP2 (SCF complex component)" (SEQ ID No:133) or a functionally active derivative thereof, or a functionally active fragment thereof, or a homolog thereof, or a variant of "SKP2 (SCF COMPLEX COMPONTENT" encoded by a nucleic acid that hybridizes to the "SKP2 (SCF COMPLEX COMPONENT" nucleic acid under low stringency conditions, and/or
   (xxiv) "SKP1 (SCF COMPLEX COMPONENT)" (SEQ ID No:135) or a functionally active derivative thereof, or a functionally active fragment thereof, or a homolog thereof, or a variant of "SKP1 (SCF COMPLEX COMPONTENT" encoded by a nucleic acid that hybridizes to the "SKP1 (SCF COMPLEX COMPONENT" nucleic acid under low stringency conditions, and/or
   (xxv) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions, and/or
   (xxvi) "β-TrCP1" (SEQ ID No:129) or a functionally active derivative thereof, or a functionally active fragment thereof, or a homolog thereof, or a variant of "β TrCP1" encoded by a nucleic acid that hybridizes to the "β TrCP1" nucleic acid under low stringency conditions, and/or
   (xxvii) "FLJ00246 (FRAGMENT)" (SEQ ID No:184) or a functionally active derivative thereof, or a functionally active fragment thereof, or a homolog thereof, or a variant of "FLJ00246 (FRAGMENT)" encoded by a nucleic acid that hybridizes to the "FLJ00246 (FRAGMENT)" nucleic acid under low stringency conditions, and/or
   (xxviii) "KIAA0379 (FRAGMENT)" (SEQ ID No:185) or a functionally active derivative thereof, or a functionally active fragment thereof, or a homolog thereof, or a variant of "KIAA0379 (FRAGMENT)" encoded by a nucleic acid that hybridizes to the "KIAA0379 (FRAGMENT)" nucleic acid under low stringency conditions, and/or
   (xxix) "COP9 SIGNALOSOME COMPLEX SUBUNIT 1" (SEQ ID No:186) or a functionally active derivative thereof, or a functionally active fragment thereof, or a homolog thereof, or a variant of "COP9 SIGNALOSOME COMPLEX SUBUNIT 1" encoded by a nucleic acid that hybridizes to the "COP9 SIGNALOSOME COMPLEX SUBUNIT 1" nucleic acid under low stringency conditions,
   is present in the complex.
41. The complex of any one of No. 1 - 8, or proteins of No. 13 or the antibody or fragment of No. 17, for use in a method of diagnosing a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
42. A method for treating or preventing a disease or disorder characterized by an aberrant amount of, activity or component composition of or intracellular localization of, the complex of anyone of No. 1-8, comprising administering to a subject in need of such treatment or prevention a therapeutically effective amount of one or more molecules that modulate the amount of, deubiquitination activity, or protein components of, said complex.
43. The method according to No. 42, wherein said disease or disorder involves decreased levels of the amount or activity of said complex.
44. The method according to No. 42 , wherein said disease or disorder involves increased levels of the amount or activity of said complex.
45. Complex of any of No. 1 - 8 and/or protein selected from the following proteins
   (i) "p100 (NF-κB2)" (SEQ ID No:72) or a functionally active derivative thereof, or a functionally active fragment thereof, or a homolog thereof, or a variant of "p100 (NF-κB2)" encoded by a nucleic acid that hybridizes to the "p100 (NF-κB2)" nucleic acid under low stringency conditions,
   (ii) "c-Rel" (SEQ ID No:71) or a functionally active derivative thereof, or a functionally active fragment thereof, or a homolog thereof, or a variant of "c-Rel" encoded by a nucleic acid that hybridizes to the "c-Rel" nucleic acid under low stringency conditions,
   (iii) "Cullin1" (SEQ ID No:136) or a functionally active derivative thereof, or a functionally active fragment thereof, or a homolog thereof, or a variant of "Cullin 1" encoded by a nucleic acid that hybridizes to the "Cullin 1" nucleic acid under low stringency conditions,
   (iv) "IκB ε" (SEQ ID No:76) or a functionally active derivative thereof, or a functionally active fragment thereof, or a homolog thereof, or a variant of "IκB ε" encoded by a nucleic acid that hybridizes to the "IκB ε" nucleic acid under low stringency conditions,
   (v) "ReIB" (SEQ ID No:81) or a functionally active derivative thereof, or a functionally active fragment thereof, or a homolog thereof, or a variant of "ReIB" encoded by a nucleic acid that hybridizes to the "ReIB" nucleic acid under low stringency conditions,
   (vi) "p105 (NF-κB1)" (SEQ ID No:74) or a functionally active derivative thereof, or a functionally active fragment thereof, or a homolog thereof, or a variant of "p105 (NF-κB1)" encoded by a nucleic acid that hybridizes to the "p105 (NF-κB1)" nucleic acid under low stringency conditions,
   (vii) "RelA (p65)" (SEQ ID No:75) or a functionally active derivative thereof, or a functionally active fragment thereof, or a homolog thereof, or a variant of "RelA (p65)" encoded by a nucleic acid that hybridizes to the "RelA (p65)" nucleic acid under low stringency conditions,
   (viii) "Roc1 / RBX1" (SEQ ID No:146) or a functionally active derivative thereof, or a functionally active fragment thereof, or a homolog thereof, or a variant of "Roc/RBX1" encoded by a nucleic acid that hybridizes to the "Roc1/RBX1" nucleic acid under low stringency conditions,
   (ix) "IκB β" (SEQ ID No:77) or a functionally active derivative thereof, or a functionally active fragment thereof, or a homolog thereof, or a variant of "IκB β" encoded by a nucleic acid that hybridizes to the "IκB β" nucleic acid under low stringency conditions,
   (x) "β TrCP2 -1" (SEQ ID No:148) or a functionally active derivative thereof, or a functionally active fragment thereof, or a homolog thereof, or a variant of "β TrCP2 -1" encoded by a nucleic acid that hybridizes to the "β TrCP2 -1" nucleic acid under low stringency conditions,
   (xi) "IKK β" (SEQ ID No:24) or a functionally active derivative thereof, or a functionally active fragment thereof, or a homolog thereof, or a variant of "IKK β" encoded by a nucleic acid that hybridizes to the "IKK β" nucleic acid under low stringency conditions,
   (xii) "IKK α" (SEQ ID No:25) or a functionally active derivative thereof, or a functionally active fragment thereof, or a homolog thereof, or a variant of "IKK α" encoded by a nucleic acid that hybridizes to the "IKK α" nucleic acid under low stringency conditions,
   (xiii) "IKK γ" (SEQ ID No:23) or a functionally active derivative thereof, or a functionally active fragment thereof, or a homolog thereof, or a variant of "IKK γ" encoded by a nucleic acid that hybridizes to the "IKK γ" nucleic acid under low stringency conditions,
   (xiv) "KIAA0685 PROTEIN" (SEQ ID No:177) or a functionally active derivative thereof, or a functionally active fragment thereof, or a homolog thereof, or a variant of "KIAA0685 PROTEIN" encoded by a nucleic acid that hybridizes to the "KIAA0685 PROTEIN" nucleic acid under low stringency conditions,
   (xv) "SERINE/THREONINE PROTEIN PHOSPHATASE 6" (SEQ ID No:178) or a functionally active derivative thereof, or a functionally active fragment thereof, or a homolog thereof, or a variant of "SERINE/THREONINE PROTEIN PHOSPHATASE 6" encoded by a nucleic acid that hybridizes to the "SERINE/THREONINE PROTEIN PHOSPHATASE 6" nucleic acid under low stringency conditions,
   (xvi) "UVEAL AUTOANTIGEN" (SEQ ID No:179) or a functionally active derivative thereof, or a functionally active fragment thereof, or a homolog thereof, or a variant of "UVEAL AUTOANTIGEN" encoded by a nucleic acid that hybridizes to the "UVEAL AUTOANTIGEN" nucleic acid under low stringency conditions,
   (xvii) "ASB-3 PROTEIN" (SEQ ID No:149) or a functionally active derivative thereof, or a functionally active fragment thereof, or a homolog thereof, or a variant of "ASB-3 PROTEIN" encoded by a nucleic acid that hybridizes to the "ASB-3 PROTEIN" nucleic acid under low stringency conditions,
   (xviii) "SIMILAR TO PYRROLINE 5-CARBOXYLATE REDUCTASE ISOFORM" (SEQ ID No:180) or a functionally active derivative thereof, or a functionally active fragment thereof, or a homolog thereof, or a variant of "SIMILAR TO PYRROLINE 5-CARBOXYLATE REDUCTASE ISOFORM" encoded by a nucleic acid that hybridizes to the "SIMILAR TO PYRROLINE 5-CARBOXYLATE REDUCTASE ISOFORM" nucleic acid under low stringency conditions,
   (xix) "RAD50 HOMOLOG" (SEQ ID No:181) or a functionally active derivative thereof, or a functionally active fragment thereof, or a homolog thereof, or a variant of "RAD50 HOMOLOG" encoded by a nucleic acid that hybridizes to the "RAD50 HOMOLOG" nucleic acid under low stringency conditions,
   (xx) "SEROLOGICALLY DEFINED BREAST CANCER ANTIGEN NY-BR-16" (SEQ ID No: 182) or a functionally active derivative thereof, or a functionally active fragment thereof, or a homolog thereof, or a variant of "SEROLOGICALLY DEFINED BREAST CANCER ANTIGEN NY-BR-16" encoded by a nucleic acid that hybridizes to the "SEROLOGICALLY DEFINED BREAST CANCER ANTIGEN NY-BR-16" nucleic acid under low stringency conditions,
   (xxi) "KIAA1115 PROTEIN" (SEQ ID No:183) or a functionally active derivative thereof, or a functionally active fragment thereof, or a homolog thereof, or a variant of "KIAA1115 PROTEIN" encoded by a nucleic acid that hybridizes to the "KIAA1115 PROTEIN" nucleic acid under low stringency conditions,
   (xxii) "Hos1" (SEQ ID No:137) or a functionally active derivative thereof, or a functionally active fragment thereof, or a homolog thereof, or a variant of "Hos1" encoded by a nucleic acid that hybridizes to the "Hos1" nucleic acid under low stringency conditions,
   (xxiii) "SKP2 (SCF complex component)" (SEQ ID No:133) or a functionally active derivative thereof, or a functionally active fragment thereof, or a homolog thereof, or a variant of "SKP2 (SCF COMPLEX COMPONTENT" encoded by a nucleic acid that hybridizes to the "SKP2 (SCF COMPLEX COMPONENT" nucleic acid under low stringency conditions,
   (xxiv) "SKP1 (SCF COMPLEX COMPONENT)" (SEQ ID No:135) or a functionally active derivative thereof, or a functionally active fragment thereof, or a homolog thereof, or a variant of "SKP1 (SCF COMPLEX COMPONTENT" encoded by a nucleic acid that hybridizes to the "SKP1 (SCF COMPLEX COMPONENT" nucleic acid under low stringency conditions,
   (xxv) "IκB α" (SEQ ID No:78) or a functionally active derivative thereof, or a functionally active fragment thereof, or a homolog thereof, or a variant of "IκB α" encoded by a nucleic acid that hybridizes to the "IκB α" nucleic acid under low stringency conditions,
   (xxvi) "β-TrCP1" (SEQ ID No:129) or a functionally active derivative thereof, or a functionally active fragment thereof, or a homolog thereof, or a variant of "β TrCP1" encoded by a nucleic acid that hybridizes to the "β TrCP1" nucleic acid under low stringency conditions,
   (xxvii) "FLJ00246 (FRAGMENT)" (SEQ ID No:184) or a functionally active derivative thereof, or a functionally active fragment thereof, or a homolog thereof, or a variant of "FLJ00246 (FRAGMENT)" encoded by a nucleic acid that hybridizes to the "FLJ00246 (FRAGMENT)" nucleic acid under low stringency conditions,
   (xxviii) "KIAA0379 (FRAGMENT)" (SEQ ID No:185) or a functionally active derivative thereof,or a functionally active fragement thereof, or a homolog thereof, or a variant of "KIAA0379 (FRAGMENT)" that hybridizes to the "KIAA0379 (FRAGMENT)" nucleic acid under low stringency conditions, and
   (xxix) "COP9 SIGNALOSOME COMPLEX SUBUNIT 1" (SEQ ID No:186) or a functionally active derivative thereof, or a functionally active fragment thereof, or a homolog thereof, or a variant of "COP9 SIGNALOSOME COMPLEX SUBUNIT 1" encoded by a nucleic acid that hybridizes to the "COP9 SIGNALOSOME COMPLEX SUBUNIT 1" nucleic acid under low stringency conditions,
   as a target for an active agent of a pharmaceutical, preferably a drug target in the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.

The present invention further relates to the TRAF6 protein complex
1. A protein complex selected from complex (I) and comprising
   (a) at least one first protein selected from the group consisting of:
      (i) "RIP2 " (SEQ ID No:187) or a functionally active derivative thereof, or a functionally active fragment thereof, or a homolog thereof, or a variant of "RIP2 encoded by a nucleic acid that hybridizes to the "RIP2 " nucleic acid under low stringency conditions,
      (ii) "TAB1 TAK1-BINDING PROTEIN 1" (SEQ ID No:188) or a functionally active derivative thereof, or a functionally active fragment thereof, or a homolog thereof, or a variant of "TAB1 TAK1-BINDING PROTEIN 1" encoded by a nucleic acid that hybridizes to the "TAB1 TAK1-BINDING PROTEIN 1" nucleic acid under low stringency conditions,
      (iii) "p38" (SEQ ID No:118) or a functionally active derivative thereof, or a functionally active fragment thereof, or a homolog thereof, or a variant of "p38" encoded by a nucleic acid that hybridizes to the "p38" nucleic acid under low stringency conditions, and
      (iv) "TRAF6" (SEQ ID No:189) or a functionally active derivative thereof, or a functionally active fragment thereof, or a homolog thereof, or a variant of "TRAF6" encoded by a nucleic acid that hybridizes to the "TRAF6" nucleic acid under low stringency conditions,
      and
   (b) at least one second protein, which second protein is selected from the group consisting of:
      (i) "PROTEASOME SUBUNIT B TYPE 5 PRECURSOR" (SEQ ID No:190) or a functionally active derivative thereof, or a functionally active fragment thereof, or a homolog thereof, or a variant of "PROTEASOME SUBUNIT β TYPE 5 PRECURSOR" encoded by a nucleic acid that hybridizes to the "PROTEASOME SUBUNIT β TYPE 5 PRECURSOR" nucleic acid under low stringency conditions,
      (ii) "26S PROTEASOME NON-ATPASE REGULATORY SUBUNIT 8" (SEQ ID No:191) or a functionally active derivative thereof, or a functionally active fragment thereof, or a homolog thereof, or a variant of "26S PROTEASOME NON-ATPASE REGULATORY SUBUNIT 8" encoded by a nucleic acid that hybridizes to the "26S PROTEASOME NON-ATPASE REGULATORY SUBUNIT 8" nucleic acid under low stringency conditions,
      (iii) "26S PROTEASE REGULATORY SUBUNIT 6A" (SEQ ID No:192) or a functionally active derivative thereof, or a functionally active fragment thereof, or a homolog thereof, or a variant of "26S PROTEASE REGULATORY SUBUNIT 6A" encoded by a nucleic acid that hybridizes to the "26S PROTEASE REGULATORY SUBUNIT 6A" nucleic acid under low stringency conditions,
      (iv) "PROTEASOME SUBUNIT α TYPE 1" (SEQ ID No:193) or a functionally active derivative thereof, or a functionally active fragment thereof, or a homolog thereof, or a variant of "PROTEASOME SUBUNIT α TYPE 1" encoded by a nucleic acid that hybridizes to the "PROTEASOME SUBUNIT α TYPE 1" nucleic acid under low stringency conditions,
      (v) "PROTEASOME SUBUNIT α TYPE 4" (SEQ ID No:194) or a functionally active derivative thereof, or a functionally active fragment thereof, or a homolog thereof, or a variant of "PROTEASOME SUBUNIT α TYPE 4" encoded by a nucleic acid that hybridizes to the "PROTEASOME SUBUNIT α TYPE 4" nucleic acid under low stringency conditions,
      (vi) "PROTEASOME (PROSOME, MACROPAIN) 26S SUBUNIT, NON-ATPASE, 1" (SEQ ID No:195) or a functionally active derivative thereof, or a functionally active fragment thereof, or a homolog thereof, or a variant of "PROTEASOME (PROSOME, MACROPAIN) 26S SUBUNIT, NON-ATPASE, 1" encoded by a nucleic acid that hybridizes to the "PROTEASOME (PROSOME, MACROPAIN) 26S SUBUNIT, NON-ATPASE, 1" nucleic acid under low stringency conditions,
      (vii) "SIMILAR TO PROTEASE (PROSOME, MACROPAIN) 26S SUBUNIT, ATPASE 1" (SEQ ID No:196) or a functionally active derivative thereof, or a functionally active fragment thereof, or a homolog thereof, or a variant of "SIMILAR TO PROTEASE (PROSOME, MACROPAIN) 26S SUBUNIT, ATPASE 1" encoded by a nucleic acid that hybridizes to the "SIMILAR TO PROTEASE (PROSOME, MACROPAIN) 26S SUBUNIT, ATPASE 1" nucleic acid under low stringency conditions,
      (viii) "HYPOTHETICAL PROTEIN FLJ20552" (SEQ ID No:197) or a functionally active derivative thereof, or a functionally active fragment thereof, or a homolog thereof, or a variant of "HYPOTHETICAL PROTEIN FLJ20552" encoded by a nucleic acid that hybridizes to the "HYPOTHETICAL PROTEIN FLJ20552" nucleic acid under low stringency conditions,
      (ix) "26S PROTEASOME REGULATORY SUBUNIT S2" (SEQ ID No:198) or a functionally active derivative thereof, or a functionally active fragment thereof, or a homolog thereof, or a variant of "26S PROTEASOME REGULATORY SUBUNIT S2" encoded by a nucleic acid that hybridizes to the "26S PROTEASOME REGULATORY SUBUNIT S2" nucleic acid under low stringency conditions,
      (x) "26S PROTEASOME SUBUNIT P40.5" (SEQ ID No:199) or a functionally active derivative thereof, or a functionally active fragment thereof, or a homolog thereof, or a variant of "26S PROTEASOME SUBUNIT P40.5" encoded by a nucleic acid that hybridizes to the "26S PROTEASOME SUBUNIT P40.5" nucleic acid under low stringency conditions,
      (xi) "26S PROTEASE REGULATORY SUBUNIT S10B" (SEQ ID No:200) or a functionally active derivative thereof, or a functionally active fragment thereof, or a homolog thereof, or a variant of "26S PROTEASE REGULATORY SUBUNIT S10B" encoded by a nucleic acid that hybridizes to the "26S PROTEASE REGULATORY SUBUNIT S10B" nucleic acid under low stringency conditions,
      (xii) "26S PROTEASE REGULATORY SUBUNIT 7" (SEQ ID No:201) or a functionally active derivative thereof, or a functionally active fragment thereof, or a homolog thereof, or a variant of "26S PROTEASE REGULATORY SUBUNIT 7" encoded by a nucleic acid that hybridizes to the "26S PROTEASE REGULATORY SUBUNIT 7" nucleic acid under low stringency conditions,
      (xiii) "26S PROTEASOME SUBUNIT P55" (SEQ ID No:202) or a functionally active derivative thereof, or a functionally active fragment thereof, or a homolog thereof, or a variant of "26S PROTEASOME SUBUNIT P55" encoded by a nucleic acid that hybridizes to the "26S PROTEASOME SUBUNIT P55" nucleic acid under low stringency conditions,
      (xiv) "VOLTAGE-GATED POTASSIUM CHANNEL PROTEIN KQT-LIKE 1" (SEQ ID No:203) or a functionally active derivative thereof, or a functionally active fragment thereof, or a homolog thereof, or a variant of "VOLTAGE-GATED POTASSIUM CHANNEL PROTEIN KQT-LIKE 1" encoded by a nucleic acid that hybridizes to the "VOLTAGE-GATED POTASSIUM CHANNEL PROTEIN KQT-LIKE 1" nucleic acid under low stringency conditions,
      (xv) "26S PROTEASOME NON-ATPASE REGULATORY SUBUNIT 6" (SEQ ID No:204) or a functionally active derivative thereof, or a functionally active fragment thereof, or a homolog thereof, or a variant of "26S PROTEASOME NON-ATPASE REGULATORY SUBUNIT 6" encoded by a nucleic acid that hybridizes to the "26S PROTEASOME NON-ATPASE REGULATORY SUBUNIT 6" nucleic acid under low stringency conditions,
      (xvi) "26S PROTEASOME REGULATORY SUBUNIT S12" (SEQ ID No:205) or a functionally active derivative thereof, or a functionally active fragment thereof, or a homolog thereof, or a variant of "26S PROTEASOME REGULATORY SUBUNIT S12" encoded by a nucleic acid that hybridizes to the "26S PROTEASOME REGULATORY SUBUNIT S12" nucleic acid under low stringency conditions,
      (xvii) "26S PROTEASOME REGULATORY SUBUNIT S3" (SEQ ID No:206) or a functionally active derivative thereof, or a functionally active fragment thereof, or a homolog thereof, or a variant of "26S PROTEASOME REGULATORY SUBUNIT S3" encoded by a nucleic acid that hybridizes to the "26S PROTEASOME REGULATORY SUBUNIT S3" nucleic acid under low stringency conditions,
      (xviii) "26S PROTEASE REGULATORY SUBUNIT 4" (SEQ ID No:207) or a functionally active derivative thereof, or a functionally active fragment thereof, or a homolog thereof, or a variant of "26S PROTEASE REGULATORY SUBUNIT 4" encoded by a nucleic acid that hybridizes to the "26S PROTEASE REGULATORY SUBUNIT 4" nucleic acid under low stringency conditions,
      (xix) "PROTEASOME SUBUNIT β TYPE 2" (SEQ ID No:208) or a functionally active derivative thereof, or a functionally active fragment thereof, or a homolog thereof, or a variant of "PROTEASOME SUBUNIT β TYPE 2" encoded by a nucleic acid that hybridizes to the "PROTEASOME SUBUNIT β TYPE 2" nucleic acid under low stringency conditions,
      (xx) "26S PROTEASOME NON-ATPASE REGULATORY SUBUNIT 11" (SEQ ID No:209) or a functionally active derivative thereof, or a functionally active fragment thereof, or a homolog thereof, or a variant of "26S PROTEASOME NON-ATPASE REGULATORY SUBUNIT 11" encoded by a nucleic acid that hybridizes to the "26S PROTEASOME NON-ATPASE REGULATORY SUBUNIT 11" nucleic acid under low stringency conditions,
      (xxi) "OXYSTEROL BINDING PROTEIN-RELATED PROTEIN 8" (SEQ ID No:55) or a functionally active derivative thereof, or a functionally active fragment thereof, or a homolog thereof, or a variant of "OXYSTEROL BINDING PROTEIN-RELATED PROTEIN 8" encoded by a nucleic acid that hybridizes to the "OXYSTEROL BINDING PROTEIN-RELATED PROTEIN 8" nucleic acid under low stringency conditions,
      (xxii) "26S PROTEASOME REGULATORY SUBUNIT S5A" (SEQ ID No:210) or a functionally active derivative thereof, or a functionally active fragment thereof, or a homolog thereof, or a variant of "26S PROTEASOME REGULATORY SUBUNIT S5A" encoded by a nucleic acid that hybridizes to the "26S PROTEASOME REGULATORY SUBUNIT S5A" nucleic acid under low stringency conditions,
      (xxiii) "PROTEASOME SUBUNIT β TYPE 6 PRECURSOR" (SEQ ID No:211) or a functionally active derivative thereof, or a functionally active fragment thereof, or a homolog thereof, or a variant of "PROTEASOME SUBUNIT β TYPE 6 PRECURSOR" encoded by a nucleic acid that hybridizes to the "PROTEASOME SUBUNIT β TYPE 6 PRECURSOR" nucleic acid under low stringency conditions,
      (xxiv) "26S PROTEASOME SUBUNIT 9" (SEQ ID No:212) or a functionally active derivative thereof, or a functionally active fragment thereof, or a homolog thereof, or a variant of "26S PROTEASOME SUBUNIT 9" encoded by a nucleic acid that hybridizes to the "26S PROTEASOME SUBUNIT 9" nucleic acid under low stringency conditions,
      (xxv) "HSPC121" (SEQ ID No:214) or a functionally active derivative thereof, or a functionally active fragment thereof, or a homolog thereof, or a variant of "HSPC121" encoded by a nucleic acid that hybridizes to the "HSPC121" nucleic acid under low stringency conditions,
      (xxvi) "26S PROTEASOME NON-ATPASE REGULATORY SUBUNIT 1" (SEQ ID No:215) or a functionally active derivative thereof, or a functionally active fragment thereof, or a homolog thereof, or a variant of "26S PROTEASOME NON-ATPASE REGULATORY SUBUNIT 1" encoded by a nucleic acid that hybridizes to the "26S PROTEASOME NON-ATPASE REGULATORY SUBUNIT 1" nucleic acid under low stringency conditions,
      (xxvii) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,
      (xxviii) "PROTEASOME SUBUNIT α TYPE 7" (SEQ ID No:216) or a functionally active derivative thereof, or a functionally active fragment thereof, or a homolog thereof, or a variant of "PROTEASOME SUBUNIT α TYPE 7" encoded by a nucleic acid that hybridizes to the "PROTEASOME SUBUNIT α TYPE 7" nucleic acid under low stringency conditions,
      (xxix) "26S PROTEASE REGULATORY SUBUNIT 8" (SEQ ID No:217) or a functionally active derivative thereof, or a functionally active fragment thereof, or a homolog thereof, or a variant of "26S PROTEASE REGULATORY SUBUNIT 8" encoded by a nucleic acid that hybridizes to the "26S PROTEASE REGULATORY SUBUNIT 8" nucleic acid under low stringency conditions,
      (xxx) "PROTEASOME SUBUNIT α TYPE 6" (SEQ ID No:218) or a functionally active derivative thereof, or a functionally active fragment thereof, or a homolog thereof, or a variant of "PROTEASOME SUBUNIT α TYPE 6" encoded by a nucleic acid that hybridizes to the "PROTEASOME SUBUNIT α TYPE 6" nucleic acid under low stringency conditions, and
      (xxxi) "26S PROTEASE NON-ATPASE REGULATORY SUBUNIT 7" (SEQ ID No:213) or a functionally active derivative thereof, or a functionally active fragment thereof, or a homolog thereof, or a variant of "26S PROTEASE NON-ATPASE REGULATORY SUBUNIT 7" encoded by a nucleic acid that hybridises to the "26S PROTEASE NON-ATPASE REGULATORY SUBUNIT 7" nucleic acid under low stringency conditions,
   and a complex (II) comprising at least two of said second proteins,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wtlvol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
2. The protein complex according to claim 1 wherein the first protein is the protein "TRAF6" (SEQ ID No:189), or a functionally active derivative thereof, or a functionally active fragment thereof, or a homolog thereof, or a variant of "TRAF6" encoded by a nucleic acid that hybridizes to the "TRAF6" nucleic acid under low stringency conditions.
3. The protein complex according to No. 1 selected from complex (I) and comprising the following proteins:
   (i) "TRAF6" (SEQ ID No:189) or a functionally active derivative thereof, or a functionally active fragment thereof, or a homolog thereof, or a variant of "TRAF6" encoded by a nucleic acid that hybridizes to the "TRAF6" nucleic acid under low stringency conditions,
   (ii) "PROTEASOME SUBUNIT β TYPE 5 PRECURSOR" (SEQ ID No:190) or a functionally active derivative thereof, or a functionally active fragment thereof, or a homolog thereof, or a variant of "PROTEASOME SUBUNIT β TYPE 5 PRECURSOR" encoded by a nucleic acid that hybridizes to the "PROTEASOME SUBUNIT β TYPE 5 PRECURSOR" nucleic acid under low stringency conditions,
   (iii) "26S PROTEASOME NON-ATPASE REGULATORY SUBUNIT 8" (SEQ ID No: 191) or a functionally active derivative thereof, or a functionally active fragment thereof, or a homolog thereof, or a variant of "26S PROTEASOME NON-ATPASE REGULATORY SUBUNIT 8" encoded by a nucleic acid that hybridizes to the "26S PROTEASOME NON-ATPASE REGULATORY SUBUNIT 8" nucleic acid under low stringency conditions,
   (iv) "26S PROTEASOME REGULATORY SUBUNIT S2" (SEQ ID No:198) or a functionally active derivative thereof, or a functionally active fragment thereof, or a homolog thereof, or a variant of "26S PROTEASOME REGULATORY SUBUNIT S2" encoded by a nucleic acid that hybridizes to the "26S PROTEASOME REGULATORY SUBUNIT S2" nucleic acid under low stringency conditions,
   (v) "26S PROTEASOME SUBUNIT P40.5" (SEQ ID No:199) or a functionally active derivative thereof, or a functionally active fragment thereof, or a homolog thereof, or a variant of "26S PROTEASOME SUBUNIT P40.5" encoded by a nucleic acid that hybridizes to the "26S PROTEASOME SUBUNIT P40.5" nucleic acid under low stringency conditions,
   (vi) "26S PROTEASE REGULATORY SUBUNIT S10B" (SEQ ID No:200) or a functionally active derivative thereof, or a functionally active fragment thereof, or a homolog thereof, or a variant of "26S PROTEASE REGULATORY SUBUNIT S10B" encoded by a nucleic acid that hybridizes to the "26S PROTEASE REGULATORY SUBUNIT S10B" nucleic acid under low stringency conditions,
   (vii) "26S PROTEASE REGULATORY SUBUNIT 7" (SEQ ID No:201) or a functionally active derivative thereof, or a functionally active fragment thereof, or a homolog thereof, or a variant of "26S PROTEASE REGULATORY SUBUNIT 7" encoded by a nucleic acid that hybridizes to the "26S PROTEASE REGULATORY SUBUNIT 7" nucleic acid under low stringency conditions,
   (viii) "26S PROTEASOME SUBUNIT P55" (SEQ ID No:202) or a functionally active derivative thereof, or a functionally active fragment thereof, or a homolog thereof, or a variant of "26S PROTEASOME SUBUNIT P55" encoded by a nucleic acid that hybridizes to the "26S PROTEASOME SUBUNIT P55" nucleic acid under low stringency conditions,
   (ix) "VOLTAGE-GATED POTASSIUM CHANNEL PROTEIN KQT-LIKE 1" (SEQ ID No:203) or a functionally active derivative thereof, or a functionally active fragment thereof, or a homolog thereof, or a variant of "VOLTAGE-GATED POTASSIUM CHANNEL PROTEIN KQT-LIKE 1" encoded by a nucleic acid that hybridizes to the "VOLTAGE-GATED POTASSIUM CHANNEL PROTEIN KQT-LIKE 1" nucleic acid under low stringency conditions,
   (x) "26S PROTEASOME NON-ATPASE REGULATORY SUBUNIT 6" (SEQ ID No:204) or a functionally active derivative thereof, or a functionally active fragment thereof, or a homolog thereof, or a variant of "26S PROTEASOME NON-ATPASE REGULATORY SUBUNIT 6" encoded by a nucleic acid that hybridizes to the "26S PROTEASOME NON-ATPASE REGULATORY SUBUNIT 6" nucleic acid under low stringency conditions,
   (xi) "26S PROTEASOME REGULATORY SUBUNIT S12" (SEQ ID No:205) or a functionally active derivative thereof, or a functionally active fragment thereof, or a homolog thereof, or a variant of "26S PROTEASOME REGULATORY SUBUNIT S12" encoded by a nucleic acid that hybridizes to the "26S PROTEASOME REGULATORY SUBUNIT S12" nucleic acid under low stringency conditions,
   (xii) "26S PROTEASOME REGULATORY SUBUNIT S3" (SEQ ID No:206) or a functionally active derivative thereof, or a functionally active fragment thereof, or a homolog thereof, or a variant of "26S PROTEASOME REGULATORY SUBUNIT S3" encoded by a nucleic acid that hybridizes to the "26S PROTEASOME REGULATORY SUBUNIT S3" nucleic acid under low stringency conditions,
   (xiii) "26S PROTEASE REGULATORY SUBUNIT 4" (SEQ ID No:207) or a functionally active derivative thereof, or a functionally active fragment thereof, or a homolog thereof, or a variant of "26S PROTEASE REGULATORY SUBUNIT 4" encoded by a nucleic acid that hybridizes to the "26S PROTEASE REGULATORY SUBUNIT 4" nucleic acid under low stringency conditions,

   (i) "PROTEASOME SUBUNIT β TYPE 2" (SEQ ID No:208) or a functionally active derivative thereof, or a functionally active fragment thereof, or a homolog thereof, or a variant of "PROTEASOME SUBUNIT β TYPE 2" encoded by a nucleic acid that hybridizes to the "PROTEASOME SUBUNIT β TYPE 2" nucleic acid under low stringency conditions,
   (xv) "26S PROTEASOME REGULATORY SUBUNIT S5A" (SEQ ID No:210) or a functionally active derivative thereof, or a functionally active fragment thereof, or a homolog thereof, or a variant of "26S PROTEASOME REGULATORY SUBUNIT S5A" encoded by a nucleic acid that hybridizes to the "26S PROTEASOME REGULATORY SUBUNIT S5A" nucleic acid under low stringency conditions,

   (i) "PROTEASOME SUBUNIT β TYPE 6 PRECURSOR" (SEQ ID No:211) or a functionally active derivative thereof,or a functionally active fragement thereof, or a homolog thereof, or a variant of "PROTEASOME SUBUNIT β TYPE 6 PRECURSOR" that hybridizes to the "PROTEASOME SUBUNIT β TYPE 6 PRECURSOR" nucleic acid under low stringency conditions,
   (xvii) "26S PROTEASOME SUBUNIT 9" (SEQ ID No:212) or a functionally active derivative thereof, or a functionally active fragment thereof, or a homolog thereof, or a variant of "26S PROTEASOME SUBUNIT 9" encoded by a nucleic acid that hybridizes to the "26S PROTEASOME SUBUNIT 9" nucleic acid under low stringency conditions, and
   (xviii) "26S PROTEASE REGULATORY SUBUNIT 8" (SEQ ID No:217) or a functionally active derivative thereof, or a functionally active fragment thereof, or a homolog thereof, or a variant of "26S PROTEASE REGULATORY SUBUNIT 8" encoded by a nucleic acid that hybridizes to the "26S PROTEASE REGULATORY SUBUNIT 8" nucleic acid under low stringency conditions;
   and a protein complex selected from complex (II) and comprising the following proteins:
   (i) "TRAF6" (SEQ ID No:189) or a functionally active derivative thereof, or a functionally active fragment thereof, or a homolog thereof, or a variant of "TRAF6" encoded by a nucleic acid that hybridizes to the "TRAF6" nucleic acid under low stringency conditions,
   (ii) "PROTEASOME SUBUNIT β TYPE 5 PRECURSOR" (SEQ ID No:190) or a functionally active derivative thereof, or a functionally active fragment thereof, or a homolog thereof, or a variant of "PROTEASOME SUBUNIT β TYPE 5 PRECURSOR" encoded by a nucleic acid that hybridizes to the "PROTEASOME SUBUNIT β TYPE 5 PRECURSOR" nucleic acid under low stringency conditions,
   (iii) "26S PROTEASE REGULATORY SUBUNIT 6A" (SEQ ID No:192) or a functionally active derivative thereof, or a functionally active fragment thereof, or a homolog thereof, or a variant of "26S PROTEASE REGULATORY SUBUNIT 6A" encoded by a nucleic acid that hybridizes to the "26S PROTEASE REGULATORY SUBUNIT 6A" nucleic acid under low stringency conditions,
   (iv) "SIMILAR TO PROTEASE (PROSOME, MACROPAIN) 26S SUBUNIT, ATPASE 1" (SEQ ID No:196) or a functionally active derivative thereof, or a functionally active fragment thereof, or a homolog thereof, or a variant of "SIMILAR TO PROTEASE (PROSOME, MACROPAIN) 26S SUBUNIT, ATPASE 1" encoded by a nucleic acid that hybridizes to the "SIMILAR TO PROTEASE (PROSOME, MACROPAIN) 26S SUBUNIT, ATPASE 1" nucleic acid under low stringency conditions,
   (v) "26S PROTEASOME REGULATORY SUBUNIT S2" (SEQ ID No:198) or a functionally active derivative thereof, or a functionally active fragment thereof, or a homolog thereof, or a variant of "26S PROTEASOME REGULATORY SUBUNIT S2" encoded by a nucleic acid that hybridizes to the "26S PROTEASOME REGULATORY SUBUNIT S2" nucleic acid under low stringency conditions,
   (vi) "26S PROTEASOME SUBUNIT P40.5" (SEQ ID No:199) or a functionally active derivative thereof, or a functionally active fragment thereof, or a homolog thereof, or a variant of "26S PROTEASOME SUBUNIT P40.5" encoded by a nucleic acid that hybridizes to the "26S PROTEASOME SUBUNIT P40.5" nucleic acid under low stringency conditions,
   (vii) "26S PROTEASE REGULATORY SUBUNIT S10B" (SEQ ID No:200) or a functionally active derivative thereof, or a functionally active fragment thereof, or a homolog thereof, or a variant of "26S PROTEASE REGULATORY SUBUNIT S10B" encoded by a nucleic acid that hybridizes to the "26S PROTEASE REGULATORY SUBUNIT S10B" nucleic acid under low stringency conditions,
   (viii) "26S PROTEASE REGULATORY SUBUNIT 7" (SEQ ID No:201) or a functionally active derivative thereof, or a functionally active fragment thereof, or a homolog thereof, or a variant of "26S PROTEASE REGULATORY SUBUNIT 7" encoded by a nucleic acid that hybridizes to the "26S PROTEASE REGULATORY SUBUNIT 7" nucleic acid under low stringency conditions,
   (ix) "26S PROTEASOME SUBUNIT P55" (SEQ ID No:202) or a functionally active derivative thereof, or a functionally active fragment thereof, or a homolog thereof, or a variant of "26S PROTEASOME SUBUNIT P55" encoded by a nucleic acid that hybridizes to the "26S PROTEASOME SUBUNIT P55" nucleic acid under low stringency conditions,
   (x) "VOLTAGE-GATED POTASSIUM CHANNEL PROTEIN KQT-LIKE 1" (SEQ ID No:203) or a functionally active derivative thereof, or a functionally active fragment thereof, or a homolog thereof, or a variant of "VOLTAGE-GATED POTASSIUM CHANNEL PROTEIN KQT-LIKE 1" encoded by a nucleic acid that hybridizes to the "VOLTAGE-GATED POTASSIUM CHANNEL PROTEIN KQT-LIKE 1" nucleic acid under low stringency conditions,
   (xi) "26S PROTEASOME NON-ATPASE REGULATORY SUBUNIT 6" (SEQ ID No:204) or a functionally active derivative thereof, or a functionally active fragment thereof, or a homolog thereof, or a variant of "26S PROTEASOME NON-ATPASE REGULATORY SUBUNIT 6" encoded by a nucleic acid that hybridizes to the "26S PROTEASOME NON-ATPASE REGULATORY SUBUNIT 6" nucleic acid under low stringency conditions,
   (xii) "26S PROTEASOME REGULATORY SUBUNIT S12" (SEQ ID No:205) or a functionally active derivative thereof, or a functionally active fragment thereof, or a homolog thereof, or a variant of "26S PROTEASOME REGULATORY SUBUNIT S12" encoded by a nucleic acid that hybridizes to the "26S PROTEASOME REGULATORY SUBUNIT S12" nucleic acid under low stringency conditions,
   (xiii) "26S PROTEASOME REGULATORY SUBUNIT S3" (SEQ ID No:206) or a functionally active derivative thereof, or a functionally active fragment thereof, or a homolog thereof, or a variant of "26S PROTEASOME REGULATORY SUBUNIT S3" encoded by a nucleic acid that hybridizes to the "26S PROTEASOME REGULATORY SUBUNIT S3" nucleic acid under low stringency conditions,
   (xiv) "26S PROTEASE REGULATORY SUBUNIT 4" (SEQ ID No:207) or a functionally active derivative thereof, or a functionally active fragment thereof, or a homolog thereof, or a variant of "26S PROTEASE REGULATORY SUBUNIT 4" encoded by a nucleic acid that hybridizes to the "26S PROTEASE REGULATORY SUBUNIT 4" nucleic acid under low stringency conditions,
   (xv) "PROTEASOME SUBUNIT β TYPE 2" (SEQ ID No:208) or a functionally active derivative thereof, or a functionally active fragment thereof, or a homolog thereof, or a variant of "PROTEASOME SUBUNIT β TYPE 2" encoded by a nucleic acid that hybridizes to the "PROTEASOME SUBUNIT β TYPE 2" nucleic acid under low stringency conditions,
   (xvi) "26S PROTEASOME NON-ATPASE REGULATORY SUBUNIT 11" (SEQ ID No:209) or a functionally active derivative thereof, or a functionally active fragment thereof, or a homolog thereof, or a variant of "26S PROTEASOME NON-ATPASE REGULATORY SUBUNIT 11" encoded by a nucleic acid that hybridizes to the "26S PROTEASOME NON-ATPASE REGULATORY SUBUNIT 11" nucleic acid under low stringency conditions,
   (xvii) "OXYSTEROL BINDING PROTEIN-RELATED PROTEIN 8" (SEQ ID No:55) or a functionally active derivative thereof, or a functionally active fragment thereof, or a homolog thereof, or a variant of "OXYSTEROL BINDING PROTEIN-RELATED PROTEIN 8" encoded by a nucleic acid that hybridizes to the "OXYSTEROL BINDING PROTEIN-RELATED PROTEIN 8" nucleic acid under low stringency conditions,
   (xviii) "26S PROTEASOME REGULATORY SUBUNIT S5A" (SEQ ID No:210) or a functionally active derivative thereof, or a functionally active fragment thereof, or a homolog thereof, or a variant of "26S PROTEASOME REGULATORY SUBUNIT S5A" encoded by a nucleic acid that hybridizes to the "26S PROTEASOME REGULATORY SUBUNIT S5A" nucleic acid under low stringency conditions,
   (xix) "PROTEASOME SUBUNIT β TYPE 6 PRECURSOR" (SEQ ID No:211) or a functionally active derivative thereof, or a functionally active fragment thereof, or a homolog thereof, or a variant of "PROTEASOME SUBUNIT β TYPE 6 PRECURSOR" encoded by a nucleic acid that hybridizes to the "PROTEASOME SUBUNIT β TYPE 6 PRECURSOR" nucleic acid under low stringency conditions,
   (xx) "26S PROTEASOME SUBUNIT 9" (SEQ ID No:212) or a functionally active derivative thereof, or a functionally active fragment thereof, or a homolog thereof, or a variant of "26S PROTEASOME SUBUNIT 9" encoded by a nucleic acid that hybridizes to the "26S PROTEASOME SUBUNIT 9" nucleic acid under low stringency conditions,
   (xxi) "26S PROTEASOME NON-ATPASE REGULATORY SUBUNIT 7" (SEQ ID No:213) or a functionally active derivative thereof, or a functionally active fragment thereof, or a homolog thereof, or a variant of "26S PROTEASOME NON-ATPASE REGULATORY SUBUNIT 7" encoded by a nucleic acid that hybridizes to the "26S PROTEASOME NON-ATPASE REGULATORY SUBUNIT 7" nucleic acid under low stringency conditions,
   (xxii) "26S PROTEASOME NON-ATPASE REGULATORY SUBUNIT 1" (SEQ ID No:215) or a functionally active derivative thereof, or a functionally active fragment thereof, or a homolog thereof, or a variant of "26S PROTEASOME NON-ATPASE REGULATORY SUBUNIT 1" encoded by a nucleic acid that hybridizes to the "26S PROTEASOME NON-ATPASE REGULATORY SUBUNIT 1" nucleic acid under low stringency conditions,
   (xxiii) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,

   (i) "PROTEASOME SUBUNIT α TYPE 7" (SEQ ID No:216) or a functionally active derivative thereof, or a functionally active fragment thereof, or a homolog thereof, or a variant of "PROTEASOME SUBUNIT α TYPE 7" encoded by a nucleic acid that hybridizes to the "PROTEASOME SUBUNIT α TYPE 7" nucleic acid under low stringency conditions,
   (xxv) "26S PROTEASE REGULATORY SUBUNIT 8" (SEQ ID No:217) or a functionally active derivative thereof,or a functionally active fragement thereof, or a homolog thereof, or a variant of "26S PROTEASE REGULATORY SUBUNIT 8" that hybridizes to the "26S PROTEASE REGULATORY SUBUNIT 8" nucleic acid under low stringency conditions, and

   (i) "PROTEASOME SUBUNIT α TYPE 6" (SEQ ID No:218) or a functionally active derivative thereof,or a functionally active fragement thereof, or a homolog thereof, or a variant of "PROTEASOME SUBUNIT α TYPE 6" that hybridizes to the "PROTEASOME SUBUNIT α TYPE 6" nucleic acid under low stringency conditions;
   and a protein complex selected from complex (III) and comprising the following proteins:
   (i) "TRAF6" (SEQ ID No:189) or a functionally active derivative thereof, or a functionally active fragment thereof, or a homolog thereof, or a variant of "TRAF6" encoded by a nucleic acid that hybridizes to the "TRAF6" nucleic acid under low stringency conditions,

   (i) "PROTEASOME SUBUNIT β TYPE 5 PRECURSOR" (SEQ ID No:190) or a functionally active derivative thereof, or a functionally active fragment thereof, or a homolog thereof, or a variant of "PROTEASOME SUBUNIT β TYPE 5 PRECURSOR" encoded by a nucleic acid that hybridizes to the "PROTEASOME SUBUNIT β TYPE 5 PRECURSOR" nucleic acid under low stringency conditions,
   (iii) "26S PROTEASOME NON-ATPASE REGULATORY SUBUNIT 8" (SEQ ID No: 191) or a functionally active derivative thereof, or a functionally active fragment thereof, or a homolog thereof, or a variant of "26S PROTEASOME NON-ATPASE REGULATORY SUBUNIT 8" encoded by a nucleic acid that hybridizes to the "26S PROTEASOME NON-ATPASE REGULATORY SUBUNIT 8" nucleic acid under low stringency conditions,
   (iv) "PROTEASOME SUBUNIT α TYPE 1" (SEQ ID No:193) or a functionally active derivative thereof, or a functionally active fragment thereof, or a homolog thereof, or a variant of "PROTEASOME SUBUNIT α TYPE 1" encoded by a nucleic acid that hybridizes to the "PROTEASOME SUBUNIT α TYPE 1" nucleic acid under low stringency conditions,
   (v) "PROTEASOME SUBUNIT α TYPE 4" (SEQ ID No:194) or a functionally active derivative thereof, or a functionally active fragment thereof, or a homolog thereof, or a variant of "PROTEASOME SUBUNIT α TYPE 4" encoded by a nucleic acid that hybridizes to the "PROTEASOME SUBUNIT α TYPE 4" nucleic acid under low stringency conditions,
   (vi) " PROTEASOME (PROSOME, MACROPAIN) 26S SUBUNIT, NON-ATPASE, 1" (SEQ ID No:195) or a functionally active derivative thereof, or a functionally active fragment thereof, or a homolog thereof, or a variant of "PROTEASOME (PROSOME, MACROPAIN) 26S SUBUNIT, NON-ATPASE, 1" encoded by a nucleic acid that hybridizes to the "PROTEASOME (PROSOME, MACROPAIN) 26S SUBUNIT, NON-ATPASE, 1" nucleic acid under low stringency conditions,
   (vii) "HYPOTHETICAL PROTEIN FLJ20552" (SEQ ID No:197) or a functionally active derivative thereof, or a functionally active fragment thereof, or a homolog thereof, or a variant of "HYPOTHETICAL PROTEIN FLJ20552" encoded by a nucleic acid that hybridizes to the "HYPOTHETICAL PROTEIN FLJ20552" nucleic acid under low stringency conditions,
   (viii) "26S PROTEASOME REGULATORY SUBUNIT S2" (SEQ ID No:198) or a functionally active derivative thereof, or a functionally active fragment thereof, or a homolog thereof, or a variant of "26S PROTEASOME REGULATORY SUBUNIT S2" encoded by a nucleic acid that hybridizes to the "26S PROTEASOME REGULATORY SUBUNIT S2" nucleic acid under low stringency conditions,
   (ix) "26S PROTEASOME SUBUNIT P40.5" (SEQ ID No:199) or a functionally active derivative thereof, or a functionally active fragment thereof, or a homolog thereof, or a variant of "26S PROTEASOME SUBUNIT P40.5" encoded by a nucleic acid that hybridizes to the "26S PROTEASOME SUBUNIT P40.5" nucleic acid under low stringency conditions,
   (x) "26S PROTEASE REGULATORY SUBUNIT S10B" (SEQ ID No:200) or a functionally active derivative thereof, or a functionally active fragment thereof, or a homolog thereof, or a variant of "26S PROTEASE REGULATORY SUBUNIT S10B" encoded by a nucleic acid that hybridizes to the "26S PROTEASE REGULATORY SUBUNIT S10B" nucleic acid under low stringency conditions,
   (xi) "26S PROTEASE REGULATORY SUBUNIT 7" (SEQ ID No:201) or a functionally active derivative thereof, or a functionally active fragment thereof, or a homolog thereof, or a variant of "26S PROTEASE REGULATORY SUBUNIT 7" encoded by a nucleic acid that hybridizes to the "26S PROTEASE REGULATORY SUBUNIT 7" nucleic acid under low stringency conditions,
   (xii) "26S PROTEASOME SUBUNIT P55" (SEQ ID No:202) or a functionally active derivative thereof, or a functionally active fragment thereof, or a homolog thereof, or a variant of "26S PROTEASOME SUBUNIT P55" encoded by a nucleic acid that hybridizes to the "26S PROTEASOME SUBUNIT P55" nucleic acid under low stringency conditions,
   (xiii) "VOLTAGE-GATED POTASSIUM CHANNEL PROTEIN KQT-LIKE 1" (SEQ ID No:203) or a functionally active derivative thereof, or a functionally active fragment thereof, or a homolog thereof, or a variant of "VOLTAGE-GATED POTASSIUM CHANNEL PROTEIN KQT-LIKE 1" encoded by a nucleic acid that hybridizes to the "VOLTAGE-GATED POTASSIUM CHANNEL PROTEIN KQT-LIKE 1" nucleic acid under low stringency conditions,
   (xiv) "26S PROTEASOME NON-ATPASE REGULATORY SUBUNIT 6" (SEQ ID No:204) or a functionally active derivative thereof, or a functionally active fragment thereof, or a homolog thereof, or a variant of "26S PROTEASOME NON-ATPASE REGULATORY SUBUNIT 6" encoded by a nucleic acid that hybridizes to the "26S PROTEASOME NON-ATPASE REGULATORY SUBUNIT 6" nucleic acid under low stringency conditions,
   (xv) "26S PROTEASOME REGULATORY SUBUNIT S12" (SEQ ID No:205) or a functionally active derivative thereof, or a functionally active fragment thereof, or a homolog thereof, or a variant of "26S PROTEASOME REGULATORY SUBUNIT S12" encoded by a nucleic acid that hybridizes to the "26S PROTEASOME REGULATORY SUBUNIT S12" nucleic acid under low stringency conditions,
   (xvi) "26S PROTEASOME REGULATORY SUBUNIT S3" (SEQ ID No:206) or a functionally active derivative thereof, or a functionally active fragment thereof, or a homolog thereof, or a variant of "26S PROTEASOME REGULATORY SUBUNIT S3" encoded by a nucleic acid that hybridizes to the "26S PROTEASOME REGULATORY SUBUNIT S3" nucleic acid under low stringency conditions,
   (xvii) "26S PROTEASE REGULATORY SUBUNIT 4" (SEQ ID No:207) or a functionally active derivative thereof, or a functionally active fragment thereof, or a homolog thereof, or a variant of "26S PROTEASE REGULATORY SUBUNIT 4" encoded by a nucleic acid that hybridizes to the "26S PROTEASE REGULATORY SUBUNIT 4" nucleic acid under low stringency conditions,
   (xviii) "PROTEASOME SUBUNIT β TYPE 2" (SEQ ID No:208) or a functionally active derivative thereof, or a functionally active fragment thereof, or a homolog thereof, or a variant of "PROTEASOME SUBUNIT β TYPE 2" encoded by a nucleic acid that hybridizes to the "PROTEASOME SUBUNIT β TYPE 2" nucleic acid under low stringency conditions,
   (xix) "26S PROTEASOME REGULATORY SUBUNIT S5A" (SEQ ID No:210) or a functionally active derivative thereof, or a functionally active fragment thereof, or a homolog thereof, or a variant of "26S PROTEASOME REGULATORY SUBUNIT S5A" encoded by a nucleic acid that hybridizes to the "26S PROTEASOME REGULATORY SUBUNIT S5A" nucleic acid under low stringency conditions,
   (xx) "PROTEASOME SUBUNIT β TYPE 6 PRECURSOR" (SEQ ID No:211) or a functionally active derivative thereof, or a functionally active fragment thereof, or a homolog thereof, or a variant of "PROTEASOME SUBUNIT β TYPE 6 PRECURSOR" encoded by a nucleic acid that hybridizes to the "PROTEASOME SUBUNIT β TYPE 6 PRECURSOR" nucleic acid under low stringency conditions,
   (xxi) "26S PROTEASOME SUBUNIT 9" (SEQ ID No:212) or a functionally active derivative thereof, or a functionally active fragment thereof, or a homolog thereof, or a variant of "26S PROTEASOME SUBUNIT 9" encoded by a nucleic acid that hybridizes to the "26S PROTEASOME SUBUNIT 9" nucleic acid under low stringency conditions,
   (xxii) "26S PROTEASOME NON-ATPASE REGULATORY SUBUNIT 7" (SEQ ID No:213) or a functionally active derivative thereof, or a functionally active fragment thereof, or a homolog thereof, or a variant of "26S PROTEASOME NON-ATPASE REGULATORY SUBUNIT 7" encoded by a nucleic acid that hybridizes to the "26S PROTEASOME NON-ATPASE REGULATORY SUBUNIT 7" nucleic acid under low stringency conditions,
   (xxiii) "HSPC121" (SEQ ID No:214) or a functionally active derivative thereof,or a functionally active fragement thereof, or a homolog thereof, or a variant of "HSPC121" that hybridizes to the "HSPC121" nucleic acid under low stringency conditions, and
   (xxiv) "26S PROTEASE REGULATORY SUBUNIT 8" (SEQ ID No:217) or a functionally active derivative thereof, or a functionally active fragment thereof, or a homolog thereof, or a variant of "26S PROTEASE REGULATORY SUBUNIT 8" encoded by a nucleic acid that hybridizes to the "26S PROTEASE REGULATORY SUBUNIT 8" nucleic acid under low stringency conditions;
   and a protein complex selected from complex (IV) and comprising the following proteins:
   (i) "RIP2 " (SEQ ID No:187) or a functionally active derivative thereof, or a functionally active fragment thereof, or a homolog thereof, or a variant of "RIP2" encoded by a nucleic acid that hybridizes to the "RIP2" nucleic acid under low stringency conditions,
   (ii) "TAB1 TAK1-BINDING PROTEIN 1" (SEQ ID No:188) or a functionally active derivative thereof, or a functionally active fragment thereof, or a homolog thereof, or a variant of "TAB1 TAK1-BINDING PROTIEN 1" encoded by a nucleic acid that hybridizes to the "TAB1 TAK1-BINDING PROTEIN 1" nucleic acid under low stringency conditions,
   (iii) "p38" (SEQ ID No:118) or a functionally active derivative thereof, or a functionally active fragment thereof, or a homolog thereof, or a variant of "p38" encoded by a nucleic acid that hybridizes to the "p38" nucleic acid under low stringency conditions,
   (iv) "TRAF6" (SEQ ID No:189) or a functionally active derivative thereof, or a functionally active fragment thereof, or a homolog thereof, or a variant of "TRAF6" encoded by a nucleic acid that hybridizes to the "TRAF6" nucleic acid under low stringency conditions,
   (v) "PROTEASOME SUBUNIT β TYPE 5 PRECURSOR" (SEQ ID No:190) or a functionally active derivative thereof, or a functionally active fragment thereof, or a homolog thereof, or a variant of "PROTEASOME SUBUNIT β TYPE 5 PRECURSOR" encoded by a nucleic acid that hybridizes to the "PROTEASOME SUBUNIT β TYPE 5 PRECURSOR" nucleic acid under low stringency conditions,
   (vi) "26S PROTEASOME NON-ATPASE REGULATORY SUBUNIT 8" (SEQ ID No:191) or a functionally active derivative thereof, or a functionally active fragment thereof, or a homolog thereof, or a variant of "26S PROTEASOME NON-ATPASE REGULATORY SUBUNIT 8" encoded by a nucleic acid that hybridizes to the "26S PROTEASOME NON-ATPASE REGULATORY SUBUNIT 8" nucleic acid under low stringency conditions,
   (vii) "26S PROTEASE REGULATORY SUBUNIT 6A" (SEQ ID No:192) or a functionally active derivative thereof, or a functionally active fragment thereof, or a homolog thereof, or a variant of "26S PROTEASE REGULATORY SUBUNIT 6A" encoded by a nucleic acid that hybridizes to the "26S PROTEASE REGULATORY SUBUNIT 6A" nucleic acid under low stringency conditions,
   (viii) "PROTEASOME SUBUNIT α TYPE 1" (SEQ ID No:193) or a functionally active derivative thereof, or a functionally active fragment thereof, or a homolog thereof, or a variant of "PROTEASOME SUBUNIT α TYPE 1" encoded by a nucleic acid that hybridizes to the "PROTEASOME SUBUNIT α TYPE 1" nucleic acid under low stringency conditions,
   (ix) "PROTEASOME SUBUNIT α TYPE 4" (SEQ ID No:194) or a functionally active derivative thereof, or a functionally active fragment thereof, or a homolog thereof, or a variant of "PROTEASOME SUBUNIT α TYPE 4" encoded by a nucleic acid that hybridizes to the "PROTEASOME SUBUNIT α TYPE 4" nucleic acid under low stringency conditions,
   (x) "PROTEASOME (PROSOME, MACROPAIN) 26S SUBUNIT, NON-ATPASE, 1" (SEQ ID No:195) or a functionally active derivative thereof, or a functionally active fragment thereof, or a homolog thereof, or a variant of "PROTEASOME (PROSOME, MACROPAIN) 26S SUBUNIT, NON-ATPASE, 1" encoded by a nucleic acid that hybridizes to the "PROTEASOME (PROSOME, MACROPAIN) 26S SUBUNIT, NON-ATPASE, 1" nucleic acid under low stringency conditions,
   (xi) "SIMILAR TO PROTEASE (PROSOME, MACROPAIN) 26S SUBUNIT, ATPASE 1" (SEQ ID No:196) or a functionally active derivative thereof, or a functionally active fragment thereof, or a homolog thereof, or a variant of "SIMILAR TO PROTEASE (PROSOME, MACROPAIN) 26S SUBUNIT, ATPASE 1" encoded by a nucleic acid that hybridizes to the "SIMILAR TO PROTEASE (PROSOME, MACROPAIN) 26S SUBUNIT, ATPASE 1" nucleic acid under low stringency conditions,
   (xii) "HYPOTHETICAL PROTEIN FLJ20552" (SEQ ID No:197) or a functionally active derivative thereof, or a functionally active fragment thereof, or a homolog thereof, or a variant of "HYPOTHETICAL PROTEIN FLJ20552" encoded by a nucleic acid that hybridizes to the "HYPOTHETICAL PROTEIN FLJ20552" nucleic acid under low stringency conditions,
   (xiii) "26S PROTEASOME REGULATORY SUBUNIT S2" (SEQ ID No:198) or a functionally active derivative thereof, or a functionally active fragment thereof, or a homolog thereof, or a variant of "26S PROTEASOME REGULATORY SUBUNIT S2" encoded by a nucleic acid that hybridizes to the "26S PROTEASOME REGULATORY SUBUNIT S2" nucleic acid under low stringency conditions,
   (xiv) "26S PROTEASOME SUBUNIT P40.5" (SEQ ID No:199) or a functionally active derivative thereof, or a functionally active fragment thereof, or a homolog thereof, or a variant of "26S PROTEASOME SUBUNIT P40.5" encoded by a nucleic acid that hybridizes to the "26S PROTEASOME SUBUNIT P40.5" nucleic acid under low stringency conditions,
   (xv) "26S PROTEASE REGULATORY SUBUNIT S10B" (SEQ ID No:200) or a functionally active derivative thereof, or a functionally active fragment thereof, or a homolog thereof, or a variant of "26S PROTEASE REGULATORY SUBUNIT S10B" encoded by a nucleic acid that hybridizes to the "26S PROTEASE REGULATORY SUBUNIT S10B" nucleic acid under low stringency conditions,
   (xvi) "26S PROTEASE REGULATORY SUBUNIT 7" (SEQ ID No:201) or a functionally active derivative thereof, or a functionally active fragment thereof, or a homolog thereof, or a variant of "26S PROTEASE REGULATORY SUBUNIT 7" encoded by a nucleic acid that hybridizes to the "26S PROTEASE REGULATORY SUBUNIT 7" nucleic acid under low stringency conditions,
   (xvii) "26S PROTEASOME SUBUNIT P55" (SEQ ID No:202) or a functionally active derivative thereof, or a functionally active fragment thereof, or a homolog thereof, or a variant of "26S PROTEASOME SUBUNIT P55" encoded by a nucleic acid that hybridizes to the "26S PROTEASOME SUBUNIT P55" nucleic acid under low stringency conditions,
   (xviii) "VOLTAGE-GATED POTASSIUM CHANNEL PROTEIN KQT-LIKE 1" (SEQ ID No:203) or a functionally active derivative thereof, or a functionally active fragment thereof, or a homolog thereof, or a variant of "VOLTAGE-GATED POTASSIUM CHANNEL PROTEIN KQT-LIKE 1" encoded by a nucleic acid that hybridizes to the "VOLTAGE-GATED POTASSIUM CHANNEL PROTEIN KQT-LIKE 1" nucleic acid under low stringency conditions,
   (xix) "26S PROTEASOME NON-ATPASE REGULATORY SUBUNIT 6" (SEQ ID No:204) or a functionally active derivative thereof, or a functionally active fragment thereof, or a homolog thereof, or a variant of "26S PROTEASOME NON-ATPASE REGULATORY SUBUNIT 6" encoded by a nucleic acid that hybridizes to the "26S PROTEASOME NON-ATPASE REGULATORY SUBUNIT 6" nucleic acid under low stringency conditions,
   (xx) "26S PROTEASOME REGULATORY SUBUNIT S12" (SEQ ID No:205) or a functionally active derivative thereof, or a functionally active fragment thereof, or a homolog thereof, or a variant of "26S PROTEASOME REGULATORY SUBUNIT S12" encoded by a nucleic acid that hybridizes to the "26S PROTEASOME REGULATORY SUBUNIT S12" nucleic acid under low stringency conditions,
   (xxi) "26S PROTEASOME REGULATORY SUBUNIT S3" (SEQ ID No:206) or a functionally active derivative thereof, or a functionally active fragment thereof, or a homolog thereof, or a variant of "26S PROTEASOME REGULATORY SUBUNIT S3" encoded by a nucleic acid that hybridizes to the "26S PROTEASOME REGULATORY SUBUNIT S3" nucleic acid under low stringency conditions,
   (xxii) "26S PROTEASE REGULATORY SUBUNIT 4" (SEQ ID No:207) or a functionally active derivative thereof, or a functionally active fragment thereof, or a homolog thereof, or a variant of "26S PROTEASE REGULATORY SUBUNIT 4" encoded by a nucleic acid that hybridizes to the "26S PROTEASE REGULATORY SUBUNIT 4" nucleic acid under low stringency conditions,
   (xxiii) "PROTEASOME SUBUNIT β TYPE 2" (SEQ ID No:208) or a functionally active derivative thereof, or a functionally active fragment thereof, or a homolog thereof, or a variant of "PROTEASOME SUBUNIT β TYPE 2" encoded by a nucleic acid that hybridizes to the "PROTEASOME SUBUNIT β TYPE 2" nucleic acid under low stringency conditions,
   (xxiv) "26S PROTEASOME NON-ATPASE REGULATORY SUBUNIT 11" (SEQ ID No:209) or a functionally active derivative thereof, or a functionally active fragment thereof, or a homolog thereof, or a variant of "26S PROTEASOME NON-ATPASE REGULATORY SUBUNIT 1" encoded by a nucleic acid that hybridizes to the "26S PROTEASOME NON-ATPASE REGULATORY SUBUNIT 1" nucleic acid under low stringency conditions,
   (xxv) "OXYSTEROL BINDING PROTEIN-RELATED PROTEIN 8" (SEQ ID No:55) or a functionally active derivative thereof, or a functionally active fragment thereof, or a homolog thereof, or a variant of "OXYSTEROL BINDING PROTEIN-RELATED PROTEIN 8" encoded by a nucleic acid that hybridizes to the "OXYSTEROL BINDING PROTEIN-RELATED PROTEIN 8" nucleic acid under low stringency conditions,
   (xxvi) "26S PROTEASOME REGULATORY SUBUNIT S5A" (SEQ ID No:210) or a functionally active derivative thereof, or a functionally active fragment thereof, or a homolog thereof, or a variant of "26S PROTEASOME REGULATORY SUBUNIT S5A" encoded by a nucleic acid that hybridizes to the "26S PROTEASOME REGULATORY SUBUNIT S5A" nucleic acid under low stringency conditions,
   (xxvii) "PROTEASOME SUBUNIT β TYPE 6 PRECURSOR" (SEQ ID No:211) or a functionally active derivative thereof, or a functionally active fragment thereof, or a homolog thereof, or a variant of "PROTEASOME SUBUNIT β TYPE 6 PRECURSOR" encoded by a nucleic acid that hybridizes to the "PROTEASOME SUBUNIT β TYPE 6 PRECURSOR" nucleic acid under low stringency conditions,
   (xxviii) "26S PROTEASOME SUBUNIT 9" (SEQ ID No:212) or a functionally active derivative thereof, or a functionally active fragment thereof, or a homolog thereof, or a variant of "26S PROTEASOME SUBUNIT 9" encoded by a nucleic acid that hybridizes to the "26S PROTEASOME SUBUNIT 9" nucleic acid under low stringency conditions,
   (xxix) "26S PROTEASOME NON-ATPASE REGULATORY SUBUNIT 7" (SEQ ID No:213) or a functionally active derivative thereof, or a functionally active fragment thereof, or a homolog thereof, or a variant of "26S PROTEASOME NON-ATPASE REGULATORY SUBUNIT 7" encoded by a nucleic acid that hybridizes to the "26S PROTEASOME NON-ATPASE REGULATORY SUBUNIT 7" nucleic acid under low stringency conditions,
   (xxx) "HSPC121" (SEQ ID No:214) or a functionally active derivative thereof, or a functionally active fragment thereof, or a homolog thereof, or a variant of "HSPC121" encoded by a nucleic acid that hybridizes to the "HSPC121" nucleic acid under low stringency conditions,
   (xxxi) "26S PROTEASOME NON-ATPASE REGULATORY SUBUNIT 1" (SEQ ID No:215) or a functionally active derivative thereof, or a functionally active fragment thereof, or a homolog thereof, or a variant of "26S PROTEASOME NON-ATPASE REGULATORY SUBUNIT 1" encoded by a nucleic acid that hybridizes to the "26S PROTEASOME NON-ATPASE REGULATORY SUBUNIT 1" nucleic acid under low stringency conditions,
   (xxxii) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,
   (xxxiii) "PROTEASOME SUBUNIT α TYPE 7" (SEQ ID No:216) or a functionally active derivative thereof, or a functionally active fragment thereof, or a homolog thereof, or a variant of "PROTEASOME SUBUNIT α TYPE 7" encoded by a nucleic acid that hybridizes to the "PROTEASOME SUBUNIT α TYPE 7" nucleic acid under low stringency conditions,
   (xxxiv) "26S PROTEASE REGULATORY SUBUNIT 8" (SEQ ID No:217) or a functionally active derivative thereof,or a functionally active fragement thereof, or a homolog thereof, or a variant of "26S PROTEASE REGULATORY SUBUNIT 8" that hybridizes to the "26S PROTEASE REGULATORY SUBUNIT 8" nucleic acid under low stringency conditions, and
   (xxxv) "PROTEASOME SUBUNIT α TYPE 6" (SEQ ID No:218) or a functionally active derivative thereof, or a functionally active fragment thereof, or a homolog thereof, or a variant of "PROTEASOME SUBUNIT α TYPE 6" encoded by a nucleic acid that hybridizes to the "PROTEASOME SUBUNIT α TYPE 6" nucleic acid under low stringency conditions.
4. The protein complex according to No. 1 selected from complex (I) comprising all but 1 - 30 of the following proteins:
   (i) "RIP2 " (SEQ ID No:187) or a functionally active derivative thereof, or a functionally active fragment thereof, or a homolog thereof, or a variant of "RIP2" encoded by a nucleic acid that hybridizes to the "RIP2" nucleic acid under low stringency conditions,
   (ii) "TAB1 TAK1-BINDING PROTEIN 1" (SEQ ID No:188) or a functionally active derivative thereof, or a functionally active fragment thereof, or a homolog thereof, or a variant of "TAB1 TAK1-BINDING PROTIEN 1" encoded by a nucleic acid that hybridizes to the "TAB1 TAK1-BINDING PROTEIN 1" nucleic acid under low stringency conditions,
   (iii) "p38" (SEQ ID No: 118) or a functionally active derivative thereof, or a functionally active fragment thereof, or a homolog thereof, or a variant of "p38" encoded by a nucleic acid that hybridizes to the "p38" nucleic acid under low stringency conditions,
   (iv) "TRAF6" (SEQ ID No:189) or a functionally active derivative thereof, or a functionally active fragment thereof, or a homolog thereof, or a variant of "TRAF6" encoded by a nucleic acid that hybridizes to the "TRAF6" nucleic acid under low stringency conditions,
   (v) "PROTEASOME SUBUNIT β TYPE 5 PRECURSOR" (SEQ ID No: 190) or a functionally active derivative thereof, or a functionally active fragment thereof, or a homolog thereof, or a variant of "PROTEASOME SUBUNIT β TYPE 5 PRECURSOR" encoded by a nucleic acid that hybridizes to the "PROTEASOME SUBUNIT β TYPE 5 PRECURSOR" nucleic acid under low stringency conditions,
   (vi) "26S PROTEASOME NON-ATPASE REGULATORY SUBUNIT 8" (SEQ ID No:191) or a functionally active derivative thereof, or a functionally active fragment thereof, or a homolog thereof, or a variant of "26S PROTEASOME NON-ATPASE REGULATORY SUBUNIT 8" encoded by a nucleic acid that hybridizes to the "26S PROTEASOME NON-ATPASE REGULATORY SUBUNIT 8" nucleic acid under low stringency conditions,
   (vii) "26S PROTEASE REGULATORY SUBUNIT 6A" (SEQ ID No:192) or a functionally active derivative thereof, or a functionally active fragment thereof, or a homolog thereof, or a variant of "26S PROTEASE REGULATORY SUBUNIT 6A" encoded by a nucleic acid that hybridizes to the "26S PROTEASE REGULATORY SUBUNIT 6A" nucleic acid under low stringency conditions,
   (viii) "PROTEASOME SUBUNIT α TYPE 1" (SEQ ID No:193) or a functionally active derivative thereof, or a functionally active fragment thereof, or a homolog thereof, or a variant of "PROTEASOME SUBUNIT α TYPE 1" encoded by a nucleic acid that hybridizes to the "PROTEASOME SUBUNIT α TYPE 1" nucleic acid under low stringency conditions,
   (ix) "PROTEASOME SUBUNIT α TYPE 4" (SEQ ID No:194) or a functionally active derivative thereof, or a functionally active fragment thereof, or a homolog thereof, or a variant of "PROTEASOME SUBUNIT α TYPE 4" encoded by a nucleic acid that hybridizes to the "PROTEASOME SUBUNIT α TYPE 4" nucleic acid under low stringency conditions,
   (x) "PROTEASOME (PROSOME, MACROPAIN) 26S SUBUNIT, NON-ATPASE, 1" (SEQ ID No:195) or a functionally active derivative thereof, or a functionally active fragment thereof, or a homolog thereof, or a variant of "PROTEASOME (PROSOME, MACROPAIN) 26S SUBUNIT, NON-ATPASE, 1" encoded by a nucleic acid that hybridizes to the "PROTEASOME (PROSOME, MACROPAIN) 26S SUBUNIT, NON-ATPASE, 1" nucleic acid under low stringency conditions,
   (xi) "SIMILAR TO PROTEASE (PROSOME, MACROPAIN) 26S SUBUNIT, ATPASE 1" (SEQ ID No:196) or a functionally active derivative thereof, or a functionally active fragment thereof, or a homolog thereof, or a variant of "SIMILAR TO PROTEASE (PROSOME, MACROPAIN) 26S SUBUNIT, ATPASE 1" encoded by a nucleic acid that hybridizes to the "SIMILAR TO PROTEASE (PROSOME, MACROPAIN) 26S SUBUNIT, ATPASE 1" nucleic acid under low stringency conditions,
   (xii) "HYPOTHETICAL PROTEIN FLJ20552" (SEQ ID No:197) or a functionally active derivative thereof, or a functionally active fragment thereof, or a homolog thereof; or a variant of "HYPOTHETICAL PROTEIN FLJ20552" encoded by a nucleic acid that hybridizes to the "HYPOTHETICAL PROTEIN FLJ20552" nucleic acid under low stringency conditions,
   (xiii) "26S PROTEASOME REGULATORY SUBUNIT S2" (SEQ ID No:198) or a functionally active derivative thereof, or a functionally active fragment thereof, or a homolog thereof, or a variant of "26S PROTEASOME REGULATORY SUBUNIT S2" encoded by a nucleic acid that hybridizes to the "26S PROTEASOME REGULATORY SUBUNIT S2" nucleic acid under low stringency conditions,
   (xiv) "26S PROTEASOME SUBUNIT P40.5" (SEQ ID No:199) or a functionally active derivative thereof, or a functionally active fragment thereof, or a homolog thereof, or a variant of "26S PROTEASOME SUBUNIT P40.5" encoded by a nucleic acid that hybridizes to the "26S PROTEASOME SUBUNIT P40.5" nucleic acid under low stringency conditions,
   (xv) "26S PROTEASE REGULATORY SUBUNIT S10B" (SEQ ID No:200) or a functionally active derivative thereof, or a functionally active fragment thereof, or a homolog thereof, or a variant of "26S PROTEASE REGULATORY SUBUNIT S10B" encoded by a nucleic acid that hybridizes to the "26S PROTEASE REGULATORY SUBUNIT S10B" nucleic acid under low stringency conditions,
   (xvi) "26S PROTEASE REGULATORY SUBUNIT 7" (SEQ ID No:201) or a functionally active derivative thereof, or a functionally active fragment thereof, or a homolog thereof, or a variant of "26S PROTEASE REGULATORY SUBUNIT 7" encoded by a nucleic acid that hybridizes to the "26S PROTEASE REGULATORY SUBUNIT 7" nucleic acid under low stringency conditions,
   (xvii) "26S PROTEASOME SUBUNIT P55" (SEQ ID No:202) or a functionally active derivative thereof, or a functionally active fragment thereof, or a homolog thereof, or a variant of "26S PROTEASOME SUBUNIT P55" encoded by a nucleic acid that hybridizes to the "26S PROTEASOME SUBUNIT P55" nucleic acid under low stringency conditions,
   (xviii) "VOLTAGE-GATED POTASSIUM CHANNEL PROTEIN KQT-LIKE 1" (SEQ ID No:203) or a functionally active derivative thereof, or a functionally active fragment thereof, or a homolog thereof, or a variant of "VOLTAGE-GATED POTASSIUM CHANNEL PROTEIN KQT-LIKE 1" encoded by a nucleic acid that hybridizes to the "VOLTAGE-GATED POTASSIUM CHANNEL PROTEIN KQT-LIKE 1" nucleic acid under low stringency conditions,
   (xix) "26S PROTEASOME NON-ATPASE REGULATORY SUBUNIT 6" (SEQ ID No:204) or a functionally active derivative thereof, or a functionally active fragment thereof, or a homolog thereof, or a variant of "26S PROTEASOME NON-ATPASE REGULATORY SUBUNIT 6" encoded by a nucleic acid that hybridizes to the "26S PROTEASOME NON-ATPASE REGULATORY SUBUNIT 6" nucleic acid under low stringency conditions,
   (xx) "26S PROTEASOME REGULATORY SUBUNIT S12" (SEQ ID No:205) or a functionally active derivative thereof, or a functionally active fragment thereof, or a homolog thereof, or a variant of "26S PROTEASOME REGULATORY SUBUNIT S12" encoded by a nucleic acid that hybridizes to the "26S PROTEASOME REGULATORY SUBUNIT S12" nucleic acid under low stringency conditions,
   (xxi) "26S PROTEASOME REGULATORY SUBUNIT S3" (SEQ ID No:206) or a functionally, active derivative thereof, or a functionally active fragment thereof, or a homolog thereof, or a variant of "26S PROTEASOME REGULATORY SUBUNIT S3" encoded by a nucleic acid that hybridizes to the "26S PROTEASOME REGULATORY SUBUNIT S3" nucleic acid under low stringency conditions,
   (xxii) "26S PROTEASE REGULATORY SUBUNIT 4" (SEQ ID No:207) or a functionally active derivative thereof, or a functionally active fragment thereof, or a homolog thereof, or a variant of "26S PROTEASE REGULATORY SUBUNIT 4" encoded by a nucleic acid that hybridizes to the "26S PROTEASE REGULATORY SUBUNIT 4" nucleic acid under low stringency conditions,
   (xxiii) "PROTEASOME SUBUNIT β TYPE 2" (SEQ ID No:208) or a functionally active derivative thereof, or a functionally active fragment thereof, or a homolog thereof, or a variant of "PROTEASOME SUBUNIT β TYPE 2" encoded by a nucleic acid that hybridizes to the "PROTEASOME SUBUNIT β TYPE 2" nucleic acid under low stringency conditions,
   (xxiv) "26S PROTEASOME NON-ATPASE REGULATORY SUBUNIT 11" (SEQ ID No:209) or a functionally active derivative thereof, or a functionally active fragment thereof, or a homolog thereof, or a variant of "26S PROTEASOME NON-ATPASE REGULATORY SUBUNIT 1" encoded by a nucleic acid that hybridizes to the "26S PROTEASOME NON-ATPASE REGULATORY SUBUNIT 1" nucleic acid under low stringency conditions,
   (xxv) "OXYSTEROL BINDING PROTEIN-RELATED PROTEIN 8" (SEQ ID No:55) or a functionally active derivative thereof, or a functionally active fragment thereof, or a homolog thereof, or a variant of "OXYSTEROL BINDING PROTEIN-RELATED PROTEIN 8" encoded by a nucleic acid that hybridizes to the "OXYSTEROL BINDING PROTEIN-RELATED PROTEIN 8" nucleic acid under low stringency conditions,
   (xxvi) "26S PROTEASOME REGULATORY SUBUNIT S5A" (SEQ ID No:210) or a functionally active derivative thereof, or a functionally active fragment thereof, or a homolog thereof, or a variant of "26S PROTEASOME REGULATORY SUBUNIT S5A" encoded by a nucleic acid that hybridizes to the "26S PROTEASOME REGULATORY SUBUNIT S5A" nucleic acid under low stringency conditions,
   (xxvii) "PROTEASOME SUBUNIT β TYPE 6 PRECURSOR" (SEQ ID No:211) or a functionally active derivative thereof, or a functionally active fragment thereof, or a homolog thereof, or a variant of "PROTEASOME SUBUNIT β TYPE 6 PRECURSOR" encoded by a nucleic acid that hybridizes to the "PROTEASOME SUBUNIT β TYPE 6 PRECURSOR" nucleic acid under low stringency conditions,
   (xxviii) "26S PROTEASOME SUBUNIT 9" (SEQ ID No:212) or a functionally active derivative thereof, or a functionally active fragment thereof, or a homolog thereof, or a variant of "26S PROTEASOME SUBUNIT 9" encoded by a nucleic acid that hybridizes to the "26S PROTEASOME SUBUNIT 9" nucleic acid under low stringency conditions,
   (xxix) "26S PROTEASOME NON-ATPASE REGULATORY SUBUNIT 7" (SEQ ID No:213) or a functionally active derivative thereof, or a functionally active fragment thereof, or a homolog thereof, or a variant of "26S PROTEASOME NON-ATPASE REGULATORY SUBUNIT 7" encoded by a nucleic acid that hybridizes to the "26S PROTEASOME NON-ATPASE REGULATORY SUBUNIT 7" nucleic acid under low stringency conditions,
   (xxx) "HSPC121" (SEQ ID No:214) or a functionally active derivative thereof, or a functionally active fragment thereof, or a homolog thereof, or a variant of "HSPC121" encoded by a nucleic acid that hybridizes to the "HSPC121" nucleic acid under low stringency conditions,
   (xxxi) "26S PROTEASOME NON-ATPASE REGULATORY SUBUNIT 1" (SEQ ID No:215) or a functionally active derivative thereof, or a functionally active fragment thereof, or a homolog thereof, or a variant of "26S PROTEASOME NON-ATPASE REGULATORY SUBUNIT 1" encoded by a nucleic acid that hybridizes to the "26S PROTEASOME NON-ATPASE REGULATORY SUBUNIT 1" nucleic acid under low stringency conditions,
   (xxxii) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,
   (xxxiii) "PROTEASOME SUBUNIT α TYPE 7" (SEQ ID No:216) or a functionally active derivative thereof, or a functionally active fragment thereof, or a homolog thereof, or a variant of "PROTEASOME SUBUNIT α TYPE 7" encoded by a nucleic acid that hybridizes to the "PROTEASOME SUBUNIT α TYPE 7" nucleic acid under low stringency conditions,
   (xxxiv) "26S PROTEASE REGULATORY SUBUNIT 8" (SEQ ID No:217) or a functionally active derivative thereof,or a functionally active fragement thereof, or a homolog thereof, or a variant of "26S PROTEASE REGULATORY SUBUNIT 8" that hybridizes to the "26S PROTEASE REGULATORY SUBUNIT 8" nucleic acid under low stringency conditions, and
   (xxxv) "PROTEASOME SUBUNIT α TYPE 6" (SEQ ID No:218) or a functionally active derivative thereof, or a functionally active fragment thereof, or a homolog thereof, or a variant of "PROTEASOME SUBUNIT α TYPE 6" encoded by a nucleic acid that hybridizes to the "PROTEASOME SUBUNIT α TYPE 6" nucleic acid under low stringency conditions;
   and a protein complex selected from complex (II) comprising all but 1 - 30 of the following proteins:
   (i) "TRAF6" (SEQ ID No:189) or a functionally active derivative thereof, or a functionally active fragment thereof, or a homolog thereof, or a variant of "TRAF6" encoded by a nucleic acid that hybridizes to the "TRAF6" nucleic acid under low stringency conditions,

   (i) "PROTEASOME SUBUNIT β TYPE 5 PRECURSOR" (SEQ ID No:190) or a functionally active derivative thereof, or a functionally active fragment thereof, or a homolog thereof, or a variant of "PROTEASOME SUBUNIT β TYPE 5 PRECURSOR" encoded by a nucleic acid that hybridizes to the "PROTEASOME SUBUNIT β TYPE 5 PRECURSOR" nucleic acid under low stringency conditions,
   (iii) "26S PROTEASOME NON-ATPASE REGULATORY SUBUNIT 8" (SEQ ID No: 191) or a functionally active derivative thereof, or a functionally active fragment thereof, or a homolog thereof, or a variant of "26S PROTEASOME NON-ATPASE REGULATORY SUBUNIT 8" encoded by a nucleic acid that hybridizes to the "26S PROTEASOME NON-ATPASE REGULATORY SUBUNIT 8" nucleic acid under low stringency conditions,
   (iv) "26S PROTEASE REGULATORY SUBUNIT 6A" (SEQ ID No:192) or a functionally active derivative thereof, or a functionally active fragment thereof, or a homolog thereof, or a variant of "26S PROTEASE REGULATORY SUBUNIT 6A" encoded by a nucleic acid that hybridizes to the "26S PROTEASE REGULATORY SUBUNIT 6A" nucleic acid under low stringency conditions,
   (v) "PROTEASOME SUBUNIT α TYPE 1" (SEQ ID No:193) or a functionally active derivative thereof, or a functionally active fragment thereof, or a homolog thereof, or a variant of "PROTEASOME SUBUNIT α TYPE 1" encoded by a nucleic acid that hybridizes to the "PROTEASOME SUBUNIT α TYPE 1" nucleic acid under low stringency conditions,
   (vi) "PROTEASOME SUBUNIT α TYPE 4" (SEQ ID No:194) or a functionally active derivative thereof, or a functionally active fragment thereof, or a homolog thereof, or a variant of "PROTEASOME SUBUNIT α TYPE 4" encoded by a nucleic acid that hybridizes to the "PROTEASOME SUBUNIT α TYPE 4" nucleic acid under low stringency conditions,
   (vii) "PROTEASOME (PROSOME, MACROPAIN) 26S SUBUNIT, NON-ATPASE, 1" (SEQ ID No:195) or a functionally active derivative thereof, or a functionally active fragment thereof, or a homolog thereof, or a variant of "PROTEASOME (PROSOME, MACROPAIN) 26S SUBUNIT, NON-ATPASE, 1" encoded by a nucleic acid that hybridizes to the "PROTEASOME (PROSOME, MACROPAIN) 26S SUBUNIT, NON-ATPASE, 1" nucleic acid under low stringency conditions,
   (viii) "SIMILAR TO PROTEASE (PROSOME, MACROPAIN) 26S SUBUNIT, ATPASE 1" (SEQ ID No:196) or a functionally active derivative thereof, or a functionally active fragment thereof, or a homolog thereof, or a variant of "SIMILAR TO PROTEASE (PROSOME, MACROPAIN) 26S SUBUNIT, ATPASE 1" encoded by a nucleic acid that hybridizes to the "SIMILAR TO PROTEASE (PROSOME, MACROPAIN) 26S SUBUNIT, ATPASE 1" nucleic acid under low stringency conditions,
   (ix) "HYPOTHETICAL PROTEIN FLJ20552" (SEQ ID No:197) or a functionally active derivative thereof, or a functionally active fragment thereof, or a homolog thereof, or a variant of "HYPOTHETICAL PROTEIN FLJ20552" encoded by a nucleic acid that hybridizes to the "HYPOTHETICAL PROTEIN FLJ20552" nucleic acid under low stringency conditions,
   (x) "26S PROTEASOME REGULATORY SUBUNIT S2" (SEQ ID No:198) or a functionally active derivative thereof, or a functionally active fragment thereof, or a homolog thereof, or a variant of "26S PROTEASOME REGULATORY SUBUNIT S2" encoded by a nucleic acid that hybridizes to the "26S PROTEASOME REGULATORY SUBUNIT S2" nucleic acid under low stringency conditions,
   (xi) "26S PROTEASOME SUBUNIT P40.5" (SEQ ID No:199) or a functionally active derivative thereof, or a functionally active fragment thereof, or a homolog thereof, or a variant of "26S PROTEASOME SUBUNIT P40.5" encoded by a nucleic acid that hybridizes to the "26S PROTEASOME SUBUNIT P40.5" nucleic acid under low stringency conditions,
   (xii) "26S PROTEASE REGULATORY SUBUNIT S10B" (SEQ ID No:200) or a functionally active derivative thereof, or a functionally active fragment thereof, or a homolog thereof, or a variant of "26S PROTEASE REGULATORY SUBUNIT S10B" encoded by a nucleic acid that hybridizes to the "26S PROTEASE REGULATORY SUBUNIT S10B" nucleic acid under low stringency conditions,
   (xiii) "26S PROTEASE REGULATORY SUBUNIT 7" (SEQ ID No:201) or a functionally active derivative thereof, or a functionally active fragment thereof, or a homolog thereof, or a variant of "26S PROTEASE REGULATORY SUBUNIT 7" encoded by a nucleic acid that hybridizes to the "26S PROTEASE REGULATORY SUBUNIT 7" nucleic acid under low stringency conditions,
   (xiv) "26S PROTEASOME SUBUNIT P55" (SEQ ID No:202) or a functionally active derivative thereof, or a functionally active fragment thereof, or a homolog thereof, or a variant of "26S PROTEASOME SUBUNIT P55" encoded by a nucleic acid that hybridizes to the "26S PROTEASOME SUBUNIT P55" nucleic acid under low stringency conditions,
   (xv) "VOLTAGE-GATED POTASSIUM CHANNEL PROTEIN KQT-LIKE 1" (SEQ ID No:203) or a functionally active derivative thereof, or a functionally active fragment thereof, or a homolog thereof, or a variant of "VOLTAGE-GATED POTASSIUM CHANNEL PROTEIN KQT-LIKE 1" encoded by a nucleic acid that hybridizes to the "VOLTAGE-GATED POTASSIUM CHANNEL PROTEIN KQT-LIKE 1" nucleic acid under low stringency conditions,
   (xvi) "26S PROTEASOME NON-ATPASE REGULATORY SUBUNIT 6" (SEQ ID No:204) or a functionally active derivative thereof, or a functionally active fragment thereof, or a homolog thereof, or a variant of "26S PROTEASOME NON-ATPASE REGULATORY SUBUNIT 6" encoded by a nucleic acid that hybridizes to the "26S PROTEASOME NON-ATPASE REGULATORY SUBUNIT 6" nucleic acid under low stringency conditions,
   (xvii) "26S PROTEASOME REGULATORY SUBUNIT S12" (SEQ ID No:205) or a functionally active derivative thereof, or a functionally active fragment thereof, or a homolog thereof, or a variant of "26S PROTEASOME REGULATORY SUBUNIT S12" encoded by a nucleic acid that hybridizes to the "26S PROTEASOME REGULATORY SUBUNIT S12" nucleic acid under low stringency conditions,
   (xviii) "26S PROTEASOME REGULATORY SUBUNIT S3" (SEQ ID No:206) or a functionally active derivative thereof, or a functionally active fragment thereof, or a homolog thereof, or a variant of "26S PROTEASOME REGULATORY SUBUNIT S3" encoded by a nucleic acid that hybridizes to the "26S PROTEASOME REGULATORY SUBUNIT S3" nucleic acid under low stringency conditions,
   (xix) "26S PROTEASE REGULATORY SUBUNIT 4" (SEQ ID No:207) or a functionally active derivative thereof, or a functionally active fragment thereof, or a homolog thereof, or a variant of "26S PROTEASE REGULATORY SUBUNIT 4" encoded by a nucleic acid that hybridizes to the "26S PROTEASE REGULATORY SUBUNIT 4" nucleic acid under low stringency conditions,
   (xx) "PROTEASOME SUBUNIT β TYPE 2" (SEQ ID No:208) or a functionally active derivative thereof, or a functionally active fragment thereof, or a homolog thereof, or a variant of "PROTEASOME SUBUNIT β TYPE 2" encoded by a nucleic acid that hybridizes to the "PROTEASOME SUBUNIT β TYPE 2" nucleic acid under low stringency conditions,
   (xxi) "26S PROTEASOME NON-ATPASE REGULATORY SUBUNIT 11" (SEQ ID No:209) or a functionally active derivative thereof, or a functionally active fragment thereof, or a homolog thereof, or a variant of "26S PROTEASOME NON-ATPASE REGULATORY SUBUNIT 1" encoded by a nucleic acid that hybridizes to the "26S PROTEASOME NON-ATPASE REGULATORY SUBUNIT 1" nucleic acid under low stringency conditions,
   (xxii) "OXYSTEROL BINDING PROTEIN-RELATED PROTEIN 8" (SEQ ID No:55) or a functionally active derivative thereof, or a functionally active fragment thereof, or a homolog thereof, or a variant of "OXYSTEROL BINDING PROTEIN-RELATED PROTEIN 8" encoded by a nucleic acid that hybridizes to the "OXYSTEROL BINDING PROTEIN-RELATED PROTEIN 8" nucleic acid under low stringency conditions,
   (xxiii) "26S PROTEASOME REGULATORY SUBUNIT S5A" (SEQ ID No:210) or a functionally active derivative thereof, or a functionally active fragment thereof, or a homolog thereof, or a variant of "26S PROTEASOME REGULATORY SUBUNIT S5A" encoded by a nucleic acid that hybridizes to the "26S PROTEASOME REGULATORY SUBUNIT S5A" nucleic acid under low stringency conditions,
   (xxiv) "PROTEASOME SUBUNIT β TYPE 6 PRECURSOR" (SEQ ID No:211) or a functionally active derivative thereof, or a functionally active fragment thereof, or a homolog thereof, or a variant of "PROTEASOME SUBUNIT β TYPE 6 PRECURSOR" encoded by a nucleic acid that hybridizes to the "PROTEASOME SUBUNIT β TYPE 6 PRECURSOR" nucleic acid under low stringency conditions,
   (xxv) "26S PROTEASOME SUBUNIT 9" (SEQ ID No:212) or a functionally active derivative thereof, or a functionally active fragment thereof, or a homolog thereof, or a variant of "26S PROTEASOME SUBUNIT 9" encoded by a nucleic acid that hybridizes to the "26S PROTEASOME SUBUNIT 9" nucleic acid under low stringency conditions,
   (xxvi) "26S PROTEASOME NON-ATPASE REGULATORY SUBUNIT 7" (SEQ ID No:213) or a functionally active derivative thereof, or a functionally active fragment thereof, or a homolog thereof, or a variant of "26S PROTEASOME NON-ATPASE REGULATORY SUBUNIT 7" encoded by a nucleic acid that hybridizes to the "26S PROTEASOME NON-ATPASE REGULATORY SUBUNIT 7" nucleic acid under low stringency conditions,
   (xxvii) "HSPC121" (SEQ ID No:214) or a functionally active derivative thereof, or a functionally active fragment thereof, or a homolog thereof, or a variant of "HSPC121" encoded by a nucleic acid that hybridizes to the "HSPC121" nucleic acid under low stringency conditions,
   (xxviii) "26S PROTEASOME NON-ATPASE REGULATORY SUBUNIT 1" (SEQ ID No:215) or a functionally active derivative thereof, or a functionally active fragment thereof, or a homolog thereof, or a variant of "26S PROTEASOME NON-ATPASE REGULATORY SUBUNIT 1" encoded by a nucleic acid that hybridizes to the "26S PROTEASOME NON-ATPASE REGULATORY SUBUNIT 1" nucleic acid under low stringency conditions,
   (xxix) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,
   (xxx) "PROTEASOME SUBUNIT α TYPE 7" (SEQ ID No:216) or a functionally active derivative thereof, or a functionally active fragment thereof, or a homolog thereof, or a variant of "PROTEASOME SUBUNIT α TYPE 7" encoded by a nucleic acid that hybridizes to the "PROTEASOME SUBUNIT α TYPE 7" nucleic acid under low stringency conditions,
   (xxxi) "26S PROTEASE REGULATORY SUBUNIT 8" (SEQ ID No:217) or a functionally active derivative thereof,or a functionally active fragement thereof, or a homolog thereof, or a variant of "26S PROTEASE REGULATORY SUBUNIT 8" that hybridizes to the "26S PROTEASE REGULATORY SUBUNIT 8" nucleic acid under low stringency conditions, and
   (xxxii) "PROTEASOME SUBUNIT α TYPE 6" (SEQ ID No:218) or a functionally active derivative thereof, or a functionally active fragment thereof, or a homolog thereof, or a variant of "PROTEASOME SUBUNIT α TYPE 6" encoded by a nucleic acid that hybridizes to the "PROTEASOME SUBUNIT α TYPE 6" nucleic acid under low stringency conditions.
5. The complex of any of No. 1 - 4 comprising a functionally active derivative of said first protein and/or a functionally active derivative of said second protein, wherein the functionally active derivative is a fusion protein comprising said first protein or said second protein fused to an amino acid sequence different from the first protein or second protein, respectively.
6. The complex of No. 5 wherein the functionally active derivative is a fusion protein comprising said first protein or said second protein fused to an affinity tag or label.
7. The complex of any of No. 1 - 4 comprising a fragment of said first protein and/or a fragment of said second protein, which fragment binds to another protein component of said complex.
8. The complex of any of No. 1 -7 that is involved in the Ubiquitination activity or IKK kinase activity.
9. A process for preparing complex of any of No. 1 - 8 and optionally the components thereof comprising the following steps:
   the expressing a protein (bait) of the complex, preferably a tagged protein, in a target cell, isolating the protein complex which is attached to the bait protein, and optionally disassociating the protein complex and isolating the individual complex members.
10. The process according to No. 9 wherein the tagged protein comprises two different tags which allow two separate affinity purification steps.
11. The process according to any of No. 9 - 10 wherein the two tags are separated by a cleavage site for a protease.
12. Component of the TRAF6 complex obtainable by a process according to any of No. 9 to 11.
13. Protein of the TRAF6 complex selected from
   (i) "HYPOTHETICAL PROTEIN FLJ20552" (SEQ ID No:197) or a functionally active derivative thereof, or a functionally active fragment thereof, or a homolog thereof, or a variant of "HYPOTHETICAL PROTEIN FLJ20552" encoded by a nucleic acid that hybridizes to the "HYPOTHETICAL PROTEIN FLJ20552" nucleic acid under low stringency conditions,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1% SDS for 1.5 hours at 55°C, and washing in a buffer consisting of 2X SSC, 25 mM Tris-HCl (pH 7.4), 5 mM EDTA, and 0.1 % SDS for 1.5 hours at 60°C.
14. Nucleic acid encoding a protein according to No. 13.
15. Construct, preferably a vector construct, comprising (a) a nucleic acid according to No. 14 and at least one further nucleic acid which is normally not associated with said nucleic acid,or(b) at least two separate nucleic acid sequences each encoding a different protein, or a functionally active fragment or a functionally active derivative thereof at least one of said proteins, or functionally active fragments or functionally active derivative thereof being selected from the first group of proteins according to No. 1(a) and at least one of said proteins, or functionally active fragments or functionally active derivative thereof being selected from the second group of proteins according to No. 1 (b).
16. Host cell, containing a vector comprising at least one of the nucleic acid of No. 14 and/or a construct of No. 15 or containing several vectors each comprising at least the nucleic acid sequence encoding at least one of the proteins, or functionally active fragments or functionally active derivatives thereof selected from the first group of proteins according to No. 1(a) and the proteins, or functionally active fragments or functionally active derivatives thereof selected from the second group of proteins according to No. 1(b).
17. An antibody or a fragment of said antibody containing the binding domain thereof, selected from an antibody or fragment thereof, which binds the complex of any of No. 1-8 and which does not bind any of the proteins of said complex when uncomplexed and an antibody or a fragment of said antibody which binds to any of the proteins according to No. 13.
18. A kit comprising in one or more container the complex of any of No. 1 - 8 and/or the proteins of No. 13optionally together with an antibody according to No. 17 and/or further components such as reagents and working instructions.
19. The kit according to No. 18 for processing a substrate of said complex.
20. The kit according to No. 18 for the diagnosis or prognosis of a disease or a disease risk, preferentially for a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
21. Array, in which at least a complex according to any of No. 1 - 8 and/or at least one protein according to No. 14 and/or at least one antibody according to No. 17 is attached to a solid carrier.
22. A process for processing a physiological substrate of the complex comprising the step of bringing into contact a complex to any of No. 1-8 with said substrate, such that said substrate is processed.
23. A pharmaceutical composition comprising the protein complex of any of No. 1 to 8 and/or any of the following the proteins:
   (i) "HYPOTHETICAL PROTEIN FLJ20552" (SEQ ID No:197) or a functionally active derivative thereof, or a functionally active fragment thereof, or a homolog thereof, or a variant of "HYPOTHETICAL PROTEIN FLJ20552" encoded by a nucleic acid that hybridizes to the "HYPOTHETICAL PROTEIN FLJ20552" nucleic acid under low stringency conditions,
   and a pharmaceutical acceptable carrier.
24. The pharmaceutical composition according to No. 23 for the treatment of diseases and disorders such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
25. A method for screening for a molecule that binds to the complex of anyone of No. 1 - 8 and/or any of the following proteins:
   (i) "HYPOTHETICAL PROTEIN FLJ20552" (SEQ ID No:197) or a functionally active derivative thereof, or a functionally active fragment thereof, or a homolog thereof, or a variant of "HYPOTHETICAL PROTEIN FLJ20552" encoded by a nucleic acid that hybridizes to the "HYPOTHETICAL PROTEIN FLJ20552" nucleic acid under low stringency conditions, comprising the steps of
      (a) exposing said complex, or a cell or organism containing same to one or more candidate molecules; and
      (b) determinig whether said candidate molecule is bound to the complex or protein.
26. A method for screening for a molecule that modulates directly or indirectly the function, activity, composition or formation of the complex of any one of No. 1 - 8 comprising the steps of (a) exposing said complex, or a cell or organism containing TRAF6 complex to one or more candidate molecules; and
   (b) determining the amount of activity of protein components of, and/or intracellular localization of, said complex and/or the transcription level of a gene dependent on the complex and/or the abundance and/or activity of a protein or protein complex dependent on the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependent on the complex and/or the abundance and/or activity of a protein or protein complex dependent on the function of the complex and/or product of a gene dependent on the complex in the absence of said candidate molecules indicates that the molecule modulates function, activity or composition of said complex.
27. The method of No. 26, wherein the amount of said complex is determined.
28. The method of No. 26, wherein the activity of said complex is determined.
29. The method of No. 28, wherein said determining step comprises isolating from the cell or organism said complex to produce said isolated complex and contacting said isolated complex in the presence or absence of a candidate molecule with a substrate of said complex and determining the processing of said substrate is modified in the presence of said candidate molecule.
30. The method of No. 26, wherein the amount of the individual protein components of said complex are determined.
31. The method of No. 30, determining step comprises determining whether
   (i) "RIP2 " (SEQ ID No:187) or a functionally active derivative thereof, or a functionally active fragment thereof, or a homolog thereof, or a variant of "RIP2" encoded by a nucleic acid that hybridizes to the "RIP2" nucleic acid under low stringency conditions, and/or
   (ii) "TAB1 TAK1-BINDING PROTEIN 1" (SEQ ID No:188) or a functionally active derivative thereof, or a functionally active fragment thereof, or a homolog thereof, or a variant of "TAB1 TAK1-BINDING PROTIEN 1" encoded by a nucleic acid that hybridizes to the "TAB1 TAK1-BINDING PROTEIN 1" nucleic acid under low stringency conditions, and/or
   (iii) "p38" (SEQ ID No:118) or a functionally active derivative thereof, or a functionally active fragment thereof, or a homolog thereof, or a variant of "p38" encoded by a nucleic acid that hybridizes to the "p38" nucleic acid under low stringency conditions, and/or
   (iv) "TRAF6" (SEQ ID No:189) or a functionally active derivative thereof, or a functionally active fragment thereof, or a homolog thereof, or a variant of "TRAF6" encoded by a nucleic acid that hybridizes to the "TRAF6" nucleic acid under low stringency conditions, and/or
   (v) "PROTEASOME SUBUNIT β TYPE 5 PRECURSOR" (SEQ ID No:190) or a functionally active derivative thereof, or a functionally active fragment thereof, or a homolog thereof, or a variant of "PROTEASOME SUBUNIT β TYPE 5 PRECURSOR" encoded by a nucleic acid that hybridizes to the "PROTEASOME SUBUNIT β TYPE 5 PRECURSOR" nucleic acid under low stringency conditions, and/or
   (vi) "26S PROTEASOME NON-ATPASE REGULATORY SUBUNIT 8" (SEQ ID No:191) or a functionally active derivative thereof, or a functionally active fragment thereof, or a homolog thereof, or a variant of "26S PROTEASOME NON-ATPASE REGULATORY SUBUNIT 8" encoded by a nucleic acid that hybridizes to the "26S PROTEASOME NON-ATPASE REGULATORY SUBUNIT 8" nucleic acid under low stringency conditions, and/or
   (vii) "26S PROTEASE REGULATORY SUBUNIT 6A" (SEQ ID No:192) or a functionally active derivative thereof, or a functionally active fragment thereof, or a homolog thereof, or a variant of "26S PROTEASE REGULATORY SUBUNIT 6A" encoded by a nucleic acid that hybridizes to the "26S PROTEASE REGULATORY SUBUNIT 6A" nucleic acid under low stringency conditions, and/or
   (viii) "PROTEASOME SUBUNIT α TYPE 1" (SEQ ID No: 193) or a functionally active derivative thereof, or a functionally active fragment thereof, or a homolog thereof, or a variant of "PROTEASOME SUBUNIT α TYPE 1" encoded by a nucleic acid that hybridizes to the "PROTEASOME SUBUNIT α TYPE 1" nucleic acid under low stringency conditions, and/or
   (ix) "PROTEASOME SUBUNIT α TYPE 4" (SEQ ID No:194) or a functionally active derivative thereof, or a functionally active fragment thereof, or a homolog thereof, or a variant of "PROTEASOME SUBUNIT α TYPE 4" encoded by a nucleic acid that hybridizes to the "PROTEASOME SUBUNIT α TYPE 4" nucleic acid under low stringency conditions, and/or
   (x) "PROTEASOME (PROSOME, MACROPAIN) 26S SUBUNIT, NON-ATPASE, 1" (SEQ ID No:195) or a functionally active derivative thereof, or a functionally active fragment thereof, or a homolog thereof, or a variant of "PROTEASOME (PROSOME, MACROPAIN) 26S SUBUNIT, NON-ATPASE, 1" encoded by a nucleic acid that hybridizes to the "PROTEASOME (PROSOME, MACROPAIN) 26S SUBUNIT, NON-ATPASE, 1" nucleic acid under low stringency conditions, and/or
   (xi) "SIMILAR TO PROTEASE (PROSOME, MACROPAIN) 26S SUBUNIT, ATPASE 1" (SEQ ID No:196) or a functionally active derivative thereof, or a functionally active fragment thereof, or a homolog thereof, or a variant of "SIMILAR TO PROTEASE (PROSOME, MACROPAIN) 26S SUBUNIT, ATPASE 1" encoded by a nucleic acid that hybridizes to the "SIMILAR TO PROTEASE (PROSOME, MACROPAIN) 26S SUBUNIT, ATPASE 1" nucleic acid under low stringency conditions, and/or
   (xii) "HYPOTHETICAL PROTEIN FLJ20552" (SEQ ID No:197) or a functionally active derivative thereof, or a functionally active fragment thereof, or a homolog thereof, or a variant of "HYPOTHETICAL PROTEIN FLJ20552" encoded by a nucleic acid that hybridizes to the "HYPOTHETICAL PROTEIN FLJ20552" nucleic acid under low stringency conditions, and/or
   (xiii) "26S PROTEASOME REGULATORY SUBUNIT S2" (SEQ ID No:198) or a functionally active derivative thereof, or a functionally active fragment thereof, or a homolog thereof, or a variant of "26S PROTEASOME REGULATORY SUBUNIT S2" encoded by a nucleic acid that hybridizes to the "26S PROTEASOME REGULATORY SUBUNIT S2" nucleic acid under low stringency conditions, and/or
   (xiv) "26S PROTEASOME SUBUNIT P40.5" (SEQ ID No:199) or a functionally active derivative thereof, or a functionally active fragment thereof, or a homolog thereof, or a variant of "26S PROTEASOME SUBUNIT P40.5" encoded by a nucleic acid that hybridizes to the "26S PROTEASOME SUBUNIT P40.5" nucleic acid under low stringency conditions, and/or
   (xv) "26S PROTEASE REGULATORY SUBUNIT S10B" (SEQ ID No:200) or a functionally active derivative thereof, or a functionally active fragment thereof, or a homolog thereof, or a variant of "26S PROTEASE REGULATORY SUBUNIT S10B" encoded by a nucleic acid that hybridizes to the "26S PROTEASE REGULATORY SUBUNIT S10B" nucleic acid under low stringency conditions, and/or
   (xvi) "26S PROTEASE REGULATORY SUBUNIT 7" (SEQ ID No:201) or a functionally active derivative thereof, or a functionally active fragment thereof, or a homolog thereof, or a variant of "26S PROTEASE REGULATORY SUBUNIT 7" encoded by a nucleic acid that hybridizes to the "26S PROTEASE REGULATORY SUBUNIT 7" nucleic acid under low stringency conditions, and/or
   (xvii) "26S PROTEASOME SUBUNIT P55" (SEQ ID No:202) or a functionally active derivative thereof, or a functionally active fragment thereof, or a homolog thereof, or a variant of "26S PROTEASOME SUBUNIT P55" encoded by a nucleic acid that hybridizes to the "26S PROTEASOME SUBUNIT P55" nucleic acid under low stringency conditions, and/or
   (xviii) "VOLTAGE-GATED POTASSIUM CHANNEL PROTEIN KQT-LIKE 1" (SEQ ID No:203) or a functionally active derivative thereof, or a functionally active fragment thereof, or a homolog thereof, or a variant of "VOLTAGE-GATED POTASSIUM CHANNEL PROTEIN KQT-LIKE 1" encoded by a nucleic acid that hybridizes to the "VOLTAGE-GATED POTASSIUM CHANNEL PROTEIN KQT-LIKE 1" nucleic acid under low stringency conditions, and/or
   (xix) "26S PROTEASOME NON-ATPASE REGULATORY SUBUNIT 6" (SEQ ID No:204) or a functionally active derivative thereof, or a functionally active fragment thereof, or a homolog thereof, or a variant of "26S PROTEASOME NON-ATPASE REGULATORY SUBUNIT 6" encoded by a nucleic acid that hybridizes to the "26S PROTEASOME NON-ATPASE REGULATORY SUBUNIT 6" nucleic acid under low stringency conditions, and/or
   (xx) "26S PROTEASOME REGULATORY SUBUNIT S12" (SEQ ID No:205) or a functionally active derivative thereof, or a functionally active fragment thereof, or a homolog thereof, or a variant of "26S PROTEASOME REGULATORY SUBUNIT S12" encoded by a nucleic acid that hybridizes to the "26S PROTEASOME REGULATORY SUBUNIT S12" nucleic acid under low stringency conditions, and/or
   (xxi) "26S PROTEASOME REGULATORY SUBUNIT S3" (SEQ ID No:206) or a functionally active derivative thereof, or a functionally active fragment thereof, or a homolog thereof, or a variant of "26S PROTEASOME REGULATORY SUBUNIT S3" encoded by a nucleic acid that hybridizes to the "26S PROTEASOME REGULATORY SUBUNIT S3" nucleic acid under low stringency conditions, and/or
   (xxii) "26S PROTEASE REGULATORY SUBUNIT 4" (SEQ ID No:207) or a functionally active derivative thereof, or a functionally active fragment thereof, or a homolog thereof, or a variant of "26S PROTEASE REGULATORY SUBUNIT 4" encoded by a nucleic acid that hybridizes to the "26S PROTEASE REGULATORY SUBUNIT 4" nucleic acid under low stringency conditions, and/or
   (xxiii) "PROTEASOME SUBUNIT p TYPE 2" (SEQ ID No:208) or a functionally active derivative thereof, or a functionally active fragment thereof, or a homolog thereof, or a variant of "PROTEASOME SUBUNIT β TYPE 2" encoded by a nucleic acid that hybridizes to the "PROTEASOME SUBUNIT β TYPE 2" nucleic acid under low stringency conditions, and/or
   (xxiv) "26S PROTEASOME NON-ATPASE REGULATORY SUBUNIT 11" (SEQ ID No:209) or a functionally active derivative thereof, or a functionally active fragment thereof, or a homolog thereof, or a variant of "26S PROTEASOME NON-ATPASE REGULATORY SUBUNIT 1" encoded by a nucleic acid that hybridizes to the "26S PROTEASOME NON-ATPASE REGULATORY SUBUNIT 1" nucleic acid under low stringency conditions, and/or
   (xxv) "OXYSTEROL BINDING PROTEIN-RELATED PROTEIN 8" (SEQ ID No:55) or a functionally active derivative thereof, or a functionally active fragment thereof, or a homolog thereof, or a variant of "OXYSTEROL BINDING PROTEIN-RELATED PROTEIN 8" encoded by a nucleic acid that hybridizes to the "OXYSTEROL BINDING PROTEIN-RELATED PROTEIN 8" nucleic acid under low stringency conditions, and/or
   (xxvi) "26S PROTEASOME REGULATORY SUBUNIT S5A" (SEQ ID No:210) or a functionally active derivative thereof, or a functionally active fragment thereof, or a homolog thereof, or a variant of "26S PROTEASOME REGULATORY SUBUNIT S5A" encoded by a nucleic acid that hybridizes to the "26S PROTEASOME REGULATORY SUBUNIT S5A" nucleic acid under low stringency conditions, and/or
   (xxvii) "PROTEASOME SUBUNIT β TYPE 6 PRECURSOR" (SEQ ID No:211) or a functionally active derivative thereof, or a functionally active fragment thereof, or a homolog thereof, or a variant of "PROTEASOME SUBUNIT β TYPE 6 PRECURSOR" encoded by a nucleic acid that hybridizes to the "PROTEASOME SUBUNIT β TYPE 6 PRECURSOR" nucleic acid under low stringency conditions, and/or
   (xxviii) "26S PROTEASOME SUBUNIT 9" (SEQ ID No:212) or a functionally active derivative thereof, or a functionally active fragment thereof, or a homolog thereof, or a variant of "26S PROTEASOME SUBUNIT 9" encoded by a nucleic acid that hybridizes to the "26S PROTEASOME SUBUNIT 9" nucleic acid under low stringency conditions, and/or
   (xxix) "26S PROTEASOME NON-ATPASE REGULATORY SUBUNIT 7" (SEQ ID No:213) or a functionally active derivative thereof, or a functionally active fragment thereof, or a homolog thereof, or a variant of "26S PROTEASOME NON-ATPASE REGULATORY SUBUNIT 7" encoded by a nucleic acid that hybridizes to the "26S PROTEASOME NON-ATPASE REGULATORY SUBUNIT 7" nucleic acid under low stringency conditions, and/or
   (xxx) "HSPC121" (SEQ ID No:214) or a functionally active derivative thereof, or a functionally active fragment thereof, or a homolog thereof, or a variant of "HSPC121" encoded by a nucleic acid that hybridizes to the "HSPC121" nucleic acid under low stringency conditions, and/or
   (xxxi) "26S PROTEASOME NON-ATPASE REGULATORY SUBUNIT 1" (SEQ ID No:215) or a functionally active derivative thereof, or a functionally active fragment thereof, or a homolog thereof, or a variant of "26S PROTEASOME NON-ATPASE REGULATORY SUBUNIT 1" encoded by a nucleic acid that hybridizes to the "26S PROTEASOME NON-ATPASE REGULATORY SUBUNIT 1" nucleic acid under low stringency conditions, and/or
   (xxxii) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions, and/or
   (xxxiii) "PROTEASOME SUBUNIT α TYPE 7" (SEQ ID No:216) or a functionally active derivative thereof, or a functionally active fragment thereof, or a homolog thereof, or a variant of "PROTEASOME SUBUNIT α TYPE 7" encoded by a nucleic acid that hybridizes to the "PROTEASOME SUBUNIT α TYPE 7" nucleic acid under low stringency conditions, and/or
   (xxxiv) "26S PROTEASE REGULATORY SUBUNIT 8" (SEQ ID No:217) or a functionally active derivative thereof, or a functionally active fragment thereof, or a homolog thereof, or a variant of "26S PROTEASE REGULATORY SUBUNIT 8" encoded by a nucleic acid that hybridizes to the "26S PROTEASE REGULATORY SUBUNIT 8" nucleic acid under low stringency conditions, and/or
   (xxxv) "PROTEASOME SUBUNIT α TYPE 6" (SEQ ID No:218) or a functionally active derivative thereof, or a functionally active fragment thereof, or a homolog thereof, or a variant of "PROTEASOME SUBUNIT α TYPE 6" encoded by a nucleic acid that hybridizes to the "PROTEASOME SUBUNIT α TYPE 6" nucleic acid under low stringency conditions,
   is present in the complex.
32. The method of any of No. 26 to 31, wherein said method is a method of screening for a drug for treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
33. Use of a molecule that modulates the amount of, activity of, or the protein components of the complex of any one of No. 1 - 8 for the manufacture of a medicament for the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
34. A method for the production of a pharmaceutical composition comprising carrying out the method of any of No. 1 - 8 to identify a molecule that modulates the function, activity, composition or formation of said complex, and further comprising mixing the identified molecule with a pharmaceutically acceptable carrier.
35. A method for diagnosing or screening for the presence of a disease or disorder or a predisposition for developing a disease or disorder in a subject, which disease or disorder is characterized by an aberrant amount of, activity of, or component composition of, or intracellular localization of the complex of any one of the No. 1-8, comprising determining the amount of, activity of, protein components of, and/or intracellular localization of, said complex and/or the transcription level of a gene dependent on the complex and/or the abundance and/or activity of a protein or protein complex dependent on the function of the complex and/or product of a gene dependent on the complex in a comparative sample derived from a subject, wherein a difference in said amount, activity, or protein components of, said complex in an analogous sample from a subject not having the disease or disorder or predisposition indicates the presence in the subject of the disease or disorder or predisposition in the subject.
36. The method of No. 35, wherein the amount of said complex is determined.
37. The method of No. 35, wherein the activity of said complex is determined.
38. The method of No. 37, wherein said determining step comprises isolating from the subject said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
39. The method of No. 35, wherein the amount of the individual protein components of said complex are determined.
40. The method of No. 39, wherein said determining step comprises determining whether
   (i) "RIP2" (SEQ ID No:187) or a functionally active derivative thereof, or a functionally active fragment thereof, or a homolog thereof, or a variant of "RIP2" encoded by a nucleic acid that hybridizes to the "RIP2" nucleic acid under low stringency conditions, and/or
   (ii) "TAB1 TAK1-BINDING PROTEIN 1" (SEQ ID No:188) or a functionally active derivative thereof, or a functionally active fragment thereof, or a homolog thereof, or a variant of "TAB1 TAK1-BINDING PROTIEN 1" encoded by a nucleic acid that hybridizes to the "TAB1 TAK1-BINDING PROTEIN 1" nucleic acid under low stringency conditions, and/or
   (iii) "p38" (SEQ ID No:118) or a functionally active derivative thereof, or a functionally active fragment thereof, or a homolog thereof, or a variant of "p38" encoded by a nucleic acid that hybridizes to the "p38" nucleic acid under low stringency conditions, and/or
   (iv) "TRAF6" (SEQ ID No:189) or a functionally active derivative thereof, or a functionally active fragment thereof, or a homolog thereof, or a variant of "TRAF6" encoded by a nucleic acid that hybridizes to the "TRAF6" nucleic acid under low stringency conditions, and/or
   (v) "PROTEASOME SUBUNIT β TYPE 5 PRECURSOR" (SEQ ID No:190) or a functionally active derivative thereof, or a functionally active fragment thereof, or a homolog thereof, or a variant of "PROTEASOME SUBUNIT β TYPE 5 PRECURSOR" encoded by a nucleic acid that hybridizes to the "PROTEASOME SUBUNIT β TYPE 5 PRECURSOR" nucleic acid under low stringency conditions, and/or
   (vi) "26S PROTEASOME NON-ATPASE REGULATORY SUBUNIT 8" (SEQ ID No:191) or a functionally active derivative thereof, or a functionally active fragment thereof, or a homolog thereof, or a variant of "26S PROTEASOME NON-ATPASE REGULATORY SUBUNIT 8" encoded by a nucleic acid that hybridizes to the "26S PROTEASOME NON-ATPASE REGULATORY SUBUNIT 8" nucleic acid under low stringency conditions, and/or
   (vii) "26S PROTEASE REGULATORY SUBUNIT 6A" (SEQ ID No:192) or a functionally active derivative thereof, or a functionally active fragment thereof, or a homolog thereof, or a variant of "26S PROTEASE REGULATORY SUBUNIT 6A" encoded by a nucleic acid that hybridizes to the "26S PROTEASE REGULATORY SUBUNIT 6A" nucleic acid under low stringency conditions, and/or
   (viii) "PROTEASOME SUBUNIT α TYPE 1" (SEQ ID No:193) or a functionally active derivative thereof, or a functionally active fragment thereof, or a homolog thereof, or a variant of "PROTEASOME SUBUNIT α TYPE 1" encoded by a nucleic acid that hybridizes to the "PROTEASOME SUBUNIT α TYPE 1" nucleic acid under low stringency conditions, and/or
   (ix) "PROTEASOME SUBUNIT α TYPE 4" (SEQ ID No:194) or a functionally active derivative thereof, or a functionally active fragment thereof, or a homolog thereof, or a variant of "PROTEASOME SUBUNIT α TYPE 4" encoded by a nucleic acid that hybridizes to the "PROTEASOME SUBUNIT α TYPE 4" nucleic acid under low stringency conditions, and/or
   (x) "PROTEASOME (PROSOME, MACROPAIN) 26S SUBUNIT, NON-ATPASE, 1" (SEQ ID No:195) or a functionally active derivative thereof, or a functionally active fragment thereof, or a homolog thereof, or a variant of "PROTEASOME (PROSOME, MACROPAIN) 26S SUBUNIT, NON-ATPASE, 1" encoded by a nucleic acid that hybridizes to the "PROTEASOME (PROSOME, MACROPAIN) 26S SUBUNIT, NON-ATPASE, 1" nucleic acid under low stringency conditions, and/or
   (xi) "SIMILAR TO PROTEASE (PROSOME, MACROPAIN) 26S SUBUNIT, ATPASE 1" (SEQ ID No:196) or a functionally active derivative thereof, or a functionally active fragment thereof, or a homolog thereof, or a variant of "SIMILAR TO PROTEASE (PROSOME, MACROPAIN) 26S SUBUNIT, ATPASE 1" encoded by a nucleic acid that hybridizes to the "SIMILAR TO PROTEASE (PROSOME, MACROPAIN) 26S SUBUNIT, ATPASE 1" nucleic acid under low stringency conditions, and/or
   (xii) "HYPOTHETICAL PROTEIN FLJ20552" (SEQ ID No:197) or a functionally active derivative thereof, or a functionally active fragment thereof, or a homolog thereof, or a variant of "HYPOTHETICAL PROTEIN FLJ20552" encoded by a nucleic acid that hybridizes to the "HYPOTHETICAL PROTEIN FLJ20552" nucleic acid under low stringency conditions, and/or
   (xiii) "26S PROTEASOME REGULATORY SUBUNIT S2" (SEQ ID No: 198) or a functionally active derivative thereof, or a functionally active fragment thereof, or a homolog thereof, or a variant of "26S PROTEASOME REGULATORY SUBUNIT S2" encoded by a nucleic acid that hybridizes to the "26S PROTEASOME REGULATORY SUBUNIT S2" nucleic acid under low stringency conditions, and/or
   (xiv) "26S PROTEASOME SUBUNIT P40.5" (SEQ ID No:199) or a functionally active derivative thereof, or a functionally active fragment thereof, or a homolog thereof, or a variant of "26S PROTEASOME SUBUNIT P40.5" encoded by a nucleic acid that hybridizes to the "26S PROTEASOME SUBUNIT P40.5" nucleic acid under low stringency conditions, and/or
   (xv) "26S PROTEASE REGULATORY SUBUNIT S10B" (SEQ ID No:200) or a functionally active derivative thereof, or a functionally active fragment thereof, or a homolog thereof, or a variant of "26S PROTEASE REGULATORY SUBUNIT S10B" encoded by a nucleic acid that hybridizes to the "26S PROTEASE REGULATORY SUBUNIT S10B" nucleic acid under low stringency conditions, and/or
   (xvi) "26S PROTEASE REGULATORY SUBUNIT 7" (SEQ ID No:201) or a functionally active derivative thereof, or a functionally active fragment thereof, or a homolog thereof, or a variant of "26S PROTEASE REGULATORY SUBUNIT 7" encoded by a nucleic acid that hybridizes to the "26S PROTEASE REGULATORY SUBUNIT 7" nucleic acid under low stringency conditions, and/or
   (xvii) "26S PROTEASOME SUBUNIT P55" (SEQ ID No:202) or a functionally active derivative thereof, or a functionally active fragment thereof, or a homolog thereof, or a variant of "26S PROTEASOME SUBUNIT P55" encoded by a nucleic acid that hybridizes to the "26S PROTEASOME SUBUNIT P55" nucleic acid under low stringency conditions, and/or
   (xviii) "VOLTAGE-GATED POTASSIUM CHANNEL PROTEIN KQT-LIKE 1" (SEQ ID No:203) or a functionally active derivative thereof, or a functionally active fragment thereof, or a homolog thereof, or a variant of "VOLTAGE-GATED POTASSIUM CHANNEL PROTEIN KQT-LIKE 1" encoded by a nucleic acid that hybridizes to the "VOLTAGE-GATED POTASSIUM CHANNEL PROTEIN KQT-LIKE 1" nucleic acid under low stringency conditions, and/or
   (xix) "26S PROTEASOME NON-ATPASE REGULATORY SUBUNIT 6" (SEQ ID No:204) or a functionally active derivative thereof, or a functionally active fragment thereof, or a homolog thereof, or a variant of "26S PROTEASOME NON-ATPASE REGULATORY SUBUNIT 6" encoded by a nucleic acid that hybridizes to the "26S PROTEASOME NON-ATPASE REGULATORY SUBUNIT 6" nucleic acid under low stringency conditions, and/or
   (xx) "26S PROTEASOME REGULATORY SUBUNIT S12" (SEQ ID No:205) or a functionally active derivative thereof, or a functionally active fragment thereof, or a homolog thereof, or a variant of "26S PROTEASOME REGULATORY SUBUNIT S12" encoded by a nucleic acid that hybridizes to the "26S PROTEASOME REGULATORY SUBUNIT S12" nucleic acid under low stringency conditions, and/or
   (xxi) "26S PROTEASOME REGULATORY SUBUNIT S3" (SEQ ID No:206) or a functionally active derivative thereof, or a functionally active fragment thereof, or a homolog thereof, or a variant of "26S PROTEASOME REGULATORY SUBUNIT S3" encoded by a nucleic acid that hybridizes to the "26S PROTEASOME REGULATORY SUBUNIT S3" nucleic acid under low stringency conditions, and/or
   (xxii) "26S PROTEASE REGULATORY SUBUNIT 4" (SEQ ID No:207) or a functionally active derivative thereof, or a functionally active fragment thereof, or a homolog thereof, or a variant of "26S PROTEASE REGULATORY SUBUNIT 4" encoded by a nucleic acid that hybridizes to the "26S PROTEASE REGULATORY SUBUNIT 4" nucleic acid under low stringency conditions, and/or
   (xxiii) "PROTEASOME SUBUNIT β TYPE 2" (SEQ ID No:208) or a functionally active derivative thereof, or a functionally active fragment thereof, or a homolog thereof, or a variant of "PROTEASOME SUBUNIT β TYPE 2" encoded by a nucleic acid that hybridizes to the "PROTEASOME SUBUNIT β TYPE 2" nucleic acid under low stringency conditions, and/or
   (xxiv) "26S PROTEASOME NON-ATPASE REGULATORY SUBUNIT 11" (SEQ ID No:209) or a functionally active derivative thereof, or a functionally active fragment thereof, or a homolog thereof, or a variant of "26S PROTEASOME NON-ATPASE REGULATORY SUBUNIT 1" encoded by a nucleic acid that hybridizes to the "26S PROTEASOME NON-ATPASE REGULATORY SUBUNIT 1" nucleic acid under low stringency conditions, and/or
   (xxv) "OXYSTEROL BINDING PROTEIN-RELATED PROTEIN 8" (SEQ ID No:55) or a functionally active derivative thereof, or a functionally active fragment thereof, or a homolog thereof, or a variant of "OXYSTEROL BINDING PROTEIN-RELATED PROTEIN 8" encoded by a nucleic acid that hybridizes to the "OXYSTEROL BINDING PROTEIN-RELATED PROTEIN 8" nucleic acid under low stringency conditions, and/or
   (xxvi) "26S PROTEASOME REGULATORY SUBUNIT S5A" (SEQ ID No:210) or a functionally active derivative thereof, or a functionally active fragment thereof, or a homolog thereof, or a variant of "26S PROTEASOME REGULATORY SUBUNIT S5A" encoded by a nucleic acid that hybridizes to the "26S PROTEASOME REGULATORY SUBUNIT S5A" nucleic acid under low stringency conditions, and/or
   (xxvii) "PROTEASOME SUBUNIT β TYPE 6 PRECURSOR" (SEQ ID No:211) or a functionally active derivative thereof, or a functionally active fragment thereof, or a homolog thereof, or a variant of "PROTEASOME SUBUNIT β TYPE 6 PRECURSOR" encoded by a nucleic acid that hybridizes to the "PROTEASOME SUBUNIT β TYPE 6 PRECURSOR" nucleic acid under low stringency conditions, and/or
   (xxviii) "26S PROTEASOME SUBUNIT 9" (SEQ ID No:212) or a functionally active derivative thereof, or a functionally active fragment thereof, or a homolog thereof, or a variant of "26S PROTEASOME SUBUNIT 9" encoded by a nucleic acid that hybridizes to the "26S PROTEASOME SUBUNIT 9" nucleic acid under low stringency conditions, and/or
   (xxix) "26S PROTEASOME NON-ATPASE REGULATORY SUBUNIT 7" (SEQ ID No:213) or a functionally active derivative thereof, or a functionally active fragment thereof, or a homolog thereof, or a variant of "26S PROTEASOME NON-ATPASE REGULATORY SUBUNIT 7" encoded by a nucleic acid that hybridizes to the "26S PROTEASOME NON-ATPASE REGULATORY SUBUNIT 7" nucleic acid under low stringency conditions, and/or
   (xxx) "HSPC121" (SEQ ID No:214) or a functionally active derivative thereof, or a functionally active fragment thereof, or a homolog thereof, or a variant of "HSPC121" encoded by a nucleic acid that hybridizes to the "HSPC121" nucleic acid under low stringency conditions, and/or
   (xxxi) "26S PROTEASOME NON-ATPASE REGULATORY SUBUNIT 1" (SEQ ID No:215) or a functionally active derivative thereof, or a functionally active fragment thereof, or a homolog thereof, or a variant of "26S PROTEASOME NON-ATPASE REGULATORY SUBUNIT 1" encoded by a nucleic acid that hybridizes to the "26S PROTEASOME NON-ATPASE REGULATORY SUBUNIT 1" nucleic acid under low stringency conditions, and/or
   (xxxii) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions, and/or
   (xxxiii) "PROTEASOME SUBUNIT α TYPE 7" (SEQ ID No:216) or a functionally active derivative thereof, or a functionally active fragment thereof, or a homolog thereof, or a variant of "PROTEASOME SUBUNIT α TYPE 7" encoded by a nucleic acid that hybridizes to the "PROTEASOME SUBUNIT α TYPE 7" nucleic acid under low stringency conditions, and/or
   (xxxiv) "26S PROTEASE REGULATORY SUBUNIT 8" (SEQ ID No:217) or a functionally active derivative thereof, or a functionally active fragment thereof, or a homolog thereof, or a variant of "26S PROTEASE REGULATORY SUBUNIT 8" encoded by a nucleic acid that hybridizes to the "26S PROTEASE REGULATORY SUBUNIT 8" nucleic acid under low stringency conditions, and/or
   (xxxv) "PROTEASOME SUBUNIT α TYPE 6" (SEQ ID No:218) or a functionally active derivative thereof, or a functionally active fragment thereof, or a homolog thereof, or a variant of "PROTEASOME SUBUNIT α TYPE 6" encoded by a nucleic acid that hybridizes to the "PROTEASOME SUBUNIT α TYPE 6" nucleic acid under low stringency conditions,
   is present in the complex.
41. The complex of any one of No. 1 - 8, or proteins of No. 13 or the antibody or fragment of No. 17, for use in a method of diagnosing a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
42. A method for treating or preventing a disease or disorder characterized by an aberrant amount of, activity or component composition of or intracellular localization of, the complex of anyone of No. 1 - 8, comprising administering to a subject in need of such treatment or prevention a therapeutically effective amount of one or more molecules that modulate the amount of, Ubiquitination activity or IKK kinase activity, or protein components of, said complex.
43. The method according to No. 42, wherein said disease or disorder involves decreased levels of the amount or activity of said complex.
44. The method according to No. 42 , wherein said disease or disorder involves increased levels of the amount or activity of said complex.
45. Complex of any of No. 1 - 8 and/or protein selected from the following proteins
   (i) "RIP2 " (SEQ ID No:187) or a functionally active derivative thereof, or a functionally active fragment thereof, or a homolog thereof, or a variant of "RIP2" encoded by a nucleic acid that hybridizes to the "RIP2" nucleic acid under low stringency conditions,
   (ii) "TAB1 TAK1-BINDING PROTEIN 1" (SEQ ID No:188) or a functionally active derivative thereof, or a functionally active fragment thereof, or a homolog thereof, or a variant of "TAB1 TAK1 -BINDING PROTIEN 1" encoded by a nucleic acid that hybridizes to the "TAB1 TAK1-BINDING PROTEIN 1" nucleic acid under low stringency conditions,
   (iii) "p38" (SEQ ID No:118) or a functionally active derivative thereof, or a functionally active fragment thereof, or a homolog thereof, or a variant of "p38" encoded by a nucleic acid that hybridizes to the "p38" nucleic acid under low stringency conditions,
   (iv) "TRAF6" (SEQ ID No:189) or a functionally active derivative thereof, or a functionally active fragment thereof, or a homolog thereof, or a variant of "TRAF6" encoded by a nucleic acid that hybridizes to the "TRAF6" nucleic acid under low stringency conditions,
   (v) "PROTEASOME SUBUNIT β TYPE 5 PRECURSOR" (SEQ ID No:190) or a functionally active derivative thereof, or a functionally active fragment thereof, or a homolog thereof, or a variant of "PROTEASOME SUBUNIT β TYPE 5 PRECURSOR" encoded by a nucleic acid that hybridizes to the "PROTEASOME SUBUNIT β TYPE 5 PRECURSOR" nucleic acid under low stringency conditions,
   (vi) "26S PROTEASOME NON-ATPASE REGULATORY SUBUNIT 8" (SEQ ID No:191) or a functionally active derivative thereof, or a functionally active fragment thereof, or a homolog thereof, or a variant of "26S PROTEASOME NON-ATPASE REGULATORY SUBUNIT 8" encoded by a nucleic acid that hybridizes to the "26S PROTEASOME NON-ATPASE REGULATORY SUBUNIT 8" nucleic acid under low stringency conditions,
   (vii) "26S PROTEASE REGULATORY SUBUNIT 6A" (SEQ ID No:192) or a functionally active derivative thereof, or a functionally active fragment thereof, or a homolog thereof, or a variant of "26S PROTEASE REGULATORY SUBUNIT 6A" encoded by a nucleic acid that hybridizes to the "26S PROTEASE REGULATORY SUBUNIT 6A" nucleic acid under low stringency conditions,
   (viii) "PROTEASOME SUBUNIT α TYPE 1" (SEQ ID No:193) or a functionally active derivative thereof, or a functionally active fragment thereof, or a homolog thereof, or a variant of "PROTEASOME SUBUNIT α TYPE 1" encoded by a nucleic acid that hybridizes to the "PROTEASOME SUBUNIT α TYPE 1" nucleic acid under low stringency conditions,
   (ix) "PROTEASOME SUBUNIT α TYPE 4" (SEQ ID No:194) or a functionally active derivative thereof, or a functionally active fragment thereof, or a homolog thereof, or a variant of "PROTEASOME SUBUNIT α TYPE 4" encoded by a nucleic acid that hybridizes to the "PROTEASOME SUBUNIT α TYPE 4" nucleic acid under low stringency conditions,
   (x) "PROTEASOME (PROSOME, MACROPAIN) 26S SUBUNIT, NON-ATPASE, 1" (SEQ ID No:195) or a functionally active derivative thereof, or a functionally active fragment thereof, or a homolog thereof, or a variant of "PROTEASOME (PROSOME, MACROPAIN) 26S SUBUNIT, NON-ATPASE, 1" encoded by a nucleic acid that hybridizes to the "PROTEASOME (PROSOME, MACROPAIN) 26S SUBUNIT, NON-ATPASE, 1" nucleic acid under low stringency conditions,
   (xi) "SIMILAR TO PROTEASE (PROSOME, MACROPAIN) 26S SUBUNIT, ATPASE 1" (SEQ ID No:196) or a functionally active derivative thereof, or a functionally active fragment thereof, or a homolog thereof, or a variant of "SIMILAR TO PROTEASE (PROSOME, MACROPAIN) 26S SUBUNIT, ATPASE 1" encoded by a nucleic acid that hybridizes to the "SIMILAR TO PROTEASE (PROSOME, MACROPAIN) 26S SUBUNIT, ATPASE 1" nucleic acid under low stringency conditions,
   (xii) "HYPOTHETICAL PROTEIN FLJ20552" (SEQ ID No:197) or a functionally active derivative thereof, or a functionally active fragment thereof, or a homolog thereof, or a variant of "HYPOTHETICAL PROTEIN FLJ20552" encoded by a nucleic acid that hybridizes to the "HYPOTHETICAL PROTEIN FLJ20552" nucleic acid under low stringency conditions,
   (xiii) "26S PROTEASOME REGULATORY SUBUNIT S2" (SEQ ID No:198) or a functionally active derivative thereof, or a functionally active fragment thereof, or a homolog thereof, or a variant of "26S PROTEASOME REGULATORY SUBUNIT S2" encoded by a nucleic acid that hybridizes to the "26S PROTEASOME REGULATORY SUBUNIT S2" nucleic acid under low stringency conditions,
   (xiv) "26S PROTEASOME SUBUNIT P40.5" (SEQ ID No:199) or a functionally active derivative thereof, or a functionally active fragment thereof, or a homolog thereof, or a variant of "26S PROTEASOME SUBUNIT P40.5" encoded by a nucleic acid that hybridizes to the "26S PROTEASOME SUBUNIT P40.5" nucleic acid under low stringency conditions,
   (xv) "26S PROTEASE REGULATORY SUBUNIT S10B" (SEQ ID No:200) or a functionally active derivative thereof, or a functionally active fragment thereof, or a homolog thereof, or a variant of "26S PROTEASE REGULATORY SUBUNIT S10B" encoded by a nucleic acid that hybridizes to the "26S PROTEASE REGULATORY SUBUNIT S10B" nucleic acid under low stringency conditions,
   (xvi) "26S PROTEASE REGULATORY SUBUNIT 7" (SEQ ID No:201) or a functionally active derivative thereof, or a functionally active fragment thereof, or a homolog thereof, or a variant of "26S PROTEASE REGULATORY SUBUNIT 7" encoded by a nucleic acid that hybridizes to the "26S PROTEASE REGULATORY SUBUNIT 7" nucleic acid under low stringency conditions,
   (xvii) "26S PROTEASOME SUBUNIT P55" (SEQ ID No:202) or a functionally active derivative thereof, or a functionally active fragment thereof, or a homolog thereof, or a variant of "26S PROTEASOME SUBUNIT P55" encoded by a nucleic acid that hybridizes to the "26S PROTEASOME SUBUNIT P55" nucleic acid under low stringency conditions,
   (xviii) "VOLTAGE-GATED POTASSIUM CHANNEL PROTEIN KQT-LIKE 1" (SEQ ID No:203) or a functionally active derivative thereof, or a functionally active fragment thereof, or a homolog thereof, or a variant of "VOLTAGE-GATED POTASSIUM CHANNEL PROTEIN KQT-LIKE 1" encoded by a nucleic acid that hybridizes to the "VOLTAGE-GATED POTASSIUM CHANNEL PROTEIN KQT-LIKE 1" nucleic acid under low stringency conditions,
   (xix) "26S PROTEASOME NON-ATPASE REGULATORY SUBUNIT 6" (SEQ ID No:204) or a functionally active derivative thereof, or a functionally active fragment thereof, or a homolog thereof, or a variant of "26S PROTEASOME NON-ATPASE REGULATORY SUBUNIT 6" encoded by a nucleic acid that hybridizes to the "26S PROTEASOME NON-ATPASE REGULATORY SUBUNIT 6" nucleic acid under low stringency conditions,
   (xx) "26S PROTEASOME REGULATORY SUBUNIT S12" (SEQ ID No:205) or a functionally active derivative thereof, or a functionally active fragment thereof, or a homolog thereof, or a variant of "26S PROTEASOME REGULATORY SUBUNIT S12" encoded by a nucleic acid that hybridizes to the "26S PROTEASOME REGULATORY SUBUNIT S12" nucleic acid under low stringency conditions,
   (xxi) "26S PROTEASOME REGULATORY SUBUNIT S3" (SEQ ID No:206) or a functionally active derivative thereof, or a functionally active fragment thereof, or a homolog thereof, or a variant of "26S PROTEASOME REGULATORY SUBUNIT S3" encoded by a nucleic acid that hybridizes to the "26S PROTEASOME REGULATORY SUBUNIT S3" nucleic acid under low stringency conditions,
   (xxii) "26S PROTEASE REGULATORY SUBUNIT 4" (SEQ ID No:207) or a functionally active derivative thereof, or a functionally active fragment thereof, or a homolog thereof, or a variant of "26S PROTEASE REGULATORY SUBUNIT 4" encoded by a nucleic acid that hybridizes to the "26S PROTEASE REGULATORY SUBUNIT 4" nucleic acid under low stringency conditions,
   (xxiii) "PROTEASOME SUBUNIT β TYPE 2" (SEQ ID No:208) or a functionally active derivative thereof, or a functionally active fragment thereof, or a homolog thereof, or a variant of "PROTEASOME SUBUNIT β TYPE 2" encoded by a nucleic acid that hybridizes to the "PROTEASOME SUBUNIT β TYPE 2" nucleic acid under low stringency conditions,
   (xxiv) "26S PROTEASOME NON-ATPASE REGULATORY SUBUNIT 11" (SEQ ID No:209) or a functionally active derivative thereof, or a functionally active fragment thereof, or a homolog thereof, or a variant of "26S PROTEASOME NON-ATPASE REGULATORY SUBUNIT 1" encoded by a nucleic acid that hybridizes to the "26S PROTEASOME NON-ATPASE REGULATORY SUBUNIT 1" nucleic acid under low stringency conditions,
   (xxv) "OXYSTEROL BINDING PROTEIN-RELATED PROTEIN 8" (SEQ ID No:55) or a functionally active derivative thereof, or a functionally active fragment thereof, or a homolog thereof, or a variant of "OXYSTEROL BINDING PROTEIN-RELATED PROTEIN 8" encoded by a nucleic acid that hybridizes to the "OXYSTEROL BINDING PROTEIN-RELATED PROTEIN 8" nucleic acid under low stringency conditions,
   (xxvi) "26S PROTEASOME REGULATORY SUBUNIT S5A" (SEQ ID No:210) or a functionally active derivative thereof, or a functionally active fragment thereof, or a homolog thereof, or a variant of "26S PROTEASOME REGULATORY SUBUNIT S5A" encoded by a nucleic acid that hybridizes to the "26S PROTEASOME REGULATORY SUBUNIT S5A" nucleic acid under low stringency conditions,
   (xxvii) "PROTEASOME SUBUNIT β TYPE 6 PRECURSOR" (SEQ ID No:211) or a functionally active derivative thereof, or a functionally active fragment thereof, or a homolog thereof, or a variant of "PROTEASOME SUBUNIT β TYPE 6 PRECURSOR" encoded by a nucleic acid that hybridizes to the "PROTEASOME SUBUNIT β TYPE 6 PRECURSOR" nucleic acid under low stringency conditions,
   (xxviii) "26S PROTEASOME SUBUNIT 9" (SEQ ID No:212) or a functionally active derivative thereof, or a functionally active fragment thereof, or a homolog thereof, or a variant of "26S PROTEASOME SUBUNIT 9" encoded by a nucleic acid that hybridizes to the "26S PROTEASOME SUBUNIT 9" nucleic acid under low stringency conditions,
   (xxix) "26S PROTEASOME NON-ATPASE REGULATORY SUBUNIT 7" (SEQ ID No:213) or a functionally active derivative thereof, or a functionally active fragment thereof, or a homolog thereof, or a variant of "26S PROTEASOME NON-ATPASE REGULATORY SUBUNIT 7" encoded by a nucleic acid that hybridizes to the "26S PROTEASOME NON-ATPASE REGULATORY SUBUNIT 7" nucleic acid under low stringency conditions,
   (xxx) "HSPC121" (SEQ ID No:214) or a functionally active derivative thereof, or a functionally active fragment thereof, or a homolog thereof, or a variant of "HSPC121" encoded by a nucleic acid that hybridizes to the "HSPC121" nucleic acid under low stringency conditions,
   (xxxi) "26S PROTEASOME NON-ATPASE REGULATORY SUBUNIT 1" (SEQ ID No:215) or a functionally active derivative thereof, or a functionally active fragment thereof, or a homolog thereof, or a variant of "26S PROTEASOME NON-ATPASE REGULATORY SUBUNIT 1" encoded by a nucleic acid that hybridizes to the "26S PROTEASOME NON-ATPASE REGULATORY SUBUNIT 1" nucleic acid under low stringency conditions,
   (xxxii) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,
   (xxxiii) "PROTEASOME SUBUNIT α TYPE 7" (SEQ ID No:216) or a functionally active derivative thereof, or a functionally active fragment thereof, or a homolog thereof, or a variant of "PROTEASOME SUBUNIT α TYPE 7" encoded by a nucleic acid that hybridizes to the "PROTEASOME SUBUNIT α TYPE 7" nucleic acid under low stringency conditions,
   (xxxiv) "26S PROTEASE REGULATORY SUBUNIT 8" (SEQ ID No:217) or a functionally active derivative thereof,or a functionally active fragement thereof, or a homolog thereof, or a variant of "26S PROTEASE REGULATORY SUBUNIT 8" that hybridizes to the "26S PROTEASE REGULATORY SUBUNIT 8" nucleic acid under low stringency conditions, and
   (xxxv) "PROTEASOME SUBUNIT α TYPE 6" (SEQ ID No:218) or a functionally active derivative thereof, or a functionally active fragment thereof, or a homolog thereof, or a variant of "PROTEASOME SUBUNIT α TYPE 6" encoded by a nucleic acid that hybridizes to the "PROTEASOME SUBUNIT α TYPE 6" nucleic acid under low stringency conditions,
as a target for an active agent of a pharmaceutical, preferably a drug target in the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.

The present invention further relates to the I-TRAF protein complex
1. A protein complex selected from complex (I) and comprising
   (a) at least one first protein selected from the group consisting of:
      (i) "TRAF1" (SEQ ID No:14) or a functionally active derivative thereof, or a functionally active fragment thereof, or a homolog thereof, or a variant of "TRAF1" encoded by a nucleic acid that hybridizes to the "TRAF1" nucleic acid under low stringency conditions,
      (ii) "IKK ε" (SEQ ID No:79) or a functionally active derivative thereof, or a functionally active fragment thereof, or a homolog thereof, or a variant of "IKK ε" encoded by a nucleic acid that hybridizes to the "IKK ε" nucleic acid under low stringency conditions,
      (iii) "TRAF3" (SEQ ID No:219) or a functionally active derivative thereof, or a functionally active fragment thereof, or a homolog thereof, or a variant of "TRAF3" encoded by a nucleic acid that hybridizes to the "TRAF3" nucleic acid under low stringency conditions,
      (iv) "TANK BINDING KINASE TBK1" (SEQ ID No:115) or a functionally active derivative thereof, or a functionally active fragment thereof, or a homolog thereof, or a variant of "TANK BINDING KINASE TBK1" encoded by a nucleic acid that hybridizes to the "TANK BINDING KINASE TBK1" nucleic acid under low stringency conditions,
      (v) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions, and
      (vi) "I-TRAF" (SEQ ID No:224) or a functionally active derivative thereof, or a functionally active fragment thereof, or a homolog thereof, or a variant of "I-TRAF" encoded by a nucleic acid that hybridizes to the "I-TRAF" nucleic acid under low stringency conditions,
      and
   (b) at least one second protein, which second protein is selected from the group consisting of:
      (i) "Keratin9" (SEQ ID No:220) or a functionally active derivative thereof, or a functionally active fragment thereof, or a homolog thereof, or a variant of "Keratin9" encoded by a nucleic acid that hybridizes to the "Keratin9" nucleic acid under low stringency conditions,
      (ii) "Keratin16" (SEQ ID No:221) or a functionally active derivative thereof, or a functionally active fragment thereof, or a homolog thereof, or a variant of "Keratin16" encoded by a nucleic acid that hybridizes to the "Keratin16" nucleic acid under low stringency conditions,
      (iii) "LYSYL-TRNA SYNTHETASE" (SEQ ID No:222) or a functionally active derivative thereof, or a functionally active fragment thereof, or a homolog thereof, or a variant of "LYSYL-TRNA SYNTHETASE" encoded by a nucleic acid that hybridizes to the "LYSYL-TRNA SYNTHETASE" nucleic acid under low stringency conditions, and
      (iv) "CDNA FLJ13715 FIS, CLONE PLACE2000404, MODERATELY SIMILAR TO PROBABLE LEUCYL-TRNA SYNTHETASE" (SEQ ID No:223) or a functionally active derivative thereof, or a functionally active fragment thereof, or a homolog thereof, or a variant of "CDNA FLJ13715 FIS, CLONE PLACE2000404, MODERATELY SIMILAR TO PROBABLE LEUCYL-TRNA SYNTHETASE" encoded by a nucleic acid that hybridizes to the "CDNA FLJ13715 FIS, CLONE PLACE2000404, MODERATELY SIMILAR TO PROBABLE LEUCYL-TRNA SYNTHETASE" nucleic acid under low stringency conditions;
   and a complex (11) comprising at least two of said second proteins,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1 % SDS for 1.5 hours at 60°C.
2. The protein complex according to claim 1 wherein the first protein is the protein "I-TRAF" (SEQ ID No:224), or a functionally active derivative thereof, or a functionally active fragment thereof, or a homolog thereof, or a variant of "I-TRAF" encoded by a nucleic acid that hybridizes to the "I-TRAF" nucleic acid under low stringency conditions.
3. The protein complex according to No. 1 selected from complex (I) and comprising the following proteins:
   (i) "I-TRAF" (SEQ ID No:224) or a functionally active derivative thereof, or a functionally active fragment thereof, or a homolog thereof, or a variant of "I-TRAF" encoded by a nucleic acid that hybridizes to the "I-TRAF" nucleic acid under low stringency conditions,
   (ii) "TRAF3" (SEQ ID No:219) or a functionally active derivative thereof, or a functionally active fragment thereof, or a homolog thereof, or a variant of "TRAF3" encoded by a nucleic acid that hybridizes to the "TRAF3" nucleic acid under low stringency conditions,
   (iii) "TANK BINDING KINASE TBK1" (SEQ ID No:115) or a functionally active derivative thereof, or a functionally active fragement thereof, or a homolog thereof, or a variant of "TANK BINDING KINASE TBK1" that hybridizes to the "TANK BINDING KINASE TBK1" nucleic acid under low stringency conditions, and
   (iv) "TRAF2" (SEQ ID No: 15) or a functionally active derivative thereof, or a functionally active fragment thereof, or a homolog thereof, or a variant of "TRAF2" encoded by a nucleic acid that hybridizes to the "TRAF2" nucleic acid under low stringency conditions; and a protein complex selected from complex (II) and comprising the following proteins:

   (i) "I-TRAF" (SEQ ID No:224) or a functionally active derivative thereof, or a functionally active fragment thereof, or a homolog thereof, or a variant of "I-TRAF" encoded by a nucleic acid that hybridizes to the "I-TRAF" nucleic acid under low stringency conditions,
   (ii) "keratin9" (SEQ ID No:220) or a functionally active derivative thereof, or a functionally active fragment thereof, or a homolog thereof, or a variant of "keratin9" encoded by a nucleic acid that hybridizes to the "keratin9" nucleic acid under low stringency conditions,
   (iii) "keratin16" (SEQ ID No:221) or a functionally active derivative thereof, or a functionally active fragment thereof, or a homolog thereof, or a variant of "keratin16" encoded by a nucleic acid that hybridizes to the "keratin16" nucleic acid under low stringency conditions,
   (iv) "TANK BINDING KINASE TBK1" (SEQ ID No:115) or a functionally active derivative thereof, or a functionally active fragment thereof, or a homolog thereof, or a variant of "TANK BINDING KINASE TBK1" encoded by a nucleic acid that hybridizes to the "TANK BINDING KINASE TBK1" nucleic acid under low stringency conditions,
   (v) "TRAF2" (SEQ ID No: 15) or a functionally active derivative thereof,or a functionally active fragement thereof, or a homolog thereof, or a variant of "TRAF2" that hybridizes to the "TRAF2" nucleic acid under low stringency conditions, and
   (vi) "TRAF3" (SEQ ID No:219) or a functionally active derivative thereof, or a functionally active fragment thereof, or a homolog thereof, or a variant of "TRAF3" encoded by a nucleic acid that hybridizes to the "TRAF3" nucleic acid under low stringency conditions; and a protein complex selected from complex (III) and comprising the following proteins:

   (i) "CDNA FLJ13715 FIS, CLONE PLACE2000404, MODERATELY SIMILAR TO PROBABLE LEUCYL-TRNA SYNTHETASE" (SEQ ID No:223) or a functionally active derivative thereof, or a functionally active fragment thereof, or a homolog thereof, or a variant of "CDNA FLJ13715 FIS, CLONE PLACE2000404, MODERATELY SIMILAR TO PROBABLE LEUCYL-TRNA SYNTHETASE" encoded by a nucleic acid that hybridizes to the "CDNA FLJ13715 FIS, CLONE PLACE2000404, MODERATELY SIMILAR TO PROBABLE LEUCYL-TRNA SYNTHETASE" nucleic acid under low stringency conditions,
   (ii) "I-TRAF" (SEQ ID No:224) or a functionally active derivative thereof, or a functionally active fragment thereof, or a homolog thereof, or a variant of "I-TRAF" encoded by a nucleic acid that hybridizes to the "I-TRAF" nucleic acid under low stringency conditions,
   (iii) "LYSYL-TRNA SYNTHETASE" (SEQ ID No:222) or a functionally active derivative thereof, or a functionally active fragment thereof, or a homolog thereof, or a variant of "LYSYL-TRNA SYNTHETASE" encoded by a nucleic acid that hybridizes to the "LYSYL-TRNA SYNTHETASE" nucleic acid under low stringency conditions,
   (iv) "TANK BINDING KINASE TBK1" (SEQ ID No:115) or a functionally active derivative thereof, or a functionally active fragment thereof, or a homolog thereof, or a variant of "TANK BINDING KINASE TBK1" encoded by a nucleic acid that hybridizes to the "TANK BINDING KINASE TBK1" nucleic acid under low stringency conditions,
   (v) "TRAF2" (SEQ ID No:15) or a functionally active derivative thereof,or a functionally active fragement thereof, or a homolog thereof, or a variant of "TRAF2" that hybridizes to the "TRAF2" nucleic acid under low stringency conditions, and
   (vi) "TRAF3" (SEQ ID No:219) or a functionally active derivative thereof, or a functionally active fragment thereof, or a homolog thereof, or a variant of "TRAF3" encoded by a nucleic acid that hybridizes to the "TRAF3" nucleic acid under low stringency conditions; and a protein complex selected from complex (IV) and comprising the following proteins:

   (i) "TRAF1" (SEQ ID No:14) or a functionally active derivative thereof, or a functionally active fragment thereof, or a homolog thereof, or a variant of "TRAF1" encoded by a nucleic acid that hybridizes to the "TRAF1" nucleic acid under low stringency conditions,
   (ii) "IKK ε" (SEQ ID No:79) or a functionally active derivative thereof, or a functionally active fragment thereof, or a homolog thereof, or a variant of "IKK ε" encoded by a nucleic acid that hybridizes to the "IKK ε" nucleic acid under low stringency conditions,
   (iii) "TRAF3" (SEQ ID No:219) or a functionally active derivative thereof, or a functionally active fragment thereof, or a homolog thereof, or a variant of "TRAF3" encoded by a nucleic acid that hybridizes to the "TRAF3" nucleic acid under low stringency conditions,
   (iv) "TANK BINDING KINASE TBK1" (SEQ ID No:115) or a functionally active derivative thereof, or a functionally active fragment thereof, or a homolog thereof, or a variant of "TANK BINDING KINASE TBK1" encoded by a nucleic acid that hybridizes to the "TANK BINDING KINASE TBK1" nucleic acid under low stringency conditions,
   (v) "keratin9" (SEQ ID No:220) or a functionally active derivative thereof, or a functionally active fragment thereof, or a homolog thereof, or a variant of "keratin9" encoded by a nucleic acid that hybridizes to the "keratin9" nucleic acid under low stringency conditions,
   (vi) "keratin16" (SEQ ID No:221) or a functionally active derivative thereof, or a functionally active fragment thereof, or a homolog thereof, or a variant of "keratin16" encoded by a nucleic acid that hybridizes to the "keratin16" nucleic acid under low stringency conditions,
   (vii) "LYSYL-TRNA SYNTHETASE" (SEQ ID No:222) or a functionally active derivative thereof, or a functionally active fragment thereof, or a homolog thereof, or a variant of "LYSYL-TRNA SYNTHETASE" encoded by a nucleic acid that hybridizes to the "LYSYL-TRNA SYNTHETASE" nucleic acid under low stringency conditions,
   (viii) "CDNA FLJ13715 FIS, CLONE PLACE2000404, MODERATELY SIMILAR TO PROBABLE LEUCYL-TRNA SYNTHETASE" (SEQ ID No:223) or a functionally active derivative thereof, or a functionally active fragment thereof, or a homolog thereof, or a variant of "CDNA FLJ13715 FIS, CLONE PLACE2000404, MODERATELY SIMILAR TO PROBABLE LEUCYL-TRNA SYNTHETASE" encoded by a nucleic acid that hybridizes to the "CDNA FLJ13715 FIS, CLONE PLACE2000404, MODERATELY SIMILAR TO PROBABLE LEUCYL-TRNA SYNTHETASE" nucleic acid under low stringency conditions,
   (ix) "TRAF2" (SEQ ID No:15) or a functionally active derivative thereof,or a functionally active fragement thereof, or a homolog thereof, or a variant of "TRAF2" that hybridizes to the "TRAF2" nucleic acid under low stringency conditions, and
   (x) "I-TRAF" (SEQ ID No:224) or a functionally active derivative thereof, or a functionally active fragment thereof, or a homolog thereof, or a variant of "I-TRAF" encoded by a nucleic acid that hybridizes to the "I-TRAF" nucleic acid under low stringency conditions.
4. The protein complex according to No. 1 selected from complex (I) comprising all but 1 - 3 of the following proteins:
   (i) "TRAF1" (SEQ ID No:14) or a functionally active derivative thereof, or a functionally active fragment thereof, or a homolog thereof, or a variant of "TRAF1" encoded by a nucleic acid that hybridizes to the "TRAF1" nucleic acid under low stringency conditions,
   (ii) "IKK ε" (SEQ ID No:79) or a functionally active derivative thereof, or a functionally active fragment thereof, or a homolog thereof, or a variant of "IKK ε" encoded by a nucleic acid that hybridizes to the "IKK ε" nucleic acid under low stringency conditions,
   (iii) "TRAF3" (SEQ ID No:219) or a functionally active derivative thereof, or a functionally active fragment thereof, or a homolog thereof, or a variant of "TRAF3" encoded by a nucleic acid that hybridizes to the "TRAF3" nucleic acid under low stringency conditions,
   (iv) "TANK BINDING KINASE TBK1" (SEQ ID No:115) or a functionally active derivative thereof, or a functionally active fragment thereof, or a homolog thereof, or a variant of "TANK BINDING KINASE TBK1" encoded by a nucleic acid that hybridizes to the "TANK BINDING KINASE TBK1" nucleic acid under low stringency conditions,
   (v) "keratin9" (SEQ ID No:220) or a functionally active derivative thereof, or a functionally active fragment thereof, or a homolog thereof, or a variant of "keratin9" encoded by a nucleic acid that hybridizes to the "keratin9" nucleic acid under low stringency conditions,
   (vi) "keratin16" (SEQ ID No:221) or a functionally active derivative thereof, or a functionally active fragment thereof, or a homolog thereof, or a variant of "keratin16" encoded by a nucleic acid that hybridizes to the "keratin16" nucleic acid under low stringency conditions,
   (vii) "LYSYL-TRNA SYNTHETASE" (SEQ ID No:222) or a functionally active derivative thereof, or a functionally active fragment thereof, or a homolog thereof, or a variant of "LYSYL-TRNA SYNTHETASE" encoded by a nucleic acid that hybridizes to the "LYSYL-TRNA SYNTHETASE" nucleic acid under low stringency conditions,
   (viii) "CDNA FLJ13715 FIS, CLONE PLACE2000404, MODERATELY SIMILAR TO PROBABLE LEUCYL-TRNA SYNTHETASE" (SEQ ID No:223) or a functionally active derivative thereof, or a functionally active fragment thereof, or a homolog thereof, or a variant of "CDNA FLJ13715 FIS, CLONE PLACE2000404, MODERATELY SIMILAR TO PROBABLE LEUCYL-TRNA SYNTHETASE" encoded by a nucleic acid that hybridizes to the "CDNA FLJ13715 FIS, CLONE PLACE2000404, MODERATELY SIMILAR TO PROBABLE LEUCYL-TRNA SYNTHETASE" nucleic acid under low stringency conditions,
   (ix) "TRAF2" (SEQ ID No:15) or a functionally active derivative thereof,or a functionally active fragement thereof, or a homolog thereof, or a variant of "TRAF2" that hybridizes to the "TRAF2" nucleic acid under low stringency conditions, and
   (x) "I-TRAF" (SEQ ID No:224) or a functionally active derivative thereof, or a functionally active fragment thereof, or a homolog thereof, or a variant of "I-TRAF" encoded by a nucleic acid that hybridizes to the "I-TRAF" nucleic acid under low stringency conditions; and a protein complex selected from complex (II) comprising all but 1 - 3 of the following proteins:

   (i) "TRAF3" (SEQ ID No:219) or a functionally active derivative thereof, or a functionally active fragment thereof, or a homolog thereof, or a variant of "TRAF3" encoded by a nucleic acid that hybridizes to the "TRAF3" nucleic acid under low stringency conditions,
   (ii) "TANK BINDING KINASE TBK1" (SEQ ID No:115) or a functionally active derivative thereof, or a functionally active fragment thereof, or a homolog thereof, or a variant of "TANK BINDING KINASE TBK1" encoded by a nucleic acid that hybridizes to the "TANK BINDING KINASE TBK1" nucleic acid under low stringency conditions,
   (iii) "keratin9" (SEQ ID No:220) or a functionally active derivative thereof, or a functionally active fragment thereof, or a homolog thereof, or a variant of "keratin9" encoded by a nucleic acid that hybridizes to the "keratin9" nucleic acid under low stringency conditions,
   (iv) "keratin16" (SEQ ID No:221) or a functionally active derivative thereof, or a functionally active fragment thereof, or a homolog thereof, or a variant of "keratin16" encoded by a nucleic acid that hybridizes to the "keratin16" nucleic acid under low stringency conditions,
   (v) "LYSYL-TRNA SYNTHETASE" (SEQ ID No:222) or a functionally active derivative thereof, or a functionally active fragment thereof, or a homolog thereof, or a variant of "LYSYL-TRNA SYNTHETASE" encoded by a nucleic acid that hybridizes to the "LYSYL-TRNA SYNTHETASE" nucleic acid under low stringency conditions,
   (vi) "CDNA FLJ13715 FIS, CLONE PLACE2000404, MODERATELY SIMILAR TO PROBABLE LEUCYL-TRNA SYNTHETASE" (SEQ ID No:223) or a functionally active derivative thereof, or a functionally active fragment thereof, or a homolog thereof, or a variant of "CDNA FLJ13715 FIS, CLONE PLACE2000404, MODERATELY SIMILAR TO PROBABLE LEUCYL-TRNA SYNTHETASE" encoded by a nucleic acid that hybridizes to the "CDNA FLJ13715 FIS, CLONE PLACE2000404, MODERATELY SIMILAR TO PROBABLE LEUCYL-TRNA SYNTHETASE" nucleic acid under low stringency conditions,
   (vii) "TRAF2" (SEQ ID No:15) or a functionally active derivative thereof,or a functionally active fragement thereof, or a homolog thereof, or a variant of "TRAF2" that hybridizes to the "TRAF2" nucleic acid under low stringency conditions, and
   (viii) "I-TRAF" (SEQ ID No:224) or a functionally active derivative thereof, or a functionally active fragment thereof, or a homolog thereof, or a variant of "I-TRAF" encoded by a nucleic acid that hybridizes to the "I-TRAF" nucleic acid under low stringency conditions.
5. The complex of any of No. 1-4 comprising a functionally active derivative of said first protein and/or a functionally active derivative of said second protein, wherein the functionally active derivative is a fusion protein comprising said first protein or said second protein fused to an amino acid sequence different from the first protein or second protein, respectively.
6. The complex of No. 5 wherein the functionally active derivative is a fusion protein comprising said first protein or said second protein fused to an affinity tag or label.
7. The complex of any of No. 1 - 4 comprising a fragment of said first protein and/or a fragment of said second protein, which fragment binds to another protein component of said complex.
8. The complex of any of No. 1 -7 that is involved in the I-TRAF binding activity.
9. A process for preparing complex of any of No. 1 - 8 and optionally the components thereof comprising the following steps:
   the expressing a protein (bait) of the complex, preferably a tagged protein, in a target cell, isolating the protein complex which is attached to the bait protein, and optionally disassociating the protein complex and isolating the individual complex members.
10. The process according to No. 9 wherein the tagged protein comprises two different tags which allow two separate affinity purification steps.
11. The process according to any of No. 9 - 10 wherein the two tags are separated by a cleavage site for a protease.
12. Component of the I-TRAF protein complex obtainable by a process according to any of No. 9 to 11.
13. Construct, preferably a vector construct, comprising at least two separate nucleic acid sequences each encoding a different protein, or a functionally active fragment or a functionally active derivative therof at least one of said proteins, or functionally active fragments or functionally active derivative therof being selected from the first group of proteins according to No. 1(a) and at least one of said proteines, or functionally active fragments of functionally active derivative thereof being selected from the second group of proteins according to No. 1(b)
14. Host cell containting a construct of No. 13 or containing several vectors each comprising at least the nucleic acid sequence encoding at least one of the proteins, or functionally active fragments or functionally active derivatives thereof selected from the first group of proteins according to No. 1(a) and the proteins, or functionally active fragments or functionally active derivatives thereof selected from the second group of proteins according to No. 1(b).
15. An antibody or a fragment of said antibody containing the binding domain thereof, selected from an antibody or fragment thereof, which binds the complex of any of No. 1-8 and which does not bind any of the proteins of said complex when uncomplexed and an antibody or a fragment of said antibody which binds to any of the proteins according to No..
16. A kit comprising in one or more container the complex of any of No. 1 - 8 optionally together with an antibody according to No. 15 and/or further components such as reagents and working instructions.
17. The kit according to No. 16 for processing a substrate of said complex.
18. A kit according to No. 16 for the diagnosis or prognosis of a disease or a disease risk, preferentially for a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
19. Array, in which at least a complex according to any of No. 1 - 8 and/or at least one antibody according to No. 15 is attached to a solid carrier.
20. A process for processing a physiological substrate of the complex comprising the step of bringing into contact a complex to any of No. 1 - 8 with said substrate, such that said substrate is processed.
21. A pharmaceutical composition comprising the protein complex of any of No. 1 to 8..
22. The pharmaceutical composition according to No. 21 for the treatment of diseases and disorders such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
23. A method for screening for a molecule that binds to the complex of anyone of No. 1 - 8. comprising the steps of
   (a) exposing said complex, or a cell or organism containing same to one or more candidate molecules; and
   (b) determinig whether said candidate molecule is bound to the complex or protein.
24. A method for screening for a molecule that modulates directly or indirectly the function, activity, composition or formation of the complex of any one of No. 1 - 8 comprising the steps of (a) exposing said complex, or a cell or organism containing I-TRAF protein complex to one or more candidate molecules; and
   (b) determining the amount of activity of protein components of, and/or intracellular localization of, said complex and/or the transcription level of a gene dependent on the complex and/or the abundance and/or activity of a protein or protein complex dependent on the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependent on the complex and/or the abundance and/or activity of a protein or protein complex dependent on the function of the complex and/or product of a gene dependent on the complex in the absence of said candidate molecules indicates that the molecule modulates function, activity or composition of said complex.
25. The method of No. 24, wherein the amount of said complex is determined.
26. The method of No. 24, wherein the activity of said complex is determined.
27. The method of No. 26, wherein said determining step comprises isolating from the cell or organism said complex to produce said isolated complex and contacting said isolated complex in the presence or absence of a candidate molecule with a substrate of said complex and determining the processing of said substrate is modified in the presence of said candidate molecule.
28. The method of No. 24, wherein the amount of the individual protein components of said complex are determined.
29. The method of No. 28, determining step comprises determining whether
   (i) "TRAF1" (SEQ ID No:14) or a functionally active derivative thereof, or a functionally active fragment thereof, or a homolog thereof, or a variant of "TRAF1" encoded by a nucleic acid that hybridizes to the "TRAF1" nucleic acid under low stringency conditions, and/or
   (ii) "IKK ε" (SEQ ID No:79) or a functionally active derivative thereof, or a functionally active fragment thereof, or a homolog thereof, or a variant of "IKK ε" encoded by a nucleic acid that hybridizes to the "IKK ε" nucleic acid under low stringency conditions, and/or
   (iii) "TRAF3" (SEQ ID No:219) or a functionally active derivative thereof, or a functionally active fragment thereof, or a homolog thereof, or a variant of "TRAF3" encoded by a nucleic acid that hybridizes to the "TRAF3" nucleic acid under low stringency conditions, and/or
   (iv) "TANK BINDING KINASE TBK1" (SEQ ID No:115) or a functionally active derivative thereof, or a functionally active fragment thereof, or a homolog thereof, or a variant of "TANK BINDING KINASE TBK1" encoded by a nucleic acid that hybridizes to the "TANK BINDING KINASE TBK1" nucleic acid under low stringency conditions, and/or
   (v) "keratin9" (SEQ ID No:220) or a functionally active derivative thereof, or a functionally active fragment thereof, or a homolog thereof, or a variant of "keratin9" encoded by a nucleic acid that hybridizes to the "keratin9" nucleic acid under low stringency conditions, and/or
   (vi) "keratin16" (SEQ ID No:221) or a functionally active derivative thereof, or a functionally active fragment thereof, or a homolog thereof, or a variant of "keratin16" encoded by a nucleic acid that hybridizes to the "keratin16" nucleic acid under low stringency conditions, and/or
   (vii) "LYSYL-TRNA SYNTHETASE" (SEQ ID No:222) or a functionally active derivative thereof, or a functionally active fragment thereof, or a homolog thereof, or a variant of "LYSYL-TRNA SYNTHETASE" encoded by a nucleic acid that hybridizes to the "LYSYL-TRNA SYNTHETASE" nucleic acid under low stringency conditions, and/or
   (viii) "CDNA FLJ13715 FIS, CLONE PLACE2000404, MODERATELY SIMILAR TO PROBABLE LEUCYL-TRNA SYNTHETASE" (SEQ ID No:223) or a functionally active derivative thereof, or a functionally active fragment thereof, or a homolog thereof, or a variant of "CDNA FLJ13715 FIS, CLONE PLACE2000404, MODERATELY SIMILAR TO PROBABLE LEUCYL-TRNA SYNTHETASE" encoded by a nucleic acid that hybridizes to the "CDNA FLJ13715 FIS, CLONE PLACE2000404, MODERATELY SIMILAR TO PROBABLE LEUCYL-TRNA SYNTHETASE" nucleic acid under low stringency conditions, and/or
   (ix) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions, and/or
   (x) "I-TRAF" (SEQ ID No:224) or a functionally active derivative thereof, or a functionally active fragment thereof, or a homolog thereof, or a variant of "I-TRAF" encoded by a nucleic acid that hybridizes to the "I-TRAF" nucleic acid under low stringency conditions,
   is present in the complex.
30. The method of any of No. 24 to 29, wherein said method is a method of screening for a drug for treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
31. Use of a molecule that modulates the amount of, activity of, or the protein components of the complex of any one of No. 1 - 8 for the manufacture of a medicament for the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
32. A method for the production of a pharmaceutical composition comprising carrying out the method of any of No. 1 - 8 to identify a molecule that modulates the function, activity, composition or formation of said complex, and further comprising mixing the identified molecule with a pharmaceutically acceptable carrier.
33. A method for diagnosing or screening for the presence of a disease or disorder or a predisposition for developing a disease or disorder in a subject, which disease or disorder is characterized by an aberrant amount of, activity of, or component composition of, or intracellular localization of the complex of any one of the No. 1 - 8, comprising determining the amount of, activity of, protein components of, and/or intracellular localization of, said complex and/or the transcription level of a gene dependent on the complex and/or the abundance and/or activity of a protein or protein complex dependent on the function of the complex and/or product of a gene dependent on the complex in a comparative sample derived from a subject, wherein a difference in said amount, activity, or protein components of, said complex in an analogous sample from a subject not having the disease or disorder or predisposition indicates the presence in the subject of the disease or disorder or predisposition in the subject.
34. The method of No. 33, wherein the amount of said complex is determined.
35. The method of No. 33, wherein the activity of said complex is determined.
36. The method of No. 35, wherein said determining step comprises isolating from the subject said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
37. The method of No. 33, wherein the amount of the individual protein components of said complex are determined.
38. The method of No. 37, wherein said determining step comprises determining whether
   (i) "TRAF1" (SEQ ID No: 14) or a functionally active derivative thereof, or a functionally active fragment thereof, or a homolog thereof, or a variant of "TRAF1" encoded by a nucleic acid that hybridizes to the "TRAF1" nucleic acid under low stringency conditions, and/or
   (ii) "IKK ε" (SEQ ID No:79) or a functionally active derivative thereof, or a functionally active fragment thereof, or a homolog thereof, or a variant of "IKK ε" encoded by a nucleic acid that hybridizes to the "IKK ε" nucleic acid under low stringency conditions, and/or
   (iii) "TRAF3" (SEQ ID No:219) or a functionally active derivative thereof, or a functionally active fragment thereof, or a homolog thereof, or a variant of "TRAF3" encoded by a nucleic acid that hybridizes to the "TRAF3" nucleic acid under low stringency conditions, and/or
   (iv) "TANK BINDING KINASE TBK1" (SEQ ID No:115) or a functionally active derivative thereof, or a functionally active fragment thereof, or a homolog thereof, or a variant of "TANK BINDING KINASE TBK1" encoded by a nucleic acid that hybridizes to the "TANK BINDING KINASE TBK1" nucleic acid under low stringency conditions, and/or
   (v) "keratin9" (SEQ ID No:220) or a functionally active derivative thereof, or a functionally active fragment thereof, or a homolog thereof, or a variant of "keratin9" encoded by a nucleic acid that hybridizes to the "keratin9" nucleic acid under low stringency conditions, and/or
   (vi) "keratin16" (SEQ ID No:221) or a functionally active derivative thereof, or a functionally active fragment thereof, or a homolog thereof, or a variant of "keratin16" encoded by a nucleic acid that hybridizes to the "keratin16" nucleic acid under low stringency conditions, and/or
   (vii) "LYSYL-TRNA SYNTHETASE" (SEQ ID No:222) or a functionally active derivative thereof, or a functionally active fragment thereof, or a homolog thereof, or a variant of "LYSYL-TRNA SYNTHETASE" encoded by a nucleic acid that hybridizes to the "LYSYL-TRNA SYNTHETASE" nucleic acid under low stringency conditions, and/or
   (viii) "CDNA FLJ13715 FIS, CLONE PLACE2000404, MODERATELY SIMILAR TO PROBABLE LEUCYL-TRNA SYNTHETASE" (SEQ ID No:223) or a functionally active derivative thereof, or a functionally active fragment thereof, or a homolog thereof, or a variant of "CDNA FLJ13715 FIS, CLONE PLACE2000404, MODERATELY SIMILAR TO PROBABLE LEUCYL-TRNA SYNTHETASE" encoded by a nucleic acid that hybridizes to the "CDNA FLJ13715 FIS, CLONE PLACE2000404, MODERATELY SIMILAR TO PROBABLE LEUCYL-TRNA SYNTHETASE" nucleic acid under low stringency conditions, and/or
   (ix) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions, and/or
   (x) "I-TRAF" (SEQ ID No:224) or a functionally active derivative thereof, or a functionally active fragment thereof, or a homolog thereof, or a variant of "I-TRAF" encoded by a nucleic acid that hybridizes to the "I-TRAF" nucleic acid under low stringency conditions,
   is present in the complex.
39. The complex of any one of No. 1 - 8 or the antibody or fragment of No. 15, for use in a method of diagnosing a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
40. A method for treating or preventing a disease or disorder characterized by an aberrant amount of, activity or component composition of or intracellular localization of, the complex of anyone of No. 1 - 8, comprising administering to a subject in need of such treatment or prevention a therapeutically effective amount of one or more molecules that modulate the amount of, I-TRAF binding activity, or protein components of, said complex.
41. The method according to No. 40, wherein said disease or disorder involves decreased levels of the amount or activity of said complex.
42. The method according to No. 40 , wherein said disease or disorder involves increased levels of the amount or activity of said complex.
43. Complex of any of No. 1 - 8 and/or protein selected from the following proteins
   (i) "TRAF1" (SEQ ID No:14) or a functionally active derivative thereof, or a functionally active fragment thereof, or a homolog thereof, or a variant of "TRAF1" encoded by a nucleic acid that hybridizes to the "TRAF1" nucleic acid under low stringency conditions,
   (ii) "IKK ε" (SEQ ID No:79) or a functionally active derivative thereof, or a functionally active fragment thereof, or a homolog thereof, or a variant of "IKK ε" encoded by a nucleic acid that hybridizes to the "IKK ε" nucleic acid under low stringency conditions,
   (iii) "TRAF3" (SEQ ID No:219) or a functionally active derivative thereof, or a functionally active fragment thereof, or a homolog thereof, or a variant of "TRAF3" encoded by a nucleic acid that hybridizes to the "TRAF3" nucleic acid under low stringency conditions,
   (iv) "TANK BINDING KINASE TBK1" (SEQ ID No:115) or a functionally active derivative thereof, or a functionally active fragment thereof, or a homolog thereof, or a variant of "TANK BINDING KINASE TBK1" encoded by a nucleic acid that hybridizes to the "TANK BINDING KINASE TBK1" nucleic acid under low stringency conditions,
   (v) "keratin9" (SEQ ID No:220) or a functionally active derivative thereof, or a functionally active fragment thereof, or a homolog thereof, or a variant of "keratin9" encoded by a nucleic acid that hybridizes to the "keratin9" nucleic acid under low stringency conditions,
   (vi) "keratin16" (SEQ ID No:221) or a functionally active derivative thereof, or a functionally active fragment thereof, or a homolog thereof, or a variant of "keratin16" encoded by a nucleic acid that hybridizes to the "keratin16" nucleic acid under low stringency conditions,
   (vii) "LYSYL-TRNA SYNTHETASE" (SEQ ID No:222) or a functionally active derivative thereof, or a functionally active fragment thereof, or a homolog thereof, or a variant of "LYSYL-TRNA SYNTHETASE" encoded by a nucleic acid that hybridizes to the "LYSYL-TRNA SYNTHETASE" nucleic acid under low stringency conditions,
   (viii) "CDNA FLJ13715 FIS, CLONE PLACE2000404, MODERATELY SIMILAR TO PROBABLE LEUCYL-TRNA SYNTHETASE" (SEQ ID No:223) or a functionally active derivative thereof, or a functionally active fragment thereof, or a homolog thereof, or a variant of "CDNA FLJ13715 FIS, CLONE PLACE2000404, MODERATELY SIMILAR TO PROBABLE LEUCYL-TRNA SYNTHETASE" encoded by a nucleic acid that hybridizes to the "CDNA FLJ13715 FIS, CLONE PLACE2000404, MODERATELY SIMILAR TO PROBABLE LEUCYL-TRNA SYNTHETASE" nucleic acid under low stringency conditions,
   (ix) "TRAF2" (SEQ ID No:15) or a functionally active derivative thereof,or a functionally active fragement thereof, or a homolog thereof, or a variant of "TRAF2" that hybridizes to the "TRAF2" nucleic acid under low stringency conditions, and
   (x) "I-TRAF" (SEQ ID No:224) or a functionally active derivative thereof, or a functionally active fragment thereof, or a homolog thereof, or a variant of "I-TRAF" encoded by a nucleic acid that hybridizes to the "I-TRAF" nucleic acid under low stringency conditions,
as a target for an active agent of a pharmaceutical, preferably a drug target in the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.

The present invention further relates to the MEKK3 protein complex
1. A protein complex selected from complex (I) and comprising
   (a) at least one first protein selected from the group consisting of:
      (i) "MEKK3" (SEQ ID No:227) or a functionally active derivative thereof, or a functionally active fragment thereof, or a homolog thereof, or a variant of "MEKK3" encoded by a nucleic acid that hybridizes to the "MEKK3" nucleic acid under low stringency conditions,
      (ii) "14-3-3ε" (SEQ ID No:228) or a functionally active derivative thereof, or a functionally active fragment thereof, or a homolog thereof, or a variant of "14-3-3ε" encoded by a nucleic acid that hybridizes to the "14-3-3ε " nucleic acid under low stringency conditions,
      (iii) "14-3-3 ζ/δ" (SEQ ID No:229) or a functionally active derivative thereof, or a functionally active fragment thereof, or a homolog thereof, or a variant of "14-3-3 ζ/δ" encoded by a nucleic acid that hybridizes to the "14-3-3 ζ/δ" nucleic acid under low stringency conditions, and
      (iv) "MEK5" (SEQ ID No:230) or a functionally active derivative thereof, or a functionally active fragment thereof, or a homolog thereof, or a variant of "MEK5" encoded by a nucleic acid that hybridizes to the "MEK5" nucleic acid under low stringency conditions, and
   (b) at least one second protein, which second protein is selected from the group consisting of:
      (i) "RNA POLYMERASE II SUBUNIT 5-MEDIATING PROTEIN" (SEQ ID No:225) or a functionally active derivative thereof, or a functionally active fragment thereof, or a homolog thereof, or a variant of "RNA POLYMERASE II SUBUNIT 5-MEDIATING PROTEIN" encoded by a nucleic acid that hybridizes to the "RNA POLYMERASE II SUBUNIT 5-MEDIATING PROTEIN" nucleic acid under low stringency conditions,
      (ii) "PEROXIREDOXIN 4" (SEQ ID No:226) or a functionally active derivative thereof, or a functionally active fragment thereof, or a homolog thereof, or a variant of "PEROXIREDOXIN 4" encoded by a nucleic acid that hybridizes to the "PEROXIREDOXIN 4" nucleic acid under low stringency conditions,
      (iii) "FANCONI ANEMIA COMPLEMENTATION GROUP D2 PROTEIN, ISOFORM 1" (SEQ ID No: 18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,
      (iv) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
      (v) "P30 DBC PROTEIN" (SEQ ID No:252) or a functionally active derivative thereof, or a functionally active fragment thereof, or a homolog thereof, or a variant of "P30 DBC PROTEIN" encoded by a nucleic acid that hybridizes to the "P30 DBC PROTEIN" nucleic acid under low stringency conditions,
      (vi) "HSP 90-α" (SEQ ID No:105) or a functionally active derivative thereof, or a functionally active fragment thereof, or a homolog thereof, or a variant of "HSP 90-α" encoded by a nucleic acid that hybridizes to the "HSP 90-α" nucleic acid under low stringency conditions,
      (vii) "HSP 90-β 1"(SEQ ID No:9) or a functionally active derivative thereof, or a functionally active fragment thereof, or a homolog thereof, or a variant of "HSP 90-β 1 "encoded by a nucleic acid that hybridizes to the "HSP 90-β1" nucleic acid under low stringency conditions,
      (viii) "PREFOLDIN 2" (SEQ ID No:232) or a functionally active derivative thereof, or a functionally active fragment thereof, or a homolog thereof, or a variant of "PREFOLDIN 2" encoded by a nucleic acid that hybridizes to the "PREFOLDIN 2" nucleic acid under low stringency conditions,
      (ix) "NAKAP95" (SEQ ID No:82) or a functionally active derivative thereof, or a functionally active fragment thereof, or a homolog thereof, or a variant of "NAKAP95" encoded by a nucleic acid that hybridizes to the "NAKAP95" nucleic acid under low stringency conditions,
      (x) "UXT PROTEIN" (SEQ ID No:233) or a functionally active derivative thereof, or a functionally active fragment thereof, or a homolog thereof, or a variant of "UXT PROTEIN" encoded by a nucleic acid that hybridizes to the "UXT PROTEIN" nucleic acid under low stringency conditions,
      (xi) "FLJ31741" (SEQ ID No:234) or a functionally active derivative thereof, or a functionally active fragment thereof, or a homolog thereof, or a variant of "FLJ31741" encoded by a nucleic acid that hybridizes to the "FLJ31741" nucleic acid under low stringency conditions,
      (xii) "PREFOLDIN 6" (SEQ ID No:235) or a functionally active derivative thereof, or a functionally active fragment thereof, or a homolog thereof, or a variant of "PREFOLDIN 6" encoded by a nucleic acid that hybridizes to the "PREFOLDIN 6" nucleic acid under low stringency conditions,
      (xiii) "TRAF-X" (SEQ ID No:236) or a functionally active derivative thereof, or a functionally active fragment thereof, or a homolog thereof, or a variant of "TRAF-X " encoded by a nucleic acid that hybridizes to the "TRAF-X " nucleic acid under low stringency conditions,
      (xiv) "DJ310O13. (FRAGMENT)" (SEQ ID No:237) or a functionally active derivative thereof, or a functionally active fragment thereof, or a homolog thereof, or a variant of "DJ310O13.3 (FRAGMENT)" encoded by a nucleic acid that hybridizes to the "DJ310O13.3 (FRAGMENT)" nucleic acid under low stringency conditions, and
      (xv) "TOLL-LIKE RECEPT 3 PRECURSOR" (SEQ ID No:238) or a functionally active derivative thereof, or a functionally active fragment thereof, or a homolog thereof, or a variant of "TOLL-LIKE RECEPT 3 PRECURSOR" encoded by a nucleic acid that hybridizes to the "TOLL-LIKE RECEPT 3 PRECURSOR" nucleic acid under low stringency conditions;
   and a complex (II) comprising at least two of said second proteins,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
2. The protein complex according to claim 1 wherein the first protein is the protein "MEKK3" (SEQ ID No:227), or a functionally active derivative thereof, or a functionally active fragment thereof, or a homolog thereof, or a variant of "MEKK3" encoded by a nucleic acid that hybridizes to the "MEKK3" nucleic acid under low stringency conditions.
3. The protein complex according to No. 1 selected from complex (I) and comprising the following proteins:
   (i) "14-3-3ε" (SEQ ID No:228) or a functionally active derivative thereof, or a functionally active fragment thereof, or a homolog thereof, or a variant of "14-3-3ε" encoded by a nucleic acid that hybridizes to the "14-3-3ε" nucleic acid under low stringency conditions,
   (ii) "14-3-3 ζ/δ" (SEQ ID No:229) or a functionally active derivative thereof, or a functionally active fragment thereof, or a homolog thereof, or a variant of "14-3-3 ζ/δ" encoded by a nucleic acid that hybridizes to the "14-3-3 ζ/δ" nucleic acid under low stringency conditions,
   (iii) "HSP 90-α" (SEQ ID No:105) or a functionally active derivative thereof, or a functionally active fragment thereof, or a homolog thereof, or a variant of "HSP 90-α" encoded by a nucleic acid that hybridizes to the "HSP90-α" nucleic acid under low stringency conditions,
   (iv) "HSP 90-β 1"(SEQ ID No:9) or a functionally active derivative thereof, or a functionally active fragment thereof, or a homolog thereof, or a variant of "HSP 90-β1" encoded by a nucleic acid that hybridizes to the "HSP 90-β1" nucleic acid under low stringency conditions,
   (v) "MEKK3" (SEQ ID No:227) or a functionally active derivative thereof, or a functionally active fragment thereof, or a homolog thereof, or a variant of "MEKK3" encoded by a nucleic acid that hybridizes to the "MEKK3" nucleic acid under low stringency conditions,
   (vi) "PREFOLDIN 2" (SEQ ID No:232) or a functionally active derivative thereof,or a functionally active fragement thereof, or a homolog thereof, or a variant of "PREFOLDIN 2" that hybridizes to the "PREFOLDIN 2" nucleic acid under low stringency conditions, and
   (vii) "RNA POLYMERASE II SUBUNIT 5-MEDIATING PROTEIN" (SEQ ID No:225) or a functionally active derivative thereof, or a functionally active fragment thereof, or a homolog thereof, or a variant of "RNA POLYMERASE II SUBUNIT 5-MEDIATING PROTEIN" encoded by a nucleic acid that hybridizes to the "RNA POLYMERASE II SUBUNIT 5-MEDIATING PROTEIN" nucleic acid under low stringency conditions;
   and a protein complex selected from complex (II) and comprising the following proteins:
   (i) "RNA POLYMERASE II SUBUNIT 5-MEDIATING PROTEIN" (SEQ ID No:225) or a functionally active derivative thereof, or a functionally active fragment thereof, or a homolog thereof, or a variant of "RNA POLYMERASE II SUBUNIT 5-MEDIATING PROTEIN" encoded by a nucleic acid that hybridizes to the "RNA POLYMERASE II SUBUNIT 5-MEDIATING PROTEIN" nucleic acid under low stringency conditions,
   (ii) "PEROXIREDOXIN 4" (SEQ ID No:226) or a functionally active derivative thereof, or a functionally active fragment thereof, or a homolog thereof, or a variant of "PEROXIREDOXIN 4" encoded by a nucleic acid that hybridizes to the "PEROXIREDOXIN 4" nucleic acid under low stringency conditions,
   (iii) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,
   (iv) "MEKK3" (SEQ ID No:227) or a functionally active derivative thereof, or a functionally active fragment thereof, or a homolog thereof, or a variant of "MEKK3" encoded by a nucleic acid that hybridizes to the "MEKK3" nucleic acid under low stringency conditions,
   (v) "14-3-3ε" (SEQ ID No:228) or a functionally active derivative thereof, or a functionally active fragment thereof, or a homolog thereof, or a variant of "14-3-3ε" encoded by a nucleic acid that hybridizes to the "14-3-3ε" nucleic acid under low stringency conditions,
   (vi) "14-3-3 ζ/δ" (SEQ ID No:229) or a functionally active derivative thereof, or a functionally active fragment thereof, or a homolog thereof, or a variant of "14-3-3 ζ/δ" encoded by a nucleic acid that hybridizes to the "14-3-3 ζ/δ" nucleic acid under low stringency conditions,
   (vii) "MEK5" (SEQ ID No:230) or a functionally active derivative thereof, or a functionally active fragment thereof, or a homolog thereof, or a variant of "MEK5" encoded by a nucleic acid that hybridizes to the "MEK5" nucleic acid under low stringency conditions,
   (viii) "HSP 90-α" (SEQ ID No:105) or a functionally active derivative thereof, or a functionally active fragment thereof, or a homolog thereof, or a variant of "HSP 90-α" encoded by a nucleic acid that hybridizes to the "HSP90-α" nucleic acid under low stringency conditions,
   (ix) "HSP 90-β1" (SEQ ID No:9) or a functionally active derivative thereof, or a functionally active fragment thereof, or a homolog thereof, or a variant of "HSP 90-β1" encoded by a nucleic acid that hybridizes to the "HSP 90-β1" nucleic acid under low stringency conditions,
   (x) "PREFOLDIN 2" (SEQ ID No:232) or a functionally active derivative thereof, or a functionally active fragment thereof, or a homolog thereof, or a variant of "PREFOLDIN 2" encoded by a nucleic acid that hybridizes to the "PREFOLDIN 2" nucleic acid under low stringency conditions,
   (xi) "NAKAP95" (SEQ ID No:82) or a functionally active derivative thereof, or a functionally active fragment thereof, or a homolog thereof, or a variant of "NAKAP95" encoded by a nucleic acid that hybridizes to the "NAKAP95" nucleic acid under low stringency conditions,
   (xii) "UXT PROTEIN" (SEQ ID No:233) or a functionally active derivative thereof, or a functionally active fragment thereof, or a homolog thereof, or a variant of "UXT PROTEIN" encoded by a nucleic acid that hybridizes to the "UXT PROTEIN" nucleic acid under low stringency conditions,
   (xiii) "FLJ31741" (SEQ ID No:234) or a functionally active derivative thereof, or a functionally active fragment thereof, or a homolog thereof, or a variant of "FLJ31741" encoded by a nucleic acid that hybridizes to the "FLJ31741" nucleic acid under low stringency conditions,
   (xiv) "PREFOLDIN 6" (SEQ ID No:235) or a functionally active derivative thereof, or a functionally active fragment thereof, or a homolog thereof, or a variant of "PREFOLDIN 6" encoded by a nucleic acid that hybridizes to the "PREFOLDIN 6" nucleic acid under low stringency conditions,
   (xv) "TRAF-X " (SEQ ID No:236) or a functionally active derivative thereof, or a functionally active fragment thereof, or a homolog thereof, or a variant of "TRAF-X" encoded by a nucleic acid that hybridizes to the "TRAF-X" nucleic acid under low stringency conditions,
   (xvi) "DJ310O13.3 (FRAGMENT)" (SEQ ID No:237) or a functionally active derivative thereof,or a functionally active fragement thereof, or a homolog thereof, or a variant of "DJ310O13 (FRAGMENT)" that hybridizes to the "DJ310O13.3 (FRAGMENT)" nucleic acid under low stringency conditions, and
   (xii) "TOLL-LIKE RECEPT 3 PRECURSOR" (SEQ ID No:238) or a functionally active derivative thereof, or a functionally active fragment thereof, or a homolog thereof, or a variant of "TOLL-LIKE RECEPT 3 PRECURSOR" encoded by a nucleic acid that hybridizes to the "TOLL-LIKE RECEPT 3 PRECURSOR" nucleic acid under low stringency conditions;
   and a protein complex selected from complex (III) and comprising the following proteins:
   (i) "14-3-3ε "(SEQ ID No:228) or a functionally active derivative thereof, or a functionally active fragment thereof, or a homolog thereof, or a variant of "14-3-3ε" encoded by a nucleic acid that hybridizes to the "14-3-3ε" nucleic acid under low stringency conditions,
   (ii) "14-3-3 ζ/δ" (SEQ ID No:229) or a functionally active derivative thereof, or a functionally active fragment thereof, or a homolog thereof, or a variant of "14-3-3 ζ/δ" encoded by a nucleic acid that hybridizes to the "14-3-3 ζ/δ" nucleic acid under low stringency conditions,
   (iii) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (iv) "HSP 90-α" (SEQ ID No:105) or a functionally active derivative thereof, or a functionally active fragment thereof, or a homolog thereof, or a variant of "HSP 90-α" encoded by a nucleic acid that hybridizes to the "HSP90-α" nucleic acid under low stringency conditions,
   (v) "HSP 90-β 1"(SEQ ID No:9) or a functionally active derivative thereof, or a functionally active fragment thereof, or a homolog thereof, or a variant of "HSP 90-β 1" encoded by a nucleic acid that hybridizes to the "HSP 90-β 1" nucleic acid under low stringency conditions,
   (vi) "MEKK3" (SEQ ID No:227) or a functionally active derivative thereof, or a functionally active fragment thereof, or a homolog thereof, or a variant of "MEKK3" encoded by a nucleic acid that hybridizes to the "MEKK3" nucleic acid under low stringency conditions,
   (vii) "P30 DBC PROTEIN" (SEQ ID No:252) or a functionally active derivative thereof, or a functionally active fragment thereof, or a homolog thereof, or a variant of "P30 DBC PROTEIN" encoded by a nucleic acid that hybridizes to the "P30 DBC PROTEIN" nucleic acid under low stringency conditions,
   (viii) "PREFOLDIN 2" (SEQ ID No:232) or a functionally active derivative thereof,or a functionally active fragement thereof, or a homolog thereof, or a variant of "PREFOLDIN 2" that hybridizes to the "PREFOLDIN 2" nucleic acid under low stringency conditions, and
   (ix) "RNA POLYMERASE II SUBUNIT 5-MEDIATING PROTEIN" (SEQ ID No:225) or a functionally active derivative thereof, or a functionally active fragment thereof, or a homolog thereof, or a variant of "RNA POLYMERASE II SUBUNIT 5-MEDIATING PROTEIN" encoded by a nucleic acid that hybridizes to the "RNA POLYMERASE II SUBUNIT 5-MEDIATING PROTEIN" nucleic acid under low stringency conditions;
   and a protein complex selected from complex (IV) and comprising the following proteins:
   (i) "RNA POLYMERASE II SUBUNIT 5-MEDIATING PROTEIN" (SEQ ID No:225) or a functionally active derivative thereof, or a functionally active fragment thereof, or a homolog thereof, or a variant of "RNA POLYMERASE 11 SUBUNIT 5-MEDIATING PROTEIN" encoded by a nucleic acid that hybridizes to the "RNA POLYMERASE II SUBUNIT 5-MEDIATING PROTEIN" nucleic acid under low stringency conditions,
   (ii) "PEROXIREDOXIN 4" (SEQ ID No:226) or a functionally active derivative thereof, or a functionally active fragment thereof, or a homolog thereof, or a variant of "PEROXIREDOXIN 4" encoded by a nucleic acid that hybridizes to the "PEROXIREDOXIN 4" nucleic acid under low stringency conditions,
   (iii) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,
   (iv) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (v) "MEKK3" (SEQ ID No:227) or a functionally active derivative thereof, or a functionally active fragment thereof, or a homolog thereof, or a variant of "MEKK3" encoded by a nucleic acid that hybridizes to the "MEKK3" nucleic acid under low stringency conditions,
   (vi) "14-3-3ε" (SEQ ID No:228) or a functionally active derivative thereof, or a functionally active fragment thereof, or a homolog thereof, or a variant of "14-3-3ε" encoded by a nucleic acid that hybridizes to the "14-3-3ε" nucleic acid under low stringency conditions,
   (vii) "14-3-3 ζ/δ" (SEQ ID No:229) or a functionally active derivative thereof, or a functionally active fragment thereof, or a homolog thereof, or a variant of "14-3-3 ζ/δ" encoded by a nucleic acid that hybridizes to the "14-3-3 ζ/δ" nucleic acid under low stringency conditions,
   (viii) "MEK5" (SEQ ID No:230) or a functionally active derivative thereof, or a functionally active fragment thereof, or a homolog thereof, or a variant of "MEK5" encoded by a nucleic acid that hybridizes to the "MEK5" nucleic acid under low stringency conditions,
   (ix) "P30 DBC PROTEIN" (SEQ ID No:252) or a functionally active derivative thereof, or a functionally active fragment thereof, or a homolog thereof, or a variant of "P30 DBC PROTEIN" encoded by a nucleic acid that hybridizes to the "P30 DBC PROTEIN" nucleic acid under low stringency conditions,
   (x) "HSP 90-α" (SEQ ID No:105) or a functionally active derivative thereof, or a functionally active fragment thereof, or a homolog thereof, or a variant of "HSP 90-α" encoded by a nucleic acid that hybridizes to the "HSP90-α" nucleic acid under low stringency conditions,
   (xi) "HSP 90-β 1"(SEQ ID No:9) or a functionally active derivative thereof, or a functionally active fragment thereof, or a homolog thereof, or a variant of "HSP 90-β 1" encoded by a nucleic acid that hybridizes to the "HSP 90-β 1" nucleic acid under low stringency conditions,
   (xii) "PREFOLDIN 2" (SEQ ID No:232) or a functionally active derivative thereof, or a functionally active fragment thereof, or a homolog thereof, or a variant of "PREFOLDIN 2" encoded by a nucleic acid that hybridizes to the "PREFOLDIN 2" nucleic acid under low stringency conditions,
   (xiii) "NAKAP95" (SEQ ID No:82) or a functionally active derivative thereof, or a functionally active fragment thereof, or a homolog thereof, or a variant of "NAKAP95" encoded by a nucleic acid that hybridizes to the "NAKAP95" nucleic acid under low stringency conditions,
   (xiv) "UXT PROTEIN" (SEQ ID No:233) or a functionally active derivative thereof, or a functionally active fragment thereof, or a homolog thereof, or a variant of "UXT PROTEIN" encoded by a nucleic acid that hybridizes to the "UXT PROTEIN" nucleic acid under low stringency conditions,
   (xv) "FLJ31741" (SEQ ID No:234) or a functionally active derivative thereof, or a functionally active fragment thereof, or a homolog thereof, or a variant of "FLJ31741" encoded by a nucleic acid that hybridizes to the "FLJ31741" nucleic acid under low stringency conditions,
   (xvi)."PREFOLDIN 6" (SEQ ID No:235) or a functionally active derivative thereof, or a functionally active fragment thereof, or a homolog thereof, or a variant of "PREFOLDIN 6" encoded by a nucleic acid that hybridizes to the "PREFOLDIN 6" nucleic acid under low stringency conditions,
   (xvii) "TRAF-X" (SEQ ID No:236) or a functionally active derivative thereof, or a functionally active fragment thereof, or a homolog thereof, or a variant of "TRAF-X" encoded by a nucleic acid that hybridizes to the "TRAF-X" nucleic acid under low stringency conditions,
   (xviii) "DJ310O13. (FRAGMENT)" (SEQ ID No:237) or a functionally active derivative thereof,or a functionally active fragement thereof, or a homolog thereof, or a variant of "DJ310O13.3 (FRAGMENT)" that hybridizes to the "DJ310O13 (FRAGMENT)" nucleic acid under low stringency conditions, and
   (xix) "TOLL-LIKE RECEPT 3 PRECURSOR" (SEQ ID No:238) or a functionally active derivative thereof,or a functionally active fragement thereof, or a homolog thereof, or a variant of "TOLL-LIKE RECEPT 3 PRECURSOR" that hybridizes to the "TOLL-LIKE RECEPT 3 PRECURSOR" nucleic acid under low stringency conditions.
4. The protein complex according to No. 1 comprising all but 1 - 14 of the following proteins:
   (i) "RNA POLYMERASE II SUBUNIT 5-MEDIATING PROTEIN" (SEQ ID No:225) or a functionally active derivative thereof, or a functionally active fragment thereof, or a homolog thereof, or a variant of "RNA POLYMERASE II SUBUNIT 5-MEDIATING PROTEIN" encoded by a nucleic acid that hybridizes to the "RNA POLYMERASE II SUBUNIT 5-MEDIATING PROTEIN" nucleic acid under low stringency conditions,
   (ii) "PEROXIREDOXIN 4" (SEQ ID No:226) or a functionally active derivative thereof, or a functionally active fragment thereof, or a homolog thereof, or a variant of "PEROXIREDOXIN 4" encoded by a nucleic acid that hybridizes to the "PEROXIREDOXIN 4" nucleic acid under low stringency conditions,
   (iii) "FANCONI ANEMIA COMPLEMENTATION GROUP D2 PROTEIN, ISOFORM 1" (SEQ ID No: 18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,
   (iv) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (v) "MEKK3" (SEQ ID No:227) or a functionally active derivative thereof, or a functionally active fragment thereof, or a homolog thereof, or a variant of "MEKK3" encoded by a nucleic acid that hybridizes to the "MEKK3" nucleic acid under low stringency conditions,
   (vi) "14-3-3ε" (SEQ ID No:228) or a functionally active derivative thereof, or a functionally active fragment thereof, or a homolog thereof, or a variant of "x14-3-3ε" encoded by a nucleic acid that hybridizes to the "14-3-3ε" nucleic acid under low stringency conditions,
   (vii) "14-3-3 ζ/δ" (SEQ ID No:229) or a functionally active derivative thereof, or a functionally active fragment thereof, or a homolog thereof, or a variant of "14-3-3 ζ/δ" encoded by a nucleic acid that hybridizes to the "14-3-3 ζ/δ" nucleic acid under low stringency conditions,
   (viii) "MEK5" (SEQ ID No:230) or a functionally active derivative thereof, or a functionally active fragment thereof, or a homolog thereof, or a variant of "MEK5" encoded by a nucleic acid that hybridizes to the "MEK5" nucleic acid under low stringency conditions,
   (ix) "P30 DBC PROTEIN" (SEQ ID No:252) or a functionally active derivative thereof, or a functionally active fragment thereof, or a homolog thereof, or a variant of "P30 DBC PROTEIN" encoded by a nucleic acid that hybridizes to the "P30 DBC PROTEIN" nucleic acid under low stringency conditions,
   (x) "HSP 90-α" (SEQ ID No:105) or a functionally active derivative thereof, or a functionally active fragment thereof, or a homolog thereof, or a variant of "HSP 90-α" encoded by a nucleic acid that hybridizes to the "HSP90-α" nucleic acid under low stringency conditions,
   (xi) "HSP 90-β 1"(SEQ ID No:9) or a functionally active derivative thereof, or a functionally active fragment thereof, or a homolog thereof, or a variant of "HSP 90-β 1" encoded by a nucleic acid that hybridizes to the "HSP 90-β 1" nucleic acid under low stringency conditions,
   (xii) "PREFOLDIN 2" (SEQ ID No:232) or a functionally active derivative thereof, or a functionally active fragment thereof, or a homolog thereof, or a variant of "PREFOLDIN 2" encoded by a nucleic acid that hybridizes to the "PREFOLDIN 2" nucleic acid under low stringency conditions,
   (xiii) "NAKAP95" (SEQ ID No:82) or a functionally active derivative thereof, or a functionally active fragment thereof, or a homolog thereof, or a variant of "NAKAP95" encoded by a nucleic acid that hybridizes to the "NAKAP95" nucleic acid under low stringency conditions,
   (xiv) "UXT PROTEIN" (SEQ ID No:233) or a functionally active derivative thereof, or a functionally active fragment thereof, or a homolog thereof, or a variant of "UXT PROTEIN" encoded by a nucleic acid that hybridizes to the "UXT PROTEIN" nucleic acid under low stringency conditions,
   (xv) "FLJ31741" (SEQ ID No:234) or a functionally active derivative thereof, or a functionally active fragment thereof, or a homolog thereof, or a variant of "FLJ31741" encoded by a nucleic acid that hybridizes to the "FLJ31741" nucleic acid under low stringency conditions,
   (xvi) "PREFOLDIN 6" (SEQ ID No:235) or a functionally active derivative thereof, or a functionally active fragment thereof, or a homolog thereof, or a variant of "PREFOLDIN 6" encoded by a nucleic acid that hybridizes to the "PREFOLDIN 6" nucleic acid under low stringency conditions,
   (xvii) "TRAF-X" (SEQ ID No:236) or a functionally active derivative thereof, or a functionally active fragment thereof, or a homolog thereof, or a variant of "TRAF-X" encoded by a nucleic acid that hybridizes to the "TRAF-X" nucleic acid under low stringency conditions,
   (xviii) "DJ310O13.3 (FRAGMENT)" (SEQ ID No:237) or a functionally active derivative thereof,or a functionally active fragement thereof, or a homolog thereof, or a variant of "DJ310O13.3 (FRAGMENT)" that hybridizes to the "DJ310O13.3 (FRAGMENT)" nucleic acid under low stringency conditions, and
   (xix) "TOLL-LIKE RECEPT 3 PRECURSOR" (SEQ ID No:238) or a functionally active derivative thereof,or a functionally active fragement thereof, or a homolog thereof, or a variant of "TOLL-LIKE RECEPT 3 PRECURSOR" that hybridizes to the "TOLL-LIKE RECEPT 3 PRECURSOR" nucleic acid under low stringency conditions.
5. The complex of any of No. 1 - 4 comprising a functionally active derivative of said first protein and/or a functionally active derivative of said second protein, wherein the functionally active derivative is a fusion protein comprising said first protein or said second protein fused to an amino acid sequence different from the first protein or second protein, respectively.
6. The complex of No. 5 wherein the functionally active derivative is a fusion protein comprising said first protein or said second protein fused to an affinity tag or label.
7. The complex of any of No. 1 - 4 comprising a fragment of said first protein and/or a fragment of said second protein, which fragment binds to another protein component of said complex.
8. The complex of any of No. 1 - 7 that is involved in the
9. A process for preparing complex of any of No. 1 - 8 and optionally the components thereof comprising the following steps:
   the expressing a protein (bait) of the complex, preferably a tagged protein, in a target cell, isolating the protein complex which is attached to the bait protein, and optionally disassociating the protein complex and isolating the individual complex members.
10. The process according to No. 9 wherein the tagged protein comprises two different tags which allow two separate affinity purification steps.
11. The process according to any of No. 9 - 10 wherein the two tags are separated by a cleavage site for a protease.
12. Component of the MEKK3 complex obtainable by a process according to any of No. 9 to 11.
13. Protein of the MEKK3 complex selected from
   (i) "UXT PROTEIN" (SEQ ID No:233) or a functionally active derivative thereof, or a functionally active fragment thereof, or a homolog thereof, or a variant of "UXT PROTEIN" encoded by a nucleic acid that hybridizes to the "UXT PROTEIN" nucleic acid under low stringency conditions,
   (ii) "FLJ31741" (SEQ ID No:234) or a functionally active derivative thereof, or a functionally active fragment thereof, or a homolog thereof, or a variant of "FLJ31741" encoded by a nucleic acid that hybridizes to the "FLJ31741" nucleic acid under low stringency conditions,
   (iii) "TRAF-X " (SEQ ID No:236) or a functionally active derivative thereof, or a functionally active fragment thereof, or a homolog thereof, or a variant of "TRAF-X " encoded by a nucleic acid that hybridizes to the "TRAF-X " nucleic acid under low stringency conditions, and
   (iv) "DJ310O13 (FRAGMENT)" (SEQ ID No:237) or a functionally active derivative thereof, or a functionally active fragment thereof, or a homolog thereof, or a variant of "DJ310O13 (FRAGMENT)" encoded by a nucleic acid that hybridizes to the "DJ310O13.3 (FRAGMENT)" nucleic acid under low stringency conditions,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
14. Nucleic acid encoding a protein according to No. 13.
15. Construct, preferably a vector construct, comprising (a) a nucleic acid according to No. 14 and at least one further nucleic acid which is normally not associated with said nucleic acid,or(b) at least two separate nucleic acid sequences each encoding a different protein, or a functionally active fragment or a functionally active derivative thereof at least one of said proteins, or functionally active fragments or functionally active derivative thereof being selected from the first group of proteins according to No. 1(a) and at least one of said proteins, or functionally active fragments or functionally active derivative thereof being selected from the second group of proteins according to No. 1(b).
16. Host cell, containing a vector comprising at least one of the nucleic acid of No. 14 and/or a construct of No. 15 or containing several vectors each comprising at least the nucleic acid sequence encoding at least one of the proteins, or functionally active fragments or functionally active derivatives thereof selected from the first group of proteins according to No. 1(a) and the proteins, or functionally active fragments or functionally active derivatives thereof selected from the second group of proteins according to No. 1(b).
17. An antibody or a fragment of said antibody containing the binding domain thereof, selected from an antibody or fragment thereof, which binds the complex of any of No. 1-8 and which does not bind any of the proteins of said complex when uncomplexed and an antibody or a fragment of said antibody which binds to any of the proteins according to No. 13.
18. A kit comprising in one or more container the complex of any of No. 1 - 8 and/or the proteins of No. 13 optionally together with an antibody according to No. 17 and/or further components such as reagents and working instructions.
19. The kit according to No. 18 for processing a substrate of said complex.
20. The kit according to No. 18 for the diagnosis or prognosis of a disease or a disease risk, preferentially for a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
21. Array, in which at least a complex according to any of No. 1-8 and/or at least one protein according to No. 14 and/or at least one antibody according to No. 17 is attached to a solid carrier.
22. A process for processing a physiological substrate of the complex comprising the step of bringing into contact a complex to any of No. 1 - 8 with said substrate, such that said substrate is processed.
23. A pharmaceutical composition comprising the protein complex of any of No. 1 to 8 and/or any of the following the proteins:
   (i) "UXT PROTEIN" (SEQ ID No:233) or a functionally active derivative thereof, or a functionally active fragment thereof, or a homolog thereof, or a variant of "UXT PROTEIN" encoded by a nucleic acid that hybridizes to the "UXT PROTEIN" nucleic acid under low stringency conditions,
   (ii) "FLJ31741" (SEQ ID No:234) or a functionally active derivative thereof, or a functionally active fragment thereof, or a homolog thereof, or a variant of "FLJ31741" encoded by a nucleic acid that hybridizes to the "FLJ31741" nucleic acid under low stringency conditions,
   (iii) "TRAF-X" (SEQ ID No:236) or a functionally active derivative thereof,or a functionally active fragement thereof, or a homolog thereof, or a variant of "TRAF-X" that hybridizes to the "TRAF-X" nucleic acid under low stringency conditions, and
   (iv) "DJ310O13.3 (FRAGMENT)" (SEQ ID No:237) or a functionally active derivative thereof, or a functionally active fragment thereof, or a homolog thereof, or a variant of "DJ310O13.3 (FRAGMENT)" encoded by a nucleic acid that hybridizes to the "DJ310O13.3 (FRAGMENT)" nucleic acid under low stringency conditions,
   and a pharmaceutical acceptable carrier.
24. The pharmaceutical composition according to No. 23 for the treatment of diseases and disorders such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
25. A method for screening for a molecule that binds to the complex of anyone of No. 1 - 8 and/or any of the following proteins:
   (i) "UXT PROTEIN" (SEQ ID No:233) or a functionally active derivative thereof, or a functionally active fragment thereof, or a homolog thereof, or a variant of "UXT PROTEIN" encoded by a nucleic acid that hybridizes to the "UXT PROTEIN" nucleic acid under low stringency conditions,
   (ii) "FLJ31741" (SEQ ID No:234) or a functionally active derivative thereof, or a functionally active fragment thereof, or a homolog thereof, or a variant of "FLJ31741" encoded by a nucleic acid that hybridizes to the "FLJ31741" nucleic acid under low stringency conditions,
   (iii) "TRAF-X " (SEQ ID No:236) or a functionally active derivative thereof,or a functionally active fragement thereof, or a homolog thereof, or a variant of "TRAF-X" that hybridizes to the "TRAF-X" nucleic acid under low stringency conditions, and
   (iv) "DJ310O13.3 (FRAGMENT)" (SEQ ID No:237) or a functionally active derivative thereof, or a functionally active fragment thereof, or a homolog thereof, or a variant of "DJ310O13.3 (FRAGMENT)" encoded by a nucleic acid that hybridizes to the "DJ310O13.3 (FRAGMENT)" nucleic acid under low stringency conditions, comprising the steps of
      (a) exposing said complex, or a cell or organism containing same to one or more candidate molecules; and
      (b) determinig whether said candidate molecule is bound to the complex or protein.
26. A method for screening for a molecule that modulates directly or indirectly the function, activity, composition or formation of the complex of any one of No. 1 - 8 comprising the steps of (a) exposing said complex, or a cell or organism containing MEKK3 complex to one or more candidate molecules; and
   (b) determining the amount of activity of protein components of, and/or intracellular localization of, said complex and/or the transcription level of a gene dependent on the complex and/or the abundance and/or activity of a protein or protein complex dependent on the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependent on the complex and/or the abundance and/or activity of a protein or protein complex dependent on the function of the complex and/or product of a gene dependent on the complex in the absence of said candidate molecules indicates that the molecule modulates function, activity or composition of said complex.
27. The method of No. 26, wherein the amount of said complex is determined.
28. The method of No. 26, wherein the activity of said complex is determined.
29. The method of No. 28, wherein said determining step comprises isolating from the cell or organism said complex to produce said isolated complex and contacting said isolated complex in the presence or absence of a candidate molecule with a substrate of said complex and determining the processing of said substrate is modified in the presence of said candidate molecule.
30. The method of No. 26, wherein the amount of the individual protein components of said complex are determined.
31. The method of No. 30, determining step comprises determining whether
   (i) "RNA POLYMERASE II SUBUNIT 5-MEDIATING PROTEIN" (SEQ ID No:225) or a functionally active derivative thereof, or a functionally active fragment thereof, or a homolog thereof, or a variant of "RNA POLYMERASE II SUBUNIT 5-MEDIATING PROTEIN" encoded by a nucleic acid that hybridizes to the "RNA POLYMERASE II SUBUNIT 5-MEDIATING PROTEIN" nucleic acid under low stringency conditions, and/or
   (ii) "PEROXIREDOXIN 4" (SEQ ID No:226) or a functionally active derivative thereof, or a functionally active fragment thereof, or a homolog thereof, or a variant of "PEROXIREDOXIN 4" encoded by a nucleic acid that hybridizes to the "PEROXIREDOXIN 4" nucleic acid under low stringency conditions, and/or
   (iii) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions, and/or
   (iv) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions, and/or
   (v) "MEKK3" (SEQ ID No:227) or a functionally active derivative thereof, or a functionally active fragment thereof, or a homolog thereof, or a variant of "MEKK3" encoded by a nucleic acid that hybridizes to the "MEKK3" nucleic acid under low stringency conditions, and/or
   (vi) "14-3-3ε " (SEQ ID No:228) or a functionally active derivative thereof, or a functionally active fragment thereof, or a homolog thereof, or a variant of "14-3-3ε" encoded by a nucleic acid that hybridizes to the "14-3-3ε" nucleic acid under low stringency conditions, and/or
   (vii) "14-3-3 ζ/δ" (SEQ ID No:229) or a functionally active derivative thereof, or a functionally active fragment thereof, or a homolog thereof, or a variant of "14-3-3 ζ/δ" encoded by a nucleic acid that hybridizes to the "14-3-3 ζ/δ" nucleic acid under low stringency conditions, and/or
   (viii) "MEK5" (SEQ ID No:230) or a functionally active derivative thereof, or a functionally active fragment thereof, or a homolog thereof, or a variant of "MEK5" encoded by a nucleic acid that hybridizes to the "MEK5" nucleic acid under low stringency conditions, and/or
   (ix) "P30 DBC PROTEIN" (SEQ ID No:252) or a functionally active derivative thereof, or a functionally active fragment thereof, or a homolog thereof, or a variant of "P30 DBC PROTEIN" encoded by a nucleic acid that hybridizes to the "P30 DBC PROTEIN" nucleic acid under low stringency conditions, and/or
   (x) "HSP 90-α" (SEQ ID No:105) or a functionally active derivative thereof, or a functionally active fragment thereof, or a homolog thereof, or a variant of "HSP 90-α" encoded by a nucleic acid that hybridizes to the "HSP90-α" nucleic acid under low stringency conditions, and/or
   (xi) "HSP 90-β 1"(SEQ ID No:9) or a functionally active derivative thereof, or a functionally active fragment thereof, or a homolog thereof, or a variant of "HSP 90-β1" encoded by a nucleic acid that hybridizes to the "HSP 90-β1" nucleic acid under low stringency conditions, and/or
   (xii) "PREFOLDIN 2" (SEQ ID No:232) or a functionally active derivative thereof, or a functionally active fragment thereof, or a homolog thereof, or a variant of "PREFOLDIN 2" encoded by a nucleic acid that hybridizes to the "PREFOLDIN 2" nucleic acid under low stringency conditions, and/or
   (xiii) "NAKAP95" (SEQ ID No:82) or a functionally active derivative thereof, or a functionally active fragment thereof, or a homolog thereof, or a variant of "NAKAP95" encoded by a nucleic acid that hybridizes to the "NAKAP95" nucleic acid under low stringency conditions, and/or
   (xiv) "UXT PROTEIN" (SEQ ID No:233) or a functionally active derivative thereof, or a functionally active fragment thereof, or a homolog thereof, or a variant of "UXT PROTEIN" encoded by a nucleic acid that hybridizes to the "UXT PROTEIN" nucleic acid under low stringency conditions, and/or
   (xv) "FLJ31741" (SEQ ID No:234) or a functionally active derivative thereof, or a functionally active fragment thereof, or a homolog thereof, or a variant of "FLJ31741" encoded by a nucleic acid that hybridizes to the "FLJ31741" nucleic acid under low stringency conditions, and/or
   (xvi) "PREFOLDIN 6" (SEQ ID No:235) or a functionally active derivative thereof, or a functionally active fragment thereof, or a homolog thereof, or a variant of "PREFOLDIN 6" encoded by a nucleic acid that hybridizes to the "PREFOLDIN 6" nucleic acid under low stringency conditions, and/or
   (xvii) "TRAF-X" (SEQ ID No:236) or a functionally active derivative thereof, or a functionally active fragment thereof, or a homolog thereof, or a variant of "TRAF-X" encoded by a nucleic acid that hybridizes to the "TRAF-X" nucleic acid under low stringency conditions, and/or
   (xviii) "DJ310O13.3 (FRAGMENT)" (SEQ ID No:237) or a functionally active derivative thereof, or a functionally active fragment thereof, or a homolog thereof, or a variant of "DJ310O13.3 (FRAGMENT)" encoded by a nucleic acid that hybridizes to the "DJ310O13.3 (FRAGMENT)" nucleic acid under low stringency conditions, and/or
   (xix) "TOLL-LIKE RECEPT 3 PRECURSOR" (SEQ ID No:238) or a functionally active derivative thereof, or a functionally active fragment thereof, or a homolog thereof, or a variant of "TOLL-LIKE RECEPT 3 PRECURSOR" encoded by a nucleic acid that hybridizes to the "TOLL-LIKE RECEPT 3 PRECURSOR" nucleic acid under low stringency conditions,
   is present in the complex.
32. The method of any of No. 26 to 31, wherein said method is a method of screening for a drug for treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
33. Use of a molecule that modulates the amount of, activity of, or the protein components of the complex of any one of No. 1 - 8 for the manufacture of a medicament for the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
34. A method for the production of a pharmaceutical composition comprising carrying out the method of any of No. 1 - 8 to identify a molecule that modulates the function, activity, composition or formation of said complex, and further comprising mixing the identified molecule with a pharmaceutically acceptable carrier.
35. A method for diagnosing or screening for the presence of a disease or disorder or a predisposition for developing a disease or disorder in a subject, which disease or disorder is characterized by an aberrant amount of, activity of, or component composition of, or intracellular localization of the complex of any one of the No. 1-8, comprising determining the amount of, activity of, protein components of, and/or intracellular localization of, said complex and/or the transcription level of a gene dependent on the complex and/or the abundance and/or activity of a protein or protein complex dependent on the function of the complex and/or product of a gene dependent on the complex in a comparative sample derived from a subject, wherein a difference in said amount, activity, or protein components of, said complex in an analogous sample from a subject not having the disease or disorder or predisposition indicates the presence in the subject of the disease or disorder or predisposition in the subject.
36. The method of No. 35, wherein the amount of said complex is determined.
37. The method of No. 35, wherein the activity of said complex is determined.
38. The method of No. 37, wherein said determining step comprises isolating from the subject said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
39. The method of No. 35, wherein the amount of the individual protein components of said complex are determined.
40. The method of No. 39, wherein said determining step comprises determining whether
   (i) "RNA POLYMERASE II SUBUNIT 5-MEDIATING PROTEIN" (SEQ ID No:225) or a functionally active derivative thereof, or a functionally active fragment thereof, or a homolog thereof, or a variant of "RNA POLYMERASE II SUBUNIT 5-MEDIATING PROTEIN" encoded by a nucleic acid that hybridizes to the "RNA POLYMERASE II SUBUNIT 5-MEDIATING PROTEIN" nucleic acid under low stringency conditions, and/or
   (ii) "PEROXIREDOXIN 4" (SEQ ID No:226) or a functionally active derivative thereof, or a functionally active fragment thereof, or a homolog thereof, or a variant of "PEROXIREDOXIN 4" encoded by a nucleic acid that hybridizes to the "PEROXIREDOXIN 4" nucleic acid under low stringency conditions, and/or
   (iii) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions, and/or
   (iv) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions, and/or
   (v) "MEKK3" (SEQ ID No:227) or a functionally active derivative thereof, or a functionally active fragment thereof, or a homolog thereof, or a variant of "MEKK3" encoded by a nucleic acid that hybridizes to the "MEKK3" nucleic acid under low stringency conditions, and/or
   (vi) "14-3-3ε " (SEQ ID No:228) or a functionally active derivative thereof, or a functionally active fragment thereof, or a homolog thereof, or a variant of "14-3-3ε" encoded by a nucleic acid that hybridizes to the "14-3-3ε" nucleic acid under low stringency conditions, and/or
   (vii) "14-3-3 ζ/δ" (SEQ ID No:229) or a functionally active derivative thereof, or a functionally active fragment thereof, or a homolog thereof, or a variant of "14-3-3 ζ/δ" encoded by a nucleic acid that hybridizes to the "14-3-3 ζ/δ" nucleic acid under low stringency conditions, and/or
   (viii) "MEK5" (SEQ ID No:230) or a functionally active derivative thereof, or a functionally active fragment thereof, or a homolog thereof, or a variant of "MEK5" encoded by a nucleic acid that hybridizes to the "MEK5" nucleic acid under low stringency conditions, and/or
   (ix) "P30 DBC PROTEIN" (SEQ ID No:252) or a functionally active derivative thereof, or a functionally active fragment thereof, or a homolog thereof, or a variant of "P30 DBC PROTEIN" encoded by a nucleic acid that hybridizes to the "P30 DBC PROTEIN" nucleic acid under low stringency conditions, and/or
   (x) "HSP 90-α" (SEQ ID No:105) or a functionally active derivative thereof, or a functionally active fragment thereof, or a homolog thereof, or a variant of "HSP 90-α" encoded by a nucleic acid that hybridizes to the "HSP90-α" nucleic acid under low stringency conditions, and/or
   (xi) "HSP 90-β 1"(SEQ ID No:9) or a functionally active derivative thereof, or a functionally active fragment thereof, or a homolog thereof, or a variant of "HSP 90-β 1" encoded by a nucleic acid that hybridizes to the "HSP 90-β 1" nucleic acid under low stringency conditions, and/or
   (xii) "PREFOLDIN 2" (SEQ ID No:232) or a functionally active derivative thereof, or a functionally active fragment thereof, or a homolog thereof, or a variant of "PREFOLDIN 2" encoded by a nucleic acid that hybridizes to the "PREFOLDIN 2" nucleic acid under low stringency conditions, and/or
   (xiii) "NAKAP95" (SEQ ID No:82) or a functionally active derivative thereof, or a functionally active fragment thereof, or a homolog thereof, or a variant of "NAKAP95" encoded by a nucleic acid that hybridizes to the "NAKAP95" nucleic acid under low stringency conditions, and/or
   (xiv) "UXT PROTEIN" (SEQ ID No:233) or a functionally active derivative thereof, or a functionally active fragment thereof, or a homolog thereof, or a variant of "UXT PROTEIN" encoded by a nucleic acid that hybridizes to the "UXT PROTEIN" nucleic acid under low stringency conditions, and/or
   (xv) "FLJ31741" (SEQ ID No:234) or a functionally active derivative thereof, or a functionally active fragment thereof, or a homolog thereof, or a variant of "FLJ31741" encoded by a nucleic acid that hybridizes to the "FLJ31741" nucleic acid under low stringency conditions, and/or
   (xvi) "PREFOLDIN 6" (SEQ ID No:235) or a functionally active derivative thereof, or a functionally active fragment thereof, or a homolog thereof, or a variant of "PREFOLDIN 6" encoded by a nucleic acid that hybridizes to the "PREFOLDIN 6" nucleic acid under low stringency conditions, and/or
   (xvii) "TRAF-X" (SEQ ID No:236) or a functionally active derivative thereof, or a functionally active fragment thereof, or a homolog thereof, or a variant of "TRAF-X" encoded by a nucleic acid that hybridizes to the "TRAF-X" nucleic acid under low stringency conditions, and/or
   (xviii) "DJ310O13.3 (FRAGMENT)" (SEQ ID No:237) or a functionally active derivative thereof, or a functionally active fragment thereof, or a homolog thereof, or a variant of "DJ310O13.3 (FRAGMENT)" encoded by a nucleic acid that hybridizes to the "DJ310O13.3 (FRAGMENT)" nucleic acid under low stringency conditions, and/or
   (xix) "TOLL-LIKE RECEPT 3 PRECURSOR" (SEQ ID No:238) or a functionally active derivative thereof, or a functionally active fragment thereof, or a homolog thereof, or a variant of "TOLL-LIKE RECEPT 3 PRECURSOR" encoded by a nucleic acid that hybridizes to the "TOLL-LIKE RECEPT 3 PRECURSOR" nucleic acid under low stringency conditions,
   is present in the complex.
41. The complex of any one of No. 1 - 8, or proteins of No. 13 or the antibody or fragment of No. 17, for use in a method of diagnosing a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
42. A method for treating or preventing a disease or disorder characterized by an aberrant amount of, activity or component composition of or intracellular localization of, the complex of anyone of No. 1 - 8, comprising administering to a subject in need of such treatment or prevention a therapeutically effective amount of one or more molecules that modulate the amount of, , or protein components of, said complex.
43. The method according to No. 42, wherein said disease or disorder involves decreased levels of the amount or activity of said complex.
44. The method according to No. 42 , wherein said disease or disorder involves increased levels of the amount or activity of said complex.
45. Complex of any of No. 1 - 8 and/or protein selected from the following proteins
   (i) "RNA POLYMERASE II SUBUNIT 5-MEDIATING PROTEIN" (SEQ ID No:225) or a functionally active derivative thereof, or a functionally active fragment thereof, or a homolog thereof, or a variant of ""RNA POLYMERASE II SUBUNIT 5-MEDIATING PROTEIN" (SEQ ID No:225) or a functionally active derivative thereof, or a functionally active fragment thereof, or a homolog thereof, or a variant of "RNA POLYMERASE II SUBUNIT 5-MEDIATING PROTEIN" encoded by a nucleic acid that hybridizes to the "RNA POLYMERASE II SUBUNIT 5-MEDIATING PROTEIN""encoded by a nucleic acid that hybridizes to the ""RNA POLYMERASE II SUBUNIT 5-MEDIATING PROTEIN" (SEQ ID No:225) or a functionally active derivative thereof, or a functionally active fragment thereof, or a homolog thereof, or a variant of "RNA POLYMERASE II SUBUNIT 5-MEDIATING PROTEIN" encoded by a nucleic acid that hybridizes to the "RNA POLYMERASE II SUBUNIT 5-MEDIATING PROTEIN"" nucleic acid under low stringency conditions,
   (ii) "PEROXIREDOXIN 4" (SEQ ID No:226) or a functionally active derivative thereof, or a functionally active fragment thereof, or a homolog thereof, or a variant of "PEROXIREDOXIN 4" encoded by a nucleic acid that hybridizes to the "PEROXIREDOXIN 4" nucleic acid under low stringency conditions,
   (iii) "FANCONI ANEMIA COMPLEMENTATION GROUP D2 PROTEIN, ISOFORM 1" (SEQ ID No:18) or a functionally active derivative thereof, or a functionally active fragment thereof, or a homolog thereof, or a variant of "FANCONI ANEMIA COMPLEMENTATION GROUP D2 PROTEIN, ISOFORM 1" encoded by a nucleic acid that hybridizes to the "FANCONI ANEMIA COMPLEMENTATION GROUP D2 PROTEIN, ISOFORM 1" nucleic acid under low stringency conditions,
   (iv) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (v) "MEKK3" (SEQ ID No:227) or a functionally active derivative thereof, or a functionally active fragment thereof, or a homolog thereof, or a variant of "MEKK3" encoded by a nucleic acid that hybridizes to the "MEKK3" nucleic acid under low stringency conditions,
   (vi) "14-3-3ε" (SEQ ID No:228) or a functionally active derivative thereof, or a functionally active fragment thereof, or a homolog thereof, or a variant of "14-3-3ε" encoded by a nucleic acid that hybridizes to the "14-3-3ε" nucleic acid under low stringency conditions,
   (vii) "14-3-3 ζ/δ" (SEQ ID No:229) or a functionally active derivative thereof, or a functionally active fragment thereof, or a homolog thereof, or a variant of "14-3-3 ζ/δ" encoded by a nucleic acid that hybridizes to the "14-3-3 ζ/δ" nucleic acid under low stringency conditions,
   (viii) "MEK5" (SEQ ID No:230) or a functionally active derivative thereof, or a functionally active fragment thereof, or a homolog thereof, or a variant of "MEK5" encoded by a nucleic acid that hybridizes to the "MEK5" nucleic acid under low stringency conditions,
   (ix) "P30 DBC PROTEIN" (SEQ ID No:252) or a functionally active derivative thereof, or a functionally active fragment thereof, or a homolog thereof, or a variant of "P30 DBC PROTEIN" encoded by a nucleic acid that hybridizes to the "P30 DBC PROTEIN" nucleic acid under low stringency conditions,
   (x) "HSP 90-α" (SEQ ID No:105) or a functionally active derivative thereof, or a functionally active fragment thereof, or a homolog thereof, or a variant of "HSP 90-α" encoded by a nucleic acid that hybridizes to the "HSP90-α" nucleic acid under low stringency conditions,
   (xi) "HSP 90-β 1"(SEQ ID No:9) or a functionally active derivative thereof, or a functionally active fragment thereof, or a homolog thereof, or a variant of "HSP 90-β 1" encoded by a nucleic acid that hybridizes to the "HSP 90-β 1" nucleic acid under low stringency conditions,
   (xii) "PREFOLDIN 2" (SEQ ID No:232) or a functionally active derivative thereof, or a functionally active fragment thereof, or a homolog thereof, or a variant of "PREFOLDIN 2" encoded by a nucleic acid that hybridizes to the "PREFOLDIN 2" nucleic acid under low stringency conditions,
   (xiii) "NAKAP95" (SEQ ID No:82) or a functionally active derivative thereof, or a functionally active fragment thereof, or a homolog thereof, or a variant of "NAKAP95" encoded by a nucleic acid that hybridizes to the "NAKAP95" nucleic acid under low stringency conditions,
   (xiv) "UXT PROTEIN" (SEQ ID No:233) or a functionally active derivative thereof, or a functionally active fragment thereof, or a homolog thereof, or a variant of "UXT PROTEIN" encoded by a nucleic acid that hybridizes to the "UXT PROTEIN" nucleic acid under low stringency conditions,
   (xv) "FLJ31741" (SEQ ID No:234) or a functionally active derivative thereof, or a functionally active fragment thereof, or a homolog thereof, or a variant of "FLJ31741" encoded by a nucleic acid that hybridizes to the "FLJ31741" nucleic acid under low stringency conditions,
   (xvi) "PREFOLDIN 6" (SEQ ID No:235) or a functionally active derivative thereof, or a functionally active fragment thereof, or a homolog thereof, or a variant of "PREFOLDIN 6" encoded by a nucleic acid that hybridizes to the "PREFOLDIN 6" nucleic acid under low stringency conditions,
   (xvii) "TRAF-X" (SEQ ID No:236) or a functionally active derivative thereof, or a functionally active fragment thereof, or a homolog thereof, or a variant of "TRAF-X" encoded by a nucleic acid that hybridizes to the "TRAF-X" nucleic acid under low stringency conditions,
   (xviii) "DJ310O13.3 (FRAGMENT)" (SEQ ID No:237) or a functionally active derivative thereof,or a functionally active fragement thereof, or a homolog thereof, or a variant of "DJ310O13.3 (FRAGMENT)" that hybridizes to the "DJ310O13.3 (FRAGMENT)" nucleic acid under low stringency conditions, and
   (xix) "TOLL-LIKE RECEPT 3 PRECURSOR" (SEQ ID No:238) or a functionally active derivative thereof, or a functionally active fragment thereof, or a homolog thereof, or a variant of "TOLL-LIKE RECEPT 3 PRECURSOR" encoded by a nucleic acid that hybridizes to the "TOLL-LIKE RECEPT 3 PRECURSOR" nucleic acid under low stringency conditions,
as a target for an active agent of a pharmaceutical, preferably a drug target in the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.

The present invention further relates to the TAK1 protein complex
1. A protein complex selected from complex (I) and comprising
   (a) at least one first protein selected from the group consisting of:
      (i) "TRAF6" (SEQ ID No:189) or a functionally active derivative thereof, or a functionally active fragment thereof, or a homolog thereof, or a variant of "TRAF6" encoded by a nucleic acid that hybridizes to the "TRAF6" nucleic acid under low stringency conditions,
      (ii) "TAK1" (SEQ ID No:243) or a functionally active derivative thereof, or a functionally active fragment thereof, or a homolog thereof, or a variant of "TAK1" encoded by a nucleic acid that hybridizes to the "TAK1" nucleic acid under low stringency conditions,
      (iii) "TAB2 TAK1-BINDING PROTEIN 2 " (SEQ ID No:244) or a functionally active derivative thereof, or a functionally active fragment thereof, or a homolog thereof, or a variant of "TAB2 TAK1-BINDING PROTEIN 2" encoded by a nucleic acid that hybridizes to the "TAB2 TAK1-BINDING PROTEIN 2" nucleic acid under low stringency conditions, and
      (iv) "TAB1 TAK1-BINDING PROTEIN 1" (SEQ ID No:188) or a functionally active derivative thereof, or a functionally active fragment thereof, or a homolog thereof, or a variant of "TAB1 TAK1-BINDING PROTEIN 1" encoded by a nucleic acid that hybridizes to the "TAB1 TAK1-BINDING PROTEIN 1" nucleic acid under low stringency conditions, and
   (b) at least one second protein, which second protein is selected from the group consisting of:
      (i) "EIF4G2 (EUKARYOTIC TRANSLATION INITIATION FACTOR 4 γ2)" (SEQ ID No:239) or a functionally active derivative thereof, or a functionally active fragment thereof, or a homolog thereof, or a variant of "EIF4G2 (EUKARYOTIC TRANSLATION INITIATION FACTOR 4 γ2)" encoded by a nucleic acid that hybridizes to the "EIF4G2 (EUKARYOTIC TRANSLATION INITIATION FACTOR 4 γ2)" nucleic acid under low stringency conditions,
      (ii) "OLFACTORY REC EPTOR" (SEQ ID No:240) or a functionally active derivative thereof, or a functionally active fragment thereof, or a homolog thereof, or a variant of "OLFACTORY REC EPTOR" encoded by a nucleic acid that hybridizes to the "OLFACTORY REC EPTOR" nucleic acid under low stringency conditions,
      (iii) "XP_064648" (SEQ ID No:241) or a functionally active derivative thereof, or a functionally active fragment thereof, or a homolog thereof, or a variant of "XP_064648" encoded by a nucleic acid that hybridizes to the "XP_064648" nucleic acid under low stringency conditions,
      (iv) "PYPAF5 PYRIN-containing APAF1-like protein 5" (SEQ ID No:242) or a functionally active derivative thereof, or a functionally active fragment thereof, or a homolog thereof, or a variant of "PYPAF5 PYRIN-containing APAF1-like protein 5" encoded by a nucleic acid that hybridizes to the "PYPAF5 PYRIN-containing APAF1-like protein 5" nucleic acid under low stringency conditions,
      (v) "XP_088678" (SEQ ID No:245) or a functionally active derivative thereof, or a functionally active fragment thereof, or a homolog thereof, or a variant of "XP_088678" encoded by a nucleic acid that hybridizes to the "XP_088678" nucleic acid under low stringency conditions,
      (vi) "HSP90 α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions,
      (vii) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
      (viii) "HSP90 β 2" (SEQ ID No: 107) or a functionally active derivative thereof, or a functionally active fragment thereof, or a homolog thereof, or a variant of "HSP90 β 2" encoded by a nucleic acid that hybridizes to the "HSP90 β 2" nucleic acid under low stringency conditions, and
      (ix) "51 KDA FK506-BINDING PROTEIN" (SEQ ID No:106) or a functionally active derivative thereof, or a functionally active fragment thereof, or a homolog thereof, or a variant of "51 KDA FK506-BINDING PROTEIN" encoded by a nucleic acid that hybridizes to the "51 KDA FK506-BINDING PROTEIN" nucleic acid under low stringency conditions;
   and a complex (II) comprising at least two of said second proteins,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
2. The protein complex according to claim 1 wherein the first protein is the protein "TAK1" (SEQ ID No:243), or a functionally active derivative thereof, or a functionally active fragment thereof, or a homolog thereof, or a variant of "TAK1" encoded by a nucleic acid that hybridizes to the "TAK1" nucleic acid under low stringency conditions.
3. The protein complex according to No. 1 selected from complex (I) and comprising the following proteins:
   (i) "TAK1" (SEQ ID No:243) or a functionally active derivative thereof, or a functionally active fragment thereof, or a homolog thereof, or a variant of "TAK1" encoded by a nucleic acid that hybridizes to the "TAK1" nucleic acid under low stringency conditions,
   (ii) "TAB2 TAK1-BINDING PROTEIN 2" (SEQ ID No:244) or a functionally active derivative thereof, or a functionally active fragment thereof, or a homolog thereof, or a variant of "TAB2 TAK1 BINDING PROTEIN2" encoded by a nucleic acid that hybridizes to the "TAB2 TAK1-BINDING PROTEIN 2" nucleic acid under low stringency conditions,
   (iii) "TAB1 TAK1-BINDING PROTEIN 1" (SEQ ID No:188) or a functionally active derivative thereof, or a functionally active fragment thereof, or a homolog thereof, or a variant of "TAB1 TAK1-BINDING PROTIEN 1" encoded by a nucleic acid that hybridizes to the "TAB1 TAK1-BINDING PROTEIN 1" nucleic acid under low stringency conditions,
   (iv) "XP_088678" (SEQ ID No:245) or a functionally active derivative thereof, or a functionally active fragment thereof, or a homolog thereof, or a variant of "XP_088678" encoded by a nucleic acid that hybridizes to the "xP_088678" nucleic acid under low stringency conditions,
   (v) "HSP90 α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions,
   (vi) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (vii) "HSP90 β 2" (SEQ ID No:107) or a functionally active derivative thereof,or a functionally active fragement thereof, or a homolog thereof, or a variant of "Hsp90 β-2" that hybridizes to the "Hsp90 β-2" nucleic acid under low stringency conditions, and
   (viii) "51 KDA FK506-BINDING PROTEIN" (SEQ ID No:106) or a functionally active derivative thereof, or a functionally active fragment thereof, or a homolog thereof, or a variant of "51 KDA FK506-BINDING PROTEIN" encoded by a nucleic acid that hybridizes to the "51 KDA FK506-BINDING PROTEIN" nucleic acid under low stringency conditions;
   and a protein complex selected from complex (II) and comprising the following proteins:
   (i) "TAK1" (SEQ ID No:243) or a functionally active derivative thereof, or a functionally active fragment thereof, or a homolog thereof, or a variant of "TAK1" encoded by a nucleic acid that hybridizes to the "TAK1" nucleic acid under low stringency conditions,
   (ii) "TAB2 TAK1-BINDING PROTEIN 2" (SEQ ID No:244) or a functionally active derivative thereof, or a functionally active fragment thereof, or a homolog thereof, or a variant of "TAB2 TAK1 BINDING PROTEIN2" encoded by a nucleic acid that hybridizes to the "TAB2 TAK1-BINDING PROTEIN 2" nucleic acid under low stringency conditions,
   (iii) "TAB1 TAK1-BINDING PROTEIN 1" (SEQ ID No:188) or a functionally active derivative thereof, or a functionally active fragment thereof, or a homolog thereof, or a variant of "TAB1 TAK1-BINDING PROTIEN 1" encoded by a nucleic acid that hybridizes to the "TAB1 TAK1-BINDING PROTEIN 1" nucleic acid under low stringency conditions,
   (iv) "XP_088678" (SEQ ID No:245) or a functionally active derivative thereof, or a functionally active fragment thereof, or a homolog thereof, or a variant of "XP_088678" encoded by a nucleic acid that hybridizes to the "xP_088678" nucleic acid under low stringency conditions,
   (v) "HSP90 α" (SEQ ID No: 105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions,
   (vi) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (vii) "HSP90 β 2" (SEQ ID No:107) or a functionally active derivative thereof, or a functionally active fragment thereof, or a homolog thereof, or a variant of "Hsp90 β-2" encoded by a nucleic acid that hybridizes to the "Hsp90 β-2" nucleic acid under low stringency conditions,
   (viii) "51 KDA FK506-BINDING PROTEIN" (SEQ ID No:106) or a functionally active derivative thereof,or a functionally active fragement thereof, or a homolog thereof, or a variant of "51 KDA FK506-BINDING PROTEIN" that hybridizes to the "51 KDA FK506-BINDING PROTEIN" nucleic acid under low stringency conditions, and
   (ix) "EIF4G2 (EUKARYOTIC TRANSLATION INITIATION FACTOR 4 γ2)" (SEQ ID No:239) or a functionally active derivative thereof, or a functionally active fragment thereof, or a homolog thereof, or a variant of "EIF4G2 (EUKARYOTIC TRANSLATION INITIATION FACTOR 4 γ2)" encoded by a nucleic acid that hybridizes to the "EIF4G2 (EUKARYOTIC TRANSLATION INITIATION FACTOR 4 γ2)" nucleic acid under low stringency conditions;
   and a protein complex selected from complex (III) and comprising the following proteins:
   (i) "OLFACTORY REC EPTOR" (SEQ ID No:240) or a functionally active derivative thereof, or a functionally active fragment thereof, or a homolog thereof, or a variant of "OLFACTORY RECEPTOR" encoded by a nucleic acid that hybridizes to the "OLFACTORY RECEPTOR" nucleic acid under low stringency conditions,
   (ii) "XP_064648" (SEQ ID No:241) or a functionally active derivative thereof, or a functionally active fragment thereof, or a homolog thereof, or a variant of "XP_064648" encoded by a nucleic acid that hybridizes to the "XP_064648" nucleic acid under low stringency conditions,
   (iii) "PYPAF5 PYRIN-containing APAF1-like protein 5" (SEQ ID No:242) or a functionally active derivative thereof, or a functionally active fragment thereof, or a homolog thereof, or a variant of "PYPAF5 PYRIN-containing APAF1-like protein 5" encoded by a nucleic acid that hybridizes to the "PYPAF5 PYRIN-containing APAF1-like protein 5" nucleic acid under low stringency conditions,
   (iv) "TAK1" (SEQ ID No:243) or a functionally active derivative thereof, or a functionally active fragment thereof, or a homolog thereof, or a variant of "TAK1" encoded by a nucleic acid that hybridizes to the "TAK1" nucleic acid under low stringency conditions,
   (v) "TAB2 TAK1-BINDING PROTEIN 2 " (SEQ ID No:244) or a functionally active derivative thereof, or a functionally active fragment thereof, or a homolog thereof, or a variant of "TAB2 TAK1 BINDING PROTEIN2" encoded by a nucleic acid that hybridizes to the "TAB2 TAK1-BINDING PROTEIN 2" nucleic acid under low stringency conditions,
   (vi) "TAB1 TAK1-BINDING PROTEIN 1" (SEQ ID No:188) or a functionally active derivative thereof, or a functionally active fragment thereof, or a homolog thereof, or a variant of "TAB1 TAK1-BINDING PROTIEN 1" encoded by a nucleic acid that hybridizes to the "TAB1 TAK1-BINDING PROTEIN 1" nucleic acid under low stringency conditions,
   (vii) "XP_088678" (SEQ ID No:245) or a functionally active derivative thereof, or a functionally active fragment thereof, or a homolog thereof, or a variant of "XP_088678" encoded by a nucleic acid that hybridizes to the "xP_088678" nucleic acid under low stringency conditions,
   (viii) "HSP90 α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions,
   (ix) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (x) "HSP90 β2" (SEQ ID No:107) or a functionally active derivative thereof,or a functionally active fragement thereof, or a homolog thereof, or a variant of "Hsp90 β-2" that hybridizes to the "Hsp90 β-2" nucleic acid under low stringency conditions, and
   (xi) "51 KDA FK506-BINDING PROTEIN" (SEQ ID No:106) or a functionally active derivative thereof, or a functionally active fragment thereof, or a homolog thereof, or a variant of "51 KDA FK506-81NDING PROTEIN" encoded by a nucleic acid that hybridizes to the "51 KDA FK506-BINDING PROTEIN" nucleic acid under low stringency conditions,
   and a protein complex selected from complex (IV) and comprising the following proteins:
   (i) "EIF4G2 (EUKARYOTIC TRANSLATION INITIATION FACTOR 4 γ2)" (SEQ ID No:239) or a functionally active derivative thereof, or a functionally active fragment thereof, or a homolog thereof, or a variant of "EIF4G2 (EUKARYOTIC TRANSLATION INITIATION FACTOR 4 γ 2)" encoded by a nucleic acid that hybridizes to the "EIF4G2 (EUKARYOTIC TRANSLATION INITIATION FACTOR 4 γ2)" nucleic acid under low stringency conditions,
   (ii) "OLFACTORY REC EPTOR" (SEQ ID No:240) or a functionally active derivative thereof, or a functionally active fragment thereof, or a homolog thereof, or a variant of "OLFACTORY RECEPTOR" encoded by a nucleic acid that hybridizes to the "OLFACTORY RECEPTOR" nucleic acid under low stringency conditions,
   (iii) "XP_064648" (SEQ ID No:241) or a functionally active derivative thereof, or a functionally active fragment thereof, or a homolog thereof, or a variant of "XP_064648" encoded by a nucleic acid that hybridizes to the "XP_064648" nucleic acid under low stringency conditions,
   (iv) "PYPAF5 PYRIN-containing APAF1-like protein 5" (SEQ ID No:242) or a functionally active derivative thereof, or a functionally active fragment thereof, or a homolog thereof, or a variant of "PYPAF5 PYRIN-containing APAF1-like protein 5" encoded by a nucleic acid that hybridizes to the "PYPAF5 PYRIN-containing APAF1-like protein 5" nucleic acid under low stringency conditions,
   (v) "TRAF6" (SEQ ID No:189) or a functionally active derivative thereof, or a functionally active fragment thereof, or a homolog thereof, or a variant of "TRAF6" encoded by a nucleic acid that hybridizes to the "TRAF6" nucleic acid under low stringency conditions,
   (vi) "TAK1" (SEQ ID No:243) or a functionally active derivative thereof, or a functionally active fragment thereof, or a homolog thereof, or a variant of "TAK1" encoded by a nucleic acid that hybridizes to the "TAK1" nucleic acid under low stringency conditions,
   (vii) "TAB2 TAK1-BINDING PROTEIN 2 " (SEQ ID No:244) or a functionally active derivative thereof, or a functionally active fragment thereof, or a homolog thereof, or a variant of "TAB2 TAK1 BINDING PROTEIN2" encoded by a nucleic acid that hybridizes to the "TAB2 TAK1-BINDING PROTEIN 2" nucleic acid under low stringency conditions,
   (viii) "TAB1 TAK1-BIND1NG PROTEIN 1" (SEQ ID No:188) or a functionally active derivative thereof, or a functionally active fragment thereof, or a homolog thereof, or a variant of "TAB1 TAK1-BINDING PROTIEN 1" encoded by a nucleic acid that hybridizes to the "TAB1 TAK1-BINDING PROTEIN 1" nucleic acid under low stringency conditions,
   (ix) "XP_088678" (SEQ ID No:245) or a functionally active derivative thereof, or a functionally active fragment thereof, or a homolog thereof, or a variant of "XP_088678" encoded by a nucleic acid that hybridizes to the "xP_088678" nucleic acid under low stringency conditions,
   (x) "HSP90 α" (SEQ ID No: 105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions,
   (xi) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (xii) "HSP90 β2" (SEQ ID No:107) or a functionally active derivative thereof,or a functionally active fragement thereof, or a homolog thereof, or a variant of "Hsp90 β-2" that hybridizes to the "Hsp90 β-2" nucleic acid under low stringency conditions, and
   (xiii) "51 KDA FK506-BINDING PROTEIN" (SEQ ID No:106) or a functionally active derivative thereof, or a functionally active fragement thereof, or a homolog thereof, or a variant of "51 KDA FK506-BINDING PROTEIN" that hybridizes to the "51 KDA FK506-BINDING PROTEIN" nucleic acid under low stringency conditions.
4. The protein complex according to No. 1 selected from complex (I) comprising all but 1 - 8 of the following proteins:
   (i) "EIF4G2 (EUKARYOTIC TRANSLATION INITIATION FACTOR 4 γ2)" (SEQ ID No:239) or a functionally active derivative thereof, or a functionally active fragment thereof, or a homolog thereof, or a variant of "EIF4G2 (EUKARYOTIC TRANSLATION INITIATION FACTOR 4 γ2)" encoded by a nucleic acid that hybridizes to the "EIF4G2 (EUKARYOTIC TRANSLATION INITIATION FACTOR 4 γ2)" nucleic acid under low stringency conditions,
   (ii) "OLFACTORY REC EPTOR" (SEQ ID No:240) or a functionally active derivative thereof, or a functionally active fragment thereof, or a homolog thereof, or a variant of "OLFACTORY RECEPTOR" encoded by a nucleic acid that hybridizes to the "OLFACTORY RECEPTOR" nucleic acid under low stringency conditions,
   (iii) "XP_064648" (SEQ ID No:241) or a functionally active derivative thereof, or a functionally active fragment thereof, or a homolog thereof, or a variant of "XP_064648" encoded by a nucleic acid that hybridizes to the "XP_064648" nucleic acid under low stringency conditions,
   (iv) "PYPAF5 PYRIN-containing APAF1-like protein 5" (SEQ ID No:242) or a functionally active derivative thereof, or a functionally active fragment thereof, or a homolog thereof, or a variant of "PYPAF5 PYRIN-containing APAF1-like protein 5" encoded by a nucleic acid that hybridizes to the "PYPAF5 PYRIN-containing APAF1-like protein 5" nucleic acid under low stringency conditions,
   (v) "TRAF6" (SEQ ID No:189) or a functionally active derivative thereof, or a functionally active fragment thereof, or a homolog thereof, or a variant of "TRAF6" encoded by a nucleic acid that hybridizes to the "TRAF6" nucleic acid under low stringency conditions,
   (vi) "TAK1" (SEQ ID No:243) or a functionally active derivative thereof, or a functionally active fragment thereof, or a homolog thereof, or a variant of "TAK1" encoded by a nucleic acid that hybridizes to the "TAK1" nucleic acid under low stringency conditions,
   (vii) "TAB2 TAK1-BINDING PROTEIN 2 " (SEQ ID No:244) or a functionally active derivative thereof, or a functionally active fragment thereof, or a homolog thereof, or a variant of "TAB2 TAK1 BINDING PROTEIN2" encoded by a nucleic acid that hybridizes to the "TAB2 TAK1-BINDING PROTEIN 2" nucleic acid under low stringency conditions,
   (viii) "TAB1 TAK1-BINDING PROTEIN 1" (SEQ ID No:188) or a functionally active derivative thereof, or a functionally active fragment thereof, or a homolog thereof, or a variant of "TAB1 TAK1-BINDING PROTIEN 1" encoded by a nucleic acid that hybridizes to the "TAB1 TAK1-BINDING PROTEIN 1" nucleic acid under low stringency conditions,
   (ix) "XP_088678" (SEQ ID No:245) or a functionally active derivative thereof, or a functionally active fragment thereof, or a homolog thereof, or a variant of "XP_088678" encoded by a nucleic acid that hybridizes to the "xP_088678" nucleic acid under low stringency conditions,
   (x) "HSP90 α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions,
   (xi) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (xii) "HSP90 β2" (SEQ ID No: 107) or a functionally active derivative thereof,or a functionally active fragement thereof, or a homolog thereof, or a variant of "Hsp90 β-2" that hybridizes to the "Hsp90 β-2" nucleic acid under low stringency conditions, and
   (xiii) "51 KDA FK506-BINDING PROTEIN" (SEQ ID No:106) or a functionally active derivative thereof, or a functionally active fragment thereof, or a homolog thereof, or a variant of "51 KDA FK506-BINDING PROTEIN" encoded by a nucleic acid that hybridizes to the "51 KDA FK506-BINDING PROTEIN" nucleic acid under low stringency conditions;
   and a protein complex selected from complex (II) comprising all but 1 - 8 of the following proteins:
   (i) "EIF4G2 (EUKARYOTIC TRANSLATION INITIATION FACTOR 4 y2)" (SEQ ID No:239) or a functionally active derivative thereof, or a functionally active fragment thereof, or a homolog thereof, or a variant of "EIF4G2 (EUKARYOTIC TRANSLATION INITIATION FACTOR 4 γ2)" encoded by a nucleic acid that hybridizes to the "EIF4G2 (EUKARYOTIC TRANSLATION INITIATION FACTOR 4 γ2)" nucleic acid under low stringency conditions,
   (ii) "OLFACTORY REC EPTOR" (SEQ ID No:240) or a functionally active derivative thereof, or a functionally active fragment thereof, or a homolog thereof, or a variant of "OLFACTORY RECEPTOR" encoded by a nucleic acid that hybridizes to the "OLFACTORY RECEPTOR" nucleic acid under low stringency conditions,
   (iii) "XP_064648" (SEQ ID No:241) or a functionally active derivative thereof, or a functionally active fragment thereof, or a homolog thereof, or a variant of "XP_064648" encoded by a nucleic acid that hybridizes to the "XP_064648" nucleic acid under low stringency conditions,
   (iv) "PYPAF5 PYRIN-containing APAF1-like protein 5" (SEQ ID No:242) or a functionally active derivative thereof, or a functionally active fragment thereof, or a homolog thereof, or a variant of "PYPAF5 PYRIN-containing APAF1-like protein 5" encoded by a nucleic acid that hybridizes to the "PYPAF5 PYRIN-containing APAF1-like protein 5" nucleic acid under low stringency conditions,
   (v) "TAK1" (SEQ ID No:243) or a functionally active derivative thereof, or a functionally active fragment thereof, or a homolog thereof, or a variant of "TAK1" encoded by a nucleic acid that hybridizes to the "TAK1" nucleic acid under low stringency conditions,
   (vi) "TAB2 TAK1-BINDING PROTEIN 2" (SEQ ID No:244) or a functionally active derivative thereof, or a functionally active fragment thereof, or a homolog thereof, or a variant of "TAB2 TAK1 BINDING PROTEIN2" encoded by a nucleic acid that hybridizes to the "TAB2 TAK1-BINDING PROTEIN 2" nucleic acid under low stringency conditions,
   (vii) "TAB1 TAK1-BINDING PROTEIN 1" (SEQ ID No:188) or a functionally active derivative thereof, or a functionally active fragment thereof, or a homolog thereof, or a variant of "TAB1 TAK1-BINDING PROTIEN 1" encoded by a nucleic acid that hybridizes to the "TAB1 TAK1-BINDING PROTEIN 1" nucleic acid under low stringency conditions,
   (viii) "XP_088678" (SEQ ID No:245) or a functionally active derivative thereof, or a functionally active fragment thereof, or a homolog thereof, or a variant of "XP_088678" encoded by a nucleic acid that hybridizes to the "xP_088678" nucleic acid under low stringency conditions,
   (ix) "HSP90 α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions,
   (x) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (xi) "HSP90 β 2" (SEQ ID No:107) or a functionally active derivative thereof,or a functionally active fragement thereof, or a homolog thereof, or a variant of "Hsp90 β-2" that hybridizes to the "Hsp90 β-2" nucleic acid under low stringency conditions, and
   (xii) "51 KDA FK506-BINDING PROTEIN" (SEQ ID No:106) or a functionally active derivative thereof, or a functionally active fragment thereof, or a homolog thereof, or a variant of "51 KDA FK506-BINDING PROTEIN" encoded by a nucleic acid that hybridizes to the "51 KDA FK506-BINDING PROTEIN" nucleic acid under low stringency conditions.
5. The complex of any of No. 1 - 4 comprising a functionally active derivative of said first protein and/or a functionally active derivative of said second protein, wherein the functionally active derivative is a fusion protein comprising said first protein or said second protein fused to an amino acid sequence different from the first protein or second protein, respectively.
6. The complex of No. 5 wherein the functionally active derivative is a fusion protein comprising said first protein or said second protein fused to an affinity tag or label.
7. The complex of any of No. 1 - 4 comprising a fragment of said first protein and/or a fragment of said second protein, which fragment binds to another protein component of said complex.
8. The complex of any of No. 1 -7 that is involved in the TAK1 kinase activity.
9. A process for preparing complex of any of No. 1-8 and optionally the components thereof comprising the following steps:
   the expressing a protein (bait) of the complex, preferably a tagged protein, in a target cell, isolating the protein complex which is attached to the bait protein, and optionally disassociating the protein complex and isolating the individual complex members.
10. The process according to No. 9 wherein the tagged protein comprises two different tags which allow two separate affinity purification steps.
11. The process according to any of No. 9 - 10 wherein the two tags are separated by a cleavage site for a protease.
12. Component of the TAK1 complex obtainable by a process according to any of No. 9 to 11.
13. Protein of the TAK1 complex selected from
   (i) "XP_064648" (SEQ ID No:241) or a functionally active derivative thereof, or a functionally active fragment thereof, or a homolog thereof, or a variant of "XP_064648" encoded by a nucleic acid that hybridizes to the "XP_064648" nucleic acid under low stringency conditions, and
   (ii) "XP_088678" (SEQ ID No:245) or a functionally active derivative thereof, or a functionally active fragment thereof, or a homolog thereof, or a variant of "XP_088678" encoded by a nucleic acid that hybridizes to the "XP_088678" nucleic acid under low stringency conditions,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
14. Nucleic acid encoding a protein according to No. 13.
15. Construct, preferably a vector construct, comprising (a) a nucleic acid according to No. 14 and at least one further nucleic acid which is normally not associated with said nucleic acid, or (b) at least two separate nucleic acid sequences each encoding a different protein, or a functionally active fragment or a functionally active derivative thereof at least one of said proteins, or functionally active fragments or functionally active derivative thereof being selected from the first group of proteins according to No. 1(a) and at least one of said proteins, or functionally active fragments or functionally active derivative thereof being selected from the second group of proteins according to No. 1 (b).
16. Host cell, containing a vector comprising at least one of the nucleic acid of No. 14 and/or a construct of No. 15 or containing several vectors each comprising at least the nucleic acid sequence encoding at least one of the proteins, or functionally active fragments or functionally active derivatives thereof selected from the first group of proteins according to No. 1(a) and the proteins, or functionally active fragments or functionally active derivatives thereof selected from the second group of proteins according to No. 1(b).
17. An antibody or a fragment of said antibody containing the binding domain thereof, selected from an antibody or fragment thereof, which binds the complex of any of No. 1-8 and which does not bind any of the proteins of said complex when uncomplexed and an antibody or a fragment of said antibody which binds to any of the proteins according to No. 13.
18. A kit comprising in one or more container the complex of any of No. 1 - 8 and/or the proteins of No. 13 optionally together with an antibody according to No. 17 and/or further components such as reagents and working instructions.
19. The kit according to No. 18 for processing a substrate of said complex.
20. The kit according to No. 18 for the diagnosis or prognosis of a disease or a disease risk, preferentially for a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
21. Array, in which at least a complex according to any of No. 1 - 8 and/or at least one protein according to No. 14 and/or at least one antibody according to No. 17 is attached to a solid carrier.
22. A process for processing a physiological substrate of the complex comprising the step of bringing into contact a complex to any of No. 1 - 8 with said substrate, such that said substrate is processed.
23. A pharmaceutical composition comprising the protein complex of any of No. 1 to 8 and/or any of the following the proteins:
   (i) "XP_064648" (SEQ ID No:241) or a functionally active derivative thereof,or a functionally active fragement thereof, or a homolog thereof, or a variant of "XP_064648" that hybridizes to the "XP_064648" nucleic acid under low stringency conditions, and
   (ii) "XP_088678" (SEQ ID No:245) or a functionally active derivative thereof, or a functionally active fragment thereof, or a homolog thereof, or a variant of "XP_088678" encoded by a nucleic acid that hybridizes to the "xP_088678" nucleic acid under low stringency conditions,
   and a pharmaceutical acceptable carrier.
24. The pharmaceutical composition according to No. 23 for the treatment of diseases and disorders such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
25. A method for screening for a molecule that binds to the complex of anyone of No. 1 - 8 and/or any of the following proteins:
   (i) "XP_064648" (SEQ ID No:241) or a functionally active derivative thereof,or a functionally active fragement thereof, or a homolog thereof, or a variant of "XP_064648" that hybridizes to the "XP_064648" nucleic acid under low stringency conditions, and
   (ii) "XP_088678" (SEQ ID No:245) or a functionally active derivative thereof, or a functionally active fragment thereof, or a homolog thereof, or a variant of "XP_088678" encoded by a nucleic acid that hybridizes to the "XP_088678" nucleic acid under low stringency conditions, comprising the steps of
      (a) exposing said complex, or a cell or organism containing same to one or more candidate molecules; and
      (b) determinig whether said candidate molecule is bound to the complex or protein.
26. A method for screening for a molecule that modulates directly or indirectly the function, activity, composition or formation of the complex of any one of No. 1-8 comprising the steps of (a) exposing said complex, or a cell or organism containing TAK1 complex to one or more candidate molecules; and
   (b) determining the amount of activity of protein components of, and/or intracellular localization of, said complex and/or the transcription level of a gene dependent on the complex and/or the abundance and/or activity of a protein or protein complex dependent on the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependent on the complex and/or the abundance and/or activity of a protein or protein complex dependent on the function of the complex and/or product of a gene dependent on the complex in the absence of said candidate molecules indicates that the molecule modulates function, activity or composition of said complex.
27. The method of No. 26, wherein the amount of said complex is determined.
28. The method of No. 26, wherein the activity of said complex is determined.
29. The method of No. 28, wherein said determining step comprises isolating from the cell or organism said complex to produce said isolated complex and contacting said isolated complex in the presence or absence of a candidate molecule with a substrate of said complex and determining the processing of said substrate is modified in the presence of said candidate molecule.
30. The method of No. 26, wherein the amount of the individual protein components of said complex are determined.
31. The method of No. 30, determining step comprises determining whether
   (i) "EIF4G2 (EUKARYOTIC TRANSLATION INITIATION FACTOR 4 γ2)" (SEQ ID No:239) or a functionally active derivative thereof, or a functionally active fragment thereof, or a homolog thereof, or a variant of "EIF4G2 (EUKARYOTIC TRANSLATION INITIATION FACTOR 4 γ2)" encoded by a nucleic acid that hybridizes to the "EIF4G2 (EUKARYOTIC TRANSLATION INITIATION FACTOR 4 γ2)" nucleic acid under low stringency conditions, and/or
   (ii) "OLFACTORY REC EPTOR" (SEQ ID No:240) or a functionally active derivative thereof, or a functionally active fragment thereof, or a homolog thereof, or a variant of "OLFACTORY RECEPTOR" encoded by a nucleic acid that hybridizes to the "OLFACTORY RECEPTOR" nucleic acid under low stringency conditions, and/or
   (iii) "XP_064648" (SEQ ID No:241) or a functionally active derivative thereof, or a functionally active fragment thereof, or a homolog thereof, or a variant of "XP_064648" encoded by a nucleic acid that hybridizes to the "XP_064648" nucleic acid under low stringency conditions, and/or
   (iv) "PYPAF5 PYRIN-containing APAF1-like protein 5" (SEQ ID No:242) or a functionally active derivative thereof, or a functionally active fragment thereof, or a homolog thereof, or a variant of "PYPAF5 PYRIN-containing APAF1-like protein 5" encoded by a nucleic acid that hybridizes to the "PYPAF5 PYRIN-containing APAF1-like protein 5" nucleic acid under low stringency conditions, and/or
   (v) "TRAF6" (SEQ ID No:189) or a functionally active derivative thereof, or a functionally active fragment thereof, or a homolog thereof, or a variant of "TRAF6" encoded by a nucleic acid that hybridizes to the "TRAF6" nucleic acid under low stringency conditions, and/or
   (vi) "TAK1" (SEQ ID No:243) or a functionally active derivative thereof, or a functionally active fragment thereof, or a homolog thereof, or a variant of "TAK1" encoded by a nucleic acid that hybridizes to the "TAK1" nucleic acid under low stringency conditions, and/or
   (vii) "TAB2 TAK1-BINDING PROTEIN 2 " (SEQ ID No:244) or a functionally active derivative thereof, or a functionally active fragment thereof, or a homolog thereof, or a variant of "TAB2 TAK1 BINDING PROTEIN2" encoded by a nucleic acid that hybridizes to the "TAB2 TAK1-BINDING PROTEIN 2" nucleic acid under low stringency conditions, and/or
   (viii) "TAB1 TAK1-BINDING PROTEIN 1" (SEQ ID No:188) or a functionally active derivative thereof, or a functionally active fragment thereof, or a homolog thereof, or a variant of "TAB1 TAK1-BINDING PROTIEN 1" encoded by a nucleic acid that hybridizes to the "TAB1 TAK1-BINDING PROTEIN 1" nucleic acid under low stringency conditions, and/or
   (ix) "XP_088678" (SEQ ID No:245) or a functionally active derivative thereof, or a functionally active fragment thereof, or a homolog thereof, or a variant of "XP_088678" encoded by a nucleic acid that hybridizes to the "xP_088678" nucleic acid under low stringency conditions, and/or
   (x) "HSP90 α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions, and/or
   (xi) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions, and/or
   (xii) "HSP90 β 2" (SEQ ID No: 107) or a functionally active derivative thereof, or a functionally active fragment thereof, or a homolog thereof, or a variant of "Hsp90 β-2" encoded by a nucleic acid that hybridizes to the "Hsp90 β-2" nucleic acid under low stringency conditions, and/or
   (xiii) "51 KDA FK506-BINDING PROTEIN" (SEQ ID No:106) or a functionally active derivative thereof, or a functionally active fragment thereof, or a homolog thereof, or a variant of "51 KDA FK506-BINDING PROTEIN" encoded by a nucleic acid that hybridizes to the "51 KDA FK506-BINDING PROTEIN" nucleic acid under low stringency conditions,
   is present in the complex,
32. The method of any of No. 26 to 31, wherein said method is a method of screening for a drug for treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
33. Use of a molecule that modulates the amount of, activity of, or the protein components of the complex of any one of No. 1 - 8 for the manufacture of a medicament for the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
34. A method for the production of a pharmaceutical composition comprising carrying out the method of any of No. 1 - 8 to identify a molecule that modulates the function, activity, composition or formation of said complex, and further comprising mixing the identified molecule with a pharmaceutically acceptable carrier.
35. A method for diagnosing or screening for the presence of a disease or disorder or a predisposition for developing a disease or disorder in a subject, which disease or disorder is characterized by an aberrant amount of, activity of, or component composition of, or intracellular localization of the complex of any one of the No. 1 - 8, comprising determining the amount of, activity of, protein components of, and/or intracellular localization of, said complex and/or the transcription level of a gene dependent on the complex and/or the abundance and/or activity of a protein or protein complex dependent on the function of the complex and/or product of a gene dependent on the complex in a comparative sample derived from a subject, wherein a difference in said amount, activity, or protein components of, said complex in an analogous sample from a subject not having the disease or disorder or predisposition indicates the presence in the subject of the disease or disorder or predisposition in the subject.
36. The method of No. 35, wherein the amount of said complex is determined.
37. The method of No. 35, wherein the activity of said complex is determined.
38. The method of No. 37, wherein said determining step comprises isolating from the subject said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
39. The method of No. 35, wherein the amount of the individual protein components of said complex are determined.
40. The method of No. 39, wherein said determining step comprises determining whether
   (i) "EIF4G2 (EUKARYOTIC TRANSLATION INITIATION FACTOR 4 γ2)" (SEQ ID No:239) or a functionally active derivative thereof, or a functionally active fragment thereof, or a homolog thereof, or a variant of "EIF4G2 (EUKARYOTIC TRANSLATION INITIATION FACTOR 4 γ2)" encoded by a nucleic acid that hybridizes to the "EIF4G2 (EUKARYOTIC TRANSLATION INITIATION FACTOR 4 γ2)" nucleic acid under low stringency conditions, and/or
   (ii) "OLFACTORY REC EPTOR" (SEQ ID No:240) or a functionally active derivative thereof, or a functionally active fragment thereof, or a homolog thereof, or a variant of "OLFACTORY RECEPTOR" encoded by a nucleic acid that hybridizes to the "OLFACTORY RECEPTOR" nucleic acid under low stringency conditions, and/or
   (iii) "XP_064648" (SEQ ID No:241) or a functionally active derivative thereof, or a functionally active fragment thereof, or a homolog thereof, or a variant of "XP_064648" encoded by a nucleic acid that hybridizes to the "XP_064648" nucleic acid under low stringency conditions, and/or
   (iv) "PYPAF5 PYRIN-containing APAF1-like protein 5" (SEQ ID No:242) or a functionally active derivative thereof, or a functionally active fragment thereof, or a homolog thereof, or a variant of "PYPAF5 PYRIN-containing APAF1-like protein 5" encoded by a nucleic acid that hybridizes to the "PYPAF5 PYRIN-containing APAF1-like protein 5" nucleic acid under low stringency conditions, and/or
   (v) "TRAF6" (SEQ ID No:189) or a functionally active derivative thereof, or a functionally active fragment thereof, or a homolog thereof, or a variant of "TRAF6" encoded by a nucleic acid that hybridizes to the "TRAF6" nucleic acid under low stringency conditions, and/or
   (vi) "TAK1" (SEQ ID No:243) or a functionally active derivative thereof, or a functionally active fragment thereof, or a homolog thereof, or a variant of "TAK1" encoded by a nucleic acid that hybridizes to the "TAK1" nucleic acid under low stringency conditions, and/or
   (vii) "TAB2 TAK1-BINDING PROTEIN 2 " (SEQ ID No:244) or a functionally active derivative thereof, or a functionally active fragment thereof, or a homolog thereof, or a variant of "TAB2 TAK1 BINDING PROTEIN2" encoded by a nucleic acid that hybridizes to the "TAB2 TAK1-BINDING PROTEIN 2" nucleic acid under low stringency conditions, and/or
   (viii) "TAB1 TAK1-BINDING PROTEIN 1" (SEQ ID No:188) or a functionally active derivative thereof, or a functionally active fragment thereof, or a homolog thereof, or a variant of "TAB1 TAK1-BINDING PROTIEN 1" encoded by a nucleic acid that hybridizes to the "TAB1 TAK1-BINDING PROTEIN 1" nucleic acid under low stringency conditions, and/or
   (ix) "XP_088678" (SEQ ID No:245) or a functionally active derivative thereof, or a functionally active fragment thereof, or a homolog thereof, or a variant of "XP_088678" encoded by a nucleic acid that hybridizes to the "xP_088678" nucleic acid under low stringency conditions, and/or
   (x) "HSP90 α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions, and/or
   (xi) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions, and/or
   (xii) "HSP90 B 2" (SEQ ID No:107) or a functionally active derivative thereof, or a functionally active fragment thereof, or a homolog thereof, or a variant of "Hsp90 β-2" encoded by a nucleic acid that hybridizes to the "Hsp90 β-2" nucleic acid under low stringency conditions, and/or
   (xiii) "51 KDA FK506-BINDING PROTEIN" (SEQ ID No:106) or a functionally active derivative thereof, or a functionally active fragment thereof, or a homolog thereof, or a variant of "51 KDA FK506-BINDING PROTEIN" encoded by a nucleic acid that hybridizes to the "51 KDA FK506-BINDING PROTEIN" nucleic acid under low stringency conditions,
   is present in the complex.
41. The complex of any one of No. 1 - 8, or proteins of No. 13 or the antibody or fragment of No. 17, for use in a method of diagnosing a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
42. A method for treating or preventing a disease or disorder characterized by an aberrant amount of, activity or component composition of or intracellular localization of, the complex of anyone of No. 1-8, comprising administering to a subject in need of such treatment or prevention a therapeutically effective amount of one or more molecules that modulate the amount of, TAK1 kinase activity, or protein components of, said complex.
43. The method according to No. 42, wherein said disease or disorder involves decreased levels of the amount or activity of said complex.
44. The method according to No. 42 , wherein said disease or disorder involves increased levels of the amount or activity of said complex.
45. Complex of any of No. 1 - 8 and/or protein selected from the following proteins
   (i) "EIF4G2 (EUKARYOTIC TRANSLATION INITIATION FACTOR 4 γ2)" (SEQ ID No:239) or a functionally active derivative thereof, or a functionally active fragment thereof, or a homolog thereof, or a variant of "EIF4G2 (EUKARYOTIC TRANSLATION INITIATION FACTOR 4 γ2)" encoded by a nucleic acid that hybridizes to the "EIF4G2 (EUKARYOTIC TRANSLATION INITIATION FACTOR 4 γ2)" nucleic acid under low stringency conditions,
   (ii) "OLFACTORY REC EPTOR" (SEQ ID No:240) or a functionally active derivative thereof, or a functionally active fragment thereof, or a homolog thereof, or a variant of "OLFACTORY RECEPTOR" encoded by a nucleic acid that hybridizes to the "OLFACTORY RECEPTOR" nucleic acid under low stringency conditions,
   (iii) "XP_064648" (SEQ ID No:241) or a functionally active derivative thereof, or a functionally active fragment thereof, or a homolog thereof, or a variant of "XP_064648" encoded by a nucleic acid that hybridizes to the "XP_064648" nucleic acid under low stringency conditions,
   (iv) "PYPAF5 PYRIN-containing APAF1-like protein 5" (SEQ ID No:242) or a functionally active derivative thereof, or a functionally active fragment thereof, or a homolog thereof, or a variant of "PYPAF5 PYRIN-containing APAF1-like protein 5" encoded by a nucleic acid that hybridizes to the "PYPAF5 PYRIN-containing APAF1-like protein 5" nucleic acid under low stringency conditions,
   (v) "TRAF6" (SEQ ID No:189) or a functionally active derivative thereof, or a functionally active fragment thereof, or a homolog thereof, or a variant of "TRAF6" encoded by a nucleic acid that hybridizes to the "TRAF6" nucleic acid under low stringency conditions,
   (vi) "TAK1" (SEQ ID No:243) or a functionally active derivative thereof, or a functionally active fragment thereof, or a homolog thereof, or a variant of "TAK1" encoded by a nucleic acid that hybridizes to the "TAK1" nucleic acid under low stringency conditions,
   (vii) "TAB2 TAK1-BINDING PROTEIN 2 " (SEQ ID No:244) or a functionally active derivative thereof, or a functionally active fragment thereof, or a homolog thereof, or a variant of "TAB2 TAK1 BINDING PROTEIN2" encoded by a nucleic acid that hybridizes to the "TAB2 TAK1-BINDING PROTEIN 2" nucleic acid under low stringency conditions,
   (viii) "TAB1 TAK1-BINDING PROTEIN 1" (SEQ ID No:188) or a functionally active derivative thereof, or a functionally active fragment thereof, or a homolog thereof, or a variant of "TAB1 TAK1-BINDING PROTIEN 1" encoded by a nucleic acid that hybridizes to the "TAB1 TAK1-BINDING PROTEIN 1" nucleic acid under low stringency conditions,
   (ix) "XP_088678" (SEQ ID No:245) or a functionally active derivative thereof, or a functionally active fragment thereof, or a homolog thereof, or a variant of "XP_088678" encoded by a nucleic acid that hybridizes to the "xP_088678" nucleic acid under low stringency conditions,
   (x) "HSP90 α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions,
   (xi) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (xii) "HSP90 β2" (SEQ ID No:107) or a functionally active derivative thereof,or a functionally active fragement thereof, or a homolog thereof, or a variant of "HSP90 β-2" that hybridizes to the "HSP90 β-2" nucleic acid under low stringency conditions, and
   (xiii) "51 KDA FK506-BINDING PROTEIN" (SEQ ID No:106) or a functionally active derivative thereof, or a functionally active fragment thereof, or a homolog thereof, or a variant of "51 KDA FK506-BINDING PROTEIN" encoded by a nucleic acid that hybridizes to the "51 KDA FK506-BINDING PROTEIN" nucleic acid under low stringency conditions,
   as a target for an active agent of a pharmaceutical, preferably a drug target in the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.

The present invention further relates to the MEKK1 protein complex
1. A protein complex selected from complex (I) and comprising
   (a) at least one first protein selected from the group consisting of:
      (i) "14-3-3 ζ/δ" (SEQ ID No:229) or a functionally active derivative thereof, or a functionally active fragment thereof, or a homolog thereof, or a variant of "14-3-3 ζ/δ" encoded by a nucleic acid that hybridizes to the "14-3-3 ζ/δ" nucleic acid under low stringency conditions,
      (ii) "14-3-3 ε" (SEQ ID No:228) or a functionally active derivative thereof, or a functionally active fragment thereof, or a homolog thereof, or a variant of "14-3-3 ε" encoded by a nucleic acid that hybridizes to the "14-3-3 ε" nucleic acid under low stringency conditions,
      (iii) "MEKK1" (SEQ ID No:251) or a functionally active derivative thereof, or a functionally active fragment thereof, or a homolog thereof, or a variant of "MEKK1" encoded by a nucleic acid that hybridizes to the "MEKK1" nucleic acid under low stringency conditions, and
      (iv) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions, and
   (b) at least one second protein, which second protein is selected from the group consisting of:
      (i) "P40 NUCLEOLAR PROTEIN" (SEQ ID No:246) or a functionally active derivative thereof, or a functionally active fragment thereof, or a homolog thereof, or a variant of "P40 NUCLEOLAR PROTEIN" encoded by a nucleic acid that hybridizes to the "P40 NUCLEOLAR PROTEIN" nucleic acid under low stringency conditions,
      (ii) "MELANOMA-ASSOCIATED ANTIGEN D1" (SEQ ID No:247) or a functionally active derivative thereof, or a functionally active fragment thereof, or a homolog thereof, or a variant of "MELANOMA-ASSOCIATED ANTIGEN D1" encoded by a nucleic acid that hybridizes to the "MELANOMA-ASSOCIATED ANTIGEN D1" nucleic acid under low stringency conditions,
      (iii) "COBW-LIKE PROTEIN" (SEQ ID No:248) or a functionally active derivative thereof, or a functionally active fragment thereof, or a homolog thereof, or a variant of "COBW-LIKE PROTEIN" encoded by a nucleic acid that hybridizes to the "COBW-LIKE PROTEIN" nucleic acid under low stringency conditions,
      (iv) "SEB4B (FRAGMENT)" (SEQ ID No:249) or a functionally active derivative thereof, or a functionally active fragment thereof, or a homolog thereof, or a variant of "SEB4B (FRAGMENT)" encoded by a nucleic acid that hybridizes to the "SEB4B (FRAGMENT)" nucleic acid under low stringency conditions,
      (v) "HSP 90-β1" (SEQ ID No:9) or a functionally active derivative thereof, or a functionally active fragment thereof, or a homolog thereof, or a variant of "HSP 90-β 1"encoded by a nucleic acid that hybridizes to the "HSP 90-β 1"nucleic acid under low stringency conditions,
      (vi) "FAF-X UBIQUITIN C-TERM HYDRO- LASE" (SEQ ID No:250) or a functionally active derivative thereof, or a functionally active fragment thereof, or a homolog thereof, or a variant of "FAF-X UBIQUITIN C-TERM HYDRO- LASE" encoded by a nucleic acid that hybridizes to the "FAF-X UBIQUITIN C-TERM HYDRO- LASE" nucleic acid under low stringency conditions,
      (vii) "HSP 90-α" (SEQ ID No:105) or a functionally active derivative thereof, or a functionally active fragment thereof, or a homolog thereof, or a variant of "HSP 90-α" encoded by a nucleic acid that hybridizes to the "HSP 90-α" nucleic acid under low stringency conditions,
      (viii) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
      (ix) "P30 DBC PROTEIN" (SEQ ID No:252) or a functionally active derivative thereof, or a functionally active fragment thereof, or a homolog thereof, or a variant of "P30 DBC PROTEIN" encoded by a nucleic acid that hybridizes to the "P30 DBC PROTEIN" nucleic acid under low stringency conditions,
      (x) "G(I)/G(S)/G(O) γ-12 SUBUNIT, GUANINE NUCLEOTIDE-BINDING PROTEIN" (SEQ ID No:112) or a functionally active derivative thereof, or a functionally active fragment thereof, or a homolog thereof, or a variant of "G(I)/G(S)/G(O) γ-12 SUBUNIT, GUANINE NUCLEOTIDE-BINDING PROTEIN" encoded by a nucleic acid that hybridizes to the "G(I)/G(S)/G(O) y-12 SUBUNIT, GUANINE NUCLEOTIDE-BINDING PROTEIN nucleic acid under low stringency conditions,
      (xi) "51 KDA FK506-BINDING PROTEIN" (SEQ ID No: 106) or a functionally active derivative thereof, or a functionally active fragment thereof, or a homolog thereof, or a variant of "51 KDA FK506-BINDING PROTEIN" encoded by a nucleic acid that hybridizes to the "51 KDA FK506-BINDING PROTEIN" nucleic acid under low stringency conditions,
      (xii) "WD-REPEAT AN11 HOMOLOG" (SEQ ID No:253) or a functionally active derivative thereof, or a functionally active fragment thereof, or a homolog thereof, or a variant of "WD-REPEAT AN11 HOMOLOG" encoded by a nucleic acid that hybridizes to the "WD-REPEAT AN11 HOMOLOG" nucleic acid under low stringency conditions, and
      (xiii) "BAG-FAMILY MOLECULAR CHAPERONE REGULATOR-5" (SEQ ID No:254) or a functionally active derivative thereof, or a functionally active fragment thereof, or a homolog thereof, or a variant of "BAG-FAMILY MOLECULAR CHAPERONE REGULATOR-5" encoded by a nucleic acid that hybridizes to the "BAG-FAMILY MOLECULAR CHAPERONE REGULATOR-5" nucleic acid under low stringency conditions;
and a complex (II) comprising at least two of said second proteins,
wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
2. The protein complex according to claim 1 wherein the first protein is the protein "MEKK1" (SEQ ID No:251), or a functionally active derivative thereof, or a functionally active fragment thereof, or a homolog thereof, or a variant of "MEKK1" encoded by a nucleic acid that hybridizes to the "MEKK1" nucleic acid under low stringency conditions.
3. The protein complex according to No. 1 selected from complex (I) and comprising the following proteins:
   (i) "HSP 90-β 1"(SEQ ID No:9) or a functionally active derivative thereof, or a functionally active fragment thereof, or a homolog thereof, or a variant of "HSP 90-β1" encoded by a nucleic acid that hybridizes to the "HSP 90-β 1" nucleic acid under low stringency conditions,
   (ii) "HSP 90-α" (SEQ ID No: 105) or a functionally active derivative thereof, or a functionally active fragment thereof, or a homolog thereof, or a variant of "HSP 90-α" encoded by a nucleic acid that hybridizes to the "HSP90-α" nucleic acid under low stringency conditions,
   (iii) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (iv) "MEKK1" (SEQ ID No:251) or a functionally active derivative thereof, or a functionally active fragment thereof, or a homolog thereof, or a variant of "MEKK1" encoded by a nucleic acid that hybridizes to the "MEKK1" nucleic acid under low stringency conditions,
   (v) "51 KDA FK506-BINDING PROTEIN" (SEQ ID No:106) or a functionally active derivative thereof,or a functionally active fragement thereof, or a homolog thereof, or a variant of "51 KDA FK506-BINDING PROTEIN" that hybridizes to the "51 KDA FK506-BINDING PROTEIN" nucleic acid under low stringency conditions, and
   (vi) "WD-REPEAT AN11 HOMOLOG" (SEQ ID No:253) or a functionally active derivative thereof, or a functionally active fragment thereof, or a homolog thereof, or a variant of "WD-REPEAT AN11 HOMOLOG" encoded by a nucleic acid that hybridizes to the "WD-REPEAT AN11 HOMOLOG" nucleic acid under low stringency conditions;
   and a protein complex selected from complex (II) and comprising the following proteins:
   (i) "P40 NUCLEOLAR PROTEIN" (SEQ ID No:246) or a functionally active derivative thereof, or a functionally active fragment thereof, or a homolog thereof, or a variant of "P40 NUCLEOLAR PROTEIN" encoded by a nucleic acid that hybridizes to the "P40 NUCLEOLAR PROTEIN" nucleic acid under low stringency conditions,
   (ii) "MELANOMA-ASSOCIATED ANTIGEN D1" (SEQ ID No:247) or a functionally active derivative thereof, or a functionally active fragment thereof, or a homolog thereof, or a variant of "MELANOMA-ASSOCIATED ANTIGEN D1" encoded by a nucleic acid that hybridizes to the "MELANOMA-ASSOCIATED ANTIGEN D1" nucleic acid under low stringency conditions,
   (iii) "COBW-LIKE PROTEIN" (SEQ ID No:248) or a functionally active derivative thereof, or a functionally active fragment thereof, or a homolog thereof, or a variant of "COBW-LIKE PROTEIN" encoded by a nucleic acid that hybridizes to the "COBW-LIKE PROTEIN" nucleic acid under low stringency conditions,
   (iv) "SEB4B (FRAGMENT)" (SEQ ID No:249) or a functionally active derivative thereof, or a functionally active fragment thereof, or a homolog thereof, or a variant of "SEB4B (FRAGMENT)" encoded by a nucleic acid that hybridizes to the "SEB4B (FRAGMENT)" nucleic acid under low stringency conditions,
   (v) "HSP 90-β1" (SEQ ID No:9) or a functionally active derivative thereof, or a functionally active fragment thereof, or a homolog thereof, or a variant of "HSP 90-β1" encoded by a nucleic acid that hybridizes to the "HSP 90-β1" nucleic acid under low stringency conditions,
   (vi) "FAF-X UBIQUITIN C-TERM HYDRO- LASE" (SEQ ID No:250) or a functionally active derivative thereof, or a functionally active fragment thereof, or a homolog thereof, or a variant of "FAF-X UBIQUITIN C-TERM HYDRO- LASE" encoded by a nucleic acid that hybridizes to the "FAF-X UBIQUITIN C-TERM HYDRO- LASE" nucleic acid under low stringency conditions,
   (vii) "HSP 90-α" (SEQ ID No:105) or a functionally active derivative thereof, or a functionally active fragment thereof, or a homolog thereof, or a variant of "HSP 90-α" encoded by a nucleic acid that hybridizes to the "HSP90-α" nucleic acid under low stringency conditions,
   (viii) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (ix) "14-3-3 ζ/δ" (SEQ ID No:229) or a functionally active derivative thereof, or a functionally active fragment thereof, or a homolog thereof, or a variant of "14-3-3 ζ/δ" encoded by a nucleic acid that hybridizes to the "14-3-3 ζ/δ" nucleic acid under low stringency conditions,
   (x) "14-3-3 ε" (SEQ ID No:228) or a functionally active derivative thereof, or a functionally active fragment thereof, or a homolog thereof, or a variant of "14-3-3 ε" encoded by a nucleic acid that hybridizes to the "14-3-3 ε" nucleic acid under low stringency conditions,
   (xi) "MEKK1" (SEQ ID No:251) or a functionally active derivative thereof, or a functionally active fragment thereof, or a homolog thereof, or a variant of "MEKK1" encoded by a nucleic acid that hybridizes to the "MEKK1" nucleic acid under low stringency conditions,
   (xii) "P30 DBC PROTEIN" (SEQ ID No:252) or a functionally active derivative thereof, or a functionally active fragment thereof, or a homolog thereof, or a variant of "P30 DBC PROTEIN" encoded by a nucleic acid that hybridizes to the "P30 DBC PROTEIN" nucleic acid under low stringency conditions,
   (xiii) "G(I)/G(S)/G(O) γ-12 SUBUNIT, GUANINE NUCLEOTIDE-BINDING PROTEIN" (SEQ ID No:112) or a functionally active derivative thereof, or a functionally active fragment thereof, or a homolog thereof, or a variant of "G(I)/G(S)/G(O) γ-12 SUBUNIT, GUANINE NUCLEOTIDE-BINDING PROTEIN" encoded by a nucleic acid that hybridizes to the "G(I)/G(S)/G(O) γ-12 SUBUNIT, GUANINE NUCLEOTIDE-BINDING PROTEIN" nucleic acid under low stringency conditions,
   (xiv) "51 KDA FK506-BINDING PROTEIN" (SEQ ID No:106) or a functionally active derivative thereof, or a functionally active fragment thereof, or a homolog thereof, or a variant of "51 KDA FK506-BINDING PROTEIN" encoded by a nucleic acid that hybridizes to the "51 KDA FK506-BINDING PROTEIN" nucleic acid under low stringency conditions,
   (xv) "WD-REPEAT AN11 HOMOLOG" (SEQ ID No:253) or a functionally active derivative thereof,or a functionally active fragement thereof, or a homolog thereof, or a variant of "WD-REPEAT AN11 HOMOLOG" that hybridizes to the "WD-REPEAT AN11 HOMOLOG" nucleic acid under low stringency conditions, and
   (xvi) "BAG-FAMILY MOLECULAR CHAPERONE REGULATOR-5" (SEQ ID No:254) or a functionally active derivative thereof, or a functionally active fragment thereof, or a homolog thereof, or a variant of "BAG-FAMILY MOLECULAR CHAPERONE REGULATOR-5" encoded by a nucleic acid that hybridizes to the "BAG-FAMILY MOLECULAR CHAPERONE REGULATOR-5" nucleic acid under low stringency conditions;
   and a protein complex selected from complex (III) and comprising the following proteins:
   (i) "P40 NUCLEOLAR PROTEIN" (SEQ ID No:246) or a functionally active derivative thereof, or a functionally active fragment thereof, or a homolog thereof, or a variant of "P40 NUCLEOLAR PROTEIN" encoded by a nucleic acid that hybridizes to the "P40 NUCLEOLAR PROTEIN" nucleic acid under low stringency conditions,
   (ii) "MELANOMA-ASSOCIATED ANTIGEN D1" (SEQ ID No:247) or a functionally active derivative thereof, or a functionally active fragment thereof, or a homolog thereof, or a variant of "MELANOMA-ASSOCIATED ANTIGEN D1" encoded by a nucleic acid that hybridizes to the "MELANOMA-ASSOCIATED ANTIGEN D1" nucleic acid under low stringency conditions,
   (iii) "COBW-LIKE PROTEIN" (SEQ ID No:248) or a functionally active derivative thereof, or a functionally active fragment thereof, or a homolog thereof, or a variant of "COBW-LIKE PROTEIN" encoded by a nucleic acid that hybridizes to the "COBW-LIKE PROTEIN" nucleic acid under low stringency conditions,
   (iv) "SEB4B (FRAGMENT)" (SEQ ID No:249) or a functionally active derivative thereof, or a functionally active fragment thereof, or a homolog thereof, or a variant of "SEB4B (FRAGMENT)" encoded by a nucleic acid that hybridizes to the "SEB4B (FRAGMENT)" nucleic acid under low stringency conditions,
   (v) "HSP 90-β 1 "(SEQ ID No:9) or a functionally active derivative thereof, or a functionally active fragment thereof, or a homolog thereof, or a variant of "HSP 90-β 1" encoded by a nucleic acid that hybridizes to the "HSP 90-β 1" nucleic acid under low stringency conditions,
   (vi) "FAF-X UBIQUITIN C-TERM HYDRO- LASE" (SEQ ID No:250) or a functionally active derivative thereof, or a functionally active fragment thereof, or a homolog thereof, or a variant of "FAF-X UBIQUITIN C-TERM HYDRO- LASE" encoded by a nucleic acid that hybridizes to the "FAF-X UBIQUITIN C-TERM HYDRO- LASE" nucleic acid under low stringency conditions,
   (vii) "HSP 90-α" (SEQ ID No:105) or a functionally active derivative thereof, or a functionally active fragment thereof, or a homolog thereof, or a variant of "HSP 90-α" encoded by a nucleic acid that hybridizes to the "HSP90-α" nucleic acid under low stringency conditions,
   (viii) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (ix) "14-3-3 ζ/δ" (SEQ ID No:229) or a functionally active derivative thereof, or a functionally active fragment thereof, or a homolog thereof, or a variant of "14-3-3 ζ/δ" encoded by a nucleic acid that hybridizes to the "14-3-3 ζ/δ" nucleic acid under low stringency conditions,
   (x) "14-3-3 ε" (SEQ ID No:228) or a functionally active derivative thereof, or a functionally active fragment thereof, or a homolog thereof, or a variant of "14-3-3 ε" encoded by a nucleic acid that hybridizes to the "14-3-3 ε" nucleic acid under low stringency conditions,
   (xi) "MEKK1" (SEQ ID No:251) or a functionally active derivative thereof, or a functionally active fragment thereof, or a homolog thereof, or a variant of "MEKK1" encoded by a nucleic acid that hybridizes to the "MEKK1" nucleic acid under low stringency conditions,
   (xii) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions,
   (xiii) "P30 DBC PROTEIN" (SEQ ID No:252) or a functionally active derivative thereof, or a functionally active fragment thereof, or a homolog thereof, or a variant of "P30 DBC PROTEIN" encoded by a nucleic acid that hybridizes to the "P30 DBC PROTEIN" nucleic acid under low stringency conditions,
   (xiv) "G(I)/G(S)/G(O) γ-12 SUBUNIT, GUANINE NUCLEOTIDE-BINDING PROTEIN" (SEQ ID No:112) or a functionally active derivative thereof, or a functionally active fragment thereof, or a homolog thereof, or a variant of "G(I)/G(S)/G(O) γ-12 SUBUNIT, GUANINE NUCLEOTIDE-BINDING PROTEIN" encoded by a nucleic acid that hybridizes to the "G(I)/G(S)/G(O) γ-12 SUBUNIT, GUANINE NUCLEOTIDE-BINDING PROTEIN" nucleic acid under low stringency conditions,
   (xv) "51 KDA FK506-BINDING PROTEIN" (SEQ ID No:106) or a functionally active derivative thereof, or a functionally active fragment thereof, or a homolog thereof, or a variant of "51 KDA FK506-BINDING PROTEIN" encoded by a nucleic acid that hybridizes to the "51 KDA FK506-BINDING PROTEIN" nucleic acid under low stringency conditions,
   (xvi) "WD-REPEAT AN11 HOMOLOG" (SEQ ID No:253) or a functionally active derivative thereof,or a functionally active fragement thereof, or a homolog thereof, or a variant of "WD-REPEAT AN11 HOMOLOG" that hybridizes to the "WD-REPEAT AN11 HOMOLOG" nucleic acid under low stringency conditions, and
   (xvii) "BAG-FAMILY MOLECULAR CHAPERONE REGULATOR-5" (SEQ ID No:254) or a functionally active derivative thereof, or a functionally active fragment thereof, or a homolog thereof, or a variant of "BAG-FAMILY MOLECULAR CHAPERONE REGULATOR-5" encoded by a nucleic acid that hybridizes to the "BAG-FAMILY MOLECULAR CHAPERONE REGULATOR-5" nucleic acid under low stringency conditions.
4. The protein complex according to No. 1 selected from complex (I) comprising all but 1 - 12 of the following proteins:
   (i) "P40 NUCLEOLAR PROTEIN" (SEQ ID No:246) or a functionally active derivative thereof, or a functionally active fragment thereof, or a homolog thereof, or a variant of "P40 NUCLEOLAR PROTEIN" encoded by a nucleic acid that hybridizes to the "P40 NUCLEOLAR PROTEIN" nucleic acid under low stringency conditions,
   (ii) "MELANOMA-ASSOCIATED ANTIGEN D1" (SEQ ID No:247) or a functionally active derivative thereof, or a functionally active fragment thereof, or a homolog thereof, or a variant of "MELANOMA-ASSOCIATED ANTIGEN D1" encoded by a nucleic acid that hybridizes to the "MELANOMA-ASSOCIATED ANTIGEN D1" nucleic acid under low stringency conditions,
   (iii) "COBW-LIKE PROTEIN" (SEQ ID No:248) or a functionally active derivative thereof, or a functionally active fragment thereof, or a homolog thereof, or a variant of "COBW-LlKE PROTEIN" encoded by a nucleic acid that hybridizes to the "COBW-LIKE PROTEIN" nucleic acid under low stringency conditions,
   (iv) "SEB4B (FRAGMENT)" (SEQ ID No:249) or a functionally active derivative thereof, or a functionally active fragment thereof, or a homolog thereof, or a variant of "SEB4B (FRAGMENT)" encoded by a nucleic acid that hybridizes to the "SEB4B (FRAGMENT)" nucleic acid under low stringency conditions,
   (v) "HSP 90-β 1"(SEQ ID No:9) or a functionally active derivative thereof, or a functionally active fragment thereof, or a homolog thereof, or a variant of "HSP 90-β 1" encoded by a nucleic acid that hybridizes to the "HSP 90-β 1" nucleic acid under low stringency conditions,
   (vi) "FAF-X UBIQUITIN C-TERM HYDRO- LASE" (SEQ ID No:250) or a functionally active derivative thereof, or a functionally active fragment thereof, or a homolog thereof, or a variant of "FAF-X UBIQUITIN C-TERM HYDRO- LASE" encoded by a nucleic acid that hybridizes to the "FAF-X UBIQUITIN C-TERM HYDRO- LASE" nucleic acid under low stringency conditions,
   (vii) "HSP 90-α" (SEQ ID No:105) or a functionally active derivative thereof, or a functionally active fragment thereof, or a homolog thereof, or a variant of "HSP 90-α" encoded by a nucleic acid that hybridizes to the "HSP90-α" nucleic acid under low stringency conditions,
   (viii) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (ix) "14-3-3 ζ/δ" (SEQ ID No:229) or a functionally active derivative thereof, or a functionally active fragment thereof, or a homolog thereof, or a variant of "14-3-3 ζ/δ" encoded by a nucleic acid that hybridizes to the "14-3-3 ζ/δ" nucleic acid under low stringency conditions,
   (x) "14-3-3 ε" (SEQ ID No:228) or a functionally active derivative thereof, or a functionally active fragment thereof, or a homolog thereof, or a variant of "14-3-3 ε" encoded by a nucleic acid that hybridizes to the "14-3-3 ε" nucleic acid under low stringency conditions,
   (xi) "MEKK1" (SEQ ID No:251) or a functionally active derivative thereof, or a functionally active fragment thereof, or a homolog thereof, or a variant of "MEKK1" encoded by a nucleic acid that hybridizes to the "MEKK1" nucleic acid under low stringency conditions,
   (xii) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions,
   (xiii) "P30 DBC PROTEIN" (SEQ ID No:252) or a functionally active derivative thereof, or a functionally active fragment thereof, or a homolog thereof, or a variant of "P30 DBC PROTEIN" encoded by a nucleic acid that hybridizes to the "P30 DBC PROTEIN" nucleic acid under low stringency conditions,
   (xiv) "G(I)/G(S)/G(O) γ-12 SUBUNIT, GUANINE NUCLEOTIDE-BINDING PROTEIN" (SEQ ID No:112) or a functionally active derivative thereof, or a functionary active fragment thereof, or a homolog thereof, or a variant of "G(I)/G(S)/G(O) γ-12 SUBUNIT, GUANINE NUCLEOTIDE-BINDING PROTEIN" encoded by a nucleic acid that hybridizes to the "G(I)/G(S)/G(O) γ-12 SUBUNIT, GUANINE NUCLEOTIDE-BINDING PROTEIN" nucleic acid under low stringency conditions,
   (xv) "51 KDA FK506-BINDING PROTEIN" (SEQ ID No:106) or a functionally active derivative thereof, or a functionally active fragment thereof, or a homolog thereof, or a variant of "51 KDA FK506-BINDING PROTEIN" encoded by a nucleic acid that hybridizes to the "51 KDA FK506-BINDING PROTEIN" nucleic acid under low stringency conditions,
   (xvi) "WD-REPEAT AN11 HOMOLOG" (SEQ ID No:253) or a functionally active derivative thereof,or a functionally active fragement thereof, or a homolog thereof, or a variant of "WD-REPEAT AN11 HOMOLOG" that hybridizes to the "WD-REPEAT AN11 HOMOLOG" nucleic acid under low stringency conditions, and
   (xvii) "BAG-FAMILY MOLECULAR CHAPERONE REGULATOR-5" (SEQ ID No:254) or a functionally active derivative thereof, or a functionally active fragment thereof, or a homolog thereof, or a variant of "BAG-FAMILY MOLECULAR CHAPERONE REGULATOR-5" encoded by a nucleic acid that hybridizes to the "BAG-FAMILY MOLECULAR CHAPERONE REGULATOR-5" nucleic acid under low stringency conditions;
   and a protein complex selected from complex (II) comprising all but 1 - 12 of the following proteins:
   (i) "P40 NUCLEOLAR PROTEIN" (SEQ ID No:246) or a functionally active derivative thereof, or a functionally active fragment thereof, or a homolog thereof, or a variant of "P40 NUCLEOLAR PROTEIN" encoded by a nucleic acid that hybridizes to the "P40 NUCLEOLAR PROTEIN" nucleic acid under low stringency conditions,
   (ii) "MELANOMA-ASSOCIATED ANTIGEN D1" (SEQ ID No:247) or a functionally active derivative thereof, or a functionally active fragment thereof, or a homolog thereof, or a variant of "MELANOMA-ASSOCIATED ANTIGEN D1" encoded by a nucleic acid that hybridizes to the "MELANOMA-ASSOCIATED ANTIGEN D1" nucleic acid under low stringency conditions,
   (iii) "COBW-LIKE PROTEIN" (SEQ ID No:248) or a functionally active derivative thereof, or a functionally active fragment thereof, or a homolog thereof, or a variant of "COBW-LIKE PROTEIN" encoded by a nucleic acid that hybridizes to the "COBW-LIKE PROTEIN" nucleic acid under low stringency conditions,
   (iv) "SEB4B (FRAGMENT)" (SEQ ID No:249) or a functionally active derivative thereof, or a functionally active fragment thereof, or a homolog thereof, or a variant of "SEB4B (FRAGMENT)" encoded by a nucleic acid that hybridizes to the "SEB4B (FRAGMENT)" nucleic acid under low stringency conditions,
   (v) "HSP 90-β 1"(SEQ ID No:9) or a functionally active derivative thereof, or a functionally active fragment thereof, or a homolog thereof, or a variant of "HSP 90-β 1" encoded by a nucleic acid that hybridizes to the "HSP 90-β 1" nucleic acid under low stringency conditions,
   (vi) "FAF-X UBIQUITIN C-TERM HYDRO- LASE" (SEQ ID No:250) or a functionally active derivative thereof, or a functionally active fragment thereof, or a homolog thereof, or a variant of "FAF-X UBIQUITIN C-TERM HYDRO- LASE" encoded by a nucleic acid that hybridizes to the "FAF-X UBIQUITIN C-TERM HYDRO- LASE" nucleic acid under low stringency conditions,
   (vii) "HSP 90-α" (SEQ ID No:105) or a functionally active derivative thereof, or a functionally active fragment thereof, or a homolog thereof, or a variant of "HSP 90-α" encoded by a nucleic acid that hybridizes to the "HSP90-α" nucleic acid under low stringency conditions,
   (viii) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (ix) "14-3-3 ζ/δ" (SEQ ID No:229) or a functionally active derivative thereof, or a functionally active fragment thereof, or a homolog thereof, or a variant of "14-3-3 ζ/δ" encoded by a nucleic acid that hybridizes to the "14-3-3 ζ/δ" nucleic acid under low stringency conditions,
   (x) "14-3-3 ε" (SEQ ID No:228) or a functionally active derivative thereof, or a functionally active fragment thereof, or a homolog thereof, or a variant of "14-3-3ε" encoded by a nucleic acid that hybridizes to the "14-3-3ε" nucleic acid under low stringency conditions,
   (xi) "MEKK1" (SEQ ID No:251) or a functionally active derivative thereof, or a functionally active fragment thereof, or a homolog thereof, or a variant of "MEKK1" encoded by a nucleic acid that hybridizes to the "MEKK1" nucleic acid under low stringency conditions,
   (xii) "P30 DBC PROTEIN" (SEQ ID No:252) or a functionally active derivative thereof, or a functionally active fragment thereof, or a homolog thereof, or a variant of "P30 DBC PROTEIN" encoded by a nucleic acid that hybridizes to the "P30 DBC PROTEIN" nucleic acid under low stringency conditions,
   (xiii) "G(I)/G(S)/G(O) γ-12 SUBUNIT, GUANINE NUCLEOTIDE-BINDING PROTEIN " (SEQ ID No:112) or a functionally active derivative thereof, or a functionally active fragment thereof, or a homolog thereof, or a variant of "G(I)/G(S)/G(O) y-12 SUBUNIT, GUANINE NUCLEOTIDE-BINDING PROTEIN" encoded by a nucleic acid that hybridizes to the "G(I)/G(S)/G(O) γ-12 SUBUNIT, GUANINE NUCLEOTIDE-BINDING PROTEIN" nucleic acid under low stringency conditions,
   (xiv) "51 KDA FK506-BINDING PROTEIN" (SEQ ID No:106) or a functionally active derivative thereof, or a functionally active fragment thereof, or a homolog thereof, or a variant of "51 KDA FK506-BINDING PROTEIN" encoded by a nucleic acid that hybridizes to the "51 KDA FK506-BINDING PROTEIN" nucleic acid under low stringency conditions,
   (xv) "WD-REPEAT AN11 HOMOLOG" (SEQ ID No:253) or a functionally active derivative thereof,or a functionally active fragement thereof, or a homolog thereof, or a variant of "WD-REPEAT AN11 HOMOLOG" that hybridizes to the "WD-REPEAT AN11 HOMOLOG" nucleic acid under low stringency conditions, and
   (xvi) "BAG-FAMILY MOLECULAR CHAPERONE REGULATOR-5" (SEQ ID No:254) or a functionally active derivative thereof, or a functionally active fragment thereof, or a homolog thereof, or a variant of "BAG-FAMILY MOLECULAR CHAPERONE REGULATOR-5" encoded by a nucleic acid that hybridizes to the "BAG-FAMILY MOLECULAR CHAPERONE REGULATOR-5" nucleic acid under low stringency conditions.
5. The complex of any of No. 1 - 4 comprising a functionally active derivative of said first protein and/or a functionally active derivative of said second protein, wherein the functionally active derivative is a fusion protein comprising said first protein or said second protein fused to an amino acid sequence different from the first protein or second protein, respectively.
6. The complex of No. 5 wherein the functionally active derivative is a fusion protein comprising said first protein or said second protein fused to an affinity tag or label.
7. The complex of any of No. 1 - 4 comprising a fragment of said first protein and/or a fragment of said second protein, which fragment binds to another protein component of said complex.
8. The complex of any of No. 1 -7 that is involved in the MEKK1 kinase activity, Ubiquitination activity or apoptosis activity.
9. A process for preparing complex of any of No. 1-8 and optionally the components thereof comprising the following steps:
   the expressing a protein (bait) of the complex, preferably a tagged protein, in a target cell, isolating the protein complex which is attached to the bait protein, and optionally disassociating the protein complex and isolating the individual complex members.
10. The process according to No. 9 wherein the tagged protein comprises two different tags which allow two separate affinity purification steps.
11. The process according to any of No. 9-10 wherein the two tags are separated by a cleavage site for a protease.
12. Component of the MEKK1 complex obtainable by a process according to any of No. 9 to 11.
13. Construct, preferably a vector construct, comprising at least two separate nucleic acid sequences each encoding a different protein, or a functionally active fragment or a functionally active derivative therof at least one of said proteins, or functionally active fragments or functionally active derivative therof being selected from the first group of proteins according to No. 1(a) and at least one of said proteines, or functionally active fragments of functionally active derivative thereof being selected from the second group of proteins according to No. 1(b)
14. Host cell containting a construct of No. 13 or containing several vectors each comprising at least the nucleic acid sequence encoding at least one of the proteins, or functionally active fragments or functionally active derivatives thereof selected from the first group of proteins according to No. 1(a) and the proteins, or functionally active fragments or functionally active derivatives thereof selected from the second group of proteins according to No. 1(b).
15. An antibody or a fragment of said antibody containing the binding domain thereof, selected from an antibody or fragment thereof, which binds the complex of any of No. 1-8 and which does not bind any of the proteins of said complex when uncomplexed and an antibody or a fragment of said antibody which binds to any of the proteins according to No..
16. A kit comprising in one or more container the complex of any of No. 1 - 8optionally together with an antibody according to No. 15 and/or further components such as reagents and working instructions.
17. The kit according to No. 16 for processing a substrate of said complex.
18. A kit according to No. 16 for the diagnosis or prognosis of a disease or a disease risk, preferentially for a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
19. Array, in which at least a complex according to any of No. 1 - 8 and/or at least one antibody according to No. 15 is attached to a solid carrier.
20. A process for processing a physiological substrate of the complex comprising the step of bringing into contact a complex to any of No. 1-8 with said substrate, such that said substrate is processed.
21. A pharmaceutical composition comprising the protein complex of any of No. 1 to 8..
22. The pharmaceutical composition according to No. 21 for the treatment of diseases and disorders such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
23. A method for screening for a molecule that binds to the complex of anyone of No. 1 - 8. comprising the steps of
   (a) exposing said complex, or a cell or organism containing same to one or more candidate molecules; and
   (b) determinig whether said candidate molecule is bound to the complex or protein.
24. A method for screening for a molecule that modulates directly or indirectly the function, activity, composition or formation of the complex of any one of No. 1 - 8 comprising the steps of (a) exposing said complex, or a cell or organism containing MEKK1 complex to one or more candidate molecules; and
   (b) determining the amount of activity of protein components of, and/or intracellular localization of, said complex and/or the transcription level of a gene dependent on the complex and/or the abundance and/or activity of a protein or protein complex dependent on the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependent on the complex and/or the abundance and/or activity of a protein or protein complex dependent on the function of the complex and/or product of a gene dependent on the complex in the absence of said candidate molecules indicates that the molecule modulates function, activity or composition of said complex.
25. The method of No. 24, wherein the amount of said complex is determined.
26. The method of No. 24, wherein the activity of said complex is determined.
27. The method of No. 26, wherein said determining step comprises isolating from the cell or organism said complex to produce said isolated complex and contacting said isolated complex in the presence or absence of a candidate molecule with a substrate of said complex and determining the processing of said substrate is modified in the presence of said candidate molecule.
28. The method of No. 24, wherein the amount of the individual protein components of said complex are determined.
29. The method of No. 28, determining step comprises determining whether
   (i) "P40 NUCLEOLAR PROTEIN" (SEQ ID No:246) or a functionally active derivative thereof, or a functionally active fragment thereof, or a homolog thereof, or a variant of "P40 NUCLEOLAR PROTEIN" encoded by a nucleic acid that hybridizes to the "P40 NUCLEOLAR PROTEIN" nucleic acid under low stringency conditions, and/or
   (ii) "MELANOMA-ASSOCIATED ANTIGEN D1" (SEQ ID No:247) or a functionally active derivative thereof, or a functionally active fragment thereof, or a homolog thereof, or a variant of "MELANOMA-ASSOCIATED ANTIGEN D1" encoded by a nucleic acid that hybridizes to the "MELANOMA-ASSOCIATED ANTIGEN D1" nucleic acid under low stringency conditions, and/or
   (iii) "COBW-LIKE PROTEIN" (SEQ ID No:248) or a functionally active derivative thereof, or a functionally active fragment thereof, or a homolog thereof, or a variant of "COBW-LIKE PROTEIN" encoded by a nucleic acid that hybridizes to the "COBW-LIKE PROTEIN" nucleic acid under low stringency conditions, and/or
   (iv) "SEB4B (FRAGMENT)" (SEQ ID No:249) or a functionally active derivative thereof, or a functionally active fragment thereof, or a homolog thereof, or a variant of "SEB4B (FRAGMENT)" encoded by a nucleic acid that hybridizes to the "SEB4B (FRAGMENT)" nucleic acid under low stringency conditions, and/or
   (v) "HSP 90-β 1"(SEQ ID No:9) or a functionally active derivative thereof, or a functionally active fragment thereof, or a homolog thereof, or a variant of "HSP 90-β 1" encoded by a nucleic acid that hybridizes to the "HSP 90-β 1" nucleic acid under low stringency conditions, and/or
   (vi) "FAF-X UBIQUITIN C-TERM HYDRO- LASE" (SEQ ID No:250) or a functionally active derivative thereof, or a functionally active fragment thereof, or a homolog thereof, or a variant of "FAF-X UBIQUITIN C-TERM HYDRO- LASE" encoded by a nucleic acid that hybridizes to the "FAF-X UBIQUITIN C-TERM HYDRO- LASE" nucleic acid under low stringency conditions, and/or
   (vii) "HSP 90-α" (SEQ ID No:105) or a functionally active derivative thereof, or a functionally active fragment thereof, or a homolog thereof, or a variant of "HSP 90-α" encoded by a nucleic acid that hybridizes to the "HSP90-α" nucleic acid under low stringency conditions, and/or
   (viii) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions, and/or
   (ix) "14-3-3 ζ/δ" (SEQ ID No:229) or a functionally active derivative thereof, or a functionally active fragment thereof, or a homolog thereof, or a variant of "14-3-3 ζ/δ" encoded by a nucleic acid that hybridizes to the "14-3-3 ζ/δ" nucleic acid under low stringency conditions, and/or
   (x) "14-3-3 ε" (SEQ ID No:228) or a functionally active derivative thereof, or a functionally active fragment thereof, or a homolog thereof, or a variant of "14-3-3 ε" encoded by a nucleic acid that hybridizes to the "14-3-3 ε" nucleic acid under low stringency conditions, and/or
   (xi) "MEKK1" (SEQ ID No:251) or a functionally active derivative thereof, or a functionally active fragment thereof, or a homolog thereof, or a variant of "MEKK1" encoded by a nucleic acid that hybridizes to the "MEKK1" nucleic acid under low stringency conditions, and/or
   (xii) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions, and/or
   (xiii) "P30 DBC PROTEIN" (SEQ ID No:252) or a functionally active derivative thereof, or a functionally active fragment thereof, or a homolog thereof, or a variant of "P30 DBC PROTEIN" encoded by a nucleic acid that hybridizes to the "P30 DBC PROTEIN" nucleic acid under low stringency conditions, and/or
   (xiv) "G(I)/G(S)/G(O) γ-12 SUBUNIT, GUANINE NUCLEOTIDE-BINDING PROTEIN" (SEQ ID No:112) or a functionally active derivative thereof, or a functionally active fragment thereof, or a homolog thereof, or a variant of "G(I)/G(S)/G(O) γ-12 SUBUNIT, GUANINE NUCLEOTIDE-BINDING PROTEIN" encoded by a nucleic acid that hybridizes to the "G(I)/G(S)/G(O) γ-12 SUBUNIT, GUANINE NUCLEOTIDE-BINDING PROTEIN" nucleic acid under low stringency conditions, and/or
   (xv) "51 KDA FK506-BINDING PROTEIN" (SEQ ID No:106) or a functionally active derivative thereof, or a functionally active fragment thereof, or a homolog thereof, or a variant of "51 KDA FK506-BINDING PROTEIN" encoded by a nucleic acid that hybridizes to the "51 KDA FK506-BINDING PROTEIN" nucleic acid under low stringency conditions, and/or
   (xvi) "WD-REPEAT AN11 HOMOLOG" (SEQ ID No:253) or a functionally active derivative thereof, or a functionally active fragment thereof, or a homolog thereof, or a variant of "WD-REPEAT AN11 HOMOLOG" encoded by a nucleic acid that hybridizes to the "WD-REPEAT AN 11 HOMOLOG" nucleic acid under low stringency conditions, and/or
   (xvii) "BAG-FAMILY MOLECULAR CHAPERONE REGULATOR-5" (SEQ ID No:254) or a functionally active derivative thereof, or a functionally active fragment thereof, or a homolog thereof, or a variant of "BAG-FAMILY MOLECULAR CHAPERONE REGULATOR-5" encoded by a nucleic acid that hybridizes to the "BAG-FAMILY MOLECULAR CHAPERONE REGULATOR-5" nucleic acid under low stringency conditions,
   is present in the complex.
30. The method of any of No. 24 to 29, wherein said method is a method of screening for a drug for treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
31. Use of a molecule that modulates the amount of, activity of, or the protein components of the complex of any one of No. 1 - 8 for the manufacture of a medicament for the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
32. A method for the production of a pharmaceutical composition comprising carrying out the method of any of No. 1 - 8 to identify a molecule that modulates the function, activity, composition or formation of said complex, and further comprising mixing the identified molecule with a pharmaceutically acceptable carrier.
33. A method for diagnosing or screening for the presence of a disease or disorder or a predisposition for developing a disease or disorder in a subject, which disease or disorder is characterized by an aberrant amount of, activity of, or component composition of, or intracellular localization of the complex of any one of the No. 1-8, comprising determining the amount of, activity of, protein components of, and/or intracellular localization of, said complex and/or the transcription level of a gene dependent on the complex and/or the abundance and/or activity of a protein or protein complex dependent on the function of the complex and/or product of a gene dependent on the complex in a comparative sample derived from a subject, wherein a difference in said amount, activity, or protein components of, said complex in an analogous sample from a subject not having the disease or disorder or predisposition indicates the presence in the subject of the disease or disorder or predisposition in the subject.
34. The method of No. 33, wherein the amount of said complex is determined.
35. The method of No. 33, wherein the activity of said complex is determined.
36. The method of No. 35, wherein said determining step comprises isolating from the subject said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
37. The method of No. 33, wherein the amount of the individual protein components of said complex are determined.
38. The method of No. 37, wherein said determining step comprises determining whether
   (i) "P40 NUCLEOLAR PROTEIN" (SEQ ID No:246) or a functionally active derivative thereof, or a functionally active fragment thereof, or a homolog thereof, or a variant of "P40 NUCLEOLAR PROTEIN" encoded by a nucleic acid that hybridizes to the "P40 NUCLEOLAR PROTEIN" nucleic acid under low stringency conditions, and/or
   (ii) "MELANOMA-ASSOCIATED ANTIGEN D1" (SEQ ID No:247) or a functionally active derivative thereof, or a functionally active fragment thereof, or a homolog thereof, or a variant of "MELANOMA-ASSOCIATED ANTIGEN D1" encoded by a nucleic acid that hybridizes to the "MELANOMA-ASSOCIATED ANTIGEN D1" nucleic acid under low stringency conditions, and/or
   (iii) "COBW-LIKE PROTEIN" (SEQ ID No:248) or a functionally active derivative thereof, or a functionally active fragment thereof, or a homolog thereof, or a variant of "COBW-LIKE PROTEIN" encoded by a nucleic acid that hybridizes to the "COBW-LIKE PROTEIN" nucleic acid under low stringency conditions, and/or
   (iv) "SEB4B (FRAGMENT)" (SEQ ID No:249) or a functionally active derivative thereof, or a functionally active fragment thereof, or a homolog thereof, or a variant of "SEB4B (FRAGMENT)" encoded by a nucleic acid that hybridizes to the "SEB4B (FRAGMENT)" nucleic acid under low stringency conditions, and/or
   (v) "HSP 90-β 1"(SEQ ID No:9) or a functionally active derivative thereof, or a functionally active fragment thereof, or a homolog thereof, or a variant of "HSP 90-β 1" encoded by a nucleic acid that hybridizes to the "HSP 90-β 1" nucleic acid under low stringency conditions, and/or
   (vi) "FAF-X UBIQUITIN C-TERM HYDRO- LASE" (SEQ ID No:250) or a functionally active derivative thereof, or a functionally active fragment thereof, or a homolog thereof, or a variant of "FAF-X UBIQUITIN C-TERM HYDRO- LASE" encoded by a nucleic acid that hybridizes to the "FAF-X UBIQUITIN C-TERM HYDRO- LASE" nucleic acid under low stringency conditions, and/or
   (vii) "HSP 90-α" (SEQ ID No:105) or a functionally active derivative thereof, or a functionally active fragment thereof, or a homolog thereof, or a variant of "HSP 90-α" encoded by a nucleic acid that hybridizes to the "HSP90-α" nucleic acid under low stringency conditions, and/or
   (viii) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions, and/or
   (ix) "14-3-3 ζ/δ" (SEQ ID No:229) or a functionally active derivative thereof, or a functionally active fragment thereof, or a homolog thereof, or a variant of "14-3-3 ζ/δ" encoded by a nucleic acid that hybridizes to the "14-3-3 ζ/δ" nucleic acid under low stringency conditions, and/or
   (x) "14-3-3 ε" (SEQ ID No:228) or a functionally active derivative thereof, or a functionally active fragment thereof, or a homolog thereof, or a variant of "14-3-3 ε" encoded by a nucleic acid that hybridizes to the "14-3-3 ε" nucleic acid under low stringency conditions, and/or
   (xi) "MEKK1" (SEQ ID No:251 ) or a functionally active derivative thereof, or a functionally active fragment thereof, or a homolog thereof, or a variant of "MEKK1" encoded by a nucleic acid that hybridizes to the "MEKK1" nucleic acid under low stringency conditions, and/or
   (xii) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions, and/or
   (xiii) "P30 DBC PROTEIN" (SEQ ID No:252) or a functionally active derivative thereof, or a functionally active fragment thereof, or a homolog thereof, or a variant of "P30 DBC PROTEIN" encoded by a nucleic acid that hybridizes to the "P30 DBC PROTEIN" nucleic acid under low stringency conditions, and/or
   (xiv) "G(I)/G(S)/G(O) γ-12 SUBUNIT, GUANINE NUCLEOTIDE-BINDING PROTEIN" (SEQ ID No:112) or a functionally active derivative thereof, or a functionally active fragment thereof, or a homolog thereof, or a variant of "G(I)/G(S)/G(O) γ-12 SUBUNIT, GUANINE NUCLEOTIDE-BINDING PROTEIN" encoded by a nucleic acid that hybridizes to the "G(I)/G(S)/G(O) γ-12 SUBUNIT, GUANINE NUCLEOTIDE-BINDING PROTEIN" nucleic acid under low stringency conditions, and/or
   (xv) "51 KDA FK506-BINDING PROTEIN" (SEQ ID No:106) or a functionally active derivative thereof, or a functionally active fragment thereof, or a homolog thereof, or a variant of "51 KDA FK506-BINDING PROTEIN" encoded by a nucleic acid that hybridizes to the "51 KDA FK506-BINDING PROTEIN" nucleic acid under low stringency conditions, and/or
   (xvi) "WD-REPEAT AN11 HOMOLOG" (SEQ ID No:253) or a functionally active derivative thereof, or a functionally active fragment thereof, or a homolog thereof, or a variant of "WD-REPEAT AN11 HOMOLOG" encoded by a nucleic acid that hybridizes to the "WD-REPEAT AN11 HOMOLOG" nucleic acid under low stringency conditions, and/or
   (xvii) "BAG-FAMILY MOLECULAR CHAPERONE REGULATOR-5" (SEQ ID No:254) or a functionally active derivative thereof, or a functionally active fragment thereof, or a homolog thereof, or a variant of "BAG-FAMILY MOLECULAR CHAPERONE REGULATOR-5" encoded by a nucleic acid that hybridizes to the "BAG-FAMILY MOLECULAR CHAPERONE REGULATOR-5" nucleic acid under low stringency conditions,
   is present in the complex.
39. The complex of any one of No. 1 - 8 or the antibody or fragment of No. 15, for use in a method of diagnosing a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
40. A method for treating or preventing a disease or disorder characterized by an aberrant amount of, activity or component composition of or intracellular localization of, the complex of anyone of No. 1-8, comprising administering to a subject in need of such treatment or prevention a therapeutically effective amount of one or more molecules that modulate the amount of, MEKK1 kinase activity, Ubiquitination activity or apoptosis activity, or protein components of, said complex.
41. The method according to No. 40, wherein said disease or disorder involves decreased levels of the amount or activity of said complex.
42. The method according to No. 40 , wherein said disease or disorder involves increased levels of the amount or activity of said complex.
43. Complex of any of No. 1-8 and/or protein selected from the following proteins
   (i) "P40 NUCLEOLAR PROTEIN" (SEQ ID No:246) or a functionally active derivative thereof, or a functionally active fragment thereof, or a homolog thereof, or a variant of "P40 NUCLEOLAR PROTEIN" encoded by a nucleic acid that hybridizes to the "P40 NUCLEOLAR PROTEIN" nucleic acid under low stringency conditions,
   (ii) "MELANOMA-ASSOCIATED ANTIGEN D1" (SEQ ID No:247) or a functionally active derivative thereof, or a functionally active fragment thereof, or a homolog thereof, or a variant of "MELANOMA-ASSOCIATED ANTIGEN D1" encoded by a nucleic acid that hybridizes to the "MELANOMA-ASSOCIATED ANTIGEN D1" nucleic acid under low stringency conditions,
   (iii) "COBW-LIKE PROTEIN" (SEQ ID No:248) or a functionally active derivative thereof, or a functionally active fragment thereof, or a homolog thereof, or a variant of "COBW-LIKE PROTEIN" encoded by a nucleic acid that hybridizes to the "COBW-LIKE PROTEIN" nucleic acid under low stringency conditions,
   (iv) "SEB4B (FRAGMENT)" (SEQ ID No:249) or a functionally active derivative thereof, or a functionally active fragment thereof, or a homolog thereof, or a variant of "SEB4B (FRAGMENT)" encoded by a nucleic acid that hybridizes to the "SEB4B (FRAGMENT)" nucleic acid under low stringency conditions,
   (v) "HSP 90-β 1"(SEQ ID No:9) or a functionally active derivative thereof, or a functionally active fragment thereof, or a homolog thereof, or a variant of "HSP 90-β 1" encoded by a nucleic acid that hybridizes to the "HSP 90-β 1" nucleic acid under low stringency conditions,
   (vi) "FAF-X UBIQUITIN C-TERM HYDRO- LASE" (SEQ ID No:250) or a functionally active derivative thereof, or a functionally active fragment thereof, or a homolog thereof, or a variant of "FAF-X UBIQUITIN C-TERM HYDRO- LASE" encoded by a nucleic acid that hybridizes to the "FAF-X UBIQUITIN C-TERM HYDRO- LASE" nucleic acid under low stringency conditions,
   (vii) "HSP 90-α" (SEQ ID No:105) or a functionally active derivative thereof, or a functionally active fragment thereof, or a homolog thereof, or a variant of "HSP 90-α" encoded by a nucleic acid that hybridizes to the "HSP90-α" nucleic acid under low stringency conditions,
   (viii) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (ix) "14-3-3 ξ/δ" (SEQ ID No:229) or a functionally active derivative thereof, or a functionally active fragment thereof, or a homolog thereof, or a variant of "14-3-3 ξ/δ" encoded by a nucleic acid that hybridizes to the "14-3-3 ξ/δ" nucleic acid under low stringency conditions,
   (x) "14-3-3 ε" (SEQ ID No:228) or a functionally active derivative thereof, or a functionally active fragment thereof, or a homolog thereof, or a variant of "14-3-3 ε" encoded by a nucleic acid that hybridizes to the "14-3-3 ε" nucleic acid under low stringency conditions,
   (xi) "MEKK1" (SEQ ID No:251) or a functionally active derivative thereof, or a functionally active fragment thereof, or a homolog thereof, or a variant of "MEKK1" encoded by a nucleic acid that hybridizes to the "MEKK1" nucleic acid under low stringency conditions,
   (xii) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions,
   (xiii) "P30 DBC PROTEIN" (SEQ ID No:252) or a functionally active derivative thereof, or a functionally active fragment thereof, or a homolog thereof, or a variant of "P30 DBC PROTEIN" encoded by a nucleic acid that hybridizes to the "P30 DBC PROTEIN" nucleic acid under low stringency conditions,
   (xiv) "G(I)/G(S)/G(O) γ-12 SUBUNIT, GUANINE NUCLEOTIDE-BINDING PROTEIN" (SEQ ID No:112) or a functionally active derivative thereof, or a functionally active fragment thereof, or a homolog thereof, or a variant of "G(I)/G(S)/G(O) γ-12 SUBUNIT, GUANINE NUCLEOTIDE-BINDING PROTEIN" encoded by a nucleic acid that hybridizes to the "G(I)/G(S)/G(O) γ-12 SUBUNIT, GUANINE NUCLEOTIDE-BINDING PROTEIN" nucleic acid under low stringency conditions,
   (xv) "51 KDA FK506-BINDING PROTEIN" (SEQ ID No:106) or a functionally active derivative thereof, or a functionally active fragment thereof, or a homolog thereof, or a variant of "51 KDA FK506-BINDING PROTEIN" encoded by a nucleic acid that hybridizes to the "51 KDA FK506-BINDING PROTEIN" nucleic acid under low stringency conditions,
   (xvi) "WD-REPEAT AN11 HOMOLOG" (SEQ ID No:253) or a functionally active derivative thereof,or a functionally active fragement thereof, or a homolog thereof, or a variant of "WD-REPEAT AN11 HOMOLOG" that hybridizes to the "WD-REPEAT AN11 HOMOLOG" nucleic acid under low stringency conditions, and
   (xvii) "BAG-FAMILY MOLECULAR CHAPERONE REGULATOR-5" (SEQ ID No:254) or a functionally active derivative thereof, or a functionally active fragment thereof, or a homolog thereof, or a variant of "BAG-FAMILY MOLECULAR CHAPERONE REGULATOR-5" encoded by a nucleic acid that hybridizes to the "BAG-FAMILY MOLECULAR CHAPERONE REGULATOR-5" nucleic acid under low stringency conditions,
as a target for an active agent of a pharmaceutical, preferably a drug target in the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.

The present invention further relates to the RIP protein complex
1. A protein complex selected from complex (I) and comprising
   (a) at least one first protein selected from the group consisting of:
      (i) "RIP" (SEQ ID No:16) or a functionally active derivative thereof, or a functionally active fragment thereof, or a homolog thereof, or a variant of "RIP" encoded by a nucleic acid that hybridizes to the "RIP" nucleic acid under low stringency conditions,
      (ii) "HSP90 β 2" (SEQ ID No:107) or a functionally active derivative thereof, or a functionally active fragment thereof, or a homolog thereof, or a variant of "HSP90 β 2" encoded by a nucleic acid that hybridizes to the "HSP90 β 2" nucleic acid under low stringency conditions,
      (iii) "MEKK3" (SEQ ID No:227) or a functionally active derivative thereof, or a functionally active fragment thereof, or a homolog thereof, or a variant of "MEKK3" encoded by a nucleic acid that hybridizes to the "MEKK3" nucleic acid under low stringency conditions,
      (iv) "RIP3 " (SEQ ID No:255) or a functionally active derivative thereof, or a functionally active fragment thereof, or a homolog thereof, or a variant of "RIP3 encoded by a nucleic acid that hybridizes to the "RIP3 " nucleic acid under low stringency conditions, (v) "IKKγ" (SEQ ID No:23) or a functionally active derivative thereof, or a functionally active fragment thereof, or a homolog thereof, or a variant of "IKKγ encoded by a nucleic acid that hybridizes to the "IKKγ" nucleic acid under low stringency conditions,
      (vi) "Caspase-8 " (SEQ ID No:256) or a functionally active derivative thereof, or a functionally active fragment thereof, or a homolog thereof, or a variant of "Caspase-8 " encoded by a nucleic acid that hybridizes to the "Caspase-8 " nucleic acid under low stringency conditions,
      (vii) "TRADD " (SEQ ID No: 17) or a functionally active derivative thereof, or a functionally active fragment thereof, or a homolog thereof, or a variant of "TRADD " encoded by a nucleic acid that hybridizes to the "TRADD " nucleic acid under low stringency conditions,
      (viii) "MEKK1" (SEQ ID No:251) or a functionally active derivative thereof, or a functionally active fragment thereof, or a homolog thereof, or a variant of "MEKK1" encoded by a nucleic acid that hybridizes to the "MEKK1" nucleic acid under low stringency conditions,
      (ix) "MITOGEN-ACTIVATED PROTEIN KINASE KINASE KINASE 14" (SEQ ID No:108) or a functionally active derivative thereof, or a functionally active fragment thereof, or a homolog thereof, or a variant of "MITOGEN-ACTIVATED PROTEIN KINASE KINASE KINASE 14" encoded by a nucleic acid that hybridizes to the "MITOGEN-ACTIVATED PROTEIN KINASE KINASE KINASE 14" nucleic acid under low stringency conditions,
      (x) "A20" (SEQ ID No:257) or a functionally active derivative thereof, or a functionally active fragment thereof, or a homolog thereof, or a variant of "A20" encoded by a nucleic acid that hybridizes to the "A20" nucleic acid under low stringency conditions,
      (xi) "TRAF1" (SEQ ID No:14) or a functionally active derivative thereof, or a functionally active fragment thereof, or a homolog thereof, or a variant of "TRAF1" encoded by a nucleic acid that hybridizes to the "TRAF1" nucleic acid under low stringency conditions, and
      (xii) "TNFR1" (SEQ ID No:21) or a functionally active derivative thereof, or a functionally active fragment thereof, or a homolog thereof, or a variant of "TNFR1" encoded by a nucleic acid that hybridizes to the "TNFR1" nucleic acid under low stringency conditions, and
   (b) at least one second protein, which second protein is selected from the group consisting of:
      (i) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions; and a complex (II) comprising at least two of said second proteins,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1 % SDS for 1.5 hours at 60°C.
2. The protein complex according to claim 1 wherein the first protein is the protein "RIP" (SEQ ID No:16), or a functionally active derivative thereof, or a functionally active fragment thereof, or a homolog thereof, or a variant of "RIP" encoded by a nucleic acid that hybridizes to the "RIP" nucleic acid under low stringency conditions.
3. The protein complex according to No. 1 selected from complex (I) and comprising the following proteins:
   (i) "RIP" (SEQ ID No:16) or a functionally active derivative thereof,or a functionally active fragement thereof, or a homolog thereof, or a variant of "RIP" that hybridizes to the "RIP" nucleic acid under low stringency conditions, and
   (ii) "HSP90 β 2" (SEQ ID No:107) or a functionally active derivative thereof, or a functionally active fragment thereof, or a homolog thereof, or a variant of "HSP90 β 2" encoded by a nucleic acid that hybridizes to the "HSP90 β 2" nucleic acid under low stringency conditions;
   and a protein complex selected from complex (II) and comprising the following proteins:
   (i) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (ii) "RIP" (SEQ ID No:16) or a functionally active derivative thereof,or a functionally active fragement thereof, or a homolog thereof, or a variant of "RIP" that hybridizes to the "RIP" nucleic acid under low stringency conditions, and
   (iii) "HSP90 β2" (SEQ ID No:107) or a functionally active derivative thereof, or a functionally active fragment thereof, or a homolog thereof, or a variant of "HSP90 β2" encoded by a nucleic acid that hybridizes to the "HSP90 β2" nucleic acid under low stringency conditions;
   and a protein complex selected from complex (III) and comprising the following proteins:
   (i) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (ii) "RIP" (SEQ ID No:16) or a functionally active derivative thereof, or a functionally active fragment thereof, or a homolog thereof, or a variant of "RIP" encoded by a nucleic acid that hybridizes to the "RIP" nucleic acid under low stringency conditions,
   (iii) "HSP90 β 2" (SEQ ID No:107) or a functionally active derivative thereof, or a functionally active fragment thereof, or a homolog thereof, or a variant of "HSP90 β2" encoded by a nucleic acid that hybridizes to the "HSP90 β2" nucleic acid under low stringency conditions,
   (iv) "MEKK3" (SEQ ID No:227) or a functionally active derivative thereof, or a functionally active fragment thereof, or a homolog thereof, or a variant of "MEKK3" encoded by a nucleic acid that hybridizes to the "MEKK3" nucleic acid under low stringency conditions,
   (v) "RIP3" (SEQ ID No:255) or a functionally active derivative thereof, or a functionally active fragment thereof, or a homolog thereof, or a variant of "RIP3" encoded by a nucleic acid that hybridizes to the "RIP3" nucleic acid under low stringency conditions,
   (vi) "IKKγ" (SEQ ID No:23) or a functionally active derivative thereof, or a functionally active fragment thereof, or a homolog thereof, or a variant of "IKKγ' encoded by a nucleic acid that hybridizes to the "IKKγ" nucleic acid under low stringency conditions,
   (vii) "caspase-8" (SEQ ID No:256) or a functionally active derivative thereof, or a functionally active fragment thereof, or a homolog thereof, or a variant of "caspase-8" encoded by a nucleic acid that hybridizes to the "caspase-8" nucleic acid under low stringency conditions,
   (viii) "TRADD " (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions,
   (ix) "MEKK1" (SEQ ID No:251) or a functionally active derivative thereof, or a functionally active fragment thereof, or a homolog thereof, or a variant of "MEKK1" encoded by a nucleic acid that hybridizes to the "MEKK1" nucleic acid under low stringency conditions,
   (x) "MITOGEN-ACTIVATED PROTEIN KINASE KINASE KINASE 14" (SEQ ID No:108) or a functionally active derivative thereof, or a functionally active fragment thereof, or a homolog thereof, or a variant of "MITOGEN-ACTIVATED PROTEIN KINASE KINASE KINASE 14" encoded by a nucleic acid that hybridizes to the "MITOGEN-ACTIVATED PROTEIN KINASE KINASE KINASE 14" nucleic acid under low stringency conditions,
   (xi) "A20" (SEQ ID No:257) or a functionally active derivative thereof, or a functionally active fragment thereof, or a homolog thereof, or a variant of "A20" encoded by a nucleic acid that hybridizes to the "A20" nucleic acid under low stringency conditions,
   (xii) "TRAF1" (SEQ ID No:14) or a functionally active derivative thereof,or a functionally active fragement thereof, or a homolog thereof, or a variant of "TRAF1" that hybridizes to the "TRAF1" nucleic acid under low stringency conditions, and
   (xiii) (iv) "TNFR1" (SEQ ID No:21) or a functionally active derivative thereof, or a functionally active fragment thereof, or a homolog thereof, or a variant of "TNRF1" encoded by a nucleic acid that hybridizes to the "TNRF1" nucleic acid under low stringency conditions.
4. The complex of any of No. 1-3 comprising a functionally active derivative of said first protein and/or a functionally active derivative of said second protein, wherein the functionally active derivative is a fusion protein comprising said first protein or said second protein fused to an amino acid sequence different from the first protein or second protein, respectively.
5. The complex of No. 4 wherein the functionally active derivative is a fusion protein comprising said first protein or said second protein fused to an affinity tag or label.
6. The complex of any of No. 1 - 3 comprising a fragment of said first protein and/or a fragment of said second protein, which fragment binds to another protein component of said complex.
7. The complex of any of No. 1 - 6 that is involved in the in vitro kinase activity or apoptosis activity.
8. A process for preparing complex of any of No. 1 - 7 and optionally the components thereof comprising the following steps:
   the expressing a protein (bait) of the complex, preferably a tagged protein, in a target cell, isolating the protein complex which is attached to the bait protein, and optionally disassociating the protein complex and isolating the individual complex members.
9. The process according to No. 8 wherein the tagged protein comprises two different tags which allow two separate affinity purification steps.
10. The process according to any of No. 8-9 wherein the two tags are separated by a cleavage site for a protease.
11. Component of the RIP complex obtainable by a process according to any of No. 8 to 10.
12. Construct, preferably a vector construct, comprising at least two separate nucleic acid sequences each encoding a different protein, or a functionally active fragment or a functionally active derivative therof at least one of said proteins, or functionally active fragments or functionally active derivative therof being selected from the first group of proteins according to No. 1(a) and at least one of said proteines, or functionally active fragments of functionally active derivative thereof being selected from the second group of proteins according to No. 1(b)
13. Host cell containting a construct of No. 12 or containing several vectors each comprising at least the nucleic acid sequence encoding at least one of the proteins, or functionally active fragments or functionally active derivatives thereof selected from the first group of proteins according to No. 1(a) and the proteins, or functionally active fragments or functionally active derivatives thereof selected from the second group of proteins according to No. 1(b).
14. An antibody or a fragment of said antibody containing the binding domain thereof, selected from an antibody or fragment thereof, which binds the complex of any of No. 1-7 and which does not bind any of the proteins of said complex when uncomplexed and an antibody or a fragment of said antibody which binds to any of the proteins according to No..
15. A kit comprising in one or more container the complex of any of No. 1-7 optionally together with an antibody according to No. 14 and/or further components such as reagents and working instructions.
16. A kit according to No.15 for processing a substrate of said complex.
17. The kit according to No. 15 for the diagnosis or prognosis of a disease or a disease risk, preferentially for a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
18. Array, in which at least a complex according to any of No. 1 - 7 and/or at least one antibody according to No. 14 is attached to a solid carrier.
19. A process for processing a physiological substrate of the complex comprising the step of bringing into contact a complex to any of No. 1 - 7 with said substrate, such that said substrate is processed.
20. A pharmaceutical composition comprising the protein complex of any of No. 1 to 7..
21. A pharmaceutical composition according to No. 20 for the treatment of diseases and disorders such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
22. A method for screening for a molecule that binds to the complex of anyone of No. 1-7 comprising the steps of
   (a) exposing said complex, or a cell or organism containing same to one or more candidate molecules; and
   (b) determinig whether said candidate molecule is bound to the complex or protein.
23. A method for screening for a molecule that modulates directly or indirectly the function, activity, composition or formation of the complex of any one of No. 1-7 comprising the steps of (a) exposing said complex, or a cell or organism containing RIP complex to one or more candidate molecules; and
   (b) determining the amount of activity of protein components of, and/or intracellular localization of, said complex and/or the transcription level of a gene dependent on the complex and/or the abundance and/or activity of a protein or protein complex dependent on the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependent on the complex and/or the abundance and/or activity of a protein or protein complex dependent on the function of the complex and/or product of a gene dependent on the complex in the absence of said candidate molecules indicates that the molecule modulates function, activity or composition of said complex.
24. The method of No. 23, wherein the amount of said complex is determined.
25. The method of No. 23, wherein the activity of said complex is determined.
26. The method of No. 25, wherein said determining step comprises isolating from the cell or organism said complex to produce said isolated complex and contacting said isolated complex in the presence or absence of a candidate molecule with a substrate of said complex and determining the processing of said substrate is modified in the presence of said candidate molecule.
27. The method of No. 23, wherein the amount of the individual protein components of said complex are determined.
28. The method of No. 27, determining step comprises determining whether
   (i) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions, and/or
   (ii) "RIP" (SEQ ID No:16) or a functionally active derivative thereof, or a functionally active fragment thereof, or a homolog thereof, or a variant of "RIP" encoded by a nucleic acid that hybridizes to the "RIP" nucleic acid under low stringency conditions, and/or
   (iii) "HSP90 β 2" (SEQ ID No:107) or a functionally active derivative thereof, or a functionally active fragment thereof, or a homolog thereof, or a variant of "HSP90 β2" encoded by a nucleic acid that hybridizes to the "HSP90 β 2" nucleic acid under low stringency conditions, and/or
   (iv) "MEKK3" (SEQ ID No:227) or a functionally active derivative thereof, or a functionally active fragment thereof, or a homolog thereof, or a variant of "MEKK3" encoded by a nucleic acid that hybridizes to the "MEKK3" nucleic acid under low stringency conditions, and/or
   (v) "RIP3 " (SEQ ID No:255) or a functionally active derivative thereof, or a functionally active fragment thereof, or a homolog thereof, or a variant of "RIP3" encoded by a nucleic acid that hybridizes to the "RIP3" nucleic acid under low stringency conditions, and/or
   (vi) "IKKγ" (SEQ ID No:23) or a functionally active derivative thereof, or a functionally active fragment thereof, or a homolog thereof, or a variant of "IKKγ" encoded by a nucleic acid that hybridizes to the "IKKγ" nucleic acid under low stringency conditions, and/or
   (vii) "caspase-8" (SEQ ID No:256) or a functionally active derivative thereof, or a functionally active fragment thereof, or a homolog thereof, or a variant of "caspase-8" encoded by a nucleic acid that hybridizes to the "caspase-8" nucleic acid under low stringency conditions, and/or
   (viii) "TRADD "(SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions, and/or
   (ix) "MEKK1" (SEQ ID No:251) or a functionally active derivative thereof, or a functionally active fragment thereof, or a homolog thereof, or a variant of "MEKK1" encoded by a nucleic acid that hybridizes to the "MEKK1" nucleic acid under low stringency conditions, and/or
   (x) "MITOGEN-ACTIVATED PROTEIN KINASE KINASE KINASE 14" (SEQ ID No:108) or a functionally active derivative thereof, or a functionally active fragment thereof, or a homolog thereof, or a variant of "MITOGEN-ACTIVATED PROTEIN KINASE KINASE KINASE 14" encoded by a nucleic acid that hybridizes to the "MITOGEN-ACTIVATED PROTEIN KINASE KINASE KINASE 14" nucleic acid under low stringency conditions, and/or
   (xi) "A20" (SEQ ID No:257) or a functionally active derivative thereof, or a functionally active fragment thereof, or a homolog thereof, or a variant of "A20" encoded by a nucleic acid that hybridizes to the "A20" nucleic acid under low stringency conditions, and/or
   (xii) "TRAF1" (SEQ ID No:14) or a functionally active derivative thereof, or a functionally active fragment thereof, or a homolog thereof, or a variant of "TRAF1" encoded by a nucleic acid that hybridizes to the "TRAF1" nucleic acid under low stringency conditions, and/or
   (xiii) "TNFR1" (SEQ ID No:21) or a functionally active derivative thereof, or a functionally active fragment thereof, or a homolog thereof, or a variant of "TNFR1" encoded by a nucleic acid that hybridizes to the "TNFR1" nucleic acid under low stringency conditions,
   is present in the complex.
29. The method of any of No. 23 to 28, wherein said method is a method of screening for a drug for treatment or prevention of a disease or disorder such as inflammation such as chronic inflammatory diseases, rheumatoid arthritis, inflammatory bowel disease; infectious diseases such as septic shock and bacterial infection; neurological diseases such as stroke-induced inflammation in neurons;neurodegenerative diseases;cancer such as prostate cancer, breast cancer and skin cancer.
30. Use of a molecule that modulates the amount of, activity of, or the protein components of the complex of any one of No. 1 - 7 for the manufacture of a medicament for the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
31. A method for the production of a pharmaceutical composition comprising carrying out the method of any of No. 1 - 7 to identify a molecule that modulates the function, activity, composition or formation of said complex, and further comprising mixing the identified molecule with a pharmaceutically acceptable carrier.
32. A method for diagnosing or screening for the presence of a disease or disorder or a predisposition for developing a disease or disorder in a subject, which disease or disorder is characterized by an aberrant amount of, activity of, or component composition of, or intracellular localization of the complex of any one of the No. 1 - 7, comprising determining the amount of, activity of, protein components of, and/or intracellular localization of, said complex and/or the transcription level of a gene dependent on the complex and/or the abundance and/or activity of a protein or protein complex dependent on the function of the complex and/or product of a gene dependent on the complex in a comparative sample derived from a subject, wherein a difference in said amount, activity, or protein components of, said complex in an analogous sample from a subject not having the disease or disorder or predisposition indicates the presence in the subject of the disease or disorder or predisposition in the subject.
33. The method of No. 32, wherein the amount of said complex is determined.
34. The method of No. 32, wherein the activity of said complex is determined.
35. The method of No. 34, wherein said determining step comprises isolating from the subject said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
36. The method of No. 32, wherein the amount of the individual protein components of said complex are determined.
37. The method of No. 36, wherein said determining step comprises determining whether
   (i) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions, and/or
   (ii) "RIP" (SEQ ID No:16) or a functionally active derivative thereof, or a functionally active fragment thereof, or a homolog thereof, or a variant of "RIP" encoded by a nucleic acid that hybridizes to the "RIP" nucleic acid under low stringency conditions, and/or
   (iii) "HSP90 β2" (SEQ ID No:107) or a functionally active derivative thereof, or a functionally active fragment thereof, or a homolog thereof, or a variant of "HSP90 β 2" encoded by a nucleic acid that hybridizes to the "HSP90 β2" nucleic acid under low stringency conditions, and/or
   (iv) "MEKK3" (SEQ ID No:227) or a functionally active derivative thereof, or a functionally active fragment thereof, or a homolog thereof, or a variant of "MEKK3" encoded by a nucleic acid that hybridizes to the "MEKK3" nucleic acid under low stringency conditions, and/or
   (v) "RIP3" (SEQ ID No:255) or a functionally active derivative thereof, or a functionally active fragment thereof, or a homolog thereof, or a variant of "RIP3" encoded by a nucleic acid that hybridizes to the "RIP3" nucleic acid under low stringency conditions, and/or
   (vi) "IKKγ" (SEQ ID No:23) or a functionally active derivative thereof, or a functionally active fragment thereof, or a homolog thereof, or a variant of "IKKγ" encoded by a nucleic acid that hybridizes to the "IKKγ" nucleic acid under low stringency conditions, and/or
   (vii) "caspase-8" (SEQ ID No:256) or a functionally active derivative thereof, or a functionally active fragment thereof, or a homolog thereof, or a variant of "caspase-8" encoded by a nucleic acid that hybridizes to the "caspase-8" nucleic acid under low stringency conditions, and/or
   (viii) "TRADD " (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions, and/or
   (ix) "MEKK1" (SEQ ID No:251) or a functionally active derivative thereof, or a functionally active fragment thereof, or a homolog thereof, or a variant of "MEKK1" encoded by a nucleic acid that hybridizes to the "MEKK1" nucleic acid under low stringency conditions, and/or
   (x) "MITOGEN-ACTIVATED PROTEIN KINASE KINASE KINASE 14" (SEQ ID No:108) or a functionally active derivative thereof, or a functionally active fragment thereof, or a homolog thereof, or a variant of "MITOGEN-ACTIVATED PROTEIN KINASE KINASE KINASE 14" encoded by a nucleic acid that hybridizes to the "MITOGEN-ACTIVATED PROTEIN KINASE KINASE KINASE 14" nucleic acid under low stringency conditions, and/or
   (xi) "A20" (SEQ ID No:257) or a functionally active derivative thereof, or a functionally active fragment thereof, or a homolog thereof, or a variant of "A20" encoded by a nucleic acid that hybridizes to the "A20" nucleic acid under low stringency conditions, and/or
   (xii) "TRAF1" (SEQ ID No:14) or a functionally active derivative thereof, or a functionally active fragment thereof, or a homolog thereof, or a variant of "TRAF1" encoded by a nucleic acid that hybridizes to the "TRAF1" nucleic acid under low stringency conditions, and/or
   (xiii) "TNFR1" (SEQ ID No:21) or a functionally active derivative thereof, or a functionally active fragment thereof, or a homolog thereof, or a variant of "TNFR1" encoded by a nucleic acid that hybridizes to the "TNFR1" nucleic acid under low stringency conditions,
   is present in the complex.
38. The complex of any one of No. 1 - 7 or the antibody or fragment of No. 14, for use in a method of diagnosing a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
39. A method for treating or preventing a disease or disorder characterized by an aberrant amount of, activity or component composition of or intracellular localization of, the complex of anyone of No. 1 - 7, comprising administering to a subject in need of such treatment or prevention a therapeutically effective amount of one or more molecules that modulate the amount of, in vitro kinase activity or apoptosis activity, or protein components of, said complex.
40. The method according to No. 39, wherein said disease or disorder involves decreased levels of the amount or activity of said complex.
41. The method according to No. 39 , wherein said disease or disorder involves increased levels of the amount or activity of said complex.
42. Complex of any of No. 1-7 and/or protein selected from the following proteins
   (i) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (ii) "RIP" (SEQ ID No:16) or a functionally active derivative thereof, or a functionally active fragment thereof, or a homolog thereof, or a variant of "RIP" encoded by a nucleic acid that hybridizes to the "RIP" nucleic acid under low stringency conditions,
   (iii) "HSP90 β 2" (SEQ ID No:107) or a functionally active derivative thereof, or a functionally active fragment thereof, or a homolog thereof, or a variant of "HSP90 β 2" encoded by a nucleic acid that hybridizes to the "HSP90 β 2" nucleic acid under low stringency conditions,
   (iv) "MEKK3" (SEQ ID No:227) or a functionally active derivative thereof, or a functionally active fragment thereof, or a homolog thereof, or a variant of "MEKK3" encoded by a nucleic acid that hybridizes to the "MEKK3" nucleic acid under low stringency conditions,
   (v) "RIP3 " (SEQ ID No:255) or a functionally active derivative thereof, or a functionally active fragment thereof, or a homolog thereof, or a variant of "RIP3" encoded by a nucleic acid that hybridizes to the "RIP3" nucleic acid under low stringency conditions,
   (vi) "IKKγ" (SEQ ID No:23) or a functionally active derivative thereof, or a functionally active fragment thereof, or a homolog thereof, or a variant of "IKKγ" encoded by a nucleic acid that hybridizes to the "IKKγ" nucleic acid under low stringency conditions,
   (vii) "caspase-8" (SEQ ID No:256) or a functionally active derivative thereof, or a functionally active fragment thereof, or a homolog thereof, or a variant of "caspase-8" encoded by a nucleic acid that hybridizes to the "caspase-8" nucleic acid under low stringency conditions,
   (viii) "TRADD " (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions,
   (ix) "MEKK1" (SEQ ID No:251) or a functionally active derivative thereof, or a functionally active fragment thereof, or a homolog thereof, or a variant of "MEKK1" encoded by a nucleic acid that hybridizes to the "MEKK1" nucleic acid under low stringency conditions,
   (x) "MITOGEN-ACTIVATED PROTEIN KINASE KINASE KINASE 14" (SEQ ID No:108) or a functionally active derivative thereof, or a functionally active fragment thereof, or a homolog thereof, or a variant of "MITOGEN-ACTIVATED PROTEIN KINASE KINASE KINASE 14" encoded by a nucleic acid that hybridizes to the "MITOGEN-ACTIVATED PROTEIN KINASE KINASE KINASE 14" nucleic acid under low stringency conditions,
   (xi) "A20" (SEQ ID No:257) or a functionally active derivative thereof, or a functionally active fragment thereof, or a homolog thereof, or a variant of "A20" encoded by a nucleic acid that hybridizes to the "A20" nucleic acid under low stringency conditions,
   (xii) "TRAF1" (SEQ ID No:14) or a functionally active derivative thereof,or a functionally active fragement thereof, or a homolog thereof, or a variant of "TRAF1" that hybridizes to the "TRAF1" nucleic acid under low stringency conditions, and
   (xiii) "TNFR1" (SEQ ID No:21) or a functionally active derivative thereof, or a functionally active fragment thereof, or a homolog thereof, or a variant of "TNFR1" encoded by a nucleic acid that hybridizes to the "TNFR1" nucleic acid under low stringency conditions,
as a target for an active agent of a pharmaceutical, preferably a drug target in the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.

The present invention further relates to the RIP2 protein complex
1. A protein complex selected from complex (I) and comprising
   (a) at least one first protein selected from the group consisting of:
      (i) "TRAF6" (SEQ ID No:189) or a functionally active derivative thereof, or a functionally active fragment thereof, or a homolog thereof, or a variant of "TRAF6" encoded by a nucleic acid that hybridizes to the "TRAF6" nucleic acid under low stringency conditions,
      (ii) "TRAF5" (SEQ ID No:258) or a functionally active derivative thereof, or a functionally active fragment thereof, or a homolog thereof, or a variant of "TRAF5" encoded by a nucleic acid that hybridizes to the "TRAF5" nucleic acid under low stringency conditions,
      (iii) "TRAF1" (SEQ ID No:14) or a functionally active derivative thereof, or a functionally active fragment thereof, or a homolog thereof, or a variant of "TRAF1" encoded by a nucleic acid that hybridizes to the "TRAF1" nucleic acid under low stringency conditions,
      (iv) "cIAP1" (SEQ ID No:35) or a functionally active derivative thereof, or a functionally active fragment thereof, or a homolog thereof, or a variant of "cIAP1" encoded by a nucleic acid that hybridizes to the "cIAP1" nucleic acid under low stringency conditions, (v) "RIP2" (SEQ ID No:187) or a functionally active derivative thereof, or a functionally active fragment thereof, or a homolog thereof, or a variant of "RIP2" encoded by a nucleic acid that hybridizes to the "RIP2" nucleic acid under low stringency conditions,
      (vi) "TRADD" (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions, and
      (vii) "TNFR1" (SEQ ID No:21) or a functionally active derivative thereof, or a functionally active fragment thereof, or a homolog thereof, or a variant of "TNFR1" encoded by a nucleic acid that hybridizes to the "TNFR1" nucleic acid under low stringency conditions, and
   (b) at least one second protein, which second protein is selected from the group consisting of:
      (i) "SERINE HYDROXYMETHYLTRANSFERASE, CYTOSOLIC" (SEQ ID No:259) or a functionally active derivative thereof, or a functionally active fragment thereof, or a homolog thereof, or a variant of "SERINE HYDROXYMETHYLTRANSFERASE, CYTOSOLIC" encoded by a nucleic acid that hybridizes to the "SERINE HYDROXYMETHYLTRANSFERASE, CYTOSOLIC" nucleic acid under low stringency conditions, and
      (ii) "HSP90α" (SEQ ID No:105) or a functionally active derivative thereof, or a functionally active fragment thereof, or a homolog thereof, or a variant of "HSP90α" encoded by a nucleic acid that hybridizes to the "HSP90α" nucleic acid under low stringency conditions;
   and a complex (II) comprising at least two of said second proteins,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1 % SDS for 1.5 hours at 60°C.
2. The protein complex according to claim 1 wherein the first protein is the protein "RIP2" (SEQ ID No:187), or a functionally active derivative thereof, or a functionally active fragment thereof, or a homolog thereof, or a variant of "RIP2" encoded by a nucleic acid that hybridizes to the "RIP2" nucleic acid under low stringency conditions.
3. The protein complex according to No. 1 selected from complex (I) and comprising the following proteins:
   (i) "HSP90α" (SEQ ID No:105) or a functionally active derivative thereof,or a functionally active fragement thereof, or a homolog thereof, or a variant of "HSP90 α" that hybridizes to the "HSP90 α" nucleic acid under low stringency conditions, and
   (ii) "RIP2" (SEQ ID No:187) or a functionally active derivative thereof, or a functionally active fragment thereof, or a homolog thereof, or a variant of "RIP2" encoded by a nucleic acid that hybridizes to the "RIP2" nucleic acid under low stringency conditions; and a protein complex selected from complex (II) and comprising the following proteins:

   (i) "SERINE HYDROXYMETHYLTRANSFERASE, CYTOSOLIC" (SEQ ID No:259) or a functionally active derivative thereof, or a functionally active fragment thereof, or a homolog thereof, or a variant of "SERINE HYDROXYMETHYLTRANSFERASE, CYTOSOLIC" encoded by a nucleic acid that hybridizes to the "SERINE HYDROXYMETHYLTRANSFERASE, CYTOSOLIC" nucleic acid under low stringency conditions,
   (ii) "HSP90α" (SEQ ID No:105) or a functionally active derivative thereof,or a functionally active fragement thereof, or a homolog thereof, or a variant of "HSP90 α" that hybridizes to the "HSP90 α" nucleic acid under low stringency conditions, and
   (iii) "RIP2" (SEQ ID No:187) or a functionally active derivative thereof, or a functionally active fragment thereof, or a homolog thereof, or a variant of "RIP2" encoded by a nucleic acid that hybridizes to the "RIP2" nucleic acid under low stringency conditions;
   and a protein complex selected from complex (III) and comprising the following proteins:
   (i) "TRAF6" (SEQ ID No:189) or a functionally active derivative thereof, or a functionally active fragment thereof, or a homolog thereof, or a variant of "TRAF6" encoded by a nucleic acid that hybridizes to the "TRAF6" nucleic acid under low stringency conditions,
   (ii) "TRAF5" (SEQ ID No:258) or a functionally active derivative thereof, or a functionally active fragment thereof, or a homolog thereof, or a variant of "TRAF5" encoded by a nucleic acid that hybridizes to the "TRAF5" nucleic acid under low stringency conditions,
   (iii) "TRAF1" (SEQ ID No: 14) or a functionally active derivative thereof, or a functionally active fragment thereof, or a homolog thereof, or a variant of "TRAF1" encoded by a nucleic acid that hybridizes to the "TRAF1" nucleic acid under low stringency conditions,
   (iv) "cIAP1" (SEQ ID No:35) or a functionally active derivative thereof, or a functionally active fragment thereof, or a homolog thereof, or a variant of "cIAP1" encoded by a nucleic acid that hybridizes to the "cIAP1" nucleic acid under low stringency conditions,
   (v) "SERINE HYDROXYMETHYLTRANSFERASE, CYTOSOLIC" (SEQ ID No:259) or a functionally active derivative thereof, or a functionally active fragment thereof, or a homolog thereof, or a variant of "SERINE HYDROXYMETHYLTRANSFERASE, CYTOSOLIC" encoded by a nucleic acid that hybridizes to the "SERINE HYDROXYMETHYLTRANSFERASE, CYTOSOLIC" nucleic acid under low stringency conditions,
   (vi) "HSP90α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions,
   (vii) "RIP2" (SEQ ID No:187) or a functionally active derivative thereof, or a functionally active fragment thereof, or a homolog thereof, or a variant of "RIP2" encoded by a nucleic acid that hybridizes to the "RIP2" nucleic acid under low stringency conditions,
   (viii) "TRADD" (SEQ ID No:17) or a functionally active derivative thereof,or a functionally active fragement thereof, or a homolog thereof, or a variant of "TRADD" that hybridizes to the "TRADD" nucleic acid under low stringency conditions, and
   (ix) (iv) "TNFR1" (SEQ ID No:21) or a functionally active derivative thereof, or a functionally active fragment thereof, or a homolog thereof, or a variant of "TNRF1" encoded by a nucleic acid that hybridizes to the "TNRF1" nucleic acid under low stringency conditions.
4. The protein complex according to No. 1 selected from complex (I) comprising all but 1 of the following proteins:
   (i) "TRAF6" (SEQ ID No:189) or a functionally active derivative thereof, or a functionally active fragment thereof, or a homolog thereof, or a variant of "TRAF6" encoded by a nucleic acid that hybridizes to the "TRAF6" nucleic acid under low stringency conditions,
   (ii) "TRAF5" (SEQ ID No:258) or a functionally active derivative thereof, or a functionally active fragment thereof, or a homolog thereof, or a variant of "TRAF5" encoded by a nucleic acid that hybridizes to the "TRAF5" nucleic acid under low stringency conditions,
   (iii) "TRAF1" (SEQ ID No:14) or a functionally active derivative thereof, or a functionally active fragment thereof, or a homolog thereof, or a variant of "TRAF1" encoded by a nucleic acid that hybridizes to the "TRAF1" nucleic acid under low stringency conditions,
   (iv) "cIAP1" (SEQ ID No:35) or a functionally active derivative thereof, or a functionally active fragment thereof, or a homolog thereof, or a variant of "cIAP1" encoded by a nucleic acid that hybridizes to the "cIAP1" nucleic acid under low stringency conditions,
   (v) "SERINE HYDROXYMETHYLTRANSFERASE, CYTOSOLIC" (SEQ ID No:259) or a functionally active derivative thereof, or a functionally active fragment thereof, or a homolog thereof, or a variant of "SERINE HYDROXYMETHYLTRANSFERASE, CYTOSOLIC" encoded by a nucleic acid that hybridizes to the "SERINE HYDROXYMETHYLTRANSFERASE, CYTOSOLIC" nucleic acid under low stringency conditions,
   (vi) "HSP90α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions,
   (vii) "RIP2" (SEQ ID No:187) or a functionally active derivative thereof, or a functionally active fragment thereof, or a homolog thereof, or a variant of "RIP2" encoded by a nucleic acid that hybridizes to the "RIP2" nucleic acid under low stringency conditions,
   (viii) "TRADD" (SEQ ID No:17) or a functionally active derivative thereof,or a functionally active fragement thereof, or a homolog thereof, or a variant of "TRADD" that hybridizes to the "TRADD" nucleic acid under low stringency conditions, and
   (ix) (iv) "TNFR1" (SEQ ID No:21) or a functionally active derivative thereof, or a functionally active fragment thereof, or a homolog thereof, or a variant of "TNRF1" encoded by a nucleic acid that hybridizes to the "TNRF1" nucleic acid under low stringency conditions;
   and a protein complex selected from complex (II) comprosing all but 1 of the following proteins:
   (i) "SERINE HYDROXYMETHYLTRANSFERASE, CYTOSOLIC" (SEQ ID No:259) or a functionally active derivative thereof, or a functionally active fragment thereof, or a homolog thereof, or a variant of "SERINE HYDROXYMETHYLTRANSFERASE, CYTOSOLIC" encoded by a nucleic acid that hybridizes to the "SERINE HYDROXYMETHYLTRANSFERASE, CYTOSOLIC" nucleic acid under low stringency conditions,
   (ii) "HSP90α" (SEQ ID No: 105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions,
   (iii) "RIP2" (SEQ ID No:187) or a functionally active derivative thereof, or a functionally active fragment thereof, or a homolog thereof, or a variant of "RIP2" encoded by a nucleic acid that hybridizes to the "RIP2" nucleic acid under low stringency conditions.
5. The complex of any of No. 1-4 comprising a functionally active derivative of said first protein and/or a functionally active derivative of said second protein, wherein the functionally active derivative is a fusion protein comprising said first protein or said second protein fused to an amino acid sequence different from the first protein or second protein, respectively.
6. The complex of No. 5 wherein the functionally active derivative is a fusion protein comprising said first protein or said second protein fused to an affinity tag or label.
7. The complex of any of No. 1 - 4 comprising a fragment of said first protein and/or a fragment of said second protein, which fragment binds to another protein component of said complex.
8. The complex of any of No. 1 -7 that is involved in the apoptosis activity or in vitro kinase activity.
9. A process for preparing complex of any of No. 1-8 and optionally the components thereof comprising the following steps:
   the expressing a protein (bait) of the complex, preferably a tagged protein, in a target cell, isolating the protein complex which is attached to the bait protein, and optionally disassociating the protein complex and isolating the individual complex members.
10. The process according to No. 9 wherein the tagged protein comprises two different tags which allow two separate affinity purification steps.
11. The process according to any of No. 9 - 10 wherein the two tags are separated by a cleavage site for a protease.
12. Component of the RIP2 complex obtainable by a process according to any of No. 9 to 11.
13. Construct, preferably a vector construct, comprising at least two separate nucleic acid sequences each encoding a different protein, or a functionally active fragment or a functionally active derivative therof at least one of said proteins, or functionally active fragments or functionally active derivative therof being selected from the first group of proteins according to No. 1(a) and at least one of said proteines, or functionally active fragments of functionally active derivative thereof being selected from the second group of proteins according to No. 1(b)
14. Host cell containting a construct of No. 13 or containing several vectors each comprising at least the nucleic acid sequence encoding at least one of the proteins, or functionally active fragments or functionally active derivatives thereof selected from the first group of proteins according to No. 1(a) and the proteins, or functionally active fragments or functionally active derivatives thereof selected from the second group of proteins according to No. 1(b).
15. An antibody or a fragment of said antibody containing the binding domain thereof, selected from an antibody or fragment thereof, which binds the complex of any of No. 1-8 and which does not bind any of the proteins of said complex when uncomplexed and an antibody or a fragment of said antibody which binds to any of the proteins according to No..
16. A kit comprising in one or more container the complex of any of No. 1-8 optionally together with an antibody according to No. 15 and/or further components such as reagents and working instructions.
17. The kit according to No.16 for processing a substrate of said complex.
18. A kit according to No. 16 for the diagnosis or prognosis of a disease or a disease risk, preferentially for a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
19. Array, in which at least a complex according to any of No. 1-8 and/or at least one antibody according to No. 15 is attached to a solid carrier.
20. A process for processing a physiological substrate of the complex comprising the step of bringing into contact a complex to any of No. 1-8 with said substrate, such that said substrate is processed.
21. A pharmaceutical composition comprising the protein complex of any of No. 1 to 8..
22. The pharmaceutical composition according to No. 21 for the treatment of diseases and disorders such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
23. A method for screening for a molecule that binds to the complex of anyone of No. 1 - 8. comprising the steps of
   (a) exposing said complex, or a cell or organism containing same to one or more candidate molecules; and
   (b) determinig whether said candidate molecule is bound to the complex or protein.
24. A method for screening for a molecule that modulates directly or indirectly the function, activity, composition or formation of the complex of any one of No. 1-8 comprising the steps of (a) exposing said complex, or a cell or organism containing RIP2 complex to one or more candidate molecules; and
   (b) determining the amount of activity of protein components of, and/or intracellular localization of, said complex and/or the transcription level of a gene dependent on the complex and/or the abundance and/or activity of a protein or protein complex dependent on the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependent on the complex and/or the abundance and/or activity of a protein or protein complex dependent on the function of the complex and/or product of a gene dependent on the complex in the absence of said candidate molecules indicates that the molecule modulates function, activity or composition of said complex.
25. The method of No. 24, wherein the amount of said complex is determined.
26. The method of No. 24, wherein the activity of said complex is determined.
27. The method of No. 26, wherein said determining step comprises isolating from the cell or organism said complex to produce said isolated complex and contacting said isolated complex in the presence or absence of a candidate molecule with a substrate of said complex and determining the processing of said substrate is modified in the presence of said candidate molecule.
28. The method of No. 24, wherein the amount of the individual protein components of said complex are determined.
29. The method of No. 28, determining step comprises determining whether
   (i) "TRAF6" (SEQ ID No:189) or a functionally active derivative thereof, or a functionally active fragment thereof, or a homolog thereof, or a variant of "TRAF6" encoded by a nucleic acid that hybridizes to the "TRAF6" nucleic acid under low stringency conditions, and/or
   (ii) "TRAF5" (SEQ ID No:258) or a functionally active derivative thereof, or a functionally active fragment thereof, or a homolog thereof, or a variant of "TRAF5" encoded by a nucleic acid that hybridizes to the "TRAF5" nucleic acid under low stringency conditions, and/or
   (iii) "TRAF1" (SEQ ID No:14) or a functionally active derivative thereof, or a functionally active fragment thereof, or a homolog thereof, or a variant of "TRAF1" encoded by a nucleic acid that hybridizes to the "TRAF1" nucleic acid under low stringency conditions, and/or
   (iv) "clAP1" (SEQ ID No:35) or a functionally active derivative thereof, or a functionally active fragment thereof, or a homolog thereof, or a variant of "clAP1" encoded by a nucleic acid that hybridizes to the "cIAP1" nucleic acid under low stringency conditions, and/or
   (v) "SERINE HYDROXYMETHYLTRANSFERASE, CYTOSOLIC" (SEQ ID No:259) or a functionally active derivative thereof, or a functionally active fragment thereof, or a homolog thereof, or a variant of "SERINE HYDROXYMETHYLTRANSFERASE, CYTOSOLIC" encoded by a nucleic acid that hybridizes to the "SERINE HYDROXYMETHYLTRANSFERASE, CYTOSOLIC" nucleic acid under low stringency conditions, and/or
   (vi) "HSP90α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions, and/or
   (vii) "RIP2" (SEQ ID No:187) or a functionally active derivative thereof, or a functionally active fragment thereof, or a homolog thereof, or a variant of "RIP2" encoded by a nucleic acid that hybridizes to the "RIP2" nucleic acid under low stringency conditions, and/or
   (viii) "TRADD" (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions, and/or
   (ix) "TNFR1" (SEQ ID No:21) or a functionally active derivative thereof, or a functionally active fragment thereof, or a homolog thereof, or a variant of "TNFR1" encoded by a nucleic acid that hybridizes to the "TNFR1" nucleic acid under low stringency conditions, is present in the complex.
30. The method of any of No. 24 to 29, wherein said method is a method of screening for a drug for treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
31. Use of a molecule that modulates the amount of, activity of, or the protein components of the complex of any one of No. 1 - 8 for the manufacture of a medicament for the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
32. A method for the production of a pharmaceutical composition comprising carrying out the method of any of No. 1-8 to identify a molecule that modulates the function, activity, composition or formation of said complex, and further comprising mixing the identified molecule with a pharmaceutically acceptable carrier.
33. A method for diagnosing or screening for the presence of a disease or disorder or a predisposition for developing a disease or disorder in a subject, which disease or disorder is characterized by an aberrant amount of, activity of, or component composition of, or intracellular localization of the complex of any one of the No. 1-8, comprising determining the amount of, activity of, protein components of, and/or intracellular localization of, said complex and/or the transcription level of a gene dependent on the complex and/or the abundance and/or activity of a protein or protein complex dependent on the function of the complex and/or product of a gene dependent on the complex in a comparative sample derived from a subject, wherein a difference in said amount, activity, or protein components of, said complex in an analogous sample from a subject not having the disease or disorder or predisposition indicates the presence in the subject of the disease or disorder or predisposition in the subject.
34. The method of No. 33, wherein the amount of said complex is determined.
35. The method of No. 33, wherein the activity of said complex is determined.
36. The method of No. 35, wherein said determining step comprises isolating from the subject said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
37. The method of No. 33, wherein the amount of the individual protein components of said complex are determined.
38. The method of No. 37, wherein said determining step comprises determining whether
   (i) "TRAF6" (SEQ ID No:189) or a functionally active derivative thereof, or a functionally active fragment thereof, or a homolog thereof, or a variant of "TRAF6" encoded by a nucleic acid that hybridizes to the "TRAF6" nucleic acid under low stringency conditions, and/or
   (ii) "TRAF5" (SEQ ID No:258) or a functionally active derivative thereof, or a functionally active fragment thereof, or a homolog thereof, or a variant of "TRAF5" encoded by a nucleic acid that hybridizes to the "TRAF5" nucleic acid under low stringency conditions, and/or
   (iii) "TRAF1" (SEQ ID No:14) or a functionally active derivative thereof, or a functionally active fragment thereof, or a homolog thereof, or a variant of "TRAF1" encoded by a nucleic acid that hybridizes to the "TRAF1" nucleic acid under low stringency conditions, and/or
   (iv) "cIAP1" (SEQ ID No:35) or a functionally active derivative thereof, or a functionally active fragment thereof, or a homolog thereof, or a variant of "cIAP1" encoded by a nucleic acid that hybridizes to the "cIAP1" nucleic acid under low stringency conditions, and/or
   (v) "SERINE HYDROXYMETHYLTRANSFERASE, CYTOSOLIC" (SEQ ID No:259) or a functionally active derivative thereof, or a functionally active fragment thereof, or a homolog thereof, or a variant of "SERINE HYDROXYMETHYLTRANSFERASE, CYTOSOLIC" encoded by a nucleic acid that hybridizes to the "SERINE HYDROXYMETHYLTRANSFERASE, CYTOSOLIC" nucleic acid under low stringency conditions, and/or
   (vi) "HSP90α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions, and/or
   (vii) "RIP2" (SEQ ID No:187) or a functionally active derivative thereof, or a functionally active fragment thereof, or a homolog thereof, or a variant of "RIP2" encoded by a nucleic acid that hybridizes to the "RIP2" nucleic acid under low stringency conditions, and/or
   (viii) "TRADD" (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions, and/or
   (ix) "TNFR1" (SEQ ID No:21) or a functionally active derivative thereof, or a functionally active fragment thereof, or a homolog thereof, or a variant of "TNFR1" encoded by a nucleic acid that hybridizes to the "TNFR1" nucleic acid under low stringency conditions, is present in the complex.
39. The complex of any one of No. 1-8 or the antibody or fragment of No. 15, for use in a method of diagnosing a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
40. A method for treating or preventing a disease or disorder characterized by an aberrant amount of, activity or component composition of or intracellular localization of, the complex of anyone of No. 1 - 8, comprising administering to a subject in need of such treatment or prevention a therapeutically effective amount of one or more molecules that modulate the amount of, apoptosis activity or in vitro kinase activity, or protein components of, said complex.
41. The method according to No. 40, wherein said disease or disorder involves decreased levels of the amount or activity of said complex.
42. The method according to No. 40 , wherein said disease or disorder involves increased levels of the amount or activity of said complex.
43. Complex of any of No. 1-8 and/or protein selected from the following proteins
   (i) "TRAF6" (SEQ ID No:189) or a functionally active derivative thereof, or a functionally active fragment thereof, or a homolog thereof, or a variant of "TRAF6" encoded by a nucleic acid that hybridizes to the "TRAF6" nucleic acid under low stringency conditions,
   (ii) "TRAF5" (SEQ ID No:258) or a functionally active derivative thereof, or a functionally active fragment thereof, or a homolog thereof, or a variant of "TRAF5" encoded by a nucleic acid that hybridizes to the "TRAF5" nucleic acid under low stringency conditions,
   (iii) "TRAF1" (SEQ ID No:14) or a functionally active derivative thereof, or a functionally active fragment thereof, or a homolog thereof, or a variant of "TRAF1" encoded by a nucleic acid that hybridizes to the "TRAF1" nucleic acid under low stringency conditions,
   (iv) "cIAP1" (SEQ ID No:35) or a functionally active derivative thereof, or a functionally active fragment thereof, or a homolog thereof, or a variant of "cIAP1" encoded by a nucleic acid that hybridizes to the "cIAP1" nucleic acid under low stringency conditions,
   (v) "SERINE HYDROXYMETHYLTRANSFERASE, CYTOSOLIC" (SEQ ID No:259) or a functionally active derivative thereof, or a functionally active fragment thereof, or a homolog thereof, or a variant of "SERINE HYDROXYMETHYLTRANSFERASE, CYTOSOLIC" encoded by a nucleic acid that hybridizes to the "SERINE HYDROXYMETHYLTRANSFERASE, CYTOSOLIC" nucleic acid under low stringency conditions,
   (vi) "HSP90α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions,
   (vii) "RIP2" (SEQ ID No:187) or a functionally active derivative thereof, or a functionally active fragment thereof, or a homolog thereof, or a variant of "RIP2" encoded by a nucleic acid that hybridizes to the "RIP2" nucleic acid under low stringency conditions,
   (viii) "TRADD" (SEQ ID No:17) or a functionally active derivative thereof,or a functionally active fragement thereof, or a homolog thereof, or a variant of "TRADD" that hybridizes to the "TRADD" nucleic acid under low stringency conditions, and
   (ix) "TNFR1" (SEQ ID No:21) or a functionally active derivative thereof, or a functionally active fragment thereof, or a homolog thereof, or a variant of "TNFR1" encoded by a nucleic acid that hybridizes to the "TNFR1" nucleic acid under low stringency conditions, as a target for an active agent of a pharmaceutical, preferably a drug target in the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.

The present invention further relates to the RIP3 protein complex
1. A protein complex selected from complex (I) and comprising
   (a) at least one first protein selected from the group consisting of:
      (i) "RIP3" (SEQ ID No:255) or a functionally active derivative thereof, or a functionally active fragment thereof, or a homolog thereof, or a variant of "RIP3" encoded by a nucleic acid that hybridizes to the "RIP3" nucleic acid under low stringency conditions, and
      (ii) "RIP" (SEQ ID No:16) or a functionally active derivative thereof, or a functionally active fragment thereof, or a homolog thereof, or a variant of "RIP"encoded by a nucleic acid that hybridizes to the "RIP"nucleic acid under low stringency conditions,
      and
   (b) at least one second protein, which second protein is selected from the group consisting of:
      (i) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
      (ii) "HSP90 β 2" (SEQ ID No:107) or a functionally active derivative thereof, or a functionally active fragment thereof, or a homolog thereof, or a variant of "HSP90 β 2" encoded by a nucleic acid that hybridizes to the "HSP90 β 2" nucleic acid under low stringency conditions,
      (iii) "REGULATOR OF G-PROTEIN SIGNALLING 10" (SEQ ID No:260) or a functionally active derivative thereof, or a functionally active fragment thereof, or a homolog thereof, or a variant of "REGULATOR OF G-PROTEIN SIGNALLING 10" encoded by a nucleic acid that hybridizes to the "REGULATOR OF G-PROTEIN SIGNALLING 10" nucleic acid under low stringency conditions, and
      (iv) "HSP90α" (SEQ ID No:105) or a functionally active derivative thereof, or a functionally active fragment thereof, or a homolog thereof, or a variant of "HSP90α" encoded by a nucleic acid that hybridizes to the "HSP90α" nucleic acid under low stringency conditions;
   and a complex (II) comprising at least two of said second proteins,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
2. The protein complex according to claim 1 wherein the first protein is the protein "RIP3" (SEQ ID No:255), or a functionally active derivative thereof, or a functionally active fragment thereof, or a homolog thereof, or a variant of "RIP3" encoded by a nucleic acid that hybridizes to the "RIP3" nucleic acid under low stringency conditions.
3. The protein complex according to No. 1 selected from complex (I) and comprising the following proteins:
   (i) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (ii) "HSP90 β 2" (SEQ ID No:107) or a functionally active derivative thereof, or a functionally active fragment thereof, or a homolog thereof, or a variant of "HSP90 β 2" encoded by a nucleic acid that hybridizes to the "HSP90 β 2" nucleic acid under low stringency conditions,
   (iii) "RIP3" (SEQ ID No:255) or a functionally active derivative thereof,or a functionally active fragement thereof, or a homolog thereof, or a variant of "RIP3" that hybridizes to the "RIP3" nucleic acid under low stringency conditions, and
   (iv) "RIP" (SEQ ID No:16) or a functionally active derivative thereof, or a functionally active fragment thereof, or a homolog thereof, or a variant of "RIP" encoded by a nucleic acid that hybridizes to the "RIP" nucleic acid under low stringency conditions,
   (v) "REGULATOR OF G-PROTEIN SIGNALLING 10" (SEQ ID No:260) or a functionally active derivative thereof,or a functionally active fragement thereof, or a homolog thereof, or a variant of "REGULATOR OF G-PROTEIN SIGNALLING 10" that hybridizes to the "REGULATOR OF G-PROTEIN SIGNALLING 10" nucleic acid under low stringency conditions, and
   (vi) "HSP90α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic add under low stringency conditions,
   and a protein complex selected from complex (II) and comprising the following proteins:
   (i) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (ii) "HSP90 β 2" (SEQ ID No:107) or a functionally active derivative thereof, or a functionally active fragment thereof, or a homolog thereof, or a variant of "HSP90 β 2" encoded by a nucleic acid that hybridizes to the "HSP90 β 2" nucleic acid under low stringency conditions,
   (iii) "RIP3" (SEQ ID No:255) or a functionally active derivative thereof,or a functionally active fragement thereof, or a homolog thereof, or a variant of "RI P3" that hybridizes to the "RIP3" nucleic acid under low stringency conditions, and
   (iv) "REGULATOR OF G-PROTEIN SIGNALLING 10" (SEQ ID No:260) or a functionally active derivative thereof, or a functionally active fragment thereof, or a homolog thereof, or a variant of "REGULATOR OF G-PROTEIN SIGNALLING 10" encoded by a nucleic acid that hybridizes to the "REGULATOR OF G-PROTEIN SIGNALLING 10" nucleic acid under low stringency conditions, and
   (v) "HSP90 β 2" (SEQ ID No:107) or a functionally active derivative thereof, or a functionally active fragment thereof, or a homolog thereof, or a variant of "HSP90 β 2" encoded by a nucleic acid that hybridizes to the "HSP90 β 2" nucleic acid under low stringency conditions.
4. The protein complex according to No. 1 selected from complex (I) comprising all but 1 - 3 of the following proteins:
   (i) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (ii) "HSP90 β2" (SEQ ID No:107) or a functionally active derivative thereof, or a functionally active fragment thereof, or a homolog thereof, or a variant of "HSP90 β2" encoded by a nucleic acid that hybridizes to the "HSP90 β2" nucleic acid under low stringency conditions,
   (iii) "RIP3" (SEQ ID No:255) or a functionally active derivative thereof, or a functionally active fragment thereof, or a homolog thereof, or a variant of "RIP3" encoded by a nucleic acid that hybridizes to the "RIP3" nucleic acid under low stringency conditions,
   (iv) "RIP"(SEQ ID No:16) or a functionally active derivative thereof, or a functionally active fragment thereof, or a homolog thereof, or a variant of "RIP" encoded by a nucleic acid that hybridizes to the "RIP" nucleic acid under low stringency conditions,
   (v) "REGULATOR OF G-PROTEIN SIGNALLING 10" (SEQ ID No:260) or a functionally active derivative thereof,or a functionally active fragement thereof, or a homolog thereof, or a variant of "REGULATOR OF G-PROTEIN SIGNALLING 10" that hybridizes to the "REGULATOR OF G-PROTEIN SIGNALLING 10" nucleic acid under low stringency conditions, and
   (vi) "HSP90α" (SEQ ID No:105) or a functionally active derivative thereof, or a functionally active fragment thereof, or a homolog thereof, or a variant of "HSP90 α" encoded by a nucleic acid that hybridizes to the "HSP90 α" nucleic acid under low stringency conditions;
   and a protein complex selected from complex (II) that comprises all but 1 - 3 of the following proteins:
   (i) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (ii) "HSP90 β 2" (SEQ ID No:107) or a functionally active derivative thereof, or a functionally active fragment thereof, or a homolog thereof, or a variant of "HSP90 β2" encoded by a nucleic acid that hybridizes to the "HSP90 β2" nucleic acid under low stringency conditions,
   (iii) "RIP3" (SEQ ID No:255) or a functionally active derivative thereof, or a functionally active fragment thereof, or a homolog thereof, or a variant of "RIP3" encoded by a nucleic acid that hybridizes to the "RIP3" nucleic acid under low stringency conditions,
   (iv) "REGULATOR OF G-PROTEIN SIGNALLING 10" (SEQ ID No:260) or a functionally active derivative thereof,or a functionally active fragement thereof, or a homolog thereof, or a variant of ""REGULATOR OF G-PROTEIN SIGNALLING 10"" that hybridizes to the ""REGULATOR OF G-PROTEIN SIGNALLING 10"" nucleic acid under low stringency conditions, and
   (v) "HSP90α" (SEQ ID No:105) or a functionally active derivative thereof.
5. The complex of any of No. 1-4 comprising a functionally active derivative of said first protein and/or a functionally active derivative of said second protein, wherein the functionally active derivative is a fusion protein comprising said first protein or said second protein fused to an amino acid sequence different from the first protein or second protein, respectively.
6. The complex of No. 5 wherein the functionally active derivative is a fusion protein comprising said first protein or said second protein fused to an affinity tag or label.
7. The complex of any of No. 1-4 comprising a fragment of said first protein and/or a fragment of said second protein, which fragment binds to another protein component of said complex.
8. The complex of any of No. 1-7 that is involved in the in vitro kinase activity or apoptosis activity.
9. A process for preparing complex of any of No. 1-8 and optionally the components thereof comprising the following steps:
   the expressing a protein (bait) of the complex, preferably a tagged protein, in a target cell, isolating the protein complex which is attached to the bait protein, and optionally disassociating the protein complex and isolating the individual complex members.
10. The process according to No. 9 wherein the tagged protein comprises two different tags which allow two separate affinity purification steps.
11. The process according to any of No. 9 - 10 wherein the two tags are separated by a cleavage site for a protease.
12. Component of the RIP3 complex obtainable by a process according to any of No. 9 to 11.
13. Construct, preferably a vector construct, comprising at least two separate nucleic acid sequences each encoding a different protein, or a functionally active fragment or a functionally active derivative therof at least one of said proteins, or functionally active fragments or functionally active derivative therof being selected from the first group of proteins according to No. 1(a) and at least one of said proteines, or functionally active fragments of functionally active derivative thereof being selected from the second group of proteins according to No. 1(b)
14. Host cell containting a construct of No. 13 or containing several vectors each comprising at least the nucleic acid sequence encoding at least one of the proteins, or functionally active fragments or functionally active derivatives thereof selected from the first group of proteins according to No. 1(a) and the proteins, or functionally active fragments or functionally active derivatives thereof selected from the second group of proteins according to No. 1(b).
15. An antibody or a fragment of said antibody containing the binding domain thereof, selected from an antibody or fragment thereof, which binds the complex of any of No. 1-8 and which does not bind any of the proteins of said complex when uncomplexed and an antibody or a fragment of said antibody which binds to any of the proteins according to No..
16. A kit comprising in one or more container the complex of any of No. 1 - 8 optionally together with an antibody according to No. 15 and/or further components such as reagents and working instructions.
17. The kit according to No.16 for processing a substrate of said complex.
18. A kit according to No. 16 for the diagnosis or prognosis of a disease or a disease risk, preferentially for a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
19. Array, in which at least a complex according to any of No. 1 - 8 and/or at least one antibody according to No. 15 is attached to a solid carrier.
20. A process for processing a physiological substrate of the complex comprising the step of bringing into contact a complex to any of No. 1 - 8 with said substrate, such that said substrate is processed.
21. A pharmaceutical composition comprising the protein complex of any of No. 1 to 8..
22. The pharmaceutical composition according to No. 21 for the treatment of diseases and disorders such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
23. A method for screening for a molecule that binds to the complex of anyone of No. 1 - 8. comprising the steps of
   (a) exposing said complex, or a cell or organism containing same to one or more candidate molecules; and
   (b) determinig whether said candidate molecule is bound to the complex or protein.
24. A method for screening for a molecule that modulates directly or indirectly the function, activity, composition or formation of the complex of any one of No. 1-8 comprising the steps of (a) exposing said complex, or a cell or organism containing RIP3 complex to one or more candidate molecules; and
   (b) determining the amount of activity of protein components of, and/or intracellular localization of, said complex and/or the transcription level of a gene dependent on the complex and/or the abundance and/or activity of a protein or protein complex dependent on the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependent on the complex and/or the abundance and/or activity of a protein or protein complex dependent on the function of the complex and/or product of a gene dependent on the complex in the absence of said candidate molecules indicates that the molecule modulates function, activity or composition of said complex.
25. The method of No. 24, wherein the amount of said complex is determined.
26. The method of No. 24, wherein the activity of said complex is determined.
27. The method of No. 26, wherein said determining step comprises isolating from the cell or organism said complex to produce said isolated complex and contacting said isolated complex in the presence or absence of a candidate molecule with a substrate of said complex and determining the processing of said substrate is modified in the presence of said candidate molecule.
28. The method of No. 24, wherein the amount of the individual protein components of said complex are determined.
29. The method of No. 28, determining step comprises determining whether
   (i) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions, and/or
   (ii) "HSP90 β2" (SEQ ID No:107) or a functionally active derivative thereof, or a functionally active fragment thereof, or a homolog thereof, or a variant of "HSP90 β2" encoded by a nucleic acid that hybridizes to the "HSP90 β2" nucleic acid under low stringency conditions, and/or
   (iii) "RIP3" (SEQ ID No:255) or a functionally active derivative thereof, or a functionally active fragment thereof, or a homolog thereof, or a variant of "RIP3" encoded by a nucleic acid that hybridizes to the "RIP3" nucleic acid under low stringency conditions, and/or
   (iv) "RIP" (SEQ ID No:16) or a functionally active derivative thereof, or a functionally active fragment thereof, or a homolog thereof, or a variant of "RIP" encoded by a nucleic acid that hybridizes to the "RIP" nucleic acid under low stringency conditions, and/or
   (v) "REGULATOR OF G-PROTEIN SIGNALLING 10" (SEQ ID No:260) or a functionally active derivative thereof, or a functionally active fragment thereof, or a homolog thereof, or a variant of ""REGULATOR OF G-PROTEIN SIGNALLING 10"" encoded by a nucleic acid that hybridizes to the ""REGULATOR OF G-PROTEIN SIGNALLING 10"" nucleic acid under low stringency conditions, and/or
   (vi) "HSP90α" (SEQ ID No:105) or a functionally active derivative thereof, or a functionally active fragment thereof, or a homolog thereof, or a variant of "HSP90α" encoded by a nucleic acid that hybridizes to the "HSP90α" nucleic acid under low stringency conditions,
   is present in the complex.
30. The method of any of No. 24 to 29, wherein said method is a method of screening for a drug for treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
31. Use of a molecule that modulates the amount of, activity of, or the protein components of the complex of any one of No. 1 - 8 for the manufacture of a medicament for the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
32. A method for the production of a pharmaceutical composition comprising carrying out the method of any of No. 1 - 8 to identify a molecule that modulates the function, activity, composition or formation of said complex, and further comprising mixing the identified molecule with a pharmaceutically acceptable carrier.
33. A method for diagnosing or screening for the presence of a disease or disorder or a predisposition for developing a disease or disorder in a subject, which disease or disorder is characterized by an aberrant amount of, activity of, or component composition of, or intracellular localization of the complex of any one of the No. 1-8, comprising determining the amount of, activity of, protein components of, and/or intracellular localization of, said complex and/or the transcription level of a gene dependent on the complex and/or the abundance and/or activity of a protein or protein complex dependent on the function of the complex and/or product of a gene dependent on the complex in a comparative sample derived from a subject, wherein a difference in said amount, activity, or protein components of, said complex in an analogous sample from a subject not having the disease or disorder or predisposition indicates the presence in the subject of the disease or disorder or predisposition in the subject.
34. The method of No. 33, wherein the amount of said complex is determined.
35. The method of No. 33, wherein the activity of said complex is determined.
36. The method of No. 35, wherein said determining step comprises isolating from the subject said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
37. The method of No. 33, wherein the amount of the individual protein components of said complex are determined.
38. The method of No. 37, wherein said determining step comprises determining whether
   (i) "CDC37" (SEQ ID No:104) or a functionally active derivative thereof, or a functionally active fragment thereof, or a homolog thereof, or a variant of "CDC37" encoded by a nucleic acid that hybridizes to the "CDC37" nucleic acid under low stringency conditions, and/or
   (ii) "HSP90 β2" (SEQ ID No: 107) or a functionally active derivative thereof, or a functionally active fragment thereof, or a homolog thereof, or a variant of "HSP90 β2" encoded by a nucleic acid that hybridizes to the "HSP90 β2" nucleic acid under low stringency conditions, and/or
   (iii) "RIP3" (SEQ ID No:255) or a functionally active derivative thereof, or a functionally active fragment thereof, or a homolog thereof, or a variant of "RIP3" encoded by a nucleic acid that hybridizes to the "RIP3" nucleic acid under low stringency conditions, and/or
   (iv) "RIP" (SEQ ID No:16) or a functionally active derivative thereof, or a functionally active fragment thereof, or a homolog thereof, or a variant of "RIP" encoded by a nucleic acid that hybridizes to the "RIP" nucleic acid under low stringency conditions, and/or
   (v) "REGULATOR OF G-PROTEIN SIGNALLING 10" (SEQ ID No:260) or a functionally active derivative thereof, or a functionally active fragment thereof, or a homolog thereof, or a variant of REGULATOR OF G-PROTEIN SIGNALLING 10"" encoded by a nucleic acid that hybridizes to the ""REGULATOR OF G-PROTEIN SIGNALLING 10"" nucleic acid under low stringency conditions, and/or
   (vi) "HSP90α" (SEQ ID No:105) or a functionally active derivative thereof, or a functionally active fragment thereof, or a homolog thereof, or a variant of "HSP90α" encoded by a nucleic acid that hybridizes to the "HSP90α" nucleic acid under low stringency conditions,
   is present in the complex.
39. The complex of any one of No. 1 - 8 or the antibody or fragment of No. 15, for use in a method of diagnosing a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
40. A method for treating or preventing a disease or disorder characterized by an aberrant amount of, activity or component composition of or intracellular localization of, the complex of anyone of No. 1-8, comprising administering to a subject in need of such treatment or prevention a therapeutically effective amount of one or more molecules that modulate the amount of, in vitro kinase activity or apoptosis activity, or protein components of, said complex.
41. The method according to No. 40, wherein said disease or disorder involves decreased levels of the amount or activity of said complex.
42. The method according to No. 40 , wherein said disease or disorder involves increased levels of the amount or activity of said complex.
43. Complex of any of No. 1-8 and/or protein selected from the following proteins
   (i) "CDC37" (SEQ ID No: 104) or a functionally active derivative thereof, or a functionally active fragment thereof, or a homolog thereof, or a variant of "CDC37" encoded by a nucleic acid that hybridizes to the "CDC37" nucleic acid under low stringency conditions,
   (ii) "HSP90 β2" (SEQ ID No:107) or a functionally active derivative thereof, or a functionally active fragment thereof, or a homolog thereof, or a variant of "HSP90 β2" encoded by a nucleic acid that hybridizes to the "HSP90 β2" nucleic acid under low stringency conditions,
   (iii) "RIP3" (SEQ ID No:255) or a functionally active derivative thereof, or a functionally active fragment thereof, or a homolog thereof, or a variant of "RIP3" encoded by a nucleic acid that hybridizes to the "RIP3" nucleic acid under low stringency conditions,
   (iv) "RIP" (SEQ ID No:16) or a functionally active derivative thereof, or a functionally active fragment thereof, or a homolog thereof, or a variant of "RIP" encoded by a nucleic acid that hybridizes to the "RIP" nucleic acid under low stringency conditions,
   (v) "REGULATOR OF G-PROTEIN SIGNALLING 10" (SEQ ID No:260) or a functionally active derivative thereof,or a functionally active fragement thereof, or a homolog thereof, or a variant of ""REGULATOR OF G-PROTEIN SIGNALLING 10"" that hybridizes to the ""REGULATOR OF G-PROTEIN SIGNALLING 10"" nucleic acid under low stringency conditions, and
   (vi) "HSP90α" (SEQ ID No:105) or a functionally active derivative thereof, or a functionally active fragment thereof, or a homolog thereof, or a variant of "HSP90α" encoded by a nucleic acid that hybridizes to the "HSP90α" nucleic acid under low stringency conditions,
as a target for an active agent of a pharmaceutical, preferably a drug target in the treatment or prevention of a disease or disorder such as inflammation such as chronic inflammatory diseases, rheumatoid arthritis, inflammatory bowel disease; infectious diseases such as septic shock and bacterial infection; neurological diseases such as stroke-induced inflammation in neurons;neurodegenerative diseases;cancer such as prostate cancer, breast cancer and skin cancer.

The present invention further relates to the TRADD protein complex
1. A protein complex selected from complex (I) and comprising
   (a) at least one first protein selected from the group consisting of:
      (i) "FADD " (SEQ ID No:22) or a functionally active derivative thereof, or a functionally active fragment thereof, or a homolog thereof, or a variant of "FADD " encoded by a nucleic acid that hybridizes to the "FADD " nucleic acid under low stringency conditions,
      (ii) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions,
      (iii) "RIP"(SEQ ID No:16) or a functionally active derivative thereof, or a functionally active fragment thereof, or a homolog thereof, or a variant of "RIP"encoded by a nucleic acid that hybridizes to the "RIP"nucleic acid under low stringency conditions,
      (iv) "TNFR1 " (SEQ ID No:21) or a functionally active derivative thereof, or a functionally active fragment thereof, or a homolog thereof, or a variant of "TNFR1 " encoded by a nucleic acid that hybridizes to the "TNFR1 " nucleic acid under low stringency conditions, and
      (v) "TRADD" (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions, and
   (b) at least one second protein, which second protein is selected from the group consisting of:
      (i) "G PROTEIN-COUPLED RECEPTOR 74" (SEQ ID No:261) or a functionally active derivative thereof, or a functionally active fragment thereof, or a homolog thereof, or a variant of "G PROTEIN-COUPLED RECEPTOR 74" encoded by a nucleic acid that hybridizes to the "G PROTEIN-COUPLED RECEPTOR 74" nucleic acid under low stringency conditions,
      (ii) "MACROPHAGE MIGRATION INHIBITORY FACTOR" (SEQ ID No:33) or a functionally active derivative thereof, or a functionally active fragment thereof, or a homolog thereof, or a variant of "MACROPHAGE MIGRATION INHIBITORY FACTOR" encoded by a nucleic acid that hybridizes to the "MACROPHAGE MIGRATION INHIBITORY FACTOR" nucleic acid under low stringency conditions,
      (iii) "COMPLEMENT-C1Q TUMOR NECROSIS FACTOR-RELATED PROTEIN 6 PRECURSOR" (SEQ ID No:262) or a functionally active derivative thereof, or a functionally active fragment thereof, or a homolog thereof, or a variant of "COMPLEMENT-C1Q TUMOR NECROSIS FACTOR-RELATED PROTEIN 6 PRECURSOR" encoded by a nucleic acid that hybridizes to the "COMPLEMENT-C1Q TUMOR NECROSIS FACTOR-RELATED PROTEIN 6 PRECURSOR" nucleic acid under low stringency conditions,
      (iv) "GRANULINS PRECURSOR" (SEQ ID No:263) or a functionally active derivative thereof, or a functionally active fragment thereof, or a homolog thereof, or a variant of "GRANULINS PRECURSOR" encoded by a nucleic acid that hybridizes to the "GRANULINS PRECURSOR" nucleic acid under low stringency conditions, and
      (v) "CARBONIC ANHYDRASE IX PRECURSOR" (SEQ ID No:264) or a functionally active derivative thereof, or a functionally active fragment thereof, or a homolog thereof, or a variant of "CARBONIC ANHYDRASE IX PRECURSOR" encoded by a nucleic acid that hybridizes to the "CARBONIC ANHYDRASE IX PRECURSOR" nucleic acid under low stringency conditions;
   and a complex (II) comprising at least two of said second proteins,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
2. The protein complex according to claim 1 wherein the first protein is the protein "TRADD" (SEQ ID No:17), or a functionally active derivative thereof, or a functionally active fragment thereof, or a homolog thereof, or a variant of "TRADD" encoded by a nucleic acid that hybridizes to the "TRADD" nucleic acid under low stringency conditions.
3. The protein complex according to No. 1 selected from complex (I) and comprising the following proteins:
   (i) "GRANULINS PRECURSOR" (SEQ ID No:263) or a functionally active derivative thereof,or a functionally active fragement thereof, or a homolog thereof, or a variant of "GRANULINS PRECURSOR" that hybridizes to the "GRANULINS PRECURSOR" nucleic acid under low stringency conditions, and
   (ii) "TRADD" (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions, and a protein complex selected from complex (II) and comprising the following proteins:

   (i) "G PROTEIN-COUPLED RECEPTOR 74" (SEQ ID No:261) or a functionally active derivative thereof, or a functionally active fragment thereof, or a homolog thereof, or a variant of "G PROTEIN-COUPLED RECEPTOR 74" encoded by a nucleic acid that hybridizes to the "G PROTEIN-COUPLED RECEPTOR 74" nucleic acid under low stringency conditions,
   (ii) "TRADD" (SEQ ID No:17) or a functionally active derivative thereof,or a functionally active fragement thereof, or a homolog thereof, or a variant of "TRADD" that hybridizes to the "TRADD" nucleic acid under low stringency conditions,
   (iii) "GRANULINS PRECURSOR" (SEQ ID No:263) or a functionally active derivative thereof, or a functionally active fragment thereof, or a homolog thereof, or a variant of "GRANULINS PRECURSOR" encoded by a nucleic acid that hybridizes to the "GRANULINS PRECURSOR" nucleic acid under low stringency conditions, and
   (iv) "CARBONIC ANHYDRASE IX PRECURSOR" (SEQ ID No:264) or a functionally active derivative thereof, or a functionally active fragment thereof, or a homolog thereof, or a variant of "CARBONIC ANHYDRASE IX PRECURSOR" encoded by a nucleic acid that hybridizes to the "CARBONIC ANHYDRASE IX PRECURSOR" nucleic acid under low stringency conditions;
   and a protein complex selected from complex (III) and comprising the following proteins:
   (i) "TRADD" (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions,
   (ii) "GRANULINS PRECURSOR" (SEQ ID No:263) or a functionally active derivative thereof, or a functionally active fragment thereof, or a homolog thereof, or a variant of "GRANULINS PRECURSOR" encoded by a nucleic acid that hybridizes to the "GRANULINS PRECURSOR" nucleic acid under low stringency conditions,
   (iii) "MACROPHAGE MIGRATION INHIBITORY FACTOR" (SEQ ID No:33) or a functionally active derivative thereof,or a functionally active fragement thereof, or a homolog thereof, or a variant of "MACROPHAGE MIGRATION INHIBITORY FACTOR" that hybridizes to the "MACROPHAGE MIGRATION INHIBITORY FACTOR" nucleic acid under low stringency conditions, and
   (iv) "COMPLEMENT-C1Q TUMOR NECROSIS FACTOR-RELATED PROTEIN 6 PRECURSOR" (SEQ ID No:262) or a functionally active derivative thereof, or a functionally active fragment thereof, or a homolog thereof, or a variant of "COMPLEMENT-C1Q TUMOR NECROSIS FACTOR-RELATED PROTEIN 6 PRECURSOR" encoded by a nucleic acid that hybridizes to the "COMPLEMENT-C1Q TUMOR NECROSIS FACTOR-RELATED PROTEIN 6 PRECURSOR" nucleic acid under low stringency conditions;
   and a protein complex selected from complex (IV) and comprising the following proteins:
   (i) "FADD" (SEQ ID No:22) or a functionally active derivative thereof, or a functionally active fragment thereof, or a homolog thereof, or a variant of "FADD" encoded by a nucleic acid that hybridizes to the "FADD" nucleic acid under low stringency conditions,
   (ii) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions,
   (iii) "RIP" (SEQ ID No:16) or a functionally active derivative thereof, or a functionally active fragment thereof, or a homolog thereof, or a variant of "RIP" encoded by a nucleic acid that hybridizes to the "RIP" nucleic acid under low stringency conditions,
   (iv) "TNFR1 " (SEQ ID No:21) or a functionally active derivative thereof, or a functionally active fragment thereof, or a homolog thereof, or a variant of "TNFR1" encoded by a nucleic acid that hybridizes to the "TNFR1" nucleic acid under low stringency conditions,
   (v) "G PROTEIN-COUPLED RECEPTOR 74" (SEQ ID No:261) or a functionally active derivative thereof, or a functionally active fragment thereof, or a homolog thereof, or a variant of "G PROTEIN-COUPLED RECEPTOR 74" encoded by a nucleic acid that hybridizes to the "G PROTEIN-COUPLED RECEPTOR 74" nucleic acid under low stringency conditions,
   (vi) "MACROPHAGE MIGRATION INHIBITORY FACTOR" (SEQ ID No:33) or a functionally active derivative thereof, or a functionally active fragment thereof, or a homolog thereof, or a variant of "MACROPHAGE MIGRATION INHIBITORY FACTOR" encoded by a nucleic acid that hybridizes to the "MACROPHAGE MIGRATION INHIBITORY FACTOR" nucleic acid under low stringency conditions,
   (vii) "COMPLEMENT-C1Q TUMOR NECROSIS FACTOR-RELATED PROTEIN 6 PRECURSOR" (SEQ ID No:262) or a functionally active derivative thereof, or a functionally active fragment thereof, or a homolog thereof, or a variant of "COMPLEMENT-C1Q TUMOR NECROSIS FACTOR-RELATED PROTEIN 6 PRECURSOR" encoded by a nucleic acid that hybridizes to the "COMPLEMENT-C1Q TUMOR NECROSIS FACTOR-RELATED PROTEIN 6 PRECURSOR" nucleic acid under low stringency conditions,
   (viii) "GRANULINS PRECURSOR" (SEQ ID No:263) or a functionally active derivative thereof, or a functionally active fragment thereof, or a homolog thereof, or a variant of "GRANULINS PRECURSOR" encoded by a nucleic acid that hybridizes to the "GRANULINS PRECURSOR" nucleic acid under low stringency conditions,
   (ix) "CARBONIC ANHYDRASE IX PRECURSOR" (SEQ ID No:264) or a functionally active derivative thereof,or a functionally active fragement thereof, or a homolog thereof, or a variant of "CARBONIC ANHYDRASE IX PRECURSOR" that hybridizes to the "CARBONIC ANHYDRASE IX PRECURSOR" nucleic acid under low stringency conditions, and
   (x) "TRADD" (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions.
4. The protein complex according to No. 1 selected from complex (I) comprising all but 1 - 4 of the following proteins:
   (i) "FADD" (SEQ ID No:22) or a functionally active derivative thereof, or a functionally active fragment thereof, or a homolog thereof, or a variant of "FADD" encoded by a nucleic acid that hybridizes to the "FADD" nucleic acid under low stringency conditions,
   (ii) "TRAF2" (SEQ ID No: 15) or a functionally active derivative thereof, or a functionally active fragment thereof, or a homolog thereof, or a variant of "TRAF2" encoded by a nucleic acid that hybridizes to the "TRAF2" nucleic acid under low stringency conditions,
   (iii) "RIP" (SEQ ID No:16) or a functionally active derivative thereof, or a functionally active fragment thereof, or a homolog thereof, or a variant of "RIP" encoded by a nucleic acid that hybridizes to the "RIP" nucleic acid under low stringency conditions,
   (iv) "TNFR1 " (SEQ ID No:21) or a functionally active derivative thereof, or a functionally active fragment thereof, or a homolog thereof, or a variant of "TNFR1" encoded by a nucleic acid that hybridizes to the "TNFR1" nucleic acid under low stringency conditions,
   (v) "G PROTEIN-COUPLED RECEPTOR 74" (SEQ ID No:261) or a functionally active derivative thereof, or a functionally active fragment thereof, or a homolog thereof, or a variant of "G PROTEIN-COUPLED RECEPTOR 74" encoded by a nucleic acid that hybridizes to the "G PROTEIN-COUPLED RECEPTOR 74" nucleic acid under low stringency conditions,
   (vi) "MACROPHAGE MIGRATION INHIBITORY FACTOR" (SEQ ID No:33) or a functionally active derivative thereof, or a functionally active fragment thereof, or a homolog thereof, or a variant of "MACROPHAGE MIGRATION INHIBITORY FACTOR" encoded by a nucleic acid that hybridizes to the "MACROPHAGE MIGRATION INHIBITORY FACTOR" nucleic acid under low stringency conditions,
   (vii) "COMPLEMENT-C1Q TUMOR NECROSIS FACTOR-RELATED PROTEIN 6 PRECURSOR" (SEQ ID No:262) or a functionally active derivative thereof, or a functionally active fragment thereof, or a homolog thereof, or a variant of "COMPLEMENT-C1Q TUMOR NECROSIS FACTOR-RELATED PROTEIN 6 PRECURSOR" encoded by a nucleic acid that hybridizes to the "COMPLEMENT-C1Q TUMOR NECROSIS FACTOR-RELATED PROTEIN 6 PRECURSOR" nucleic acid under low stringency conditions,
   (viii) "GRANULINS PRECURSOR" (SEQ ID No:263) or a functionally active derivative thereof, or a functionally active fragment thereof, or a homolog thereof, or a variant of "GRANULINS PRECURSOR" encoded by a nucleic acid that hybridizes to the "GRANULINS PRECURSOR" nucleic acid under low stringency conditions,
   (ix) "CARBONIC ANHYDRASE IX PRECURSOR" (SEQ ID No:264) or a functionally active derivative thereof,or a functionally active fragement thereof, or a homolog thereof, or a variant of "CARBONIC ANHYDRASE IX PRECURSOR" that hybridizes to the "CARBONIC ANHYDRASE IX PRECURSOR" nucleic acid under low stringency conditions, and
   (x) "TRADD" (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions; and a protein complex selected from complex (II) comprising all but 1-4 of the following proteins:

   (i) "G PROTEIN-COUPLED RECEPTOR 74" (SEQ ID No:261) or a functionally active derivative thereof, or a functionally active fragment thereof, or a homolog thereof, or a variant of "G PROTEIN-COUPLED RECEPTOR 74" encoded by a nucleic acid that hybridizes to the "G PROTEIN-COUPLED RECEPTOR 74" nucleic acid under low stringency conditions,
   (ii) "MACROPHAGE MIGRATION INHIBITORY FACTOR" (SEQ ID No:33) or a functionally active derivative thereof, or a functionally active fragment thereof, or a homolog thereof, or a variant of "MACROPHAGE MIGRATION INHIBITORY FACTOR" encoded by a nucleic acid that hybridizes to the "MACROPHAGE MIGRATION INHIBITORY FACTOR" nucleic acid under low stringency conditions,
   (iii) "COMPLEMENT-C1Q TUMOR NECROSIS FACTOR-RELATED PROTEIN 6 PRECURSOR" (SEQ ID No:262) or a functionally active derivative thereof, or a functionally active fragment thereof, or a homolog thereof, or a variant of "COMPLEMENT-C1Q TUMOR NECROSIS FACTOR-RELATED PROTEIN 6 PRECURSOR" encoded by a nucleic acid that hybridizes to the "COMPLEMENT-C1Q TUMOR NECROSIS FACTOR-RELATED PROTEIN 6 PRECURSOR" nucleic acid under low stringency conditions,
   (iv) "GRANULINS PRECURSOR" (SEQ ID No:263) or a functionally active derivative thereof, or a functionally active fragment thereof, or a homolog thereof, or a variant of "GRANULINS PRECURSOR" encoded by a nucleic acid that hybridizes to the "GRANULINS PRECURSOR" nucleic acid under low stringency conditions,
   (v) "CARBONIC ANHYDRASE IX PRECURSOR" (SEQ ID No:264) or a functionally active derivative thereof,or a functionally active fragement thereof, or a homolog thereof, or a variant of "CARBONIC ANHYDRASE IX PRECURSOR" that hybridizes to the "CARBONIC ANHYDRASE IX PRECURSOR" nucleic acid under low stringency conditions, and
   (vi) "TRADD" (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions.
5. The complex of any of No. 1 - 4 comprising a functionally active derivative of said first protein and/or a functionally active derivative of said second protein, wherein the functionally active derivative is a fusion protein comprising said first protein or said second protein fused to an amino acid sequence different from the first protein or second protein, respectively.
6. The complex of No. 5 wherein the functionally active derivative is a fusion protein comprising said first protein or said second protein fused to an affinity tag or label.
7. The complex of any of No. 1 - 4 comprising a fragment of said first protein and/or a fragment of said second protein, which fragment binds to another protein component of said complex.
8. The complex of any of No. 1 - 7 that is involved in the apoptosis activity.
9. A process for preparing complex of any of No. 1 - 8 and optionally the components thereof comprising the following steps:
   the expressing a protein (bait) of the complex, preferably a tagged protein, in a target cell, isolating the protein complex which is attached to the bait protein, and optionally disassociating the protein complex and isolating the individual complex members.
10. The process according to No. 9 wherein the tagged protein comprises two different tags which allow two separate affinity purification steps.
11. The process according to any of No. 9-10 wherein the two tags are separated by a cleavage site for a protease.
12. Component of the TRADD complex obtainable by a process according to any of No. 9 to 11.
13. Protein of the TRADD complex selected from
   (i) "G PROTEIN-COUPLED RECEPTOR 74" (SEQ ID No:261) or a functionally active derivative thereof, or a functionally active fragment thereof, or a homolog thereof, or a variant of "G PROTEIN-COUPLED RECEPTOR 74" encoded by a nucleic acid that hybridizes to the "G PROTEIN-COUPLED RECEPTOR 74" nucleic acid under low stringency conditions,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
14. Nucleic acid encoding a protein according to No. 13.
15. Construct, preferably a vector construct, comprising (a) a nucleic acid according to No. 14 and at least one further nucleic acid which is normally not associated with said nucleic acid,or(b) at least two separate nucleic acid sequences each encoding a different protein, or a functionally active fragment or a functionally active derivative thereof at least one of said proteins, or functionally active fragments or functionally active derivative thereof being selected from the first group of proteins according to No. 1(a) and at least one of said proteins, or functionally active fragments or functionally active derivative thereof being selected from the second group of proteins according to No. 1(b).
16. Host cell, containing a vector comprising at least one of the nucleic acid of No. 14 and/or a construct of No. 15 or containing several vectors each comprising at least the nucleic acid sequence encoding at least one of the proteins, or functionally active fragments or functionally active derivatives thereof selected from the first group of proteins according to No. 1(a) and the proteins, or functionally active fragments or functionally active derivatives thereof selected from the second group of proteins according to No. 1(b).
17. An antibody or a fragment of said antibody containing the binding domain thereof, selected from an antibody or fragment thereof, which binds the complex of any of No. 1-8 and which does not bind any of the proteins of said complex when uncomplexed and an antibody or a fragment of said antibody which binds to any of the proteins according to No. 13.
18. A kit comprising in one or more container the complex of any of No. 1 - 8 and/or the proteins of No. 13optionally together with an antibody according to No. 17 and/or further components such as reagents and working instructions.
19. The kit according to No. 18 for processing a substrate of said complex.
20. The kit according to No. 18 for the diagnosis or prognosis of a disease or a disease risk, preferentially for a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
21. Array, in which at least a complex according to any of No. 1 - 8 and/or at least one protein according to No. 14 and/or at least one antibody according to No. 17 is attached to a solid carrier.
22. A process for processing a physiological substrate of the complex comprising the step of bringing into contact a complex to any of No. 1 - 8 with said substrate, such that said substrate is processed.
23. A pharmaceutical composition comprising the protein complex of any of No. 1 to 8 and/or any of the following the proteins:
   (i) "G PROTEIN-COUPLED RECEPTOR 74" (SEQ ID No:261) or a functionally active derivative thereof, or a functionally active fragment thereof, or a homolog thereof, or a variant of "G PROTEIN-COUPLED RECEPTOR 74" encoded by a nucleic acid that hybridizes to the "G PROTEIN-COUPLED RECEPTOR 74" nucleic acid under low stringency conditions,
   and a pharmaceutical acceptable carrier.
24. The pharmaceutical composition according to No. 23 for the treatment of diseases and disorders such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
25. A method for screening for a molecule that binds to the complex of anyone of No. 1-8 and/or any of the following proteins:
   (i) "G PROTEIN-COUPLED RECEPTOR 74" (SEQ ID No:261) or a functionally active derivative thereof, or a functionally active fragment thereof, or a homolog thereof, or a variant of "G PROTEIN-COUPLED RECEPTOR 74" encoded by a nucleic acid that hybridizes to the "G PROTEIN-COUPLED RECEPTOR 74" nucleic acid under low stringency conditions, comprising the steps of
      (a) exposing said complex, or a cell or organism containing same to one or more candidate molecules; and
      (b) determinig whether said candidate molecule is bound to the complex or protein.
26. A method for screening for a molecule that modulates directly or indirectly the function, activity, composition or formation of the complex of any one of No. 1 - 8 comprising the steps of (a) exposing said complex, or a cell or organism containing TRADD complex to one or more candidate molecules; and
   (b) determining the amount of activity of protein components of, and/or intracellular localization of, said complex and/or the transcription level of a gene dependent on the complex and/or the abundance and/or activity of a protein or protein complex dependent on the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependent on the complex and/or the abundance and/or activity of a protein or protein complex dependent on the function of the complex and/or product of a gene dependent on the complex in the absence of said candidate molecules indicates that the molecule modulates function, activity or composition of said complex.
27. The method of No. 26, wherein the amount of said complex is determined.
28. The method of No. 26, wherein the activity of said complex is determined.
29. The method of No. 28, wherein said determining step comprises isolating from the cell or organism said complex to produce said isolated complex and contacting said isolated complex in the presence or absence of a candidate molecule with a substrate of said complex and determining the processing of said substrate is modified in the presence of said candidate molecule.
30. The method of No. 26, wherein the amount of the individual protein components of said complex are determined.
31. The method of No. 30, determining step comprises determining whether
   (i) "FADD " (SEQ ID No:22) or a functionally active derivative thereof, or a functionally active fragment thereof, or a homolog thereof, or a variant of "FADD" encoded by a nucleic acid that hybridizes to the "FADD" nucleic acid under low stringency conditions, and/or
   (ii) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions, and/or
   (iii) "RIP" (SEQ ID No:16) or a functionally active derivative thereof, or a functionally active fragment thereof, or a homolog thereof, or a variant of "RIP" encoded by a nucleic acid that hybridizes to the "RIP" nucleic acid under low stringency conditions, and/or
   (iv) "TNFR1 " (SEQ ID No:21) or a functionally active derivative thereof, or a functionally active fragment thereof, or a homolog thereof, or a variant of "TNFR1" encoded by a nucleic acid that hybridizes to the "TNFR1" nucleic acid under low stringency conditions, and/or
   (v) "G PROTEIN-COUPLED RECEPTOR 74" (SEQ ID No:261) or a functionally active derivative thereof, or a functionally active fragment thereof, or a homolog thereof, or a variant of "G PROTEIN-COUPLED RECEPTOR 74" encoded by a nucleic acid that hybridizes to the "G PROTEIN-COUPLED RECEPTOR 74" nucleic acid under low stringency conditions, and/or
   (vi) "MACROPHAGE MIGRATION INHIBITORY FACTOR" (SEQ ID No:33) or a functionally active derivative thereof, or a functionally active fragment thereof, or a homolog thereof, or a variant of "MACROPHAGE MIGRATION INHIBITORY FACTOR" encoded by a nucleic acid that hybridizes to the "MACROPHAGE MIGRATION INHIBITORY FACTOR" nucleic acid under low stringency conditions, and/or
   (vii) "COMPLEMENT-C1Q TUMOR NECROSIS FACTOR-RELATED PROTEIN 6 PRECURSOR" (SEQ ID No:262) or a functionally active derivative thereof, or a functionally active fragment thereof, or a homolog thereof, or a variant of "COMPLEMENT-C1Q TUMOR NECROSIS FACTOR-RELATED PROTEIN 6 PRECURSOR" encoded by a nucleic acid that hybridizes to the "COMPLEMENT-C1Q TUMOR NECROSIS FACTOR-RELATED PROTEIN 6 PRECURSOR" nucleic acid under low stringency conditions, and/or
   (viii) "GRANULINS PRECURSOR" (SEQ ID No:263) or a functionally active derivative thereof, or a functionally active fragment thereof, or a homolog thereof, or a variant of "GRANULINS PRECURSOR" encoded by a nucleic acid that hybridizes to the "GRANULINS PRECURSOR" nucleic acid under low stringency conditions, and/or
   (ix) "CARBONIC ANHYDRASE IX PRECURSOR" (SEQ ID No:264) or a functionally active derivative thereof, or a functionally active fragment thereof, or a homolog thereof, or a variant of "CARBONIC ANHYDRASE IX PRECURSOR" encoded by a nucleic acid that hybridizes to the "CARBONIC ANHYDRASE IX PRECURSOR" nucleic acid under low stringency conditions, and/or
   (x) "TRADD" (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions, is present in the complex.
32. The method of any of No. 26 to 31, wherein said method is a method of screening for a drug for treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
33. Use of a molecule that modulates the amount of, activity of, or the protein components of the complex of any one of No. 1 - 8 for the manufacture of a medicament for the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
34. A method for the production of a pharmaceutical composition comprising carrying out the method of any of No. 1 - 8 to identify a molecule that modulates the function, activity, composition or formation of said complex, and further comprising mixing the identified molecule with a pharmaceutically acceptable carrier.
35. A method for diagnosing or screening for the presence of a disease or disorder or a predisposition for developing a disease or disorder in a subject, which disease or disorder is characterized by an aberrant amount of, activity of, or component composition of, or intracellular localization of the complex of any one of the No. 1 - 8, comprising determining the amount of, activity of, protein components of, and/or intracellular localization of, said complex and/or the transcription level of a gene dependent on the complex and/or the abundance and/or activity of a protein or protein complex dependent on the function of the complex and/or product of a gene dependent on the complex in a comparative sample derived from a subject, wherein a difference in said amount, activity, or protein components of, said complex in an analogous sample from a subject not having the disease or disorder or predisposition indicates the presence in the subject of the disease or disorder or predisposition in the subject.
36. The method of No. 35, wherein the amount of said complex is determined.
37. The method of No. 35, wherein the activity of said complex is determined.
38. The method of No. 37, wherein said determining step comprises isolating from the subject said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
39. The method of No. 35, wherein the amount of the individual protein components of said complex are determined.
40. The method of No. 39, wherein said determining step comprises determining whether
   (i) "FADD " (SEQ ID No:22) or a functionally active derivative thereof, or a functionally active fragment thereof, or a homolog thereof, or a variant of "FADD" encoded by a nucleic acid that hybridizes to the "FADD" nucleic acid under low stringency conditions, and/or
   (ii) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions, and/or
   (iii) "RIP" (SEQ ID No:16) or a functionally active derivative thereof, or a functionally active fragment thereof, or a homolog thereof, or a variant of "RIP" encoded by a nucleic acid that hybridizes to the "RIP" nucleic acid under low stringency conditions, and/or
   (iv) "TNFR1 " (SEQ ID No:21) or a functionally active derivative thereof, or a functionally active fragment thereof, or a homolog thereof, or a variant of "TNFR1" encoded by a nucleic acid that hybridizes to the "TNFR1" nucleic acid under low stringency conditions, and/or
   (v) "G PROTEIN-COUPLED RECEPTOR 74" (SEQ ID No:261) or a functionally active derivative thereof, or a functionally active fragment thereof, or a homolog thereof, or a variant of "G PROTEIN-COUPLED RECEPTOR 74" encoded by a nucleic acid that hybridizes to the "G PROTEIN-COUPLED RECEPTOR 74" nucleic acid under low stringency conditions, and/or
   (vi) "MACROPHAGE MIGRATION INHIBITORY FACTOR" (SEQ ID No:33) or a functionally active derivative thereof, or a functionally active fragment thereof, or a homolog thereof, or a variant of "MACROPHAGE MIGRATION INHIBITORY FACTOR" encoded by a nucleic acid that hybridizes to the "MACROPHAGE MIGRATION INHIBITORY FACTOR" nucleic acid under low stringency conditions, and/or
   (vii) "COMPLEMENT-C1Q TUMOR NECROSIS FACTOR-RELATED PROTEIN 6 PRECURSOR" (SEQ ID No:262) or a functionally active derivative thereof, or a functionally active fragment thereof, or a homolog thereof, or a variant of "COMPLEMENT-C1Q TUMOR NECROSIS FACTOR-RELATED PROTEIN 6 PRECURSOR" encoded by a nucleic acid that hybridizes to the "COMPLEMENT-C1Q TUMOR NECROSIS FACTOR-RELATED PROTEIN 6 PRECURSOR" nucleic acid under low stringency conditions, and/or
   (viii) "GRANULINS PRECURSOR" (SEQ ID No:263) or a functionally active derivative thereof, or a functionally active fragment thereof, or a homolog thereof, or a variant of "GRANULINS PRECURSOR" encoded by a nucleic acid that hybridizes to the "GRANULINS PRECURSOR" nucleic acid under low stringency conditions, and/or
   (ix) "CARBONIC ANHYDRASE IX PRECURSOR" (SEQ ID No:264) or a functionally active derivative thereof, or a functionally active fragment thereof, or a homolog thereof, or a variant of "CARBONIC ANHYDRASE IX PRECURSOR" encoded by a nucleic acid that hybridizes to the "CARBONIC ANHYDRASE IX PRECURSOR" nucleic acid under low stringency conditions, and/or
   (x) "TRADD" (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions, is present in the complex.
41. The complex of any one of No. 1 - 8, or proteins of No. 13 or the antibody or fragment of No. 17, for use in a method of diagnosing a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
42. A method for treating or preventing a disease or disorder characterized by an aberrant amount of, activity or component composition of or intracellular localization of, the complex of anyone of No. 1 - 8, comprising administering to a subject in need of such treatment or prevention a therapeutically effective amount of one or more molecules that modulate the amount of, apoptosis activity, or protein components of, said complex.
43. The method according to No. 42, wherein said disease or disorder involves decreased levels of the amount or activity of said complex.
44. The method according to No. 42 , wherein said disease or disorder involves increased levels of the amount or activity of said complex.
45. Complex of any of No. 1 - 8 and/or protein selected from the following proteins
   (i) "FADD " (SEQ ID No:22) or a functionally active derivative thereof, or a functionally active fragment thereof, or a homolog thereof, or a variant of "FADD" encoded by a nucleic acid that hybridizes to the "FADD" nucleic acid under low stringency conditions,
   (ii) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions,
   (iii) "RIP" (SEQ ID No:16) or a functionally active derivative thereof, or a functionally active fragment thereof, or a homolog thereof, or a variant of "RIP" encoded by a nucleic acid that hybridizes to the "RIP" nucleic acid under low stringency conditions,
   (iv) "TNFR1 " (SEQ ID No:21) or a functionally active derivative thereof, or a functionally active fragment thereof, or a homolog thereof, or a variant of "TNFR1" encoded by a nucleic acid that hybridizes to the "TNFR1" nucleic acid under low stringency conditions,
   (v) "G PROTEIN-COUPLED RECEPTOR 74" (SEQ ID No:261) or a functionally active derivative thereof, or a functionally active fragment thereof, or a homolog thereof, or a variant of "G PROTEIN-COUPLED RECEPTOR 74" encoded by a nucleic acid that hybridizes to the "G PROTEIN-COUPLED RECEPTOR 74" nucleic acid under low stringency conditions,
   (vi) "MACROPHAGE MIGRATION INHIBITORY FACTOR" (SEQ ID No:33) or a functionally active derivative thereof, or a functionally active fragment thereof, or a homolog thereof, or a variant of "MACROPHAGE MIGRATION INHIBITORY FACTOR" encoded by a nucleic acid that hybridizes to the "MACROPHAGE MIGRATION INHIBITORY FACTOR" nucleic acid under low stringency conditions,
   (vii) "COMPLEMENT-C1Q TUMOR NECROSIS FACTOR-RELATED PROTEIN 6 PRECURSOR" (SEQ ID No:262) or a functionally active derivative thereof, or a functionally active fragment thereof, or a homolog thereof, or a variant of "COMPLEMENT-C1Q TUMOR NECROSIS FACTOR-RELATED PROTEIN 6 PRECURSOR" encoded by a nucleic acid that hybridizes to the "COMPLEMENT-C1Q TUMOR NECROSIS FACTOR-RELATED PROTEIN 6 PRECURSOR" nucleic acid under low stringency conditions,
   (viii) "GRANULINS PRECURSOR" (SEQ ID No:263) or a functionally active derivative thereof, or a functionally active fragment thereof, or a homolog thereof, or a variant of "GRANULINS PRECURSOR" encoded by a nucleic acid that hybridizes to the "GRANULINS PRECURSOR" nucleic acid under low stringency conditions,
   (ix) "CARBONIC ANHYDRASE IX PRECURSOR" (SEQ ID No:264) or a functionally active derivative thereof,or a functionally active fragement thereof, or a homolog thereof, or a variant of "CARBONIC ANHYDRASE IX PRECURSOR" that hybridizes to the "CARBONIC ANHYDRASE IX PRECURSOR" nucleic acid under low stringency conditions, and
   (x) "TRADD" (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions, as a target for an active agent of a pharmaceutical, preferably a drug target in the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.

The present invention further relates to the FADD protein complex
1. A protein complex selected from complex (I) and comprising
   (a) at least one first protein selected from the group consisting of:
      (i) "TRADD" (SEQ ID No:17) or a functionally active derivative thereof, or a functionally active fragment thereof, or a homolog thereof, or a variant of "TRADD " encoded by a nucleic acid that hybridizes to the "TRADD " nucleic acid under low stringency conditions,
      (ii) "TNFR1" (SEQ ID No:21) or a functionally active derivative thereof, or a functionally active fragment thereof, or a homolog thereof, or a variant of "TNFR1" encoded by a nucleic acid that hybridizes to the "TNFR1" nucleic acid under low stringency conditions,
      (iii) "FADD" (SEQ ID No:22) or a functionally active derivative thereof, or a functionally active fragment thereof, or a homolog thereof, or a variant of "FADD" encoded by a nucleic acid that hybridizes to the "FADD" nucleic acid under low stringency conditions, and
      (iv) "caspase 8 " (SEQ ID No:256) or a functionally active derivative thereof, or a functionally active fragment thereof, or a homolog thereof, or a variant of "caspase 8 " encoded by a nucleic acid that hybridizes to the "caspase 8 " nucleic acid under low stringency conditions,
      and
   (b) at least one second protein, which second protein is selected from the group consisting of:
      (i) "SERINE HYDROXYMETHYLTRANSFERASE, CYTOSOLIC" (SEQ ID No:259) or a functionally active derivative thereof, or a functionally active fragment thereof, or a homolog thereof, or a variant of "SERINE HYDROXYMETHYLTRANSFERASE, CYTOSOLIC" encoded by a nucleic acid that hybridizes to the "SERINE HYDROXYMETHYLTRANSFERASE, CYTOSOLIC" nucleic acid under low stringency conditions;
   and a complex (II) comprising at least two of said second proteins,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
2. The protein complex according to claim 1 wherein the first protein is the protein "FADD" (SEQ ID No:22), or a functionally active derivative thereof, or a functionally active fragment thereof, or a homolog thereof, or a variant of "FADD" encoded by a nucleic acid that hybridizes to the "FADD" nucleic acid under low stringency conditions.
3. The protein complex according to No. 1 selected from complex (I) and comprising the following proteins:
   (i) "FADD" (SEQ ID No:22) or a functionally active derivative thereof,or a functionally active fragement thereof, or a homolog thereof, or a variant of "FADD" that hybridizes to the "FADD" nucleic acid under low stringency conditions, and
   (ii) "SERINE HYDROXYMETHYLTRANSFERASE, CYTOSOLIC" (SEQ ID No:259) or a functionally active derivative thereof, or a functionally active fragment thereof, or a homolog thereof, or a variant of "SERINE HYDROXYMETHYLTRANSFERASE, CYTOSOLIC" encoded by a nucleic acid that hybridizes to the "SERINE HYDROXYMETHYLTRANSFERASE, CYTOSOLIC" nucleic acid under low stringency conditions;
   and a protein complex selected from complex (II) and comprising the following proteins:
   (i) "TRADD " (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions,
   (ii) (iv) "TNFR1" (SEQ ID No:21) or a functionally active derivative thereof, or a functionally active fragment thereof, or a homolog thereof, or a variant of "TNRF1" encoded by a nucleic acid that hybridizes to the "TNRF1" nucleic acid under low stringency conditions,
   (iii) "FADD" (SEQ ID No:22) or a functionally active derivative thereof, or a functionally active fragment thereof, or a homolog thereof, or a variant of "FADD" encoded by a nucleic acid that hybridizes to the "FADD" nucleic acid under low stringency conditions,
   (iv) "caspase 8 " (SEQ ID No:256) or a functionally active derivative thereof,or a functionally active fragement thereof, or a homolog thereof, or a variant of "caspase 8" that hybridizes to the "caspase8" nucleic acid under low stringency conditions, and (v) "SERINE HYDROXYMETHYLTRANSFERASE, CYTOSOLIC" (SEQ ID No:259) or a functionally active derivative thereof,or a functionally active fragement thereof, or a homolog thereof, or a variant of "SERINE HYDROXYMETHYL TRANSFERASE, CYTOSOLIC" that hybridizes to the "SERINE HYDROXYMETHYLTRANSFERASE, CYTOSOLIC" nucleic acid under low stringency conditions.
4. The protein complex according to No. 1 comprising all but 1 of the following proteins:
   (i) "TRADD " (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions,
   (ii) (iv) "TNFR1" (SEQ ID No:21) or a functionally active derivative thereof, or a functionally active fragment thereof, or a homolog thereof, or a variant of "TNRF1" encoded by a nucleic acid that hybridizes to the "TNRF1" nucleic acid under low stringency conditions,
   (iii) "FADD" (SEQ ID No:22) or a functionally active derivative thereof, or a functionally active fragment thereof, or a homolog thereof, or a variant of "FADD" encoded by a nucleic acid that hybridizes to the "FADD" nucleic acid under low stringency conditions,
   (iv) "caspase 8 " (SEQ ID No:256) or a functionally active derivative thereof,or a functionally active fragement thereof, or a homolog thereof, or a variant of "caspase 8" that hybridizes to the "caspase8" nucleic acid under low stringency conditions, and
   (v) "SERINE HYDROXYMETHYLTRANSFERASE, CYTOSOLIC" (SEQ ID No:259) or a functionally active derivative thereof,or a functionally active fragement thereof, or a homolog thereof, or a variant of "SERINE HYDROXYMETHYLTRANSFERASE, CYTOSOLIC" that hybridizes to the "SERINE HYDROXYMETHYLTRANSFERASE, CYTOSOLIC" nucleic acid under low stringency conditions.
5. The complex of any of No. 1 - 4 comprising a functionally active derivative of said first protein and/or a functionally active derivative of said second protein, wherein the functionally active derivative is a fusion protein comprising said first protein or said second protein fused to an amino acid sequence different from the first protein or second protein, respectively.
6. The complex of No. 5 wherein the functionally active derivative is a fusion protein comprising said first protein or said second protein fused to an affinity tag or label.
7. The complex of any of No. 1 - 4 comprising a fragment of said first protein and/or a fragment of said second protein, which fragment binds to another protein component of said complex.
8. The complex of any of No. 1 -7 that is involved in the.
9. A process for preparing complex of any of No. 1 - 8 and optionally the components thereof comprising the following steps:
   the expressing a protein (bait) of the complex, preferably a tagged protein, in a target cell, isolating the protein complex which is attached to the bait protein, and optionally disassociating the protein complex and isolating the individual complex members.
10. The process according to No. 9 wherein the tagged protein comprises two different tags which allow two separate affinity purification steps.
11. The process according to any of No. 9-10 wherein the two tags are separated by a cleavage site for a protease.
12. Component of the FADD complex obtainable by a process according to any of No. 9 to 11.
13. Construct, preferably a vector construct, comprising at least two separate nucleic acid sequences each encoding a different protein, or a functionally active fragment or a functionally active derivative therof at least one of said proteins, or functionally active fragments or functionally active derivative therof being selected from the first group of proteins according to No. 1(a) and at least one of said proteines, or functionally active fragments of functionally active derivative thereof being selected from the second group of proteins according to No. 1(b)
14. Host cell containting a construct of No. 13 or containing several vectors each comprising at least the nucleic acid sequence encoding at least one of the proteins, or functionally active fragments or functionally active derivatives thereof selected from the first group of proteins according to No. 1(a) and the proteins, or functionally active fragments or functionally active derivatives thereof selected from the second group of proteins according to No. 1(b).
15. An antibody or a fragment of said antibody containing the binding domain thereof, selected from an antibody or fragment thereof, which binds the complex of any of No. 1-8 and which does not bind any of the proteins of said complex when uncomplexed and an antibody or a fragment of said antibody which binds to any of the proteins according to No..
16. A kit comprising in one or more container the complex of any of No. 1 - 8 optionally together with an antibody according to No. 15 and/or further components such as reagents and working instructions.
17. The kit according to No.16 for processing a substrate of said complex.
18. A kit according to No. 16 for the diagnosis or prognosis of a disease or a disease risk, preferentially for a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
19. Array, in which at least a complex according to any of No. 1 - 8 and/or at least one antibody according to No. 15 is attached to a solid carrier.
20. A process for processing a physiological substrate of the complex comprising the step of bringing into contact a complex to any of No. 1 - 8 with said substrate, such that said substrate is processed.
21. A pharmaceutical composition comprising the protein complex of any of No. 1 to 8..
22. The pharmaceutical composition according to No. 21 for the treatment of diseases and disorders such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
23. A method for screening for a molecule that binds to the complex of anyone of No. 1 - 8. comprising the steps of
   (a) exposing said complex, or a cell or organism containing same to one or more candidate molecules; and
   (b) determinig whether said candidate molecule is bound to the complex or protein.
24. A method for screening for a molecule that modulates directly or indirectly the function, activity, composition or formation of the complex of any one of No. 1 - 8 comprising the steps of (a) exposing said complex, or a cell or organism containing FADD complex to one or more candidate molecules; and
   (b) determining the amount of activity of protein components of, and/or intracellular localization of, said complex and/or the transcription level of a gene dependent on the complex and/or the abundance and/or activity of a protein or protein complex dependent on the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependent on the complex and/or the abundance and/or activity of a protein or protein complex dependent on the function of the complex and/or product of a gene dependent on the complex in the absence of said candidate molecules indicates that the molecule modulates function, activity or composition of said complex.
25. The method of No. 24, wherein the amount of said complex is determined.
26. The method of No. 24, wherein the activity of said complex is determined.
27. The method of No. 26, wherein said determining step comprises isolating from the cell or organism said complex to produce said isolated complex and contacting said isolated complex in the presence or absence of a candidate molecule with a substrate of said complex and determining the processing of said substrate is modified in the presence of said candidate molecule.
28. The method of No. 24, wherein the amount of the individual protein components of said complex are determined.
29. The method of No. 28, determining step comprises determining whether
   (i) "TRADD " (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions, and/or
   (ii) (iv) "TNFR1" (SEQ ID No:21) or a functionally active derivative thereof, or a functionally active fragment thereof, or a homolog thereof, or a variant of "TNRF1" encoded by a nucleic acid that hybridizes to the "TNRF1" nucleic acid under low stringency conditions, and/or
   (iii) "FADD" (SEQ ID No:22) or a functionally active derivative thereof, or a functionally active fragment thereof, or a homolog thereof, or a variant of "FADD" encoded by a nucleic acid that hybridizes to the "FADD" nucleic acid under low stringency conditions, and/or
   (iv) "caspase 8 " (SEQ ID No:256) or a functionally active derivative thereof, or a functionally active fragment thereof, or a homolog thereof, or a variant of "caspase 8" encoded by a nucleic acid that hybridizes to the "caspase 8" nucleic acid under low stringency conditions, and/or
   (v) "SERINE HYDROXYMETHYLTRANSFERASE, CYTOSOLIC" (SEQ ID No:259) or a functionally active derivative thereof, or a functionally active fragment thereof, or a homolog thereof, or a variant of "SERINE HYDROXYMETHYLTRANSFERASE, CYTOSOLIC" encoded by a nucleic acid that hybridizes to the "SERINE HYDROXYMETHYLTRANSFERASE, CYTOSOLIC" nucleic acid under low stringency conditions,
   is present in the complex.
30. The method of any of No. 24 to 29, wherein said method is a method of screening for a drug for treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
31. Use of a molecule that modulates the amount of, activity of, or the protein components of the complex of any one of No. 1 - 8 for the manufacture of a medicament for the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
32. A method for the production of a pharmaceutical composition comprising carrying out the method of any of No. 1 - 8 to identify a molecule that modulates the function, activity, composition or formation of said complex, and further comprising mixing the identified molecule with a pharmaceutically acceptable carrier.
33. A method for diagnosing or screening for the presence of a disease or disorder or a predisposition for developing a disease or disorder in a subject, which disease or disorder is characterized by an aberrant amount of, activity of, or component composition of, or intracellular localization of the complex of any one of the No. 1 - 8, comprising determining the amount of, activity of, protein components of, and/or intracellular localization of, said complex and/or the transcription level of a gene dependent on the complex and/or the abundance and/or activity of a protein or protein complex dependent on the function of the complex and/or product of a gene dependent on the complex in a comparative sample derived from a subject, wherein a difference in said amount, activity, or protein components of, said complex in an analogous sample from a subject not having the disease or disorder or predisposition indicates the presence in the subject of the disease or disorder or predisposition in the subject.
34. The method of No. 33, wherein the amount of said complex is determined.
35. The method of No. 33, wherein the activity of said complex is determined.
36. The method of No. 35, wherein said determining step comprises isolating from the subject said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
37. The method of No. 33, wherein the amount of the individual protein components of said complex are determined.
38. The method of No. 37, wherein said determining step comprises determining whether
   (i) "TRADD " (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions, and/or
   (ii) (iv) "TNFR1" (SEQ ID No:21) or a functionally active derivative thereof, or a functionally active fragment thereof, or a homolog thereof, or a variant of "TNRF1" encoded by a nucleic acid that hybridizes to the "TNRF1" nucleic acid under low stringency conditions, and/or
   (iii) "FADD" (SEQ ID No:22) or a functionally active derivative thereof, or a functionally active fragment thereof, or a homolog thereof, or a variant of "FADD" encoded by a nucleic acid that hybridizes to the "FADD" nucleic acid under low stringency conditions, and/or
   (iv) "caspase 8 " (SEQ ID No:256) or a functionally active derivative thereof, or a functionally active fragment thereof, or a homolog thereof, or a variant of "caspase 8" encoded by a nucleic acid that hybridizes to the "caspase 8" nucleic acid under low stringency conditions, and/or
   (v) "SERINE HYDROXYMETHYLTRANSFERASE, CYTOSOLIC" (SEQ ID No:259) or a functionally active derivative thereof, or a functionally active fragment thereof, or a homolog thereof, or a variant of "SERINE HYDROXYMETHYLTRANSFERASE, CYTOSOLIC" encoded by a nucleic acid that hybridizes to the "SERINE HYDROXYMETHYLTRANSFERASE, CYTOSOLIC" nucleic acid under low stringency conditions,
   is present in the complex.
39. The complex of any one of No. 1 - 8 or the antibody or fragment of No. 15, for use in a method of diagnosing a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.
40. A method for treating or preventing a disease or disorder characterized by an aberrant amount of, activity or component composition of or intracellular localization of, the complex of anyone of No. 1 - 8, comprising administering to a subject in need of such treatment or prevention a therapeutically effective amount of one or more molecules that modulate the amount of, , or protein components of, said complex.
41. The method according to No. 40, wherein said disease or disorder involves decreased levels of the amount or activity of said complex.
42. The method according to No. 40, wherein said disease or disorder involves increased levels of the amount or activity of said complex.
43. Complex of any of No. 1 - 8 and/or protein selected from the following proteins
   (i) "TRADD " (SEQ ID No:17) or a functionally active derivative thereof, or a functionally active fragment thereof, or a homolog thereof, or a variant of "TRADD" encoded by a nucleic acid that hybridizes to the "TRADD" nucleic acid under low stringency conditions,
   (ii) (iv) "TNFR1" (SEQ ID No:21) or a functionally active derivative thereof, or a functionally active fragment thereof, or a homolog thereof, or a variant of "TNRF1" encoded by a nucleic acid that hybridizes to the "TNRF1" nucleic acid under low stringency conditions,
   (iii) "FADD" (SEQ ID No:22) or a functionally active derivative thereof, or a functionally active fragment thereof, or a homolog thereof, or a variant of "FADD" encoded by a nucleic acid that hybridizes to the "FADD" nucleic acid under low stringency conditions,
   (iv) "caspase 8" (SEQ ID No:256) or a functionally active derivative thereof,or a functionally active fragement thereof, or a homolog thereof, or a variant of "caspase 8" that hybridizes to the "caspase8" nucleic acid under low stringency conditions, and
   (v) "SERINE HYDROXYMETHYLTRANSFERASE, CYTOSOLIC" (SEQ ID No:259) or a functionally active derivative thereof, or a functionally active fragment thereof, or a homolog thereof, or a variant of "SERINE HYDROXYMETHYLTRANSFERASE, CYTOSOLIC" encoded by a nucleic acid that hybridizes to the "SERINE HYDROXYMETHYLTRANSFERASE, CYTOSOLIC" nucleic acid under low stringency conditions,
as a target for an active agent of a pharmaceutical, preferably a drug target in the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as prostate cancer, breast cancer and skin cancer.

The present invention further relates to the c-IAP protein complex
1. A protein complex selected from complex (I) and comprising
   (a) at least one first protein selected from the group consisting of:
      (i) "c-IAP1" (SEQ ID No:35) or a functionally active derivative thereof, or a functionally active fragment thereof, or a homolog thereof, or a variant of "c-IAP1" encoded by a nucleic acid that hybridizes to the "c-IAP1" nucleic acid under low stringency conditions,
      (ii) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions, and
      (iii) "TRAF1" (SEQ ID No:14) or a functionally active derivative thereof, or a functionally active fragment thereof, or a homolog thereof, or a variant of "TRAF1" encoded by a nucleic acid that hybridizes to the "TRAF1" nucleic acid under low stringency conditions, and
   (b) at least one second protein, which second protein is selected from the group consisting of:
      (i) "SMAC PROTEIN, MITOCHONDRIAL PRECURSOR" (SEQ ID No:265) or a functionally active derivative thereof, or a functionally active fragment thereof, or a homolog thereof, or a variant of "SMAC PROTEIN, MITOCHONDRIAL PRECURSOR" encoded by a nucleic acid that hybridizes to the "SMAC PROTEIN, MITOCHONDRIAL PRECURSOR" nucleic acid under low stringency conditions,
      (ii) "WERNER HELICASE INTERACTING PROTEIN" (SEQ ID No:266) or a functionally active derivative thereof, or a functionally active fragment thereof, or a homolog thereof, or a variant of "WERNER HELICASE INTERACTING PROTEIN" encoded by a nucleic acid that hybridizes to the "WERNER HELICASE INTERACTING PROTEIN" nucleic acid under low stringency conditions,
      (iii) "SERINE PROTEASE HTRA2, MITOCHONDRIAL PRECURSOR" (SEQ ID No:267) or a functionally active derivative thereof, or a functionally active fragment thereof, or a homolog thereof, or a variant of "SERINE PROTEASE HTRA2, MITOCHONDRIAL PRECURSOR" encoded by a nucleic acid that hybridizes to the "SERINE PROTEASE HTRA2, MITOCHONDRIAL PRECURSOR" nucleic acid under low stringency conditions,
      (iv) "CITRATE SYNTHASE, MITOCHONDRIAL PRECURSOR" (SEQ ID No:268) or a functionally active derivative thereof, or a functionally active fragment thereof, or a homolog thereof, or a variant of "CITRATE SYNTHASE, MITOCHONDRIAL PRECURSOR" encoded by a nucleic acid that hybridizes to the "CITRATE SYNTHASE, MITOCHONDRIAL PRECURSOR" nucleic acid under low stringency conditions,
      (v) "c-IAP2" (SEQ ID No:26) or a functionally active derivative thereof, or a functionally active fragment thereof, or a homolog thereof, or a variant of "c-IAP2" encoded by a nucleic acid that hybridizes to the "c-IAP2" nucleic acid under low stringency conditions,
      (vi) "MITOGEN-ACTIVATED PROTEIN KINASE KINASE KINASE 14" (SEQ ID No:108) or a functionally active derivative thereof, or a functionally active fragment thereof, or a homolog thereof, or a variant of "MITOGEN-ACTIVATED PROTEIN KINASE KINASE KINASE 14" encoded by a nucleic acid that hybridizes to the "MITOGEN-ACTIVATED PROTEIN KINASE KINASE KINASE 14" nucleic acid under low stringency conditions, and
      (vii) "TANK BINDING KINASE TBK1" (SEQ ID No:115) or a functionally active derivative thereof, or a functionally active fragment thereof, or a homolog thereof, or a variant of "TANK BINDING KINASE TBK1" encoded by a nucleic acid that hybridizes to the "TANK BINDING KINASE TBK1" nucleic acid under low stringency conditions;
   and a complex (II) comprising at least two of said second proteins,
   wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 ug/ml denatured salmon sperm DNA, and 10% (wt/vol) dextran sulfate for 18-20 hours at 40°C, washing in a buffer consisting of 2X SSC, 25 mM Tris-HCl (pH 7.4), 5 mM EDTA, and 0.1 % SDS for 1.5 hours at 55°C, and washing in a buffer consisting of 2X SSC, 25 mM Tris-HCl (pH 7.4), 5 mM EDTA, and 0.1% SDS for 1.5 hours at 60°C.
2. The protein complex according to claim 1 wherein the first protein is the protein "c-IAP1" (SEQ ID No:35), or a functionally active derivative thereof, or a functionally active fragment thereof, or a homolog thereof, or a variant of "c-IAP1" encoded by a nucleic acid that hybridizes to the "c-IAP1" nucleic acid under low stringency conditions.
3. The protein complex according to No. 1 selected from complex (I) and comprising the following proteins:
   (i) "SMAC PROTEIN, MITOCHONDRIAL PRECURSOR" (SEQ ID No:265) or a functionally active derivative thereof, or a functionally active fragment thereof, or a homolog thereof, or a variant of "SMAC PROTEIN, MITOCHONDRIAL PRECURSOR" encoded by a nucleic acid that hybridizes to the "SMAC PROTEIN, MITOCHONDRIAL PRECURSOR" nucleic acid under low stringency conditions,
   (ii) "SERINE PROTEASE HTRA2, MITOCHONDRIAL PRECURSOR" (SEQ ID No:267) or a functionally active derivative thereof, or a functionally active fragment thereof, or a homolog thereof, or a variant of "SERINE PROTEASE HTRA2, MITOCHONDRIAL PRECURSOR" encoded by a nucleic acid that hybridizes to the "SERINE PROTEASE HTRA2, MITOCHONDRIAL PRECURSOR" nucleic acid under low stringency conditions,
   (iii) "CITRATE SYNTHASE, MITOCHONDRIAL PRECURSOR" (SEQ ID No:268) or a functionally active derivative thereof, or a functionally active fragment thereof, or a homolog thereof, or a variant of "CITRATE SYNTHASE, MITOCHONDRIAL PRECURSOR" encoded by a nucleic acid that hybridizes to the "CITRATE SYNTHASE, MITOCHONDRIAL PRECURSOR" nucleic acid under low stringency conditions,
   (iv) "c-IAP2" (SEQ ID No:26) or a functionally active derivative thereof, or a functionally active fragment thereof, or a homolog thereof, or a variant of "c-IAP2" encoded by a nucleic acid that hybridizes to the "c-IAP2" nucleic acid under low stringency conditions,
   (v) "c-IAP1" (SEQ ID No:35) or a functionally active derivative thereof, or a functionally active fragment thereof, or a homolog thereof, or a variant of "c-IAP1" encoded by a nucleic acid that hybridizes to the "c-IAP1" nucleic acid under low stringency conditions,
   (vi) "TRAF2" (SEQ ID No: 15) or a functionally active derivative thereof,or a functionally active fragement thereof, or a homolog thereof, or a variant of "TRAF2" that hybridizes to the "TRAF2" nucleic acid under low stringency conditions, and
   (vii) "TRAF1" (SEQ ID No: 14) or a functionally active derivative thereof, or a functionally active fragment thereof, or a homolog thereof, or a variant of "TRAF1" encoded by a nucleic acid that hybridizes to the "TRAF1" nucleic acid under low stringency conditions; and a protein complex selected from complex (II) and comprising the following proteins:

   (i) "SMAC PROTEIN, MITOCHONDRIAL PRECURSOR" (SEQ ID No:265) or a functionally active derivative thereof, or a functionally active fragment thereof, or a homolog thereof, or a variant of "SMAC PROTEIN, MITOCHONDRIAL PRECURSOR" encoded by a nucleic acid that hybridizes to the "SMAC PROTEIN, MITOCHONDRIAL PRECURSOR" nucleic acid under low stringency conditions,
   (ii) "WERNER HELICASE INTERACTING PROTEIN" (SEQ ID No:266) or a functionally active derivative thereof, or a functionally active fragment thereof, or a homolog thereof, or a variant of "WERNER HELICASE INTERACTING PROTEIN" encoded by a nucleic acid that hybridizes to the "WERNER HELICASE INTERACTING PROTEIN" nucleic acid under low stringency conditions,
   (iii) "SERINE PROTEASE HTRA2, MITOCHONDRIAL PRECURSOR" (SEQ ID No:267) or a functionally active derivative thereof, or a functionally active fragment thereof, or a homolog thereof, or a variant of "SERINE PROTEASE HTRA2, MITOCHONDRIAL PRECURSOR" encoded by a nucleic acid that hybridizes to the "SERINE PROTEASE HTRA2, MITOCHONDRIAL PRECURSOR" nucleic acid under low stringency conditions,
   (iv) "CITRATE SYNTHASE, MITOCHONDRIAL PRECURSOR" (SEQ ID No:268) or a functionally active derivative thereof, or a functionally active fragment thereof, or a homolog thereof, or a variant of "CITRATE SYNTHASE, MITOCHONDRIAL PRECURSOR" encoded by a nucleic acid that hybridizes to the "CITRATE SYNTHASE, MITOCHONDRIAL PRECURSOR" nucleic acid under low stringency conditions,
   (v) "c-IAP2" (SEQ ID No:26) or a functionally active derivative thereof, or a functionally active fragment thereof, or a homolog thereof, or a variant of "c-IAP2" encoded by a nucleic acid that hybridizes to the "c-IAP2" nucleic acid under low stringency conditions,
   (vi) "MITOGEN-ACTIVATED PROTEIN KINASE KINASE KINASE 14" (SEQ ID No:108) or a functionally active derivative thereof, or a functionally active fragment thereof, or a homolog thereof, or a variant of "MITOGEN-ACTIVATED PROTEIN KINASE KINASE KINASE 14" encoded by a nucleic acid that hybridizes to the "MITOGEN-ACTIVATED PROTEIN KINASE KINASE KINASE 14" nucleic acid under low stringency conditions,
   (vii) "c-tAP1" (SEQ ID No:35) or a functionally active derivative thereof, or a functionally active fragment thereof, or a homolog thereof, or a variant of "c-IAP1" encoded by a nucleic acid that hybridizes to the "c-IAP1" nucleic acid under low stringency conditions,
   (viii) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions,
   (ix) "TRAF1" (SEQ ID No:14) or a functionally active derivative thereof,or a functionally active fragement thereof, or a homolog thereof, or a variant of "TRAF1" that hybridizes to the "TRAF1" nucleic acid under low stringency conditions, and
   (x) "TANK BINDING KINASE TBK1" (SEQ ID No:115) or a functionally active derivative thereof, or a functionally active fragment thereof, or a homolog thereof, or a variant of "TANK BINDING KINASE TBK1" encoded by a nucleic acid that hybridizes to the "TANK BINDING KINASE TBK1" nucleic acid under low stringency conditions.
4. The protein complex according to No. 1 comprising all but 1 - 6 of the following proteins:
   (i) "SMAC PROTEIN, MITOCHONDRIAL PRECURSOR" (SEQ ID No:265) or a functionally active derivative thereof, or a functionally active fragment thereof, or a homolog thereof, or a variant of "SMAC PROTEIN, MITOCHONDRIAL PRECURSOR" encoded by a nucleic acid that hybridizes to the "SMAC PROTEIN, MITOCHONDRIAL PRECURSOR" nucleic acid under low stringency conditions,
   (ii) "WERNER HELICASE INTERACTING PROTEIN" (SEQ ID No:266) or a functionally active derivative thereof, or a functionally active fragment thereof, or a homolog thereof, or a variant of "WERNER HELICASE INTERACTING PROTEIN" encoded by a nucleic acid that hybridizes to the "WERNER HELICASE INTERACTING PROTEIN" nucleic acid under low stringency conditions,
   (iii) "SERINE PROTEASE HTRA2, MITOCHONDRIAL PRECURSOR" (SEQ ID No:267) or a functionally active derivative thereof, or a functionally active fragment thereof, or a homolog thereof, or a variant of "SERINE PROTEASE HTRA2, MITOCHONDRIAL PRECURSOR" encoded by a nucleic acid that hybridizes to the "SERINE PROTEASE HTRA2, MITOCHONDRIAL PRECURSOR" nucleic acid under low stringency conditions,
   (iv) "CITRATE SYNTHASE, MITOCHONDRIAL PRECURSOR" (SEQ ID No:268) or a functionally active derivative thereof, or a functionally active fragment thereof, or a homolog thereof, or a variant of "CITRATE SYNTHASE, MITOCHONDRIAL PRECURSOR" encoded by a nucleic acid that hybridizes to the "CITRATE SYNTHASE, MITOCHONDRIAL PRECURSOR" nucleic acid under low stringency conditions,
   (v) "c-IAP2" (SEQ ID No:26) or a functionally active derivative thereof, or a functionally active fragment thereof, or a homolog thereof, or a variant of "c-IAP2" encoded by a nucleic acid that hybridizes to the "c-IAP2" nucleic acid under low stringency conditions,
   (vi) "MITOGEN-ACTIVATED PROTEIN KINASE KINASE KINASE 14" (SEQ ID No:108) or a functionally active derivative thereof, or a functionally active fragment thereof, or a homolog thereof, or a variant of "MITOGEN-ACTIVATED PROTEIN KINASE KINASE KINASE 14" encoded by a nucleic acid that hybridizes to the "MITOGEN-ACTIVATED PROTEIN KINASE KINASE KINASE 14" nucleic acid under low stringency conditions,
   (vii) "c-IAP1" (SEQ ID No:35) or a functionally active derivative thereof, or a functionally active fragment thereof, or a homolog thereof, or a variant of "c-IAP1" encoded by a nucleic acid that hybridizes to the "c-IAP1" nucleic acid under low stringency conditions,
   (viii) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions,
   (ix) "TRAF1" (SEQ ID No:14) or a functionally active derivative thereof,or a functionally active fragement thereof, or a homolog thereof, or a variant of "TRAF1" that hybridizes to the "TRAF1" nucleic acid under low stringency conditions, and
   (x) "TANK BINDING KINASE TBK1" (SEQ ID No:115) or a functionally active derivative thereof, or a functionally active fragment thereof, or a homolog thereof, or a variant of "TANK BINDING KINASE TBK1" encoded by a nucleic acid that hybridizes to the "TANK BINDING KINASE TBK1" nucleic acid under low stringency conditions.
5. The complex of any of No. 1 - 4 comprising a functionally active derivative of said first protein and/or a functionally active derivative of said second protein, wherein the functionally active derivative is a fusion protein comprising said first protein or said second protein fused to an amino acid sequence different from the first protein or second protein, respectively.
6. The complex of No. 5 wherein the functionally active derivative is a fusion protein comprising said first protein or said second protein fused to an affinity tag or label.
7. The complex of any of No. 1 - 4 comprising a fragment of said first protein and/or a fragment of said second protein, which fragment binds to another protein component of said complex.
8. The complex of any of No. 1 -7 that is involved in the in vitro ubiquitination activity or induction of cell death activity.
9. A process for preparing complex of any of No. 1 - 8 and optionally the components thereof comprising the following steps:
   the expressing a protein (bait) of the complex, preferably a tagged protein, in a target cell, isolating the protein complex which is attached to the bait protein, and optionally disassociating the protein complex and isolating the individual complex members.
10. The process according to No. 9 wherein the tagged protein comprises two different tags which allow two separate affinity purification steps.
11. The process according to any of No. 9 - 10 wherein the two tags are separated by a cleavage site for a protease.
12. Component of the c-IAP1 protein complex obtainable by a process according to any of No. 9 to 11.
13. Construct, preferably a vector construct, comprising at least two separate nucleic acid sequences each encoding a different protein, or a functionally active fragment or a functionally active derivative therof at least one of said proteins, or functionally active fragments or functionally active derivative therof being selected from the first group of proteins according to No. 1(a) and at least one of said proteines, or functionally active fragments of functionally active derivative thereof being selected from the second group of proteins according to No. 1(b)
14. Host cell containting a construct of No. 13 or containing several vectors each comprising at least the nucleic acid sequence encoding at least one of the proteins, or functionally active fragments or functionally active derivatives thereof selected from the first group of proteins according to No. 1(a) and the proteins, or functionally active fragments or functionally active derivatives thereof selected from the second group of proteins according to No. 1(b).
15. An antibody or a fragment of said antibody containing the binding domain thereof, selected from an antibody or fragment thereof, which binds the complex of any of No. 1-8 and which does not bind any of the proteins of said complex when uncomplexed and an antibody or a fragment of said antibody which binds to any of the proteins according to No..
16. A kit comprising in one or more container the complex of any of No. 1 - 8 optionally together with an antibody according to No. 15 and/or further components such as reagents and working instructions.
17. The kit according to No.16 for processing a substrate of said complex.
18. A kit according to No. 16 for the diagnosis or prognosis of a disease or a disease risk, preferentially for a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as solid cancers such as prostate cancer, breast cancer and skin cancer; cancer such as haematological cancer such as leukemia.
19. Array, in which at least a complex according to any of No. 1 - 8 and/or at least one antibody according to No. 15 is attached to a solid carrier.
20. A process for processing a physiological substrate of the complex comprising the step of bringing into contact a complex to any of No. 1 - 8 with said substrate, such that said substrate is processed.
21. A pharmaceutical composition comprising the protein complex of any of No. 1 to 8..
22. The pharmaceutical composition according to No. 21 for the treatment of diseases and disorders such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as solid cancers such as prostate cancer, breast cancer and skin cancer; cancer such as haematological cancer such as leukemia.
23. A method for screening for a molecule that binds to the complex of anyone of No. 1 - 8. comprising the steps of
   (a) exposing said complex, or a cell or organism containing same to one or more candidate molecules; and
   (b) determinig whether said candidate molecule is bound to the complex or protein.
24. A method for screening for a molecule that modulates directly or indirectly the function, activity, composition or formation of the complex of any one of No. 1 - 8 comprising the steps of (a) exposing said complex, or a cell or organism containing c-IAP1 protein complex to one or more candidate molecules; and
   (b) determining the amount of activity of protein components of, and/or intracellular localization of, said complex and/or the transcription level of a gene dependent on the complex and/or the abundance and/or activity of a protein or protein complex dependent on the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependent on the complex and/or the abundance and/or activity of a protein or protein complex dependent on the function of the complex and/or product of a gene dependent on the complex in the absence of said candidate molecules indicates that the molecule modulates function, activity or composition of said complex.
25. The method of No. 24, wherein the amount of said complex is determined.
26. The method of No. 24, wherein the activity of said complex is determined.
27. The method of No. 26, wherein said determining step comprises isolating from the cell or organism said complex to produce said isolated complex and contacting said isolated complex in the presence or absence of a candidate molecule with a substrate of said complex and determining the processing of said substrate is modified in the presence of said candidate molecule.
28. The method of No. 24, wherein the amount of the individual protein components of said complex are determined.
29. The method of No. 28, determining step comprises determining whether
   (i) "SMAC PROTEIN, MITOCHONDRIAL PRECURSOR" (SEQ ID No:265) or a functionally active derivative thereof, or a functionally active fragment thereof, or a homolog thereof, or a variant of "SMAC PROTEIN, MITOCHONDRIAL PRECURSOR" encoded by a nucleic acid that hybridizes to the "SMAC PROTEIN, MITOCHONDRIAL PRECURSOR" nucleic acid under low stringency conditions, and/or
   (ii) "WERNER HELICASE INTERACTING PROTEIN" (SEQ ID No:266) or a functionally active derivative thereof, or a functionally active fragment thereof, or a homolog thereof, or a variant of "WERNER HELICASE INTERACTING PROTEIN" encoded by a nucleic acid that hybridizes to the "WERNER HELICASE INTERACTING PROTEIN" nucleic acid under low stringency conditions, and/or
   (iii) "SERINE PROTEASE HTRA2, MITOCHONDRIAL PRECURSOR" (SEQ ID No:267) or a functionally active derivative thereof, or a functionally active fragment thereof, or a homolog thereof, or a variant of "SERINE PROTEASE HTRA2, MITOCHONDRIAL PRECURSOR" encoded by a nucleic acid that hybridizes to the "SERINE PROTEASE HTRA2, MITOCHONDRIAL PRECURSOR" nucleic acid under low stringency conditions, and/or
   (iv) "CITRATE SYNTHASE, MITOCHONDRIAL PRECURSOR" (SEQ ID No:268) or a functionally active derivative thereof, or a functionally active fragment thereof, or a homolog thereof, or a variant of "CITRATE SYNTHASE, MITOCHONDRIAL PRECURSOR" encoded by a nucleic acid that hybridizes to the "CITRATE SYNTHASE, MITOCHONDRIAL PRECURSOR" nucleic acid under low stringency conditions, and/or (v) "c-IAP2" (SEQ ID No:26) or a functionally active derivative thereof, or a functionally active fragment thereof, or a homolog thereof, or a variant of "c-IAP2" encoded by a nucleic acid that hybridizes to the "c-IAP2" nucleic acid under low stringency conditions, and/or
   (vi) "MITOGEN-ACTIVATED PROTEIN KINASE KINASE KINASE 14" (SEQ ID No:108) or a functionally active derivative thereof, or a functionally active fragment thereof, or a homolog thereof, or a variant of "MITOGEN-ACTIVATED PROTEIN KINASE KINASE KINASE 14" encoded by a nucleic acid that hybridizes to the "MITOGEN-ACTIVATED PROTEIN KINASE KINASE KINASE 14" nucleic acid under low stringency conditions, and/or
   (vii) "c-IAP1" (SEQ ID No:35) or a functionally active derivative thereof, or a functionally active fragment thereof, or a homolog thereof, or a variant of "c-IAP1" encoded by a nucleic acid that hybridizes to the "c-IAP1" nucleic acid under low stringency conditions, and/or
   (viii) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions, and/or
   (ix) "TRAF1" (SEQ ID No:14) or a functionally active derivative thereof, or a functionally active fragment thereof, or a homolog thereof, or a variant of "TRAF1" encoded by a nucleic acid that hybridizes to the "TRAF1" nucleic acid under low stringency conditions, and/or
   (x) "TANK BINDING KINASE TBK1" (SEQ ID No:115) or a functionally active derivative thereof, or a functionally active fragment thereof, or a homolog thereof, or a variant of "TANK BINDING KINASE TBK1" encoded by a nucleic acid that hybridizes to the "TANK BINDING KINASE TBK1" nucleic acid under low stringency conditions,
   is present in the complex.
30. The method of any of No. 24 to 29, wherein said method is a method of screening for a drug for treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as solid cancers such as prostate cancer, breast cancer and skin cancer; cancer such as haematological cancer such as leukemia.
31. Use of a molecule that modulates the amount of, activity of, or the protein components of the complex of any one of No. 1 - 8 for the manufacture of a medicament for the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as solid cancers such as prostate cancer, breast cancer and skin cancer; cancer such as haematological cancer such as leukemia.
32. A method for the production of a pharmaceutical composition comprising carrying out the method of any of No. 1 - 8 to identify a molecule that modulates the function, activity, composition or formation of said complex, and further comprising mixing the identified molecule with a pharmaceutically acceptable carrier.
33. A method for diagnosing or screening for the presence of a disease or disorder or a predisposition for developing a disease or disorder in a subject, which disease or disorder is characterized by an aberrant amount of, activity of, or component composition of, or intracellular localization of the complex of any one of the No. 1-8, comprising determining the amount of, activity of, protein components of, and/or intracellular localization of, said complex and/or the transcription level of a gene dependent on the complex and/or the abundance and/or activity of a protein or protein complex dependent on the function of the complex and/or product of a gene dependent on the complex in a comparative sample derived from a subject, wherein a difference in said amount, activity, or protein components of, said complex in an analogous sample from a subject not having the disease or disorder or predisposition indicates the presence in the subject of the disease or disorder or predisposition in the subject.
34. The method of No. 33, wherein the amount of said complex is determined.
35. The method of No. 33, wherein the activity of said complex is determined.
36. The method of No. 35, wherein said determining step comprises isolating from the subject said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.
37. The method of No. 33, wherein the amount of the individual protein components of said complex are determined.
38. The method of No. 37, wherein said determining step comprises determining whether
   (i) "SMAC PROTEIN, MITOCHONDRIAL PRECURSOR" (SEQ ID No:265) or a functionally active derivative thereof, or a functionally active fragment thereof, or a homolog thereof, or a variant of "SMAC PROTEIN, MITOCHONDRIAL PRECURSOR" encoded by a nucleic acid that hybridizes to the "SMAC PROTEIN, MITOCHONDRIAL PRECURSOR" nucleic acid under low stringency conditions, and/or
   (ii) "WERNER HELICASE INTERACTING PROTEIN" (SEQ ID No:266) or a functionally active derivative thereof, or a functionally active fragment thereof, or a homolog thereof, or a variant of "WERNER HELICASE INTERACTING PROTEIN" encoded by a nucleic acid that hybridizes to the "WERNER HELICASE INTERACTING PROTEIN" nucleic acid under low stringency conditions, and/or
   (iii) "SERINE PROTEASE HTRA2, MITOCHONDRIAL PRECURSOR" (SEQ ID No:267) or a functionally active derivative thereof, or a functionally active fragment thereof, or a homolog thereof, or a variant of "SERINE PROTEASE HTRA2, MITOCHONDRIAL PRECURSOR" encoded by a nucleic acid that hybridizes to the "SERINE PROTEASE HTRA2, MITOCHONDRIAL PRECURSOR" nucleic acid under low stringency conditions, and/or
   (iv) "CITRATE SYNTHASE, MITOCHONDRIAL PRECURSOR" (SEQ ID No:268) or a functionally active derivative thereof, or a functionally active fragment thereof, or a homolog thereof, or a variant of "CITRATE SYNTHASE, MITOCHONDRIAL PRECURSOR" encoded by a nucleic acid that hybridizes to the "CITRATE SYNTHASE, MITOCHONDRIAL PRECURSOR" nucleic acid under low stringency conditions, and/or
   (v) "c-IAP2" (SEQ ID No:26) or a functionally active derivative thereof, or a functionally active fragment thereof, or a homolog thereof, or a variant of "c-IAP2" encoded by a nucleic acid that hybridizes to the "c-IAP2" nucleic acid under low stringency conditions, and/or
   (vi) "MITOGEN-ACTIVATED PROTEIN KINASE KINASE KINASE 14" (SEQ ID No:108) or a functionally active derivative thereof, or a functionally active fragment thereof, or a homolog thereof, or a variant of "MITOGEN-ACTIVATED PROTEIN KINASE KINASE KINASE 14" encoded by a nucleic acid that hybridizes to the "MITOGEN-ACTIVATED PROTEIN KINASE KINASE KINASE 14" nucleic acid under low stringency conditions, and/or
   (vii) "c-IAP1" (SEQ ID No:35) or a functionally active derivative thereof, or a functionally active fragment thereof, or a homolog thereof, or a variant of "c-IAP1" encoded by a nucleic acid that hybridizes to the "c-IAP1" nucleic acid under low stringency conditions, and/or
   (viii) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions, and/or
   (ix) "TRAF1" (SEQ ID No:14) or a functionally active derivative thereof, or a functionally active fragment thereof, or a homolog thereof, or a variant of "TRAF1" encoded by a nucleic acid that hybridizes to the "TRAF1" nucleic acid under low stringency conditions, and/or
   (x) "TANK BINDING KINASE TBK1" (SEQ ID No:115) or a functionally active derivative thereof, or a functionally active fragment thereof, or a homolog thereof, or a variant of "TANK BINDING KINASE TBK1" encoded by a nucleic acid that hybridizes to the "TANK BINDING KINASE TBK1" nucleic acid under low stringency conditions,
   is present in the complex.
39. The complex of any one of No. 1 - 8 or the antibody or fragment of No. 15, for use in a method of diagnosing a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as solid cancers such as prostate cancer, breast cancer and skin cancer; cancer such as haematological cancer such as leukemia.
40. A method for treating or preventing a disease or disorder characterized by an aberrant amount of, activity or component composition of or intracellular localization of, the complex of anyone of No. 1 - 8, comprising administering to a subject in need of such treatment or prevention a therapeutically effective amount of one or more molecules that modulate the amount of, in vitro ubiquitination activity or induction of cell death activity, or protein components of, said complex.
41. The method according to No. 40, wherein said disease or disorder involves decreased levels of the amount or activity of said complex.
42. The method according to No. 40 , wherein said disease or disorder involves increased levels of the amount or activity of said complex.
43. Complex of any of No. 1-8 and/or protein selected from the following proteins
   (i) "SMAC PROTEIN, MITOCHONDRIAL PRECURSOR" (SEQ ID No:265) or a functionally active derivative thereof, or a functionally active fragment thereof, or a homolog thereof, or a variant of "SMAC PROTEIN, MITOCHONDRIAL PRECURSOR" encoded by a nucleic acid that hybridizes to the "SMAC PROTEIN, MITOCHONDRIAL PRECURSOR" nucleic acid under low stringency conditions,
   (ii) "WERNER HELICASE INTERACTING PROTEIN" (SEQ ID No:266) or a functionally active derivative thereof, or a functionally active fragment thereof, or a homolog thereof, or a variant of "WERNER HELICASE INTERACTING PROTEIN" encoded by a nucleic acid that hybridizes to the "WERNER HELICASE INTERACTING PROTEIN" nucleic acid under low stringency conditions,
   (iii) "SERINE PROTEASE HTRA2, MITOCHONDRIAL PRECURSOR" (SEQ ID No:267) or a functionally active derivative thereof, or a functionally active fragment thereof, or a homolog thereof, or a variant of "SERINE PROTEASE HTRA2, MITOCHONDRIAL PRECURSOR" encoded by a nucleic acid that hybridizes to the "SERINE PROTEASE HTRA2, MITOCHONDRIAL PRECURSOR" nucleic acid under low stringency conditions,
   (iv) "CITRATE SYNTHASE, MITOCHONDRIAL PRECURSOR" (SEQ ID No:268) or a functionally active derivative thereof, or a functionally active fragment thereof, or a homolog thereof, or a variant of "CITRATE SYNTHASE, MITOCHONDRIAL PRECURSOR" encoded by a nucleic acid that hybridizes to the "CITRATE SYNTHASE, MITOCHONDRIAL PRECURSOR" nucleic acid under low stringency conditions,
   (v) "c-IAP2" (SEQ ID No:26) or a functionally active derivative thereof, or a functionally active fragment thereof, or a homolog thereof, or a variant of "c-IAP2" encoded by a nucleic acid that hybridizes to the "c-IAP2" nucleic acid under low stringency conditions,
   (vi) "MITOGEN-ACTIVATED PROTEIN KINASE KINASE KINASE 14" (SEQ ID No:108) or a functionally active derivative thereof, or a functionally active fragment thereof, or a homolog thereof, or a variant of "MITOGEN-ACTIVATED PROTEIN KINASE KINASE KINASE 14" encoded by a nucleic acid that hybridizes to the "MITOGEN-ACTIVATED PROTEIN KINASE KINASE KINASE 14" nucleic acid under low stringency conditions,
   (vii) "c-IAP1" (SEQ ID No:35) or a functionally active derivative thereof, or a functionally active fragment thereof, or a homolog thereof, or a variant of "c-IAP1" encoded by a nucleic acid that hybridizes to the "c-IAP1" nucleic acid under low stringency conditions,
   (viii) "TRAF2" (SEQ ID No:15) or a functionally active derivative thereof, or a functionally active fragment thereof, or a homolog thereof, or a variant of "TRAF2" encoded by a nucleic acid that hybridizes to the "TRAF2" nucleic acid under low stringency conditions,
   (ix) "TRAF1" (SEQ ID No:14) or a functionally active derivative thereof,or a functionally active fragement thereof, or a homolog thereof, or a variant of "TRAF1" that hybridizes to the "TRAF1" nucleic acid under low stringency conditions, and
   (x) "TANK BINDING KINASE TBK1" (SEQ ID No:115) or a functionally active derivative thereof, or a functionally active fragment thereof, or a homolog thereof, or a variant of "TANK BINDING KINASE TBK1" encoded by a nucleic acid that hybridizes to the "TANK BINDING KINASE TBK1" nucleic acid under low stringency conditions,
   as a target for an active agent of a pharmaceutical, preferably a drug target in the treatment or prevention of a disease or disorder such as inflammation such as chronic inflammatory diseases, rheumatoid arthritis, inflammatory bowel disease;infectious diseases such as septic shock and bacterial infection; neurological diseases such as stroke-induced inflammation in neurons;neurodegenerative diseases;cancer such as solid cancers such as prostate cancer, breast cancer and skin cancer; cancer such as haematological cancer such as leukemia.

### 5. PROTOCOLS:

The TAP-technology, which is more fully describted in WO/0009716 and in Rigaut, G et.al. (1999), Nature Biotechnology. Vol 17 (10): 1030-1032 respectively was used and further adapted as described below for protein purification. Proteins were identified using Mass-spectrometry as described further below.
For the purification of TNFR1, in addition, an immunoprecipitation-strategy was applied. The endogenous complex was purified as described in section 5.5.4. The proteins of the complex were further identified as described in section 5.6.

### 5.1 Construction of TAP-tagged bait

The cDNAs encoding the complete ORF were obtained by RT-PCR. Total RNA was prepared from appropriate cell lines using the RNeasy Mini Kit (Qiagen). Both cDNA synthesis and PCR were performed with the SUPERSCRIPT One-Step RT-PCR for Long templates Kit (Life Technologies) using gene-specific primers.
After 35-40 cycles of amplification PCR-products with the expected size were gel-purified with the MinElute PCR Purification Kit (Qiagen) and, if necessary, used for further amplification. Low-abundant RNAs were amplified by nested PCR before gel-purification. Restriction sites for Notl were attached to PCR primers to allow subcloning of amplified cDNAs into the retroviral vectors pIE94-N/C-TAP thereby generating N- or C-terminal fusions with the TAP-tag (Rigaut et al., 1999).
N-terminal tagging was chosen for the following baits/entry points: ABIN-2, TNFR1, ReIA (p65), p50 (NF-κB1), IKKα, IKKγ, p38, p105 (NF-κB1), IκBα, IκBβ, IκBε, I-TRAF, MEKK3, TAK1, RIP, RIP2, RIP3, TRADD, FADD,
C-terminal tagging was chosen for the following baits/entry points: TNFR2, MEKK3, MEKK1, c-IAP-1.
Clones were analyzed by restriction digest, DNA sequencing and by in vitro translation using the TNT T7 Quick Coupled Transcription/Translation System (Promega inc.). The presence of the proteins was proven by Western blotting using the protein A part of the TAP-tag for detection. Briefly, separation of proteins by standard SDS-PAGE was followed by semi-dry transfer onto a nitrocellulose membrane (PROTRAN, Schleicher&Schuell) using the Multiphoril blotting apparatus from Pharmacia Biotech. The transfer buffer consisted of 48 mM Tris, 39 mM glycine, 10% methanol and 0,0375% sodium dodecylsulfate. After blocking in phosphate-buffered saline (PBS) supplemented with 10% dry milk powder and 0, 1 % Tween 20 transferred proteins were probed with the Peroxidase-Anti-Peroxidase Soluble Complex (Sigma) diluted in blocking solution. After intensive washing immunoreactive proteins were visualized by enhanced chemiluminescence (ECL; Amersham Pharmacia Biotech).

### 5.2 Preparation of Virus and infection

As a vector, a MoMLV-based recombinant virus was used.

The preparation has been carried out as follows:

### 5.2.1 Preparation of Virus

293 gp cells were grown to 100% confluency. They were split 1:5 on poly-L-Lysine plates (1:5 diluted Poly-L-Lysine [0.01% stock solution, Sigma P-4832] in PBS, left on plates for at least 10 min.)

On Day 2, 63 microgram of retroviral Vector DNA together with 13 microgram of DNA of plasmid encoding an appropriate envelope protein were transfected into 293 gp cells (Somia, NV et al (1999) Proc Natl Acad Scie USA 96: 12667-12672; Somia, NV et al (2000) J. Virol 74: 4420-4424)

On Day 3, the medium was replaced with 15 ml DMEM + 10% FBS per 15-cm dish.

On Day 4, the medium containing viruses (supernatant) was harvested (at 24 h following medium change after transfection). When a second collection was planned, DMEM 10 % FBS was added to the plates and the plates were incubated for another 24 h.
All collections were done as follows:
The supernatant was filtered through 0.45 micrometer filter (Corning GmbH, cellulose acetate, 431155).

Filter was placed into konical polyallomer centrifuge tubes (Beckman, 358126) that are placed in buckets of a SW 28 rotor (Beckman).
The filtered supernatant was ultracentrifuged at 19400 rpm in the SW 28 rotor, for 2 hours at 21 degree Celsius. The supernatant was discarded. The pellet containing viruses was resuspended in a small volume (for example 300 microliter) of Hank's Balanced Salt Solution [Gibco BRL, 14025-092], by pipetting up and down 100-times, using an aerosol-safe tip.
The viruses were used for transfection as described below.

### 5.2.2 Infection

Cells that were infected were plated one day before into one well of a 6-well plate. 4 hours before infection, the old medium on the cells was replaced with fresh medium. Only a minimal volume was added, so that the cells are completely covered (e.g. 700 microliter). During infection, the cells were actively dividing.

### A description of the cells and their growth conditions is given in 5.2.3

To the concentrated virus, polybrene (Hexadimethrine Bromide; Sigma, H 9268) was added to achieve a final concentration of 8 microgram/ml (this is equivalent to 2.4 microliter of the 1 milligram/ml polybrene stock per 300 microliter of concentrated retrovirus). The virus was incubated in polybrene at room temperature for 1 hour.

For infection, the virus/polybrene mixture was added to the cells and incubated at 37 degree Celsius at the appropriate CO₂ concentration for several hours (e.g. over-day or over-night).

Following infection, the medium on the infected cells was replaced with fresh medium. The cells were passaged as usual after they became confluent. The cells contain the retrovirus integrated into their chromosomes and stably express the gene of interest.

### 5.2.3. Cell lines

For expression,
Hek-293 cells (American Type Culture Collection-No. CRL-1573) were used. Cells were grown in 90% + 10% Fetal calf serum.
Jurkat-Cells, clone E6.1 (American Type Culture Collection-No. TIB-152) were used expressing the TAP-tagged TNFR1. Cells were grown in 0% RPMI 1640 + 10% FBS. Spinner cultures were supplemented with 10 mM HEPES.

The expression pattern of the TAP-tagged protein was checked by immunoblot-analysis as described in 5.3.3 and/or by immunofluorescence as described in 5.3.1 or 5.3.2.

### 5.3 Checking of expression pattern of TAP-tagged proteins

The expression pattern of the TAP-tagged protein was checked by immunoblot-analysis and/or by immunofluorescence.

Immunofluorescence analysis was either carried out according to section 5.3.1 or to section 5.3.2 depending on the type of the TAP-tagged protein.

### 5.3.1. Protocol for the indirect Immunofluorescence staining of fixed mammalian cells for plasma membrane and ER bound proteins:

Cells were grown in FCS media on Polylysine coated 8 well chamber slides to 50% confluency. Then fixation of the cells was performed in 4% Para FormAldehyde diluted in Phosphate Buffer Saline (PBS) solution (0.14M Phosphate, 0.1 M NaCl pH 7.4). The cells were incubated for 30 minutes at room temperature in 300 microliters per well. Quenching was performed in 0.1M Glycine in PBS for 2x 20 minutes at room temperature. Blocking was performed with 1% Bovine Serum Albumin (BSA) in 0.3% Saponin + PBS for at least 1 hour at room temperature. Incubation of the primary antibodies was performed in the blocking solution overnight at +4 C. The proper dilution of the antibodies was determined in a case to case basis. Cells were washed in PBS containing 0.3% Saponin for 2x 20 minutes at room temperature. Incubation of the secondary antibodies is performed in the blocking solution. Alexa 594 coupled Goat anti-rabbit is diluted 1:1000 (Molecular Probes). Alexa 488 coupled Goat anti-mouse is diluted 1:1000 (Molecular Probes). DAPI was used to label DNA. If Phalloidin was used to label F-actin, the drug is diluted 1:500 and incubated with the secondary antibodies. Cells were then washed again 2x20 minutes at room temperature in PBS. The excess of buffer was removed and cells were mounted in a media containing an anti-bleaching agent (Vectashield, Vector Laboratories).

### 5.3.2. Protocol for the indirect Immunofluorescence staining of fixed mammalian cells for non-plasma membrane bound proteins:

Cells were grown in FCS media on Polylysine coated 8 well chamber slides to 50% confluency. Fixation of the cells was performed in 4% ParaFormAldehyde diluted in Phosphate Buffer Saline (PBS) solution (0.14M Phosphate, 0.1M NaCl pH 7.4) for 30 minutes at Room Temperature (RT), 300 microliters per well. Quenching was performed in 0.1M Glycine in PBS for 2x 20 minutes at ROOM TEMPERATURE. Permeabilization of cells was done with 0.5% Triton X-100 in PBS for 10 minutes at room temperature. Blocking was then done in 1% Bovine Serum Albumin (BSA) in 0.3% Saponin + PBS for at least 1 hour at RT (Blocking solution). Incubation of the primary antibodies was performed in the blocking solution, overnight at +4 C. The proper dilution of the antibodies has to be determined in a case to case basis. Cells were washed in PBS containing 0.3% Saponin, for 2x 20 minutes at RT. Incubation of the secondary antibodies was performed in the blocking solution. Alexa 594 coupled Goat anti-rabbit is diluted 1:1000 (Molecular Probes), Alexa 488 coupled Goat anti-mouse is diluted 1:1000 (Molecular Probes). DAPI was used to label DNA. If Phalloidin is used to label F-actin, the drug is diluted 1:500 and incubated with the secondary antibodies. Cells were washed 2x 20 minutes at RT in PBS. The excess of buffer was removed and cells were mounted in a media containing an anti-bleaching agent (Vectashield, Vector Laboratories).

### 5.3.3 Immunoblot analysis

To analyze expression levels of TAP-tagged proteins, a cell pellet (from a 6-well dish) was lyzed in 60 µL DNAsel buffer (5% Glycerol, 100 mM NaCl, 0.8 % NP-40 (IGEPAL), 5 mM magnesium sulfate, 100 µg/ml DNAse I (Roche Diagnostics), 50 mM Tris, pH 7.5, protease inhibitor cocktail) for 15 min on ice.

Each sample was split into two aliquots. The first half was centrifuged at 13,000 rpm for 5 min. to yield the NP-40-extractable material in the supernatant; the second half (total material) was carefully triturated. 50 µg each of the NP-40-extractable material and the total material are mixed with DTT-containing sample buffer for 30 min at 50 oC on a shaker and separated by SDS polyacrylamide gel electrophoresis on a precast 4-12% Bis-Tris gel (Invitrogen).
Proteins were then transferred to nitrocellulose using a semi-dry procedure with a discontinuous buffer system. Briefly, gel and nitrocellulose membrane were stacked between filter papers soaked in either anode buffer (three layers buffer A1 (0.3 M Tris-HCl) and three layers buffer A2 (0.03 M Tris-HCl)) or cathode buffer (three layers of 0.03 M Tris-HCl, pH 9.4, 0.1 % SDS, 40 mM ε-aminocapronic acid). Electrotransfer of two gels at once was performed at 600 mA for 25 min.
Transferred proteins were visualized with Ponceau S solution for one min to control transfer efficiency and then destained in water. The membrane was blocked in 5% nonfat milk powder in TBST (TBS containing 0.05% Tween-20) for 30 min at room temperature. It was subsequently incubated with HRP-coupled PAP antibody (1:5000 diluted in 5% milk/TBST) for 1 h at room temperature, washed three times for 10 min in TBST. The blot membrane was finally soaked in chemiluminescent substrate (ECL, Roche Diagnostics) for 2 min. and either exposed to X-ray film or analyzed on an imaging station.

### 5.4. Treatment of Cells

10 microg of recombinant human TNF α (SIGMA T6674) was reconstituted in 1 ml sterile filtered PBS containing 0.5% BSA to produce a working solution of 10 microg/ml. Adherent cells (HEK293) have been stimulated for either 10 (cells from which the Abin-2-complex, c-IAP-complex, FADD-complex, lκBα-complex, IκBβ-complex, IκBε-complex, IKKα-complex, IKKγ-complex, MEKK1-complex, MEKK3-complex, P38α-complex, P105-complex, RIP-complex, RIP2-complex, RIP3-complex, TAK1-complex, TNFR1-complex, TNFR2-complex, TRAF6-complex, I-TRAF-complex, TRADD-complex have been purified) or 30 min (cells from which the p50-complex has been purified) with 10ng/ml (final concentration, in DMEM (10%FCS)).

Cells growing in suspension (Jurkat) have been stimulated at a cell density of 0,5-1x10exp7/ml with 10 ng/ml (final concentration, in RPMI1640 (10%FCS)). Jurkat-cells have been used for the purfication of the TNFR1-complex.

In addition, cells expressing the TAP-tagged p50-protein, have also been subjected to the following treatment:
10 mikrog of recombinant human TNF α (SIGMA T6674) was reconstituted in 1 ml sterile filtered PBS containing 0.5% BSA to produce a working solution of 10 ug/ml. Leptomycin B was purchased from Sigma (L2913), as a 5 microgram/ml working solution in 70% methanol. Adherent cells (HEK293) were brought in suspension using cell dissociation buffer (Invitrogen, # 13151-014), before they were resuspended in DMEM 10% FCS at a density of 2 x 10exp7 cells/ml. 40 ml cell suspension were stimulated with 20ng/ml TNF α and 5 nM leptomycin B for 60 min at 37°C, while gently rotating in a closed 50 ml tube, prior to being harvested by centrifugation.

### 5.5. Purification or protein complexes

Depending on the type of the TAP-tagged proteins, purification was carried out using the appropriate protocol of the protocols below.

### 5.5.1 Purification of cytoplasmic proteins

For the purification of cytoplasmic TAP-tagged proteins 5 x 10⁸ adherent cells (corresponding to 40 15 cm plates) were used. The cells were harvested and washed 3 times in cold PBS (3 min, 1300 rpm, Heraeus centrifuge). The cells were frozen in liquid Nitrogen and stored at -80°C, or the TAP purification was directly continued.
The cells were lysed in 10 ml CZ lysis buffer (50 mM Tris, pH 7.5, 5 % Glycerol, 0,2 % IGEPAL, 1.5 mM MgCl₂, 100 mM NaCl, 25 mM NaF, 1 mM Na3VO4, 1 mM DTT, containing 1 tablet of Protease inhibitor cocktail (Roche) per 25 ml of buffer) by pipetting 2 times up and down, followed by a homogenizing step (10 strokes in a dounce homogenizer with tight pestle). The lysate was incubated for 30 min on ice. After spinning for 10 min at 20 000g, the supernatant was subjected to an ultracentrifugation step of 1 h at 100 000 g. The supernatant was frozen in liquid Nitrogen and stored at - 80°C, or the TAP purification was directly continued.

The lysates were thawn quickly in a 37°C waterbath. 0.4 ml of unsettled rabbit IgG-Agarose beads (Sigma, washed 3 times in CZ lysis buffer) were added, and incubated for 2 h rotating at 4°C. Protein complexes bound to the beads were obtained by centrifugation (1 min, 1300 rpm, Heraeus centrifuge). The beads were transferred into 0.8 ml Mobicol M1002 columns (Pierce) and washed with 10 ml CZ lysis buffer (containing 1 tablet of Protease inhibitor cocktail (Roche) per 50 ml of buffer). After an additional washing step with 5 ml TEV cleavage buffer (10 mM Tris, pH 7.5, 100 mM NaCl, 0.1 % IGEPAL, 0.5 mM EDTA, 1 mM DTT), the protein-complexes were eluted from the beads by adding 150 µl TEV cleavage buffer, containing 5µl of TEV-protease (GibcoBRL, Cat.No. 10127-017). For better elution the columns were incubated at 16OC for 1 h (shaking with 850 rpm). The eluate was applied on fresh Mobicol columns, containing 0.2 ml settled Calmodulin affinity resin (Stratagene, washed 3 times with CBP wash buffer). 0.2 ml 2 times CBP binding buffer (10 mM Tris, pH 7:5, 100 mM NaCl, 0,1 % IGEPAL, 2mM MgAc, 2mM Imidazole, 4 mM CaCl₂, 1mM DTT) was added followed by an incubation of 1 h at 4°C, rotating. Protein-complexes bound to the Calmodulin affinity resin were washed with 10 ml of CBP wash buffer (10 mM Tris, pH 7.5, 100 mM NaCl, 0,1 % IGEPAL, 1mM MgAc, 1mM Imidazole, 2 mM CaCl₂, 1mM DTT). They were eluted by addition of 600 µl CBP elution buffer (10 mM Tris, pH 8.0, 5 mM EGTA) for 5 min at 37°C (shaking with 850 rpm). The eluates were transferred into a siliconized tube and lyophilised. The Calmodulin resin was boiled for 5 min in 50 µl 4x Laemmli sample buffer. The fractions were combined and applied on gradient NuPAGE gels (Invitrogen, 4-12%, 1.5 mm, 10 well).

### 5.5.2 Purification of membrane proteins

For the purification of membrane TAP-tagged proteins 5 x 10⁸ adherent cells (corresponding to 40 15 cm plates) were used. The cells were harvested and washed 3 times in cold PBS (3 min, 1300 rpm, Heraeus centrifuge). The cells were frozen in liquid Nitrogen and stored at -80°C, or the TAP purification was directly continued.
The cells were lysed in 10 ml Membrane lysis buffer (50 mM Tris, pH 7.5, 7.5 % Glycerol, 1 mM EDTA, 150 mM NaCl, 25 mM NaF, 1 mM Na3VO4, 1 mM DTT, containing 1 tablet of Protease inhibitor cocktail (Roche) per 25 ml of buffer) by pipetting 2 times up and down, followed by a homogenizing step (10 strokes in a dounce homogenizer with tight pestle). After spinning for 10 min at 1300 rpm (Heraeus centrifuge), the supernatant was subjected to an ultracentrifugation step of 1 h at 100000g. The "Default" supernatant was frozen in liquid Nitrogen and stored at -80°C, or the TAP purification was directly continued. The "Membrane" pellet was resuspended in 7.5 ml Membrane lysis buffer (+ 0.8% IGEPAL) by pipetting, followed by resuspension through a gauge needle for 2 times. After incubation for 1 h at 4°C (rotating) the lysate was cleared by a centrifugation step of 1h at 100 000 g. The "Membrane" supernatant was frozen in liquid Nitrogen and stored at -80°C, or the TAP purification was directly continued.

The lysates were thawn quickly in a 37°C waterbath. 0.4 ml of unsettled rabbit IgG-Agarose beads (Sigma, washed 3 times in Membrane lysis buffer) were added, and incubated for 2 h rotating at 4°C. Protein complexes bound to the beads were obtained by centrifugation (1 min, 1300 rpm, Heraeus centrifuge). The beads were transferred into 0.8 ml Mobicol M1002 columns (Pierce) and the Membrane fractions were washed with 10 ml Membrane lysis buffer (containing 0.8% IGEPAL, and 1 tablet of Protease inhibitor cocktail (Roche) per 50 ml of buffer). The Default fractions were treated the same way, but the buffer was containing only 0.2% IGEPAL. After an additional washing step with 5 ml TEV cleavage buffer (10 mM Tris, pH 7.5, 100 mM NaCl, 0.5 mM EDTA, 1 mM DTT, containing 0.5% IGEPAL for the Membrane fraction and 0.1% IGEPAL for the Default fraction), the protein-complexes were eluted from the beads by adding 150 µl TEV cleavage buffer, containing 5 µl of TEV-protease (GibcoBRL, Cat.No. 10127-017). For better elution the columns were incubated at 16°C for 1 h (shaking with 850 rpm). For the Membrane fraction 3 additional µl of TEV-protease were added and incubated for another hour. The eluate was applied on fresh Mobicol columns, containing 0.2 ml settled Calmodulin affinity resin (Stratagene, washed 3 times with CBP wash buffer). 0.2 ml 2 times CBP binding buffer (10 mM Tris, pH 7.5, 100 mM NaCl, 0,1 % IGEPAL, 2mM MgAc, 2mM Imidazole, 4 mM CaCl₂, 1 mM DTT) was added followed by an incubation of 1 h at 4°C rotating. Protein-complexes bound to the Calmodulin affinity resin were washed with 10 ml of CBP wash buffer (10 mM Tris, pH 7.5, 100 mM NaCl, 0,1 % IGEPAL, 1 mM MgAc, 1 mM Imidazole, 2 mM CaCl₂, 1 mM DTT). They were eluted by addition of 600 µl CBP elution buffer (10 mM Tris, pH 8.0, 5 mM EGTA) for 5 min at 37°C (shaking with 850 rpm). The eluates were transferred into a siliconized tube and lyophilised. The Calmodulin resin was boiled for 5 min in 50 µl 4x Laemmli sample buffer. The fractions were combined and applied on gradient NuPAGE gels (Invitrogen, 4-12%, 1.5 mm, 10 well).

### 5.5.3 Purification of nuclear proteins

For the purification of nuclear TAP-tagged proteins 5 x 10⁸ adherent cells (corresponding to 40 15 cm plates) were used. The cells were harvested and washed 3 times in cold PBS (3 min, 1300 rpm, Heraeus centrifuge). The cells were frozen in liquid Nitrogen and stored at -80°C, or the TAP purification was directly continued.
The cells were lysed in 10 ml Hypotonic cell lysis buffer A (10 mM Tris, pH 7.5, 1.5 mM MgCl₂, 10 mM KCI, 25 mM NaF, 0.5 mM Na3VO4, 1 mM DTT, containing 1 tablet of Protease inhibitor cocktail (Roche) per 25 ml of buffer) by pipetting 2 times up and incubation for 10 min on ice, followed by a homogenizing step (20 strokes in a dounce homogenizer with tight pestle). After spinning the lysate for 10 min at 2000g, "Default" supernatant and "Nuclear" pellet were treated separately. The Default fraction was made up to a final of 90 mM NaCl, 0,2 % NP40 and 5 % Glycerol. This was cleared by centrifugation for 1 h at 100 000g. The "Default" supernatant was frozen in liquid Nitrogen and stored at -80°C, or the TAP purification was directly continued. The Nuclear pellet was resuspended in 5 ml Nuclear lysis buffer B (50 mM Tris, pH 7.5, 1.5 mM MgCl₂, 20 % Glycerol, 420 mM NaCl, 25 mM NaF, 0.5 mM Na3VO4, 1 mM DTT, containing 1 tablet of Protease inhibitor cocktail (Roche) per 25 ml of buffer) and incubated for 30 min on ice, shaking frequently. To precipitate the DNA Dilution buffer (50 mM Tris, pH 7.5, 0.26% IGEPAL, 1.5 mM MgCl₂, 25 mM NaF, 0.5 mM Na3VO4, 1 mM DTT, containing 1 tablet of Protease inhibitor cocktail (Roche) per 50 ml of buffer) was added in a ratio of 1:3 resuspended pellet to dilution buffer, and incubated for 10 more min on ice. After spinning for 1 h at 100 000g the supernatant was frozen in liquid Nitrogen and stored at -80°C, or the TAP purification was directly continued.

The lysates were thawn quickly in a 37°C waterbath. The Nuclear fractions were subjected to a centrifugation step of 20 min at 100 000g. 0.4 ml of unsettled rabbit IgG-Agarose beads (Sigma, washed 3 times in CZ lysis buffer) were added, and incubated for 2 h rotating at 4°C. Protein complexes bound to the beads were obtained by centrifugation (1 min, 1300 rpm, Heraeus centrifuge). The beads were transferred into 0.8 ml Mobicol M1002 columns (Pierce) and washed with 10 ml CZ lysis buffer (containing 1 tablet of Protease inhibitor cocktail (Roche) per 50 ml of buffer). After an additional washing step with 5 ml TEV cleavage buffer (10 mM Tris, pH 7.5, 100 mM NaCl, 0.1 % IGEPAL, 0.5 mM EDTA, 1 mM DTT), the protein-complexes were eluted from the beads by adding 150 µl TEV cleavage buffer, containing 5µl of TEV-protease (GibcoBRL, Cat. No. 10127-017). For better elution the columns were incubated at 16OC for 1 h (shaking with 850 rpm). The eluate was applied on fresh Mobicol columns, containing 0.2 ml settled Calmodulin affinity resin (Stratagene, washed 3 times with CBP wash buffer). 0.2 ml 2 times CBP binding buffer (10 mM Tris, pH 7.5, 100 mM NaCl, 0,1 % IGEPAL, 2mM MgAc, 2mM Imidazole, 4 mM CaCl2, 1mM DTT) was added followed by an incubation of 1 h at 4OC rotating. Protein-complexes bound to the Calmodulin affinity resin were washed with 10 ml of CBP wash buffer (10 mM Tris, pH 7.5, 100 mM NaCl, 0,1 % IGEPAL, 1mM MgAc, 1mM Imidazole, 2 mM CaCl₂, 1mM DTT). They were eluted by addition of 600 µl CBP elution buffer (10 mM Tris, pH 8.0, 5 mM EGTA) for 5 min at 37°C (shaking with 850 rpm). The eluates were transferred into a siliconized tube and lyophilised. The Calmodulin resin was boiled for 5 min in 50 µl 4x Laemmli sample buffer. The fractions were combined and applied on gradient NuPAGE gels (Invitrogen, 4-12%, 1.5 mm, 10 well).

### 5.5.4 Immunoprecipitation of the endogenous TNFR1-complex

### 5.5.4.1 Cross-linking of the antibody :

Protein A or G beads (120-180 µl dry) were washed twice with sodium borate 0.2 M pH 9 and then twice with PBS. The antibody 50-80 µg (monoclonal) or 100-300 µg (polyclonal) was incubated with the beads in 1 ml PBS for 90 minutes at room temperature (RT) with gentle agitation. Beads were then washed twice with 1 ml 50 mM sodium borate pH 9, then twice with 1 ml 50 mM sodium borate pH 9 + 3 M NaCl. Beads were incubated for 45 minutes in the same buffer containing the cross-linker, Dimethyl pimelimidate at 20 mM final (the pH should be readjusted to 8.3-8.4). The reaction was then stopped by washing once with ethanolamine ph 8.0. The beads were let at RT in ethanolamine for 30 minutes. They were then washed once more with ethanolamine and then twice with Glycine 100 mM pH 3. Finally beads were washed twice in lysis buffer and let to equilibrate in this buffer for at least 20 minutes before use.

### 5.5.4.2 Cell lysis :

Cells (2x 10exp9 Hela S3; 4x 10exp9 Jurkat) were lysed in 20 mM TRIS pH 7.5, 150 mM NaCl, 1% Triton X100, 1 mM DTT, 1 mM Sodium vanadate, 10 mM Sodium fluoride and proteases inhibitors (and 5 mM MgCl2 for Jurkat) and incubated in this buffer for 1 hour at 4 °C. The lysates are then briefly spun for 10 minutes at 16 000 g and then at 100 000 g in an ultracentrifuge for 1 hour. Supernatant was precleared with 120 µl (dry) of protein A or protein G for at least 40 minutes. Cleared lysate (150 mg of total proteins) was then incubated with the antibody coupled to beads at 4 °
C for 5 hours. Supernatant is then centrifuged for 1 min at 3.000 g. Beads were then washed 3 times in lysis buffer (one short wash and two 5 minutes washes) and once in lysis buffer without detergent. Beads were then resuspended in denaturating buffer. Beads were boiled for 5 min at 90 ° C and supernatant was loaded on denaturating gels.

### 5.6 Protein Identification by Mass Spectrometry

### 5.6.1 Protein digestion prior to mass spectrometric analysis:

Gel-separated proteins were reduced, alkylated and digested in gel essentially following the procedure described by Shevchenko et al. (Shevchenko, A., Wilm, M., Vorm, O., Mann, M. Anal Chem 1996, 68, 850-858). Briefly, gel-separated proteins were excised from the gel using a clean scalpel, reduced using 10 mM DTT (in 5mM ammonium bicarbonate, 54 °C, 45 min) and subsequently alkylated with 55 mM iodoacetamid (in 5 mM ammonium bicarbonate) at room temperature in the dark (30 min). Reduced and alkylated proteins were digested in gel with porcine trypsin (Promega) at a protease concentration of 12.5 ng/µl in 5mM ammonium bicarbonate. Digestion was allowed to proceed for 4 hours at 37 °C and the reaction was subsequently stopped using 5 µl 5% formic acid.

### 5.6.2. Sample preparation prior to analysis by Mass-spectrometry

Gel plugs were extracted twice with 20 µl 1% TFA and pooled with acidified digest supernatants. Samples were dried in a a vaccum centrifuge and resuspended in 13 µl 1% TFA.

### 5.6.3. Mass-spectrometric data acquisition

Peptide samples were injected into a nano LC system (CapLC, Waters or Ultimate, Dionex) which was directly coupled either to a quadrupole TOF (QTOF2, QTOF Ultima, QTOF Micro, Micromass or QSTAR Pulsar, Sciex) or ion trap (LCQ Deca XP) mass spectrometer. Peptides were separated on the LC system using a gradient of aqueous and organic solvents (see below). Solvent A was 5% acetonitrile in 0.5% formic acid and solvent B was 70% acetonitrile in 0.5% formic acid.

| Time (min) | % solvent A | % solvent B |
|---|---|---|
| 0 | 95 | 5 |
| 5.33 | 92 | 8 |
| 35 | 50 | 50 |
| 36 | 20 | 80 |
| 40 | 20 | 80 |
| 41 | 95 | 5 |
| 50 | 95 | 5 |
| Peptides eluting off the LC system were partially sequenced within the mass spectrometer. | | |

### 5.6.4 Protein identification

The peptide mass and fragmentation data generated in the LC-MS/MS experiments were used to query fasta formatted protein and nucleotide sequence databases maintained and updated regularly at the NCBI (for the NCBInr, dbEST and the human and mouse genomes) and European Bioinformatics Institute (EBI, for the human, mouse, Drosophila and C. Elegans proteome databases). Proteins were identified by correlating the measured peptide mass and fragmentation data with the same data computed from the entries in the database using the software tool Mascot (Matrix Science, Perkins, D. N., Pappin, D. J., Creasy, D. M., Cottrell, J. S., Electrophoresis 1999, 20, 3551-67). Search criteria varied depending on which mass spectrometer was used for the analysis.

### .5. 7 ASSAYS FOR ASSAYING THE ACTIVITIES OF THE COMPLEXES PRESENTED IN THE INVENTION

Assay for assaying the biochemical activities of the individual complexes of the invention are described in supra.

In addition, assays are provided to assay the effects of modulating the activity of any of the complexes/components of the TNF-α-pathway as provided herein on the activity of the pathway in general.

An example of such assay is the NF-κB Luciferase Reporter Assay, in which a modulating effect on any part of the TNF-α-pathway is reflected in a change in the expression of a luciferase reporter gene.

### The assay is described below:

In general, one day before transfection 2-5x105 HEK293, HeLa or Jurkat cells were plated in each well of a 24-well plate. On the following day cells were transfected with LipofectAMINE 2000 reagent (Invitrogen) according to the manufacturer's instructions. Briefly, 0.2 mikrog of the NF-κB-dependent-luciferase reporter gene plasmid pNF-κB-Luc (Stratagene) and various amounts of expression constructs were cotransfected together with 0,1 ng of ubiquitin promoter-driven Renilla luciferase reporter construct to normalize for transfection efficiency. The total amount of transfected plasmid DNA (0,8 µg per well) was kept constant by supplementation with pBS SK-. Cells were incubated with LipofectAMINE 2000 for 18-20 h in the presence of serum. About 40 h posttransfection, cells were treated with TNF-α (10 ng/ml) for about 6 h prior to cell lysis in Passive Lysis Buffer® (Promega). Reporter gene activity was determined by Dual-Luciferase® Reporter Assay System (Promega).

### Expression vector suitable for the NF-κB Luciferase Reporter Assay:

Mammalian expression vectors were generated by directional cloning of PCR-amplified ORFs into a modified version of a MoMLV-based retroviral vector via the GATEWAY site-specific recombination system (Invitrogen) thereby adding one HA-tag to the N-terminus of the respective ORF. The pUbi-RL (expressing renilla) has been described by Westermarck et al. (2002/EMBO J. 21, 451-460).

Other examples or reporter genes include, but are not limited to CAT (chloramphenicol acetyl transferase) (Alton and Vapnek (1979), Nature 282: 864-869), luciferase, and other enzyme detection systems, such as β-galactosidase; firefly luciferase (deWet et al (1987) Mol Cell Biol 7: 725-737); bacterial luciferase (Engebrecht and Silverman (1984) Proc Natl Acad Sci USA 1: 4154-4158; Baldwin et al (1984) Biochemistry 23: 3663-3667); alkaline phosphatase (Toh et al (1989) Eur J. Biochem 182: 231-238; Hall et al (1983) J Mol Appl Gen 2: 101), human placental secreted alkaline phosphatase (Cullen and Malim (1992) Methods in Enzymol 216: 362-368). The amount of transcription from the reporter gene may be measured using any suitable method as known by those skilled in the art.

The assays can be carried out either in the cells naturally expressing the TNFα-pathway. In still further embodiments, the TNF-α-signalling pathway is generated in whole cells, taking advantage of cell culture techniques to support the subject assay. The components of the TNF-a-signalling pathway can be endogenous to the cell selected to support the assay. Alternatively, some or all of the components can be derived from exogenous sources.

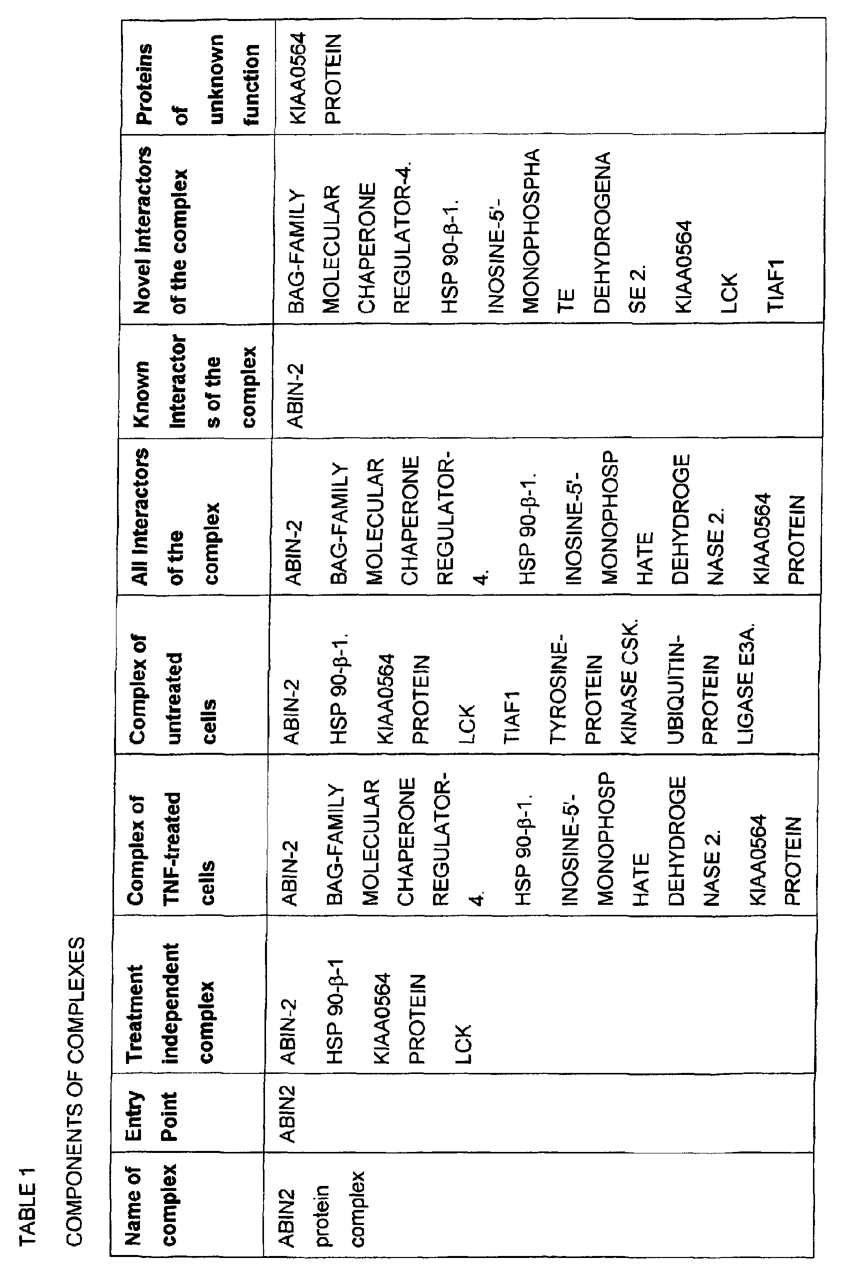

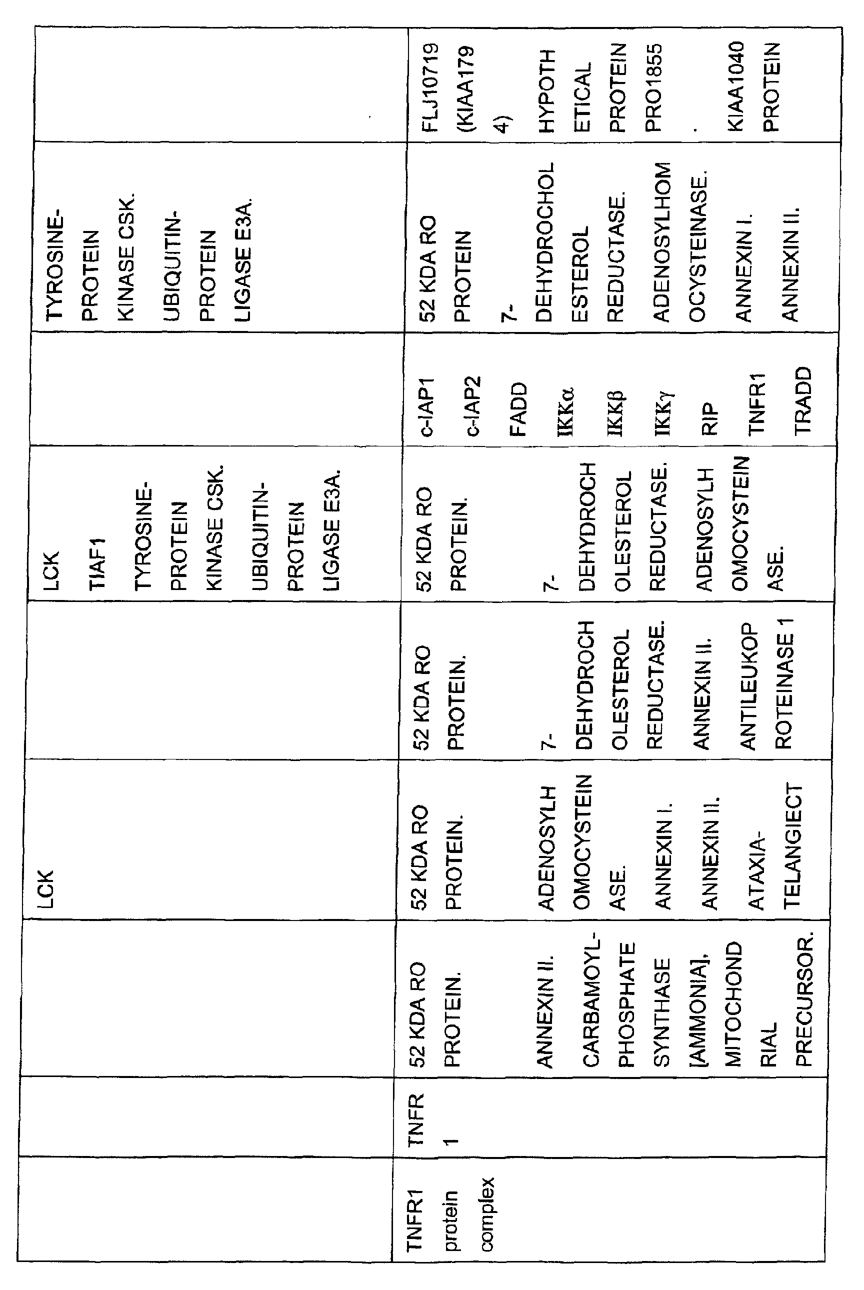

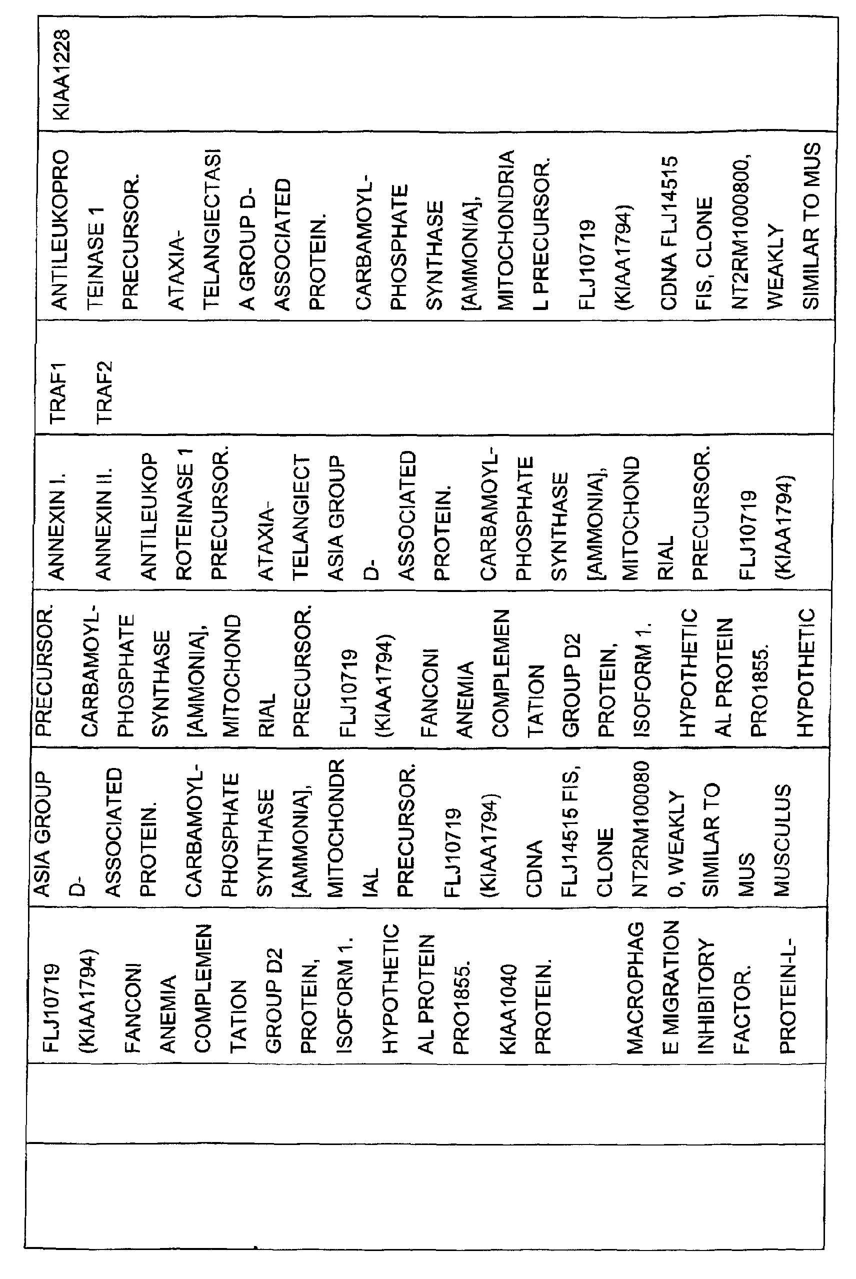

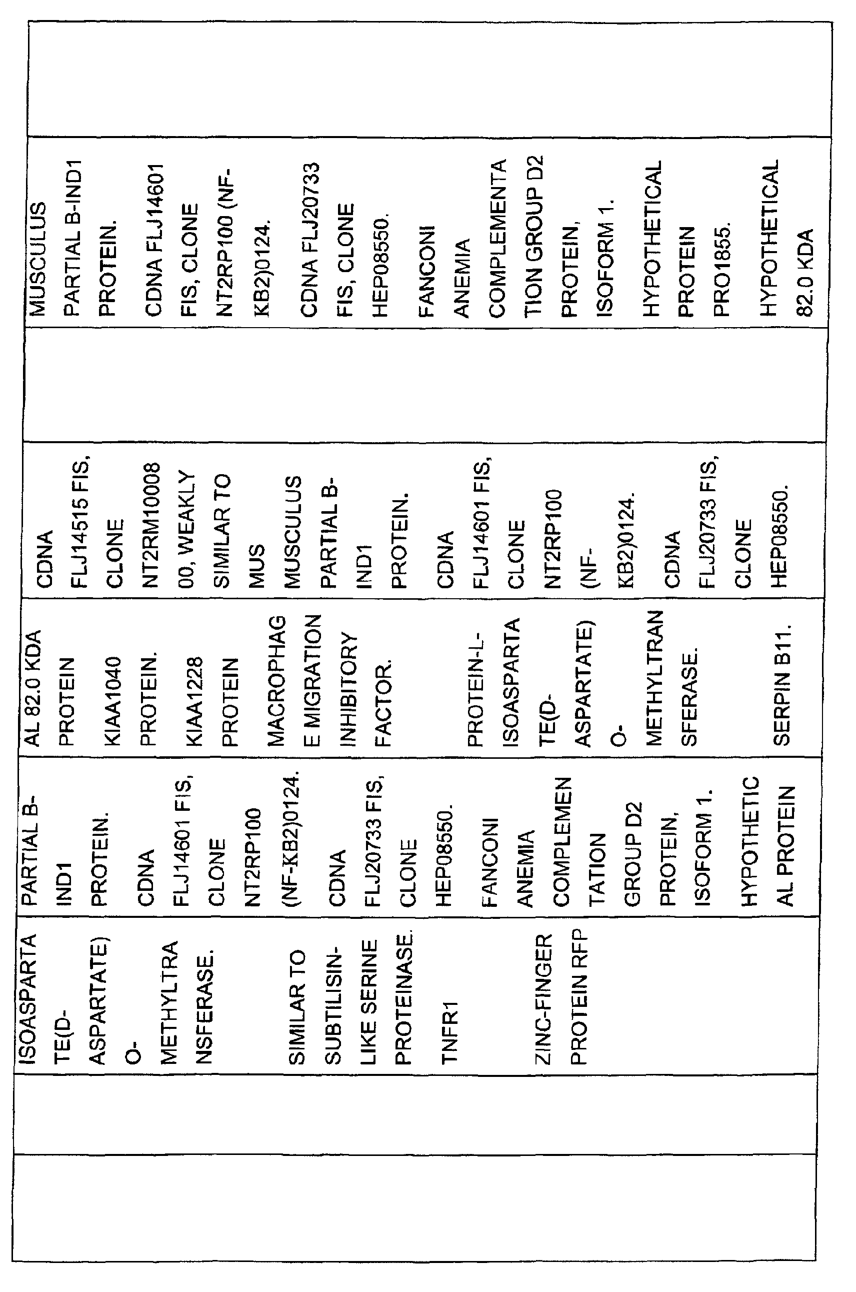

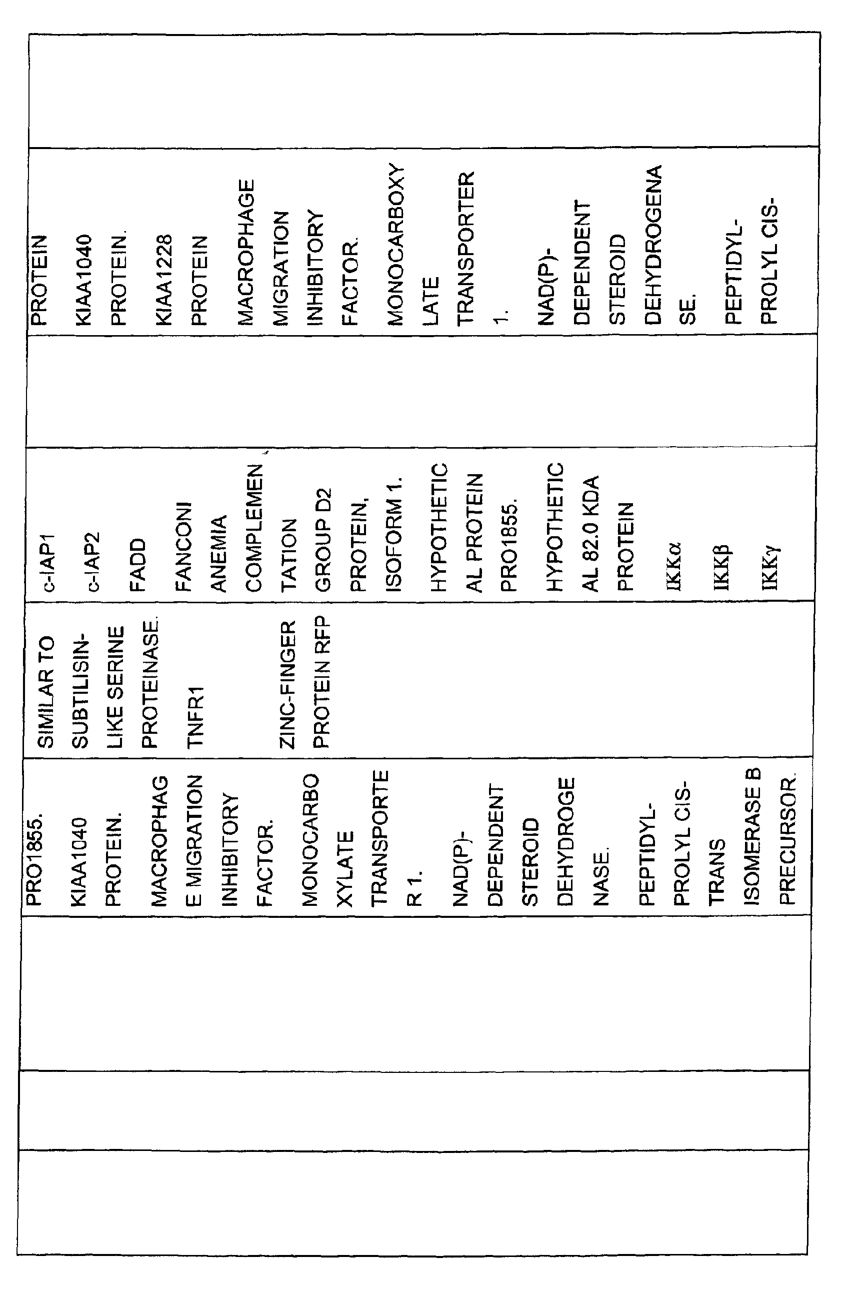

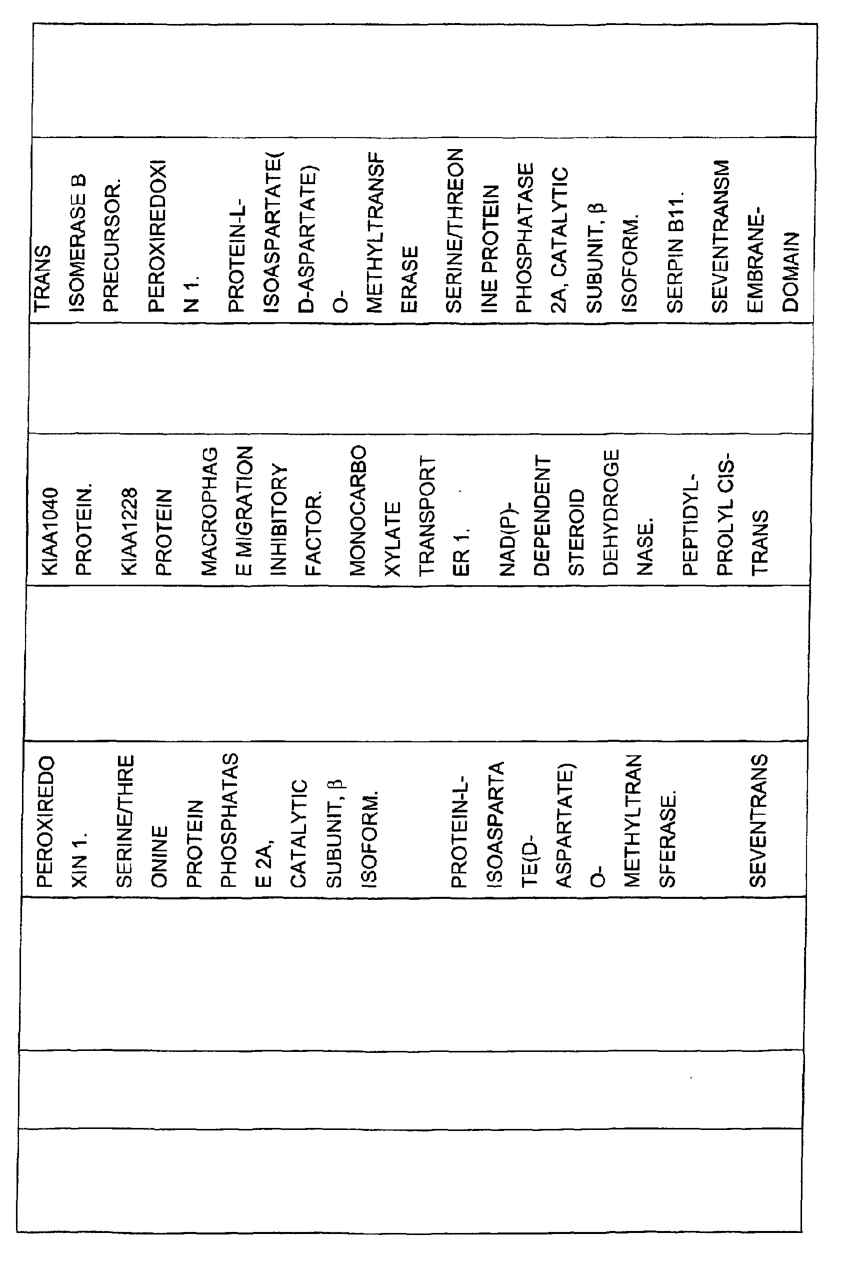

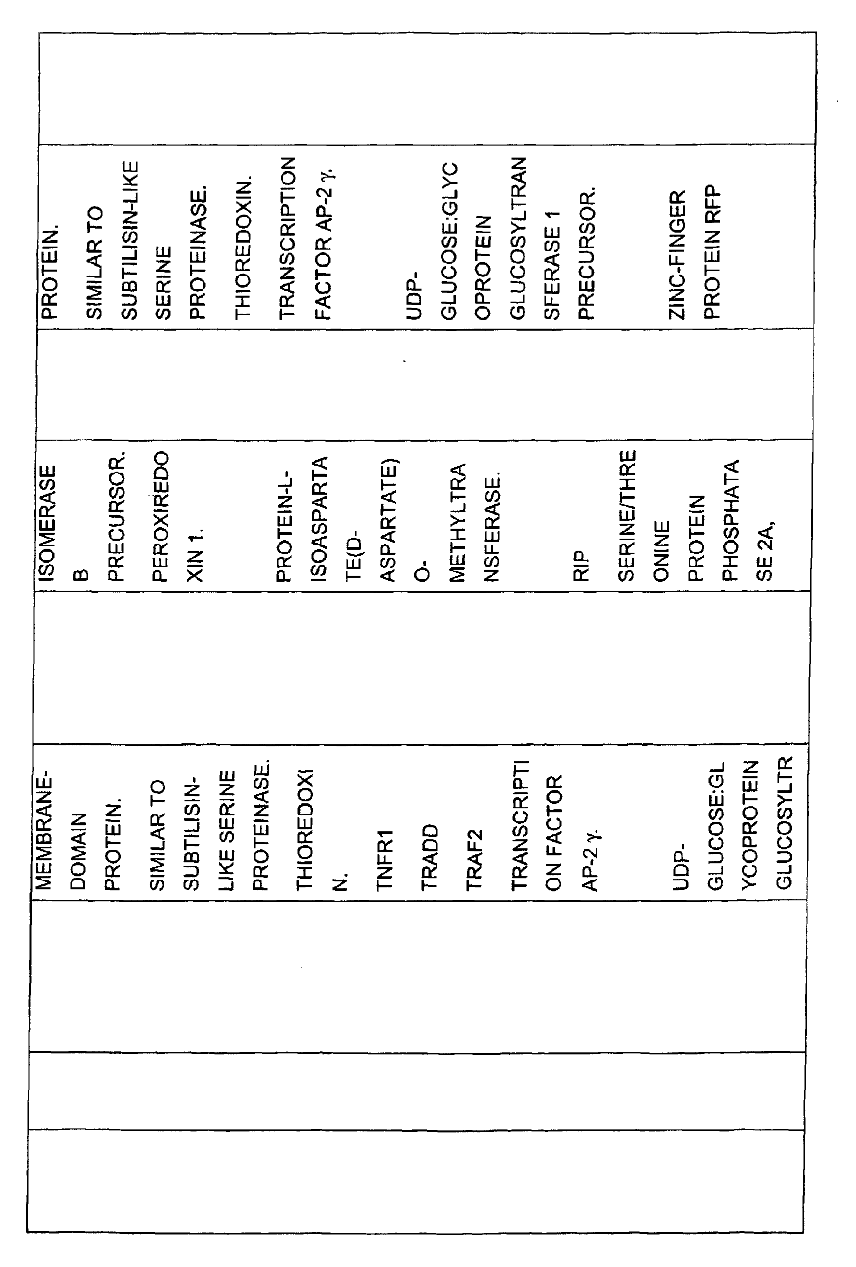

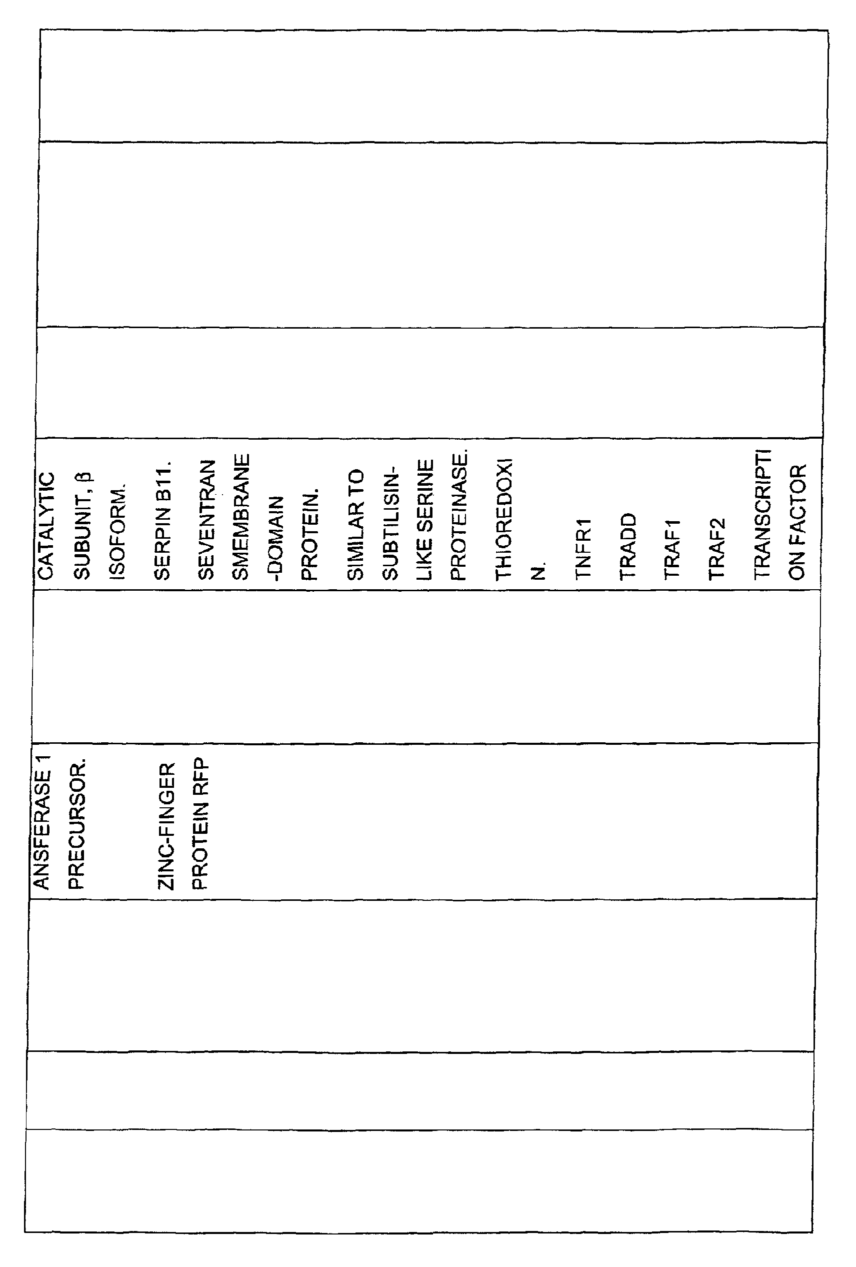

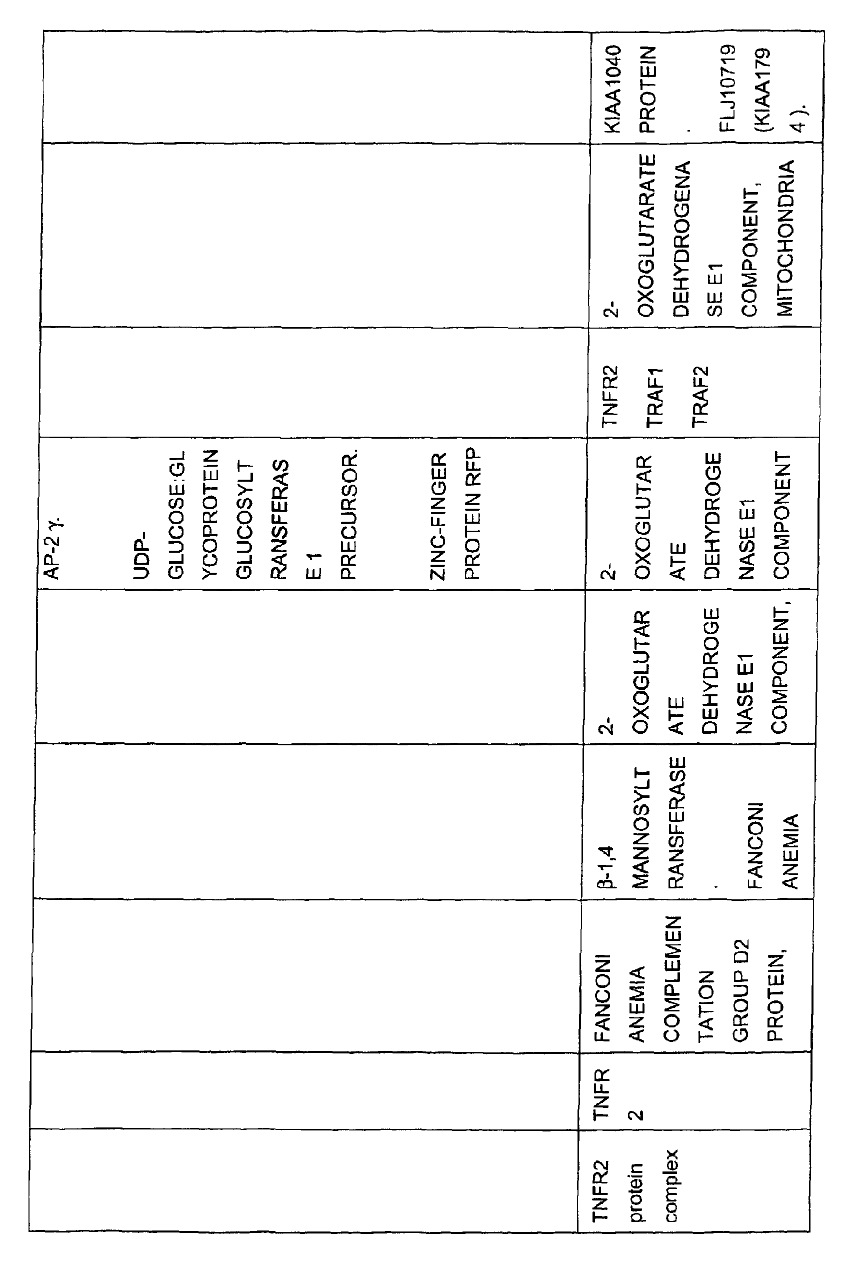

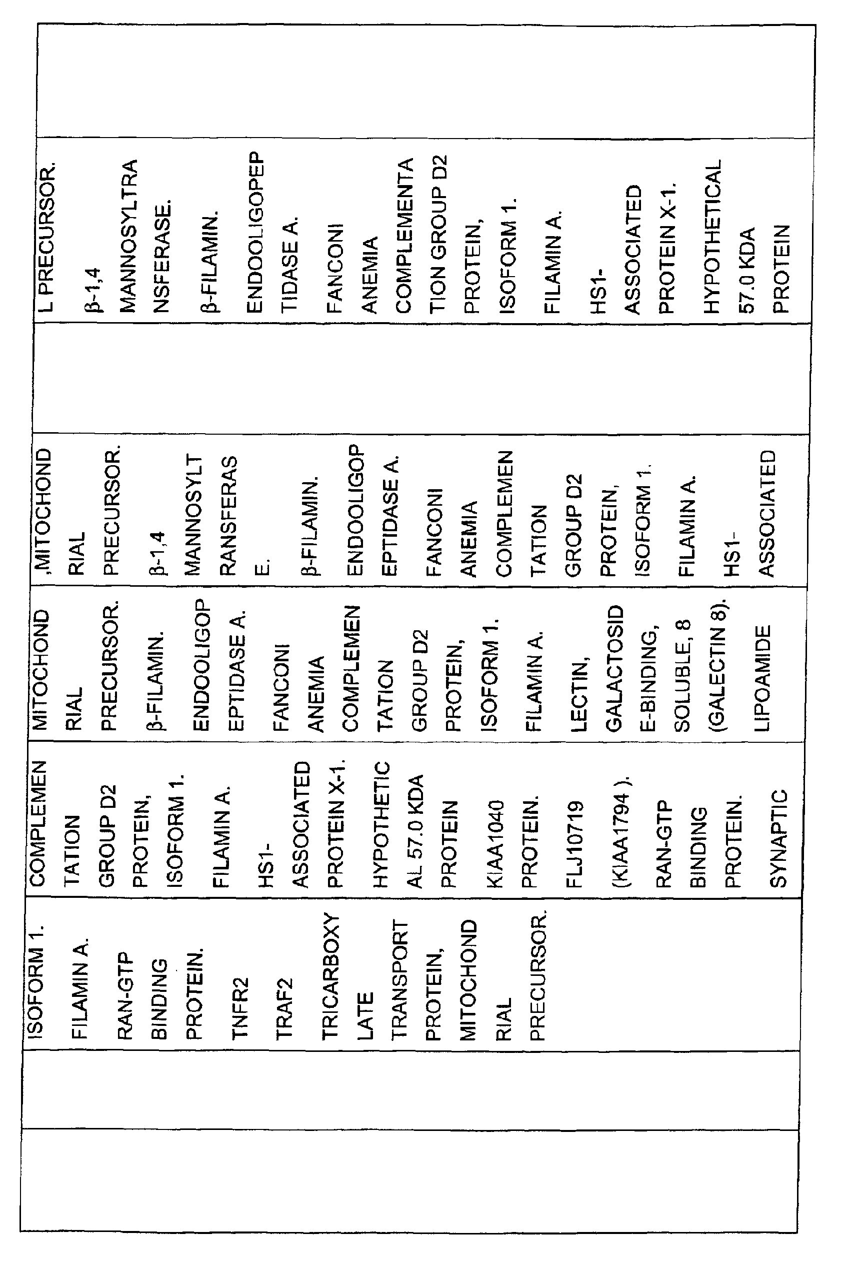

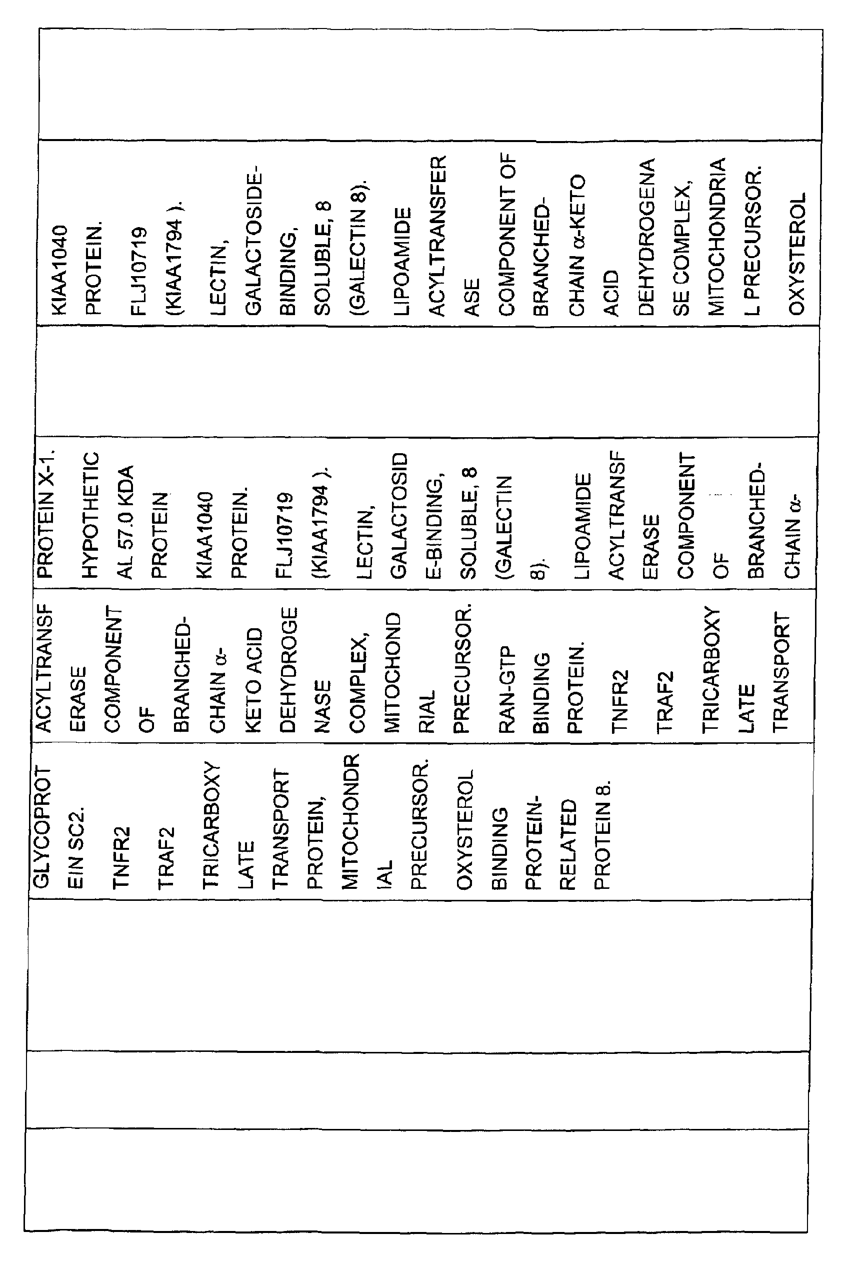

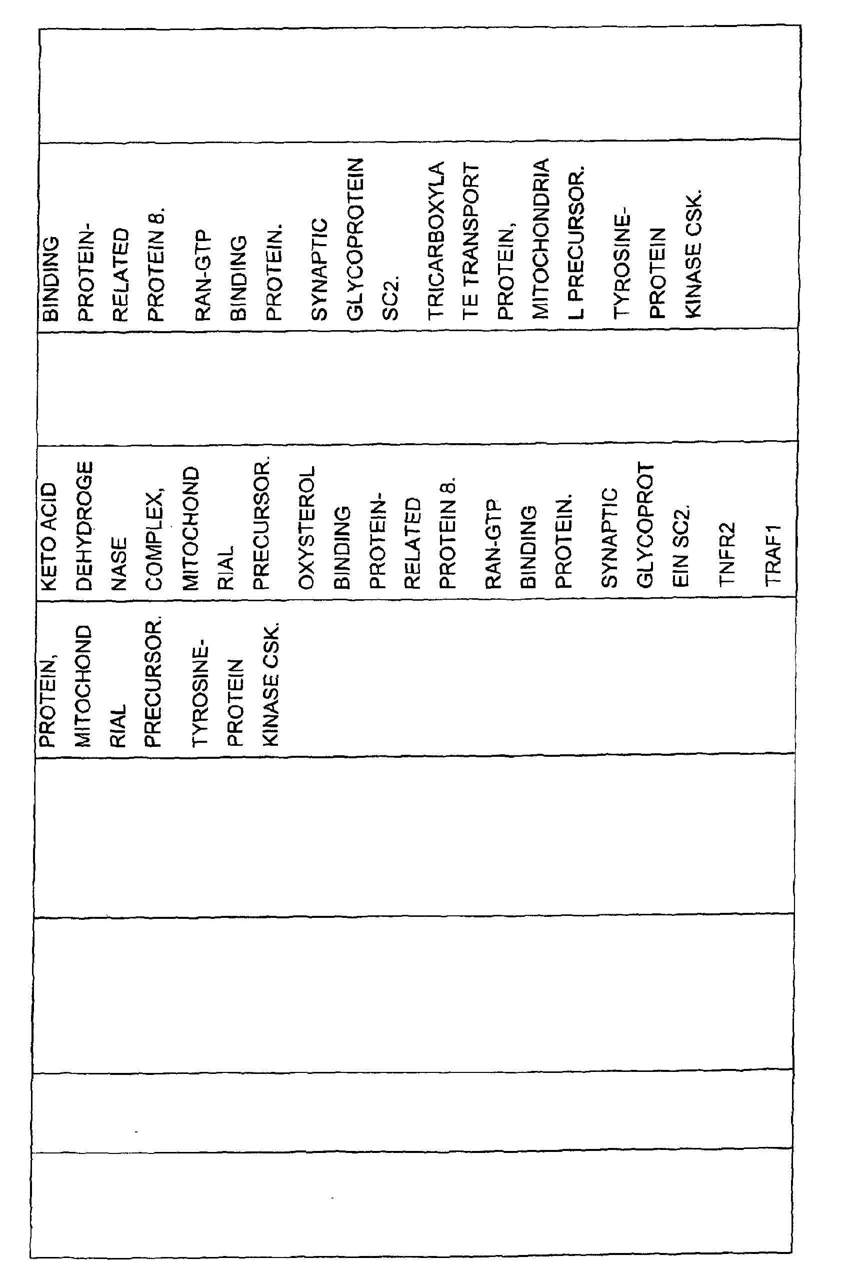

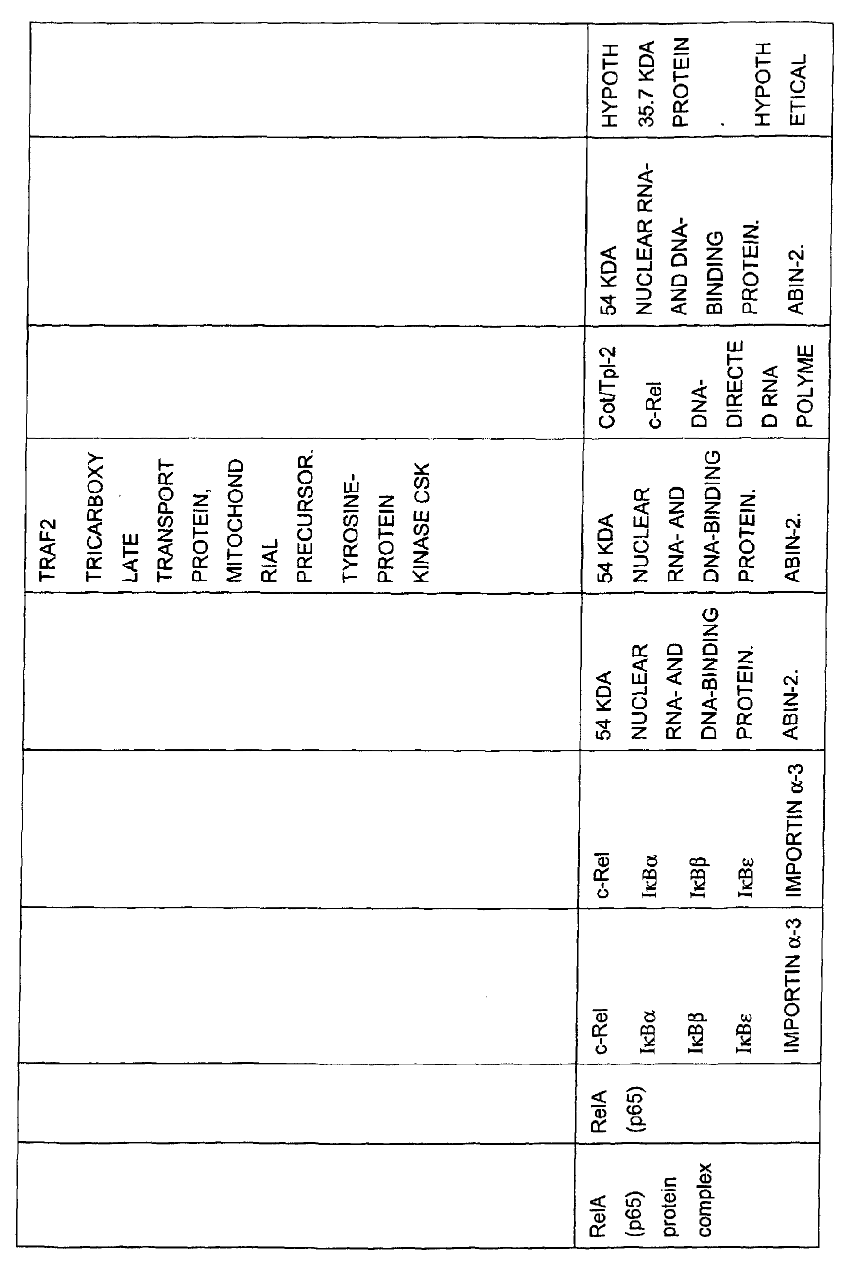

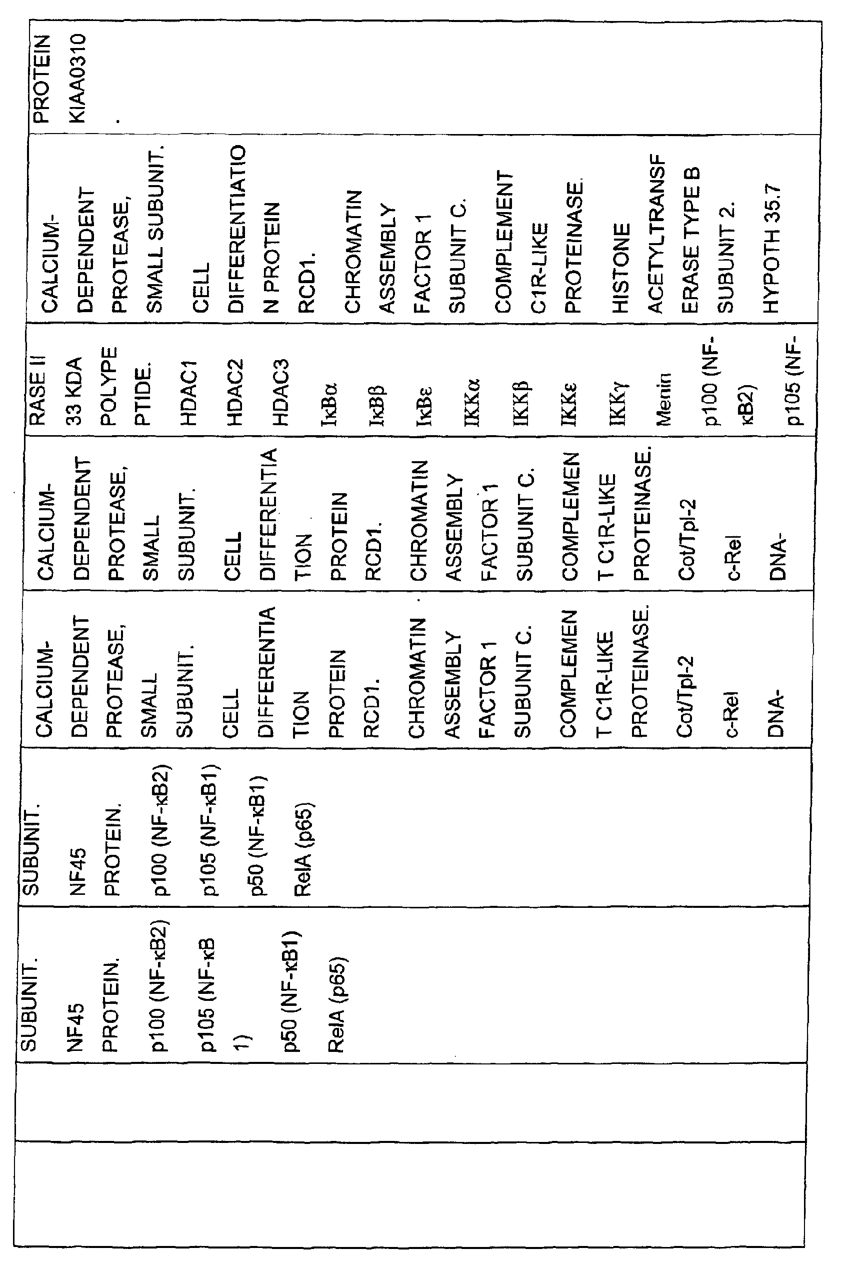

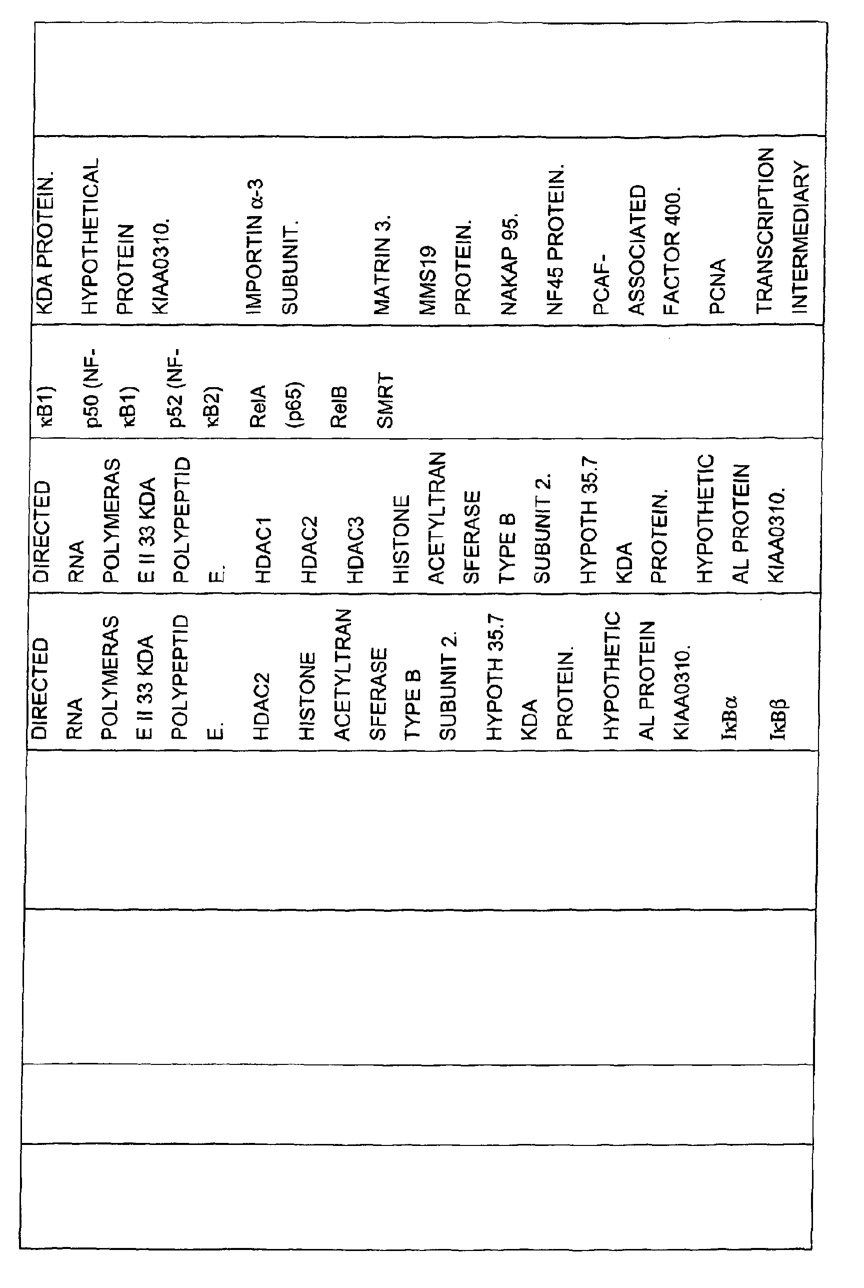

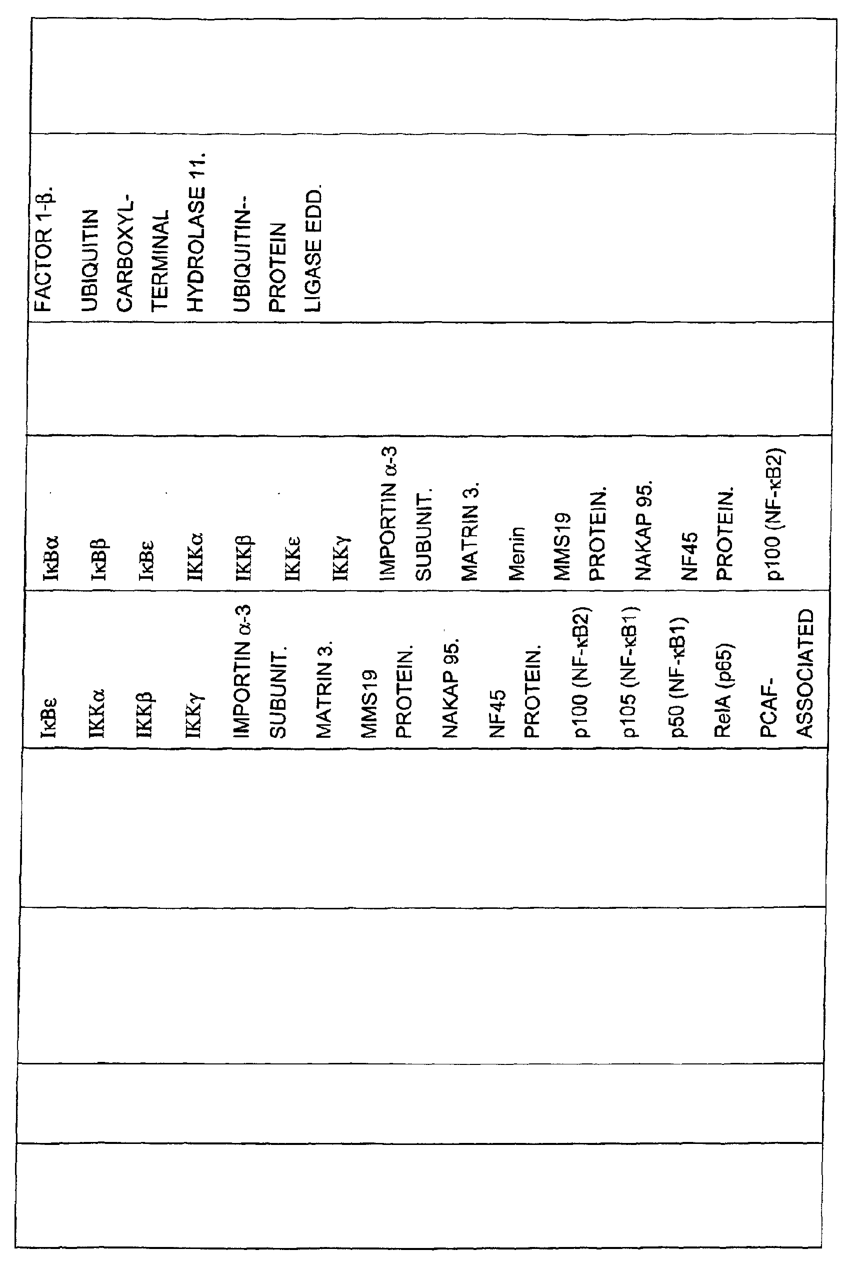

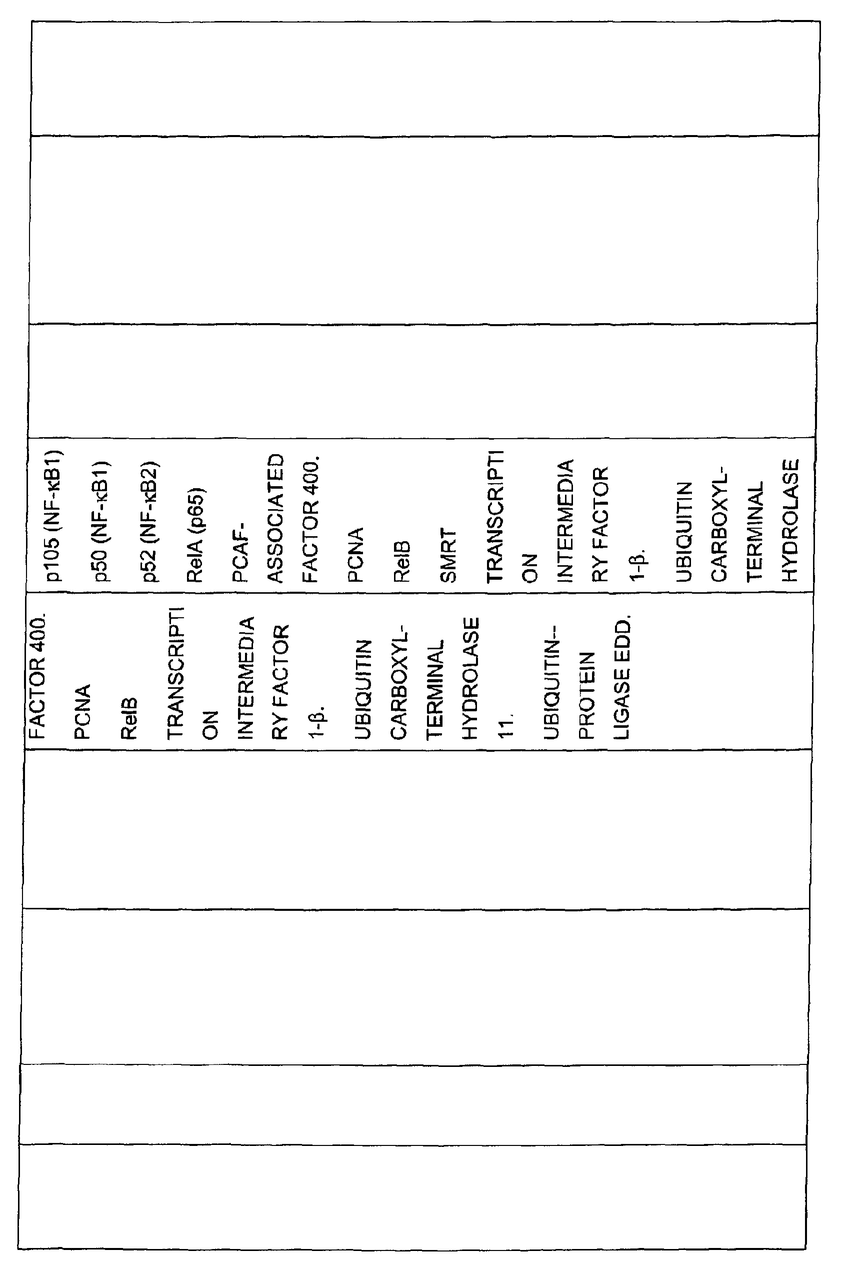

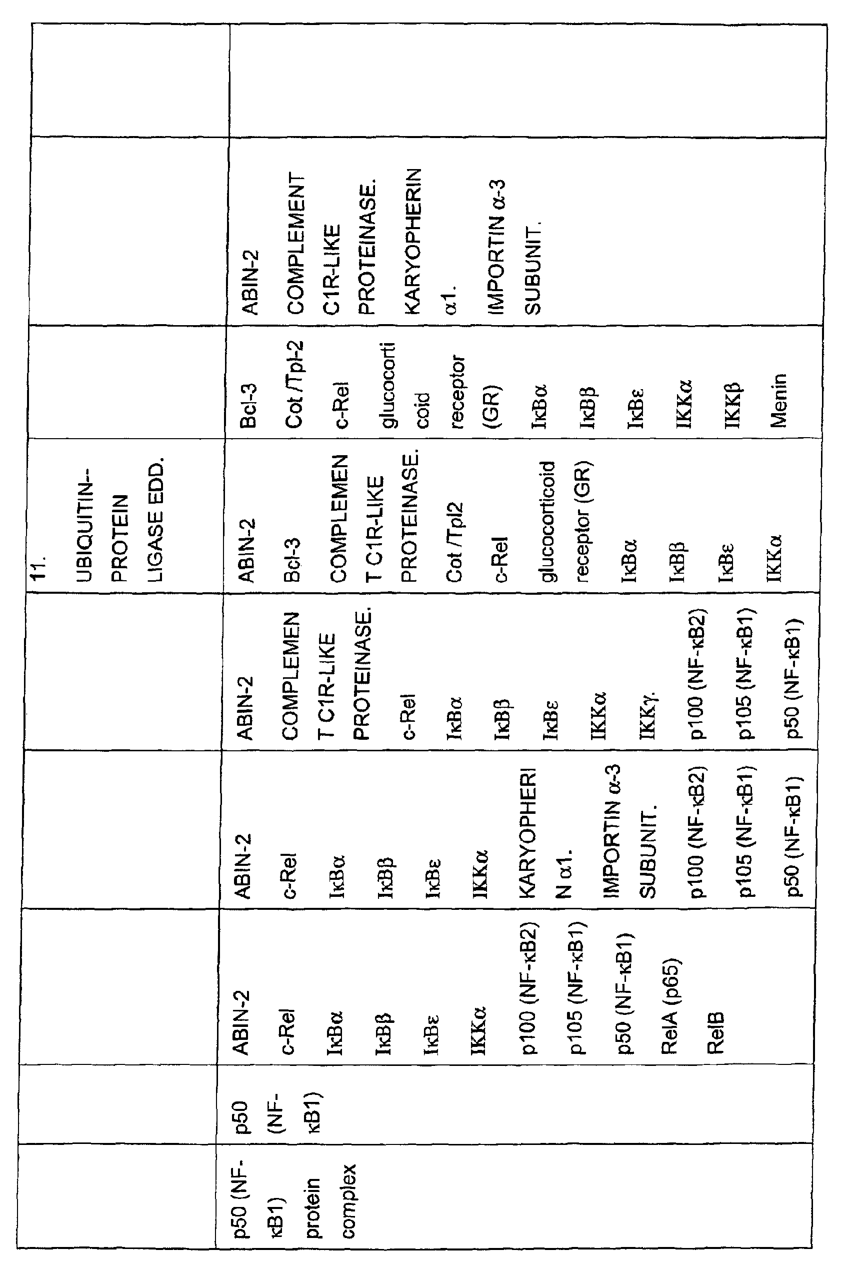

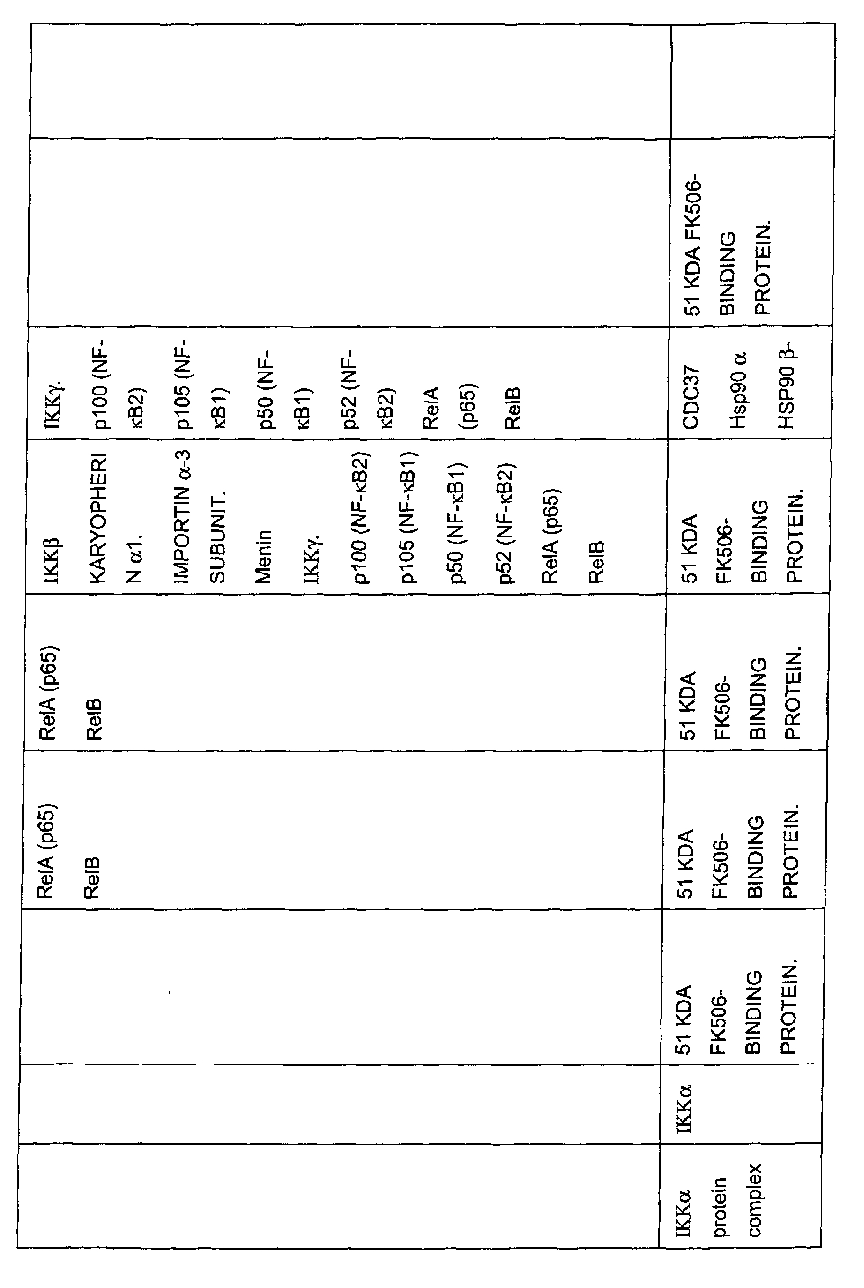

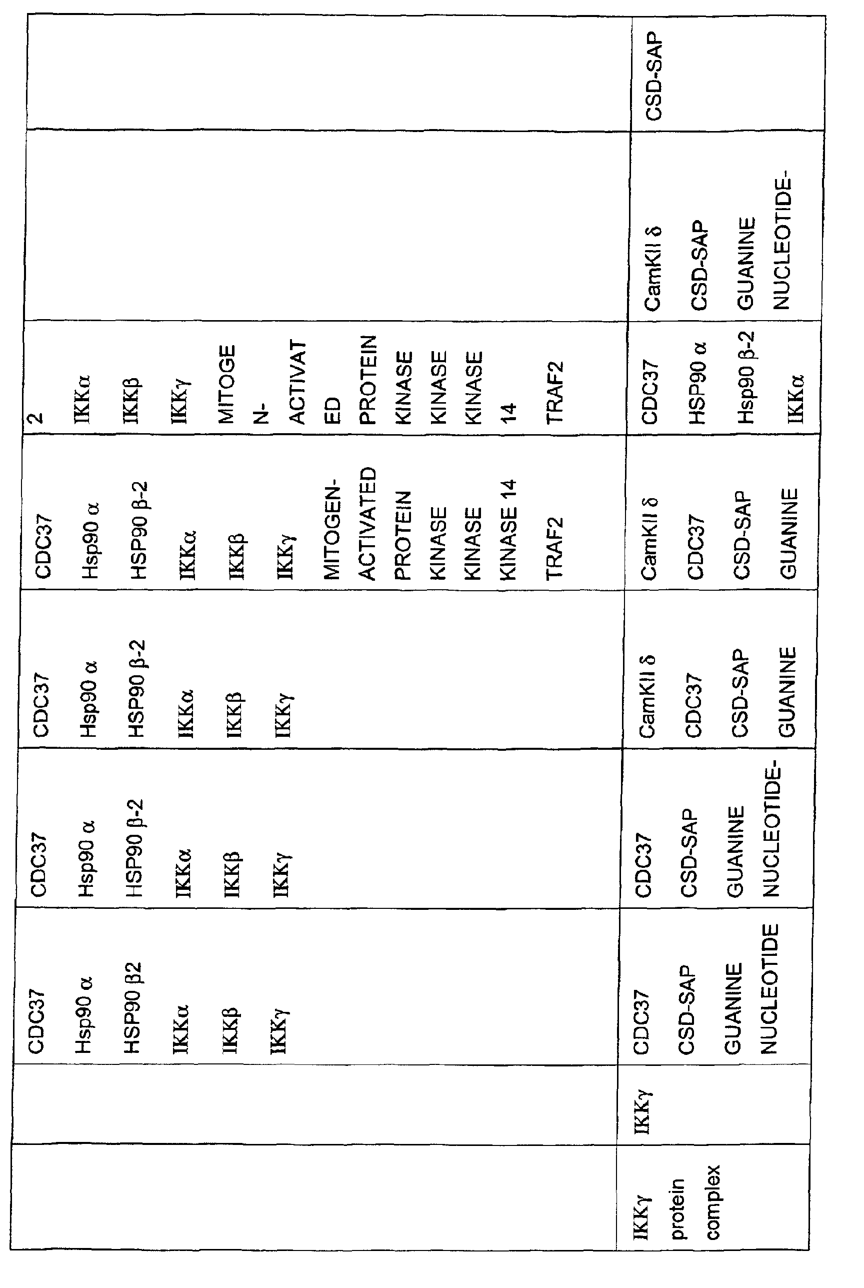

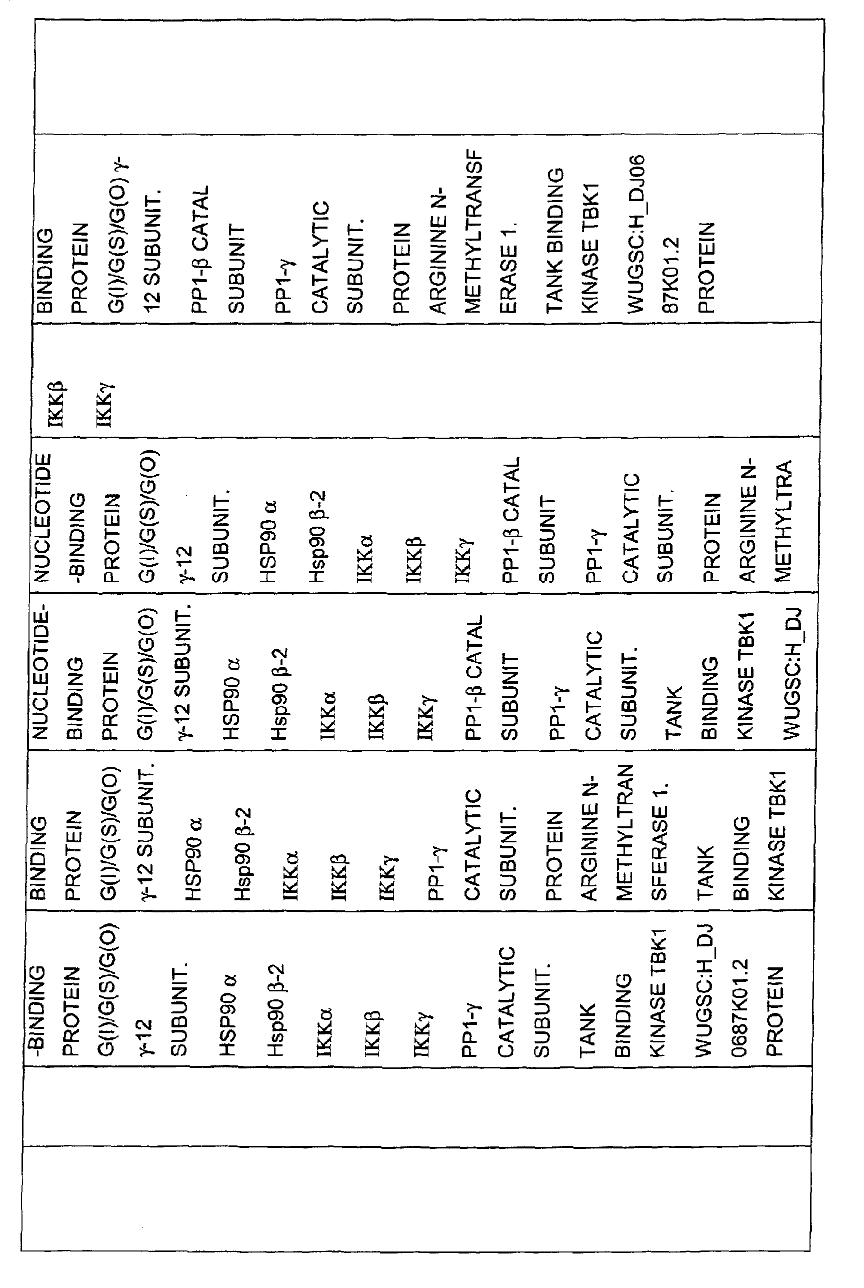

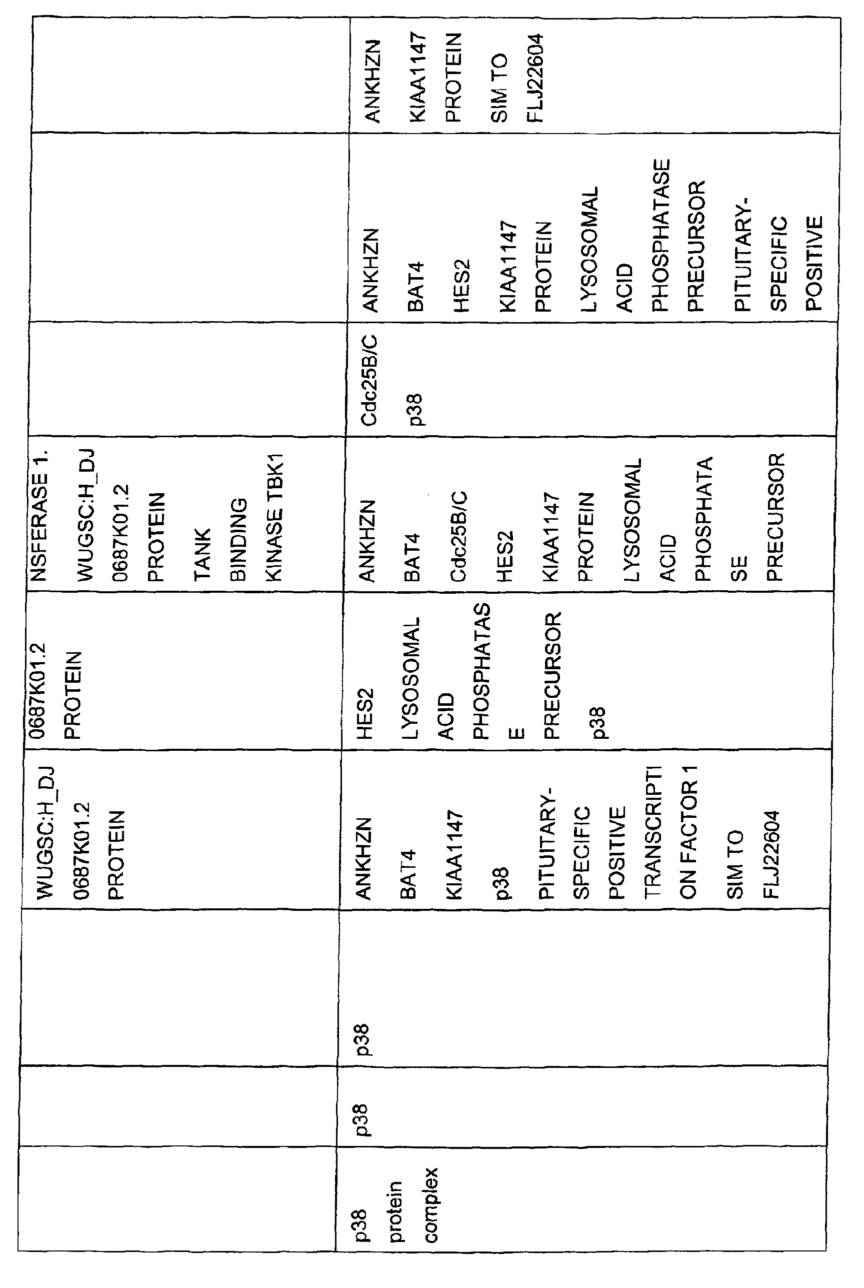

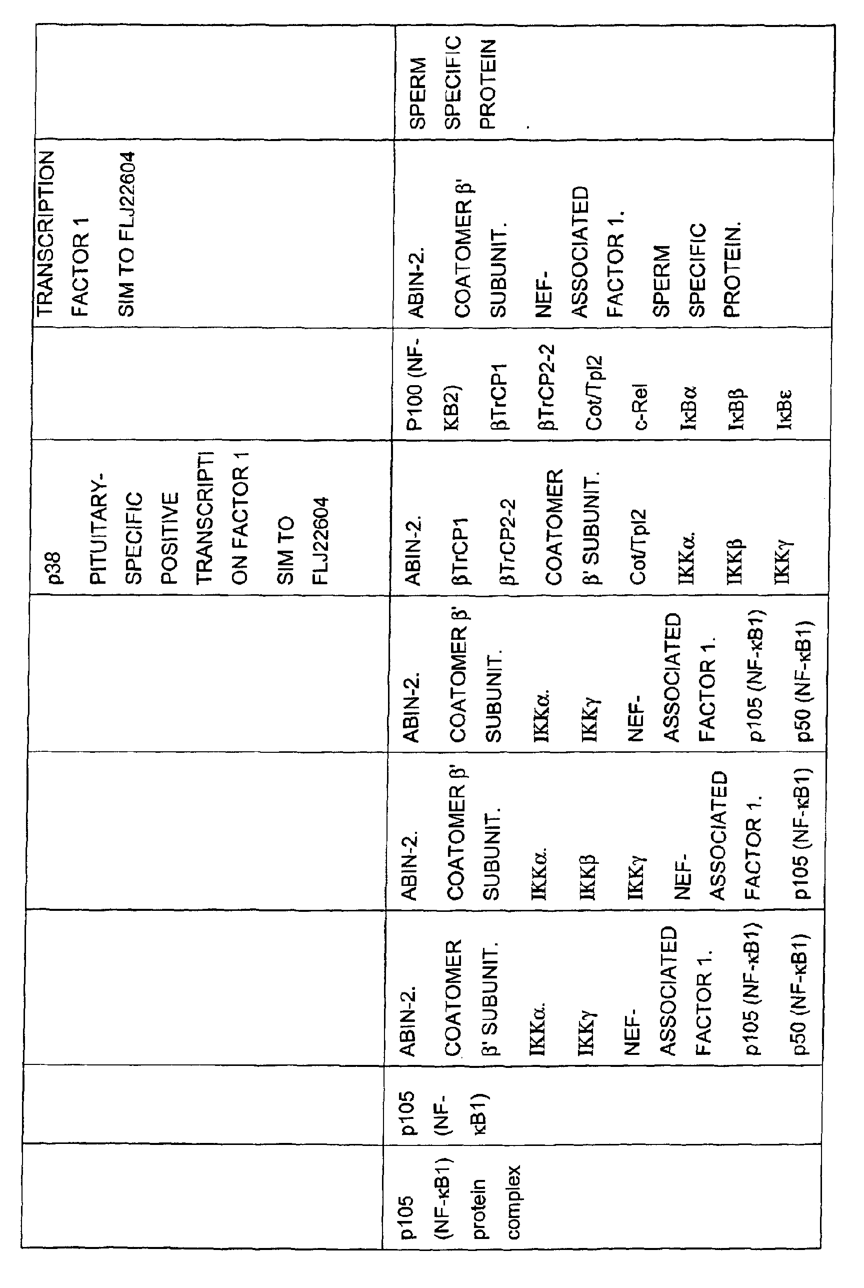

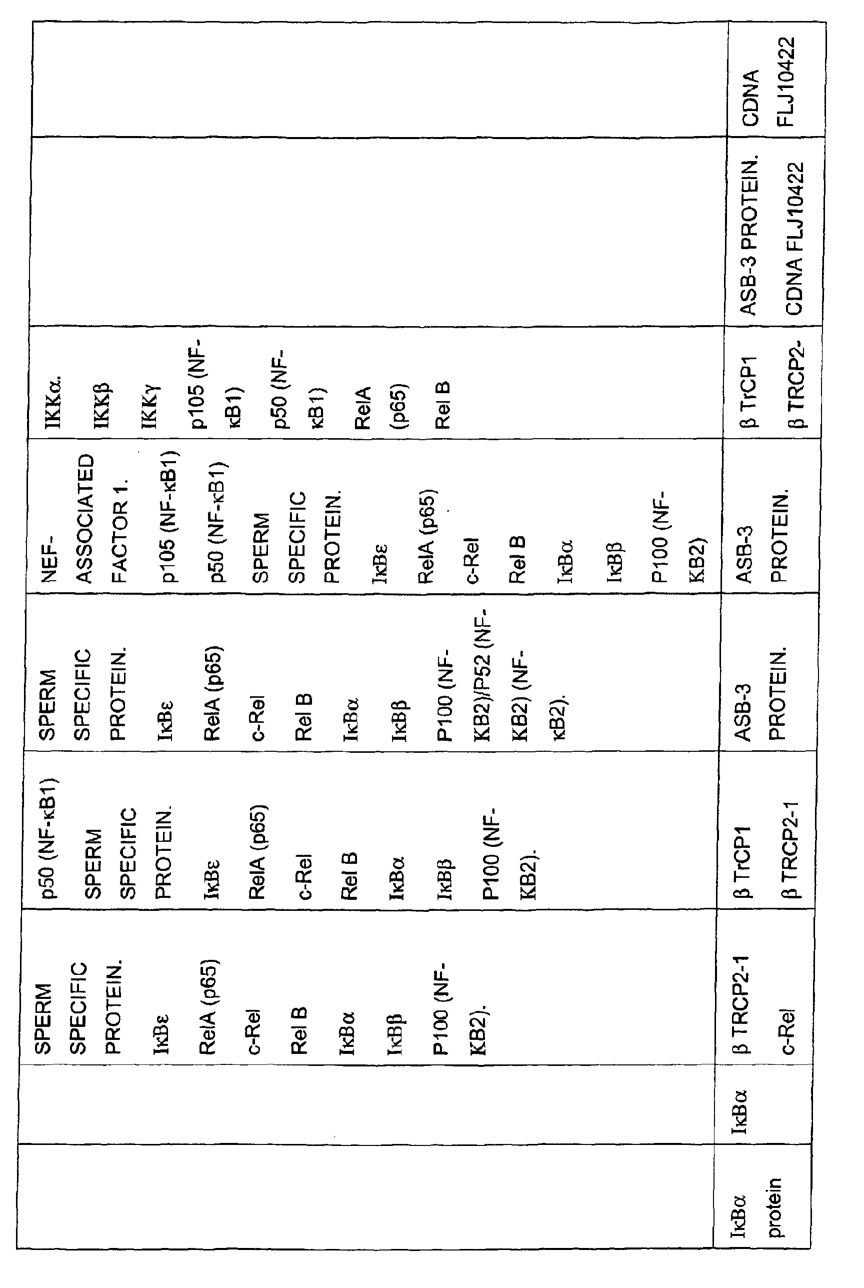

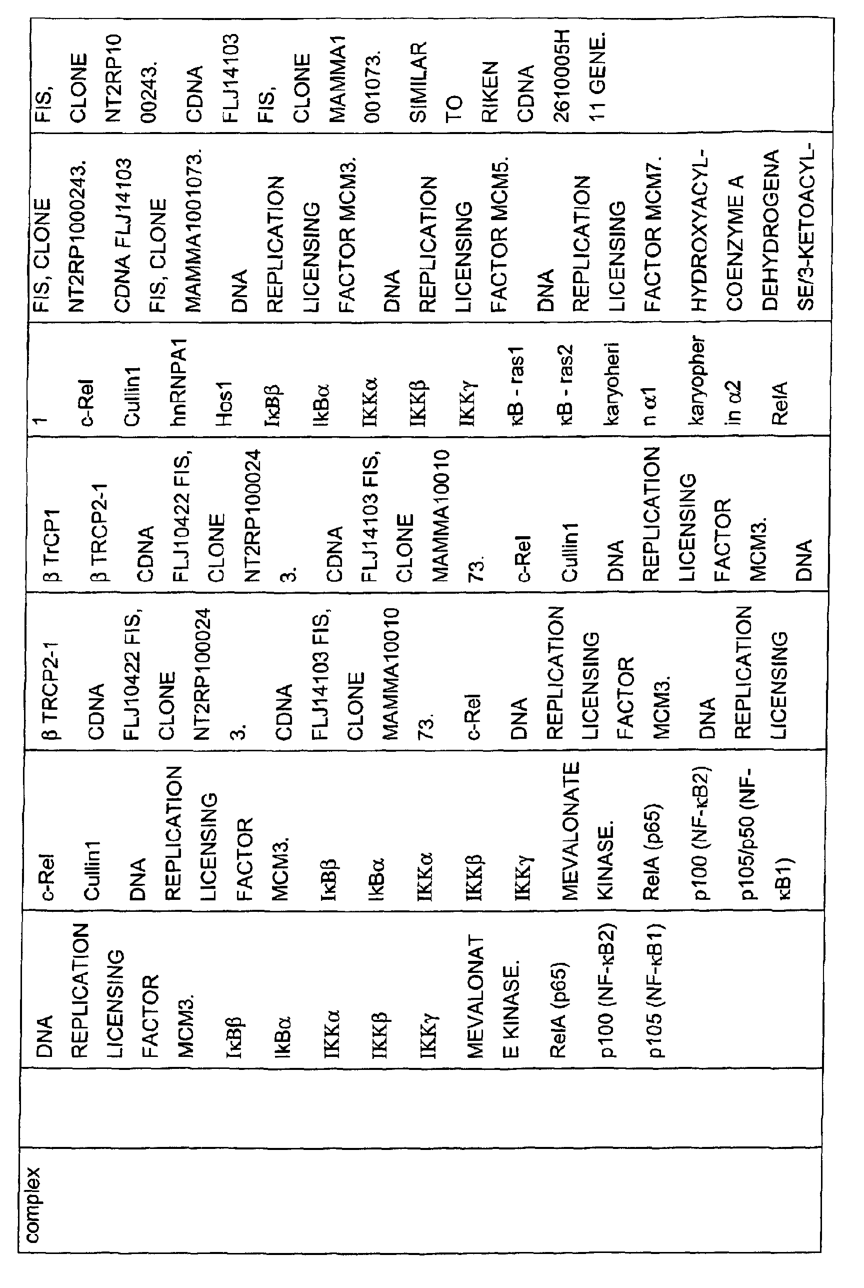

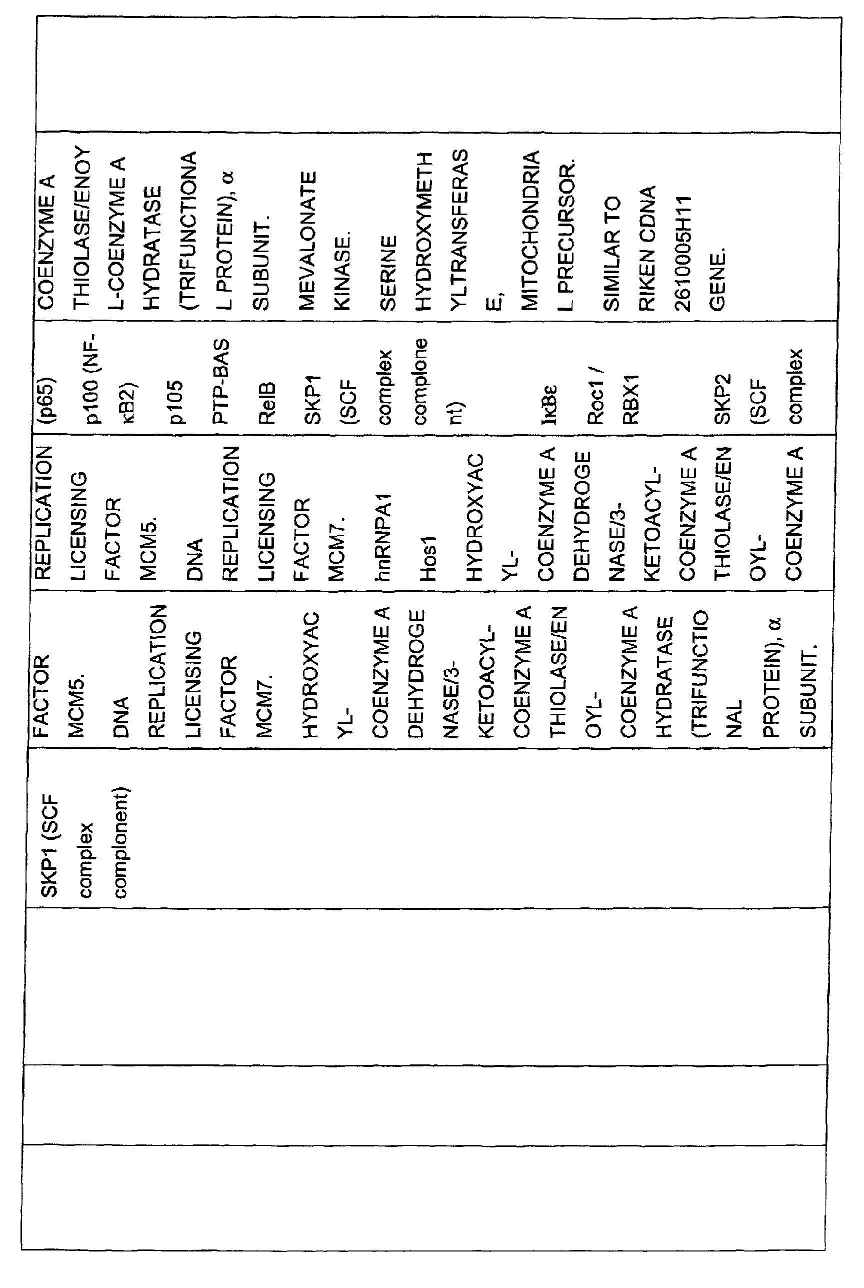

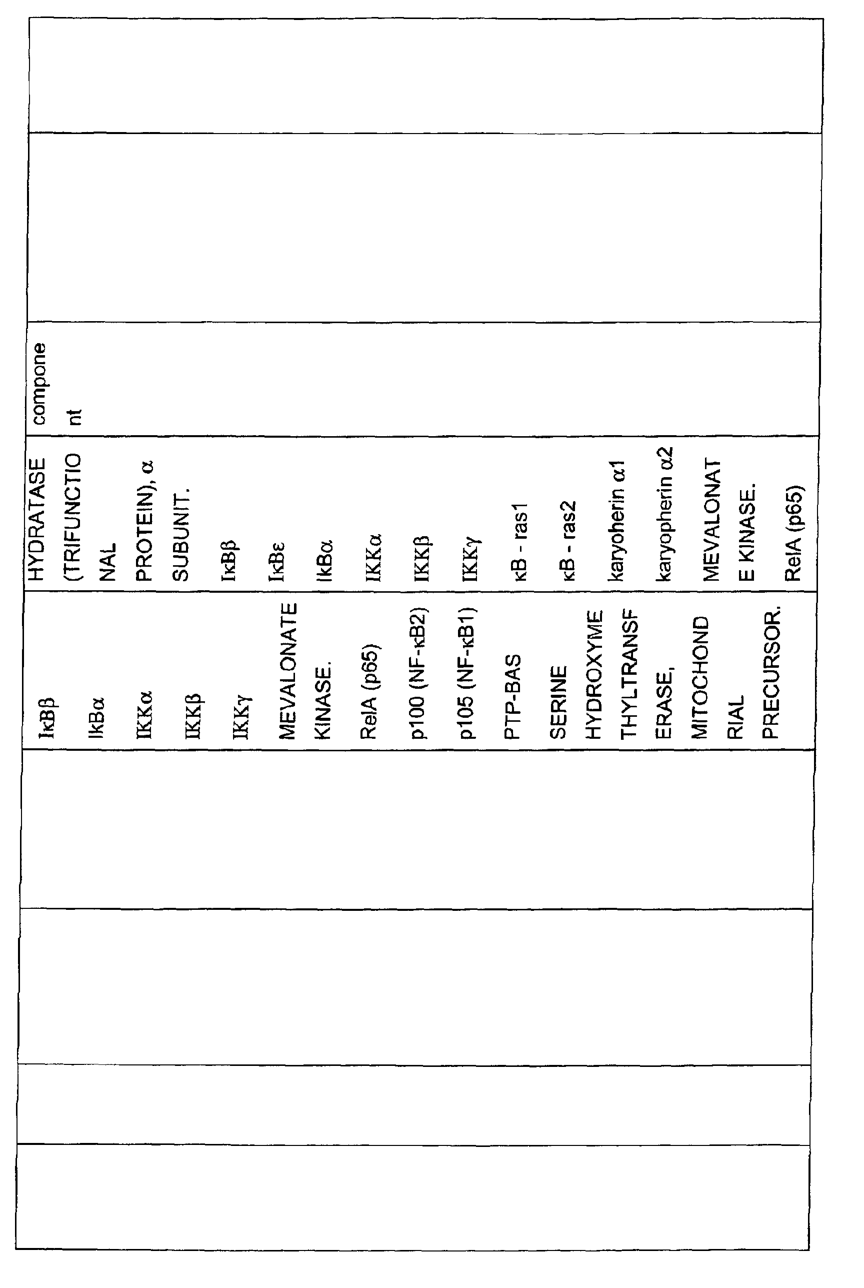

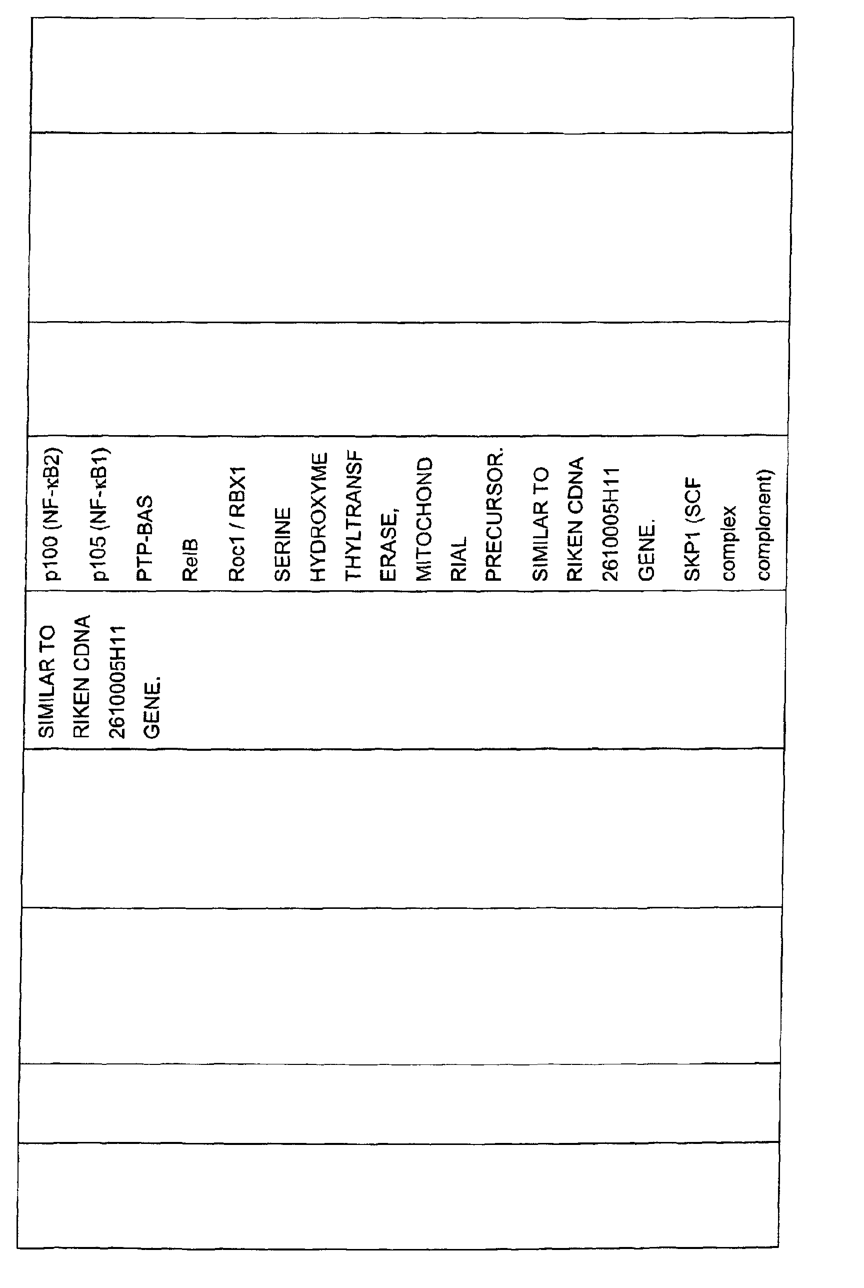

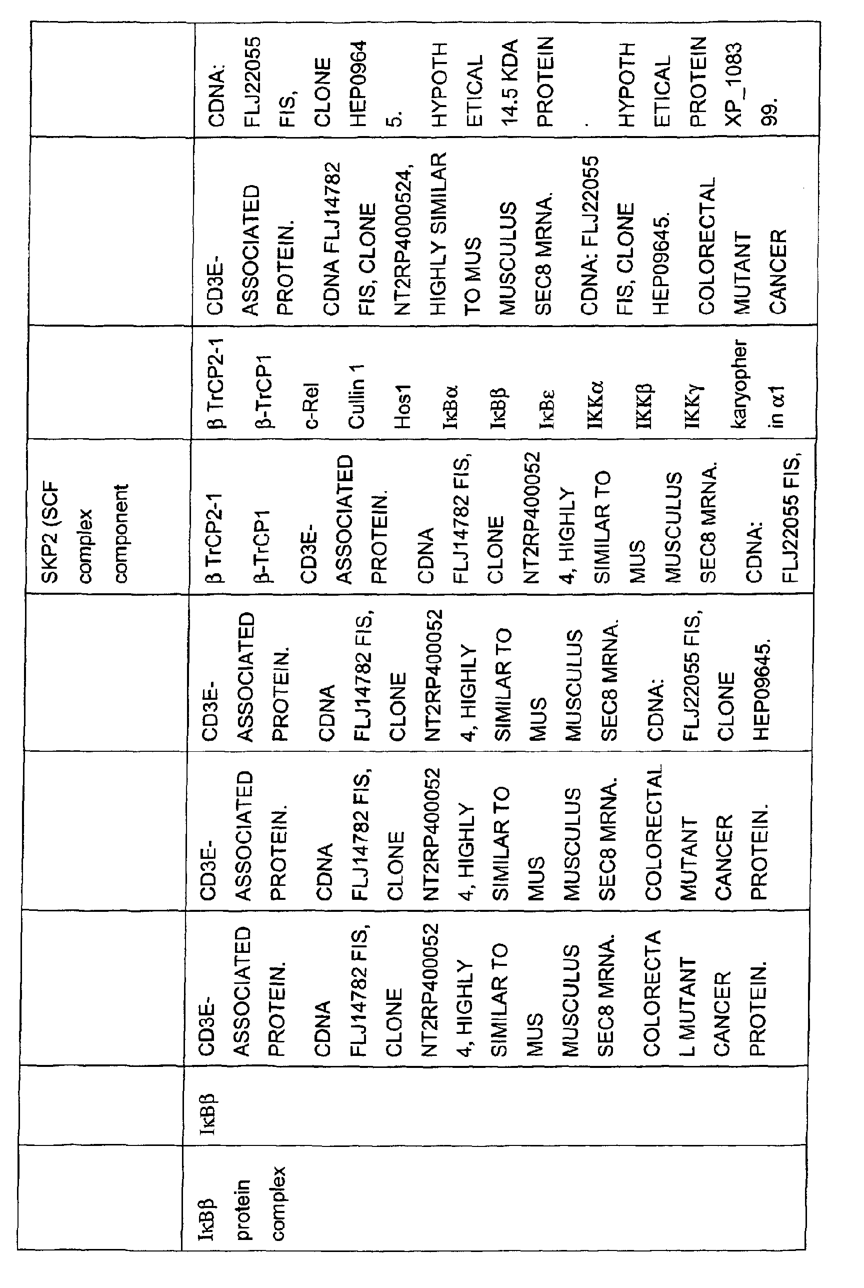

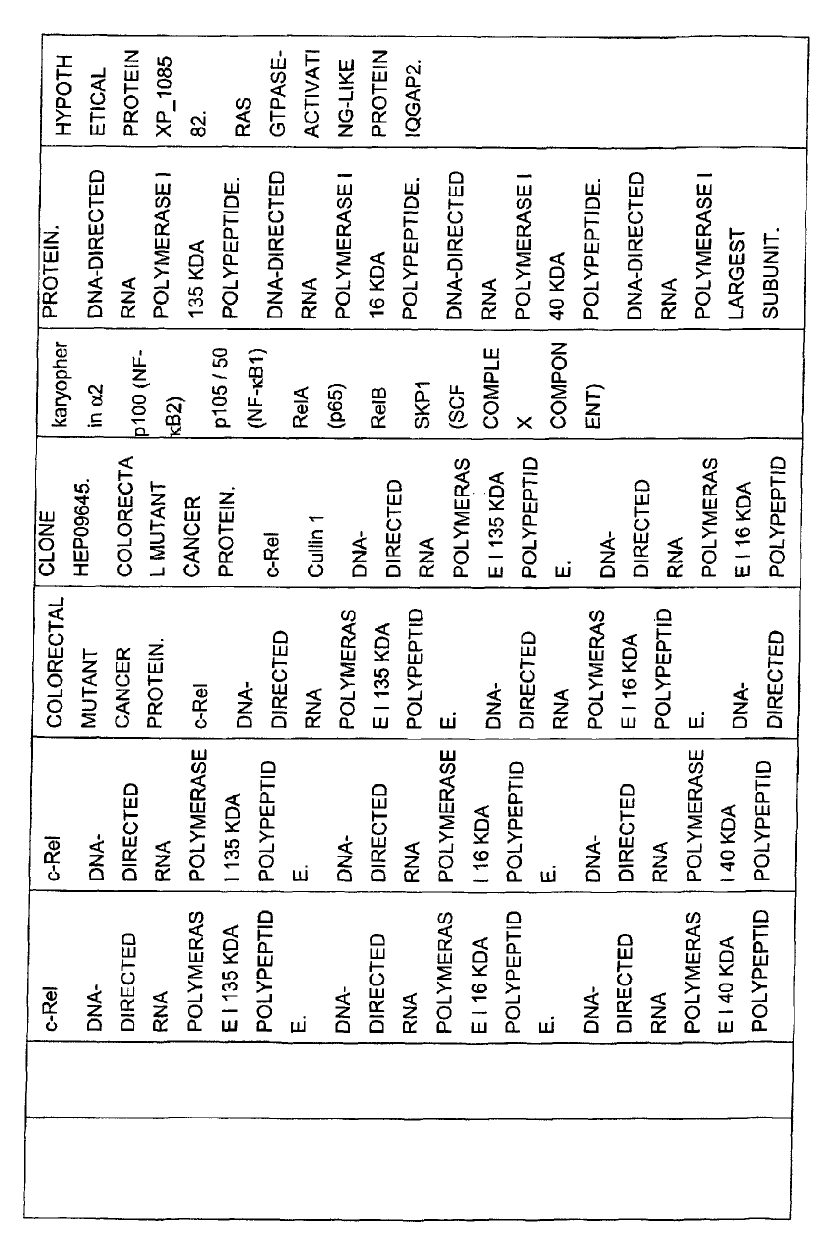

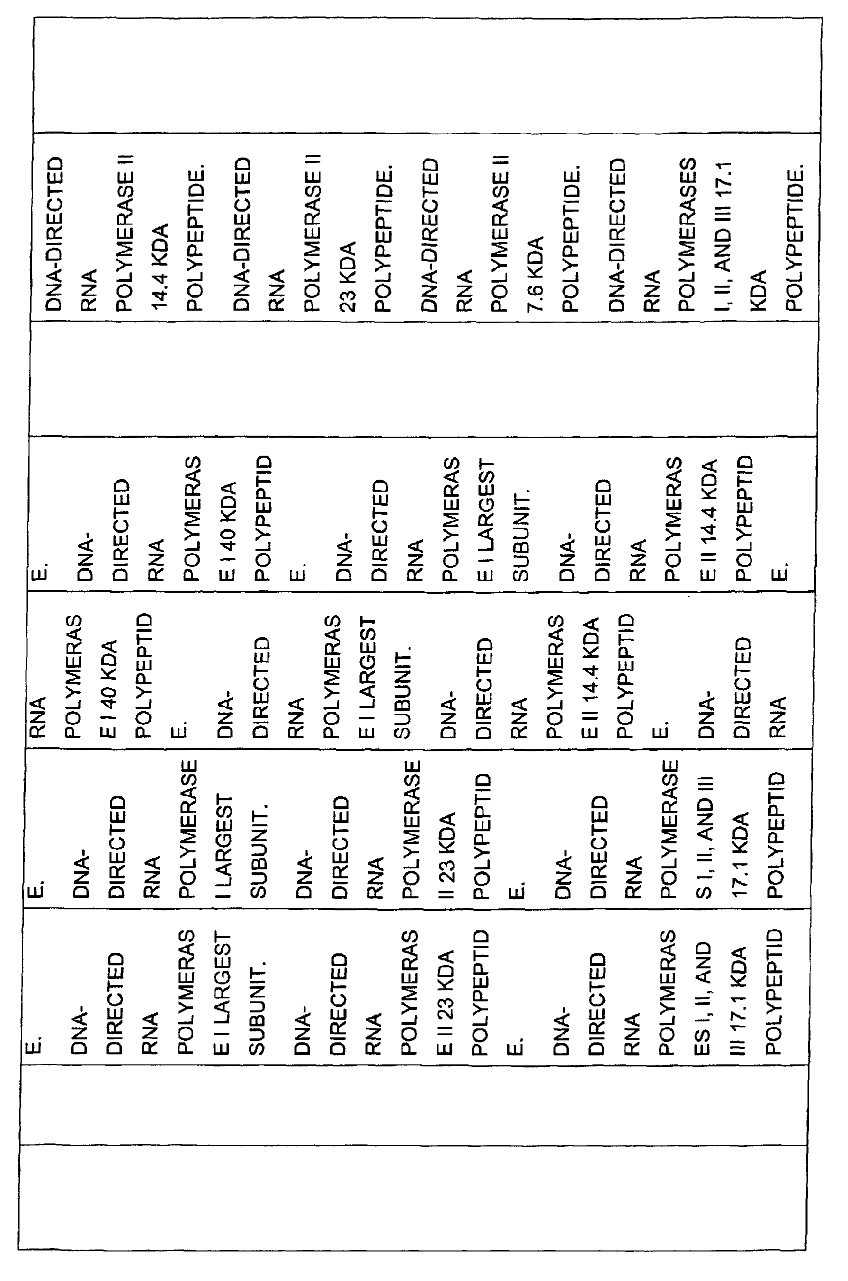

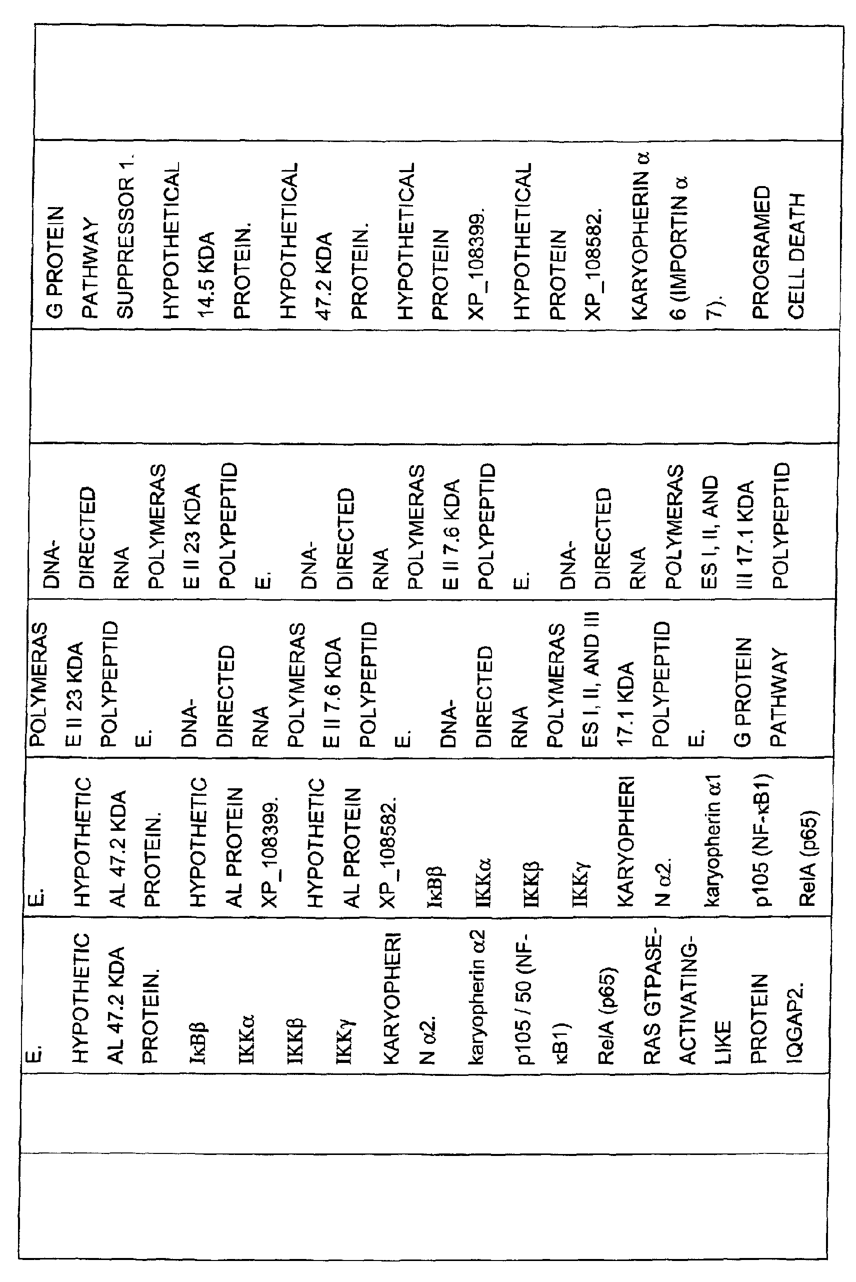

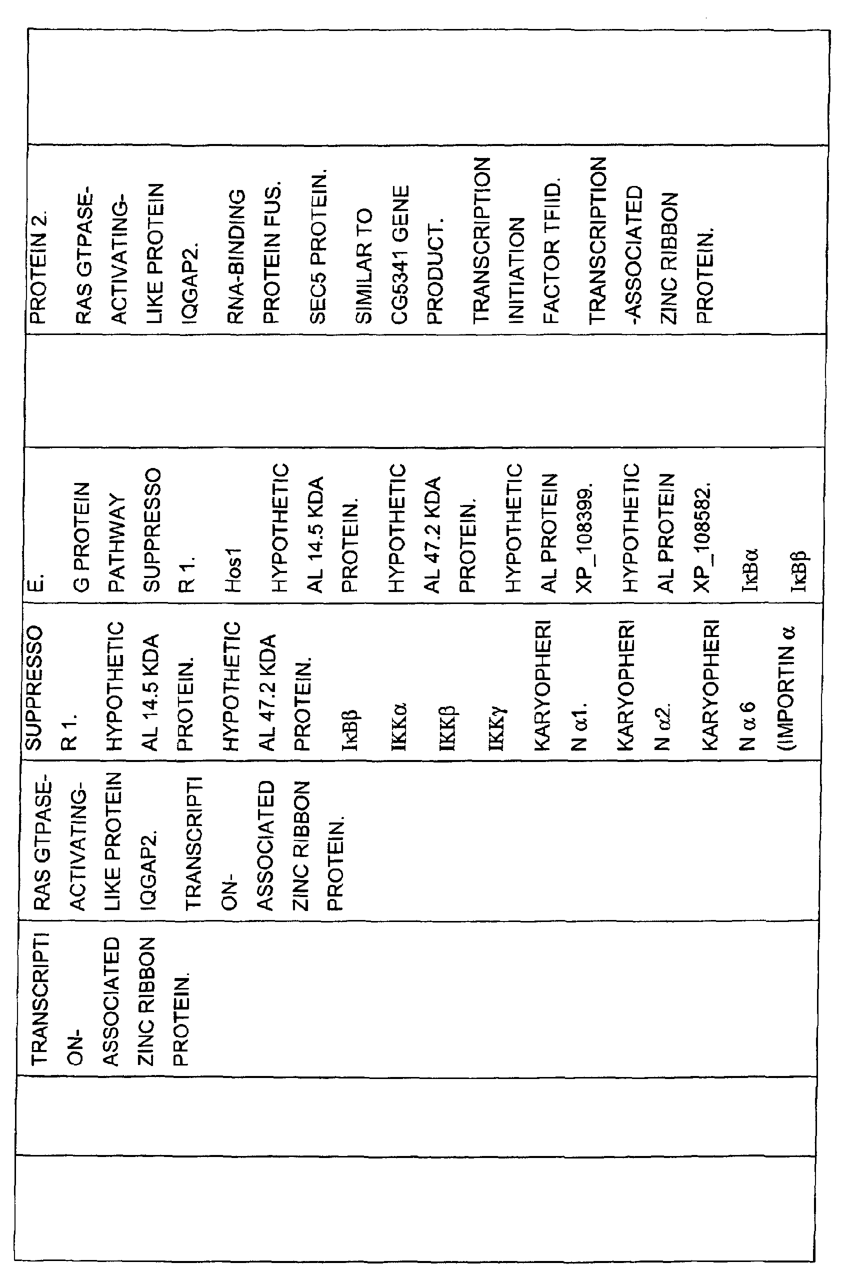

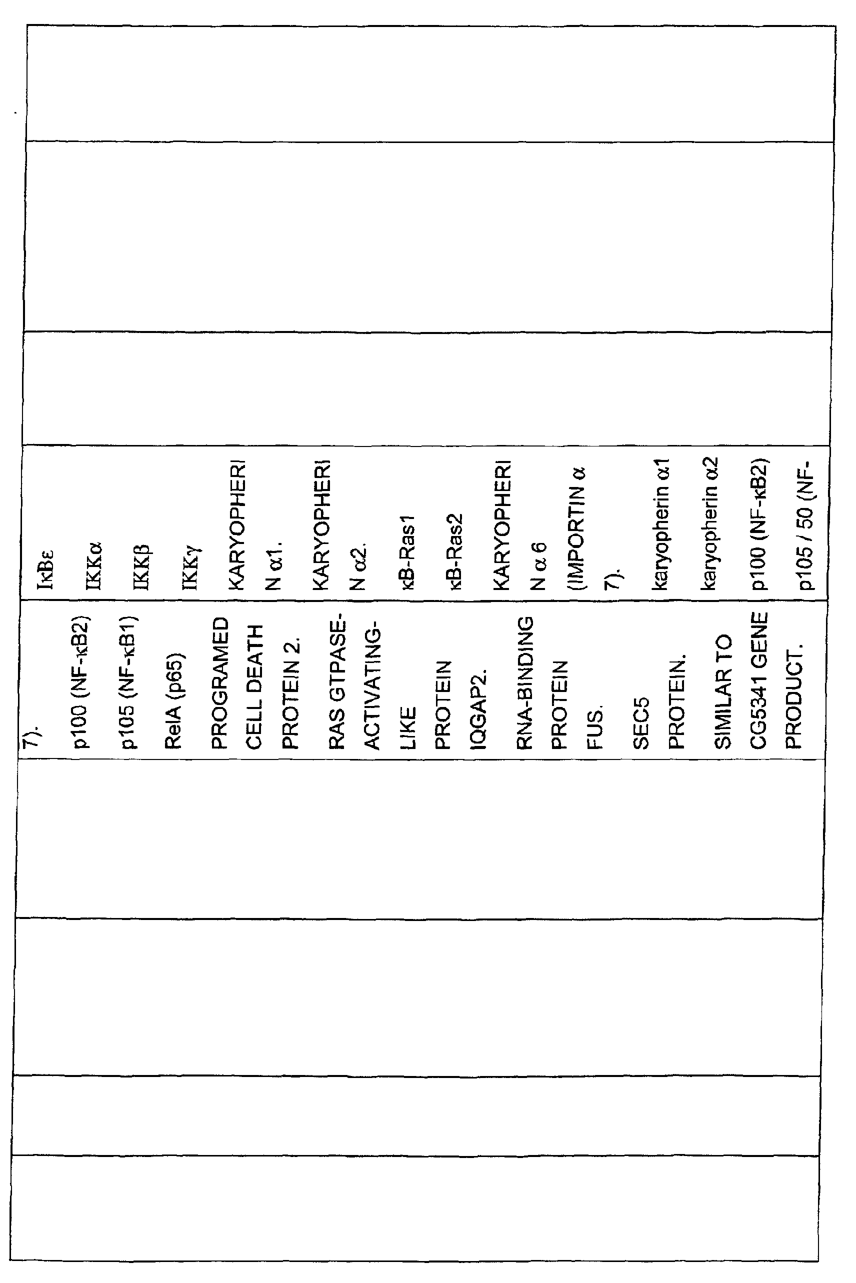

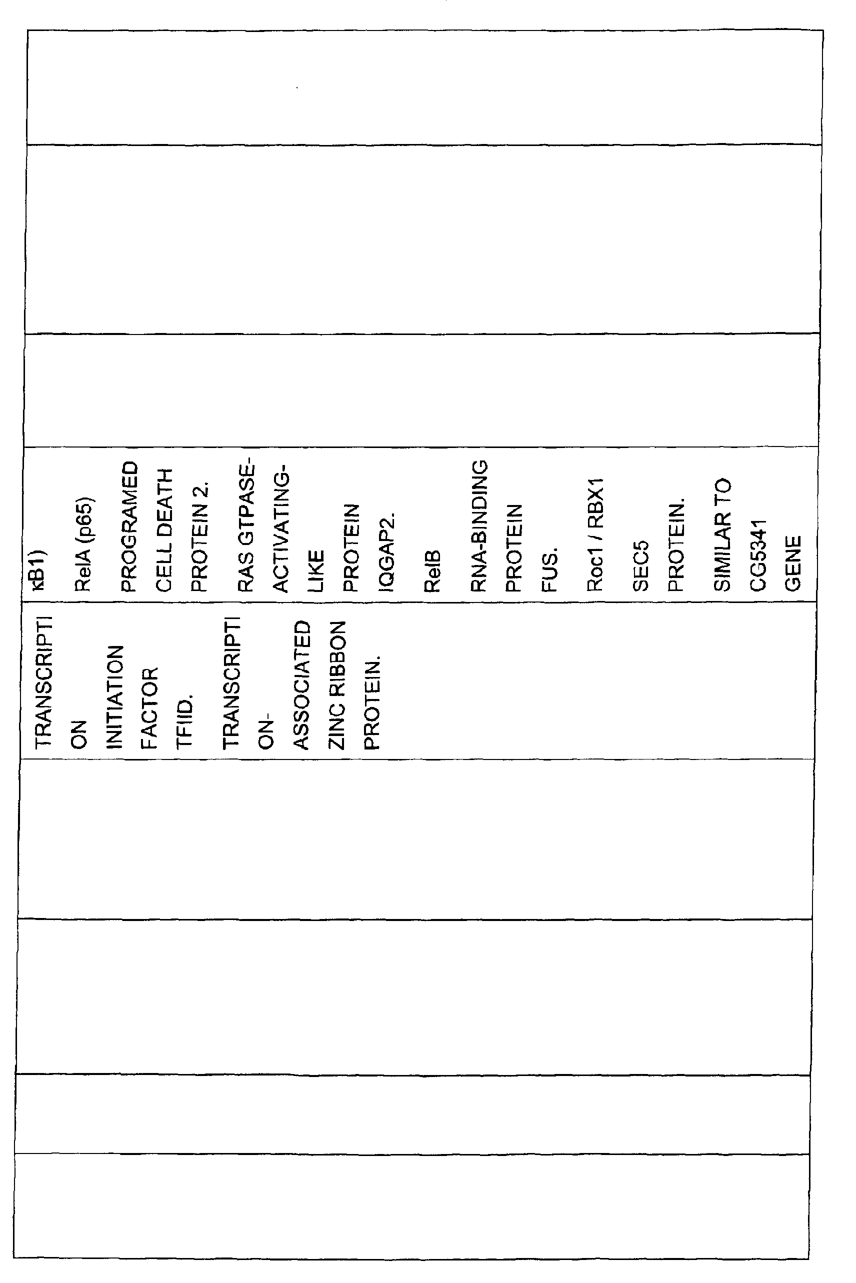

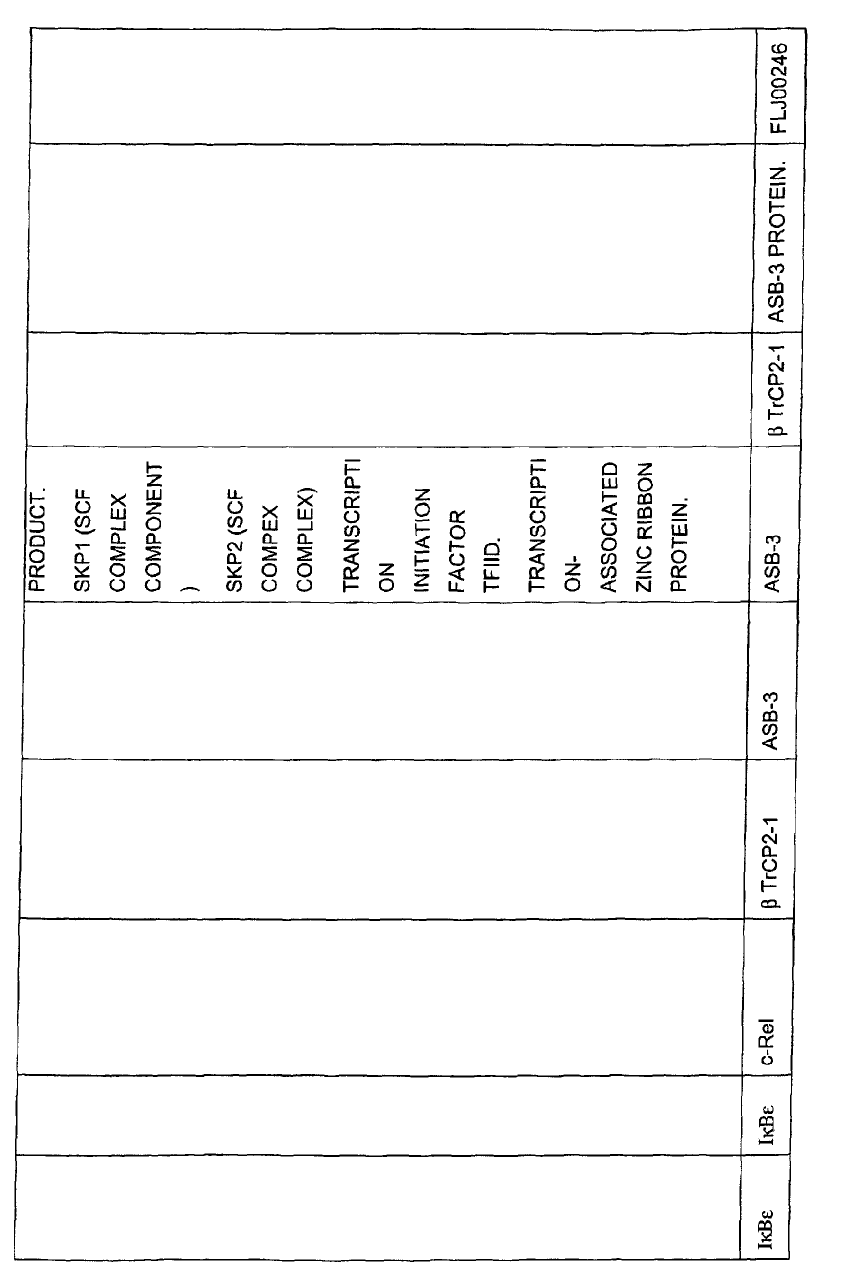

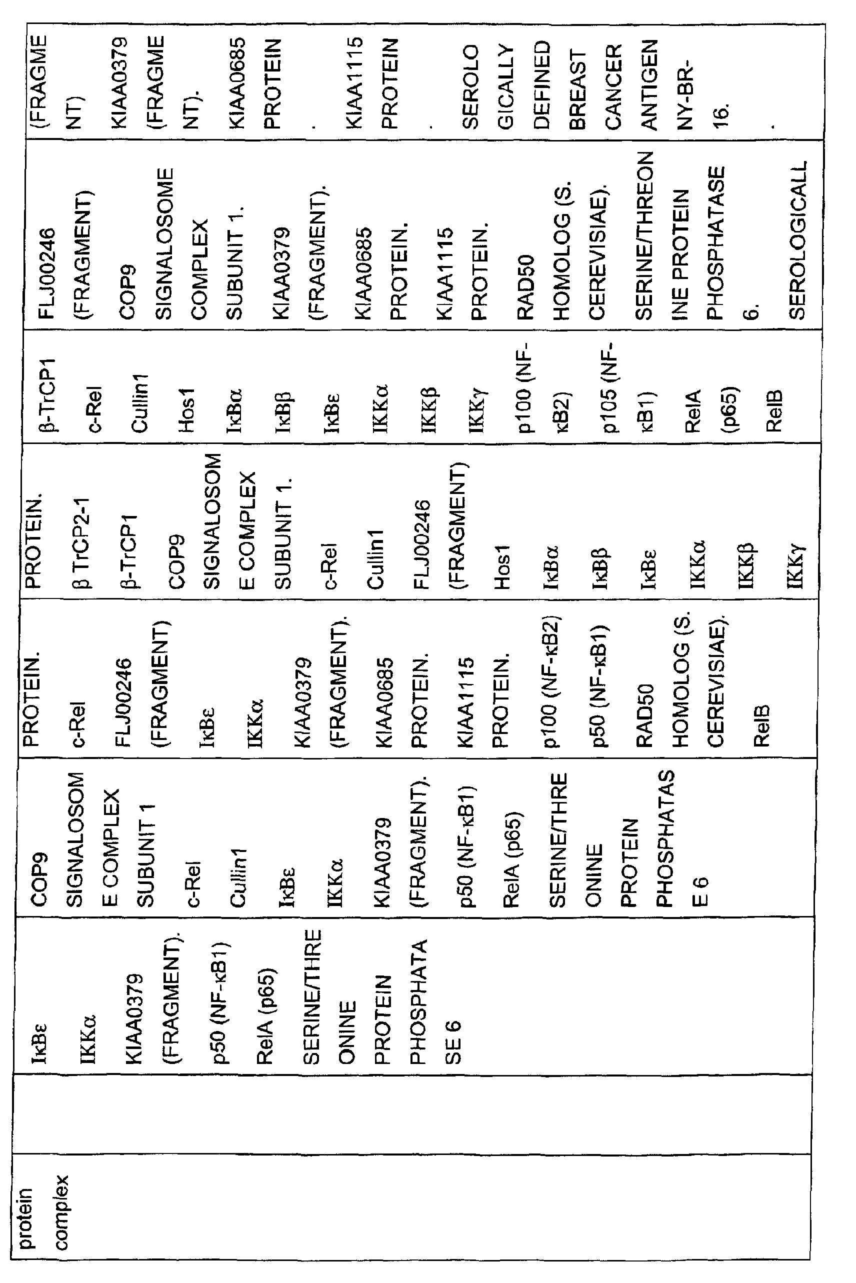

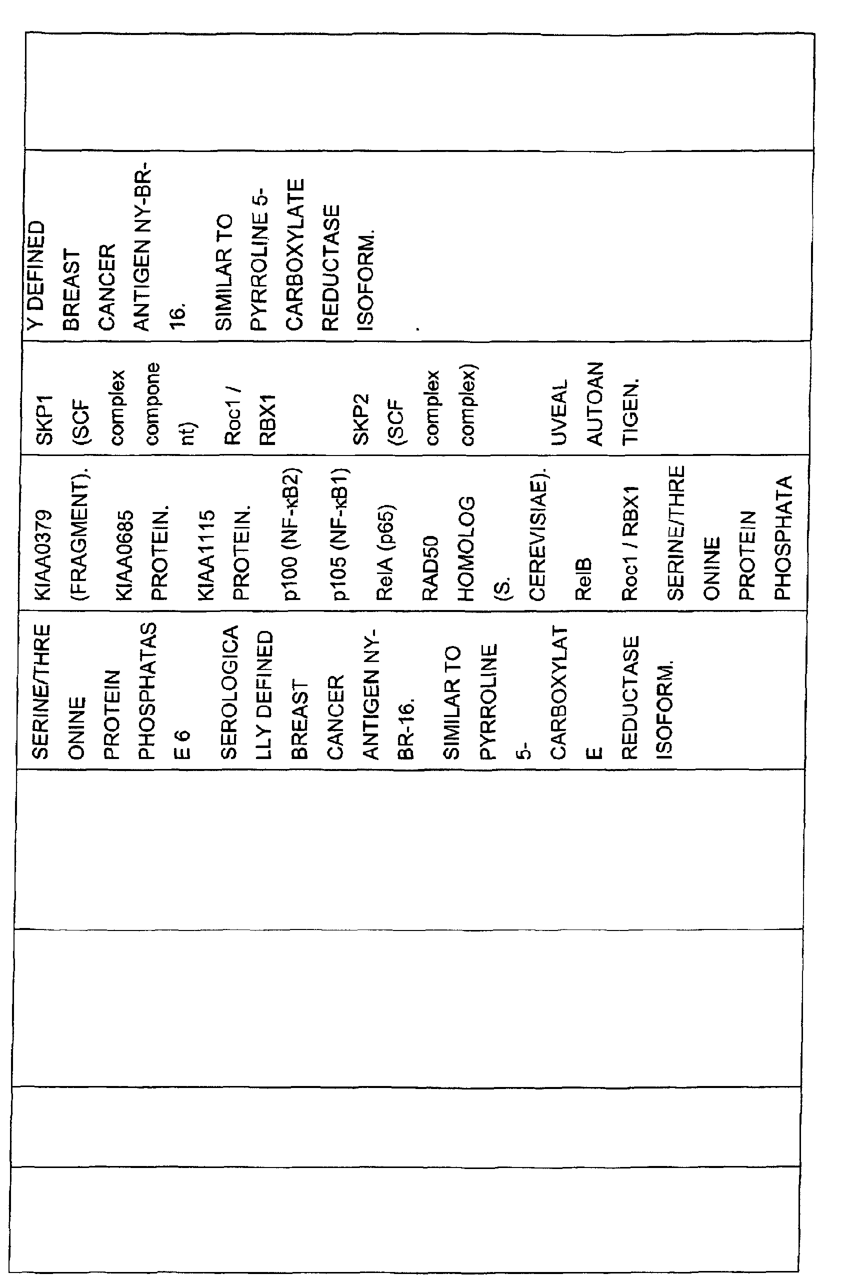

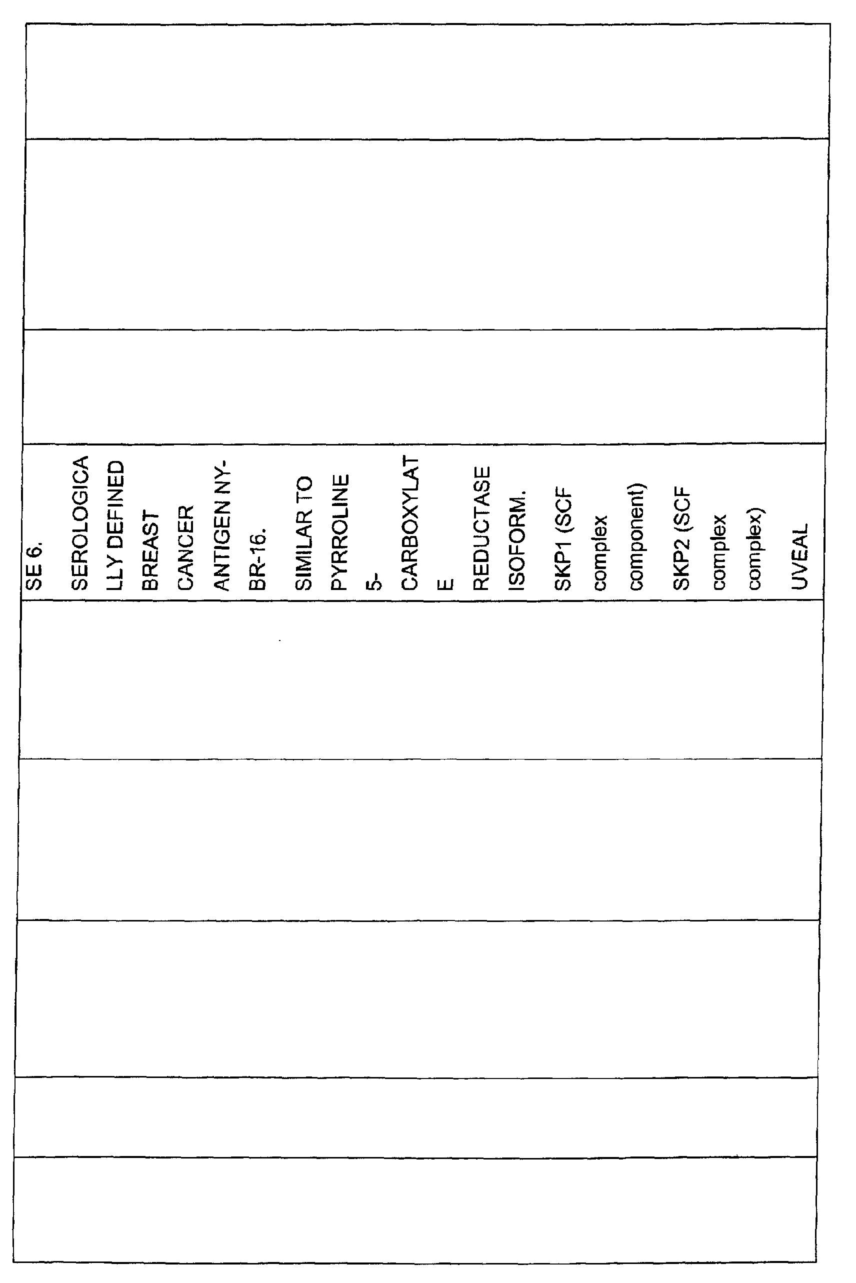

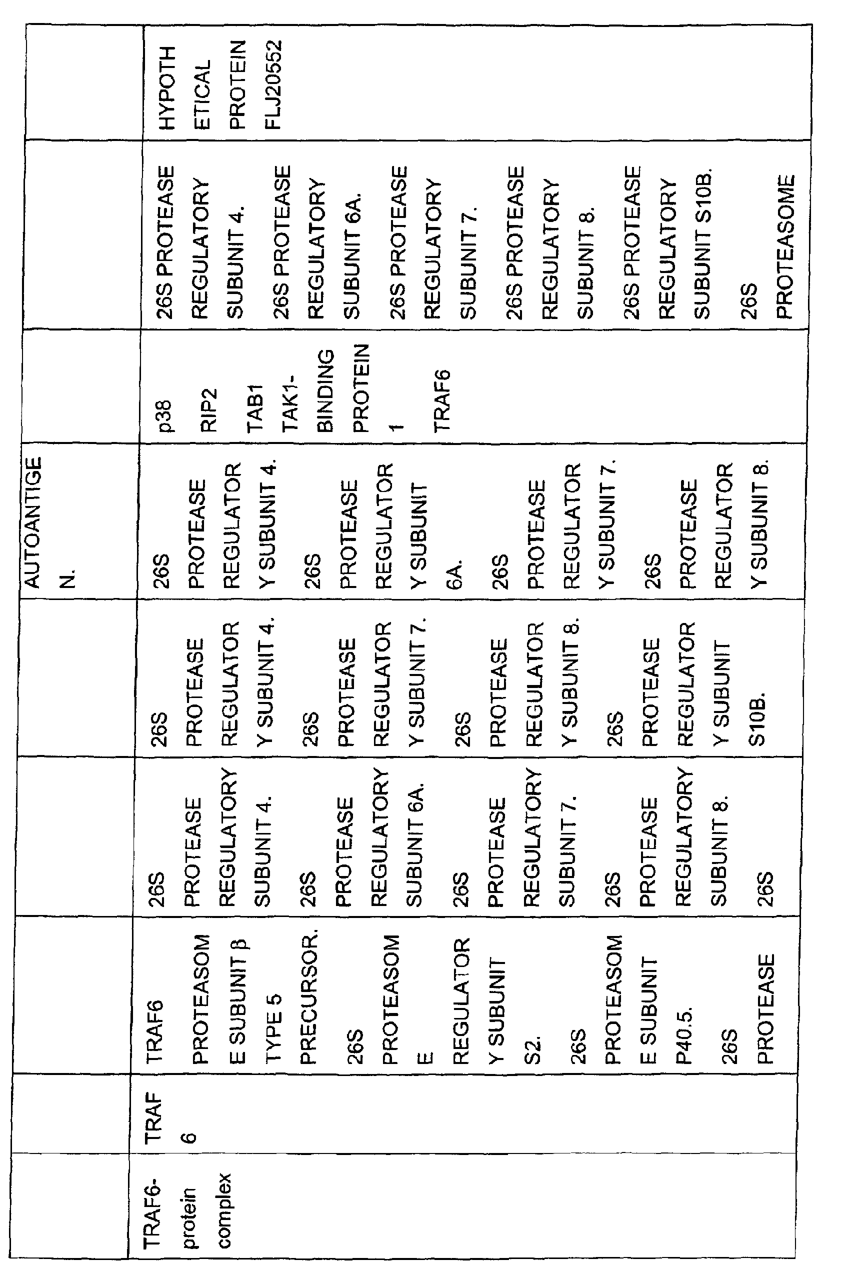

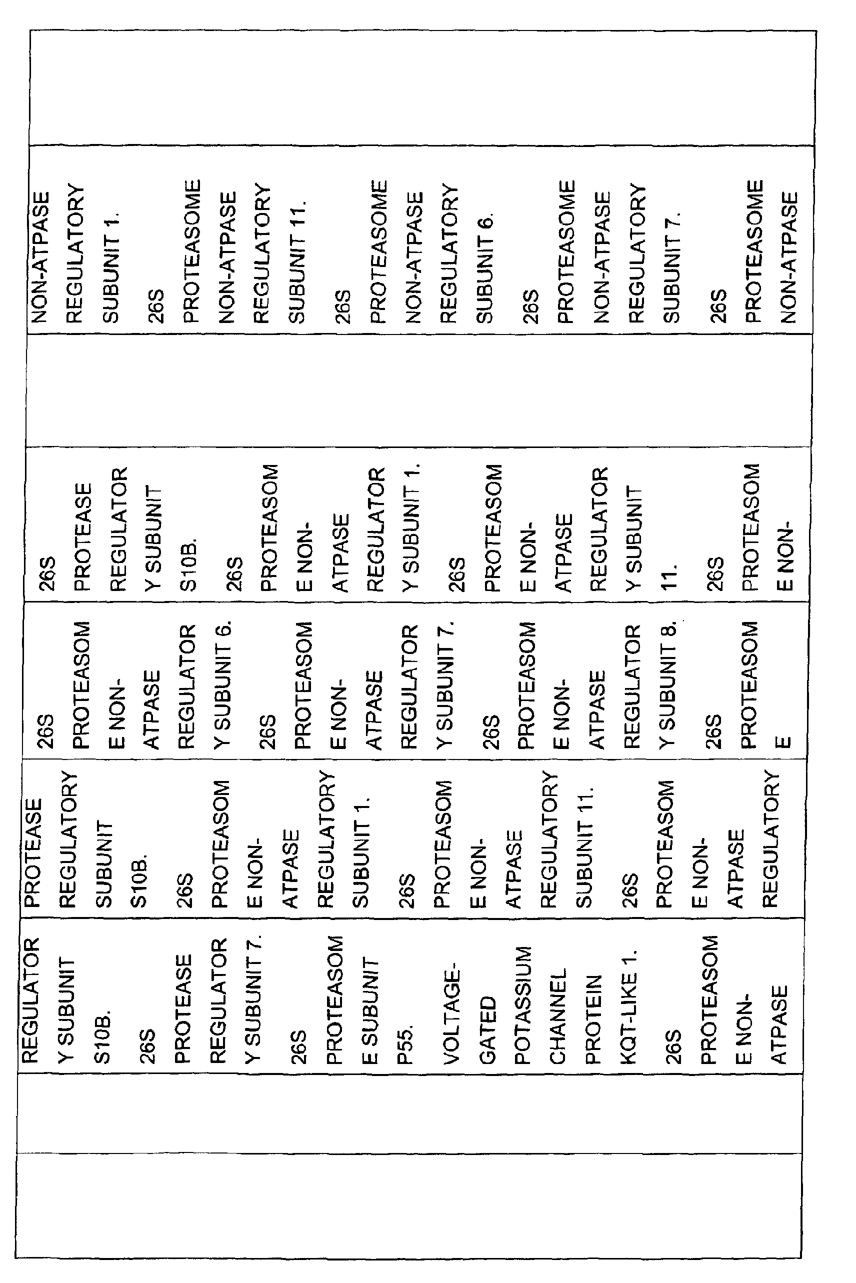

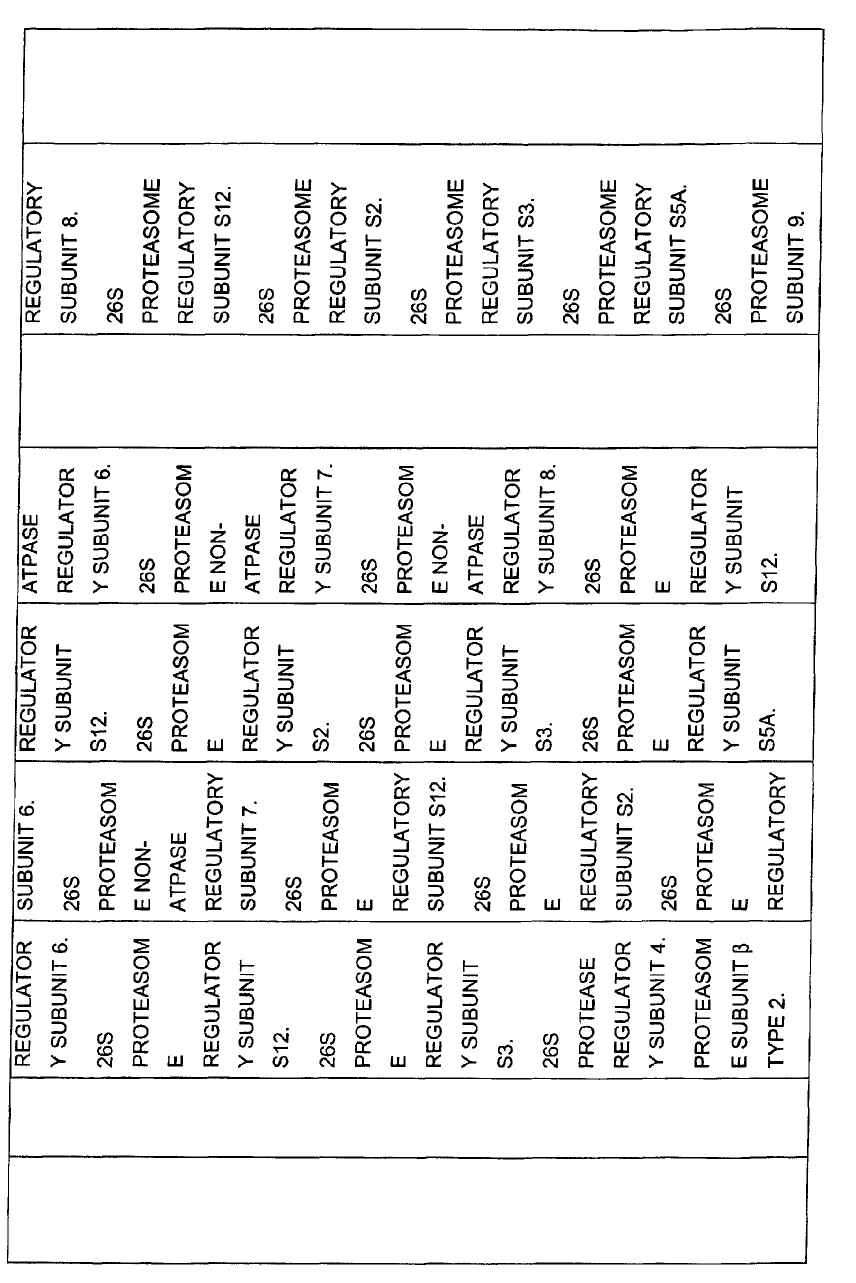

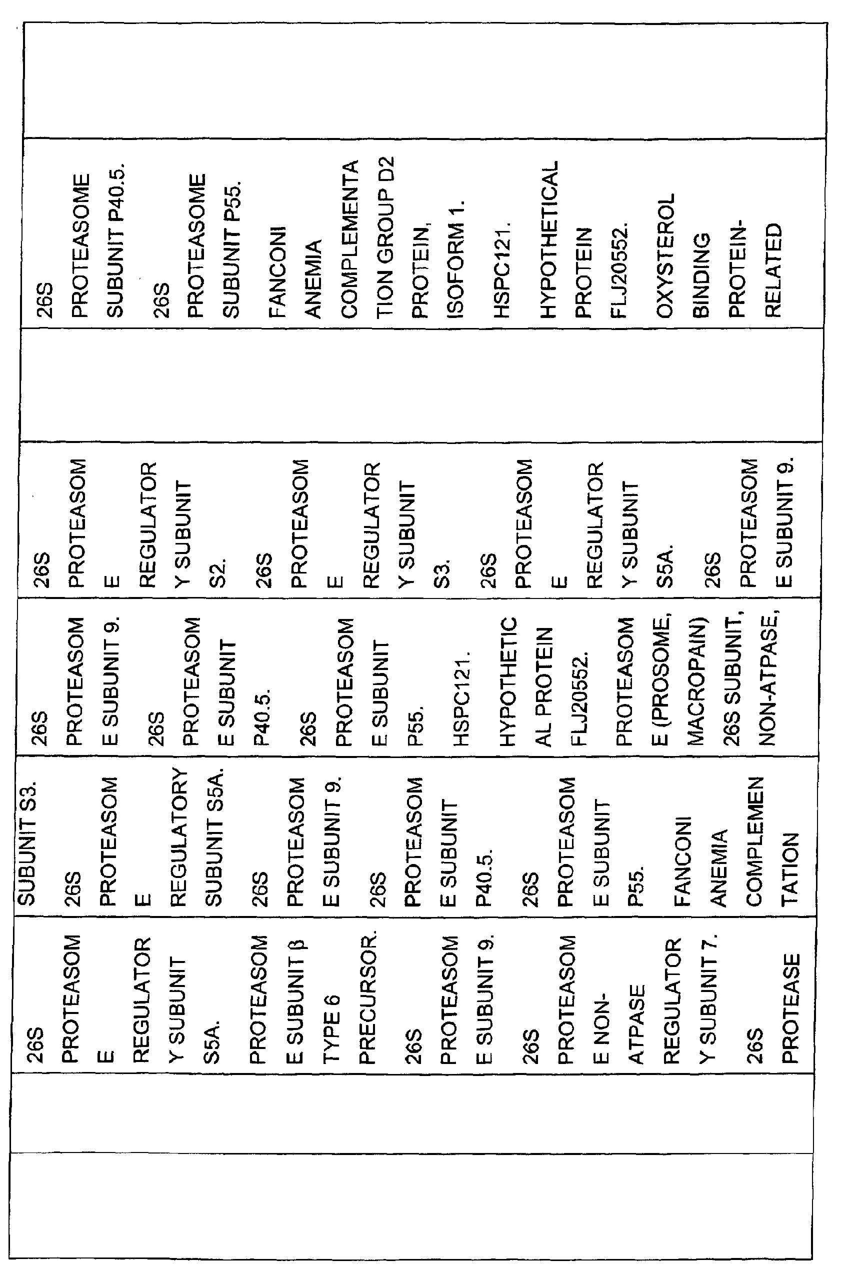

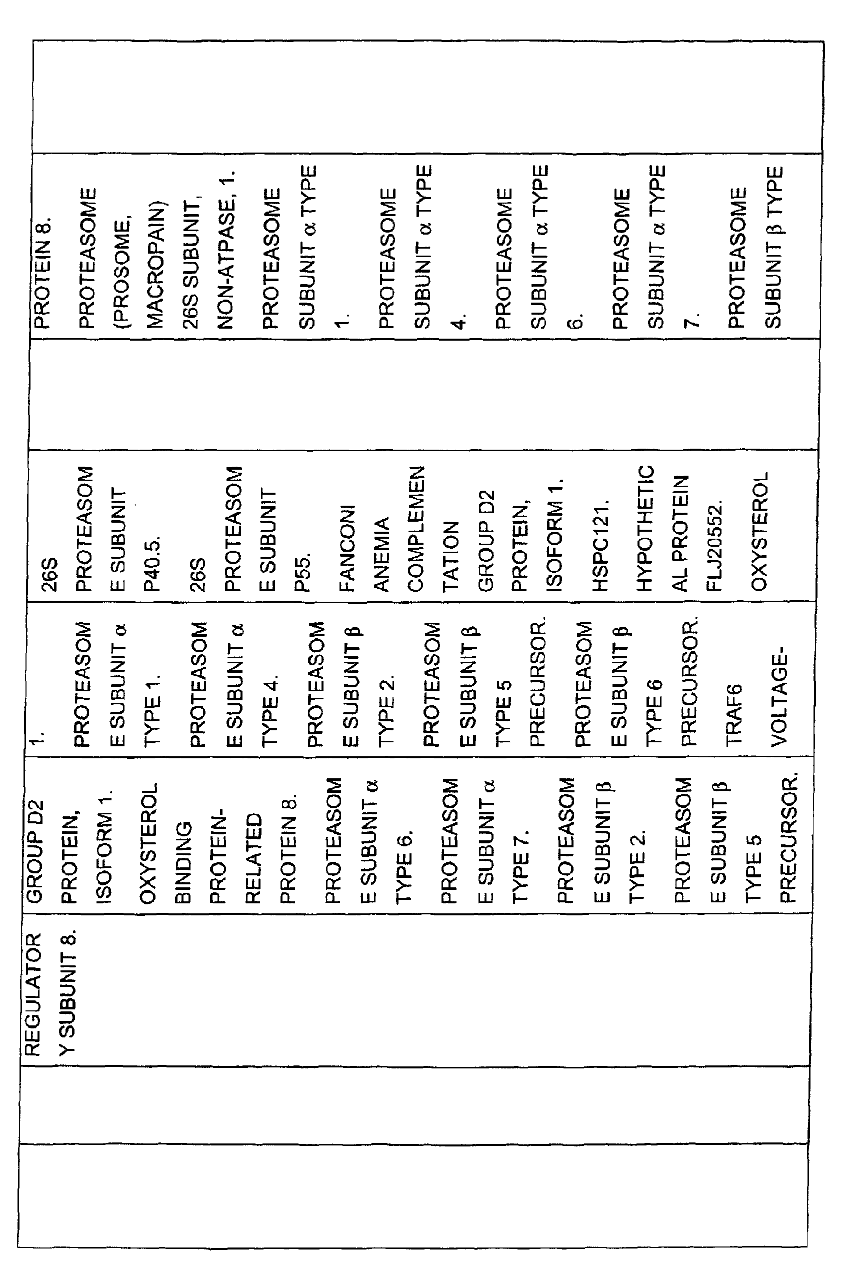

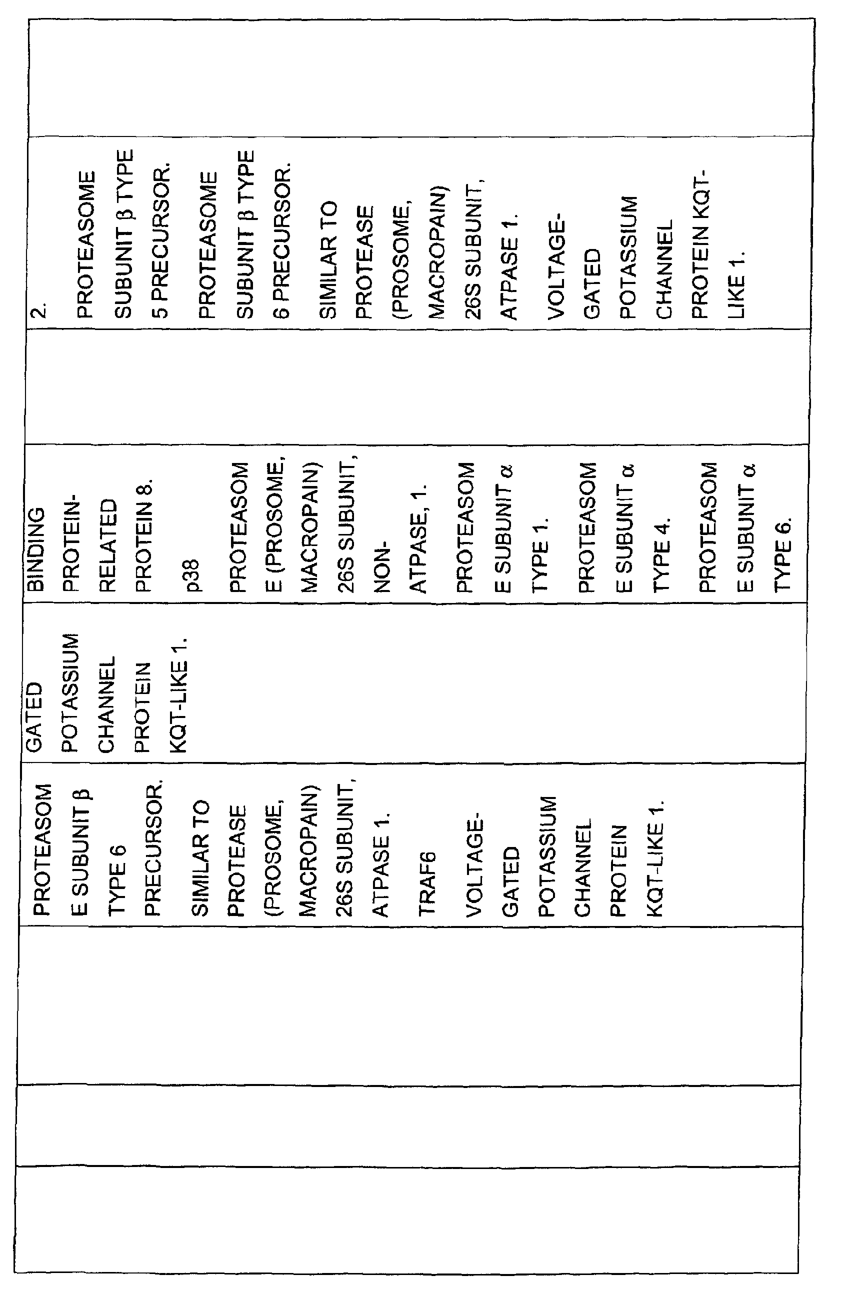

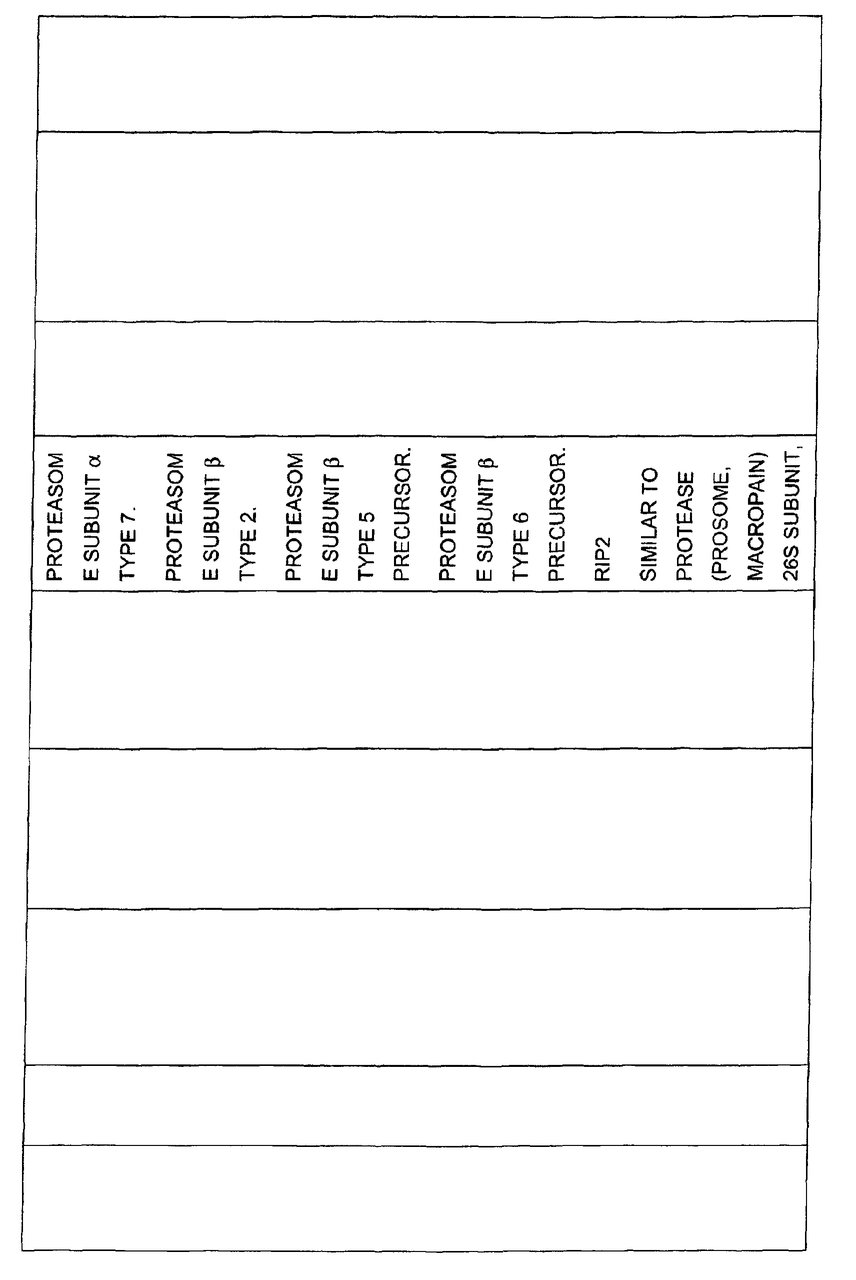

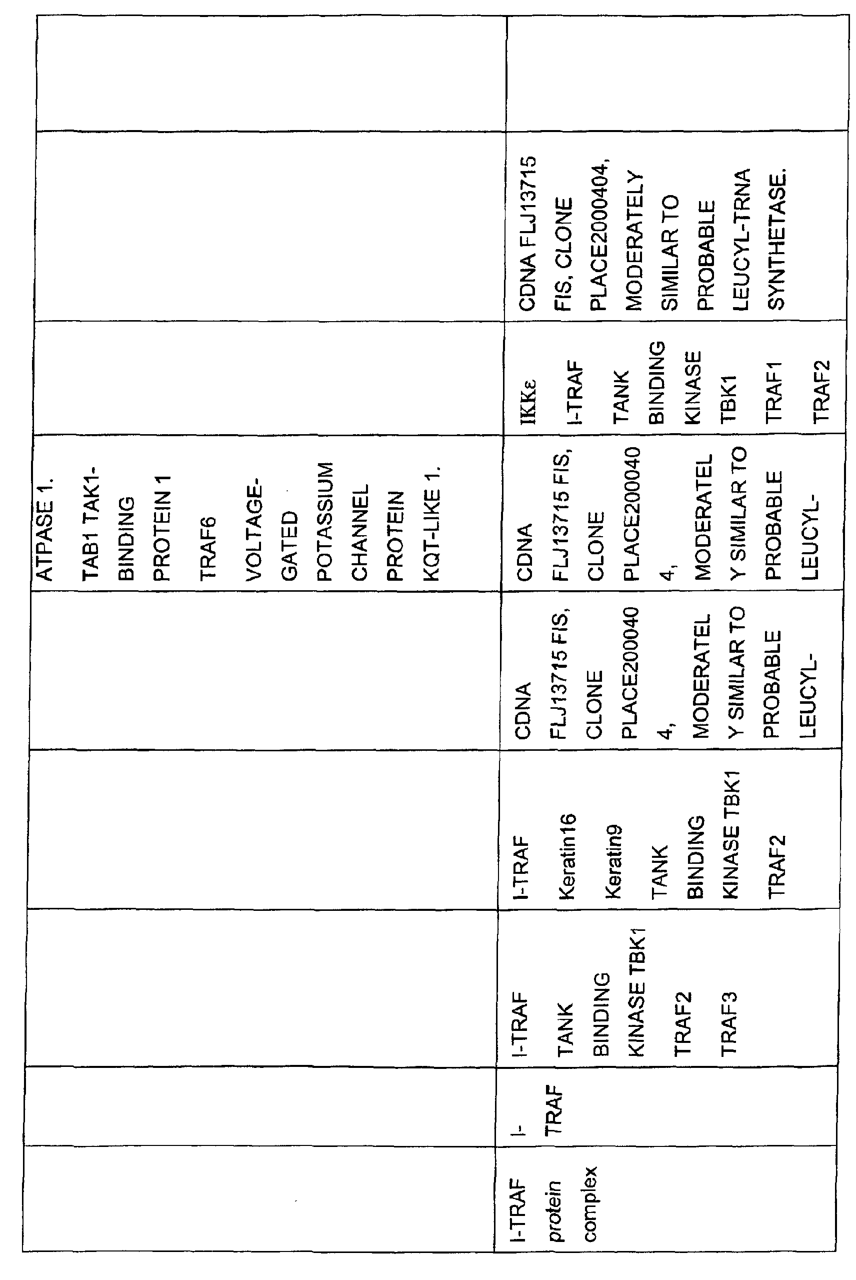

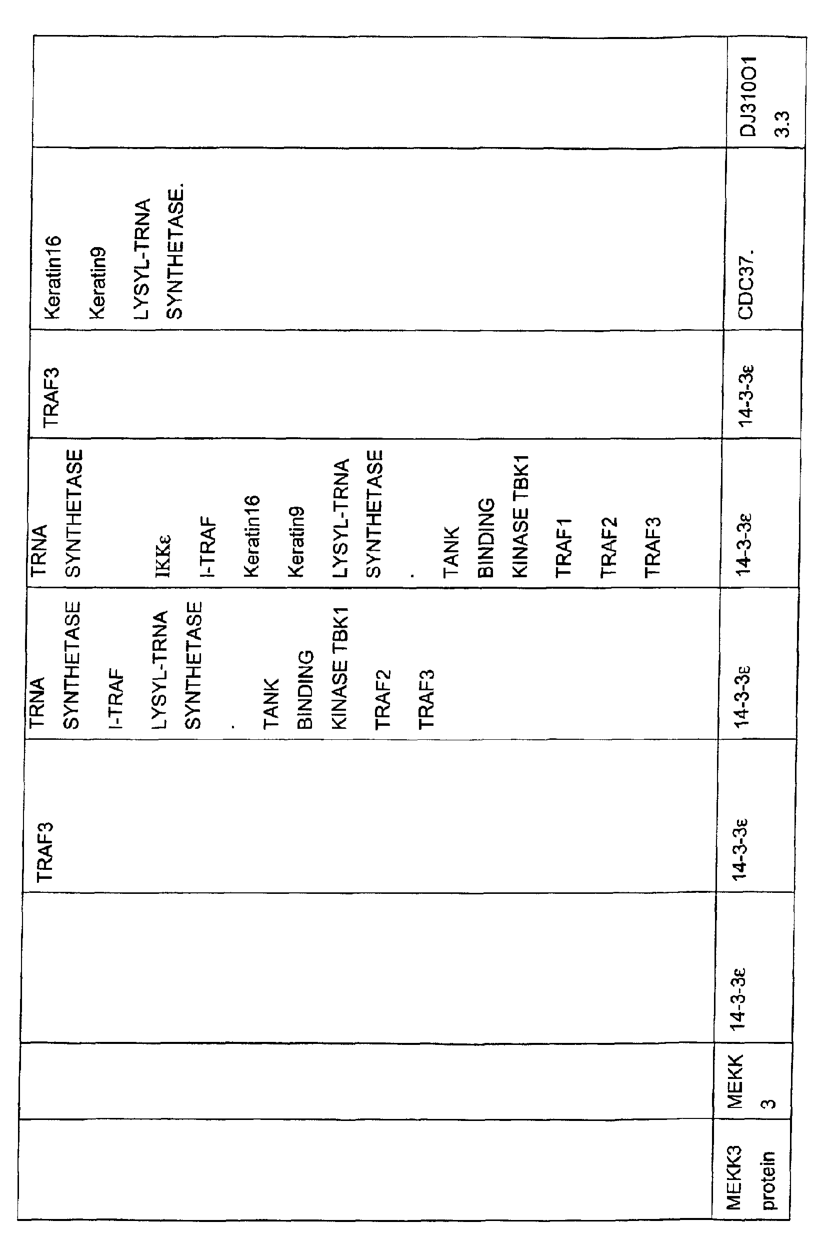

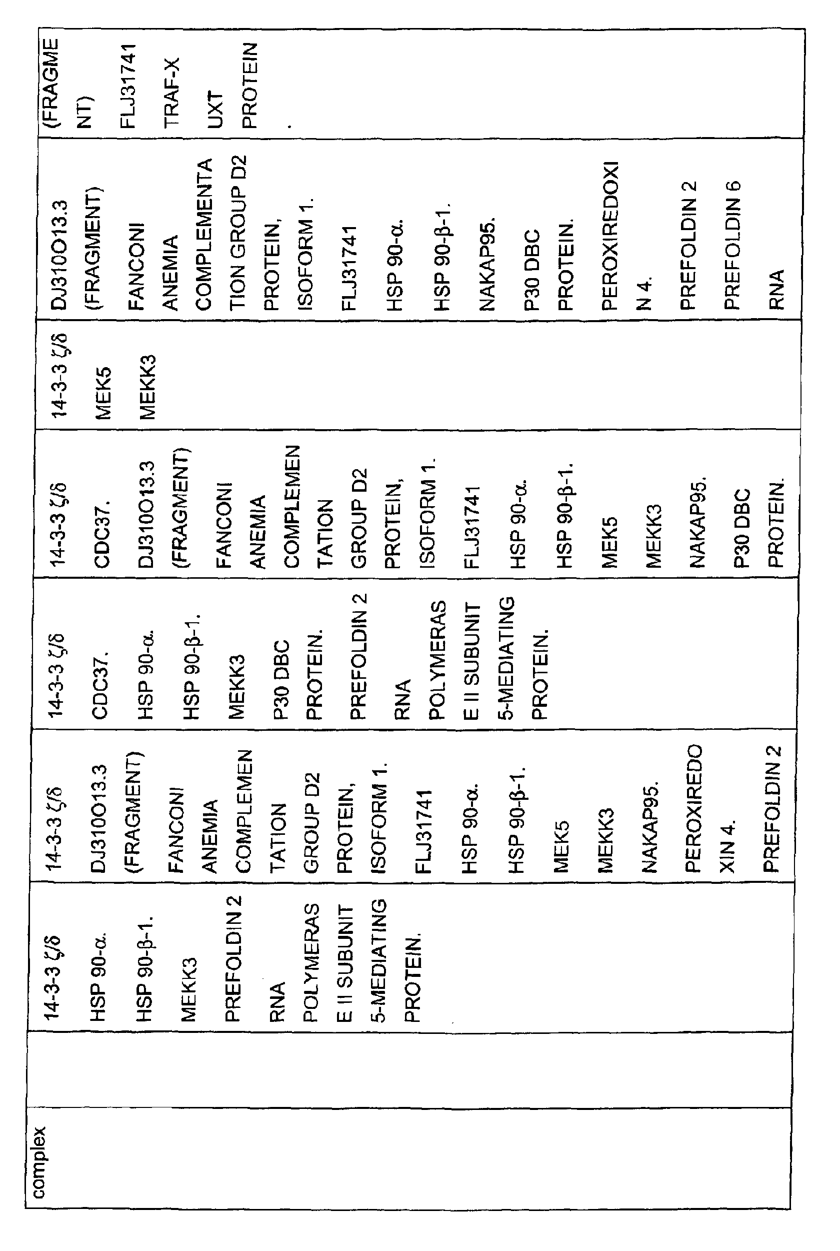

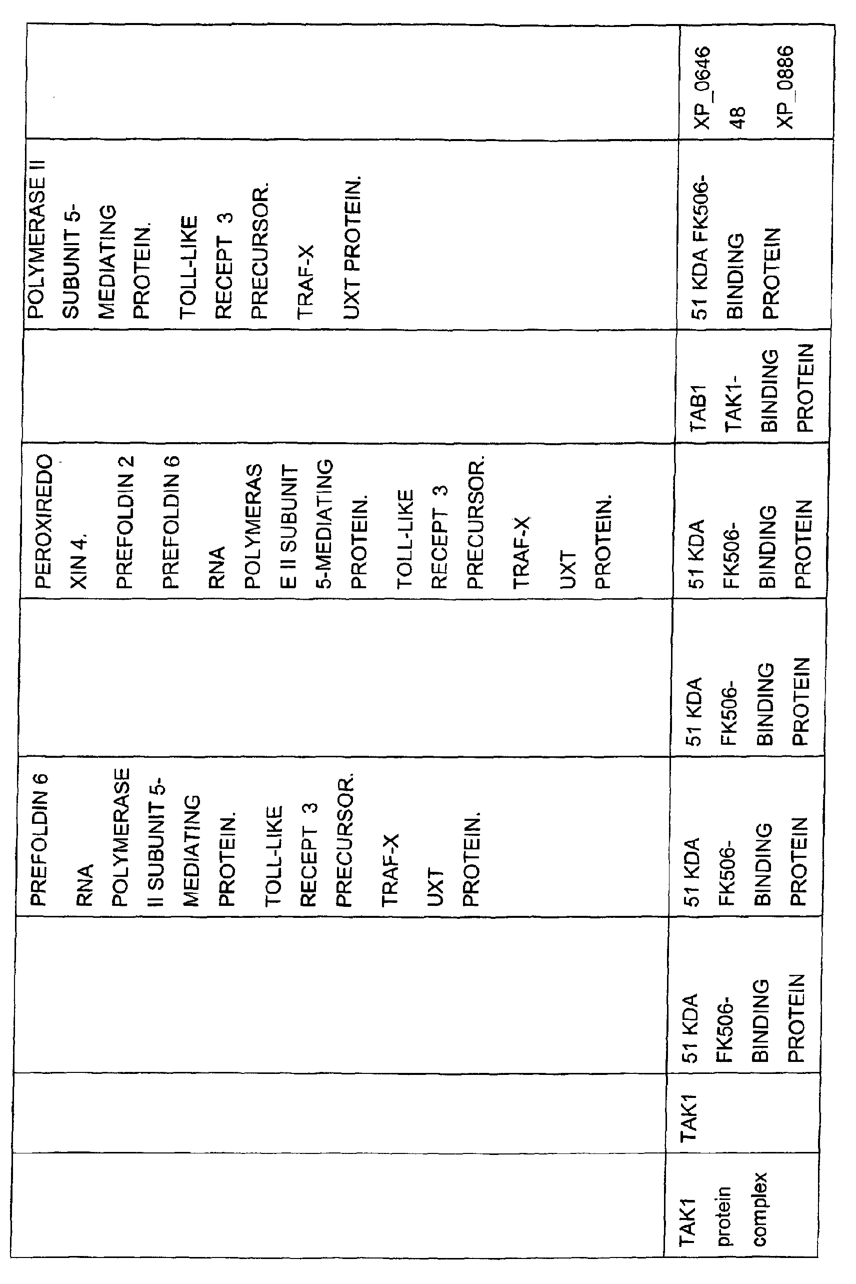

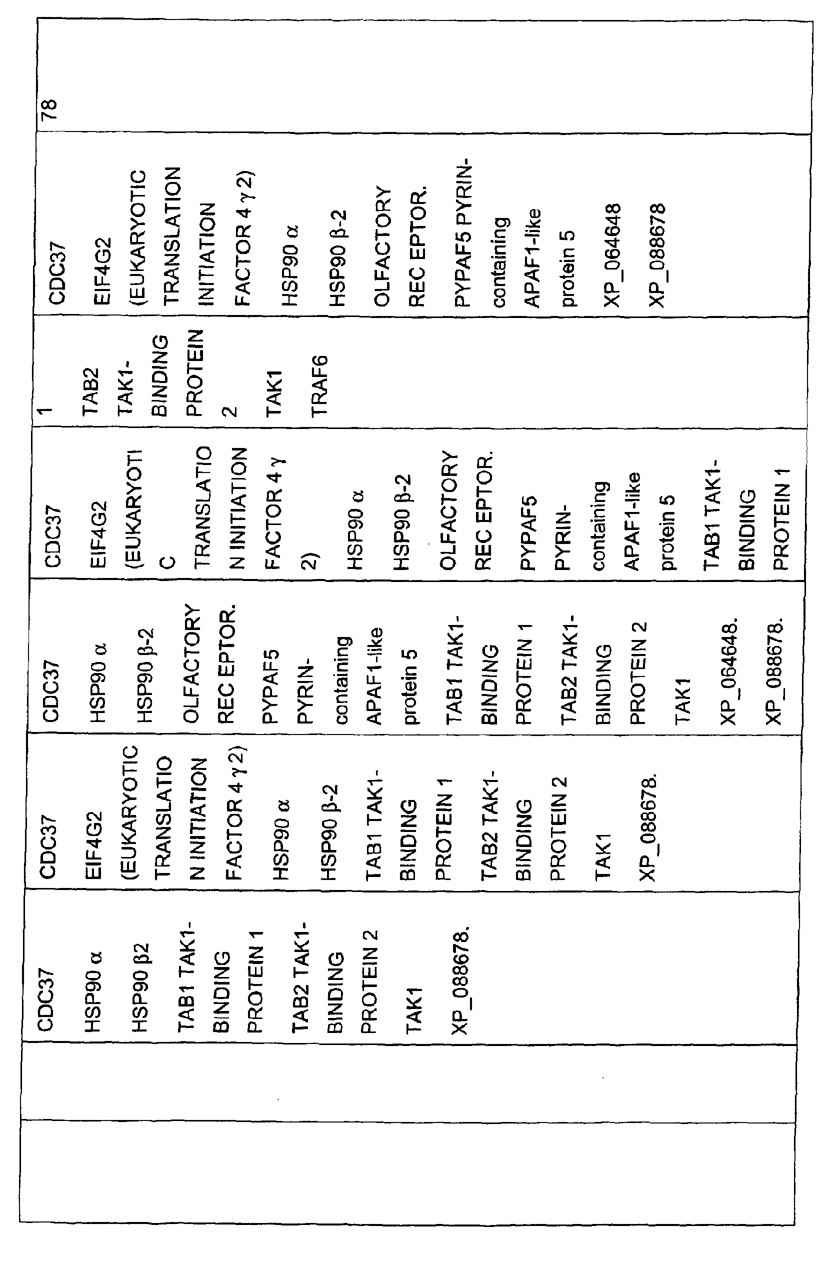

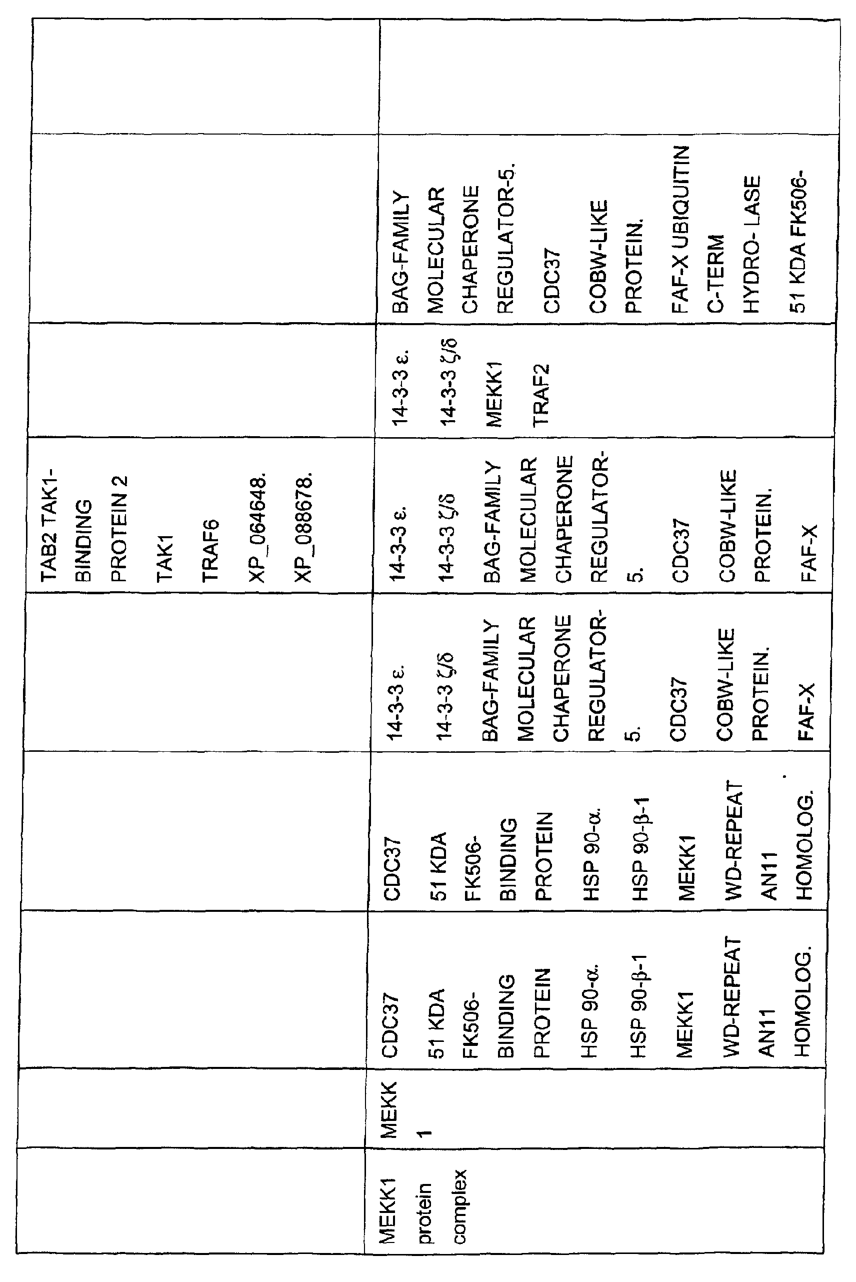

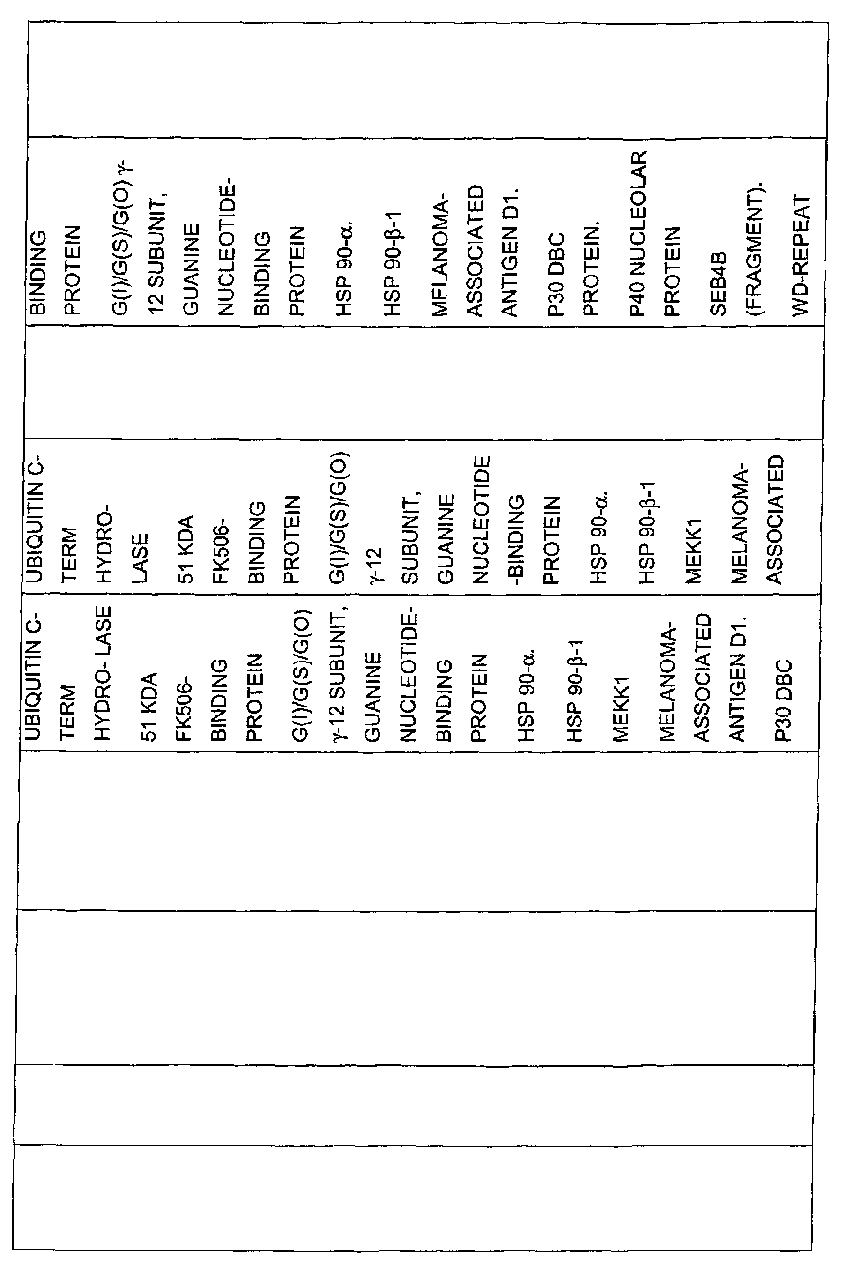

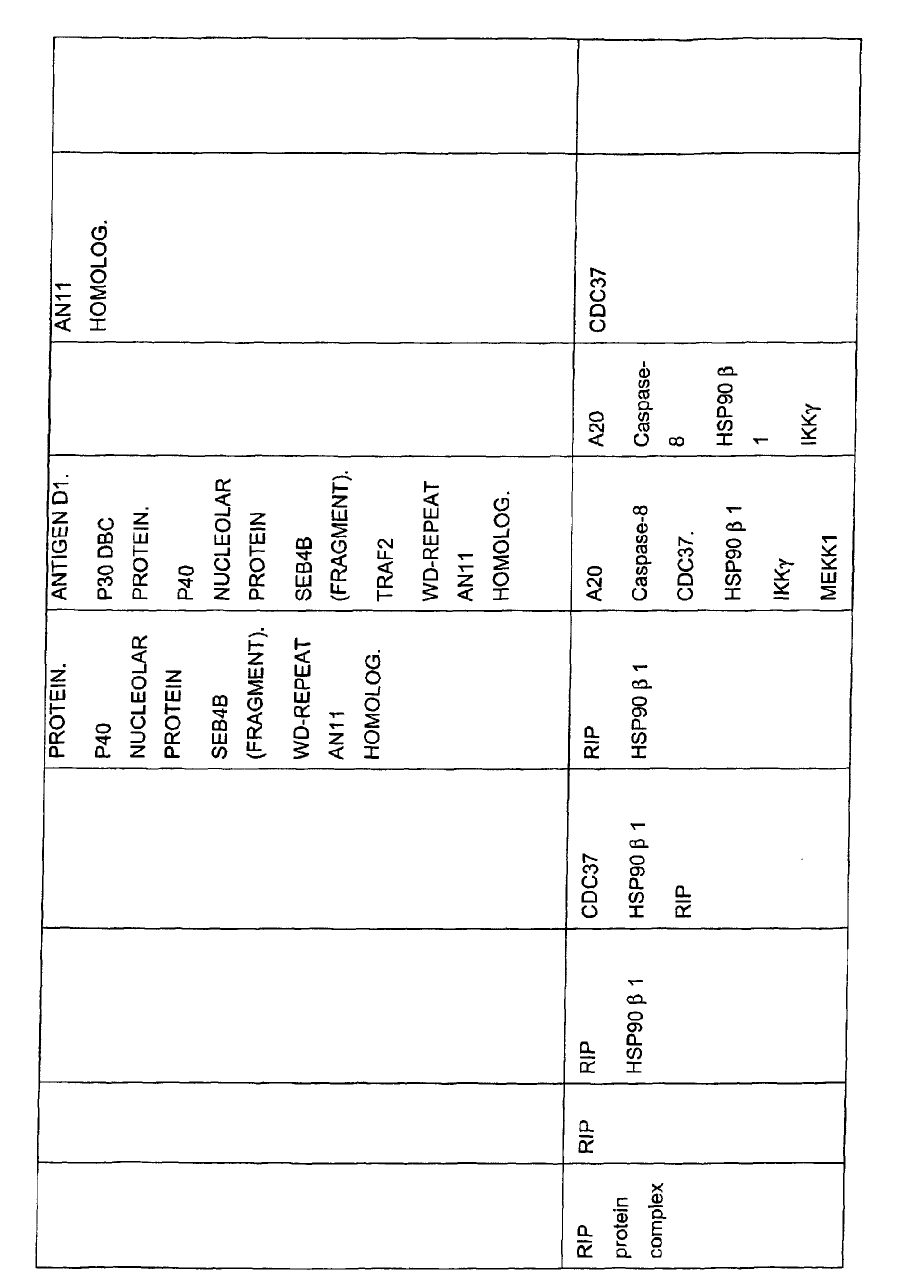

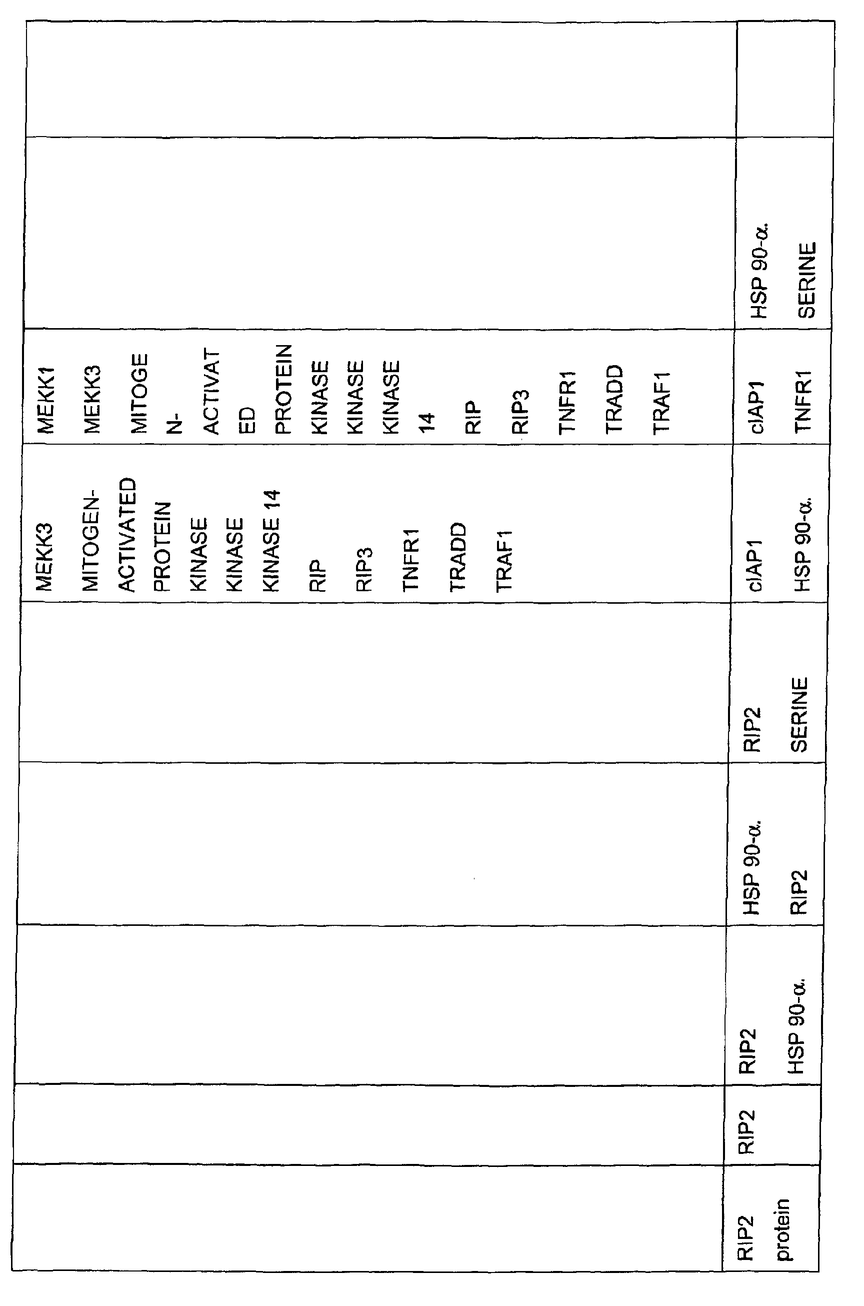

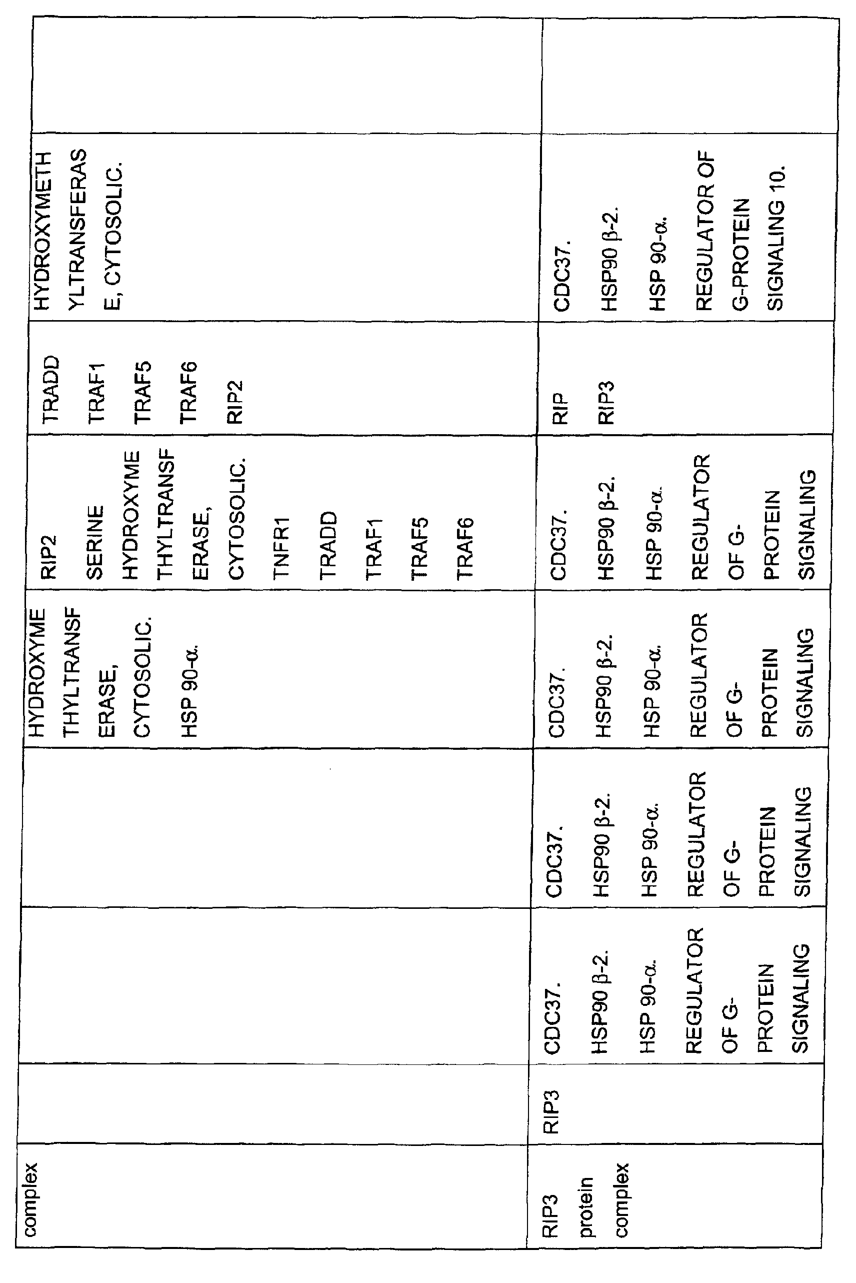

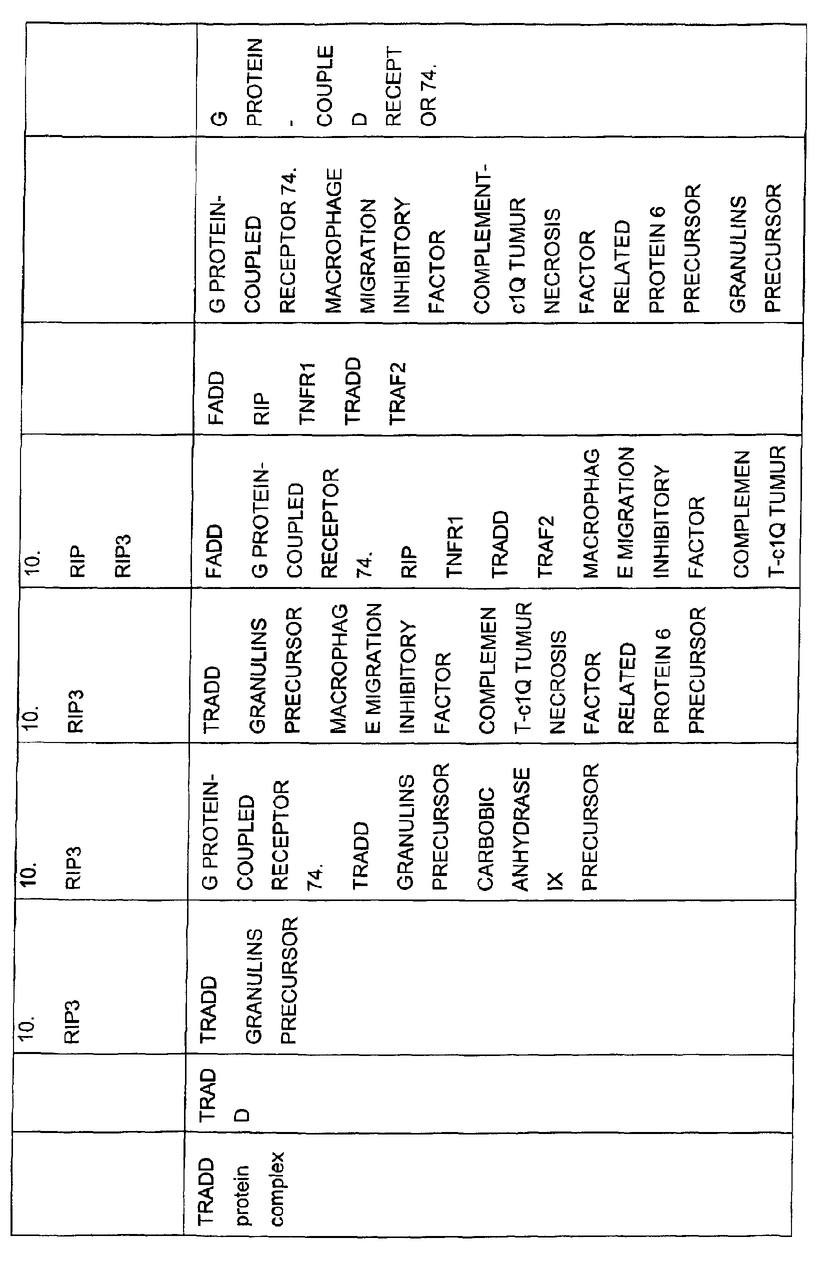

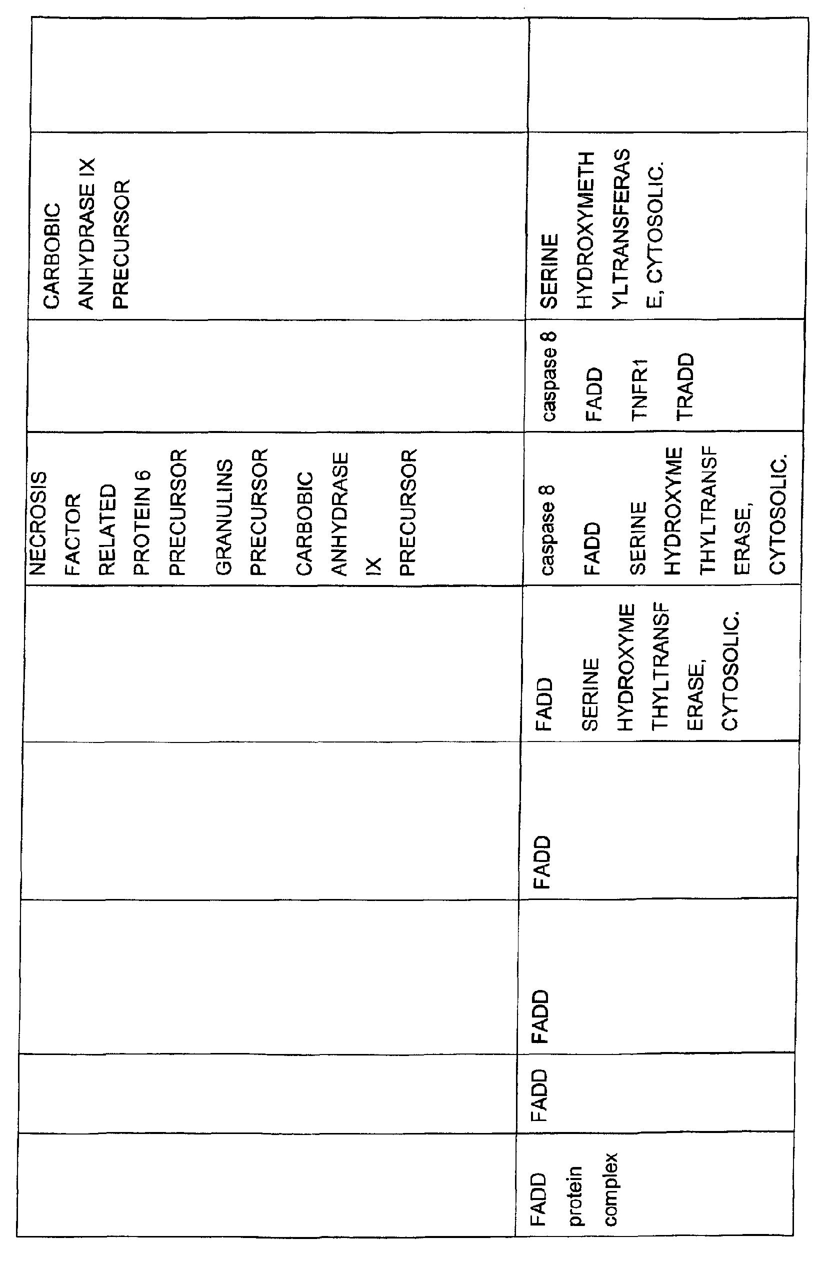

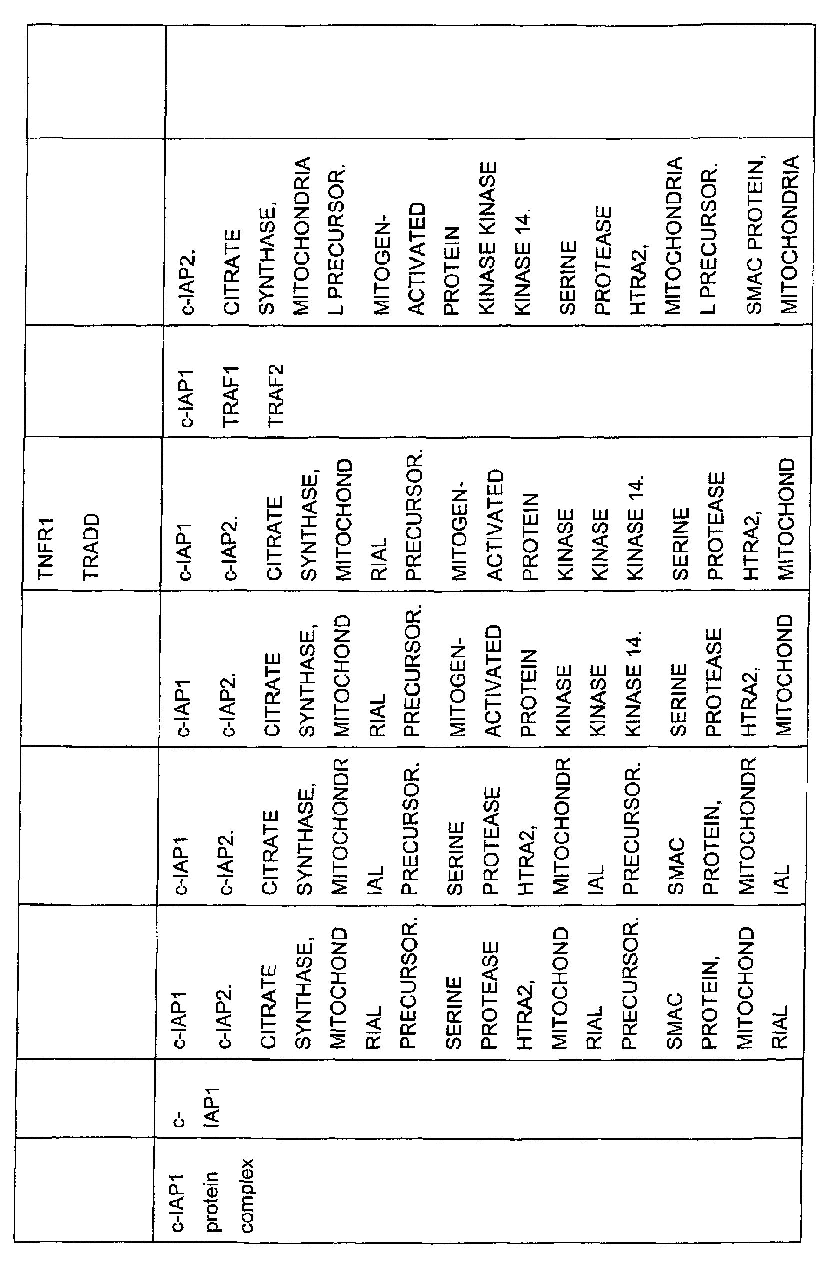

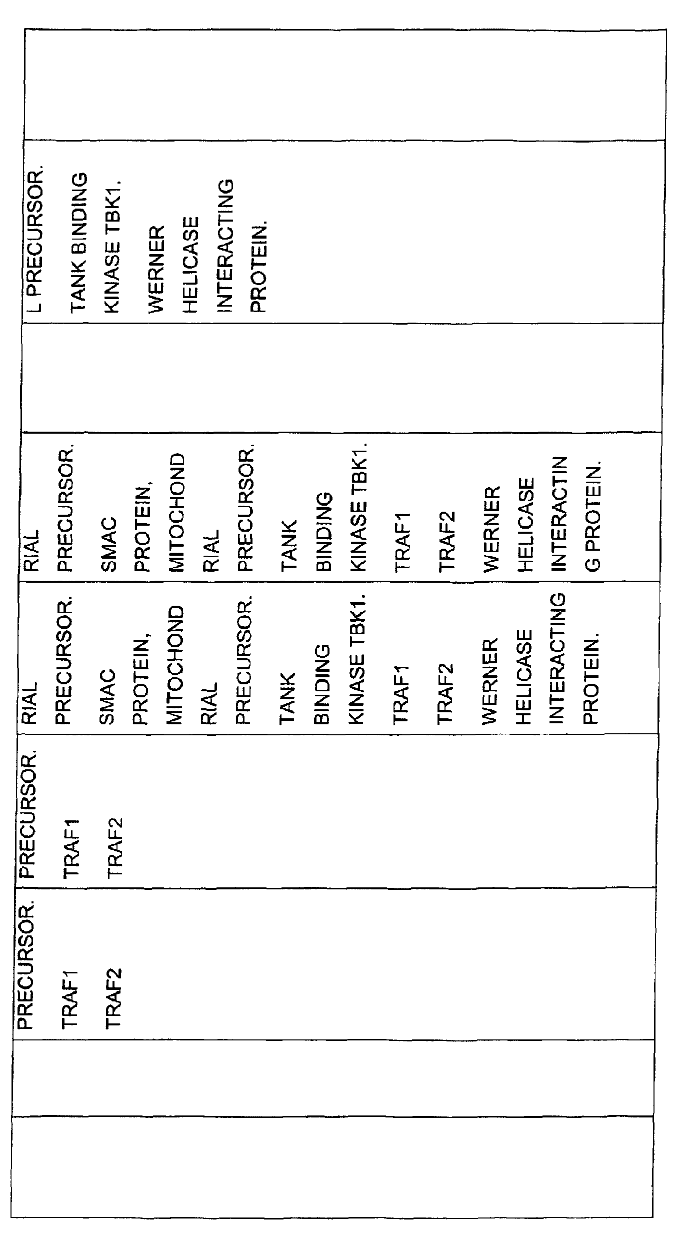

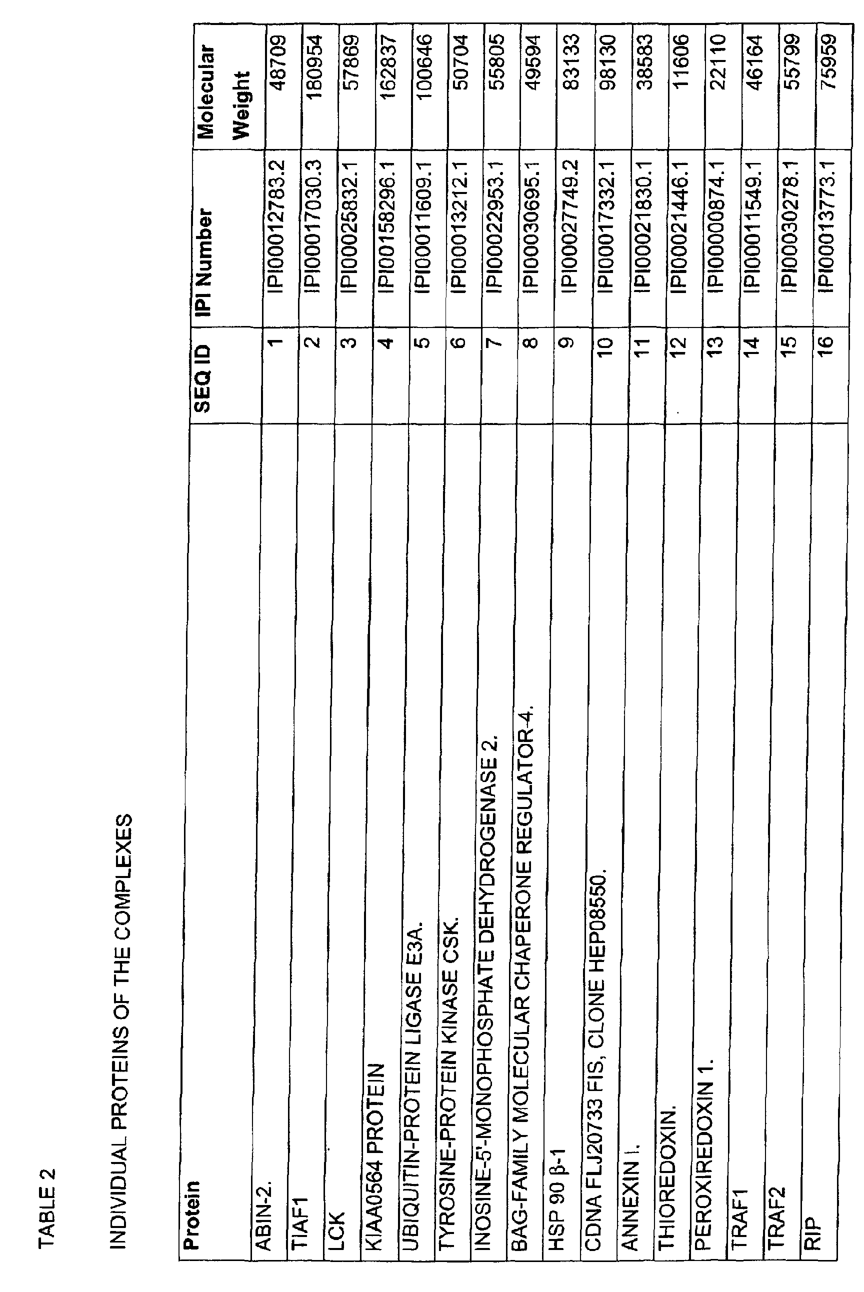

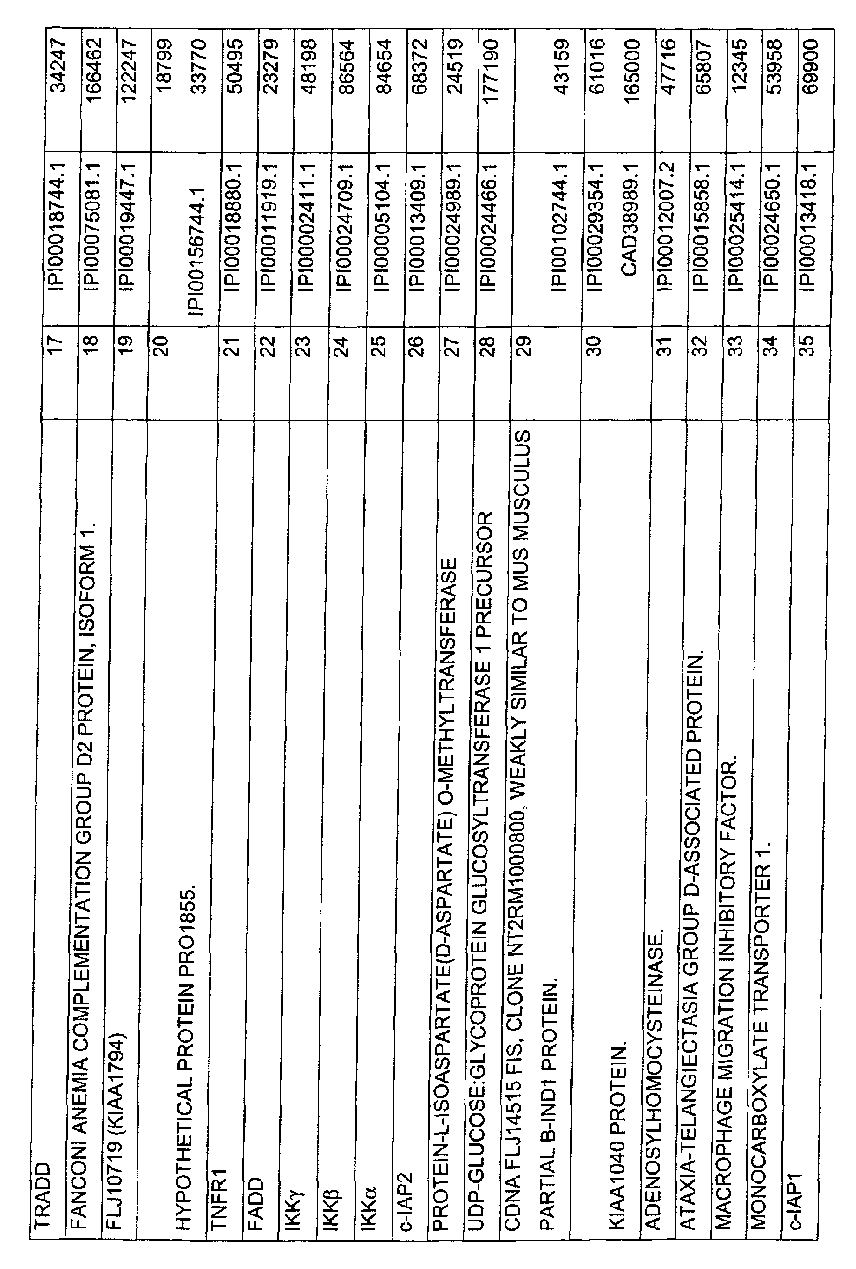

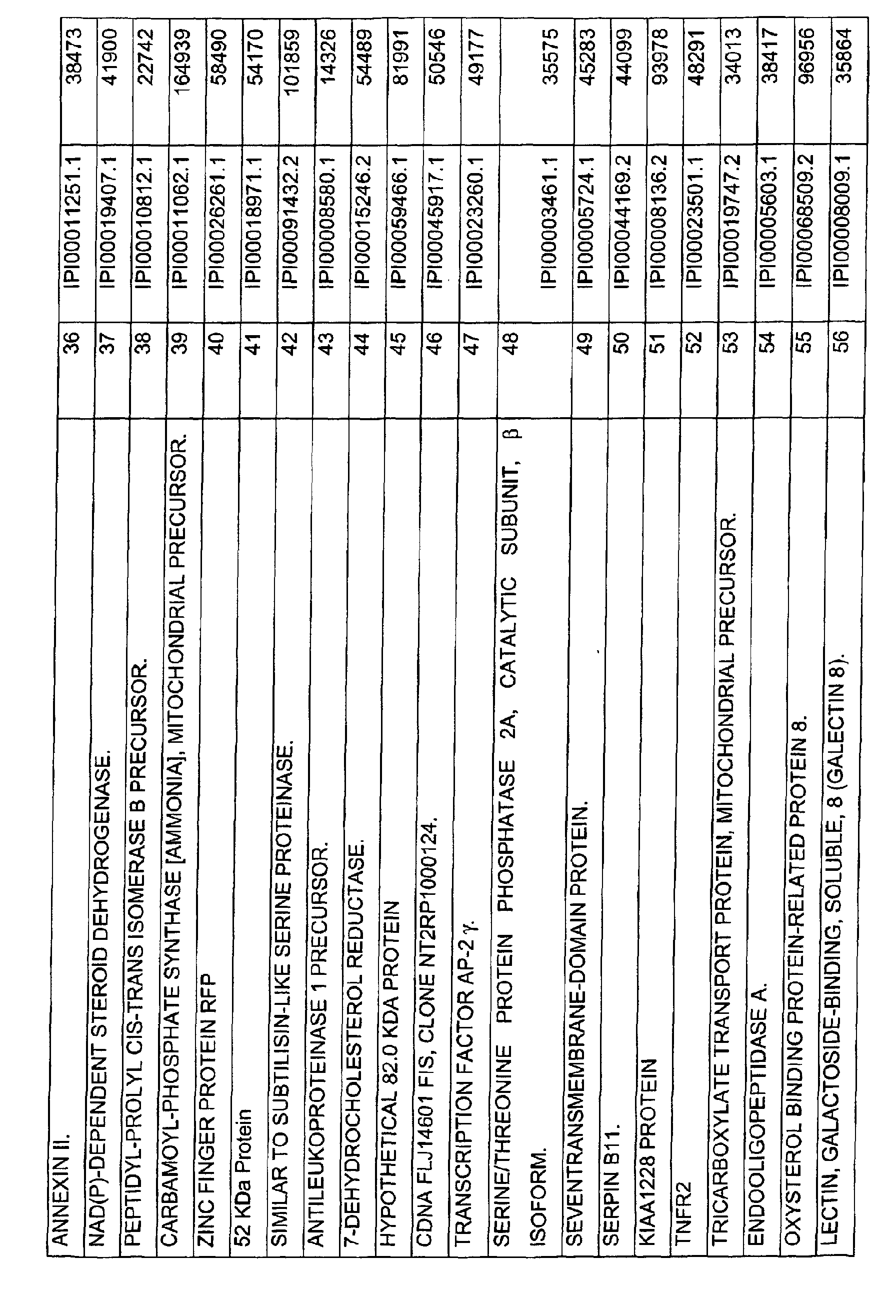

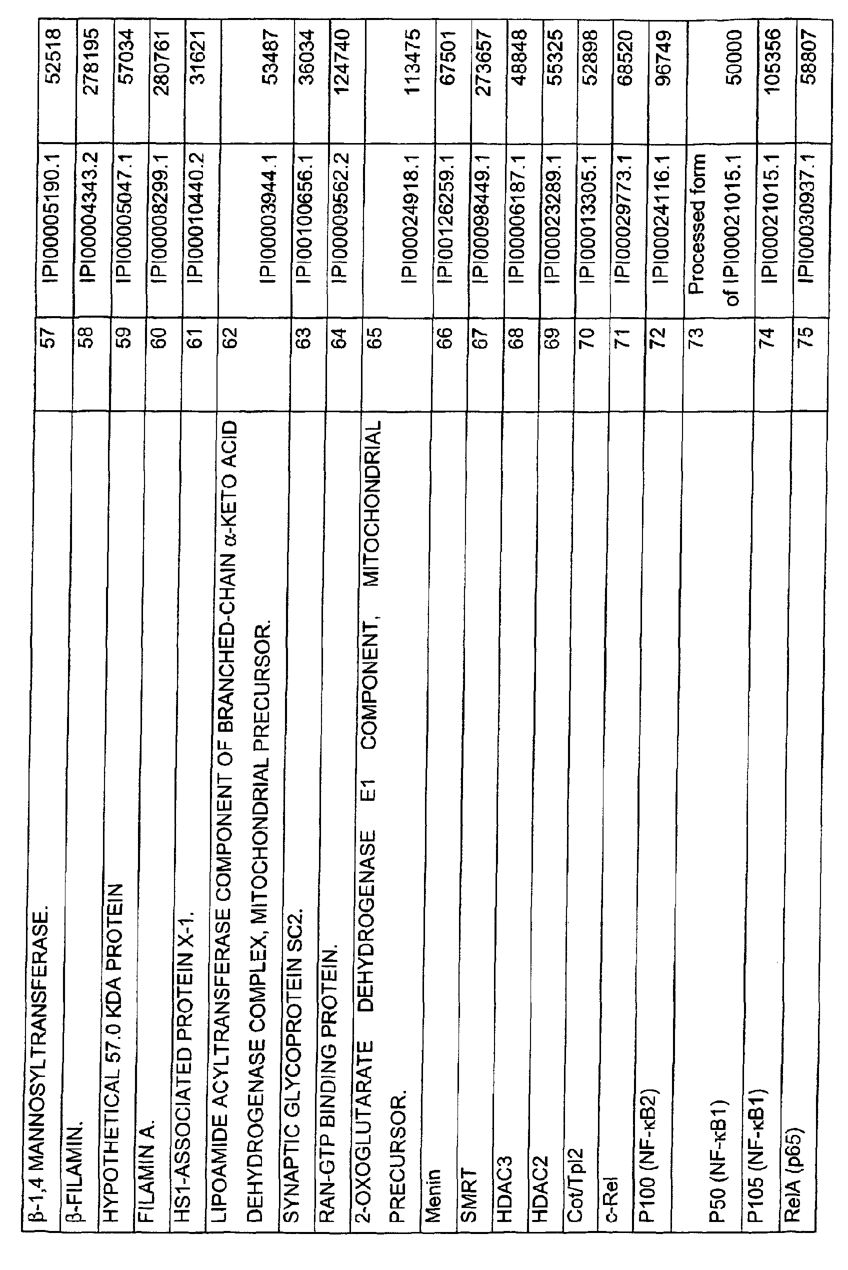

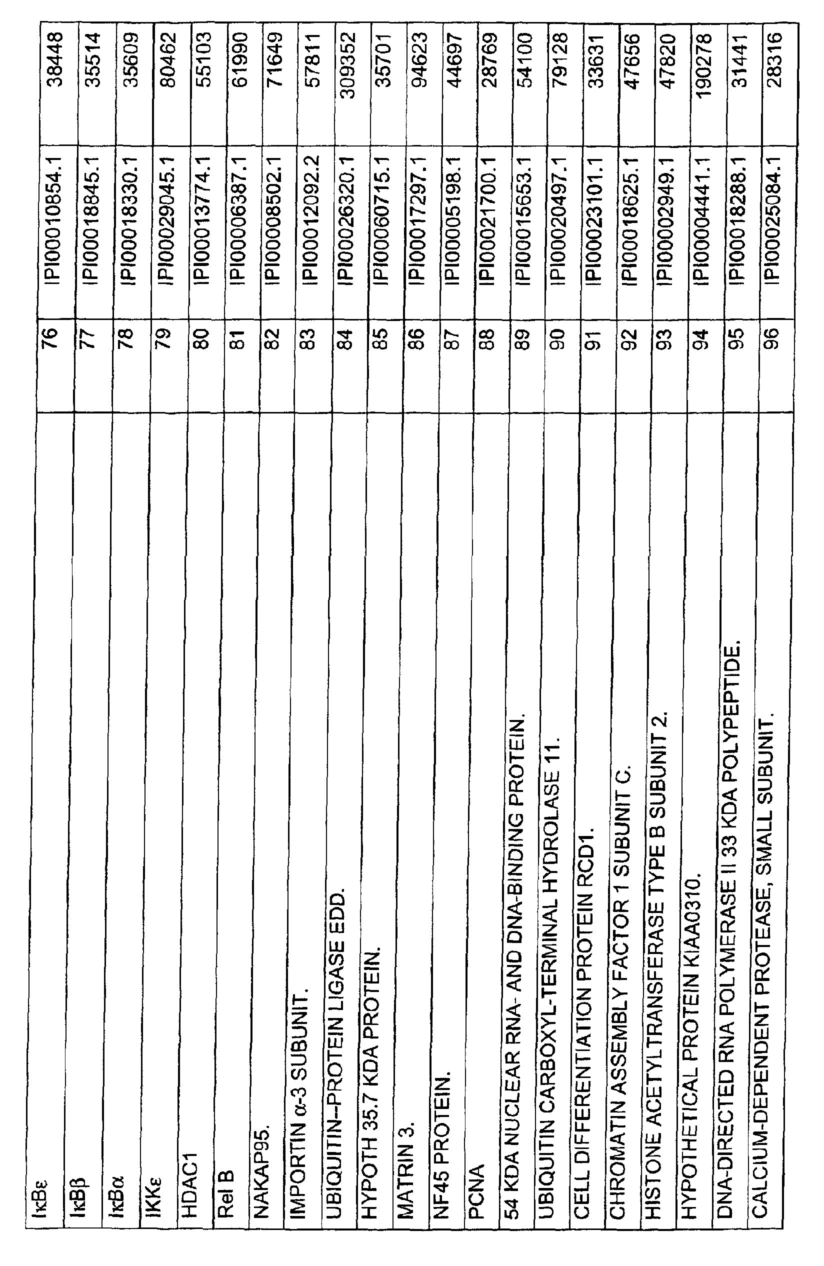

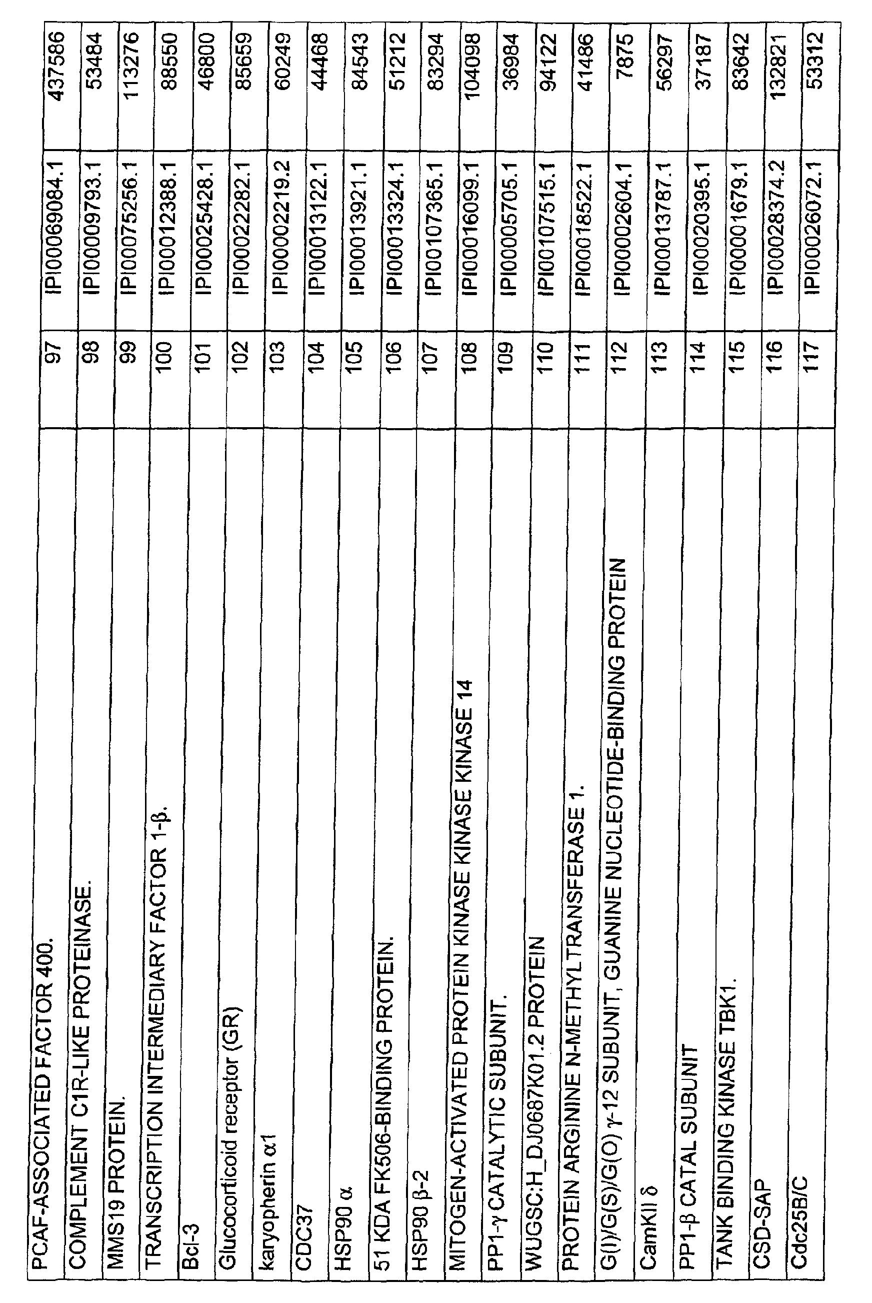

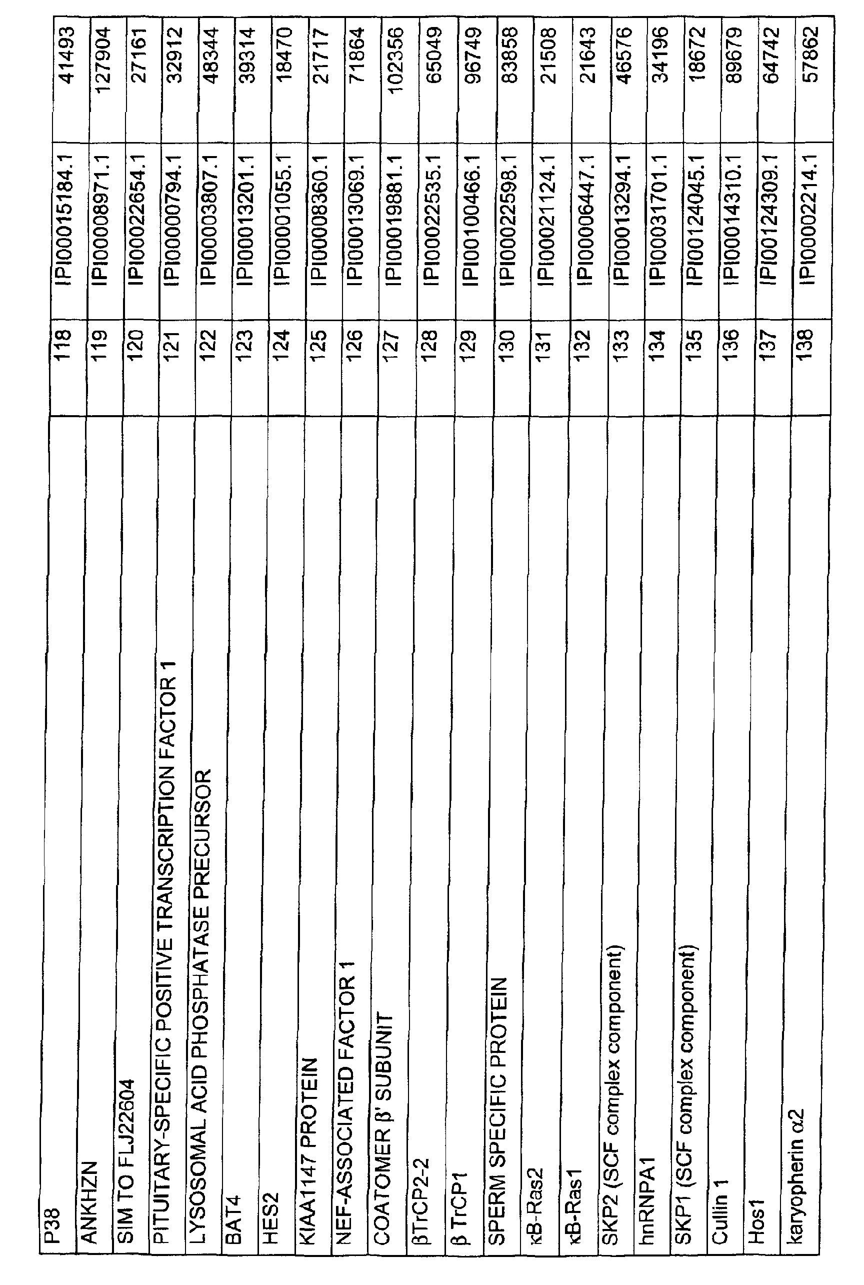

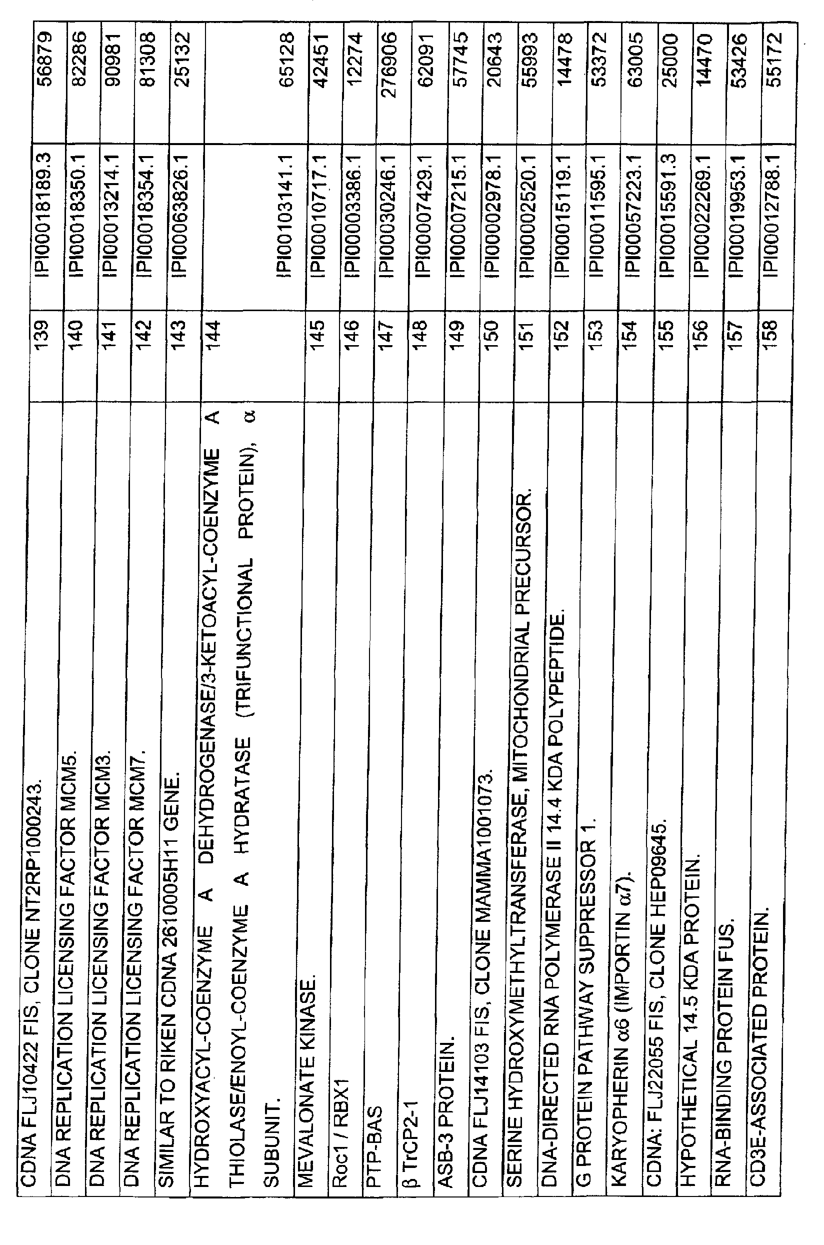

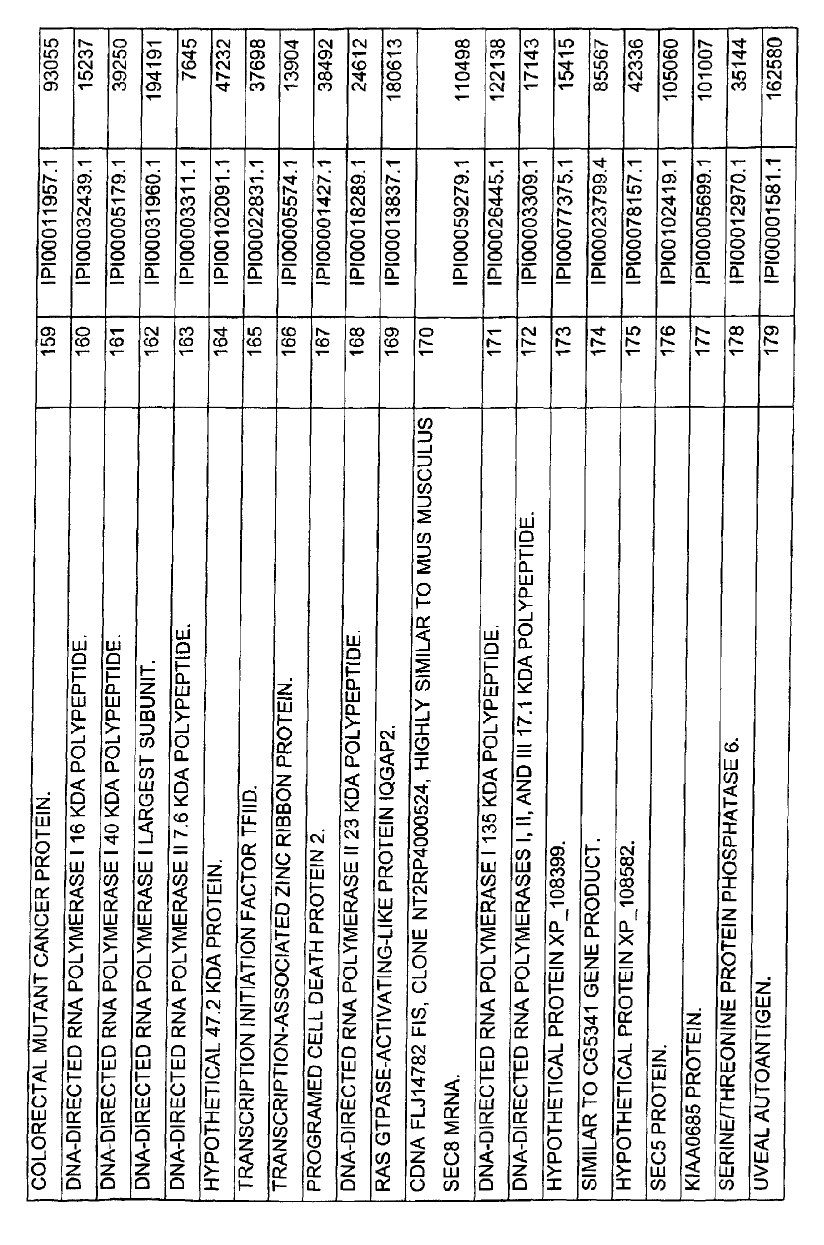

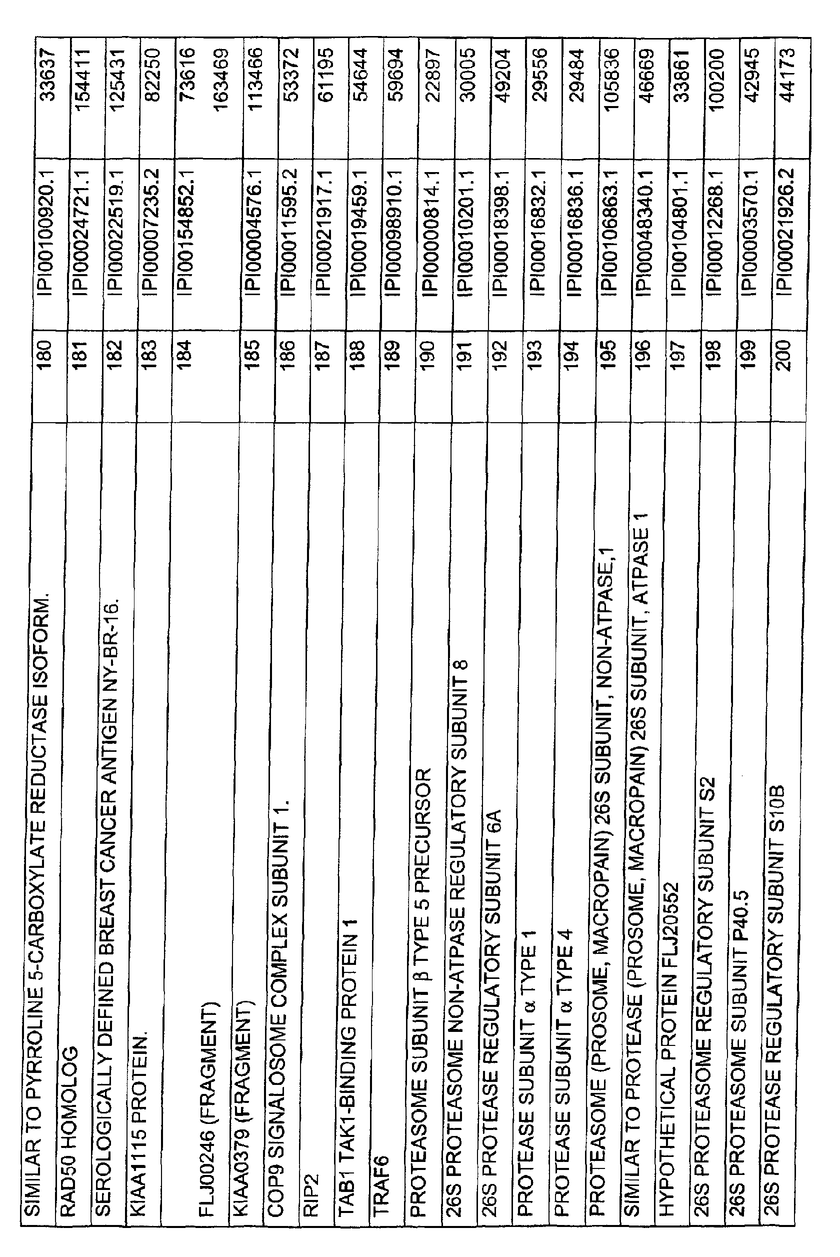

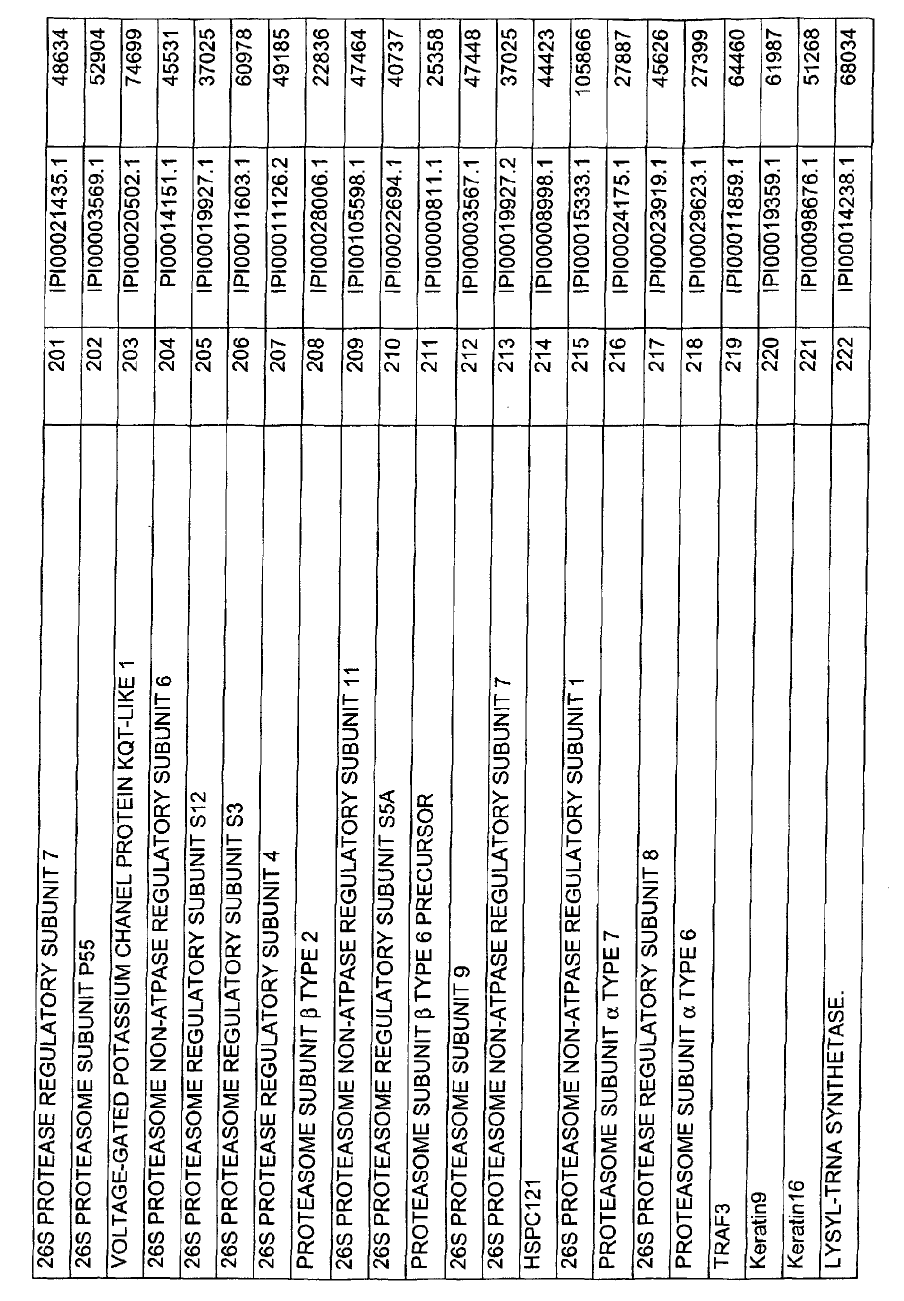

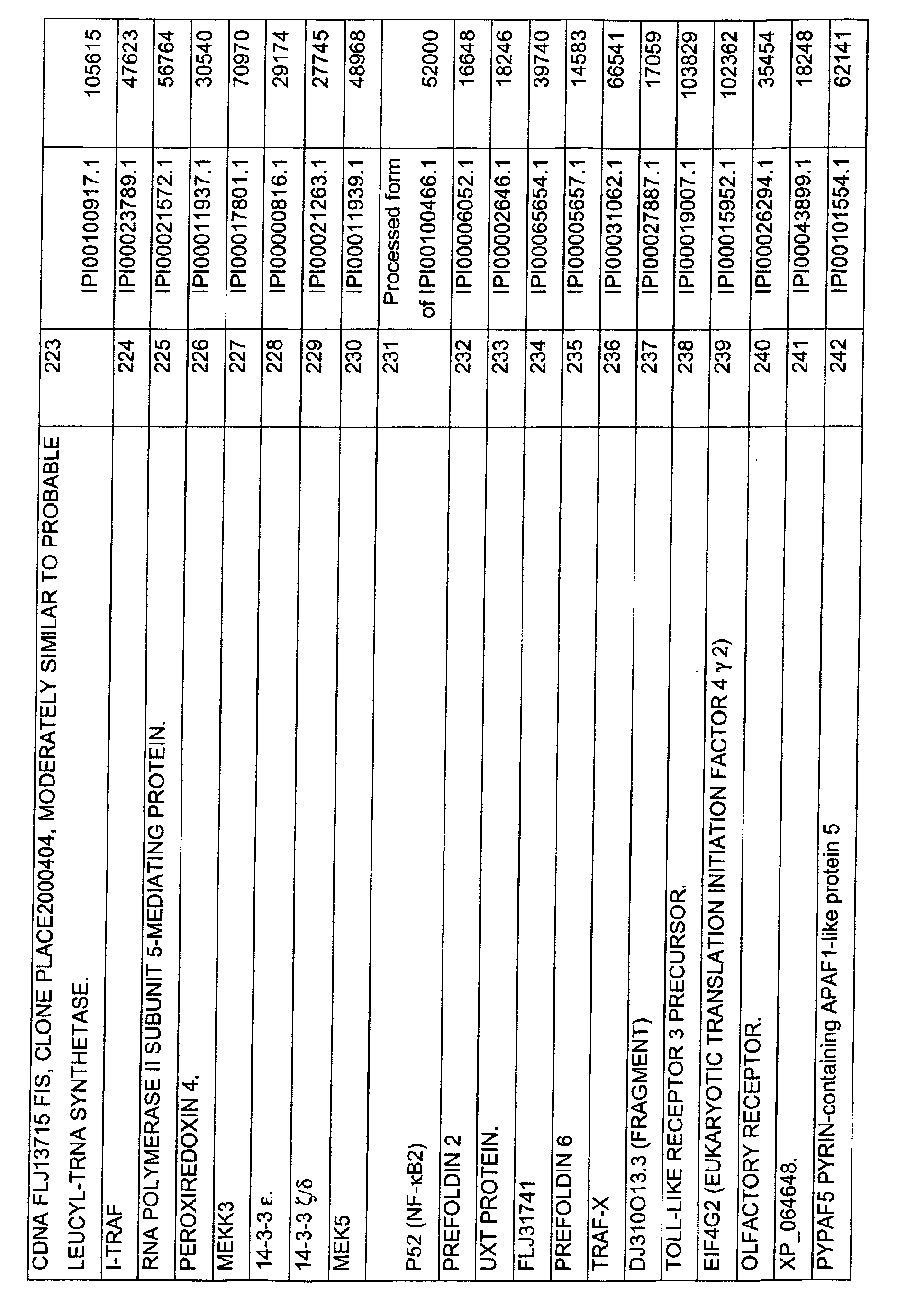

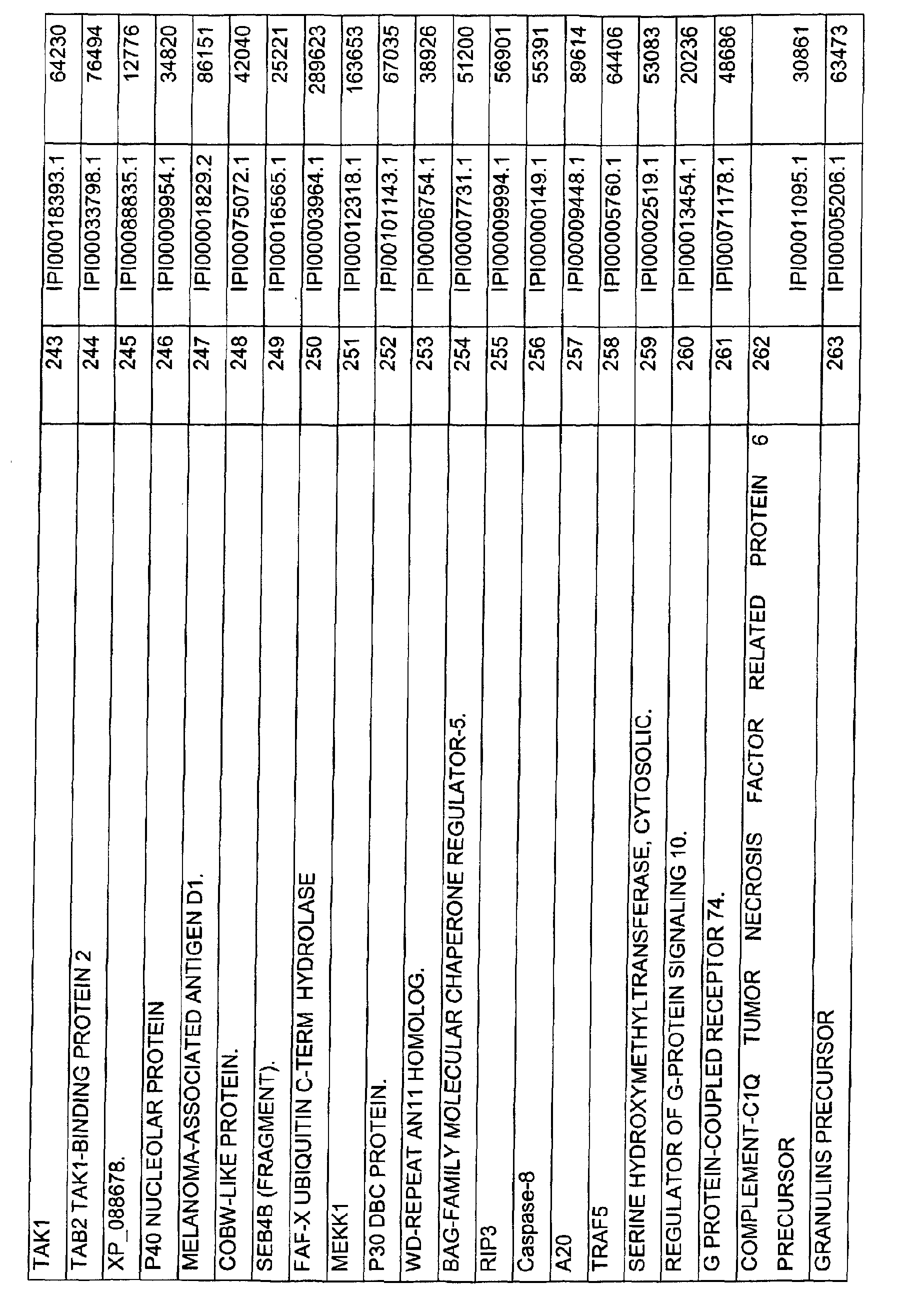

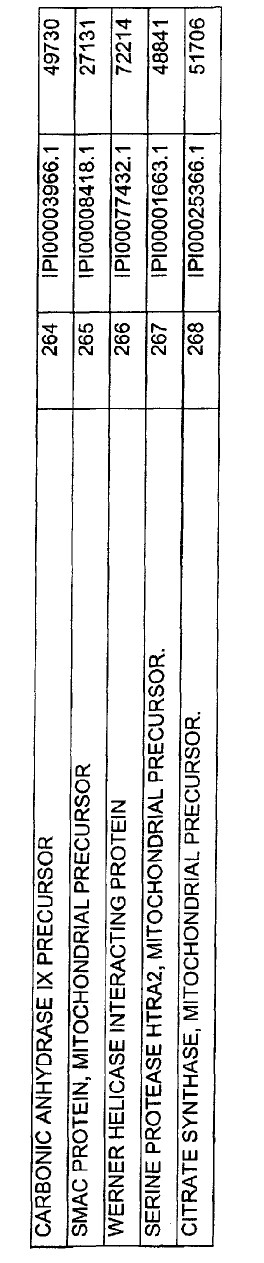

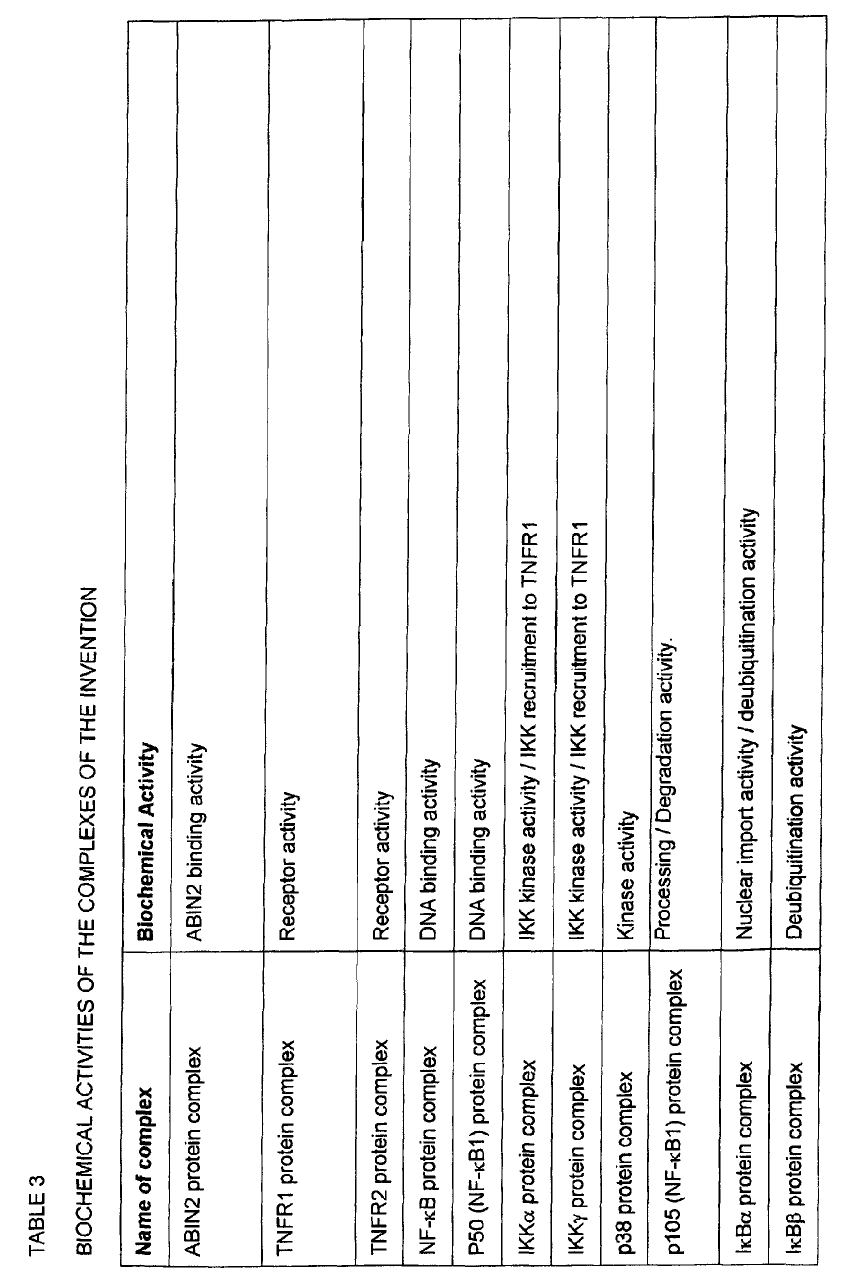

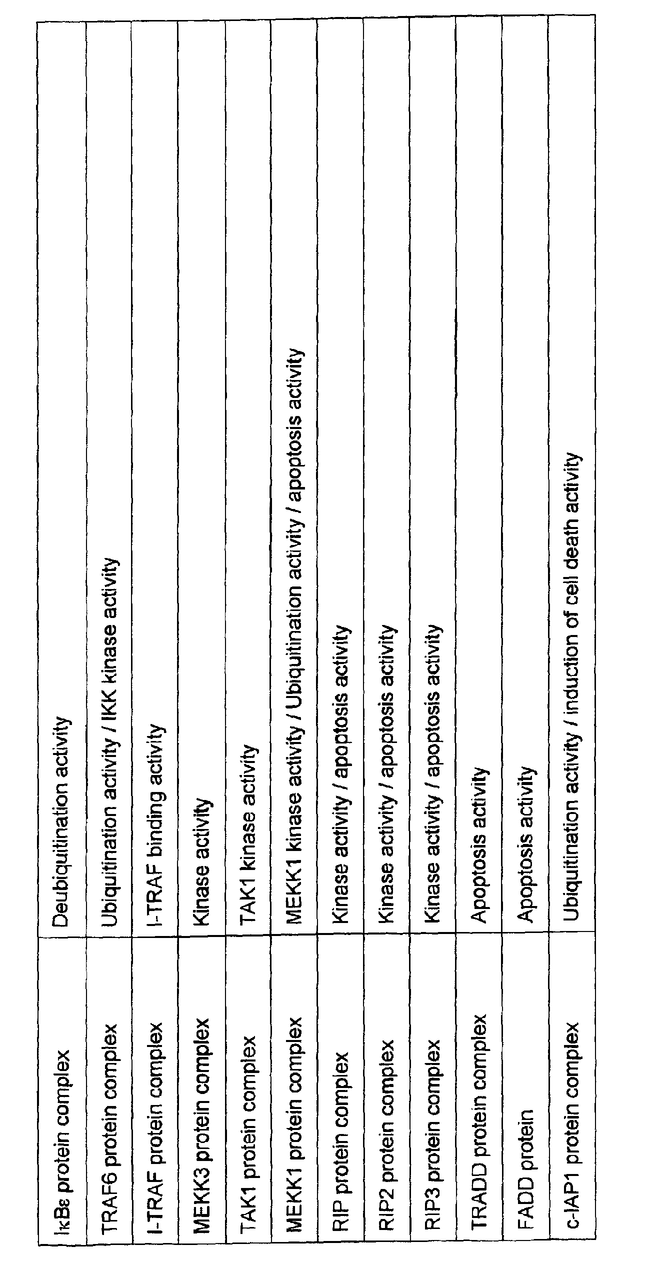

The present invention is not to be limited in scope by the specific embodiments described herein. Indeed, various modifications of the invention in addition to those described herein will become apparent to those skilled in the art from the foregoing description and accompanying figures. Such modifications are intended to fall within the scope of the appended claims.

Various publications are cited herein, the disclosures of which are incorporated by reference in their entireties.

SEQUENCES

SEQ ID No:1









SEQ ID No:2
MFNLMKKDKDKDGGRKEKKEKKEKKERMSAAELRSLEEMSLRRGFFNLNRSSKRESK
TRLEISNPIPIKVASGSDLHLTDIDSDSNRGSVILDSGHLSTASSSDDLKGEEGSFRGSVL
QRAAKFGSLAKQNSQMIVKRFSFSQRSRDESASETSTPSEHSAAPSPQVEVRTLEGQL
VQHPGPGIPRPGHRSRAPELVTKKFPVDLRLPPVVPLPPPTLRELELQRRPTGDFGFSL
RRTTMLDRGPEGQACRRVVHFAEPGAGTKDLALGLVPGDRLVEINGHNVESKSRDEIV
EMIRQSGDSVRLKVQPIPELSELSRSWLRSGEGPRREPSDAKTEEQIAAEEAWNETEK
VWLVHRDGFSLASQLKSEELNLPEGKVRVKLDHDGAILDVDEDDVEKANAPSCDRLED
LASLVYLNESSVLHTLRQRYGASLLHTYAGPSLLVLGPRGAPAVYSEKVMHMFKGCRR
EDMAPHIYAVAQTAYRAMLMSRQDQSIILLGSSGSGKTTSCQHLVQYLATIAGISGNKVF
SVEKWQALYTLLEAFGNSPTIINGNATRFSQILSLDFDQAGQVASASIQTMLLEKLRVAR
RPASEATFNVFYYLLACGDGTLRTELHLNHLAENNVFGIVPLAKPEEKQKAAQQFSKLQ
AAMKVLGISPDEQKACWFILAAIYHLGAAGATKDPFFHSTAGRKQFARHEWAQKAAYLL
GCSLEELSSAIFKHQHKGGTLQRSTSFRQGPEESGLGDGTGPKLSALECLEGMAAGLY
SELFTLLVSLVNRALKSSQHSLCSMMIVDTPGFQNPEQGGSARGASFEELCHNYTQDR
LQRLFHERTFVQELERYKEENIELAFDDLEPPTDDSVAAVDQASHQSLVRSLARTDEAR
GLLWLLEEEALVPGASEDTLLERLFSYYGPQEGDKKGQSPLLHSSKPHHFLLGHSHGT
NWVEYNVTGWLNYTKQNPATQNAPRLLQDSQKKIISNLFLGRAGSATVLSGSIAGLEGG
SQLALRRATSMRKTFTTGMAAVKKKSLCIQMKLQVDALIDTIKKSKLHFVHCFLPVAEG
WAGEPRSASSRRVSSSSELDLPSGDHCEAGLLQLDVPLLRTQLRGSRLLDAMRMYRQ

GYPDHMVFSEFRRRFDVLAPHLTKKHGRNYIVVDERRVVEELLECLDLEKSSCCMGLS
RVFFRAGTLARLEEQRDEQTSRNLTLFQAACRGYLARQHFKKRKIQDLAIRCVQKNIKK
NKGVKDWPWWKLFTTVRPLIEVQLSEEQIRNKDEEIQQLRSKLEKAEKERNELRLNSDR
LESRISELTSELTDERNTGESASQLLDAETAERLRAEKEMKELQTQYDALKKQMEVME
MEVMEARLIRAAEINGEVDDDDAGGEWRLKYERAVREVDFTKKRLQQEFEDKLEVEQQ
NKRQLERRLGDLQADSEESQRALQQLKKKCQRLTAELQDTKLHLEGQQVRNHELEKK
QRRFDSELSQAHEEAQREKLQREKLQREKDMLLAEAFSLKQQLEEKDMDIAGFTQKVV
SLEAELQDISSQESKDEASLAKVKKQLRDLEAKVKDQEEELDEQAGTIQMLEQAKLRLE
MEMERMRQTHSKEMESRDEEVEEARQSCQKKLKQMEVQLEEEYEDKQKVLREKREL
EGKLATLSDQVNRRDFESEKRLRKDLKRTKALLADAQLMLDHLKNSAPSKREIAQLKNQ
LEESEFTCAAAVKARKAMEVEIEDLHLQIDDIAKAKTALEEQLSRLQREKNEIQNRLEED
QEDMNELMKKHKAAVAQASRDLAQINDLQAQLEEANKEKQELQEKLQALQSQVEFLEQ
SMVDKSLVSRQEAKIRELETRLEFERTQVKRLESLASRLKENMEKLTEERDQRIAAENR
EKEQNKRLQRQLRDTKEEMGELARKEAEASRKKHELEMDLESLEAANQSLQADLKLAF
KRIGDLQAAIEDEMESDENEDLINSEGDSDVDSELEDRVDGVKSWLSKNKGPSKAASD
DGSLKSSSPTSYWKSLAPDRSDDEHDPLDNTSRPRYSHSYLSDSDTEAKLTETNA

SEQ ID No:3
GCGCSSHPEDDWMENIDVCENCHYPIVPLDGKGTLLIRNGSEVRDPLVTYEGSNPPAS
PLQDNLVIALHSYEPSHDGDLGFEKGEQLRILEQSGEWWKAQSLTTGQEGFIPFNFVAK
ANSLEPEPWFFKNLSRKDAERQLLAPGNTHGSFLIRESESTAGSFSLSVRDFDQNQGE
VVKHYKIRNLDNGGFYISPRITFPGLHELVRHYTNASDGLCTRLSRPCQTQKPQKPWW
EDEWEVPRETLKLVERLGAGQFGEVWMGYYNGHTKVAVKSLKQGSMSPDAFLAEANL
MKQLQHQRLVRLYAVVTQEPIYIITEYMENGSLVDFLKTPSGIKLTINKLLDMAAQIAEGM
AFIEERNYIHRDLRAANILVSDTLSCKIADFGLARLIEDNEYTAREGAKFPIKWTAPEAINY
GTFTIKSDVWSFGILLTEIVTHGRIPYPGMTNPEVIQNLERGYRMVRPDNCPEELYQLMR
LCWKERPEDRPTFDYLRSVLEDFFTATEGQYQPQP

SEQ ID No:4
GYNIEPIMLYQDMTARDLLQQRYTLPNGDTAWRSSPLVNAALEGKLVLLDGIHRVNAGT
LAVLQRLIHDRELSLYDGSRLLREDRYMRLKEELQLSDEQLQKRSIFPIHPSFRIIALAEP
PVIGSTAHQWLGPEFLTMFFFHYMKPLVKSEEIQVIKEKVPNVPQEALDKLLSFTHKLRE

TQDPTAQSLAASLSTRQLLRISRRLSQYPNENLHSAVTKACLSRFLPSLARSALEKNLAD
ATIEINTDDNLEPELKDYKCEVTSGTLRIGAVSAPIYNAHEKMKVPDVLFYDNIQHVIVME
DMLKDFLLGEHLLLVGNQGVGKNKIVDRFLHLLNRPREYIQLHRDTTVQTLTLQPSVKD
GLIVYEDSPLVKAVKLGHILVVDEADKAPTNVTCILKTLVENGEMILADGRRIVANSANVN
GRENVVVIHPDFRMIVLANRPGFPFLGNDFFGTLGDIFSCHAVDNPKPHSELEMLRQYG
PNVPEPILQKLVAAFGELRSLADQGIINYPYSTREVVNIVKHLQKFPTEGLSSVVRNVFDF
DSYNNDMREILINTLHKYGIPIGAKPTSVQLAKELTLPEQTFMGYWTIGQARSGMQKLLC
PVETHHIDIKGPALINIQEYPIERHEERSLNFTEECASWRIPLDEINIICDIATSHENEQNTL
YVVTCNPASLYFMNMTGKSGFFVDFFDIFPRTANGVWHPFVTVAPLGSPLKGQVVLHE
QQSNVILLLDTTGRALHRLILPSEKFTSKKPFWWNKEEAETYKMCKEFSHKNWLVFYKE
KGNSLTVLDVLEGRTHTISLPINLKTVFLVAEDKWLLVESKTNQKYLLTKPAHIESEGSGV
CQLYVLKEEPPSTGFGVTQETEFSIPHKISSDQLSSEHLSSAVEQKIASPNRILSDEKNYA
TIVVGFPDLMSPSEVYSWKRPSSLHKRSGTDTSFYRGKKKRGTPKQSNCVTLLDTNQV
VRILPPGEVPLKDIYPKDVTPPQTSGYIEVTDLQSKKLRYIPIPRSESLSPYTTWLSTISDT
DALLAEWDKSGVVTVDMGGHIRLWETGLERLQRSLMEWRNMIGQDDRNMQITINRDS
GEDVSSPKHGKEDPDNMPHVGGNTWAGGTGGRDTAGLGGKGGPYRLDAGHTVYQV
SQAEKDAVPEEVKRAAREMGQRAFQQRLKEIQMSEYDAATYERFSGAVRRQVHSLRII
LDNLQAKGKERQWLRHQATGELDDAKIIDGLTGEKAIYKRRGELEPQLGSPQQKPKRL
RLVVDVSGSMYRFNRMDGRLERTMEAVCMVMEAFENYEEKFQYDIVGHSGDGYNIGL
VPMNKIPKDNKQRLEILKTMHAHSQFCMSGDHTLEGTEHAIKEIVKEEADEYFVIVLSDA
NLSRYGIHPAKFAQILTRDPQVNAFAIFIGSLGDQATRLQRTLPAGRSFVAMDTKDIPQIL
QQIFTSTMLSSV

SEQ ID No:5
MEKLHQCYWKSGEPQSDDIEASRMKRAAAKHLIERYYHQLTEGCGNEACTNEFCASC
PTFLRMDNNAAAIKALELYKINAKLCDPHPSKKGASSAYLENSKGAPNNSCSEIKMNKK
GARIDFKDVTYLTEEKVYEILELCREREDYSPLIRVIGRVFSSAEALVQSFRKVKQHTKEE
LKSLQAKDEDKDEDEKEKAACSAAAMEEDSEASSSRIGDSSQGDNNLQKLGPDDVSV
DIDAIRRVYTRLLSNEKIETAFLNALVYLSPNVECDLTYHNVYSRDPNYLNLFIIGMENRN
LHSPEYLEMALPLFCKAMSKLPLAAQGKLIRLWSKYNADQIRRMMETFQQLITYKVISNE
FNSRNLVNDDDAIVAASKCLKMVYYANVVGGEVDTNHNEEDDEEPIPESSELTLQELLG
EERRNKKGPRVDPLETELGVKTLDCRKPLIPFEEFINEPLNEVLEMDKDYTFFKVETENK
FSFMTCPFILNAVTKNLGLYYDNRIRMYSERRITVLYSLVQGQQLNPYLRLKVRRDHIIDD

ALVRLEMIAMENPADLKKQLYVEFEGEQGVDEGGVSKEFFQLVVEEIFNPDIGMFTYDE
STKLFWFNPSSFETEGQFTLIGIVLGLAIYNNCILDVHFPMVVYRKLMGKKGTFRDLGDS
HPVLYQSLKDLLEYEGNVEDDMMITFQISQTDLFGNPMMYDLKENGDKIPITNENRKEF
VNLYSDYILNKSVEKQFKAFRRGFHMVTNESPLKYLFRPEEIELLICGSRNLDFQALEET
TEYDGGYTRDSVLIREFWEIVHSFTDEQKRLFLQFTTGTDRAPVGGLGKLKMIIAKNGPD
TERLPTSHTCFNVLLLPEYSSKEKLKERLLKAITYAKGFGML

SEQ ID No:6
MSAIQAAWPSGTECIAKYNFHGTAEQDLPFCKGDVLTIVAVTKDPNWYKAKNKVGREGI
IPANYVQKREGVKAGTKLSLMPWFHGKITREQAERLLYPPETGLFLVRESTNYPGDYTL
CVSCDGKVEHYRIMYHASKLSIDEEVYFENLMQLVEHYTSDADGLCTRLIKPKVMEGTV
AAQDEFYRSGWALNMKELKLLQTIGKGEFGDVMLGDYRGNKVAVKCIKNDATAQAFLA
EASVMTQLRHSNLVQLLGVIVEEKGGLYIVTEYMAKGSLVDYLRSRGRSVLGGDCLLKF
SLDVCEAMEYLEGNNFVHRDLAARNVLVSEDNVAKVSDFGLTKEASSTQDTGKLPVKW
TAPEALREKKFSTKSDVWSFGILLWEIYSFGRVPYPRIPLKDVVPRVEKGYKMDAPDGC
PPAVYEVMKNCWHLDAAMRPSFLQLREQLEHIKTHELHL

SEQ ID No:7
MADYLISGGTSYVPDDGLTAQQLFNCGDGLTYNDFLILPGYIDFTADQVDLTSALTKKITL
KTPLVSSPMDTVTEAGMAIAMALTGGIGFIHHNCTPEFQANEVRKVKKYEQGFITDPVVL
SPKDRVRDVFEAKARHGFCGIPITDTGRMGSRLVGIISSRDIDFLKEEEHDCFLEEIMTK
REDLVVAPAGITLKEANEILQRSKKGKLPIVNEDDELVAIIARTDLKKNRDYPLASKDAKK
QLLCGAAIGTHEDDKYRLDLLAQAGVDVVVLDSSQGNSIFQINMIKYIKDKYPNLQVIGG
NVVTAAQAKNLIDAGVDALRVGMGSGSICITQEVLACGRPQATAVYKVSEYARRFGVPV
IADGGIQNVGHIAKALALGASTVMMGSLLAATTEAPGEYFFSDGIRLKKYRGMGSLDAM
DKHLSSQNRYFSEADKIKVAQGVSGAVQDKGSIHKFVPYLIAGIQHSCQDIGAKSLTQVR
AMMYSGELKFEKRTSSAQVEGGVHSLHSYEKRLF

SEQ ID No:8
MSALRRSGYGPSDGPSYGRYYGPGGGDVPVHPPPPLYPLRPEPPQPPISWRVRGGG
PAETTWLGEGGGGDGYYPSGGAWPEPGRAGGSHQEQPPYPSYNSNYWNSTARSRA

PYPSTYPVRPELQGQSLNSYTNGAYGPTYPPGPGANTASYSGAYYAPGYTQTSYSTEV
PSTYRSSGNSPTPVSRWIYPQQDCQTEAPPLRGQVPGYPPSQNPGMTLPHYPYGDGN
RSVPQSGPTVRPQEDAWASPGAYGMGGRYPWPSSAPSAPPGNLYMTESTSPWPSS
GSPQSPPSPPVQQPKDSSYPYSQSDQSMNRHNFPCSVHQYESSGTVNNDDSDLLDS
QVQYSAEPQLYGNATSDHPNNQDQSSSLPEECVPSDESTPPSIKKIIHVLEKVQYLEQE
VEEFVGKKTDKAYWLLEEMLTKELLELDSVETGGQDSVRQARKEAVCKIQAILEKLEKK
GL

SEQ ID No:9
PEEVHHGEEEVETFAFQAEIAQLMSLIINTFYSNKEIFLRELISNASDALDKIRYESLTDPS
KLDSGKELKIDIIPNPQERTLTLVDTGIGMTKADLINNLGTIAKSGTKAFMEALQAGADISM
IGQFGVGFYSAYLVAEKVVVITKHNDDEQYAWESSAGGSFTVRADHGEPIGRGTKVILH
LKEDQTEYLEERRVKEVVKKHSQFIGYPITLYLEKEREKEISDDEAEEEKGEKEEEDKDD
EEKPKIEDVGSDEEDDSGKDKKKKTKKIKEKYIDQEELNKTKPIWTRNPDDITQEEYGEF
YKSLTNDWEDHLAVKHFSVEGQLEFRALLFIPRRAPFDLFENKKKKNNIKLYVRRVFIMD
SCDELIPEYLNFIRGVVDSEDLPLNISREMLQQSKILKVIRKNIVKKCLELFSELAEDKENY
KKFYEAFSKNLKLGIHEDSTNRRRLSELLRYHTSQSGDEMTSLSEYVSRMKETQKSIYYI
TGESKEQVANSAFVERVRKRGFEVVYMTEPIDEYCVQQLKEFDGKSLVSVTKEGLELP
EDEEEKKKMEESKAKFENLCKLMKEILDKKVEKVTISNRLVSSPCCIVTSTYGWTANME
RIMKAQALRDNSTMGYMMAKKHLEINPDHPIVETLRQKAEADKNDKAVKDLVVLLFETA
LLSSGFSLEDPQTHSNRIYRMIKLGLGIDEDEVAAEEPNAAVPDEIPPLEGDEDASRMEE
VD

SEQ ID No:10
MAGAAAESGRELWTFAGSRDPSAPRLAYGYGPGSLRELRAREFSRLAGTVYLDHAGA
TLFSQSQLESFTSDLMENTYGNPHSQNISSKLTHDTVEQVRYRILAHFHTTAEDYTVIFT
AGSTAALKLVAEAFPWVSQGPESSGSRFCYLTDSHTSVVGMRNVTMAINVISIPVRPED
LWSAEERGASASNPDCQLPHLFCYPAQSNFSGVRYPLSWIEEVKSGRLRPVSTPGKW
FVLLDAASYVSTSPLDLSAHQADFVPISFYKIFGFPTGLGALLVHNRAAPLLRKTYFGGG
TASAYLAGEDFYIPRQSVAQRFEDGTISFLDVIALKHGFDTLERLTGGMENIKQHTFTLA
QYTYMALSSLQYPNGAPVVRIYSDSEFSSPEVQGPIINFNVLDDKGNIIGYSQVDKMASL
YNIHLRTGCFCNTGACQRHLGISNEMVRKHFQAGHVCGDNMDLIDGQPTGSVRISFGY

MSTLDDVQAFLRFIIDTRLHSSGDWPVPQAHADTGETGAPSADSQADVIPAVMGRRSL
SPQEDALTGSRVWNNSSTVNAVPVAPPVCDVARTQPTPSEKAAGVLEGALGPHVVTN
LYLYPIKSCAAFEVTRWPVGNQGLLYDRSWMVVNHNGVCLSQKQEPRLCLIQPFIDLR
QRIMVIKAKGMEPIEVPLEENSERTQIRQSRVCADRVSTYDCGEKISSWLSTFFGRPCN
LIKQSSNSQRNAKKKHGKDQLPGTMATLSLVNEAQYLLINTSSILELHRQLNTSDENGKE
ELFSLKDLSLRFRANIIINGKRAFEEEKWDEISIGSLRFQVLGPCHRCQMICIDQQTGQRN
QHVFQKLSESRETKVNFGMYLMHASLDLSSPCFLSVGSQVLPVLKENVEGHDLPASEK
HQDVTS

SEQ ID No:11
AMVSEFLKQAWFIENEEQEYVQTVKSSKGGPGSAVSPYPTFNPSSDVAALHKAIMVKG
VDEATIIDILTKRNNAQRQQIKAAYLQETGKPLDETLKKALTGHLEEVVLALLKTPAQFDA
DELRAAMKGLGTDEDTLIEILASRTNKEIRDINRVYREELKRDLAKDITSDTSGDFRNALL
SLAKGDRSEDFGVNEDLADSDARALYEAGERRKGTDVNVFNTILTTRSYPQLRRVFQK
YTKYSKHDMNKVLDLELKGDIEKCLTAIVKCATSKPAFFAEKLHQAMKGVGTRHKALIRI
MVSRSEIDMNDIKAFYQKMYGISLCQAILDETKGDYEKILVALCGGN

SEQ ID No:12
VKQIESKTAFQEALDAAGDKLVVVDFSATWCGPCKMIKPFFHSLSEKYSNVIFLEVDVD
DCQDVASECEVKCMPTFQFFKKGQKVGEFSGANKEKLEATINELV

SEQ ID No:13
MSSGNAKIGHPAPNFKATAVMPDGQFKDISLSDYKGKYVVFFFYPLDFTFVCPTEIIAFS
DRAEEFKKLNCQVIGASVDSHFCHLAWVNTPKKQGGLGPMNIPLVSDPKRTIAQDYGV
LKADEGISFRGLFIIDDKGILRQITVNDLPVGRSVDETLRLVQAFQFTDKHGEVCPAGWK
PGSDTIKPDVQKSKEYFSKQK

SEQ ID No:14
MASSSGSSPRPAPDENEFPFGCPPTVCQDPKEPRALCCAGCLSENPRNGEDQICPKC
RGEDLQSISPGSRLRTQEKAHPEVAEAGIGCPFAGVGCSFKGSPQSVQEHEVTSQTSH

LNLLLGFMKQWKARLGCGLESGPMALEQNLSDLQLQAAVEVAGDLEVDCYRAPCSES
QEELALQHFMKEKLLAELEGKLRVFENIVAVLNKEVEASHLALATSIHQSQLDRERILSLE
QRVVELQQTLAQKDQALGKLEQSLRLMEEASFDGTFLWKITNVTRRCHESACGRTVSL
FSPAFYTAKYGYKLCLRLYLNGDGTGKRTHLSLFIVIMRGEYDALLPWPFRNKVTFMLLD
QNNREHAIDAFRPDLSSASFQRPQSETNVASGCPLFFPLSKLQSPKHAYVKDDTMFLK
CIVETST

SEQ ID No:15
MAAASVTPPGSLELLQPGFSKTLLGTKLEAKYLCSACRNVLRRPFQAQCGHRYCSFCL
ASILSSGPQNCAACVHEGIYEEGISILESSSAFPDNAARREVESLPAVCPSDGCTWKGTL
KEYESCHEGRCPLMLTECPACKGLVRLGEKERHLEHECPERSLSCRHCRAPCCGADV
KAHHEVCPKFPLTCDGCGKKKIPREKFQDHVKTCGKCRVPCRFHAIGCLETVEGEKQQ
EHEVQWLREHLAMLLSSVLEAKPLLGDQSHAGSELLQRCESLEKKTATFENIVCVLNRE
VERVAMTAEACSRQHRLDQDKIEALSSKVQQLERSIGLKDLAMADLEQKVRPFQAQCG
HRYCSFCLASILRKLQEAVAGRIPAIFSPAFYTSRYGYKMCLRIYLNGDGTGRGTHLSLF
FVVMKGPNDALLRWPFNQKVTLMLLDQNNREHVIDAFRPDVTSSSFQRPVNDMNIASG
CPLFCPVSKMEAKNSYVRDDAIFIKAIVDLTGL

SEQ ID No:16
MQPDMSLNVIKMKSSDFLESAELDSGGFGKVSLCFHRTQGLMIMKTVYKGPNCIEHNE
ALLEEAKMMNRLRHSRVVKLLGVIIEEGKYSLVMEYMEKGNLMHVLKAEMSTPLSVKGR
IILEIIEGMCYLHGKGVIHKDLKPENILVDNDFHIKIADLGLASFKMWSKLNNEEHNELREV
DGTAKKNGGTLYYMAPEHLNDVNAKPTEKSDVYSFAVVLWAIFANKEPYENAICEQQLI
MCIKSGNRPDVDDITEYCPREIISLMKLCWEANPEARPTFPGIEEKFRPFYLSQLEESVE
EDVKSLKKEYSNENAVVKRMQSLQLDCVAVPSSRSNSATEQPGSLHSSQGLGMGPVE
ESWFAPSLEHPQEENEPSLQSKLQDEANYHLYGSRMDRQTKQQPRQNVAYNREEER
RRRVSHDPFAQQRPYENFQNTEGKGTVYSSAASHGNAVHQPSGLTSQPQVLYQNNG
LYSSHGFGTRPLDPGTAGPRVWYRPIPSHMPSLHNIPVPETNYLGNTPTMPFSSLPPTD
ESIKYTIYNSTGIQIGAYNYMEIGGTSSSLLDSTNTNFKEEPAAKYQAIFDNTTSLTDKHLD
PIRENLGKHWKNCARKLGFTQSQIDEIDHDYERDGLKEKVYQMLQKWVMREGIKGATV
GKLAQALHQCSRIDLLSSLIYVSQN

SEQ ID No:17
MAAGQNGHEEWVGSAYLFVESSLDKVVLSDAYAHPQQKVAVYRALQAALAESGGSPD
VLQMLKIHRSDPQLIVQLRFCGRQPCGRFLRAYREGALRAALQRSLAAALAQHSVPLQL
ELRAGAERLDALLADEERCLSCILAQQPDRLRDEELAELEDALRNLKCGSGARGGDGE
VASAPLQPPVPSLSEVKPPPPPPPAQTFLFQGQPVVNRPLSLKDQQTFARSVGLKWRK
VGRSLQRGCRALRDPALDSLAYEYEREGLYEQAFQLLRRFVQAEGRRATLQRLVEALE
ENELTSLAEDLLGLTDPNGGLA

SEQ ID No:18



























SEQ ID No:19
MFKDVPLTAEEVEFVVEKALSMFSKMNLQEIPPLVYQLLVLSSKGSRKSVLEGIIAFFSAL
DKQHNEEQSGDELLDVVTVPSGELRHVEGTIILHIVFAIKLDYELGRELVKHLKVGQQGD
SNNNLSPFSIALLLSVTRIQRFQDQVLDLLKTSVVKSFKDLQLLQGSKFLQNLVPHRSYV
STMILEVVKNSVHSWDHVTQGLVELGFILMDSYGPKKVLDGKTIETSPSLSRMPNQHAC
KLGANILLETFKIHEMIRQEILEQVLNRVVTRASSPISHFLDLLSNIVMYAPLVLQSCSSKV
TEAFDYLSFLPLQTVQRLLKAVQPLLKVSMSMRDCLILVLRKAMFANQLDARKSAVAGF
LLLLKNFKVLGSLSSSQCSQSLSVSQVHVDVHSHYNSVANETFCLEIMDSLRRCLSQQA
DVRLTLYEGFYDVLRRNSQLANSVMQTLLSQLKQFYEPKPDLLPPLKLEACILTQGDKIS
LQEPLDYLLCCIQHCLAWYKNTVIPLQQGEEEEEEEEAFYEDLDDILESITNRMIKSELED
FELDKSADFSQSTSIGIKNNISAFLVMGVCEVLIEYNFSISSFSKNRFEDILSLFMCYKKLS
DILNEKAGKAKTKMANKTSDSLLSMKFVSSLLTALFRVLLWRYTSIPTSVEESGKKEKGK
SISLLCLEGLQKIFSAVQQFYQPKIQQFLRALDVTDKEGEEREDADVSVTQRTAFQIRQF
QRSLLNLLSSQEEDFNSKEALLLVTVLTSLSKLLEPSSPQFVQMLSWTSKICKENSREDA
LFCKSLMNLLFSLHVSYKSPVILLRDLSQDIHGHLGDIDQDVEVEKTNHFAIVNLRTAAPT
VCLLVLSQAEKVLEEVDWLITKLKGQVSQETLSEASSQATLPNQPVEKAIIMQLGTLLTFF
HELVQTALPSGSCVDTLLKDLCKMYTTLTALVRYYLQVCQSSGGIPKNMEKLVKLSGSH
LTPLCYSFISYVQNKSKSLNYTGEKKEKPAAVATAMARVLRETKPIPNLIFAIEQYEKFLIH
LSKKSKVNLMQHMKLSTSRDFKIKGNILDMVLREDGEDENEEGTASEHGGQNKEPAKK

SEQ ID No:20
MKAVQADQERERQRRLEVEREAEKKREAKQRAKEAQERELRKREKAEEKERRRKEYD
ALKAAKREQEKKPKKEANQAPKSKSGSRPRKPPPRKHTRSWAVLKLLLLLLLFGVAGG
LVACRVTELQQQPLCTSVNTIYDNAVQGLRRHEILQWVLQTDSQQ

SEQ ID No:21










SEQ ID No:22
MDPFLVLLHSVSSSLSSSELTELKFLCLGRVGKRKLERVQSGLDLFSMLLEQNDLEPGH
TELLRELLASLRRHDLLRRVDDFEAGAAAGAAPGEEDLCAAFNVICDNVGKDWRRLAR
QLKVSDTKIDSIEDRYPRNLTERVRESLRIWKNTEKENATVAHLVGALRSCQMNLVADL
VQEVQQARDLQNRSGAMSPMSWNSDASTSEAS

SEQ ID No:23
MNRHLWKSQLCEMVQPSGGPAADQDVLGEESPLGKPAMLHLPSEQGAPETLQRCLE
ENQELRDAIRQSNQILRERCEELLHFQASQREEKEFLMCKFQEARKLVERLGLEKLDLK
RQKEQALREVEHLKRCQQQMAEDKASVKAQVTSLLGELQESQSRLEAATKECQALEG
RARAASEQARQLESEREALQQQHSVQVDQLRMQGQSVEAALRMERQAASEEKRKLA
QLQVAYHQLFQEYDNHIKSSVVGSERKRGMQLEDLKQQLQQAEEALVAKQEVIDKLKE
EAEQHKIVMETVPVLKAQADIYKADFQAERQAREKLAEKKELLQEQLEQLQREYSKLKA
SCQESARIEDMRKRHVEVSQAPLPPAPAYLSSPLALPSQRRSPPEEPPDFCCPKCQYQ
APDMDTLQIHVMECIE

SEQ ID No:24















SEQ ID No:25
MERPPGLRPGAGGPWEMRERLGTGGFGNVCLYQHRELDLKIAIKSCRLELSTKNRER
WCHEIQIMKKLNHANVVKACDVPEELNILIHDVPLLAMEYCSGGDLRKLLNKPENCCGL
KESQILSLLSDIGSGIRYLHENKIIHRDLKPENIVLQDVGGKIIHKIIDLGYAKDVDQGSLCT
SFVGTLQYLAPELFENKPYTATVDYWSFGTMVFECIAGYRPFLHHLQPFTWHEKIKKKD
PKCIFACEEMSGEVRFSSHLPQPNSLCSLIVEPMENWLQLMLNWDPQQRGGPVDLTLK
QPRCFVLMDHILNLKIVHILNMTSAKIISFLLPPDESLHSLQSRIERETGINTGSQELLSET
GISLDPRKPASQCVLDGVRGCDSYMVYLFDKSKTVYEGPFASRSLSDCVNYIVQDSKIQ
LPIIQLRKVWAEAVHYVSGLKEDYSRLFQGQRAAMLSLLRYNANLTKMKNTLISASQQL
KAKLEFFHKSIQLDLERYSEQMTYGISSEKMLKAWKEMEEKAIHYAEVGVIGYLEDQIMS
LHAEIMELQKSPYGRRQGDLMESLEQRAIDLYKQLKHRPSDHSYSDSTEMVKIIVHTVQ
SQDRVLKELFGHLSKLLGCKQKIIDLLPKVEVALSNIKEADNTVMFMQGKRQKEIWHLLKI
ACTQSSARSLVGSSLEGAVTPQTSAWLPPTSAEHDHSLSCVVTPQDGETSAQMIEENL
NCLGHLSTIIHEANEEQGNSMMNLDWSWLTE

SEQ ID No:26
MNIVENSIFLSNLMKSANTFELKYDLSCELYRMSTYSTFPAGVPVSERSLARAGFYYTG
VNDKVKCFCCGLMLDNWKRGDSPTEKHKKLYPSCRFVQSLNSVNNLEATSQPTFPSS
VTNSTHSLLPGTENSGYFRGSYSNSPSNPVNSRANQDFSALMRSSYHCAMNNENARL
LTFQTWPLTFLSPTDLAKAGFYYIGPGDRVACFACGGKLSNWEPKDNAMSEHLRHFPK
CPFIENQLQDTSRYTVSNLSMQTHAARFKTFFNWPSSVLVNPEQLASAGFYYVGNSDD
VKCFCCDGGLRCWESGDDPWVQHAKWFPRCEYLIRIKGQEFIRQVQASYPHLLEQLLS
TSDSPGDENAESSIIHFEPGEDHSEDAIMMNTPVINAAVEMGFSRSLVKQTVQRKILATG
ENYRLVNDLVLDLLNAEDEIREEERERATEEKESNDLLLIRKNRMALFQHLTCVIPILDSL
LTAGIINEQEHDVIKQKTQTSLQARELIDTILVKGNIAATVFRNSLQEAEAVLYEHLFVQQD

IKYIPTEDVSDLPVEEQLRRLQEERTCKVCMDKEVSIVFIPCGHLVVCKDCAPSLRKCPIC
RSTIKGTVRTFLS

SEQ ID No:27
AWKSGGASHSELIHNLRKNGIIKTDKVFEVMLATDRSHYAKCNPYMDSPQSIGFQATISA
PHMHAYALELLFDQLHEGAKALDVGSGSGILTACFARMVGCTGKVIGIDHIKELVDDSIN
NVRKDDPTLLSSGRVQLVVGDGRMGYAEEAPYDAIHVGAAAPVVPQALIDQLKPGGRLI
LPVGPAGGNQMLEQYDKLQDGSIKMKPLMGVIYVPLTDKEKQWSRWK

SEQ ID No:28
MGCKGDASGACAAGALPVTGVCYKMGVLVVLTVLWLFSSVKADSKAITTSLTTKWFST
PLLLEASEFLAEDSQEKFWNFVEASQNIGSSDHDGTDYSYYHAILEAAFQFLSPLQQNL
FKFCLSLRSYSATIQAFQQIAADEPPPEGCNSFFSVHGKKTCESDTLEALLLTASERPKP
LLFKGDHRYPSSNPESPVVIFYSEIGSEEFSNFHRQLISKSNAGKINYVFRHYIFNPRKEP
VYLSGYGVELAIKSTEYKAKDDTQVKGTEVNTTVIGENDPIDEVQGFLFGKLRDLHPDLE
GQLKELRKHLVESTNEMAPLKVWQLQDLSFQTAARILASPVELALVVMKDLSQNFPTKA
RAITKTAVSSELRTEVEENQKYFKGTLGLQPGDSALFINGLHMDLDTQDIFSLFDVLRNE
ARVMEGLHRLGIEGLSLHNVLKLNIQPSEADYAVDIRSPAISWVNNLEVDSRYNSWPSS
LQELLRPTFPGVIRQIRKNLHNMVFIVDPAHETTAELMNTAEMFLSNHIPLRIGFIFVVND
SEDVDGMQDAGVAVLRAYNYVAQEVDDYHAFQTLTHIYNKVRTGEKVKVEHVVSVLEK
KYPYVEVNSILGIDSAYDRNRKEARGYYEQTGVGPLPVVLFNGMPFEREQLDPDELETI
TMHKILETTTFFQRAVYLGELPHDQDVVEYIMNQPNVVPRINSRILTAERDYLDLTASNN
FFVDDYARFTILDSQGKTAAVANSMNYLTKKGMSSKEIYDDSFIRPVTFWIVGDFDSPS
GRQLLYDAIKHQKSSNNVRISMINNPAKEISYENTQISRAIWAALQTQTSNAAKNFITKMA
KEGAAEALAAGADIAEFSVGGMDFSLFKEVFESSKMDFILSHAVYCRDVLKLKKGQRAV
ISNGRIIGPLEDSELFNQDDFHLLENIILKTSGQKIKSHIQQLRVEEDVASDLVMKVDALLS
AQPKGDPRIEYQFFEDRHSAIKLRPKEGETYFDVVAVVDPVTREAQRLAPLLLVLAQLIN
MNLRVFMNCQSKLSDMPLKSFYRYVLEPEISFTSDNSFAKGPIAKFLDMPQSPLFTLNL
NTPESWMVESVRTPYDLDNIYLEEVDSVVAAEYELEYLLLEGHCYDITTGQPPRGLQFT
LGTSANPVIVDTIVMANLGYFQLKANPGAWILRLRKGRSEDIYRIYSHDGTDSPPDADEV
VIVLNNFKSKIIKVKVQKKADMVNEDLLSDGTSENESGFWDSFKWGFTGQKTEEVKQD
KDDIINIFSVASGHLYERFLRIMMLSVLKNTKTPVKFWFLKNYLSPTFKEFIPYMANEYNF

QYELVQYKWPRWLHQQTEKQRIIWGYKILFLDVLFPLVVDKFLFVDADQIVRTDLKELRD
FNLDGAPYGYTPFCDSRREMDGYRFWKSGYWASHLAGRKYHISALYVVDLKKFRKIAA
GDRLRGQYQGLSQDPNSLSNLDQDLPNNMIHQVPIKSLPQEWLWCETWCDDASKKRA
KTIDLCNNPMTKEPKLEAAVRIVPEWQDYDQEIKQLQIRFQKEKETGALYKEKTKEPSRE
GPQKREEL

SEQ ID No:29








SEQ ID No:30
QIVSPVRDSDKPETPPVVNVPVPVLIGPISGMSRPFVRTDSIGEKLGRYSSSEPPIVTDEL
EDLNLWWAAWNTWYRIGSESTKPPITFDKLTFIPSQPFLTALIQIFPALYQHIKTGFNMDD
LQKLGVILHSAISVPISSDASPFILPSYTEAVLTSLQEAVLTALDVLQKAICVGPENMQIMY
PAIFDQLLAFVEFSCKPPQYGQLETKHIANAKYNQIQLFAPAEWVALNYVPFAERSLEVV
VDLYQKTACHKAVVNEKVLQNIIKTLRVPLSLKYSCPSESTWKLAVSSLLRVLSIGLPVAR
QHASSGKFDSMWPELANTFEDFLFTKSIPPDNLSIQEFQRNENIDVEVVQLISNEILPYA
NFIPKEFVGQIMTMLNKGSIHSQSSSFTEAEIDIRLREEFSKMCFETLLQFSFSNKVTTPQ
EGYISRMALSVLLKRSQDVLHRYIEDERLSGKCPLPRQQVTEIIFVLKAVSTLIDSLKKTQ
PENVDGNTWAQVIALYPTLVECITCSSSEVCSALKEALVPFKDFMQPPASRVQNGES

SEQ ID No:31










SEQ ID No:32












SEQ ID No:33
PMFIVNTNVPRASVPDGFLSELTQQLAQATGKPPQYIAVHVVPDQLMAFGGSSEPCAL
CSLHSIGKIGGAQNRSYSKLLCGLLAERLRISPDRVYINYYDMNAANVGWNNSTFA

SEQ ID No:34
MPPAVGGPVGYTPPDGGWGWAVVIGAFISIGFSYAFPKSITVFFKEIEGIFHATTSEVSW
ISSIMLAVMYGGGPISSILVNKYGSRIVMIVGGCLSGCGLIAASFCNTVQQLYVCIGVIGGL
GLAFNLNPALTMIGKYFYKRRPLANGLAMAGSPVFLCTLAPLNQVFFGIFGWRGSFLILG
GLLLNCCVAGALMRPIGPKPTKAGKDKSKASLEKAGKSGVKKDLHDANTDLIGRHPKQE
KRSVFQTINQFLDLTLFTHRGFLLYLSGNVIMFFGLFAPLVFLSSYGKSQHYSSEKSAFLL
SILAFVDMVARPSMGLVANTKPIRPRIQYFFAASVVANGVCHMLAPLSTTYVGFCVYAG
FFGFAFGWLSSVLFETLMDLVGPQRFSSAVGLVTIVECCPVLLGPPLLGRLNDMYGDYK

YTYWACGVVLIISGIYLFIGMGINYRLLAKEQKANEQKKESKEEETSIDVAGKPNEVTKAA
ESPDQKDTEGGPKEEESPV

SEQ ID No:35
MHKTASQRLFPGPSYQNIKSIMEDSTILSDWTNSNKQKMKYDFSCELYRMSTYSTFPA
GVPVSERSLARAGFYYTGVNDKVKCFCCGLMLDNWKLGDSPIQKHKQLYPSCSFIQNL
VSASLGSTSKNTSPMRNSFAHSLSPTLEHSSLFSGSYSSLSPNPLNSRAVEDISSSRTN
PYSYAMSTEEARFLTYHMWPLTFLSPSELARAGFYYIGPGDRVACFACGGKLSNWEPK
DDAMSEHRRHFPNCPFLENSLETLRFSISNLSMQTHAARMRTFMYWPSSVPVQPEQLA
SAGFYYVGRNDDVKCFCCDGGLRCWESGDDPWVEHAKWFPRCEFLIRMKGQEFVDE
IQGRYPHLLEQLLSTSDTTGEENADPPIIHFGPGESSSEDAVMMNTPVVKSALEMGFNR
DLVKQTVQSKILTTGENYKTVNDIVSALLNAEDEKREEEKEKQAEEMASDDLSLIRKNRM
ALFQQLTCVLPILDNLLKANVINKQEHDIIKQKTQIPLQARELIDTILVKGNAAANIFKNCLK
EIDSTLYKNLFVDKNMKYIPTEDVSGLSLEEQLRRLQEERTCKVCMDKEVSVVFIPCGHL
VVCQECAPSLRKCPICRGIIKGTVRTFLS

SEQ ID No:36
STVHEILCKLSLEGDHSTPPSAYGSVKAYTNFDAERDALNIETAIKTKGVDEVTIVNILTN
RSNAQRQDIAFAYQRRTKKELASALKSALSGHLETVILGLLKTPAQYDASELKASMKGL
GTDEDSLIEIICSRTNQELQEINRVYKEMYKTDLEKDIISDTSGDFRKLMVALAKGRRAED
GSVIDYELIDQDARDLYDAGVKRKGTDVPKWISIMTERSVPHLQKVFDRYKSYSPYDML
ESIRKEVKGDLENAFLNLVQCIQNKPLYFADRLYDSMKGKGTRDKVLIRIMVSRSEVDML
KIRSEFKRKYGKSLYYYIQQDTKGDYQKALLYLCGGDD

SEQ ID No:37









SEQ ID No:38
MKVLLAAALIAGSVFFLLLPGPSAADEKKKGPKVTVKVYFDLRIGDEDVGRVIFGLFGKT
VPKTVDNFVALATGEKGFGYKNSKFHRVIKDFMIQGGDFTRGDGTGGKSIYGERFPDE
NFKLKHYGPGWVSMANAGKDTNGSQFFITTVKTAWLDGKHVVFGKVLEGMEVVRKVE
STKTDSRDKPLKDVIIADCGKIEVEKPFAIAKE

SEQ ID No:39




























SEQ ID No:40
MASGSVAECLQQETTCPVCLQYFAEPMMLDCGHNICCACLARCWGTAETNVSCPQCR
ETFPQRHMRPNRHLANVTQLVKQLRTERPSGPGGEMGVCEKHREPLKLYCEEDQMPI
CVVCDRSREHRGHSVLPLEEAVEGFKEQIQNQLDHLKRVKDLKKRRRAQGEQARAELL
SLTQMEREKIVWEFEQLYHSLKEHEYRLLARLEELDLAIYNSINGAITQFSCNISHLSSLIA
QLEEKQQQPTRELLQDIGDTLSRAERIRIPEPWITPPDLQEKIHIFAQKCLFLTESLKQFT
EKMQSDMEKIQELREAQLYSVDVTLDPDTAYPSLILSDNLRQVRYSYLQQDLPDNPERF
NLFPCVLGSPCFIAGRHYWEVEVGDKAKWTIGVCEDSVCRKGGVTSAPQNGFWAVSL
WYGKEYWALTSPMTALPLRTPLQRVGIFLDYDAGEVSFYNVTERCHTFTFSHATFCGP
VRPYFSLSYSGGKSAAPLIICPMSGIDGFSGHVGNHGHSMETSP

SEQ ID No:41










SEQ ID No:42



















SEQ ID No:43
MKSSGLFPFLVLLALGTLAPWAVEGSGKSFKAGVCPPKKSAQCLRYKKPECQSDWQC
PGKKRCCPDTCGIKCLDPVDTPNPTRRKPGKCPVTYGQCLMLNPPNFCEMDGQCKRD
LKCCMGMCGKSCVSPVKA

SEQ ID No:44










SEQ ID No:45
MGKKYKNIVLLKGLEVINDYHFRMVKSLLSNDLKLNLKMREEYDKIQIADLMEEKFRGDA
GLGKLIKIFEDIPTLEDLAETLKKEKLKVKGPALSRKRKKEVDATSPAPSTSSTVKTEGAE
ATPGAQKRKKSTKEKAGPKGSKVSEEQTQPPSPAGAGMSTAMGRSPSPKTSLSAPPN
TSSTENPKTVAKCQVTPRRNVLQKRPVIVKVLSTTKPFEYETPEMEKKIMFHATVATQT
QFFHVKVLNTSLKEKFNGKKIIIISDYLEYDSLLEVNEESTVSEAGPNQTFEVPNKIINRAK
ETLKIDILHKQASGNIVYGVFMLHKKTVNQKTTIYEIQDDRGKMDVVGTGQCHNIPCEEG
DKLQLFCFRLRKKNQMSKLISEMHSFIQIKKKTNPRNNDPKSMKLPQEQSQLPNPSEAS
TTFPESHLRTPQMPPTTPSSSFFTKKSEDTISKMNDFMRMQILKEGSHFPGPFMTSIGP
AESHPHTPQMPPSTPSSSFLTTLKPRLKTEPEEVSIEDSAQSDLKEVMVLNATESFVYE
PKEQKKMFHATVATENEVFRVKVFNIDLKEKFTPKKIIAIANYVCRNGFLEVYPFTLVADV
NADRNMEIPKGLIRSASVTPKINQLCSQTKGSFVNGVFEVHKKNVRGEFTYYEIQDNTG
KMEVVVHGRLTTINCEEGDKLKLTCFELAPKSGNTGELRSVIHSHIKVIKTRKNKKDILNP
DSSMETSPDFFF

SEQ ID No:46









SEQ ID No:47
MLWKITDNVKYEEDCEDRHDGSSNGNPRVPHLSSAGQHLYSPAPPLSHTGVAEYQPP
PYFPPPYQQLAYSQSADPYSHLGEAYAAAINPLHQPAPTGSQQQAWPGRQSQEGAGL
PSHHGRPAGLLPHLSGLEAGAVSARRDAYRRSDLLLPHAHALDAAGLAENLGLHDMPH
QMDEVQNVDDQHLLLHDQTVIRKGPISMTKNPLNLPCQKELVGAVMNPTEVFCSVPGR
LSLLSSTSKYKVTVAEVQRRLSPPECLNASLLGGVLRRAKSKNGGRSLREKLDKIGLNL
PAGRRKAAHVTLLTSLVEGEAVHLARDFAYVCEAEFPSKPVAEYLTRPHLGGRNEMAA

RKNMLLAAQQLCKEFTELLSQDRTPHGTSRLAPVLETNIQNCLSHFSLITHGFGSQAICA
AVSALQNYIKEALIVIDKSYMNPGDQSPADSNKTLEKMEKHRK

SEQ ID No:48
MDDKAFTKELDQWVEQLNECKQLNENQVRTLCEKAKEILTKESNVQEVRCPVTVCGDV
HGQFHDLMELFRIGGKSPDTNYLFMGDYVDRGYYSVETVTLLVALKVRYPERITILRGN
HESRQITQVYGFYDECLRKYGNANVWKYFTDLFDYLPLTALVDGQIFCLHGGLSPSIDTL
DHIRALDRLQEVPHEGPMCDLLWSDPDDRGGWGISPRGAGYTFGQDISETFNHANGL
TLVSRAHQLVMEGYNWCHDRNVVTIFSAPNYCYRCGNQAAIMELDDTLKYSFLQFDPA
PRRGEPHVTRRTPDYFL

SEQ ID No:49
MAQRAFPNPYADYNKSLAEGYFDAAGRLTPEFSQRLTNKIRELLQQMERGLKSADPRD
GTGYTGWAGIAVLYLHLYDVFGDPAYLQLAHGYVKQSLNCLTKRSITFLCGDAGPLAVA
AVLYHKMNNEKQAEDCITRLIHLNKIDPHAPNEMLYGRIGYIYALLFVNKNFGVEKIPQSH
IQQICETILTSGENLARKRNFTAKSPLMYEWYQEYYVGAAHGLAGIYYYLMQPSLQVSQ
GKLHSLVKPSVDYVCQLKFPSGNYPPCIGDNRDLLVHWCHGAPGVIYMLIQAYKVFREE
KYLCDAYQCADVIWQYGLLKKGYGLCHGSAGNAYAFLTLYNLTQDMKYLYRACKFAEW
CLEYGEHGCRTPDTPFSLFEGMAGTIYFLADLLVPTKARFPAFEL

SEQ ID No:50








SEQ ID No:51
MSGARGEGPEAGAGGAGGRAAPENPGGVLSVELPGLLAQLARSFALLLPVYALGYLGL
SFSWVLLALALLAWCRRSRGLKALRLCRALALLEDEERVVRLGVRACDLPAWVHFPDT
ERAEWLNKTVKHMWPFICQFIEKLFRETIEPAVRGANTHLSTFSFTKVDVGQQPLRING
VKVYTENVDKRQIILDLQISFVGNCEIDLEIKRYFCRAGVKSIQIHGTMRVILEPLIGDMPL
VGALSIFFLRKPLLEINWTGLTNLLDVPGLNGLSDTIILDIISNYLVLPNRITVPLVSEVQIAQ
LRFPVPKGVLRIHFIEAQDLQGKDTYLKGLVKGKSDPYGIIRVGNQIFQSRVIKENLSPKW
NEVYEALVYEHPGQELEIELFDEDPDKDDFLGSLMIDLIEVEKERLLDEWFTLDEVPKGK
LHLRLEWLTLMPNASNLDKVLTDIKADKDQANDGLSSALLILYLDSARNLPSGKKISSNP
NPVVQMSVGHKAQESKIRYKTNEPVWEENFTFFIHNPKRQDLEVEVRDEQHQCSLGNL
KVPLSQLLTSEDMTVSQRFQLSNSGPNSTIKMKIALRVLHLEKRERPPDHQHSAQVKRP
SVSKEGRKTSIKSHMSGSPGPGGSNTAPSTPVIGGSDKPGMEEKAQPPEAGPQGLHD
LGRSSSSLLASPGHISVKEPTPSIASDISLPIATQELRQRLRQLENGTTLGQSPLGQIQLTI
RHSSQRNKLIVVVHACRNLIAFSEDGSDPYVRMYLLPDKRRSGRRKTHVSKKTLNPVFD
QSFDFSVSLPEVQRRTLDVAVKNSGGFLSKDKGLLGKVLVALASEELAKGWTQWYDLT
EDGTRPQAMT

SEQ ID No:52
MAPVAVWAALAVGLELWAAAHALPAQVAFTPYAPEPGSTCRLREYYDQTAQMCCSKC
SPGQHAKVFCTKTSDTVCDSCEDSTYTQLWNWVPECLSCGSRCSSDQVETQACTRE
QNRICTCRPGWYCALSKQEGCRLCAPLRKCRPGFGVARPGTETSDVVCKPCAPGTFS
NTTSSTDICRPHQICNVVAIPGNASMDAVCTSTSPTRSMAPGAVHLPQPVSTRSQHTQP
TPEPSTAPSTSFLLPMGPSPPAEGSTGDFALPVGLIVGVTALGLLIIGVVNCVIMTQVKKK
PLCLQREAKVPHLPADKARGTQGPEQQHLLITAPSSSSSSLESSASALDRRAPTRNQP
QAPGVEASGAGEARASTGSSDSSPGGHGTQVNVTCIVNVCSSSDHSSQCSSQASSTM
GDTDSSPSESPKDEQVPFSKEECAFRSQLETPETLLGSTEEKPLPLGVPDAGMKPS

SEQ ID No:53
MPAPRAPRALAAAAPASGKAKLTHPGKAILAGGLAGGIEICITFPTEYVKTQLQLDERSH
PPRYRGIGDCVRQTVRSHGVLGLYRGLSSLLYGSIPKAAVRFGMFEFLSNHMRDAQGR
LDSTRGLLCGLGAGVAEAVVVVCPMETIKVKFIHDQTSPNPKYRGFFHGVREIVREQGL
KGTYQGLTATVLKQGSNQAIRFFVMTSLRNWYRGDNPNKPMNPLITGVFGAIAGAASV

FGNTPLDVIKTRMQGLEAHKYRNTWDCGLQILKKEGLKAFYKGTVPRLGRVCLDVAIVF
VIYDEVVKLLNKVWKTD

SEQ ID No:54
MDGEDIPDFSSLKEETAYWKELSLKYKQSFQEARDELVEFQEGSRELEAELEAQLVQA
EQRNRDLQADNQRLKYEVEALKEKLEHQYAQSYKQVSVLEDDLSQTRAIKEQLHKYVR
ELEQANDDLERAKRATIVSLEDFEQRLNQAIERNAFLESELDEKESLLVSVQRLKDEARD
LRQELAVRERQQEVTRKSAPSSPTLDCEKMDSAVQASLSLPATPVGKGTENSFPSPKAI
PNGFSTSPLTPSARISALNIVGDLLRKVGALESKLAACRNFAKDQASRKSYISGNVNCGV
LNGNGTKFSRSGHTSFFDKGAVNGFDPAPPPPGLGSSRPLSAPGMLPLSV

SEQ ID No:55
MSQRQGKEAYPTPTKDLHQPSLSPASPHSQGFERGKEDISQNKDESSLSMSKSKSES
KLYNGSEKDSSTSSKLTKKESLKVQKKNYREEKKRATKELLSTITDPSVIVMADWLKIRG
TLKSWTKLWCVLKPGVLLIYKTQKNGQWVGTVLLNACEIIERPSKKDGFCFKLFHPLEQ
SIWAVKGPKGEAVGSITQPLPSSYLIIRATSESDGRCWMDALELALKCSSLLKRTMIREG
KEHDLSVSSDSTHVTFYGLLRANNLHSGDNFQLNDSEIERQHFKDQDMYSDKSDKEND
QEHDESDNEVMGKSEESDTDTSERQDDSYIEPEPVEPLKETTYTEQSHEELGEAGEAS
QTETVSEENKSLIWTLLKQVRPGMDLSKVVLPTFILEPRSFLDKLSDYYYHADFLSEAAL
EENPYFRLKKVVKWYLSGFYKKPKGLKKPYNPILGETFRCLWIHPRTNSKTFYIAEQVSH
HPPISAFYVSNRKDGFCLSGSILAKSKFYGNSLSAILEGEARLTFLNRGEDYVMTMPYAH
CKGILYGTMTLELGGTVNITCQKTGYSAILEFKLKPFLGSSDCVNQISGKLKLGKEVLATL
EGHWDSEVFITDKKTDNSEVFWNPTPDIKQWRLIRHTVKFEEQGDFESEKLWQRVTRA
INAKDQTEATQEKYVLEEAQRQAARDRKTKNEEWSCKLFELDPLTGEWHYKFADTRP
WDPLNDMIQFEKDGVIQTKVKHRTPMVSVPKMKHKPTRQQKKVAKGYSSPEPDIQDSS
GSEAQSVKPSTRRKKGIELGDIQSSIESIKQTQEEIKRNIMALRNHLVSSTPATDYFLQQK
DYFIIFLLILLQVIINFMFK

SEQ ID No:56
MMLSLNNLQNIIYNPVIPFVGTIPDQLDPGTLIVIRGHVPSDADRFQVDLQNGSSMKPRA
DVAFHFNPRFKRAGCIVCNTLINEKWGREEITYDTPFQKEKKSFEIVIMVLKAKFQVAVN

GKHTLLYGHRIGPEKIDTLGIYGKVNIHSIGFSFSSDLQSTQASSLELTEISRENVPKSGT
PQLRLPFAARLNTPMGPGRTVVVKGEVNANAKSFNVDLLAGKSKDIALHLNPRLNIKAF
VRNSFLQESWGEEERNITSFPFSPGMYFEMIIYCDVREFKVAVNGVHSLEYKHRFKELS
SIDTLEINGDIHLLEVRSW

SEQ ID No:57









SEQ ID No:58
MPVTEKDLAEDAPWKKIQQNTFTRWCNEHLKCVNKRIGNLQTDLSDGLRLIALLEVLSQ
KRMYRKYHQRPTFRQMQLENVSVALEFLDRESIKLVSIDSKAIVDGNLKLILGLVWTLILH
YSISMPVWEDEGDDDAKKQTPKQRLLGWIQNKIPYLPITNFNQNWQDGKALGALVDSC
APGLCPDWESWDPQKPVDNAREAMQQADDWLGVPQVITPEEIIHPDVDEHSVMTYLS
QFPKAKLKPGAPLKPKLNPKKARAYGRGIEPTGNMVKQPAKFTVDTISAGQGDVMVFV
EDPEGNKEEAQVTPDSDKNKTYSVEYLPKVTGLHKVTVLFAGQHISKSPFEVSVDKAQ
GDASKVTAKGPGLEAVGNIANKPTYFDIYTAGAGVGDIGVEVEDPQGKNTVELLVEDKG
NQVYRCVYKPMQPGPHVVKIFFAGDTIPKSPFVVQVGEACNPNACRASGRGLQPKGV
RIRETTDFKVDTKAAGSGELGVTMKGPKGLEELVKQKDFLDGVYAFEYYPSTPGRYSIAI
TWGGHHIPKSPFEVQVGPEAGMQKVRAWGPGLHGGIVGRSADFVVESIGSEVGSLGF
AIEGPSQAKIEYNDQNDGSCDVKYWPKEPGEYAVHIMCDDEDIKDSPYMAFIHPATGGY
NPDLVRAYGPGLEKSGCIVNNLAEFTVDPKDAGKAPLKIFAQDGEGQRIDIQMKNRMDG
TYACSYTPVKAIKHTIAVVWGGVNIPHSPYRVNIGQGSHPQKVKVFGPGVERSGLKANE
PTHFTVDCTEAGEGDVSVGIKCDARVLSEDEEDVDFDIIHNANDTFTVKYVPPAAGRYTI
KVLFASQEIPASPFRVKVDPSHDASKVKAEGPGLSKAGVENGKPTHFTVYTKGAGKAPL
NVQFNSPLPGDAVKDLDIIDNYDYSHTVKYTPTQQGNMQVLVTYGGDPIPKSPFTVGVA

APLDLSKIKLNGLENRVEVGKDQEFTVDTRGAGGQGKLDVTILSPSRKVVPCLVTPVTG
RENSTAKFIPREEGLYAVDVTYDGHPVPGSPYTVEASLPPDPSKVKAHGPGLEGGLVG
KPAEFTIDTKGAGTGGLGLTVEGPCEAKIECSDNGDGTCSVSYLPTKPGEYFVNILFEEV
HIPGSPFKADIEMPFDPSKVVASGPGLEHGKVGEAGLLSVNCSEAGPGALGLEAVSDS
GTKAEVSIQNNKDGTYAVTYVPLTAGMYTLTMKYGGELVPHFPARVKVEPAVDTSRIKV
FGPGIEGKDVFREATTDFTVDSRPLTQVGGDHIKAHIANPSGASTECFVTDNADGTYQV
EYTPFEKGLHVVEVTYDDVPIPNSPFKVAVTEGCQPSRVQAQGPGLKEAFTNKPNVFT
VVTRGAGIGGLGITVEGPSESKINCRDNKDGSCSAEYIPFAPGDYDVNITYGGAHIPGSP
FRVPVKDVVDPSKVKIAGPGLGSGVRARVLQSFTVDSSKAGLAPLEVRVLGPRGLVEP
VNMVDNGDGTHTVTYTPSQEGPYMVSVKYADEEIPRSPFKVKVLPTYDASKVTASGPG
LSSYGVPASLPVDFAIDARDAGEGLLAVQITDQEGKPKRAIVHDNKDGTYAVTYIPDKTG
RYMIGVTYGGDDIPLSPYRIRATQTGDASKCLATGPGIASTVKTGEEVGFVVDAKTAGK
GKVTCTVLTPDGTEAEADVIENEDGTYDIFYTAAKPGTYVIYVRFGGVDIPNSPFTVMAT
DGEVTAVEEAPVNACPPGFRPWVTEEAYVPVSDMNGLGFKPFDLVIPFAVRKGEITGE
VHMPSGKTATPEIVDNKDGTVTVRYAPTEVGLHEMHIKYMGSHIPESPLQFYVNYPNSG
SVSAYGPGLVYGVANKTATFTIVTEDAGEGGLDLAIEGPSKAEISCIDNKDGTCTVTYLP
TLPGDYSILVKYNDKHIPGSPFTAKITDDSRRCSQVKLGSAADFLLDISETDLSSLTASIKA
PSGRDEPCLLKRLPNNHIGISFIPREVGEHLVSIKKNGNHVANSPVSIMVVQSEIGDARR
AKVYGRGLSEGRTFEMSDFIVDTRDAGYGGISLAVEGPSKVDIQTEDLEDGTCKVSYFP
TVPGVYIVSTKFADEHVPGSPFTVKISGEGRVKESITRTSRAPSVATVGSICDLNLKIPEIN
SSDMSAHVTSPSGRVTEAEIVPMGKNSHCVRFVPQEMGVHTVSVKYRGQHVTGSPFQ
FTVGPLGEGGAHKVRAGGPGLERGEAGVPAEFSIWTREAGAGGLSIAVEGPSKAEITF
DDHKNGSCGVSYIAQEPGNYEVSIKFNDEHIPESPYLVPVIAPSDDARRLTVMSLQESG
LKVNQPASFAIRLNGAKGKIDAKVHSPSGAVEECHVSELEPDKYAVRFIPHENGVHTIDV
KFNGSHVVGSPFKVRVGEPGQAGNPALVSAYGTGLEGGTTGIQSEFFINTTRAGPGTL
SVTIEGPSKVKMDCQETPEGYKVMYTPMAPGNYLISVKYGGPNHIVGSPFKAKVTGQR
LVSPGSANETSSILVESVTRSSTETCYSAIPKASSDASKVTSKGAGLSKAFVGQKSSFLV
DCSKAGSNMLLIGVHGPTTPCEEVSMKHVGNQQYNVTYVVKERGDYVLAVKWGEEHI
PGSPFHVTVP

SEQ ID No:59
MPWLLSAPKLVPAVANVRGLSGCMLCSQRRYSLQPVPERRIPNRYLGQPSPFTHPHLL
RPGEVTPGLSQVEYALRRHKLMSLIQKEAQGQSGTDQTVVVLSNPTYYMSNDIPYTFH

QDNNFLYLCGFQEPDSILVLQSLPGKQLPSHKAILFVPRRDPSRELWDGPRSGTDGAIA
LTGVDEAYTLEEFQHLLPKMKAETNMVWYDWMRPSHAQLHSDYMQPLTEAKAKSKNK
VRGVQQLIQRLRLIKSPAEIERMQIAGKLTSQAFIETMFTSKAPVEEAFLYAKFEFECRAR
GADILAYPPVVAGGNRSNTLHYVKNNQLIKDGEMVLLDGGCESSCYVSDITRTWPVNG
RFTAPQAELYEAVLEIQRDCLALCFPGTSLENIYSMMLTLIGQKLKDLGIMKNIKENNAFK
AARKYCPHHVGHYLGMDVHDTPDMPRSLPLQPGMVITIEPGIYIPEDDKDAPEKFRGLG
VRIEDDVVVTQDSPLILSADCPKEMNDIEQICSQAS

SEQ ID No:60
MSSSHSRAGQSAAGAAPGGGVDTRDAEMPATEKDLAEDAPWKKIQQNTFTRWCNEH
LKCVSKRIANLQTDLSDGLRLIALLEVLSQKKMHRKHNQRPTFRQMQLENVSVALEFLD
RESIKLVSIDSKAIVDGNLKLILGLIWTLILHYSISMPMWDEEEDEEAKKQTPKQRLLGWIQ
NKLPQLPITNFSRDWQSGRALGALVDSCAPGLCPDWDSWDASKPVTNAREAMQQAD
DWLGIPQVITPEEIVDPNVDEHSVMTYLSQFPKAKLKPGAPLRPKLNPKKARAYGPGIEP
TGNMVKKRAEFTVETRSAGQGEVLVYVEDPAGHQEEAKVTANNDKNRTFSVWYVPEV
TGTHKVTVLFAGQHIAKSPFEVYVDKSQGDASKVTAQGPGLEPSGNIANKTTYFEIFTA
GAGTGEVEVVIQDPMGQKGTVEPQLEARGDSTYRCSYQPTMEGVHTVHVTFAGVPIP
RSPYTVTVGQACNPSACRAVGRGLQPKGVRVKETADFKVYTKGAGSGELKVTVKGPK
GEERVKQKDLGDGVYGFEYYPMVPGTYIVTITWGGQNIGRSPFEVKVGTECGNQKVRA
WGPGLEGGVVGKSADFVVEAIGDDVGTLGFSVEGPSQAKIECDDKGDGSCDVRYWPQ
EAGEYAVHVLCNSEDIRLSPFMADIRDAPQDFHPDRVKARGPGLEKTGVAVNKPAEFT
VDAKHGGKAPLRVQVQDNEGCPVEALVKDNGNGTYSCSYVPRKPVKHTAMVSWGGV
SIPNSPFRVNVGAGSHPNKVKVYGPGVAKTGLKAHEPTYFTVDCAEAGQGDVSIGIKCA
PGVVGPAEADIDFDIIRNDNDTFTVKYTPRGAGSYTIMVLFADQATPTSPIRVKVEPSHD
ASKVKAEGPGLSRTGVELGKPTHFTVNAKAAGKGKLDVQFSGLTKGDAVRDVDIIDHH
DNTYTVKYTPVQQGPVGVNVTYGGDPIPKSPFSVAVSPSLDLSKIKVSGLGEKVDVGKD
QEFTVKSKGAGGQGKVASKIVGPSGAAVPCKVEPGLGADNSVVRFLPREEGPYEVEVT
YDGVPVPGSPFPLEAVAPTKPSKVKAFGPGLQGGSAGSPARFTIDTKGAGTGGLGLTV
EGPCEAQLECLDNGDGTCSVSYVPTEPGDYNINILFADTHIPGSPFKAHVVPCFDASKV
KCSGPGLERATAGEVGQFQVDCSSAGSAELTIEICSEAGLPAEVYIQDHGDGTHTITYIP
LCPGAYTVTIKYGGQPVPNFPSKLQVEPAVDTSGVQCYGPGIEGQGVFREATTEFSVD
ARALTQTGGPHVKARVANPSGNLTETYVQDRGDGMYKVEYTPYEEGLHSVDVTYDGS
PVPSSPFQVPVTEGCDPSRVRVHGPGIQSGTTNKPNKFTVETRGAGTGGLGLAVEGPS

EAKMSCMDNKDGSCSVEYIPYEAGTYSLNVTYGGHQVPGSPFKVPVHDVTDASKVKC
SGPGLSPGMVRANLPQSFQVDTSKAGVAPLQVKVQGPKGLVEPVDVVDNADGTQTVN
YVPSREGPYSISVLYGDEEVPRSPFKVKVLPTHDASKVKASGPGLNTTGVPASLPVEFTI
DAKDAGEGLLAVQITDPEGKPKKTHIQDNHDGTYTVAYVPDVTGRYTILIKYGGDEIPFS
PYRVRAVPTGDASKCTVTVSIGGHGLGAGIGPTIQIGEETVITVDTKAAGKGKVTCTVCT
PDGSEVDVDVVENEDGTFDIFYTAPQPGKYVICVRFGGEHVPNSPFQVTALAGDQPSV
QPPLRSQQLAPQYTYAQGGQQTWAPERPLVGVNGLDVTSLRPFDLVIPFTIKKGEITGE
VRMPSGKVAQPTITDNKDGTVTVRYAPSEAGLHEMDIRYDNMHIPGSPLQFYVDYVNC
GHVTAYGPGLTHGVVNKPATFTVNTKDAGEGGLSLAIEGPSKAEISCTDNQDGTCSVS
YLPVLPGDYSILVKYNEQHVPGSPFTARVTGDDSMRMSHLKVGSAADIPINISETDLSLL
TATVVPPSGREEPCLLKRLRNGHVGISFVPKETGEHLVHVKKNGQHVASSPIPVVISQS
EIGDASRVRVSGQGLHEGHTFEPAEFIIDTRDAGYGGLSLSIEGPSKVDINTEDLEDGTC
RVTYCPTEPGNYIINIKFADQHVPGSPFSVKVTGEGRVKESITRRRRAPSVANVGSHCD
LSLKIPEISIQDMTAQVTSPSGKTHEAEIVEGENHTYCIRFVPAEMGTHTVSVKYKGQHV
PGSPFQFTVGPLGEGGAHKVRAGGPGLERAEAGVPAEFSIWTREAGAGGLAIAVEGPS
KAEISFEDRKDGSCGVAYVVQEPGDYEVSVKFNEEHIPDSPFVVPVASPSGDARRLTVS
SLQESGLKVNQPASFAVSLNGAKGAIDAKVHSPSGALEECYVTEIDQDKYAVRFIPREN
GVYLIDVKFNGTHIPGSPFKIRVGEPGHGGDPGLVSAYGAGLEGGVTGNPAEFVVNTS
NAGAGALSVTIDGPSKVKMDCQECPEGYRVTYTPMAPGSYLISIKYGGPYHIGGSPFKA
KVTGPRLVSNHSLHETSSVFVDSLTKATCAPQHGAPGPGPADASKVVAKGLGLSKAYV
GQKSSFTVDCSKAGNNMLLVGVHGPRTPCEEILVKHVGSRLYSVSYLLKDKGEYTLVV
KWGHEHIPGSPYRVVVP

SEQ ID No:61
MSLFDLFRGFFGFPGPRSHRDPFFGGMTRDEDDDEEEEEEGGSWGRGNPRFHSPQH
PPEEFGFGFSFSPGGGIRFHDNFGFDDLVRDFNSIFSDMGAWTLPSHPPELPGPESET
PGERLREGQTLRDSMLKYPDSHQPRIFGGVLESDARSESPQPAPDWGSQRPFHRFDD
VWPMDPHPRTREDNDLDSQVSQEGLGPVLQPQPKSYFKSISVTKITKPDGIVEERRTVV
DSEGRTETTVTRHEADSSPRGDPESPRPPALDDAFSILDLFLGRWFRSR

SEQ ID No:62
MAAVRMLRTWSRNAGKLICVRYFQTCGNVHVLKPNYVCFFGYPSFKYSHPHHFLKTTA

ALRGQVVQFKLSDIGEGIREVTVKEWYVKEGDTVSQFDSICEVQSDKASVTITSRYDGVI
KKLYYNLDDIAYVGKPLVDIETEALKDSEEDVVETPAVSHDEHTHQEIKGRKTLATPAVR
RLAMENNIKLSEVVGSGKDGRILKEDILNYLEKQTGAILPPSPKVEIMPPPPKPKDMTVPI
LVSKPPVFTGKDKTEPIKGFQKAMVKTMSAALKIPHFGYCDEIDLTELVKLREELKPIAFA
RGIKLSFMPFFLKAASLGLLQFPILNASVDENCQNITYKASHNIGIAMDTEQGLIVPNVKN
VQICSIFDIATELNRLQKLGSVGQLSTTDLTGGTFTLSNIGSIGGTFAKPVIMPPEVAIGAL
GSIKAIPRFNQKGEVYKAQIMNVSWSADHRVIDGATMSRFSNLWKSYLENPAFMLLDLK

SEQ ID No:63







SEQ ID No:64
MPATASAGVPATVSEKQEFYQLLKNLINPSCMVRRQAEEIYENIPGLCKTTFLLDAVRNR
RAGYEVRQMAAALLRRLLSSGFEEVYPNLPADVQRDVKIELILAVKLETHASMRKKLCDI
FAVLARNLIDEDGTNHWPEGLKFLIDSIYSKNVVLWEVALHVFWHFPGIFGTQERHDLDII
KRLLDQCIQDQEHPAIRTLSARAAAAFVLANENNIALFKDFADLLPGILQAVNDSCYQDD
DSVLESLVEIADTVPKYLGPYLEDTLQLSLKLCGDSRLSNLQRQLALEVIVTLSETATPML
KKHTNIIAQAVPHILAMMVDLQDDEDWVNADEMEEDDFDSNAVAAESALDRLACGLGG
KVVLPMTKEHIMQMLQSPDWKYRHAGLMALSAIGEGCHQQMESILDETVNSVLLFLQD
PHPRVRAAACTTLGQMATDFAPNFQKKFHETVIAALLRTMENQGNQRVQSHAASALIIFI
EDCPKSLLVLYVDSMVKNLHSVLVIKLQELIRNGTKLALEQLVTTIASVADTIEEKFVPYYD
IFMPSLKHIVELAVQKELKLLRGKTIECISHIGLAVGKEKFMQDASNVMQLLLKTQSDLNN
MEDDDPQTSYMVSAWARMCKILGKDFQQYLPLVIEPLIKTASAKPDVALLDTQDVENMS
DDDGWQFVNLGDQQSFGIKTSGLEAKATACQMLVYYAKELREGFVEYTEQVVKLMVPL
LKFYFHDNVRVAAAESMPFLLECARIRGPEYLAQMWQFICDPLIKAIGTEPDTDVLSEIM
NSFAKSIEVMGDGCLNDEHLEELGGILKAKLEGHFKNQELRQVKRQEENYDQQVEMSL
QDEDECDVYILTKVSDILHSLFSTYKEKILPWFEQLLPLIVNLICSSRPWPDRQWGLCIFD

DIIEHCSPTSFKYVEYFRWPMLLNMRDNNPEVRQAAAYGLGVMAQFGGDDYRSLCSE
AVPLLVKVIKCANSKTKKNVIATENCISAIGKILKFKPNCVNVDEVLPHWLSWLPLHEDKE
EAIQTLSFLCDLIESNHPVVIGPNNSNLPKIISIIAEGKINETINYEDPCAKRLANVVRQVQT
SEDLWLECVSQLDDEQQEALQELLNFA

SEQ ID No:65
MFHLRTCAAKLRPLTASQTVKTFSQNRPAAARTFQQIRCYSAPVAAEPFLSGTSSNYVE
EMYCAWLENPKSVHKSWDIFFRNTNAGAPPGTAYQSPLPLSRGSLAAVAHAQSLVEAQ
PNVDKLVEDHLAVQSLIRAYQIRGHHVAQLDPLGILDADLDSSVPADIISSTDKLGFYGLD
ESDLDKVFHLPTTTFIGGQESALPLREIIRRLEMAYCQHIGVEFMFINDLEQCQWIRQKFE
TPGIMQFTNEEKRTLLARLVRSTRFEEFLQRKWSSEKRFGLEGCEVLIPALKTIIDKSSEN
GVDYVIMGMPHRGRLNVLANVIRKELEQIFCQFDSKLEAADEGSGDVKYHLGMYHRRIN
RVTDRNITLSLVANPSHLEAADPVVMGKTKAEQFYCGDTEGKKVMSILLHGDAAFAGQ
GIVYETFHLSDLPSYTTHGTVHVVVNNQIGFTTDPRMARSSPYPTDVARVVNAPIFHVN
SDDPEAVMYVCKVAAEWRSTFHKDVVVDLVCYRRNGHNEMDEPMFTQPLMYKQIRKQ
KPVLQKYAELLVSQGVVNQPEYEEEISKYDKICEEAFARSKDEKILHIKHWLDSPWPGFF
TLDGQPRSMSCPSTGLTEDILTHIGNVASSVPVENFTIHGGLSRILKTRGEMVKNRTVD
WALAEYMAFGSLLKEGIHIRLSGQDVERGTFSHRHHVLHDQNVDKRTCIPMNHLWPNQ
APYTVCNSSLSEYGVLGFEAGLRMASPNALVLWEAQFGDFHNTAQCIIDQFICPGQAK
WVRQNGIVLLLPHGMEGMGPEHSSARPERFLQMCNDDPDVLPDLKEANFDINQLYDC
NWVVVNCSTPGNFFHVLRRQILLPFRKPLIIFTPKSLLRHPEARSSFDEMLPGTHFQRVI
PEDGPAAQNPENVKRLLFCTGKVYYDLTRERKARDMVGQVAITRIEQLSPFPFDLLLKE
VQKYPNAELAWCQEEHKNQGYYDYVKPRLRTTISRAKPVWYAGRNPAAAPATGNKKT
H

SEQ ID No:66
MGLKAAQKTLFPLRSIDDVVRLFAAELGREEPDLVLLSLVLGFVEHFLAVNRVIPTNVPEL
TFQPSPAPDPPGGLTYFPVADLSIIAALYARFTAQIRGAVDLSLYPREGGVSSRELVKKV
SDVIWNSLSRSYFKDRAHIQSLFSFITGTKLDSSGVAFAVVGACQALGLRDVHLALSEDH
AWVVFGPNGEQTAEVTWHGKGNEDRRGQTVNAGVAERSWLYLKGSYMRCDRKMEV
AFMVCAINPSIDLHTDSLELLQLQQKLLWLLYDLGHLERYPMALGNLADLEELEPTPGRP
DPLTLYHKGIASAKTYYQDEHIYPYMYLAGYHCRNRNVREALQAWADTATVIQDYNYCR

EDEEIYKEFFEVANDVIPNLLKEAASLLETGEERTGEQAQGTQGQGSALQDPECFAHLL
RFYDGICKWEEGSPTPVLHVGWATFLVQSLGRFEGQVRQKVHIVSREAEAAEAEEPW
GDEAREGRRRGPRRESKPEEPPPPKKPALDKGPGSGQSAGSGPPRKTSGTVPGTTR
GGQEVGNAAQAPAPAASPPPEGPVLTFQSEKMKGMKELLVATKINSSAIKLQLTAQSQ
VQMKKQKVSTPSDYTLSFLKRQRKGL

SEQ ID No:67
MSGSTQPVAQTWRATEPRYPPHSLSYPVQIARTHTDVGLLEYQHHSRDYASHLSPGSII
QPQRRRPSLLSEFQPGNERSQELHLRPESHSYLPELGKSEMEFIESKRPRLELLPDPLL
RPSPLLATGQPAGSEDLTKDRSLTGKLEPVSPPSPPHTDPELELVPPRLSKEELIQNMD
RVDREITMVEQQISKLKKKQQQLEEEAAKPPEPEKPVSPPPIESKHRSLVQIIYDENRKK
AEAAHRILEGLGPQVELPLYNQPSDTRQYHENIKINQAMRKKLILYFKRRNHARKQWEQ
KFCQRYDQLMEAWEKKVERIENNPRRRAKESKVREYYEKQFPEIRKQRELQERMQSR
VGQRGSGLSMSAARSEHEVSEIIDGLSEQENLEKQMRQLAVIPPMLYDADQQRIKFINM
NGLMADPMKVYKDRQVMNMWSEQEKETFREKFMQHPKNFGLIASFLERKTVAECVLY
YYLTKKNENYKSLVRRSYRRRGKSQQQQQQQQQQQQQQPMPRSSQEEKDEKE
KEKEAEKEEEKPEVENDKEDLLKEKTDDTSGEDNDEKEAVASKGRKTANSQGRRKGRI
TRSMANEANSEEAITPQQSAELASMELNESSRWTEEEMETAKKGLLEHGRNWSAIARM
VGSKTVSQCKNFYFNYKKRQNLDEILQQHKLKMEKERNARRKKKKAPAAASEEAAFPP
VVEDEEMEASGVSGNEEEMVEEAEALHASGNEVPRGECSGPATVNNSSDTESIPSPH
TEAAKDTGQNGPKPPATLGADGPPPGPPTPPPEDIPAPTEPTPASEATGAPTPPPAPPS
PSAPPPVVPKEEKEEETAAAPPVEEGEEQKPPAAEELAVDTGKAEEPVKSECTEEAEE
GPAKGKDAEAAEATAEGALKAEKKEGGSGRATTAKSSGAPQDSDSSATCSADEVDEA
EGGDKNRLLSPRPSLLTPTGDPRANASPQKPLDLKQLKQRAAAIPPIQVTKVHEPPRED
AAPTKPAPPAPPPPQNLQPESDAPQQPGSSPRGKSRSPAPPADKEAFAAEAQKLPGD
PPCWTSGLPFPVPPREVIKASPHAPDPSAFSYAPPGHPLPLGLHDTARPVLPRPPTISN
PPPLISSAKHPSVLERQIGAISQGMSVQLHVPYSEHAKAPVGPVTMGLPLPMDPKKLAP
FSGVKQEQLSPRGQAGPPESLGVPTAQEASVLRGTALGSVPGGSITKGIPSTRVPSDS
AITYRGSITHGTPADVLYKGTITRIIGEDSPSRLDRGREDSLPKGHVIYEGKKGHVLSYEG
GMSVTQCSKEDGRSSSGPPHETAAPKRTYDMMEGRVGRAISSASIEGLMGRAIPPERH
SPHHLKEQHHIRGSITQGIPRSYVEAQEDYLRREAKLLKREGTPPPPPPSRDLTEAYKT
QALGPLKLKPAHEGLVATVKEAGRSIHEIPREELRHTPELPLAPRPLKEGSITQGTPLKY
DTGASTTGSKKHDVRSLIGSPGRTFPPVHPLDVMADARALERACYEESLKSRPGTASS

SGGSIARGAPVIVPELGKPRQSPLTYEDHGAPFAGHLPRGSPVTTREPTPRLQEGSLSS
SKASQDRKLTSTPREIAKSPHSTVPEHHPHPISPYEHLLRGVSGVDLYRSHIPLAFDPTSI
PRGIPLDAAAAYYLPRHLAPNPTYPHLYPPYLIRGYPDTAALENRQTIINDYITSQQMHHN
AATAMAQRADMLRGLSPRESSLALNYAAGPRGIIDLSQVPHLPVLVPPTPGTPATAMDR
LAYLPTAPQPFSSRHSSSPLSPGGPTHLTKPTTTSSSERERDRDRERDRDREREKSILT
STTTVEHAPIWRPGTEQSSGSSGGGGGSSSRPASHSHAHQHSPISPRTQDALQQRPS
VLHNTGMKGIITAVEPSTPTVLRSTSTSSPVRPAATFPPATHCPLGGTLDGVYPTLMEPV
LLPKEAPRVARPERPRADTGHAFLAKPPARSGLEPASSPSKGSEPRPLVPPVSGHATIA
RTPAKNLAPHHASPDPPAPPASASDPHREKTQSKPFSIQELELRSLGYHGSSYSPEGV
EPVSPVSSPSLTHDKGLPKHLEELDKSHLEGELRPKQPGPVKLGGEAAHLPHLRPLPES
QPSSSPLLQTAPGVKGHQRVVTLAQHISEVITQDYTRHHPQQLSAPLPAPLYSFPGASC
PVLDLRRPPSDLYLPPPDHGAPARGSPHSEGGKRSPEPNKTSVLGGGEDGIEPVSPPE
GMTEPGHSRSAVYPLLYRDGEQTEPSRMGSKSPGNTSQPPAFFSKLTESNSAMVKSK
KQEINKKLNTHNRNEPEYNISQPGTEIFNMPAITGTGLMTYRSQAVQEHASTNMGLEAII
RKALMGKYDQWEESPPLSANAFNPLNASASLPAAMPITAADGRSDHTLTSPGGGGKAK
VSGRPSSRKAKSPAPGLASGDRPPSVSSVHSEGDCNRRTPLTNRVWEDRPSSAGSTP
FPYNPLIMRLQAGVMASPPPPGLPAGSGPLAGPHHAWDEEPKPLLCSQYETLSDSE

SEQ ID No:68









SEQ ID No:69
MAYSQGGGKKKVCYYYDGDIGNYYYGQGHPMKPHRIRMTHNLLLNYGLYRKMEIYRP
HKATAEEMTKYHSDEYIKFLRSIRPDNMSEYSKQMHIFNVGEDCPAFDGLFEFCQLSTG
GSVAGAVKLNRQQTDMAVNWAGGLHHAKKYEASGFCYVNDIVLAILELLKYHQRVLYID

IDIHHGDGVEEAFYTTDRVMTVSFHKYGEYFPGTGDLRDIGAGKGKYYAVNFPMCDGID
DESYGQIFKPIISKVMEMYQPSAVVLQCGADSLSGDRLGCFNLTVKGHAKCVEVVKTFN
LPLLMLGGGGYTIRNVARCWTYETAVALDCEIPNELPYNDYFEYFGPDFKLHISPSNMT
NQNTPEYMEKIKQRLFENLRMLPHAPGVQMQAIPEDAVHEDSGDEDGEDPDKRISIRA
SDKRIACDEEFSDSEDEGEGGRRNVADHKKGAKKARIEEDKKETEDKKTDVKEEDKSK
DNSGEKTDTKGTKSEQLSNP

SEQ ID No:70









SEQ ID No:71
MASGAYNPYIEIIEQPRQRGMRFRYKCEGRSAGSIPGEHSTDNNRTYPSIQIMNYYGKG
KVRITLVTKNDPYKPHPHDLVGKDCRDGYYEAEFGQERRPLFFQNLGIRCVKKKEVKEA
IITRIKAGINPFNVPEKQLNDIEDCDLNVVRLCFQVFLPDEHGNLTTALPPVVSNPIYDNR
APNTAELRICRVNKNCGSVRGGDEIFLLCDKVQKDDIEVRFVLNDWEAKGIFSQADVHR
QVAIVFKTPPYCKAITEPVTVKMQLRRPSDQEVSESMDFRYLPDEKDTYGNKAKKQKTT
LLFQKLCQDHVETGFRHVDQDGLELLTSGDPPTLASQSAGITVNFPERPRPGLLGSIGE
GRYFKKEPNLFSHDAVVREMPTGVSSQAESYYPSPGPISSGLSHHASMAPLPSSSWSS
VAHPTPRSGNTNPLSSFSTRTLPSNSQGIPPFLRIPVGNDLNASNACIYNNADDIVGMEA
SSMPSADLYGISDPNMLSNCSVNMMTTSSDSMGETDNPRLLSMNLENPSCNSVLDPR
DLRQLHQMSSSSMSAGANSNTTVFVSQSDAFEGSDFSCADNSMINESGPSNSTNPNS
HGFVQDSQYSGIGSMQNEQLSDSFPYEFFQV

SEQ ID No:72
MESCYNPGLDGIIEYDDFKLNSSIVEPKEPAPETADGPYLVIVEQPKQRGFRFRYGCEG
PSHGGLPGASSEKGRKTYPTVKICNYEGPAKIEVDLVTHSDPPRAHAHSLVGKQCSEL
GICAVSVGPKDMTAQFNNLGVLHVTKKNMMGTMIQKLQRQRLRSRPQGLTEAEQREL
EQEAKELKKVMDLSIVRLRFSAFLRASDGSFSLPLKPVTSQPIHDSKSPGASNLKISRMD
KTAGSVRGGDEVYLLCDKVQKDDIEVRFYEDDENGWQAFGDFSPTDVHKQYAIVFRTP
PYHKMKIERPVTVFLQLKRKRGGDVSDSKQFTYYPLVEDKEEVQRKRRKALPTFSQPF
GGGSHMGGGSGGAAGGYGGAGGGGSLGFFPSSLAYSPYQSGAGPMRCYPGGGGG
AQMAATVPSRDSGEEAAEPSAPSRTPQCEPQAPEMLQRAREYNARLFGLAHAAPSPT
RLLRHRGRRALLAGQRHLLTAQDENGDTPLHLAIIHGQTSVIEQIVYVIHHAQDLGVVNL
TNHLHQTPLHLAVITGQTSVVSFLLRVGADPALLDRHGDSAMHLALRAGAGAPELLRAL
LQSGAPAVPQLLHMPDFEGLYPVHLAVRARSPECLDLLVDSGAEVEATERQGGRTALH
LATEMEELGLVTHLVTKLRANVNARTFAGNTPLHLAAGLGYPTLTRLLLKAGADIHAENE
EPLCPLPSPPTSDSDSDSEGPEKDTRSSFRGHTPLDLTCSTLVKTLLLNAAQNTMEPPL
TPPSPAGPGLSLGDTALQNLEQLLDGPEAQGSWAELAERLGLRSLVDTYRQTTSPSGS
LLRSYELAGGDLAGLLEALSDMGLEEGVRLLRGPETRDKLPSTEVKEDSAYGSQSVEQ
EAEKLGPPPEPPGGLSHGHPQPQVH

SEQ ID No: 73
MAEDDPYLGRPEQMFHLDPSLTHTIFNPEVFQPQMALPTDGPYLQILEQPKQRGFRFR
YVCEGPSHGGLPGASSEKNKKSYPQVKICNYVGPAKVIVQLVTNGKNIHLHAHSLVGKH
CEDGICTVTAGPKDMVVGFANLGILHVTKKKVFETLEARMTEACIRGYNPGLLVHPDLA
YLQAEGGGDRQLGDREKELIRQAALQQTKEMDLSVVRLMFTAFLPDSTGSFTRRLEPV
VSDAIYDSKAPNASNLKIVRMDRTAGCVTGGEEIYLLCDKVQKDDIQIRFYEEEENGGV
WEGFGDFSPTDVHRQFAIVFKTPKYKDINITKPASVFVQLRRKSDLETSEPKPFLYYPEI
KDKEEVQRKRQKLMPNFSDSFGGGSGAGAGGGGMFGSGGGGGGTGSTGPGYSFPH
YGFPTYGGITFHPGTTKSNAGMKHGT

SEQ ID No:74
MAEDDPYLGRPEQMFHLDPSLTHTIFNPEVFQPQMALPTDGPYLQILEQPKQRGFRFR
YVCEGPSHGGLPGASSEKNKKSYPQVKICNYVGPAKVIVQLVTNGKNIHLHAHSLVGKH
CEDGICTVTAGPKDMVVGFANLGILHVTKKKVFETLEARMTEACIRGYNPGLLVHPDLA

YLQAEGGGDRQLGDREKELIRQAALQQTKEMDLSVVRLMFTAFLPDSTGSFTRRLEPV
VSDAIYDSKAPNASNLKIVRMDRTAGCVTGGEEIYLLCDKVQKDDIQIRFYEEEENGGV
WEGFGDFSPTDVHRQFAIVFKTPKYKDINITKPASVFVQLRRKSDLETSEPKPFLYYPEI
KDKEEVQRKRQKLMPNFSDSFGGGSGAGAGGGGMFGSGGGGGGTGSTGPGYSFPH
YGFPTYGGITFHPGTTKSNAGMKHGTMDTESKKDPEGCDKSDDKNTVNLFGKVIETTE
QDQEPSEATVGNGEVTLTYATGTKEESAGVQDNLFLEKAMQLAKRHANALFDYAVTGD
VKMLLAVQRHLTAVQDENGDSVLHLAIIHLHSQLVRDLLEVTSGLISDDIINMRNDLYQTP
LHLAVITKQEDVVEDLLRAGADLSLLDRLGNSVLHLAAKEGHDKVLSILLKHKKAALLLDH
PNGDGLNAIHLAMMSNSLPCLLLLVAAGADVNAQEQKSGRTALHLAVEHDNISLAGCLL
LEGDAHVDSTTYDGTTPLHIAAGRGSTRLAALLKAAGADPLVENFEPLYDLDDSWENAG
EDEGVVPGTTPLDMATSWQVFDILNGKPYEPEFTSDDLLAQGDMKQLAEDVKLQLYKL
LEIPDPDKNWATLAQKLGLGILNNAFRLSPAPSKTLMDNYEVSGGTVRELVEALRQMGY
TEAIEVIQAASSPVKTTSQAHSLPLSPASTRQQIDELRDSDSVCDSGVETSFRKLSFTES
LTSGASLLTLNKMPHDYGQEGPLEGKI

SEQ ID No:75
MDELFPLIFPAEQPKQRGMRFRYKCEGRSAGSIPGERSTDTTKTHPTIKINGYTGPGTV
RISLVTKDPPHRPHPHELVGKDCRDGFYEAELCPDRCIHSFQNLGIQCVKKRDLEQAIS
QRIQTNNNPFQVPIEEQRGDYDLNAVRLCFQVTVRDPSGRPLRLPPVLSHPIFDNRAPN
TAELKICRVNRNSGSCLGGDEIFLLCDKVQKEDIEVYFTGPGWEARGSFSQADVHRQV
AIVFRTPPYADPSLQAPVRVSMQLRRPSDRELSEPMEFQYLPDTDDRHRIEEKRKRTYE
TFKSIMKKSPFSGPTDPRPPPRRIAVPSRSSASVPKPAPQPYPFTSSLSTINYDEFPTMV
FPSGQISQASALAPAPPQVLPQAPAPAPAPAMVSALAQAPAPVPVLAPGPPQAVAPPA
PKPTQAGEGTLSEALLQLQFDDEDLGALLGNSTDPAVFTDLASVDNSEFQQLLNQGIPV
APHTTEPMLMEYPEAITRLVTAQRPPDPAPAPLGAPGLPNGLLSGDEDFSSIADMDFSA
LLSQISS

SEQ ID No:76
MSEARKGPDEAEESQYDSGIESLRSLRSLPESTSAPASGPSDGSPQPCTHPPGPVKEP
QEKEDADGERADSTYGSSSLTYTLSLLGGPEAEDPPPRLPLPHVGALSPQQLEALTYIS
EDGDTLVHLAVIHEAPAVLLCCLALLPQEVLDIQNNLYQTALHLAVHLDQPGAVRALVLK
GASRALQDRHGDTALHVACQRQSWPVPAACWKGGPEPGRGTSHSLDLQLQNWQGL

ACLHIATLQKNQPLMELLLRNGADIDVQEGSSGKTALHLAVETQERGLVQFLLQAGAQV
DARMLNGCTPLHLAAGRGLMGISSTLCKAGADSLLRNVEDETPQDLTEESLVLLPFDDL
KISGKLLLCTD

SEQ ID No:77
MAGVACLGKAADADEWCDTGLGSLGPDAAAPGGPGLGAELGPGLSWAPLVFGYVTE
DGDTALHLAVIHQHEPFLDFLLGFSAGTEYMDLQNDLGQTALHLAAILGETSTVEKLYAA
GAGLCVAERRGHTALHLACRVGAHACARALLQPRPRRPREAPDTYLAQGPDRTPDTN
HTPVALYPDSDLEKEEEESEEDWKLQLEAENYEGHTPLHVAVIHKDVEMVRLLRDAGA
DLDKPEPTCGRSPLHLAVEAQAADVLELLLRAGANPAARMYGGRTPLGSAMLRPNPILA
RLLRAHGAPEPEGEDEKSGPCSSSSDSDGGDEGVSQEERQGSPAGGSG

SEQ ID No:78







SEQ ID No:79
MQSTANYLWHTDDLLGQGATASVYKARNKKSGELVAVKVFNTTSYLRPREVQVREFEV
LRKLNHQNIVKLFAVEETGGSRQKVLVMEYCSSGSLLSVLESPENAFGLPEDEFLVVLR
CVVAGMNHLRENGIVHRDIKPGNIMRLVGEEGQSIYKLTDFGAARELDDDEKFVSVYGT
EEYLHPDMYERAVLRKPQQKAFGVTVDLWSIGVTLYHAATGSLPFIPFGGPRRNKEIMY
RITTEKPAGAIAGAQRRENGPLEWSYTLPITCQLSLGLQSQLVPILANILEVEQAKCWGF
DQFFAETSDILQRVVVHVFSLSQAVLHHIYIHAHNTIAIFQEAVHKQTSVAPRHQEYLFEG
HLCVLEPSVSAQHIAHTTASSPLTLFSTAIPKGLAFRDPALDVPKFVPKVDLQADYNTAK
GVLGAGYQALRLARALLDGQELMFRGLHWVMEVLQATCRRTLEVARTSLLYLSSSLGT
ERFSSVAGTPEIQELKAAAELRSRLRTLAEVLSRCSQNITETQESLSSLNRELVKSRDQV
HEDRSIQQIQCCLDKMNFIYKQFKKSRMRPGLGYNEEQIHKLDKVNFSHLAKRLLQVFQ

EECVQKYQASLVTHGKRMRVVHETRNHLRLVGCSVAACNTEAQGVQESLSKLLEELSH
QLLQDRAKGAQASPPPIAPYPSPTRKDLLLHMQELCEGMKLLASDLLDNNRIIERLNRVP
APPDV

SEQ ID No:80
MAQTQGTRRKVCYYYDGDVGNYYYGQGHPMKPHRIRMTHNLLLNYGLYRKMEIYRPH
KANAEEMTKYHSDDYIKFLRSIRPDNMSEYSKQMQRFNVGEDCPVFDGLFEFCQLSTG
GSVASAVKLNKQQTDIAVNWAGGLHHAKKSEASGFCYVNDIVLAILELLKYHQRVLYIDI
DIHHGDGVEEAFYTTDRVMTVSFHKYGEYFPGTGDLRDIGAGKGKYYAVNYPLRDGID
DESYEAIFKPVMSKVMEMFQPSAVVLQCGSDSLSGDRLGCFNLTIKGHAKCVEFVKSF
NLPMLMLGGGGYTIRNVARCWTYETAVALDTEIPNELPYNDYFEYFGPDFKLHISPSNM
TNQNTNEYLEKIKQRLFENLRMLPHAPGVQMQAIPEDAIPEESGDEDEDDPDKRISICSS
DKRIACEEEFSDSEEEGEGGRKNSSNFKKAKRVKTEDEKEKDPEEKKEVTEEEKTKEE
KPEAKGVKEEVKLA

SEQ ID No:81
MLRSGPASGPSVPTGRAMPSRRVARPPAAPELGALGSPDLSSLSLAVSRSTDELEIIDE
YIKENGFGLDGGQPGPGEGLPRLVSRGAASLSTVTLGPVAPPATPPPWGCPLGRLVSP
APGPGPQPHLVITEQPKQRGMPFRYECEGRSAGSILGESSTEASKTLPAIELRDCGGLR
EVEVTACLVWKDWPHRVHPHSLVGKDCTDGICRVRLRPHVSPRHSFNNLGIQCVRKKE
IEAAIERKIQLGIDPYNAGSLKNHQEVDMNVVRICFQASYRDQQGQMRRMDPVLSEPVY
DKKSTNTSELRICRINKESGPCTGGEELYLLCDKVQKEDISVVFSRASWEGRADFSQAD
VHRQIAIVFKTPPYEDLEIVEPVTVNVFLQRLTDGVCSEPLPFTYLPRDHDSYGVDKKAK
RGMPDVLGELNSSDPHGIESKRRKKKPAILDHFLPNHGSGPFLPPSALLPDPDFFSGTV
SLPGLEPPGGPDLLDDGFAYDPTAPTLFTMLDLLPPAPPHASAVVCSGGAGAVVGETP
GPEPLTLDSYQAPGPGDGGTASLVGSNMFPNHYREAAFGGGLLSPGPEAT

SEQ ID No:82
MSYTGFVQGSETTLQSTYSDTSAQPTCDYGYGTWNSGTNRGYEGYGYGYGYGQDNT
TNYGYGMATSHSWEMPSSDTNANTSASGSASADSVLSRINQRLDMVPHLETDMMQG
GVYGSGGERYDSYESCDSRAVLSERDLYRSGYDYSELDPEMEMAYEGQYDAYRDQF

RMRGNDTFGPRAQGWARDARSGRPMASGYGRMWEDPMGARGQCMSGASRLPSLF
SQNIIPEYGMFQGMRGGGAFPGGSRFGFGFGNGMKQMRRTWKTWTTADFRTKKKKR
KQGGSPDEPDSKATRTDCSDNSDSDNDEGTEGEATEGLEGTEAVEKGSRVDGEDEE
GKEDGREEGKEDPEKGALTTQDENGQTKRKLQAGKKSQDKQKKRQRDRMVERIQFV
CSLCKYRTFYEDEMASHLDSKFHKEHFKYVGTKLPKQTADFLQEYVTNKTKKTEELRKT
VEDLDGLIQQIYRDQDLTQEIAMEHFVKKVEAAHCAACDLFIPMQFGIIQKHLKTMDHNR
NRRLMMEQSKKSSLMVARSILNNKLISKKLERYLKGENPFTDSPEEEKEQEEAEGGALD
EGAQGEAAGISEGAEGVPAQPPVPPEPAPGAVSPPPPPPPEEEEEGAVPLLGGALQR
QIRGIPGLDVEDDEEGGGGAP

SEQ ID No:83










SEQ ID No:84
MTSIHFVVHPLPGTEDQLNDRLREVSEKLNKYNLNSHPPLNVLEQATIKQCVVGPNHAA
FLLEDGRVCRIGFSVQPDRLELGKPDNNDGSKLNSNSGAGRTSRPGRTSDSPWFLSG
SETLGRLAGNTLGSRWSSGVGGSGGGSSGRSSAGARDSRRQTRVIRTGRDRGSGLL
GSQPQPVIPASVIPEELISQAQVVLQGKSRSVIIRELQRTNLDVNLAVNNLLSRDDEDGD
DGDDTASESYLPGEDLMSLLDADIHSAHPSVIIDADAMFSEDISYFGYPSFRRSSLSRLG
SSRVLLLPLERDSELLRERESVLRLRERRWLDGASFDNERGSTSKEGEPNLDKKNTPV
QSPVSLGEDLQWWPDKDGTKFICIGALYSELLAVSSKGELYQWKWSESEPYRNAQNP
SLHHPRATFLGLTNEKIVLLSANSIRATVATENNKVATWVDETLSSVASKLEHTAQTYSE
LQGERIVSLHCCALYTCAQLENSLYWWGVVPFSQRKKMLEKARAKNKKPKSSAGISSM
PNITVGTQVCLRNNPLYHAGAVAFSISAGIPKVGVLMESVWNMNDSCRFQLRSPESLKN

MEKASKTTEAKPESKQEPVKTEMGPPPSPASTCSDASSIASSASMPYKRRRSTPAPKE
EEKVNEEQWSLREVVFVEDVKNVPVGKVLKVDGAYVAVKFPGTSSNTNCQNSSGPDA
DPSSLLQDCRLLRIDELQVVKTGGTPKVPDCFQRTPKKLCIPEKTEILAVNVDSKGVHAV
LKTGNWVRYCIFDLATGKAEQENNFPTSSIAFLGQNERNVAIFTAGQESPIILRDGNGTIY
PMAKDCMGGIRDPDWLDLPPISSLGMGVHSLINLPANSTIKKKAAVIIMAVEKQTLMQHIL
RCDYEACRQYLMNLEQAVVLEQNLQMLQTFISHRCDGNRNILHACVSVCFPTSNKETK
EEEEAERSERNTFAERLSAVEAIANAISVVSSNGPGNRAGSSSSRSLRLREMMRRSLR
AAGLGRHEAGASSSDHQDPVSPPIAPPSWVPDPPAMDPDGDIDFILAPAVGSLTTAATG
TGQQGPSTSTIPGPSTEPSVVESKDRKANAHFILKLLCDSVVLQPYLRELLSAKDARGMT
PFMSAVSGRAYPAAITILETAQKIAKAEISSSEKEEDVFMGMVCPSGTNPDDSPLYVLCC
NDTCSFTWTGAEHINQDIFECRTCGLLESLCCCTECARVCHKGHDCKLKRTSPTAYCD
CWEKCKCKTLIAGQKSARLDLLYRLLTATNLVTLPNSRGEHLLLFLVQTVARQTVEHCQ
YRPPRIREDRNRKTASPEDSDMPDHDLEPPRFAQLALERVLQDWNALKSMIMFGSQEN
KDPLSASSRIGHLLPEEQVYLNQQSGTIRLDCFTHCLIVKCTADILLLDTLLGTLVKELQN
KYTPGRREEAIAVTMRFLRSVARVFVILSVEMASSKKKNNFIPQPIGKCKRVFQALLPYA
VEELCNVAESLIVPVRMGIARPTAPFTLASTSIDAMQGSEELFSVEPLPPRPSSDQSSSS
SQSQSSYIIRNPQQRRISQSQPVRGRDEEQDDIVSADVEEVEVVEGVAGEEDHHDEQE
EHGEENAEAEGQHDEHDEDGSDMELDLLAAAETESDSESNHSNQDNASGRRSVVTAA
TAGSEAGASSVPAFFSEDDSQSNDSSDSDSSSSQSDDIEQETFMLDEPLERTTNSSHA
NGAAQAPRSMQWAVRNTQHQRAASTAPSSTSTPAASSAGLIYIDPSNLRRSGTISTSAA
AAAAALEASNASSYLTSASSLARAYSIVIRQISDLMGLIPKYNHLVYSQIPAAVKLTYQDA
VNLQNYVEEKLIPTWNWMVSIMDSTEAQLRYGSALASAGDPGHPNHPLHASQNSARR
ERMTAREEASLRTLEGRRRATLLSARQGMMSARGDFLNYALSLMRSHNDEHSDVLPV
LDVCSLKHVAYVFQALIYWIKAMNQQTTLDTPQLERKRTRELLELGIDNEDSEHENDDD
TNQSATLNDKDDDSLPAETGQNHPFFRRSDSMTFLGCIPPNPFEVPLAEAIPLADQPHL
LQPNARKEDLFGRPSQGLYSSSASSGKCLMEVTVDRNCLEVLPTKMSYAANLKNVMN
MQNRQKKEGEEQPVLPEETESSKPGPSAHDLAAQLKSSLLAEIGLTESEGPPLTSFRPQ
CSFMGMVISHDMLLGRWRLSLELFGRVFMEDVGAEPGSILTELGGFEVKESKFRREME
KLRNQQSRDLSLEVDRDRDLLIQQTMRQLNNHFGRRCATTPMAVHRVKVTFKDEPGE
GSGVARSFYTAIAQAFLSNEKLPNLECIQNANKGTHTSLMQRLRNRGERDRERERERE
MRRSSGLRAGSRRDRDRDFRRQLSIDTRPFRPASEGNPSDDPEPLPAHRQALGERLY
PRVQAMQPAFASKITGMLLELSPAQLLLLLASEDSLRARVDEAMELIIAHGRENGADSIL
DLGLVDSSEKVQQENRKRHGSSRSVVDMDLDDTDDGDDNAPLFYQPGKRGFYTPRP
GKNTEARLNCFRNIGRILGLCLLQNELCPITLNRHVIKVLLGRKVNWHDFAFFDPVMYES

LRQLILASQSSDADAVFSAMDLAFAIDLCKEEGGGQVELIPNGVNIPVTPQNVYEYVRKY
AEHRMLVVAEQPLHAMRKGLLDVLPKNSLEDLTAEDFRLLVNGCGEVNVQMLISFTSFN
DESGENAEKLLQFKRWFWSIVEKMSMTERQDLVYFWTSSPSLPASEEGFQPMPSITIR
PPDDQHLPTANTCISRLYVPLYSSKQILKQKLLLAIKTKNFGFV

SEQ ID No:85
MALADSTRGLPNGGGGGGGSGSSSSSAEPPLFPDIVELNVGGQVYVTRRCTVVSVPD
SLLWRMFTQQQPQELARDSKGRFFLDRDGFLFRYILDYLRDLQLVLPDYFPERSRLQR
EAEYFELPELVRRLGAPQQPGPGPPPSRRGVHKEGSLGDELLPLGYSEPEQQEGASA
GAPSPTLELASRSPSGGAAGPLLTPSQSLDGSRRSGYITIGYRGSYTIGRDAQADAKFR
RVARITVCGKTSLAKEVFGDTLNESRDPDRPPERYTSRYYLKFNFLEQAFDKLSESGFH
MVACSSTGTCAFASSTDQSEDKIWTSYTEYVFCRE

SEQ ID No:86
MSKSFQQSSLSRDSQGHGRDLSAAGIGLLAAATQSLSMPASLGRMNQGTARLASLMN
LGMSSSLNQQGAHSALSSASTSSHNLQSIFNIGSRGPLPLSSQHRGDADQASNILASFG
LSARDLDELSRYPEDKITPENLPQILLQLKRRRTEEGPTLSYGRDGRSATREPPYRVPR
DDWEEKRHFRRDSFDDRGPSLNPVLDYDHGSRSQESGYYDRMDYEDDRLRDGERCR
DDSFFGETSHNYHKFDSEYERMGRGPGPLQERSLFEKKRGAPPSSNIEDFHGLLPKGY
PHLCSICDLPVHSNKEWSQHINGASHSRRCQLLLEIYPEWNPDNDTGHTMGDPFMLQQ
STNPAPGILGPPPPSFHLGGPAVGPRGNLGAGNGNLQGPRHMQKGRVETSRVVHIMD
FQRGKNLRYQLLQLVEPFGVISNHLILNKINEAFIEMATTEDAQAAVDYYTTTPALVFGKP
VRVHLSQKYKRIKKPEGKPDQKFDQKQELGRVIHLSNLPHSGYSDSAVLKLAEPYGKIK
NYILMRMKSQAFIEMETREDAMAMVDHCLKKALWFQGRCVKVDLSEKYKKLVLRIPNR
GIDLLKKDKSRKRSYSPDGKESPSDKKSKTDGSQKTESSTEGKEQEEKSGEDGEKDTK
DDQTEQEPNMLLESEDELLVDEEEAALLESGSSVGDETDLANLGDVASDGKKEPSDK
AVKKDGSASAAAKKKLKKVDKIEELDQENEAALENGIKNEENTEPGAESSENADDPNKD
TSENADGQSDENKDDYTIPDEYRIGPYQPNVPVGIDYVIPKTGFYCKLCSLFYTNEEVAK
NTHCSSLPHYQKLKKFLNKLAEERRQKKET

SEQ ID No:87
MRGDRGRGRGGRFGSRGGPGGGFRPFVPHIPFDFYLCEMAFPRVKPAPDETSFSEAL
LKRNQDLAPNSAEQASILSLVTKINNVIDNLIVAPGTFEVQIEEVRQVGSYKKGTMTTGH
NVADLVVILKILPTLEAVAALGNKVVESLRAQDPSEVLTMLTNETGFEISSSDATVKILITT
VPPNLRKLDPELHLDIKVLQSALAAIRHARWFEENASQSTVKVLIRLLKDLRIRFPGFEPL
TPWILDLLGHYAVMNNPTRQPLALNVAYRRCLQILAAGLFLPGSVGITDPCESGNFRVH
TVMTLEQQDMVCYTAQTLVRILSHGGFRKILGQEGDASYLASEISTWDGVIVTPSEKAY
EKPPEKKEGEEEEENTERTTSRRGRRKHGNSGVTFPSLLFLPKGKTGA

SEQ ID No:88
MFEARLVQGSILKKVLEALKDLINEACWDISSSGVNLQSMDSSHVSLVQLTLRSEGFDT
YRCDRNLAMGVNLTSMSKILKCAGNEDIITLRAEDNADTLALVFEAPNQEKVSDYEMKL
MDLDVEQLGIPEQEYSCVVKMPSGEFARICRDLSHIGDAVVISCAKDGVKFSASGELGN
GNIKLSQTSNVDKEEEAVTIEMNEPVQLTFALRYLNFFTKATPLSSTVTLSMSADVPLVV
EYKIADMGHLKYYLAPKIEDEEGS

SEQ ID No:89
QSNKTFNLEKQNHTPRKHHQHHHQQHHQQQQQPPPPPIPANGQQASSQNEGLTI
DLKNFRKPGEKTFTQRSRLFVGNLPPDITEEEMRKLFEKYGKAGEVFIHKDKGFGFIRLE
TRTLAEIAKVELDNMPLRGKQLRVRFACHSASLTVRNLPQYVSNELLEEAFSVFGQVER
AVVIVDDRGRPSGKGIVEFSGKPAARKALDRCSEGSFLLTTFPRPVTVEPMDQLDDEEG
LPEKLVIKNQQFHKEREQPPRFAQPGSFEYEYAMRWKALIEMEKQQQDQVDRNIKEAR
EKLEMEMEAARHEHQVMLMRQDLMRRQEELRRMEELHNQEVQKRKQLELRQEEERR
RREEEMRRQQEEMMRRQQEGFKGTFPDAREQEIRMGQMAMGGAMGINNRGAMPPA
PVPAGTPAPPGPATMMPDGTLGLTPPTTERFGQAATMEGIGAIGGTPPAFNRAAPGAE
FAPNKRRRY

SEQ ID No:90
METRKKDGTWPSAQLHVMNNNMSEEDEDFKGQPGICGLTNLGNTCFMNSALQCLSNV
PQLTEYFLNNCYLEELNFRNPLGMKGEIAEAYADLVKQAWSGHHRSIVPHVFKNKVGH
FASQFLGYQQHDSQELLSFLLDGLHEDLNRVKKKEYVELCDAAGRPDQEVAQEAWQN

HKRRNDSVIVDTFHGLFKSTLVCPDCGNVSVTFDPFCYLSVPLPISHKRVLEVFFIPMDP
RRKPEQHRLVVPKKGKISDLCVALSKHTGISPERMMVADVFSHRFYKLYQLEEPLSSILD
RDDIFVYEVSGRIEAIEGSREDIVVPVYLRERTPARDYNNSYYGLMLFGHPLLVSVPRDR
FTWEGLYNVLMYRLSRYVTKPNSDDEDDGDEKEDDEEDKDDVPGPSTGGSLRDPEPE
QAGPSSGVTNRCPFLLDNCLGTSQWPPRRRRKQLFTLQTVNSNGTSDRTTSPEEVHA
QPYIAIDWEPEMKKRYYDEVEAEGYVKHDCVGYVMKKAPVRLQECIELFTTVETLEKEN
PWYCPSCKQHQLATKKLDLWMLPEILIIHLKRFSYTKFSREKLDTLVEFPIRDLDFSEFVI
QPQNESNPELYKYDLIAVSNHYGGMRDGHYTTFACNKDSGQWHYFDDNSVSPVNEN
QIESKAAYVLFYQRQDVARRLLSPAGSSGAPASPACSSPPSSEFMDVN

SEQ ID No:91
MHSLATAAPVPTTLAQVDREKIYQWINELSSPETRENALLELSKKRESVPDLAPMLWHS
FGTIAALLQEIVNIYPSINPPTLTAHQSNRVCNALALLQCVASHPETRSAFLAAHIPLFLYP
FLHTVSKTRPFEYLRLTSLGVIGALVKTDEQEVINFLLTTEIIPLCLRIMESGSELSKTVATF
ILQKILLDDTGLAYICQTYERFSHVAMILGKMVLQLSKEPSARLLKHVVRCYLRLSDNPRA
REALRQCLPDQLKDTTFAQVLKDDTTTKRWLAQLVKNLQEGQVTDPRGIPLPPQ

SEQ ID No:92
MADKEAAFDDAVEERVINEEYKIWKKNTPFLYDLVMTHALEWPSLTAQWLPDVTRPEG
KDFSIHRLVLGTHTSDEQNHLVIASVQLPNDDAQFDASHYDSEKGEFGGFGSVSGKIEIE
IKINHEGEVNRARYMPQNPCIIATKTPSSDVLVFDYTKHPSKPDPSGECNPDLRLRGHQ
KEGYGLSWNPNLSGHLLSASDDHTICLWDISAVPKEGKVVDAKTIFTGHTAVVEDVSWH
LLHESLFGSVADDQKLMIWDTRSNNTSKPSHSVDAHTAEVNCLSFNPYSEFILATGSAD
KTVALWDLRNLKLKLHSFESHKDEIFQVQWSPHNETILASSGTDRRLNVWDLSKIGEEQ
SPEDAEDGPPELLFIHGGHTAKISDFSWNPNEPWVICSVSEDNIMQVWQMAENIYNDE
DPEGSVDPEGQGS

SEQ ID No:93
MASKEMFEDTVEERVINEEYKIWKKNTPFLYDLVMTHALQWPSLTVQWLPEVTKPEGK
DYALHWLVLGTHTSDEQNHLVVARVHIPNDDAQFDASHCDSDKGEFGGFGSVTGKIEC
EIKINHEGEVNRARYMPQNPHIIATKTPSSDVLVFDYTKHPAKPDPSGECNPDLRLRGH

QKEGYGLSWNSNLSGHLLSASDDHTVCLWDINAGPKEGKIVDAKAIFTGHSAVVEDVA
WHLLHESLFGSVADDQKLMIWDTRSNTTSKPSHLVDAHTAEVNCLSFNPYSEFILATGS
ADKTVALWDLRNLKLKLHTFESHKDEIFQVHWSPHNETILASSGTDRRLNVWDLSKIGE
EQSAEDAEDGPPELLFIHGGHTAKISDFSWNPNEPWVICSVSEDNIMQIWQMAENIYND
EESDVTTSELEGQGS

SEQ ID No:94
GENTSLSGIPTSSVLSLSLPSSVAQSNFPQGSGASEMVSNQPANLLVQPPSQPVPENL
VPESQKDRKAGSALPGFANSPAGSTSVVLVPPAHGTLVPDGNKANHSSHQEDTYGAL
DFALSRTLENPVNVYNPSHSDSLASQQSVASHPRQSGPGAPNLDRFYQQVTKDAQGQ
PGLERAQQELAPPQQQASPPQLPKAMFSELSNPESLPAQGQAQNSAQSPASLVLVDA
GQQLPPRPPQSSSVSLVSSGSGQAAVPSEQPWPQPVPALAPGPPPQDLAAYYYYRPL
YDAYQPQYSLPYPPEPGAASLYYQDVYSLYEPRYRPYDGAASAYAQNYRYPEPERPS
SRASHSSERPPPRQGYPEGYYSSKSGWSSQSDYYASYYSSQYDYGDPGHWDRYHYS
ARVRDPRTYDRRYWCDAEYDAYRREHSAFGDRPEKRDNNWRYDPRFTGSFDDDPDP
HRDPYGEEVDRRSVHSEHSARSLHSAHSLASRRSSLSSHSHQSQIYRSHNVAAGSYEA
PLPPGSFHGDFAYGTYRSNFSSGPGFPEYGYPADTVWPAMEQVSSRPTSPEKFSVPH
VCARFGPGGQLIKVIPNLPSEGQPALVEVHSMEALLQHTSEQEEMRAFPGPLAKDDTH
KVDVINFAQNKAMKCLQNENLIDKESASLLWNFIVLLCRQNGTVVGTDIAELLLRDHRTV
WLPGKSPNEANLIDFTNEAVEQVEEEESGEAQLSFLTGGPAAAASSLERETERFRELLL
YGRKKDALESAMKNGLWGHALLLASKMDSRTHARVMTRFANSLPINDPLQTVYQLMS
GRMPAASTCCGDEKWGDWRPHLAMVLSNLNNNMDVESRTMATMGDTLASRGLLDAA
HFCYLMAQAGFGVYTKKTTKLVLIGSNHSLPFLKFATNEAIQRTEAYEYAQSLGAETCPL
PSFQVFKFIYSCRLAEMGLATQAFHYCEAIAKSILTQPHLYSPVLISQLVQMASQLRLFDP
QLKEKPEEESLAAPTWLVHLQQVERQIKEGAGVWHQDGALPQQCPGTPSSEMEQLDR
PGLSQPGALGIANPLLAVPAPSPEHSSPSVRLLPSAPQTLPDGPLASPARVPMFPVPLP
PGPLEPGPGCVTPGPALGFLEPSGPGLPPGVPPLQERRHLLQEARSPDPGIVPQEAPV
GNSLSELSEENFDGKFANLTPSRTVPDSEAPPGWDRADSGPTQPPLSLSPAPETKRPG
QAAKKETKEPKKGESWFFRWLPGKKKTEAYLPDDKNKSIVWDEKKNQWVNLNEPEEE
KKAPPPPPTSMPKTVQAAPPALPGPPGAPVNMYSRRAAGTRARYVDVLNPSGTQRSE
PALAPADFVAPLAPLPIPSNLFVPTPDAEEPQLPDGTGREGPAAARGLANPEPAPEPKA
PGDLPAAGGPPSGAMPFYNPAQLAQACATSGSSRLGRIGQRKHLVLN

SEQ ID No:95
MPYANQPTVRITELTDENVKFIIENTDLAVANSIRRVFIAEVPIIAIDWVQIDANSSVLHDEF
IAHRLGLIPLISDDIVDKLQYSRDCTCEEFCPECSVEFTLDVRCNEDQTRHVTSRDLISNS
PRVIPVTSRNRDNDPNDYVEQDDILIVKLRKGQELRLRAYAKKGFGKEHAKWNPTAGVA
FEYDPDNALRHTVYPKPEEWPKSEYSELDEDESQAPYDPNGKPERFYYNVESCGSLR
PETIVLSALSGLKKKLSDLQTQLSHEIQSDVLTIN

SEQ ID No:96
MFLVNSFLKGGGGGGGGGGGLGGGLGNVLGGLISGAGGGGGGGGGGGGGGGG
GGTAMRILGGVISAISEAAAQYNPEPPPPRTHYSNIEANESEEVRQFRRLFAQLAGDDM
EVSATELMNILNKVVTRHPDLKTDGFGIDTCRSMVAVMDSDTTGKLGFEEFKYLWNNIK
RWQAIYKQFDTDRSGTICSSELPGAFEAAGFHLNEHLYNMIIRRYSDESGNMDFDNFIS
CLVRLDAMFRAFKSLDKDGTGQIQVNIQEWLQLTMYS

SEQ ID No:97




































































SEQ ID No:98
MPGPRVWGKYLWRSPHSKGCPGAMWWLLLWGVLQACPTRGSVLLAQELPQQLTSP
GYPEPYGKGQESSTDIKAPEGFAVRLVFQDFDLEPSQDCAGDSVTISFVGSDPSQFCG
QQGSPLGRPPGQREFVSSGRSLRLTFRTQPSSENKTAHLHKGFLALYQTVAVNYSQPI
SEASRGSEAINAPGDNPAKVQNHCQEPYYQAAAAGALTCATPGTWKDRQDGEEVLQC
MPVCGRPVTPIAQNQTTLGSSRAKLGNFPWQAFTSIHGRGGGALLGDRWILTAAHTVY
PKDSVSLRKNQSVNVFLGHTAIDEMLKLGNHPVHRVVVHPDYRQNESHNFSGDIALLEL
QHSIPLGPNVLPVCLPDNETLYRSGLLGYVSGFGMEMGWLTTELKYSRLPVAPREACN
AWLQKRQRPEVFSDNMFCVGDETQRHSVCQGDSGSVYVVWDNHAHHWVATGIVSW
GIGCGEGYDFYTKVLSYVDWIKGVMNGKN

SEQ ID No:99




















SEQ ID No:100
MAASAAAASAAAASAASGSPGPGEGSAGGEKRSTAPSAAASASASAAASSPAGGGAE
ALELLEHCGVCRERLRPEREPRLLPCLHSACSACLGPAAPAAANSSGDGGAAGDGTVV
DCPVCKQQCFSKDIVENYFMRDSGSKAATDAQDANQCCTSCEDNAPATSYCVECSEP
LCETCVEAHQRVKYTKDHTVRSTGPAKSRDGERTVYCNVHKHEPLVLFCESCDTLTCR
DCQLNAHKDHQYQFLEDAVRNQRKLLASLVKRLGDKHATLQKSTKEVRSSIRQVSDVQ
KRVQVDVKMAILQIMKELNKRGRVLVNDAQKVTEGQQERLERQHWTMTKIQKHQEHIL
RFASWALESDNNTALLLSKKLIYFQLHRALKMIVDPVEPHGEMKFQWDLNAWTKSAEAF
GKIVAERPGTNSTGPAPMAPPRAPGPLSKQGSGSSQPMEVQEGYGFGSGDDPYSSAE
PHVSGVKRSRSGEGEVSGLMRKVPRVSLERLDLDLTADSQPPVFKVFPGSTTEDYNLI
VIERGAAAAATGQPGTAPAGTPGAPPLAGMAIVKEEETEAAIGAPPTATEGPETKPVLM
ALAEGPGAEGPRLASPSGSTSSGLEVVAPEGTSAPGGGPGTLDDSATICRVCQKPGDL
VMCNQCEFCFHLDCHLPALQDVPGEEWSCSLCHVLPDLKEEDGSLSLDGADSTGVVA
KLSPANQRKCERVLLALFCHEPCRPLHQLATDSTFSLDQPGGTLDLTLIRARLQEKLSPP
YSSPQEFAQDVGRMFKQFNKLTEDKADVQSIIGLQRFFETRMNEAFGDTKFSAVLVEPP
PMSLPGAGLSSQELSGGPGDGP

SEQ ID No:101
MDEGPVDLRTRPKAAGLPGAALPLRKRPLRAPSPEPAAPRGAAGLVVPLDPLRGGCDL
PAVPGPPHGLARPEALYYPGALLPLYPTRAMGSPFPLVNLPTPLYPMMCPMEHPLSADI
AMATRADEDGDTPLHIAVVQGNLPAVHRLVNLFQQGGRELDIYNNLRQTPLHLAVITTLP
SVVRLLVTAGASPMALDRHGQTAAHLACEHRSPTCLRALLDSAAPGTLDLEARNYDGL
TALHVAVNTECQETVQLLLERGADIDAVDIKSGRSPLIHAVENNSLSMVQLLLQHGANVN
AQMYSGSSALHSASGRGLLPLVRTLVRSGADSSLKNCHNDTPLMVARSRRVIDILRGKA
TRPASTSQPDPSPDRSANTSPESSSRLSSNGLLSASPSSSPSQSPPRDPPGFPMAPPN
FFLPSPSPPAFLPFAGVLRGPGRPVPPSPAPGGS

SEQ ID No:102
MDSKESLTPGREENPSSVLAQERGDVMDFYKTLRGGATVKVSASSPSLAVASQSDSK
QRRLLVDFPKGSVSNAQQPDLSKAVSLSMGLYMGETETKVMGNDLGFPQQGQISLSS
GETDLKLLEESIANLNRSTSVPENPKSSASTAVSAAPTEKEFPKTHSDVSSEQQHLKGQ
TGTNGGNVKLYTTDQSTFDILQDLEFSSGSPGKETNESPWRSDLLIDENCLLSPLAGED
DSFLLEGNSNEDCKPLILPDTKPKIKDNGDLVLSSPSNVTLPQVKTEKEDFIELCTPGVIK
QEKLGTVYCQASFPGANIIGNKMSAISVHGVSTSGGQMYHYDMNTASLSQQQDQKPIF
NVIPPIPVGSENWNRCQGSGDDNLTSLGTLNFPGRTVFSNGYSSPSMRPDVSSPPSSS
STATTGPPPKLCLVCSDEASGCHYGVLTCGSCKVFFKRAVEGQHNYLCAGRNDCIIDKI
RRKNCPACRYRKCLQAGMNLEARKTKKKIKGIQQATTGVSQETSENPGNKTIVPATLPQ
LTPTLVSLLEVIEPEVLYAGYDSSVPDSTWRIMTTLNMLGGRQVIAAVKWAKAIPGFRNL
HLDDQMTLLQYSWMFLMAFALGWRSYRQSSANLLCFAPDLIINEQRMTLPCMYDQCK
HMLYVSSELHRLQVSYEEYLCMKTLLLLSSVPKDGLKSQELFDEIRMTYIKELGKAIVKR
EGNSSQNWQRFYQLTKLLDSMHEVVENLLNYCFQTFLDKTMSIEFPEMLAEIITNQIPKY
SNGNIKKLLFHQK

SEQ ID No:103
MTTPGKENFRLKSYKNKSLNPDEMRRRREEEGLQLRKQKREEQLFKRRNVATAEEET
EEEVMSDGGFHEAQINNMEMAPGGVITSDMIEMIFSKSPEQQLSATQKFRKLLSKEPNP
PIDEVISTPGVVARFVEFLKRKENCTLQFESAWVLTNIASGNSLQTRIVIQAGAVPIFIELL
SSEFEDVQEQAVWALGNIAGDSTMCRDYVLDCNILPPLLQLFSKQNRLTMTRNAVWAL
SNLCRGKSPPPEFAKVSPCLNVLSWLLFVSDTDVLADACWALSYLSDGPNDKIQAVIDA
GVCRRLVELLMHNDYKVVSPALRAVGNIVTGDDIQTQVILNCSALQSLLHLLSSPKESIK
KEACWTISNITAGNRAQIQTVIDANIFPALISILQTAEFRTRKEAAWAITNATSGGSAEQIK

YLVELGCIKPLCDLLTVMDSKIVQVALNGLENILRLGEQEAKRNGTGINPYCALIEEAYGL
DKIEFLQSHENQEIYQKAFDLIEHYFGTEDEDSSIAPQVDLNQQQYIFQQCEAPMEGFQL

SEQ ID No:104
MVDYSVWDHIEVSDDEDETHPNIDTASLFRWRHQARVERMEQFQKEKEELDRGCREC
KRKVAECQRKLKELEVAEGGKAELERLQAEAQQLRKEERSWEQKLEEMRKKEKSMPW
NVDTLSKDGFSKSMVNTKPEKTEEDSEEVREQKHKTFVEKYEKQIKHFGMLRRWDDS
QKYLSDNVHLVCEETANYLVIWCIDLEVEEKCALMEQVAHQTIVMQFILELAKSLKVDPR
ACFRQFFTKIKTADRQYMEGFNDELEAFKERVRGRAKLRIEKAMKEYEEEERKKRLGP
GGLDPVEVYESLPEELQKCFDVKDVQMLQDAISKMDPTDAKYHMQRCIDSGLWVPNS
KASEAKEGEEAGPGDPLLEAVPKTGDEKDVSV

SEQ ID No:105
PEETQTQDQPMEEEEVETFAFQAEIAQLMSLIINTFYSNKEIFLRELISNSSDALDKIRYET
LTDPSKLDSGKELHINLIPNKQDRTLTIVDTGIGMTKADLINNLGTIAKSGTKAFMEALQA
GADISMIGQFGVGFYSAYLVAEKVTVITKHNDDEQYAWESSAGGSFTVRTDTGEPMGR
GTKVILHLKEDQTEYLEERRIKEIVKKHSQFIGYPITLFVEKERDKEVSDDEAEEKEDKEE
EKEKEEKESEDKPEIEDVGSDEEEEKKDGDKKKKKKIKEKYIDQEELNKTKPIWTRNPD
DITNEEYGEFYKSLTNDWEDHLAVKHFSVEGQLEFRALLFVPRRAPFDLFENRKKKNNI
KLYVRRVFIMDNCEELIPEYLNFIRGVVDSEDLPLNISREMLQQSKILKVIRKNLVKKCLEL
FTELAEDKENYKKFYEQFSKNIKLGIHEDSQNRKKLSELLRYYTSASGDEMVSLKDYCT
RMKENQKHIYYITGETKDQVANSAFVERLRKHGLEVIYMIEPIDEYCVQQLKEFEGKTLV
SVTKEGLELPEDEEEKKKQEEKKTKFENLCKIMKDILEKKVEKVVVSNRLVTSPCCIVTS
TYGWTANMERIMKAQALRDNSTMGYMAAKKHLEINPDHSIIETLRQKAEADKNDKSVKD
LVILLYETALLSSGFSLEDPQTHANRIYRMIKLGLGIDEDDPTADDTSAAVTEEMPPLEGD
DDTSRMEEVD

SEQ ID No:106
MTTDEGAKNNEESPTATVAEQGEDITSKKDRGVLKIVKRVGNGEETPMIGDKVYVHYK
GKLSNGKKFDSSHDRNEPFVFSLGKGQVIKAWDIGVATMKKGEICHLLCKPEYAYGSA
GSLPKIPSNATLFFEIELLDFKGEDLFEDGGIIRRTKRKGEGYSNPNEGATVEIHLEGRCG
GRMFDCRDVAFTVGEGEDHDIPIGIDKALEKMQREEQCILYLGPRYGFGEAGKPKFGIE
PNAELIYEVTLKSFEKAKESWEMDTKEKLEQAAIVKEKGTVYFKGGKYMQAVIQYGKIVS
WLEMEYGLSEKESKASESFLLAAFLNLAMCYLKLREYTKAVECCDKALGLDSANEKGLY

RRGEAQLLMNEFESAKGDFEKVLEVNPQNKAARLQISMCQKKAKEHNERDRRIYANMF
KKFAEQDAKEEANKAMGKKTSEGVTNEKGTDSQAMEEEKPEGHV

SEQ ID No:107
MPEEVHHGEEEVETFAFQAEIAQLMSLIINTFYSNKEIFLRELISNASDALDKIRYESLTDP
SKLDSGKELKIDIIPNPQERTLTLVDTGIGMTKADLINNLGTIAKSGTKAFMEALQAGADIS
MIGQFGVGFYSAYLVAEKVVVIRKHNDDEQYAWESSAGGSFTVRADHGEPIGMGTKVI
LHLKEDQTEYLEERRVKEVVKKHSQFIGYPITLYLEKEREKEISDDEAEEEKGEKEEEDK
DDEEKPKIEDVGSDEEDDSGKDKKKKTKKIKEKYIDQEELNKTKPIWTRNPDDITQEEYG
EFYKSLTNDWEDHLAVKHFSVEGQLEFRALLFIPRRAPFDLFENKKKKNNIKLYVRRVFI
MDSCDELIPEYLNFIRGVVDSEDLPLNISREMLQQSKILKVIRKNIVKKCLELFSELAEDKE
NYKKFYEAFSKNLKLGIHEDSTNRRRLSELLRYHTSQSGDEMTSLSEYVSRMKETQKSI
YYITGESKEQVANSAFVERVRKRGFEVVYMTEPIDEYCVQQLKEFDGKSLVSVTKEGLE
LPEDEEEKKKMEESKAKFENLCKLMKEILDKKVEKVTISNRLVSSPCCIVTSTYGWTANM
ERIMKAQALRDNSTMGYMMAKKHLEINPDHPIVETLRQKAEADKNDKAVKDLVVLLFET
ALLSSGFSLEDPQTHSNRIYRMIKLGLGIDEDEVAAEEPNAAVPDEIPPLEGDEDASRME
EVD

SEQ ID No:108
MAVMEMACPGAPGSAVGQQKELPKPKEKTPPLGKKQSSVYKLEAVEKSPVFCGKWEI
LNDVITKGTAKEGSEAGPAAISIIAQAECENSQEFSPTFSERIFIAGSKQYSQSESLDQIP
NNVAHATEGKMARVCWKGKRRSKARKKRKKKSSKSLAHAGVALAKPLPRTPEQESCTI
PVQEDESPLGAPYVRNTPQFTKPLKEPGLGQLCFKQLGEGLRPALPRSELHKLISPLQC
LNHVWKLHHPQDGGPLPLPTHPFPYSRLPHPFPFHPLQPWKPHPLESFLGKLACVDSQ
KPLPDPHLSKLACVDSPKPLPGPHLEPSCLSRGAHEKFSVEEYLVHALQGSVSSSQAH
SLTSLAKTWAARGSRSREPSPKTEDNEGVLLTEKLKPVDYEYREEVHWATHQLRLGRG
SFGEVHRMEDKQTGFQCAVKKVRLEVFRAEELMACAGLTSPRIVPLYGAVREGPWVNI
FMELLEGGSLGQLVKEQGCLPEDRALYYLGQALEGLEYLHSRRILHGDVKADNVLLSSD
GSHAALCDFGHAVCLQPDGLGKSLLTGDYIPGTETHMAPEVVLGRSCDAKVDVWSSC
CMMLHMLNGCHPWTQFFRGPLCLKIASEPPPVREIPPSCAPLTAQAIQEGLRKEPIHRV
SAAELGGKVNRALQQVGGLKSPWRGEYKEPRHPPPNQANYHQTLHAQPRELSPRAP
GPRPAEETTGRAPKLQPPLPPEPPEPNKSPPLTLSKEESGMWEPLPLSSLEPAPARNP
SSPERKATVPEQELQQLEIELFLNSLSQPFSLEEQEQILSCLSIDSLSLSDDSEKNPSKAS
QSSRDTLSSGVHSWSSQAEARSSSWNMVLARGRPTDTPSYFNGVKVQIQSLNGEHLH

IREFHRVKVGDIATGISSQIPAAAFSLVTKDGQPVRYDMEVPDSGIDLQCTLAPDGSFAW
SWRVKHGQLENRP

SEQ ID No:109
MADLDKLNIDSIIQRLLEVRGSKPGKNVQLQENEIRGLCLKSREIFLSQPILLELEAPLKIC
GDIHGQYYDLLRLFEYGGFPPESNYLFLGDYVDRGKQSLETICLLLAYKIKYPENFFLLR
GNHECASINRIYGFYDECKRRYNIKLWKTFTDCFNCLPIAAIVDEKIFCCHGGLSPDLQS
MEQIRRIMRPTDVPDQGLLCDLLWSDPDKDVLGWGENDRGVSFTFGAEVVAKFLHKH
DLDLICRAHQVVEDGYEFFAKRQLVTLFSAPNYCGEFDNAGAMMSVDETLMCSFQILKP
AEKKKPNATRPVTPPRGMITKQAKK

SEQ ID No:110
MVGKHLGARKDHPGLKAKENDENCGPTTTVFVGNISEKASDMLIRQLLAKCGLVLSWK
RVQGASGKLQAFGFCEYKEPESTLRALRLLHDLQIGEKKLLVKVDAKTKAQLDEWKAKK
KASNGNARPETVTNDDEEALDEETKRRDQMIKGAIEVLIREYSSELNAPSQESDSHPRK
KKKEKKEDIFRRFPVAPLIPYPLITKEDINAIEMEEDKRDLISREISKFRDTHKKLEEEKGK
KEKERQEIEKERRERERERERRERERERERERERERKEKERERERERDRDRDRT
KERDRDRDRERDRDRDRERSSDRNKDRSRSREKSRDRERERERERERERERERE
RERERERERERERERERERKDKKRDREEDEEDAYERRKLERKLREKEAAYQERLKNWEIR
ERKKTREYEKEAEREEERRREMAKEAKRLKEFLEDYDDDRDDPKYYRGSALQKRLRD
REKEMEADERDRKREKEELEEIRQRLLAEGHPDPDAELQRMEQEAERRRQPQIKQEPE
SEEEEEEKQEKEEKREEPMEEEEEPEQKPCLKPTLRPISSAPSVSSASGNATPNTPGD
ESPCGIIIPHENSPDQQQPEEHRPKIGLSLKLGASNSPGQPNSVKRKKLPVDSVFNKFE
DEDSDDVPRKRKLVPLDYGEDDKNATKGTVNTEEKRKHIKSLIEKIPTAKPELFAYPLDW
SIVDSILMERRIRPWINKKIIEYIGEEEATLVDFVCSKVMAHSSPQSILDDVAMVLDEEAEV
FIVKMWRLLIYETEAKKIGLVK

SEQ ID No:111
MENFVATLANGMSLQPPLEEVSCGQAESSEKPNAEDMTSKDYYFDSYAHFGIHEEMLK
DEVRTLTYRNSMFHNRHLFKDKVVLDVGSGTGILCMFAAKAGARKVIGIVCSSISDYAVK
IVKANKLDHVVTIIKGKVEEVELPVEKVDIIISEWMGYCLFYESMLNTVLYARDKWLAPDG
LIFPDRATLYVTAIEDRQYKDYKIHWWENVYGFDMSCIKDVAIKEPLVDVVDPKQLVTNA
CLIKEVDIYTVKVEDLTFTSPFCLQVKRNDYVHALVAYFNIEFTRCHKRTGFSTSPESPYT

HWKQTVFYMEDYLTVKTGEEIFGTIGMRPNAKNNRDLDFTIDLDFKGQLCELSCSTDYR
MR

SEQ ID No:112
SSKTASTNNIAQARRTVQQLRLEASIERIKVSKASADLMSYCEEHARSDPLLIGIPTSENP
FKDKKTCIIL

SEQ ID No:113










SEQ ID No:114
MADGELNVDSLITRLLEVRGCRPGKIVQMTEAEVRGLCIKSREIFLSQPILLELEAPLKIC
GDIHGQYTDLLRLFEYGGFPPEANYLFLGDYVDRGKQSLETICLLLAYKIKYPENFFLLR
GNHECASINRIYGFYDECKRRFNIKLWKTFTDCFNCLPIAAIVDEKIFCCHGGLSPDLQS
MEQIRRIMRPTDVPDTGLLCDLLWSDPDKDVQGWGENDRGVSFTFGADVVSKFLNRH
DLDLICRAHQVVEDGYEFFAKRQLVTLFSAPNYCGEFDNAGGMMSVDETLMCSFQILK
PSEKKAKYQYGGLNSGRPVTPPRTANPPKKR

SEQ ID No:115















SEQ ID No:116
MAQFGGQKNPPWATQFTATAVSQPAALGVQQPSLLGASPTIYTQQTALAAAGLTTQTP
ANYQLTQTAALQQQAAAAAAALQQQYSQPQQALYSVQQQLQQPQQTLLTQPAVALPT
SLSLSTPQPTAQITVSYPTPRSSQQQTQPQKQRVFTGVVTKLHDTFGFVDEDVFFQLSA
VKGKTPQVGDRVLVEATYNPNMPFKWNAQRIQTLPNQNQSQTQPLLKTPPAVLQPIAP
QTTFGVQTQPQPQSLLQAQISAASITPLLQTQPQPLLQQPQQKAGLLQPPVRIVSQPQP
ARRLDPPSRFSGRNDRGDQVPNRKDDRSRERERERRRSRERSPQRKRSRERSPRRE
RERSPRRVRRVVPRYTVQFSKFSLDCPSCDMMELRRRYQNLYIPSDFFDAQFTWVDA
FPLSRPFQLGNYCNFYVMHREVESLEKNMAILDPPDADHLYSAKVMLMASPSMEDLYH
KSCALAEDPQELRDGFQHPARLVKFLVGMKGKDEAMAIGGHWSPSLDGPDPEKDPSV
LIKTAIRCCKALTGIDLSVCTQWYRFAEIRYHRPEETHKGRTVPAHVETVVLFFPDVWHC
LPTRSEWETLSRGYKQQLVEKLQGERKEADGEQDEEEKDDGEAKEISTPTHWSKLDP
KTMKVNDLRKELESRALSSKGLKSQLIARLTKQLKVEEQKEEQKELEKSEKEEDEDDDR
KSEDDKEEEERKRQEEIERQRRERRYILPDEPAIIVHPNWAAKSGKFDCSIMSLSVLLDY
RLEDNKEHSFEVSLFAELFNEMLQRDFGVRIYKSLLSLPEKEDKKEKDKKSKKDERKDK
KEERDDETDEPKPKRRKSGDDKDKKEDRDERKKEDKRKDDSKDDDETEEDNNQDEY
DPMEAEEAEDEEDDRDEEEMTKRDDKRDINRYCKERPSKDKEKEKTQMITINRDLLMA
FVYFDQSHCGYLLEKDLEEILYTLGLHLSRAQVKKLLNKVVLRESCFYRKLTDTSKDEEN
HEESESLQEDMLGNRLLLPTPTVKQESKDVEENVGLIVYNGAMVDVGSLLQKLEKSEKV
RAEVEQKLQLLEEKTDEDEKTILNLENSNKSLSGELREVKKDLSQLQENLKISENMNLQF
ENQMNKTIRNLSTVMDEIHTVLKKDNVKNEDKDQKSKENGASV

SEQ ID No:117
MSTELFSSTREEGSSGSGPSFRSNQRKMLNLLLERDTSFTVCPDVPRTPVGKFLGDSA
NLSILSGGTPKCCLDLSNLSSGEITATQLTTSADLDETGHLDSSGLQEVHLAGMNHDQH
LMKCSPAQLLCSTPNGLDRGHRKRDAMCSSSANKENDNGNLVDSEMKYLGSPITTVPK
LDKNPNLGEDQAEEISDELMEFSLKDQEAKVSRSGLYRSPSMPENLNRPRLKQVEKFK

DNTIPDKVKKKYFSGQGKLRKGLCLKKTVSLCDITITQMLEEDSNQGHLIGDFSKVCALP
TVSGKHQDLKYVNPETVAALLSGKFQGLIEKFYVIDCRYPYEYLGGHIQGALNLYSQEEL
FNFFLKKPIVPLDTQKRIIIVFHCEFSSERGPRMCRCLREEDRSLNQYPALYYPELYILKG
GYRDFFPEYMELCEPQSYCPMHHQDHKTELLRCRSQSKVQEGERQLREQIALLVKDM
SP

SEQ ID No:118
MSQERPTFYRQELNKTIWEVPERYQNLSPVGSGAYGSVCAAFDTKTGLRVAVKKLSRP
FQSIIHAKRTYRELRLLKHMKHENVIGLLDVFTPARSLEEFNDVYLVTHLMGADLNNIVKC
QKLTDDHVQFLIYQILRGLKYIHSADIIHRDLKPSNLAVNEDCELKILDFGLARHTDDEMT
GYVATRWYRAPEIMLNWMHYNQTVDIWSVGCIMAELLTGRTLFPGTDHINQLQQIMRLT
GTPPAYLINRMPSHEARNYIQSLTQMPKMNFANVFIGANPLAVDLLEKMLVLDSDKRITA
AQALAHAYFAQYHDPDDEPVADPYDQSFESRDLLIDEWKSLTYDEVISFVPPPLDQEEM
ES

SEQ ID No:119
MAEEEVAKLEKHLMLLRQEYVKLQKKLAETEKRCALLAAQANKESSSKSFISRLLAIVAD
LYEQEQYSDLKIKVGDRHISAHKFVLAARSDSWSLANLSSTKELDLSDANPEVTVTMLR
WIYTDELEFREDDVFLTELMKLANRFQLQLLRERCEKGVMSLVNVRNCIRFYQTAEELN
ASTLMNYCAEIIASHWDDLRKEDFSSMSPQLLYKMIKSKTEYPLHKAIKVEREDVVSLYLI
EMDSQLPGKLNEADHNGDLALDLALSRRLENIATTLVSHKADVAWVPRVLELLHKGIQE
DLFAATFLIKNGAFVNAATLGCQETPLHLVALYSSRNHSADVMSEMAQIAEALLQAGAN
PNMQDSKGRTPLHVSIMAGDEYVFSHLLQCKQLDLELKDHEGSTALWLAVQHITVSSD
QSVNPFEDVPVVNGTSFDENSFAARLIQRGSHTDAPDTATGNCLLQRAAGAGNEAAAL
FLATNGAHVNHRNKWGETPLHTACRHGLANLTAELLQQGANPNLQTEEALPLPKEAAS
LTSLADSVHLQTPLHMAIAYNHPDVVSVILEQKANALHATNNLQIIPDFSLKDSRDQTVLG
LALWTGMHTIAAQLLGSGAAINDTMSDGQTLLHMAIQRQDSKSALFLLEHRADINVRPQ
AGETALQLAIRNQLPLVVDAICTRGADMSVPDEEGNPPLWLALANNLEDIASTLVRHGC
GATCWGPGPGGCLQTLLHRAIDENNEPTACFLIRSGCDVNSPRQPGANGEGEEEARD
GQTPLHLAASWGLETVQCLLEFGADVNPQDAEGRTPCHVAISSQHGVIIQLLVSHPISSL
NVRDKQGLTPFACAMTFKDNKSAEAILKRGSGAAEQVDNKGRNSLHVAVQNSDIESVL
SLISVHANVNSRAQDAPKLTPLHLVVQEGSEIIVRNLLLAGAKVTELTKHRKTALHLAAQE
DLPTICSVLLENAVDFAAVDENGNNALHLAVMHGRLNNIRVLLTECTVDAEAFNLRGQS
PLHILGQYGKENAAAIFDLFLECMPGYPLDKPDADGSTVLLLAYMKGNANLCRAIVRSG

ARLGVNNNQGVNIFNYQVATKQLLFRLLDMLSKEPPWCDGSYCYECTARFGVATRKHH
CRHCGRLLCHKCSTKEIPIIKFDLNKPVRVCTICFDVLTLGGVS

SEQ ID No:120
MAGGVRPLRGLRALCRVLLFLSQFCILSGGEQSQALAQSIKDPGPTRTFTVVPRAAEST
EIPPYVMKCPSNGLCSRLPADCIDCTTNFSCTYGKPVTFDCAVKPSVTCVDQDFKSQKN
FIINMTCRFCWQLPETDYECTNSTSCMTVSCPRQRYPANCTVRDHVHCLGNRTFPKML
YCNWTGGYKWSTALALSITLGGFGADRFYLGQWREGLGKLFSFGGLGIWTLIDVLLIGV
GYVGPADGSLYI

SEQ ID No:121
MSCQAFTSADTFIPLNSDASATLPLIMHHSAAECLPVSNHATNVMSTATGLHYSVPSCH
YGNQPSTYGVMAGSLTPCLYKFPDHTLSHGFPPIHQPLLAEDPTAADFKQELRRKSKLV
EEPIDMDSPEIRELEKFANEFKVRRIKLGYTQTNVGEALAAVHGSEFSQTTICRFENLQL
SFKNACKLKAILSKWLEEAEQVGALYNEKVGANERKRKRRTTISIAAKDALERHFGEQN
KPSSQEIMRMAEELNLEKEVVRVWFCNRRQREKRVKTSLNQSLFSISKEHLECR

SEQ ID No:122
MAGKRSGWSRAALLQLLLGVNLVVMPPTRARSLRFVTLLYRHGDRSPVKTYPKDPYQE
EEWPQGFGQLTKEGMLQHWELGQALRQRYHGFLNTSYHRQEVYVRSTDFDRTLMSA
EANLAGLFPPNGMQRFNPNISWQPIPVHTVPITEDRLLKFPLGPCPRYEQLQNETRQTP
EYQNESSRNAQFLDMVANETGLTDLTLETVWNVYDTLFCEQTHGLRLPPWASPQTMQ
RLSRLKDFSFRFLFGIYQQAEKARLQGGVLLAQIRKNLTLMATTSQLPKLLVYSAHDTTL
VALQMALDVYNGEQAPYASCHIFELYQEDSGNFSVEMYFRNESDKAPWPLSLPGCPH
RCPLQDFLRLTEPVVPKDWQQECQLASGPADTEVIVALAVCGSILFLLIVLLLTVLFRMQ
AQPPGYRHVADGEDHA

SEQ ID No:123
MSRPLLITFTPATDPSDLWKDGQQQPQPEKPESTLDGAAARAFYEALIGDESSAPDSQ
RSQTEPARERKRKKRRIMKAPAAEAVAEGASGRHGQGRSLEAEDKMTHRILRAAQEG
DLPELRRLLEPHEAGGAGGNINARDAFWWTPLMCAARAGQGAAVSYLLGRGAAWVG
VCELSGRDAAQLAEEAGFPEVARMVRESHGETRSPENRSPTPSLQYCENCDTHFQDS
NHRTSTAHLLSLSQGPQPPNLPLGVPISSPGFKLLLRGGWEPGMGLGPRGEGRANPIP

TVLKRDQEGLGYRSAPQPRVTHFPAWDTRAVAGRERPPRVATLSWREERRREEKDRA
WERDLRTYMNLEF

SEQ ID No:124
MGLPRRAGDAAELRKSLKPLLEKRRRARINQSLSQLKGLILPLLGRENSNCSKLEKADVL
EMTVRFLQELPASSWPTAAPLPCDSYREGYSACVARLARVLPACRVLEPAVSARLLEH
LWRRAASATLDGGRAGDSSGPSAPAPAPASAPEPASAPVPSPPSPPCGPGLWRPW

SEQ ID No:125
VGVVCYRVYCCCCLANVSLPGIGGTIPESKPFFYVNVADIESLEVEVSYVACTTEKIFEE
KRELYDVYVDNQNVKTHHDHLQPLLKINSADREKYRRLNEQRQMLLYSQEVEEDYNPC
EEDLFVLFFLEQNNRIFQTLLEVSASQDKTLTAEHARGMGLDPQGDRSFLLDLLEAYGID
VMLVIDNPCCP

SEQ ID No:126
MEGRGPYRIYDPGGSVPSGEASAAFERLVKENSRLKEKMQGIKMLGELLEESQMEATR
LRQKAEELVKDNELLPPPSPSLGSFDPLAELTGKDSNVTASPTAPACPSDKPAPVQKPP
SSGTSSEFEVVTPEEQNSPESSSHANAMALGPLPREDGNLMLHLQRLETTLSVCAEEP
DHGQLFTHLGRMALEFNRLASKVHKNEQRTSILQTLCEQLRKENEALKAKLDKGLEQR
DQAAERLREENLELKKLLMSNGNKEGASGRPGSPKMEGTGKKAVAGQQQASVTAGKV
PEVVALGAAEKKVKMLEQQRSELLEVNKQWDQHFRSMKQQYEQKITELRQKLADLQK
QVTDLEAEREQKQRDFDRKLLLAKSKIEMEETDKEQLTAEAKELRQKVKYLQDQLSPLT
RQREYQEKEIQRLNKALEEALSIQTPPSSPPTAFGSPEGAGALLRKQELVTQNELLKQQ
VKIFEEDFQRERSDRERMNEEKEELKKQVEKLQAQVTLSNAQLKAFKDEEKAREALRQ
QKRKAKASGERYHVEPHPEHLCGAYPYAYPPMPAMVPHHGFEDWSQIRYPPPPMAM
EHPPPLPNSRLFHLPEYTWRLPCGGVRNPNQSSQVMDPPTARPTEPESPKNDREGPQ

SEQ ID No:127
PLRLDIKRKLTARSDRVKSVDLHPTEPWMLASLYNGSVCVWNHETQTLVKTFEVCDLP
VRAAKFVARKNWVVTGADDMQIRVFNYNTLERVHMFEAHSDYIRCIAVHPTQPFILTSS
DDMLIKLWDWDKKWSCSQVFEGHTHYVMQIVINPKDNNQFASASLDRTIKVWQLGSSS
PNFTLEGHEKGVNCIDYYSGGDKPYLISGADDRLVKIWDYQNKTCVQTLEGHAQNVSC
ASFHPELPIIITGSEDGTVRIWHSSTYRLESTLNYGMERVWCVASLRGSNNVALGYDEG
SIIVKLGREEPAMSMDANGKIIWAKHSEVQQANLKAMGDAEIKDGERLPLAVKDMGSCE

IYPQTIQHNPNGRFVVVCGDGEYIIYTAMALRNKSFGSAQEFAWAHDSSEYAIRESNSIV
KIFKNFKEKKSFKPDFGAESIYGGFLLGVRSVNGLAFYDWDNTELIRRIEIQPKHIFWSDS
GELVCIATEESFFILKYLSEKVLAAQETHEGVTEDGIEDAFEVLGEIQEIVKTGLWVGDCFI
YTSSVNRLNYYVGGEIVTIAHLDRTMYLLGYIPKDNRLYLGDKELNIISYSLLVSVLEYQTA
VMRRDFSMADKVLPTIPKEQRTRVAHFLEKQGFKQQALTVSTDPEHRFELALQLGELKI
AYQLAVEAESEQKWKQLAELAISKCQFGLAQECLHHAQDYGGLLLLATASGNANMVNK
LAEGAERDGKNNVAFMSYFLQGKVDACLELLIRTGRLPEAAFLARTYLPSQVSRVVKLW
RENLSKVNQKAAESLADPTEYENLFPGLKEAFVVEEWVKETHADLWPAKQYPLVTPNE
ERNVMEEGKDFQPSRSTAQQELDGKPASPTPVIVASHTANKEEKSLLELEVDLDNLELE
DIDTTDINLDEDILDD

SEQ ID No:128
MDPAEAVLQEKALKFMNSSEREDCNNGEPPRKIIPEKNSLRQTYNSCARLCLNQETVCL
ASTAMKTENCVAKTKLANGTSSMIVPKQRKLSASYEKEKELCVKYFEQWSESDQVEFV
EHLISQMCHYQHGHINSYLKPMLQRDFITALPARGLDHIAENILSYLDAKSLCAAELVCKE
WYRVTSDGMLWKKLIERMVRTDSLWRGLAERRGWGQYLFKNKPPDGNAPPNSFYRA
LYPKIIQDIETIESNWRCGRHSLQRIHCRSETSKGVYCLQYDDQKIVSGLRDNTIKIWDKN
TLECKRILTGHTGSVLCLQYDERVIITGSSDSTVRVWDVNTGEMLNTLIHHCEAVLHLRF
NNGMMVTCSKDRSIAVWDMASPTDITLRRVLVGHRAAVNVVDFDDKYIVSASGDRTIKV
WNTSTCEFVRTLNGHKRGIACLQYRDRLVVSGSSDNTIRLWDIECGACLRVLEGHEELV
RCIRFDNKRIVSGAYDGKIKVWDLVAALDPRAPAGTLCLRTLVEHSGRVFRLQFDEFQIV
SSSHDDTILIWDFLNDPAAQAEPPRSPSRTYTYISR

SEQ ID No:129
MDPAEAVLQEKALKFMCSMPRSLWLGCSSLADSMPSLRCLYNPGTGALTAFQNSSER
EDCNNGEPPRKIIPEKNSLRQTYNSCARLCLNQETVCLASTAMKTENCVAKTKLANGTS
SMIVPKQRKLSASYEKEKELCVKYFEQWSESDQVEFVEHLISQMCHYQHGHINSYLKP
MLQRDFITALPARGLDHIAENILSYLDAKSLCAAELVCKEWYRVTSDGMLWKKLIERMVR
TDSLWRGLAERRGWGQYLFKNKPPDGNAPPNSFYRALYPKIIQDIETIESNWRCGRHS
LQRIHCRSETSKGVYCLQYDDQKIVSGLRDNTIKIWDKNTLECKRILTGHTGSVLCLQYD
ERVIITGSSDSTVRVWDVNTGEMLNTLIHHCEAVLHLRFNNGMMVTCSKDRSIAVWDM
ASPTDITLRRVLVGHRAAVNVVDFDDKYIVSASGDRTIKVWNTSTCEFVRTLNGHKRGIA
CLQYRDRLVVSGSSDNTIRLWDIECGACLRVLEGHEELVRCIRFDNKRIVSGAYDGKIKV

WDLVAALDPRAPAGTLCLRTLVEHSGRVFRLQFDEFQIVSSSHDDTILIWDFLNDPAAQ
AEPPRSPSRTYTYISR

SEQ ID No:130 CANCEL = IPI00024116.2 NUCLEAR FACTOR NF-KAPPA-B P100/P49
SUBUNITS

















SEQ ID No:130
MIHNYMEHLERTKLHQLSGSDQLESTAHSRIRKERPISLGIFPLPAGDGLLTPDAQKGGE
TPGSEQWKFQELSQPRSHTSLKDELSDVSQGGSKATTPASTANSDVATIPTDTPLKEE
NEGFVKVTDAPNKSEISKHIEVQVAQETRNVSTGSAENEEKSEVQAIIESTPELDMDKDL
SGYKGSSTPTKGIENKAFDRNTESLFEELSSAGSGLIGDVDEGADLLGMGREVENLILE
NTQLLETKNALNIVKNDLIAKVDELTCEKDVLQGELEAVKQAKLKLEEKNRELEEELRKA
RAEAEDARQKAKDDDDSDIPTAQRKRFTRVEMARVLMERNQYKERLMELQEAVRWTE
MIRASRENPAMQEKKRSSIWQFFSRLFSSSSNTTKKPEPPVNLKYNAPTSHVTPSVKKR
SSTLSQLPGDKSKAFDFLSEETEASLASRREQKREQYRQVKAHVQKEDGRVQAFGWS
LPQKYKQVTNGQGENKMKNLPVPVYLRPLDKKDTSMKLWCAVGVNLSGGKTRDGGS
VVGASVFYKDVAGLDTEGSKQRSASQSSLDKLDQELKEQQKELKNQEELSSLVWICTS
THSATKVLIIDAVQPGNILDSFTVCNSHVLCIASVPGARETDYPAGEDLSESGQVDKASL

CGSMTSNSSAETDSLLGGITVVGCSAEGVTGAATSPSTNGASPVMDKPPEMEAENSEV
DENVPTAEEATEATEGNAGSAEDTVDISQTGVYTEHVFTDPLGVQIPEDLSPVYQSRYN
NGSST

SEQ ID No:131
MGKSCKVVVCGQASVGKTSILEQLLYGNHVVGSEMIETQEDIYVGSIETDRGVREQVRF
YDTRGLRDGAELPRHCFSCTDGYVLVYSTDSRESFQRVELLKKEIDKSKDKKEVTIVVL
GNKCDLQEQRRVDPDVAQHWAKSEKVKLWEVSVADRRSLLEPFVYLASKMTQPQSKS
AFPLSRKNKGSGSLDG

SEQ ID No:132
MGKGCKVVVCGLLSVGKTAILEQLLYGNHTIGMEDCETMEDVYMASVETDRGVKEQLH
LYDTRGLQEGVELPKHYFSFADGFVLVYSVNNLESFQRVELLKKEIDKFKDKKEVAIVVL
GNKIDLSEQRQVDAEVAQQWAKSEKVRLWEVTVTDRKTLIEPFTLLASKLSQPQSKSSF
PLPGRKNKGNSNSEN

SEQ ID No:133
MHRKHLQEIPDLSSNVATSFTWGWDSSKTSELLSGMGVSALEKEEPDSENIPQELLSNL
GHPESPPRKRLKSKGSDKDFVIVRRPKLNRENFPGVSWDSLPDELLLGIFSCLCLPELL
KVSGVCKRWYRLASDESLWQTLDLTGKNLHPDVTGRLLSQGVIAFRCPRSFMDQPLAE
HFSPFRVQHMDLSNSVIEVSTLHGILSQCSKLQNLSLEGLRLSDPIVNTLAKNSNLVRLN
LSGCSGFSEFALQTLLSSCSRLDELNLSWCFDFTEKHVQVAVAHVSETITQLNLSGYRK
NLQKSDLSTLVRRCPNLVHLDLSDSVMLKNDCFQEFFQLNYLQHLSLSRCYDIIPETLLL
VTRAGVRIRLDSDIGCPQTYRTSKLKSSHKLFCQHVRVICIFVCDFYFYRLVLKQ

SEQ ID No:134
MSKSESPKEPEQLRKLFIGGLSFETTDESLRSHFEQWGTLTDCVVMRDPNTKRSRGFG
FVTYATVEEVDAAMNARPHKVDGRVVEPKRAVSREDSQRPGAHLTVKKIFVGGIKEDT
EEHHLRDYFEQYGKIEVIEIMTDRGSGKKRGFAFVTFDDHDSVDKIVIQKYHTVNGHNC
EVRKALSKQEMASASSSQRGRSGSGNFGGGRGGGFGGNDNFGRGGNFSGRGGFGG
SRGGGGYGGSGDGYNGFGNDGSNFGGGGSYNDFGNYNNQSSNFGPMKGGNFGGR
SSGPYGGGGQYFAKPRNQGGYGGSSSSSSYGSGRRF

SEQ ID No:135
MPTIKLQSSDGEIFEVDVEIAKQSVTIKTMLEDLGMDDEGDDDPVPLPNVNAAILKKVIQ
WCTHHKDDPPPPEDDENKEKRTDDIPVWDQEFLKVDQGTLFELILAANYLDIKGLLDVT
CKTVANMIKGKTPEEIRKTFNIKNDFTEEEEAQVRKENQWCEEK

SEQ ID No:136
MSSTRSQNPHGLKQIGLDQIWDDLRAGIQQVYTRQSMAKSRYMELYTHVYNYCTSVH
QSNQARGAGVPPSKSKKGQTPGGAQFVGLELYKRLKEFLKNYLTNLLKDGEDLMDESV
LKFYTQQWEDYRFSSKVLNGICAYLNRHWVRRECDEGRKGIYEIYSLALVTWRDCLFR
PLNKQVTNAVLKLIEKERNGETINTRLISGVVQSYVELGLNEDDAFAKGPTLTVYKESFE
SQFLADTERFYTRESTEFLQQNPVTEYMKKAEARLLEEQRRVQVYLHESTQDELARKC
EQVLIEKHLEIFHTEFQNLLDADKNEDLGRMYNLVSRIQDGLGELKKLLETHIHNQGLAAI
EKCGEAALNDPKMYVQTVLDVHKKYNALVMSAFNNDAGFVAALDKACGRFINNNAVTK
MAQSSSKSPELLARYCDSLLKKSSKNPEEAELEDTLNQVMVVFKYIEDKDVFQKFYAKM
LAKRLVHQNSASDDAEASMISKLKQACGFEYTSKLQRMFQDIGVSKDLNEQFKKHLTNS
EPLDLDFSIQVLSSGSWPFQQSCTFALPSELERSYQRFTAFYASRHSGRKLTWLYQLSK
GELVTNCFKNRYTLQASTFQMAILLQYNTEDAYTVQQLTDSTQIKMDILAQVLQILLKSKL
LVLEDENANVDEVELKPDTLIKLYLGYKNKKLRVNINVPMKTEQKQEQETTHKNIEEDRK
LLIQAAIVRIMKMRKVLKHQQLLGEVLTQLSSRFKPRVPVIKKCIDILIEKEYLERVDGEKD
TYSYLA

SEQ ID No:137
MEPDSVIEDKTIELMCSVPRSLWLGCANLVESMCALSCLQSMPSVRCLQNTSVMEDQN
EDESPKKSALWQISNGTSSVIVSRKRPSEGNYQKEKDLCIKYFDQWSESDQVEFVEHLI
SRMCHYQHGHINSYLKPMLQRDFITALPEQGLDHIAENILSYLDARSLCAAELVCKEWQ
RVISEGMLWKKLIERMVRTDPLWKGLSERRGWDQYLFKNRPTDGPPNSFYRSLYPKIIQ
DIETIESNWRCGRHNLQRIQCRSENSKGVYCLQYDDDKIISGLRDNSIKIWDKSSLECLK
VLTGHTGSVLCLQYDERVIVTGSSDSTVRVWDVNTGEVLNTLIHHNEAVLHLRFSNGLM
VTCSKDRSIAVWDMAFCHRYHFTPCSGWPRAAVNVVDFDDKYIVSASGDRTIKVWSTS
TCEFVRTLNGHKRGIACLQYRDRLVVSGSSDNTIRLWDIECGACLRVLEGHEELVRCIR
FDNKRIVSGAYDGKIKVWDLQAALDPRAPASTLCLRTLVEHSGRVFRLQFDEFQIISSSH
DDTILIWDFLNVPPSAQNETRSPFRTYTYISR

SEQ ID No:138










SEQ ID No:139
MRQKRKGDLGPAELMMLTIGDVIKQLIEAHEQGKDIDLNKVKTKTAAKYGLSAQPRLVDI
IAAVPPQYRKVLMPKLKAKPIRTASGIAVVAVMCKPHRCPHISFTGNICVYCPGGPDSDF
EYSTQSYTGYEPTSMRAIRARYDPFLQTRHRIEQLKQLGHSVDKVEFIVMGGTFMALPE
EYRDYFIRNLHDALSGHTSNNIYEAVKYSERSLTKCIGITIETRPDYCMKRHLSDMLTYG
CTRLEIGVQSVYEDVARDTNRGHTVKAVCESFHLAKDSGFKVVAHMMPDLPNVGLERD
IEQFTEFFENPAFRPDGLKLYPTLVIRGTGLYELWKSGRYKSYSPSDLVELVARILALVPP
WTRVYRVQRDIPMPLVSSGVEHGNLRELALARMKDLGIQCRDVRTREVGIQEIHHKVR
PYQVELVRRDYVANGGWETFLSYEDPDQDILIGLLRLRKCSERIAREEHGSGKIAVISGV
GTRNYYRKIGYRLQGPYMVKMLK

SEQ ID No:140
MSGFDDPGIFYSDSFGGDAQADEGQARKSQLQRRFKEFLRQYRVGTDRTGFTFKYRD
ELKRHYNLGEYWIEVEMEDLASFDEDLADYLYKQPAEHLQLLEEAAKEVADEVTRPRPS
GEEVLQDIQVMLKSDASPSSIRSLKSDMMSHLVKIPGIIIAASAVRAKATRISIQCRSCRNT
LTNIAMRPGLEGYALPRKCNTDQAGRPKCPLDPYFIMPDKCKCVDFQTLKLQELPDAVP
HGEMPRHMQLYCDRYLCDKVVPGNRVTIMGIYSIKKFGLTTSRGRDRVGVGIRSSYIRV
LGIQVDTDGSGRSFAGAVSPQEEEEFRRLAALPNVYEVISKSIAPSIFGGTDMKKAIACLL
FGGSRKRLPDGLTRRGDINLLMLGDPGTAKSQLLKFVEKCSPIGVYTSGKGSSAAGLTA
SVMRDPSSRNFIMEGGAMVLADGGVVCIDEFDKMREDDRVAIHEAMEQQTISIAKAGIT
TTLNSRCSVLAAANSVFGRWDETKGEDNIDFMPTILSRFDMIFIVKDEHNEERDVMLAK
HVITLHVSALTQTQAVEGEIDLAKLKKFIAYCRVKCGPRLSAEAAEKLKNRYIIMRSGARQ
HERDSDRRSSIPITVRQLEAIVRIAEALSKMKLQPFATEADVEEALRLFQVSTLDAALSGT

LSGVEGFTSQEDQEMLSRIEKQLKRRFAIGSQVSEHSIIKDFTKQKYPEHAIHKVLQLML
RRGEIQHRMQRKVLYRLK

SEQ ID No:141
MAGTVVLDDVELREAQRDYLDFLDDEEDQGIYQSKVRELISDNQYRLIVNVNDLRRKNE
KRANRLLNNAFEELVAFQRALKDFVASIDATYAKQYEEFYVGLEGSFGSKHVSPRTLTS
CFLSCVVCVEGIVTKCSLVRPKVVRSVHYCPATKKTIERRYSDLTTLVAFPSSSVYPTKD
EENNPLETEYGLSVYKDHQTITIQEMPEKAPAGQLPRSVDVILDDDLVDKAKPGDRVQV
VGTYRCLPGKKGGYTSGTFRTVLIACNVKQMSKDAQPSFSAEDIAKIKKFSKTRSKDIFD
QLAKSLAPSIHGHDYVKKAILCLLLGGVERDLENGSHIRGDINILLIGDPSVAKSQLLRYVL
CTAPRAIPTTGRGSSGVGLTAAVTTDQETGERRLEAGAMVLADRGVVCIDEFDKMSDM
DRTAIHEVMEQGRVTIAKAGIHARLNARCSVLAAANPVYGRYDQYKTPMENIGLQDSLL
SRFDLLFIMLDQMDPEQDREISDHVLRMHRYRAPGEQDGDAMPLGSAVDILATDDPNF
SQEDQQDTQIYEKHDNLLHGTKKKKEKMVSAAFMKKYIHVAKIIKPVLTQESATYIAEEY
SRLRSQDSMSSDTARTSPVTARTLETLIRLATAHAKARMSKTVDLQDAEEAVELVQYAY
FKKVLEKEKKRKKRSEDESETEDEEEKSQEDQEQKRKRRKTRQPDAKDGDSYDPYDF
SDTEEEMPQVHTPKTADSQETKESQKVELSESRLKAFKVALLDVFREAHAQSIGMNRLT
ESINRDSEEPFSSVEIQAALSKMQDDNQVMVSEGIIFLI

SEQ ID No:142














SEQ ID No:143
MRSGEPACTMDQARGLDDAAARGGQCPGLGPAPTPTPPGRLGAPYSEAWGYFHLAP
GRPGHPSGHWATCRLCGEQVGRGPGFHAGTSALWRHLRSAHRRELESSGAGSSPPA
APCPPPPGPAAAPEGDWARLLEQMGALAVRGSRRERELERRELAVEQGERALERRRR
ALQEEERAAAQARRELQAEREALQARLRDVSRREGALGWAPAAPPPLKDDPEGDRDG
CVITKVLL

SEQ ID No:144
MMLTGRSIRADRAKKMGLVDQLVEPLGPGLKPPEERTIEYLEEVAITFAKGLADKKISPK
RDKGLVEKLTAYAMTIPFVRQQVYKKVEEKVRKQTKGLYPAPLKIIDVVKTGIEQGSDAG
YLCESQKFGELVMTKESKALMGLYHGQVLCKKNKFGAPQKDVKHLAILGAGLMGAGIA
QVSVDKGLKTILKDATLTALDRGQQQVFKGLNDKVKKKALTSFERDSIFSNLTGQLDYQ
GFEKADMVIEAVFEDLSLKHRVLKEVEAVIPDHCIFASNTSALPISEIAAVSKRPEKVIGM
HYFSPVDKMQLLEIITTEKTSKDTSASAVAVGLKQGKVIIVVKDGPGFYTTRCLAPMMSE
VIRILQEGVDPKKLDSLTTSFGFPVGAATLVDEVGVDVAKHVAEDLGKVFGERFGGGNP
ELLTQMVSKGFLGRKSGKGFYIYQEGVKRKDLNSDMDSILASLKLPPKSEVSSDEDIQF
RLVTRFVNEAVMCLQEGILATPAEGDIGAVFGLGFPPCLGGWSRRLGRKLTLAWSPCC
VSFNEVLFLYSGPFRFVDLYGAQKIVDRLKKYEAAYGKQFTPCQLLADHANSPNKKFYQ

SEQ ID No:145








SEQ ID No:146



SEQ ID No:147
MHVSLAEALEVRGGPLQEEEIWAVLNQSAESLQELFRKVSLADPAALGFIISPWSLLLLP

SGSVSFTDENISNQDLRAFTAPEVLQNQSLTSLSDVEKIHIYSLGMTLYWGADYEVPQS
QPIKLGDHLNSILLGMCEDVIYARVSVRTVLDACSAHIRNSNCAPSFSYVKHLVKLVLGN
LSGTDQLSCNSEQKPDRSQAIRDRLRGKGLPTGRSSTSDVLDIQKPPLSHQTFLNKGLS
KSMGFLSIKDTQDENYFKDILSDNSGREDSENTFSPYQFKTSGPEKKPIPGIDVLSKKKI
WASSMDLLCTADRDFSSGETATYRRCHPEAVTVRTSTTPRKKEARYSDGSIALDIFGPQ
KMDPIYHTRELPTSSAISSALDRIRERQKKLQVLREAMNVEEPVRRYKTYHGDVFSTSS
ESPSIISSESDFRQVRRSEASKRFESSSGLPGVDETLSQGQSQRPSRQYETPFEGNLIN
QEIMLKRQEEELMQLQAKMALRQSRLSLYPGDTIKASMLDITRDPLREIALETAMTQRKL
RNFFGPEFVKMTIEPFISLDLPRSILTKKGKNEDNRRKVNIMLLNGQRLELTCDTKTICKD
VFDMVVAHIGLVEHHLFALATLKDNEYFFVDPDLKLTKVAPEGWKEEPKKKTKATVNFT
LFFRIKFFMDDVSLIQHTLTCHQYYLQLRKDILEERMHCDDETSLLLASLALQAEYGDYQ
PEVHGVSYFRMEHYLPARVMEKLDLSYIKEELPKLHNTYVGASEKETELEFLKVCQRLT
EYGVHFHRVHPEKKSQTGILLGVCSKGVLVFEVHNGVRTLVLRFPWRETKKISFSKKKIT
LQNTSDGIKHGFQTDNSKICQYLLHLCSYQHKFQLQMRARQSNQDAQDIERASFRSLN
LQAESVRGFNMGRAISTGSLASSTLNKLAVRPLSVQAEILKRLSCSELSLYQPLQNSSKE
KNDKASWEEKPREMSKSYHDLSQASLYPHRKNVIVNMEPPPQTVAELVGKPSHQMSR
SDAESLAGVTKLNNSKSVASLNRSPERRKHESDSSSIEDPGQAYVLGMTMHSSGNSSS
QVPLKENDVLHKRWSIVSSPEREITLVNLKKDAKYGLGFQIIGGEKMGRLDLGIFISSVAP
GGPADLDGCLKPGDRLISVNSVSLEGVSHHAAIEILQNAPEDVTLVISQPKEKISKVPSTP
VHLTNEMKNYMKKSSYMQDSAIDSSSKDHHWSRGTLRHISENSFGPSGGLREGSLSS
QDSRTESASLSQSQVNGFFASHLGDQTWQESQHGSPSPSVISKATEKETFTDSNQSKT
KKPGISDVTDYSDRGDSDMDEATYSSSQDHQTPKQESSSSVNTSNKMNFKTFSSSPP
KPGDIFEVELAKNDNSLGISVTGGVNTSVRHGGIYVKAVIPQGAAESDGRIHKGDRVLAV
NGVSLEGATHKQAVETLRNTGQVVHLLLEKGQSPTSKEHVPVTPQCTLSDQNAQGQG
PEKVKKTTQVKDYSFVTEENTFEVKLFKNSSGLGFSFSREDNLIPEQINASIVRVKKLFP
GQPAAESGKIDVGDVILKVNGASLKGLSQQEVISALRGTAPEVFLLLCRPPPGVLPEIDT
ALLTPLQSPAQVLPNSSKDSSQPSCVEQSTSSDENEMSDKSKKQCKSPSRRDSYSDS
SGSGEDDLVTAPANISNSTWSSALHQTLSNMVSQAQSHHEAPKSQEDTICTMFYYPQK
IPNKPEFEDSNPSPLPPDMAPGQSYQPQSESASSSSMDKYHIHHISEPTRQENWTPLK
NDLENHLEDFELEVELLITLIKSEKGSLGFTVTKGNQRIGCYVHDVIQDPAKSDGRLKPG
DRLIKVNDTDVTNMTHTDAVNLLRAASKTVRLVIGRVLELPRIPMLPHLLPDITLTCNKEE
LGFSLCGGHDSLYQVVYISDINPRSVAAIEGNLQLLDVIHYVNGVSTQGMTLEEVNRALD
MSLPSLVLKATRNDLPVVPSSKRSAVSAPKSTKGNGSYSVGSCSQPALTPNDSFSTVA
GEEINEISYPKGKCSTYQIKGSPNLTLPKESYIQEDDIYDDSQEAEVIQSLLDVVDEEAQN

LLNENNAAGYSCGPGTLKMNGKLSEERTEDTDCDGSPLPEYFTEATKMNGCEEYCEE
KVKSESLIQKPQEKKTDDDEITWGNDELPIERTNHEDSDKDHSFLTNDELAVLPVVKVLP
SGKYTGANLKSVIRVLRGLLDQGIPSKELENLQELKPLDQCLIGQTKENRRKNRYKNILP
YDATRVPLGDEGGYINASFIKIPVGKEEFVYIACQGPLPTTVGDFWQMIWEQKSTVIAM
MTQEVEGEKIKCQRYWPNILGKTTMVSNRLRLALVRMQQLKGFVVRAMTLEDIQTREV
RHISHLNFTAWPDHDTPSQPDDLLTFISYMRHIHRSGPIITHCSAGIGRSGTLICIDVVLGL
ISQDLDFDISDLVRCMRLQRHGMVQTEDQYIFCYQVILYVLTRLQAEEEQKQQPQLLK

SEQ ID No:148
MEPDSVIEDKTIELMCSVPRSLWLGCANLVESMCALSCLQSMPSVRCLQISNGTSSVIV
SRKRPSEGNYQKEKDLCIKYFDQWSESDQVEFVEHLISRMCHYQHGHINSYLKPMLQR
DFITALPEQGLDHIAENILSYLDARSLCAAELVCKEWQRVISEGMLWKKLIERMVRTDPL
WKGLSERRGWDQYLFKNRPTDGPPNSFYRSLYPKIIQDIETIESNWRCGRHNLQRIQC
RSENSKGVYCLQYDDEKIISGLRDNSIKIWDKTSLECLKVLTGHTGSVLCLQYDERVIVT
GSSDSTVRVWDVNTGEVLNTLIHHNEAVLHLRFSNGLMVTCSKDRSIAVWDMASATDIT
LRRVLVGHRAAVNVVDFDDKYIVSASGDRTIKVWSTSTCEFVRTLNGHKRGIACLQYRD
RLVVSGSSDNTIRLWDIECGACLRVLEGHEELVRCIRFDNKRIVSGAYDGKIKVWDLQA
ALDPRAPASTLCLRTLVEHSGRVFRLQFDEFQIISSSHDDTILIWDFLNVPPSAQNETRS
PSRTYTYISR

SEQ ID No:149
MDFTEAYADTCSTVGLAAREGNVKVLRKLLKKGRSVDVADNRGWMPIHEAAYHNSVE
CLQMLINADSSENYIKMKTFEGFCALHLAASQGHWKIVQILLEAGADPNATTLEETTPLF
LAVENGQIDVLRLLLQHGANVNGSHSMCGWNSLHQASFQENAEIIKLLLRKGANKECQ
DDFGITPLFVAAQYGKLESLSILISSGANVNCQALDKATPLFIAAQEGHTKCVELLLSSGA
DPDLYCNEDSWQLPIHAAAQMGHTKILDLLIPLTNRACDTGLNKVSPVYSAVFGGHEDC
LEILLRNGYSPDAQACLVFGFSSPVCMAFQKDCEFFGIVNILLKYGAQINELHLAYCLKYE
KFSIFRYFLRKGCSLGPWNHIYEFVNHAIKAQAKYKEWLPHLLVAGFDPLILLCNSWIDS
VSIDTLIFTLEFTNWKTLAPAVERMLSARASNAWILQQHIATVPSLTHLCRLEIRSSLKSE
RLRSDSYISQLPLPRSLHNYLLYEDVLRMYEVPELAAIQDG

SEQ ID No:150
MEPQLGPEAAALRPGWLALLLWVSALSCSFSLPASSLSSLVPQVRTSYNFGRTFLGLD
KCNACIGTSICKKFFKEEIRSDNWLASHLGLPPDSLLSYPANYSDDSKIWRPVEIFRLVSK

YQNEISDRKICASASAPKTCSIERVLRKTERFQKWLQAKRLTPDLVQDCHQGQRELKFL
CMLR

SEQ ID No:151










SEQ ID No:152
MSDNEDNFDGDDFDDVEEDEGLDDLENAEEEGQENVEILPSGERPQANQKRITTPYMT
KYERARVLGTRALQIAMCAPVMVELEGETDPLLIAMKELKARKIPIIIRRYLPDGSYEDWG
VDELIITD

SEQ ID No:153
MQIDVDPQEDPQNAPDVNYVVENPSLDLEQYAASYSGLMRIERLQFIADHCPTLRVEAL
KMALSFVQRTFNVDMYEEIHRKLSEATRELQNAPDAIPESGVEPPALDTAWVEATRKKA
LLKLEKLDTDLKNYKGNSIKESIRRGHDDLGDHYLDCGDLSNALKCYSRARDYCTSAKH
VINMCLNVIKVSVYLQNWSHVLSYVSKAESTPEIAEQRGERDSQTQAILTKLKCAAGLAE
LAARKYKQAAKCLLLASFDHCDFPELLSPSNVAIYGGLCALATFDRQELQRNVISSSSFK
LFLELEPQVRDIIFKFYESKYASCLKMLDEMKDNLLLDMYLAPHVRTLYTQIRNRALIQYF
SPYVSADMHRMAAAFNTTVAALEDELTQLILEGLISARVDSHSKILYARDVDQRSTTFEK
SLLMGKEFQRRAKAMMLRAAVLRNQIHVKSPPREGSQGELTPANSQSRMSTNM

SEQ ID No:154
MALLPASLSAVLEGEASVQDSPGSVMKSYVSETMASPGKDNYRMKSYKNNALNPEEM
RRRREEEGIQLRKQKREQQLFKRRNVELINEEAAMFDSLLMDSYVSSTTGESVITREMV
EMLFSDDSDLQLATTQKFRKLLSKEPSPPIDEVINTPRVVDRFVEFLKRNENCTLQFEAA
WALTNIASGTSQQTKIVIEAGAVPIFIELLNSDFEDVQEQAVWALGNIAGDSSVCRDYVL

NCSILNPLLTLLTKSTRLTMTRNAVWALSNLCRGKNPPPEFAKVSPCLPVLSRLLFSSDS
DLLADACWALSYLSDGPNEKIQAVIDSGVCRRLVELLMHNDYKVASPALRAVGNIVTGD
DIQTQVILNCSALPCLLHLLSSPKESIRKEACWTISNITAGNRAQIQAVIDANIFPVLIEILQK
AEFRTRKEAAWAITNATSGGTPEQIRYLVSLGCIKPLCDLLTVMDSKIVQVALNGLENILR
LGEQEGKRSGSGVNPYCGLIEEAYGLDKIEFLQSHENQEIYQKAFDLIEHYFGVEDDDS
SLAPQVDETQQQFIFQQPEAPMEGFQL

SEQ ID No:155
MLVMRNMFSHRLPVHRKYDLKGSLVSREASDKEKVKELPTLKDMDFLNKNQKVYIGEE
EKKIFLEKLKRDVEFLVQLKIMDYSLLLGIHDIIRGSEPEEEAPVREDESEVDGDCSLTGP
PALVGSYGTSPEGIGGYIHSHRPLGPGEFESFIDVYAIRSAEGAPQKEVYFMGLIDILTQY
DAKKKAAHAAKTVKHGAGAEISTVHPEQYAKRFLDFITNIFA

SEQ ID No:156
MPFFGNTFSPKKTPPRKSASLSNLHSLDRSTREVELGLEYGSPTMNLAGQSLKFENGQ
WIAETGVSGGVDRREVQRLRRRNQQLEEENNLLRLKVDILLDMLSESTAESHLMEKEL
DELRISRKRK

SEQ ID No:157
MASNDYTQQATQSYGAYPTQPGQGYSQQSSQPYGQQSYSGYSQSTDTSGYGQSSY
SSYGQSQNTGYGTQSTPQGYGSTGGYGSSQSSQSSYGQQSSYPGYGQQPAPSSTS
GSYGSSSQSSSYGQPQSGSYSQQPSYGGQQQSYGQQQSYNPPQGYGQQNQYNSS
SGGGGGGGGGNYGQDQSSMSSGGGSGGGYGNQDQSGGGGSGGYGQQDRGGR
GRGGSGGGGGGGGGYNRSSGGYEPRGRGGGRGGRGGMGGSDRGGFNKFGGPR
DQGSRHDSEQDNSDNNTIFVQGLGENVTIESVADYFKQIGIIKTNKKTGQPMINLYTDRE
TGKLKGEATVSFDDPPSAKAAIDWFDGKEFSGNPIKVSFATRRADFNRGGGNGRGGR
GRGGPMGRGGYGGGGSGGGGRGGFPSGGGGGGGQQRAGDWKCPNPTCENMNFS
WRNECNQCKAPKPDGPGGGPGGSHMGGNYGDDRRGGRGGYDRGGYRGRGGDRG
GFRGGRGGGDRGGFGPGKMDSRGEHRQDRRERPY

SEQ ID No:158
MEEPQAGGEDAARFSCPPNFTAKPPASESPRFSLEALTGPDTELWLIQAPADFAPECF
NGRHVPLSGSQIVKGKLAGKRHRYRVLSSCPQAGEATLLAPSTEAGGGLTCASAPQGT
LRILEGPQQSLSGSPLQPIPASPPPQIPPGLRPRFCAFGGNPPVTGPRSALAPNLLTSGK

KKKEMQVTEAPVTQEAVNGHGALEVDMALGSPEMDVRKKKKKKNQQLKEPEAAGPVG
TEPTVETLEPLGVLFPSTTKKRKKPKGKETFEPEDKTVKQEQINTEPLEDTVLSPTKKRK
RQKGTEGMEPEEGVTVESQPQVKVEPLEEAIPLPPTKKRKKEKGQMAMMEPGTEAME
PVEPEMKPLESPGGTMAPQQPEGAKPQAQAALAAPKKKTKKEKQQDATVEPETEVVG
PELPDDLEPQAAPTSTKKKKKKKERGHTVTEPIQPLEPELPGEGQPEARATPGSTKKRK
KQSQESRMPETVPQEEMPGPPLNSESGEEAPTGRDKKRKQQQQQPV

SEQ ID No:159
MNSGVAMKYGNDSSAELSELHSAALASLKGDIVELNKRLQQTERERDLLEKKLAKAQC
EQSHLMREHEDVQERTTLRYEERITELHSVIAELNKKIDRLQGTTIREEDEYSELRSELS
QSQHEVNEDSRSMDQDQTSVSIPENQSTMVTADMDNCSDLNSELQRVLTGLENVVCG
RKKSSCSLSVAEVDRHIEQLTTASEHCDLAIKTVEEIEGVLGRDLYPNLAEERSRWEKEL
AGLREENESLTAMLCSKEEELNRTKATMNAIREERDRLRRRVRELQTRLQSVQATGPS
SPGRLTSTNRPINPSTGELSTSSSSNDIPIAKIAERVKLSKTRSESSSSDRPVLGSEISSIG
VSSSSVAEHLAHSLQDCSNIQEIFQTLYSHGSAISESKIREFEVETERLNSRIEHLKSQNDL
LTITLEECKSNAERMSMLVGKYESNATALRLALQYSEQCIEAYELLLALAESEQSLILGQF
RAAGVGSSPGDQSGDENITQMLKRAHDCRKTAENAAKALLMKLDGSCGGAFAVAGCS
VQPWESLSSNSHTSTTSSTASSCDTEFTKEDEQRLKDYIQQLKNDRAAVKLTMLELESI
HIDPLSYDVKPRGDSQRLDLENAVLMQELMAMKEEMAELKAQLYLLEKEKKALELKLST
REAQEQAYLVHIEHLKSEVEEQKEQRMRSLSSTSSGSKDKPGKECADAASPALSLAEL
RTTCSENELAAEFTNAIRREKKLKARVQELVSALERLTKSSEIRHQQSAEFVNDLKRANS
NLVAAYEKAKKKHQNKLKKLESQMMAMVERHETQVRMLKQRIALLEEENSRPHTNETS
L

SEQ ID No:160
MEEDQELERKISGLKTSMAEGERKTALEMVQAAGTDRHCVTFVLHEEDHTLGNSLRYM
IMKNPEVEFCGYTTTHPSESKINLRIQTRGTLPAVEPFQRGLNELMNVCQHVLDKFEASI
KDYKDQKASRNESTF

SEQ ID No:161








SEQ ID No:162
MLISKNMPWRRLQGISFGMYSAEELKKLSVKSITNPRYLDSLGNPSANGLYDLALGPAD
SKEVCSTCVQDFSNCSGHLGHIELPLTVYNPLLFDKLYLLLRGSCLNCHMLTCPRAVIHL
LLCQLRVLEVGALQAVYELERILSRFLEENADPSASEIREELEQYTTEIVQNNLLGSQGA
HVKNVCESKSKLIALFWKAHMNAKRCPHCKTGRSVVRKEHNSKLTITFPAMVHRTAGQ
KDSEPLGIEEAQIGKRGYLTPTSAREHLSALWKNEGFFLNYLFSGMDDDGMESRFNPS
VFFLDFLVVPPSRSRPVSRLGDQMFTNGQTVNLQAVMKDVVLIRKLLALMAQEQKLPEE
VATPTTDEEKDSLIAIDRSFLSTLPGQSLIDKLYNIWIRLQSHVNIVFDSEMDKLMRDKYP
GIRQILEKKEGLFRKHMMGKRVDSTARSVICPDMYINTNEIGIPMVFATKLTYPQPVTPW
NVQELRQAVINGPNVHPGASMVINEDGSRTALSAVDMTQREAVAKQLLTPATGAPKPQ
GTKIVCRHVKNGDILLLNRQPTLHRPSIQAHRARILPEEKVLRLHYANCKAYNADFDGDE
MNAHFPQSELGRAEAYVLACTDQQYLVPKDGQPLAGLIQDHMVSGASMTTRGCFFTR
EHYMELVYRGLTDKVGRVKLLSPSILKPFPLWTGKQVVSTLLINIIPEDHIPLNLSGKAKIT
GKAWVKETPRSVPGFNPDSMCESQVIIREGELLCGVLDKAHYGSSAYGLVHCCYEIYG
GETSGKVLTCLARLFTAYLQLYRGFTLGVEDILVKPKRDVKRQRIIEESTHCGPQAVRAA
LNLPEAASYDEVRGKWQDAHLGKDQRDFNMIDLKFKEEVNHYSNEINKACMPFGLHRQ
FPENTLQLMVQSGAKGSTVNTMQISCLLGQIELEGRRSTPLMASGKSLPCFEPYEFTPRA
GGFVTGRFLTGIKPPEFFFHCMAGREGLVDTAVKTSRSGYLQRCIIKHLEGLVVQYDLT
VRDSDGSVVQFLYGEDGLDIPKTQFLQPKQFPFLASNYEVIMKSQHLHEVLSRADPKKA
LHHFRAIKKWQSKHPNTLLRRGAFLSYSQKIQEAVKALKLESENRNGRRPWDSGRMLR
MWYELDEESRRKYQKKAAACPDPSLSVWRPDIYFASVSETFETKVDDYSQEWAAQTE
KSYEKSELSLDRLRTLLQLKWQRSLCEPGEAVGLLAAQSIGEPSTQMTLNTFHFAGRGE
MNVTLGIPRLREILMVASANIKTPMMSVPVLNTKKALKRVKSLKKQLTRVCLGEVLQKID
VQESFCMEEKQNKFQVYQLRFQFLPHAYYQQEKCLRPEDILRFMETRFFKLLMESIKKK
NNKASAFRNVNTRRATQRDLDNAGELGRSRGEQEGDEEEEGHIVDAEAEEGDADASD
AKRKEKQEEEVDYESEEEEEREGEENDDEDMQEERNPHREGARKTQEQDEEVGLGG
PVPSHPPDAAPETHPQPGAPGAEAMERRVQAVREIHPFIDDYQYDTEESLWCQVTVKL
PLMKINFDMSSLVVSLAHGAVIYATKGITRCLLNETTNNKNEKELVLNTEGINLPELFKYA
EVLDLRRLYSNDIHAIANTYGIEALRVIEKEIKDVFAVYGIAVDPRHLSLVADYMCFEGVY
KPLNRFGIRSNSSPLQQMTFETSFQFLKQATMLGSHDELRSPSACLVVGKVVRGGTGL
FELKQPLR

SEQ ID No:163
MIIPVRCFTCGKIVGNKWEAYLGLLQAEYTEGDALDALGLKRYCCRRMLLAHVDLIEKLL
NYAPLEK

SEQ ID No:164
MAAEVLPSARWQYCGAPDGSQRAVLVQFSNGKLQSPGNMRFTLYENKDSTNPRKRN
QRILAAETDRLSYVGNNFGTGALKCNTLCRHFVGILNKTSGQMEVYDAELFNMQPLFSD
VSVESELALESQTKTYREKMDSCIEAFGTTKQKRALNTRRMNRVGNESLNRAVAKAAE
TIIDTKGVTALVSDAIHNDLQDDSLYLPPCYDDAAKPEDVYKFEDLLSPAEYEALQSPSE
AFRNVTSEEILKMIEENSHCTFVIEALKSLPSDVESRDRQARCIWFLDTLIKFRAHRVVKR
KSALGPGVPHIINTKLLKHFTCLTYNNGRLRNLISDSMKAKITAYVIILALHIHDFQIDLTVL
QRDLKLSEKRMMEIAKAMRLKISKRKVSVAAGSEEDHKLGTLSLPLPPAQTSDRLAKRR
KIT

SEQ ID No:165
MDQNNSLPPYAQGLASPQGAMTPGIPIFSPMMPYGTGLTPQPIQNTNSLSILEEQQRQ
QQQQQQQQQQQQQQQQQQQQQQQQQQQQQQQQAVAAAAVQQSTSQQA
TQGTSGQAPQLFHSQTLTTAPLPGTTPLYPSPMTPMTPITPATPASESSGIVPQLQNIVS
TVNLGCKLDLKTIALRARNAEYNPKRFAAVIMRIREPRTTALIFSSGKMVCTGAKSEEQS
RLAARKYARVVQKLGFPAKFLDFKIQNMVGSCDVKFPIRLEGLVLTHQQFSSYEPELFP
GLIYRMIKPRIVLLIFVSGKVVLTGAKVRAEIYEAFENIYPILKGFRKTT

SEQ ID No:166
MSVMDLANTCSSFQSDLDFCSDCGSVLPLPGAQDTVTCIRCGFNINVRDFEGKVVKTS
VVFHQLGTAMPMSVEEGPECQGPVVDRRCPRCGHEGMAYHTRQMRSADEGQTVFYT
CTNCKFQEKEDS

SEQ ID No:167
MAAAGARPVELGFAESAPAWRLRSEQFPSKVGGRPAWLGAAGLPGPQALACELCGR
PLSFLLQVYAPLPGRPDAFHRCIFLFCCREQPCCAGLRVFRNQLPRKNDFYSYEPPSEN
PPPETGESVCLQLKSGAHLCRVCGCLGPKTCSRCHKAYYCSKEHQTLDWRLGHKQAC
AQPDHLDHIIPDHNFLFPEFEIVIETEDEIMPEVVEKEDYSEIIGSMGEALEEGLDSMAKH

ESREDKIFQKFKTQIALEPEQILRYGRGIAPIWISGENIPQEKDIPDCPCGAKRILEFQVMP
QLLNYLKADRLGKSIDWGILAAFTCAESCSLGTGYTEEFVWKQDVTDTP

SEQ ID No:168
MDDEEETYRLWKIRKTIMQLCHDRGYLVTQDELDQTLEEFKAQFGDKPSEGRPRRTDL
TVLVAHNDDPTDQMFVFFPEEPKVGIKTIKVYCQRMQEENITRALIVVQQGMTPSAKQS
LVDMAPKYILEQFLEQELLINITEHELVPEHVVMTKEEVTELLARYKLRENQLPRIQAGDP
VARYFGIKRGQVVKIIRPSETAGRYITYRLVQ

SEQ ID No:169
MPHEELPSLQRPRYGSIVDDERLSAEEMDERRRQNIAYEYLCHLEEAKRWMEVCLVEE
LPPTTELEEGLRNGVYLAKLAKFFAPKMVSEKKIYDVEQTRYKKSGLHFRHTDNTVQWL
RAMESIGLPKIFYPETTDVYDRKNIPRMIYCIHALSLYLFKLGIAPQIQDLLGKVDFTEEEIS
NMRKELEKYGIQMPSFSKIGGILANELSVDEAALHAAVIAINEAVEKGIAEQTVVTLRNPN
AVLTLVDDNLAPEYQKELWDAKKKKEENARLKNSCISEEERDAYEELLTQAEIQGNINKV
NRQAAVDHINAVIPEGDPENTLLALKKPEAQLPAVYPFAAAMYQNELFNLQKQNTMNYL
AHEELLIAVEMLSAVALLNQALESNDLVSVQNQLRSPAIGLNNLDKAYVERYANTLLSVK
LEVLSQGQDNLSWNEIQNCIDMVNAQIQEENDRVVAVGYINEAIDEGNPLRTLETLLLPT
ANISDVDPAHAQHYQDVLYHAKSQKLGDSESVSKVLWLDEIQQAVDEANVDEDRAKQ
WVTLVVDVNQCLEGKKSSDILSVLKSSTSNANDIIPECADKYYDALVKAKELKSERVSSD
GSWLKLNLHKKYDYYYNTDSKESSWVTPESCFYKESWLTGKEIEDIIEEVTVGYIRENIW
SASEELLLRFQATSSGPILREEFEARKSFLHEQEENVVKIQAFWKGYKQRKEYMHRRQT
FIDNTDSVVKIQSWFRMATARKSYLSRLQYFRDHNNEIVKIQSLLRANKARDDYKTLVGS
ENPPLTVIRKFVYLLDQSDLDFQEELEVARLREEVVTKIRANQQLEKDLNLMDIKIGLLVK
NRITLEDVISHSKKLNKKKGGEMEILNNTDNQGIKSLSKERRKTLETYQQLFYLLQTNPLY
LAKLIFQMPQNKSTKFMDTVIFTLYNYASNQREEYLLLKLFKTALEEEIKSKVDQVQDIVT
GNPTVIKMVVSFNRGARGQNTLRQLLAPVVKEIIDDKSLIINTNPVEVYKAWVNQLETQT
GEASKLPYDVTTEQALTYPEVKNKLEASIENLRRVTDKVLNSIISSLDLLPYGLRYIAKVLK
NSIHEKFPDATEDELLKIVGNLLYYRYMNPAIVAPDGFDIIDMTAGGQINSDQRRNLGSV
AKVLQHAASNKLFEGENEHLSSMNNYLSETYQEFRKYFKEACNVPEPEEKFNMDKYTD
LVTVSKPVIYISIEEIISTHSLLLEHQDAIAPEKNDLLSELLGSLGEVPTVESFLGEGAVDPN
DPNKANTLSQLSKTEISLVLTSKYDIEDGEAIDSRSLMIKTKKLIIDVIRNQPGNTLTEILET
PATAQQEVDHATDMVSRAMIDSRTPEEMKHSQSMIEDAQLPLEQKKRKIQRNLRTLEQ
TGHVSSENKYQDILNEIAKDIRNQRIYRKLRKAELAKLQQTLNALNKKAAFYEEQINYYDT

YIKTCLDNLKRKNTRRSIKLDGKGEPKGAKRAKPVKYTAAKLHEKGVLLDIDDLQTNQFK
NVTFDIIATEDVGIFDVRSKFLGVEMEKVQLNIQDLLQMQYEGVAVMKMFDKVKVNVNL
LIYLLNKKFYGK

SEQ ID No:170


















SEQ ID No:171
MDPGSRWRNLPSGPSLKHLTDPSYGIPREQQKAALQELTRAHVESFNYAVHEGLGLAV
QADINWAVNGISKGIIKQFLGYVPIMVKSKLCNLRNLPPQALIEHHEEAEEMGGYFIINGIE
KVIRMLIMPRRNFPIAMIRPKWKTRGPGYTQYGVSMHCVREEHSAVNMNLHYLENGTV
MLNFIYRKELFFRPLGFALKALVSFSDYQIFQELIKGKEDDSFLRNSVSQMLRIVMEEGC
LTQKQVLNYLGECFRVKLNVPDWYPNEQAAEFLFNQCICIHLKSNTEKFYMLCLMTRKL
FALAKGECMEDNPDSLVNQEVLTPGQLFLMFLKEKLEGWLVSIKIAFDKKAQKTSVSMN
TDNLMRIFTMGIDLTKPFEYLFATGNLRSKTGLGLLQDSGLCVVADKLNFIRYLSHFRCV
HRGADFAKMRTTTVRRLLPESWGFLCPVHTPDGEPCGLMNHLTAVCEVVTQFVYTASI
PALLCNLGVTPIDGAPHRSYSECYPVLLDGVMVGWVDKDLAPGIADSLRHFKVLREKRI
PPWMEVVLIPMTGKPSLYPGLFLFTTPCRLVRPVQNLALGKEELIGTMEQIFMNVAIFED

EVFAGVTTHQELFPHSLLSVIANFIPFSDHNQSPRNMYQCQMGKQTMGFPLLTYQDRS
DNKLYRLQTPQSPLVRPSMYDYYDMDNYPIGTNAIVAVISYTGYDMEDAMIVNKASWER
GFAHGSVYKSEFIDLSEKIKQGDSSLVFGIKPGDPRVLQKLDDDGLPFIGAKLQYGDPYY
SYLNLNTGESFVMYYKSKENCVVDNIKVCSNDTGSGKFKCVRITMRVPRNPTIGDKFAS
RHGRKGILSRLWPAEDMPFTESGMVPDILFNPHGFPSRMTIGMLIESMAGKSAALHGLC
HDATPFIFSEENSALEYFGEMLKAAGYNFYGTERLYSGISGLELEADIFIGVVYYQRLRH
MVSDKFQVRTTGARDRVTNQPIGGRNVQGGIRFGEMERDALLAHGTSFLLHDRLFNCS
DRSVAHVCVKCGSLLSPLLEKPPPSWSAMRNRKYNCTLCSRSDTIDTVSVPYVFRYFV
AELAAMNIKVKLDVV

SEQ ID No:172
MAGILFEDIFDVKDIDPEGKKFDRVSRLHCESESFKMDLILDVNIQIYPVDLGDKFRLVIAS
TLYEDGTLDDGEYNPTDDRPSRADQFEYVMYGKVYRIEGDETSTEAATRLSAYVSYGG
LLMRLQGDANNLHGFEVDSRVYLLMKKLAF

SEQ ID No:173
MSTCGSNQHPLASAYICSKNVNGAREQSEEDWLRLGRAGSQERGDRIRKAEERILRAE
TPNSFGKAAQRNCVSRLADTRSTILAAVLHTNGLANLLKMICFMHEKSIGTDCVLYILDR
TQEHPLLEKVNLLPMSAFAEG

SEQ ID No:174
MKETDREAVATAVQRVAGMLQRPDQLDKVEQYRRREARKKASVEARLKAAIQSQLDG
VRTGLSQLHNALNDVKDIQQSLADVSKDWRQSINTIESLKDVKDAVVQHSQLAAAVENL
KNIFSVPEIVRETQDLIEQGALLQAHRKLMDLECSRDGLMYEQYRMDSGNTRDMTLIHG
YFGSTQGLSDELAKQLWMVLQRSLVTVRRDPTLLVSVVRIIEREEKIDRRILDRKKQTGF
VPPGRPKNWKEKMFTILERTVTTRIEGTQADTRESDKMWLVRHLEIIRKYVLDDLIVAKN
LMVQCFPPHYEIFKNLLNMYHQALSTRMQDLASEDLEANEIVSLLTWVLNTYTSTEMMR
NVELAPEVDVGTLEPLLSPHVVSELLDTYMSTLTSNIIAWLRKALETDKKDWVKETEPEA
DQDGYYQTTLPAIVFQMFEQNLQVAAQISEDLKTKVLVLCLQQMNSFLSRYKDEAQLYK
EEHLRNRQHPHCYVQYMIAIINNCQTFKESIVSLKRKYLKNEVEEGVSPSQPSMDGILDA
IAKEGCSGLLEEVFLDLEQHLNELMTKKWLLGSNAVDIICVTVEDYFNDFAKIKKPYKKR
MTAEAHRRVVVEYLRAVMQKRISFRSPEERKEGAEKMVREAEQLRFLFRKLASGFGED
VDGYCDTIVAVAEVIKLTDPSLLYLEVSTLVSKYPDIRDDHIGALLAVRGDASRDMKQTIM
ETLEQGPAQASPSYVPLFKDIVVPSLNVAKLLK

SEQ ID No:175
MQGGFLNALSERRLKGRTVNWPAQHTVAVPHVSPGPTVLGEDDPRTPGTLGRRRVEA
TKPPGARKVSLGHASLPLGKQRFCDSGPEAWRVRAAVSAGKVKATGQGLQLESGQGS
DCPPSPTLAFLTSRGVNSVRINPSLNLAELEGSSAEVSAWVRMPAQSLCEPEFVYLHNE
VEPSPLATWEIWRQPWKSPKPRARHRGRTQWAIAVLTTGTFIGDAWQCQTWLGAETS
RAKRNDTGSHASPALGACDGTGSKLMMTGIARGHTHSEAPVIRFMNLPKGVWGTLWG
TGRSAIVAMIHSFSLPLMASESRGPDELALSFLVKMRTLSPDDFKENVDGRSCSYRLVT
GKLLYEENISVFLQIIRESHNEFSVDKVGNCVVDCGQVKRPFN

SEQ ID No:176
MSRSRQPPLVTGISPNEGIPWTKVTIRGENLGTGPTDLIGLTICGHNCLLTAEWMSASKI
VCRVGQAKNDKGDIIVTTKSGGRGTSTVSFKLLKPEKIGILDQSAVWVDEMNYYDMRTD
RNKGIPPLSLRPANPLGIEIEKSKFSQKDLEMLFHGMSADFTSENFSAAWYLIENHSNTS
FEQLKMAVTNLKRQANKKSEGSLAYVKGGLSTFFEAQDALSAIHQKLEADGTEKVEGS
MTQKLENVLNRASNTADTLFQEVLGRKDKADSTRNALNVLQRFKFLFNLPLNIERNIQK
GDYDVVINDYEKAKSLFGKTEVQVFKKYYAEVETRIEALRELLLDKLLETPSTLHDQKRYI
RYLSDLHASGDPAWQCIGAQHKWILQLMHSCKEGYVKDLKGKDFSSNVLCNPGLHSP
MLDLDNDTRPSVLGHLSQTASLKRGSSFQSGRDDTWRYKTPHRVAFVEKLTKLVLSQL
PNFWKLWISYVNGSLFSETAEKSGQIERSKNVRQRQNDFKKMIQEVMHSLVKLTRGAL
LPLSIRDGEAKQYGGWEVKCELSGQWLAHAIQTVRLTHESLTALEIPNDLLQTIQDLILDL
RVRCVMATLQHTAEEIKRLAEKEDWIVDNEGLTSLPCQFEQCIVCSLQSLKGVLECKPG
EASVFQQPKTQEEVCQLSINIMQVFIYCLEQLSTKPDADIDTTHLSVDVSSPDLFGSIHED
FSLTSEQRLLIVLSNCCYLERHTFLNIAEHFEKHNFQGIEKITQVSMASLKELDQRLFENYI
ELKADPIVGSLEPGIYAGYFDWKDCLPPTGVRNYLKEALVNIIAVHAEVFTISKELVPRVL
SKVIEAVSEELSRLMQCVSSFSKNGALQARLEICALRDTVAVYLTPESKSSFKQALEALP
QLSSGADKKLLEELLNKFKSSMHLQLTCFQAASSTMMKT

SEQ ID No:177
MFWKFDLNTTSHVDKLLDKEHVTLQELMDEDDILQECKAQNQKLLDFLCRQQCMEELV
SLITQDPPLDMEEKVRFKYPNTACELLTCDVPQISDRLGGDESLLSLLYDFLDHEPPLNP
LLASFFSKTIGNLIARKTEQVITFLKKKDKFISLVLKHIGTSALMDLLLRLVSCVEPAGLRQ
DVLHWLNEEKVIQRLVELIHPSQDEDRQSNASQTLCDIVRLGRDQGSQLQEALEPDPLL
TALESQDCVEQLLKNMFDGDRTESCLVSGTQVLLTLLETRRVGTEGLVDSFSQGLERS

YAVSSSVLHGIEPRLKDFHQLLLNPPKKKAILTTIGVLEEPLGNARLHGARLMAALLHTNT
PSINQELCRLNTMDLLLDLFFKYTWNNFLHFQVELCIAAILSHAAREERTEASGSESRVE
PPHENGNRSLETPQPAASLPDNTMVTHLFQKCCLVQRILEAWEANDHTQAAGGMRRG
NMGHLTRIANAVVQNLERGPVQTHISEVIRGLPADCRGRWESFVEETLTETNRRNTVDL
AFSDYQIQQMTANFVDQFGFNDEEFADQDDNINAPFDRIAEINFNIDADEDSPSAALFEA
CCSDRIQPFDDDEDEDIWEDSDTRCAARVMARPRFGAPHASESCSKNGPERGGQDG
KASLEAHRDAPGAGAPPAPGKKEAPPVEGDSEAGAMWTAVFDEPANSTPTAPGVVRD
VGSSVWAAGTSAPEEKGWAKFTDFQPFCCSESGPRCSSPVDTECSHAEGSRSQGPE
KAFSPASPCAWNVCVTRKAPLLASDSSSSGGSHSEDGDQKAASAMDAVSRGPGREAP
PLPTVARTEEAVGRVGCADSRLLSPACPAPKEVTAAPAVAVPPEATVAITTALSKAGPAI
PTPAVSSALAVAVPLGPIMAVTAAPAMVATLGTVTKDGQMPRQKELP

SEQ ID No:178
MAPLDLDKYVEIARLCKYLPENDLKRLCDYVCDLLLEESNVQPVSTPVTVCGDIHGQFY
DLCELFRTGGQVPDTNYIFMGDFVDRGYYSLETFTYLLALKAKWPDRITLLRGNHESRQ
ITQVYGFYDECQTKYGNANAWRYCTKVFDMLTVAALIDEQILCVHGGLSPDIKTLDQIRTI
ERNQEIPHKGAFCDLVWSDPEDVDTWAISPRGAGWLFGAKVTNEFVHINNLKLICRAH
QLVHEGYKFMFDEKLVTVWSAPNYCYRCGNIASIMVFKDVNTREPKLFRAVPDSERVIP
PRTTTPYFL

SEQ ID No:179
MKSLKSRLRRQDVPGPASSGAAAASAHAADWNKYDDRLMKAAERGDVEKVTSILAKK
GVNPGKLDVEGRSVFHVVTSKGNLECLNAILIHGVDITTSDTAGRNALHLAAKYGHALCL
QKLLQYNCPTEHADLQGRTALQKKAMADCPSSIQLLCDHGASVNAKDVKGRTPLVLAT
QMSRPTICQLLIDRGADVNSRDKQNRTALMLGCEYGCRDAVEVLIKNGADISLLDALGH
DSSYYARIGDNLDILTLLKTASENTNKGRELWKKGPSLQQRNLTHMQDEVNVKSHQRE
HQNIQDLEIENEDLKERLRKIQQEQRILLDKVNGLQLQLNEEVMVADDLESEREKLKSLL
AAKEKQHEESLRTIEALKNRFKYFESDHLGSGSHFSNRKEDMLLKQGQMYMADSQCTS
PGIPAHMQSRSMLRPLELSLPSQTSYSENEILKKELEAMRTFCESAKQDRLKLQNELAH
KVAECKALALECERVKEDSDEQIKQLEDALKDVQKRMYESEGKVKQMQTHFLALKEHL
TSEAASGNHRLTEELKDQLKDLKVKYEGASAEVGKLRNQIKQNEMIVEEFKRDEGKLIE
ENKRLQKELSMCEMEREKKGRKVTEMEGQAKELSAKLALSIPAEKFENMKSSLSNEVN
EKAKKLVEMEREHEKSLSEIRQLKRELENVKAKLAQHVKPEEHEQVKSRLEQKSGELG
KKITELTLKNQTLQKEIEKVYLDNKLLKEQAHNLTIEMKNHYVPLKVSEDMKKSHDAIIDD

LNRKLLDVTQKYTEKKLEMEKLLLENDSLSKDVSRLETVFVPPEKHEKEIIALKSNIVELK
KQLSELKKKCGEDQEKIHALTSENTNLKKMMSNQYVPVKTHEEVKMTLNDTLAKTNREL
LDVKKKFEDINQEFVKIKDKNEILKRNLENTQNQIKAEYISLAEHEAKMSSLSQSMRKVQ
DSNAEILANYRKGQEEIVTLHAEIKAQKKELDTIQECIKVKYAPIVSFEECERKFKATEKEL
KDQLSEQTQKYSVSEEEVKKNKQENDKLKKEIFTLQKDLRDKTVLIEKSHEMERALSRK
TDELNKQLKDLSQKYTEVKNVKEKLVEENAKQTSEILAVQNLLQKQHVPLEQVEALKKS
LNGTIENLKEELKSMQRCYEKEQQTVTKLHQLLENQKNSSVPLAEHLQIKEAFEKEVGII
KASLREKEEESQNKMEEVSKLQSEVQNTKQALKKLETREVVDLSKYKATKSDLETQISS
LNEKLANLNRKYEEVCEEVLHAKKKEISAKDEKELLHFSIEQEIKDQKERCDKSLTTITEL
QRRIQESAKQIEAKDNKITELLNDVERLKQALNGLSQLTYTSGNPTKRQSQLIDTLQHQV
KSLEQQLADADRQHQEVIAIYRTHLLSAAQGHMDEDVQEALLQIIQMRQGLVC

SEQ ID No:180







SEQ ID No:181
MLIFSVRDMFAKMSILGVRSFGIEDKDKQIITFFSPLTILVGPNGAGKTTIIECLKYICTGDF
PPGTKGNTFVHDPKVAQETDVRAQIRLQFRDVNGELIAVQRSMVCTQKSKKTEFKTLE
GVITRTKHGEKVSLSSKCAEIDREMISSLGVSKAVLNNVIFCHQEDSNWPLSEGKALKQ
KFDEIFSATRYIKALETLRQVRQTQGQKVEEYQMELKYLKQYKEKACEIRDQITSKEAQL
TSSKEIVKSYENELDPLKNRLKEIEHNLSKIMKLDNEIKALDSRKKQMEKDNSELEEKME
KVFQGTDEQLNDLYHNHQRTVREKERKLVDCHRELEKLNKESRLLNQEKSELLVEQGR
LQLQADRHQEHIRARDSLIQSLATQLELDGFERGPFSERQIKNFHKLVRERQEGEAKTA
NQLMNDFAEKETLKQKQIDEIRDKKTGLGRIIELKSEILSKKQNELKNVKYELQQLEGSSD
RILELDQELIKAERELSKAEKNSNVETLKMEVISLQNEKADLDRTLRKLDQEMEQLNHHT
TTRTQMEMLTKDKADKDEQIRKIKSRHSDELTSLLGYFPNKKQLEDWLHSKSKEINQTR
DRLAKLNKELASSEQNKNHINNELERKEEQLSSYEDKLFDVCGSQDFESDLDRLKEEIE
KSSKQRAMLAGATAVYSQFITQLTDENQSCCPVCQRVFQTEAELQEAISDLQSKLRLAP
DKLKSTESELKKKEKRRDEMLGLAPMRQSIIDLKEKEIPELRNKLQNVNRDIQRLKNDIEE

QETLLGTIMPEEESAKVCLTDVTIMERFQMELKDVERKIAQQAAKLQGIDLDRTVQQVN
QEKQEKQHKLDTVSSKIELNRKLIQDQQEQIQHLKSTTNELKSEKLQISTNLQRRQQLEE
QTVELSTEVQSLYREIKDAKEQVSPLETTLEKFQQEKEELINKKNTSNKIAQDKLNDIKEK
VKNIHGYMKDIENHIQDGKDDYMKQKETELNKVIAQLSECEKHKEKINEDMRLMRQDID
TQKIQERWLQDNLTLRKRNEELKEVEEEGKQHLKEMGQMQVLQMKSEHQKLEENIDNI
KRNHNLALGRQKGYEEEIIHFKKELREPQFRDAEEKYREMMIVMRTTELVNKDLDIYYKT
LDQAIMKFHSMKMEEINKIIRDLWRSTYRGQDIEYIEIRSDADENVSASDKRRNYNYRVV
MLKGDTALDMRGRCSAGQKVLASLIIRLALAETFCLNCGIIALDEPTTNLDRENIESLAHA
LVEIIKSRSQQRNFQLLVITHDEDFVELLGRSEYVEKFYRIKKNIDQCSEIVKCSVSSLGF
NVH

SEQ ID No:182
MEKATVPVAAATAAEGEGSPPAVAAVAGPPAAAEVGGGVGGSSRARSASSPRGMVR
VCDLLLKKKPPQQQHHKAKRNRTCRPPSSSESSSDSDNSGGGGGGGGGGGGGGT
SSNNSEEEEDDDEEEEVSEVESFILDQDDLENPMLETASKLLLSGTADGADLRTVDPE
TQARLEALLEAAGIGKLSTADGKAFADPEVLRRLTSSVSCALDEAAAALTRMRAESTAN
AGQSDNRSLAEACSEGDVNAVRKLLIEGRSVNEHTEEGESLLCLACSAGYYELAQVLLA
MHANVEDRGIKGDITPLMAAANGGHVKIVKLLLAHKADVNAQSSTGNTALTYACAGGYV
DVVKVLLESGASIEDHNENGHTPLMEAGSAGHVEVARLLLENGAGINTHSNEFKESALT
LACYKGHLEMVRFLLEAGADQEHKTDEMHTALMEACMDGHVEVARLLLDSGAQVNMP
ADSFESPLTLAACGGHVELAALLIERGASLEEVNDEGYTPLMEAAREGHEEMVALLLGQ
GANINAQTEETQETALTLACCGGFLEVADFLIKAGADIELGCSTPLMEAAQEGHLELVKY
LLAAGANVHATTATGDTALTYACENGHTDVADVLLQAGADLEHESEGGRTPLMKAARA
GHVCTVQFLISKGAECESEPQLIMTILSALACAGGHLAVVELLLAHGADPTHRLKDGSTM
LIEAPKGGHTSVGCYLLDYPNNLLSAPPPDVTQLTPPSHDLNRAPRVPVQALPMVVPPQ
EPDKPPANVATTLPIRNKAASKQKSSSHLPANSQDVQGYITNQSPESIVEEAQGKLTELE
QRIKEAIEKNAQLQSLELAHADQLTKEKIEELNKTREEQIQKKQKILEELQKVERELQLKT
QQQLKKQYLEVKAQRIQLQQQQQQSCQHLGLLTPVGVGEQLSEGDYARLQQVDPVLL
KDEPQQTAAQMGFAPIQPLAMPQALPLAAGPLPPGSIANLTELQGVIVGQPVLGQAQLA
GLGQGILTETQQGLMVASPAQTLNDTLDDIMAAVSGRASAMSNTPTHSIAASISQPQTP
TPSPIISPSAMLPIYPAIDIDAQTESNHDTALTLACAGGHEELVQTLLERGASIEHRDKKG
FTPLILAATSWSCWCCGNIAGQWCSTLKPSLKEPRTHHSPWLVLGEDRRWLELLLGSR
GQLQEHRKVLITHL

SEQ ID No:183














SEQ ID No:184
MEASQEGHLELVKYLLASGANVHATTATGDTALTYACENGHTDVADVLLQAGADLDKQ
EDMKTILEGIDPAKHQEHESEGGRTPLMKAARAGHLCTVQFLISKGANVNRATANNDHT
VVSLACAGGHLAVVELLLAHGADPTHRLKDGSTMLIEAAKGGHTNVVSYLLDYPNNVLS
VPTTDVSQLPPPSQDQSQEPDRTSQENSPALLGVQKAVSTRVPTGSNSSSQTTECLTP
ESCSQTTSNVASQSMPPVYPSVDVDAHTESNHDTALTLACAGGHEELVSVLIARDAKIE
HRDKKGFTPLILAATAGHVGVVEILLDKGGDIEAQSERTKDTPLSLACSGGRQEVVDLLL
ARGANKEHRNVSDYTPLSLAASGGYVNIIKILLNAGAEINSRTGSKLGISPLMLAAMNGH
VPAVKLLLDMGSDINAQIETNRNTALTLACFQGRAEVVSLLLDRKANVEHRAKTGLTPLM
EAASGGYAEVGRVLLDKGADVNAPPVPSSRDTALTIAADKGHYKFCELLIHRGAHIDVR
NKKGNTPLWLASNGGHFDVVQLLVQAGADVDAADNRKITPLMSAFRKGHVKVVQYLVK
EVNQFPSDIECMRYIATITDKELLKKCHQCVETIVKAKDQQAAEANKNASILLKELDLEKS
REESRKQALAAKREKRKEKRKKKKKKKKKKKK

SEQ ID No:185
GAEATAMAFLKLRDQPSLVQAIFNGDPDEVRALIFKKEDVNFQDNEKRTPLHAAAYLGD
AEIIELLILSGARVNAKDSKWLTPLHRAVASCSEEAVQVLLKHSADVNARDKNWQTPLHI
AAANKAVKCAEALVPLLSNVNVSDRAGRTALHHAAFSGHGEMVKLLLSRGANINAFDKK
DRRAIHWAAYMGHIEVVKLLVSHGAEVTCKDKKSYTPLHAAASSGMISVVKYLLDLGVD

MNEPNAYGNTPLHVACYNGQDVVVNELIDCGAIVNQKNEKGFTPLHFAAASTHGALCL
ELLVGNGADVNMKSKDGKTPLHMTALHGRFSRSQTIIQSGAVIDCEDKNGNTPLHIAAR
YGHELLINTLITSGADTAKRGIHGMFPLHLAALSGFSDCCRKLLSSGFDIDTPDDFGRTC
LHAAAAGGNLECLNLLLNTGADFNKKDKFGRSPLHYAAANCNYQCLFALVGSGASVND
LDERGCTPLHYAATSDTDGKCLEYLLRNDANPGIRDKQGYNAVHYSAAYGHRLCLQLIA
SETPLDVLMETSGTDMLSDSDNRATISPLHLAAYHGHHQALEVLVQSLLDLDVRNSSGR
TPLDLAAFKGHVECVDVLINQGASILVKDYILKRTPIHAAATNGHSECLRLLIGNAEPQNA
VDIQDGNGQTPLMLSVLNGHTDCVYSLLNKGANVDAKDKWGRTALHRGAVTGHEECV
DALLQHGAKCLLRDSRGRTPIHLSAACGHIGVLGALLQSAASMDANPATADNHGYTALH
WACYNGHETCVELLLEQEVFQKTEGNAFSPLHCAVINDNEGAAEMLIDTLGASIVNATD
SKGRTPLHAAAFTDHVECLQLLLSHNAQVNSVDSTGKTPLMMAAENGQTNTVEMLVSS
ASAELTLQDNSKNTALHLACSKGHETSALLILEKITDRNLINATNAALQTPLHVAARNGLT
MVVQELLGKGASVLAVDENGYTPALACAPNKDVADCLALILATMMPVSSSSPLSSLTFN
AINRYTNTSKTVSFEALPIMRNEPSSYCSFNNIGGEQEYLYTDVDELNDSDSETY

SEQ ID No:186
MQIDVDPQEDPQNAPDVNYVVENPSLDLEQYAASYSGLMRIERLQFIADHCPTLRVEAL
KMALSFVQRTFNVDMYEEIHRKLSEATRELQNAPDAIPESGVEPPALDTAWVEATRKKA
LLKLEKLDTDLKNYKGNSIKESIRRGHDDLGDHYLDCGDLSNALKCYSRARDYCTSAKH
VINMCLNVIKVSVYLQNWSHVLSYVSKAESTPEIAEQRGERDSQTQAILTKLKCAAGLAE
LAARKYKQAAKCLLLASFDHCDFPELLSPSNVAIYGGLCALATFDRQELQRNVISSSSFK
LFLELEPQVRDIIFKFYESKYASCLKMLDEMKDNLLLDMYLAPHVRTLYTQIRNRALIQYF
SPYVSADMHRMAAAFNTTVAALEDELTQLILEGLISARVDSHSKILYARDVDQRSTTFEK
SLLMGKEFQRRAKAMMLRAAVLRNQIHVKSPPREGSQGELTPANSQSRMSTNM

SEQ ID No:187
MNGEAICSALPTIPYHKLADLRYLSRGASGTVSSARHADWRVQVAVKHLHIHTPLLDSE
RKDVLREAEILHKARFSYILPILGICNEPEFLGIVTEYMPNGSLNELLHRKTEYPDVAWPL
RFRILHEIALGVNYLHNMTPPLLHHDLKTQNILLDNEFHVKIADFGLSKWRMMSLSQSRS
SKSAPEGGTIIYMPPENYEPGQKSRASIKHDIYSYAVITWEVLSRKQPFEDVTNPLQIMY
SVSQGHRPVINEESLPYDIPHRARMISLIESGWAQNPDERPSFLKCLIELEPVLRTFEEIT
FLEAVIQLKKTKLQSVSSAIHLCDKKKMELSLNIPVNHGPQEESCGSSQLHENSGSPETS
RSLPAPQDNDFLSRKAQDCYFMKLHHCPGNHSWDSTISGSQRAAFCDHKTTPCSSAII
NPLSTAGNSERLQPGIAQQWIQSKREDIVNQMTEACLNQSLDALLSRDLIMKEDYELVS

TKPTRTSKVRQLLDTTDIQGEEFAKVIVQKLKDNKQMGLQPYPEILVVSRSPSLNLLQNK
SM

SEQ ID No:188
MAAQRRSLLQSEQQPSWTDDLPLCHLSGVGSASNRSYSADGKGTESHPPEDSWLKF
RSENNCFLYGVFNGYDGNRVTNFVAQRLSAELLLGQLNAEHAEADVRRVLLQAFDVVE
RSFLESIDDALAEKASLQSQLPEGVPQHQLPPQYQKILERLKTLEREISGGAMAVVAVLL
NNKLYVANVGTNRALLCKSTVDGLQVTQLNVDHTTENEDELFRLSQLGLDAGKIKQVGII
CGQESTRRIGDYKVKYGYTDIDLLSAAKSKPIIAEPEIHGAQPLDGVTGFLVLMSEGLYK
ALEAAHGPGQANQEIAAMIDTEFAKQTSLDAVAQAVVDRVKRIHSDTFASGGERARFCP
RHEDMTLLVRNFGYPLGEMSQPTPSPAPAAGGRVYPVSVPYSSAQSTSKTSVTLSLVM
PSQGQMVNGAHSASTLDEATPTLTNQSPTLTLQSTNTHTQSSSSSSDGGLFRSRPAHS
LPPGEDGRVEPYVDFAEFYRLWSVDHGEQSVVTAP

SEQ ID No:189
MSLLNCENSCGSSQSESDCCVAMASSCSAVTKDDSVGGTASTGNLSSSFMEEIQGYD
VEFDPPLESKYECPICLMALREAVQTPCGHRFCKACIIKSIRDAGHKCPVDNEILLENQLF
PDNFAKREILSLMVKCPNEGCLHKMELRHLEDHQAHCEFALMDCPQCQRPFQKFHINI
HILKDCPRRQVSCDNCAASMAFEDKEIHDQNCPLANVICEYCNTILIREQMPNHYDLDC
PTAPIPCTFSTFGCHEKMQRNHLARHLQENTQSHMRMLAQAVHSLSVIPDSGYISEVRN
FQETIHQLEGRLVRQDHQIRELTAKMETQSMYVSELKRTIRTLEDKVAEIEAQQCNGIYI
WKIGNFGMHLKCQEEEKPVVIHSPGFYTGKPGYKLCMRLHLQLPTAQRCANYISLFVHT
MQGEYDSHLPWPFQGTIRLTILDQSEAPVRQNHEEIMDAKPELLAFQRPTIPRNPKGFG
YVTFMHLEALRQRTFIKDDTLLVRCEVSTRFDMGSLRREGFQPRSTDAGV

SEQ ID No:190





SEQ ID No:191
MYEQLKGEWNRKSPNLSKCGEELGRLKLVLLELNFLPTTGTKLTKQQLILARDILEIGAQ
WSILRKDIPSFERYMAQLKCYYFDYKEQLPESAYMHQLLGLNLLFLLSQNRVAEFHTEL

ERLPAKDIQTNVYIKHPVSLEQYLMEGSYNKVFLAKGNIPAESYTFFIDILLDTIRDEIAGCI
EKAYEKILFTEATRILFFNTPKKMTDYAKKRGWVLGPNNYYSFASQQQKPEDTTIPSTEL
AKQVIEYARQLEMIV

SEQ ID No:192
MNLLPNIESPVTRQEKMATVWDEAEQDGIGEEVLKMSTEEIIQRTRLLDSEIKIMKSEVL
RVTHELQAMKDKIKENSEKIKVNKTLPYLVSNVIELLDVDPNDQEEDGANIDLDSQRKGK
CAVIKTSTRQTYFLPVIGLVDAEKLKPGDLVGVNKDSYLILETLPTEYDSRVKAMEVDER
PTEQYSDIGGLDKQIQELVEAIVLPMNHKEKFENLGIQPPKGVLMYGPPGTGKTLLARAC
AAQTKATFLKLAGPQLVQMFIGDGAKLVRDAFALAKEKAPSIIFIDELDAIGTKRFDSEKA
GDREVQRTMLELLNQLDGFQPNTQVKVIAATNRVDILDPALLRSGRLDRKIEFPMPNEE
ARARIMQIHSRKMNVSPDVNYEELARCTDDFNGAQCKAVCVEAGMIALRRGATELTHE
DYMEGILEVQAKKKANLQYYA

SEQ ID No:193
MFRNQYDNDVTVWSPQGRIHQIEYAMEAVKQGSATVGLKSKTHAVLVALKRAQSELAA
HQKKILHVDNHIGISIAGLTADARLLCNFMRQECLDSRFVFDRPLPVSRLVSLIGSKTQIP
TQRYGRRPYGVGLLIAGYDDMGPHIFQTCPSANYFDCRAMSIGARSQSARTYLERHMS
EFMECNLNELVKHGLRALRETLPAEQDLTTKNVSIGIVGKDLEFTIYDDDDVSPFLEGLE
ERPQRKAQPAQPADEPAEKADEPMEH

SEQ ID No:194
MSRRYDSRTTIFSPEGRLYQVEYAMEAIGHAGTCLGILANDGVLLAAERRNIHKLLDEVF
FSEKIYKLNEDMACSVAGITSDANVLTNELRLIAQRYLLQYQEPIPCEQLVTALCDIKQAY
TQFGGKRPFGVSLLYIGWDKHYGFQLYQSDPSGNYGGWKATCIGNNSAAAVSMLKQD
YKEGEMTLKSALALAIKVLNKTMDVSKLSAEKVEIATLTRENGKTVIRVLKQKEVEQLIKK
HEEEEAKAEREKKEKEQKEKDK

SEQ ID No:195
MITSAAGIISLLDEDEPQLKEFALHKLNAVVNDFWAEISESVDKIEVLYEDEGFRSRQFAA
LVASKVFYHLGAFEESLNYALGAGDLFNVNDNSEYVETIIAKCIDHYTKQCVENADLPEG
EKKPIDQRLEGIVNKMFQRCLDDHKYKQAIGIALETRRLDVFEKTILESNDVPGMLAYSL
KLCMSLMQNKQFRNKVLRVLVKIYMNLEKPDFINVCQCLIFLDDPQAVSDILEKLVKEDN
LLMAYQICFDLYESASQQFLSSVIQNLRTVGTPIASVPGSTNTGTVPGSEKDSDSMETE

EKTSSAFVGKTPEASPEPKDQTLKMIKILSGEMAIELHLQFLIRNNNTDLMILKNTKDAVR
NSVCHTATVIANSFMHCGTTSDQFLRDNLEWLARATNWAKFTATASLGVIHKGHEKEAL
QLMATYLPKDTSPGSAYQEGGGLYALGLIHANHGGDIIDYLLNQLKNASNDIVRHGGSL
GLGLAAMGTARQDVYDLLKTNLYQDDAVTGEAAGLALGLVMLGSKNAQAIEDMVGYAQ
ETQHEKILRGLAVGIALVMYGRMEEADALIESLCRDKDPILRRSGMYTVAMAYCGSGNN
KAIRRLLHVAVSDVNDDVRRAAVESLGFILFRTPEQCPSVVSLLSESYNPHVRYGAAMA
LGICCAGTGNKEAINLLEPMTNDPVNYVRQGALIASALIMIQQTEITCPKVNQFRQLYSKV
INDKHDDVMAKFGAILAQGILDAGGHNVTISLQSRTGHTHMPSVVGVLVFTQFWFWFPL
SHFLSLAYTPTCVIGLNKDLKMPKVQYKSNCKPSTFAYPAPLEVPKEKEKEKVSTAVLSI
TAKAKKKEKEKEKKEEEKMEVDEAEKKEEKEKKKEPEPNFQLLDNPARVMPAQLKVLT
MPETCRYQPFKPLSIGGIIILKDTSEDIEELVEPVAAHGPKIEEEEQEPEPPEPFEYIDD

SEQ ID No:196
MIVEVEELPAVAAQVAKTRVAEKEEKTKGQDVASKLPLVTLHTQCRLKLLKLERIKDYLL
MVEEFIRNQEQIKLLEEKQEEGRSKVDDLRGTPMSVGNLEEIIDDNHAIVSTSVGSEHYD
SIISFVEKDLLEPGCSILLRHKVHAVIGVLMDDTGPLVTMMKVEKAPQETYVNTGGLDNQ
IQEIKESMELPLPHPEYYEEMGTKPPKGVILCGPPGTATFLRVVGSELIQKYLGDGPKLV
RQVFQVAEEHAPSIMFTDEIEAIGTKRYDSNSGGEREIQQTMLELELLNQLGGFDSRED
VKVIMATKQVETLDPVLIRPGRIDKKIEFHLPDEKTKKHIFQIHTSRMTLANDVTLDDLIMA
KDDFSGADIKAICTEAGLMALREHRMKATNEDFKKSIESVLYKKHEGIPEGLYL

SEQ ID No:197
MAEASPHPGRYFCHCCSVEIVPRLPDYICPRCESGFIEELPEETRSTENGSAPSTAPTD
QSRPPLEHVDQHLFTLPQGYGQFAFGIFDDSFEIPTFPPGAQADDGRDPESRRERDHP
SRHRYGARQPRARLTTRRATGRHEGVPTLEGIIQQLVNGIITPATIPSLGPWGVLHSNP
MDYAWGANGLDAIITQLLNQFENTGPPPADKEKIQALPTVPVTEEHVGSGLECPVCKDD
YALGERVRQLPCNHLFHDGCIVPWLEQHDSCPVCRKSLTGQNTATNPPGLTGVSFSSS
SSSSSSSPSNENATSNS

SEQ ID No:198
MEEGGRDKAPVQPQQSPAAAPGGTDEKPSGKERRDAGDKDKEQELSEEDKQLQDEL
EMLVERLGEKDTSLYRPALEELRRQIRSSTTSMTSVPKPLKFLRPHYGKLKEIYENMAP
GENKRFAADIISVLAMTMSGERECLKYRLVGSQEELASWGHEYVRHLAGEVAKEWQEL
DDAEKVQREPLLTLVKEIVPYNMAHNAEHEACDLLMEIEQVDMLEKDIDENAYAKVCLYL

TSCVNYVPEPENSALLRCALGVFRKFSRFPEALRLALMLNDMELVEDIFTSCKDVVVQK
QMAFMLGRHGVFLELSEDVEEYEDLTEIMSNVQLNSNFLALARELDIMEPKVPDDIYKT
HLENNRFGGSGSQVDSARMNLASSFVNGFVNAAFGQDKLLTDDGNKWLYKNKDHGM
LSAAASLGMILLWDVDGGLTQIDKYLYSSEDYIKSGALLACGIVNSGVRNECDPALALLS
DYVLHNSNTMRLGSIFGLGLAYAGSNREDVLTLLLPVMGDSKSSMEVAGVTALACGMIA
VGSCNGDVTSTILQTIMEKSETELKDTYARWLPLGLGLNHLGKGEAIEAILAALEVVSEPF
RSFANTLVDVCAYAGSGNVLKVQQLLHICSEHFDSKEKEEDKDKKEKKDKDKKEAPAD
MGAHQGVAVLGIALIAMGEEIGAEMALRTFGHLLRYGEPTLRRAVPLALALISVSNPRLNI
LDTLSKFSHDADPEVSYNSIFAMGMVGSGTNNARLAAMLRQLAQYHAKDPNNLFMVRL
AQGLTHLGKGTLTLCPYHSDRQLMSQVAVAGLLTVLVSFLDVRNIILGKSHYVLYGLVAA
MQPRMLVTFDEELRPLPVSVRVGQAVDVVGQAGKPKTITGFQTHTTPVLLAHGERAEL
ATEEFLPVTPILEGFVILRKNPNYDL

SEQ ID No:199
MKDVPGFLQQSQNSGPGQPAVWHRLEELYTKKLWHQLTLQVLDFVQDPCFAQGDGLI
KLYENFISEFEHRVNPLSLVEIILHVVRQMTDPNVALTFLEKTREKVKSSDEAVILCKTAIG
ALKLNIGDLQVTKETIEDVEEMLNNLPGVTSVHSRFYDLSSKYYQTIGNHASYYKDALRF
LGCVDIKDLPVSEQQERAFTLGLAGLLGEGVFNFGELLMHPVLESLRNTDRQWLIDTLY
AFNSGNVERFQTLKTAWGQQPDLAANEAQLLRKIQLLCLMEMTFTRPANHRQLTFEEIA
KSAKITVNEVELLVMKALSVGLVKGSIDEVDKRVHMTWVQPRVLDLQQIKGMKDRLEF
WCTDVKSMEMLVEHQAHDILT

SEQ ID No:200
MADPRDKALQDYRKKLLEHKEIDGRLKELREQLKELTKQYEKSENDLKALQSVGQIVGE
VLKQLTEEKFIVKATNGPRYVVGCRRQLDKSKLKPGTRVALDMTTLTIMRYLPREVDPL
VYNMSHEDPGNVSYSEIGGLSEQIRELREVIELPLTNPELFQRVGIIPPKGCLLYGPPGT
GKTLLARAVASQLDCNFLKVVSSSIVDKYIGESARLIREMFNYARDHQPCIIFMDEIDAIG
GRRFSEGTSADREIQRTLMELLNQMDGFDTLHRVKMIMATNRPDTLDPALLRPGRLDR
KIHIDLPNEQARLDILKIHAGPITKHGEIDYEAIVKLSDGFNGADLRNVCTEAGMFAIRADH
DFVVQEDFMKAVRKVADSKKLESKLDYKPV

SEQ ID No:201
MPDYLGADQRKTKEDEKDDKPIRALDEGDIALLKTYGQSTYSRQIKQVEDDIQQLLKKIN
ELTGIKESDTGLAPPALWDLAADKQTLQSEQPLQVARCTKIINADSEDPKYIINVKQFAKF

VVDLSDQVAPTDIEEGMRVGVDRNKYQIHIPLPPKIDPTVTMMQVEEKPDVTYSDVGGC
KEQIEKLREVVETPLLHPERFVNLGIEPPKGVLLFGPPGTGKTLCARAVANRTDACFIRVI
GSELVQKYVGEGARMVRELFEMARTKKACLIFFDEIDAIGGARFDDGAGGDNEVQRTM
LELINQLDGFDPRGNIKVLMATNRPDTLDPALMRPGRLDRKIEFSLPDLEGRTHIFKIHAR
SMSVERDIRFELLARLCPNSTGAEIRSVCTEAGMFAIRARRKIATEKDFLEAVNKVIKSYA
KFSATPRYMTYN

SEQ ID No:202
MADGGSERADGRIVKMEVDYSATVDQRLPECAKLAKEGRLQEVIETLLSLEKQTRTASD
MVSTSRILVAVVKMCYEAKEWDLLNENIMLLSKRRSQLKQAVAKMVQQCCTYVEEITDL
PIKLRLIDTLRMVTEGKIYVEIERARLTKTLATIKEQNGDVKEAASILQELQVETYGSMEKK
ERVEFILEQMRLCLAVKDYIRTQIISKKINTKFFQEENTEKLKLKYYNLMIQLDQHEGSYLS
ICKHYRAIYDTPCIQAESEKWQQALKSVVLYVILAPFDNEQSDLVHRISGDKKLEEIPKYK
DLLKLFTTMELMRWSTLVEDYGMELRKGSLESPATDVFGSTEEGEKRWKDLKNRVVE
HNIRIMAKYYTRITMKRMAQLLDLSVDESEAFLSNLVVNKTIFAKVDRLAGIINFQRPKDP
NNLLNDWSQKLNSLMSLVNKTTHLIAKEEMIHNLQ

SEQ ID No:203













SEQ ID No:204
MPLENLEEEGLPKNPDLRIAQLRFLLSLPEHRGDAAVRDELMAAVRDNNMAPYYEALC
KSLDWQIDVDLLNKMKKANEDELKRLDEELEDAEKNLGESEIRDAMMAKAEYLCRIGDK

EGALTAFRKTYDKTVALGHRLDIVFYLLRIGLFYMDNDLITRNTEKAKSLIEEGGDWDRR
NRLKVYQGLYCVAIRDFKQAAELFLDTVSTFTSYELMDYKTFVTYTVYVSMIALERPDLR
EKVIKGAEILEVLHSLPAVRQYLFSLYECRYSVFFQSLAVVEQEMKKDWLFAPHYRYYV
REMRIHAYSQLLESYRSLTLGYMAEAFGVGVEFIDQELSRFIAAGRLHCKIDKVNEIVET
NRPDSKNWQYQETIKKGDLLLNRVQKLSRVINM

SEQ ID No:205
MPELAVQKVVVHPLVLLSVVDHFNRIGKVGNQKRVVGVLLGSWQKKVLDVSNSFAVPF
DEDDKDDSVWFLDHDYLENMYGMFKKVNARERIVGWYHTGPKLHKNDIAINELMKRYC
PNSVLVIIDVKPKDLGLPTEAYISVEEVHDDGTPTSKTFEHVTSEIGAEEAEEVGVEHLLR
DIKDTTVGTLSQRITNQVHGLKGLNSKLLDIRSYLEKVATGKLPINHQIIYQLQDVFNLLPD
VSLQEFVKAFYLKTNDQMVVVYLASLIRSVVALHNLINNKIANRDAEKKEGQEKEESKKD
RKEDKEKDKDKEKSDVKKEEKKEKK

SEQ ID No:206
MKQEGSARRRGADKAKPPPGGGEQEPPPPPAPQDVEMKEEAATGGGSTGEADGKTA
AAAAEHSQRELDTVTLEDIKEHVKQLEKAVSGKEPRFVLRALRMLPSTSRRLNHYVLYK
AVQGFFTSNNATRDFLLPFLEEPMDTEADLQFRPRTGKAASTPLLPEVEAYLQLLVVIFM
MNSKRYKEAQKISDDLMQKISTQNRRALDLVAAKCYYYHARVYEFLDKLDVVRSFLHAR
LRTATLRHDADGQATLLNLLLRNYLHYSLYDQAEKLVSKSVFPEQANNNEWARYLYYT
GRIKAIQLEYSEARRTMTNALRKAPQHTAVGFKQTVHKLLIVVELLLGEIPDRLQFRQPSL
KRSLMPYFLLTQAVRTGNLAKFNQVLDQFGEKFQADGTYTLIIRLRHNVIKTGVRMISLS
YSRISLADIAQKLQLDSPEDAEFIVAKAIRDGVIEASINHEKGYVQSKEMIDIYSTREPQLA
FHQRISFCLDIHNMSVKAMRFPPKSYNKDLESAEERREREQQDLEFAKEMAEDDDDSF
P

SEQ ID No:207
MGQSQSGGHGPGGGKKDDKDKKKKYEPPVPTRVGKKKKKTKGPDAASKLPLVTPHT
QCRLKLLKLERIKDYLLMEEEFIRNQEQMKPLEEKQEEERSKVDDLRGTPMSVGTLEEII
DDNHAIVSTSVGSEHYVSILSFVDKDLLEPGCSVLLNHKVHAVIGVLMDDTDPLVTVMKV
EKAPQETYADIGGLDNQIQEIKESVELPLTHPEYYEEMGIKPPKGVILYGPPGTGKTLLAK
AVANQTSATFLRVVGSELIQKYLGDGPKLVRELFRVAEEHAPSIVFIDEIDAIGTKRYDSN
SGGEREIQRTMLELLNQLDGFDSRGDVKVIMATNRIETLDPALIRPGRIDRKIEFPLPDEK

TKKRIFQIHTSRMTLADDVTLDDLIMAKDDLSGADIKAICTEAGLMALRERRMKVTNEDF
KKSKENVLYKKQEGTPEGLYL

SEQ ID No:208
MEYLIGIQGPDYVLVASDRVAASNIVQMKDDHDKMFKMSEKILLLCVGEAGDTVQFAEYI
QKNVQLYKMRNGYELSPTAAANFTRRNLADCLRSRTPYHVNLLLAGYDEHEGPALYYM
DYLAALAKAPFAAHGYGAFLTLSILDRYYTPTISRERAVELLRKCLEELQKRFILNLPTFSV
RIIDKNGIHDLDNISFPKQGS

SEQ ID No:209
MAAAAVVEFQRAQSLLSTDREASIDILHSIVKRDIQENDEEAVQVKEQSILELGSLLAKTG
QAAELGGLLKYVRPFLNSISKAKAARLVRSLLDLFLDMEAATGQEVELCLECIEWAKSEK
RTFLRQALEARLVSLYFDTKRYQEALHLGSQLLRELKKMDDKALLVEVQLLESKTYHAL
SNLPKARAALTSARTTANAIYCPPKLQATLDMQSGIIHAAEEKDWKTAYSYFYEAFEGYD
SIDSPKAITSLKYMLLCKIMLNTPEDVQALVSGKLALRYAGRQTEALKCVAQASKNRSLA
DFEKALTDYRAELRDDPIISTHLAKLYDNLLEQNLIRVIEPFSRVQIEHISSLIKLSKADVER
KLSQMILDKKFHGILDQGEGVLIIFDEPPVDKTYEAALETIQNMSKVVDSLYNKAKKLT

SEQ ID No:210
MVLESTMVCVDNSEYMRNGDFLPTRLQAQQDAVNIVCHSKTRSNPENNVGLITLANDC
EVLTTLTPDTGRILSKLHTVQPKGKITFCTGIRVAHLALKHRQGKNHKMRIIAFVGSPVED
NEKDLVKLAKRLKKEKVNVDIINFGEEEVNTEKLTAFVNTLNGKDGTGSHLVTVPPGPSL
ADALISSPILAGEGGAMLGLGASDFEFGVDPSADPELALALRVSMEEQRQRQEEEARR
AAAASAAEAGIATTGTEDSDDALLKMTISQQEFGRTGLPDLSSMTEEEQIAYAMQMSLQ
GAEFGQAESADIDASSAMDTSEPAKEEDDYDVMQDPEFLQSVLENLPGVDPNNEAIRN
AMGSLASQATKDGKKDKKEEDKK

SEQ ID No:211
MAATLLAARGAGPAPAWGPEAFTPDWESREVSTGTTIMAVQFDGGVVLGADSRTTTG
SYIANRVTDKLTPIHDRIFCCRSGSAADTQAVADAVTYQLGFHSIELNEPPLVHTAASLFK
EMCYRYREDLMAGIIIAGWDPQEGGQVYSVPMGGMMVRQSFAIGGSGSSYIYGYVDA
TYREGMTKEECLQFTANALALAMERDGSSGGVIRLAAIAESGVERQVLLGDQIPKFAVA
TLPPA

SEQ ID No:212








SEQ ID No:213
MPELAVQKVVVHPLVLLSVVDHFNRIGKVGNQKRVVGVLLGSWQKKVLDVSNSFAVPF
DEDDKDDSVWFLDHDYLENMYGMFKKVNARERIVGWYHTGPKLHKNDIAINELMKRYC
PNSVLVIIDVKPKDLGLPTEAYISVEEVHDDGTPTSKTFEHVTSEIGAEEAEEVGVEHLLR
DIKDTTVGTLSQRITNQVHGLKGLNSKLLDIRSYLEKVATGKLPINHQIIYQLQDVFNLLPD
VSLQEFVKAFYLKTNDQMVVVYLASLIRSVVALHNLINNKIANRDAEKKEGQEKEESKKD
RKEDKEKDKDKEKSDVKKEEKKEKK

SEQ ID No:214
MENQVLTPHVYWAQRHRELYLRVELSDVQNPAISITENVLHFKAQGHGAKGDNVYEFH
LEFLDLVKPEPVYKLTQRQVNITVQKKVSQWWERLTKQEKRPLFLAPDFDRWLDESDA
EMELRAKEEERLNKLRLESEGSPETLTNLRKGYLFMYNLVQFLGFSWIFVNLTVRFCILG
KESFYDTFHTVADMMYFCQMLAVVETINAAIGVTTSPVLPSLIQLLGRNFILFIIFGTMEE
MQNKAVVFFVFYLWSAIEIFRYSFYMLTCIDMDWKVLTWLRYTLWIPLYPLGCLVEAVSV
IQSIPIFNETGRFSFTLPYPVKIKVRFSFFLQIYLIMIFLGLYINFRHLYKQRRRRYGKKRKR
STKKKDLDGFLPV

SEQ ID No:215


















SEQ ID No:216
MSYDRAITVFSPDGHLFQVEYAQEAVKKGSTAVGVRGRDIVVLGVEKKSVAKLQDERT
VRKICALDDNVCMAFAGLTADARIVINRARVECQSHRLTVEDPVTVEYITRYIASLKQRYT
QSNGRRPFGISALIVGFDFDGTPRLYQTDPSGTYHAWKANAIGRGAKSVREFLEKNYTD
EAIETDDLTIKLVIKALLEVVQSGGKNIELAVMRRDQSLKILNPEEIEKYVAEIEKEKEENE
KKKQKKAS

SEQ ID No:217
MALDGPEQMELEEGKAGSGLRQYYLSKIEELQLIVNDKSQNLRRLQAQRNELNAKVRLL
REELQLLQEQGSYVGEVVRAMDKKKVLVKVHPEGKFVVDVDKNIDINDVTPNCRVALR
NDSYTLHKILPNKVDPLVSLMMVEKVPDSTYEMIGGLDKQIKEIKEVIELPVKHPELFEAL
GIAQPKGVLLYGPPGTGKTLLARAVAHHTDCTFIRVSGSELVQKFIGEGARMVRELFVM
AREHAPSIIFMDEIDSIGSSRLEGGSGGDSEVQRTMLELLNQLDGFEATKNIKVIMATNRI
DILDSALLRPGRIDRKIEFPPPNEEARLDILKIHSRKMNLTRGINLRKIAELMPGASGAEVK
GVCTEAGMYALRERRVHVTQEDFEMAVAKVMQKDSEKNMSIKKLWK

SEQ ID No:218
MSRGSSAGFDRHITIFSPEGRLYQVEYAFKAINQGGLTSVAVRGKDCAVIVTQKKVPDK
LLDSSTVTHLFKITENIGCVMTGMTADSRSQVQRARYEAANWKYKYGYEIPVDMLCKRI
ADISQVYTQNAEMRPLGCCMILIGIDEEQGPQVYKCDPAGYYCGFKATAAGVKQTESTS
FLEKKVKKKFDWTFEQTVETAITCLSTVLSIDFKPSEIEVGVVTVENPKFRILTEAEIDAHL
VALAERD

SEQ ID No:219











SEQ ID No:220
MSCRQFSSSYLTSGGGGGGGLGSGGSIRSSYSRFSSSGGRGGGGRFSSSSGYGGGS
SRVCGRGGGGSFGYSYGGGSGGGFSASSLGGGFGGGSRGFGGASGGGYSSSGGF
GGGFGGGSGGGFGGGYGSGFGGLGGFGGGAGGGDGGILTANEKSTMQELNSRLAS
YLDKVQALEEANNDLENKIQDWYDKKGPAAIQKNYSPYYNTIDDLKDQIVDLTVGNNKTL
LDIDNTRMTLDDFRIKFEMEQNLRQGVDADINGLRQVLDNLTMEKSDLEMQYETLQEEL
MALKKNHKEEMSQLTGQNSGDVNVEINVAPGKDLTKTLNDMRQEYEQLIAKNRKDIEN
QYETQITQIEHEVSSSGQEVQSSAKEVTQLRHGVQELEIELQSQLSKKAALEKSLEDTK
NRYCGQLQMIQEQISNLEAQITDVRQEIECQNQEYSLLLSIKMRLEKEIETYHNLLEGGQ
EDFESSGAGKIGLGGRGGSGGSYGRGSRGGSGGSYGGGGSGGGYGGGSGSRGGS
GGSYGGGSGSGGGSGGGYGGGSGGGHSGGSGGGHSGGSGGNYGGGSGSGGGSG
GGYGGGSGSRGGSGGSHGGGSGFGGESGGSYGGGEEASGSGGGYGGGSGKSSHS

SEQ ID No:221
MTTCSRQFTSSSSMKGSCGIGGGIGGGSSRISSVLAGGSCRAPSTYGGGLSVSSRFSS
GGACGLGGGYGGGFSSSSSFGSGFGGGYGGGLGAGFGGGLGAGFGGGFAGGDGLL
VGSEKVTMQNLNDRLASYLDKVRALEEANADLEVKIRDWYQRQRPSEIKDYSPYFKTIE
DLRNKIIAATIENAQPILQIDNARLAADDFRTKYEHELALRQTVEADVNGLRRVLDELTLA
RTDLEMQIEGLKEELAYLRKNHEEEMLALRGQTGGDVNVEMDAAPGVDLSRILNEMRD
QYEQMAEKNRRDAETWFLSKTEELNKEVASNSELVQSSRSEVTELRRVLQGLEIELQS
QLSMKASLENSLEETKGRYCMQLSQIQGLIGSVEEQLAQLRCEMEQQSQEYQILLDVKT

RLEQEIATYRRLLEGEDAHLSSQQASGQSYSSREVFTSSSSSSSRQTRPILKEQSSSSF
SQGGQSS

SEQ ID No:222
MAAVQAAEVKVDGSEPKLSKNELKRRLKAEKKVAEKEAKQKELSEKQLSQATAAATNH
TTDNGVGPEEESVDPNQYYKIRSQAIHQLKVNGEDPYPHKFHVDISLTDFIQKYSHLQP
GDHLTDITLKVAGRIHAKRASGGKLIFYDLRGEGVKLQVMANSRNYKSEEEFIHINNKLR
RGDIIGVQGNPGKTKKGELSIIPYEITLLSPCLHMLPHLHFGLKDKETRYRQRYLDLILND
FVRQKFIIRSKIITYIRSFLDELGFLEIETPMMNIIPGGAVAKPFITYHNELDMNLYMRIAPEL
YHKMLVVGGIDRVYEIGRQFRNEGIDLTHNPEFTTCEFYMAYADYHDLMEITEKMVSGM
VKHITGSYKVTYHPDGPEGQAYDVDFTPPFRRINMVEELEKALGMKLPETNLFETEETR
KILDDICVAKAVECPPPRTTARLLDKLVGEFLEVTCINPTFICDHPQIMSPLAKWHRSKEG
LTERFELFVMKKEICNAYTELNDPMRQRQLFEEQAKAKAAGDDEAMFIDENFCTALEYG
LPPTAGWGMGIDRVAMFLTDSNNIKEVLLFPAMKPEDKKENVATTDTLESTTVGTSV

SEQ ID No:223

















SEQ ID No:224









SEQ ID No:225
MRLGNVDFTLGSNVPCVYLVFSVNRKKVDNDYNALRERLSTLPDKLSYNIMVPFGPFAF
MPGKLVHTNEVTVLLGDNWFAKCSAKQAVGLVEHRKEHVRKTIDDLKKVMKNFESRVE
FTEDLQKMSDAAGDIVDIREEIKCDFEFKAKHRIAHKPHSKPKTSDIFEADIANDVKSKDL
LADKELWARLEELERQEELLGELDSKPDTVIANGEDTTSSEEEKEDRNTNVNAMHQVT
DSHTPCHKDVASSEPFSGQVNSQLNCSVNGSSSYHSDDDDDDDDDDDNIDDDDGD
NDHEALGVGDNSIPTIYFSHTVEPKRVRINTGKNTTLKFSEKKEEAKRKRKNSTGSGHS
AQELPTIRTPADIYRAFVDVVNGEYVPRKSILKSRSRENSVCSDTSESSAAEFDDGRGV
LRSISCEEATCSDTSESILEEEPQENQKKLLPLSVTPEAFSGTVIEKEFVSPSLTPPPAIAH
PALPTIPERKEVLLEASEETGKRVSKFKAARLQQKD

SEQ ID No:226
MEALPLLAATTPDHGRHRRLLLLPLLLFLLPAGAVQGWETEERPRTREEECHFYAGGQ
VYPGEASRVSVADHSLHLSKAKISKPAPYWEGTAVIDGEFKELKLTDYRGKYLVFFFYPL
DFTFVCPTEIIAFGDRLEEFRSINTEVVACSVDSQFTHLAWINTPRRQGGLGPIRIPLLSD
LTHQISKDYGVYLEDSGHTLRGLFIIDDKGILRQITLNDLPVGRSVDETLRLVQAFQYTDK
HGEVCPAGWKPGSETIIPDPAGKLKYFDKLN

SEQ ID No:227
MDEQEALNSIMNDLVALQMNRRHRMPGYETMKNKDTGHSNRQSDVRIKFEHNGERRII
AFSRPVKYEDVEHKVTTVFGQPLDLHYMNNELSILLKNQDDLDKAIDILDRSSSMKSLRIL
LLSQDRNHNSSSPHSEVSRQVRIKASQSAGDINTIYQPPEPRSRHLSVSSQNPGRSSPP
PGYVPERQQHIARQGSYTSINSEGEFIPETSEQCMLDPLSSAENSLSGSCQSLDRSADS
PSFRKSRMSRAQSFPDNRQEYSDRETQLYDKGVKGGTYPRRYHVSVHHKDYSDGRR

TFPRIRRHQGNLFTLVPSSRSLSTNGENMGLAVQYLDPRGRLRSADSENALSVQERNV
PTKSPSAPINWRRGKLLGQGAFGRVYLCYDVDTGRELASKQVQFDPDSPETSKEVSAL
ECEIQLLKNLQHERIVQYYGCLRDRAEKTLTIFMEYMPGGSVKDQLKAYGALTESVTRK
YTRQILEGMSYLHSNMIVHRDIKGANILRDSAGNVKLGDFGASKRLQTICMSGTGMRSV
TGTPYWMSPEVISGEGYGRKADVWSLGCTVVEMLTEKPPWAEYEAMAAIFKIATQPTN
PQLPSHISEHGRDFLRRIFVEARQRPSAEELLTHHFAQLMY

SEQ ID No:228
MDDREDLVYQAKLAEQAERYDEMVESMKKVAGMDVELTVEERNLLSVAYKNVIGARR
ASWRIISSIEQKEENKGGEDKLKMIREYRQMVETELKLICCDILDVLDKHLIPAANTGESK
VFYYKMKGDYHRYLAEFATGNDRKEAAENSLVAYKAASDIAMTELPPTHPIRLGLALNF
SVFYYEILNSPDRACRLAKAAFDDAIAELDTLSEESYKDSTLIMQLLRDNLTLWTSDMQG
DGEEQNKEALQDVEDENQ

SEQ ID No:229
MDKNELVQKAKLAEQAERYDDMAACMKSVTEQGAELSNEERNLLSVAYKNVVGARRS
SWRVVSSIEQKTEGAEKKQQMAREYREKIETELRDICNDVLSLLEKFLIPNASQAESKVF
YLKMKGDYYRYLAEVAAGDDKKGIVDQSQQAYQEAFEISKKEMQPTHPIRLGLALNFSV
FYYEILNSPEKACSLAKTAFDEAIAELDTLSEESYKDSTLIMQLLRDNLTLWTSDTQGDEA
EAGEGGEN

SEQ ID No:230
MLWLALGPFPAMENQVLVIRIKIPNSGAVDWTVHSGPQLLFRDVLDVIGQVLPEATTTAF
EYEDEDGDRITVRSDEEMKAMLSYYYSTVMEQQVNGQLIEPLQIFPRACKPPGERNIHG
LKVNTRAGPSQHSSPAVSDSLPSNSLKKSSAELKKILANGQMNEQDIRYRDTLGHGNG
GTVYKAYHVPSGKILAVKVILLDITLELQKQIMSELEILYKCDSSYIIGFYGAFFVENRISICT
EFMDGGSLDVYRKMPEHVLGRIAVAVVKGLTYLWSLKILHRDVKPSNMLVNTRGQVKL
CDFGVSTQLVNSIAKTYVGTNAYMAPERISGEQYGIHSDVWSLGISFMEIQKNQGSLMP
LQLLQCIVDEDSPVLPVGEFSEPFVHFITQCMRKQPKERPAPEELMGHPFIVQFNDGNA
AVVSMWVCRALEERRSQQGPP

SEQ ID No:231
MESCYNPGLDGIIEYDDFKLNSSIVEPKEPAPETADGPYLVIVEQPKQRGFRFRYGCEG
PSHGGLPGASSEKGRKTYPTVKICNYEGPAKIEVDLVTHSDPPRAHAHSLVGKQCSEL

GICAVSVGPKDMTAQFNNLGVLHVTKKNMMGTMIQKLQRQRLRSRPQGLTEAEQREL
EQEAKELKKVMDLSIVRLRFSAFLRASDGSFSLPLKPVTSQPIHDSKSPGASNLKISRMD
KTAGSVRGGDEVYLLCDKVQKDDIEVRFYEDDENGWQAFGDFSPTDVHKQYAIVFRTP
PYHKMKIERPVTVFLQLKRKRGGDVSDSKQFTYYPLVEDKEEVQRKRRKALPTFSQPF
GGGSHMGGGSGGAAGGYGGAGGGGSLGFFPSSLAYSPYQSGAGPMRCYPGGGGG
A

SEQ ID No:232
MAENSGRAGKSSGSGAGKGAVSAEQVIAGFNRLRQEQRGLASKAAELEMELNEHSLVI
DTLKEVDETRKCYRMVGGVLVERTVKEVLPALENNKEQIQKIIETLTQQLQAKGKELNEF
REKHNIRLMGEDEKPAAKENSEGAGAKASSAGVLVS

SEQ ID No:233
MATPPKRRAVEATGEKVLRYETFISDVLQRDLRKVLDHRDKVYEQLAKYLQLRNVIERL
QEAKHSELYMQVDLGCNFFVDTVVPDTSRIYVALGYGFFLELTLAEALKFIDRKSSLLTE
LSNSLTKDSMNIKAHIHMLLEGLRELQGLQNFPEKPHH

SEQ ID No:234
MSAFEKPQIIAHIQKGFNYTVFDCKWVPCSAKFVTMGNFARGTGVIQLYEIQHGDLKLLR
EIEKAKPIKCGTFGATSLQQRYLATGDFGGNLHIWNLEAPEMPVYSVKGHKEIINAIDGIG
GLGIGEGAPEIVTGSRDGTVKVWDPRQKDDPVANMEPVQGENKRDCWTVAFGNAYN
QEERVVCAGYDNGDIKLFDLRNMALRWETNIKNGVCSLEFDRKDISMNKLVATSLEGKF
HVFDMRTQHPTKGFASVSEKAHKSTVWQVRHLPQNRELFLTAGGAGGLHLWKYEYPI
QRSKKDSEGIEMGVAGSVSLLQNVTLSTQPISSLDWSPDKRGLCVCSSFDQTVRVLIVT
KLNKI

SEQ ID No:235
MAELIQKKLQGEVEKYQQLQKDLSKSMSGRQKLEAQLTENNIVKEELALLDGSNVVFKL
LGPVLVKQELGEARATVGKRLDYITAEIKRYESQLRDLERQSEQQRETLAQLQQEFQRA
QAAKAGAPGKA

SEQ ID No:236
MPPISTPRRSDSAISVRSLHSESSMSLRSTFSLPEEEEEPEPLVFAEQPSVKLCCQLCC
SVFKDPVITTCGHTFCRRCALKSEKCPVDNVKLTVVVNNIAVAEQIGELFIHCRHGCRVA

GSGKPPIFEVDPRGCPFTIKLSARKDHEGSCDYRPVRCPNNPSCPPLLRMNLEAHLKE
CEHIKCPHSKYGCTFIGNQDTYETHLETCRFEGLKEFLQQTDDRFHEMHVALAQKDQEI
AFLRSMLGKLSEKIDQLEKSLELKFDVLDENQSKLSEDLMEFRRDASMLNDELSHINARL
NMGILGSYDPQQIFKCKGTFVGHQGPVWCLCVYSMGDLLFSGSSDKTIKVWDTCTTYK
CQKTLEGHDGIVLALCIQGCKLYSGSADCTIIVWDIQNLQKVNTIRAHDNPVCTLVSSHN
VLFSGSLKAIKVWDIVGTELKLKKELTGLNHWVRALVAAQSYLYSGSYQTIKIWDIRTLDC
IHVLQTSGGSVYSIAVTNHHIVCGTYENLIHVWDIESKEQVRTLTGHVGTVYALAVISTPD
QTKVFSASYDRSLRVWSMDNMICTQTLLRHQGSVTALAVSRGRLFSGAVDSTVKVWT
C

SEQ ID No:237
GDPGVAREPQAELVGAMLSPEAERVLRYLVEVEELAEEVLADKRQIVDLDTKRNQNRE
GLRALQKDLSLSEDVMVCFGNMFIKMPHPETKEMIEKDQDHLDKEIEKLRKQLKVKVNR
LFEAQGKPELKGFNLNPLNQDELKALKVILKG

SEQ ID No:238
MRQTLPCIYFWGGLLPFGMLCASSTTKCTVSHEVADCSHLKLTQVPDDLPTNITVLNLT
HNQLRRLPAANFTRYSQLTSLDVGFNTISKLEPELCQKLPMLKVLNLQHNELSQLSDKT
FAFCTNLTELHLMSNSIQKIKNNPFVKQKNLITLDLSHNGLSSTKLGTQVQLENLQELLLS
NNKIQALKSEELDIFANSSLKKLELSSNQIKEFSPGCFHAIGRLFGLFLNNVQLGPSLTEK
LCLELANTSIRNLSLSNSQLSTTSNTTFLGLKWTNLTMLDLSYNNLNVVGNDSFAWLPQ
LEYFFLEYNNIQHLFSHSLHGLFNVRYLNLKRSFTKQSISLASLPKIDDFSFQWLKCLEHL
NMEDNDIPGIKSNMFTGLINLKYLSLSNSFTSLRTLTNETFVSLAHSPLHILNLTKNKISKIE
SDAFSWLGHLEVLDLGLNEIGQELTGQEWRGLENIFEIYLSYNKYLQLTRNSFALVPSLQ
RLMLRRVALKNVDSSPSPFQPLRNLTILDLSNNNIANINDDMLEGLEKLEILDLQHNNLAR
LWKHANPGGPIYFLKGLSHLHILNLESNGFDEIPVEVFKDLFELKIIDLGLNNLNTLPASVF
NNQVSLKSLNLQKNLITSVEKKVFGPAFRNLTELDMRFNPFDCTCESIAWFVNWINETH
TNIPELSSHYLCNTPPHYHGFPVRLFDTSSCKDSAPFELFFMINTSILLIFIFIVLLIHFEGW
RISFYWNVSVHRVLGFKEIDRQTEQFEYAAYIIHAYKDKDWVWEHFSSMEKEDQSLKFC
LEERDFEAGVFELEAIVNSIKRSRKIIFVITHHLLKDPLCKRFKVHHAVQQAIEQNLDSIILV
FLEEIPDYKLNHALCLRRGMFKSHCILNWPVQKERIGAFRHKLQVALGSKNSVH

SEQ ID No:239
MESAIAEGGASRFSASSGGGGSRGAPQHYPKTAGNSEFLGKTPGQNAQKWIPARSTR

RDDNSAANNSANEKERHDAIFRKVRGILNKLTPEKFDKLCLELLNVGVESKLILKGVILLIV
DKALEEPKYSSLYAQLCLRLAEDAPNFDGPAAEGQPGQKQSTTFRRLLISKLQDEFENR
TRNVDVYDKRENPLLPEEEEQRAIAKIKMLGNIKFIGELGKLDLIHESILHKCIKTLLEKKK
RVQLKDMGEDLECLCQIMRTVGPRLDHERAKSLMDQYFARMCSLMLSKELPARIRFLL
QDTVELREHHWVPRKAFLDNGPKTINQIRQDAVKDLGVFIPAPMAQGMRSDFFLEGPF
MPPRMKMDRDPLGGLADMFGQMPGSGIGTGPGVIQDRFSPTMGRHRSNQLFNGHGG
HIMPPTQSQFGEMGGKFMKSQGLSQLYHNQSQGLLSQLQGQSKDMPPRFSKKGQLN
ADEISLRPAQSFLMNKNQVPKLQPQITMIPPSAQPPRTQTPPLGQTPQLGLKTNPPLIQE
KPAKTSKKPPPSKEELLKLTETVVTEYLNSGNANEAVNGVREMRAPKHFLPEMLSKVIIL
SLDRSDEDKEKASSLISLLKQEGIATSDNFMQAFLNVLDQCPKLEVDIPLVKSYLAQFAA
RAIISELVSISELAQPLESGTHFPLFLLCLQQLAKLQDREWLTELFQQSKVNMQKMLPEID
QNKDRMLEILEGKGLSFLFPLLKLEKELLKQIKLDPSPQTIYKWIKDNISPKLHVDKGFVNI
LMTSFLQYISSEVNPPSDETDSSSAPSKEQLEQEKQLLLSFKPVMQKFLHDHVDLQVSA
LYALQVHCYNSNFPKGMLLRFFVHFYDMEIIEEEAFLAWKEDITQEFPGKGKALFQVNQ
WLTWLETAEEEESEEEAD

SEQ ID No:240
MLMKKNASFEDFFILLGFSNWPHLEVVLFVVILIFYLITLIGNLFIIILSYLDSHLHTPMYFFL
SNLSFLDLCYTTSSIPQLLVNLWGPEKTISYAGCTVQLYFVLALGTAECVLLVVMSYDRY
AAVCRPLHYTVLMHPRFCRLLAAASWVSGFTTSALHSSFTFWIPLCRHRLVDHFFCEVP
ALLRLSCVDTQANELTLMVMSSIFVLIPLILILTSYGAIARAVLSMQSTTGLQKVLRTCGAH
LMVVSLFFIPVMCMYLQPPSENSQDQGKFIALFYTVVTPSLNPLIYTFRNKDVRGAVKRL
MGWEWGM

SEQ ID No:241




SEQ ID No:242
LLPTALLLVTTRAAAPGRLQGRLCSPQCAEVRGFSDKDKKKYFYKFFRDERRAERAYR
FVKENETLFALCFVPFVCWIVCTVLRQQLELGRDLSRTSKTTTSVYLLFITSVLSSAPVAD
GPRLQGDLRNLCRLAREGVLGRRAQFAEKELEQLELRGSKVQTLFLSKKELPGVLETE
VTYQFIDQSFQEFLAALSYLLEDGGVPRTAAGGVGTLLRGDAQPHSHLVLTTRFLFGLL

SAERMRDIERHFGCMVSERVKQEALRWVQGQGQGCPGVAPEVTEGAKGLEDTEEPE
EEEEGEEPNYPLELLYCLYETQEDAFVRQALCRFPELALQRVRFCRMDVAVLSYCVRC
CPAGQALRLISCRLVAAQEKKKKSLGKRLQASLGGGSSQGTTKQLPASLLHPLFQAMT
DPLCHLSSLTLSHCKLPDAVCRDLSEALRAAPALTELGLLHNRLSEAGLRMLSEGLAWP
QCRVQTVRVQLPDPQRGLQYLVGMLRQSPALTTLDLSGCQLPAPMVTYLCAVLQHQG
CGLQTLSLASVELSEQSLQELQAVKRAKPDLVITH

SEQ ID No:243
MSTASAASSSSSSSAGEMIEAPSQVLNFEEIDYKEIEVEEVVGRGAFGVVCKAKWRAKD
VAIKQIESESERKAFIVELRQLSRVNHPNIVKLYGACLNPVCLVMEYAEGGSLYNVLHGA
EPLPYYTAAHAMSWCLQCSQGVAYLHSMQPKALIHRDLKPPNLLLVAGGTVLKICDFGT
ACDIQTHMTNNKGSAAWMAPEVFEGSNYSEKCDVFSWGIILWEVITRRKPFDEIGGPAF
RIMWAVHNGTRPPLIKNLPKPIESLMTRCWSKDPSQRPSMEEIVKIMTHLMRYFPGADE
PLQYPCQYSDEGQSNSATSTGSFMDIASTNTSNKSDTNMEQVPATNDTIKRLESKLLKN
QAKQQSESGRLSLGASRGSSVESLPPTSEGKRMSADMSEIEARIAATTGNGQPRRRSI
QDLTVTGTEPGQVSSRSSSPSVRMITTSGPTSEKPTRSHPWTPDDSTDTNGSDNSIPM
AYLTLDHQLQPLAPCPNSKESMAVFEQHCKMAQEYMKVQTEIALLLQRKQELVAELDQ
DEKDQQNTSRLVQEHKKLLDENKSLSTYYQQCKKQLEVIRSQQQKRQGTS

SEQ ID No:244













SEQ ID No:245
MERLAKQLKLEKEELERLKSEVNGMEHDLMQRRLRRVSCTTAIPTPEEMTRLRSMNRQ
LQINVDCTLKEVDLLQSREVYYSRSSLKLSFTQLTRLEMVFIFCGKYTD

SEQ ID No:246
MDTPPLSDSESESDESLVTDRELQDAFSRGLLKPGLNVVLEGPKKAVNDVNGLKQCLA
EFKRDLEWVERLDVTLGPVPEIGGSEAPAPQNKDQKAVDPEDDFQREMSFYRQAQAA
VLAVLPRLHQLKVPTKRPTDYFAEMAKSDLQVQKIRQKLQTKQAAMERSEKAKQLRAL
RKYGKKVQTEVLQKRQQEKAHMMNAIKKYQKGFSDKLDFLEGDQKPLAQRKKAGAKG
QQMRKGPSAKRRYKNQKFGFGGKKKGSKWNTRESYDDVSSFRAKTAHGRGLKRPGK
KGSNKRPGKRTREKMKNRTH

SEQ ID No:247
MAQKMDCGAGLLGFQAEASVEDSALLMQTLMEAIQISEAPPTNQATAAASPQSSQPPT
ANEMADIQVSAAAARPKSAFKVQNATTKGPNGVYDFSQAHNAKDVPNTQPKAAFKSQ
NATSKGPNAAYDFSQAATTGELAANKSEMAFKAQNATTKVGPNATYNFSQSLNANDLA
NSRPKTPFKAWNDTTKAPTADTQTQNVNQAKMATSQADIETDPGISEPDGATAQTSAD
GSQAQNLESRTIIRGKRTRKINNLNVEENSSGDQRRAPLAAGTWRSAPVPVTTQNPPG
APPNVLWQTPLAWQNPSGWQNQTARQTPPARQSPPARQTPPAWQNPVAWQNPVIW
PNPVIWQNPVIWPNPIVWPGPVVWPNPLAWQNPPGWQTPPGWQTPPGWQGPPDWQ
GPPDWPLPPDWPLPPDWPLPTDWPLPPDWIPADWPIPPDWQNLRPSPNLRPSPNSRA
SQNPGAAQPRDVALLQERANKLVKYLMLKDYTKVPIKRSEMLRDIIREYTDVYPEIIERA
CFVLEKKFGIQLKEIDKEEHLYILISTPESLAGILGTTKDTPKLGLLLVILGVIFMNGNRASE
AVLWEALRKMGLRPGVRHPLLGDLRKLLTYEFVKQKYLDYRRVPNSNPPEYEFLWGLR
SYHETSKMKVLRFIAEVQKRDPRDWTAQFMEAADEALDALDAAAAEAEARAEARTRM
GIGDEAVSGPWSWDDIEFELLTWDEEGDFGDPWSRIPFTFWARYHQNARSRFPQTFA
GPIIGPGGTASANFAANFGAIGFFWVE

SEQ ID No:248









SEQ ID No:249
RRMQYNRRFVNVVPTFGKKKGTTFTKIFVGGLPYHTTDASLRKYFEGFGDIEEAVVITD
RQTGKSRGYGFVTMADRAAAERACKDPNPIIDGRKANVNLAYLGAKPWCLQTGFAIGV
QQLHPTLIQRTYGLTPHYIYPPAIVQPSVVIPAAPVPSLSSPYIEYTPASPVYAQYPPATY
DQYPYAASPATADSFVGYSYPAAVHQALSAAAPAGTTFVQYQAPQLQPDRMQ

SEQ ID No:250
MTATTRGSPVGGNDNQGQAPDGQSLPPLQQNQTSSPDSSNENSPATPPDEQGQGDA
PPQLEDEEPAFPHTDLAKLDDMINRPRWVVPVLPKGELEVLLEAAIDLSKKGLDVKSEA
CQRFFRDGLTISFTKILTDEAVSGWKFEIHRCLVELCVAKLSQDWFPLLELLAMALNPHC
KFHIYNGTRPCESVSSSVQLPEDELFARSPDPRSPKGWLVDLLNKFGTLNGFQILHDRFI
NGSALNVQIIAALIKPFGQCYEFLTLHTVKKYFLPIIEMVPQFLENLTDEELKKEAKNEAKN
DALSMIIKSLKNLASRVPGQEETVKNLEIFRLKMILRLLQISSFNGKMNALNEVNKVISSVS
YYTHRHGNPEEEEWLTAERMAEWIQQNNILSIVLRDSLHQPQYVEKLEKILRFVIKEKAL
TLQDLDNIWAAQAGKHEAIVKNVHDLLAKLAWDFSPEQLDHPFDCFKASRTNASKKQR
EKLLELIRRLAEDDKDGVMAHRVLNLLWNLAHSDDVPVDIMDLALSAHIKILDYSCSQDR
DTQKIQWIDRFIEELRTNDKWVIPALKQIREICSLFGEAPQNLSQTQRSPHVFYRHDLINQ
LQHNHALVTLVAENLVTYMESMRLYARDHEDYDPQTVRLGSRYSHVQEVQERLNFLRF
LLKDGQLWLCAPQAKQIWKCLAENAVYLCDREACFKWYSKLMGDEPDLDPDINKDFFE
SNVLQLDPSLLTENGMKCFERFFKAVNCREGKLVAKRRAYMMDDLELIGLDYLWRVVI
QSNDDIASRAIDLLKEIYTNLGPRLQVNQVVIHEDFIQSCFDRLKASYDTLCVLDGDKDSV
NCARQEAVRMVRVLTVLREYINECDSDYHEERTILPMSRAFRGKHLSFVVRFPNQGRQ
VDDLEVWSHTNDTIGSVRRCILNRIKANVAHTKIELFVGGELIDPADDRKLIGQLNLKDKS
LITAKLTQISSNMPSSPDSSSDSSTGSPGNHGNHYSDGPNPEVESCLPGVIMSLHPRYI
SFLWQVADLGSSLNMPPLRDGARVLMKLMPPDSTTIEKLRAICLDHAKLGESSLSPSLD
SLFFGPSASQVLYLTEVVYALLMPAGAPLADDSSDFQFHFLKSGGLPLVLSMLTRNNFL
PNADMETRRGAYLNALKIAKLLLTAIGYGHVRAVAEACQPGVEGVNPMTQINQVTHDQA
VVLQSALQSIPNPSSECMLRNVSVRLAQQISDEASRYMPDICVIRAIQKIIWASGCGSLQL
VFSPNEEITKIYEKTNAGNEPDLEDEQVCCEALEVMTLCFALIPTALDALSKEKAWQTFII
DLLLHCHSKTVRQVAQEQFFLMCTRCCMGHRPLLFFITLLFTVLGSTARERAKHSGDYF
TLLRHLLNYAYNSNINVPNAEVLFNNEIDWLKRIRDDVKRTGETGIEETILEGHLGVTKEL

LAFQTSEKKFHIGCEKGGANLIKELIDDFIFPASNVYLQYMRNGELPAEQAIPVCGSPPTI
NAGFELLVALAVGCVRNLKQIVDSLTEMYYIGTAITTCEALTEWEYLPPVGPRPPKGFVG
LKNAGATCYMNSVIQQLYMIPSIRNGILAIEGTGSDVDDDMSGDEKQDNESNVDPRDDV
FGYPQQFEDKPALSKTEDRKEYNIGVLRHLQVIFGHLAASRLQYYVPRGFWKQFRLWG
EPVNLREQHDALEFFNSLVDSLDEALKALGHPAMLSKVLGGSFADQKICQGCPHRYEC
EESFTTLNVDIRNHQNLLDSLEQYVKGDLLEGANAYHCEKCNKKVDTVKRLLIKKLPPVL
AIQLKRFDYDWERECAIKFNDYFEFPRELDMEPYTVAGVAKLEGDNVNPESQLIQQSEQ
SESETAGSTKYRLVGVLVHSGQASGGHYYSYIIQRNGGDGERNRWYKFDDGDVTECK
MDDDEEMKNQCFGGEYMGEVFDHMMKRMSYRRQKRWWNAYIPFYERMDTIDQDDE
LIRYISELAITTRPHQIIMPSAIERSVRKQNVQFMHNRMQYSMEYFQFMKKLLTCNGVYL
NPPPGQDHLLPEAEEITMISIQLAARFLFTTGFHTKKVVRGSASDWYDALCILLRHSKNV
RFWFAHNVLFNVSNRFSEYLLECPSAEVRGAFAKLIVFIAHFSLQDGPCPSPFASPGPS
SQAYDNLSLSDHLLRAVLNLLRREVSEHGRHLQQYFNLFVMYANLGVAEKTQLLKLSVP
ATFMLVSLDEGPGPPIKYQYAELGKLYSVVSQLIRCCNVSSRMQSSINGNPPLPNPFGD
PNLSQPIMPIQQNVADILFVRTSYVKKIIEDCSNSEETVKLLRFCCWENPQFSSTVLSELL
WQVAYSYPYELRPYLDLLLQILLIEDSWQTHRIHNALKGIPDDRDGLFDTIQRSKNHYQK
RAYQCIKCMVALFSNCPVAYQILQGNGDLKRKWTWAVEWLGDELERRPYTGNPQYTY
NNWSPPVQSNETSNGYFLERSHSARMTLAKACELCPEEEPDDQDAPDEHESPPPEDA
PLYPHSPGSQYQQNNHVHGQPYTGPAAHHMNNPQRTGQRAQENYEGSEEVSPPQTK
DQ

SEQ ID No:251
PSPEAGGGGGALKASSARAAAAGLLREAGSGGRERADWRRRQLRKVRSVELDQLPE
QPLFLAASPPASSTSPSPEPADAAGSGTGFQPVAVPPPHGAASRRGAHLTESVAAPDS
GASSPAAAEPGEKRAPAAEPSPAAAPAGREMENKETLKGLHKMDDRPEERMIREKLKA
TCMPAWKHEWLERRNRRGPVVVKPIPVKGDGSEMNHLAAESPGEVQASAASPASKG
RRSPSPGNSPSGRTVKSESPGVRRKRVSPVPFQSGRITPPRRAPSPDGFSPYSPEETN
RRVNKVMRARLYLLQQIGPNSFLIGGDSPDNKYRVFIGPQNCSCAHGTFCIHLLFVMLR
VFQLEPSDPMLWRKTLKNFEVESLFQKYHSRRSSRIKAPSRNTIQKFVSRMSNSHTLSS
SSTSTSSSENSIKDEEEQMCPICLLGMLDEESLTVCEDGCRNKLHHHCMSIWAEECRR
NREPLICPLCRSKWRSHDFYSHELSSPVDSPSSLRAAQQQTVQQQPLAGSRRNQESN
FNLTHYGTQQIPPAYKDLAEPWIQVFGMELVGCLFSRNWNVREMALRRLSHDVSGALL
LANGESTGNSGGSSGSSPSGGATSGSSQTSISGDVVEACCSVLSMVCADPVYKVYVA
ALKTLRAMLVYTPCHSLAERIKLQRLLQPVVDTILVKCADANSRTSQLSISTLLELCKGQA

GELAVGREILKAGSIGIGGVDYVLNCILGNQTESNNWQELLGRLCLIDRLLLEFPAEFYPH
IVSTDVSQAEPVEIRYKKLLSLLTFALQSIDNSHSMVGKLSRRIYLSSARMVTTVPHVFSK
LLEMLSVSSVSTHFTRMRRRLMAYADEVEIAEAIQLGVEDTLQRQQHNSFCRHLFPTTI
WKPQRTVPLECTVHLEKTGKGLCATKLSASSEDISERLARISVGPSSSTTTTTTTTEQPK
PMVQTKGRPHSQCLNSSPLSHHSQLMFPALSTPSSSTPSVPAGTATDVSKHRLQGFIP
CRIPSASPQTQRKFSLQFHRNCPENKDSDKLSPVFTQSRPLPSSNIHRPKPSRPTPGNT
SKQGDPSKNSMTLDLNSSSKCDDSFGLSSNSSNCCYTSDETVFTPVEEKCRLDVNTEL
NSSIEDLLEASMPSSDTTVTFKSEVAVLSPEKAENDDTYKDDVNHNQKCKEKMEAEEE
EALAIAMAMSASQVALPIVPQLQVENGEDIIIIQQDTPETLPGHTKAKQPYREDTEWLKG
QQIGLGAFSSCYQAQDVGTGTLMAVKQVTYVRNTSSEQEEVVEALREEIRMMSHLNHP
NIIRMLGATCEKSNYNLFIEWMAGGSVAHLLSKYGAFKESVVINYTEQLLRGLSYLHENQ
IIHRDVKGANLLIDSTGQRLRIADFGAAARLASKGTGAGEFQGQLLGTIAFMAPEVLRGQ
QYGRSCDVWSVGCAIIEMACAKPPWNAEKHSNHLALIFKIASATTAPSIPSHLSPGLRDV
ALRCLELQPQDRPPSRELLKHPVFRTTW

SEQ ID No:252
MLFVDDMAEPRETPEHPLKQIKFLLGRKEEEAVLVGGEWSPSLDGLDPQADPQVLVRT
AIRCAQAQTGIDLSGCTKWWRFAEFQYLQPGPPRRLQTVVVYLPDVWTIMPTLEEWEA
LCQQKAAEAAPPTQEAQGETEPTEQAPDALEQAADTSRRNAETPEATTQQETDTDLPE
APPPPLEPAVIARPGCVNLSLHGIVEDRRPKERISFEVMVLAELFLEMLQRDFGYRVYK
MLLSLPEKVVSPPEPEKEEAAKEEATKEEEAIKEEVVKEPKDEAQNEGPATESEAPLKE
DGLLPKPLSSGGEEEEKPRGEASEDLCEMALDPELLLLRDDGEEEFAGAKLEDSEVRS
VASNQSEMEFSSLQDMPKELDPSAVLPLDCLLAFVFFDANWCGYLHRRDLERILLTLGI
RLSAEQAKQLVSRVVTQNICQYRSLQYSRQEGLDGGLPEEVLFGNLDLLPPPGKSTKP
GAAPTEHKALVSHNGSLINVGSLLQRAEQQDSGRLYLENKIHTLELKLEESHNRFSATE
VTNKTLAAEMQELRVRLAEAEETARTAERQKSQLQRLLQELRRRLTPLQLEIQRVVEKA
DSWVEKEEPAPSN

SEQ ID No:253
MSLHGKRKEIYKYEAPWTVYAMNWSVRPDKRFRLALGSFVEEYNNKVQLVGLDEESS
EFICRNTFDHPYPTTKLMWIPDTKGVYPDLLATSGDYLRVWRVGETETRLECLLNNNKN
SDFCAPLTSFDWNEVDPYLLGTSSIDTTCTIWGLETGQVLGRVNLVSGHVKTQLIAHDK
EVYDIAFSRAGGGRDMFASVGADGSVRMFDLRHLEHSTIIYEDPQHHPLLRLCWNKQD

PNYLATMAMDGMEVVILDVRVPCTPVARLNNHRACVNGIAWAPHSSCHICTAADDHQA
LIWDIQQMPRAIEDPILAYTAEGEINNVQWASTQPDWIAICYNNCLEILRV

SEQ ID No:254
MDMGNQHPSISRLQEIQKEVKSVEQQVIGFSGLSDDKNYKKLERILTKQLFEIDSVDTEG
KGDIQQARKRAAQETERLLKELEQNANHPHRIEIQNIFEEAQSLVREKIVPFYNGGNCVT
DEFEEGIQDIILRLTHVKTGGKISLRKARYHTLTKICAVQEIIEDCMKKQPSLPLSEDAHPS
VAKINFVMCEVNKARGVLIALLMGVNNNETCRHLSCVLSGLIADLDALDVCGRTEIRNYR
REVVEDINKLLKYLDLEEEADTTKAFDLRQNHSILKIEKVLKRMREIKNELLQAQNPSELY
LSSKTELQGLIGQLDEVSLEKNPCIREARRRAVIEVQTLITYIDLKEALEKRKLFACEEHPS
HKAVWNVLGNLSEIQGEVLSFDGNRTDKNYIRLEELLTKQLLALDAVDPQGEEKCKAAR
KQAVRLAQNILSYLDLKSDEWEY

SEQ ID No:255
MSCVKLWPSGAPAPLVSIEELENQELVGKDGFGTVFRAQHRKWGYDVAVKIVNSKAIS
REVKAMASLDNEFVLRLEGVIEKVNWDQDPKPALVTKFMENGSLSGLLQSQCPRPWPL
LCRLLKEVVLGMFYLHDQNPVLLHRDLKPSNVLPDPELHVKLADFGLSTFQGGSQSGT
GSGEPGGTLGYLAPELFVNVNRKASTASDVYSFGILMWAVLAGREVELPTEPSLVYEAV
CNRQNRPSLAELPQAGPETPGLEGLKELMQLCWSSEPKDRPSFQECLPKTDEVFQMV
ENNMNAAVSTVKDFLSQLKSSNRRFSIPESGQGGTEMDGFRRTIENQHSRNDVMVSE
WLNKLNLEEPPSSVPKKCPSLTKRSRAQEEQVPQAWTAGTSSDSMAQPPQTPETSTF
RNQMPSPTSTGTPSPGPRGNQGAERQGMNWSCRTPEPNPVTGRPLVNIYNCSGVQV
GDNNYLTMQQTTALPTWGLAPSGKGRGLQHPPPVGSQEGPKDPEAWSRPQGWYNH
SGK

SEQ ID No:256
MDFSRNLYDIGEQLDSEDLASLKFLSLDYIPQRKQEPIKDALMLFQRLQEKRMLEESNLS
FLKELLFRINRLDLLITYLNTRKEEMERELQTPGRAQISAYRVMLYQISEEVSRSELRSFK
FLLQEEISKCKLDDDMNLLDIFIEMEKRVILGEGKLDILKRVCAQINKSLLKIINDYEEFSKE
RSSSLEGSPDEFSNGEELCGVMTISDSPREQDSESQTLDKVYQMKSKPRGYCLIINNH
NFAKAREKVPKLHSIRDRNGTHLDAGALTTTFEELHFEIKPHDDCTVEQIYEILKIYQLMD
HSNMDCFICCILSHGDKGIIYGTDGQEAPIYELTSQFTGLKCPSLAGKPKVFFIQACQGD
NYQKGIPVETDSEEQPYLEMDLSSPQTRYIPDEADFLLGMATVNNCVSYRNPAEGTWYI
QSLCQSLRERCPRGDDILTILTEVNYEVSNKDDKKNMGKQMPQPTFTLRKKLVFPSD

SEQ ID No:257















SEQ ID No:258
MAYSEEHKGMPCGFIRQNSGNSISLDFEPSIEYQFVERLEERYKCAFCHSVLHNPHQT
GCGHRFCQHCILSLRELNTVPICPVDKEVIKSQEVFKDNCCKREVLNLYVYCSNAPGCN
AKVILGRYQDHLQQCLFQPVQCSNEKCREPVLRKDLKEHLSASCQFRKEKCLYCKKDV
VVINLQNHEENLCPEYPVFCPNNCAKIILKTEVDEHLAVCPEAEQDCPFKHYGCAVTDK
RRNLQQHEHSALREHMRLVLEKNVQLEEQISDLHKSLEQKESKIQQLAETIKKLEKEFKQ
FAQLFGKNGSFLPNIQVFASHIDKSAWLEAQVHQLLQMVNQQQNKFDLRPLMEAVDTV
KQKITLLENNDQRLAVLEEETNKHDTHINIHKAQLSKNEERFKLLEGTCYNGKLIWKVTD
YKMKKREAVDGHTVSIFSQSFYTSRCGYRLCARAYLNGDGSGRGSHLSLYFVVMRGE
FDSLLQWPFRQRVTLMLLDQSGKKNIMETFKPDPNSSSFKRPDGEMNIASGCPRFVAH
SVLENAKNAYIKDDTLFLKVAVDLTDLEDL

SEQ ID No:259
MTMPVNGAHKDADLWSSHDKMLAQPLKDSDVEVYNIIKKESNRQRVGLELIASENFAS
RAVLEALGSCLNNKYSEGYPGQRYYGGTEFIDELETLCQKRALQAYKLDPQCWGVNV
QPYSGSPANFAVYTALVEPHGRIMGLDLPDGGHLTHGFMTDKKKISATSIFFESMPYKV

NPDTGYINYDQLEENARLFHPKLIIAGTSCYSRNLEYARLRKIADENGAYLMADMAHISG
LVAAGVVPSPFEHCHVVTTTTHKTLRGCRAGMIFYRKGVKSVDPKTGKEILYNLESLINS
AVFPGLQGGPHNHAIAGVAVALKQAMTLEFKVYQHQVVANCRALSEALTELGYKIVTGG
SDNHLILVDLRSKGTDGGRAEKVLEACSIACNKNTCPGDRSALRPSGLRLGTPALTSRG
LLEKDFQKVAHFIHRGIELTLQIQSDTGVRATLKEFKERLAGDKYQAAVQALREEVESFA
SLFPLPGLPDF

SEQ ID No:260
MQSELCFADIHDSDGSSSSSHQSLKSTAKWAASLENLLEDPEGVKRFREFLKKEFSEE
NVLFWLACEDFKKMQDKTQMQEKAKEIYMTFLSSKASSQVNVEGQSRLNEKILEEPHP
LMFQKLQDQIFNLMKYDSYSRFLKSDLFLKHKRTEEEEEDLPDAQTAAKRASRIYNT

SEQ ID No:261
MNEKWDTNSSENWHPIWNVNDTKHHLYSDINITYVNYYLHQPQVAAIFIISYFLIFFLCM
MGNTVVCFIVMRNKHMHTVTNLFILNLAISDLLVGIFCMPITLLDNIIAGWPFGNTMCKISG
LVQGISVAASVFTLVAIAVDRFQCVVYPFKPKLTIKTAFVIIMIIWVLAITIMSPSAVMLHVQ
EEKYYRVRLNSQNKTSPVYWCREDWPNQEMRKIYTTVLFANIYLAPLSLIVIMYGRIGISL
FRAAVPHTGRKNQEQWHVVSRKKQKIIKMLLIVALLFILSWLPLWTLMMLSDYADLSPNE
LQIINIYIYPFAHWLAFGNSSVNPIIYGFFNENFRRGFQEAFQLQLCQKRAKPMEAYALKA
KSHVLINTSNQLVQESTFQNPHGETLLYRKSAEKPQQELVMEELKETTNSSEI

SEQ ID No:262
MQWLRVRESPGEATGHRVTMGTAALGPVWAALLLFLLMCEIPMVELTFDRAVASGCQ
RCCDSEDPLDPAHVSSASSSGRPHALPEIRPYINITILKGDKGDPGPMGLPGYMGREGP
QGEPGPQGSKGDKGEMGSPGAPCQKRFFAFSVGRKTALHSGEDFQTLLFERVFVNLD
GCFDMATGQFAAPLRGIYFFSLNVHSWNYKETYVHIMHNQKEAVILYAQPSERSIMQSQ
SVMLDLAYGDRVWVRLFKRQRENAIYSNDFDTYITFSGHLIKAEDD

SEQ ID No:263
MWTLVSWVALTAGLVAGTRCPDGQFCPVACCLDPGGASYSCCRPLLDKWPTTLSRHL
GGPCQVDAHCSAGHSCIFTVSGTSSCCPFPEAVACGDGHHCCPRGFHCSADGRSCF
QRSGNNSVGAIQCPDSQFECPDFSTCCVMVDGSWGCCPMPQASCCEDRVHCCPHG
AFCDLVHTRCITPTGTHPLAKKLPAQRTNRAVALSSSVMCPDARSRCPDGSTCCELPS
GKYGCCPMPNATCCSDHLHCCPQDTVCDLIQSKCLSKENATTDLLTKLPAHTVGDVKC

DMEVSCPDGYTCCRLQSGAWGCCPFTQAVCCEDHIHCCPAGFTCDTQKGTCEQGPH
QVPWMEKAPAHLSLPDPQALKRDVPCDNVSSCPSSDTCCQLTSGEWGCCPIPEAVCC
SDHQHCCPQGYTCVAEGQCQRGSEIVAGLEKMPARRASLSHPRDIGCDQHTSCPVGG
TCCPSLGGSWACCQLPHAVCCEDRQHCCPAGYTCNVKARSCEKEVVSAQPATFLARS
PHVGVKDVECGEGHFCHDNQTCCRDNRQGWACCPYRQGVCCADRRHCCPAGFRCA
ARGTKCLRREAPRWDAPLRDPALRQLL

SEQ ID No:264
MAPLCPSPWLPLLIPAPAPGLTVQLLLSLLLLMPVHPQRLPRMQEDSPLGGGSSGEDDP
LGEEDLPSEEDSPREEDPPGEEDLPGEEDLPGEEDLPEVKPKSEEEGSLKLEDLPTVEA
PGDPQEPQNNAHRDKEGDDQSHWRYGGDPPWPRVSPACAGRFQSPVDIRPQLAAFC
PALRPLELLGFQLPPLPELRLRNNGHSVQLTLPPGLEMALGPGREYRALQLHLHWGAA
GRPGSEHTVEGHRFPAEIHVVHLSTAFARVDEALGRPGGLAVLAAFLEEGPEENSAYE
QLLSRLEEIAEEGSETQVPGLDISALLPSDFSRYFQYEGSLTTPPCAQGVIWTVFNQTVM
LSAKQLHTLSDTLWGPGDSRLQLNFRATQPLNGRVIEASFPAGVDSSPRAAEPVQLNS
CLAAGDILALVFGLLFAVTSVAFLVQMRRQHRRGTKGGVSYRPAEVAETGA

SEQ ID No:265






SEQ ID No:266














SEQ ID No:267









SEQ ID No:268 citrate synthase
MALLTAAARLLGTKNASCLVLAARHASASSTNLKDILADLIPKEQARIKTFRQQHGKTVV
GQITVDMMYGGMRGMKGLVYETSVLDPDEGIRFRGFSIPECQKLLPKAKGGEEPLPEG
LFWLLVTGCIPTEEQVSWLSKEWAKRAALPSHVVTMLDNFPTNLHPMSQLSAAVTALN
SESNFAQAYARGISRTKYWELIYEDSVDLIAKLPCVAAKIYRNLYWEGSGIGAIDSNLDW
SHNFTNMLGYTDHQFTELMRLYLTIHSDHEGGNVSAHTSHLVGSALSDPYLSFAAAMN
GLAGPLHGLANQEVLVWLTQLQKEVGKDVSDEKLRDYIWNTLNSGRVVPGYGHAVLR
KTDPRYTCQREFALKHLPNDPMFKLVAQLYKIVPNVLLEQGKAKNPWPNVDAHSGVLL
QYYGMTEMNYYTVLFGVSRALGVLAQLIWSRALGFPLERPKSMSTEGLMKFVDSKSG

## Claims

1. A protein complex selected from complex (I) and comprising
(a) at least one first protein, which first protein is selected from the group of proteins in table 1, seventh column of a given complex, or a functionally active derivative thereof, or a functionally active fragment thereof, or a homolog thereof, or a variant of said protein encoded by a nucleic acid that hybridizes to the nucleic acid encoding said protein under low stringency conditions; and
(b) at least one second protein, which second protein is selected from the group of proteins in table 1, eighth column of said complex, or a functionally active derivative thereof, or a functionally active fragment thereof, or a homolog thereof, or a variant of said second protein encoded by a nucleic acid that hybridizes to the nucleic acid encoding said protein under low stringency conditions;
and a complex (II) comprising at least two of said second proteins,
wherein said low stringency conditions comprise hybridization in a buffer comprising 35% formamide, 5X SSC, 50mM Tris-HCl (pH 7.5), 5 mM EDTA, 0.02% PVP, 0.02% BSA, 100 µg/ml denatured salmon sperm DNA, and 10% (wt/vol) dextran sulfate for 18-20 hours at 40°C, washing in a buffer containing 2X SSC, 25 mM Tris-HCl (pH 7.4), 5 mM EDTA, and 0.1 % SDS for 1-5 hours at 55°C, and washing in a buffer containing 2X SSC, 26 mM Tris-HCl (pH 7.4), 5 mM EDTA, and 0.1% SDS for 1.5 hours at 60°C.

2. A protein complex comprising a first protein selected from the proteins listed in table 1, second column of a given complex or a homolog or variant thereof, or a functionally active fragment or functionally active derivative of said first protein, the variant being encoded by a nucleic acid that hybridizes to the nucleic acid of said first protein under low stringency conditions, and at least one second protein selected from the group of proteins in table 1, eighth column of a given complex, or a variant or homolog thereof, or a functionally active fragment or a functionally active derivative of said second protein, the variant of said second protein being encoded by a nucleic acid that hybridizes to the nucleic acid of said second protein under low-stringency conditions, and wherein said low stringency conditions comprise hybridization in a buffer comprising 35% formamide, 5X SSC, 50mM Tris-HCl (pH 7.5), 5 mM EDTA, 0.02% PVP, 0.02% BSA, 100 µg/ml denatured salmon sperm DNA, and 10% (wt/vol) dextran sulfate for 18-20 hours at 40°C, washing in a buffer containing 2X SSC, 25 mM Tris-HCl (pH 7.4), 5 mM EDTA, and 0.1% SDS for 1-5 hours at 55°C, and washing in a buffer containing 2X SSC, 26 mM Tris-HCl (pH 7.4) 5 mM EDTA, and 0.1 % SDS for 1.5 hours at 60°C.

3. A protein complex selected from complex (I) and comprising the proteins from table 1, third column of a given complex at least one of said proteins can be a homolog, or a variant or functionally active fragment or functionally active derivative of of at least one of said proteins, said variant being encoded by a nucleic acid that hybridizes to the nucleic acid of said protein under low stringency conditions; and a protein complex selected from complex (II) and comprising the proteins selected from table 1, fourth column at least one of said proteins can be a homolog, or variant or functionally active fragment or functionally active derivative of az least one of said proteins, the variant being encoded by a nucleic acid that hybridizes to the nucleic acid of said protein under low stringency conditions, to the nucleic acid of said protein under low stringency conditions;
and a protein complex selected from complex (III) and comprising the proteins selected from table 1, fifth column at least one of said proteins can be a homolog, or variant or functionally active fragment or functionally active derivative of said at least one proteins, the variant being encoded by a nucleic acid that hybridizes to the nucleic acid of said protein under low stringency conditions;
and a protein complex selected from complex (IV) and comprising the proteins selected from the proteins in table 1, sixth column or at least one of said proteins can be a homolog or a variant thereof or a functionally active fragment or a functionally active derivative of said at least one proteins, the variant being encoded by a nucleic acid that hybridizes to the nucleic acid of said protein under low stringency conditions;
wherein said low stringency conditions comprise hybridization in a buffer comprising 35% formamide, 5X SSC, 50mM Tris-HCl (pH 7.5), 5mM EDTA, 0.02% PVP, 0.02% Ficoll, 0.2% BSA, 100 microg/ml denatured salmon sperm DNA, and 10% (wt/vol) dextran sulfate for 18-20 hours at 40°C, washing in a buffer containing 2X SSC, 25mM Tris-HCl (pH 7.4), 5mM EDTA, and 0.1% SDS for 1-5 hours at 55°C, and washing in a buffer containing 2X SSC, 25mM Tris-HCl (pH 7.4), 5mM EDTA, and 0.1% SDS for 1.5 hours at 60°C.

4. A protein complex that comprises all proteins as listed in table 1, sixth column for a given complex or at least one protein of said all proteins is a homolog or a variant thereof, or a functionally active fragment or a functionally active derivative thereof, the variant being encoded by a nucleic acid that hybridizises to the nucleic acid of any of said proteins under low stringency conditions, but 1 to the number of proteins listed in table 1, eighth column of said complex, or a homolog or a variant thereof, or a functionally active fragment or functionally active derivative thereof, the variant being encoded by a nucleic acid that hybridizes to the nucleic acid of any of said proteins of said eigth column under low stringency conditions, wherein said low stringency conditions comprise hybridization in a buffer comprising 35% formamide, 5X SSC, 50mM Tris-HCl (pH 7.5), 5mM EDTA, 0.02% PVP, 0.02% Ficoll, 0.2% BSA, 100 µg/ml denatured salmon sperm DNA, and 10% (wt/vol) dextran sulfate for 18-20 hours at 40°C, washing in a buffer containing 2X SSC, 25mM Tris-HCl (pH 7.4), 5mM EDTA, and 0.1% SDS for 1-5 hours at 55°C, and washing in a buffer containing 2X SSC, 25mM Tris-HCl (pH 7.4), 5mM EDTA, and 0.1% SDS for 1.5 hours at 60°C.

5. The complex of any of claims 1 to 4 comprising at least one functionally active derivative of said first protein and/or a functionally active derivative of said second protein, wherein the functionally active derivative is a fusion protein comprising said first protein or said second protein fused to an amino acid sequence different from the first protein or second protein.

6. The complex of claim 5 wherein the functionally active derivative is a fusion protein comprising said first protein or said second protein fused to an affinity tag or label.

7. The complex of any of claims 1 to 4 comprising a fragment of said first protein and/or a fragment of said second protein, which fragment binds to another protein component of said complex.

8. The complex of any of claims 1 to 7 that is involved in the biochemical activity as stated in table 1, tenth column.

9. A process for preparing a complex of any of claims 1-8 and optionally the components thereof comprising the following steps:
Expressing a protein of the complex, preferably a tagged protein, in a target cell, isolating the protein complex which is attached to the protein, preferably a tagged protein, and optionally disassociating the protein complex and isolating the individual complex members.

10. The process according to claim 9 wherein the tagged protein comprises two different tags which allow two separate affinity purification steps.

11. The process according to any of claims 9-10 wherein the two tags are separated by a cleavage site for a protease.

12. Component of a protein complex obtainable by a process according to any of claims 9-11.

13. Protein selected from the group of proteins in table 1, ninth column of a given complex, a homolog thereof, and a variant of thereof, or a functionally active fragment or a functionally active derivative of said protein, the variant being encoded by a nucleic acid that hybridizes to the nucleic acid encoding said protein under low stringency conditions, wherein said low stringency conditions comprise hybridization in a buffer comprising 35% formamide, 5X SSC, 50 mM Tris-HCl (pH 7.5), 5 mM EDTA, 0.02% PVP, 0.02% Ficoll, 0.2% BSA, 100 µg/ml denatured salmon sperm DNA, and 10% (wt/vol) dextran sulfate for 18-20 hours at 40°C, washing in a buffer containing 2X SSC, 25 mM Tris-HCl (pH 7.4), 5 mM EDTA, and 0.1 % SDS for 1.5 hours at 55°C, and washing in a buffer containing 2X SSC, 25 mM Tris-HCl (pH 7.4), 5 mM EDTA, and 0.1 % SDS for 1.5 hours at 60°C.

14. Nucleic acid encoding a protein according to claim 13.

15. Construct, preferably a vector construct, comprising
(a) a nucleic acid according to claim 14 and at least one further nucleic acid which is normally not associated with said nucleic acid, or
(b) at least two separate nucleic acid sequences each encoding a different protein, or a functionally active fragment or a functionally active derivative thereof, or a homolog or a variant thereof, at least one of said proteins being selected from the first group of proteins according to claim 1 (a) and at least one of said proteins, being selected from the second group of proteins according to claim 1(b).

16. Host cell, containing a vector comprising at least one of the nucleic acid of claim 14 and /or a construct of claim 15 or containing several vectors each comprising at least the nucleic acid encoding at least one protein selected from the first group of proteins according to claim 1(a) and the nucleic acid encoding at least one protein selected from the second group of proteins according to claim 1(b).

17. An antibody or a fragment of said antibody containing the binding domain thereof, which binds the complex of any of claims 1 to 8 and which does not bind any of the proteins of said complex when uncomplexed and/or an antibody of a fragment of said antibody containing the binding domain thereof which binds to any of the group of proteins according to claim 13.

18. A kit comprising in one or more containers the complex of any of claims 1-8 and/or the proteins of claim 13, optionally together with an antibody according to claim 17 and/or further components such as reagents and working instructions.

19. The kit according to claim 18 for processing a substrate of a complex of any one of claims 1-8.

20. The kit according to claim 18 for the diagnosis or prognosis of a disease or a disease risk, preferentially for a disease or disorder such as inflammation, such as chronic inflammatory diseases, rheumatoid arthritis, inflammatory bowel disease; infectious diseases such as septic shock and bacterial infections; neurological diseases such as stroke-induced inflammation in neurons; neurodegenerative diseases; cancer such as prostate cancer, breast cancer and skin cancer.

21. Array in which at least a complex according to any of claims 1 to 8 and/or at least one protein according to claim 13 and/or at least one antibody according to claim 17 is attached to a solid carrier.

22. A process for processing a substrate of a complex of any one of claims 1-8 comprising the step of bringing into contact a complex of any of claims 1-8 with said substrate, such that said substrate is processed.

23. A pharmaceutical composition comprising the protein complex of any of claims 1-8 and/or any of the proteins according to claim 13.

24. A pharmaceutical composition according to claim 23 for the treatment of diseases and disorders, preferentially for diseases or disorders such as inflammation, such as chronic inflammatory diseases, rheumatoid arthritis, inflammatory bowel disease; infectious diseases such as septic shock and bacterial infections; neurological diseases such as stroke-induced inflammation in neurons; neurodegenerative diseases; cancer such as prostate cancer, breast cancer and skin cancer.

25. A method for screening for a molecule that binds to the complex of any one of claims 1-8 and/or any of the proteins of claim 13 , comprising the following steps:
(a) exposing said complex or protein, or a cell or organism containing said complex or said protein, to one or more candidate molecules; and
(b) determining whether said candidate molecule is bound to the complex or protein.

26. A method for screening for a molecule that modulates directly or indirectly the function, activity, composition or formation of the complex of any one of claims 1-8 comprising the steps of:
(a) exposing said complex, or a cell or organism containing said complex to one or more candidate molecules; and
(b) determining the amount of, activity of, protein components of, and/or intracellular localization of said complex and/or the transcription level of a gene dependent on the complex and/or the abundance and/or activity of a protein or protein complex dependent upon the function of the complex and/or product of a gene dependent on the complex in the presence of the one or more candidate molecules, wherein a change in said amount, activity, protein components or intracellular localization relative to said amount, activity, protein components and/or intracellular localization and/or a change in the transcription level of a gene dependent on the complex and/or the abundance and/or activity of a protein or protein complex dependent on the function of the complex and/or product of a gene dependent on the complex in the absence of said candidate molecules indicates that the molecule modulates function, activity, or composition of said complex.

27. The method of claim 26, wherein the amount of said complex is determined.

28. The method of claim 26, wherein the activity of said complex is determined.

29. The method of claim 28, wherein said determining step comprises isolating from the cell or organism said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.

30. The method of claim 26, wherein the amount of the individual protein components of said complex are determined.

31. The method of claim 30, wherein said determining step comprises determining whether any of the proteins listed in table 1, sixth column of said complex, or a functionally active fragment or a functionally active derivative thereof, or a variant or a homolog thereof, the variant being encoded by a nucleic acid that hybridizes to the nucleic acid of said protein under low-stringency conditions, is present in the complex.

32. The method of any of claims 26 to 31, wherein said method is a method of screening for a drug for treatment or prevention of a disease or disorder, preferentially of a disease or disorder selected from the diseases or disorders such as inflammation, such as chronic inflammatory diseases, rheumatoid arthritis, inflammatory bowel disease; infectious diseases such as septic shock and bacterial infections; neurological diseases such as stroke-induced inflammation in neurons; neurodegenerative diseases; cancer such as prostate cancer, breast cancer and skin cancer.

33. Use of a molecule that modulates the amount of, activity of, or the protein components of the complex of any one of claims 1-8 for the manufacture of a medicament for the treatment or prevention of a disease or disorder, preferentially of a disease or disorder such as inflammation, such as chronic inflammatory diseases, rheumatoid arthritis, inflammatory bowel disease; infectious diseases such as septic shock and bacterial infections; neurological diseases such as stroke-induced inflammation in neurons; neurodegenerative diseases; cancer such as prostate cancer, breast cancer and skin cancer.

34. A method for the production of a pharmaceutical composition comprising carrying out the method of claims 26-31 to identify a molecule that modulates the function, activity, composition or formation of said complex, and further comprising mixing the identified molecule with a pharmaceutically acceptable carrier.

35. A method for diagnosing or screening for the presence of a disease or disorder or a predisposition for developing a disease or disorder in a subject, which disease or disorder is **characterized by** an aberrant amount of, component disposition of, or intracellular localization of the complex of any one of the claims 1-8, comprising determining the amount of, activity of, protein components of, and/or intracellular localization of, said complex and/or the transcription level of a gene dependent on the complex and/or the abundance and/or activity of a protein or protein complex dependent on the function of the complex and/or product of a gene dependent on the complex in a comparative sample derived from a subject, wherein a difference in said amount, activity, or protein components of, said complex to an analogous sample from a subject not having the disease or disorder or predisposition indicates the presence in the subject of the disease or disorder or predisposition in the subject.

36. The method of claim 35, wherein the amount of said complex is determined.

37. The method of claim 35, wherein the activity of said complex is determined.

38. The method of claim 37, wherein said determining step comprises isolating from the cell or organism said complex to produce said isolated complex and contacting said isolated complex in the presence or absence of a candidate molecule with a substrate of said complex and determining whether said substrate is processed in the absence of the candidate molecule and whether the processing of said substrate is modified in the presence of said candidate molecule.

39. The method of claim 35, wherein the amount of the individual protein components of said complex is determined.

40. The method of claim 39, wherein said determining step comprises determining whether any of the proteins according to claim 13 is present in the complex.

41. The complex of any one of claims 1-8, or proteins of claim 13 or the antibody or fragment of claim 17, for use in a method of diagnosing a disease or disorder, preferentially of a disease or disorder such as inflammation, such as chronic inflammatory diseases, rheumatoid arthritis, inflammatory bowel disease; infectious diseases such as septic shock and bacterial infections; neurological diseases such as stroke-induced inflammation in neurons; neurodegenerative diseases; cancer such as prostate cancer, breast cancer and skin cancer.

42. A method for treating or preventing a disease or disorder **characterized by** an aberrant amount of, activity of, component composition of or intracellular localization of, the complex of any one of claims 1-8, comprising administering to a subject in need of such treatment or prevention a therapeutically effective amount of one or more molecules that modulate the amount of, activity or, or protein components of, said complex.

43. The method according to claim 42, wherein said disease or disorder involves decreased levels of the amount or activity of said complex.

44. The method according to claim 42, wherein said disease or disorder involves increased levels of the amount or activity of said complex.

45. Complex of claims 1-8 and/or any of the proteins listed in table 1, eighth column of said complex as a target for an active agent of a pharmaceutical, preferably a drug target in the treatment or prevention of a disease or disorder, preferentially of a disease or disorder such as inflammation, such as chronic inflammatory diseases, rheumatoid arthritis, inflammatory bowel disease; infectious diseases such as septic shock and bacterial infections; neurological diseases such as stroke-induced inflammation in neurons; neurodegenerative diseases; cancer such as prostate cancer, breast cancer and skin cancer.
